(19) Europäisches Patentamt
European Patent Office
Office européen des brevets

(11) EP 3 972 649 B1

(12) EUROPEAN PATENT SPECIFICATION

(45) Date of publication and mention of the grant of the patent:
**13.05.2026 Bulletin 2026/20**

(21) Application number: **20730922.0**

(22) Date of filing: **19.05.2020**

(51) International Patent Classification (IPC):
***A61K 47/68*** *(2017.01)*

(52) Cooperative Patent Classification (CPC):
**A61K 47/6889; A61K 47/6803; A61K 47/6867**

(86) International application number:
**PCT/US2020/033602**

(87) International publication number:
**WO 2020/236817 (26.11.2020 Gazette 2020/48)**

(54) **MCL-1 INHIBITOR ANTIBODY-DRUG CONJUGATES AND METHODS OF USE**

MCL-1-INHIBITOR-ANTIKÖRPER-WIRKSTOFF-KONJUGATE UND VERWENDUNGSVERFAHREN

CONJUGUÉS ANTICORPS-MÉDICAMENT INHIBITEURS DE MCL-1 ET MÉTHODES D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.05.2019 US 201962850098 P**

(43) Date of publication of application:
**30.03.2022 Bulletin 2022/13**

(73) Proprietors:
- **Novartis AG**
  **4056 Basel (CH)**
- **Les Laboratoires Servier**
  **92284 Suresnes Cedex (FR)**

(72) Inventors:
- **BURGER, Matthew, T.**
  **Cambridge, MA 02139 (US)**
- **CHANRION, Maia**
  **92130 Issy Les Moulineaux (FR)**
- **COLLAND, Frédéric**
  **95380 Puiseux-En-France (FR)**
- **CSEKEI, Marton**
  **2120 Dunakeszi (HU)**
- **DELACOUR, Lea**
  **1116 Luxembourg (LU)**
- **DESOS, Patrice**
  **92270 Bois Colombes (FR)**
- **GENESTE, Olivier**
  **92500 Rueil-Malmaison (FR)**
- **HENLIN, Jean-Michel**
  **92150 Suresnes (FR)**
- **KOSTOVA, Vesela**
  **92210 Saint Cloud (FR)**
- **KOTSCHY, Andras**
  **2045 Torokbalint (HU)**
- **MARAGNO, Ana, Leticia**
  **78290 Croissy-Sur-Seine (FR)**
- **MCNEILL, Eric**
  **Cambridge, MA 02139 (US)**
- **PALERMO, Mark, G.**
  **Cambridge, MA 02139 (US)**
- **ROCCHETTI, Francesca**
  **75005 Paris (FR)**
- **STARCK, Jérôme**
  **92500 Rueill-Malmaison (FR)**
- **YU, Bing**
  **Cambridge, MA 02139 (US)**
- **ZHANG, Qiang**
  **Cambridge, MA 02139 (US)**
- **PROSZENYÁK, Ágnes**
  **1037 Budapest (HU)**
- **SIPOS, Szabolcs**
  **Budapest 1165 (HU)**
- **CHEN, Zhuoliang**
  **Cambridge, MA 02139 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **NAKAJIMA, Katsumasa**
  **Cambridge, MA 02139 (US)**
- **D'ALESSIO, Joseph, Anthony**
  **Cambridge, MA 02139 (US)**
- **BLANKENSHIP, John, William**
  **Cambridge, MA 02139 (US)**

(74) Representative: **Elkington and Fife LLP**
**Prospect House**
**8 Pembroke Road**
**Sevenoaks, Kent TN13 1XR (GB)**



(56) References cited:
**EP-A1- 2 886 545** **WO-A1-2016/207216**
**WO-A1-2019/035899**

- **CRISTINA L. ABRAHAMS ET AL: "Targeting CD74 in multiple myeloma with the novel, site-specific antibody-drug conjugate STRO-001", ONCOTARGET, vol. 9, no. 102, 28 December 2018 (2018-12-28), pages 37700 - 37714, XP055725165, DOI: 10.18632/oncotarget.26491**
- **S. V. GOVINDAN ET AL: "Milatuzumab-SN-38 Conjugates for the Treatment of CD74+ Cancers", MOLECULAR CANCER THERAPEUTICS, vol. 12, no. 6, 1 June 2013 (2013-06-01), US, pages 968 - 978, XP055280453, ISSN: 1535-7163, DOI: 10.1158/1535-7163.MCT-12-1170**

## Description

### SEQUENCE LISTING

The instant application contains a Sequence Listing which has been submitted electronically in ASCII format and is appended hereto. Said ASCII copy, created on June 25, 2020, is named 132043-00220_SL.txt and is 76,528 bytes in size.

### FIELD OF THE INVENTION

The present disclosure relates to antibody-drug conjugates (ADCs) comprising an Mcl-1 inhibitor and an anti-CD74 antibody or antigen-binding fragment thereof that binds an antigen target, e.g., an antigen expressed on a tumor or other cancer cell. The disclosure further relates to methods and compositions useful in the treatment and/or diagnosis of cancers that express the target antigen CD74 and/or are amenable to treatment by modulating Mcl-1 expression and/or activity, as well as methods of making those compositions. Linker-drug conjugates comprising an Mcl-1 inhibitor drug moiety and methods of making same are also disclosed.

### BACKGROUND OF THE INVENTION

Apoptosis, or programmed cell death, is a physiological process that is crucial for embryonic development and maintenance of tissue homeostasis. Apoptotic-type cell death generally involves morphological changes such as condensation of the nucleus and DNA fragmentation, as well as biochemical changes such as the activation of caspases that can cause damage to key structural components of the cell. Regulation of apoptosis is complex and typically involves the activation or repression of several intracellular signaling pathways (Cory et al. (2002) Nature Review Cancer 2:647-656).

Deregulation of apoptosis is associated with certain pathologies. For instance, increased apoptosis is associated with neurodegenerative diseases such as Parkinson's disease, Alzheimer's disease, and ischemia. Conversely, deficits in apoptosis can play a role in the development of cancers and chemoresistance, autoimmune diseases, inflammatory diseases, and viral infections. The absence of apoptosis is one of the phenotypic signatures of cancer (Hanahan et al. (2000) Cell 100:57-70). Anti-apoptotic proteins of the Bcl-2 family are associated with numerous types of cancer, such as colon cancer, breast cancer, small cell lung cancer, non-small cell lung cancer, bladder cancer, ovarian cancer, prostate cancer, chronic lymphoid leukemia, lymphoma, myeloma, and pancreatic cancer.

Myeloid cell leukemia 1 (Mcl-1), an anti-apoptotic Bcl-2 family member, is a regulator of cell survival. Amplification of the Mcl-1 gene and/or overexpression of the Mcl-1 protein has been observed in multiple cancer types and is commonly implicated in tumor development (Beroukhim et al. (2010) Nature 463(7283):899-905). Mcl-1 is one of the most frequently amplified genes in human cancer and is also a critical survival factor that has been shown to mediate drug resistance to a variety of anti-cancer agents.

Mcl-1 is believed to promote cell survival by binding to and neutralizing the deathinducing activities of pro-apoptotic proteins such as Bim, Noxa, Bak, and Bax. Inhibition of Mcl-1 releases these pro-apoptotic proteins, often leading to the induction of apoptosis in tumor cells dependent on Mcl-1 for survival. Therapeutically targeting Mcl-1 or proteins upstream and/or downstream of it in an apoptotic signaling pathway, therefore, may represent promising strategies to treat various malignancies and to overcome drug resistance in certain human cancers.

CD74 (DHLAG) is an established and attractive target for antibody drug conjugates due to its restricted expression on normal tissues and significant upregulation in a range of hematological malignancies. CD74 functions as a chaperone that is necessary for the assembly and trafficking of MHC class II complexes as well as a receptor for macrophage migration inhibitory receptor (MIF). In oncology, it has been well established that CD74 is significantly upregulated at both the RNA and protein level in a range of B-cell and myeloid cell malignancies including acute myeloid leukemia, multiple myeloma, and diffuse large B-cell lymphoma. Additionally CD74 is known to rapidly internalize upon antibody engagement and traffic to the lysosome as well as to to be rapidly repopulated on the surface of tumor cells following internalization. Antibodies and antibody drug conjugates targeting CD74 have been shown previously to demonstrate anti-tumor activity in preclinical models of cancer.

CRISTINA L. ABRAHAMS ET AL: "Targeting CD74 in multiple myeloma with the novel, site-specific antibody-drug conjugate STRO-001 ", ONCOTARGET, vol. 9, no. 102, 28 December 2018 (2018-12-28), pages 37700-37714 is a study of the potential pharmacodynamics and anti-tumor effects of STRO-001 in multiple myeloma (MM). CD74 expression was assessed in MM cell lines and primary bone marrow (BM) MM biopsies S. V. GOVINDAN ET AL: "Milatuzumab-SN-38 Conjugates for the Treatment of CD74+ Cancers", MOLECULAR CANCER THERAPEUTICS, vol. 12, no. 6, 1 June 2013 (2013-06-01), pages 968-978, examines the humanized anti-CD74 antibody, milatuzumab, for the therapy of CD74-expressing solid tumors. Milatuzumab-doxorubicin and two milatuzumab-SN-38 conjugates with

cleavable linkers, differing in their stability in serum and how they release SN-38 in the lysosome, were prepared. CD74 expression was determined by flow cytometry and immunohistology. In vitro cytotoxicity and in vivo therapeutic studies were conducted in the human cancer cell lines A-375 (melanoma), HuH-7 and Hep-G2 (hepatoma), Capan-1 (pancreatic), NCI-N87 (gastric), and Raji Burkitt lymphoma. EP2886545A1 relates to new thienopyrimidine derivatives, a process for their preparation and pharmaceutical compositions containing them. WO2016/207216A1 relates to new hydroxyacid derivatives, to a process for their preparation and to pharmaceutical compositions containing them. WO2019/035899A1 relates to inhibitors of induced myeloid leukemia cell differentiation protein (MCL-1), compositions containing compounds described therein, and methods of treatment thereof.

## SUMMARY OF THE INVENTION

In some embodiments, the present disclosure provides, in part, novel antibody-drug conjugate (ADC) compounds with biological activity against cancer cells. The compounds may slow, inhibit, and/or reverse tumor growth in mammals, and/or may be useful for treating human cancer patients. The present disclosure more specifically relates, in some embodiments, to ADC compounds that are capable of binding and killing cancer cells. In some embodiments, the ADC compounds disclosed herein comprise a linker that attaches an Mcl-1 inhibitor to a full-length anti-CD74 antibody or an antigen-binding fragment. In some embodiments, the ADC compounds are also capable of internalizing into a target cell after binding.

In a first aspect of the present invention, there is provided ADC compounds represented by Formula (1):

$$\text{Ab-(L-D)}_p \qquad (1)$$

wherein:

Ab is an anti-CD74 antibody or an antigen-binding fragment thereof;
*p* is an integer from 1 to 16; and
-(L-D) is of the formula (C):

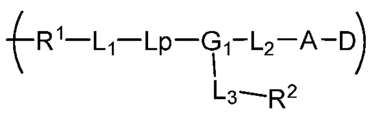

(C),

wherein:

$R^1$ is an attachment group;
$L_1$ is a bridging spacer;
$L_p$ is a peptide group comprising 1 to 6 amino acids;
D is an Mcl-1 inhibitor, wherein

(1) D comprises:

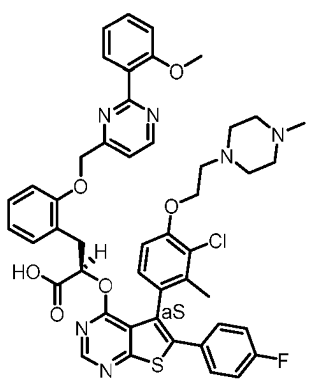

,

or an enantiomer, diastereoisomer, atropisomer, deuterated derivative, and/or pharmaceutically acceptable salt of any of the foregoing;
(2) D comprises:

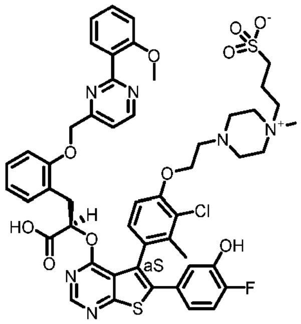

,

or an enantiomer, diastereoisomer, atropisomer, deuterated derivative, and/or pharmaceutically acceptable salt of any of the foregoing;

(3) D comprises:

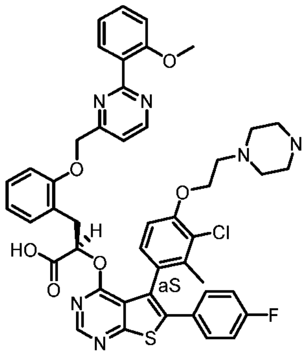

,

or an enantiomer, diastereoisomer, atropisomer, deuterated derivative, and/or pharmaceutically acceptable salt of any of the foregoing;

(4) D comprises:

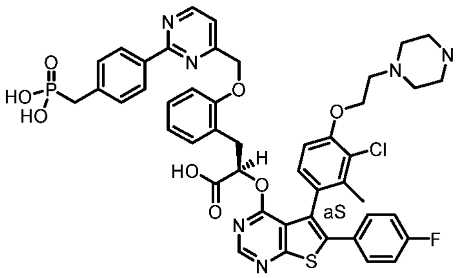

,

or an enantiomer, diastereoisomer, atropisomer, deuterated derivative, and/or pharmaceutically acceptable salt of any of the foregoing;

(5) D comprises:

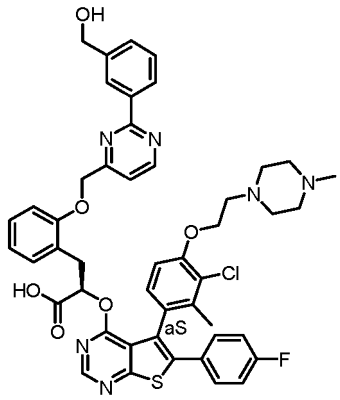

,

or an enantiomer, diastereoisomer, atropisomer, deuterated derivative, and/or pharmaceutically acceptable salt of any of the foregoing;

(6) D comprises:

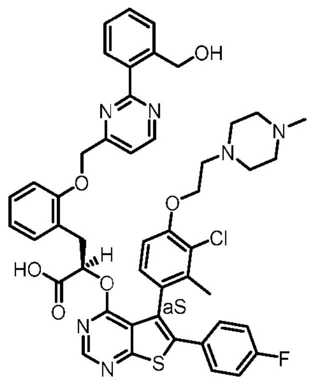

,

or an enantiomer, diastereoisomer, atropisomer, deuterated derivative, and/or pharmaceutically acceptable salt of any of the foregoing;

(7) D comprises:

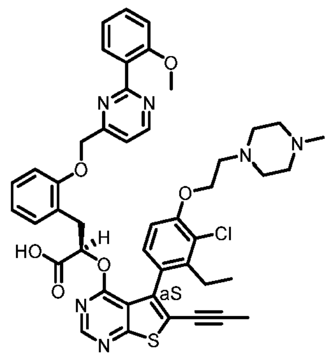

,

or an enantiomer, diastereoisomer, atropisomer, deuterated derivative, and/or pharmaceutically acceptable salt of any of the foregoing;

(8) D comprises:

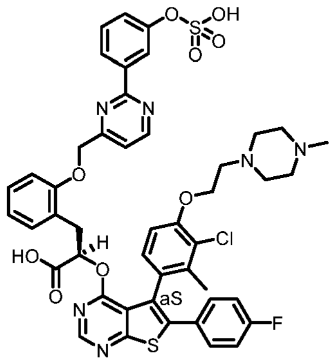

,

or an enantiomer, diastereoisomer, atropisomer, deuterated derivative, and/or pharmaceutically acceptable salt of any of the foregoing;

(9) D comprises:

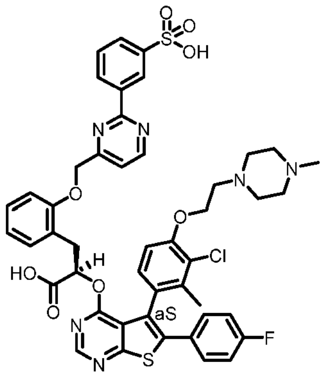

,

or an enantiomer, diastereoisomer, atropisomer, deuterated derivative, and/or pharmaceutically acceptable salt of any of the foregoing;

(10) D comprises:

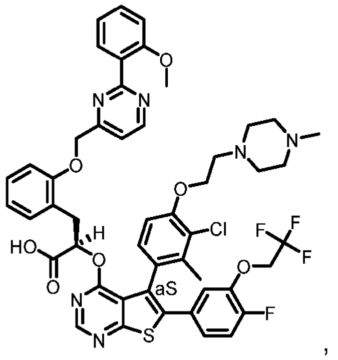

,

or an enantiomer, diastereoisomer, atropisomer, deuterated derivative, and/or pharmaceutically acceptable salt of any of the foregoing;

(11) D comprises:

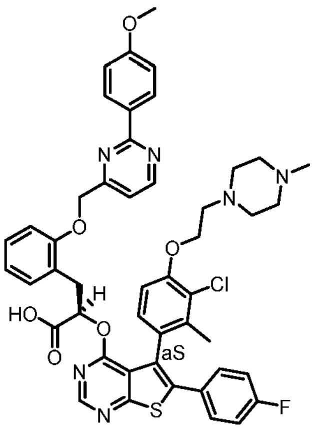

,

or an enantiomer, diastereoisomer, atropisomer, deuterated derivative, and/or pharmaceutically acceptable salt of any of the foregoing;

(12) D comprises:

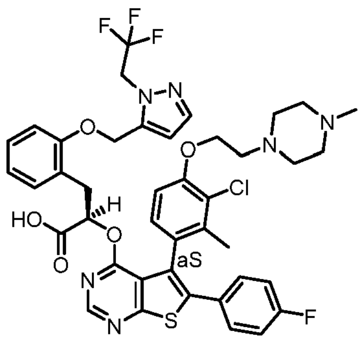

,

or an enantiomer, diastereoisomer, atropisomer, deuterated derivative, and/or pharmaceutically acceptable salt of any of the foregoing; or

(13) D comprises:

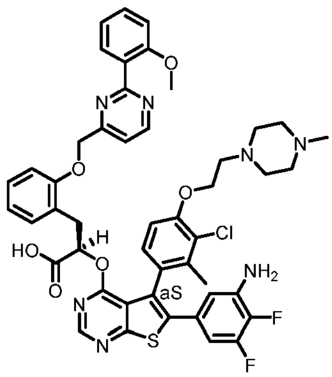

,

or an enantiomer, diastereoisomer, atropisomer, deuterated derivative, and/or pharmaceutically acceptable salt of any of the foregoing;

(14) D comprises:

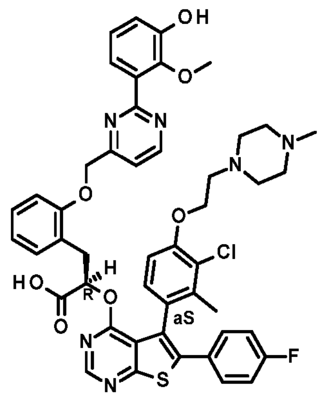

,

or an enantiomer, diastereoisomer, atropisomer, deuterated derivative, and/or pharmaceutically acceptable salt of any of the foregoing;

(15) D comprises:

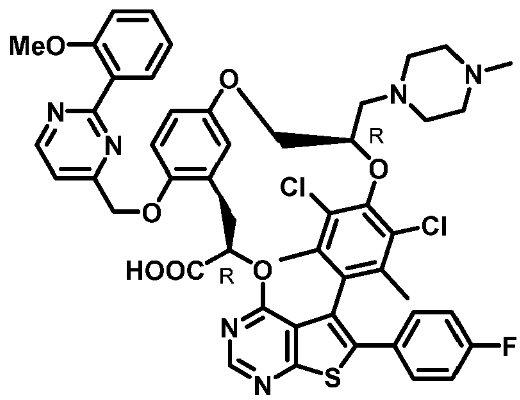

,

or an enantiomer, diastereoisomer, atropisomer, deuterated derivative, and/or pharmaceutically acceptable salt of any of the foregoing;

(16) D comprises:

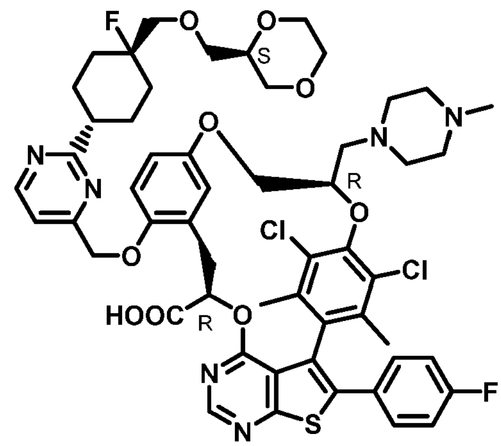

,

or an enantiomer, diastereoisomer, atropisomer, deuterated derivative, and/or pharmaceutically acceptable salt of any of the foregoing; or

(17) D comprises:

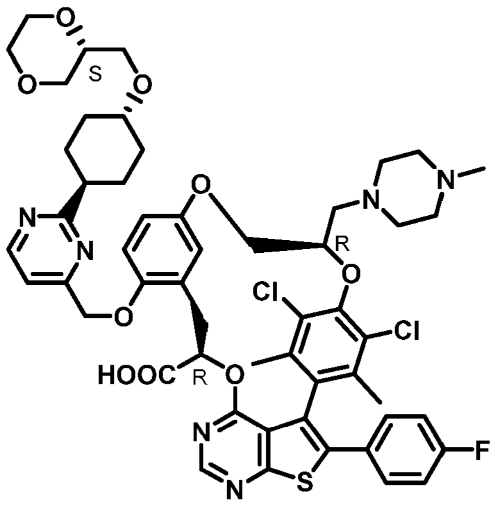,

or an enantiomer, diastereoisomer, atropisomer, deuterated derivative, and/or pharmaceutically acceptable salt of any of the foregoing;

$G_1$-$L_2$-A is a self-immolative spacer;
$L_2$ is a bond, a methylene, a neopentylene or a $C_2$-$C_3$ alkenylene;
A is a bond, -OC(=O)-*,

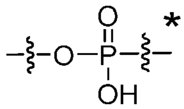, 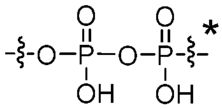, 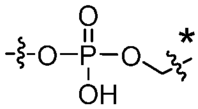,

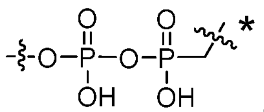, .

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety; and
$R^2$ is a hydrophilic moiety.

In an embodiment of the first aspect, -(L-D) is of Formula (D):
wherein:

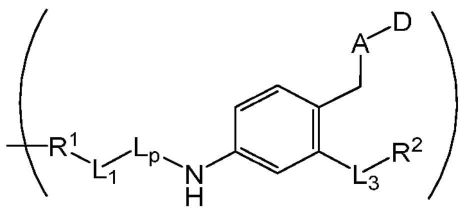 (D)

$R^1$ is an attachment group;
$L_1$ is a bridging spacer;
Lp is a peptide group comprising 1 to 6 amino acids;
A is a bond, -OC(=O)-*,

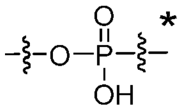,
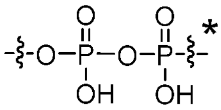,
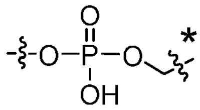,

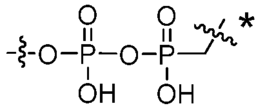, .

$-OC(=O)N(CH_3)CH_2CH_2N(CH_3)C(=O)-*$ or $-OC(=O)N(CH_3)C(R^a)_2C(R^a)_2N(CH_3)C(=O)-*$,
wherein each $R^a$ is independently selected from H, $C_1$-$C_6$ alkyl, and $C_3$-$C_8$ cycloalkyl and the * of A indicates the point of attachment to D;
$L_3$ is a spacer moiety; and
$R^2$ is a hydrophilic moiety.

In an embodiment of the first aspect:

(i) $L_1$
comprises:

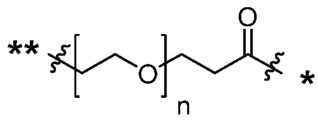,
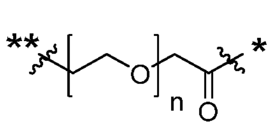,

or

*-CH(OH)CH(OH)CH(OH)CH(OH)-**,

wherein each n is an integer from 1 to 12, wherein the * of $L_1$ indicates the point of direct or indirect attachment to Lp, and the ** of $L_1$ indicates the point of direct or indirect attachment to $R^1$;

(ii) $L_1$ is

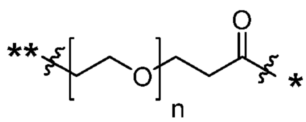,

and n is an integer from 1 to 12 or n is 1 or n is 12, wherein the * of $L_1$ indicates the point of direct or indirect attachment to Lp, and the ** of $L_1$ indicates the point of direct or indirect attachment to $R^1$;

(iii) $L_1$ is

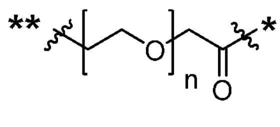,

and n is an integer from 1 to 12, wherein the * of $L_1$ indicates the point of direct or indirect attachment to Lp, and the ** of $L_1$ indicates the point of direct or indirect attachment to $R^1$;

(iv) $L_1$ comprises

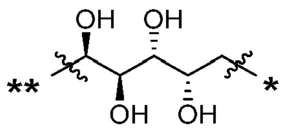,

wherein the * of $L_1$ indicates the point of direct or indirect attachment to Lp, and the ** of $L_1$ indicates the point of direct or indirect attachment to $R^1$; or

(v) $L_1$ is a bridging spacer comprising:

$*-C(=O)(CH_2)_mO(CH_2)_m-**$; $*-C(=O)((CH_2)_mO)_t(CH_2)_n-**$; $*-C(=O)(CH_2)_m-**$;

$*-C(=O)NH((CH_2)_mO)_t(CH_2)_n-**$;

$*-C(=O)O(CH_2)_mSSC(R^3)_2(CH_2)_mC(=O)NR^3(CH_2)_mNR^3C(=O)(CH_2)_m-**$;

$*-C(=O)O(CH_2)_mC(=O)NH(CH_2)_m-**$; $*-C(=O)(CH_2)_mNH(CH_2)_m-**$;

$*-C(=O)(CH_2)_mNH(CH_2)_nC(=O)-**$; $*-C(=O)(CH_2)_mX_1(CH_2)_m-**$;

$*-C(=O)((CH_2)_mO)_t(CH_2)_nX_1(CH_2)_n-**$; $*-C(=O)(CH_2)_mNHC(=O)(CH_2)_n-**$;

$*-C(=O)((CH_2)_mO)_t(CH_2)_nNHC(=O)(CH_2)_n-**$;

$*-C(=O)(CH_2)_mNHC(=O)(CH_2)_nX_1(CH_2)_n-**$;

$*-C(=O)((CH_2)_mO)_t(CH_2)_nNHC(=O)(CH_2)_nX_1(CH_2)_n-**$;

$*-C(=O)((CH_2)_mO)_t(CH_2)_nC(=O)NH(CH_2)_m-**$; $*-C(=O)(CH_2)_mC(R^3)_2-**$

or $*-C(=O)(CH_2)_mC(=O)NH(CH_2)_m-**$, wherein the * of $L_1$ indicates the point of direct or indirect attachment to Lp, and the ** of $L_1$ indicates the point of direct or indirect attachment to $R^1$;

$X_1$ is

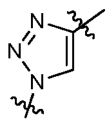, 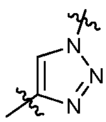, 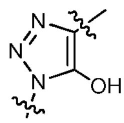 or 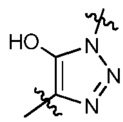;

and
each m is independently selected from 1, 2, 3, 4, 5, 6, 7, 8, 9 and 10;
each n is independently selected from 1, 2, 3, 4, 5, 6, 7, 8, 9 and 10; and
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
and each $R^3$ is independently selected from H and $C_1$-$C_6$alkyl.

In an embodiment of the first aspect:

(1) $R^2$ is a hydrophilic moiety comprising polyethylene glycol, polyalkylene glycol, a polyol, a polysarcosine, a sugar, an oligosaccharide, a polypeptide, or $C_2$-$C_6$ alkyl substituted with 1 to 3

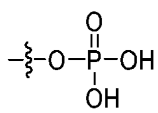 or 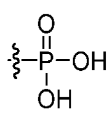

groups;

(2) $R^2$ is

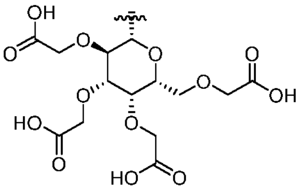, 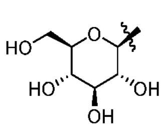, 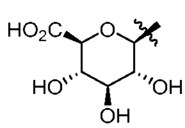, 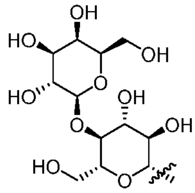,

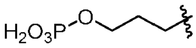, 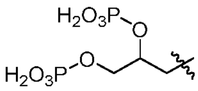, 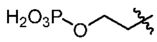, 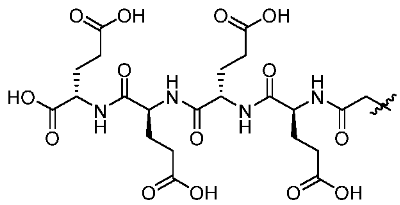,

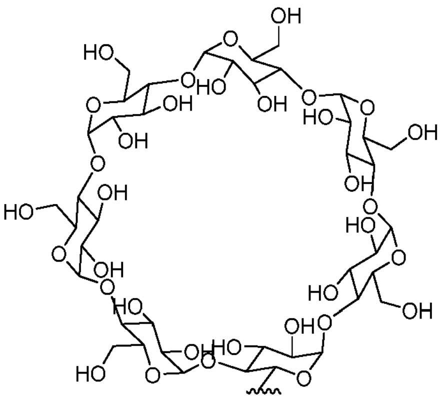, 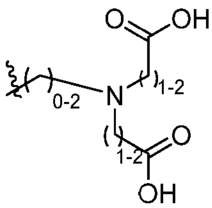, 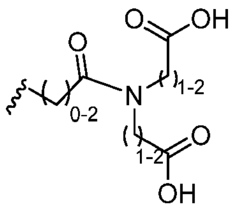,

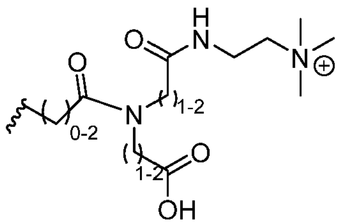, 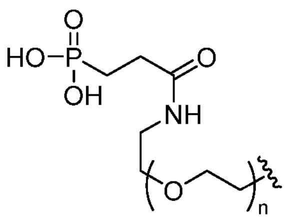

wherein n is an integer between 1 and 6,

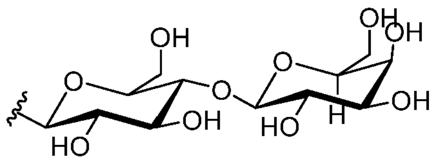,

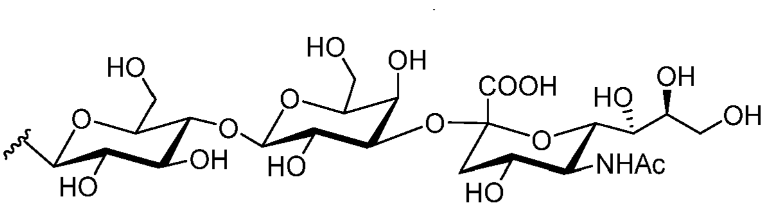,

or

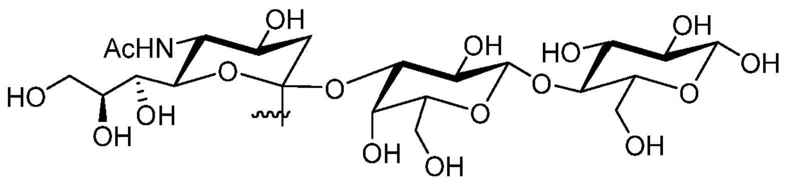

;

(3) the hydrophilic moiety represented by $R^2$ comprises:

(i) a polysarcosine, e.g., with the following moiety:

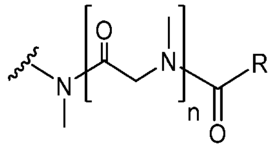

,

wherein n is an integer between 3 and 25; and R is H, $-CH_3$ or - $CH_2CH_2C(=O)OH$; or

(ii) a polyethylene glycol of formula:

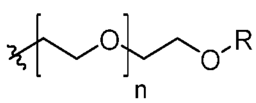

or

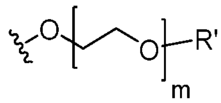

,

wherein R is H, $-CH_3$, $CH_2CH_2NHC(=O)OR_a$, $-CH_2CH_2NHC(=O)R_a$, or $-CH_2CH_2C(=O)OR_a$, R' is OH, - $OCH_3$, $-CH_2CH_2NHC(=O)OR_a$,
$-CH_2CH_2NHC(=O)R_a$, or $-OCH_2CH_2C(=O)OR_a$, in which $R^a$ is H or $C_{1-4}$ alkyl optionally substituted with either OH or $C_{1-4}$ alkoxyl, and each of m and n is independently an integer between 2 and 25; or

(4) the hydrophilic moiety represented by $R^2$ comprises

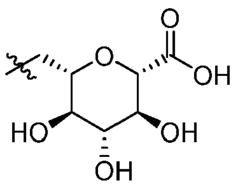

.

In an embodiment of the first aspect:

(i) $L_3$ is a spacer moiety having the structure

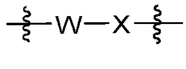

,

wherein:

W is $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, $-NHC(=O)C(R^b)_2NHC(=O)-$, $-CH_2N(X-R^2)C(=O)O-$, $-C(=O)N(X-R^2)-$, - $CH_2N(X-R^2)C(=O)-$, $-C(=O)NR^b-$, $-C(=O)NH-$, $-CH_2N$ $R^b$ $C(=O)-$, $-CH_2N$ $R^b$ $C(=O)NH-$, - $CH_2NR^bC(=O)NR^b-$, $-NHC(=O)-$, $-NHC(=O)O-$, $-NHC(=O)NH-$, $-OC(=O)NH-$, $-S(O)_2NH-$, $-NHS(O)_2-$, $-C(=O)-$, $-C(=O)O-$ or $-NH-$, wherein each $R^b$ is independently selected from H, $C_1$-$C_6$alkyl, and $C_3$-$C_8$ cycloalkyl; and
X is a bond, triazolyl, or $-CH_2$-triazolyl-; or

(ii) $L_3$ is a spacer moiety having the structure

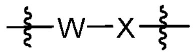,

wherein:

W is $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, $-NHC(=O)C(R^b)_2NHC(=O)-$, $-CH_2N(X-R^2)C(=O)O-$, $-C(=O)N(X-R^2)-$, $-CH_2N(X-R^2)C(=O)-$, $-C(=O)NR^b-$, $-C(=O)NH-$, $-CH_2NR^bC(=O)-$, $-CH_2NR^bC(=O)NH-$, $-CH_2NR^bC(=O)NR^b-$, $-NHC(=O)-$, $-NHC(=O)O-$, $-NHC(=O)NH-$, $-OC(=O)NH-$, $-S(O)_2NH-$, $-NHS(O)_2-$, $-C(=O)-$, $-C(=O)O-$ or $-NH-$, wherein each $R^b$ is independently selected from H, $C_1$-$C_6$alkyl, and $C_3$-$C_8$ cycloalkyl; and
X is $-CH_2$-triazolyl-$C_{1-4}$ alkylene-$OC(O)NHS(O)_2NH-$, $-C_{4-6}$ cycloalkylene-$OC(O)NHS(O)_2NH-$, $-(CH_2CH_2O)_n-C(O)NHS(O)_2NH-$, $-(CH_2CH_2O)_n-C(O)NHS(O)_2NH-(CH_2CH_2O)_n-$, or $-CH_2$-triazolyl-$C_{1-4}$ alkylene-$OC(O)NHS(O)_2NH-(CH_2CH_2O)_n-$, wherein each n independently is 1, 2, or 3.

In an embodiment of the first aspect:

(i) the attachment group is formed by a reaction comprising at least one reactive group;

(ii) the attachment group is formed by reacting:

a first reactive group that is attached to the linker, and
a second reactive group that is attached to the antibody or is an amino acid residue of the antibody;

(iii) the attachment group is formed by a reaction comprising at least one reactive group, wherein at least one of the reactive groups comprises:

a thiol,
a maleimide,
a haloacetamide,
an azide,
an alkyne,
a cyclooctene,
a triaryl phosphine,
an oxanorbornadiene,
a cyclooctyne,
a diaryl tetrazine,
a monoaryl tetrazine,
a norbornene,
an aldehyde,
a hydroxylamine,
a hydrazine,
$NH_2-NH-C(=O)-$,
a ketone,
a vinyl sulfone,
an aziridine,
an amino acid residue,

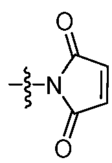,

$-ONH_2$, $-NH_2$,

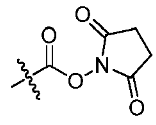, 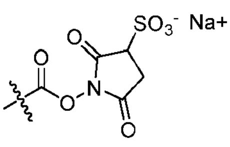, 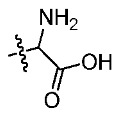, 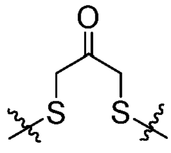,

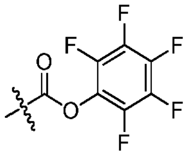, 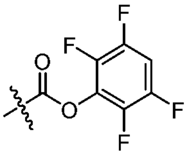, 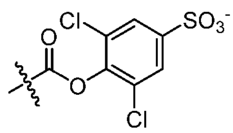,

$-N_3$,

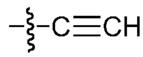,

-SH, $-SR^3$, $-SSR^4$, $-S(=O)_2(CH=CH_2)$, $-(CH_2)_2S(=O)_2(CH=CH_2)$, $-NHS(=O)_2(CH=CH_2)$, $-NHC(=O)CH_2Br$, $-NHC(=O)CH_2I$,

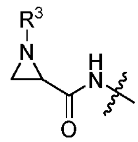,

$-C(O)NHNH_2$,

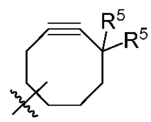,

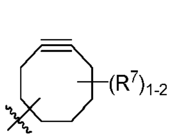, 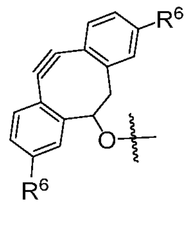, 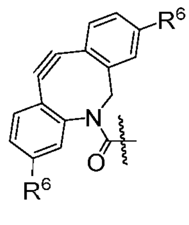, 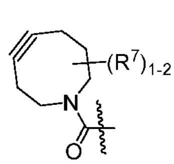, 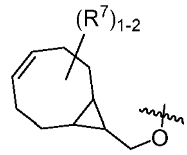,

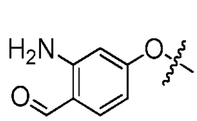, 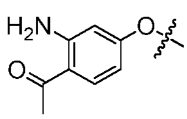, 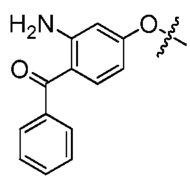, 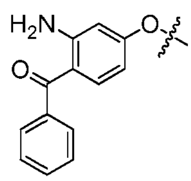,

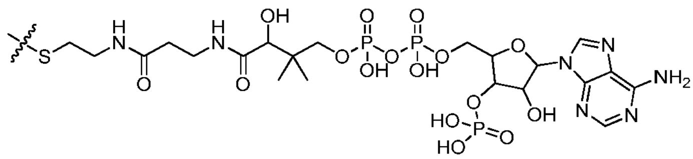,

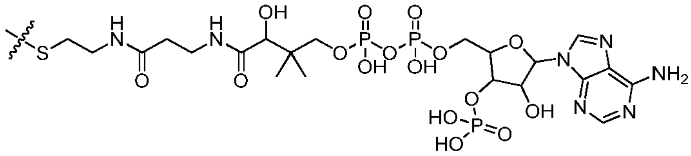
,

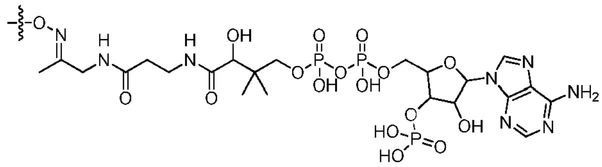
,

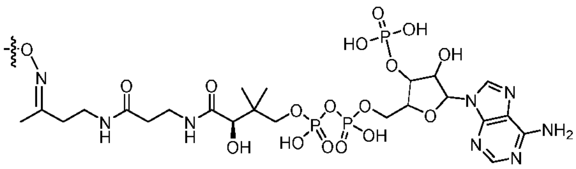
,

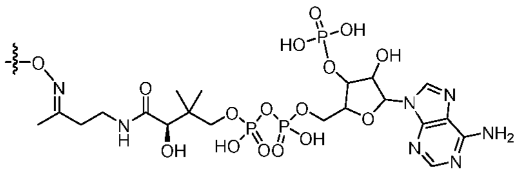
,

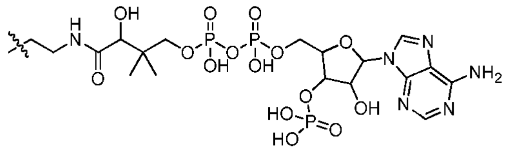
,

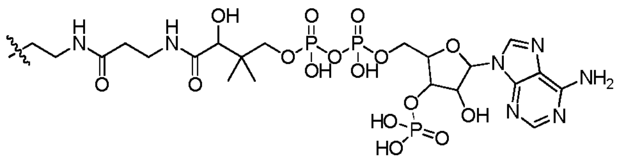
,

or

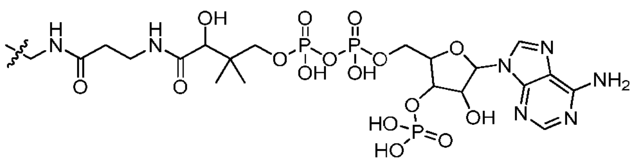
;

wherein:

each $R^3$ is independently selected from H and $C_1$-$C_6$alkyl;
each $R^4$ is 2-pyridyl or 4-pyridyl;
each $R^5$ is independently selected from H, $C_1$-$C_6$alkyl, F, Cl, and -OH;
each $R^6$ is independently selected from H, $C_1$-$C_6$alkyl, F, Cl, $-NH_2$, $-OCH_3$, - $OCH_2CH_3$, $-N(CH_3)_2$, -CN, $-NO_2$ and -OH;
each $R^7$ is independently selected from H, $C_{1-6}$alkyl, fluoro, benzyloxy substituted with -C(=O)OH, benzyl substituted with -C(=O)OH, $C_{1-4}$alkoxy substituted with -C(=O)OH and $C_{1-4}$alkyl substituted with -C(=O)OH;

(iv) the attachment group is formed by reacting that is attached to the linker and a second reactive group that is attached to the antibody or is an amino acid residue of the antibody, wherein the first reactive group and second reactive group comprise:

a thiol and a maleimide,
a thiol and a haloacetamide,
a thiol and a vinyl sulfone,
a thiol and an aziridine,
an azide and an alkyne,
an azide and a cyclooctyne,
an azide and a cyclooctene,
an azide and a triaryl phosphine,
an azide and an oxanorbornadiene,
a diaryl tetrazine and a cyclooctene,
a monoaryl tetrazine and a norbornene,
an aldehyde and a hydroxylamine,
an aldehyde and a hydrazine,
an aldehyde and $NH_2$-NH-C(=O)-,
a ketone and a hydroxylamine,
a ketone and a hydrazine,
a ketone and $NH_2$-NH-C(=O)-,
a hydroxylamine and

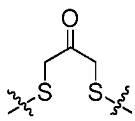,

an amine and

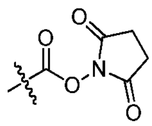, 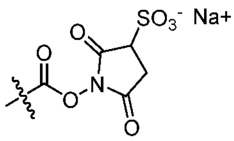, 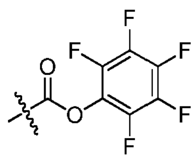, 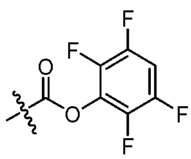,

or

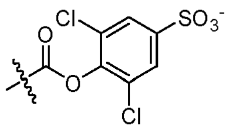,

or
a CoA or CoA analogue and a serine residue; or

(v) the attachment group comprises a group selected from:

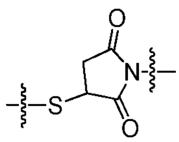;

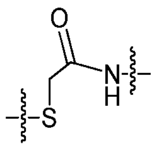
;

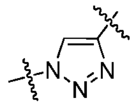 or 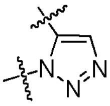 ;

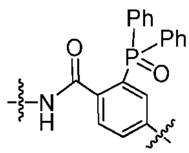 ;

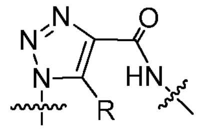 ;

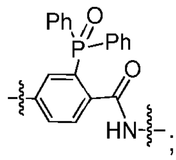 ;

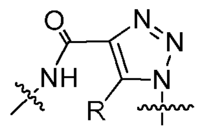 ;

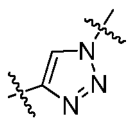 or 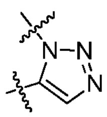 ;

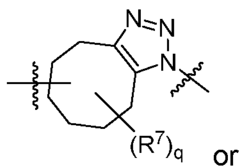 or 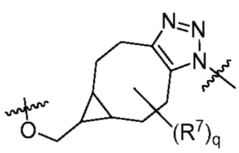 or 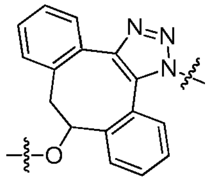 ;

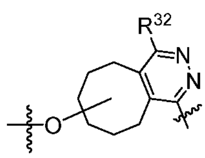 or 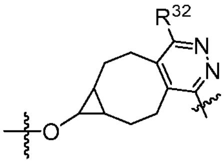 ;

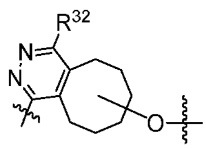 or 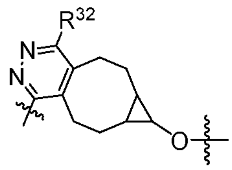 ;

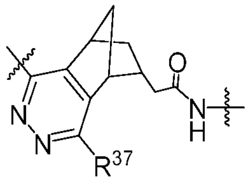 ;

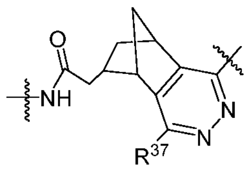 ;

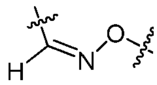 ;

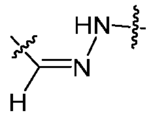 ;

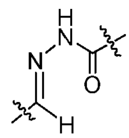 ;

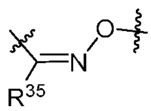 ;

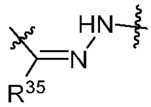 ;

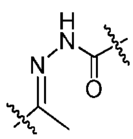 ;

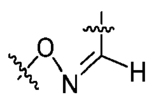 ;

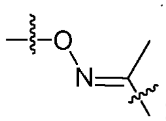 ;

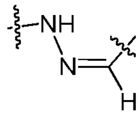 ;

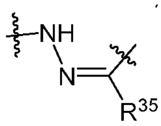 ;

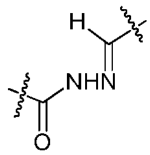 ;

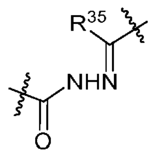 ;

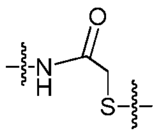 ;

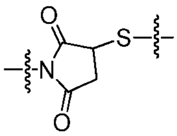 ;

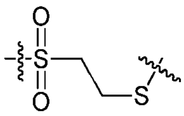 ;

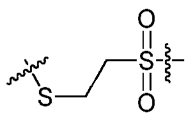 ;

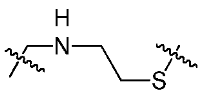 or 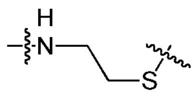 ;

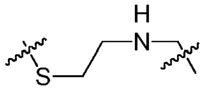 or 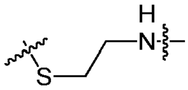 ;

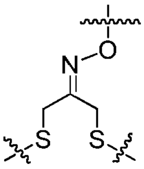 ;

amide;

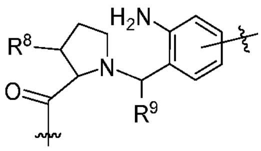 ;

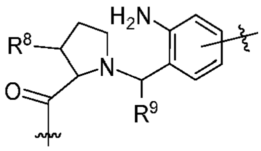 ;

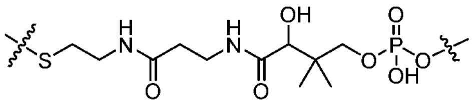 ;

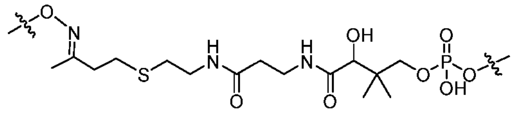 ;

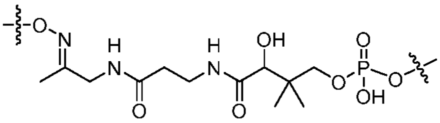 ;

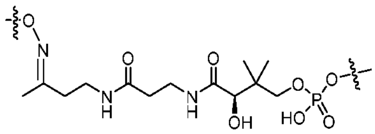 ;

,

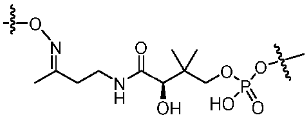 ;

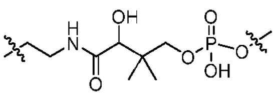 ;

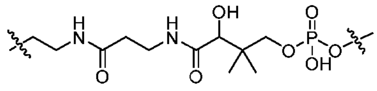

;

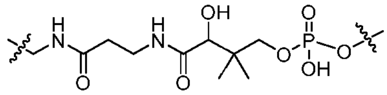

; and
disulfide,

wherein:

$R^{32}$ is H, $C_{1-4}$ alkyl, phenyl, pyrimidine or pyridine;
$R^{35}$ is H, $C_{1-6}$ alkyl, phenyl or $C_{1-4}$ alkyl substituted with 1 to 3 -OH groups;
each $R^7$ is independently selected from H, $C_{1-6}$ alkyl, fluoro, benzyloxy substituted with -C(=O)OH, benzyl substituted with -C(=O)OH, $C_{1-4}$ alkoxy substituted with - C(=O)OH and $C_{1-4}$ alkyl substituted with -C(=O)OH;
$R^{37}$ is independently selected from H, phenyl and pyridine;
q is 0, 1, 2 or 3;
$R^8$ is H or methyl; and
$R^9$ is H, $-CH_3$ or phenyl.

In an embodiment of the first aspect:

(i) the peptide group comprises 1 to 4 amino acid residues, 1 to 3 amino acid residues, or 1 to 2 amino acid residues;
(ii) the peptide group comprises amino acid residues selected from L-glycine (Gly), L-valine (Val), L-citrulline (Cit), L-cysteic acid (sulfo-Ala), L-lysine (Lys), L-isoleucine (Ile), L-phenylalanine (Phe), L-methionine (Met), L-asparagine (Asn), L-proline (Pro), L-alanine (Ala), L-leucine (Leu), L-tryptophan (Trp), and L-tyrosine (Tyr);
(iii) the peptide group comprises Val-Cit, Phe-Lys, Val-Ala, Val-Lys, Leu-Cit, sulfo-Ala-Val, and/or sulfo-Ala-Val-Ala; or
(iv) Lp is selected from:

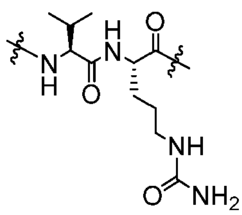
,
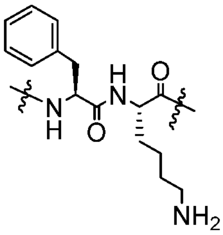
,
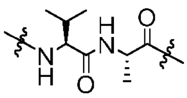
,
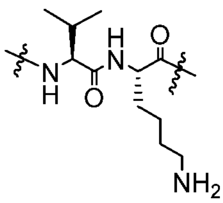
,
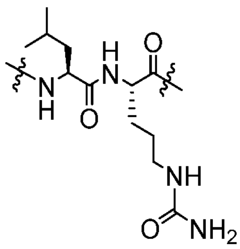
,

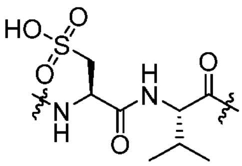
, and
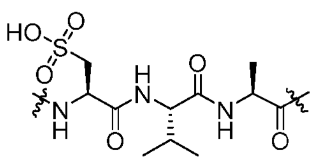
.

In a second aspect, there is provided an antibody-drug conjugate of formula (1):

$$Ab\text{-}(L\text{-}D)_p \qquad (1)$$

wherein:

Ab is an anti-CD74 antibody or an antigen-binding fragment thereof;
p is an integer from 1 to 16; and

wherein -(L-D) comprises or is formed from a compound of formula:

(1)

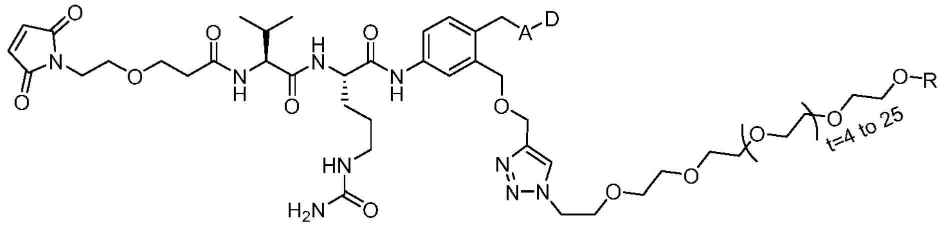

,

wherein:

R is H, $-CH_3$ or $-CH_2CH_2C(=O)OH$;
A is a bond, -OC(=O)-*,

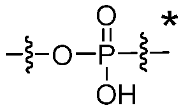 , 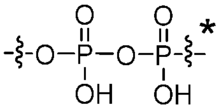 , 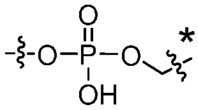 ,

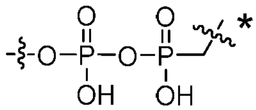 ,

$-OC(=O)N(CH_3)CH_2CH_2N(CH_3)C(=O)-$* or

$-OC(=O)N(CH_3)C(R^a)_2C(R^a)_2N(CH_3)C(=O)-$*,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(2)

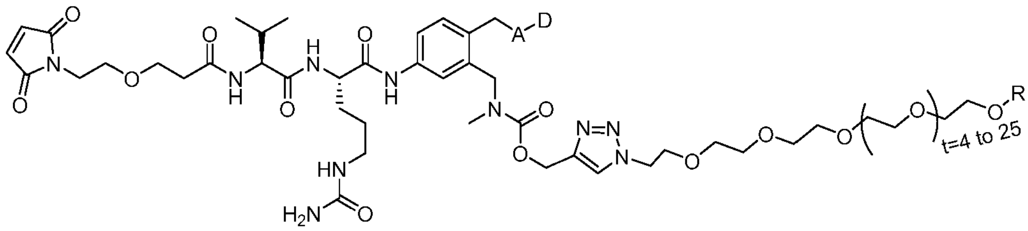

,

wherein:

R is H, $-CH_3$ or $-CH_2CH_2C(=O)OH$;
A is a bond, -OC(=O)-*,

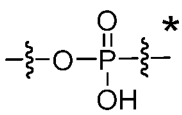 , 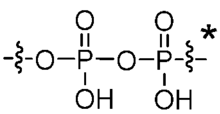 , 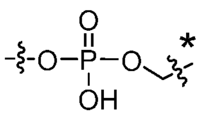 ,

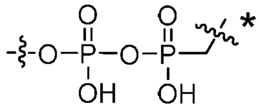,

$-OC(=O)N(CH_3)CH_2CH_2N(CH_3)C(=O)-*$ or

$$-OC(=O)N(CH_3)C(R^a)_2C(R^a)_2N(CH_3)C(=O)-*,$$

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(3)

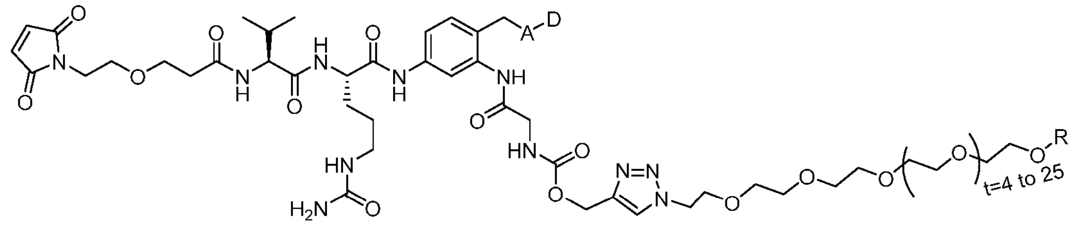,

wherein:

R is H, $-CH_3$ or $-CH_2CH_2C(=O)OH$;
A is a bond, -OC(=O)-*,

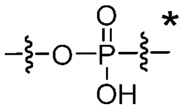, 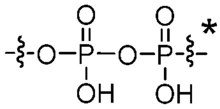, 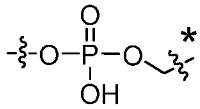,

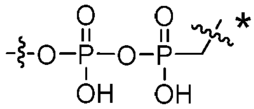,

$-OC(=O)N(CH_3)CH_2CH_2N(CH_3)C(=O)-*$ or

$$-OC(=O)N(CH_3)C(R^a)_2C(R^a)_2N(CH_3)C(=O)-*,$$

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(4)

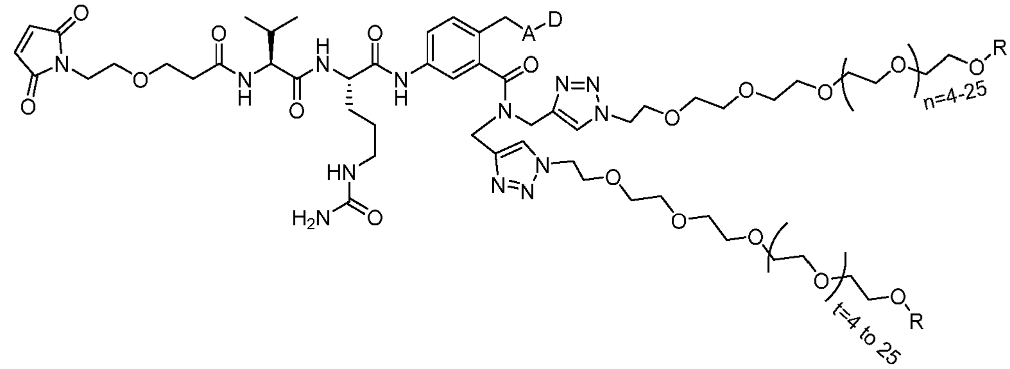

,

wherein:

each R is independently selected from H, $-CH_3$, and $-CH_2CH_2C(=O)OH$;
A is a bond, -OC(=O)-*,

, 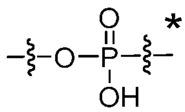 , 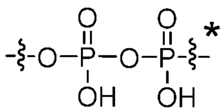 , 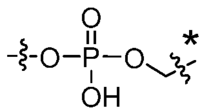 ,

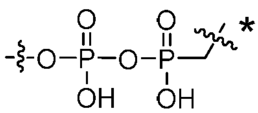 ,

$-OC(=O)N(CH_3)CH_2CH_2N(CH_3)C(=O)-$* or

$-OC(=O)N(CH_3)C(R^a)_2C(R^a)_2N(CH_3)C(=O)-$*,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(5)

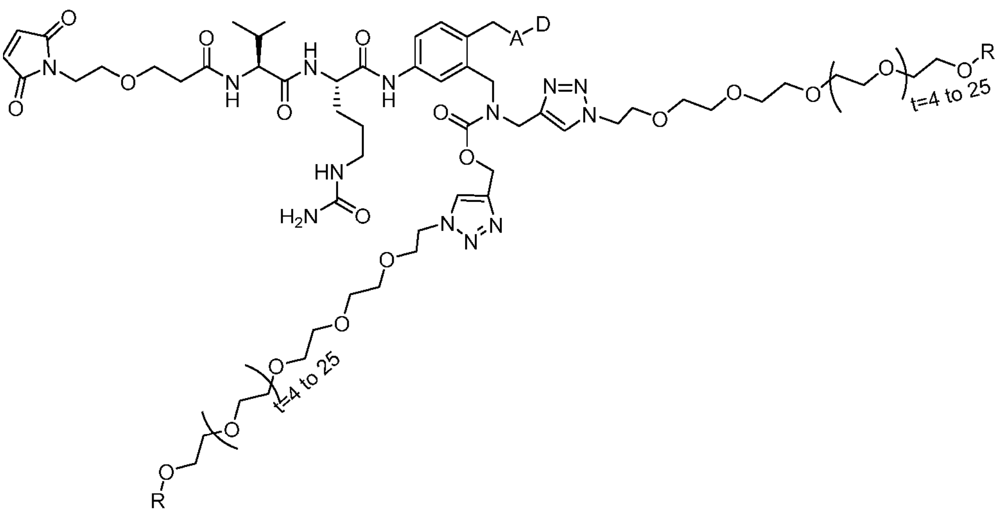

,

wherein:

each R is independently selected from H, $-CH_3$, and $-CH_2CH_2C(=O)OH$;
A is a bond, -OC(=O)-*,

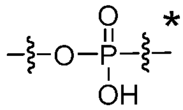, 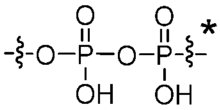, 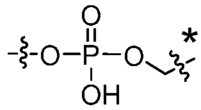,

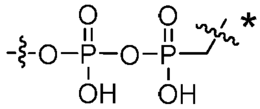,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(6)

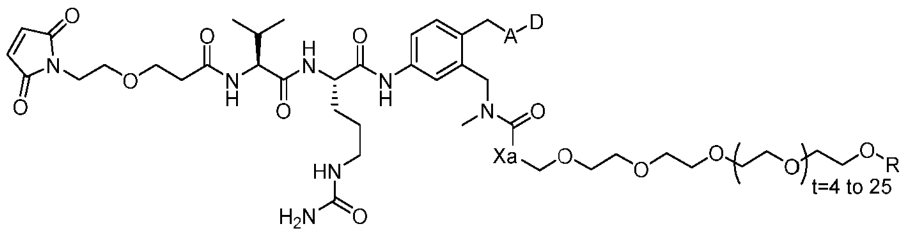,

wherein:

Xa is $-CH_2-$, $-OCH_2-$, $-NHCH_2-$ or $-NRCH_2-$ and each R independently is H, $-CH_3$ or $-CH_2CH_2C(=O)OH$;
A is a bond, -OC(=O)-*,

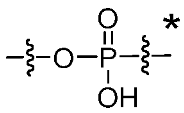, 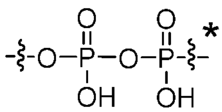, 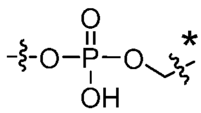,

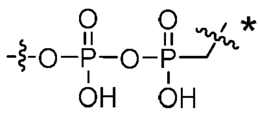,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(7)

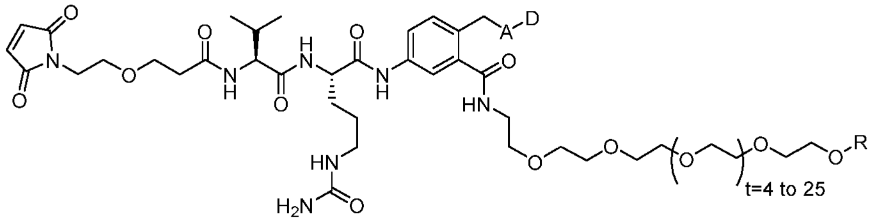

,

wherein:

R is H, $-CH_3$ or $-CH_2CH_2C(=O)OH$;
A is a bond, -OC(=O)-*,

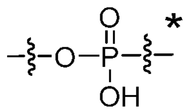 , 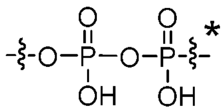 , 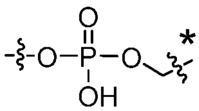 ,

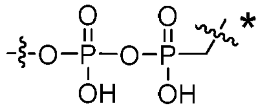 ,

$-OC(=O)N(CH_3)CH_2CH_2N(CH_3)C(=O)-$* or

$-OC(=O)N(CH_3)C(R^a)_2C(R^a)_2N(CH_3)C(=O)-$*,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(8)

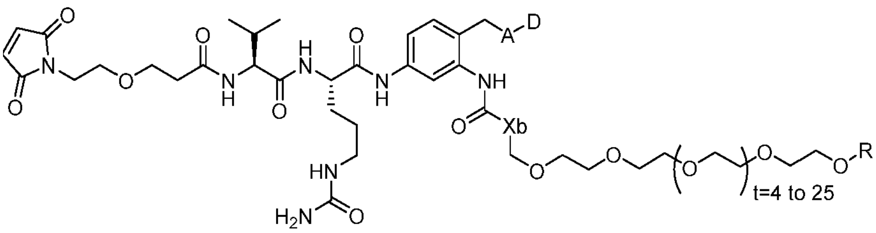

,

wherein:

Xb is $-CH_2-$, $-OCH_2-$, $-NHCH_2-$ or $-NRCH_2-$ and each R independently is H, $-CH_3$ or - $CH_2CH_2C(=O)OH$;
A is a bond, -OC(=O)-*,

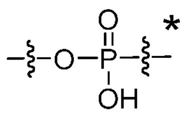 , 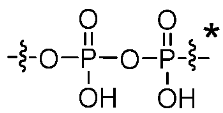 , 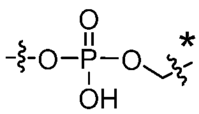 ,

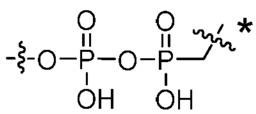 ,

$-OC(=O)N(CH_3)CH_2CH_2N(CH_3)C(=O)-$* or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(9)

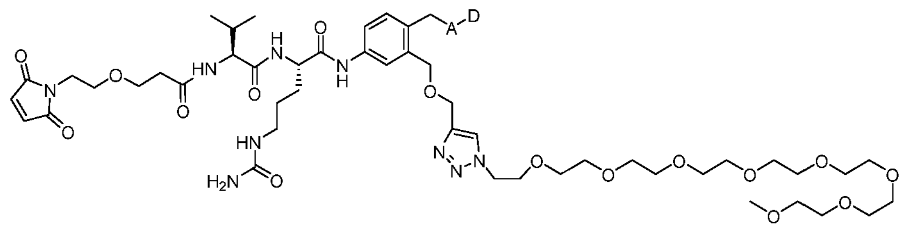
,

wherein:

A is a bond, -OC(=O)-*,

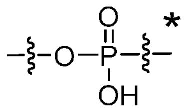
,
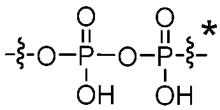
,
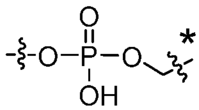
,

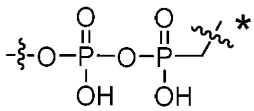
,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(10)

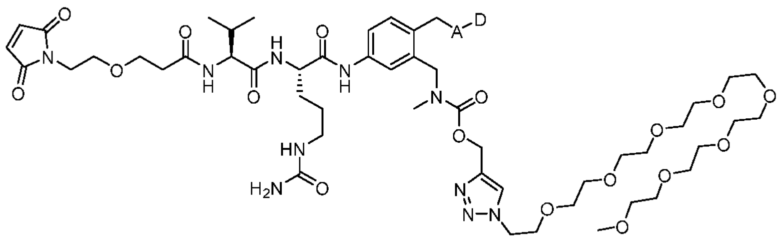
,

wherein:

A is a bond, -OC(=O)-*,

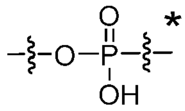 , 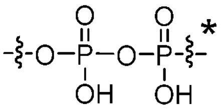 , 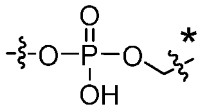 ,

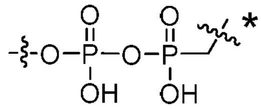 ,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(11)

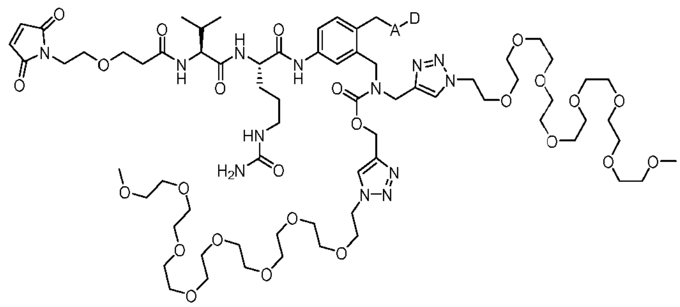 ,

wherein:

A is a bond, -OC(=O)-*

, 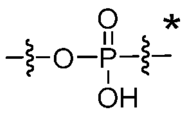 , 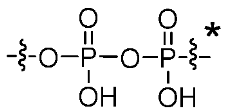 , 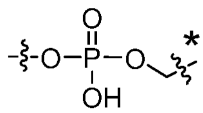 ,

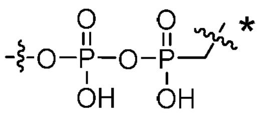 ,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(12)

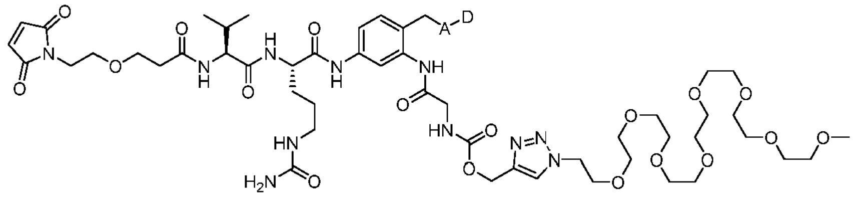
,

wherein:

A is a bond, -OC(=O)-*,

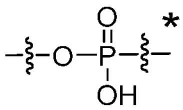
,
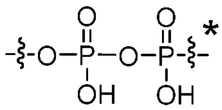
,
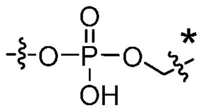
,

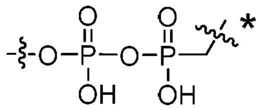
,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(13)

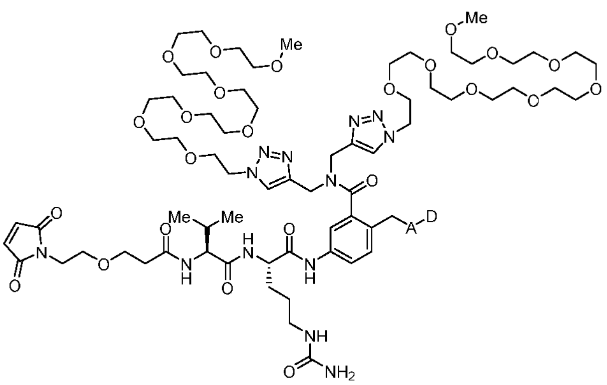
,

wherein:

A is a bond, -OC(=O)-*,

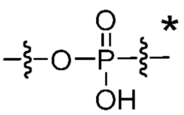
,
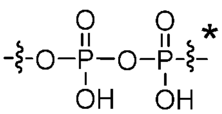
,
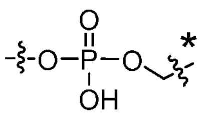
,

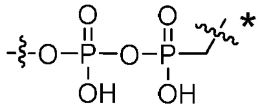,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(14)

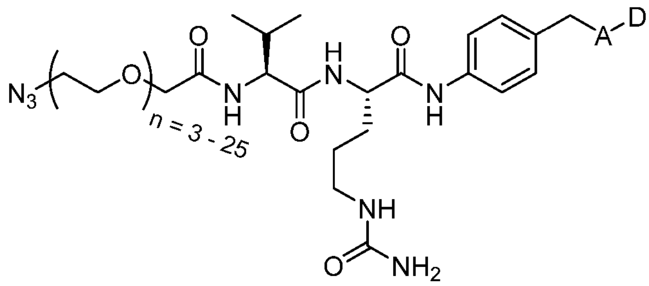,

wherein:

A is a bond, -OC(=O)-*,

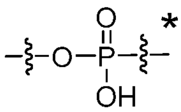, 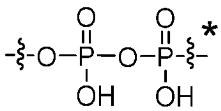, 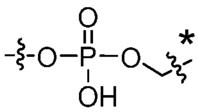,

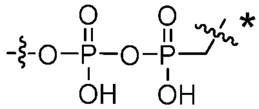,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect;

(15)

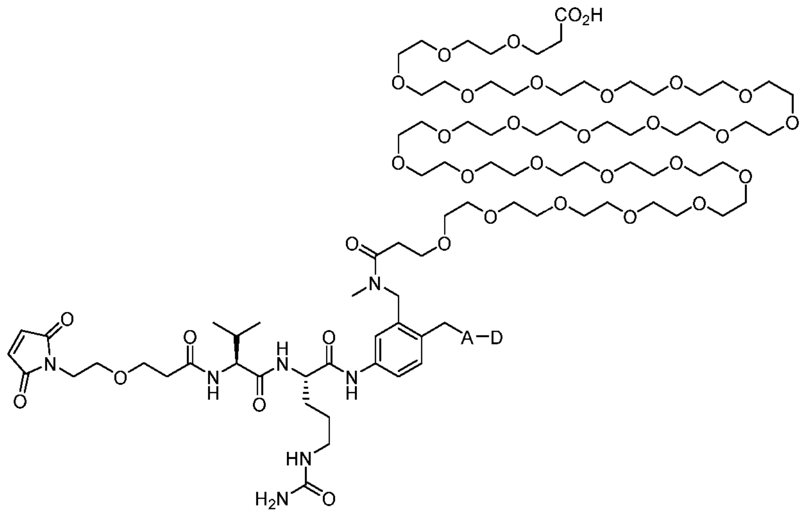

,

wherein:

A is a bond, -OC(=O)-*,

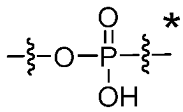, 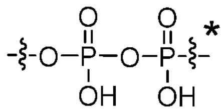, 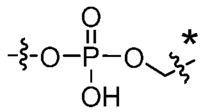,

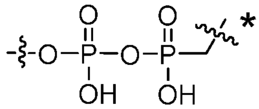,

$-OC(=O)N(CH_3)CH_2CH_2N(CH_3)C(=O)-*$ or

$-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in the first aspect; or

(16)

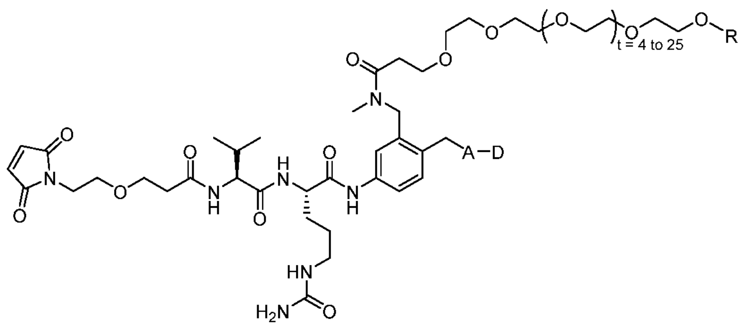

,

wherein:

each R independently is H, $-CH_3$ or $-CH_2CH_2C(=O)OH$;

A is a bond, -OC(=O)-*,

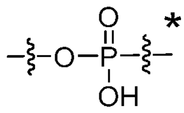, 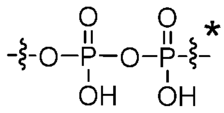, 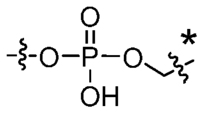,

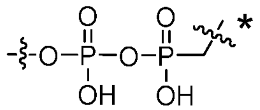, -

-OC(=O)N($CH_3$)$CH_2CH_2$N($CH_3$)C(=O)-* or

-OC(=O)N($CH_3$)C($R^a$)$_2$C($R^a$)$_2$N($CH_3$)C(=O)-*,

wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in the first aspect.

In an embodiment of the first and second aspect, A is a bond.

In an embodiment of the first and second aspect, R is $-CH_3$.

In an embodiment of the first and second aspect, -(L-D) is formed from a compound selected from Table A or an enantiomer, diastereoisomer, atropisomer, deuterated derivative, and/or pharmaceutically acceptable salt thereof.

In an embodiment of the first and second aspect, -(L-D) comprises or is formed from the following compound:

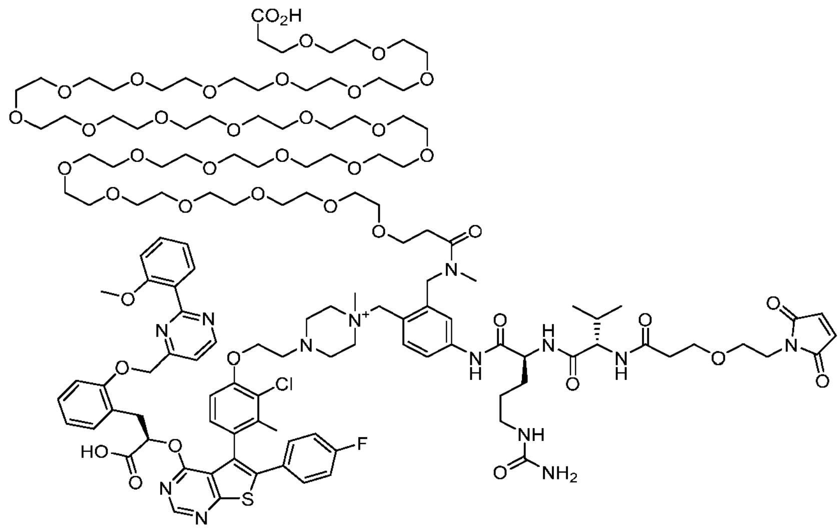

L11-P1,

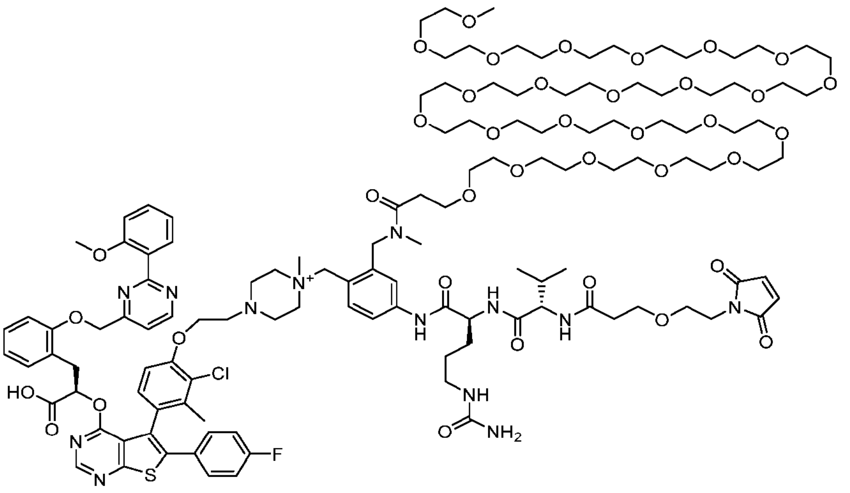

L30-P1,

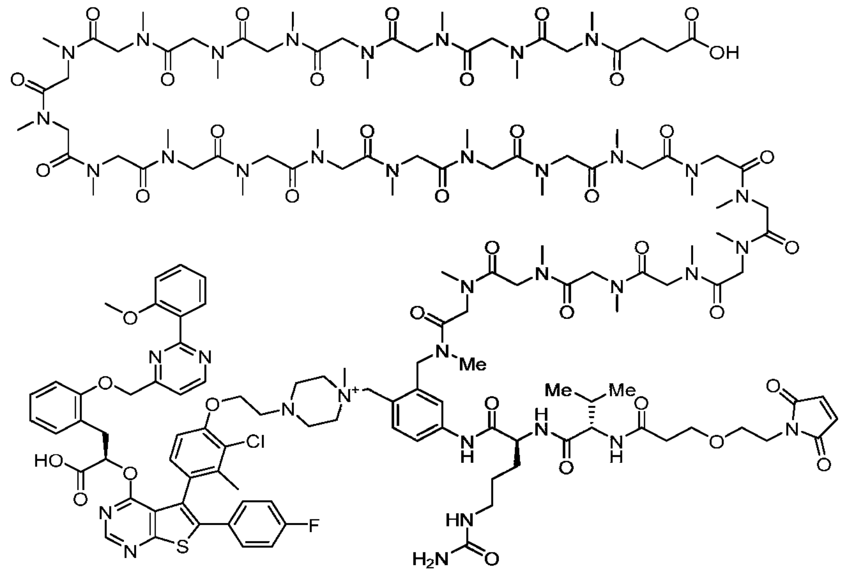

L37-P1,

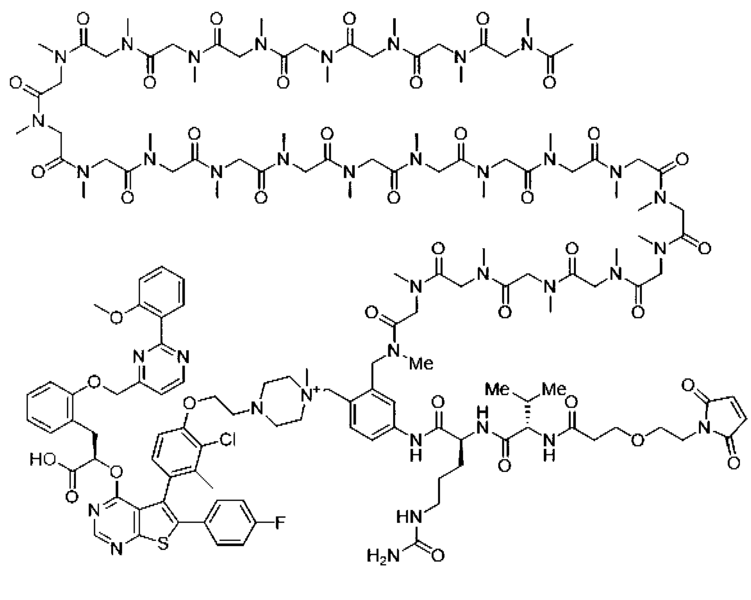

L40-P1,

or

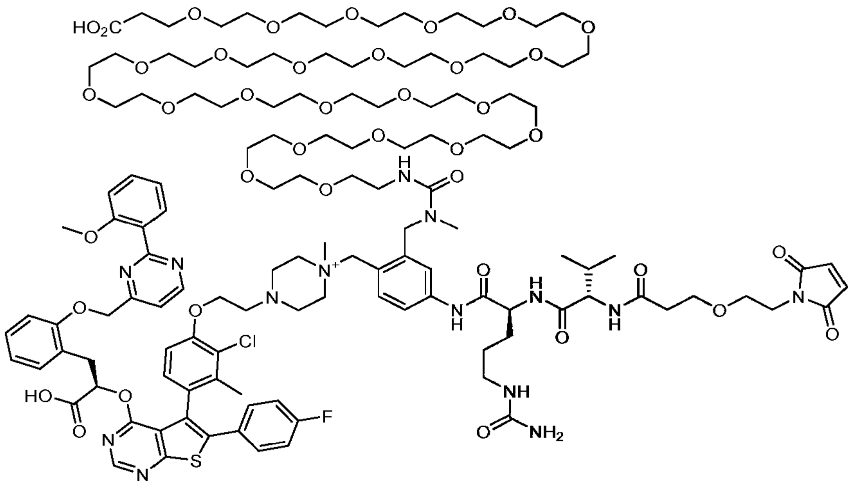

L42-P1.

In an embodiment of the first and second aspect:

(1) the anti-CD74 antibody comprises an anti-CD74 antibody or antigen binding fragment comprising three heavy chain CDRs and three light chain CDRs as follows:

(i) heavy chain CDR1 (HCDR1) consisting of SEQ ID NO:1, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:16, light chain CDR2 (LCDR2) consisting of SEQ ID NO:70, and light chain CDR3 (LCDR3) consisting of SEQ ID NO:18;
(ii) heavy chain CDR1 (HCDR1) consisting of SEQ ID NO:4, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:16, light chain CDR2 (LCDR2) consisting of SEQ ID NO:70, and light chain CDR3 (LCDR3) consisting of SEQ ID NO:18;
(iii) heavy chain CDR1 (HCDR1) consisting of SEQ ID NO:5, heavy chain CDR2 (HCDR2) consisting of SEQ ID NO:6, heavy chain CDR3 (HCDR3) consisting of SEQ ID NO:3; light chain CDR1 (LCDR1) consisting of SEQ ID NO:19, light chain CDR2 (LCDR2) consisting of SEQ ID NO:20, and light chain CDR3 (LCDR3) consisting of SEQ ID NO:21;
(iv) heavy chain CDR1 (HCDR1) consisting of SEQ ID NO:7, heavy chain CDR2 (HCDR2) consisting of SEQ ID NO:8, heavy chain CDR3 (HCDR3) consisting of SEQ ID NO:9; light chain CDR1 (LCDR1) consisting of SEQ ID NO:22, light chain CDR2 (LCDR2) consisting of SEQ ID NO:20, and light chain CDR3 (LCDR3) consisting of SEQ ID NO:18;
(v) heavy chain CDR1 (HCDR1) consisting of SEQ ID NO:1, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:35, light chain CDR2 (LCDR2) consisting of SEQ ID NO:70, and light chain CDR3 (LCDR3) consisting of SEQ ID NO:18;
(vi) heavy chain CDR1 (HCDR1) consisting of SEQ ID NO:4, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:35, light chain CDR2 (LCDR2) consisting of SEQ ID NO:70, and light chain CDR3 (LCDR3) consisting of SEQ ID NO:18;
(vii) heavy chain CDR1 (HCDR1) consisting of SEQ ID NO:5, heavy chain CDR2 (HCDR2) consisting of SEQ ID NO:6, heavy chain CDR3 (HCDR3) consisting of SEQ ID NO:3; light chain CDR1 (LCDR1) consisting of SEQ ID NO:71, light chain CDR2 (LCDR2) consisting of SEQ ID NO:20, and light chain CDR3 (LCDR3) consisting of SEQ ID NO:21.or
(viii) heavy chain CDR1 (HCDR1) consisting of SEQ ID NO:7, heavy chain CDR2 (HCDR2) consisting of SEQ ID NO:8, heavy chain CDR3 (HCDR3) consisting of SEQ ID NO:9; light chain CDR1 (LCDR1) consisting of SEQ ID NO:17, light chain CDR2 (LCDR2) consisting of SEQ ID NO:20, and light chain CDR3 (LCDR3) consisting of SEQ ID NO:18;

(2) the anti-CD74 antibody or antigen-binding fragment thereof comprises:

(i) a heavy chain variable region comprising the amino acid sequence of SEQ ID NO:10, and a light chain variable

region comprising the amino acid sequence of SEQ ID NO:23;

(ii) a heavy chain variable region comprising the amino acid sequence of SEQ ID NO:10, and a light chain variable region comprising the amino acid sequence of SEQ ID NO:27;(iii) a heavy chain variable region comprising the amino acid sequence of SEQ ID NO:10, and a light chain variable region comprising the amino acid sequence of SEQ ID NO:31;

(iv) a heavy chain variable region comprising the amino acid sequence of SEQ ID NO:10, and a light chain variable region comprising the amino acid sequence of SEQ ID NO:36;

(v) a heavy chain variable region comprising the amino acid sequence of SEQ ID NO:10, and a light chain variable region comprising the amino acid sequence of SEQ ID NO:40; or

(vi) a heavy chain variable region comprising the amino acid sequence of SEQ ID NO:10, and a light chain variable region comprising the amino acid sequence of SEQ ID NO:44; or

(3) the anti-CD74 antibody comprises:

(a) the heavy chain amino acid sequence of SEQ ID NO:12 and the light chain amino acid sequence of SEQ ID NO:25;

(b) the heavy chain amino acid sequence of SEQ ID NO:14 and the light chain amino acid sequence of SEQ ID NO:25;

(c) the heavy chain amino acid sequence of SEQ ID NO:15 and the light chain amino acid sequence of SEQ ID NO:25;

(d) the heavy chain amino acid sequence of SEQ ID NO:12 and the light chain amino acid sequence of SEQ ID NO:29;

(e) the heavy chain amino acid sequence of SEQ ID NO:14 and the light chain amino acid sequence of SEQ ID NO:29;

(f) the heavy chain amino acid sequence of SEQ ID NO:15 and the light chain amino acid sequence of SEQ ID NO:29;

(g) the heavy chain amino acid sequence of SEQ ID NO:12 and the light chain amino acid sequence of SEQ ID NO:33;

(h) the heavy chain amino acid sequence of SEQ ID NO:14 and the light chain amino acid sequence of SEQ ID NO:33;

(i) the heavy chain amino acid sequence of SEQ ID NO:15 and the light chain amino acid sequence of SEQ ID NO:33;

(j) the heavy chain amino acid sequence of SEQ ID NO:12 and the light chain amino acid sequence of SEQ ID NO:38;

(k) the heavy chain amino acid sequence of SEQ ID NO:14 and the light chain amino acid sequence of SEQ ID NO:38;

(l) the heavy chain amino acid sequence of SEQ ID NO:15 and the light chain amino acid sequence of SEQ ID NO:38;

(m) the heavy chain amino acid sequence of SEQ ID NO:12 and the light chain amino acid sequence of SEQ ID NO:42;

(n) the heavy chain amino acid sequence of SEQ ID NO:14 and the light chain amino acid sequence of SEQ ID NO:42;

(o) the heavy chain amino acid sequence of SEQ ID NO:15 and the light chain amino acid sequence of SEQ ID NO:42;

(p) the heavy chain amino acid sequence of SEQ ID NO:12 and the light chain amino acid sequence of SEQ ID NO:46;

(q) the heavy chain amino acid sequence of SEQ ID NO:14 and the light chain amino acid sequence of SEQ ID NO:46; or

(r) the heavy chain amino acid sequence of SEQ ID NO:15 and the light chain amino acid sequence of SEQ ID NO:46.

In an embodiment of the first and second aspect, the Ab is a Fc silent antibody.

In some embodiments, *p* is an integer from 1 to 8. In some embodiments, *p* is an integer from 1 to 5. In some embodiments, *p* is an integer from 2 to 4. In some embodiments, *p is* 2. In some embodiments, *p* is 4. In some embodiments, *p* is determined by liquid chromatography-mass spectrometry (LC-MS).

In some embodiments, the linker (L) comprises an attachment group, at least one spacer group, and at least one cleavable group. In some cases, the cleavable group comprises a pyrophosphate group and/or a self-immolative group. In specific embodiments, L comprises an attachment group; at least one bridging spacer group; and at least one cleavable

group comprising a pyrophosphate group and/or a self-immolative group.

In some embodiments, the antibody-drug conjugate comprises a linker-drug (or "linker-payload") moiety -(L-D) is of the formula (A):

$$-\left(R^1-L_1-E-D\right) \quad (A),$$

wherein $R^1$ is an attachment group, $L_1$ is a bridging spacer group, and E is a cleavable group.

In some embodiments, the cleavable group comprises a pyrophosphate group. In some embodiments, the cleavable group comprises:

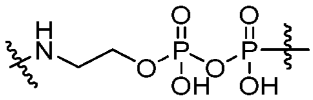
.

In some embodiments, the bridging spacer group comprises a polyoxyethylene (PEG) group. In some cases, the PEG group may be selected from PEG1, PEG2, PEG3, PEG4, PEG5, PEG6, PEG7, PEG8, PEG9, PEG10, PEG11, PEG12, PEG13, PEG14, and PEG15. In some embodiments, the bridging spacer group may comprise: $-CO-CH_2-CH_2-PEG12-$. In other embodiments, the bridging spacer group comprises a butanoyl, pentanoyl, hexanoyl, heptanoyl, or octanoyl group. In some embodiments, the bridging spacer group comprises a hexanoyl group.

In some embodiments the attachment group is formed from at least one reactive group selected from a maleimide group, thiol group, cyclooctyne group, and an azido group. For example, maleimide group may have the structure:

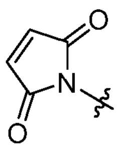
.

The azido group may have the structure: $-N=N^+=N^-$.

The cyclooctyne group may have the structure:

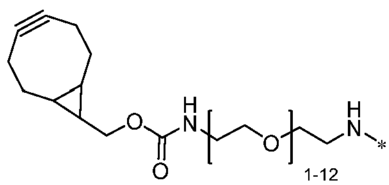
or
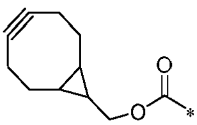
,

and wherein-* is a bond to the antibody.

In some cases, the cyclooctyne group has the structure:

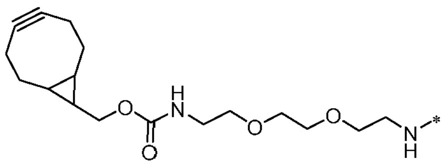
,

and wherein -* is a bond to the antibody.

In some embodiments, the attachment group has a formula comprising

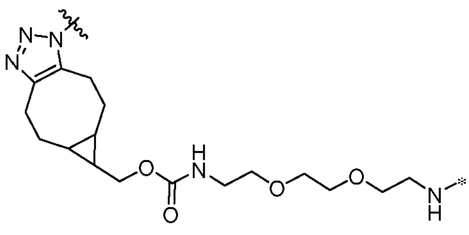 or 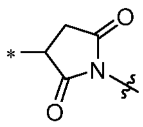,

and wherein -* is a bond to the antibody.

In some embodiments, the antibody is joined to the linker (L) by an attachment group selected from:

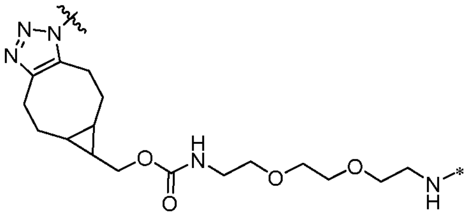 and 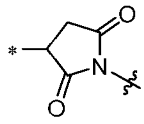,

wherein -* is a bond to the antibody, and wherein

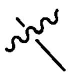

is a bond to the bridging spacer group.

In some embodiments, the bridging spacer group is joined to a cleavable group.

In some embodiments, the bridging spacer group is $-CO-CH_2-CH_2-PEG12-$.

In some embodiments, the cleavable group is -pyrophosphate-$CH_2-CH_2-NH_2$-.

In some embodiments, the cleavable group is joined to the Mcl-1 inhibitor (D).

In some embodiments, the cleavable group is joined to the Mcl-1 inhibitor (D) group through a phenyl-pyrimidinyl group.

In some embodiments, the linker comprises: an attachment group, at least one bridging spacer group, a peptide group, and at least one cleavable group.

In some embodiments, the antibody-drug conjugate comprises a linker-drug moiety, -(L-D), is of the formula (B):

$$\left(-R^1\text{-}L_1-Lp-E-(L_2)_m-D\right) \quad \text{(B)},$$

wherein $R^1$ is an attachment group, $L_1$ is a bridging spacer, Lp is a peptide group comprising 1 to 6 amino acid residues, E is a cleavable group, $L_2$ is a bridging spacer, m is 0 or 1; and D is an Mcl-1 inhibitor. In some cases, m is 1 and the bridging spacer comprises:

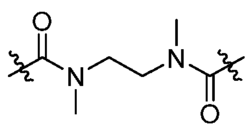.

In some embodiments, the at least one bridging spacer comprises a PEG group. In some cases, the PEG group is selected from, PEG1, PEG2, PEG3, PEG4, PEG5, PEG6, PEG7, PEG8, PEG9, PEG10, PEG11, PEG12, PEG13, PEG14, and PEG15. In some cases, the at least one bridging spacer is selected from *-C(O)-$CH_2$-$CH_2$-PEG1-**, *-C(O)-$CH_2$-PEG3-**, *-C(O)-$CH_2$-$CH_2$-PEG12**, *-NH-CH2-CH2-PEG1-**, a polyhydroxyalkyl group, and *-C(O)-N($CH_3$)-$CH_2$-$CH_2$-N($CH_3$)-C(O)-**, wherein ** indicates the point of direct or indirect attachment of the at least one bridging spacer to the attachment group and * indicates the point of direct or indirect attachment of the at least one bridging spacer to the peptide group..

In some embodiments, $L_1$ is selected from *-C(O)-$CH_2$-$CH_2$-PEG1-**, *-C(O)-$CH_2$-PEG3-**, *-C(O)-$CH_2$-$CH_2$-PEG12**, *-NH-CH2-CH2-PEG1-**, and a polyhydroxyalkyl group, wherein ** indicates the point of

direct or indirect attachment of $L_1$ to $R^1$ and * indicates the point of direct or indirect attachment of $L_1$ to Lp..

In some embodiments, m is 1 and $L_2$ is $-C(O)-N(CH_3)-CH_2-CH_2-N(CH_3)-C(O)-$.

In some embodiments, the peptide group comprises 1 to 12 amino acid residues. In some embodiments, the peptide group (Lp) comprises 1 to 10 amino acid residues. In some embodiments, the peptide group (Lp) comprises 1 to 8 amino acid residues. In some embodiments, the peptide group (Lp) comprises 1 to 6 amino acid residues. In some embodiments, the peptide group comprises 1 to 4 amino acid residues. In some embodiments, the peptide group comprises 1 to 3 amino acid residues. In some embodiments the peptide group comprises 1 to 2 amino acid residues. In some cases, the amino acid residues are selected from L-glycine (Gly), L-valine (Val), L-citrulline (Cit), L-cysteic acid (sulfo-Ala), L-lysine (Lys), L-isoleucine (Ile), L-phenylalanine (Phe), L-methionine (Met), L-asparagine (Asn), L-proline (Pro), L-alanine (Ala), L-leucine (Leu), L-tryptophan (Trp), and L-tyrosine (Tyr). For example, the peptide group may comprise Val-Cit, Val-Ala, Val-Lys, and/or sulfo-Ala-Val-Ala. In some embodiments, the peptide group (Lp) comprises 1 amino acid residue linked to a

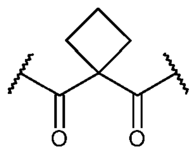

group. In some embodiments, the peptide group (Lp) comprises a group selected from:

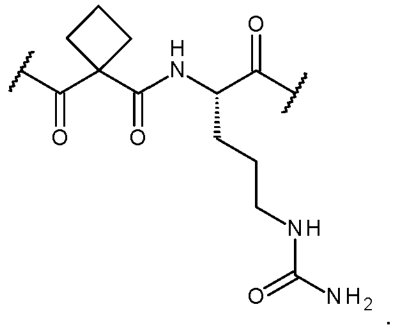

In some cases, the peptide group comprises a group selected from:

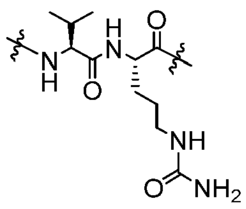
,
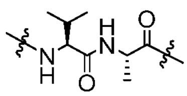
,
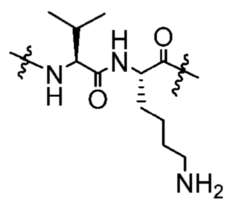
, and
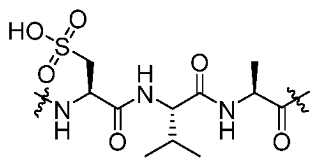
.

In some embodiments, the self-immolative group comprises para-aminobenzyl-carbamate, para-aminobenzyl-ammonium, para-amino-(sulfo)benzyl-ammonium, para-amino-(sulfo)benzyl-carbamate, para-amino-(alkoxy-PEG-alkyl)benzyl-carbamate, para-amino-(polyhydroxycarboxytetrahydropyranyl)alkyl-benzyl-carbamate, or para-amino-(polyhydroxycarboxytetrahydropyranyl)alkyl-benzyl-ammonium.

In some embodiments, m is 1 and the bridging spacer comprises

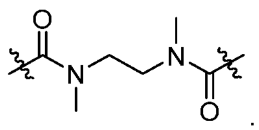

In some embodiments, the linker-drug moiety, -(L-D), is formed from a compound selected from:

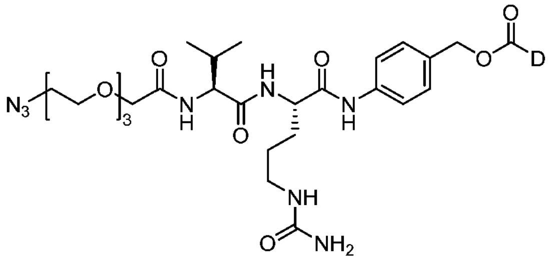

,

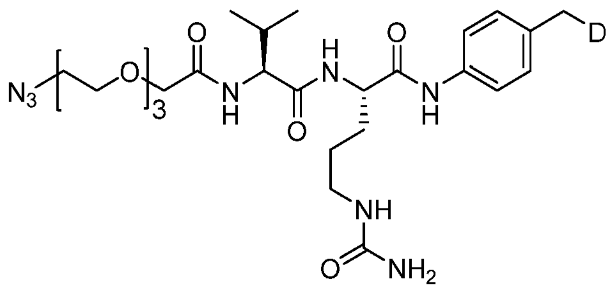

,

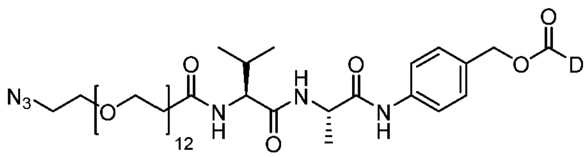

,

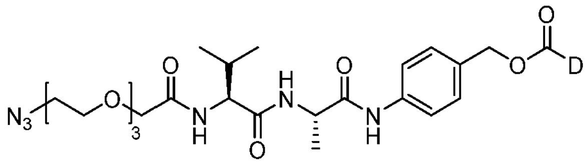

,

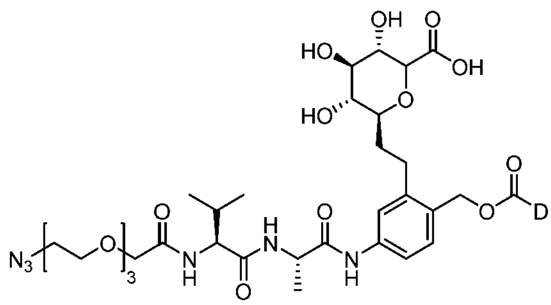

,

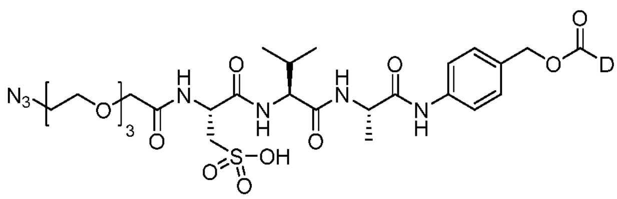

,

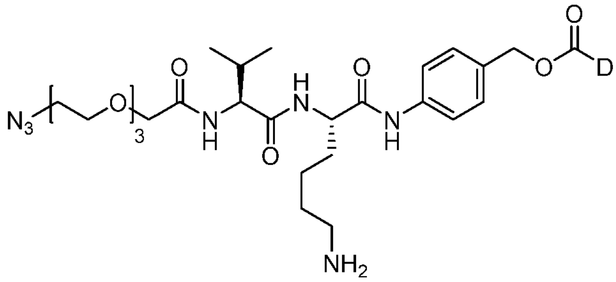

,

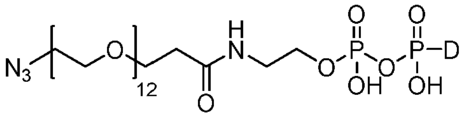

,

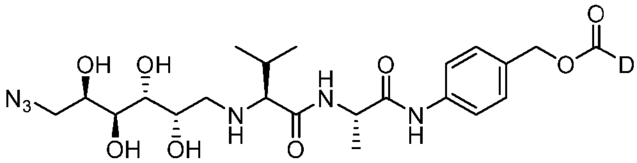

,

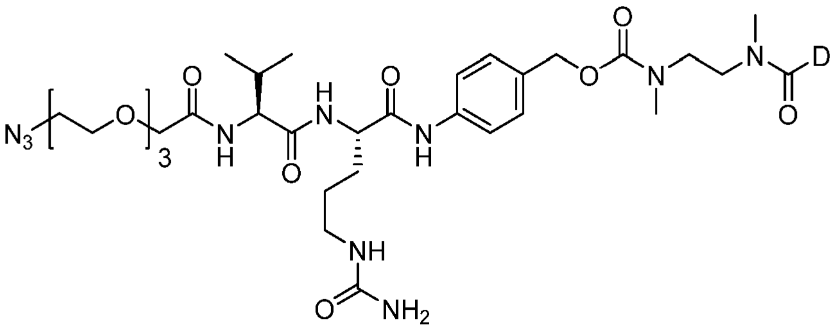

,

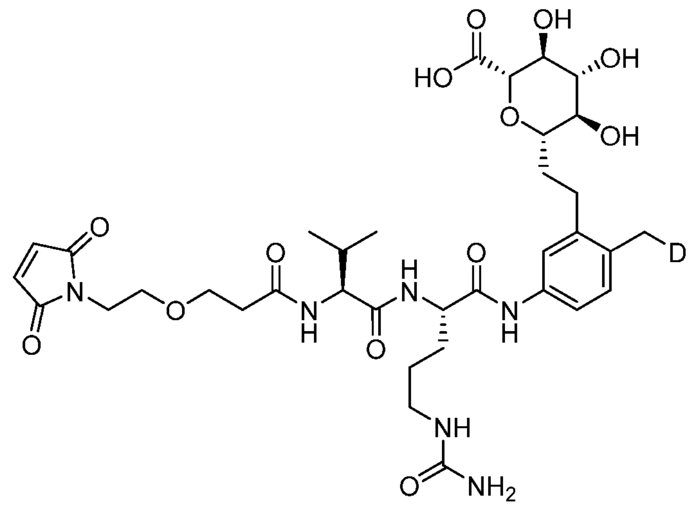

,

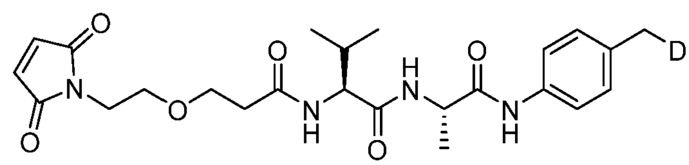

,

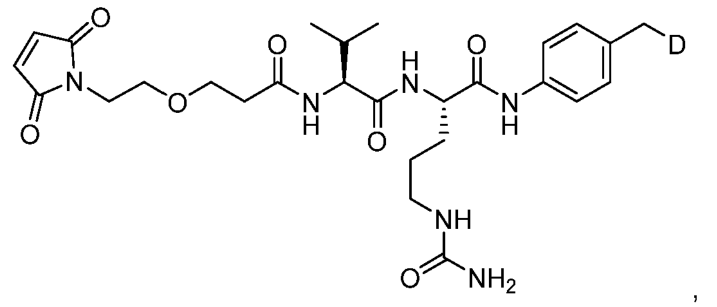

,

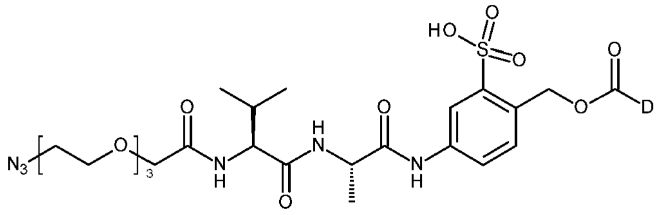

,

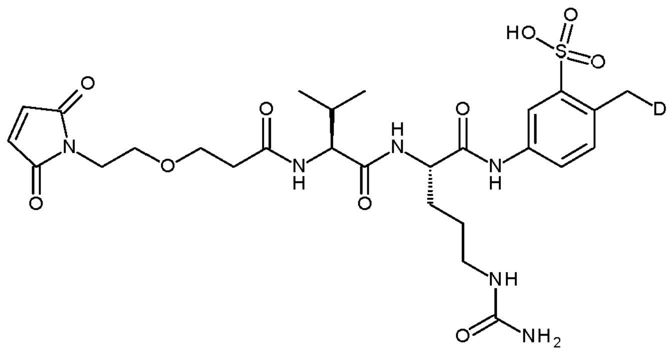

,

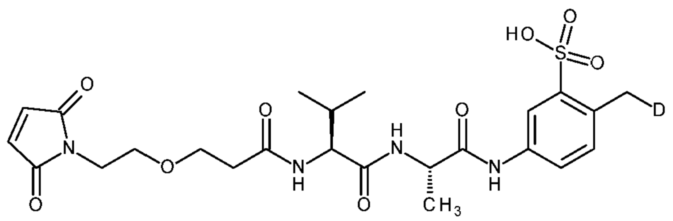

,

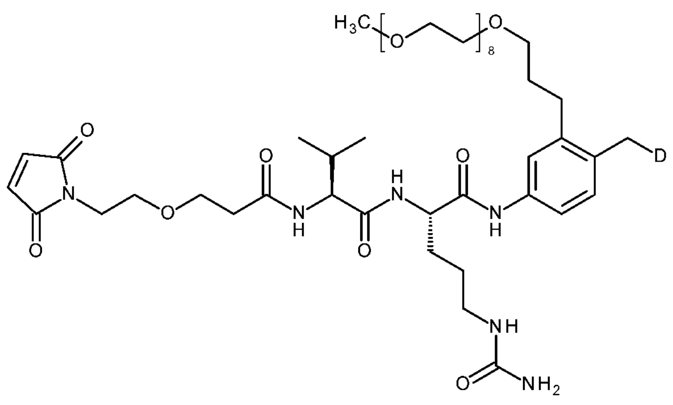

,

and

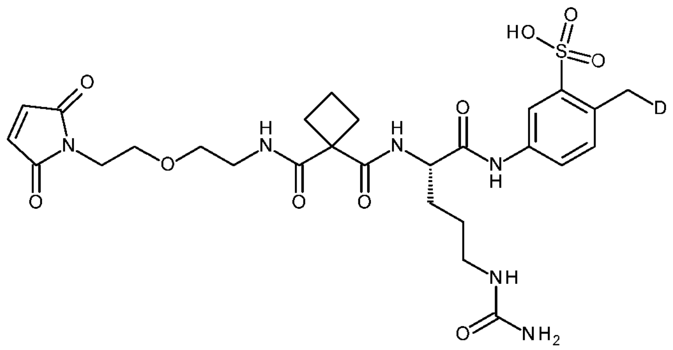

.

In some embodiments, the antibody-drug conjugate comprises the linker-drug group, -(L-D), which comprises a formula selected from:

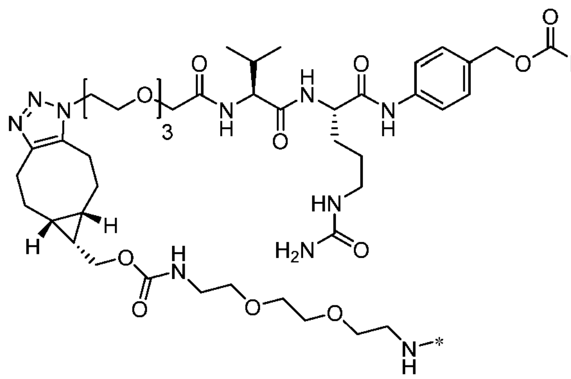

,

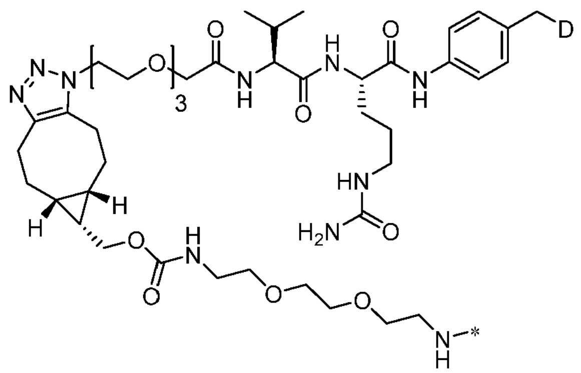

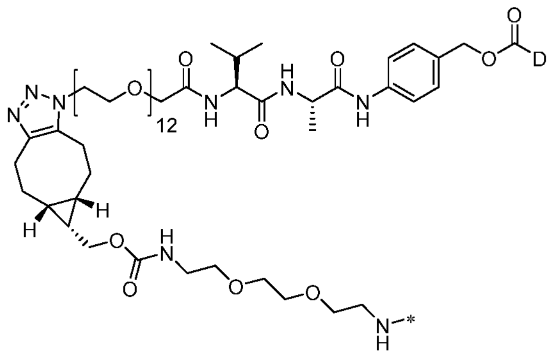

,

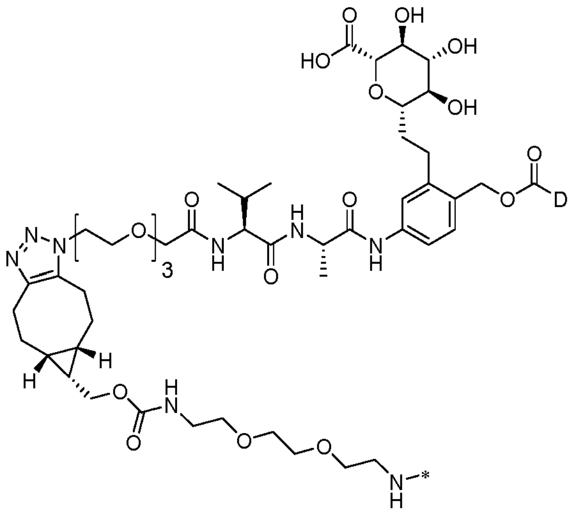

,

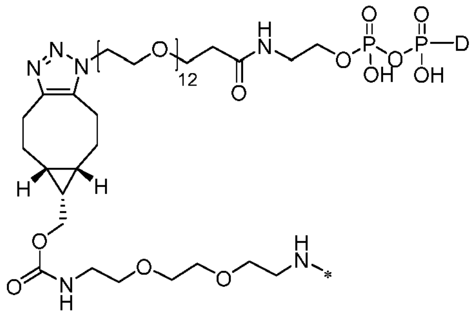

,

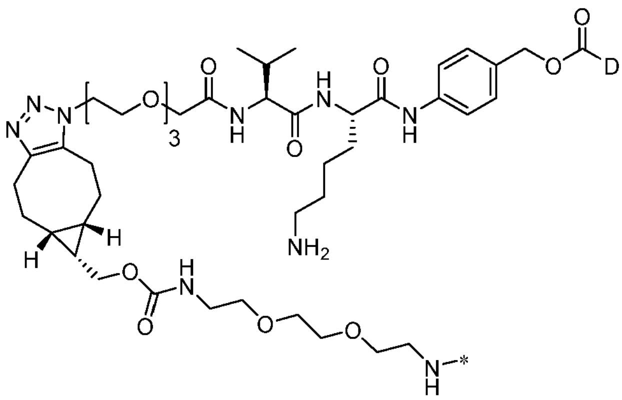

,

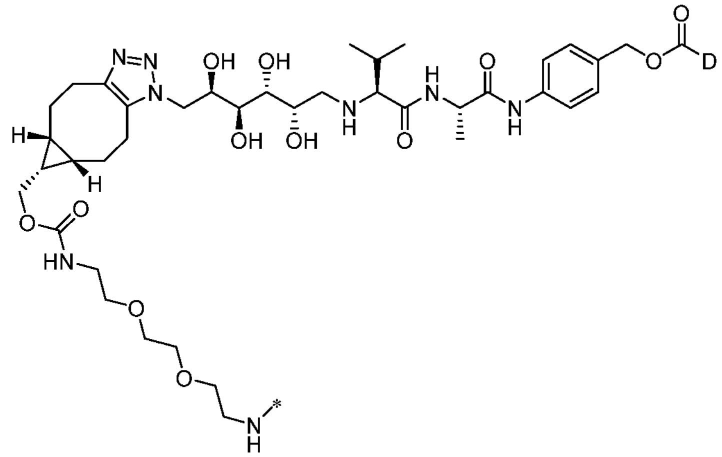

,

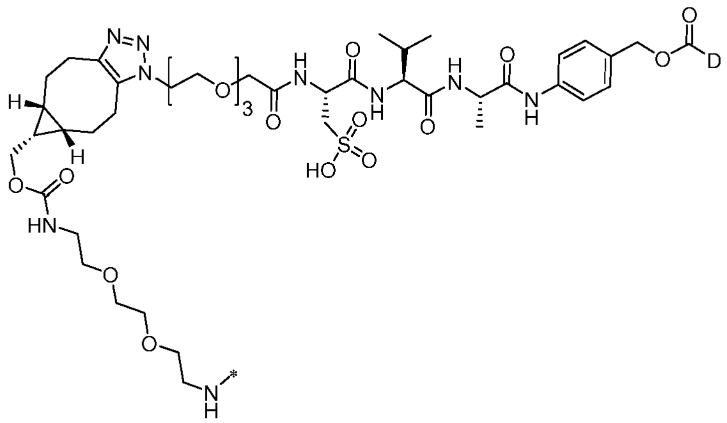

,

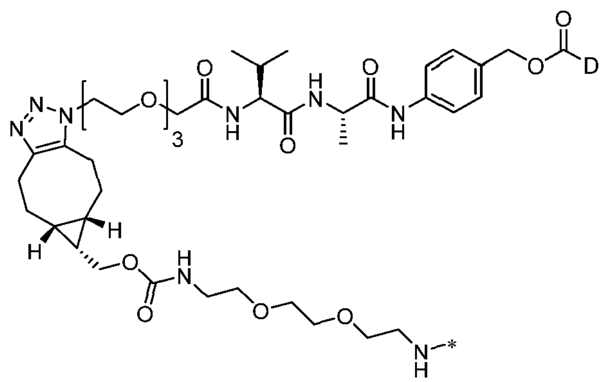

,

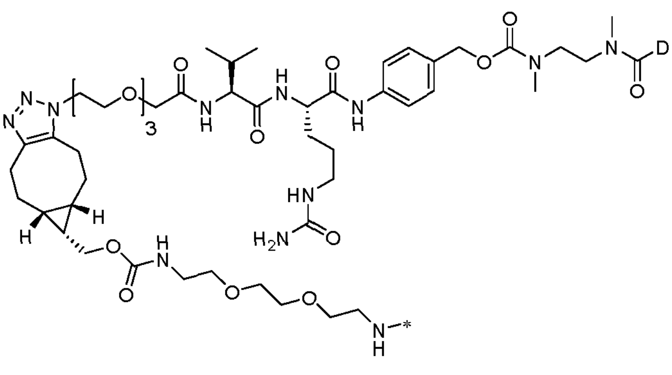

,

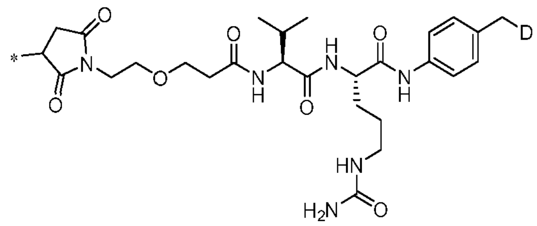

,

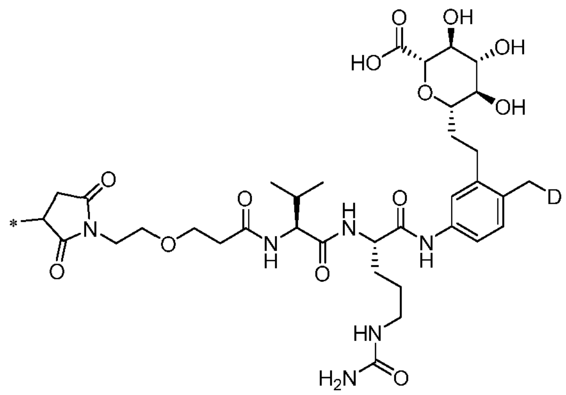

,

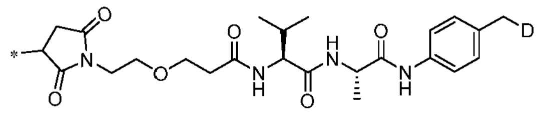

,

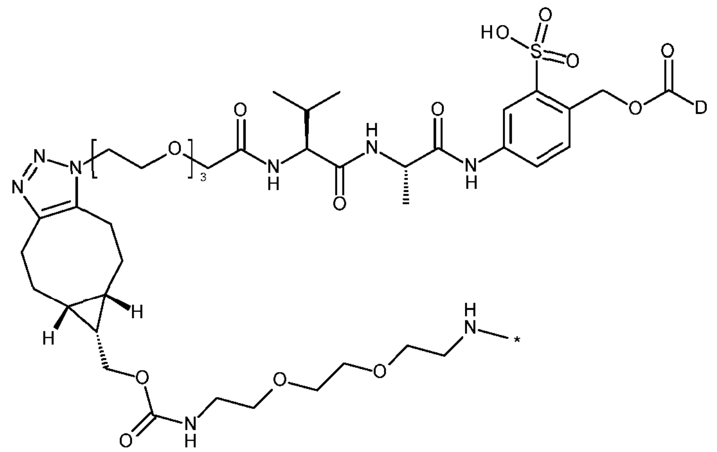

,

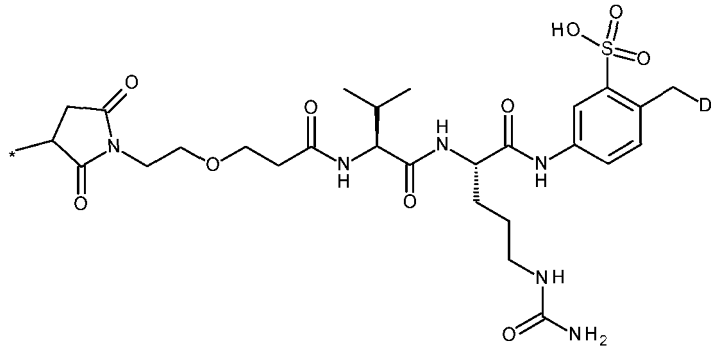

,

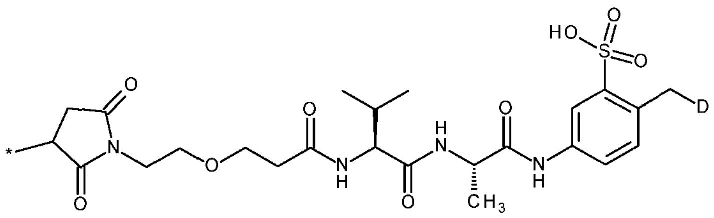

,

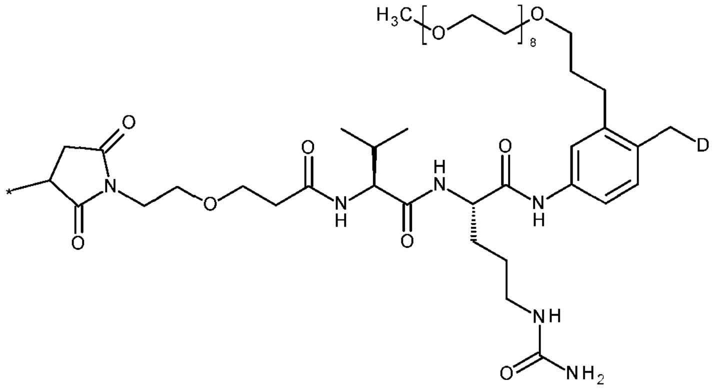

,

and

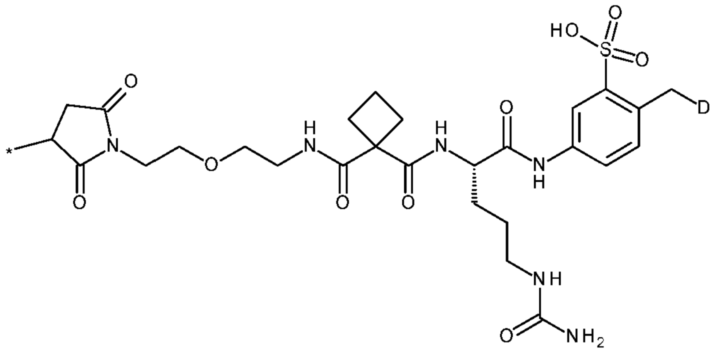

,

and

wherein -* is a bond to the antibody.

In some embodiments, the antibody-drug conjugate comprises the linker drug group, -(L-D), which is of the formula (C):

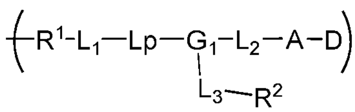

(C),

wherein: $R^1$ is an attachment group, $L_1$ is a bridging spacer; $L_p$ is a peptide group comprising 1 to 6 amino acids; D is an Mcl-1 inhibitor; $G_1$-$L_2$-A is a self-immolative spacer; $L_2$ is a bond, a methylene, a neopentylene or a $C_2$-$C_3$ alkenylene; A is a bond, -OC(=O)-*,

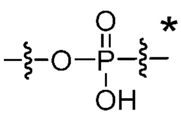

,

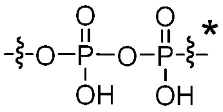, 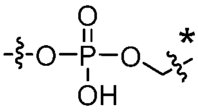, 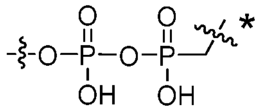, -OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; $L_3$ is a spacer moiety; and $R^2$ is a hydrophilic moiety.

In some embodiments, the antibody-drug conjugate comprises the linker drug group, -(L-D), which is of the formula (D):

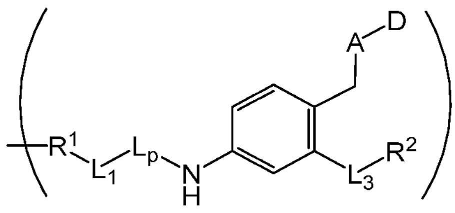 (D),

wherein: $R^1$ is an attachment group; $L_1$ is a bridging spacer; Lp is a peptide group comprising 1 to 6 amino acids; A is a bond, -OC(=O)-*

, 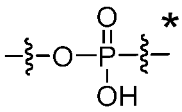, 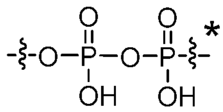,

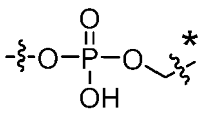, 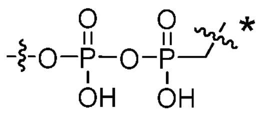,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; $L_3$ is a spacer moiety; and $R^2$ is a hydrophilic moiety.

In some embodiments, $L_1$ comprises:

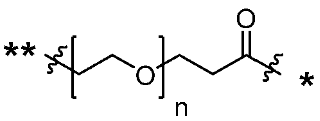, 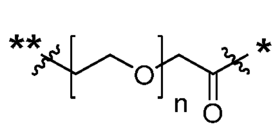,

or

*-CH(OH)CH(OH)CH(OH)CH(OH)-**,wherein each n is an integer from 1 to 12, wherein the * of $L_1$ indicates the point of direct or indirect attachment to Lp, and the ** of $L_1$ indicates the point of direct or indirect attachment to $R^1$.

In some embodiments, $L_1$ is

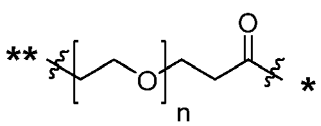

, and n is an integer from 1 to 12 wherein the * of $L_1$ indicates the point of direct or indirect attachment to Lp, and the ** of $L_1$ indicates the point of direct or indirect attachment to $R^1$.

In some embodiments, $L_1$ is

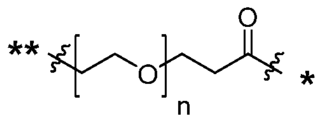

,

and n is 1, wherein the * of $L_1$ indicates the point of direct or indirect attachment to Lp, and the ** of $L_1$ indicates the point of direct or indirect attachment to $R^1$.

In some embodiments, $L_1$ is

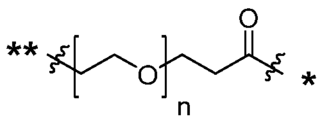

,

and n is 12, wherein the * of $L_1$ indicates the point of direct or indirect attachment to Lp, and the ** of $L_1$ indicates the point of direct or indirect attachment to $R^1$.

In some embodiments, $L_1$ is

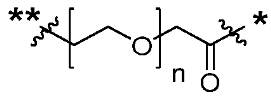

,

and n is an integer from 1 to 12, wherein the * of $L_1$ indicates the point of direct or indirect attachment to Lp, and the ** of $L_1$ indicates the point of direct or indirect attachment to $R^1$.

In some embodiments, $L_1$ comprises

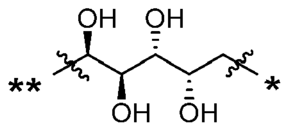

,

wherein the * of $L_1$ indicates the point of direct or indirect attachment to Lp, and the ** of $L_1$ indicates the point of direct or indirect attachment to $R^1$.

In some embodiments, $L_1$ is a bridging spacer comprising:

$*-C(=O)(CH_2)_mO(CH_2)_m-**$; $*-C(=O)((CH_2)_mO)_t(CH_2)_n-**$; $*-C(=O)(CH_2)_m-**$;

$*-C(=O)NH((CH_2)_mO)_t(CH_2)_n-**$;

$*-C(=O)O(CH_2)_mSSC(R^3)_2(CH_2)_mC(=O)NR^3(CH_2)_mNR^3C(=O)(CH_2)_m-**$;

$*-C(=O)O(CH_2)_mC(=O)NH(CH_2)_m-**$; $*-C(=O)(CH_2)_mNH(CH_2)_m-**$;

$*-C(=O)(CH_2)_mNH(CH_2)_nC(=O)-**$; $*-C(=O)(CH_2)_mX_1(CH_2)_m-**$;

$*-C(=O)((CH_2)_mO)_t(CH_2)_nX_1(CH_2)_n-**$; $*-C(=O)(CH_2)_mNHC(=O)(CH_2)_n-**$;

$*-C(=O)((CH_2)_mO)_t(CH_2)_nNHC(=O)(CH_2)_n-**$; $*-C(=O)(CH_2)_mNHC(=O)(CH_2)_nX_1(CH_2)_n-**$;

$*-C(=O)((CH_2)_mO)_t(CH_2)_nNHC(=O)(CH_2)_nX_1(CH_2)_n-**$;

$*-C(=O)((CH_2)_mO)_t(CH_2)_nC(=O)NH(CH_2)_m-**$; $*-C(=O)(CH_2)_mC(R^3)_2-**$

or

$*-C(=O)(CH_2)_mC(=O)NH(CH_2)_m-**$,

where the * of $L_1$ indicates the point of direct or indirect attachment to Lp, and the ** of $L_1$ indicates the point of direct or indirect attachment to $R^1$, wherein $X_1$ is

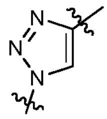 , 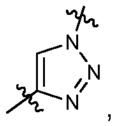 , 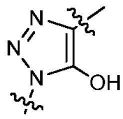 or 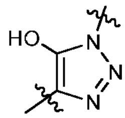 ;

and

each m is independently selected from 1, 2, 3, 4, 5, 6, 7, 8, 9 and 10;
each n is independently selected from 1, 2, 3, 4, 5, 6, 7, 8, 9 and 10; and
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30.

In some embodiments, $R^2$ is a hydrophilic moiety comprising polyethylene glycol, polyalkylene glycol, a polyol, a polysarcosine, a sugar, an oligosaccharide, a polypeptide, or $C_2$-$C_6$ alkyl substituted with 1 to 3

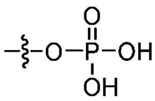 or 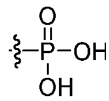

groups. In some embodiments, $R^2$ is

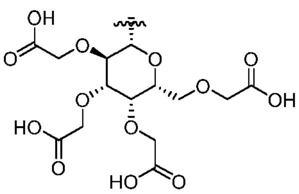 , 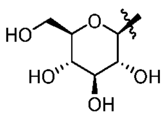 , 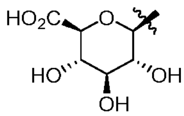 , 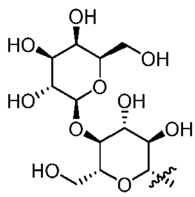 , 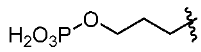 ,

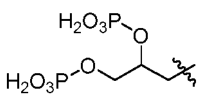 , 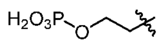 , 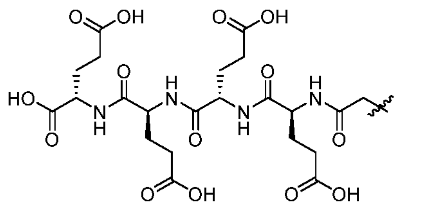

or

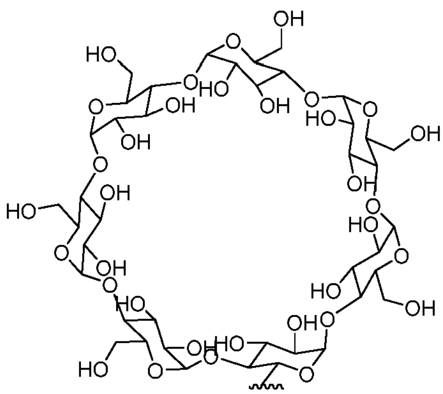 , 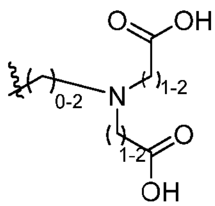 , 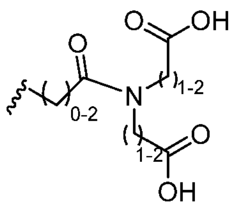 ,

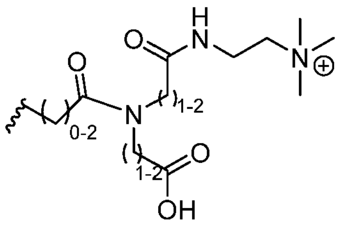

,

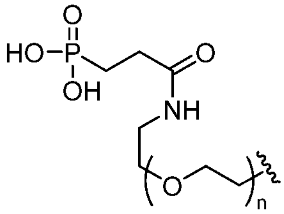

wherein n is an interger between 1 and 6,

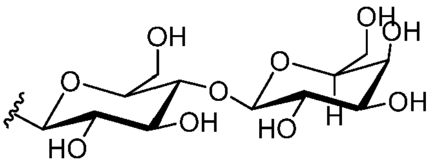

,

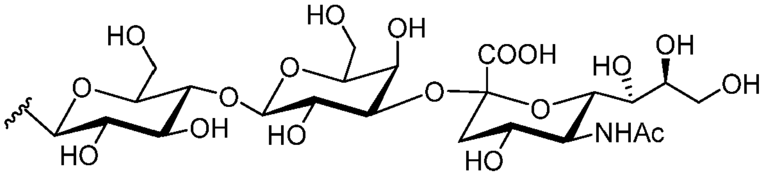

,

or

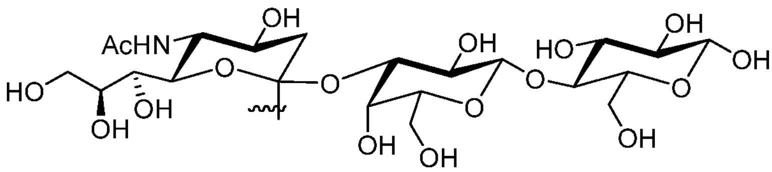

.

In some embodiments, the hydrophilic moiety comprises a polyethylene glycol of formula:

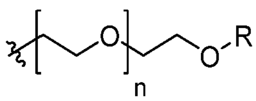

or

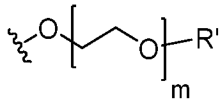

,

wherein R is H, $-CH_3$, $-CH_2CH_2NHC(=O)OR_a$, $-CH_2CH_2NHC(=O)R_a$, or $-CH_2CH_2C(=O)OR_a$, R' is OH, $-OCH_3$, $-CH_2CH_2NHC(=O)OR_a$, $-CH_2CH_2NHC(=O)R_a$, or $-OCH_2CH_2C(=O)OR_a$, in which $R^a$ is H or $C_{1-4}$ alkyl optionally substiltuted with either OH or $C_{1-4}$ alkoxyl, and each of m and n is an integer between 2 and 25 (e.g. between 3 and 25).

In some embodiments,
the hydrophilic moiety comprises

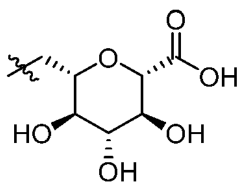

.

In some embodiments, the hydrophilic moiety comprises a polysarcosine, e.g., with the following moiety

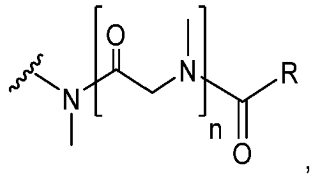,

wherein n is an integer between 3 and 25; and R is H, $-CH_3$ or $-CH_2CH_2C(=O)OH$.

In some embodiments, $L_3$ is a spacer moiety having the structure

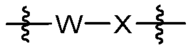,

wherein:

W is $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, $-NHC(=O)C(R^b)_2NHC(=O)-$, $-CH_2N(X-R^2)C(=O)O-$, $-C(=O)N(X-R^2)-$, $-CH_2N(X-R^2)C(=O)-$, $-C(=O)NR^b-$, $-C(=O)NH-$, $-CH_2NR^bC(=O)-$, $-CH_2NR^bC(=O)NH-$, $-CH_2NR^bC(=O)NR^b-$, $-NHC(=O)-$, $-NHC(=O)O-$, $-NHC(=O)NH-$, $-OC(=O)NH-$, $-S(O)_2NH-$, $-NHS(O)_2-$, $-C(=O)-$, $-C(=O)O-$ or $-NH-$, wherein each $R^b$ is independently selected from H, $C_1$-$C_6$alkyl, and $C_3$-$C_8$ cycloalkyl; and

X is a bond, triazolyl, or $-CH_2$-triazolyl-.

In some embodiments, $L_3$ is a spacer moiety having the structure

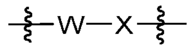,

wherein:

W is $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, $-NHC(=O)C(R^b)_2NHC(=O)-$, $-CH_2N(X-R^2)C(=O)O-$, $-C(=O)N(X-R^2)-$, $-CH_2N(X-R^2)C(=O)-$, $-C(=O)NR^b-$, $-C(=O)NH-$, $-CH_2NR^bC(=O)-$, $-CH_2NR^bC(=O)NH-$, $-CH_2NR^bC(=O)NR^b-$, $-NHC(=O)-$, $-NHC(=O)O-$, $-NHC(=O)NH-$, $-OC(=O)NH-$, $-S(O)_2NH-$, $-NHS(O)_2-$, $-C(=O)-$, $-C(=O)O-$ or $-NH-$, wherein each $R^b$ is independently selected from H, $C_1$-$C_6$alkyl, and $C_3$-$C_8$ cycloalkyl; and

X is $-CH_2$-triazolyl-$C_{1-4}$ alkylene-$OC(O)NHS(O)_2NH-$, $-C_{4-6}$ cycloalkylene-$OC(O)NHS(O)_2NH-$, $-(CH_2CH_2O)_n-C(O)NHS(O)_2NH-$, $-(CH_2CH_2O)_n-C(O)NHS(O)_2NH-(CH_2CH_2O)_n-$, or $-CH_2$-triazolyl-$C_{1-4}$ alkylene-$OC(O)NHS(O)_2NH-(CH_2CH_2O)_n-$, wherein each n independently is 1, 2, or 3.

In some embodiments, the attachment group is formed by a reaction comprising at least one reactive group. In some cases, the attachment group is formed by reacting: a first reactive group that is attached to the linker, and a second reactive group that is attached to the antibody or is an amino acid residue of the antibody.

In some embodiments, at least one of the reactive groups comprises:

a thiol,
a maleimide,
a haloacetamide,
an azide,
an alkyne,
a cyclooctene,
a triaryl phosphine,
an oxanorbornadiene,
a cyclooctyne,
a diaryl tetrazine,
a monoaryl tetrazine,
a norbornene,
an aldehyde,
a hydroxylamine,
a hydrazine,
$NH_2$-NH-C(=O)-,
a ketone,

a vinyl sulfone,
an aziridine,
an amino acid residue,

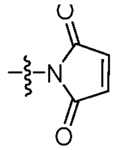,

$-ONH_2$, $-NH_2$,

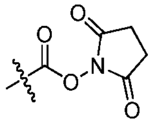, 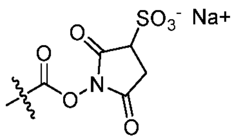, 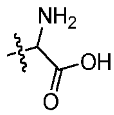, 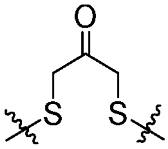,

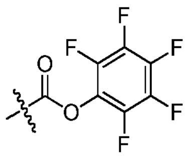, 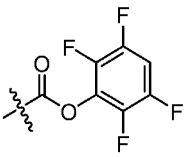, 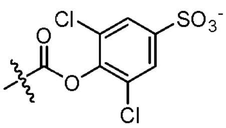, $-N_3$, 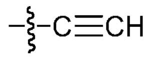,

$-SH$, $-SR^3$, $-SSR^4$, $-S(=O)_2(CH=CH_2)$, $-(CH_2)_{2S}(=O)_2(CH=CH_2)$, $-NHS(=O)_2(CH=CH_2)$, $-NHC(=O)CH_2Br$, $-NHC(=O)CH_2I$,

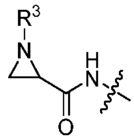,

$-C(O)NHNH_2$,

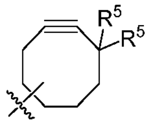,

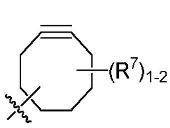, 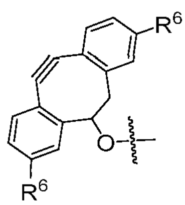, 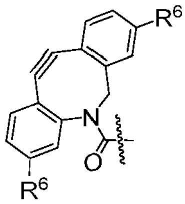, 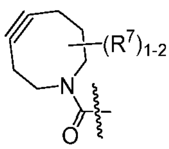, 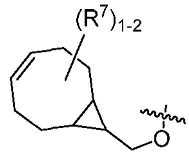,

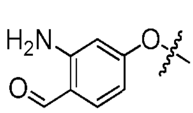, 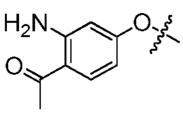, 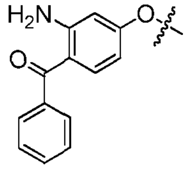, 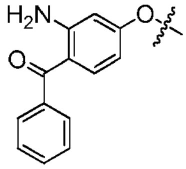,

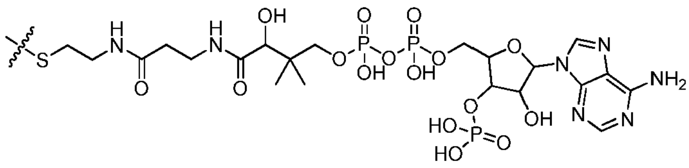

,

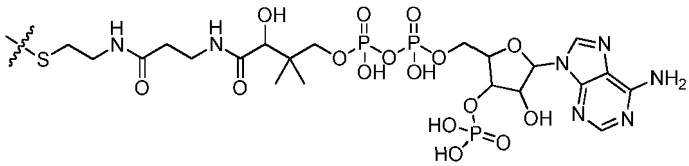

,

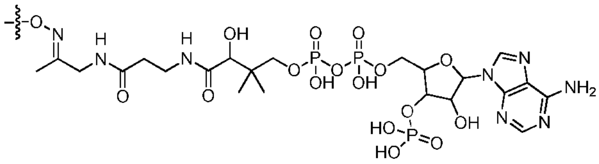

,

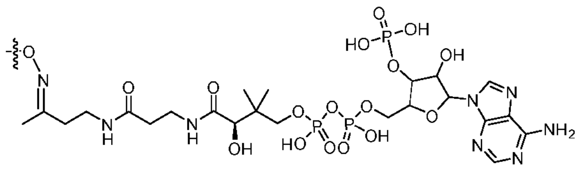

,

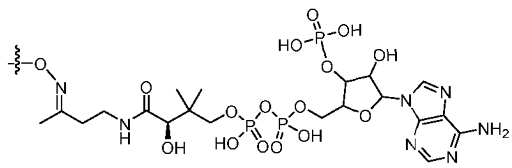

,

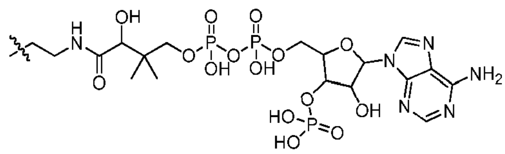

,

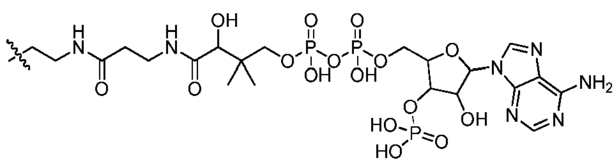

,

or

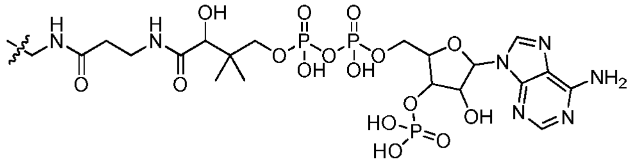

;

wherein:

each $R^3$ is independently selected from H and $C_1$-$C_6$alkyl;
each $R^4$ is 2-pyridyl or 4-pyridyl;
each $R^5$ is independently selected from H, $C_1$-$C_6$alkyl, F, Cl, and -OH;
each $R^6$ is independently selected from H, $C_1$-$C_6$alkyl, F, Cl, $-NH_2$, $-OCH_3$, $-OCH_2CH_3$, - $N(CH_3)_2$, -CN, $-NO_2$ and-OH;
each $R^7$ is independently selected from H, $C_{1-6}$alkyl, fluoro, benzyloxy substituted with - C(=O)OH, benzyl substituted with -C(=O)OH, $C_{1-4}$alkoxy substituted with -C(=O)OH and $C_{1-4}$alkyl substituted with -C(=O)OH.

In some embodiments, the first reactive group and second reactive group comprise:

a thiol and a maleimide,
a thiol and a haloacetamide,
a thiol and a vinyl sulfone,
a thiol and an aziridine,
an azide and an alkyne,
an azide and a cyclooctyne,
an azide and a cyclooctene,
an azide and a triaryl phosphine,
an azide and an oxanorbornadiene,
a diaryl tetrazine and a cyclooctene,
a monoaryl tetrazine and a norbornene,
an aldehyde and a hydroxylamine,
an aldehyde and a hydrazine,
an aldehyde and $NH_2$-NH-C(=O)-,
a ketone and a hydroxylamine,
a ketone and a hydrazine,
a ketone and NH2-NH-C(=O)-,
a hydroxylamine and

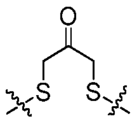,

an amine and

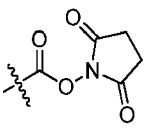, 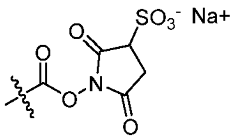, 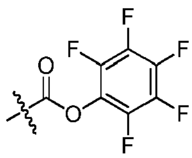, 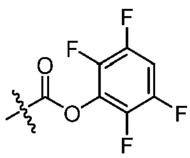,

or

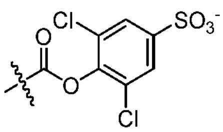

, or
a CoA or CoA analogue and a serine residue.

In some embodiments, the attachment group comprises a group selected from:

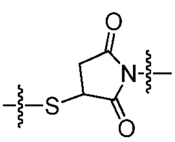;

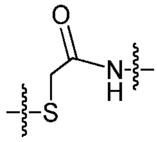;

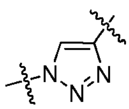 or 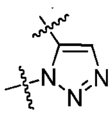;

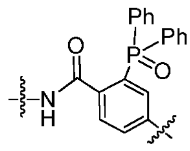;

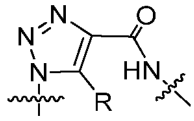;

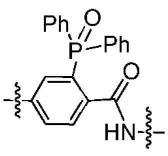;

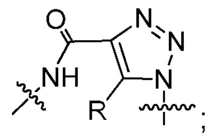;

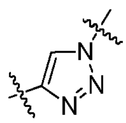 or 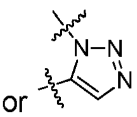;

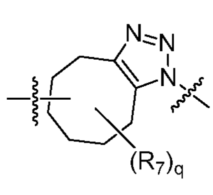 or 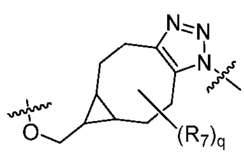 or 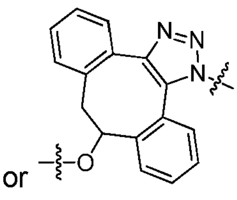;

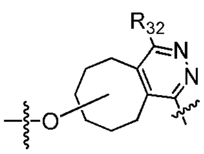 or 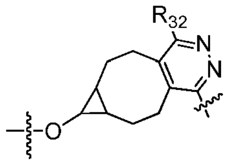;

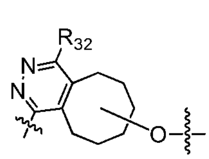 or 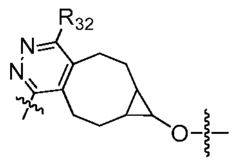 ;

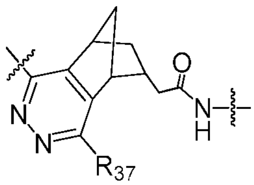 ;

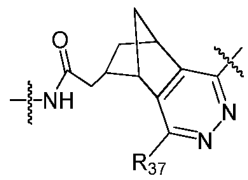 ;

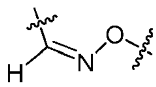 ;

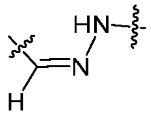 ;

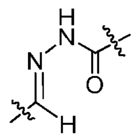 ;

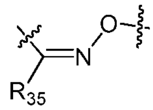 ;

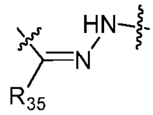 ;

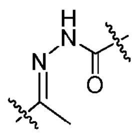 ;

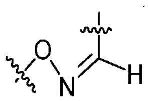 ;

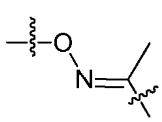
;

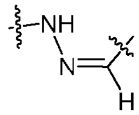
;

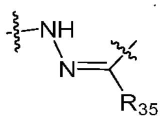
;

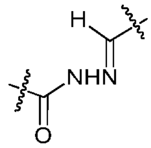
;

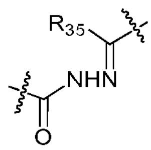
;

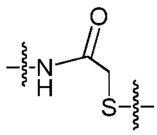
;

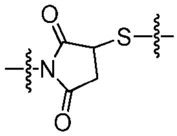
;

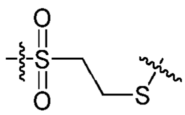
;

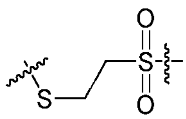
;

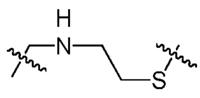 or 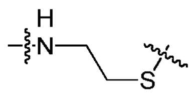
;

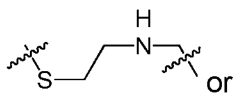 or 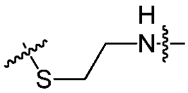;

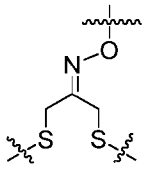;

amide;

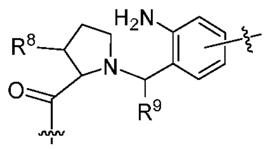;

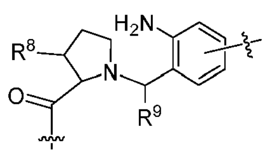;

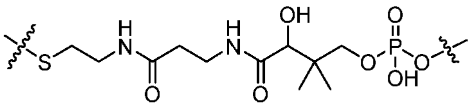;

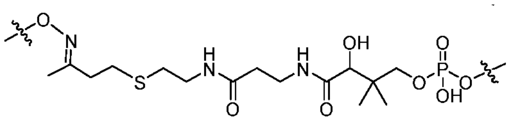;

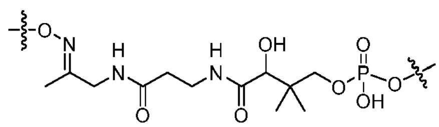;

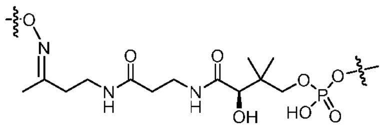;

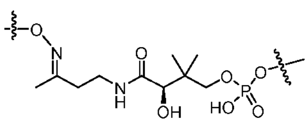;

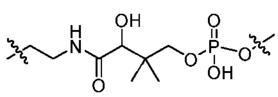;

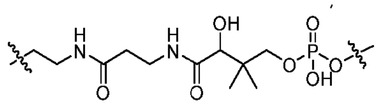

:

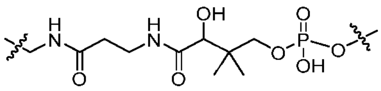

;

and
disulfide,
wherein:

$R^{32}$ is H, $C_{1-4}$ alkyl, phenyl, pyrimidine or pyridine;
$R^{35}$ is H, $C_{1-6}$ alkyl, phenyl or $C_{1-4}$ alkyl substituted with 1 to 3 -OH groups;
each $R^7$ is independently selected from H, $C_{1-6}$ alkyl, fluoro, benzyloxy substituted with -C(=O)OH, benzyl substituted with -C(=O)OH, $C_{1-4}$ alkoxy substituted with -C(=O)OH and $C_{1-4}$ alkyl substituted with -C(=O)OH;
$R^{37}$ is independently selected from H, phenyl and pyridine;
q is 0, 1, 2 or 3;
$R^8$ is H or methyl; and
$R^9$ is H, $-CH_3$ or phenyl.

In some embodiments, the peptide group (Lp) comprises 1 to 6 amino acid residues. In some embodiments, the peptide group (Lp) comprises 1 to 4 amino acid residues. In some embodiments, the peptide group comprises 1 to 3 amino acid residues. In some embodiments, the peptide group comprises 1 to 2 amino acid residues. In some embodiments, the amino acid residues are selected from L-glycine (Gly), L-valine (Val), L-citrulline (Cit), L-cysteic acid (sulfo-Ala), L-lysine (Lys), L-isoleucine (Ile), L-phenylalanine (Phe), L-methionine (Met), L-asparagine (Asn), L-proline (Pro), L-alanine (Ala), L-leucine (Leu), L-tryptophan (Trp), and L-tyrosine (Tyr). In some embodiments, the peptide group comprises Val-Cit, Phe-Lys, Val-Ala, Val-Lys, Leu-Cit, sulfo-Ala-Val, and/or sulfo-Ala-Val-Ala. In some embodiments, Lp is selected from:

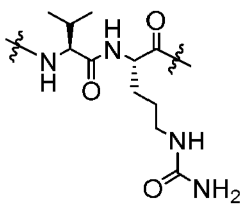, 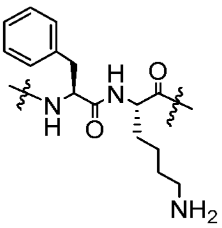, 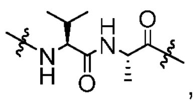, 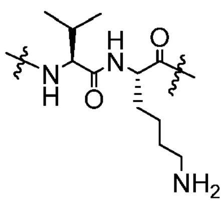, 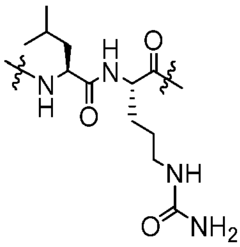,

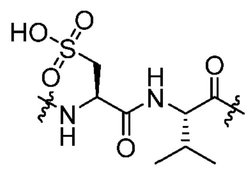, and 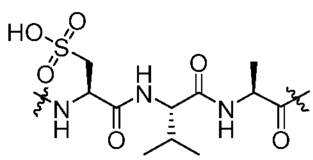.

In some embodiments, the linker-drug group -(L-D) comprises or is formed from a compound of formula:

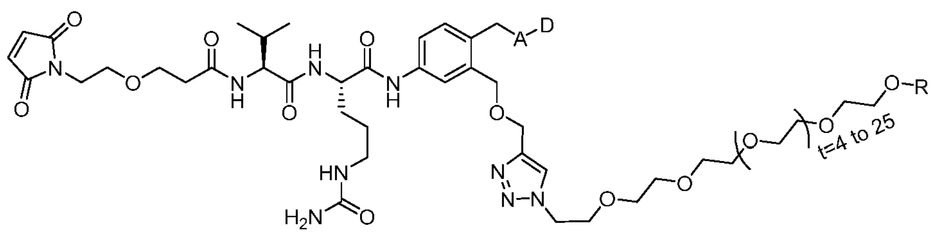

,

wherein:

R is H, $-CH_3$ or $-CH_2CH_2C(=O)OH$;

A is a bond, -OC(=O)-*,

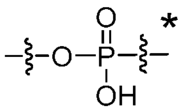 , 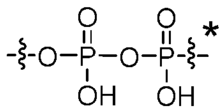 , 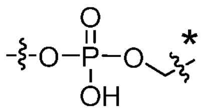 ,

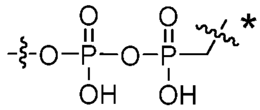 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and
D is an Mcl-1 inhibitor.

In some embodiments, the linker-drug group -(L-D) comprises or is formed from a compound of formula:

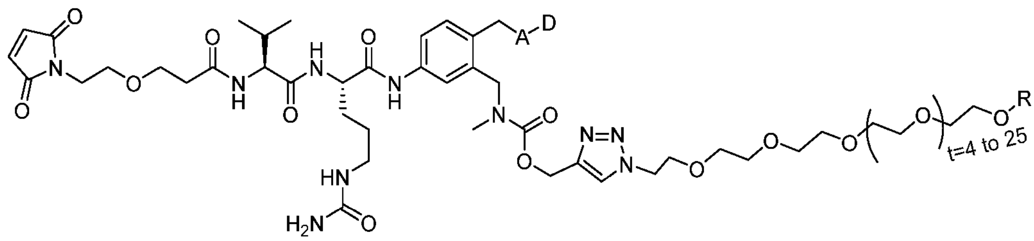 ,

wherein:
R is H, $-CH_3$ or $-CH_2CH_2C(=O)OH$;

A is a bond, -OC(=O)-*,

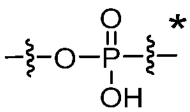 , 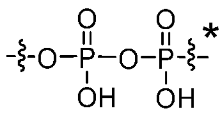 , 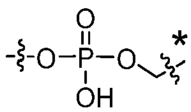 ,

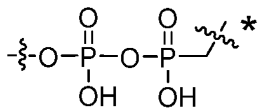 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and
D is an Mcl-1 inhibitor.

In some embodiments, the linker-drug group -(L-D) comprises or is formed from a compound of formula:

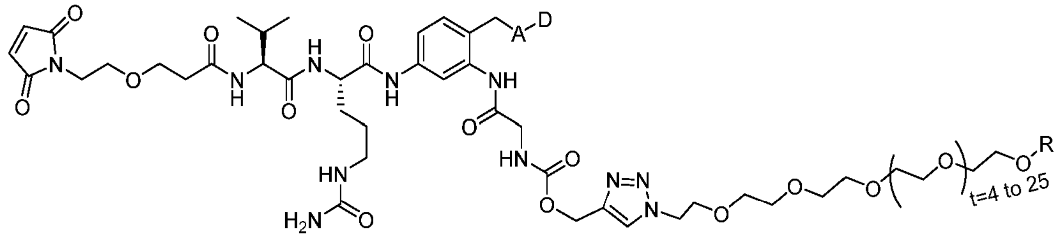
,

wherein:
R is H, $-CH_3$ or $-CH_2CH_2C(=O)OH$;

A is a bond, -OC(=O)-*,

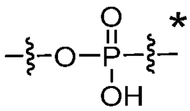
,
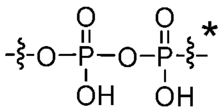
,
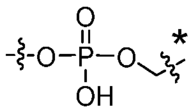
,

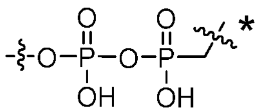
,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and
D is an Mcl-1 inhibitor.

In some embodiments, the linker-drug group -(L-D) comprises or is formed from a compound of formula:

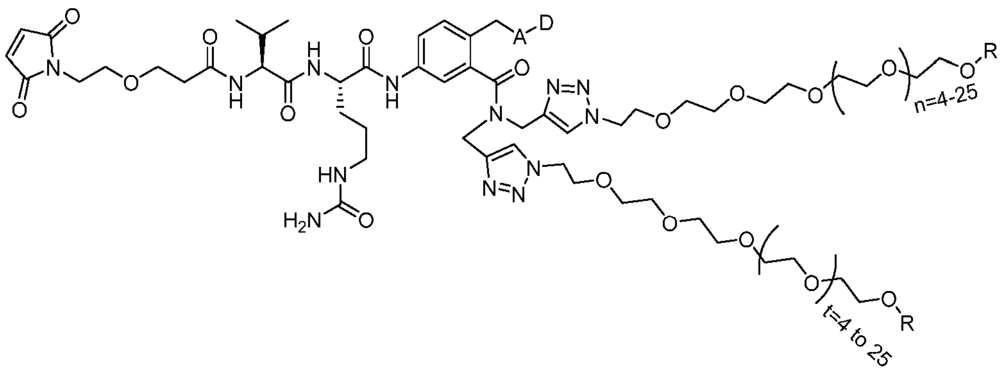
,

wherein:
each R is independently selected from H, $-CH_3$, and $-CH_2CH_2C(=O)OH$;

A is a bond, -OC(=O)-*,

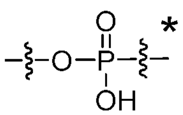
,
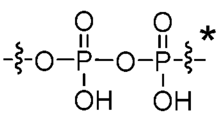
,
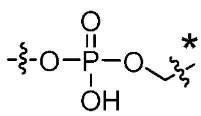
,

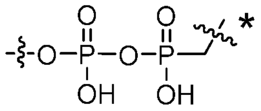

,

-OC(=O)N($CH_3$)$CH_2CH_2$N($CH_3$)C(=O)-* or -OC(=O)N($CH_3$)C($R^a$)$_{2C}$($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

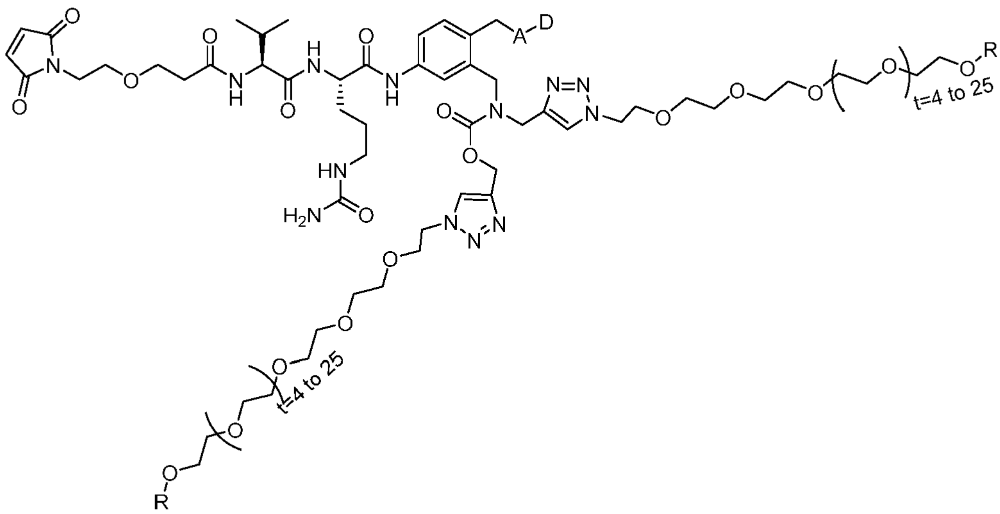

,

wherein:
each R is independently selected from H, $-CH_3$, and $-CH_2CH_2C(=O)OH$;

A is a bond, -OC(=O)-*,

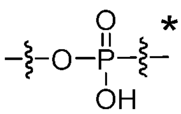

,

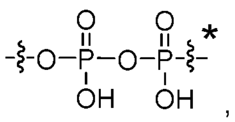

,

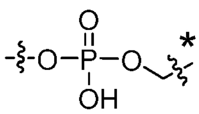

,

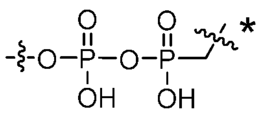

,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

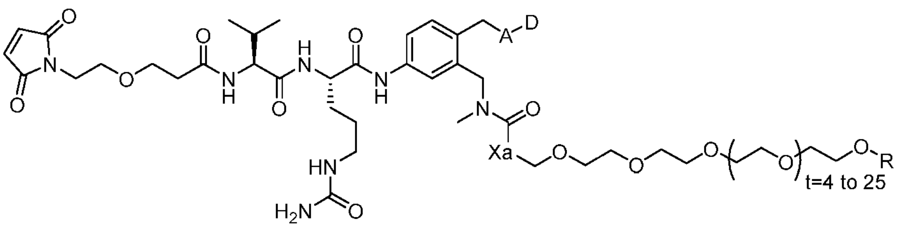

,

wherein:
Xa is $-CH_2-$, $-OCH_2-$, $-NHCH_2-$ or $-NRCH_2-$ and each R independently is H, $-CH_3$ or $-CH_2CH_2C(=O)OH$;

A is a bond, -OC(=O)-*

, 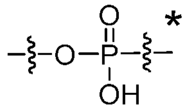 , 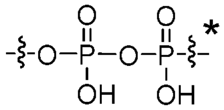 , 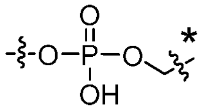 ,

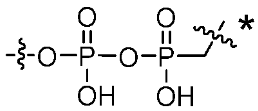 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

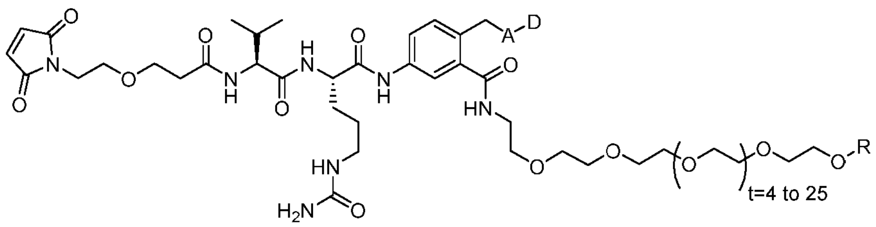

,

wherein:
R is H, $-CH_3$ or $-CH_2CH_2C(=O)OH$;

A is a bond, -OC(=O)-*,

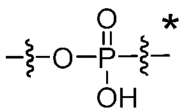 , 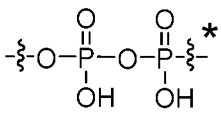 , 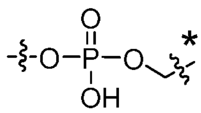 ,

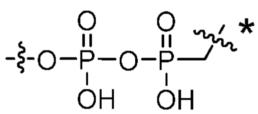 ,

$-OC(=O)N(CH_3)CH_2CH_2N(CH_3)C(=O)-*$ or
$-OC(=O)N(CH_3)C(R^a)_{2C}(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and
D is an Mcl-1 inhibitor.

In some embodiments, the linker-drug group -(L-D) comprises or is formed from a compound of formula:

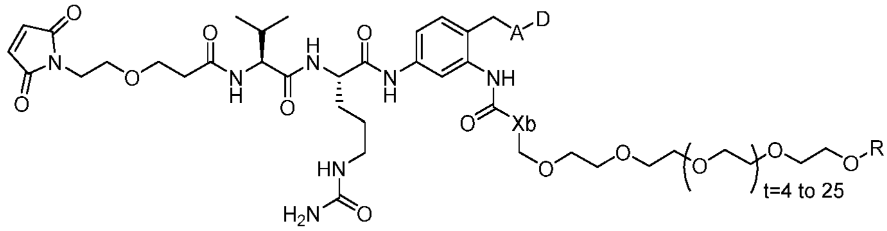

,

wherein:

Xb is $-CH_2-$, $-OCH_2-$, $-NHCH_2-$ or $-NRCH_2-$ and each R independently is H, $-CH_3$ or $-CH_2CH_2C(=O)OH$;

A is a bond, -OC(=O)-*,

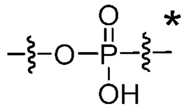 , 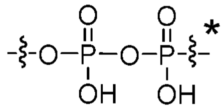 , 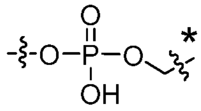 ,

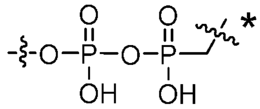 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and

D is an Mcl-1 inhibitor.

In some embodiments, the linker-drug group -(L-D) comprises or is formed from a compound of formula:

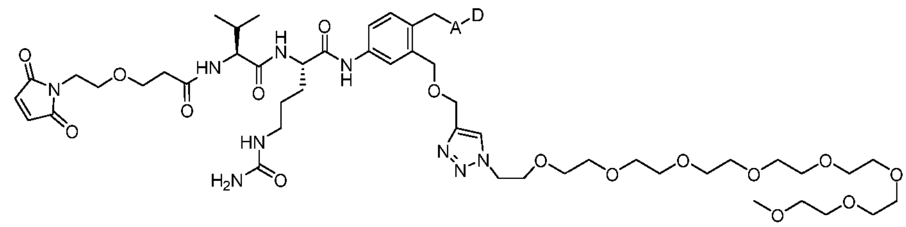

, wherein:

A is a bond, -OC(=O)-*,

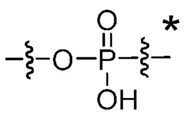 , 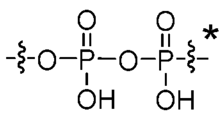 , 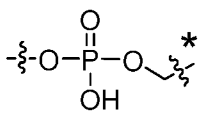 ,

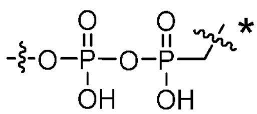 , -

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and

D is an Mcl-1 inhibitor.

In some embodiments, the linker-drug group -(L-D) comprises or is formed from a compound of formula:

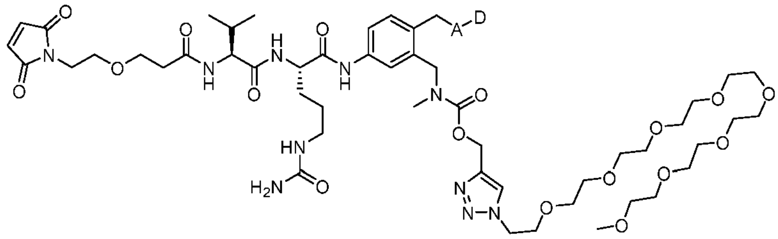
,

wherein:

A is a bond, -OC(=O)-*,

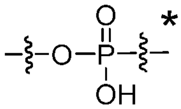
,
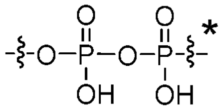
,
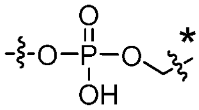
,

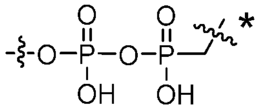
,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

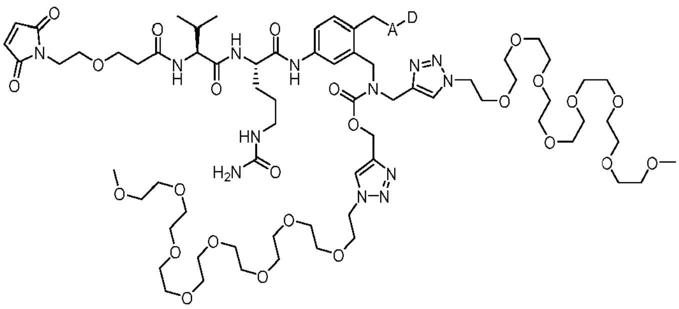
,

wherein:

A is a bond, -OC(=O)-*,

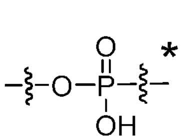
,
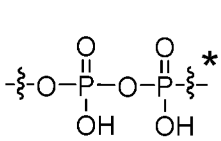
,
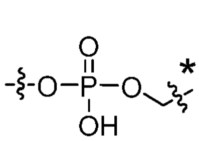
,

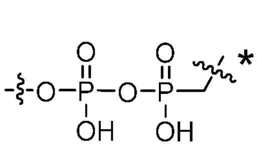
, .

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

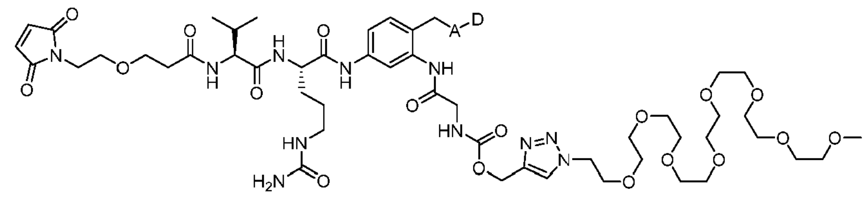,

wherein:

A is a bond, -OC(=O)-*,

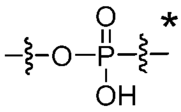, 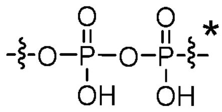, 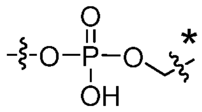,

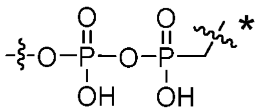,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

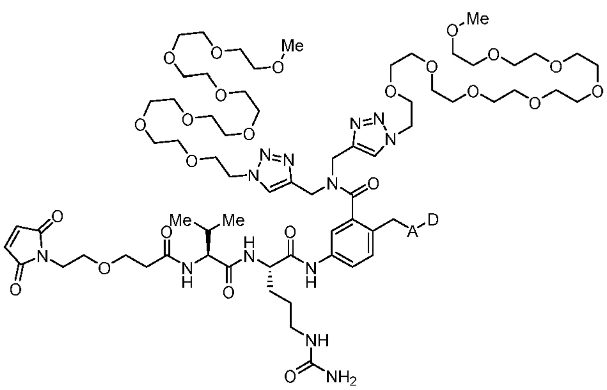,

wherein:

A is a bond, -OC(=O)-*,

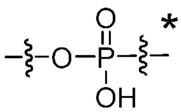, 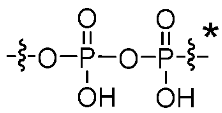, 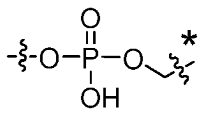,

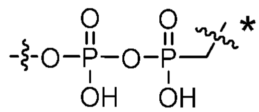,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is inde-

pendently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

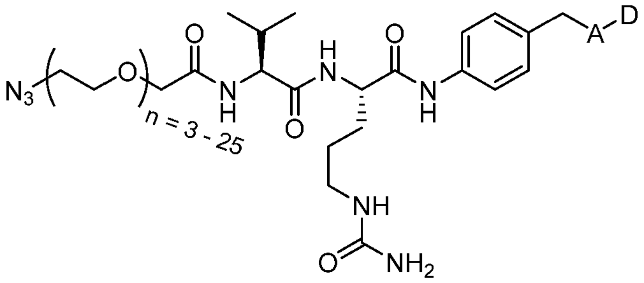

,

wherein:
A is a bond, -OC(=O)-*,

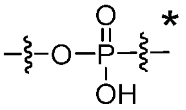
,
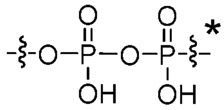
,
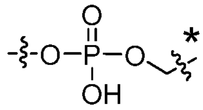
,

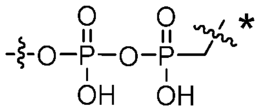
,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

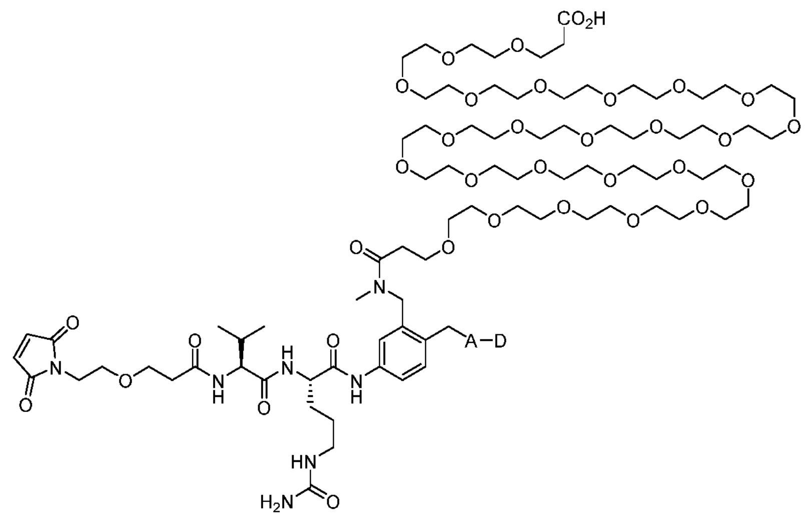

,

wherein:
A is a bond, -OC(=O)-*,

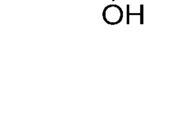
,
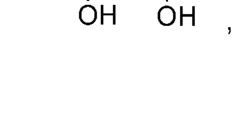
,
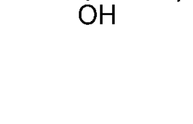
,

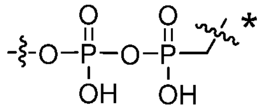,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and D is an Mcl-1 inhibitor.

In some embodiments, the linker-drug group -(L-D) comprises or is formed from a compound of formula:

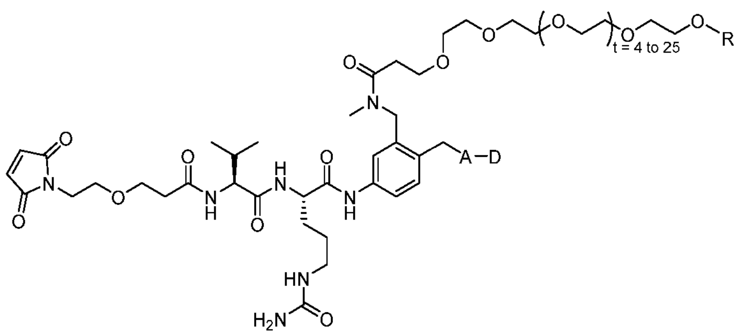,

wherein:

each R independently is H, $-CH_3$ or $-CH_2CH_2C(=O)OH$;
A is a bond, -OC(=O)-*,

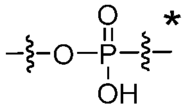, 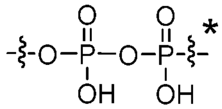, 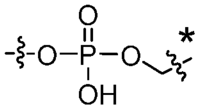,

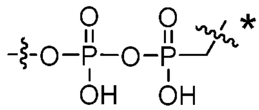,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and
D is an Mcl-1 inhibitor.

In some embodiments, A is a bond.
In some embodiments, R is $-CH_3$.
In some embodiments, the Mcl-1 inhibitor (D) comprises a compound of Formula (I):

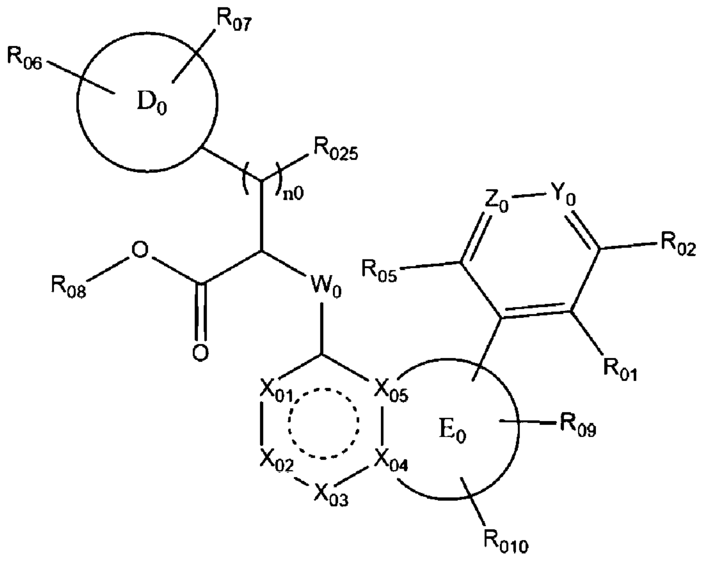

(I)

wherein:

Ring $D_0$ is a cycloalkyl group, a heterocycloalkyl group, an aryl group or a heteroaryl group,
Ring $E_0$ is a furyl, thienyl or pyrrolyl ring,
$X_{01}$, $X_{03}$, $X_{04}$ and $X_{05}$ independently of one another is a carbon atom or a nitrogen atom,
$X_{02}$ is a C-$R_{026}$ group or a nitrogen atom,

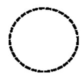

means that the ring is aromatic,
$Y_0$ is a nitrogen atom or a C-$R_{03}$ group,
$Z_0$ is a nitrogen atom or a C-$R_{04}$ group,
$R_{01}$ is a halogen atom, a linear or branched ($C_1$-$C_6$)alkyl group, a linear or branched ($C_2$-$C_6$)alkenyl group, a linear or branched ($C_2$-$C_6$)alkynyl group, a linear or branched ($C_1$-$C_6$)haloalkyl group, a hydroxy group, a hydroxy($C_1$-$C_6$)alkyl group, a linear or branched ($C_1$-$C_6$)alkoxy group, -S-($C_1$-$C_6$)alkyl group, a cyano group, a nitro group, -$Cy_{08}$, -($C_0$-$C_6$)alkyl-$NR_{011}R_{011}$', -O-($C_1$-$C_6$)alkyl-$NR_{011}R_{011}$', -O-($C_1$-$C_6$)alkyl-$R_{012}$, -C(O)-$OR_{011}$, -O-C(O)-$R_{011}$, -C(O)-$NR_{011}R_{011}$', -$NR_{011}$-C(O)-$R_{011}$', -$NR_{011}$-C(O)-$OR_{011}$', -($C_1$-$C_6$)alkyl-$NR_{011}$-C(O)-$R_{011}$', -$SO_2$-$NR_{011}R_{011}$', or -$SO_2$-($C_1$-$C_6$)alkyl,
$R_{02}$, $R_{03}$, $R_{04}$ and $R_{05}$ independently of one another are a hydrogen atom, a halogen atom, a linear or branched ($C_1$-$C_6$)alkyl group, a linear or branched ($C_2$-$C_6$)alkenyl group, a linear or branched ($C_2$-$C_6$)alkynyl group, a linear or branched ($C_1$-$C_6$)haloalkyl, a hydroxy group, a hydroxy($C_1$-$C_6$)alkyl group, a linear or branched ($C_1$-$C_6$)alkoxy group, a -S-($C_1$-$C_6$)alkyl group, a cyano group, a nitro group, -($C_0$-$C_6$)alkyl-$NR_{011}$ $R_{011}$', -O-$Cy_{01}$, -($C_0$-$C_6$)alkyl-$Cy_{01}$, -($C_2$-$C_6$)alkenyl-$Cy_{01}$, -($C_2$-$C_6$)alkynyl-$Cy_{01}$, -O-($C_1$-$C_6$)alkyl-$NR_{011}R_{011}$', -O-($C_1$-$C_6$)alkyl-$R_{031}$, -O-($C_1$-$C_6$)alkyl-$R_{012}$, -C(O)-$OR_{011}$, -O-C(O)-$R_{011}$, -C(O)-$NR_{011}$ $R_{011}$', -$NR_{011}$-C(O)-$R_{011}$', -$NR_{011}$-C(O)-$OR_{011}$', -($C_1$-$C_6$)alkyl-$NR_{011}$-C(O)-$R_{011}$', -$SO_2$-$NR_{011}R_{011}$', or -$SO_2$-($C_1$-$C_6$)alkyl,
or the pair ($R_{01}$, $R_{02}$), ($R_{02}$, $R_{03}$), ($R_{03}$, $R_{04}$), or ($R_{04}$, $R_{05}$) together with the carbon atoms to which they are attached form an aromatic or non-aromatic ring containing 5 to 7 ring members, which optionally contains 1 to 3 heteroatoms selected from O, S and N, wherein the resulting ring is optionally substituted by 1 or 2 groups selected from halogen, linear or branched ($C_1$-$C_6$)alkyl, ($C_0$-$C_6$)alkyl-$NR_{011}R_{011}$', -$NR_{013}R_{013}$', -($C_0$-$C_6$)alkyl-$Cy_{01}$ or oxo,
$R_{06}$ and $R_{07}$ independently of one another are a hydrogen atom, a halogen atom, a linear or branched ($C_1$-$C_6$)alkyl group, a linear or branched ($C_2$-$C_6$)alkenyl group, a linear or branched ($C_2$-$C_6$)alkynyl group, a linear or branched ($C_1$-$C_6$)haloalkyl, a hydroxy group, a linear or branched ($C_1$-$C_6$)alkoxy group, a -S-($C_1$-$C_6$)alkyl group, a cyano group, a nitro group, -($C_0$-$C_6$)alkyl-$NR_{011}R_{011}$', -O-($C_1$-$C_6$)alkyl-$NR_{011}R_{011}$', -O-$Cy_{01}$, -($C_0$-$C_6$)alkyl-$Cy_{01}$, -($C_2$-$C_6$)alkenyl-$Cy_{01}$, -($C_2$-$C_6$)alkynyl-$Cy_{01}$, -O-($C_1$-$C_6$)alkyl-$R_{012}$, -C(O)-$OR_{11}$, -O-C(O)-$R_{011}$, -C(O)-$NR_{011}R_{011}$', -$NR_{11}$-C(O)-$R_{011}$', -$NR_{011}$-C(O)-$OR_{011}$', -($C_1$-$C_6$)alkyl-$NR_{011}$-C(O)-$R_{011}$', -$SO_2$-$NR_{011}R_{011}$', or -$SO_2$-($C_1$-$C_6$)alkyl,
or the pair ($R_{06}$, $R_{07}$), when fused with the two adjacent carbon atoms, together with the carbon atoms to which they are

attached form an aromatic or non-aromatic ring containing 5 to 7 ring members, which optionally contains 1 to 3 heteroatoms selected from O, S and N, wherein the resulting ring is optionally substituted by a linear or branched ($C_1$-$C_6$)alkyl group, -$NR_{013}R_{013}$', -($C_0$-$C_6$)alkyl-$Cy_{01}$ or an oxo,

$W_0$ is a -$CH_2$- group, a -NH- group or an oxygen atom,

$R_{08}$ is a hydrogen atom, a linear or branched ($C_1$-$C_8$)alkyl group, a -$CHR_{0a}R_{0b}$ group, an aryl group, a heteroaryl group, an aryl($C_1$-$C_6$)alkyl group or a heteroaryl($C_1$-$C_6$)alkyl group,

$R_{09}$ is a hydrogen atom, a linear or branched ($C_1$-$C_6$)alkyl group, a linear or branched ($C_2$-$C_6$)alkenyl group, a linear or branched ($C_2$-$C_6$)alkynyl group, -$Cy_{02}$, -($C_1$-$C_6$)alkyl-$Cy_{02}$, -($C_2$-$C_6$)alkenyl-$Cy_{02}$, -($C_2$-$C_6$)alkynyl-$Cy_{02}$, -$Cy_{02}$-$Cy_{03}$, -($C_2$-$C_6$)alkynyl-O-$Cy_{02}$, -$Cy_{02}$-($C_0$-$C_6$)alkyl-O-($C_6$-$C_6$)alkyl-$Cy_{03}$, a halogen atom, a cyano group, -C(O)-$R_{014}$, or -C(O)-$NR_{014}R_{014}$',

$R_{010}$ is a hydrogen atom, a linear or branched ($C_1$-$C_6$)alkyl group, a linear or branched ($C_2$-$C_6$)alkenyl group, a linear or branched ($C_2$-$C_6$)alkynyl group, an aryl($C_1$-$C_6$)alkyl group, a ($C_1$-$C_6$)cycloalkylalkyl group, a linear or branched ($C_1$-$C_6$)haloalkyl, or -($C_1$-$C_6$)alkyl-O-$Cy_{04}$,

or the pair ($R_{09}$, $R_{010}$), when fused with the two adjacent carbon atoms, together with the carbon atoms to which they are attached form an aromatic or non-aromatic ring containing 5 to 7 ring members, which optionally contains 1 to 3 heteroatoms selected from O, S and N,

$R_{011}$ and $R_{011}$' independently of one another are a hydrogen atom, an optionally substituted linear or branched ($C_1$-$C_6$) alkyl group, or -($C_0$-$C_6$)alkyl-$Cy_{01}$, or the pair ($R_{011}$, $R_{011}$') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the nitrogen may be substituted by 1 or 2 groups selected from a hydrogen atom and a linear or branched ($C_1$-$C_6$)alkyl group, and wherein one or more of the carbon atoms of the linear or branched ($C_1$-$C_6$)alkyl group is optionally deuterated,

$R_{012}$ is -$Cy_{05}$, -$Cy_{05}$-($C_0$-$C_6$)alkyl-O-($C_0$-$C_6$)alkyl-$Cy_{06}$, -$Cy_{05}$-($C_0$-$C_6$)alkyl-$Cy_{06}$, -$Cy_{05}$-($C_0$-$C_6$)alkyl-$NR_{011}$-($C_0$-$C_6$) alkyl-$Cy_{06}$, -$Cy_{05}$-$Cy_{06}$-O-($C_0$-$C_6$)alkyl-$Cy_{07}$, -$Cy_{05}$-($C_0$-$C_6$)alkyl-O-($C_0$-$C_6$)alkyl-$Cy_{09}$, -$Cy_{05}$-($C_0$-$C_6$)alkyl-$Cy_{09}$, -NH-C(O)-NH-$R_{011}$, -$Cy_{05}$-($C_0$-$C_6$)alkyl-$NR_{011}$-($C_0$-$C_6$)alkyl-$Cy_{09}$, -C(O)-$NR_{011}R_{011}$', -$NR_{011}R_{011}$', -$OR_{011}$, -$NR_{011}$-C(O)-$R_{011}$', -O-($C_1$-$C_6$)alkyl-$OR_{011}$, -$SO_2$-$R_{011}$, and -C(O)-$OR_{011}$,

$R_{013}$, $R_{013}$', $R_{014}$ and $R_{014}$' independently of one another are a hydrogen atom or an optionally substituted linear or branched ($C_1$-$C_6$)alkyl group,

$R_{0a}$ is a hydrogen atom or a linear or branched ($C_1$-$C_6$)alkyl group,

$R_{0b}$ is a -O-C(O)-O-$R_{0c}$ group, a -O-C(O)-$NR_{0c}R_{0c}$' group, or a -O-P(O)($OR_{0c}$)$_2$ group,

$R_{0c}$ and $R_{0c}$' independently of one another are a hydrogen atom, a linear or branched ($C_1$-$C_8$)alkyl group, a cycloalkyl group, a ($C_1$-$C_6$)alkoxy($C_1$-$C_6$)alkyl group, or a ($C_1$-$C_6$)alkoxycarbonyl($C_1$-$C_6$)alkyl group,

or the pair ($R_{0c}$, $R_{0c}$') together with the nitrogen atom to which they are attached form a non-aromatic ring composed of from 5 to 7 ring members, which may contain in addition to the nitrogen atom from 1 to 3 heteroatoms selected from oxygen and nitrogen, wherein the nitrogen is optionally substituted by a linear or branched ($C_1$-$C_6$)alkyl group,

$Cy_{01}$, $Cy_{02}$, $Cy_{03}$, $Cy_{04}$, $Cy_{05}$, $Cy_{06}$, $Cy_{07}$, $Cy_{08}$ and $Cy_{010}$ independently of one another, represent a cycloalkyl group, a heterocycloalkyl group, an aryl group or a heteroaryl group, each of which is optionally substituted,

$Cy_{09}$ is

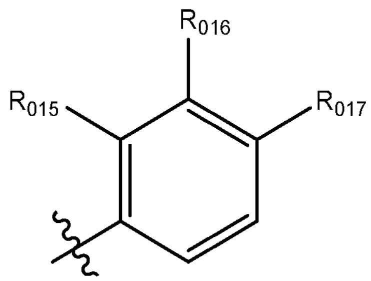

,

or $Cy_{09}$ is a heteroaryl group which is substituted by a group selected from -O-P(O)($OR_{020}$)$_2$; -O-P(O)($O^-M^+$)$_2$; -($CH_2$)$_{p0}$-O-($CHR_{018}$-$CHR_{019}$-0)$_{q0}$-$R_{020}$; hydroxy; hydroxy($C_1$-$C_6$)alkyl; -($CH_2$)$_{r0}$-$U_0$-($CH_2$)$_{s0}$-heterocycloalkyl; and -$U_0$-($CH_2$)$_{q0}$-$NR_{021}R_{021}$',

$R_{015}$ is a hydrogen atom; a -($CH_2$)$_{p0}$-O-($CHR_{018}$-$CHR_{019}$-O)$_{q0}$-$R_{020}$ group; a linear or branched ($C_1$-$C_6$)alkoxy($C_1$-$C_6$) alkyl group; a -$U_0$-($CH_2$)$_{q0}$-$NR_{021}R_{021}$' group; or a -($CH_2$)$_{r0}$-$U_0$-($CH_2$)$_{s0}$-heterocycloalkyl group,

$R_{016}$ is a hydrogen atom; a hydroxy group; a hydroxy($C_1$-$C_6$)alkyl group; a -($CH_2$)$_{r0}$-$U_0$-($CH_2$)$_{s0}$-heterocycloalkyl group; a ($CH_2$)$_{r0}$-$U_0$-$V_0$-O-P(O)($OR_{020}$)$_2$ group; a -O-P(O)($O^-M^+$)$_2$ group; a -O-S(O)$_2OR_{020}$ group; a -S(O)$_2OR_{020}$

group; a $-(CH_2)_{p0}-O-(CHR_{018}-CHR_{019}-O)_{q0}-R_{020}$ group; a $-(CH_2)_{p0}-O-C(O)-NR_{022}R_{023}$ group; or a $-U_0-(CH_2)_{q0}-NR_{021}R_{021}'$ group,

$R_{017}$ is a hydrogen atom; a $-(CH_2)_{p0}-O-(CHR_{018}-CHR_{019}-O)_{q0}-R_{020}$ group; a $-CH_2-P(O)(OR_{020})_2$ group, a $-O-P(O)(OR_{020})_2$ group; a $-O-P(O)(O^-M^+)_2$ group; a hydroxy group; a hydroxy$(C_1-C_6)$alkyl group; a $-(CH_2)_{r0}-U_0-(CH_2)_{s0}-$heterocycloalkyl group; a $-U_0-(CH_2)_{q0}-NR_{021}R_{021}'$ group; or an aldonic acid,

$M^+$ is a pharmaceutically acceptable monovalent cation,

$U_0$ is a bond or an oxygen atom,

$V_0$ is a $-(CH_2)_{s0}-$ group or a $-C(O)-$ group,

$R_{018}$ is a hydrogen atom or a $(C_1-C_6)$alkoxy$(C_1-C_6)$alkyl group,

$R_{019}$ is a hydrogen atom or a hydroxy$(C_1-C_6)$alkyl group,

$R_{020}$ is a hydrogen atom or a linear or branched $(C_1-C_6)$alkyl group,

$R_{021}$ and $R_{021}'$ independently of one are a hydrogen atom, a linear or branched $(C_1-C_6)$alkyl group, or a hydroxy$(C_1-C_6)$alkyl group,

or the pair $(R_{021}, R_{021}')$ together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the resulting ring is optionally substituted by a hydrogen atom or a linear or branched $(C_1-C_6)$alkyl group,

$R_{022}$ is a $(C_1-C_6)$alkoxy$(C_1-C_6)$alkyl group, a $-(CH_2)_{p0}-NR_{024}R_{024}'$ group, or a $-(CH_2)_{p0}-O-(CHR_{018}-CHR_{019}-O)_{q0}-R_{020}$ group,

$R_{023}$ is a hydrogen atom or a $(C_1-C_6)$alkoxy$(C_1-C_6)$alkyl group, or the pair $(R_{022}, R_{023})$ together with the nitrogen atom to which they are attached form an aromatic or non-aromatic ring containing 5 to 18 ring members, which optionally contains, in addition to the nitrogen atom, 1 to 5 heteroatoms selected from O, S and N, wherein the resulting ring is optionally substituted by a hydrogen atom, a linear or branched $(C_1-C_6)$alkyl group or a heterocycloalkyl group,

$R_{024}$ and $R_{024}'$ independently of one another are a hydrogen atom or a linear or branched $(C_1-C_6)$alkyl group,

or the pair $(R_{024}, R_{024}')$ together with the nitrogen atom to which they are attached form an aromatic or non-aromatic ring composed of from 5 to 7 ring members, which may contain in addition to the nitrogen atom from 1 to 3 heteroatoms selected from O, S and N, and wherein the resulting ring is optionally substituted by a hydrogen atom or a linear or branched $(C_1-C_6)$alkyl group,

$R_{025}$ is a hydrogen atom, a hydroxy group, or a hydroxy$(C_1-C_6)$alkyl group,

$R_{026}$ is a hydrogen atom, a halogen atom, a linear or branched $(C_1-C_6)$alkyl group, or a cyano group,

$R_{027}$ is a hydrogen atom or a linear or branched $(C_1-C_6)$alkyl group,

$R_{028}$ is a $-O-P(O)(O^-)(O^-)$ group, a $-O-P(O)(O^-)(OR_{030})$ group, a $-O-P(O)(OR_{030})(OR_{030}')$ group, a $-(CH_2)_{p0}-O-SO_2-O^-$ group, a $-(CH_2)_{p0}-SO_2-O^-$ group, a $-(CH_2)_{p0}-O-SO_2-OR_{030}$ group, $-Cy_{010}$, a $-(CH2)_{p0}-SO_2-OR_{030}$ group, a $-O-C(O)-R_{029}$ group, a $-O-C(O)-OR_{029}$ group or a $-O-C(O)-NR_{029}R_{029}'$ group;

$R_{029}$ and $R_{029}'$ independently of one another represent a hydrogen atom, a linear or branched $(C_1-C_6)$alkyl group or a linear or branched amino$(C_1-C_6)$alkyl group,

$R_{030}$ and $R_{030}'$ independently of one another are a hydrogen atom, a linear or branched $(C_1-C_6)$alkyl group or an aryl$(C_1-C_6)$alkyl group,

$R_{031}$ is

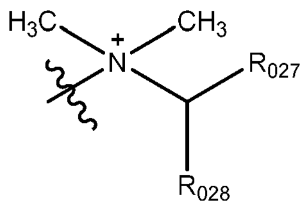
,
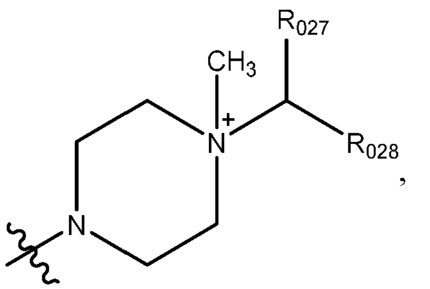
,

or

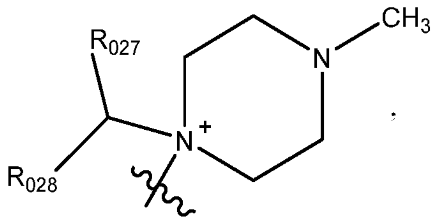

,

wherein the ammonium cation optionally exists as a zwitterionic form or has a monovalent anionic counterion,
$n_0$ is an integer equal to 0 or 1,
$p_0$ is an integer equal to 0, 1, 2, or 3,
$q_0$ is an integer equal to 1, 2, 3 or 4,
$r_0$ and $s_0$ are independently an integer equal to 0 or 1;
wherein, at most, one of the $R_{03}$, $R_{09}$, or $R_{012}$ groups, if present, is covalently attached to the linker, and wherein the valency of an atom is not exceeded by virtue of one or more substituents bonded thereto,
or an enantiomer, diastereoisomer, atropisomer, deuterated derivative, and/or
pharmaceutically acceptable salt of any of the foregoing.

In some embodiments, $Cy_{01}$, $Cy_{02}$, $Cy_{03}$, $Cy_{04}$, $Cy_{05}$, $Cy_{06}$, $Cy_{07}$, $Cy_{08}$ and $Cy_{010}$, independently of one another, is a cycloalkyl group, a heterocycloalkyl group, an aryl group or a heteroaryl group, each of which is optionally substituted by one or more groups selected from halo; $-(C_1-C_6)$alkoxy; $-(C_1-C_6)$haloalkyl; $-(C_1-C_6)$haloalkoxy; $-(CH_2)_{p0}-O-SO_2-OR_{030}$; $-(CH_2)_{p0}-SO_2-OR_{030}$; $-O-P(O)(OR_{020})_2$; $-O-P(O)(O^-M^+)_2$; $-CH_2-P(O)(OR_{020})_2$; $-(CH_2)_{p0}-O-(CHR_{018}-CHR_{019}-O)_{q0}-R_{020}$; hydroxy; hydroxy$(C_1-C_6)$alkyl; $-(CH_2)_{r0}-U_0-(CH_2)_{s0}$-heterocycloalkyl; or $-U_0-(CH_2)_{q0}-NR_{021}R_{021}'$.

In some embodiments, D comprises a compound of Formula (II):

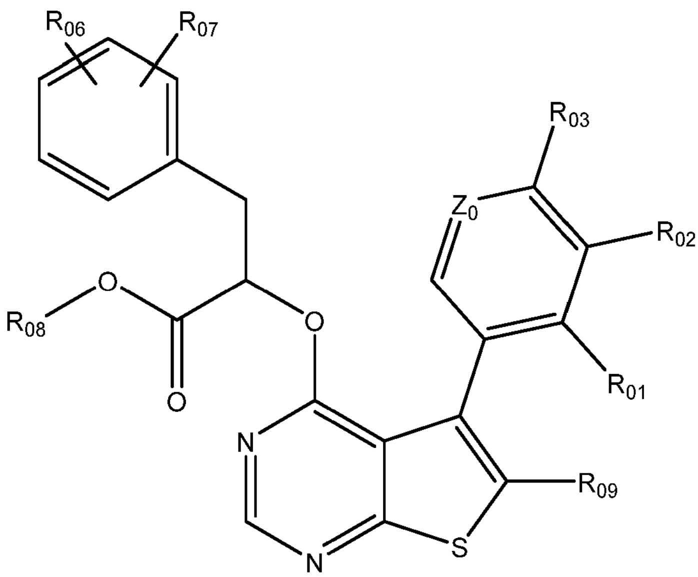

**(II)**

wherein:

$Z_0$ is a nitrogen atom or a $C-R_{04}$ group,
$R_{01}$ is a halogen atom, a linear or branched $(C_1-C_6)$alkyl group, a linear or branched $(C_2-C_6)$alkenyl group, a linear or branched $(C_2-C_6)$alkynyl group, a linear or branched $(C_1-C_6)$haloalkyl group, a hydroxy group, a linear or branched $(C_1-C_6)$alkoxy group, a $-S-(C_1-C_6)$alkyl group, a cyano group, $-Cy_{08}$, $-NR_{011}R_{011}'$,
$R_{02}$, $R_{03}$ and $R_{04}$ independently of one another are a hydrogen atom, a halogen atom, a linear or branched $(C_1-C_6)$ alkyl group, a linear or branched $(C_2-C_6)$alkenyl group, a linear or branched $(C_2-C_6)$alkynyl group, a linear or branched $(C_1-C_6)$haloalkyl, a hydroxy group, a linear or branched $(C_1-C_6)$alkoxy group, a $-S-(C_1-C_6)$alkyl group, a cyano group, a nitro group, $-(C_0-C_6)$alkyl-$NR_{011}R_{011}'$, $-O-Cy_{01}$, $-(C_0-C_6)$alkyl-$Cy_{01}$,

$-(C_2-C_6)$alkenyl-$Cy_{01}$, $-(C_2-C_6)$alkynyl-$Cy_{01}$, $-O-(C_1-C_6)$alkyl-$NR_{011}R_{011}'$, $-O-(C_1-C_6)$alkyl-$R_{031}$, $-C(O)-OR_{011}$,

-O-C(O)-$R_{011}$, -C(O)-$NR_{011}R_{011}$', -$NR_{011}$-C(O)-$R_{011}$', -$NR_{011}$-C(O)-$OR_{011}$', -($C_1$-$C_6$)alkyl-$NR_{011}$-C(O)-$R_{011}$', -$SO_2$-$NR_{011}R_{011}$', or -$SO_2$-($C_1$-$C_6$)alkyl,
or the pair ($R_{02}$, $R_{03}$) or ($R_{03}$, $R_{04}$) together with the carbon atoms to which they are attached form an aromatic or non-aromatic ring containing 5 to 7 ring members, which optionally contains 1 to 3 heteroatoms selected from O, S and N, wherein the ring is optionally substituted by a group selected from a linear or branched ($C_1$-$C_6$)alkyl, -$NR_{013}R_{013}$', -($C_0$-$C_6$)alkyl-$Cy_{01}$ and oxo,

$R_{06}$ and $R_{07}$ independently of one another are a hydrogen atom, a halogen atom, a linear or branched ($C_1$-$C_6$)alkyl group, a linear or branched ($C_2$-$C_6$)alkenyl group, a linear or branched ($C_2$-$C_6$)alkynyl group, a linear or branched ($C_1$-$C_6$)haloalkyl, a hydroxy group, a linear or branched ($C_1$-$C_6$)alkoxy group, a -S-($C_1$-$C_6$)alkyl group, a cyano group, a nitro group, -($C_0$-$C_6$)alkyl-$NR_{011}R_{011}$', -O-$Cy_{01}$, -($C_0$-$C_6$)alkyl-$Cy_{01}$, -($C_2$-$C_6$)alkenyl-$Cy_{01}$,

-($C_2$-$C_6$)alkynyl-$Cy_{01}$, -O-($C_1$-$C_6$)alkyl-$R_{012}$, -C(O)-$OR_{011}$, -O-C(O)-$R_{011}$, -C(O)-$NR_{011}R_{011}$', -$NR_{011}$-C(O)-$R_{011}$', -$NR_{011}$-C(O)-$OR_{011}$', -($C_1$-$C_6$)alkyl-$NR_{011}$-C(O)-$R_{011}$', -$SO_2$-$NR_{011}R_{011}$', or -$SO_2$-($C_1$-$C_6$) alkyl,
or the pair ($R_{06}$, $R_{07}$), when fused with two adjacent carbon atoms, together with the carbon atoms to which they are attached form an aromatic or non-aromatic ring containing 5 to 7 ring members, which optionally contains 1 to 3 heteroatoms selected from O, S and N, and wherein the resulting ring is optionally substituted by a group selected from a linear or branched ($C_1$-$C_6$)alkyl group, -$NR_{013}R_{013}$', -($C_0$-$C_6$)alkyl-$Cy_{01}$, and an oxo,

$R_{08}$ is a hydrogen atom, a linear or branched ($C_1$-$C_8$)alkyl group, an aryl group, a heteroaryl group, an aryl-($C_1$-$C_6$) alkylgroup, or a heteroaryl($C_1$-$C_6$)alkyl group,
$R_{09}$ is a linear or branched ($C_1$-$C_6$)alkyl group, a linear or branched ($C_2$-$C_6$)alkenyl group, a linear or branched ($C_2$-$C_6$) alkynyl group, -$Cy_{02}$, -($C_1$-$C_6$)alkyl-$Cy_{02}$, -($C_2$-$C_6$)alkenyl-$Cy_{02}$, -($C_2$-$C_6$)alkynyl-$Cy_{02}$, -$Cy_{02}$-$Cy_{03}$, -($C_2$-$C_6$)alkynyl-O-$Cy_{02}$, -$Cy_{02}$-($C_0$-$C_6$)alkyl-O-($C_0$-$C_6$)alkyl-$Cy_{03}$, a halogen atom, a cyano group, -C(O)-$R_{014}$, -C(O)-$NR_{014}R_{014}$',
$R_{011}$ and $R_{011}$' independently of one another are a hydrogen atom, an optionally substituted linear or branched ($C_1$-$C_6$) alkyl group, or -($C_0$-$C_6$)alkyl-$Cy_{01}$, or the pair ($R_{011}$, $R_{011}$') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom is optionally substituted by a linear or branched ($C_1$-$C_6$)alkyl group, and wherein one or more of the carbon atoms of the linear or branched ($C_1$-$C_6$)alkyl group is optionally deuterated,
$R_{012}$ represents -$Cy_{05}$, -$Cy_{05}$-($C_0$-$C_6$)alkyl-$Cy_{06}$, -$Cy_{05}$-($C_0$-$C_6$)alkyl-O-($C_0$-$C_6$)alkyl-$Cy_{06}$, -$Cy_{05}$-($C_0$-$C_6$)alkyl-$NR_{011}$-($C_0$-$C_6$)alkyl-$Cy_{06}$, -$Cy_{05}$-$Cy_{06}$-O-($C_0$-$C_6$)alkyl-$Cy_{07}$, -$Cy_{05}$-($C_0$-$C_6$)alkyl-$Cy_{09}$, -NH-C(O)-NH-$R_{011}$, -C(O)-$NR_{011}R_{011}$', -$NR_{011}R_{011}$', -$OR_{011}$, -$NR_{011}$C(O)-$R_{011}$', -O-($C_1$-$C_6$)alkyl-$OR_{011}$, -$SO_2$-$R_{011}$, or -C(O)-$OR_{011}$,
$R_{013}$, $R_{013}$', $R_{014}$ and $R_{014}$' independently of one another are a hydrogen atom, or an optionally substituted linear or branched ($C_1$-$C_6$)alkyl group,
$Cy_{01}$, $Cy_{02}$, $Cy_{03}$, $Cy_{05}$, $Cy_{06}$, $Cy_{07}$ and $Cy_{08}$ independently of one another, are an optionally substituted cycloalkyl group, an optionally substituted heterocycloalkyl group, an optionally substituted aryl group or an optionally substituted heteroaryl group,
$Cy_{09}$ is

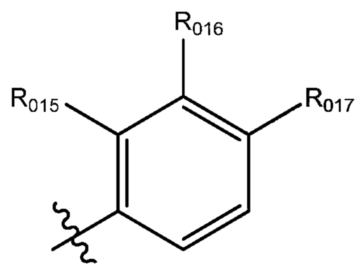
,

wherein $R_{015}$, $R_{016}$, and $R_{017}$ are as defined for formula (I),
$R_{031}$ is

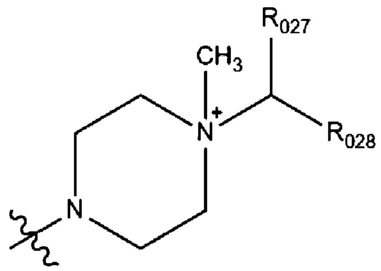

,

wherein $R_{027}$ and $R_{028}$ are as defined for formula (I)
wherei n, at most, one of the $R_{03}$, $R_{09}$, or $R_{012}$ groups, if present, is covalently attached to the linker,
or an enantiomer, diastereoisomer, atropisomer, deuterated derivative, and/or pharmaceutically acceptable salt of any of the foregoing.

In some embodiments, D comprises a compound of Formula (III): wherein:

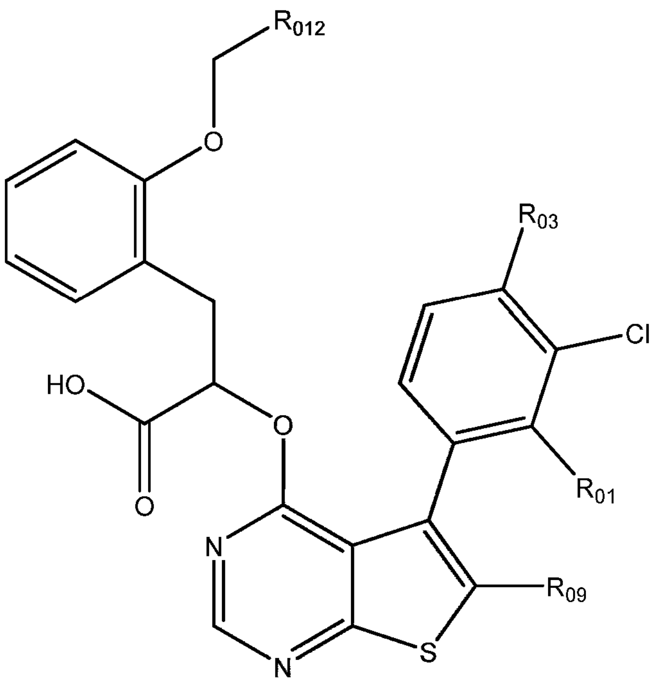

**(III)**

$R_{01}$ is a linear or branched $(C_1\text{-}C_6)$alkyl group,
$R_{03}$ is $\text{-O-}(C_1\text{-}C_6)\text{alkyl-NR}_{011}R_{011}'$,
or

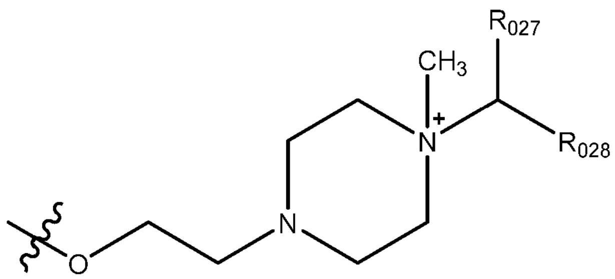

,

wherein $R_{011}$ and $R_{011}'$ independently of one another are a hydrogen atom, an optionally substituted linear or branched $(C_1\text{-}C_6)$alkyl group, or $\text{-}(C_0\text{-}C_6)\text{alkyl-Cy}_{01}$;
or the pair ($R_{011}$, $R_{011}'$) together with the nitrogen atom to which they are attached form an aromatic or non-aromatic ring containing 5 to 7 ring members, which optionally contains, in addition to the nitrogen atom, 1 to 3 heteroatoms

selected from O, S and N, wherein the N atom may be substituted by 1 or 2 groups selected from a hydrogen atom or a linear or branched ($C_1$-$C_6$)alkyl group,
and wherein $R_{027}$ is a hydrogen atom and $R_{028}$ is a -$(CH_2)_{p0}$-O-$SO_2$-$O^-$ group or a -$(CH_2)_{p0}$-$SO_2$-$OR_{030}$ group;
$R_{09}$ is a linear or branched ($C_2$-$C_6$)alkynyl group or -$Cy_{02}$,
$R_{012}$ is -$Cy_{05}$, -$Cy_{05}$-($C_0$-$C_6$)alkyl-$Cy_{06}$, or -$Cy_{05}$-($C_0$-$C_6$)alkyl-$Cy_{09}$,
$Cy_{01}$, $Cy_{02}$, $Cy_{05}$ and $Cy_{06}$ independently of one another, are a cycloalkyl group, a heterocycloalkyl group, an aryl group or a heteroaryl group, each of which is optionally substituted,
$Cy_{09}$ is

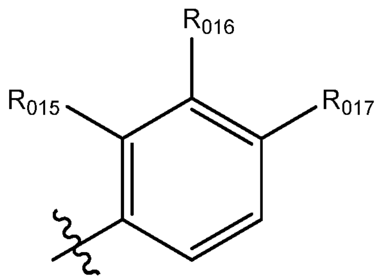

,

$R_{015}$, $R_{016}$, and $R_{017}$ are as defined for formula (I),
wherein, at most, one of the $R_{03}$, $R_{09}$, or $R_{012}$ groups, if present, is covalently attached to the linker,
or the enantiomer, diastereoisomer, atropisomer, deuterated derivative, and/or pharmaceutically acceptable salt of any of the foregoing.

In some embodiments, $Cy_{01}$, $Cy_{02}$, $Cy_{05}$, $Cy_{06}$, independently of one another, is a cycloalkyl group, a heterocycloalkyl group, an aryl group or a heteroaryl group, each of which is optionally substituted by one or more groups selected from halo; -($C_1$-$C_6$)alkoxy;

-($C_1$-$C_6$)haloalkyl; -($C_1$-$C_6$)haloalkoxy; -$(CH_2)_{p0}$-O-$SO_2$-$OR_{030}$; -$(CH_2)_{p0}$-$SO_2$-$OR_{030}$;
-O-P(O)$(OR_{020})_2$; -O-P(O)$(O^-M^+)_2$; -$CH_2$-P(O)$(OR_{020})_2$;
-$(CH_2)_{p0}$-O-$(CHR_{018}$-$CHR_{019}$-$O)_{q0}$-$R_{020}$; hydroxy; hydroxy($C_1$-$C_6$)alkyl;
-$(CH_2)_{r0}$-$U_0$-$(CH_2)_{s0}$-heterocycloalkyl; or -$U_0$-$(CH_2)_{q0}$-$NR_{021}R_{021}$'.

In some embodiments, $R_{01}$ is methyl or ethyl.
In some embodiments, $R_{03}$ is -O-$CH_2$-$CH_2$-$NR_{011}R_{011}$' in which $R_{011}$ and $R_{011}$' form, together with the nitrogen atom carrying them, a piperazinyl group which may be substituted by a substituted by a hydrogen atom or a linear or branched ($C_1$-$C_6$)alkyl group.
In some embodiments, $R_{03}$ comprises the formula:

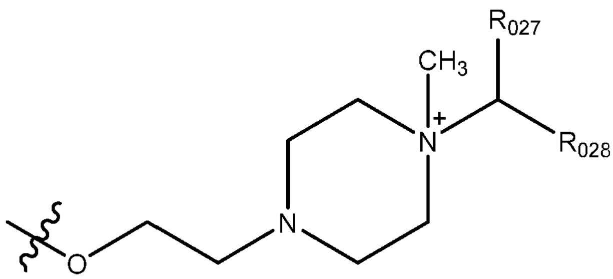

,

wherein $R_{027}$ is a hydrogen atom and $R_{028}$ is a -$(CH_2)_{p0}$-O-$SO_2$-$OR_{030}$ group, $p_0$ is an integer equal to 0, 1, 2, or 3; and wherein $R_{030}$ represents a hydrogen atom, a linear or branched ($C_1$-$C_6$)alkyl group or an aryl($C_1$-$C_6$)alkyl group.
In some embodiments, $R_{03}$ comprises the formula:

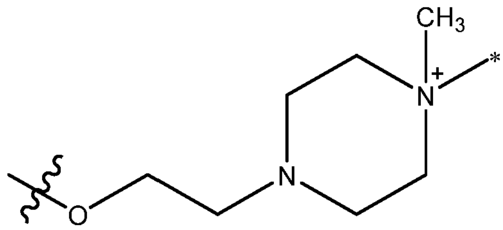

,

wherein -* is a bond to the linker.

In some embodiments, $Cy_{01}$, $Cy_{02}$, $Cy_{03}$, $Cy_{04}$, $Cy_{05}$, $Cy_{06}$, $Cy_{07}$, $Cy_{08}$ and $Cy_{010}$ independently of one another, are an optionally substituted cycloalkyl group, an optionally substituted heterocycloalkyl group, an optionally substituted aryl group or an optionally substituted heteroaryl group, wherein the optional substituents are selected from optionally substituted linear or branched $(C_1-C_6)$alkyl, optionally substituted linear or branched $(C_2-C_6)$alkenyl group, optionally substituted linear or branched $(C_2-C_6)$alkynyl group, optionally substituted linear or branched $(C_1-C_6)$alkoxy, optionally substituted $(C_1-C_6)$alkyl-S-, hydroxy, oxo (or N-oxide where appropriate), nitro, cyano, -C(O)-$OR_0$', -O-C(O)-$R_0$', -C(O)-$NR_0$'$R_0$", -$NR_0$'$R_0$", -(C=$NR_0$')-$OR_0$", linear or branched $(C_1-C_6)$ haloalkyl, trifluoromethoxy, or halogen, wherein $R_0$' and $R_0$" are each independently a hydrogen atom or an optionally substituted linear or branched $(C_1-C_6)$alkyl group, and wherein one or more of the carbon atoms of linear or branched $(C_1-C_6)$alkyl group is optionally deuterated.

In some embodiments, $R_{09}$ is a $Cy_{02}$ group, preferably an aryl group, more preferably a phenyl group. In some embodiments, $Cy_{02}$ is an optionally substituted aryl group.

In some embodiments, $Cy_{05}$ comprises a heteroaryl group selected from a pyrazolyl group and a pyrimidinyl group.

In some embodiments, $Cy_{05}$ is a pyrimidinyl group.

In some embodiments, $Cy_{05}$ is a pyrimidinyl group and $Cy_{06}$ is phenyl group.

In some embodiments, the linker (L) is attached to D by a covalent bond from L to $R_{03}$ of formulas (I), (II), or (III). In some embodiments, the linker (L) is attached to D by a covalent bond from L to $R_{09}$ of formulas (I), (II), or (III).

In some embodiments, D comprises:

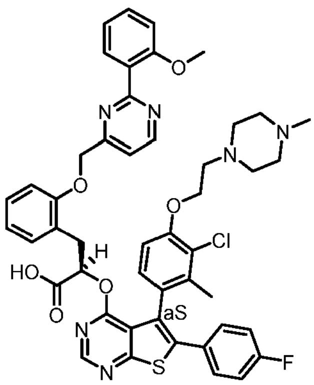

,

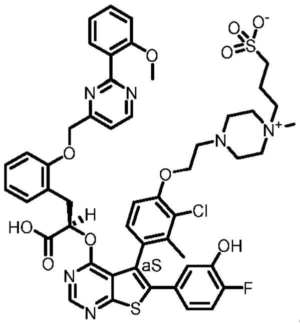

,

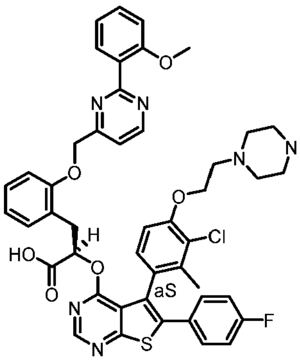

,

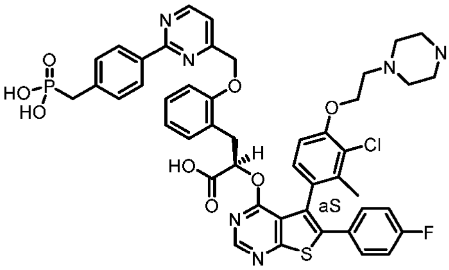

,

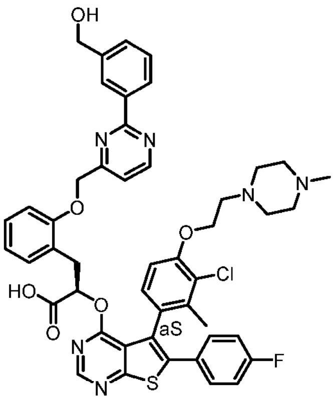

,

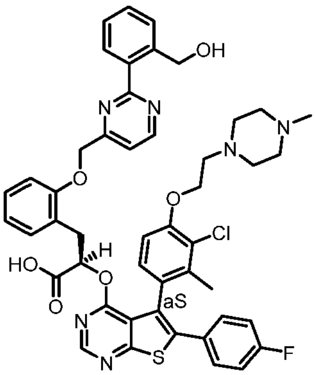

,

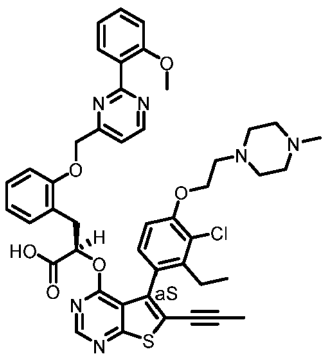 , 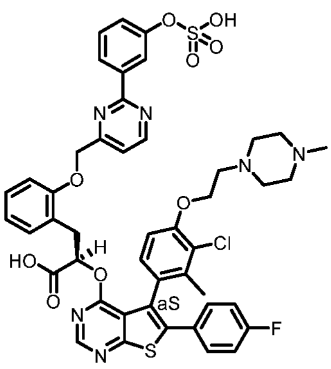 , 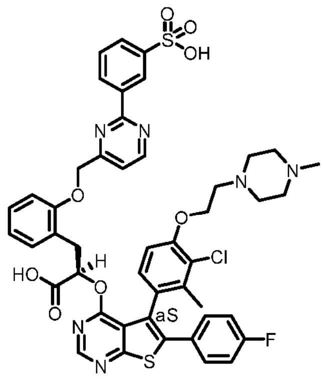 ,

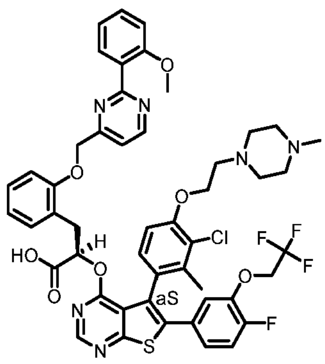 , 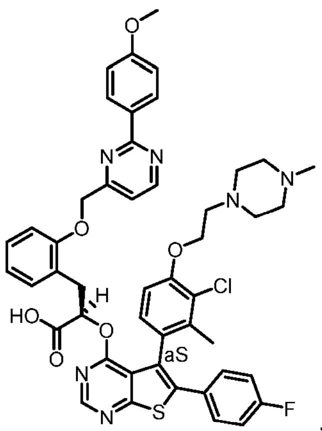 , 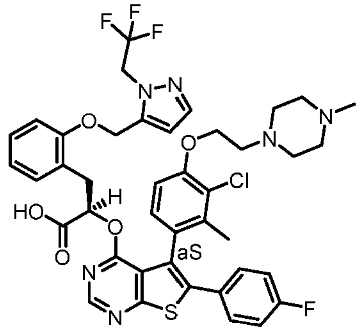 ,

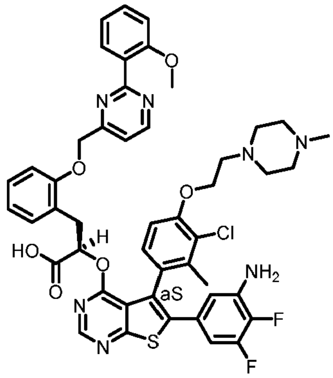 , or 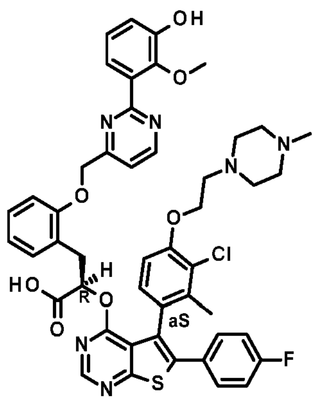 ,

or

an enantiomer, diastereoisomer, atropisomer, deuterated derivative, and/or a pharmaceutically acceptable salt of any of the foregoing.

In some embodiments, -(L-D) is formed from a compound selected from Table A or an enantiomer, diastereoisomer, atropisomer, deuterated derivative, and/or pharmaceutically acceptable salt thereof. For compounds in Table A, depending on their electronic charge, these compounds can contain one pharmaceutically acceptable monovalent anionic counterion $M_1^-$. In some embodiments, the monovalent anionic counterion $M_1^-$ can be selected from bromide, chloride, iodide, acetate, trifluoroacetate, benzoate, mesylate, tosylate, triflate, formate, or the like. In some embodiments, the monovalent anionic counterion $M_1^-$ is trifluoroacetate or formate.

**Table A. Exemplary Linker Drug Groups**

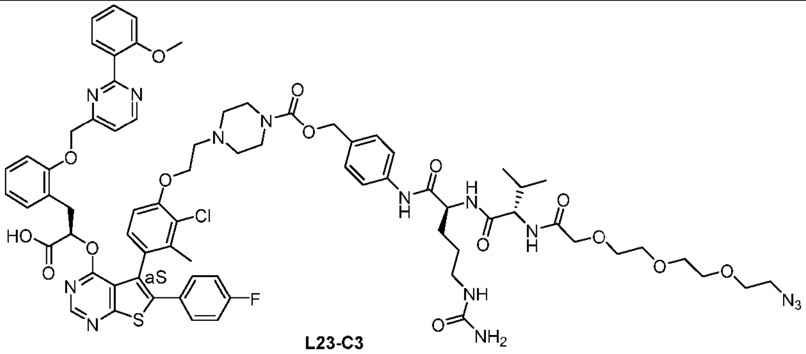

L23-C3

,

| |
|---|
| 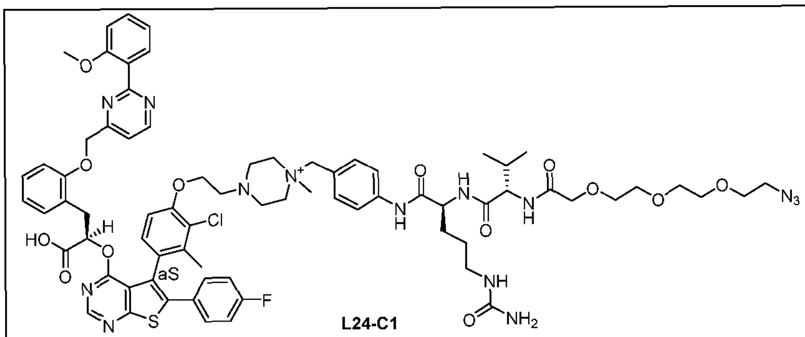 L24-C1 , |
| 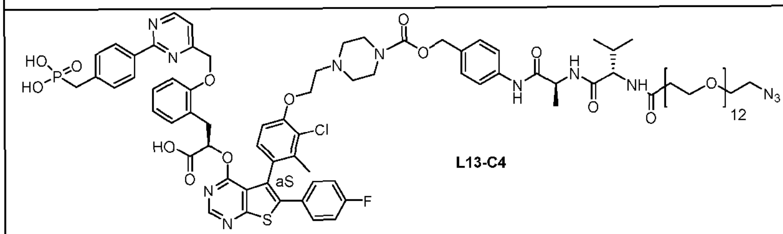 L13-C4 , |
| 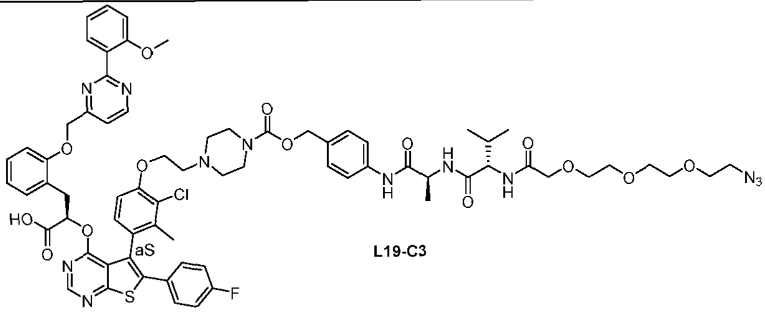 L19-C3 , |
| 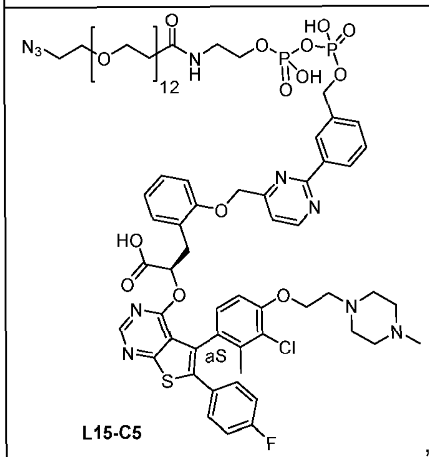 L15-C5 , |
| 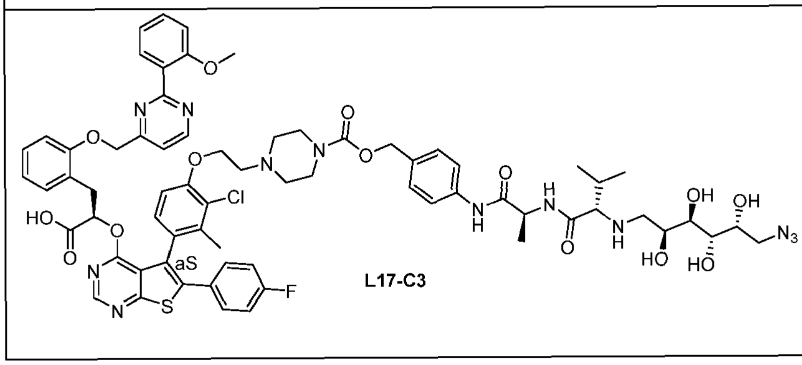 L17-C3 , |

| |
|---|
| 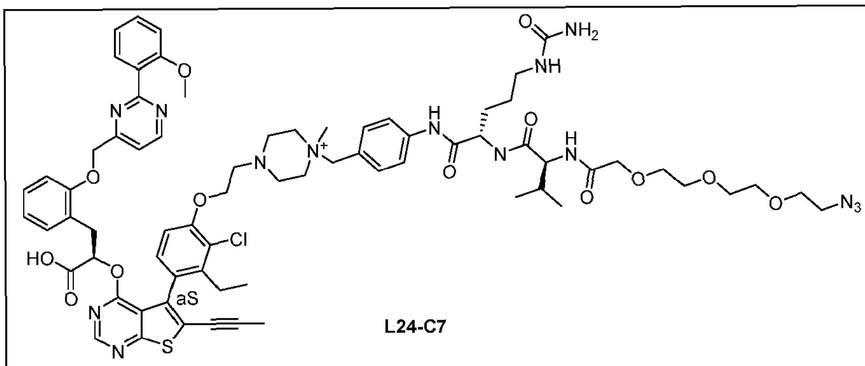 L24-C7 , |
| 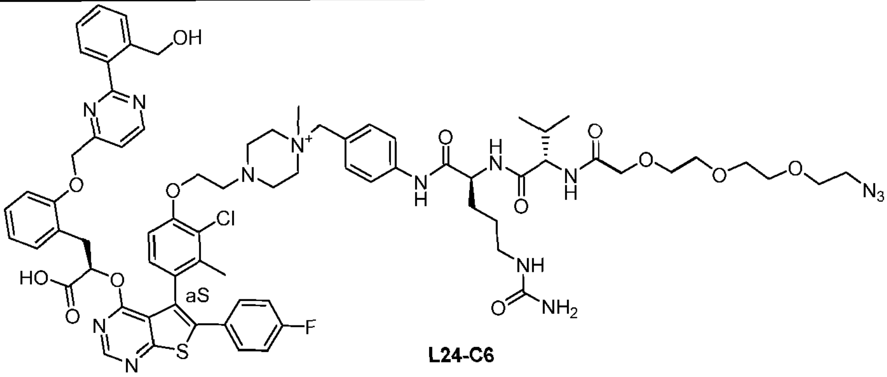 L24-C6 , |
| 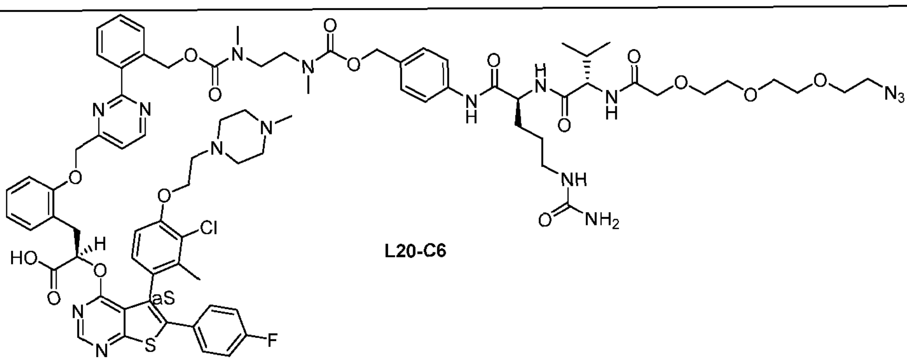 L20-C6 , |
| 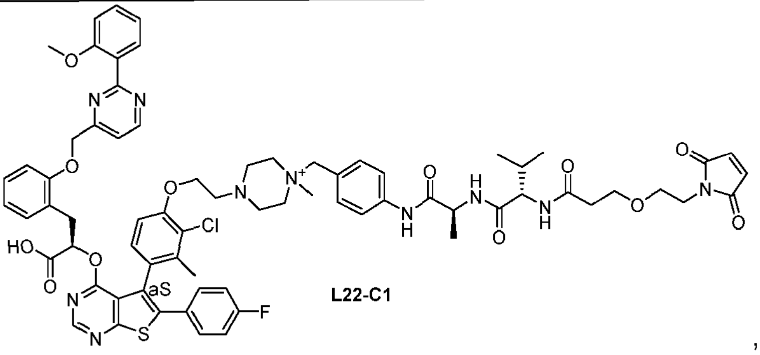 L22-C1 , |

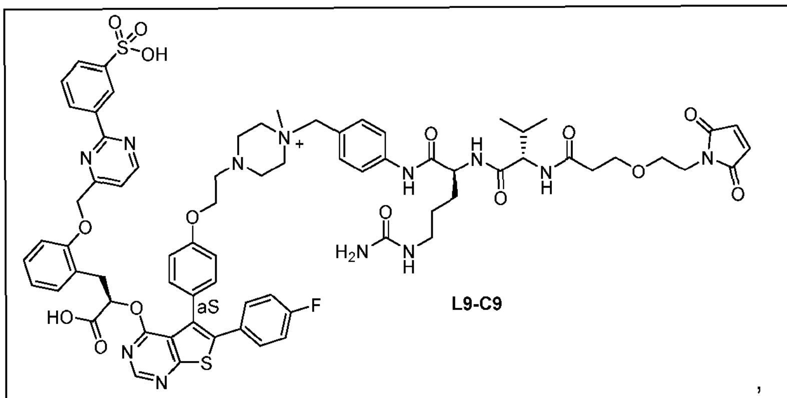

L9-C9

,

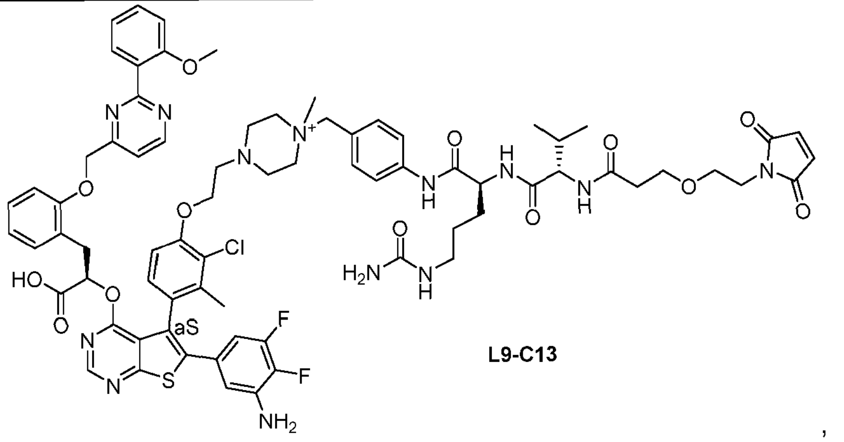

L9-C13

,

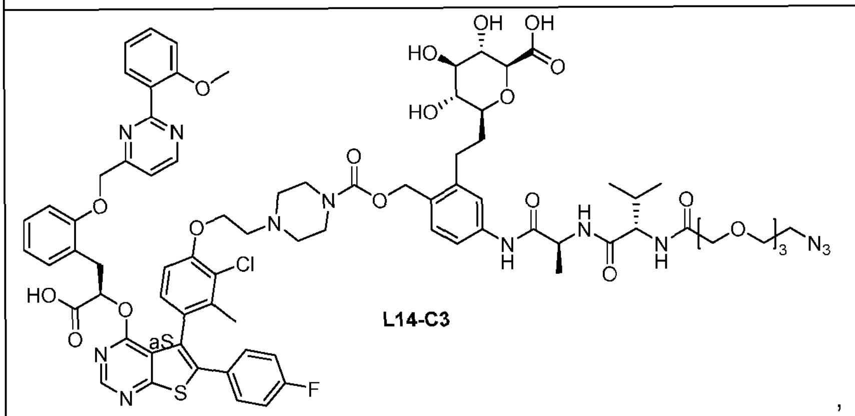

L14-C3

,

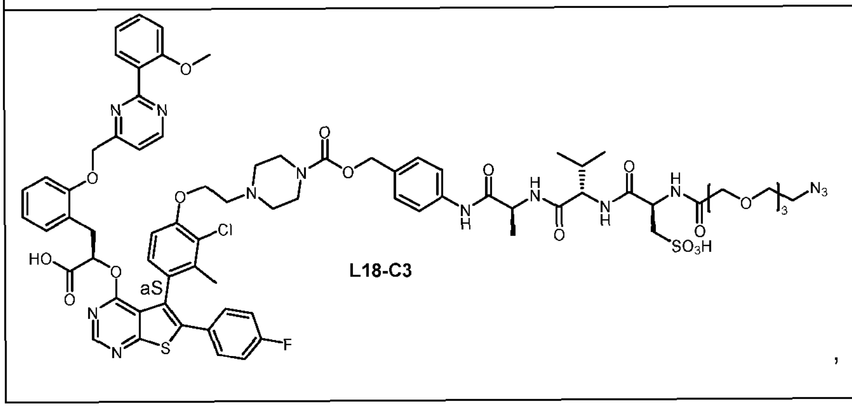

L18-C3

,

| |
|---|
| 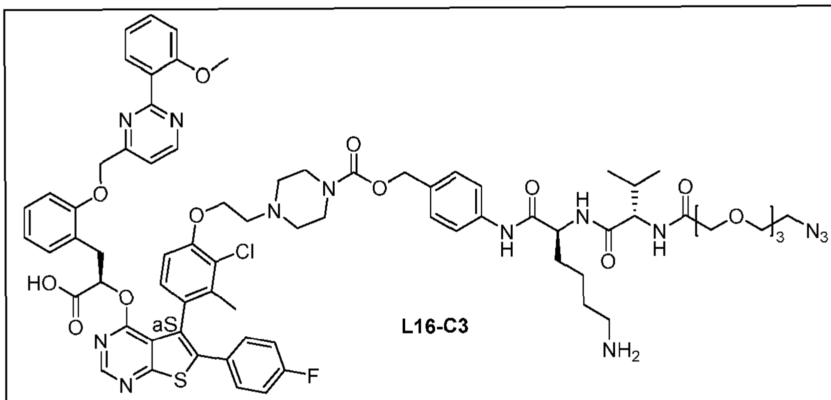 L16-C3 , |
| 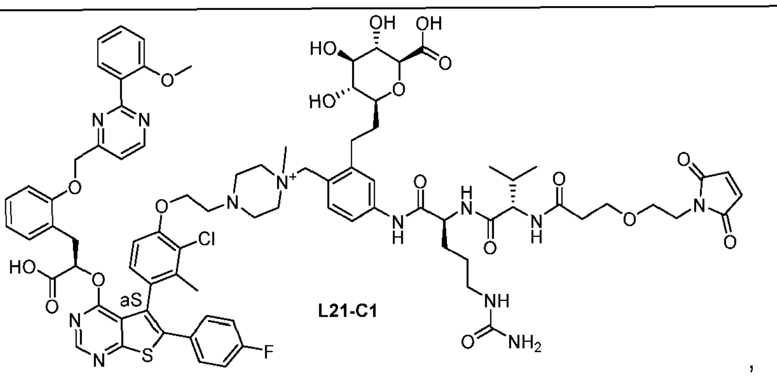 L21-C1 , |
| 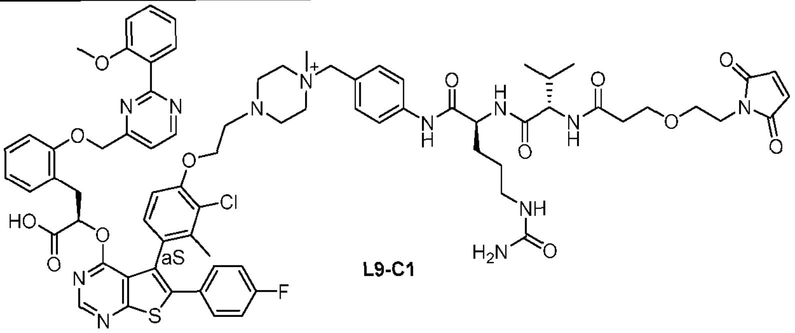 L9-C1 , |
| 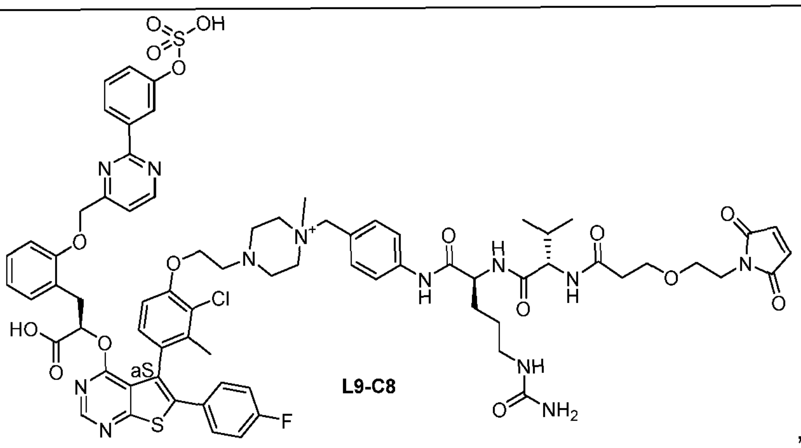 L9-C8 , |

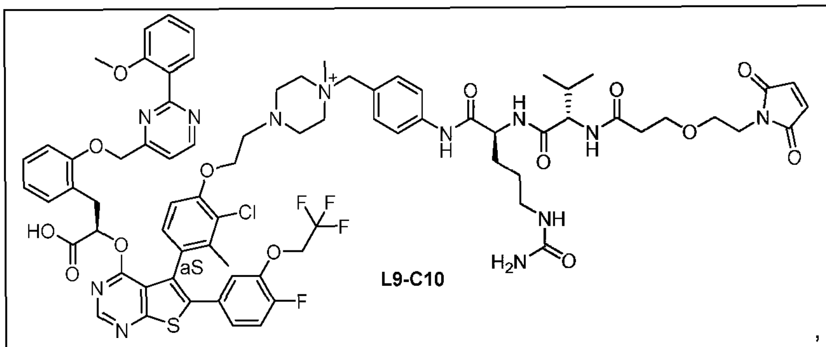

L9-C10 ,

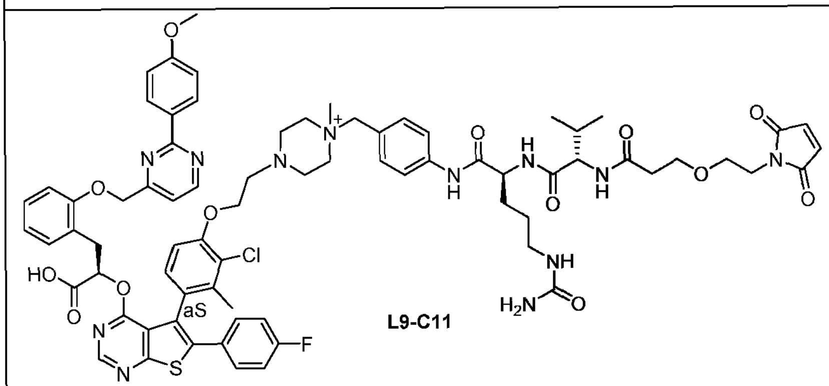

L9-C11 ,

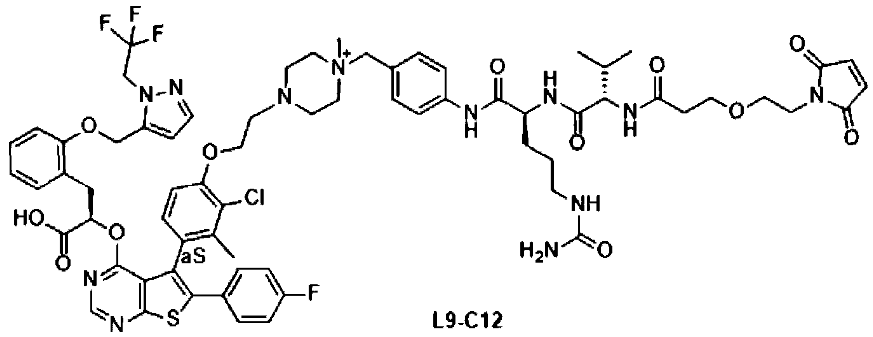

L9-C12 ,

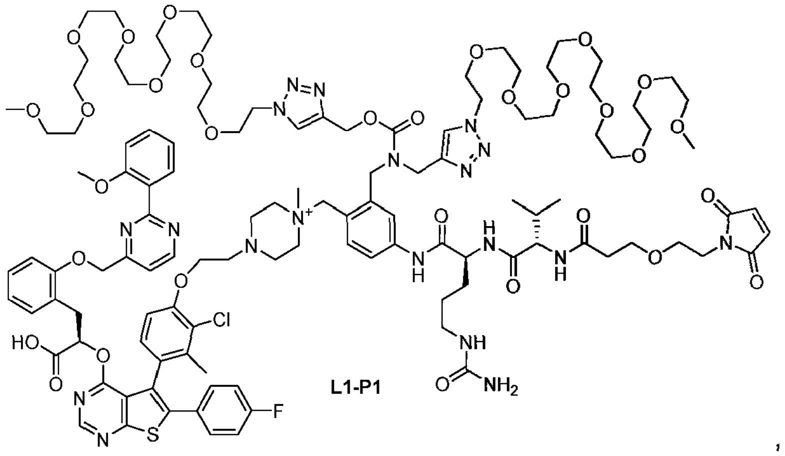

L1-P1 ,

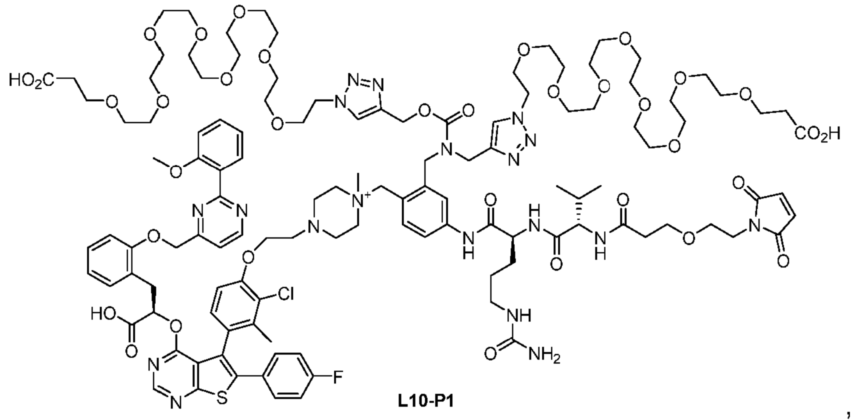

L10-P1

,

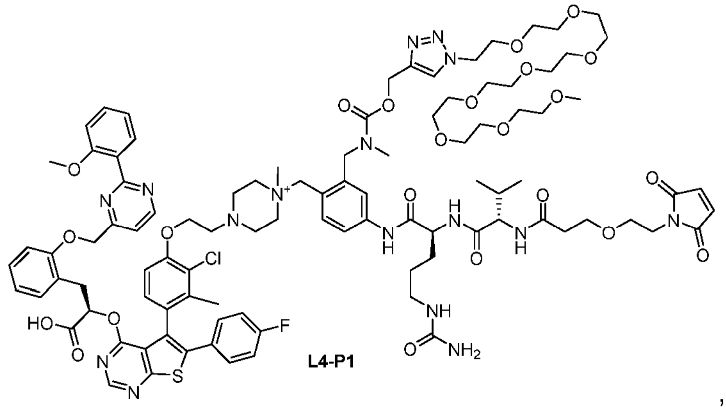

L4-P1

,

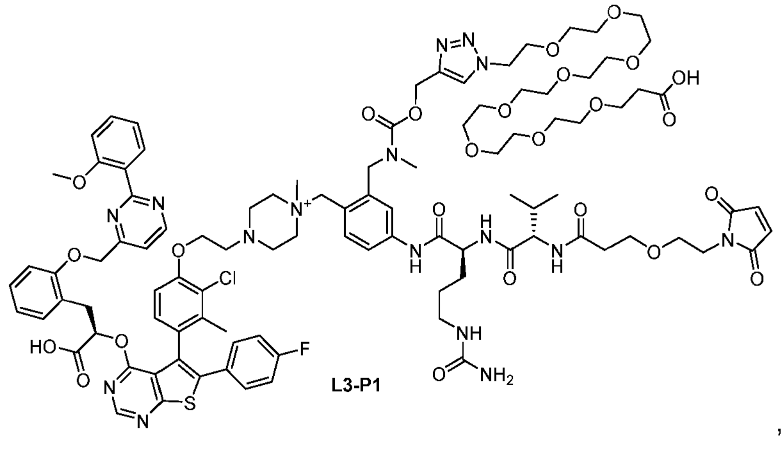

L3-P1

,

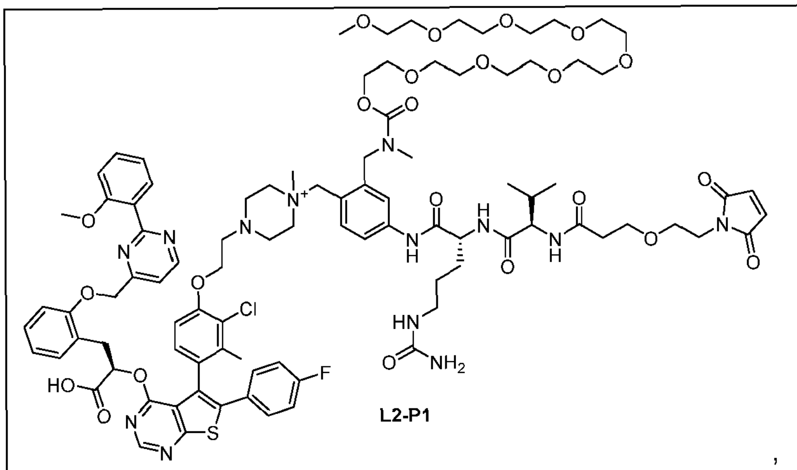

L2-P1

,

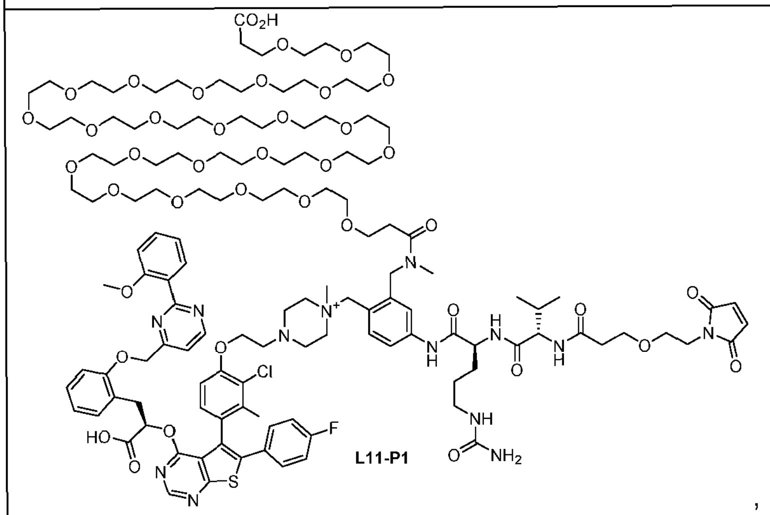

L11-P1

,

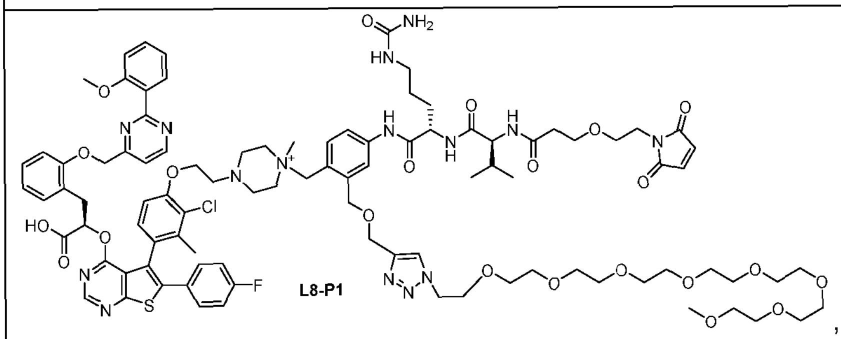

L8-P1

,

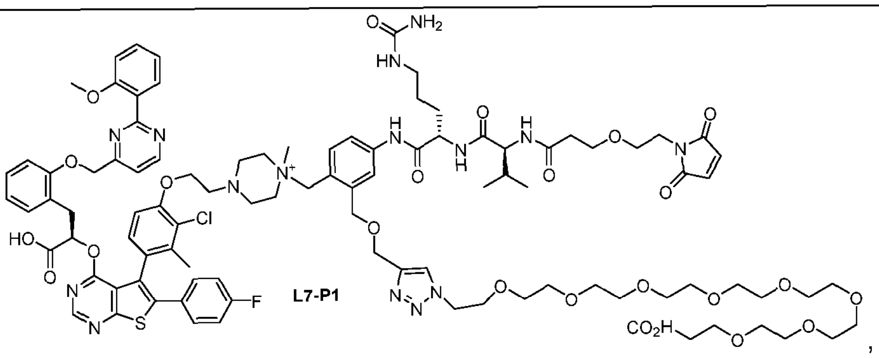

L7-P1

,

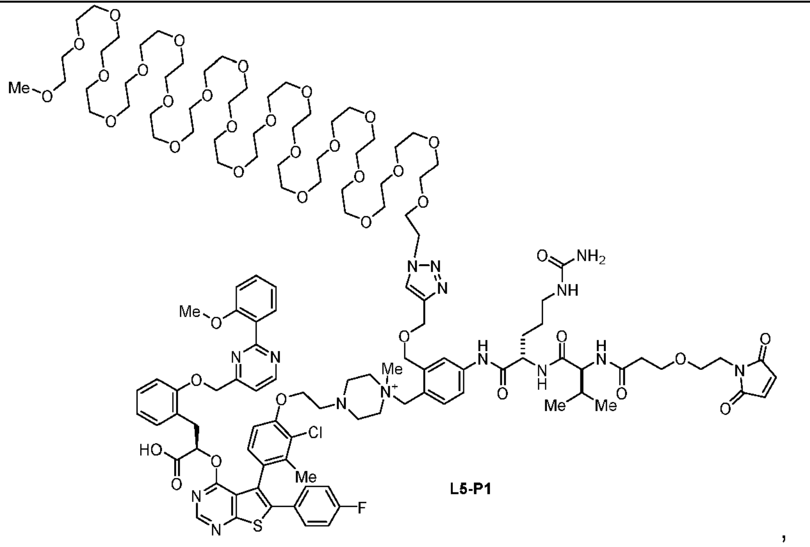

L5-P1

,

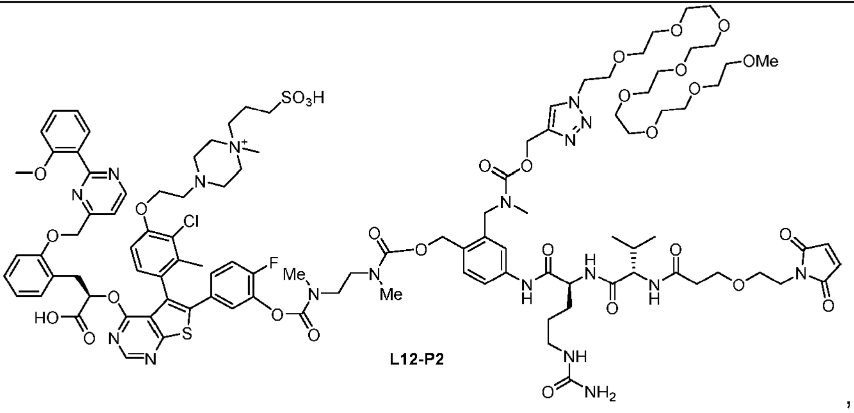

L12-P2

,

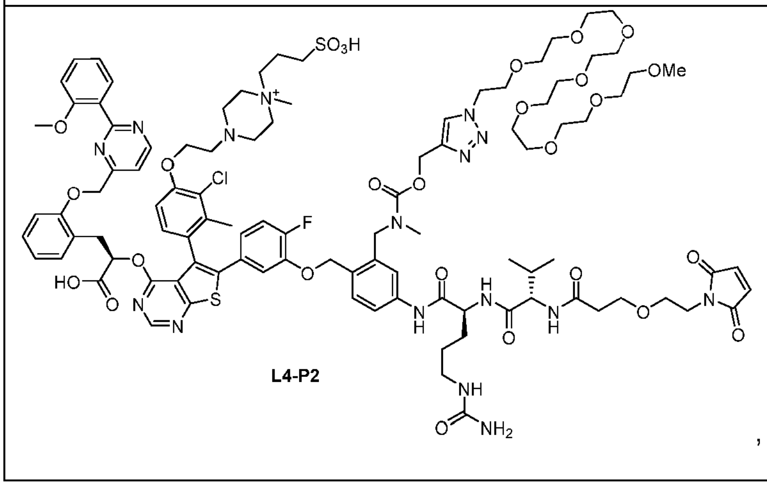

L4-P2

,

| |
|---|
| 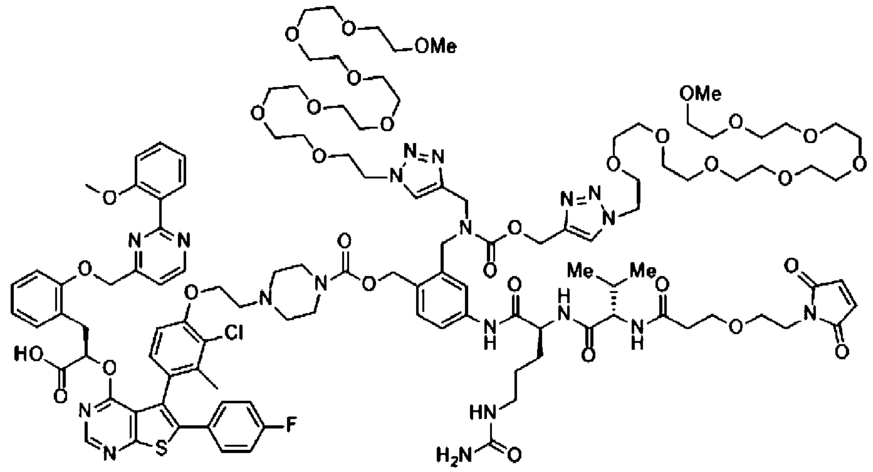 **L1-P3**, |
| 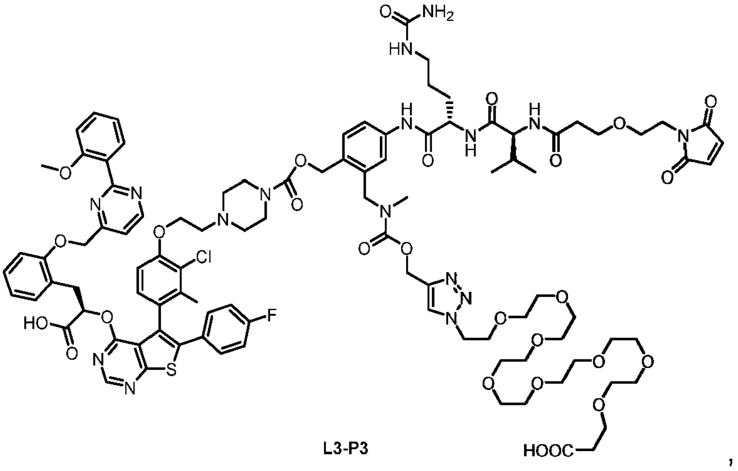 L3-P3 , |
| 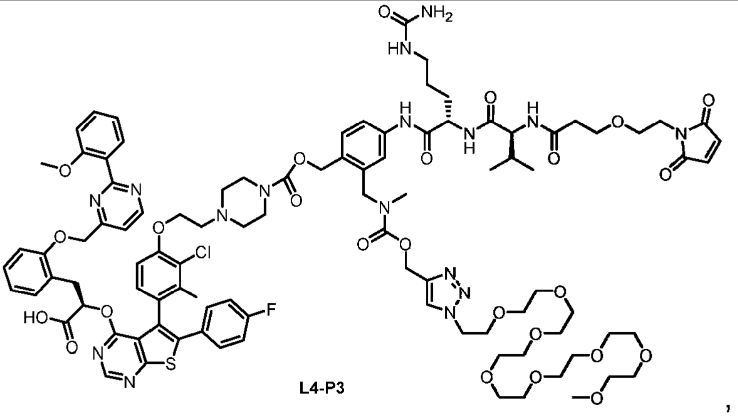 L4-P3 , |
| 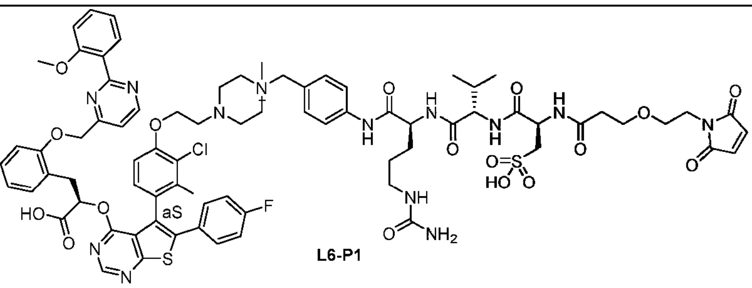 L6-P1 , |

| |
|---|
| 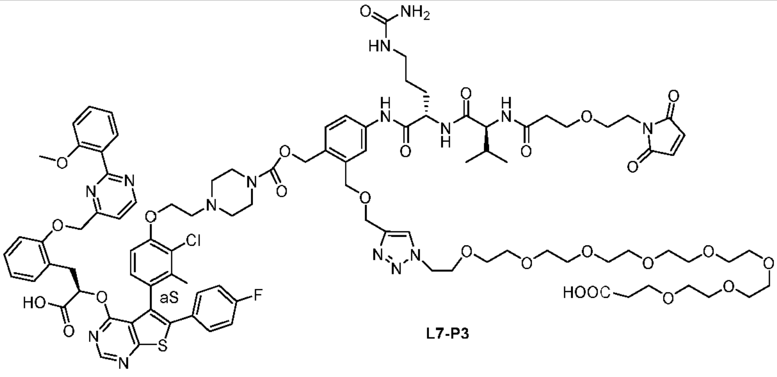 L7-P3 , |
| 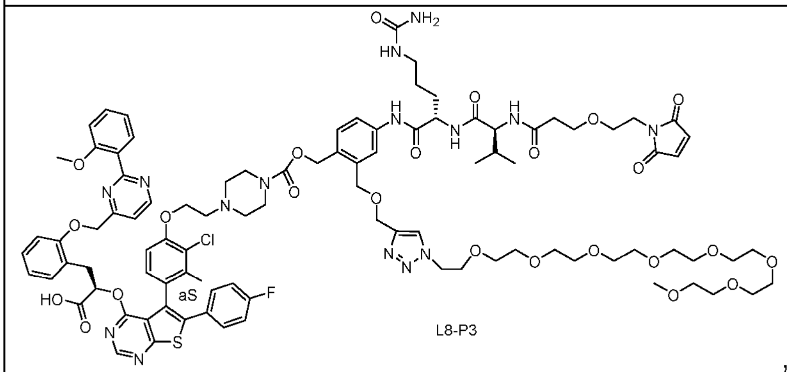 L8-P3 , |
| 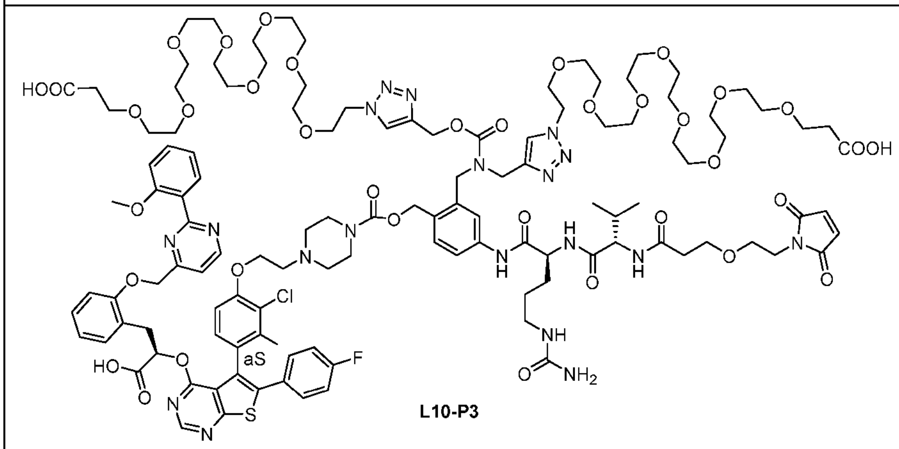 L10-P3 , |
| 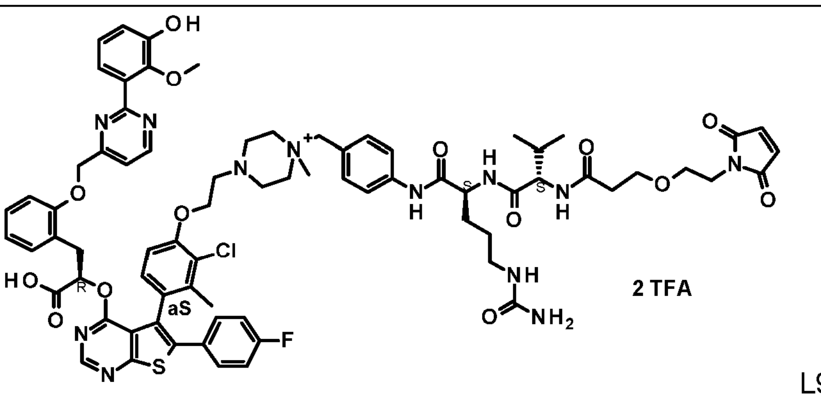 2 TFA L9-C14, |

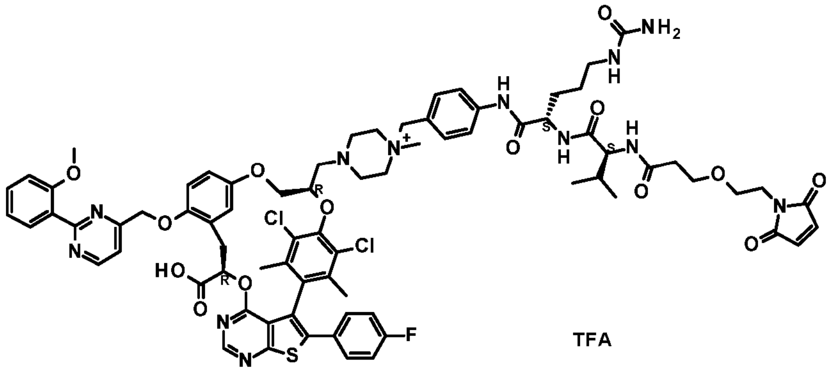

TFA

L9-P15,

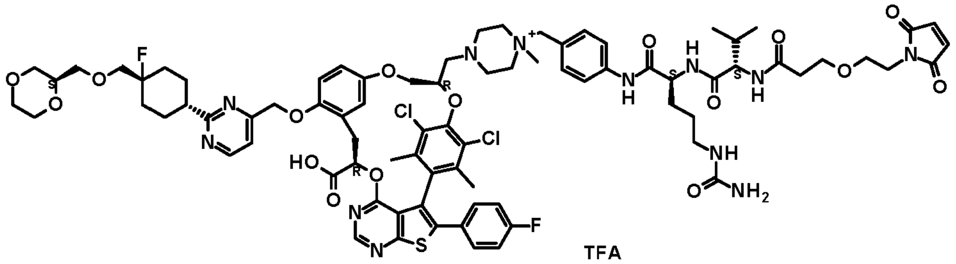

TFA

L9-P16,

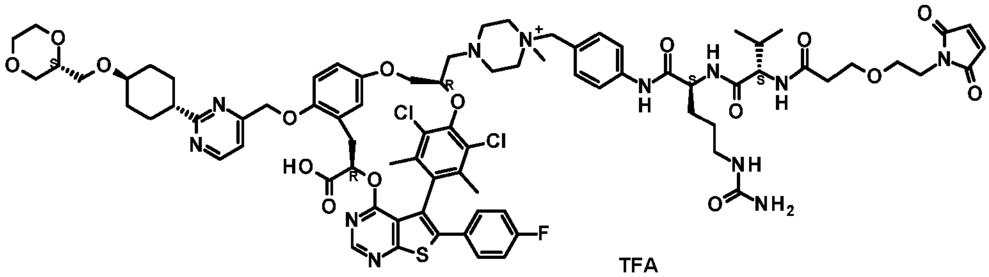

TFA

L9-P17,

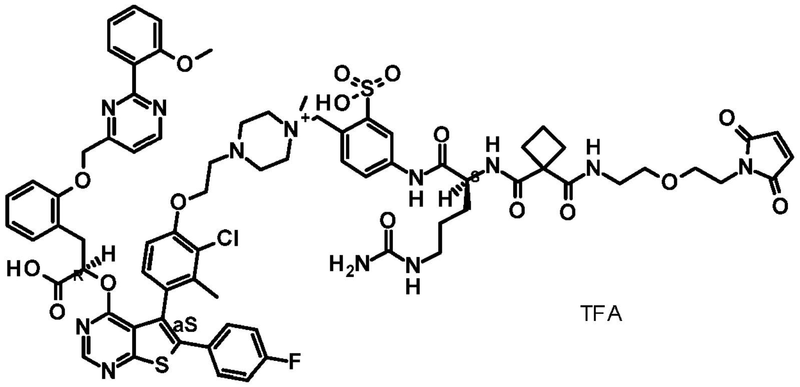

TFA

L25-P1.

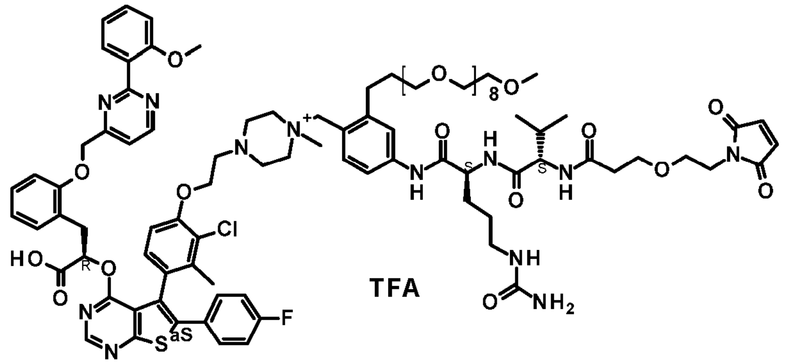

L26-P1,

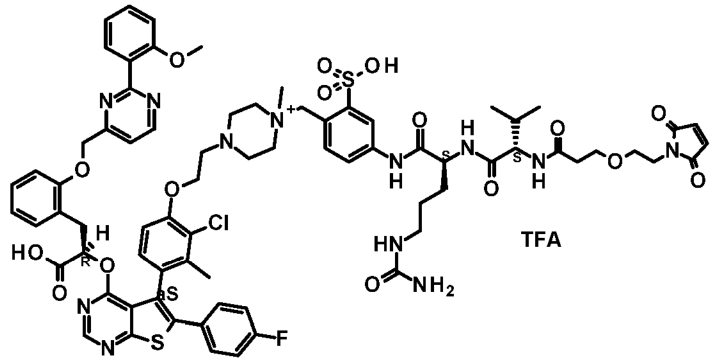

L27-P1,

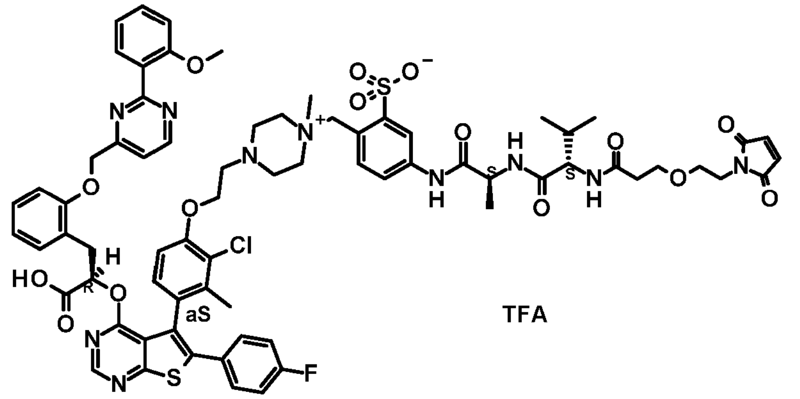

L28-P1,

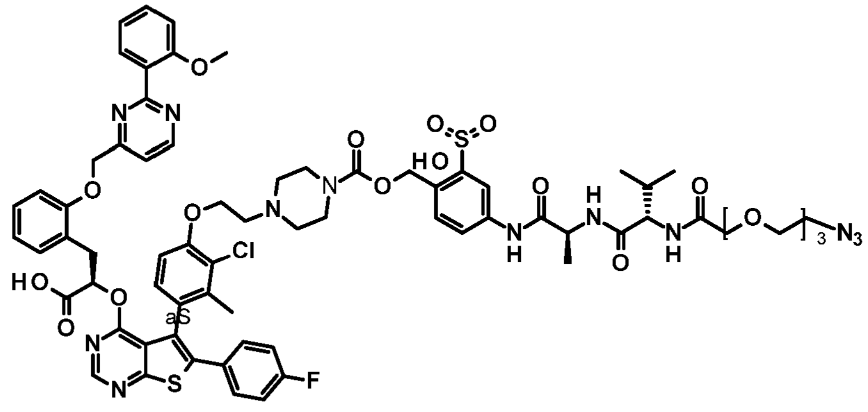

L29-C3,

| |
|---|
| 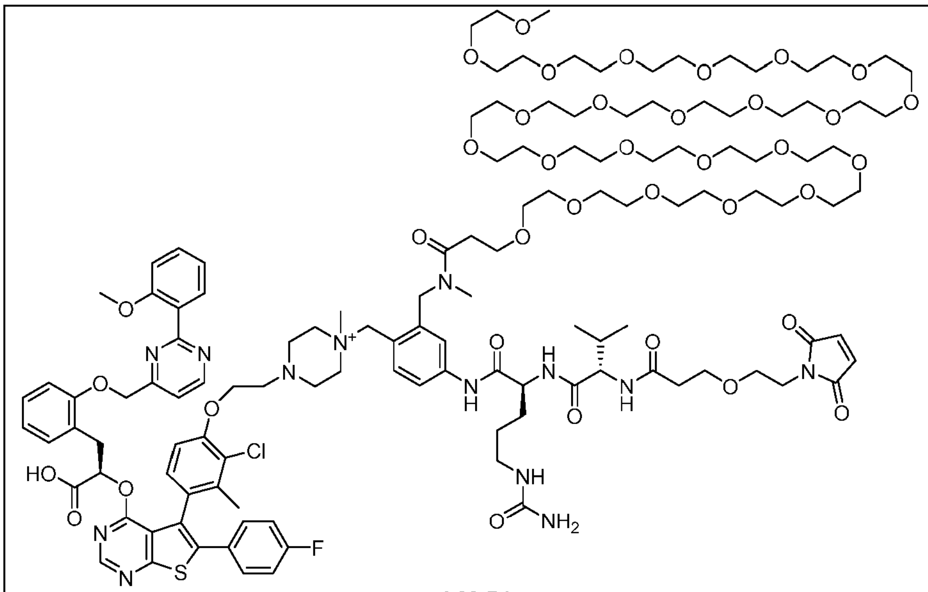 **L30-P1** , |
| 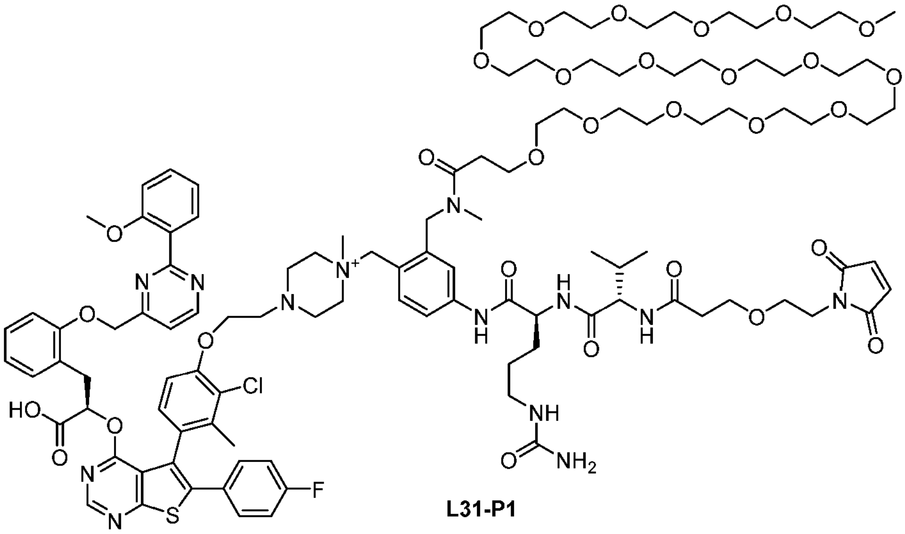 **L31-P1** , |
| 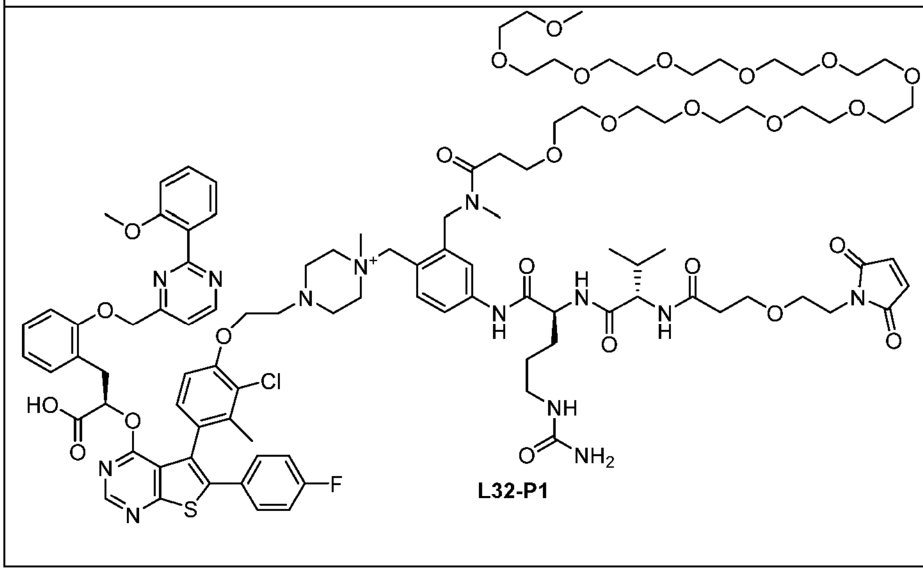 **L32-P1** , |

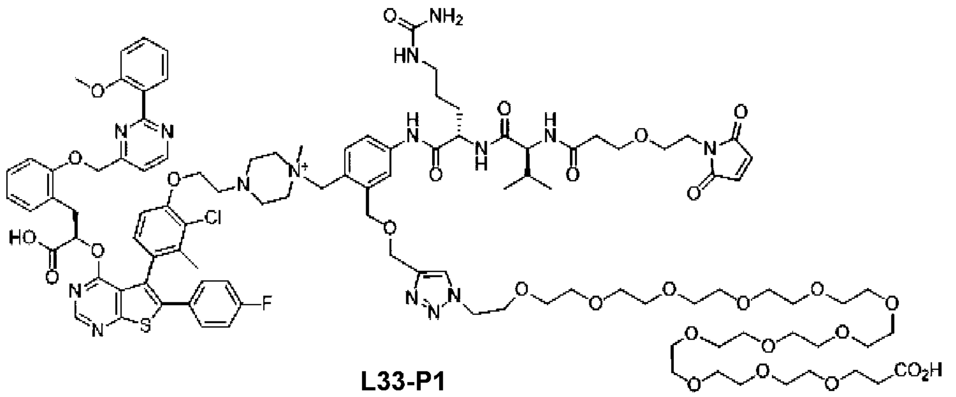

L33-P1

,

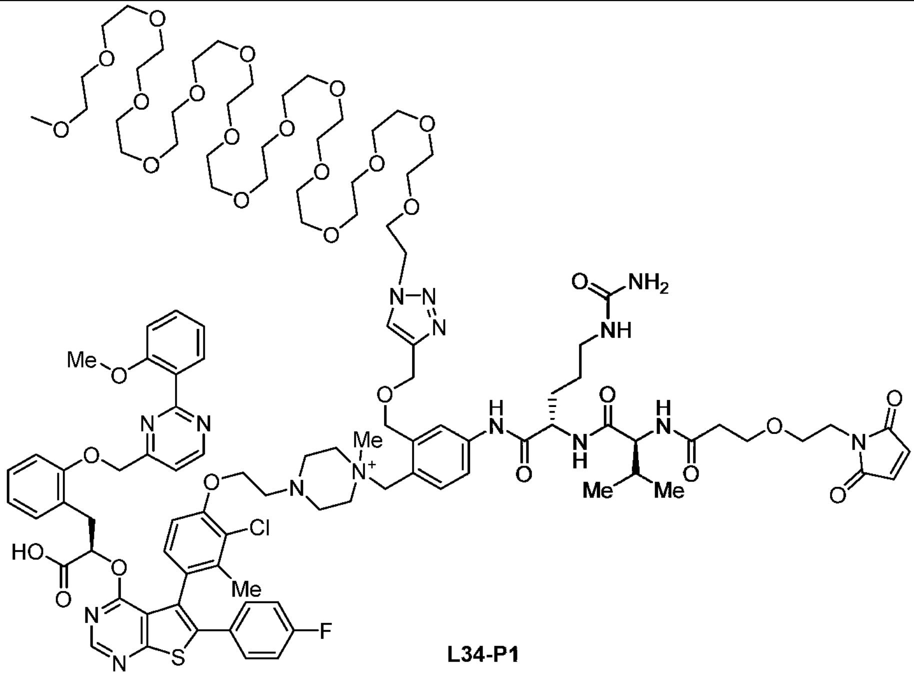

L34-P1

,

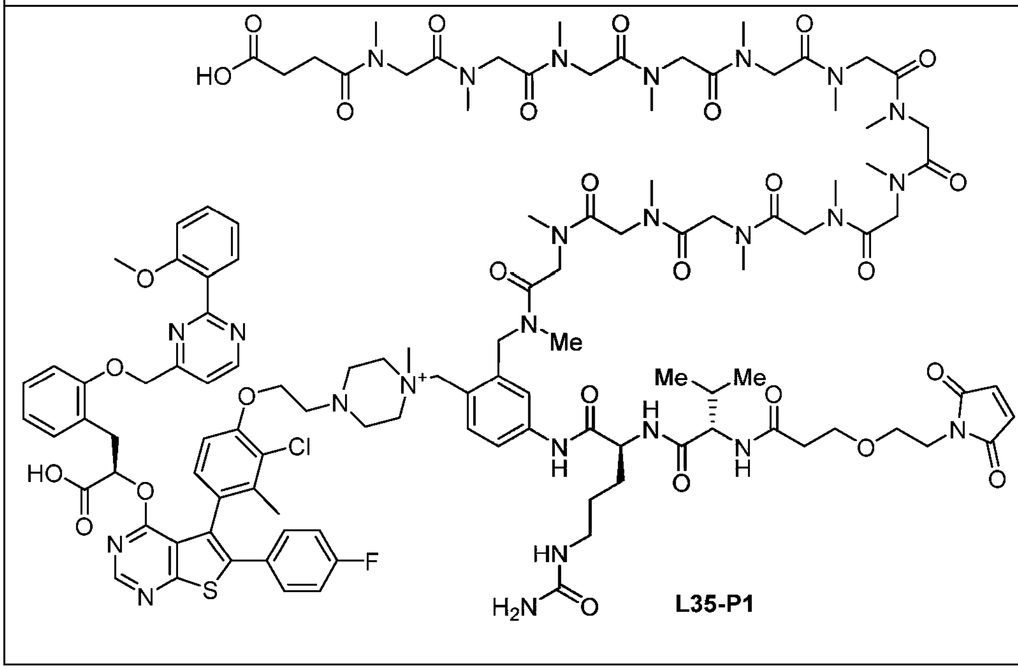

L35-P1

,

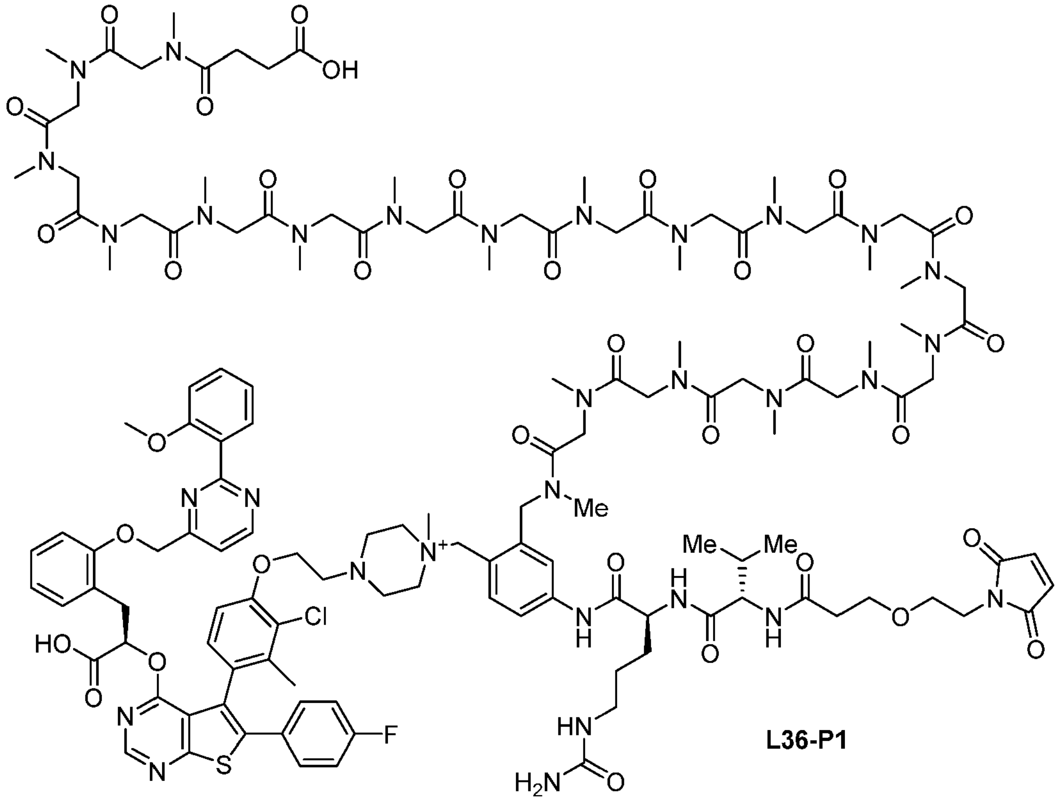

L36-P1

,

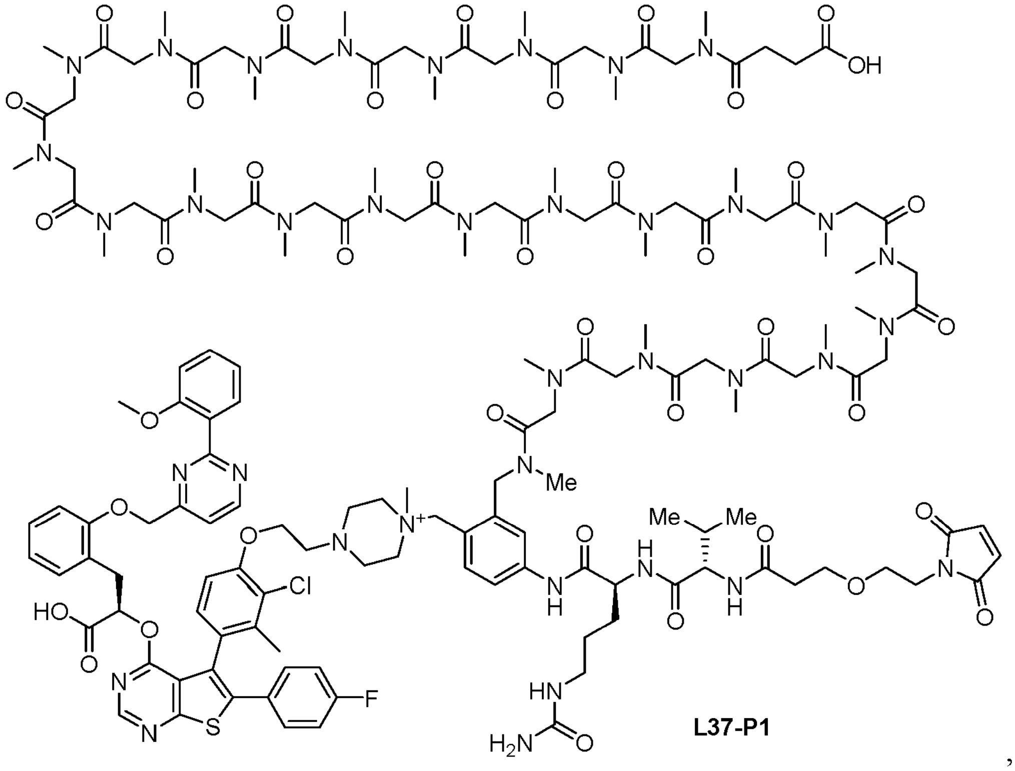

L37-P1

,

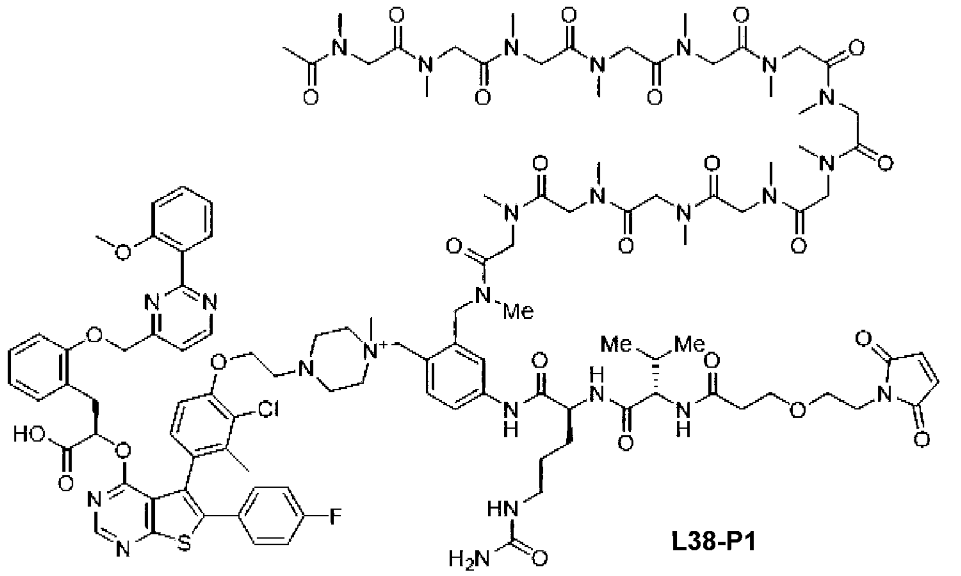

L38-P1

,

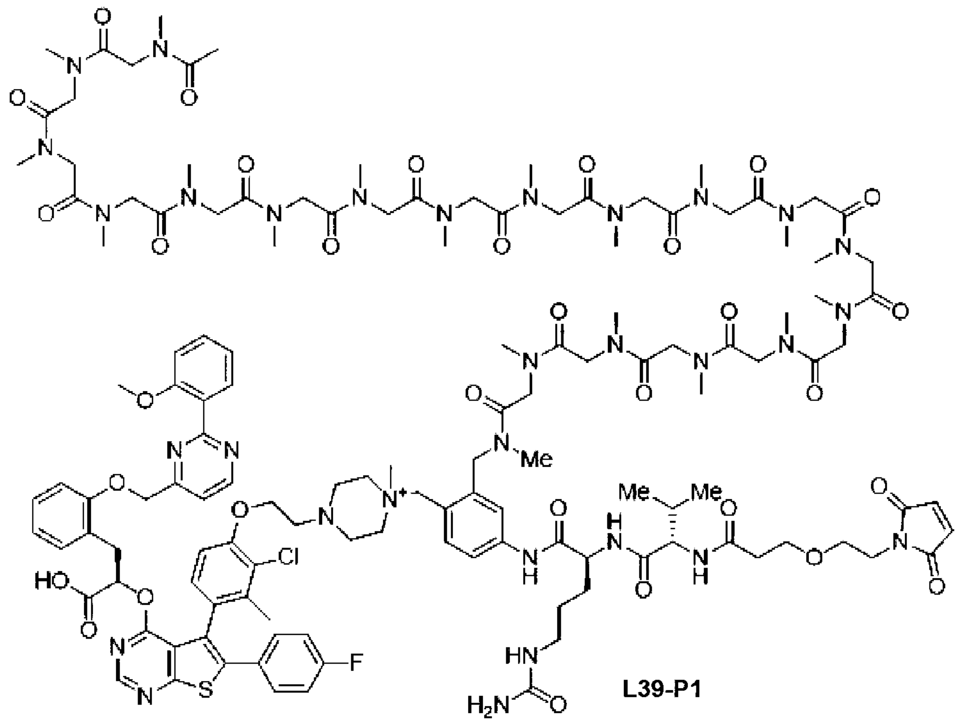

L39-P1

,

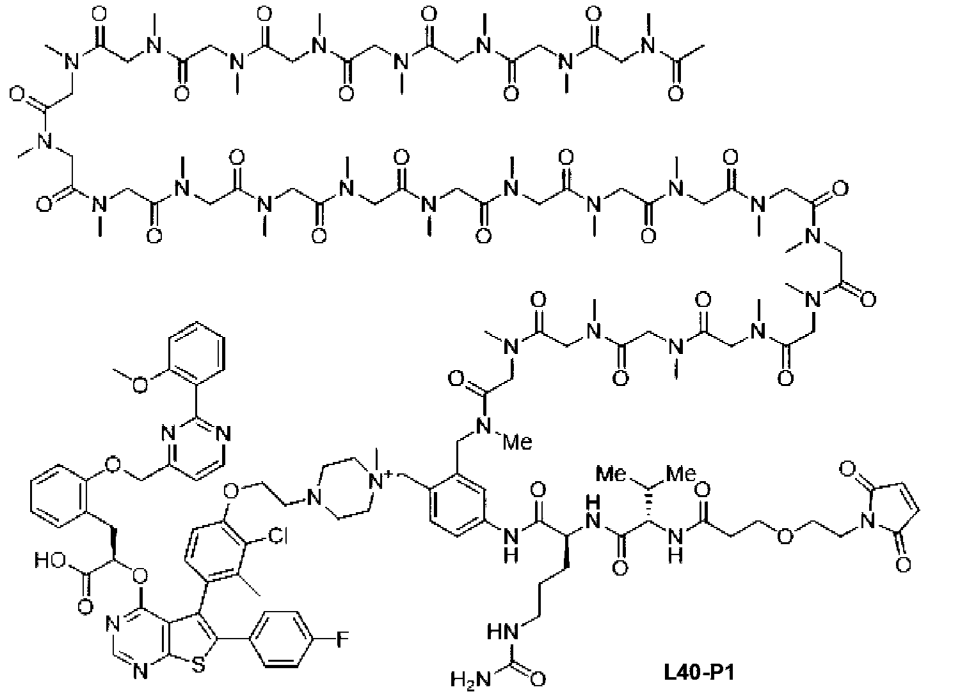

L40-P1

,

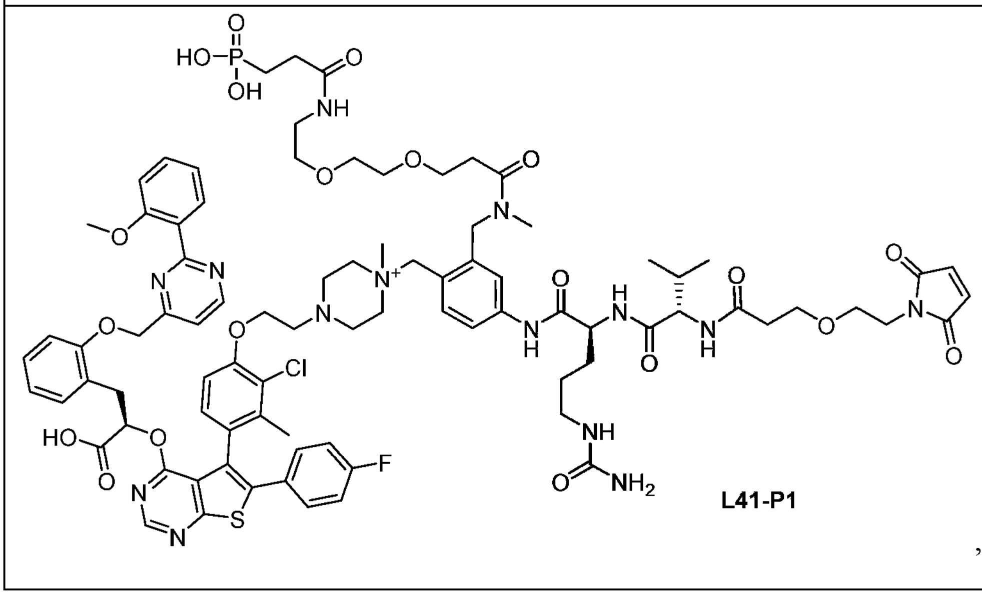

L41-P1

,

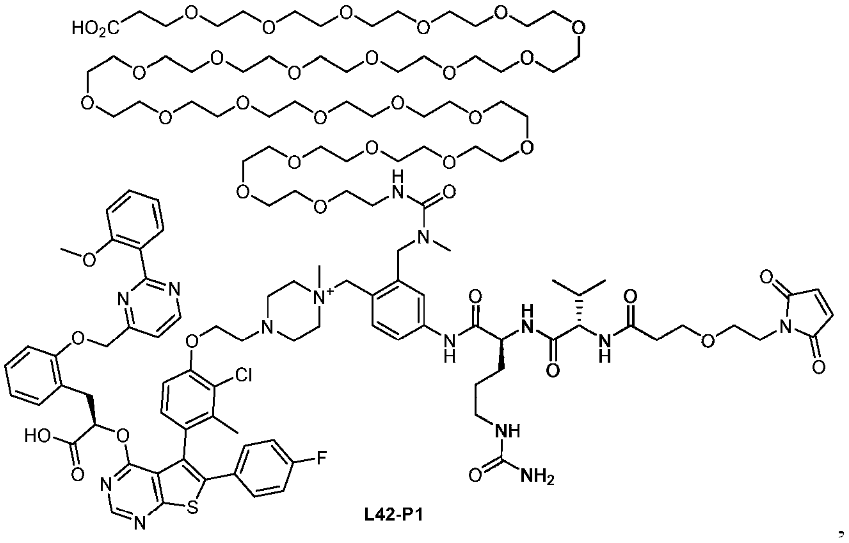

L42-P1

,

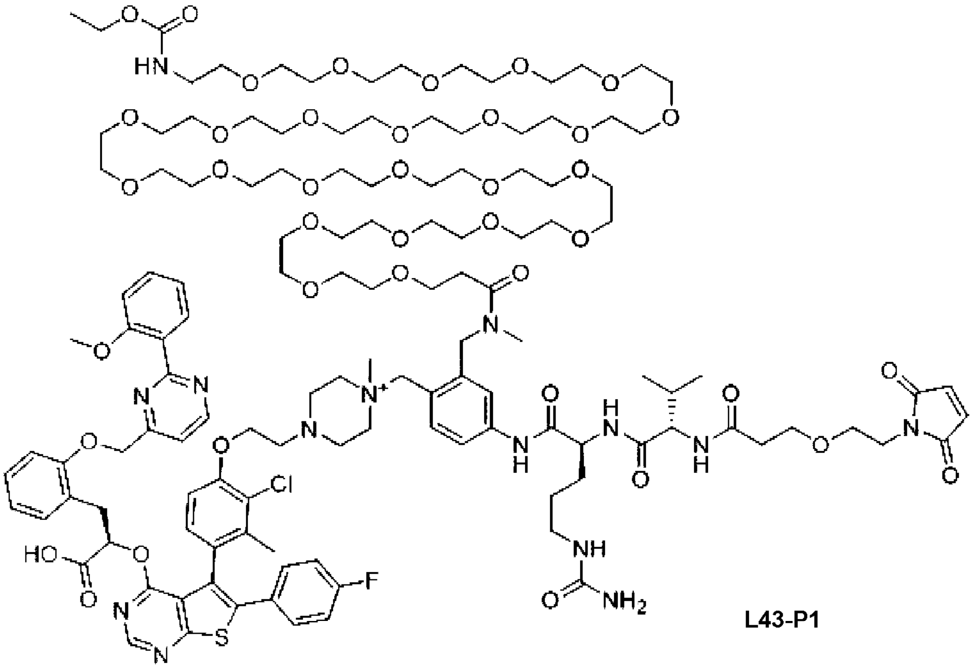

L43-P1

,

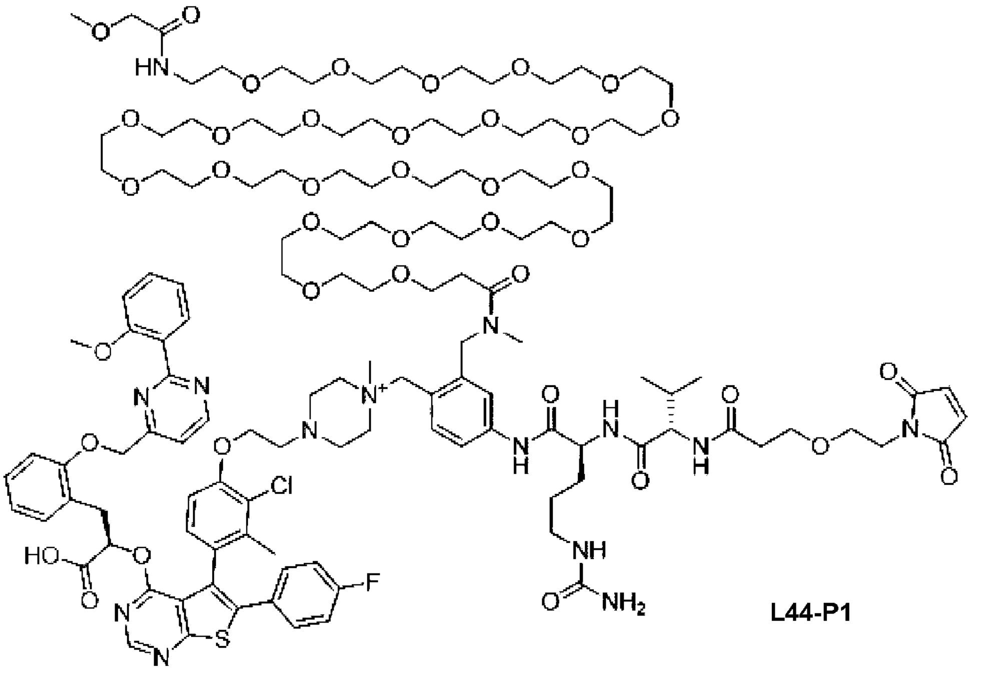

L44-P1

,

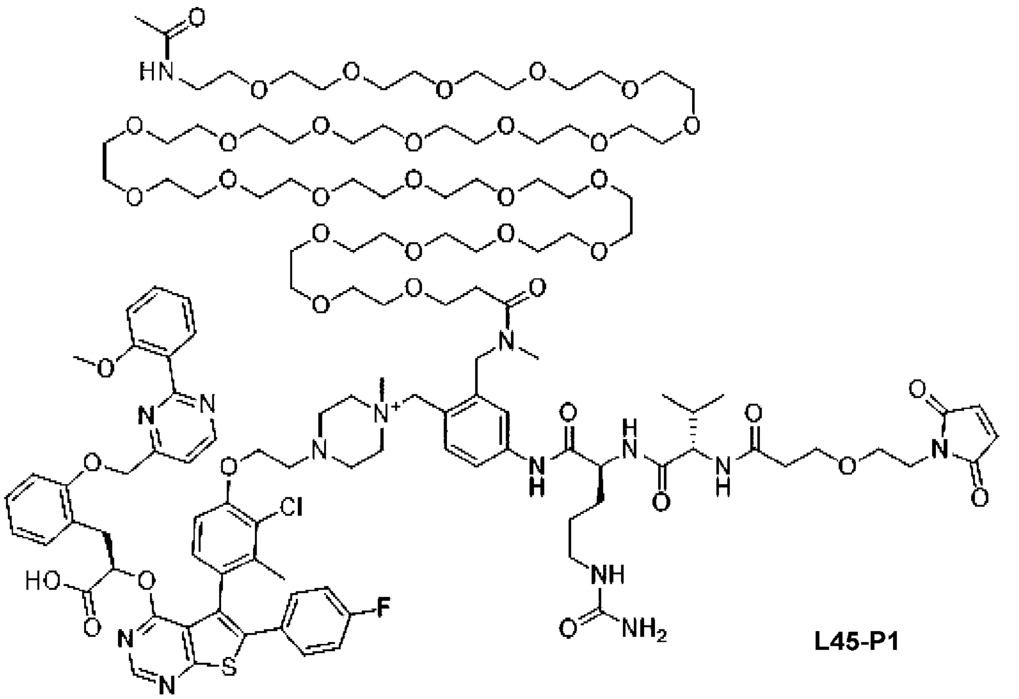

L45-P1

,

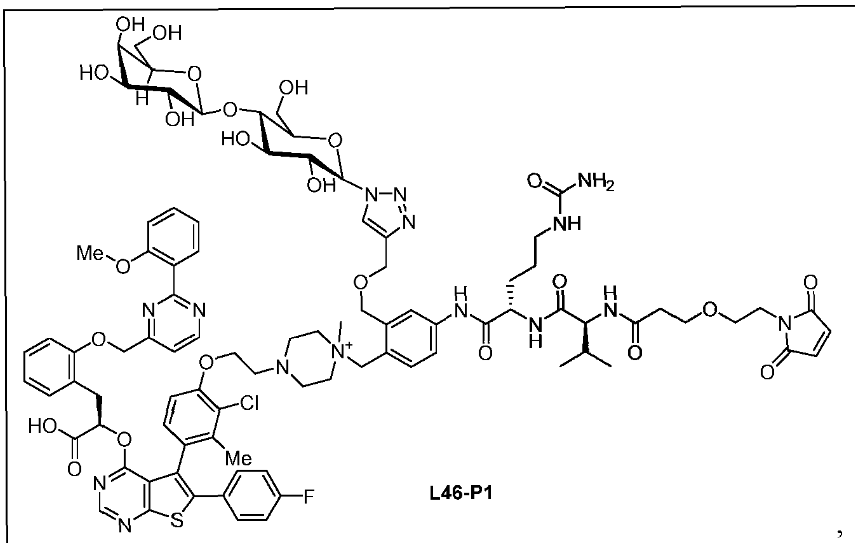

L46-P1

,

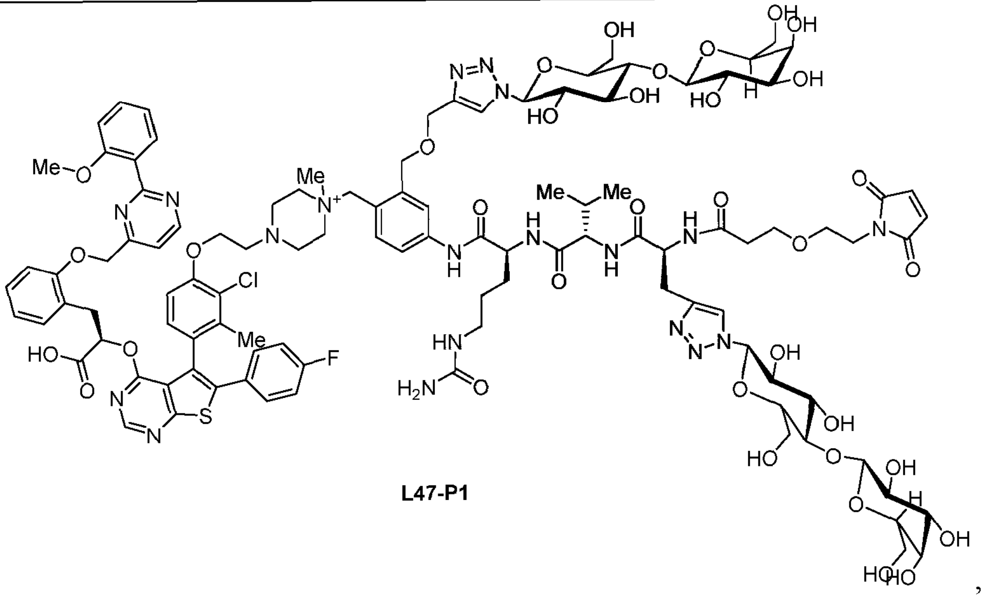

L47-P1

,

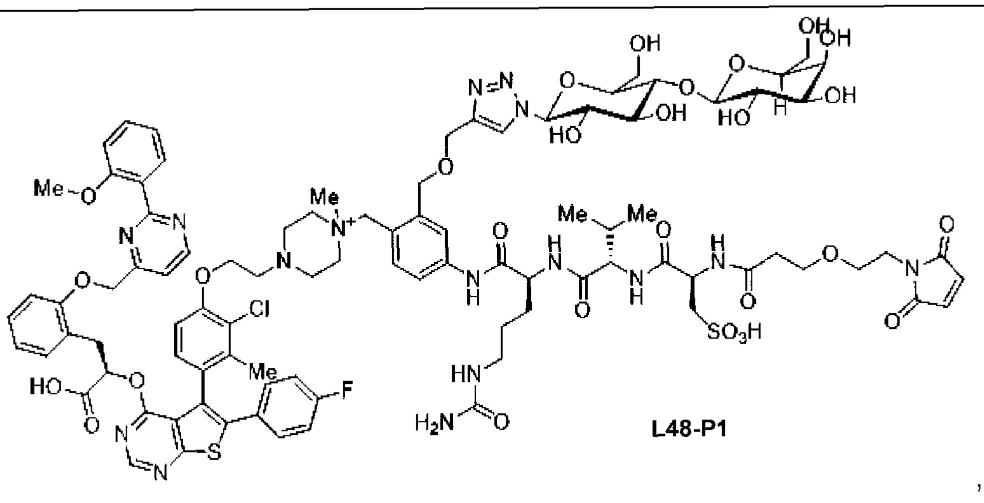

L48-P1

,

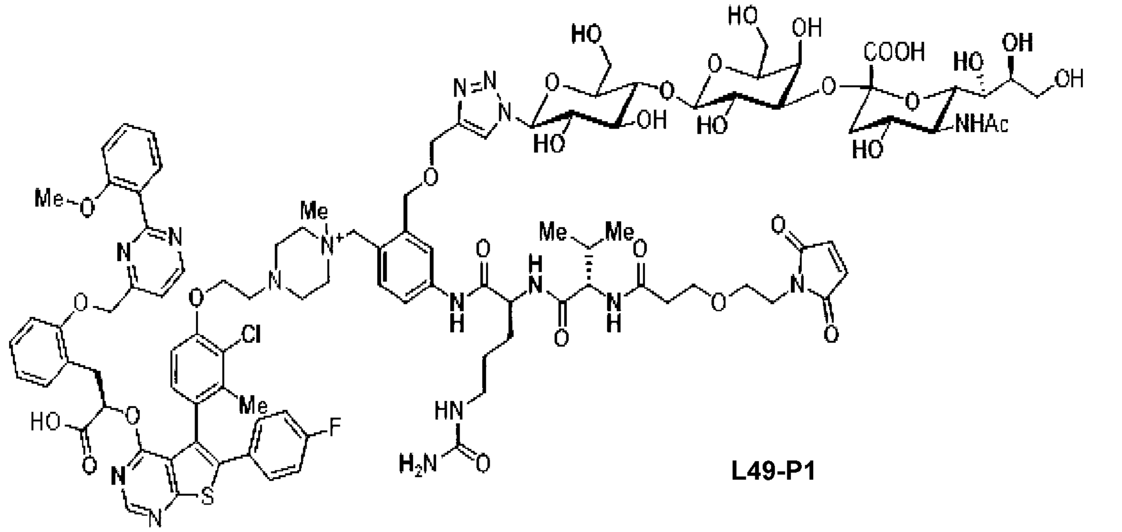

L49-P1

,

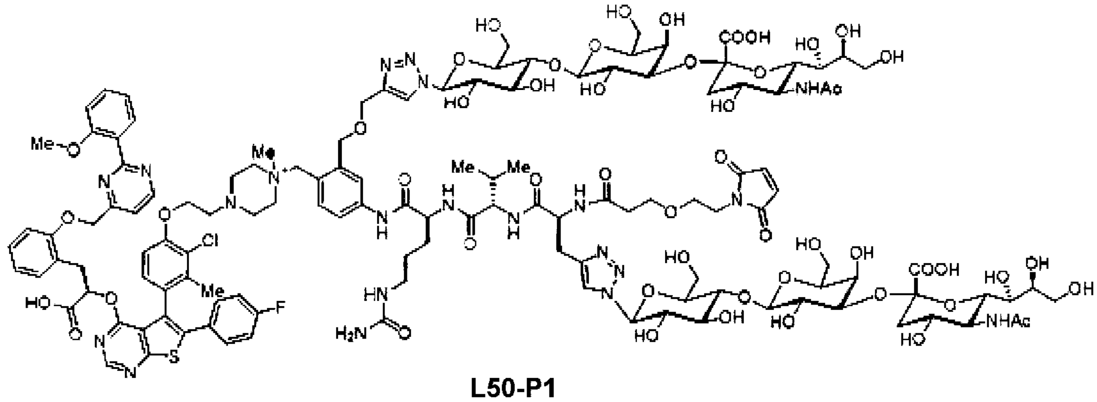

L50-P1

,

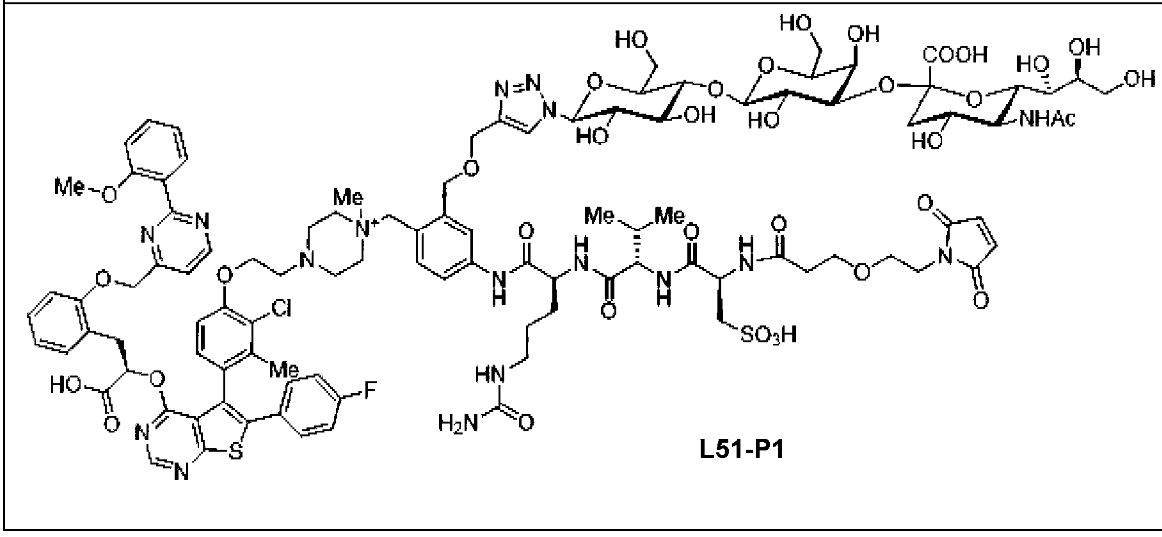

L51-P1

,

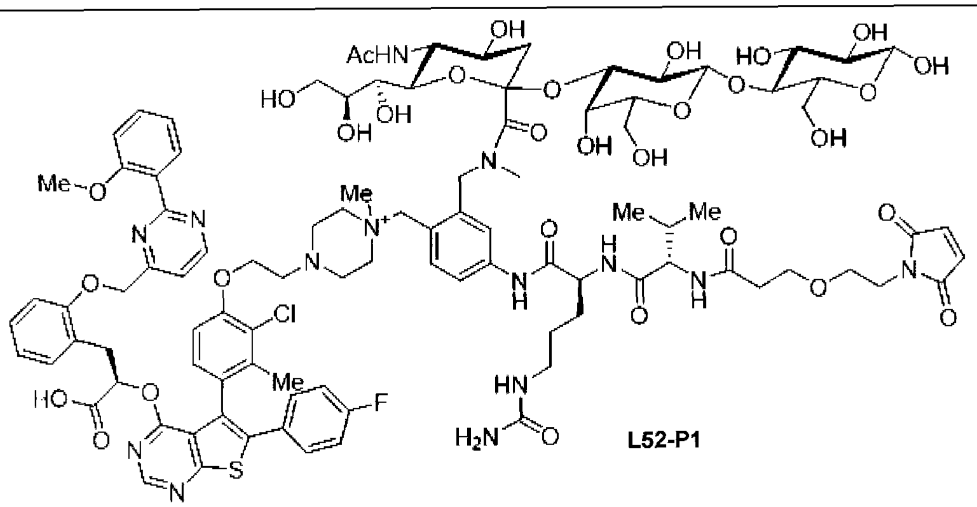

**L52-P1**

,

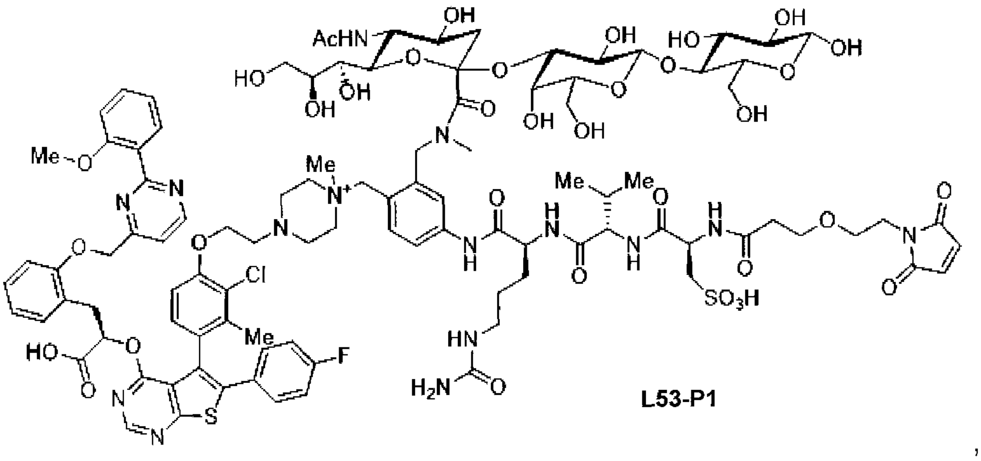

**L53-P1**

,

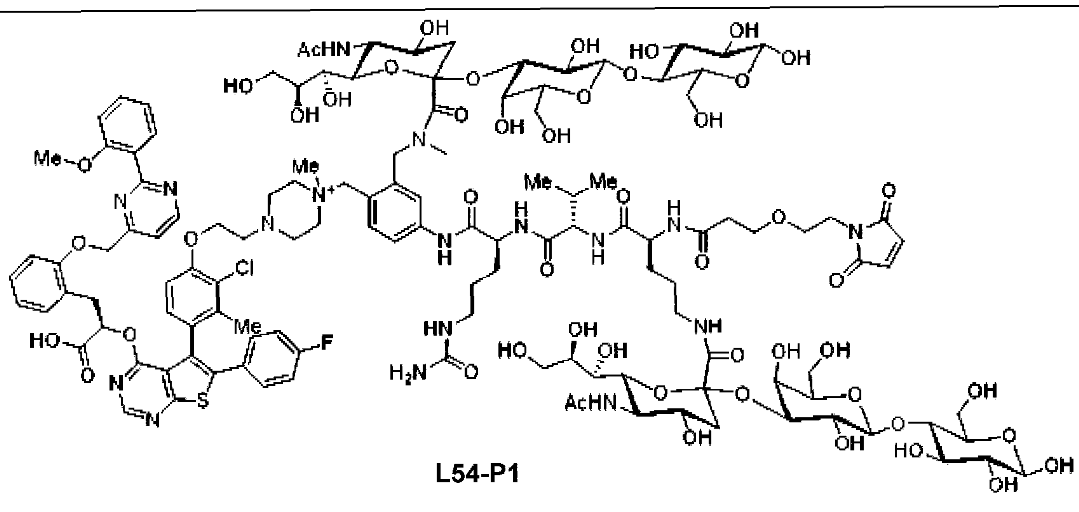

**L54-P1**

,

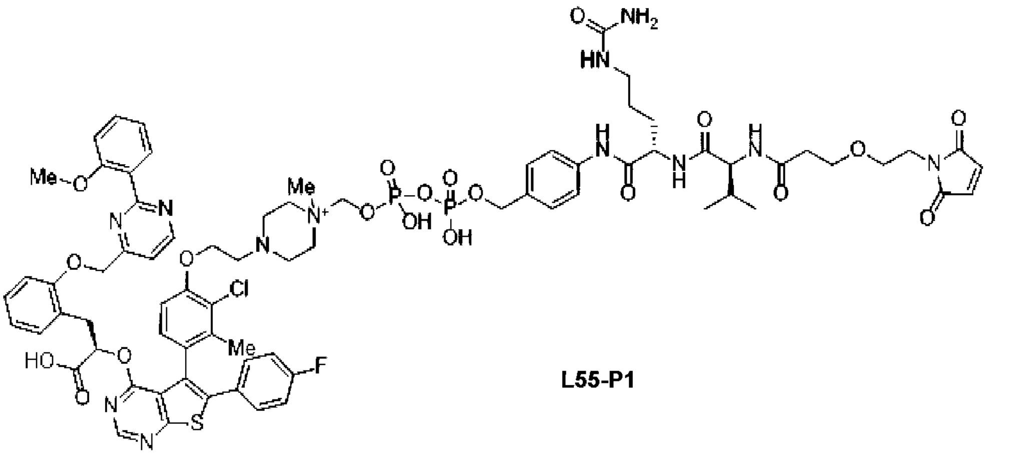

L55-P1

,

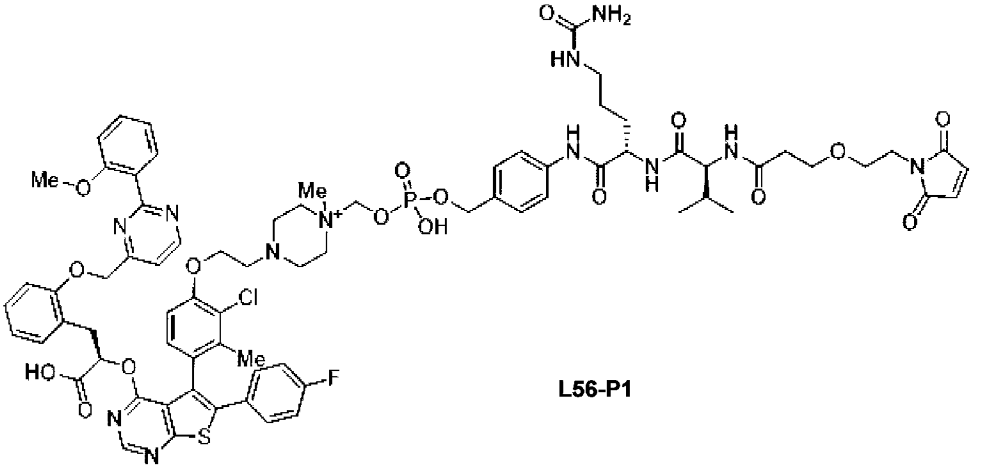

L56-P1

,

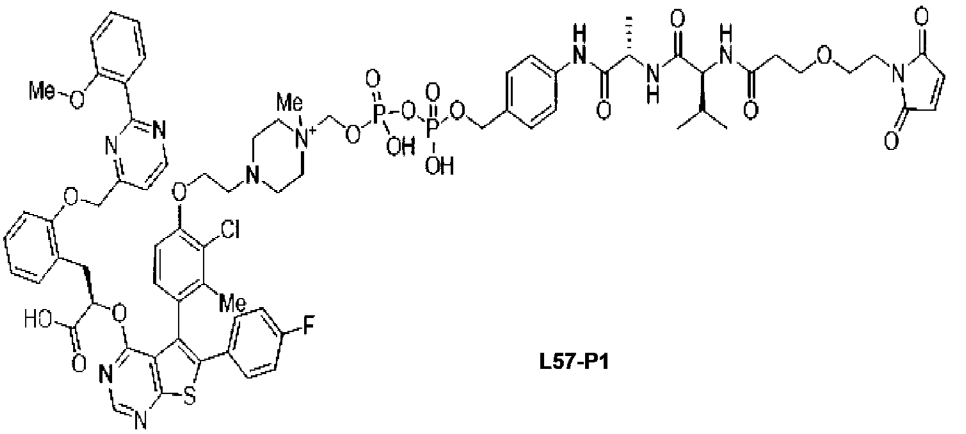

L57-P1

,

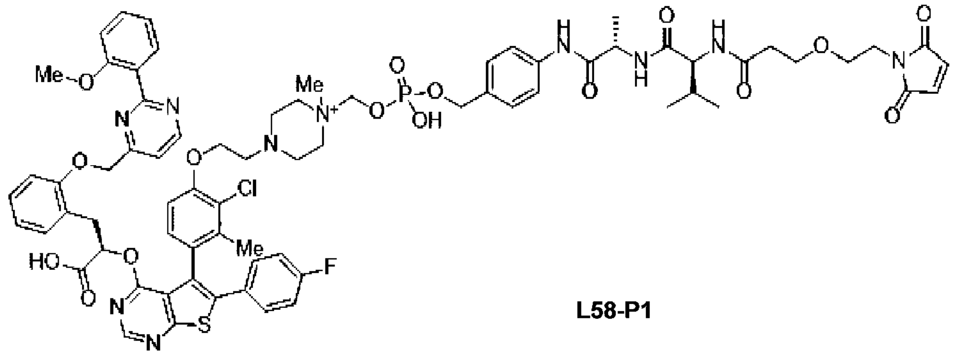

L58-P1

,

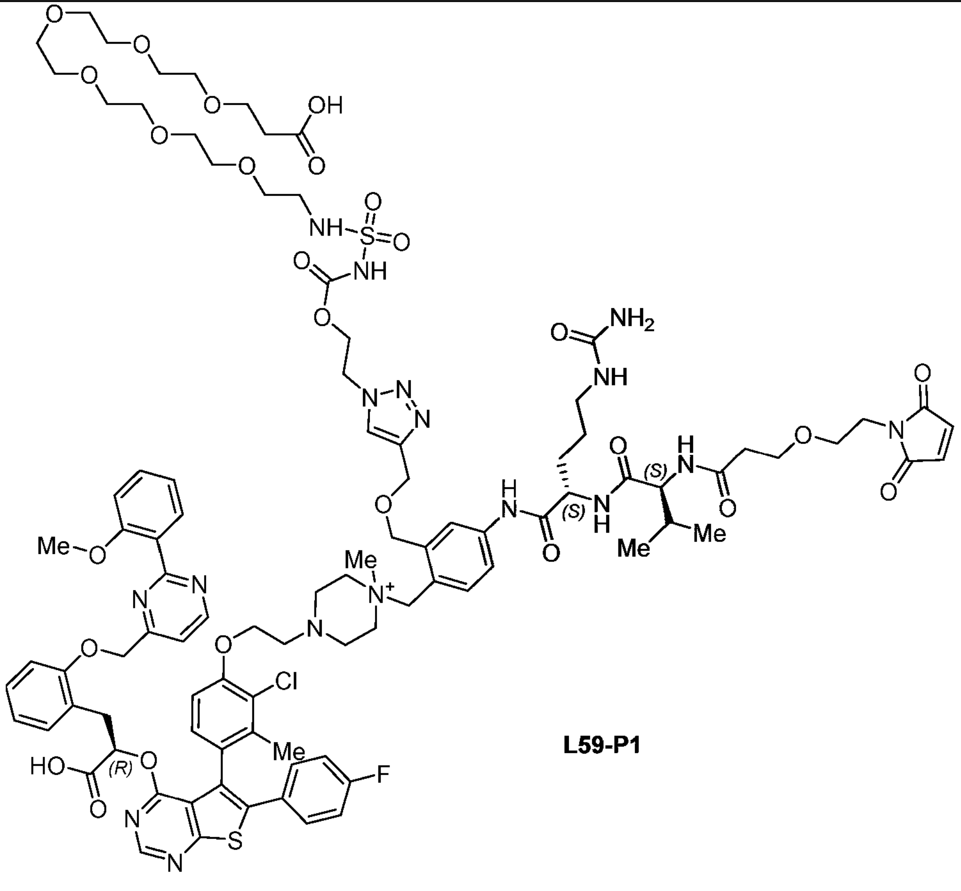

L59-P1

,

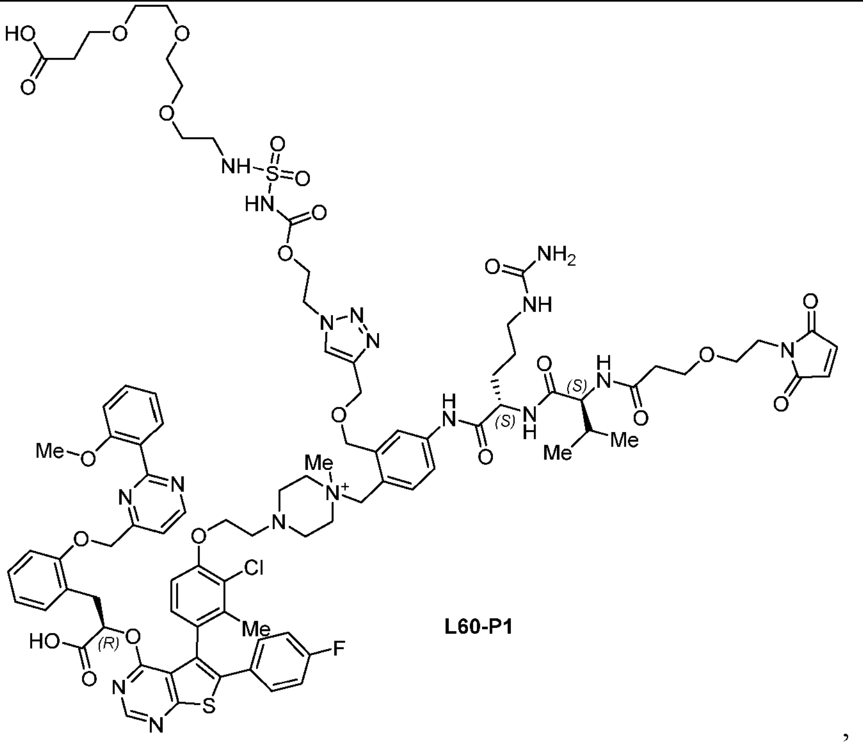

L60-P1

,

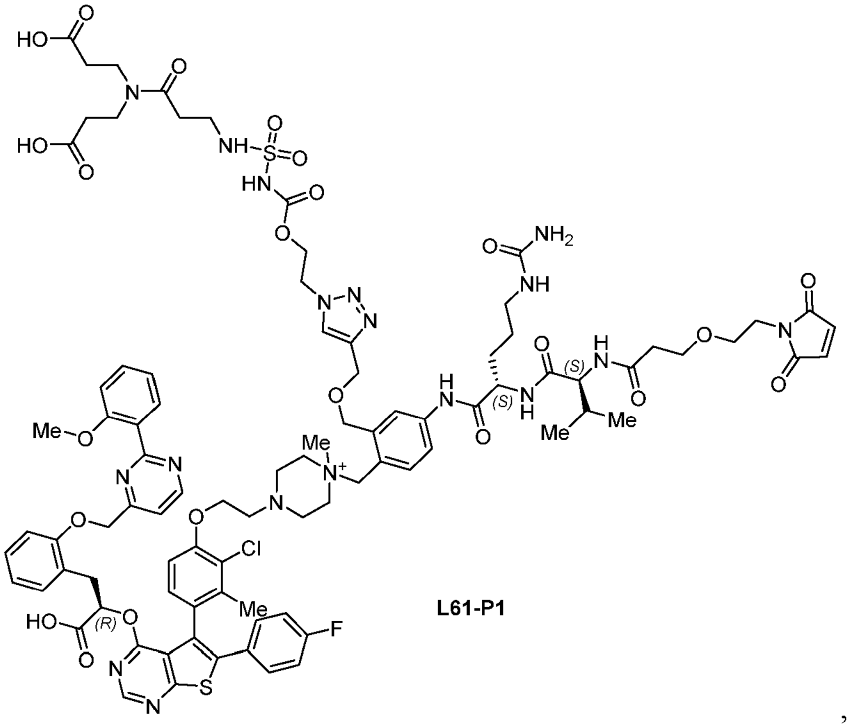

L61-P1

,

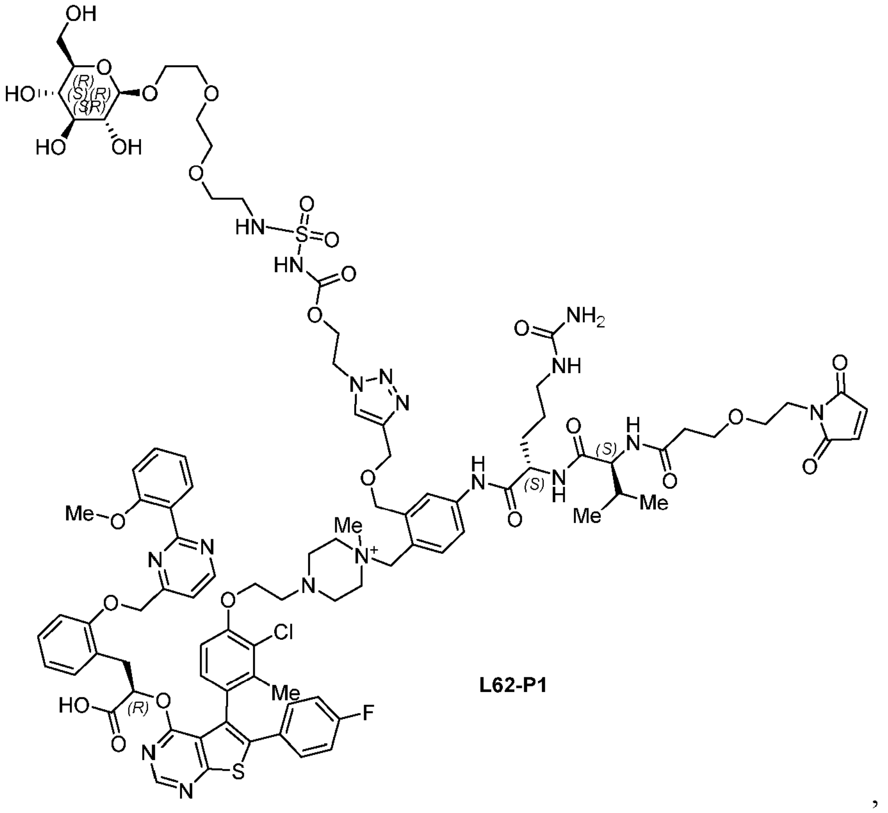

L62-P1

,

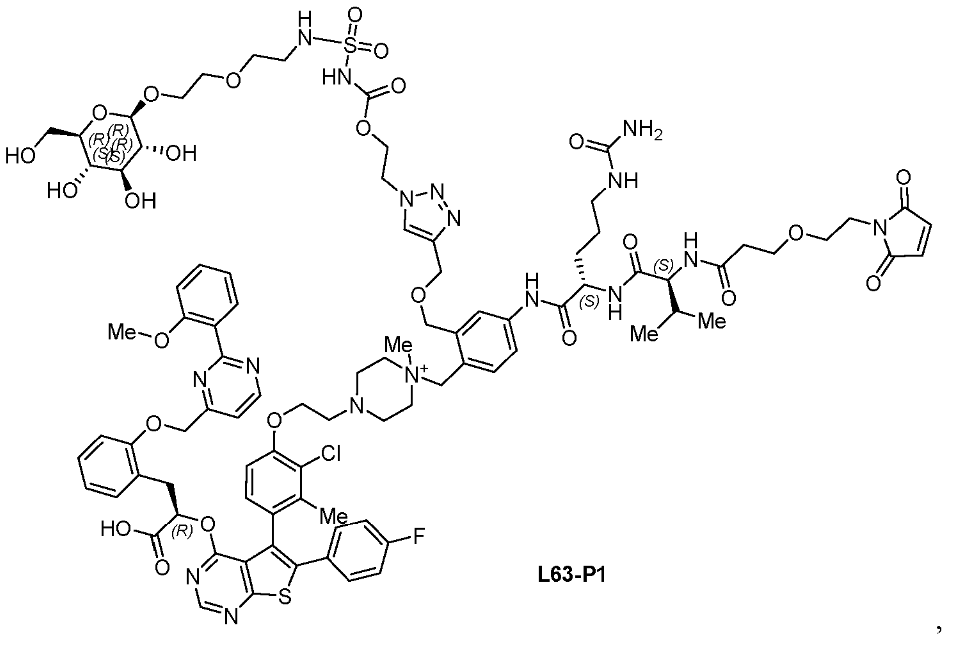

L63-P1

,

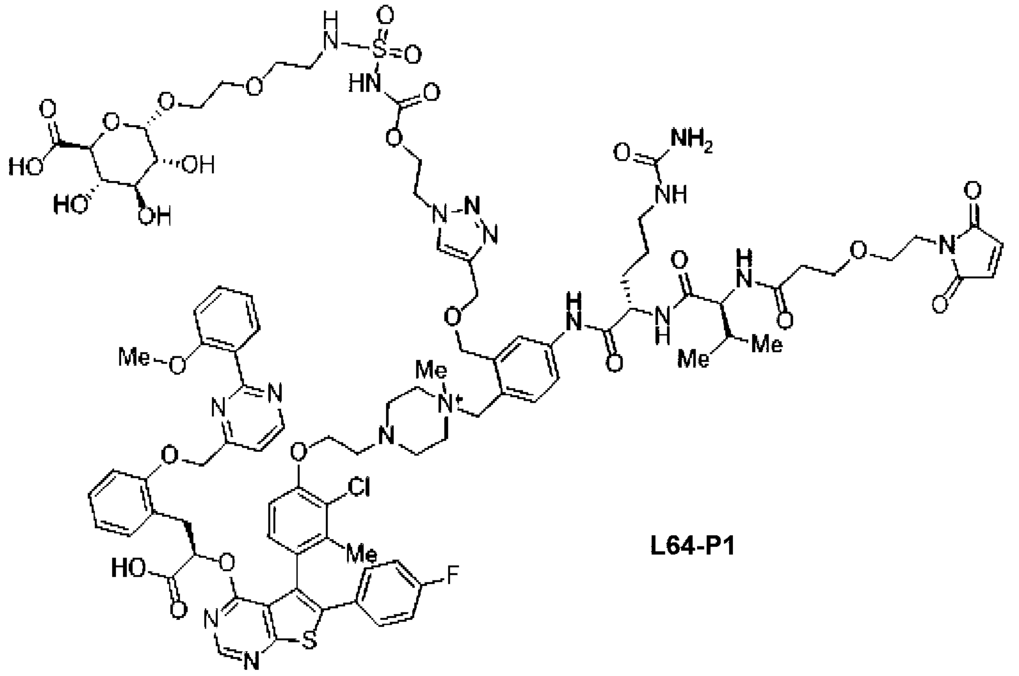

L64-P1

,

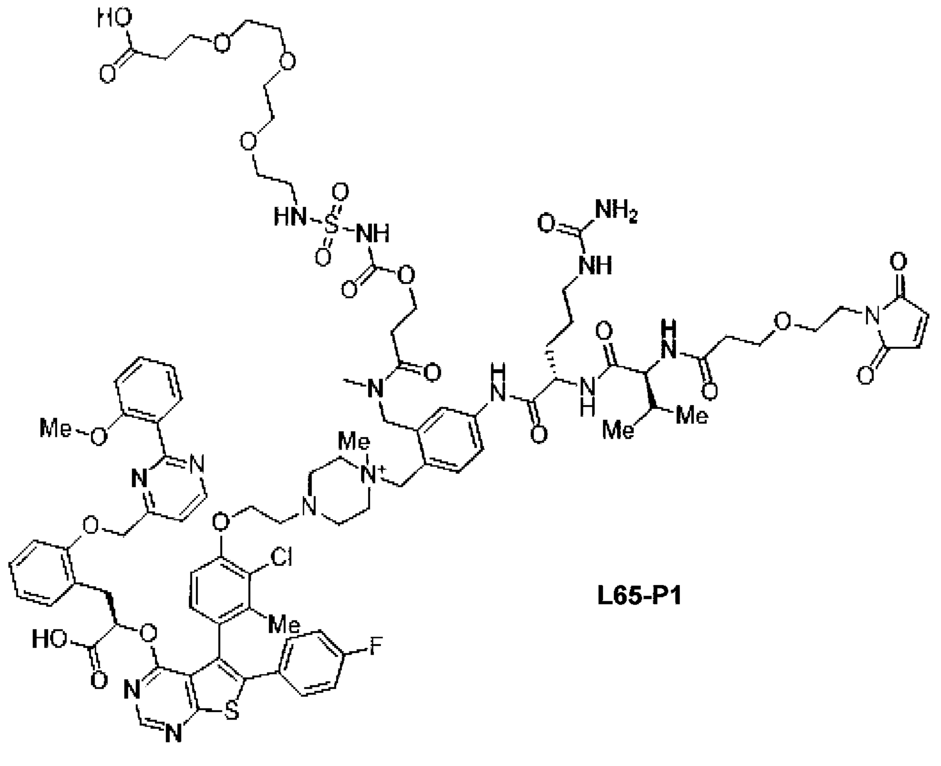

L65-P1

,

| |
|---|
| 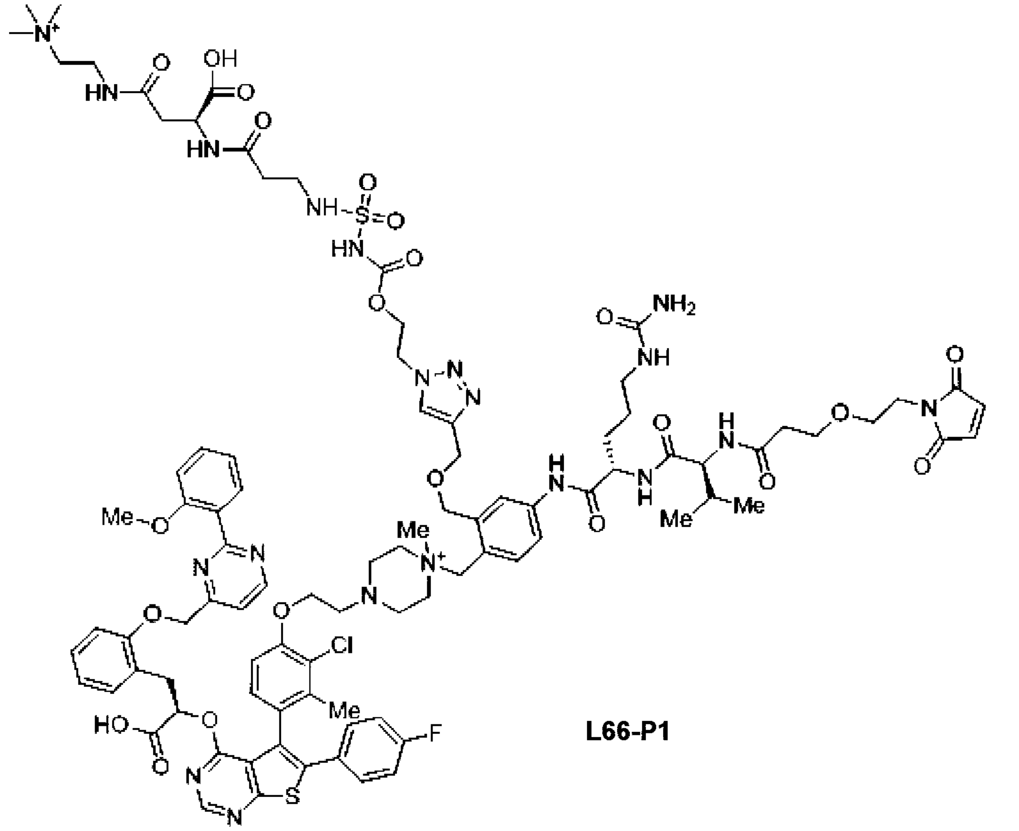 L66-P1 , |
| 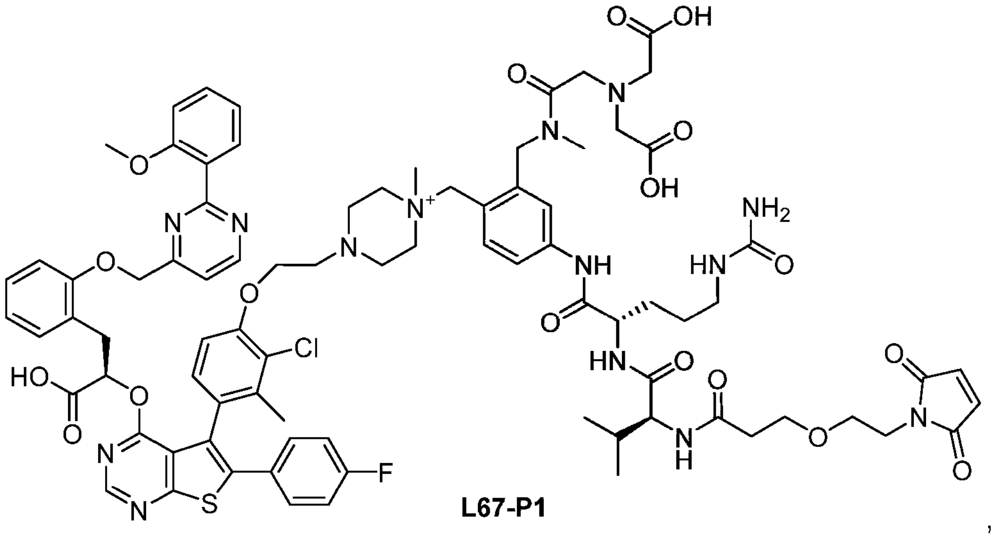 L67-P1 , |

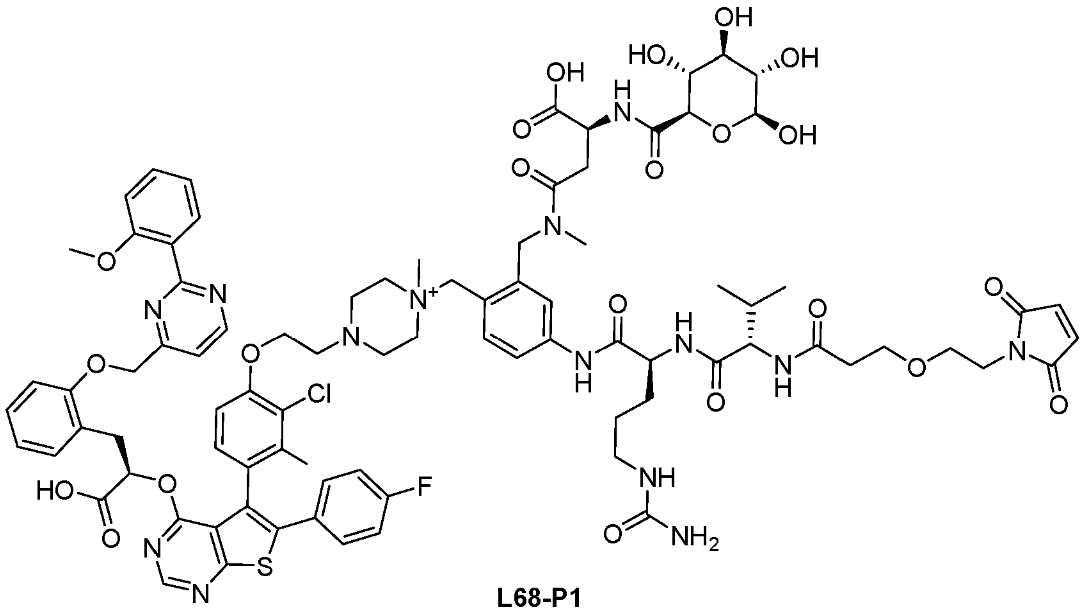

**L68-P1**

,

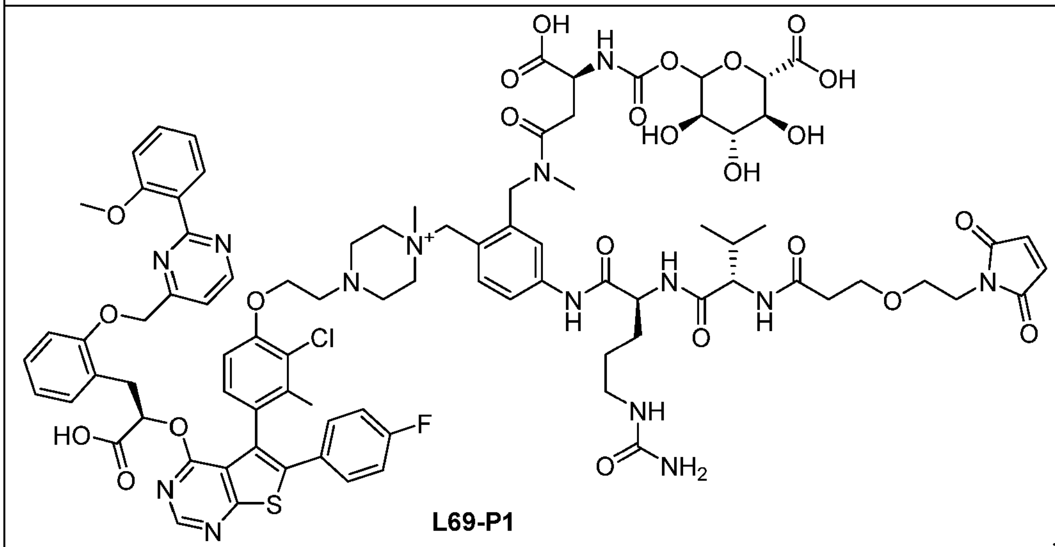

**L69-P1**

,

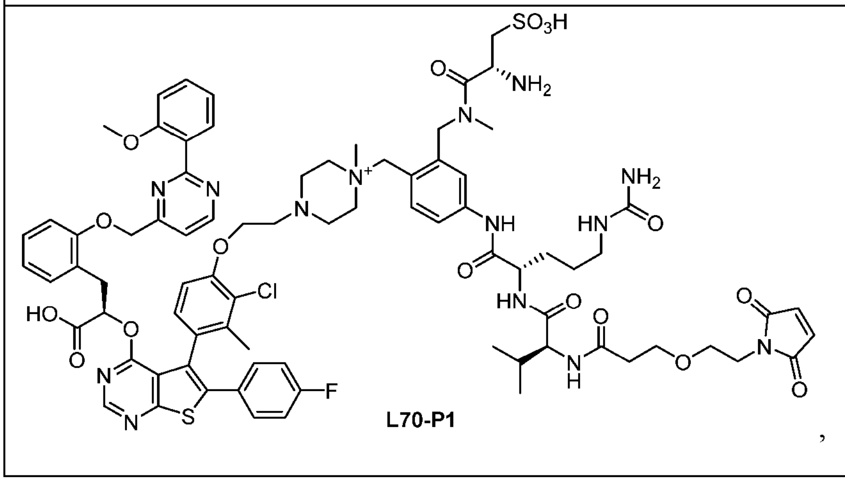

**L70-P1**

,

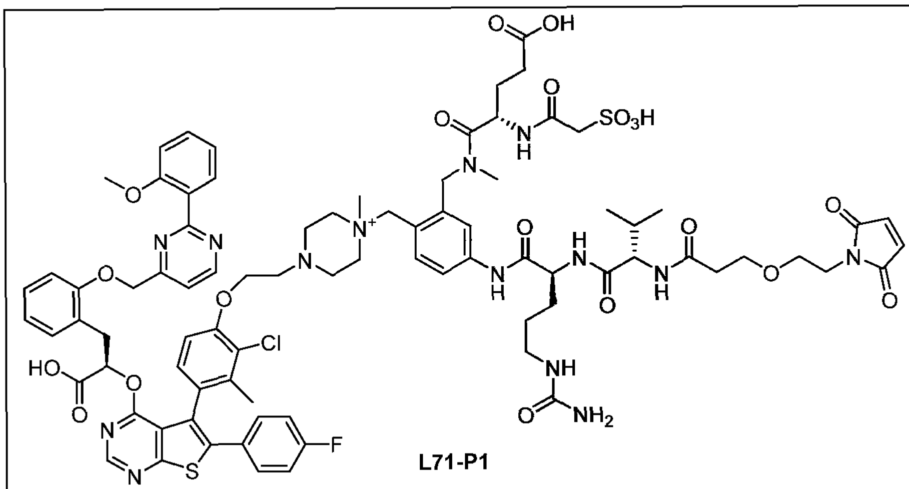

L71-P1

,

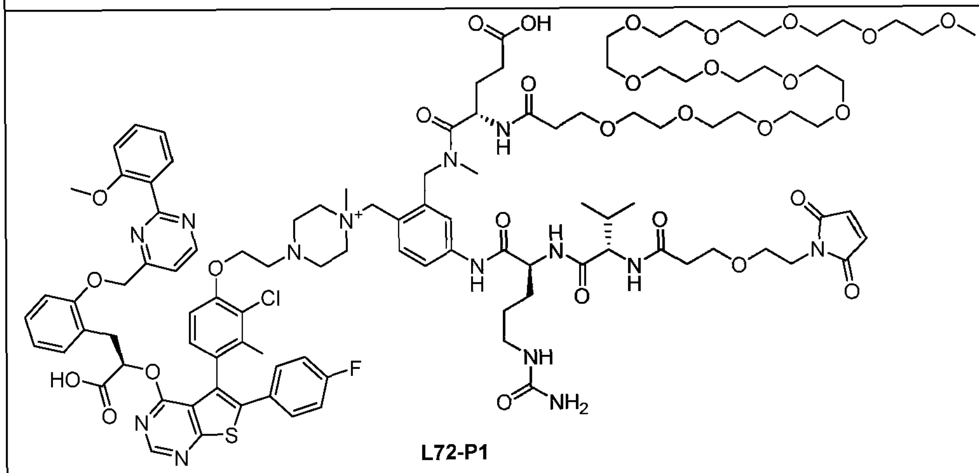

L72-P1

,

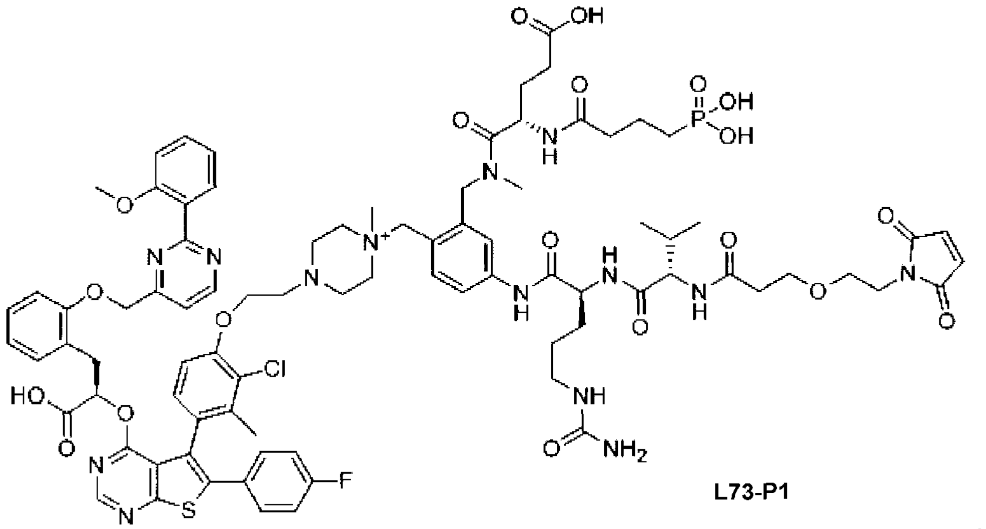

L73-P1

,

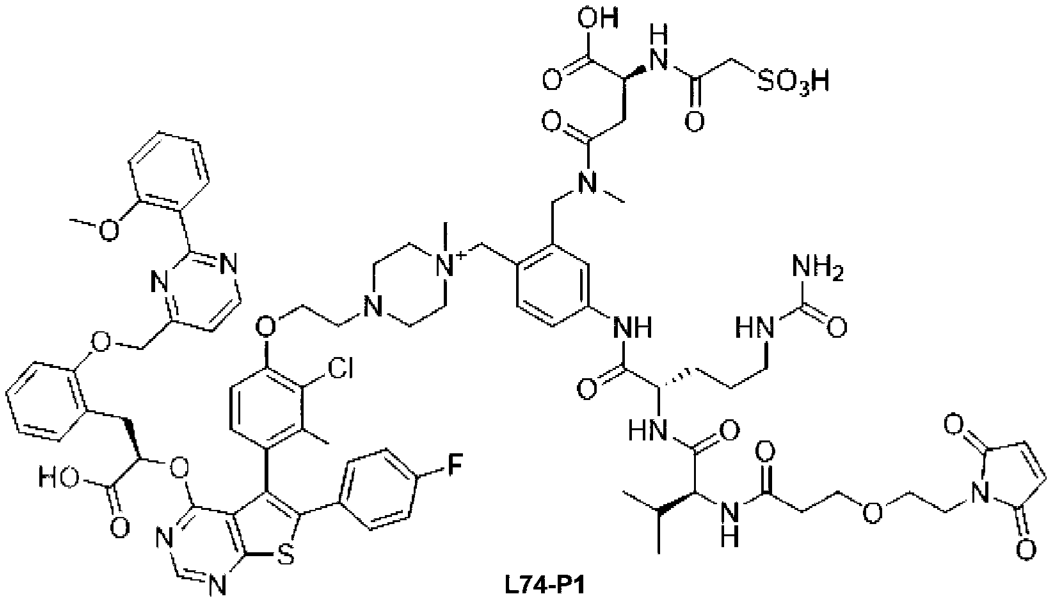

L74-P1

,

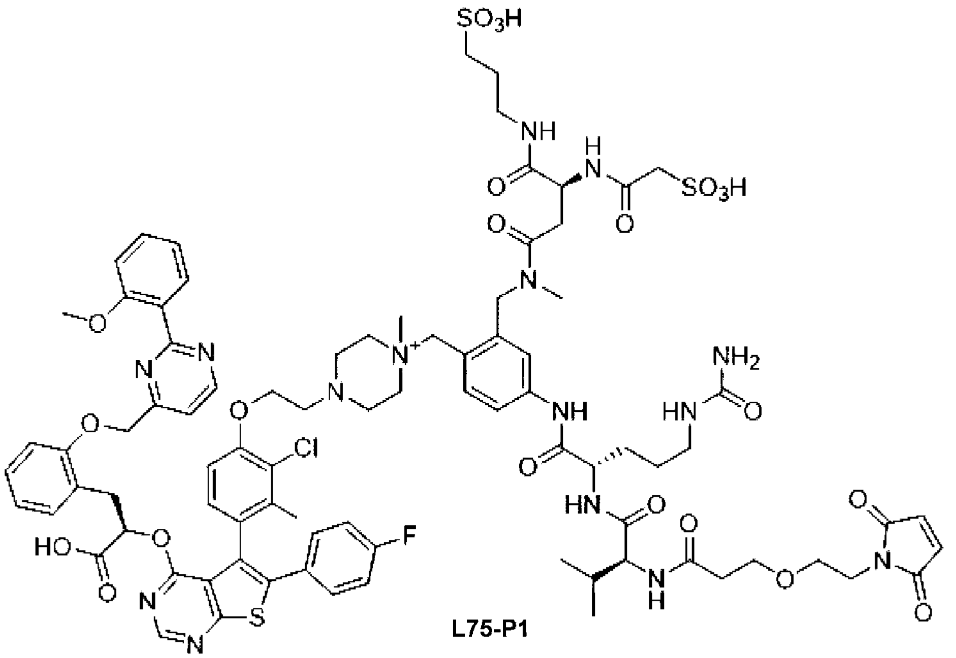

L75-P1

,

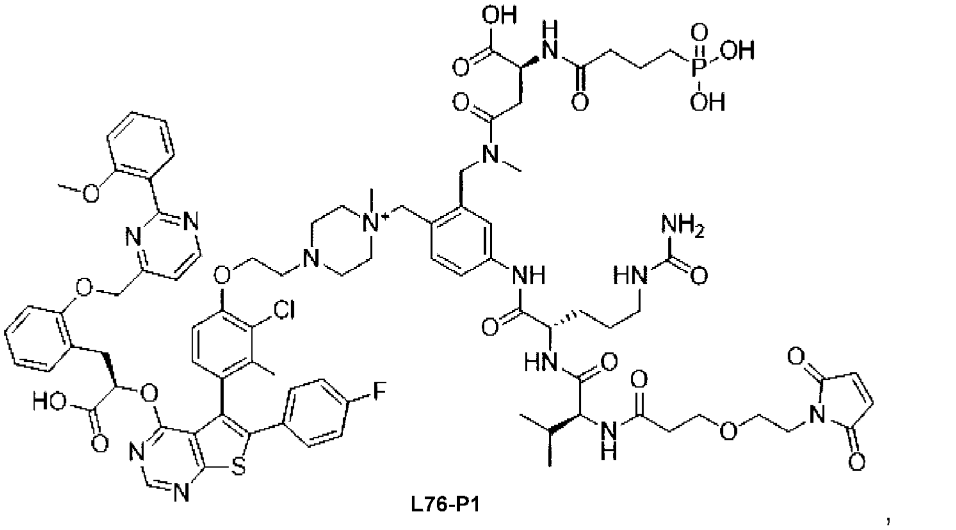

L76-P1

,

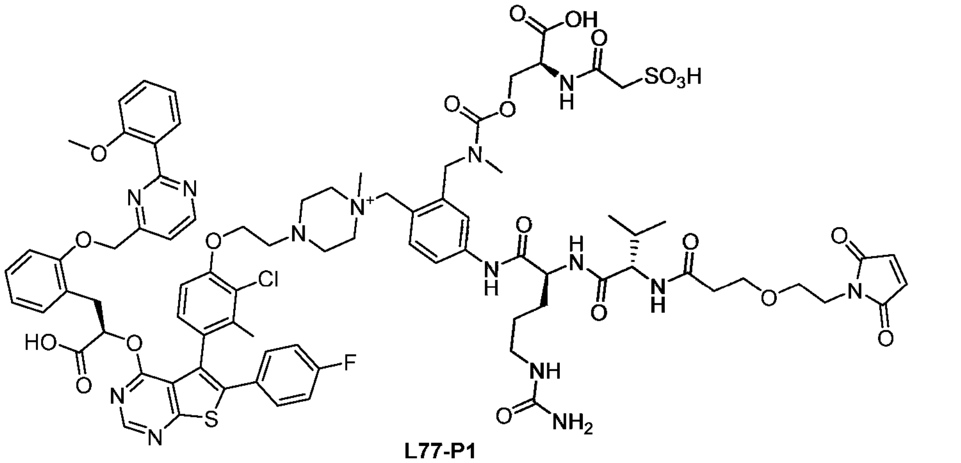

L77-P1

,

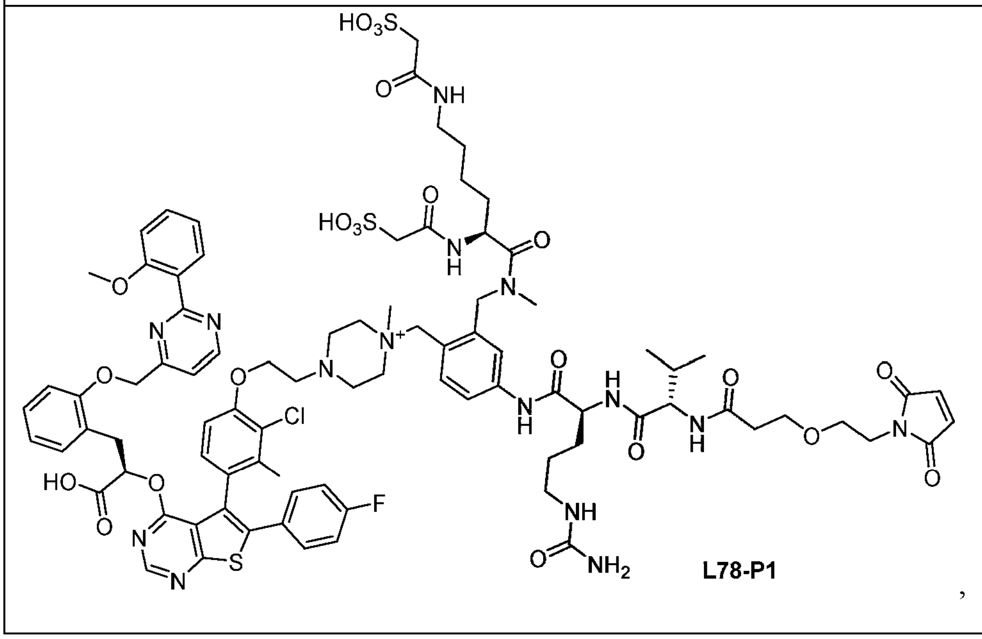

L78-P1

,

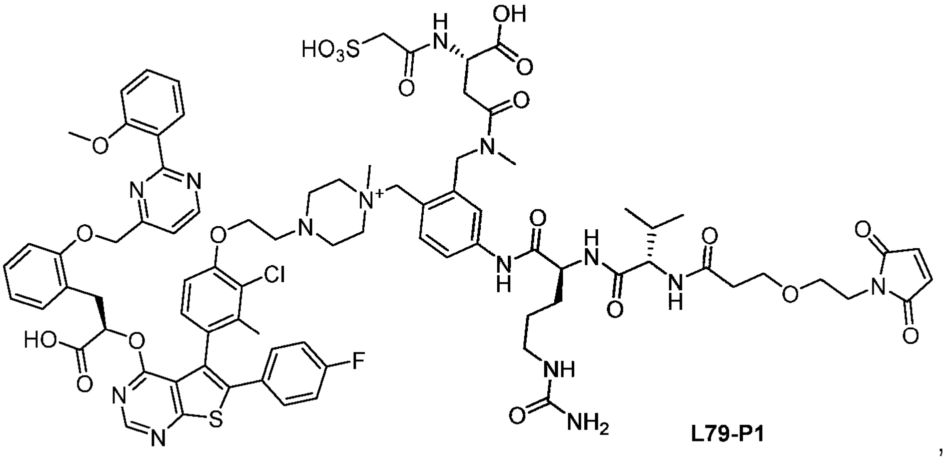

L79-P1

,

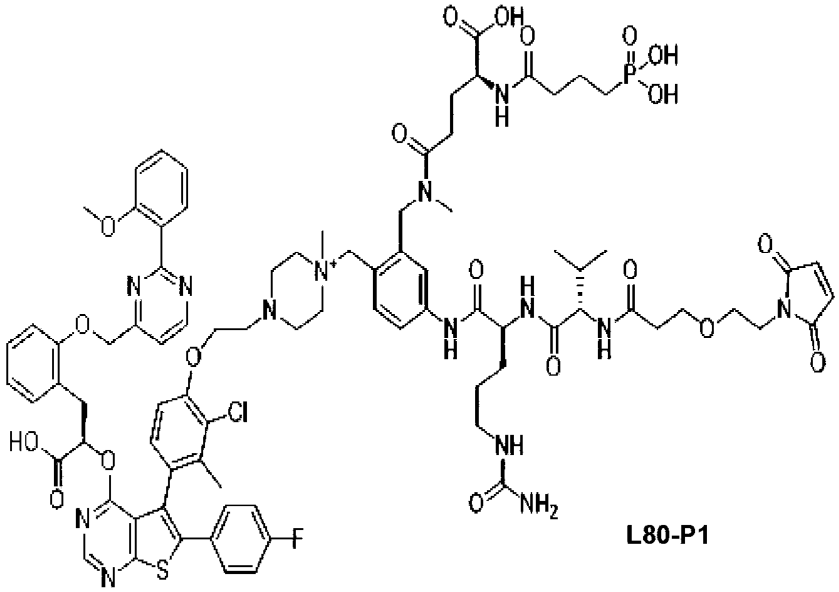

L80-P1

,

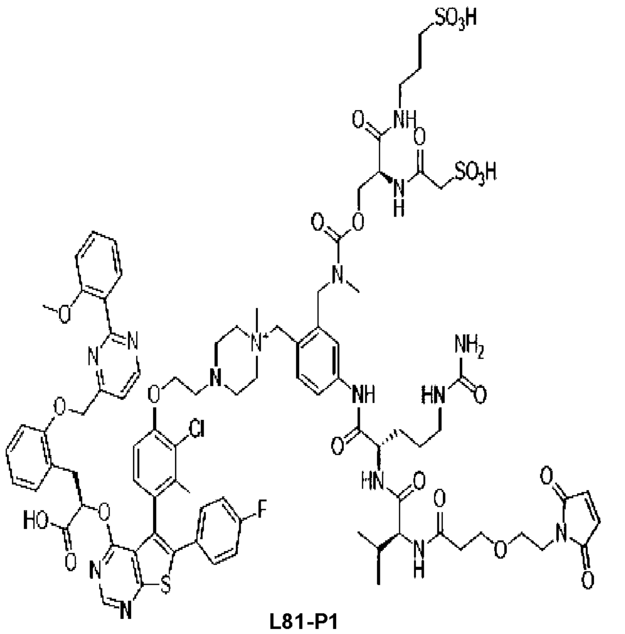

L81-P1

,

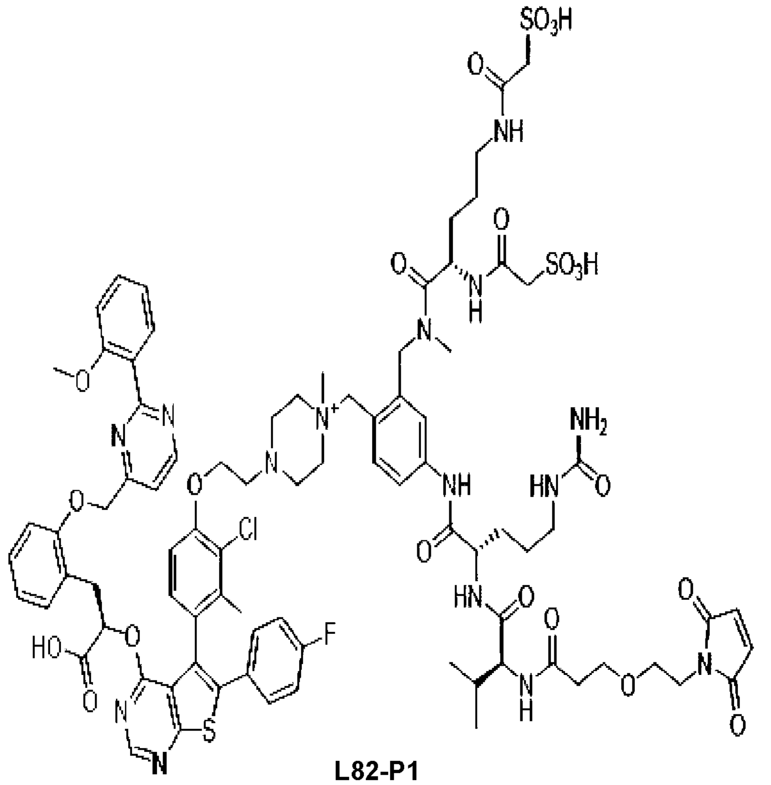

L82-P1

,

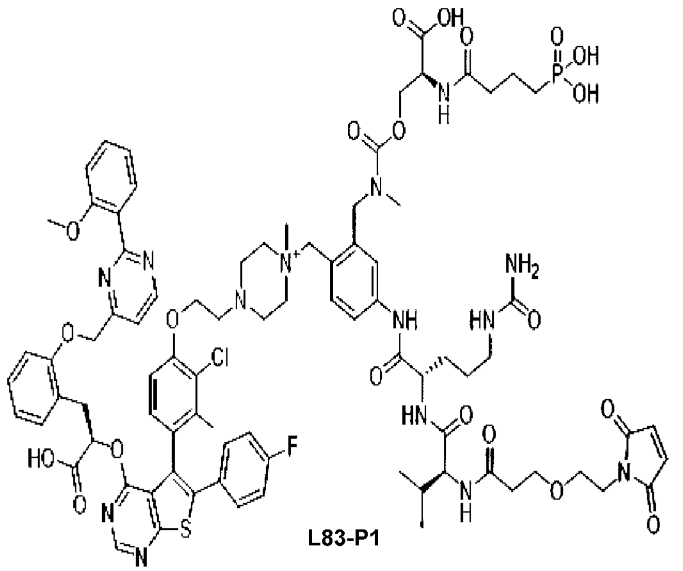

L83-P1

,

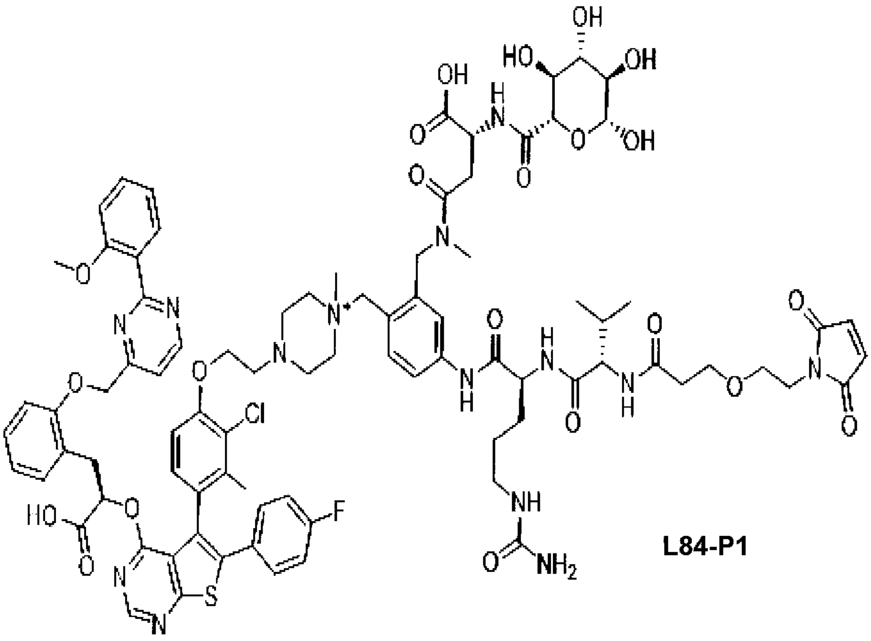

L84-P1

,

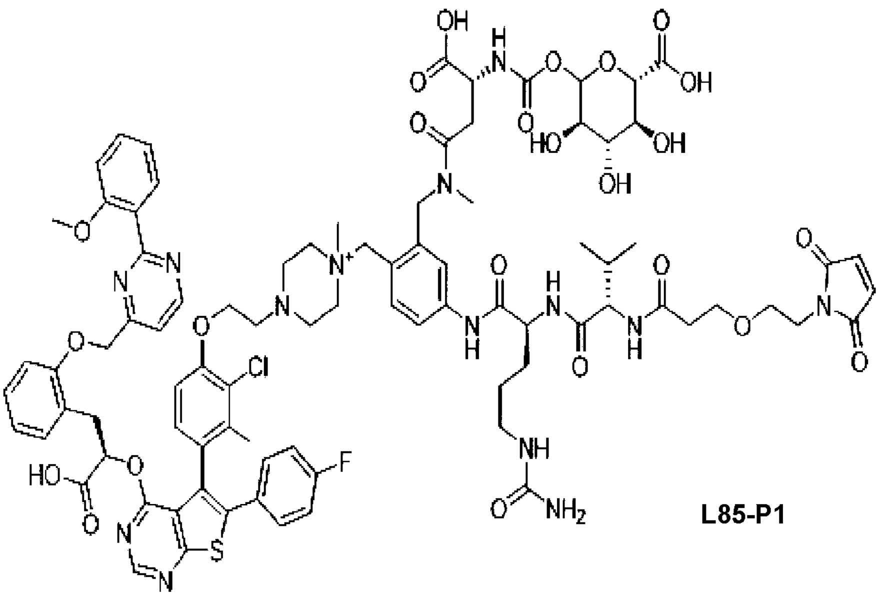

L85-P1

,

| |
|---|
| 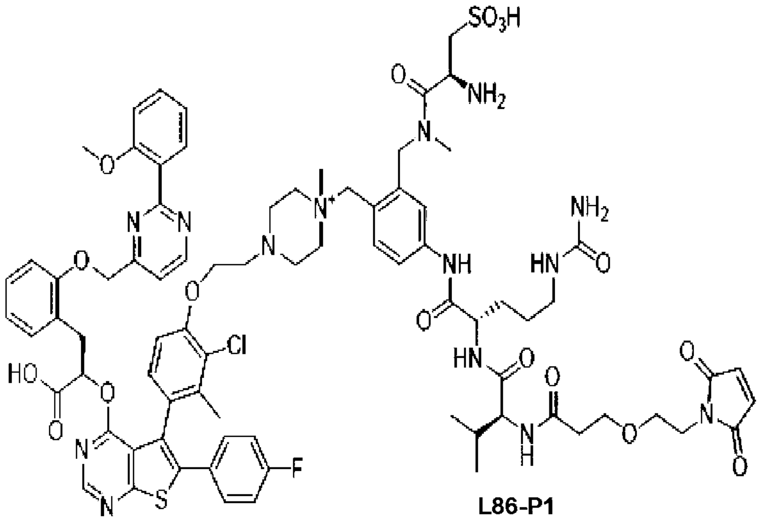 L86-P1 , |
| 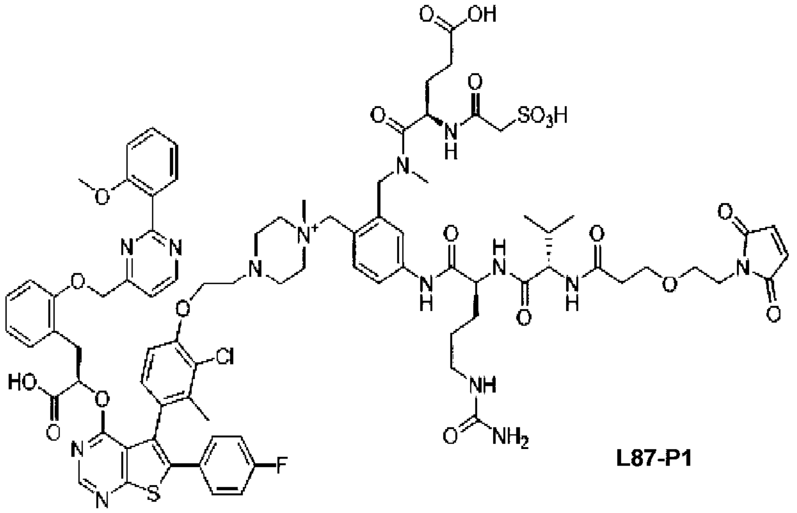 L87-P1 , |
| 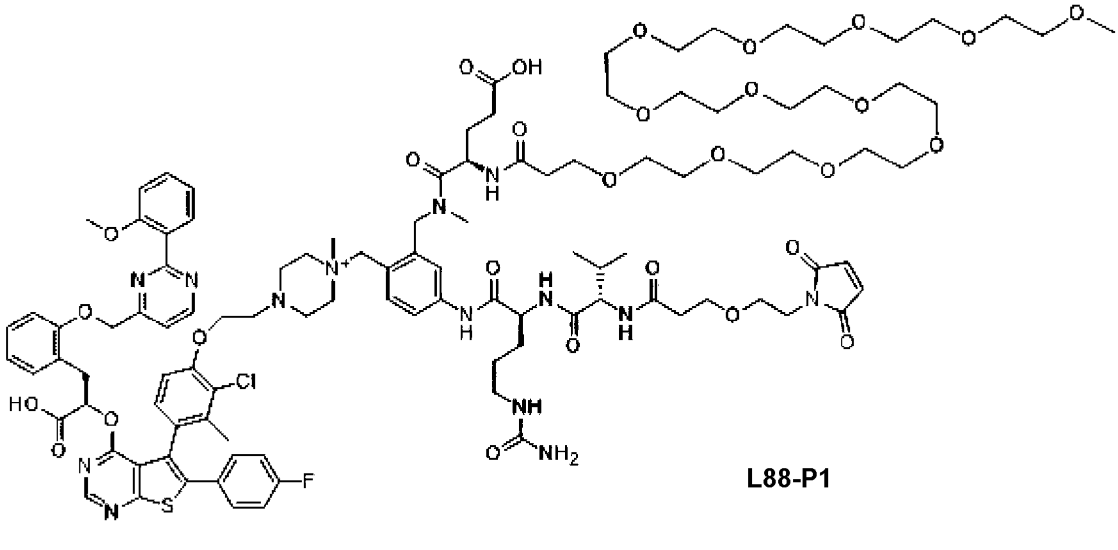 L88-P1 , |

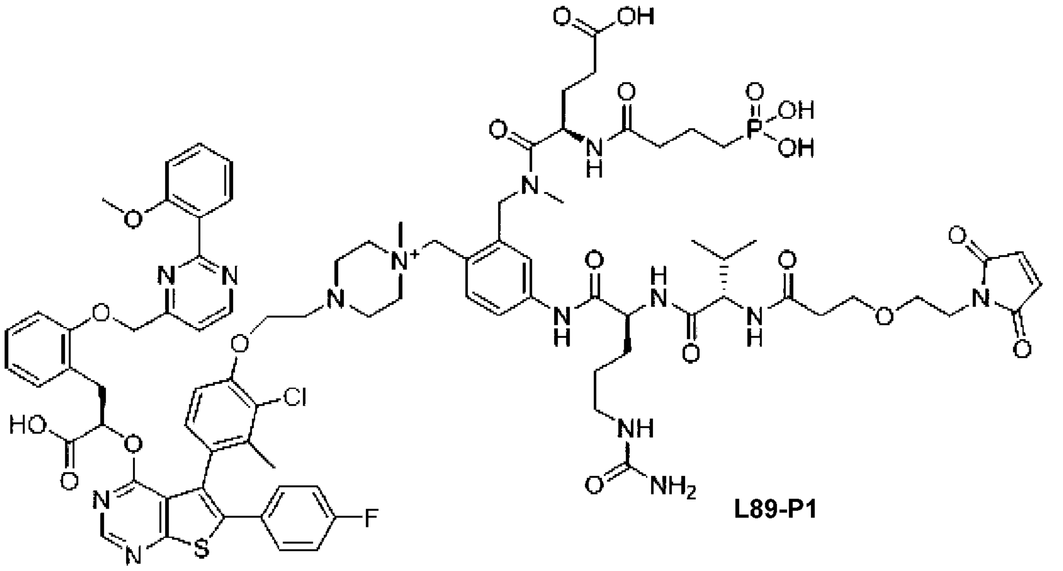

L89-P1

,

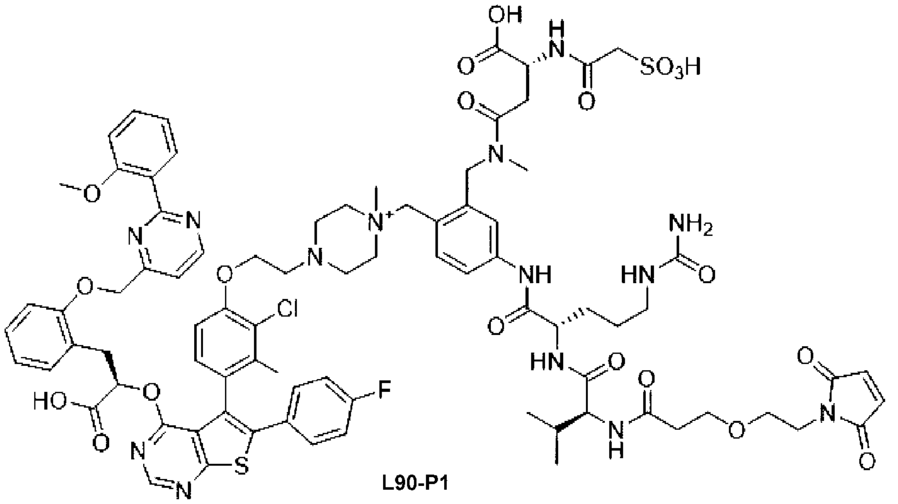

L90-P1

,

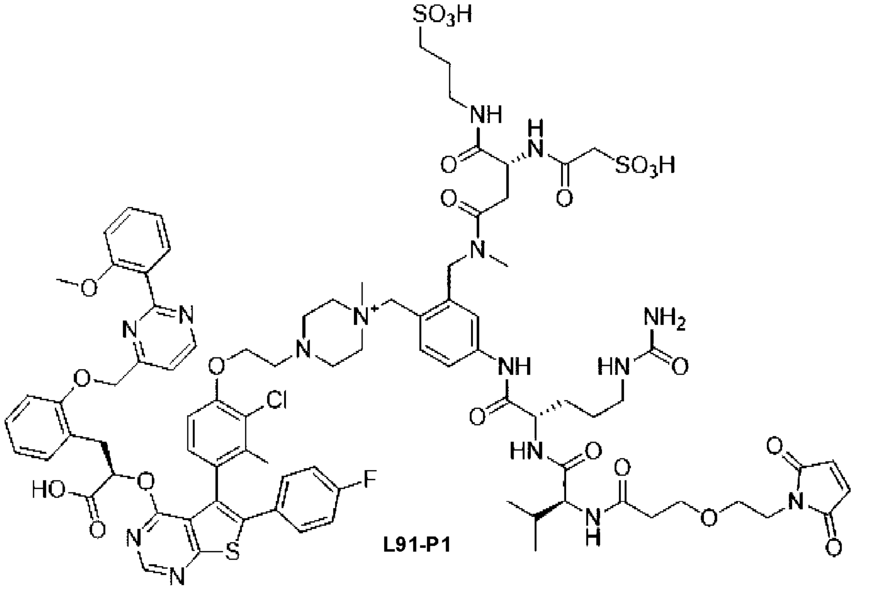

L91-P1

,

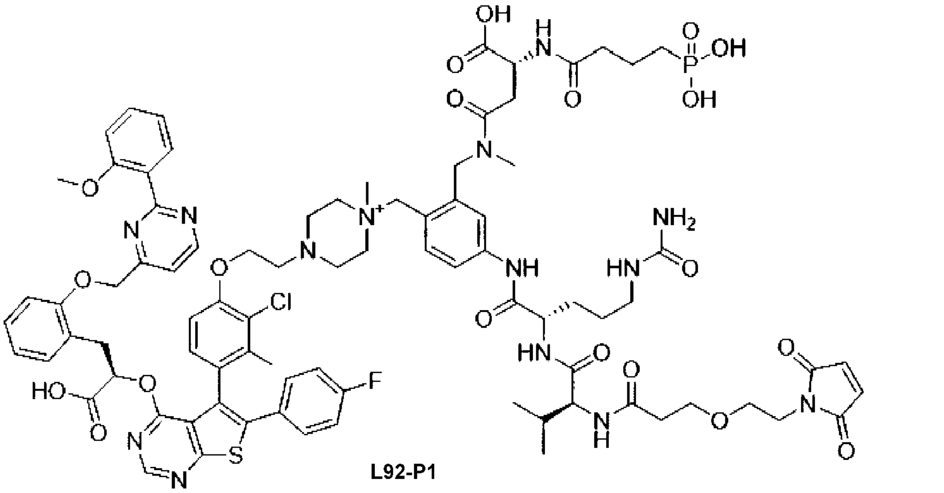

L92-P1

,

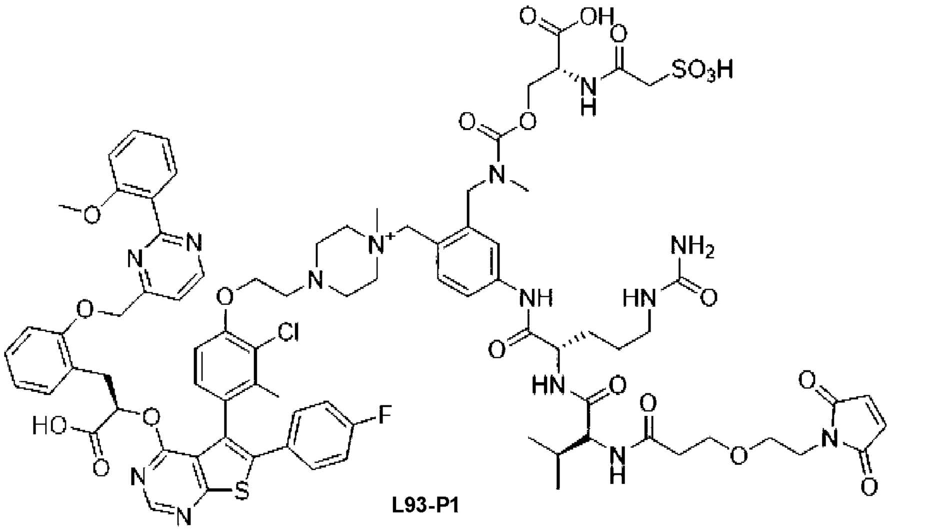

L93-P1

,

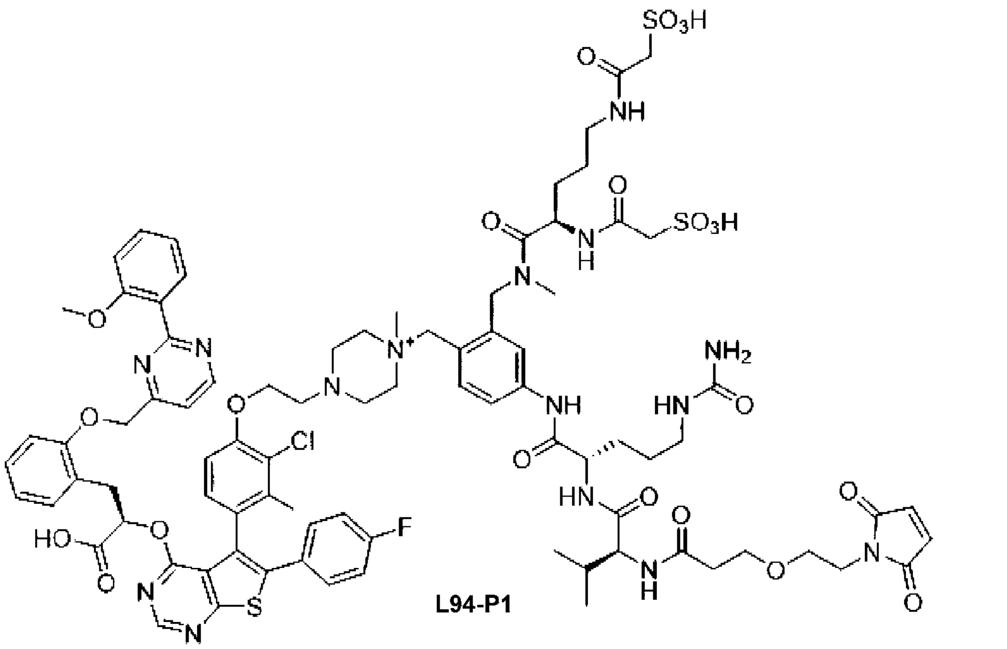

L94-P1

,

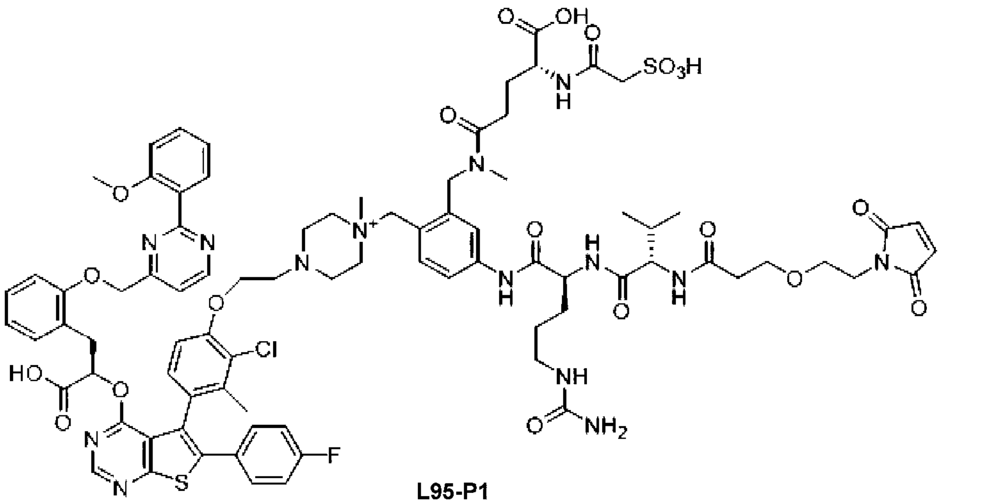

L95-P1

,

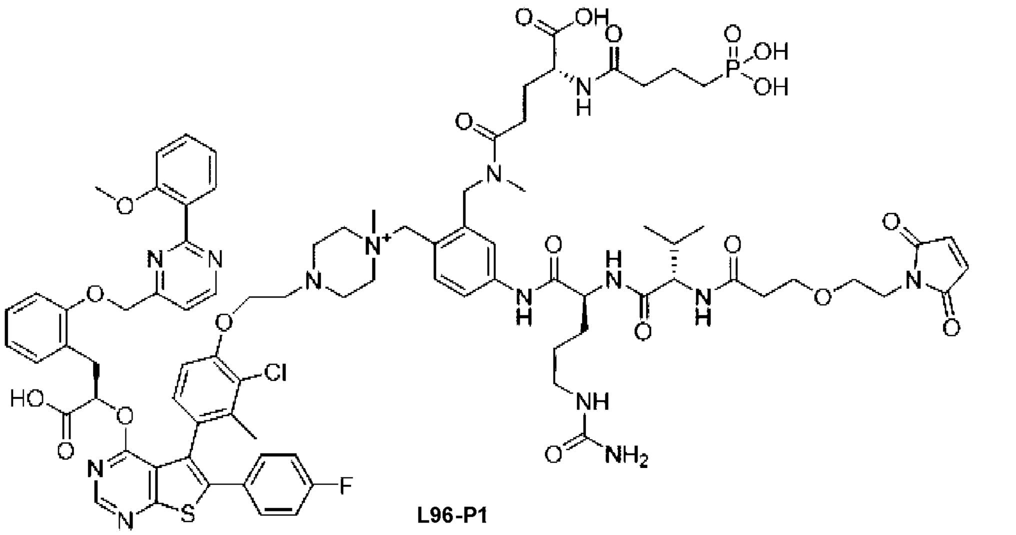

L96-P1

,

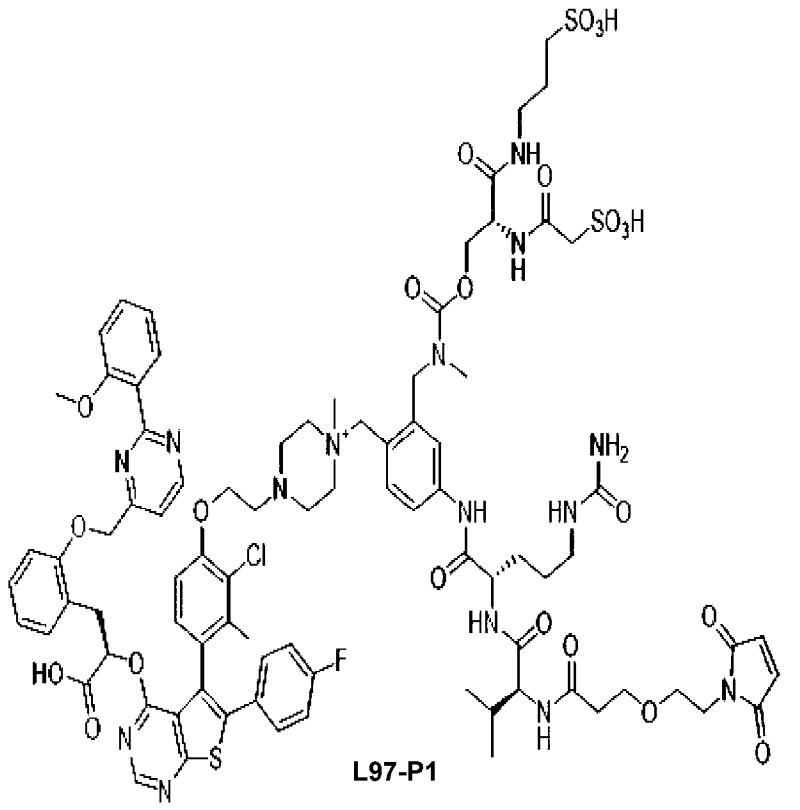

L97-P1

,

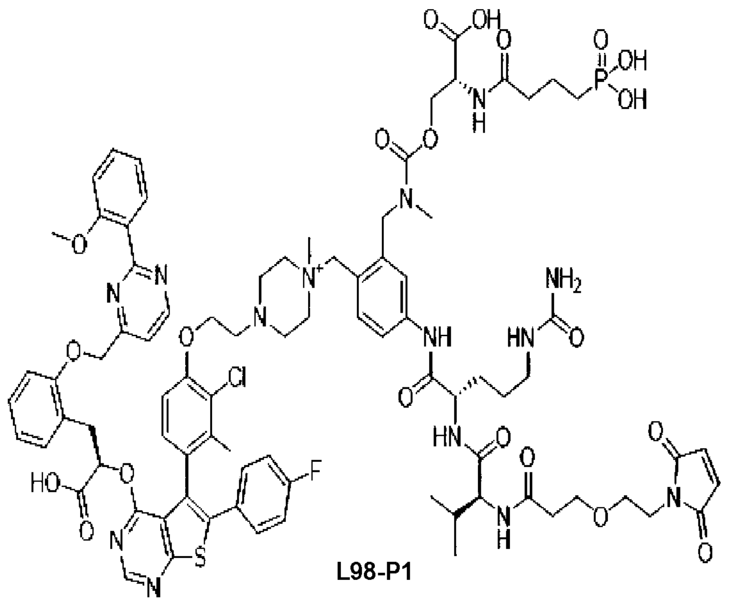

L98-P1

,

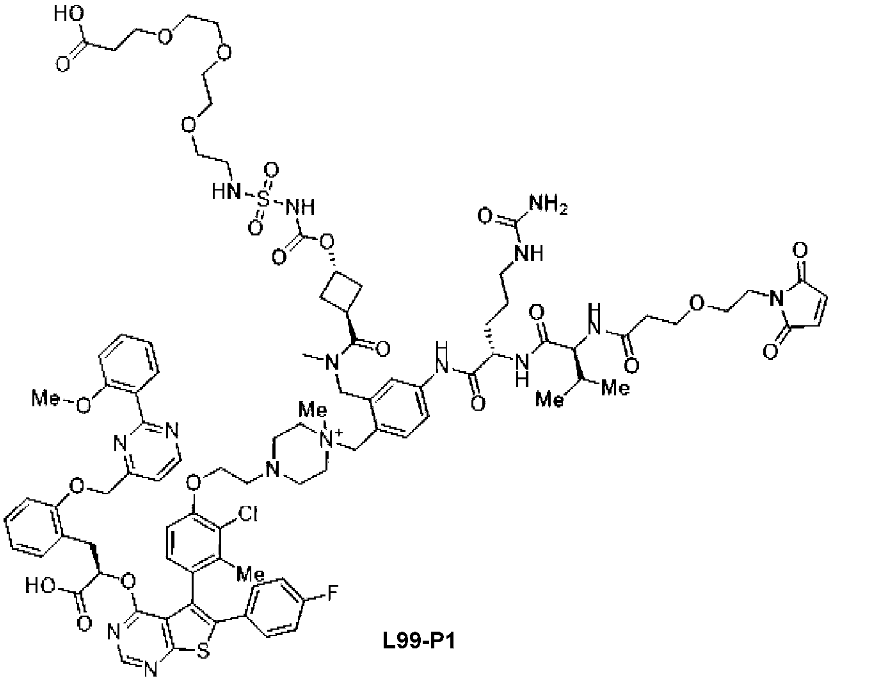

L99-P1

,

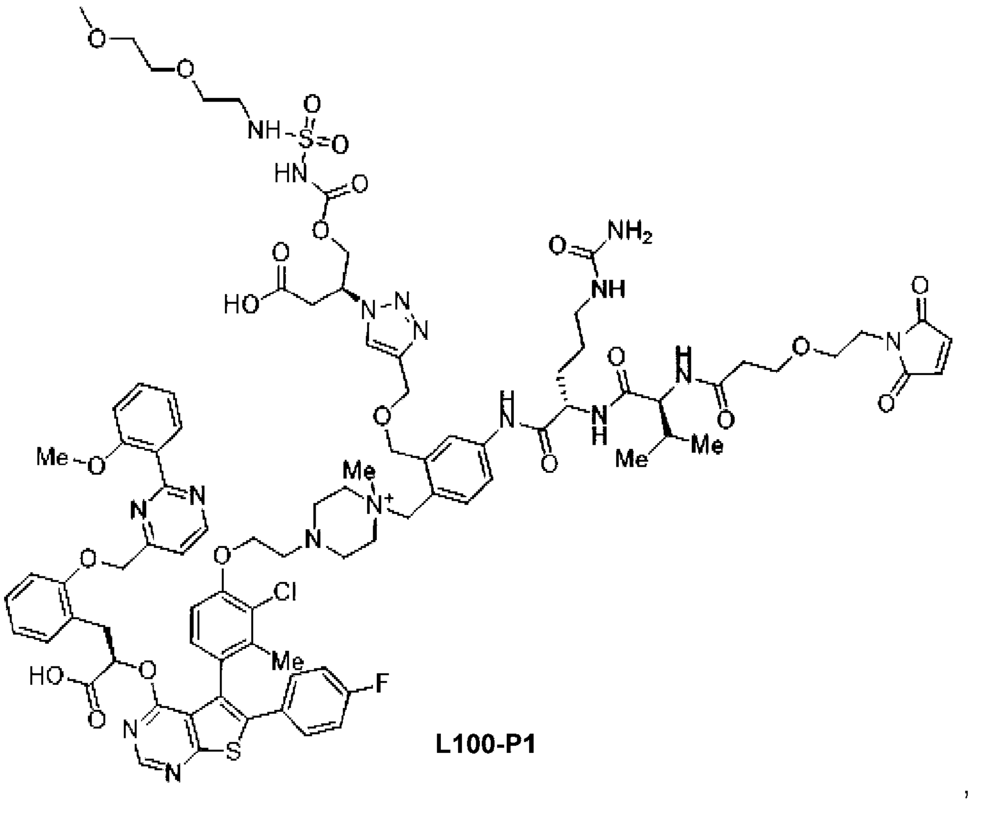

L100-P1

,

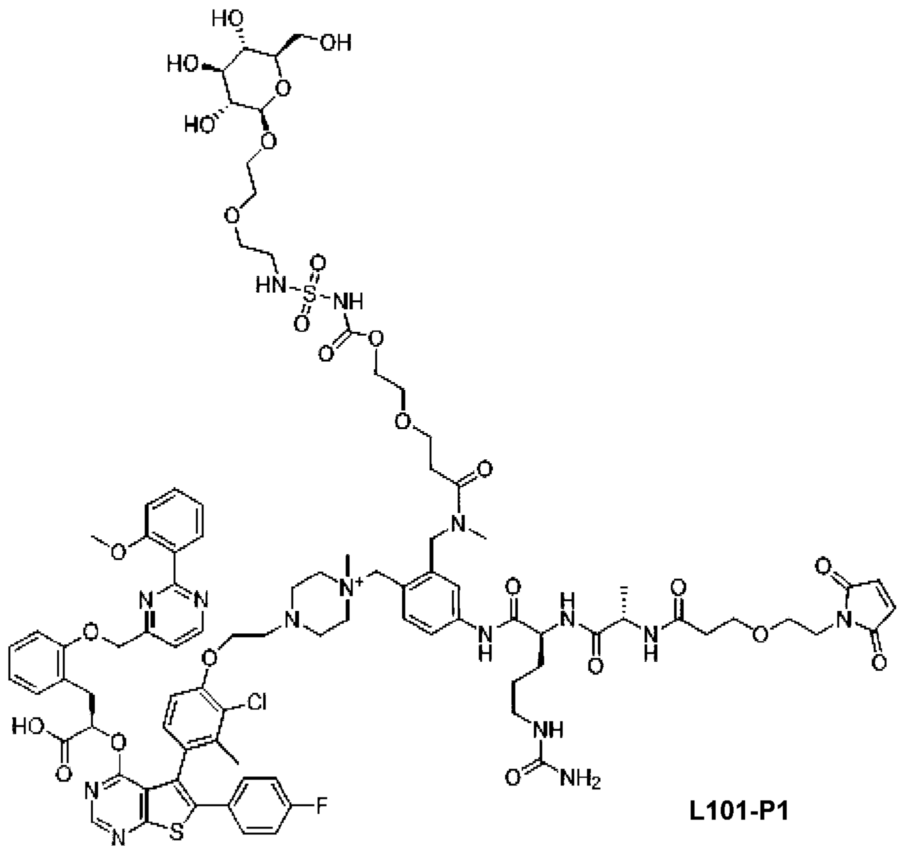

L101-P1

,

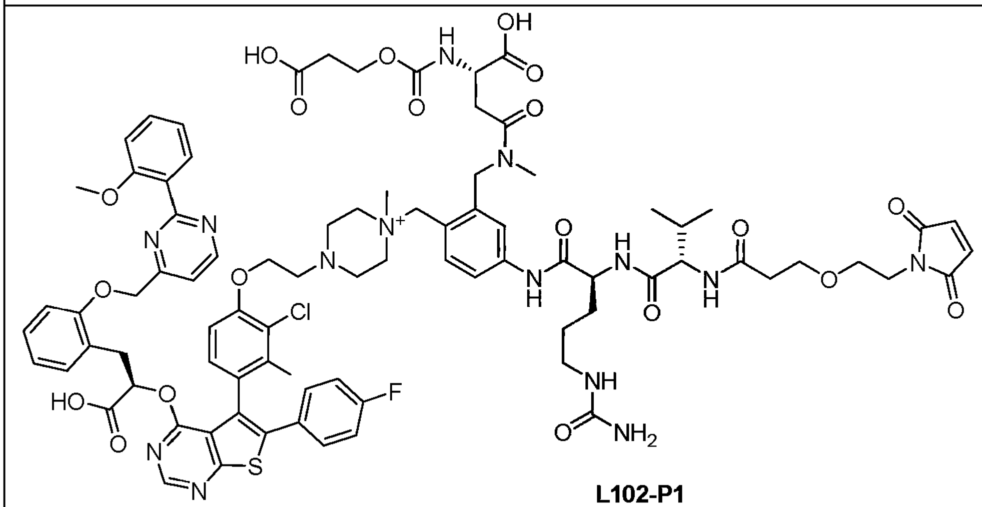

L102-P1

,

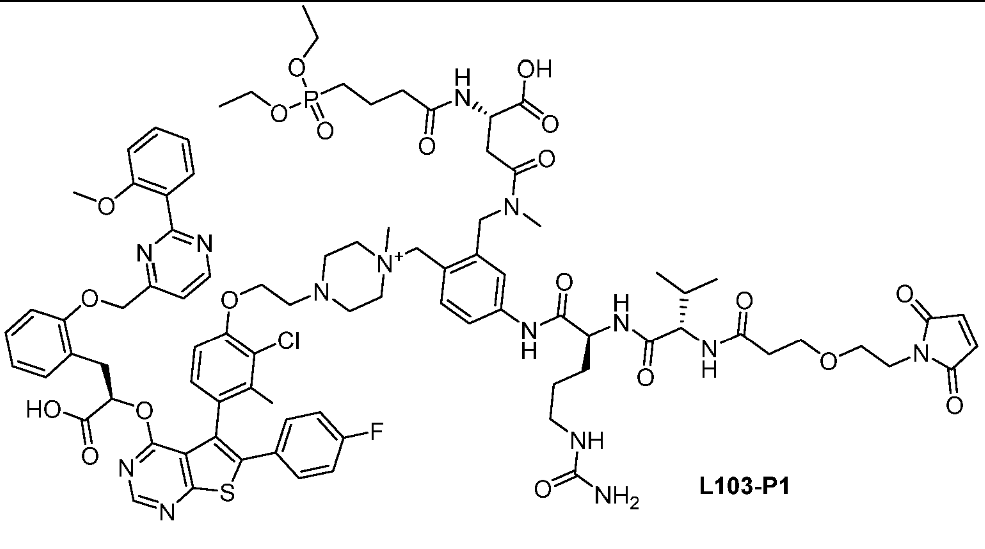

L103-P1

,

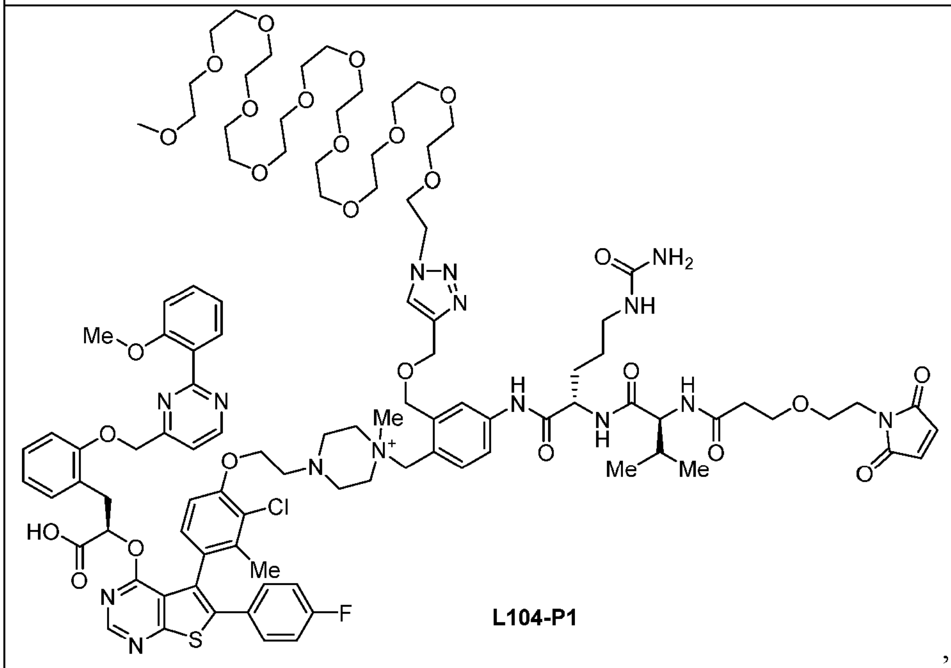

L104-P1

, and

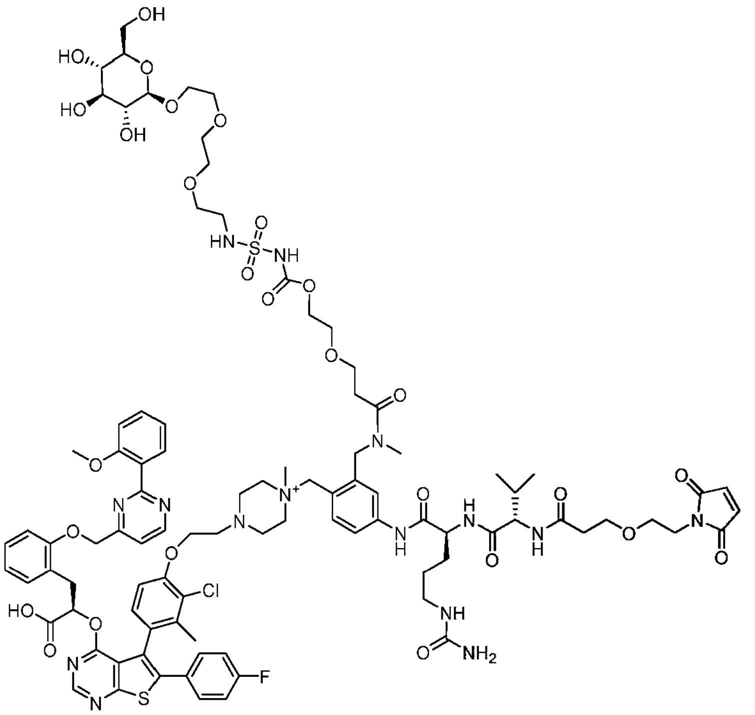

L105-P1

.

In some embodiments, the antibody-drug conjugate has a formula according to any one of the structures shown in Table B.

Table B. ADC Structures

| *ADC Structure* | *L/P Name* |
|---|---|
| 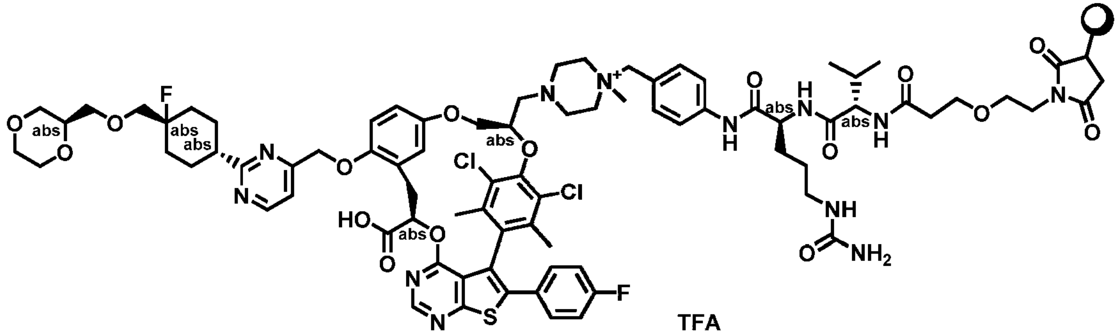 TFA | L9-P16 |
| 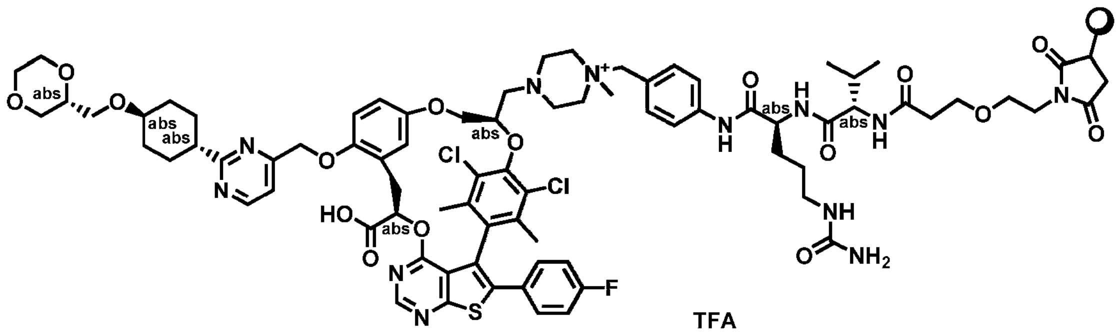 TFA | L9-P17 |

(continued)

| L/P Name | ADC Structure |
|---|---|
| L9-P15 | 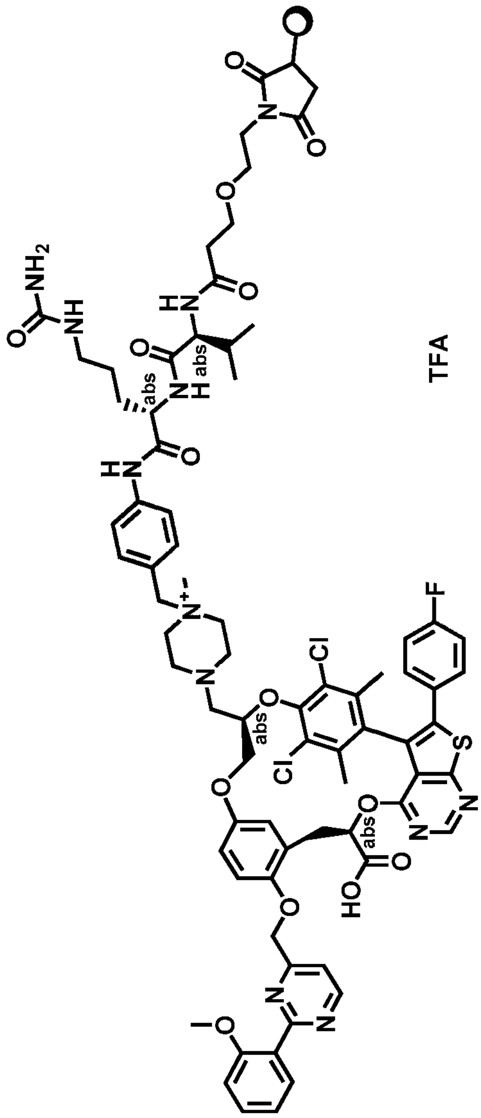 TFA |
| L9-C1 | 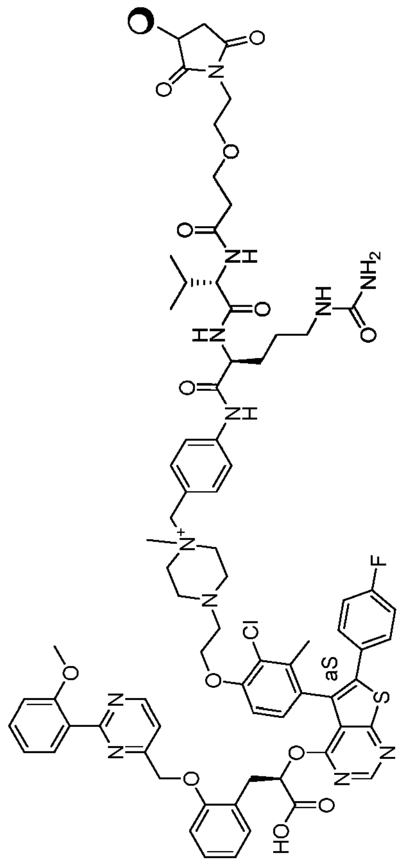 |

(continued)

| ADC Structure | L/P Name |
|---|---|
| 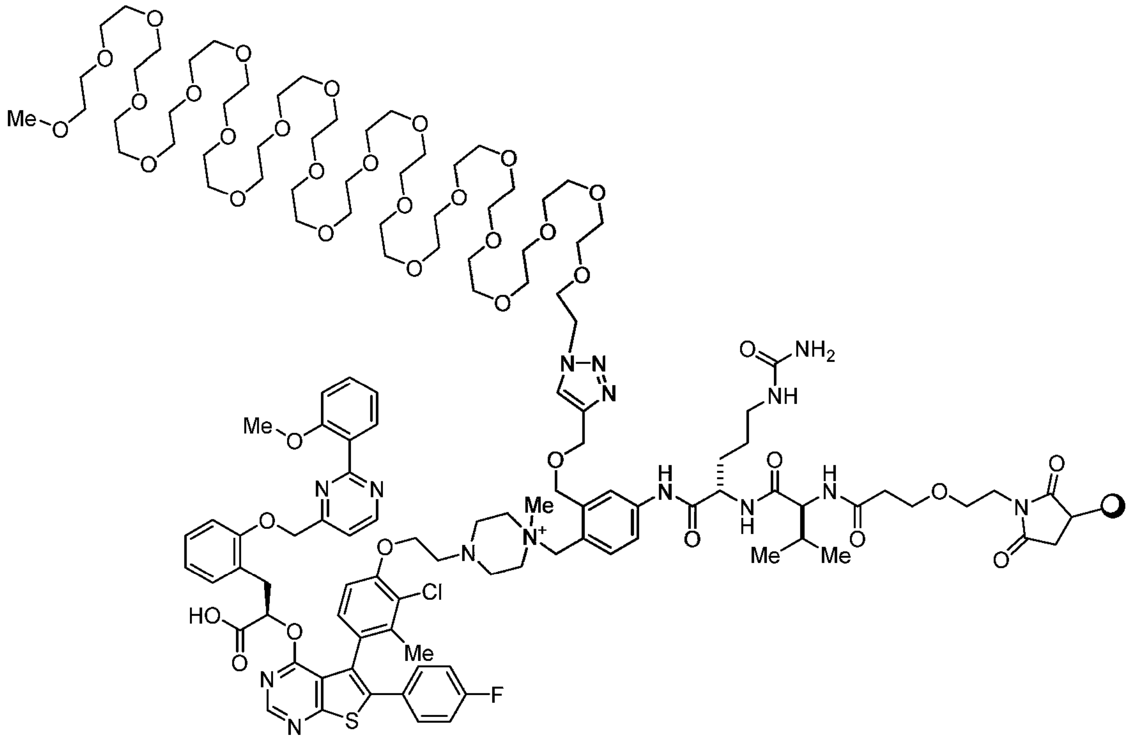 | L5-P1 |

(continued)

| ADC Structure | L/P Name |
|---|---|
| 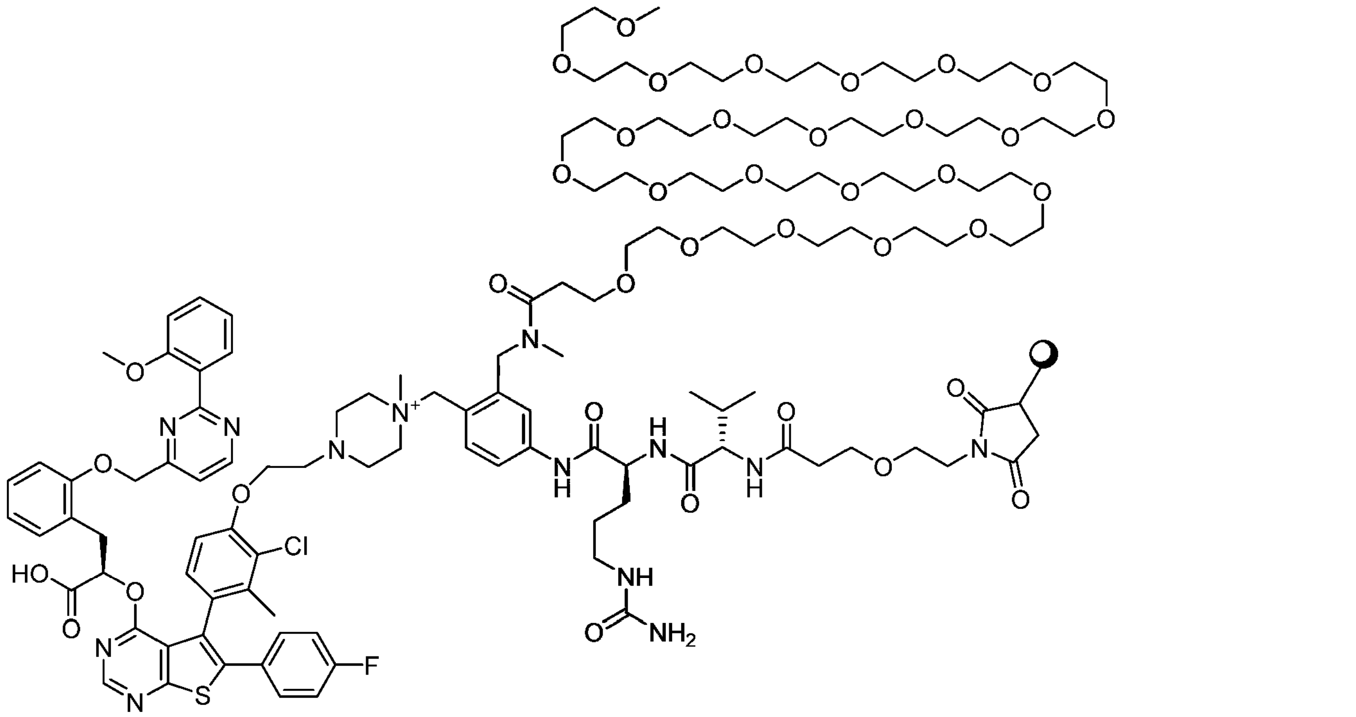 | L30-P1 |

(continued)

| ADC Structure | L/P Name |
|---|---|
| 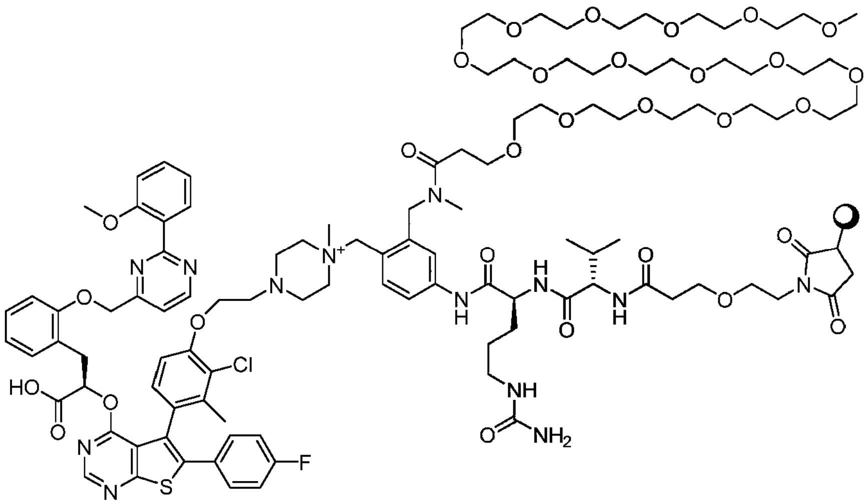 | L31-P1 |
| 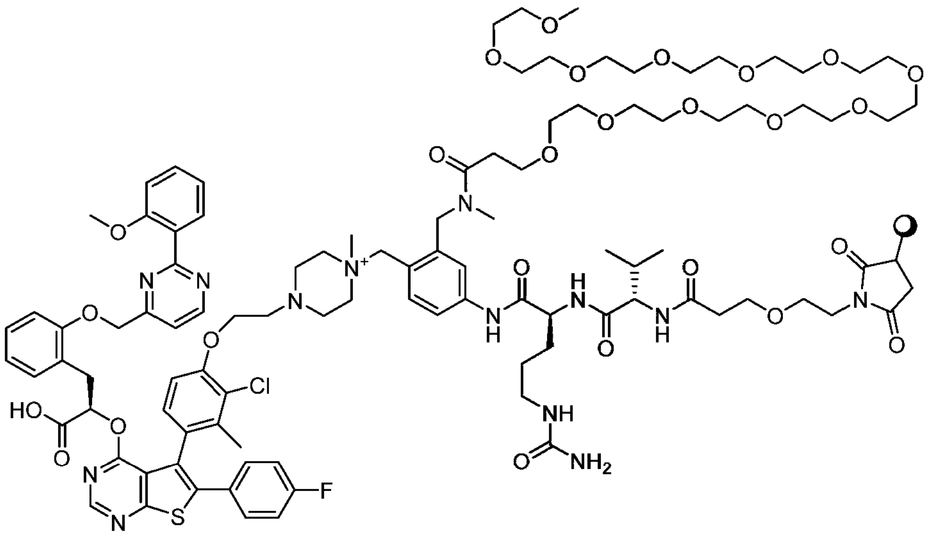 | L32-P1 |

(continued)

| ADC Structure | L/P Name |
|---|---|
| 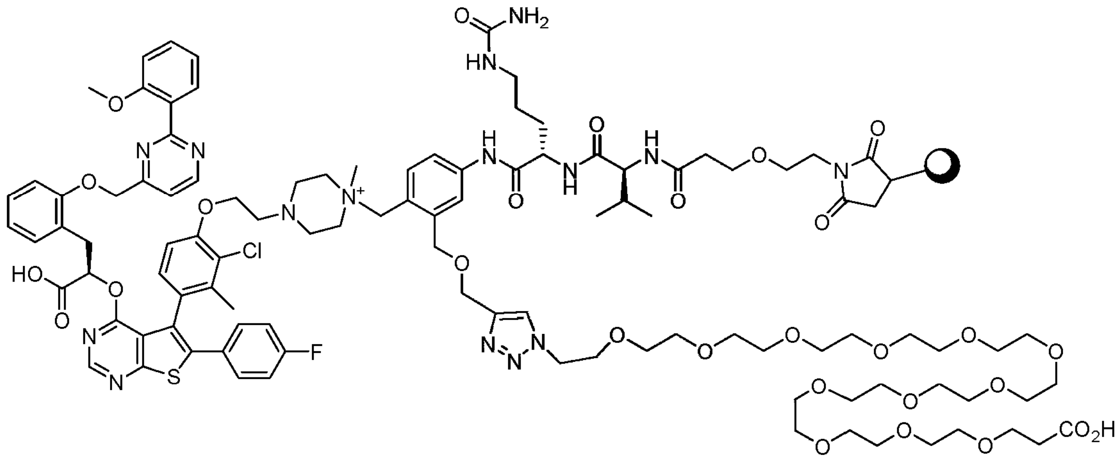 | L33-P1 |
| 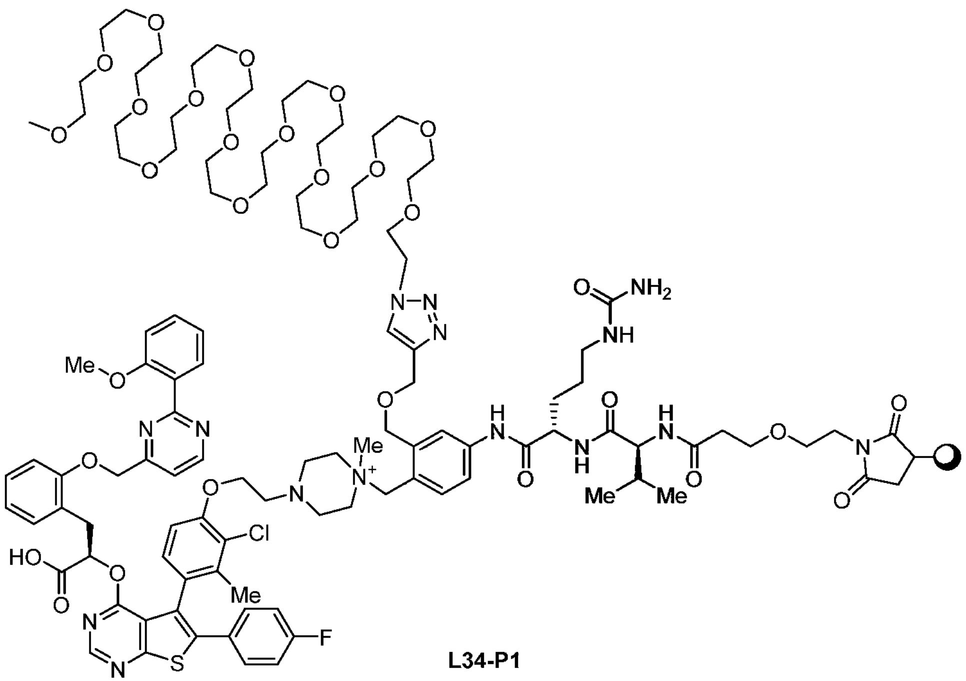 L34-P1 | L34-P1 |

(continued)

| ADC Structure | L/P Name |
|---|---|
| 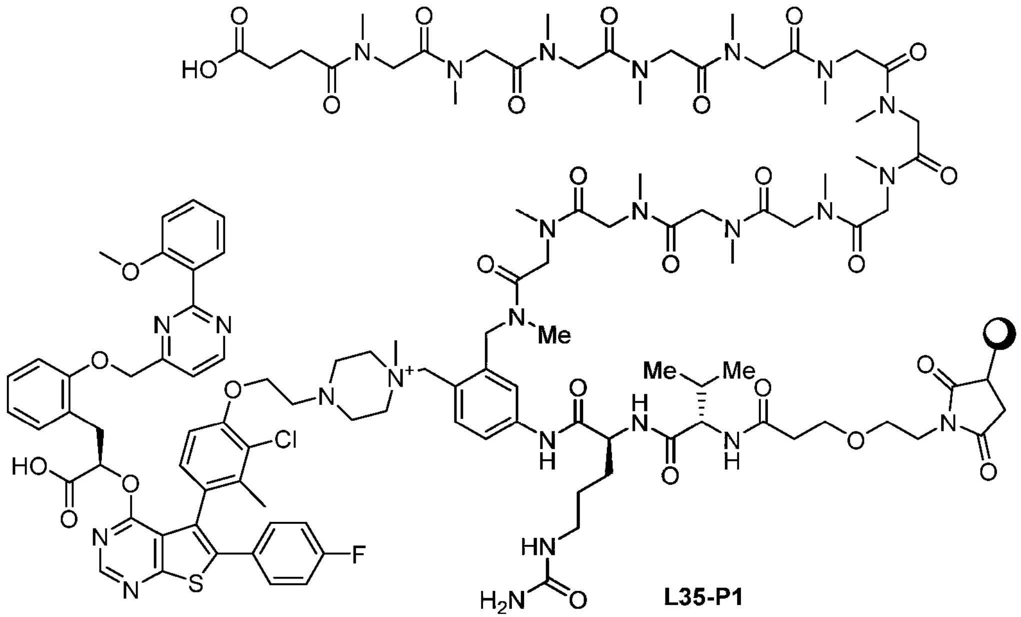 **L35-P1** | L35-P1 |

(continued)

| ADC Structure | L/P Name |
|---|---|
| 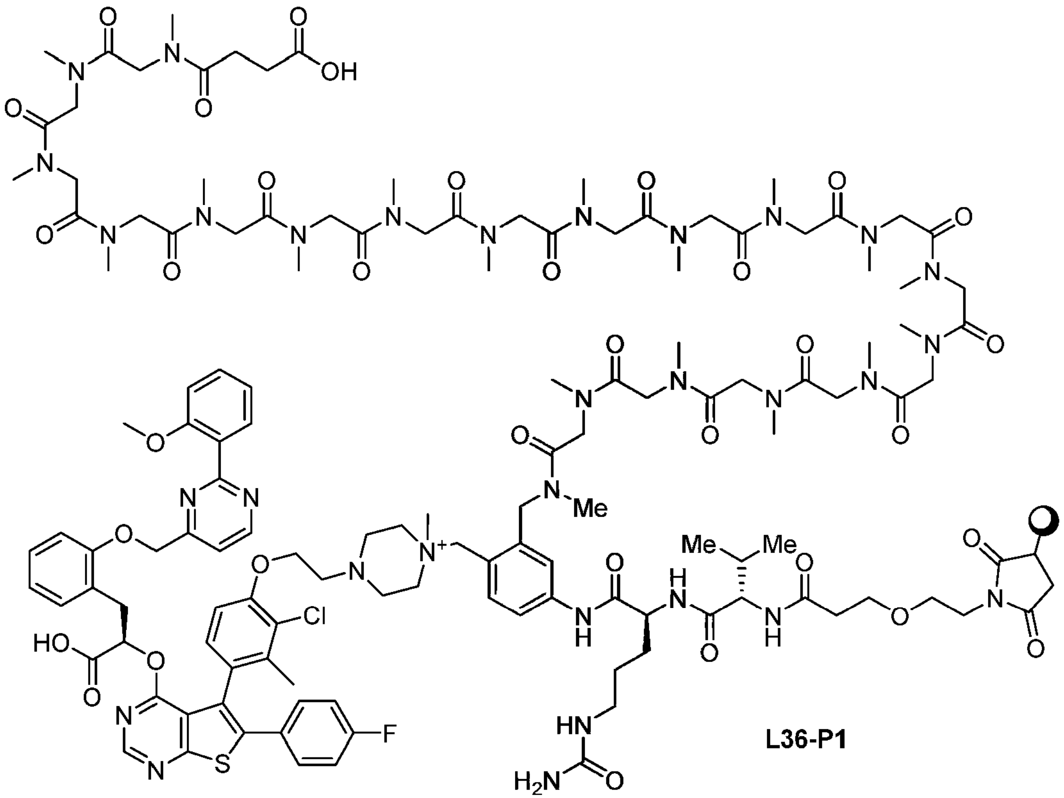 L36-P1 | L36-P1 |

(continued)

| ADC Structure | L/P Name |
| --- | --- |
| 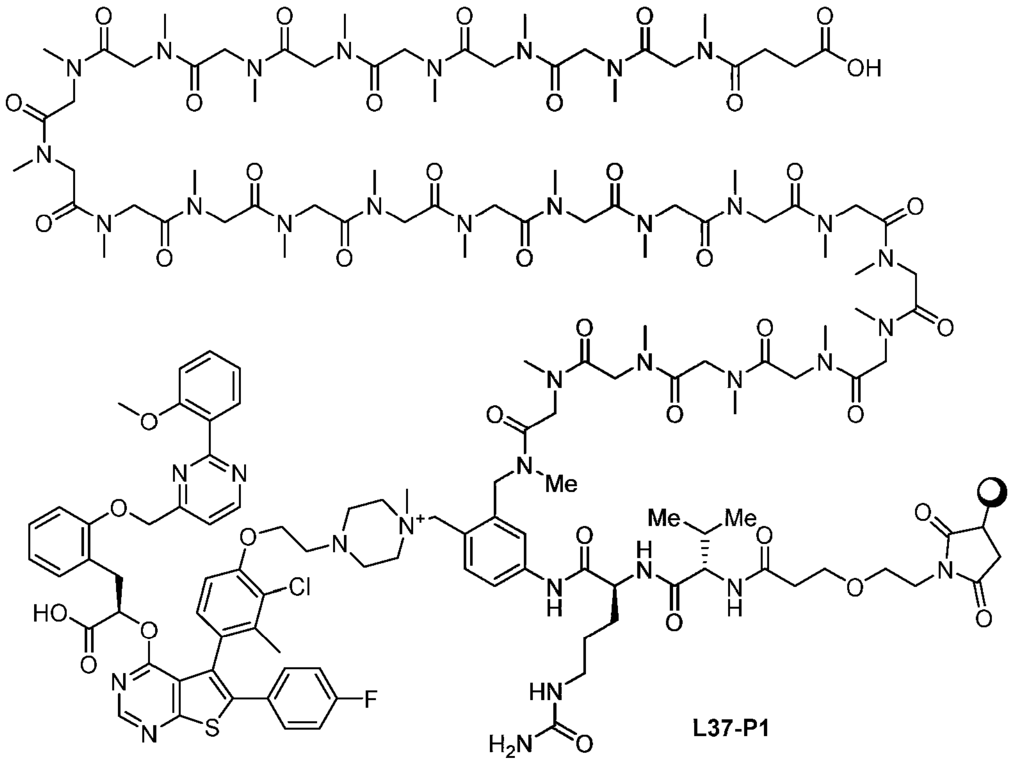 L37-P1 | L37-P1 |

(continued)

| ADC Structure | L/P Name |
| --- | --- |
| 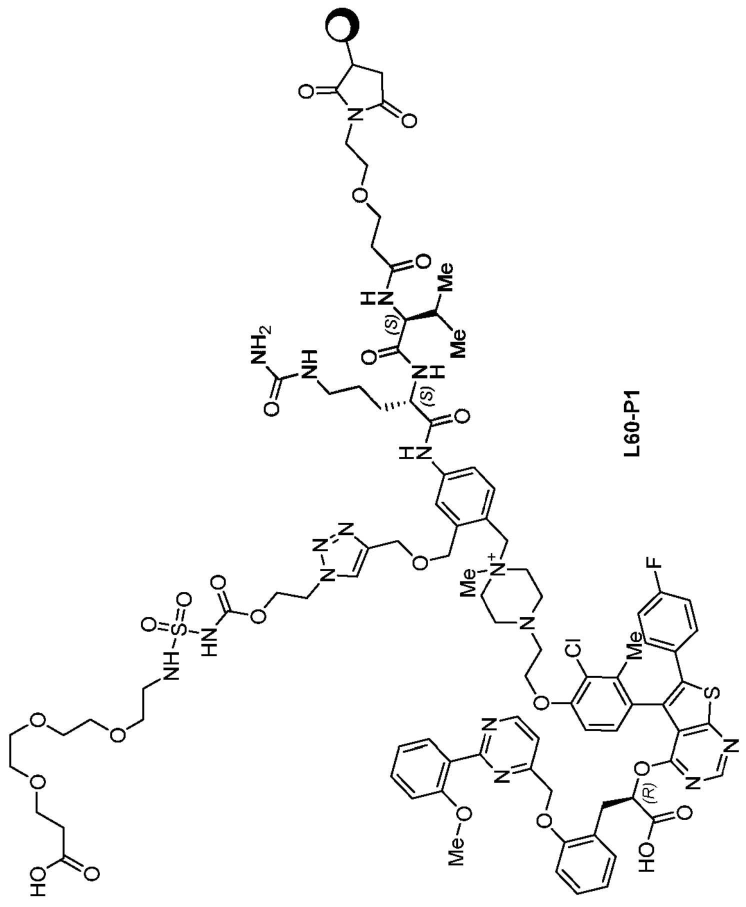 L60-P1 | L60-P1 |

(continued)

| ADC Structure | L/P Name |
|---|---|
| 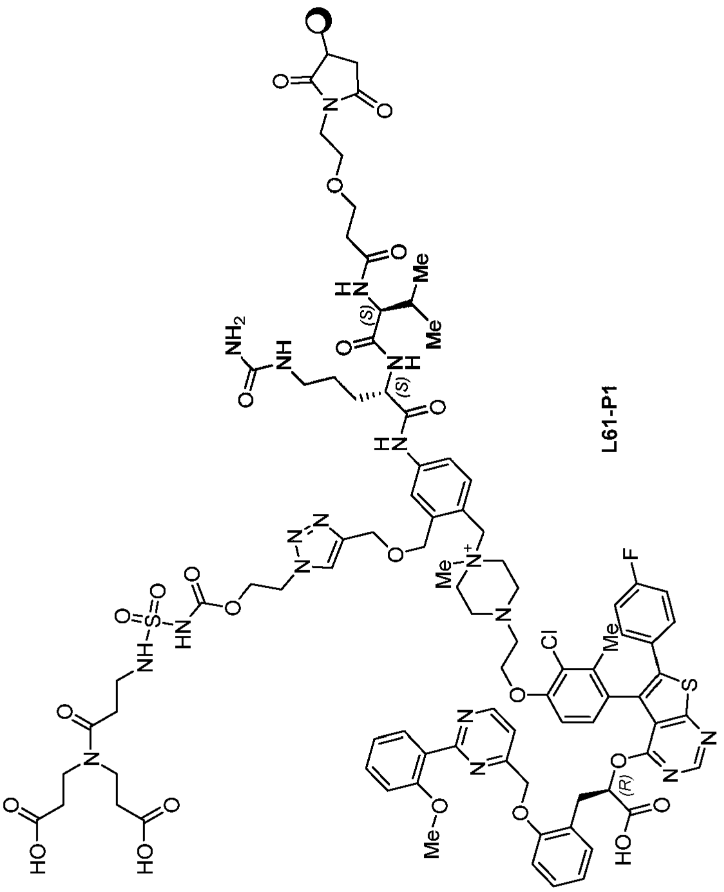 L61-P1 | L61-P1 |

(continued)

| ADC Structure | L/P Name |
| --- | --- |
| 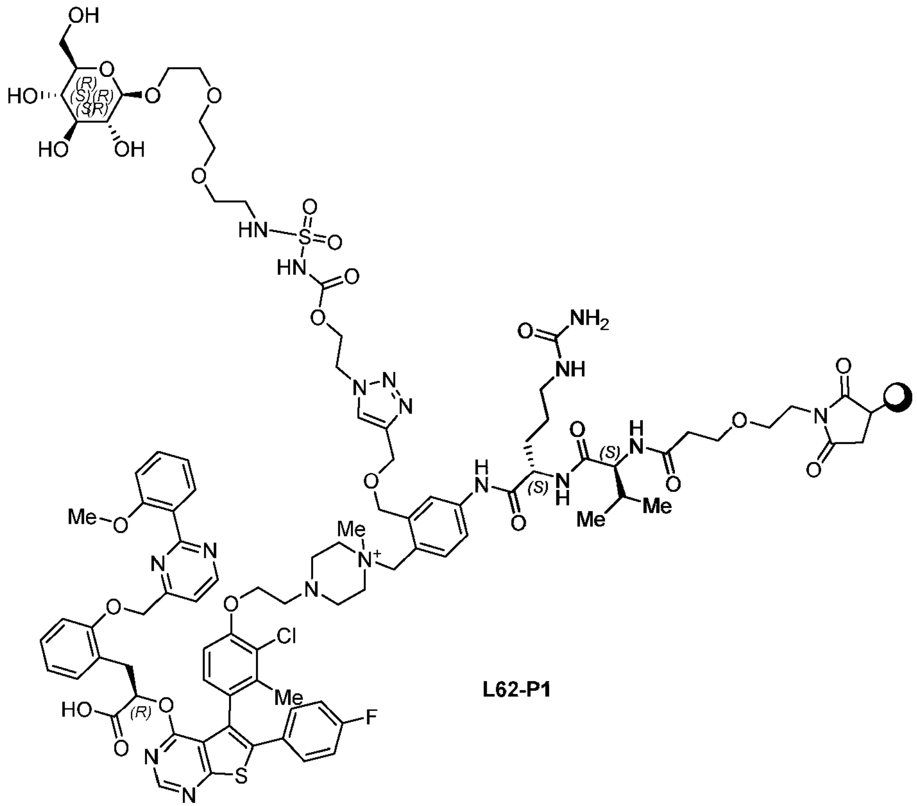 L62-P1 | L62-P1 |

(continued)

| ADC Structure | L/P Name |
|---|---|
| 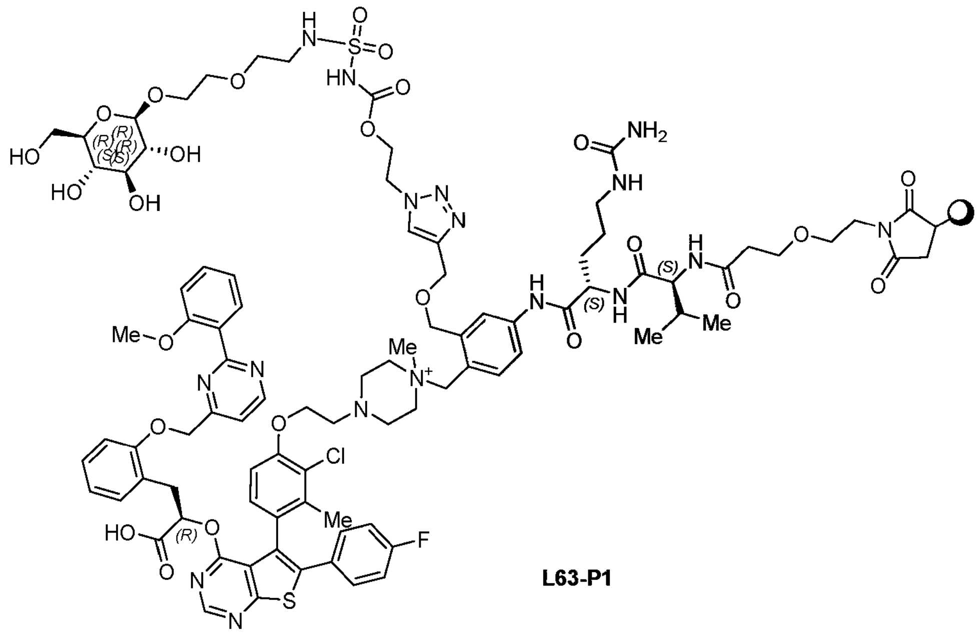 L63-P1 | L63-P1 |

(continued)

| ADC Structure | L/P Name |
|---|---|
| 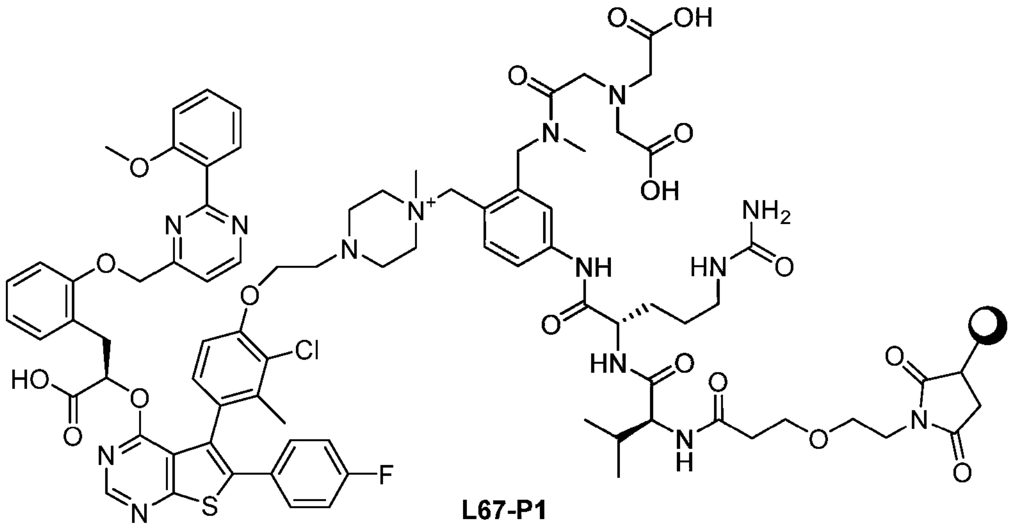 L67-P1 | L67-P1 |
| 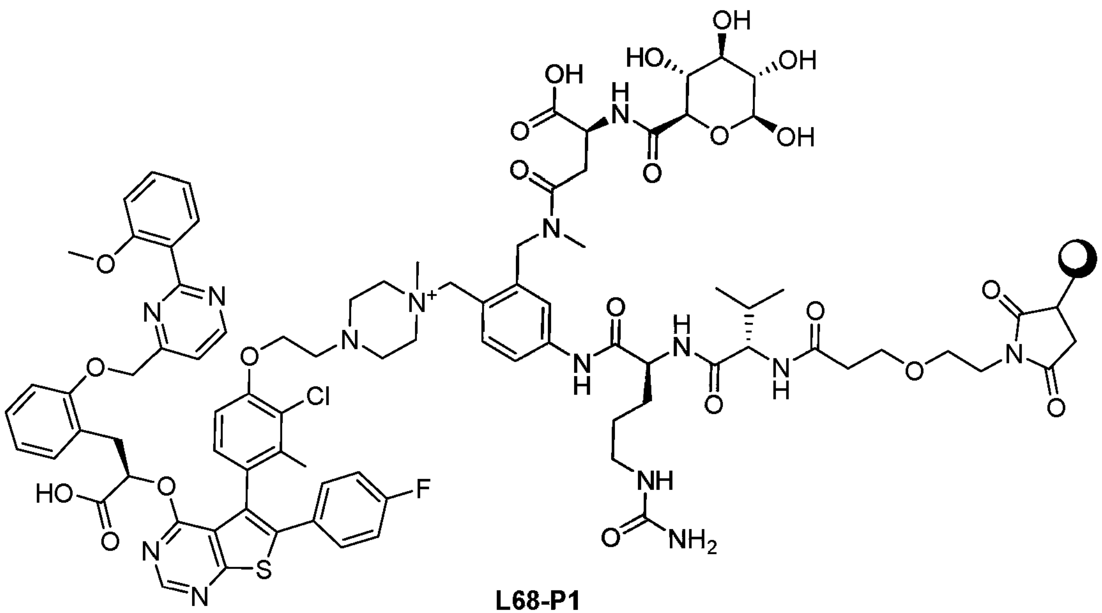 L68-P1 | L68-P1 |

(continued)

| ADC Structure | L/P Name |
| --- | --- |
| 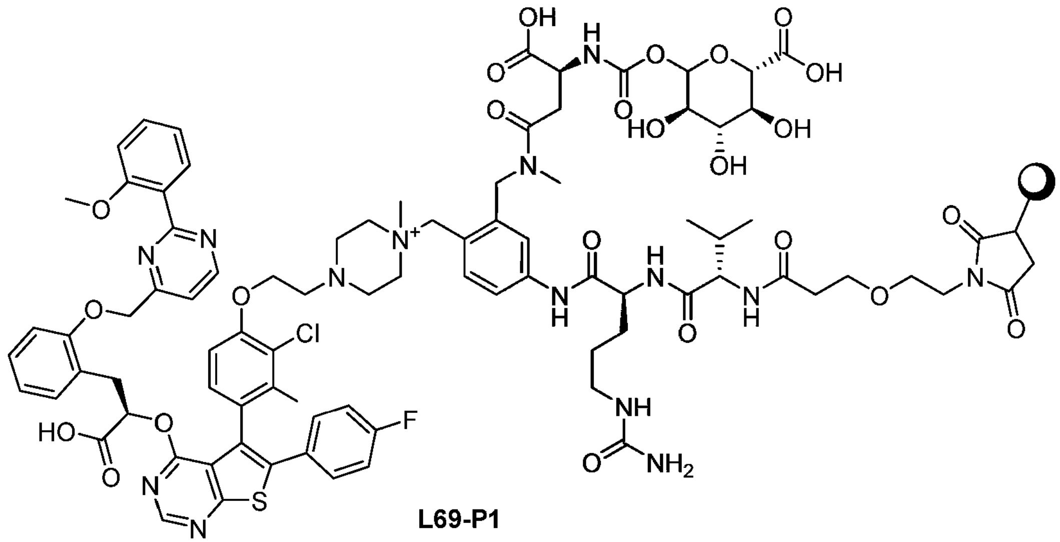 **L69-P1** | L69-P1 |
| 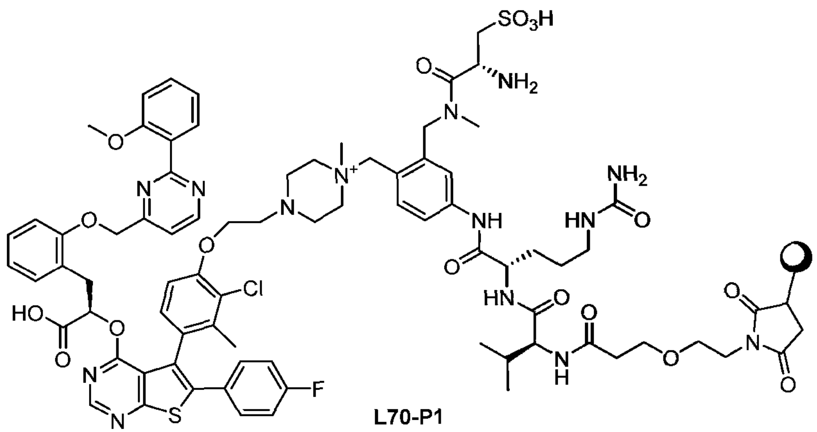 **L70-P1** | L70-P1 |

(continued)

| ADC Structure | L/P Name |
| --- | --- |
| 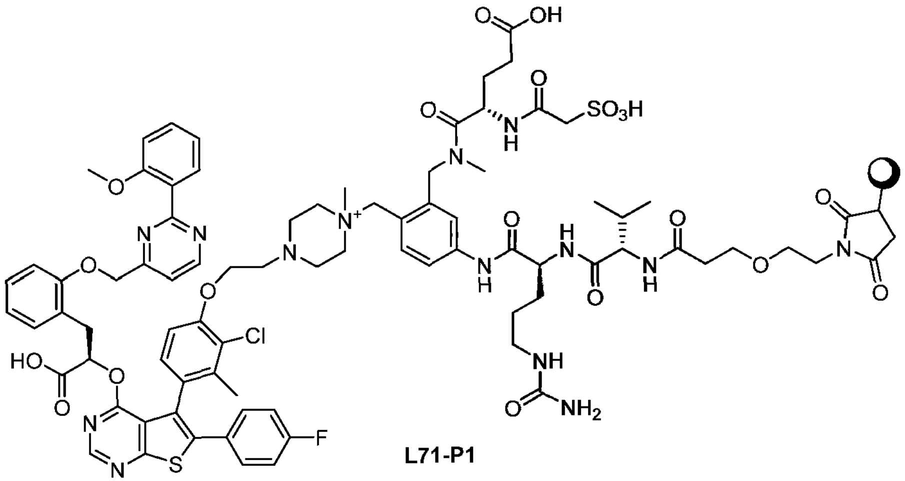 L71-P1 | L71-P1 |
| 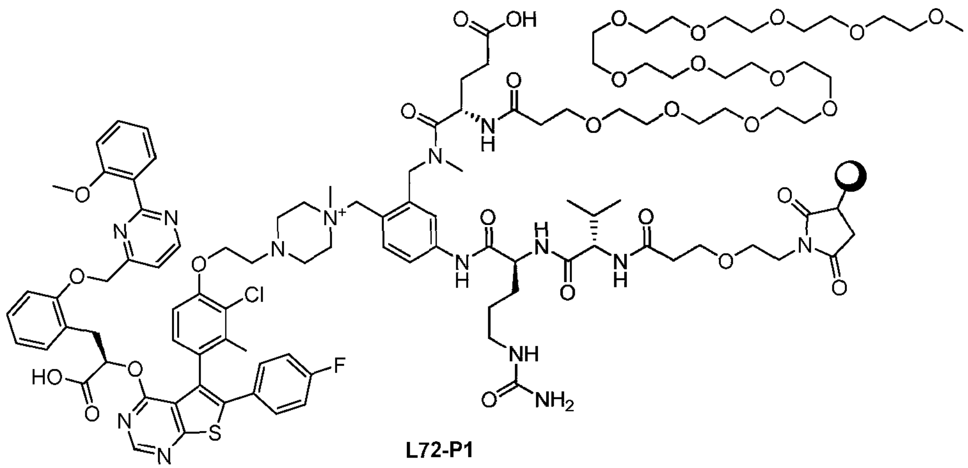 L72-P1 | L72-P1 |

(continued)

| ADC Structure | L/P Name |
|---|---|
| 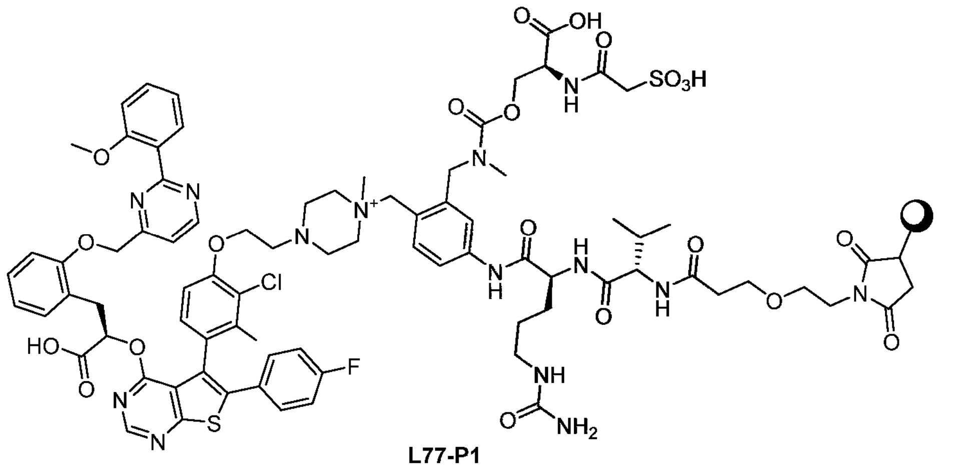 **L77-P1** | L77-P1 |
| 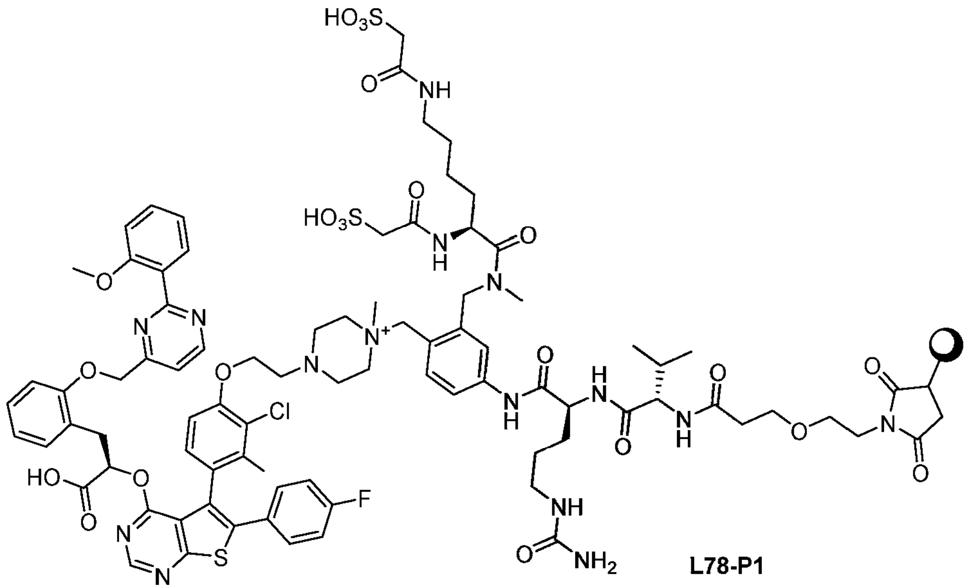 **L78-P1** | L78-P1 |

(continued)

| *ADC Structure* | *L/P Name* |
|---|---|
| 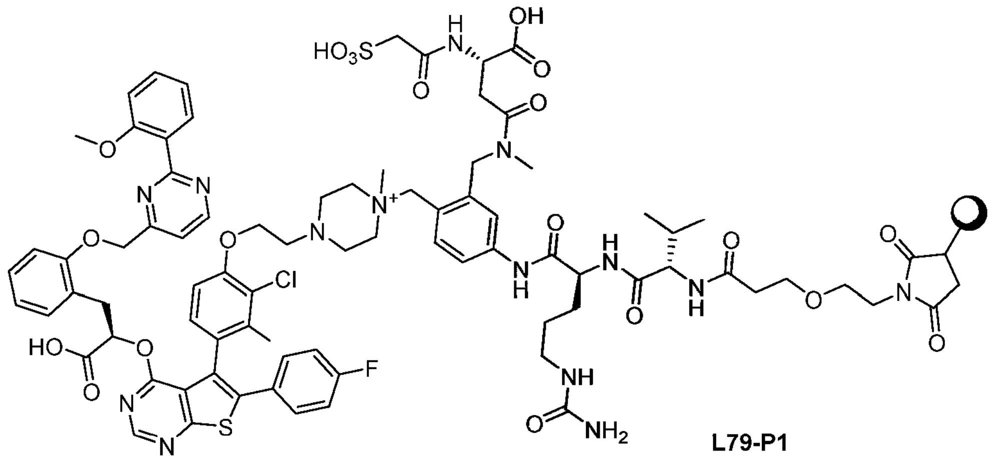 **L79-P1** | L79-P1 |
| 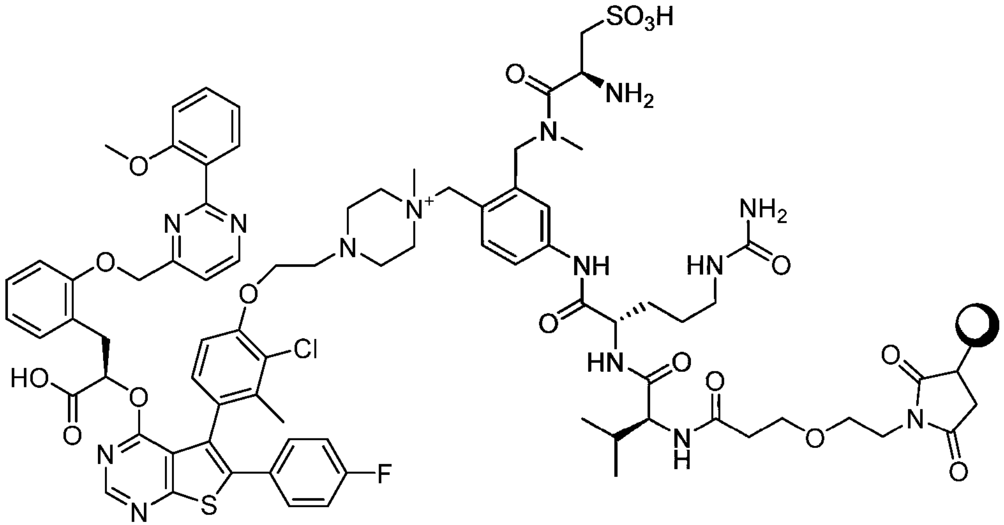 | L86-P1 |
| 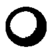 = **anti-CD74 antibody or an antigen-binding fragment thereof** The ADCs depicted above can also be represented by the following formula: Ab-(L-D)$_p$, | |

(continued)

| ***ADC Structure*** | ***L/P Name*** |
|---|---|
| wherein 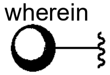 is an anti-CD74 antibody or an antigen-binding fragment thereof covalently linked to the linker-payload (L/P) depicted above; p is an integer from 1 to 16. In some embodiments, *p* is an integer from 1 to 8. In some embodiments, *p* is an integer from 1 to 5. In some embodiments, p is an integer from 2 to 4. In some embodiments, *p* is 2. In some embodiments, p is 4. In some embodiments, *p* is determined by liquid chromatography-mass spectrometry (LC-MS). | |

As used herein, "L/P" refers to the linker-payloads, linker-drugs, or linker-compounds disclosed herein and the terms "L#-P#" and "L#-C#" are used interchangeably to refer to a specific linker-drug disclosed herein, while the codes "P#" and "C#" are used interchangeably to refer to a specific compound unless otherwise specified. For example, both "L1-C1" and "L1-P1" refer to the same linker-payload structure disclosed herein, while both "C1" and "P1" indicate the same compound disclosed herein, including an enantiomer, diastereoisomer, atropisomer, deuterated derivative, and/or pharmaceutically acceptable salt of any of the foregoing.

In some embodiments, the antibody or antigen-binding fragment binds to the target antigen CD74 on a cancer cell. In some embodiments, CD74 is a human CD74 isoform. In some embodiments, the human CD74 isoform is isoform 1 (NP_001020330.1) having an amino acid sequence of:

MHRRRSRSCREDQKPVMDDQRDLISNNEQLPMLGRRPGAPESKCSRGALYTGFSILVTLL
LAGQATTAYFLYQQQGRLDKLTVTSQNLQLENLRMKLPKPPKPVSKMRMATPLLMQALPM
GALPQGPMQNATKYGNMTEDHVMHLLQNADPLKVYPPLKGSFPENLRHLKNTMETIDWKV
FESWMHHWLLFEMSRHSLEQKPTDAPPKVLTKCQEEVSHIPAVHPGSFRPKCDENGNYLP
LQCYGSIGYCWCVFPNGTEVPNTRSRGHHNCSESLELEDPSSGLGVTKQDLGPVPM
(SEQ ID NO:61).

In some embodiments, the human CD74 isoform is isoform 2 (NP_004346.1) having an amno acid sequence of:

MHRRRSRSCREDQKPVMDDQRDLISNNEQLPMLGRRPGAPESKCSRGALYTGFSILVTLL
LAGQATTAYFLYQQQGRLDKLTVTSQNLQLENLRMKLPKPPKPVSKMRMATPLLMQALPM
GALPQGPMQNATKYGNMTEDHVMHLLQNADPLKVYPPLKGSFPENLRHLKNTMETIDWKV
FESWMHHWLLFEMSRHSLEQKPTDAPPKESLELEDPSSGLGVTKQDLGPVPM (SEQ ID
NO:62).

In a third aspect of the present invention, there is provided compositions comprising multiple copies of an antibody-drug conjugate of the first and second aspect, wherein the average *p* of the antibody-drug conjugates in the composition is from about 2 to about 16, e.g., about 2 to about 8, e.g., about 2 to about 4. In some embodiments, the average *p* of the antibody-drug conjugates in the composition is from about 2 to about 4.

In a fourth aspect of the present invention, there is provided pharmaceutical compositions comprising an antibody-drug conjugate of the first and second aspect or a composition of the third aspect, and a pharmaceutically acceptable carrier.

Further provided herein, in some embodiments, are therapeutic uses for the described ADC compounds and compositions, e.g., in treating a cancer.

In a the fifth aspect, there is provided an antibody-drug conjugate of the first and second aspect, a composition of the third aspect, or pharmaceutical composition of the fourth aspect, for use in treating a subject having or suspected of having a cancer. In some embodiments, the cancer expresses the target antigen CD74. In some embodiments, the cancer is a tumor or a hematological cancer. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer.

In a sixth aspect, there is provided an antibody-drug conjugate of the first and second aspect, a composition of the third aspect, or pharmaceutical composition of the fourth aspect, for use in reducing or inhibiting the growth of a tumor in a subject. In some embodiments, the tumor expresses the target antigen CD74. In some embodiments, the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer. In some embodiments, the tumor is a gastric cancer. In some embodiments, administration of the antibody-drug conjugate, composition, or pharmaceutical composition reduces or inhibits the growth of the tumor by at least about 10%, at least about 20%, at least about 30%, at least about 40%, at least about 50%, at least about 60%, at least about 70%, at least about 80%, at least about 90%, at least about 95%, or at least about 99%.

In a seventh aspect, there is provided an antibody-drug conjugate of the first and second aspect, a composition of the third aspect, or pharmaceutical composition of the fourth aspect, for use in reducing or slowing the expansion of a cancer cell population in a subject (e.g., any of the exemplary antibody-drug conjugates, compositions, or pharmaceutical compositions disclosed herein). In some embodiments, the cancer cell population expresses the target antigen CD74. In some embodiments, the cancer cell population is from a tumor or a hematological cancer. In some embodiments, the cancer cell population is from a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer cell population is from a lymphoma or gastric cancer. In some embodiments, administration of the antibody-drug conjugate, composition, or pharmaceutical composition reduces the cancer cell population by at least about 10%, at least about 20%, at least about 30%, at least about 40%, at least about 50%, at least about 60%, at least about 70%, at least about 80%, at least about 90%, at least about 95%, or at least about 99%. In some embodiments, administration of the antibody-drug conjugate, composition, or pharmaceutical composition slows the expansion of the cancer cell population by at least about 10%, at least about 20%, at least about 30%, at least about 40%, at least about 50%, at least about 60%, at least about 70%, at least about 80%, at least about 90%, at least about 95%, or at least about 99%.

In a eighth aspect, there is provided a method of determining whether a subject having or suspected of having a cancer will be responsive to treatment with an antibody-drug conjugate of the first and second aspect, composition of the third aspect, or pharmaceutical composition of the fourth aspect, by providing a biological sample from the subject; contacting the sample with the antibody-drug conjugate; and detecting binding of the antibody-drug conjugate to cancer cells in the sample. In some embodiments, the cancer cells in the sample express the target antigen CD74. In some embodiments, the cancer expresses the target antigen CD74. In some embodiments, the cancer is a tumor or a hematological cancer. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer. In some embodiments, the sample is a tissue biopsy sample, a blood sample, or a bone marrow sample.

Methods of producing the described ADC compounds and compositions are also disclosed. In an ninth aspect, there is a method of producing an antibody-drug conjugate of the first and second aspect by reacting an antibody or antigen-binding fragment with a cleavable linker joined to an Mcl-1 inhibitor under conditions that allow conjugation.

## BRIEF DESCRIPTION OF THE DRAWINGS

**FIG. 1A** shows *in vitro* activity of CD74-L7-P1 and P1 payload in DLBCL cell lines (CTG 72h).

**FIG. 1B** shows *in vitro* activity of CD74-L7-P1, IgG-L7-P1 and P1 payload in DLBCL cell lines (CTG 72h).

**FIG. 2** shows *in vitro* activity of various CD74 ADCs and corresponding payloads in Monomac1 cell line (CTG 72h).

**FIG. 3** shows the *in vitro* activity of CD74 MCL-1 antibody drug conjugate CD74-L7-P1, Isotype IgG-L7-P1 ADC, and MCL-1 free payload P1 against endogenous cancer cell lines including KMS-21BM, KMS-20, MONO-MAC-1, NOMO-1, Kasumi-6, and EOL-1.

**FIG. 4** shows the *in vitro* activity of CD74 MCL-1 antibody drug conjugate CD74-L5-P1, Isotype IgG-L5-P1 ADC, and MCL-1 free payload P1, alone or in combination with venetoclax, against MONO-MAC-1, NOMO-1, and EOL-1.

**FIG. 5** shows the *in vitro* activity of CD74 MCL-1 antibody drug conjugate CD74-L5-P1, Isotype IgG-L5-P1 ADC, and MCL-1 free payload P1, alone or in combination with Compound A1, against MONO-MAC-1, NOMO-1, and EOL-1.

**FIG. 6** shows tumor volume ($mm^3$) of Nomo1-grafted female SCID mice upon treatment with IgG1-Linker-Payload Fc silent, anti-CD74_CysmAb Fc silent and anti-CD74_CysmAb Fc silent_L5-P1 (30 mg/kg, administered once IV), alone or in combination with venetoclax (50mpk, administered 4ON/3OFF/4ON PO) (n=6). Triangle and diamond shapes indicate treatment schedules of venetoclax and antibodies, respectively.

**FIG. 7** shows % of body weight loss of Nomo1-grafted female SCID mice upon treatment with IgG1-Linker-Payload Fc silent, anti-CD74_CysmAb Fc silent and anti-CD74_CysmAb Fc silent_L5-P1 (30 mg/kg, administered once IV), alone or in combination with venetoclax (50mpk, administered 4ON/3OFF/4ON PO) (n=6).

**FIG. 8** shows tumor volume ($mm^3$) of Karpas422-grafted female NSG mice upon treatment with IgG1-Linker-Payload Fc silent, anti-CD74_CysmAb Fc silent and anti-CD74_CysmAb Fc silent_L5-P1 (30 mg/kg, administered once IV),

alone or in combination with venetoclax (50mpk, administered 4ON/3OFF/4ON PO) (n=6). Triangle and diamond shapes indicate treatment schedules of venetoclax and antibodies, respectively.

**FIG. 9** shows % of body weight loss of Karpas422-grafted female NSG mice upon treatment with IgG1-Linker-Payload Fc silent, anti-CD74_CysmAb Fc silent and anti-CD74_CysmAb Fc silent_L5-P1 (30 mg/kg, administered once IV), alone or in combination with venetoclax (50mpk, administered 4ON/3OFF/4ON PO) (n=6)

**FIG. 10** shows tumor volume ($mm^3$) of Monomac1-grafted female SCID mice upon treatment with IgG1-Linker-Payload Fc silent, anti-CD74_CysmAb Fc silent and anti-CD74_CysmAb Fc silent_L5-P1 (30 mg/kg, administered once IV), alone or in combination with venetoclax (50mpk, administered 2ON/2OFF/5ON/2OFF/4ON PO) (n=6). Triangle and diamond shapes indicate treatment schedules of venetoclax and antibodies, respectively.

**FIG. 11** shows % of body weight loss of Monomac1-grafted female SCID mice upon treatment with IgG1-Linker-Payload Fc silent, anti-CD74_CysmAb Fc silent and anti-CD74_CysmAb Fc silent_L5-P1 (30 mg/kg, administered once IV), alone or in combination with venetoclax (50mpk, administered 2ON/2OFF/5ON/2OFF/4ON PO) (n=6).

**FIG. 12** shows tumor volume ($mm^3$) of EOL1 human acute myeloid (eosinophilic) leukemia xenografted femal SCID mice upon treatment with CD74-L5-P1 Fc silent in combination with venetoclax along with various control groups.

**FIG. 13** shows % of body weight loss of EOL1 human acute myeloid (eosinophilic) leukemia xenografted femal SCID mice upon treatment with CD74-L5-P1 Fc silent in combination with venetoclax along with various control groups.

**FIG. 14** shows tumor volume ($mm^3$) of EOL1 human acute myeloid (eosinophilic) leukemia xenografted femal SCID mice upon treatment with CD74-ADCs with different linker payloads (L5-P1, L30-P1, L31-P1, L32-P1, L33-P1 and L34-P1).

**FIG. 15** shows % of body weight loss of EOL1 human acute myeloid (eosinophilic) leukemia xenografted femal SCID mice upon treatment with CD74-ADCs with different linker payloads (L5-P1, L30-P1, L31-P1, L32-P1, L33-P1 and L34-P1).

**FIG. 16** shows an exemplary site-specific antibody conjugation using bacterial transglutaminase (BTG).

## DETAILED DESCRIPTION OF ILLUSTRATIVE EMBODIMENTS

The disclosed compositions and methods may be understood more readily by reference to the following detailed description taken in connection with the accompanying figures, which form a part of this disclosure.

Throughout this text, the descriptions refer to compositions and methods of using the compositions. Where the disclosure describes or claims a feature or embodiment associated with a composition, such a feature or embodiment is equally applicable to the methods of using the composition. Likewise, where the disclosure describes or claims a feature or embodiment associated with a method of using a composition, such a feature or embodiment is equally applicable to the composition.

When a range of values is expressed, it includes embodiments using any particular value within the range. Further, reference to values stated in ranges includes each and every value within that range. All ranges are inclusive of their endpoints and combinable. When values are expressed as approximations, by use of the antecedent "about," it will be understood that the particular value forms another embodiment. Reference to a particular numerical value includes at least that particular value, unless the context clearly dictates otherwise. The use of "or" will mean "and/or" unless the specific context of its use dictates otherwise. Where a reference and the specification conflict, the specification will control.

Unless the context of a description indicates otherwise, e.g., in the absence of symbols indicating specific point(s) of connectivity, when a structure or fragment of a structure is drawn, it may be used on its own or attached to other components of an ADC, and it may do so with any orientation, e.g., with the antibody attached at any suitable attachment point to a chemical moiety such as a linker-drug. Where indicated, however, components of an ADC are attached in the orientation shown in a given formula. For example, if Formula (1) is described as Ab-(L-D)$_p$ and the group "-(L-D)" is described as

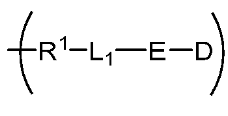
,

then the elaborated structure of Formula (1) is

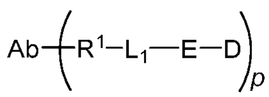
.

It is not

$$Ab\left(D-E-L_1-R_1\right)_p .$$

It is to be appreciated that certain features of the disclosed compositions and methods, which are, for clarity, described herein in the context of separate embodiments, may also be provided in combination in a single embodiment. Conversely, various features of the disclosed compositions and methods that are, for brevity, described in the context of a single embodiment, may also be provided separately or in any sub-combination.

As used throughout this application, antibody drug conjugates can be identified using a naming convention in the general format of "target antigen/antibody-linker-payload". For example only, if an antibody drug conjugate is referred to as "Target X-L0-P0", such a conjugate would comprise an antibody that binds Target X, a linker designated as L0, and a payload designated as P0. Alternatively, if an antibody drug conjugate is referred to as "anti-Target X-L0-P0", such a conjugate would comprise an antibody that binds Target X, a linker designated as L0, and a payload designated as P0. In another alternative, if an antibody drug conjugate is referred to as "AbX-L0-P0", such a conjugate would comprise the antibody designated as AbX, a linker designated as L0, and a payload designated as P0. An control antibody drug conjugate comprising a non-specific, isotype control antibody may be referenced as "isotype control IgG1-L0-P0" or "IgG1-L0-P0".

Any formula given herein is also intended to represent unlabeled forms as well as isotopically labeled forms of the compounds. Isotopically labeled compounds have structures depicted by the formulae given herein except that one or more atoms are replaced by an atom having a selected atomic mass or mass number. Isotopes that can be incorporated into compounds of the invention include, for example, isotopes of hydrogen, carbon, nitrogen, oxygen, fluorine, and chlorine, such as $^{3}H$, $^{11}C$, $^{13}C$, $^{14}C$, $^{15}N$, $^{18}F$, and $^{36}Cl$. Accordingly, it should be understood that the present disclosure includes compounds that incorporate one or more of any of the aforementioned isotopes, including for example, radioactive isotopes, such as $^{3}H$ and $^{14}C$, or those into which non-radioactive isotopes, such as $^{2}H$ and $^{13}C$ are present. Such isotopically labelled compounds are useful in metabolic studies (with $^{14}C$), reaction kinetic studies (with, for example $^{2}H$ or $^{3}H$), detection or imaging techniques, such as positron emission tomography (PET) or single-photon emission computed tomography (SPECT) including drug or substrate tissue distribution assays, or in radioactive treatment of patients. In particular, an $^{18}F$ or labeled compound may be particularly desirable for PET or SPECT studies. Isotopically-labeled compounds can generally be prepared by conventional techniques known to those skilled in the art, e.g., using an appropriate isotopically-labeled reagents in place of the non-labeled reagent previously employed.

## Definitions

Various terms relating to aspects of the description are used throughout the specification and claims. Such terms are to be given their ordinary meaning in the art unless otherwise indicated. Other specifically defined terms are to be construed in a manner consistent with the definitions provided herein.

As used herein, the singular forms "a," "an," and "the" include plural forms unless the context clearly dictates otherwise. The terms "comprising", "having", "being of" as in "being of a chemical formula", "including", and "containing" are to be construed as open terms (i.e., meaning "including but not limited to") unless otherwise noted. Additionally whenever "comprising" or another open-ended term is used in an embodiment, it is to be understood that the same embodiment can be more narrowly claimed using the intermediate term "consisting essentially of" or the closed term "consisting of".

The term "about" or "approximately," when used in the context of numerical values and ranges, refers to values or ranges that approximate or are close to the recited values or ranges such that the embodiment may perform as intended, as is apparent to the skilled person from the teachings contained herein. In some embodiments, about means plus or minus 20%, 15%, 10%, 5%, 1%, 0.5%, or 0.1 % of a numerical amount. In one embodiment, the term "about" refers to a range of values which are 10% more or less than the specified value. In another embodiment, the term "about" refers to a range of values which are 5% more or less than the specified value. In another embodiment, the term "about" refers to a range of values which are 1% more or less than the specified value.

The terms "antibody-drug conjugate," "antibody conjugate," "conjugate," "immunoconjugate," and "ADC" are used interchangeably, and refer to one or more therapeutic compounds (e.g., an Mcl-1 inhibitor) that is linked to one or more antibodies or antigen-binding fragments. In some embodiments, the ADC is defined by the generic formula: $Ab\text{-}(L\text{-}D)_p$ (Formula 1), wherein Ab = an antibody or antigen-binding fragment, L = a linker moiety, D = a drug moiety (e.g., an Mcl-1 inhibitor drug moiety), and *p* = the number of drug moieties per antibody or antigen-binding fragment. In ADCs comprising an Mcl-1 inhibitor drug moiety, "*p*" refers to the number of Mcl-1 inhibitor compounds linked to the antibody or antigen-binding fragment.

The term "antibody" is used in the broadest sense to refer to an immunoglobulin molecule that recognizes and

specifically binds to a target, such as a protein, polypeptide, carbohydrate, polynucleotide, lipid, or combinations of the foregoing through at least one antigen recognition site within the variable region of the immunoglobulin molecule. An antibody can be polyclonal or monoclonal, multiple or single chain, or an intact immunoglobulin, and may be derived from natural sources or from recombinant sources. An "intact" antibody is a glycoprotein that typically comprises at least two heavy (H) chains and two light (L) chains inter-connected by disulfide bonds. Each heavy chain is comprised of a heavy chain variable region (abbreviated herein as VH) and a heavy chain constant region. The heavy chain constant region comprises three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxyl-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. An antibody can be a monoclonal antibody, human antibody, humanized antibody, camelised antibody, or chimeric antibody. The antibodies can be of any isotype (e.g., IgG, IgE, IgM, IgD, IgA and IgY), class (e.g., IgG1, IgG2, IgG3, IgG4, IgA1 and IgA2), or subclass. An antibody can be an intact antibody or an antigen-binding fragment thereof.

The term "antibody fragment" or "antigen-binding fragment" or "functional antibody fragment," as used herein, refers to at least one portion of an antibody that retains the ability to specifically interact with (e.g., by binding, steric hinderance, stabilizing/destabilizing, spatial distribution) an epitope of an antigen (e.g., CD74). Antigen-binding fragments may also retain the ability to internalize into an antigen-expressing cell. In some embodiments, antigen-binding fragments also retain immune effector activity. The terms antibody, antibody fragment, antigen-binding fragment, and the like, are intended to embrace the use of binding domains from antibodies in the context of larger macromolecules such as ADCs. It has been shown that fragments of a full-length antibody can perform the antigen binding function of a full-length antibody. Examples of antibody fragments include, but are not limited to, Fab, Fab', F(ab')2, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody. An antigen-binding fragment can also be incorporated into single domain antibodies, maxibodies, minibodies, nanobodies, intrabodies, diabodies, triabodies, tetrabodies, bispecific or multi-specific antibody constructs, ADCs, v-NAR and bis-scFv (see, e.g., Holliger and Hudson (2005) Nat Biotechnol. 23(9):1126-36). Antigen-binding fragments can also be grafted into scaffolds based on polypeptides such as a fibronectin type III (Fn3) (see US Patent No. 6,703,199, which describes fibronectin polypeptide minibodies). The term "scFv" refers to a fusion protein comprising at least one antigen-binding fragment comprising a variable region of a light chain and at least one antigen-binding fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, an scFv may have the VL and VH variable regions in either order, e.g., with respect to the N-terminal and C-terminal ends of the polypeptide, the scFv may comprise VL-linker-VH or may comprise VH-linker-VL. Antigen-binding fragments are obtained using conventional techniques known to those of skill in the art, and the binding fragments are screened for utility (e.g., binding affinity, internalization) in the same manner as are intact antibodies. Antigen-binding fragments, for example, may be prepared by cleavage of the intact protein, e.g., by protease or chemical cleavage.

The term "complementarity determining region" or "CDR," as used herein, refers to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991) "Sequences of Proteins of Immunological Interest," 5th Ed. Public Health Service, National Institutes of Health, Bethesda, MD ("Kabat" numbering scheme); Al-Lazikani et al. (1997) J Mol Biol. 273(4):927-48 ("Chothia" numbering scheme); ImMunoGenTics (IMGT) numbering (Lefranc (2001) Nucleic Acids Res. 29(1):207-9; Lefranc et al. (2003) Dev Comp Immunol. 27(1):55-77) ("IMGT" numbering scheme); or a combination thereof. In a combined Kabat and Chothia numbering scheme for a given CDR region (for example, HC CDR1, HC CDR2, HC CDR3, LC CDR1, LC CDR2, or LC CDR3), in some embodiments, the CDRs correspond to the amino acid residues that are defined as part of the Kabat CDR, together with the amino acid residues that are defined as part of the Chothia CDR. As used herein, the CDRs defined according to the "Chothia" number scheme are also sometimes referred to as "hypervariable loops."

In some embodiments, under Kabat, the CDR amino acid residues in the heavy chain variable domain (VH) are

numbered 31-35 (HCDR1) (e.g., insertion(s) after position 35), 50-65 (HCDR2), and 95-102 (HCDR3); and the CDR amino acid residues in the light chain variable domain (VL) are numbered 24-34 (LCDR1) (e.g., insertion(s) after position 27), 50-56 (LCDR2), and 89-97 (LCDR3). In some embodiments, under Chothia, the CDR amino acids in the VH are numbered 26-32 (HCDR1) (e.g., insertion(s) after position 31), 52-56 (HCDR2), and 95-102 (HCDR3); and the amino acid residues in VL are numbered 26-32 (LCDR1) (e.g., insertion(s) after position 30), 50-52 (LCDR2), and 91-96 (LCDR3). By combining the CDR definitions of both Kabat and Chothia, in some embodiments, the CDRs comprise or consist of, e.g., amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. In some embodiments, under IMGT, the CDR amino acid residues in the VH are numbered approximately 26-35 (CDR1), 51-57 (CDR2) and 93-102 (CDR3), and the CDR amino acid residues in the VL are numbered approximately 27-32 (CDR1), 50-52 (CDR2), and 89-97 (CDR3). In some embodiments, under IMGT, the CDR regions of an antibody may be determined using the program IMGT/DomainGap Align.

The term "monoclonal antibody," as used herein, refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic epitope. In contrast, conventional (polyclonal) antibody preparations typically include a multitude of antibodies directed against (or specific for) different epitopes. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al. (1975) Nature 256:495, or may be made by recombinant DNA methods (see, e.g., US Patent No. 4,816,567). Monoclonal antibodies may also be isolated from phage antibody libraries using the techniques described in Clackson et al. (1991) Nature 352:624-8, and Marks et al. (1991) J Mol Biol. 222:581-97, for example. The term also includes preparations of antibody molecules of single molecular composition. A monoclonal antibody composition displays a single binding specificity and affinity for a particular epitope.

The monoclonal antibodies described herein can be non-human, human, or humanized. The term specifically includes "chimeric" antibodies, in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they specifically bind the target antigen and/or exhibit the desired biological activity.

The term "human antibody," as used herein, refers an antibody produced by a human or an antibody having an amino acid sequence of an antibody produced by a human. The term includes antibodies having variable regions in which both the framework and CDR regions are derived from sequences of human origin. Furthermore, if the antibody contains a constant region, the constant region is also derived from such human sequences, e.g., human germline sequences, or mutated versions of human germline sequences or antibody containing consensus framework sequences derived from human framework sequences analysis, for example, as described in Knappik et al. ((2000) J Mol Biol. 296(1):57-86). The structures and locations of immunoglobulin variable domains, e.g., CDRs, may be defined using well known numbering schemes, e.g., the Kabat numbering scheme, the Chothia numbering scheme, or a combination of Kabat and Chothia, and/or ImMunoGenTics (IMGT) numbering. The human antibodies of the invention may include amino acid residues not encoded by human sequences (e.g., mutations introduced by random or site-specific mutagenesis in vitro or by somatic mutation in vivo, or a conservative substitution to promote stability or manufacturing). However, the term "human antibody," as used herein, is not intended to include antibodies in which CDR sequences derived from the germline of another mammalian species, such as a mouse, have been grafted onto human framework sequences.

The term "recombinant human antibody," as used herein, refers to a human antibody that is prepared, expressed, created, or isolated by recombinant means, such as antibodies isolated from an animal (e.g., a mouse) that is transgenic or transchromosomal for human immunoglobulin genes or a hybridoma prepared therefrom, antibodies isolated from a host cell transformed to express the human antibody, e.g., from a transfectoma, antibodies isolated from a recombinant, combinatorial human antibody library, and antibodies prepared, expressed, created or isolated by any other means that involve splicing of all or a portion of a human immunoglobulin gene, sequences to other DNA sequences. Such recombinant human antibodies have variable regions in which the framework and CDR regions are derived from human germline immunoglobulin sequences. In some embodiments, however, such recombinant human antibodies can be subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo.

The term "chimeric antibody," as used herein, refers to antibodies wherein the amino acid sequence of the immunoglobulin molecule is derived from two or more species. In some instances, the variable regions of both heavy and light chains correspond to the variable regions of antibodies derived from one species with the desired specificity, affinity,

and activity while the constant regions are homologous to antibodies derived from another species (e.g., human) to minimize an immune response in the latter species.

As used herein, the term "humanized antibody" refers to forms of antibodies that contain sequences from non-human (e.g., murine) antibodies as well as human antibodies. Such antibodies are a type of chimeric antibody which contain minimal sequence derived from non-human immunoglobulin. In general, the humanized antibody will comprise substantially all of at least one, and typically two, variable domains, in which all or substantially all of the hypervariable loops correspond to those of a non-human immunoglobulin and all or substantially all of the framework (FR) regions are those of a human immunoglobulin sequence. The humanized antibody optionally also will comprise at least a portion of an immunoglobulin constant region (Fc), typically that of a human immunoglobulin. The humanized antibody can be further modified by the substitution of residues, either in the Fv framework region and/or within the replaced non-human residues to refine and optimize antibody specificity, affinity, and/or activity.

The term "Fc region," as used herein, refers to a polypeptide comprising the CH3, CH2 and at least a portion of the hinge region of a constant domain of an antibody. Optionally, an Fc region may include a CH4 domain, present in some antibody classes. An Fc region may comprise the entire hinge region of a constant domain of an antibody. In some embodiments, an antibody or antigen-binding fragment comprises an Fc region and a CH1 region of an antibody. In some embodiments, an antibody or antigen-binding fragment comprises an Fc region CH3 region of an antibody. In some embodiments, an antibody or antigen-binding fragment comprises an Fc region, a CH1 region, and a kappa/lambda region from the constant domain of an antibody. In some embodiments, an antibody or antigen-binding fragment comprises a constant region, e.g., a heavy chain constant region and/or a light chain constant region. In some embodiments, such a constant region is modified compared to a wild-type constant region. That is, the polypeptide may comprise alterations or modifications to one or more of the three heavy chain constant domains (CH1, CH2, or CH3) and/or to the light chain constant region domain (CL). Example modifications include additions, deletions, or substitutions of one or more amino acids in one or more domains. Such changes may be included to optimize effector function, half-life, etc.

"Internalizing" as used herein in reference to an antibody or antigen-binding fragment refers to an antibody or antigen-binding fragment that is capable of being taken through the cell's lipid bilayer membrane to an internal compartment (i.e., "internalized") upon binding to the cell, preferably into a degradative compartment in the cell. For example, an internalizing anti-HER2 antibody is one that is capable of being taken into the cell after binding to HER2 on the cell membrane. In some embodiments, the antibody or antigen-binding fragment used in the ADCs disclosed herein targets a cell surface antigen (e.g., CD74) and is an internalizing antibody or internalizing antigen-binding fragment (i.e., the ADC transfers through the cellular membrane after antigen binding). In some embodiments, the internalizing antibody or antigen-binding fragment binds a receptor on the cell surface. An internalizing antibody or internalizing antigen-binding fragment that targets a receptor on the cell membrane may induce receptor-mediated endocytosis. In some embodiments, the internalizing antibody or internalizing antigen-binding fragment is taken into the cell via receptor-mediated endocytosis.

"Non-internalizing" as used herein in reference to an antibody or antigen-binding fragment refers to an antibody or antigen-binding fragment that remains at the cell surface upon binding to the cell. In some embodiments, the antibody or antigen-binding fragment used in the ADCs disclosed herein targets a cell surface antigen and is a non-internalizing antibody or non-internalizing antigen-binding fragment (i.e., the ADC remains at the cell surface and does not transfer through the cellular membrane after antigen binding). In some embodiments, the non-internalizing antibody or antigen-binding fragment binds a non-internalizing receptor or other cell surface antigen. Exemplary non-internalizing cell surface antigens include but are not limited to CA125 and CEA, and antibodies that bind to non-internalizing antigen targets are also known in the art (see, e.g., Bast et al. (1981) J Clin Invest. 68(5):1331-7; Scholler and Urban (2007) Biomark Med. 1(4):513-23; and Boudousq et al. (2013) PLoS One 8(7):e69613).

The term "binding specificity," as used herein, refers to the ability of an individual antibody or antigen binding fragment to preferentially react with one antigenic determinant over a different antigenic determinant. The degree of specificity indicates the extent to which an antibody or fragment preferentially binds to one antigenic determinant over a different antigenic determinant. Also, as used herein, the term "specific," "specifically binds," and "binds specifically" refers to a binding reaction between an antibody or antigen-binding fragment (e.g., an anti-CD74 antibody) and a target antigen (e.g., CD74) in a heterogeneous population of proteins and other biologics. Antibodies can be tested for specificity of binding by comparing binding to an appropriate antigen to binding to an irrelevant antigen or antigen mixture under a given set of conditions. If the antibody binds to the appropriate antigen with at least 2, 5, 7, 10 or more times more affinity than to the irrelevant antigen or antigen mixture, then it is considered to be specific. A "specific antibody" or a "target-specific antibody" is one that only binds the target antigen (e.g., CD74), but does not bind (or exhibits minimal binding) to other antigens. In some embodiments, an antibody or antigen-binding fragment that specifically binds a target antigen (e.g., CD74) has a $K_D$ of less than $1x10^{-6}$ M, less than $1x10^{-7}$ M, less than $1x10^{-8}$ M, less than $1x10^{-9}$ M, less than $1x10^{-10}$ M, less than $1x10^{-11}$ M, less than $1x10^{-12}$ M, or less than $1x10^{-13}$ M. In some embodiments, the $K_D$ is 1 pM to 500 pM. In some embodiments, the $K_D$ is between 500 pM to 1 $\mu$M, 1 $\mu$M to 100 nM, or 100 mM to 10 nM.

The term "affinity," as used herein, refers to the strength of interaction between antibody and antigen at single

antigenic sites. Without being bound by theory, within each antigen binding site, the variable region of the antibody "arm" interacts through weak noncovalent forces with the antigen at numerous sites; the more interactions, typically the stronger the affinity. The binding affinity of an antibody is the sum of the attractive and repulsive forces operating between the antigenic determinant and the binding site of the antibody.

The term "$k_{on}$" or "$k_a$" refers to the on-rate constant for association of an antibody to the antigen to form the antibody/antigen complex. The rate can be determined using standard assays, such as a surface plasmon resonance, biolayer inferometry, or ELISA assay.

The term "$k_{off}$" or "$k_d$" refers to the off-rate constant for dissociation of an antibody from the antibody/antigen complex. The rate can be determined using standard assays, such as a surface plasmon resonance, biolayer inferometry, or ELISA assay.

The term "$K_D$" refers to the equilibrium dissociation constant of a particular antibody-antigen interaction. $K_D$ is calculated by $k_a/k_d$. The rate can be determined using standard assays, such as a surface plasmon resonance, biolayer inferometry, or ELISA assay.

The term "epitope" refers to the portion of an antigen capable of being recognized and specifically bound by an antibody (or antigen-binding fragment). Epitope determinants generally consist of chemically active surface groupings of molecules such as amino acids or carbohydrate or sugar side chains and can have specific three-dimensional structural characteristics, as well as specific charge characteristics. When the antigen is a polypeptide, epitopes can be formed from contiguous amino acids or noncontiguous amino acids juxtaposed by tertiary folding of the polypeptide. An epitope may be "linear" or "conformational." Conformational and linear epitopes are distinguished in that the binding to the former but not the latter is lost in the presence of denaturing solvents. The epitope bound by an antibody (or antigen-binding fragment) may be identified using any epitope mapping technique known in the art, including X-ray crystallography for epitope identification by direct visualization of the antigen-antibody complex, as well as monitoring the binding of the antibody to fragments or mutated variations of the antigen, or monitoring solvent accessibility of different parts of the antibody and the antigen. Exemplary strategies used to map antibody epitopes include, but are not limited to, array-based oligo-peptide scanning, limited proteolysis, site-directed mutagenesis, high-throughput mutagenesis mapping, hydrogen-deuterium exchange, and mass spectrometry (see, e.g., Gershoni et al. (2007) BioDrugs 21:145-56; and Hager-Braun and Tomer (2005) Expert Rev Proteomics 2:745-56).

Competitive binding and epitope binning can also be used to determine antibodies sharing identical or overlapping epitopes. Competitive binding can be evaluated using a cross-blocking assay, such as the assay described in "Antibodies, A Laboratory Manual," Cold Spring Harbor Laboratory, Harlow and Lane (1st edition 1988, 2nd edition 2014). In some embodiments, competitive binding is identified when a test antibody or binding protein reduces binding of a reference antibody or binding protein to a target antigen such as CD74 (e.g., a binding protein comprising CDRs and/or variable domains selected from those identified in Tables 3-5), by at least about 50% in the cross-blocking assay (e.g., 50%, 60%, 70%, 80%, 90%, 95%, 99%, 99.5%, or more, or any percentage in between), and/or vice versa. In some embodiments, competitive binding can be due to shared or similar (e.g., partially overlapping) epitopes, or due to steric hindrance where antibodies or binding proteins bind at nearby epitopes (see, e.g., Tzartos, Methods in Molecular Biology (Morris, ed. (1998) vol. 66, pp. 55-66)). In some embodiments, competitive binding can be used to sort groups of binding proteins that share similar epitopes. For example, binding proteins that compete for binding can be "binned" as a group of binding proteins that have overlapping or nearby epitopes, while those that do not compete are placed in a separate group of binding proteins that do not have overlapping or nearby epitopes.

As used herein, the terms "peptide," "polypeptide," and "protein" are used interchangeably to refer to a polymer of amino acid residues. The terms encompass amino acid polymers comprising two or more amino acids joined to each other by peptide bonds, amino acid polymers in which one or more amino acid residues is an artificial chemical mimetic of a corresponding naturally-occurring amino acid, as well as naturally-occurring amino acid polymers and non-naturally-occurring amino acid polymers. The terms include, for example, biologically active fragments, substantially homologous polypeptides, oligopeptides, homodimers, heterodimers, variants of polypeptides, modified polypeptides, derivatives, analogs, fusion proteins, among others. The terms also include natural peptides, recombinant peptides, synthetic peptides, or a combination thereof. Unless otherwise indicated, a particular polypeptide sequence also implicitly encompasses conservatively modified variants thereof.

A "recombinant" protein refers to a protein (e.g., an antibody) made using recombinant techniques, e.g., through the expression of a recombinant nucleic acid.

An "isolated" protein refers to a protein unaccompanied by at least some of the material with which it is normally associated in its natural state. For example, a naturally-occurring polynucleotide or polypeptide present in a living organism is not isolated, but the same polynucleotide or polypeptide separated from some or all of the coexisting materials in the living organism, is isolated. The definition includes the production of an antibody in a wide variety of organisms and/or host cells that are known in the art.

An "isolated antibody," as used herein, is an antibody that has been identified and separated from one or more (e.g., the majority) of the components (by weight) of its source environment, e.g., from the components of a hybridoma cell

culture or a different cell culture that was used for its production. In some embodiments, the separation is performed such that it sufficiently removes components that may otherwise interfere with the suitability of the antibody for the desired applications (e.g., for therapeutic use). Methods for preparing isolated antibodies are known in the art and include, without limitation, protein A chromatography, anion exchange chromatography, cation exchange chromatography, virus retentive filtration, and ultrafiltration.

As used herein, the term "variant" refers to a nucleic acid sequence or an amino acid sequence that differs from a reference nucleic acid sequence or amino acid sequence respectively, but retains one or more biological properties of the reference sequence. A variant may contain one or more amino acid substitutions, deletions, and/or insertions (or corresponding substitution, deletion, and/or insertion of codons) with respect to a reference sequence. Changes in a nucleic acid variant may not alter the amino acid sequence of a peptide encoded by the reference nucleic acid sequence, or may result in amino acid substitutions, additions, deletions, fusions, and/or truncations. In some embodiments, a nucleic acid variant disclosed herein encodes an identical amino acid sequence to that encoded by the unmodified nucleic acid or encodes a modified amino acid sequence that retains one or more functional properties of the unmodified amino acid sequence. Changes in the sequence of peptide variants are typically limited or conservative, so that the sequences of the unmodified peptide and the variant are closely similar overall and, in many regions, identical. In some embodiments, a peptide variant retains one or more functional properties of the unmodified peptide sequence. A variant and unmodified peptide can differ in amino acid sequence by one or more substitutions, additions, deletions in any combination.

A variant of a nucleic acid or peptide can be a naturally-occurring variant or a variant that is not known to occur naturally. Variants of nucleic acids and peptides may be made by mutagenesis techniques, by direct synthesis, or by other techniques known in the art. A variant does not necessarily require physical manipulation of the reference sequence. As long as a sequence contains a different nucleic acid or amino acid as compared to a reference sequence, it is considered a "variant" regardless of how it was synthesized. In some embodiments, a variant has high sequence identity (i.e., 60% nucleic acid or amino acid sequence identity or higher) as compared to a reference sequence. In some embodiments, a peptide variant encompasses polypeptides having amino acid substitutions, deletions, and/or insertions as long as the polypeptide has at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% amino acid sequence identity with a reference sequence, or with a corresponding segment (e.g., a functional fragment) of a reference sequence, e.g., those variants that also retain one or more functions of the reference sequence. In some embodiments, a nucleic acid variant encompasses polynucleotides having amino acid substitutions, deletions, and/or insertions as long as the polynucleotide has at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% nucleic acid sequence identity with a reference sequence, or with a corresponding segment (e.g., a functional fragment) of a reference sequence.

The term "conservatively modified variant" applies to both amino acid and nucleic acid sequences. For nucleic acid sequences, conservatively modified variants refer to those nucleic acids which encode identical or essentially identical amino acid sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence. For polypeptide sequences, conservatively modified variants include individual substitutions, deletions, or additions to a polypeptide sequence which result in the substitution of an amino acid with a chemically similar amino acid. Conservative substitutions providing functionally similar amino acids are well known in the art.

The term "conservative sequence modifications," as used herein, refers to amino acid modifications that do not significantly affect or alter the binding characteristics of, e.g., an antibody or antigen-binding fragment containing the amino acid sequence. Such conservative modifications include amino acid substitutions, additions, and deletions. Modifications can be introduced into an antibody or antigen-binding fragment by standard techniques known in the art, such as, e.g., site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, in some

embodiments, one or more amino acid residues within an antibody can be replaced with other amino acid residues from the same side chain family and the altered antibody can be tested using the functional assays described herein.

The term "homologous" or "identity," as used herein, refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions. For example, if half (e.g., five positions in a polymer ten subunits in length) of the positions in two sequences are matched or homologous, the two sequences are 50% homologous; if 90% of the positions (e.g., 9 of 10), are matched or homologous, the two sequences are 90% homologous.

Percentage of "sequence identity" can be determined by comparing two optimally aligned sequences over a comparison window, where the fragment of the amino acid sequence in the comparison window may comprise additions or deletions (e.g., gaps or overhangs) as compared to the reference sequence (which does not comprise additions or deletions) for optimal alignment of the two sequences. The percentage can be calculated by determining the number of positions at which the identical amino acid residue occurs in both sequences to yield the number of matched positions, dividing the number of matched positions by the total number of positions in the window of comparison, and multiplying the result by 100 to yield the percentage of sequence identity. The output is the percent identity of the subject sequence with respect to the query sequence. The percent identity between the two sequences is a function of the number of identical positions shared by the sequences, taking into account the number of gaps, and the length of each gap, which need to be introduced for optimal alignment of the two sequences. Generally, the amino acid identity or homology between proteins disclosed herein and variants thereof, including variants of target antigens (such as CD74) and variants of antibody variable domains (including individual variant CDRs), is at least 80% to the sequences depicted herein, e.g., identities or homologies of at least 85%, 90%, 91%, 92%, 93%, 94%, 95%, 96%, 97%, 98%, 99%, almost 100%, or 100%.

The comparison of sequences and determination of percent identity between two sequences can be accomplished using a mathematical algorithm. In some embodiments, the percent identity between two amino acid sequences is determined using the Needleman and Wunsch ((1970) J Mol Biol. 48:444-53) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. In some embodiments, the percent identity between two nucleotide sequences is determined using the GAP program in the GCG software package, using a NWSgapdna.CMP matrix and a gap weight of 40, 50, 60, 70, or 80 and a length weight of 1, 2, 3, 4, 5, or 6. An exemplary set of parameters is a Blossum 62 scoring matrix with a gap penalty of 12, a gap extend penalty of 4, and a frameshift gap penalty of 5. The percent identity between two amino acid or nucleotide sequences can also be determined using the algorithm of Meyers and Miller ((1989) CABIOS 4:11-17) which has been incorporated into the ALIGN program (version 2.0), using a PAM120 weight residue table, a gap length penalty of 12 and a gap penalty of 4.

The term "agent" is used herein to refer to a chemical compound, a mixture of chemical compounds, a biological macromolecule, an extract made from biological materials, or a combination of two or more thereof. The term "therapeutic agent" or "drug" refers to an agent that is capable of modulating a biological process and/or has biological activity. The Mcl-1 inhibitors and the ADCs comprising them, as described herein, are exemplary therapeutic agents.

The term "chemotherapeutic agent" or "anti-cancer agent" is used herein to refer to all agents that are effective in treating cancer (regardless of mechanism of action). Inhibition of metastasis or angiogenesis is frequently a property of a chemotherapeutic agent. Chemotherapeutic agents include antibodies, biological molecules, and small molecules, and encompass the Mcl-1 inhibitors and ADCs comprising them, as described herein. A chemotherapeutic agent may be a cytotoxic or cytostatic agent. The term "cytostatic agent" refers to an agent that inhibits or suppresses cell growth and/or multiplication of cells. The term "cytotoxic agent" refers to a substance that causes cell death primarily by interfering with a cell's expression activity and/or functioning.

The term "myeloid cell leukemia 1" or "Mcl-1," as used herein, refers to any native form of human Mcl-1, an anti-apoptotic member of the Bcl-2 protein family. The term encompasses full-length human Mcl-1 (e.g., UniProt Reference Sequence: Q07820; SEQ ID NO:63), as well as any form of human Mcl-1 that may result from cellular processing. The term also encompasses functional variants or fragments of human Mcl-1, including but not limited to splice variants, allelic variants, and isoforms that retain one or more biologic functions of human Mcl-1 (i.e., variants and fragments are encompassed unless the context indicates that the term is used to refer to the wild-type protein only). Mcl-1 can be isolated from human, or may be produced recombinantly or by synthetic methods.

The term "inhibit" or "inhibition" or "inhibiting," as used herein, means to reduce a biological activity or process by a measurable amount, and can include but does not require complete prevention or inhibition. In some embodiments, "inhibition" means to reduce the expression and/or activity of Mcl-1 and/or one or more upstream modulators or downstream targets thereof.

The term "Mcl-1 inhibitor," as used herein, refers to an agent capable of reducing the expression and/or activity of Mcl-1 and/or one or more upstream modulators or downstream targets thereof. Exemplary Mcl-1 modulators (including

exemplary inhibitors of Mcl-1) are described in WO 2015/097123; WO 2016/207216; WO 2016/207217; WO 2016/207225; WO 2016/207226; WO 2017/125224; WO 2019/035899, WO 2019/035911, WO 2019/035914, WO 2019/035927, US 2019/0055264, WO 2016/033486, WO 2017/147410, WO 2018/183418, and WO 2017/182625. Each of which are exemplary Mcl-1 modulators, including exemplary Mcl-1 inhibitors, that can be included as drug moieties in the disclosed ADCs. For example, exemplary Mcl-1 inhibitors that can be included as drug moieties in the disclosed ADCs are those of formula:

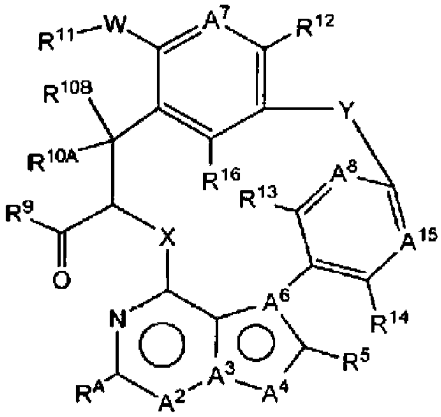

,

wherein each variable is defined as in WO2019/035911; WO 2019/035899; WO 2019/035914; or WO 2019/035927. Specific examples include, e.g.,

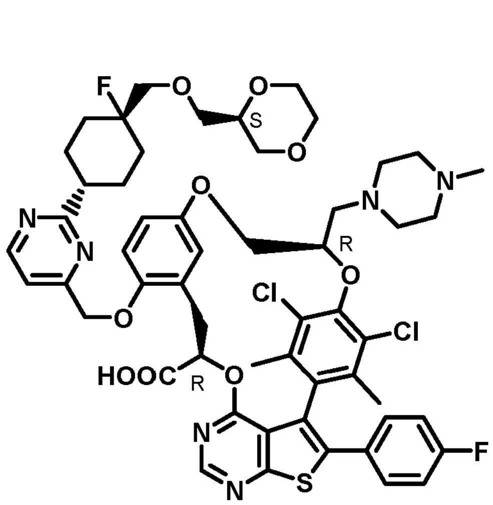

(P16),

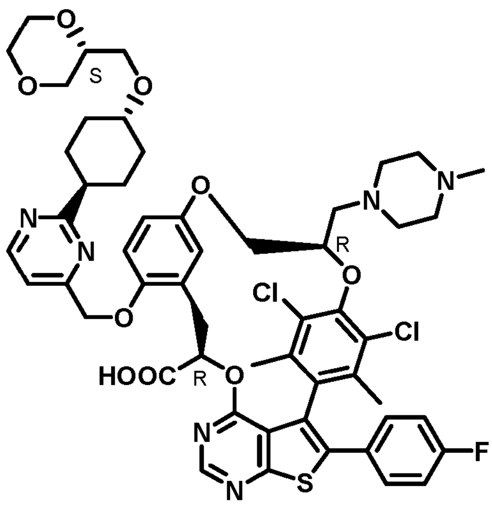

(P17),

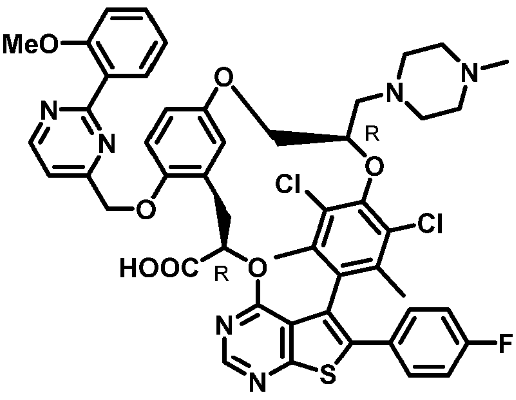

(P15),

wherein each compound as a drug payload can be conjugated to an antibody or a linker via the nitrogen atom of the N-methyl in piperazinyl functional group of the compound. As used herein, the terms "derivative" and "analog" when referring to an Mcl-1 inhibitor, or the like, means any such compound that retains essentially the same, similar, or enhanced biological function or activity as compared to the original compound but has an altered chemical or biological structure.

As used herein, a "Mcl-1 inhibitor drug moiety", "Mcl-1 inhibitor", and the like refer to the component of an ADC or composition that provides the structure of an Mcl-1 inhibitor compound or a compound modified for attachment to an ADC that retains essentially the same, similar, or enhanced biological function or activity as compared to the original compound. In some embodiments, Mcl-1 inhibitor drug moiety is component (D) in an ADC of Formula (1).

The term "cancer," as used herein, refers to the presence of cells possessing characteristics typical of cancer-causing cells, such as uncontrolled proliferation, immortality, metastatic potential, rapid growth and proliferation rate, and/or certain morphological features. Often, cancer cells can be in the form of a tumor or mass, but such cells may exist alone within a subject, or may circulate in the blood stream as independent cells, such as leukemic or lymphoma cells. The term "cancer" includes all types of cancers and cancer metastases, including hematological cancers, solid tumors, sarcomas, carcinomas and other solid and non-solid tumor cancers. Hematological cancers may include B-cell malignancies, cancers of the blood (leukemias), cancers of plasma cells (myelomas, e.g., multiple myeloma), or cancers of the lymph nodes (lymphomas). Exemplary B-cell malignancies include chronic lymphocytic leukemia (CLL), follicular lymphoma, mantle cell lymphoma, and diffuse large B-cell lymphoma. Leukemias may include acute lymphoblastic leukemia (ALL), acute myeloid leukemia (AML), chronic lymphocytic leukemia (CLL), chronic myelogenous leukemia (CML), chronic myelomonocytic leukemia (CMML), acute monocytic leukemia (AMoL), etc. Lymphomas may include Hodgkin's lymphoma, non-Hodgkin's lymphoma, etc. Other hematologic cancers may include myelodysplasia syndrome (MDS). Solid tumors may include carcinomas such as adenocarcinoma, e.g., breast cancer, pancreatic cancer, prostate cancer, colon or colorectal cancer, lung cancer, gastric cancer, cervical cancer, endometrial cancer, ovarian cancer, cholangiocarcinoma, glioma, melanoma, etc. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer.

In some embodiments, the cancer is a hematological cancer, e.g., a leukemia, a lymphoma, or a myeloma. For example, an combination described herein can be used to treat cancers malignancies, and related disorders, including, but not limited to, e.g., an acute leukemia, e.g., B-cell acute lymphoid leukemia (BALL), T-cell acute lymphoid leukemia (TALL), acute myeloid leukemia (AML), acute lymphoid leukemia (ALL); a chronic leukemia, e.g., chronic myelogenous leukemia (CML), chronic lymphocytic leukemia (CLL); an additional hematologic cancer or hematologic condition, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia, myelofibrosis, amyloid light chain amyloidosis, chronic neutrophilic leukemia, essential thrombocythemia, chronic eosinophilic leukemia, chronic myelomonocytic leukemia, Richter Syndrome, mixed phenotrype acute leukemia, acute biphenotypic leukemia, and "preleukemia" which are a diverse collection of hematological conditions united by ineffective production (or dysplasia) of myeloid blood cells, and the like.

As used herein, the term "tumor" refers to any mass of tissue that results from excessive cell growth or proliferation, either benign or malignant, including precancerous lesions. In some embodiments, the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer. In some embodiments, the tumor is a gastric cancer.

The terms "tumor cell" and "cancer cell" may be used interchangeably herein and refer to individual cells or the total population of cells derived from a tumor or cancer, including both non-tumorigenic cells and cancer stem cells. The terms "tumor cell" and "cancer cell" will be modified by the term "non-tumorigenic" when referring solely to those cells lacking the capacity to renew and differentiate to distinguish those cells from cancer stem cells.

The term "target-negative," "target antigen-negative," or "antigen-negative," as used herein, refers to the absence of target antigen expression by a cell or tissue. The term "target-positive," "target antigen-positive," or "antigen-positive" refers to the presence of target antigen expression. For example, a cell or a cell line that does not express a target antigen may be described as target-negative, whereas a cell or cell line that expresses a target antigen may be described as target-positive.

The terms "subject" and "patient" are used interchangeably herein to refer to any human or non-human animal in need of treatment. Non-human animals include all vertebrates (e.g., mammals and non-mammals) such as any mammal. Non-limiting examples of mammals include humans, chimpanzees, apes, monkeys, cattle, horses, sheep, goats, swine,

rabbits, dogs, cats, rats, mice, and guinea pigs. Non-limiting examples of non-mammals include birds and fish. In some embodiments, the subject is a human.

The term "a subject in need of treatment," as used herein, refers to a subject that would benefit biologically, medically, or in quality of life from a treatment (e.g., a treatment with any one or more of the exemplary ADC compounds described herein).

As used herein, the term "treat," "treating," or "treatment" refers to any improvement of any consequence of disease, disorder, or condition, such as prolonged survival, less morbidity, and/or a lessening of side effects which result from an alternative therapeutic modality. In some embodiments, treatment comprises delaying or ameliorating a disease, disorder, or condition (i.e., slowing or arresting or reducing the development of a disease or at least one of the clinical symptoms thereof). In some embodiments, treatment comprises delaying, alleviating, or ameliorating at least one physical parameter of a disease, disorder, or condition, including those which may not be discernible by the patient. In some embodiments, treatment comprises modulating a disease, disorder, or condition, either physically (e.g., stabilization of a discernible symptom), physiologically (e.g., stabilization of a physical parameter), or both. In some embodiments, treatment comprises administration of a described ADC compound or composition to a subject, e.g., a patient, to obtain a treatment benefit enumerated herein. The treatment can be to cure, heal, alleviate, delay, prevent, relieve, alter, remedy, ameliorate, palliate, improve, or affect a disease, disorder, or condition (e.g., a cancer), the symptoms of a disease, disorder, or condition (e.g., a cancer), or a predisposition toward a disease, disorder, or condition (e.g., a cancer). In some embodiments, in addition to treating a subject having a disease, disorder, or condition, a composition disclosed herein can also be provided prophylactically to prevent or reduce the likelihood of developing that disease, disorder, or condition.

As used herein, the term "prevent", "preventing," or "prevention" of a disease, disorder, or condition refers to the prophylactic treatment of the disease, disorder, or condition; or delaying the onset or progression of the disease, disorder, or condition.

As used herein, a "pharmaceutical composition" refers to a preparation of a composition, e.g., an ADC compound or composition, in addition to at least one other (and optionally more than one other) component suitable for administration to a subject, such as a pharmaceutically acceptable carrier, stabilizer, diluent, dispersing agent, suspending agent, thickening agent, and/or excipient. The pharmaceutical compositions provided herein are in such form as to permit administration and subsequently provide the intended biological activity of the active ingredient(s) and/or to achieve a therapeutic effect. The pharmaceutical compositions provided herein preferably contain no additional components which are unacceptably toxic to a subject to which the formulation would be administered.

As used herein, the terms "pharmaceutically acceptable carrier" and "physiologically acceptable carrier," which may be used interchangeably, refer to a carrier or a diluent that does not cause significant irritation to a subject and does not abrogate the biological activity and properties of the administered ADC compound or composition and/or any additional therapeutic agent in the composition. Pharmaceutically acceptable carriers may enhance or stabilize the composition or can be used to facilitate preparation of the composition. Pharmaceutically acceptable carriers can include solvents, dispersion media, coatings, surfactants, antioxidants, preservatives (e.g., antibacterial agents, antifungal agents), isotonic agents, absorption delaying agents, salts, preservatives, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated. The carrier may be selected to minimize adverse side effects in the subject, and/or to minimize degradation of the active ingredient(s). An adjuvant may also be included in any of these formulations.

As used herein, the term "excipient" refers to an inert substance added to a pharmaceutical composition to further facilitate administration of an active ingredient. Formulations for parenteral administration can, for example, contain excipients such as sterile water or saline, polyalkylene glycols such as polyethylene glycol, vegetable oils, or hydrogenated napthalenes. Other exemplary excipients include, but are not limited to, calcium bicarbonate, calcium phosphate, various sugars and types of starch, cellulose derivatives, gelatin, ethylene-vinyl acetate co-polymer particles, and surfactants, including, for example, polysorbate 20.

The term "pharmaceutically acceptable salt," as used herein, refers to a salt which does not abrogate the biological activity and properties of the compounds of the invention, and does not cause significant irritation to a subject to which it is administered. Examples of such salts include, but are not limited to: (a) acid addition salts formed with inorganic acids, for example, hydrochloric acid, hydrobromic acid, sulfuric acid, phosphoric acid, nitric acid and the like; and salts formed with organic acids, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (b) salts formed from elemental anions such as chlorine, bromine, and iodine. See, e.g., Haynes et al., "Commentary: Occurrence of Pharmaceutically Acceptable Anions and Cations in the Cambridge Structural Database," J. Pharmaceutical Sciences, vol. 94, no. 10 (2005), and Berge et al., "Pharmaceutical Salts," J. Pharmaceutical

Sciences, vol. 66, no. 1 (1977).

In some embodiments, depending on their electronic charge, the antibody-drug conjugates (ADCs), linkers, payloads and linker-payloads described herein can contain a monovalent anionic counterion $M_1^-$. Any suitable anionic counterion can be used. In certain embodiments, the monovalent anionic counterion is a pharmaceutically acceptable monovalent anionic counterion. In certain embodiments, the monovalent anionic counterion $M_1^-$ can be selected from bromide, chloride, iodide, acetate, trifluoroacetate, benzoate, mesylate, tosylate, triflate, formate, or the like. In some embodiments, the monovalent anionic counterion $M_1^-$ is trifluoroacetate or formate.

As used herein, the term "therapeutically effective amount" or "therapeutically effective dose," refers to an amount of a compound described herein, e.g., an ADC compound or composition described herein, to effect the desired therapeutic result (i.e., reduction or inhibition of an enzyme or a protein activity, amelioration of symptoms, alleviation of symptoms or conditions, delay of disease progression, a reduction in tumor size, inhibition of tumor growth, prevention of metastasis). In some embodiments, a therapeutically effective amount does not induce or cause undesirable side effects. In some embodiments, a therapeutically effective amount induces or causes side effects but only those that are acceptable by a treating clinician in view of a patient's condition. In some embodiments, a therapeutically effective amount is effective for detectable killing, reduction, and/or inhibition of the growth or spread of cancer cells, the size or number of tumors, and/or other measure of the level, stage, progression and/or severity of a cancer. The term also applies to a dose that will induce a particular response in target cells, e.g., a reduction, slowing, or inhibition of cell growth. A therapeutically effective amount can be determined by first administering a low dose, and then incrementally increasing that dose until the desired effect is achieved. A therapeutically effective amount can also vary depending upon the intended application (in vitro or in vivo), or the subject and disease condition being treated, e.g., the weight and age of the subject, the severity of the disease condition, the manner of administration and the like, which can readily be determined by one of ordinary skill in the art. The specific amount may vary depending on, for example, the particular pharmaceutical composition, the subject and their age and existing health conditions or risk for health conditions, the dosing regimen to be followed, the severity of the disease, whether it is administered in combination with other agents, timing of administration, the tissue to which it is administered, and the physical delivery system in which it is carried. In the case of cancer, a therapeutically effective amount of an ADC may reduce the number of cancer cells, reduce tumor size, inhibit (e.g., slow or stop) tumor metastasis, inhibit (e.g., slow or stop) tumor growth, and/or relieve one or more symptoms.

As used herein, the term "prophylactically effective amount" or "prophylactically effective dose," refers to an amount of a compound disclosed herein, e.g., an ADC compound or composition described herein, that is effective, at dosages and for periods of time necessary, to achieve the desired prophylactic result. Typically, since a prophylactic dose is used in subjects prior to or at an earlier stage of disease, the prophylactically effective amount will be less than the therapeutically effective amount. In some embodiments, a prophylactically effective amount can prevent the onset of disease symptoms, including symptoms associated with a cancer.

The term *"p"* or "drug loading" or "drug:antibody ratio" or "drug-to-antibody ratio" or "DAR" refers to the number of drug moieties per antibody or antigen-binding fragment, i.e., drug loading, or the number of -L-D moieties per antibody or antigen-binding fragment (Ab) in ADCs of Formula (1). In ADCs comprising an Mcl-1 inhibitor drug moiety, *"p"* refers to the number of Mcl-1 inhibitor compounds linked to the antibody or antigen-binding fragment. For example, if two Mcl-1 inhibitor compounds are linked to an antibody or antigen-binding fragment, $p = 2$. In compositions comprising multiple copies of ADCs of Formula (1), "average *p"* refers to the average number of -L-D moieties per antibody or antigen-binding fragment, also referred to as "average drug loading."

Antibody-Drug Conjugates

The antibody-drug conjugate (ADC) compounds of the present disclosure include those with anti-cancer activity. In particular, the ADC compounds include an antibody or antigen-binding fragment conjugated (i.e., covalently attached by a linker) to a drug moiety (e.g., an Mcl-1 inhibitor), wherein the drug moiety when not conjugated to an antibody or antigen-binding fragment has a cytotoxic or cytostatic effect. In some embodiments, the drug moiety when not conjugated to an antibody or antigen-binding fragment is capable of reducing the expression and/or activity of Mcl-1 and/or one or more upstream modulators or downstream targets thereof. Without being bound by theory, by targeting Mcl-1 expression and/or activity, in some embodiments, the ADCs disclosed herein may provide potent anti-cancer agents. Also, without being bound by theory, by conjugating the drug moiety to an antibody that binds an antigen associated with expression in a tumor cell or cancer, the ADC may provide improved activity, better cytotoxic specificity, and/or reduced off-target killing as compared to the drug moiety when administered alone.

In some embodiments, therefore, the components of the ADC are selected to (i) retain one or more therapeutic properties exhibited by the antibody and drug moieties in isolation, (ii) maintain the specific binding properties of the antibody or antigen-binding fragment; (iii) optimize drug loading and drug-to-antibody ratios; (iv) allow delivery, e.g., intracellular delivery, of the drug moiety via stable attachment to the antibody or antigen-binding fragment; (v) retain ADC stability as an intact conjugate until transport or delivery to a target site; (vi) minimize aggregation of the ADC prior to or after

administration; (vii) allow for the therapeutic effect, e.g., cytotoxic effect, of the drug moiety after cleavage or other release mechanism in the cellular environment; (viii) exhibit in vivo anti-cancer treatment efficacy comparable to or superior to that of the antibody and drug moieties in isolation; (ix) minimize off-target killing by the drug moiety; and/or (x) exhibit desirable pharmacokinetic and pharmacodynamics properties, formulatability, and toxicologic/immunologic profiles. Each of these properties may provide for an improved ADC for therapeutic use (Ab et al. (2015) Mol Cancer Ther. 14:1605-13).

The ADC compounds of the present disclosure may selectively deliver an effective dose of a cytotoxic or cytostatic agent to cancer cells or to tumor tissue. In some embodiments, the cytotoxic and/or cytostatic activity of the ADC is dependent on target antigen expression in a cell. In some embodiments, the disclosed ADCs are particularly effective at killing cancer cells expressing a target antigen while minimizing off-target killing. In some embodiments, the disclosed ADCs do not exhibit a cytotoxic and/or cytostatic effect on cancer cells that do not express a target antigen.

Provided herein, in certain aspects, are ADC compounds comprising an antibody or antigen-binding fragment thereof (Ab) which targets a cancer cell, an Mcl-1 inhibitor drug moiety (D), and a linker moiety (L) that covalently attaches Ab to D. In some embodiments, the antibody or antigen-binding fragment is able to bind to a tumor-associated antigen (e.g., BCMA, CD33, PCAD, or HER2), e.g., with high specificity and high affinity. In some embodiments, the antibody or antigen-binding fragment is internalized into a target cell upon binding, e.g., into a degradative compartment in the cell. In some embodiments, the ADCs internalize upon binding to a target cell, undergo degradation, and release the Mcl-1 inhibitor drug moiety to kill cancer cells. The Mcl-1 inhibitor drug moiety may be released from the antibody and/or the linker moiety of the ADC by enzymatic action, hydrolysis, oxidation, or any other mechanism.

An exemplary ADC has Formula (1):

$$\text{Ab-(L-D)}_p \qquad (1)$$

wherein Ab = an antibody or antigen-binding fragment, L = a linker moiety, D = an Mcl-1 inhibitor drug moiety, and *p* = the number of Mcl-1 inhibitor drug moieties per antibody or antigen-binding fragment.

<u>Antibodies</u>

The antibody or antigen-binding fragment (Ab) of Formula (1) includes within its scope any antibody or antigen-binding fragment that specifically binds to a target antigen on a cancer cell. The antibody or antigen-binding fragment may bind to a target antigen with a dissociation constant ($K_D$) of ≤1 mM, ≤100 nM or ≤10 nM, or any amount in between, as measured by, e.g., BIAcore® analysis. In some embodiments, the $K_D$ is 1 pM to 500 pM. In some embodiments, the $K_D$ is between 500 pM to 1 μM, 1 μM to 100 nM, or 100 mM to 10 nM.

In some embodiments, the antibody or antigen-binding fragment is a four-chain antibody (also referred to as an immunoglobulin or a full-length or intact antibody), comprising two heavy chains and two light chains. In some embodiments, the antibody or antigen-binding fragment is an antigen-binding fragment of an immunoglobulin. In some embodiments, the antibody or antigen-binding fragment is an antigen-binding fragment of an immunoglobulin that retains the ability to bind a target cancer antigen and/or provide at least one function of the immunoglobulin.

In some embodiments, the antibody or antigen-binding fragment is an internalizing antibody or internalizing antigen-binding fragment thereof. In some embodiments, the internalizing antibody or internalizing antigen-binding fragment thereof binds to a target cancer antigen expressed on the surface of a cell and enters the cell upon binding. In some embodiments, the Mcl-1 inhibitor drug moiety of the ADC is released from the antibody or antigen-binding fragment of the ADC after the ADC enters and is present in a cell expressing the target cancer antigen (i.e., after the ADC has been internalized), e.g., by cleavage, by degradation of the antibody or antigen-binding fragment, or by any other suitable release mechanism.

Amino acid sequences of exemplary antibodies of the present disclosure, in addition to exemplary antigen targets, are set forth in Tables C, D and E.

**Table C. Amino acid sequences of mAb CDRs**

| Ab | SEQ ID NO | IgG chain | Amino acid sequence |
|---|---|---|---|
| milatuzumab | SEQ ID NO:1 (Combined) | HCDR1 | GYTFTNYGVN |
| | SEQ ID NO:2 (Combined) | HCDR2 | WINPNTGEPTFDDDFKG |
| | SEQ ID NO: 3 (Combined) | HCDR3 | SRGKNEAWFAY |
| | SEQ ID NO:4 (Kabat) | HCDR1 | NYGVN |
| | SEQ ID NO:2 (Kabat) | HCDR2 | WINPNTGEPTFDDDFKG |
| | SEQ ID NO: 3 (Kabat) | HCDR3 | SRGKNEAWFAY |

(continued)

| Ab | SEQ ID NO | IgG chain | Amino acid sequence |
|---|---|---|---|
| | SEQ ID NO: 5 (Chothia) | HCDR1 | GYTFTNY |
| | SEQ ID NO:6 (Chothia) | HCDR2 | NPNTGE |
| | SEQ ID NO:3 (Chothia) | HCDR3 | SRGKNEAWFAY |
| | SEQ ID NO:7 (IMGT) | HCDR1 | GYTFTNYG |
| | SEQ ID NO:8 (IMGT) | HCDR2 | INPNTGEP |
| | SEQ ID NO:9 (IMGT) | HCDR3 | SRSRGKNEAWFAY |
| | SEQ ID NO:16 (Combined) | LCDR1 | RSSQSLVHRNGNTYLH |
| | SEQ ID NO:70 (Combined) | LCDR2 | TVSNRFS |
| | SEQ ID NO:18 (Combined) | LCDR3 | SQSSHVPPT |
| | SEQ ID NO:16 (Kabat) | LCDR1 | RSSQSLVHRNGNTYLH |
| | SEQ ID NO:70 (Kabat) | LCDR2 | TVSNRFS |
| | SEQ ID NO:18 (Kabat) | LCDR3 | SQSSHVPPT |
| | SEQ ID NO:19 (Chothia) | LCDR1 | SQSLVHRNGNTY |
| | SEQ ID NO:20 (Chothia) | LCDR2 | TVS |
| | SEQ ID NO:21 (Chothia) | LCDR3 | SSHVPP |
| | SEQ ID NO:22 (IMGT) | LCDR1 | QSLVHRNGNTY |
| | SEQ ID NO:20 (IMGT) | LCDR2 | TVS |
| | SEQ ID NO:18 (IMGT) | LCDR3 | SQSSHVPPT |
| Mil_HC x hzVk1a | SEQ ID NO:1 (Combined) | HCDR1 | GYTFTNYGVN |
| | SEQ ID NO:2 (Combined) | HCDR2 | WINPNTGEPTFDDDFKG |
| | SEQ ID NO:3 (Combined) | HCDR3 | SRGKNEAWFAY |
| | SEQ ID NO:4 (Kabat) | HCDR1 | NYGVN |
| | SEQ ID NO:2 (Kabat) | HCDR2 | WINPNTGEPTFDDDFKG |
| | SEQ ID NO:3 (Kabat) | HCDR3 | SRGKNEAWFAY |
| | SEQ ID NO:5 (Chothia) | HCDR1 | GYTFTNY |
| | SEQ ID NO:6 (Chothia) | HCDR2 | NPNTGE |
| | SEQ ID NO:3 (Chothia) | HCDR3 | SRGKNEAWFAY |
| | SEQ ID NO:7 (IMGT) | HCDR1 | GYTFTNYG |
| | SEQ ID NO:8 (IMGT) | HCDR2 | INPNTGEP |
| | SEQ ID NO:9 (IMGT) | HCDR3 | SRSRGKNEAWFAY |
| | SEQ ID NO:16 (Combined) | LCDR1 | RSSQSLVHRNGNTYLH |
| | SEQ ID NO:70 (Combined) | LCDR2 | TVSNRFS |
| | SEQ ID NO:18 (Combined) | LCDR3 | SQSSHVPPT |
| | SEQ ID NO:16 (Kabat) | LCDR1 | RSSQSLVHRNGNTYLH |
| | SEQ ID NO:70 (Kabat) | LCDR2 | TVSNRFS |
| | SEQ ID NO:18 (Kabat) | LCDR3 | SQSSHVPPT |
| | SEQ ID NO:19 (Chothia) | LCDR1 | SQSLVHRNGNTY |
| | SEQ ID NO:20 (Chothia) | LCDR2 | TVS |
| | SEQ ID NO:21 (Chothia) | LCDR3 | SSHVPP |

(continued)

| Ab | SEQ ID NO | IgG chain | Amino acid sequence |
|---|---|---|---|
| | SEQ ID NO:22 (IMGT) | LCDR1 | QSLVHRNGNTY |
| | SEQ ID NO:20 (IMGT) | LCDR2 | TVS |
| | SEQ ID NO:18 (IMGT) | LCDR3 | SQSSHVPPT |
| Hcmil x LCmil_NQ | SEQ ID NO:1(Combined) | HCDR1 | GYTFTNYGVN |
| | SEQ ID NO:2 (Combined) | HCDR2 | WINPNTGEPTFDDDFKG |
| | SEQ ID NO:3 (Combined) | HCDR3 | SRGKNEAWFAY |
| | SEQ ID NO:4 (Kabat) | HCDR1 | NYGVN |
| | SEQ ID NO:2 (Kabat) | HCDR2 | WINPNTGEPTFDDDFKG |
| | SEQ ID NO:3 (Kabat) | HCDR3 | SRGKNEAWFAY |
| | SEQ ID NO:5 (Chothia) | HCDR1 | GYTFTNY |
| | SEQ ID NO:6 (Chothia) | HCDR2 | NPNTGE |
| | SEQ ID NO:3 (Chothia) | HCDR3 | SRGKNEAWFAY |
| | SEQ ID NO:7 (IMGT) | HCDR1 | GYTFTNYG |
| | SEQ ID NO:8 (IMGT) | HCDR2 | INPNTGEP |
| | SEQ ID NO:9 (IMGT) | HCDR3 | SRSRGKNEAWFAY |
| | SEQ ID NO:35 (Combined) | LCDR1 | RSSQSLVHRNQNTYLH |
| | SEQ ID NO:70 (Combined) | LCDR2 | TVSNRFS |
| | SEQ ID NO:18 (Combined) | LCDR3 | SQSSHVPPT |
| | SEQ ID NO:35 (Kabat) | LCDR1 | RSSQSLVHRNQNTYLH |
| | SEQ ID NO:70 (Kabat) | LCDR2 | TVSNRFS |
| | SEQ ID NO:18 (Kabat) | LCDR3 | SQSSHVPPT |
| | SEQ ID NO:71 (Chothia) | LCDR1 | SQSLVHRNQNTY |
| | SEQ ID NO:20 (Chothia) | LCDR2 | TVS |
| | SEQ ID NO:21 (Chothia) | LCDR3 | SSHVPP |
| | SEQ ID NO:17 (IMGT) | LCDR1 | QSLVHRNQNTY |
| | SEQ ID NO:20 (IMGT) | LCDR2 | TVS |
| | SEQ ID NO:18 (IMGT) | LCDR3 | SQSSHVPPT |
| VHmil x VK1aNQ | SEQ ID NO:1 (Combined) | HCDR1 | GYTFTNYGVN |
| | SEQ ID NO:2 (Combined) | HCDR2 | WINPNTGEPTFDDDFKG |
| | SEQ ID NO:3 (Combined) | HCDR3 | SRGKNEAWFAY |
| | SEQ ID NO:4 (Kabat) | HCDR1 | NYGVN |
| | SEQ ID NO:2 (Kabat) | HCDR2 | WINPNTGEPTFDDDFKG |
| | SEQ ID NO:3 (Kabat) | HCDR3 | SRGKNEAWFAY |
| | SEQ ID NO:5 (Chothia) | HCDR1 | GYTFTNY |
| | SEQ ID NO:6 (Chothia) | HCDR2 | NPNTGE |
| | SEQ ID NO:3 (Chothia) | HCDR3 | SRGKNEAWFAY |
| | SEQ ID NO:7 (IMGT) | HCDR1 | GYTFTNYG |
| | SEQ ID NO:8 (IMGT) | HCDR2 | INPNTGEP |
| | SEQ ID NO:9 (IMGT) | HCDR3 | SRSRGKNEAWFAY |

(continued)

| Ab | SEQ ID NO | IgG chain | Amino acid sequence |
|---|---|---|---|
| | SEQ ID NO:35 (Combined) | LCDR1 | RSSQSLVHRNQNTYLH |
| | SEQ ID NO:70 (Combined) | LCDR2 | TVSNRFS |
| | SEQ ID NO:18 (Combined) | LCDR3 | SQSSHVPPT |
| | SEQ ID NO:35 (Kabat) | LCDR1 | RSSQSLVHRNQNTYLH |
| | SEQ ID NO:70 (Kabat) | LCDR2 | TVSNRFS |
| | SEQ ID NO:18 (Kabat) | LCDR3 | SQSSHVPPT |
| | SEQ ID NO:71 (Chothia) | LCDR1 | SQSLVHRNQNTY |
| | SEQ ID NO:20 (Chothia) | LCDR2 | TVS |
| | SEQ ID NO:21 (Chothia) | LCDR3 | SSHVPP |
| | SEQ ID NO:17 (IMGT) | LCDR1 | QSLVHRNQNTY |
| | SEQ ID NO:20 (IMGT) | LCDR2 | TVS |
| | SEQ ID NO:18 (IMGT) | LCDR3 | SQSSHVPPT |
| VHmil x VK1bNQ | SEQ ID NO:1 (Combined) | HCDR1 | GYTFTNYGVN |
| | SEQ ID NO:2 (Combined) | HCDR2 | WINPNTGEPTFDDDFKG |
| | SEQ ID NO:3 (Combined) | HCDR3 | SRGKNEAWFAY |
| | SEQ ID NO:4 (Kabat) | HCDR1 | NYGVN |
| | SEQ ID NO:2 (Kabat) | HCDR2 | WINPNTGEPTFDDDFKG |
| | SEQ ID NO:3 (Kabat) | HCDR3 | SRGKNEAWFAY |
| | SEQ ID NO:5 (Chothia) | HCDR1 | GYTFTNY |
| | SEQ ID NO:6 (Chothia) | HCDR2 | NPNTGE |
| | SEQ ID NO:3 (Chothia) | HCDR3 | SRGKNEAWFAY |
| | SEQ ID NO:7 (IMGT) | HCDR1 | GYTFTNYG |
| | SEQ ID NO:8 (IMGT) | HCDR2 | INPNTGEP |
| | SEQ ID NO:9 (IMGT) | HCDR3 | SRSRGKNEAWFAY |
| | SEQ ID NO:35 (Combined) | LCDR1 | RSSQSLVHRNQNTYLH |
| | SEQ ID NO:70 (Combined) | LCDR2 | TVSNRFS |
| | SEQ ID NO:18 (Combined) | LCDR3 | SQSSHVPPT |
| | SEQ ID NO:35 (Kabat) | LCDR1 | RSSQSLVHRNQNTYLH |
| | SEQ ID NO:70 (Kabat) | LCDR2 | TVSNRFS |
| | SEQ ID NO:18 (Kabat) | LCDR3 | SQSSHVPPT |
| | SEQ ID NO:71 (Chothia) | LCDR1 | SQSLVHRNQNTY |
| | SEQ ID NO:20 (Chothia) | LCDR2 | TVS |
| | SEQ ID NO:21 (Chothia) | LCDR3 | SSHVPP |
| | SEQ ID NO:17 (IMGT) | LCDR1 | QSLVHRNQNTY |
| | SEQ ID NO:20 (IMGT) | LCDR2 | TVS |
| | SEQ ID NO:18 (IMGT) | LCDR3 | SQSSHVPPT |

**Table D. Amino acid sequence and nucleic acid sequnces of mAb variable regions**

| Ab | SEQ ID NO | IgG chain | Amino acid sequence |
|---|---|---|---|
| milatuzumab | 10 | VH | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSS |
| | 11 | DNA VH | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCT |
| | 23 | VL | DIQLTQSPLSLPVTLGQPASISCRSSQSLVHRNGNTYLHWFQQRPGQSPRLLIYTVSNRFSGVPDRFSGSGSGTDFTLKISRVEAEDVGVYFCSQSSHVPPTFGAGTRLEIK |
| | 24 | DNA VL | GACATTCAGCTGACACAGAGCCCTCTGAGCCTGCCTGTTACACTGGGACAGCCTGCCAGCATCAGCTGTAGAAGCAGCCAGAGCCTGGTGCACAGAAACGGCAACACCTACCTGCACTGGTTCCAGCAGAGGCCTGGCCAGTCTCCTAGACTGCTGATCTACACCGTGTCCAACAGATTCAGCGGCGTGCCCGATAGATTTTCCGGCAGCGGCTCTGGCACCGACTTCACCCTGAAGATTAGCAGAGTGGAAGCCGAGGACGTGGGCGTGTACTTCTGTAGCCAGTCTAGCCACGTGCCACCTACCTTTGGCGCCGGAACCAGACTGGAAATCAAG |
| Mil_HC x hzVk1a | 10 | VH | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSS |
| | SEQ ID NO:11 | DNA VH | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCT |

(continued)

| Ab | SEQ ID NO | IgG chain | Amino acid sequence |
|---|---|---|---|
| | SEQ ID NO:27 | VL | DIVMTQTPLSLPVTPGEPASISCRSSQSLVHRNGNTYLHWYLQKPGQSPQLLIYTVSNRFSGVPDRFSGSGSGTDFTLKISRVEAEDVGVYFCSQSSHVPPTFGQGTKLEIK |
| | SEQ ID NO:28 | DNA VL | GACATTGTGATGACACAGACCCCTCTGAGCCTGCCTGTGACACCTGGCGAACCTGCCAGCATCAGCTGTAGAAGCAGCCAGAGCCTGGTGCACAGAAACGGCAACACCTACCTGCACTGGTATCTGCAGAAGCCCGGCCAGTCTCCTCAGCTGCTGATCTACACCGTGTCCAACAGATTCAGCGGCGTGCCCGATAGATTTTCCGGCAGCGGCTCTGGCACCGACTTCACCCTGAAGATCTCCAGAGTGGAAGCCGAGGACGTGGGCGTGTACTTCTGTAGCCAGTCTAGCCACGTGCCACCTACCTTTGGCCAGGGGACCAAGCTGGAAATCAAG |
| Mil_HC x hzVk1b | SEQ ID NO:10 | VH | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSS |
| | SEQ ID NO:11 | DNA VH | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCT |
| | SEQ ID NO:31 | VL | DVVMTQSPLSLPVTLGQPASISCRSSQSLVHRNGNTYLHWYQQRPGQSPRLLIYTVSNRFSGVPDRFSGSGSGTDFTLKISRVEAEDVGVYFCSQSSHVPPTFGQGTKLEIK |
| | SEQ ID NO:32 | DNA VL | GATGTGGTTATGACACAGAGCCCTCTGAGCCTGCCTGTGACACTTGGACAGCCTGCCAGCATCAGCTGCAGATCTAGCCAGAGCCTGGTGCACAGAAACGGCAACACCTACCTGCACTGGTATCAGCAGAGGCCCGGACAGTCTCCCAGACTGCTGATCTACACCGTGTCCAACAGATTCAGCGGCGTGCCCGATAGATTTTCCGGCAGCGGCTCTGGCACCGACTTCACCCTGAAGATCTCCAGAGTGGAAGCCGAGGACGTGGGCGTGTACTTCTGTAGCCAGTCTAGCCACGTGCCACCTACCTTTGGCCAGGGCACCAAGCTGGAAATCAAG |

(continued)

| Ab | SEQ ID NO | IgG chain | Amino acid sequence |
|---|---|---|---|
| Hcmil x LCmil_NQ | SEQ ID NO:10 | VH | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSS |
| | SEQ ID NO:11 | DNA VH | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCT |
| | SEQ ID NO:36 | VL | DIQLTQSPLSLPVTLGQPASISCRSSQSLVHRNQNTYLHWFQQRPGQSPRLLIYTVSNRFSGVPDRFSGSGSGTDFTLKISRVEAEDVGVYFCSQSSHVPPTFGAGTRLEIK |
| | SEQ ID NO:37 | DNA VL | GACATTCAGCTGACACAGAGCCCTCTGAGCCTGCCTGTTACACTGGGACAGCCTGCCAGCATCAGCTGTAGAAGCAGCCAGAGCCTGGTGCACAGAAACCAGAACACCTACCTGCACTGGTTCCAGCAGAGGCCTGGCCAGTCTCCTAGACTGCTGATCTACACCGTGTCCAACAGATTCAGCGGCGTGCCCGATAGATTTTCCGGCAGCGGCTCTGGCACCGACTTCACCCTGAAGATTAGCAGAGTGGAAGCCGAGGACGTGGGCGTGTACTTCTGTAGCCAGTCTAGCCACGTGCCACCTACCTTTGGCGCCGGAACCAGACTGGAAATCAAG |
| VHmil x VK1aNQ | SEQ ID NO:10 | VH | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSS |
| | SEQ ID NO:11 | DNA VH | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCT |

(continued)

| Ab | SEQ ID NO | IgG chain | Amino acid sequence |
|---|---|---|---|
| | SEQ ID NO:40 | VL | DIVMTQTPLSLPVTPGEPASISCRSSQSLVHRNQNTYLH WYLQKPGQSPQLLIYTVSNRFSGVPDRFSGSGSGTDFTL KISRVEAEDVGVYFCSQSSHVPPTFGQGTKLEIK |
| | SEQ ID NO:41 | DNA VL | GACATTGTGATGACACAGACCCCTCTGAGCCTGCCTGT GACACCTGGCGAACCTGCCAGCATCAGCTGTAGAAGC AGCCAGAGCCTGGTGCACCGGAACCAGAATACCTACC TGCACTGGTATCTGCAGAAGCCCGGCCAGTCTCCTCAG CTGCTGATCTACACCGTGTCCAACAGATTCAGCGGCGT GCCCGATAGATTTTCCGGCAGCGGCTCTGGCACCGAC TTCACCCTGAAGATCTCCAGAGTGGAAGCCGAGGACG TGGGCGTGTACTTCTGTAGCCAGTCTAGCCACGTGCCA CCTACCTTTGGCCAGGGGACCAAGCTGGAAATCAAG |
| VHmil x VK1bNQ | SEQ ID NO:10 | VH | QVQLQQSGSELKKPGASVKVSCKASGYTFTNYGVNWIK QAPGQGLQWMGWINPNTGEPTFDDDFKGRFAFSLDTS VSTAYLQISSLKADDTAVYFCSRSRGKNEAWFAYWGQG TLVTVSS |
| | SEQ ID NO:11 | DNA VH | CAGGTTCAGCTCCAGCAGTCTGGCAGCGAGCTGAAAA AACCTGGCGCCTCCGTGAAGGTGTCCTGCAAGGCTTCT GGCTACACCTTTACCAACTACGGCGTGAACTGGATCAA GCAGGCCCCTGGACAAGGCCTCCAATGGATGGGCTGG ATCAACCCCAATACCGGCGAGCCCACCTTCGACGACG ATTTCAAGGGCAGATTCGCCTTCAGCCTGGACACCTCT GTGTCCACAGCCTACCTCCAGATCAGCAGCCTGAAGG CCGATGATACCGCCGTGTACTTCTGCTCCAGAAGCCGG GGAAAGAACGAGGCTTGGTTTGCCTATTGGGGCCAGG GCACACTGGTCACCGTTAGCTCT |
| | SEQ ID NO:44 | VL | DVVMTQSPLSLPVTLGQPASISCRSSQSLVHRNQNTYLH WYQQRPGQSPRLLIYTVSNRFSGVPDRFSGSGSGTDFTL KISRVEAEDVGVYFCSQSSHVPPTFGQGTKLEIK |
| | SEQ ID NO:45 | DNA VL | GATGTGGTTATGACACAGAGCCCTCTGAGCCTGCCTGT GACACTTGGACAGCCTGCCAGCATCAGCTGCAGATCT AGCCAGAGCCTGGTGCACCGGAACCAGAACACATACC TGCACTGGTATCAGCAGAGGCCCGGACAGTCTCCCAG ACTGCTGATCTACACCGTGTCCAACAGATTCAGCGGCG TGCCCGATAGATTTTCCGGCAGCGGCTCTGGCACCGA CTTCACCCTGAAGATCTCCAGAGTGGAAGCCGAGGAC GTGGGCGTGTACTTCTGTAGCCAGTCTAGCCACGTGCC ACCTACCTTTGGCCAGGGCACCAAGCTGGAAATCAAG |

**Table E. amino acid and nucleic acid sequences of full length mAb IgG chains (all Heavy Chain sequences include E152C and S375C mutations for site-directed conjugation)**

| Ab | SEQ ID NO | IgG chain | Amino acid sequence |
|---|---|---|---|
| milatuzumab | SEQ ID NO:12 | Heavy Chain (Wild Type Fc) | QVQLQQSGSELKKPGASVKVSCKASGYTFTNYGVNWIK QAPGQGLQWMGWINPNTGEPTFDDDFKGRFAFSLDTS VSTAYLQISSLKADDTAVYFCSRSRGKNEAWFAYWGQG TLVTVSSASTKGPSVFPLAPSSKSTSGGTAALGCLVKDYFP CPVTVSWNSGALTSGVHTFPAVLQSSGLYSLSSVVTVPSS SLGTQTYICNVNHKPSNTKVDKRVEPKSCDKTHTCPPCP APELLGGPSVFLFPPKPKDTLMISRTPEVTCVVVDVSHED PEVKFNWYVDGVEVHNAKTKPREEQYNSTYRVVSVLTV LHQDWLNGKEYKCKVSNKALPAPIEKTISKAKGQPREPQ VYTLPPSREEMTKNQVSLTCLVKGFYPCDIAVEWESNGQ PENNYKTTPPVLDSDGSFFLYSKLTVDKSRWQQGNVFSC SVMHEALHNHYTQKSLSLSPGK |
| | SEQ ID NO:13 | DNA Heavy Chain (Wild Type Fc) | CAGGTTCAGCTCCAGCAGTCTGGCAGCGAGCTGAAAA AACCTGGCGCCTCCGTGAAGGTGTCCTGCAAGGCTTCT GGCTACACCTTTACCAACTACGGCGTGAACTGGATCAA GCAGGCCCCTGGACAAGGCCTCCAATGGATGGGCTGG ATCAACCCCAATACCGGCGAGCCCACCTTCGACGACG ATTTCAAGGGCAGATTCGCCTTCAGCCTGGACACCTCT |

(continued)

| Ab | SEQ ID NO | IgG chain | Amino acid sequence |
|---|---|---|---|
| | | | GTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCTGCTAGCACCAAGGGCCCAAGTGTGTTTCCCCTGGCCCCCAGCAGCAAGTCTACTTCCGGCGGAACTGCTGCCCTGGGTTGCCTGGTGAAGGACTACTTCCCCTGTCCCGTGACAGTGTCCTGGAACTCTGGGGCTCTGACTTCCGGCGTGCACACCTTCCCCGCCGTGCTGCAGAGCAGCGGCCTGTACAGCCTGAGCAGCGTGGTGACAGTGCCCTCCAGCTCTCTGGGAACCCAGACCTATATCTGCAACGTGAACCACAAGCCCAGCAACACCAAGGTGGACAAGAGAGTGGAGCCCAAGAGCTGCGACAAGACCCACACCTGCCCCCCCTGCCCAGCTCCAGAACTGCTGGGAGGGCCTTCCGTGTTCCTGTTCCCCCCCAAGCCCAAGGACACCCTGATGATCAGCAGGACCCCCGAGGTGACCTGCGTGGTGGTGGACGTGTCCCACGAGGACCCAGAGGTGAAGTTCAACTGGTACGTGGACGGCGTGGAGGTGCACAACGCCAAGACCAAGCCCAGAGAGGAGCAGTACAACAGCACCTACAGGGTGGTGTCCGTGCTGACCGTGCTGCACCAGGACTGGCTGAACGGCAAAGAATACAAGTGCAAAGTCTCCAACAAGGCCCTGCCAGCCCCAATCGAAAAGACAATCAGCAAGGCCAAGGGCCAGCCACGGGAGCCCCAGGTGTACACCCTGCCCCCCAGCCGGGAGGAGATGACCAAGAACCAGGTGTCCCTGACCTGTCTGGTGAAGGGCTTCTACCCCTGTGATATCGCCGTGGAGTGGGAGAGCAACGGCCAGCCCGAGAACAACTACAAGACCACCCCCCCAGTGCTGGACAGCGACGGCAGCTTCTTCCTGTACAGCAAGCTGACCGTGGACAAGTCCAGGTGGCAGCAGGGCAACGTGTTCAGCTGCAGCGTGATGCACGAGGCCCTGCACAACCACTACACCCAGAAGTCCCTGAGCCTGAGCCCCGGCAAG |
| | SEQ ID NO:14 | Heavy Chain (Fc Silenced DAPA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |

(continued)

| Ab | SEQ ID NO | IgG chain | Amino acid sequence |
|---|---|---|---|
| | SEQ ID NO:15 | Heavy Chain (Fc Silenced DANA-PA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYASTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:25 | Light Chain | DIQLTQSPLSLPVTLGQPASISCRSSQSLVHRNGNTYLHWFQQRPGQSPRLLIYTVSNRFSGVPDRFSGSGSGTDFTLKISRVEAEDVGVYFCSQSSHVPPTFGAGTRLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | SEQ ID NO:26 | DNA Light Chain | GACATTCAGCTGACACAGAGCCCTCTGAGCCTGCCTGTTACACTGGGACAGCCTGCCAGCATCAGCTGTAGAAGCAGCCAGAGCCTGGTGCACAGAAACGGCAACACCTACCTGCACTGGTTCCAGCAGAGGCCTGGCCAGTCTCCTAGACTGCTGATCTACACCGTGTCCAACAGATTCAGCGGCGTGCCCGATAGATTTTCCGGCAGCGGCTCTGGCACCGACTTCACCCTGAAGATTAGCAGAGTGGAAGCCGAGGACGTGGGCGTGTACTTCTGTAGCCAGTCTAGCCACGTGCCACCTACCTTTGGCGCCGGAACCAGACTGGAAATCAAGCGTACGGTGGCCGCTCCCAGCGTGTTCATCTTCCCCCCCAGCGACGAGCAGCTGAAGAGTGGCACCGCCAGCGTGGTGTGCCTGCTGAACAACTTCTACCCCCGGGAGGCCAAGGTGCAGTGGAAGGTGGACAACGCCCTGCAGAGCGGCAACAGCCAGGAGAGCGTCACCGAGCAGGACAGCAAGGACTCCACCTACAGCCTGAGCAGCACCCTGACCCTGAGCAAGGCCGACTACGAGAAGCATAAGGTGTACGCCTGCGAGGTGACCCACCAGGGCCTGTCCAGCCCCGTGACCAAGAGCTTCAACAGGGGCGAGTGC |

(continued)

| Ab | SEQ ID NO | IgG chain | Amino acid sequence |
|---|---|---|---|
| Mil_HC x hzVk1a | SEQ ID NO:12 | Heavy Chain (Wild Type Fc) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:13 | DNA Heavy Chain (Wild Type Fc) | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAA |

(continued)

| Ab | SEQ ID NO | IgG chain | Amino acid sequence |
|---|---|---|---|
| | | | GCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCTGCTAGCACCAAGGGCCCAAGTGTGTTTCCCCTGGCCCCCAGCAGCAAGTCTACTTCCGGCGGAACTGCTGCCCTGGGTTGCCTGGTGAAGGACTACTTCCCCTGTCCCGTGACAGTGTCCTGGAACTCTGGGGCTCTGACTTCCGGCGTGCACACCTTCCCCGCCGTGCTGCAGAGCAGCGGCCTGTACAGCCTGAGCAGCGTGGTGACAGTGCCCTCCAGCTCTCTGGGAACCCAGACCTATATCTGCAACGTGAACCACAAGCCCAGCAACACCAAGGTGGACAAGAGAGTGGAGCCCAAGAGCTGCGACAAGACCCACACCTGCCCCCCCTGCCCAGCTCCAGAACTGCTGGGAGGGCCTTCCGTGTTCCTGTTCCCCCCCAAGCCCAAGGACACCCTGATGATCAGCAGGACCCCCGAGGTGACCTGCGTGGTGGTGGACGTGTCCCACGAGGACCCAGAGGTGAAGTTCAACTGGTACGTGGACGGCGTGGAGGTGCACAACGCCAAGACCAAGCCCAGAGAGGAGCAGTACAACAGCACCTACAGGGTGGTGTCCGTGCTGACCGTGCTGCACCAGGACTGGCTGAACGGCAAAGAATACAAGTGCAAAGTCTCCAACAAGGCCCTGCCAGCCCCAATCGAAAAGACAATCAGCAAGGCCAAGGGCCAGCCACGGGAGCCCCAGGTGTACACCCTGCCCCCCAGCCGGGAGGAGATGACCAAGAACCAGGTGTCCCTGACCTGTCTGGTGAAGGGCTTCTACCCCTGTGATATCGCCGTGGAGTGGGAGAGCAACGGCCAGCCCGAGAACAACTACAAGACCACCCCCCCAGTGCTGGACAGCGACGGCAGCTTCTTCCTGTACAGCAAGCTGACCGTGGACAAGTCCAGGTGGCAGCAGGGCAACGTGTTCAGCTGCAGCGTGATGCACGAGGCCCTGCACAACCACTACACCCAGAAGTCCCTGAGCCTGAGCCCCGGCAAG |

(continued)

| Ab | SEQ ID NO | IgG chain | Amino acid sequence |
|---|---|---|---|
| | SEQ ID NO:14 | Heavy Chain (Fc Silenced DAPA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:15 | Heavy Chain (Fc Silenced DANA-PA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYASTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:29 | Light Chain | DIVMTQTPLSLPVTPGEPASISCRSSQSLVHRNGNTYLHWYLQKPGQSPQLLIYTVSNRFSGVPDRFSGSGSGTDFTLKISRVEAEDVGVYFCSQSSHVPPTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| Ab | SEQ ID NO | IgG chain | Amino acid sequence |
|---|---|---|---|
| | SEQ ID NO:30 | DNA Light Chain | GACATTGTGATGACACAGACCCCTCTGAGCCTGCCTGTGACACCTGGCGAACCTGCCAGCATCAGCTGTAGAAGCAGCCAGAGCCTGGTGCACAGAAACGGCAACACCTACCTGCACTGGTATCTGCAGAAGCCCGGCCAGTCTCCTCAGCTGCTGATCTACACCGTGTCCAACAGATTCAGCGGCGTGCCCGATAGATTTTCCGGCAGCGGCTCTGGCACCGACTTCACCCTGAAGATCTCCAGAGTGGAAGCCGAGGACGTGGGCGTGTACTTCTGTAGCCAGTCTAGCCACGTGCCACCTACCTTTGGCCAGGGGACCAAGCTGGAAATCAAGCGTACGGTGGCCGCTCCCAGCGTGTTCATCTTCCCCCCCAGCGACGAGCAGCTGAAGAGTGGCACCGCCAGCGTGGTGTGCCTGCTGAACAACTTCTACCCCCGGGAGGCCAAGGTGCAGTGGAAGGTGGACAACGCCCTGCAGAGCGGCAACAGCCAGGAGAGCGTCACCGAGCAGGACAGCAAGGACTCCACCTACAGCCTGAGCAGCACCCTGACCCTGAGCAAGGCCGACTACGAGAAGCATAAGGTGTACGCCTGCGAGGTGACCCACCAGGGCCTGTCCAGCCCCGTGACCAAGAGCTTCAACAGGGGCGAGTGC |
| Mil_HC x hzVk1b | SEQ ID NO:12 | Heavy Chain (Wild Type Fc) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |

(continued)

| Ab | SEQ ID NO | IgG chain | Amino acid sequence |
|---|---|---|---|
| | SEQ ID NO:13 | DNA Heavy Chain (Wild Type Fc) | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCTGCTAGCACCAAGGGCCCAAGTGTGTTTCCCCTGGCCCCCAGCAGCAAGTCTACTTCCGGCGGAACTGCTGCCCTGGGTTGCCTGGTGAAGGACTACTTCCCCTGTCCCGTGACAGTGTCCTGGAACTCTGGGGCTCTGACTTCCGGCGTGCACACCTTCCCCGCCGTGCTGCAGAGCAGCGGCCTGTACAGCCTGAGCAGCGTGGTGACAGTGCCCTCCAGCTCTCTGGGAACCCAGACCTATATCTGCAACGTGAACCACAAGCCCAGCAACACCAAGGTGGACAAGAGAGTGGAGCCCAAGAGCTGCGACAAGACCCACACCTGCCCCCCCTGCCCAGCTCCAGAACTGCTGGGAGGGCCTTCCGTGTTCCTGTTCCCCCCCAAGCCCAAGGACACCCTGATGATCAGCAGGACCCCCGAGGTGACCTGCGTGGTGGTGGACGTGTCCCACGAGGACCCAGAGGTGAAGTTCAACTGGTACGTGGACGGCGTGGAGGTGCACAACGCCAAGACCAAGCCCAGAGAGGAGCAGTACAACAGCACCTACAGGGTGGTGTCCGTGCTGACCGTGCTGCACCAGGACTGGCTGAACGGCAAAGAATACAAGTGCAAAGTCTCCAACAAGGCCCTGCCAGCCCCAATCGAAAAGACAATCAGCAAGGCCAAGGGCCAGCCACGGGAGCCCCAGGTGTACACCCTGCCCCCCAGCCGGGAGGAGATGACCAAGAACCAGGTGTCCCTGACCTGTCTGGTGAAGGGCTTCTACCCCTGTGATATCGCCGTGGAGTGGGAGAGCAACGGCCAGCCCGAGAACAACTACAAGACCACCCCCCCAGTGCTGGACAGCGACGGCAGCTTCTTCCTGTACAGCAAGCTGACCGTGGACAAGTCCAGGTGGCAGCAGGGCAACGTGTTCAGCTGCAGCGTGATGCACGAGGCCCTGCACAACCACTACACCCAGAAGTCCCTGAGCCTGAGCCCCGGCAAG |

(continued)

| Ab | SEQ ID NO | IgG chain | Amino acid sequence |
|---|---|---|---|
| | SEQ ID NO:14 | Heavy Chain (Fc Silenced DAPA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:15 | Heavy Chain (Fc Silenced DANA-PA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYASTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:33 | Light Chain | DVVMTQSPLSLPVTLGQPASISCRSSQSLVHRNGNTYLHWYQQRPGQSPRLLIYTVSNRFSGVPDRFSGSGSGTDFTLKISRVEAEDVGVYFCSQSSHVPPTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| Ab | SEQ ID NO | IgG chain | Amino acid sequence |
|---|---|---|---|
| | SEQ ID NO:34 | DNA Light Chain | GATGTGGTTATGACACAGAGCCCTCTGAGCCTGCCTGTGACACTTGGACAGCCTGCCAGCATCAGCTGCAGATCTAGCCAGAGCCTGGTGCACAGAAACGGCAACACCTACCTGCACTGGTATCAGCAGAGGCCCGGACAGTCTCCCAGACTGCTGATCTACACCGTGTCCAACAGATTCAGCGGCGTGCCCGATAGATTTTCCGGCAGCGGCTCTGGCACCGACTTCACCCTGAAGATCTCCAGAGTGGAAGCCGAGGACGTGGGCGTGTACTTCTGTAGCCAGTCTAGCCACGTGCCACCTACCTTTGGCCAGGGCACCAAGCTGGAAATCAAGCGTACGGTGGCCGCTCCCAGCGTGTTCATCTTCCCCCCCAGCGACGAGCAGCTGAAGAGTGGCACCGCCAGCGTGGTGTGCCTGCTGAACAACTTCTACCCCCGGGAGGCCAAGGTGCAGTGGAAGGTGGACAACGCCCTGCAGAGCGGCAACAGCCAGGAGAGCGTCACCGAGCAGGACAGCAAGGACTCCACCTACAGCCTGAGCAGCACCCTGACCCTGAGCAAGGCCGACTACGAGAAGCATAAGGTGTACGCCTGCGAGGTGACCCACCAGGGCCTGTCCAGCCCCGTGACCAAGAGCTTCAACAGGGGCGAGTGC |
| Hcmil x LCmil_NQ | SEQ ID NO:12 | Heavy Chain (Wild Type Fc) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |

(continued)

| Ab | SEQ ID NO | IgG chain | Amino acid sequence |
|---|---|---|---|
| | SEQ ID NO:13 | DNA Heavy Chain (Wild Type Fc) | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCTGCTAGCACCAAGGGCCCAAGTGTGTTTCCCCTGGCCCCCAGCAGCAAGTCTACTTCCGGCGGAACTGCTGCCCTGGGTTGCCTGGTGAAGGACTACTTCCCCTGTCCCGTGACAGTGTCCTGGAACTCTGGGGCTCTGACTTCCGGCGTGCACACCTTCCCCGCCGTGCTGCAGAGCAGCGGCCTGTACAGCCTGAGCAGCGTGGTGACAGTGCCCTCCAGCTCTCTGGGAACCCAGACCTATATCTGCAACGTGAACCACAAGCCCAGCAACACCAAGGTGGACAAGAGAGTGGAGCCCAAGAGCTGCGACAAGACCCACACCTGCCCCCCCTGCCCAGCTCCAGAACTGCTGGGAGGGCCTTCCGTGTTCCTGTTCCCCCCCAAGCCCAAGGACACCCTGATGATCAGCAGGACCCCCGAGGTGACCTGCGTGGTGGTGGACGTGTCCCACGAGGACCCAGAGGTGAAGTTCAACTGGTACGTGGACGGCGTGGAGGTGCACAACGCCAAGACCAAGCCCAGAGAGGAGCAGTACAACAGCACCTACAGGGTGGTGTCCGTGCTGACCGTGCTGCACCAGGACTGGCTGAACGGCAAAGAATACAAGTGCAAAGTCTCCAACAAGGCCCTGCCAGCCCCAATCGAAAAGACAATCAGCAAGGCCAAGGGCCAGCCACGGGAGCCCCAGGTGTACACCCTGCCCCCCAGCCGGGAGGAGATGACCAAGAACCAGGTGTCCCTGACCTGTCTGGTGAAGGGCTTCTACCCCTGTGATATCGCCGTGGAGTGGGAGAGCAACGGCCAGCCCGAGAACAACTACAAGACCACCCCCCCAGTGCTGGACAGCGACGGCAGCTTCTTCCTGTACAGCAAGCTGACCGTGGACAAGTCCAGGTGGCAGCAGGGCAACGTGTTCAGCTGCAGCGTGATGCACGAGGCCCTGCACAACCACTACACCCAGAAGTCCCTGAGCCTGAGCCCCGGCAAG |

(continued)

| Ab | SEQ ID NO | IgG chain | Amino acid sequence |
|---|---|---|---|
| | SEQ ID NO:14 | Heavy Chain (Fc Silenced DAPA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:15 | Heavy Chain (Fc Silenced DANA-PA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYASTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:38 | Light Chain | DIQLTQSPLSLPVTLGQPASISCRSSQSLVHRNQNTYLHWFQQRPGQSPRLLIYTVSNRFSGVPDRFSGSGSGTDFTLKISRVEAEDVGVYFCSQSSHVPPTFGAGTRLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| Ab | SEQ ID NO | IgG chain | Amino acid sequence |
|---|---|---|---|
| | SEQ ID NO:39 | DNA Light Chain | GACATTCAGCTGACACAGAGCCCTCTGAGCCTGCCTGTTACACTGGGACAGCCTGCCAGCATCAGCTGTAGAAGCAGCCAGAGCCTGGTGCACAGAAACCAGAACACCTACCTGCACTGGTTCCAGCAGAGGCCTGGCCAGTCTCCTAGACTGCTGATCTACACCGTGTCCAACAGATTCAGCGGCGTGCCCGATAGATTTTCCGGCAGCGGCTCTGGCACCGACTTCACCCTGAAGATTAGCAGAGTGGAAGCCGAGGACGTGGGCGTGTACTTCTGTAGCCAGTCTAGCCACGTGCCACCTACCTTTGGCGCCGGAACCAGACTGGAAATCAAGCGTACGGTGGCCGCTCCCAGCGTGTTCATCTTCCCCCCCAGCGACGAGCAGCTGAAGAGTGGCACCGCCAGCGTGGTGTGCCTGCTGAACAACTTCTACCCCCGGGAGGCCAAGGTGCAGTGGAAGGTGGACAACGCCCTGCAGAGCGGCAACAGCCAGGAGAGCGTCACCGAGCAGGACAGCAAGGACTCCACCTACAGCCTGAGCAGCACCCTGACCCTGAGCAAGGCCGACTACGAGAAGCATAAGGTGTACGCCTGCGAGGTGACCCACCAGGGCCTGTCCAGCCCCGTGACCAAGAGCTTCAACAGGGGCGAGTGC |
| Vhmil x VK1aNQ | SEQ ID NO:12 | Heavy Chain (Wild Type Fc) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |

(continued)

| Ab | SEQ ID NO | IgG chain | Amino acid sequence |
|---|---|---|---|
| | SEQ ID NO:13 | DNA Heavy Chain (Wild Type Fc) | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCTGCTAGCACCAAGGGCCCAAGTGTGTTTCCCCTGGCCCCCAGCAGCAAGTCTACTTCCGGCGGAACTGCTGCCCTGGGTTGCCTGGTGAAGGACTACTTCCCCTGTCCCGTGACAGTGTCCTGGAACTCTGGGGCTCTGACTTCCGGCGTGCACACCTTCCCCGCCGTGCTGCAGAGCAGCGGCCTGTACAGCCTGAGCAGCGTGGTGACAGTGCCCTCCAGCTCTCTGGGAACCCAGACCTATATCTGCAACGTGAACCACAAGCCCAGCAACACCAAGGTGGACAAGAGAGTGGAGCCCAAGAGCTGCGACAAGACCCACACCTGCCCCCCCTGCCCAGCTCCAGAACTGCTGGGAGGGCCTTCCGTGTTCCTGTTCCCCCCCAAGCCCAAGGACACCCTGATGATCAGCAGGACCCCCGAGGTGACCTGCGTGGTGGTGGACGTGTCCCACGAGGACCCAGAGGTGAAGTTCAACTGGTACGTGGACGGCGTGGAGGTGCACAACGCCAAGACCAAGCCCAGAGAGGAGCAGTACAACAGCACCTACAGGGTGGTGTCCGTGCTGACCGTGCTGCACCAGGACTGGCTGAACGGCAAAGAATACAAGTGCAAAGTCTCCAACAAGGCCCTGCCAGCCCCAATCGAAAAGACAATCAGCAAGGCCAAGGGCCAGCCACGGGAGCCCCAGGTGTACACCCTGCCCCCCAGCCGGGAGGAGATGACCAAGAACCAGGTGTCCCTGACCTGTCTGGTGAAGGGCTTCTACCCCTGTGATATCGCCGTGGAGTGGGAGAGCAACGGCCAGCCCGAGAACAACTACAAGACCACCCCCCCAGTGCTGGACAGCGACGGCAGCTTCTTCCTGTACAGCAAGCTGACCGTGGACAAGTCCAGGTGGCAGCAGGGCAACGTGTTCAGCTGCAGCGTGATGCACGAGGCCCTGCACAACCACTACACCCAGAAGTCCCTGAGCCTGAGCCCCGGCAAG |
| | SEQ ID NO:14 | Heavy Chain (Fc Silenced DAPA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFP |

(continued)

| Ab | SEQ ID NO | IgG chain | Amino acid sequence |
|---|---|---|---|
| | | | CPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:15 | Heavy Chain (Fc Silenced DANA-PA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYASTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:42 | Light Chain | DIVMTQTPLSLPVTPGEPASISCRSSQSLVHRNQNTYLHWYLQKPGQSPQLLIYTVSNRFSGVPDRFSGSGSGTDFTLKISRVEAEDVGVYFCSQSSHVPPTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| Ab | SEQ ID NO | IgG chain | Amino acid sequence |
|---|---|---|---|
| | SEQ ID NO:43 | DNA Light Chain | GACATTGTGATGACACAGACCCCTCTGAGCCTGCCTGTGACACCTGGCGAACCTGCCAGCATCAGCTGTAGAAGCAGCCAGAGCCTGGTGCACCGGAACCAGAATACCTACCTGCACTGGTATCTGCAGAAGCCCGGCCAGTCTCCTCAGCTGCTGATCTACACCGTGTCCAACAGATTCAGCGGCGTGCCCGATAGATTTTCCGGCAGCGGCTCTGGCACCGACTTCACCCTGAAGATCTCCAGAGTGGAAGCCGAGGACGTGGGCGTGTACTTCTGTAGCCAGTCTAGCCACGTGCCACCTACCTTTGGCCAGGGGACCAAGCTGGAAATCAAGCGTACGGTGGCCGCTCCCAGCGTGTTCATCTTCCCCCCCAGCGACGAGCAGCTGAAGAGTGGCACCGCCAGCGTGGTGTGCCTGCTGAACAACTTCTACCCCCGGGAGGCCAAGGTGCAGTGGAAGGTGGACAACGCCCTGCAGAGCGGCAACAGCCAGGAGAGCGTCACCGAGCAGGACAGCAAGGACTCCACCTACAGCCTGAGCAGCACCCTGACCCTGAGCAAGGCCGACTACGAGAAGCATAAGGTGTACGCCTGCGAGGTGACCCACCAGGGCCTGTCCAGCCCCGTGACCAAGAGCTTCAACAGGGGCGAGTGC |
| Vhmil x VK1bNQ | SEQ ID NO:12 | Heavy Chain (Wild Type Fc) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |

(continued)

| Ab | SEQ ID NO | IgG chain | Amino acid sequence |
|---|---|---|---|
| | SEQ ID NO:13 | DNA Heavy Chain (Wild Type Fc) | CAGGTTCAGCTCCAGCAGTCTGGCAGCGAGCTGAAAAAACCTGGCGCCTCCGTGAAGGTGTCCTGCAAGGCTTCTGGCTACACCTTTACCAACTACGGCGTGAACTGGATCAAGCAGGCCCCTGGACAAGGCCTCCAATGGATGGGCTGGATCAACCCCAATACCGGCGAGCCCACCTTCGACGACGATTTCAAGGGCAGATTCGCCTTCAGCCTGGACACCTCTGTGTCCACAGCCTACCTCCAGATCAGCAGCCTGAAGGCCGATGATACCGCCGTGTACTTCTGCTCCAGAAGCCGGGGAAAGAACGAGGCTTGGTTTGCCTATTGGGGCCAGGGCACACTGGTCACCGTTAGCTCTGCTAGCACCAAGGGCCCAAGTGTGTTTCCCCTGGCCCCCAGCAGCAAGTCTACTTCCGGCGGAACTGCTGCCCTGGGTTGCCTGGTGAAGGACTACTTCCCCTGTCCCGTGACAGTGTCCTGGAACTCTGGGGCTCTGACTTCCGGCGTGCACACCTTCCCCGCCGTGCTGCAGAGCAGCGGCCTGTACAGCCTGAGCAGCGTGGTGACAGTGCCCTCCAGCTCTCTGGGAACCCAGACCTATATCTGCAACGTGAACCACAAGCCCAGCAACACCAAGGTGGACAAGAGAGTGGAGCCCAAGAGCTGCGACAAGACCCACACCTGCCCCCCCTGCCCAGCTCCAGAACTGCTGGGAGGGCCTTCCGTGTTCCTGTTCCCCCCCAAGCCCAAGGACACCCTGATGATCAGCAGGACCCCCGAGGTGACCTGCGTGGTGGTGGACGTGTCCCACGAGGACCCAGAGGTGAAGTTCAACTGGTACGTGGACGGCGTGGAGGTGCACAACGCCAAGACCAAGCCCAGAGAGGAGCAGTACAACAGCACCTACAGGGTGGTGTCCGTGCTGACCGTGCTGCACCAGGACTGGCTGAACGGCAAAGAATACAAGTGCAAAGTCTCCAACAAGGCCCTGCCAGCCCCAATCGAAAAGACAATCAGCAAGGCCAAGGGCCAGCCACGGGAGCCCCAGGTGTACACCCTGCCCCCCAGCCGGGAGGAGATGACCAAGAACCAGGTGTCCCTGACCTGTCTGGTGAAGGGCTTCTACCCCTGTGATATCGCCGTGGAGTGGGAGAGCAACGGCCAGCCCGAGAACAACTACAAGACCACCCCCCCAGTGCTGGACAGCGACGGCAGCTTCTTCCTGTACAGCAAGCTGACCGTGGACAAGTCCAGGTGGCAGCAGGGCAACGTGTTCAGCTGCAGCGTGATGCACGAGGCCCTGCACAACCACTACACCCAGAAGTCCCTGAGCCTGAGCCCCGGCAAG |
| | SEQ ID NO:14 | Heavy Chain (Fc Silenced DAPA) | QVQLQQSGSELKKPGASVKVSCKASGYTFTNYGVNWIK |

(continued)

| Ab | SEQ ID NO | IgG chain | Amino acid sequence |
|---|---|---|---|
| | | | QAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:15 | Heavy Chain (Fc Silenced DANA-PA) | QVQLQQSGSELKKPGASVKVSCKASGYTFTNYGVNWIKQAPGQGLQWMGWINPNTGEPTFDDDFKGRFAFSLDTSVSTAYLQISSLKADDTAVYFCSRSRGKNEAWFAYWGQGTLVTVSSASTKGPSVFPLAPSSKSTSGGTAALGCLVKDYFPCPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVAVSHEDPEVKFNWYVDGVEVHNAKTKPREEQYASTYRVVSVLTVLHQDWLNGKEYKCKVSNKALAAPIEKTISKAKGQPREPQVYTLPPSREEMTKNQVSLTCLVKGFYPCDIAVEWESNGQPENNYKTTPPVLDSDGSFFLYSKLTVDKSRWQQGNVFSCSVMHEALHNHYTQKSLSLSPGK |
| | SEQ ID NO:46 | Light Chain | DVVMTQSPLSLPVTLGQPASISCRSSQSLVHRNQNTYLHWYQQRPGQSPRLLIYTVSNRFSGVPDRFSGSGSGTDFTLKISRVEAEDVGVYFCSQSSHVPPTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| Ab | SEQ ID NO | IgG chain | Amino acid sequence |
|---|---|---|---|
| | SEQ ID NO:47 | DNA Light Chain | GATGTGGTTATGACACAGAGCCCTCTGAGCCTGCCTGTGACACTTGGACAGCCTGCCAGCATCAGCTGCAGATCTAGCCAGAGCCTGGTGCACCGGAACCAGAACACATACCTGCACTGGTATCAGCAGAGGCCCGGACAGTCTCCCAGACTGCTGATCTACACCGTGTCCAACAGATTCAGCGGCGTGCCCGATAGATTTTCCGGCAGCGGCTCTGGCACCGACTTCACCCTGAAGATCTCCAGAGTGGAAGCCGAGGACGTGGGCGTGTACTTCTGTAGCCAGTCTAGCCACGTGCCACCTACCTTTGGCCAGGGCACCAAGCTGGAAATCAAGCGTACGGTGGCCGCTCCCAGCGTGTTCATCTTCCCCCCCAGCGACGAGCAGCTGAAGAGTGGCACCGCCAGCGTGGTGTGCCTGCTGAACAACTTCTACCCCCGGGAGGCCAAGGTGCAGTGGAAGGTGGACAACGCCCTGCAGAGCGGCAACAGCCAGGAGAGCGTCACCGAGCAGGACAGCAAGGACTCCACCTACAGCCTGAGCAGCACCCTGACCCTGAGCAAGGCCGACTACGAGAAGCATAAGGTGTACGCCTGCGAGGTGACCCACCAGGGCCTGTCCAGCCCCGTGACCAAGAGCTTCAACAGGGGCGAGTGC |

In some embodiments, the antibody or antigen-binding fragment of an ADC disclosed herein may comprise any set of heavy and light chain variable domains listed in the tables above or a set of six CDRs from any set of heavy and light chain variable domains listed in the tables above. In some embodiments, the antibody or antigen-binding fragment of an ADC disclosed herein may comprise amino acid sequences that are conservatively modified and/or homologous to the sequences listed in the tables above, so long as the ADC retains the ability to bind to its target cancer antigen (e.g., with a $K_D$ of less than $1x10^{-8}$ M) and retains one or more functional properties of the ADCs disclosed herein (e.g., ability to internalize, bind to an antigen target, e.g., an antigen expressed on a tumor or other cancer cell, etc.).

In some embodiments, the antibody or antigen-binding fragment of an ADC disclosed herein further comprises human heavy and light chain constant domains or fragments thereof. For instance, the antibody or antigen-binding fragment of the described ADCs may comprise a human IgG heavy chain constant domain (such as an IgG1) and a human kappa or lambda light chain constant domain. In some embodiments, the antibody or antigen-binding fragment of the described ADCs comprises a human immunoglobulin G subtype 1 (IgG1) heavy chain constant domain with a human Ig kappa light chain constant domain.

In some embodiments, the anti-CD74 antibody or antigen-binding fragment thereof comprises three heavy chain CDRs and three light chain CDRs as follows: heavy chain CDR1 (HCDR1) consisting of SEQ ID NO:1, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:16, light chain CDR2 (LCDR2) consisting of SEQ ID NO:70, and light chain CDR3 (LCDR3) consisting of SEQ ID NO: 18.

In some embodiments, the anti-CD74 antibody or antigen-binding fragment thereof comprises three heavy chain CDRs and three light chain CDRs as follows: heavy chain CDR1 (HCDR1) consisting of SEQ ID NO:4, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:16, light chain CDR2 (LCDR2) consisting of SEQ ID NO:70, and light chain CDR3 (LCDR3) consisting of SEQ ID NO:18.

In some embodiments, the anti-CD74 antibody or antigen-binding fragment thereof comprises three heavy chain CDRs and three light chain CDRs as follows: heavy chain CDR1 (HCDR1) consisting of SEQ ID NO:5, heavy chain CDR2 (HCDR2) consisting of SEQ ID NO:6, heavy chain CDR3 (HCDR3) consisting of SEQ ID NO:3; light chain CDR1 (LCDR1) consisting of SEQ ID NO:19, light chain CDR2 (LCDR2) consisting of SEQ ID NO:20, and light chain CDR3 (LCDR3) consisting of SEQ ID NO:21.

In some embodiments, the anti-CD74 antibody or antigen-binding fragment thereof comprises three heavy chain CDRs and three light chain CDRs as follows: heavy chain CDR1 (HCDR1) consisting of SEQ ID NO:7, heavy chain CDR2

(HCDR2) consisting of SEQ ID NO:8, heavy chain CDR3 (HCDR3) consisting of SEQ ID NO:9; light chain CDR1 (LCDR1) consisting of SEQ ID NO:22, light chain CDR2 (LCDR2) consisting of SEQ ID NO:20, and light chain CDR3 (LCDR3) consisting of SEQ ID NO: 18.

In some embodiments, the anti-CD74 antibody or antigen-binding fragment thereof comprises three heavy chain CDRs and three light chain CDRs as follows: heavy chain CDR1 (HCDR1) consisting of SEQ ID NO:1, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:35, light chain CDR2 (LCDR2) consisting of SEQ ID NO:70, and light chain CDR3 (LCDR3) consisting of SEQ ID NO:18.

In some embodiments, the anti-CD74 antibody or antigen-binding fragment thereof comprises three heavy chain CDRs and three light chain CDRs as follows: heavy chain CDR1 (HCDR1) consisting of SEQ ID NO:4, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:35, light chain CDR2 (LCDR2) consisting of SEQ ID NO:70, and light chain CDR3 (LCDR3) consisting of SEQ ID NO:18.

In some embodiments, the anti-CD74 antibody or antigen-binding fragment thereof comprises three heavy chain CDRs and three light chain CDRs as follows: heavy chain CDR1 (HCDR1) consisting of SEQ ID NO:5, heavy chain CDR2 (HCDR2) consisting of SEQ ID NO:6, heavy chain CDR3 (HCDR3) consisting of SEQ ID NO:3; light chain CDR1 (LCDR1) consisting of SEQ ID NO:71, light chain CDR2 (LCDR2) consisting of SEQ ID NO:20, and light chain CDR3 (LCDR3) consisting of SEQ ID NO:21.

In some embodiments, the anti-CD74 antibody or antigen-binding fragment thereof comprises three heavy chain CDRs and three light chain CDRs as follows: heavy chain CDR1 (HCDR1) consisting of SEQ ID NO:7, heavy chain CDR2 (HCDR2) consisting of SEQ ID NO:8, heavy chain CDR3 (HCDR3) consisting of SEQ ID NO:9; light chain CDR1 (LCDR1) consisting of SEQ ID NO:17, light chain CDR2 (LCDR2) consisting of SEQ ID NO:20, and light chain CDR3 (LCDR3) consisting of SEQ ID NO:18.

In some embodiments, the anti-CD74 antibody or antigen-binding fragment thereof comprises a heavy chain variable region comprising the amino acid sequence of SEQ ID NO:10, and a light chain variable region comprising the amino acid sequence of SEQ ID NO:23. In some embodiments, the anti-CD74 antibody or antigen-binding fragment thereof comprises the heavy chain variable region amino acid sequence of SEQ ID NO:10 and the light chain variable region amino acid sequence of SEQ ID NO:23, or sequences that are at least 95% identical to the disclosed sequences. In some embodiments, the anti-CD74 antibody or antigen-binding fragment thereof has a heavy chain variable region amino acid sequence that is at least 96%, at least 97%, at least 98%, or at least 99% identical to SEQ ID NO:10 and/or a light chain variable region amino acid sequence that is at least 96%, at least 97%, at least 98%, or at least 99% identical to SEQ ID NO:23.

In some embodiments, the anti-CD74 antibody or antigen-binding fragment thereof comprises a heavy chain variable region comprising the amino acid sequence of SEQ ID NO:10, and a light chain variable region comprising the amino acid sequence of SEQ ID NO:27. In some embodiments, the anti-CD74 antibody or antigen-binding fragment thereof comprises the heavy chain variable region amino acid sequence of SEQ ID NO:10 and the light chain variable region amino acid sequence of SEQ ID NO:27, or sequences that are at least 95% identical to the disclosed sequences. In some embodiments, the anti-CD74 antibody or antigen-binding fragment thereof has a heavy chain variable region amino acid sequence that is at least 96%, at least 97%, at least 98%, or at least 99% identical to SEQ ID NO:10 and/or a light chain variable region amino acid sequence that is at least 96%, at least 97%, at least 98%, or at least 99% identical to SEQ ID NO:27.

In some embodiments, the anti-CD74 antibody or antigen-binding fragment thereof comprises a heavy chain variable region comprising the amino acid sequence of SEQ ID NO:10, and a light chain variable region comprising the amino acid sequence of SEQ ID NO:31. In some embodiments, the anti-CD74 antibody or antigen-binding fragment thereof comprises the heavy chain variable region amino acid sequence of SEQ ID NO:10 and the light chain variable region amino acid sequence of SEQ ID NO:31, or sequences that are at least 95% identical to the disclosed sequences. In some embodiments, the anti-CD74 antibody or antigen-binding fragment thereof has a heavy chain variable region amino acid sequence that is at least 96%, at least 97%, at least 98%, or at least 99% identical to SEQ ID NO:10 and/or a light chain variable region amino acid sequence that is at least 96%, at least 97%, at least 98%, or at least 99% identical to SEQ ID NO:31.

In some embodiments, the anti-CD74 antibody or antigen-binding fragment thereof comprises a heavy chain variable region comprising the amino acid sequence of SEQ ID NO:10, and a light chain variable region comprising the amino acid sequence of SEQ ID NO:36. In some embodiments, the anti-CD74 antibody or antigen-binding fragment thereof comprises the heavy chain variable region amino acid sequence of SEQ ID NO:10 and the light chain variable region amino acid sequence of SEQ ID NO:36, or sequences that are at least 95% identical to the disclosed sequences. In some embodiments, the anti-CD74 antibody or antigen-binding fragment thereof has a heavy chain variable region amino acid sequence that is at least 96%, at least 97%, at least 98%, or at least 99% identical to SEQ ID NO:10 and/or a light chain variable region amino acid sequence that is at least 96%, at least 97%, at least 98%, or at least 99% identical to SEQ ID

NO:36.

In some embodiments, the anti-CD74 antibody or antigen-binding fragment thereof comprises a heavy chain variable region comprising the amino acid sequence of SEQ ID NO:10, and a light chain variable region comprising the amino acid sequence of SEQ ID NO:40. In some embodiments, the anti-CD74 antibody or antigen-binding fragment thereof comprises the heavy chain variable region amino acid sequence of SEQ ID NO:10 and the light chain variable region amino acid sequence of SEQ ID NO:40, or sequences that are at least 95% identical to the disclosed sequences. In some embodiments, the anti-CD74 antibody or antigen-binding fragment thereof has a heavy chain variable region amino acid sequence that is at least 96%, at least 97%, at least 98%, or at least 99% identical to SEQ ID NO:10 and/or a light chain variable region amino acid sequence that is at least 96%, at least 97%, at least 98%, or at least 99% identical to SEQ ID NO:40.

In some embodiments, the anti-CD74 antibody or antigen-binding fragment thereof comprises a heavy chain variable region comprising the amino acid sequence of SEQ ID NO:10, and a light chain variable region comprising the amino acid sequence of SEQ ID NO:44. In some embodiments, the anti-CD74 antibody or antigen-binding fragment thereof comprises the heavy chain variable region amino acid sequence of SEQ ID NO:10 and the light chain variable region amino acid sequence of SEQ ID NO:44, or sequences that are at least 95% identical to the disclosed sequences. In some embodiments, the anti-CD74 antibody or antigen-binding fragment thereof has a heavy chain variable region amino acid sequence that is at least 96%, at least 97%, at least 98%, or at least 99% identical to SEQ ID NO:10 and/or a light chain variable region amino acid sequence that is at least 96%, at least 97%, at least 98%, or at least 99% identical to SEQ ID NO:44.

In some embodiments, the anti-CD74 antibody comprises the heavy chain amino acid sequence of SEQ ID NO:12 or a sequence that is at least 95% identical to SEQ ID NO:12, and the light chain amino acid sequence of SEQ ID NO:25 or a sequence that is at least 95% identical to SEQ ID NO:25. In some embodiments, the anti-CD74 antibody comprises the heavy chain amino acid sequence of SEQ ID NO:12 and the light chain amino acid sequence of SEQ ID NO:25, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:12 and a light chain amino acid sequence that is at least 96%, at least 97%, at least 98%, or at least 99% identical to SEQ ID NO:25. In some embodiments, the anti-CD74 antibody is milatuzumab (see US Patent No. 7931903), or an antigen-binding fragment thereof. In some embodiments, the anti-CD74 antibody comprises the heavy chain amino acid sequence of SEQ ID NO:14 or a sequence that is at least 95% identical to SEQ ID NO:14, and the light chain amino acid sequence of SEQ ID NO:25 or a sequence that is at least 95% identical to SEQ ID NO:25. In some embodiments, the anti-CD74 antibody comprises the heavy chain amino acid sequence of SEQ ID NO:14 and the light chain amino acid sequence of SEQ ID NO:25, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:14 and a light chain amino acid sequence that is at least 96%, at least 97%, at least 98%, or at least 99% identical to SEQ ID NO:25.

In some embodiments, the anti-CD74 antibody comprises the heavy chain amino acid sequence of SEQ ID NO: 15 or a sequence that is at least 95% identical to SEQ ID NO:15, and the light chain amino acid sequence of SEQ ID NO:25 or a sequence that is at least 95% identical to SEQ ID NO:25. In some embodiments, the anti-CD74 antibody comprises the heavy chain amino acid sequence of SEQ ID NO:15 and the light chain amino acid sequence of SEQ ID NO:25, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:15 and a light chain amino acid sequence that is at least 96%, at least 97%, at least 98%, or at least 99% identical to SEQ ID NO:25.

In some embodiments, the anti-CD74 antibody comprises the heavy chain amino acid sequence of SEQ ID NO:12 or a sequence that is at least 95% identical to SEQ ID NO:12, and the light chain amino acid sequence of SEQ ID NO:29 or a sequence that is at least 95% identical to SEQ ID NO:29. In some embodiments, the anti-CD74 antibody comprises the heavy chain amino acid sequence of SEQ ID NO:12 and the light chain amino acid sequence of SEQ ID NO:29, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:12 and a light chain amino acid sequence that is at least 96%, at least 97%, at least 98%, or at least 99% identical to SEQ ID NO:29.

In some embodiments, the anti-CD74 antibody comprises the heavy chain amino acid sequence of SEQ ID NO:14 or a sequence that is at least 95% identical to SEQ ID NO:14, and the light chain amino acid sequence of SEQ ID NO:29 or a sequence that is at least 95% identical to SEQ ID NO:29. In some embodiments, the anti-CD74 antibody comprises the heavy chain amino acid sequence of SEQ ID NO:14 and the light chain amino acid sequence of SEQ ID NO:29, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:14 and a light chain amino acid sequence that is at least 96%, at least 97%, at least 98%, or at least 99%

identical to SEQ ID NO:29.

In some embodiments, the anti-CD74 antibody comprises the heavy chain amino acid sequence of SEQ ID NO: 15 or a sequence that is at least 95% identical to SEQ ID NO:15, and the light chain amino acid sequence of SEQ ID NO:29 or a sequence that is at least 95% identical to SEQ ID NO:29. In some embodiments, the anti-CD74 antibody comprises the heavy chain amino acid sequence of SEQ ID NO:15 and the light chain amino acid sequence of SEQ ID NO:29, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:15 and a light chain amino acid sequence that is at least 96%, at least 97%, at least 98%, or at least 99% identical to SEQ ID NO:29.

In some embodiments, the anti-CD74 antibody comprises the heavy chain amino acid sequence of SEQ ID NO:12 or a sequence that is at least 95% identical to SEQ ID NO:12, and the light chain amino acid sequence of SEQ ID NO:33 or a sequence that is at least 95% identical to SEQ ID NO:33. In some embodiments, the anti-CD74 antibody comprises the heavy chain amino acid sequence of SEQ ID NO:12 and the light chain amino acid sequence of SEQ ID NO:33, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:12 and a light chain amino acid sequence that is at least 96%, at least 97%, at least 98%, or at least 99% identical to SEQ ID NO:33.

In some embodiments, the anti-CD74 antibody comprises the heavy chain amino acid sequence of SEQ ID NO:14 or a sequence that is at least 95% identical to SEQ ID NO:14, and the light chain amino acid sequence of SEQ ID NO:33 or a sequence that is at least 95% identical to SEQ ID NO:33. In some embodiments, the anti-CD74 antibody comprises the heavy chain amino acid sequence of SEQ ID NO:14 and the light chain amino acid sequence of SEQ ID NO:33, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:14 and a light chain amino acid sequence that is at least 96%, at least 97%, at least 98%, or at least 99% identical to SEQ ID NO:33.

In some embodiments, the anti-CD74 antibody comprises the heavy chain amino acid sequence of SEQ ID NO: 15 or a sequence that is at least 95% identical to SEQ ID NO:15, and the light chain amino acid sequence of SEQ ID NO:33 or a sequence that is at least 95% identical to SEQ ID NO:33. In some embodiments, the anti-CD74 antibody comprises the heavy chain amino acid sequence of SEQ ID NO: 15 and the light chain amino acid sequence of SEQ ID NO:33, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:15 and a light chain amino acid sequence that is at least 96%, at least 97%, at least 98%, or at least 99% identical to SEQ ID NO:33.

In some embodiments, the anti-CD74 antibody comprises the heavy chain amino acid sequence of SEQ ID NO:12 or a sequence that is at least 95% identical to SEQ ID NO:12, and the light chain amino acid sequence of SEQ ID NO:38 or a sequence that is at least 95% identical to SEQ ID NO:38. In some embodiments, the anti-CD74 antibody comprises the heavy chain amino acid sequence of SEQ ID NO:12 and the light chain amino acid sequence of SEQ ID NO:38, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:12 and a light chain amino acid sequence that is at least 96%, at least 97%, at least 98%, or at least 99% identical to SEQ ID NO:38.

In some embodiments, the anti-CD74 antibody comprises the heavy chain amino acid sequence of SEQ ID NO:14 or a sequence that is at least 95% identical to SEQ ID NO:14, and the light chain amino acid sequence of SEQ ID NO:38 or a sequence that is at least 95% identical to SEQ ID NO:38. In some embodiments, the anti-CD74 antibody comprises the heavy chain amino acid sequence of SEQ ID NO:14 and the light chain amino acid sequence of SEQ ID NO:38, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:14 and a light chain amino acid sequence that is at least 96%, at least 97%, at least 98%, or at least 99% identical to SEQ ID NO:38.

In some embodiments, the anti-CD74 antibody comprises the heavy chain amino acid sequence of SEQ ID NO: 15 or a sequence that is at least 95% identical to SEQ ID NO:15, and the light chain amino acid sequence of SEQ ID NO:38 or a sequence that is at least 95% identical to SEQ ID NO:38. In some embodiments, the anti-CD74 antibody comprises the heavy chain amino acid sequence of SEQ ID NO: 15 and the light chain amino acid sequence of SEQ ID NO:38, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:15 and a light chain amino acid sequence that is at least 96%, at least 97%, at least 98%, or at least 99% identical to SEQ ID NO:38.

In some embodiments, the anti-CD74 antibody comprises the heavy chain amino acid sequence of SEQ ID

NO:12 or a sequence that is at least 95% identical to SEQ ID NO:12, and the light chain amino acid sequence of SEQ ID NO:42 or a sequence that is at least 95% identical to SEQ ID NO:42. In some embodiments, the anti-CD74 antibody comprises the heavy chain amino acid sequence of SEQ ID NO:12 and the light chain amino acid sequence of SEQ ID NO:42, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:12 and a light chain amino acid sequence that is at least 96%, at least 97%, at least 98%, or at least 99% identical to SEQ ID NO:42.

In some embodiments, the anti-CD74 antibody comprises the heavy chain amino acid sequence of SEQ ID NO:14 or a sequence that is at least 95% identical to SEQ ID NO:14, and the light chain amino acid sequence of SEQ ID NO:42 or a sequence that is at least 95% identical to SEQ ID NO:42. In some embodiments, the anti-CD74 antibody comprises the heavy chain amino acid sequence of SEQ ID NO:14 and the light chain amino acid sequence of SEQ ID NO:42, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:14 and a light chain amino acid sequence that is at least 96%, at least 97%, at least 98%, or at least 99% identical to SEQ ID NO:42.

In some embodiments, the anti-CD74 antibody comprises the heavy chain amino acid sequence of SEQ ID NO: 15 or a sequence that is at least 95% identical to SEQ ID NO:15, and the light chain amino acid sequence of SEQ ID NO:42 or a sequence that is at least 95% identical to SEQ ID NO:42. In some embodiments, the anti-CD74 antibody comprises the heavy chain amino acid sequence of SEQ ID NO: 15 and the light chain amino acid sequence of SEQ ID NO:42, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:15 and a light chain amino acid sequence that is at least 96%, at least 97%, at least 98%, or at least 99% identical to SEQ ID NO:42.

In some embodiments, the anti-CD74 antibody comprises the heavy chain amino acid sequence of SEQ ID NO:12 or a sequence that is at least 95% identical to SEQ ID NO:12, and the light chain amino acid sequence of SEQ ID NO:46 or a sequence that is at least 95% identical to SEQ ID NO:46. In some embodiments, the anti-CD74 antibody comprises the heavy chain amino acid sequence of SEQ ID NO:12 and the light chain amino acid sequence of SEQ ID NO:46, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:12 and a light chain amino acid sequence that is at least 96%, at least 97%, at least 98%, or at least 99% identical to SEQ ID NO:46.

In some embodiments, the anti-CD74 antibody comprises the heavy chain amino acid sequence of SEQ ID NO:14 or a sequence that is at least 95% identical to SEQ ID NO:14, and the light chain amino acid sequence of SEQ ID NO:46 or a sequence that is at least 95% identical to SEQ ID NO:46. In some embodiments, the anti-CD74 antibody comprises the heavy chain amino acid sequence of SEQ ID NO:14 and the light chain amino acid sequence of SEQ ID NO:46, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:14 and a light chain amino acid sequence that is at least 96%, at least 97%, at least 98%, or at least 99% identical to SEQ ID NO:46.

In some embodiments, the anti-CD74 antibody comprises the heavy chain amino acid sequence of SEQ ID NO: 15 or a sequence that is at least 95% identical to SEQ ID NO:15, and the light chain amino acid sequence of SEQ ID NO:46 or a sequence that is at least 95% identical to SEQ ID NO:46. In some embodiments, the anti-CD74 antibody comprises the heavy chain amino acid sequence of SEQ ID NO:15 and the light chain amino acid sequence of SEQ ID NO:46, or sequences that are at least 95% identical to the disclosed sequences. In some embodiments, the anti-CD74 antibody has a heavy chain amino acid sequence that is at least 96%, at least 97%, at least 98%, or at least 99% identical to SEQ ID NO:15 and a light chain amino acid sequence that is at least 96%, at least 97%, at least 98%, or at least 99% identical to SEQ ID NO:46.

Residues in two or more polypeptides are said to "correspond" if the residues occupy an analogous position in the polypeptide structures. Analogous positions in two or more polypeptides can be determined by aligning the polypeptide sequences based on amino acid sequence or structural similarities. Those skilled in the art understand that it may be necessary to introduce gaps in either sequence to produce a satisfactory alignment.

In some embodiments, amino acid substitutions are of single residues. Insertions usually will be on the order of from about 1 to about 20 amino acid residues, although considerably larger insertions may be tolerated as long as biological function is retained (e.g., binding to a target antigen). Deletions usually range from about 1 to about 20 amino acid residues, although in some cases deletions may be much larger. Substitutions, deletions, insertions, or any combination thereof may be used to arrive at a final derivative or variant. Generally, these changes are done on a few amino acids to minimize the alteration of the molecule, particularly the immunogenicity and specificity of the antigen binding protein. However, larger changes may be tolerated in certain circumstances. Conservative substitutions can be

made in accordance with the following chart depicted as Table 1.

**Table 1**

| Original Residue | Exemplary Substitutions |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

In some embodiments where variant antibody sequences are used in an ADC, the variants typically exhibit the same qualitative biological activity and will elicit the same immune response, although variants may also be selected to modify the characteristics of the antigen binding proteins as needed. Alternatively, the variant may be designed such that the biological activity of the antigen binding protein is altered. For example, glycosylation sites may be altered or removed.

The immunoconjugates of the invention may comprise modified antibodies or antigen binding fragments thereof that further comprise modifications to framework residues within VH and/or VL, *e.g.* to improve the properties of the antibody. In some embodiments, the framework modifications are made to decrease the immunogenicity of the antibody. For example, one approach is to "back-mutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "back-mutated" to the germline sequence by, for example, site-directed mutagenesis. Such "back-mutated" antibodies are also intended to be encompassed by the invention.

Another type of framework modification involves mutating one or more residues within the framework region, or even within one or more CDR regions, to remove T-cell epitopes to thereby reduce the potential immunogenicity of the antibody. This approach is also referred to as "deimmunization" and is described in further detail in U.S. Patent Publication No. 20030153043 by Carr et al.

In addition or in the alternative to modifications made within the framework or CDR regions, antibodies of the invention may be engineered to include modifications within the Fc region, typically to alter one or more functional properties of the antibody, such as serum half-life, complement fixation, Fc receptor binding, and/or antigen-dependent cellular cytotoxicity (ADCC). Furthermore, an antibody of the invention may be chemically modified (e.g., one or more chemical moieties can be attached to the antibody) or be modified to alter its glycosylation, again to alter one or more functional properties of the antibody. Each of these embodiments is described in further detail below.

In one embodiment, the hinge region of CH1 is modified such that the number of cysteine residues in the hinge region is altered, e.g., increased or decreased. This approach is described further in U.S. Patent No. 5,677,425 by Bodmer et al. The number of cysteine residues in the hinge region of CH1 is altered to, for example, facilitate assembly of the light and heavy chains or to increase or decrease the stability of the antibody.

In some embodiments, the antibody or antibody fragment disclosed herein include modified or engineered amino acid residues, e.g., one or more cysteine residues, as sites for conjugation to a drug moiety (Junutula JR, et al., Nat Biotechnol 2008, 26:925-932). In one embodiment, the invention provides a modified antibody or antibody fragment

comprising a substitution of one or more amino acids with cysteine at the positions described herein. Sites for cysteine substitution are in the constant regions of the antibody or antibody fragment and are thus applicable to a variety of antibody or antibody fragment, and the sites are selected to provide stable and homogeneous conjugates. A modified antibody or fragment can have one, two or more cysteine substitutions, and these substitutions can be used in combination with other modification and conjugation methods as described herein. Methods for inserting cysteine at specific locations of an antibody are known in the art, see, *e.g.,* Lyons et al., (1990) Protein Eng., 3:703-708, WO 2011/005481, WO2014/124316, WO 2015/138615. In certain embodiments, a modified antibody comprises a substitution of one or more amino acids with cysteine on its constant region selected from positions 117, 119, 121, 124, 139, 152, 153, 155, 157, 164, 169, 171, 174, 189, 191, 195, 197, 205, 207, 246, 258, 269, 274, 286, 288, 290, 292, 293, 320, 322, 326, 333, 334, 335, 337, 344, 355, 360, 375, 382, 390, 392, 398, 400 and 422 of a heavy chain of the antibody, and wherein the positions are numbered according to the EU system. In some embodiments a modified antibody or antibody fragment comprises a substitution of one or more amino acids with cysteine on its constant region selected from positions 107, 108, 109, 114, 129, 142, 143, 145, 152, 154, 156, 159, 161, 165, 168, 169, 170, 182, 183, 197, 199, and 203 of a light chain of the antibody or antibody fragment, wherein the positions are numbered according to the EU system, and wherein the light chain is a human kappa light chain. In certain embodiments a modified antibody or antibody fragment thereof comprises a combination of substitution of two or more amino acids with cysteine on its constant regions wherein the combinations comprise substitutions at positions 375 of an antibody heavy chain, position 152 of an antibody heavy chain, position 360 of an antibody heavy chain, or position 107 of an antibody light chain and wherein the positions are numbered according to the EU system. In certain embodiments a modified antibody or antibody fragment thereof comprises a substitution of one amino acid with cysteine on its constant regions wherein the substitution is position 375 of an antibody heavy chain, position 152 of an antibody heavy chain, position 360 of an antibody heavy chain, position 107 of an antibody light chain, position 165 of an antibody light chain or position 159 of an antibody light chain and wherein the positions are numbered according to the EU system, and wherein the light chain is a kappa chain. In particular embodiments a modified antibody or antibody fragment thereof comprises a combination of substitution of two amino acids with cysteine on its constant regions wherein the combinations comprise substitutions at positions 375 of an antibody heavy chain and position 152 of an antibody heavy chain, wherein the positions are numbered according to the EU system. In particular embodiments a modified antibody or antibody fragment thereof comprises a substitution of one amino acid with cysteine at position 360 of an antibody heavy chain, wherein the positions are numbered according to the EU system. In other particular embodiments a modified antibody or antibody fragment thereof comprises a substitution of one amino acid with cysteine at position 107 of an antibody light chain and wherein the positions are numbered according to the EU system, and wherein the light chain is a kappa chain.

In additional embodiments antibodies or antibody fragments (e.g., antigen binding fragment) useful in immunoconjugates of the invention include modified or engineered antibodies, such as an antibody modified to introduce one or more other reactive amino acid (other than cysteine), including Pcl, pyrrolysine, peptide tags (such as S6, A1 and ybbR tags), and non-natural amino acids, in place of at least one amino acid of the native sequence, thus providing a reactive site on the antibody or antigen binding fragment for conjugation to a drug moiety or a linker-drug moiety with complementary reactivity. For example, the antibodies or antibody fragments can be modified to incorporate Pcl or pyrrolysine (W. Ou, et al., (2011) PNAS 108 (26), 10437-10442; WO2014124258) or unnatural amino acids (J.Y. Axup, et al., Proc Natl Acad Sci U S A, 109 (2012), pp. 16101-16106; for review, see C.C. Liu and P.G. Schultz (2010) Annu Rev Biochem 79, 413-444; C.H. Kim, et al., (2013) Curr Opin Chem Biol. 17, 412-419) as sites for conjugation to a drug. Similarly, peptide tags for enzymatic conjugation methods can be introduced into an antibody (Strop P., et al., Chem Biol. 2013, 20(2):161-7; Rabuka D., Curr Opin Chem Biol. 2010 Dec;14(6):790-6; Rabuka D, et al., Nat Protoc. 2012, 7(6):1052-67). One other example is the use of 4'-phosphopantetheinyl transferases (PPTase) for the conjugation of Co-enzyme A analogs (WO2013184514), and (Grünewald et al., (2015) Bioconjugate Chem. 26 (12), 2554-62). Methods for conjugating such modified or engineered antibodies with payloads or linker-payload combinations are known in the art.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Patent No. 6,165,745 by Ward et al.

In yet other embodiments, the Fc region is altered by replacing at least one amino acid residue with a different amino acid residue to alter the effector functions of the antibody. For example, one or more amino acids can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in, *e.g.,* U.S. Patent Nos. 5,624,821 and 5,648,260, both by Winter et al.

In another embodiment, one or more amino acids selected from amino acid residues can be replaced with a different amino acid residue such that the antibody has altered C1q binding and/or reduced or abolished complement dependent cytotoxicity (CDC). This approach is described in, *e.g.,* U.S. Patent Nos. 6,194,551 by Idusogie et al.

In another embodiment, one or more amino acid residues are altered to thereby alter the ability of the antibody to fix complement. This approach is described in, e.g., the PCT Publication WO 94/29351 by Bodmer et al. Allotypic amino acid residues include, but are not limited to, constant region of a heavy chain of the IgG1, IgG2, and IgG3 subclasses as well as constant region of a light chain of the kappa isotype as described by Jefferis et al., MAbs. 1:332-338 (2009).

In some embodiments, the antibodies comprise mutations that mediate reduced or no antibody-dependent cellular cytotoxicity (ADCC) or complement-dependent cytotoxicity (CDC). In some embodiments, these mutations are known as Fc Silencing, Fc Silent, or Fc Silenced mutations. In some embodiments, amino acid residues L234 and L235 of the IgG1 constant region are substituted to A234 and A235 (also known as "LALA"). In some embodiments, amino acid residue N297 of the IgG1 constant region is substituted to A297 (also known as "N297A"). In some embodiments, amino acid residues D265 and P329 of the IgG1 constant region are substituted to A265 and A329 (also known as "DAPA"). Other antibody Fc silencing mutations may also be used. In some embodiments, the Fc silencing mutations are used in combination, for example D265A, N297A and P329A (also known as "DANAPA").

In another embodiment, one or more amino acid residues are altered to thereby alter the ability of the antibody to fix complement. This approach is described in, e.g., the PCT Publication WO 94/29351 by Bodmer et al. In a specific embodiment, one or more amino acids of an antibody or antigen binding fragment thereof of the present invention are replaced by one or more allotypic amino acid residues. Allotypic amino acid residues also include, but are not limited to, the constant region of the heavy chain of the IgG1, IgG2, and IgG3 subclasses as well as the constant region of the light chain of the kappa isotype as described by Jefferis et al., MAbs. 1:332-338 (2009).

In still another embodiment, the glycosylation of an antibody is modified. For example, an aglycosylated antibody can be made *(i.e.,* the antibody lacks glycosylation). Glycosylation can be altered to, for example, increase the affinity of the antibody for "antigen." Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in, *e.g.,* U.S. Patent Nos. 5,714,350 and 6,350,861 by Co et al.

In another embodiment, the antibody is modified to increase its biological half-life. Various approaches are possible. For example, one or more of the following mutations can be introduced: T252L, T254S, T256F, as described in U.S. Patent No. 6,277,375 to Ward. Alternatively, to increase the biological half-life, the antibody can be altered within the CH1 or CL region to contain a salvage receptor binding epitope taken from two loops of a CH2 domain of an Fc region of an IgG, as described in U.S. Patent Nos. 5,869,046 and 6,121,022 by Presta et al.

## Linkers

In some embodiments, the linker in an ADC is stable extracellularly in a sufficient manner to be therapeutically effective. In some embodiments, the linker is stable outside a cell, such that the ADC remains intact when present in extracellular conditions (e.g., prior to transport or delivery into a cell). The term "intact," used in the context of an ADC, means that the antibody or antigen-binding fragment remains attached to the drug moiety (e.g., the Mcl-1 inhibitor).

As used herein, "stable," in the context of a linker or ADC comprising a linker, means that no more than 20%, no more than about 15%, no more than about 10%, no more than about 5%, no more than about 3%, or no more than about 1% of the linkers (or any percentage in between) in a sample of ADC are cleaved (or in the case of an overall ADC are otherwise not intact) when the ADC is present in extracellular conditions. In some embodiments, the linkers and/or ADCs disclosed herein are stable compared to alternate linkers and/or ADCs with alternate linkers and/or Mcl-1 inhibitor payloads. In some embodiments, the ADCs disclosed herein can remain intact for more than about 48 hours, more than 60 hours, more than about 72 hours, more than about 84 hours, or more than about 96 hours.

Whether a linker is stable extracellularly can be determined, for example, by including an ADC in plasma for a predetermined time period (e.g., 2, 4, 6, 8, 16, 24, 48, or 72 hours) and then quantifying the amount of free drug moiety present in the plasma. Stability may allow the ADC time to localize to target cancer cells and prevent the premature release of the drug moiety, which could lower the therapeutic index of the ADC by indiscriminately damaging both normal and cancer tissues. In some embodiments, the linker is stable outside of a target cell and releases the drug moiety from the ADC once inside of the cell, such that the drug can bind to its target. Thus, an effective linker will: (i) maintain the specific binding properties of the antibody or antigen-binding fragment; (ii) allow delivery, e.g., intracellular delivery, of the drug moiety via stable attachment to the antibody or antigen-binding fragment; (iii) remain stable and intact until the ADC has been transported or delivered to its target site; and (iv) allow for the therapeutic effect, e.g., cytotoxic effect, of the drug moiety after cleavage or alternate release mechanism.

Linkers may impact the physico-chemical properties of an ADC. As many cytotoxic agents are hydrophobic in nature, linking them to the antibody with an additional hydrophobic moiety may lead to aggregation. ADC aggregates are insoluble and often limit achievable drug loading onto the antibody, which can negatively affect the potency of the ADC. Protein aggregates of biologics, in general, have also been linked to increased immunogenicity. As shown below, linkers

disclosed herein result in ADCs with low aggregation levels and desirable levels of drug loading.

A linker may be "cleavable" or "non-cleavable" (Ducry and Stump (2010) Bioconjugate Chem. 21:5-13). Cleavable linkers are designed to release the drug moiety (e.g., an Mcl-1 inhibitor) when subjected to certain environment factors, e.g., when internalized into the target cell, whereas non-cleavable linkers generally rely on the degradation of the antibody or antigen-binding fragment itself.

The term "alkyl", as used herein, refers to a straight or branched hydrocarbon chain radical consisting solely of carbon and hydrogen atoms, containing no unsaturation. The term "$C_1$-$C_6$alkyl", as used herein, refers to a straight or branched hydrocarbon chain radical consisting solely of carbon and hydrogen atoms, containing no unsaturation, having from one to six carbon atoms, and which is attached to the rest of the molecule by a single bond. Non-limiting examples of "$C_1$-$C_6$alkyl" groups include methyl (a $C_1$alkyl), ethyl (a $C_2$alkyl), 1-methylethyl (a $C_3$alkyl), n-propyl (a $C_3$alkyl), isopropyl (a $C_3$alkyl), n-butyl (a $C_4$alkyl), isobutyl (a $C_4$alkyl), sec-butyl (a $C_4$alkyl), tert-butyl (a $C_4$alkyl), n-pentyl (a $C_5$alkyl), isopentyl (a $C_5$alkyl), neopentyl (a $C_5$alkyl) and hexyl (a $C_6$alkyl).

The term "alkenyl", as used herein, refers to a straight or branched hydrocarbon chain radical group consisting solely of carbon and hydrogen atoms, containing at least one double bond. The term "$C_2$-$C_6$alkenyl", as used herein, refers to a straight or branched hydrocarbon chain radical group consisting solely of carbon and hydrogen atoms, containing at least one double bond, having from two to six carbon atoms, which is attached to the rest of the molecule by a single bond. Non-limiting examples of "$C_2$-$C_6$alkenyl" groups include ethenyl (a $C_2$alkenyl), prop-1-enyl (a $C_3$alkenyl), but-1-enyl (a $C_4$alkenyl), pent-1-enyl (a $C_5$alkenyl), pent-4-enyl (a $C_5$alkenyl), penta-1,4-dienyl (a $C_5$alkenyl), hexa-1-enyl (a $C_6$alkenyl), hexa-2-enyl (a $C_6$alkenyl), hexa-3-enyl (a $C_6$alkenyl), hexa-1-,4-dienyl (a $C_6$alkenyl), hexa-1-,5-dienyl (a $C_6$alkenyl) and hexa-2-,4-dienyl (a $C_6$alkenyl). The term "$C_2$-$C_3$alkenyl", as used herein, refers to a straight or branched hydrocarbon chain radical group consisting solely of carbon and hydrogen atoms, containing at least one double bond, having from two to three carbon atoms, which is attached to the rest of the molecule by a single bond. Non-limiting examples of "$C_2$-$C_3$alkenyl" groups include ethenyl (a $C_2$alkenyl) and prop-1-enyl (a $C_3$alkenyl).

The term "alkylene", as used herein, refers to a bivalent straight or branched hydrocarbon chain radical consisting solely of carbon and hydrogen atoms and containing no unsaturation. The term "$C_1$-$C_6$alkylene", as used herein, refers to a bivalent straight or branched hydrocarbon chain radical consisting solely of carbon and hydrogen atoms, containing no unsaturation, having from one to six carbon atoms. Non-limiting examples of "$C_1$-$C_6$alkylene" groups include methylene (a $C_1$alkylene), ethylene (a $C_2$alkylene), 1-methylethylene (a $C_3$alkylene), n-propylene (a $C_3$alkylene), isopropylene (a $C_3$alkylene), n-butylene (a $C_4$alkylene), isobutylene (a $C_4$alkylene), sec-butylene (a $C_4$alkylene), tert-butylene (a $C_4$alkylene), n-pentylene (a $C_5$alkylene), isopentylene (a $C_5$alkylene), neopentylene (a $C_5$alkylene), and hexylene (a $C_6$alkylene).

The term "alkenylene", as used herein, refers to a bivalent straight or branched hydrocarbon chain radical consisting solely of carbon and hydrogen atoms and containing at least one double bond. The term "$C_2$-$C_6$alkenylene", as used herein, refers to a bivalent straight or branched hydrocarbon chain radical group consisting solely of carbon and hydrogen atoms, containing at least one double bond, and having from two to six carbon atoms. Non-limiting examples of "$C_2$-$C_6$alkenylene" groups include ethenylene (a $C_2$alkenylene), prop-1-enylene (a $C_3$alkenylene), but-1-enylene (a $C_4$alkenylene), pent-1-enylene (a $C_5$alkenylene), pent-4-enylene (a $C_5$alkenylene), penta-1,4-dienylene (a $C_5$alkenylene), hexa-1-enylene (a $C_6$alkenylene), hexa-2-enylene (a $C_6$alkenylene), hexa-3-enylene (a $C_6$alkenylene), hexa-1-,4-dienylene (a $C_6$alkenylene), hexa-1-,5-dienylene (a $C_6$alkenylene) and hexa-2-,4-dienylene (a $C_6$alkenylene). The term "$C_2$-$C_6$alkenylene", as used herein, refers to a bivalent straight or branched hydrocarbon chain radical group consisting solely of carbon and hydrogen atoms, containing at least one double bond, and having from two to thee carbon atoms. Non-limiting examples of "$C_2$-$C_3$alkenylene" groups include ethenylene (a $C_2$alkenylene) and prop-1-enylene (a $C_3$alkenylene).

The term "cycloalkyl," or "$C_3$-$C_8$cycloalkyl," as used herein, refers to a saturated, monocyclic, fused bicyclic, fused tricyclic or bridged polycyclic ring system. Non-limiting examples of fused bicyclic or bridged polycyclic ring systems include bicyclo[1.1.1]pentane, bicyclo[2.1.1]hexane, bicyclo[2.2.1]heptane, bicyclo[3.1.1]heptane, bicyclo[3.2.1]octane, bicyclo[2.2.2]octane and adamantanyl. Non-limiting examples monocyclic $C_3$-$C_8$cycloalkyl groups include cyclopropyl, cyclobutyl, cyclopentyl, cyclohexyl, cycloheptyl and cyclooctyl groups.

The term "haloalkyl," as used herein, refers to a linear or branched alkyl chain substituted with one or more halogen groups in place of hydrogens along the hydrocarbon chain. Examples of halogen groups suitable for substitution in the haloalkyl group include Fluorine, Bromine, Chlorine, and Iodine. Haloalkyl groups may include substitution with multiple halogen groups in place of hydrogens in an alkyl chain, wherein said halogen groups can be attached to the same carbon or to another carbon in the alkyl chain.

As used herein, the alkyl, alkenyl, alkynyl, alkoxy, amino, aryl, heteroaryl, cycloalkyl, and heterocycloalkyl groups may be optionally substituted by 1 to 4 groups selected from optionally substituted linear or branched ($C_1$-$C_6$)alkyl, optionally substituted linear or branched ($C_2$-$C_6$)alkenyl group, optionally substituted linear or branched ($C_2$-$C_6$)alkynyl group, optionally substituted linear or branched ($C_1$-$C_6$)alkoxy, optionally substituted ($C_1$-$C_6$)alkyl-S-, hydroxy, oxo (or N-oxide where appropriate), nitro, cyano, -C(O)-$OR_0$', -O-C(O)-$R_0$', -C(O)-$NR_0$'$R_0$", -$NR_0$'$R_0$", -(C=$NR_0$')-$OR_0$", linear or

branched ($C_1$-$C_6$) haloalkyl, trifluoromethoxy, or halogen, wherein $R_0$' and $R_0$" are each independently a hydrogen atom or an optionally substituted linear or branched ($C_1$-$C_6$)alkyl group, and wherein one or more of the carbon atoms of linear or branched ($C_1$-$C_6$)alkyl group is optionally deuterated.

The term "polyoxyethylene", "polyethylene glycol" or "PEG", as used herein, refers to a linear chain, a branched chain or a star shaped configuration comprised of ($OCH_2CH_2$) groups. In certain embodiments a polyethylene or PEG group is -$(OCH_2CH_2)_t$*-, where t is 4-40, and where the "-" indicates the end directed toward the self-immolative spacer and the "*-" indicates the point of attachment to a terminal end group R' where R' is OH, $OCH_3$ or $OCH_2CH_2C(=O)OH$. In other embodiments a polyethylene or PEG group is -$(CH_2CH_2O)_t$*-, where t is 4-40, and where the "-" indicates the end directed toward the self-immolative spacer and the "*-" indicates the point of attachment to a terminal end group R" where R" is H, $CH_3$ or $CH_2CH_2C(=O)OH$. For example, the term "PEG12" as used herein means that t is 12.

The term "polyalkylene glycol", as used herein, refers to a linear chain, a branched chain or a star shaped configuration comprised of $(O(CH_2)_m)_n$ groups. In certain embodiments a polyethylene or PEG group is -$(O(CH_2)_m)_t$*-, where m is 1-10, t is 4-40, and where the "-" indicates the end directed toward the self-immolative spacer and the "*-" indicates the point of attachment to a terminal end group R' where R' is OH, $OCH_3$ or $OCH_2CH_2C(=O)OH$. In other embodiments a polyethylene or PEG group is -$((CH_2)_mO)_t$*-, where m is 1-10, t is 4-40, and where the "-" indicates the end directed toward the self-immolative spacer and the "*-" indicates the point of attachment to a terminal end group R" where R" is H, $CH_3$ or $CH_2CH_2C(=O)OH$.

The term "reactive group", as used herein, is a functional group capable of forming a covalent bond with a functional group of an antibody, an antibody fragment, or another reactive group attached to an antibody or antibody fragment. Non limiting examples of such functional groups include reactive groups of Table 2 provided herein.

The term "attachment group" or "coupling group", as used herein, refers to a bivalent moiety which links the bridging spacer to the antibody or fragment thereof. The attachment or coupling group is a bivalent moiety formed by the reaction between a reaction group and a functional group on the antibody or fragment thereof. Non limiting examples of such bivalent moieties include the bivalent chemical moieties given in Table 2 and Table 3 provided herein.

The term "bridging spacer", as used herein, refers to one or more linker components which are covalently attached together to form a bivalent moiety which links the bivalent peptide spacer to the reactive group, links the bivalent peptide space to the coupling group, or links the attachment group to the at least one cleavable group. In certain embodiments the "bridging spacer" comprises a carboxyl group attached to the N-terminus of the bivalent peptide spacer via an amide bond.

The term "spacer moiety", as used herein, refers to one or more linker components which are covalently attached together to form a moiety which links the self-immolative spacer to the hydrophilic moiety.

The term "bivalent peptide spacer", as used herein, refers to bivalent linker comprising one or more amino acid residues covalently attached together to form a moiety which links the bridging spacer to the self immolative spacer. The one or more amino acid residues can be an residue of amino acids selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucune (Nle), selenocysteine (Sec), pyrrolysine (Pyl), homoserine, homocysteine, and desmethyl pyrrolysine.

In certain embodiments a "bivalent peptide spacer" is a combination of 2 to four amino acid residues where each residue is independently selected from a residue of an amino acid selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu),methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucune (Nle), selenocysteine (Sec), pyrrolysine (Pyl), homoserine, homocysteine, and desmethyl pyrrolysine, for example -ValCit*; -CitVal*; -AlaAla*; -AlaCit*; - CitAla*; -AsnCit*; -CitAsn*; -CitCit*; -ValGlu*; -GluVal*; -SerCit*; -CitSer*; -LysCit*; -CitLys*; - AspCit*; -CitAsp*; -AlaVal*; -ValAla*; -PheAla*; -AlaPhe*; -PheLys*; -LysPhe*; -ValLys*; - LysVal*; -AlaLys*; -LysAla*; -PheCit*; -CitPhe*; -LeuCit*; -CitLeu*; -IleCit*; -CitIle*; -PheArg*; -ArgPhe*; -CitTrp*; -TrpCit*; -PhePheLys*; -LysPhePhe*; -DphePheLys*; -DlysPhePhe*; - GlyPheLys*; -LysPheGly*; -GlyPheLeuGly- [SEQ ID NO:69]; -GlyLeuPheGly- [SEQ ID NO:64]; -AlaLeuAlaLeu- [SEQ ID NO:65], -GlyGlyGly*; -GlyGlyGlyGly- [SEQ ID NO:66]; - GlyPheValGly- [SEQ ID NO:67]; and -GlyValPheGly- [SEQ ID NO:68], wher the "-" indicates the point of attachment to the bridging spacer and the "*" indicates the point of attachment to the self-immolative spacer.

The term "linker component", as used herein, refers to a chemical moiety that is a part of the linker. Examples of linker components include: an alkylene group: -$(CH_2)_n$- which can either be linear or branched (where in this instance n is 1-18); an alkenylene group; an alkynylene group; an alkenyl group; an alkynyl group; an ethylene glycol unit: -$OCH_2CH_2$- or -$CH_2CH_2O$-; an polyethylene glycol unit: $(-CH_2CH_2O-)_x$ (where x in this instance is 2-20); -O-; -S-; a carbonyl: -C(=O); an ester: C(=O)-O or O-C(=O); a carbonate: -OC(=O)O-; an amine: - NH-; an tertiary amine; an amide: -C(=O)-NH-, -NH-C(=O)- or -C(=O)N($C_{1-6}$alkyl); a carbamate: -OC(=O)NH- or -NHC(=O)O; a urea: -NHC(=O)NH; a sulfonamide: -$S(O)_2$NH- or -NHS$(O)_2$;an ether: -$CH_2O$- or -$OCH_2$-; an alkylene substituted with one or more groups independently

selected from carboxy, sulfonate, hydroxyl, amine, amino acid, saccharide, phosphate and phosphonate); an alkenylene substituted with one or more groups independently selected from carboxy, sulfonate, hydroxyl, amine, amino acid, saccharide, phosphate and phosphonate); an alkynylene substituted with one or more groups independently selected from carboxy, sulfonate, hydroxyl, amine, amino acid, saccharide, phosphate and phosphonate); a $C_1$-$C_{10}$alkylene in which one or more methylene groups is replace by one or more -S-, -NH- or -O- moieties; a ring systems having two available points of attachment such as a divalent ring selected from phenyl (including 1,2- 1,3- and 1,4- di-substituted phenyls), a $C_5$-$C_6$ heteroaryl, a $C_3$-$C_8$ cycloalkyl (including 1, 1-disubstituted cyclopropyl, cyclobutyl, cyclopentyl or cyclohexyl, and 1,4-disubstituted cyclohexyl), and a $C_4$-$C_8$ heterocycloalkyl; a residue of an amino acid selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu),methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucune (Nle), selenocysteine (Sec), pyrrolysine (Pyl), homoserine, homocysteine, and desmethyl pyrrolysine; a combination of 2 or more amino acid residues where each residue is independently selected from a residue of an amino acid selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu),methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucune (Nle), selenocysteine (Sec), pyrrolysine (Pyl), homoserine, homocysteine, and desmethyl pyrrolysine, for example Val-Cit; Cit-Val; Ala-Ala; Ala-Cit; Cit-Ala; Asn-Cit; Cit-Asn; Cit-Cit; Val-Glu; Glu-Val; Ser-Cit; Cit-Ser; Lys-Cit; Cit-Lys; Asp-Cit; CitAsp; Ala-Val; Val-Ala; Phe-Lys; Lys-Phe; Val-Lys; Lys-Val; Ala-Lys; Lys-Ala; Phe-Cit; CitPhe; Leu-Cit; Cit-Leu; Ile-Cit; Cit-Ile; Phe-Arg; Arg-Phe; Cit-Trp; and Trp-Cit; and a self-immolative spacer, wherein the self-immolative spacer comprises one or more protecting (triggering) groups which are susceptible to acid-induced cleavage, peptidase-induced cleavage, esterase-induced cleavage, glycosidase induced cleavage, phosphodiesterase induced cleavage, phosphatase induced cleavage, protease induced cleavage, lipase induced cleavage or disulfide bond cleavage.

Non-limiting examples of such self-immolative spacers include:

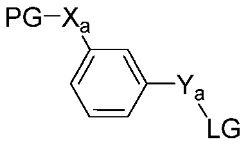
,
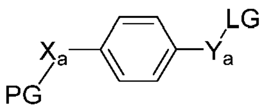
,
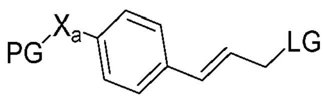
,
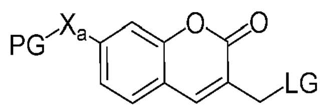
,

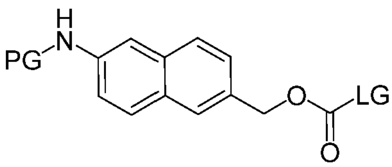
,
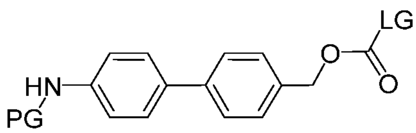
,
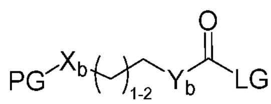
,

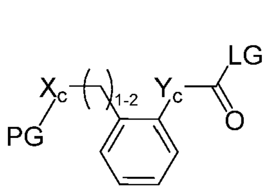
and
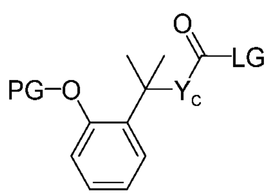
,

where:

PG is a protecting (triggering) group;

$X_a$ is O, NH or S;

$X_b$ is O, NH, $NCH_3$ or S;

$X_c$ is O or NH;

$Y_a$ is $CH_2$, $CH_2O$ or $CH_2NH$;

$Y_b$ is $CH_2$, O or NH;

$Y_c$ is a bond, $CH_2$, O or NH, and

LG is a leaving group such as a Drug moiety (D) of the Linker-Drug group of the invention.

Additional non-limiting examples of such self-immolative spacers are described in Angew. Chem. Int. Ed. 2015, 54, 7492 - 7509.

In addition, a linker component can be a chemical moiety which is readily formed by reaction between two reactive groups. Non-limiting examples of such chemical moieties are given in Table 2.

Table 2

| Reactive Group 1 (RG1) | Reactive Group 2 (RG2) | Chemical Moiety |
|---|---|---|
| a thiol | a thiol | -S-S- |
| a thiol | a maleimide | 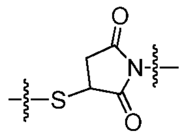 |
| a thiol | a haloacetamide | 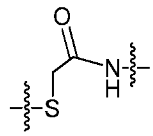 |
| an azide | an alkyne | 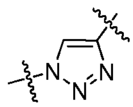 or 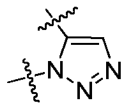 |
| an azide | a triaryl phosphine | 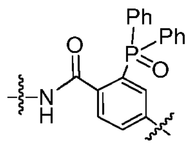 |
| an azide | a cyclooctyne | 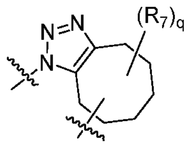 or 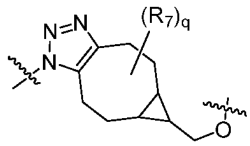 or 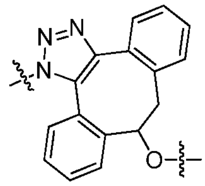 |
| an azide | an oxanobornadiene | 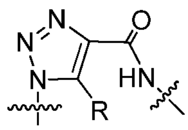 |
| a triaryl phosphine | an azide | 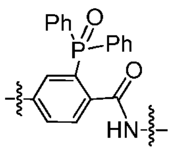 |

(continued)

| Reactive Group 1 (RG1) | Reactive Group 2 (RG2) | Chemical Moiety |
|---|---|---|
| an oxanobornadiene | an azide | 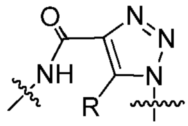 |
| an alkyne | an azide | 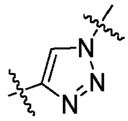 or 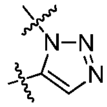 |
| a cyclooctyne | azide | 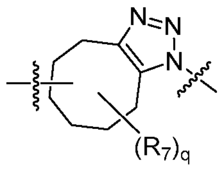 or 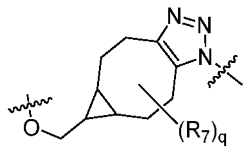 or 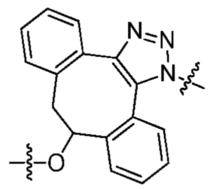 |
| a cyclooctene | a diaryl tetrazine | 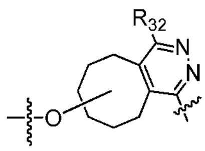 or 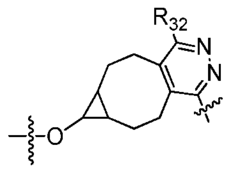 |
| a diaryl tetrazine | a cyclooctene | 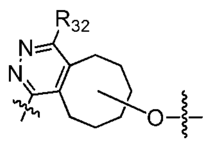 or 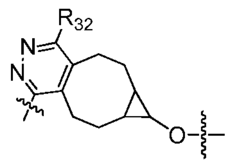 |
| a monoaryl tetrazine | a norbornene | 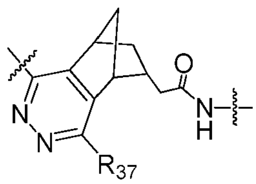 |
| a norbornene | a monoaryl tetrazine | 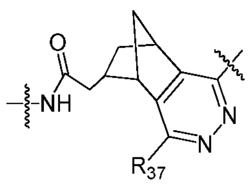 |
| an aldehyde | a hydroxylamine | 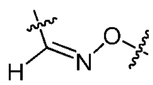 |

(continued)

| Reactive Group 1 (RG1) | Reactive Group 2 (RG2) | Chemical Moiety |
|---|---|---|
| an aldehyde | a hydrazine | 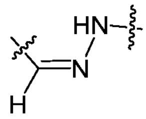 |
| an aldehyde | $NH_2$-NH-C(=O)- | 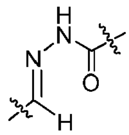 |
| a ketone | a hydroxylamine | 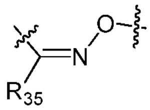 |
| a ketone | a hydrazine | 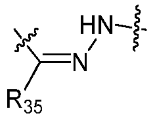 |
| a ketone | $NH_2$-NH-C(=O)- | 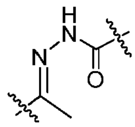 |
| a hydroxylamine | an aldehyde | 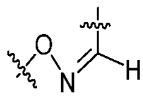 |
| a hydroxylamine | a ketone | 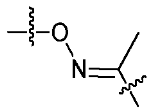 |
| a hydrazine | an aldehyde | 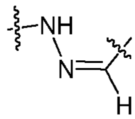 |
| a hydrazine | a ketone | 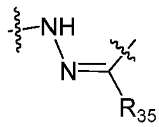 |
| $NH_2$-NH-C(=O)- | an aldehyde | 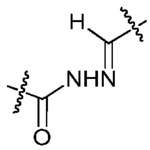 |
| $NH_2$-NH-C(=O)- | a ketone | 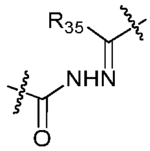 |

(continued)

| Reactive Group 1 (RG1) | Reactive Group 2 (RG2) | Chemical Moiety |
|---|---|---|
| a haloacetamide | a thiol | 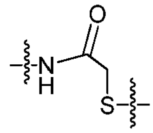 |
| a maleimide | a thiol | 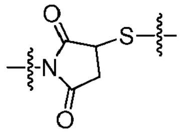 |
| a vinyl sulfone | a thiol | 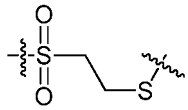 |
| a thiol | a vinyl sulfone | 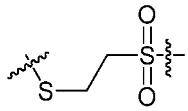 |
| an aziridine | a thiol | 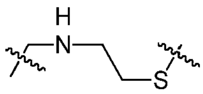 or 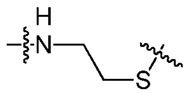 |
| a thiol | an aziridine | 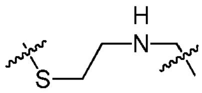 or 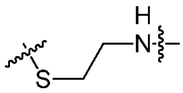 |
| 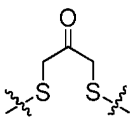 | hydroxylamine | 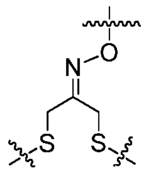 |
| 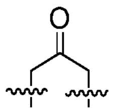 | hydroxylamine | 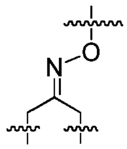 |
| 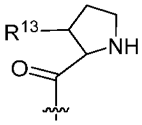 | 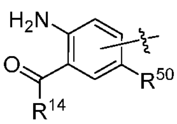 | 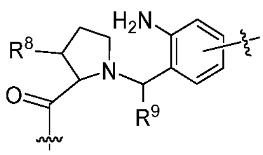 |
| 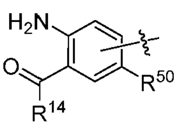 | 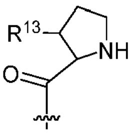 | 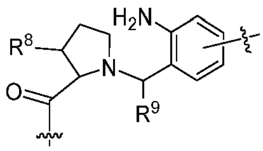 |

(continued)

| Reactive Group 1 (RG1) | Reactive Group 2 (RG2) | Chemical Moiety |
|---|---|---|
| 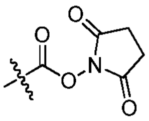<br>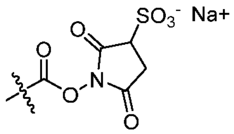<br>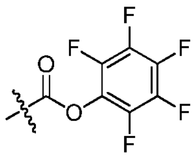<br>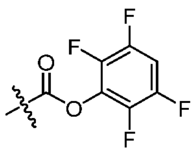<br>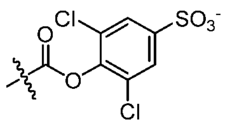 | $-NH_2$, | amide |
| $-NH_2$, | 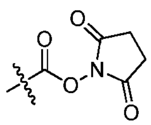<br>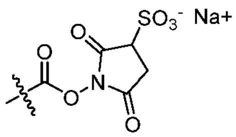<br>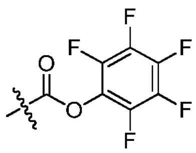<br>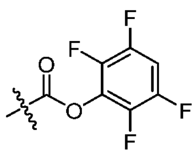<br>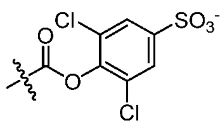 | amide |

(continued)

| Reactive Group 1 (RG1) | Reactive Group 2 (RG2) | Chemical Moiety |
|---|---|---|
| CoA or CoA analogue | Serine residue | 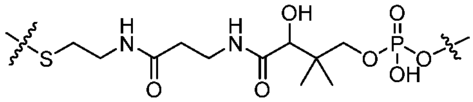<br>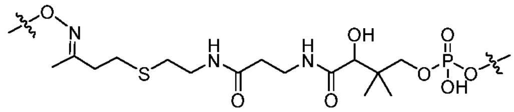<br>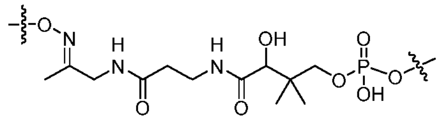<br>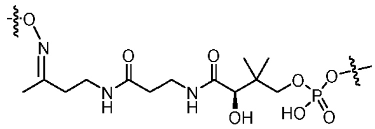<br>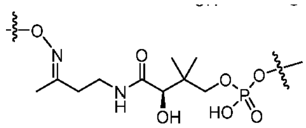<br>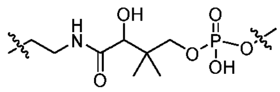<br>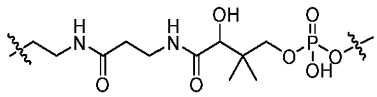<br>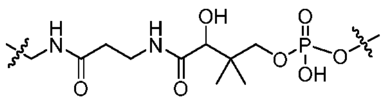 |
| pyridyldithiol | thiol | disulfide |

where: $R^{32}$ in Table 2 is H, $C_{1-4}$ alkyl, phenyl, pyrimidine or pyridine; $R^{35}$ in Table 2 is H, $C_{1-6}$alkyl, phenyl or $C_{1-4}$alkyl substituted with 1 to 3 -OH groups; each $R^7$ in Table 2 is independently selected from H, $C_{1-6}$alkyl, fluoro, benzyloxy substituted with - C(=O)OH, benzyl substituted with -C(=O)OH, $C_{1-4}$alkoxy substituted with -C(=O)OH and $C_{1-4}$alkyl substituted with -C(=O)OH; $R^{37}$ in Table 2 is independently selected from H, phenyl and pyridine; q in Table 2 is 0, 1, 2 or 3; $R^8$ and $R^{13}$ in Table 2 are each H or methyl; and $R^9$ and $R^{14}$ in Table 2 are each H, $-CH_3$ or phenyl; R in Table 2 is H or any suitable substituent; and $R^{50}$ in Table 2 is H.

In addition, a linker component can be a group listed in Table 3 below.

**Table 3.**

| | | | | |
|---|---|---|---|---|
| 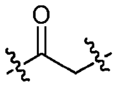 | 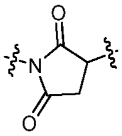 | 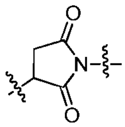 | 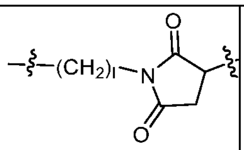 | 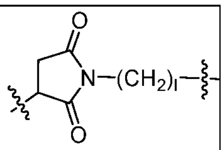 |
| 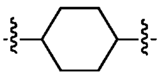 | 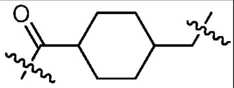 | 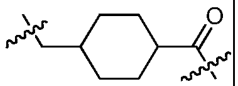 | 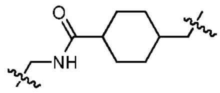 | 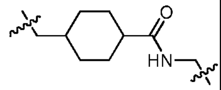 |
| 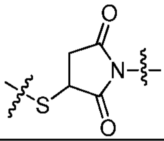 | 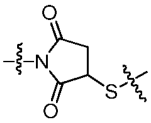 | 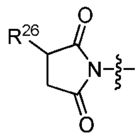 | 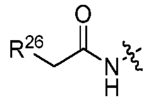 | 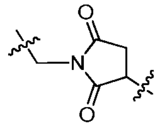 |
| 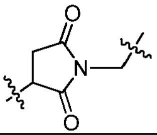 | 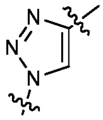 | 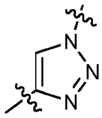 | 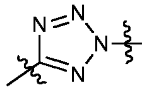 | 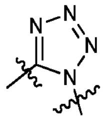 |
| 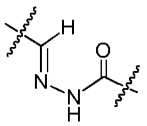 | 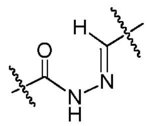 | 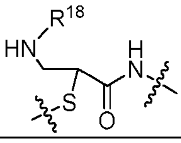 | 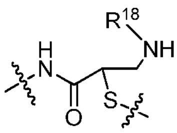 | 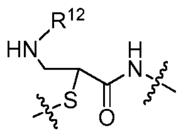 |

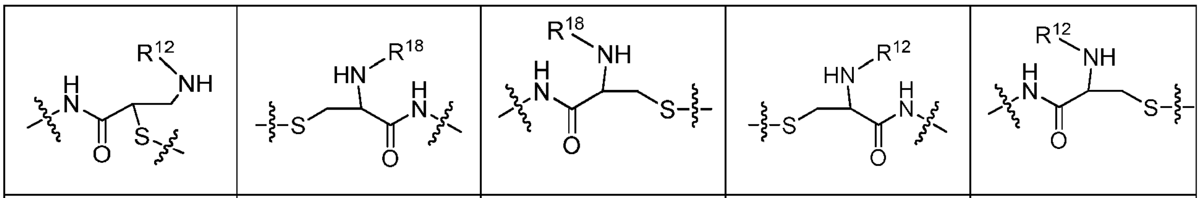

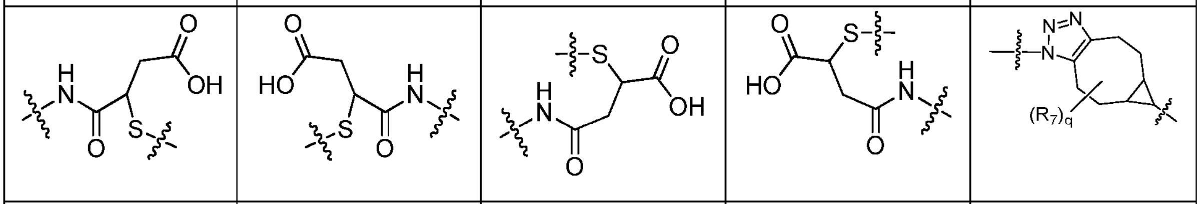

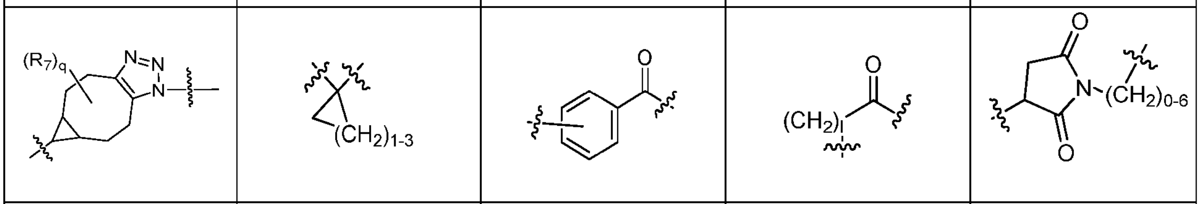

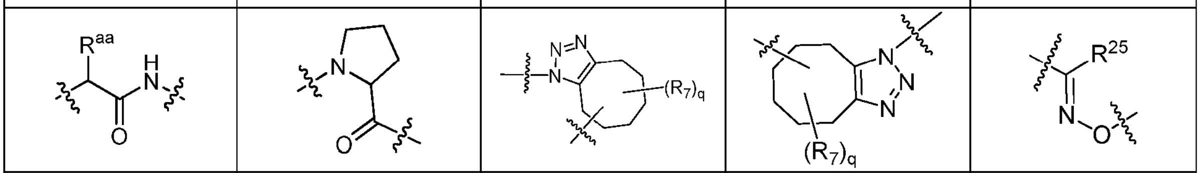

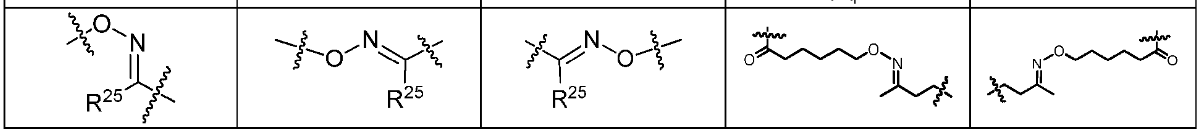

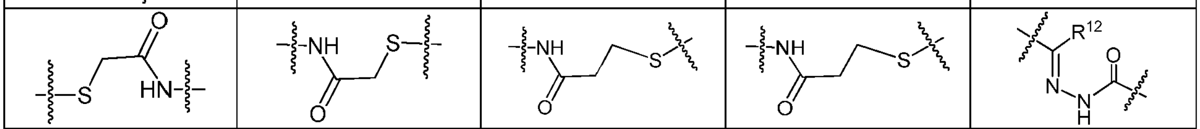

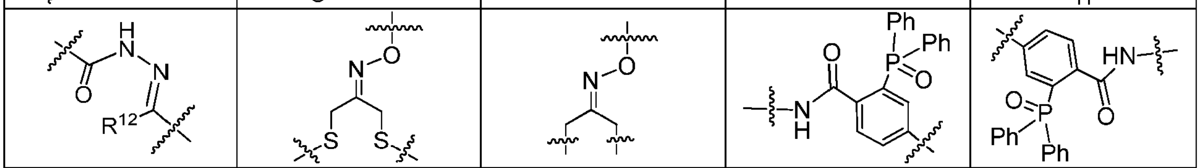

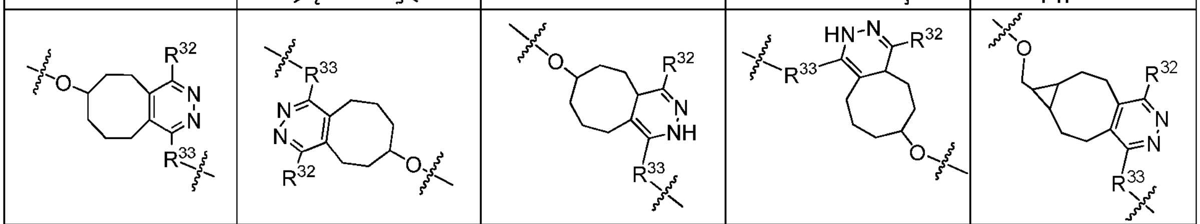

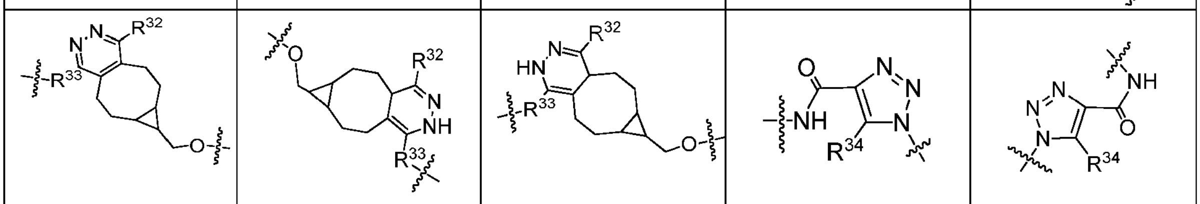

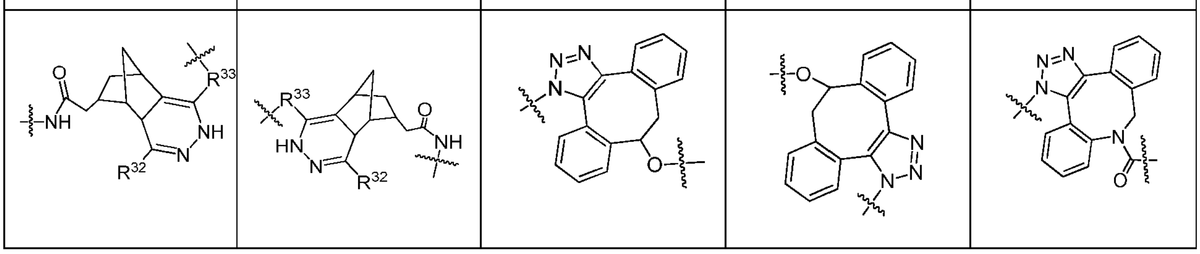

| | | | | |
|---|---|---|---|---|
| 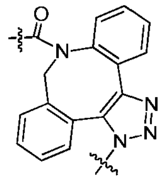 | 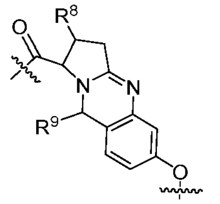 | 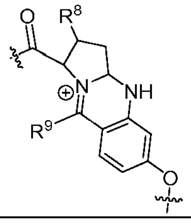 | 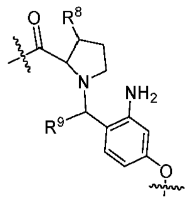 | 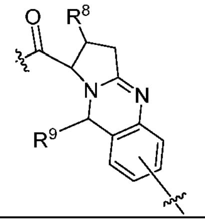 |
| 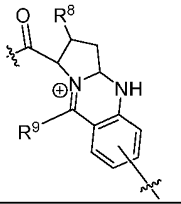 | 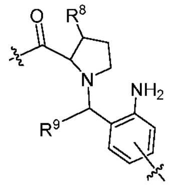 | 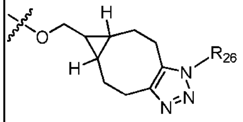 | 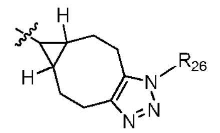 | 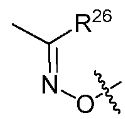 |

each $R^7$ is independently selected from H, $C_{1-6}$alkyl, fluoro, benzyloxy substituted with –C(=O)OH, benzyl substituted with –C(=O)OH, $C_{1-4}$alkoxy substituted with –C(=O)OH and $C_{1-4}$alkyl substituted with –C(=O)OH;

each $R^{12}$ is independently selected from H and $C_1$-$C_6$alkyl

$R^8$ is H or methyl;

$R^9$ is H, -$CH_3$ or phenyl;

each $R^{25}$ is independently selected from H or $C_{1-4}$ alkyl;

each $R^{18}$ is independently selected from a $C_1$-$C_6$alkyl, a $C_1$-$C_6$alkyl which is substituted with azido and a $C_1$-$C_6$alkyl which is substituted with 1 to 5 hydroxyl;

q is 0, 1, 2 or 3;

l is 1, 2, 3, 4, 5 or 6;

$R^{26}$ is 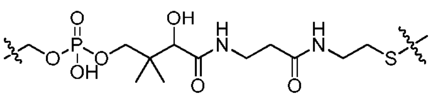, 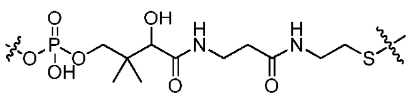, 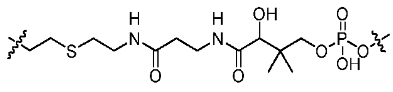, 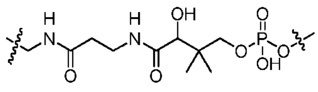, 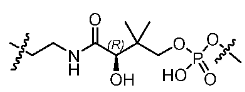, 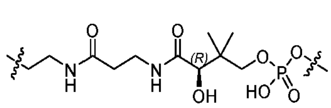, 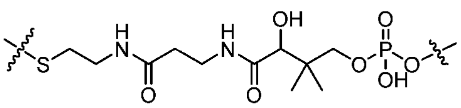, 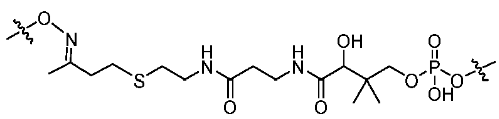, 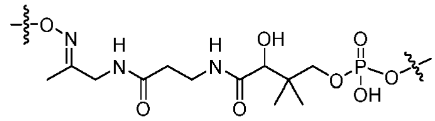, 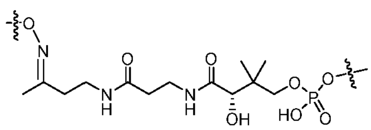, 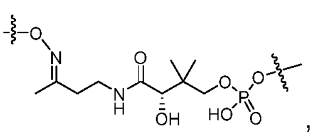, 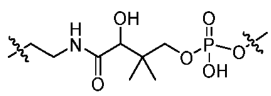, 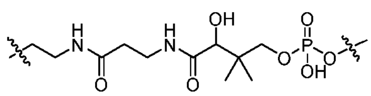, or 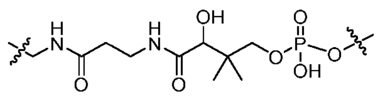

$R^{32}$ is independently selected from H, $C_{1-4}$ alkyl, phenyl, pyrimidine and pyridine;

$R^{33}$ is independently selected from 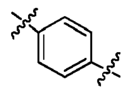, 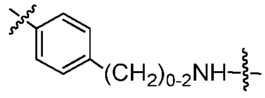,

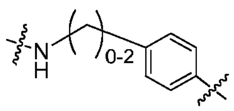, 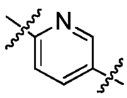, 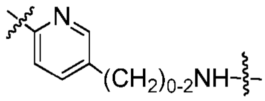, 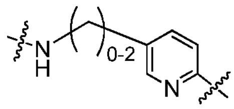,

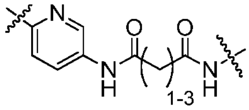, and 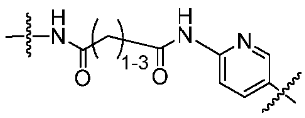;

$R^{34}$ is independently selected from H, $C_{1-4}$ alkyl, and $C_{1-6}$ haloalkyl, and

$R^{aa}$ is an amino acid side chain.

As used herein, when a partial structure of a compound is illustrated, a wavy line (∿∿∿) indicates the point of attachment of the partial structure to the rest of the molecule.

The terms "self-immolative spacer" and "self-immolative group", as used herein, refer a moiety comprising one or more triggering groups (TG) which are activated by acid-induced cleavage, peptidase-induced cleavage, esterase-induced cleavage, glycosidase induced cleavage, phosphodiesterase induced cleavage, phosphatase induced cleavage, protease induced cleavage, lipase induced cleavage or disulfide bond cleavage, and after activation the protecting group is removed, which generates a cascade of disassembling reactions leading to the temporally sequential release of a leaving group. Such cascade of reactions can be, but not limited to, 1,4-, 1,6- or 1,8- elimination reactions.

Non-limiting examples of self-immolative spacer or group include:

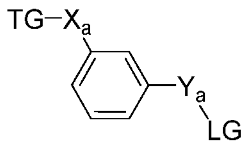, 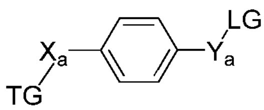, 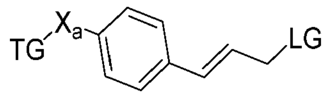,

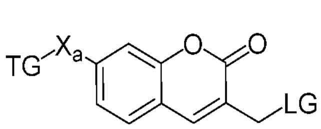, 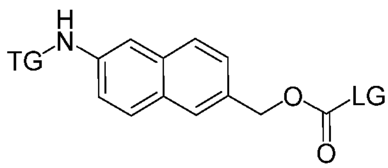,

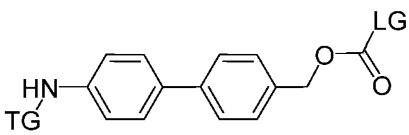, 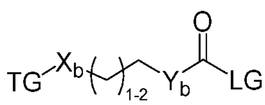, 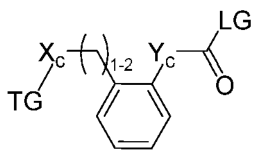

and

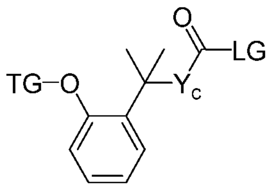

,

wherein such groups can be optionally substituted, and wherein:

TG is a triggering group;
$X_a$ is O, NH or S;
$X_b$ is O, NH, $NCH_3$ or S;
$X_c$ is O or NH;
$Y_a$ is $CH_2$, $CH_2O$ or $CH_2NH$;
$Y_b$ is $CH_2$, O or NH;
$Y_c$ is a bond, $CH_2$, O or NH, and
LG is a leaving group such as a Drug moiety (D) of the Linker-Drug group of the invention.

Additional non-limiting examples of self-immolative spacers are described in Angew. Chem. Int. Ed. 2015, 54, 7492 - 7509.

In certain embodiment the self-immolative spacer is moiety having the structure

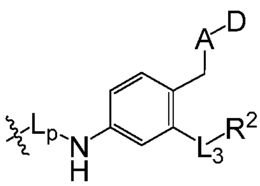

,

where Lp is an enzymatically cleavable bivalent peptide spacer and A, D, $L_3$ and $R^2$ are as defined herein.

In preferred embodiments, the self-immolative spacer is moiety having the structure

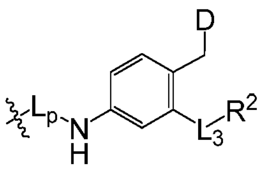

,

where Lp is an enzymatically cleavable bivalent peptide spacer and D, $L_3$ and $R^2$ are as defined herein. In some embodiments, D is a quaternized tertiary amine-containing MCl1 inhibitor.

In other preferred embodiments, the self-immolative spacer is moiety having the structure

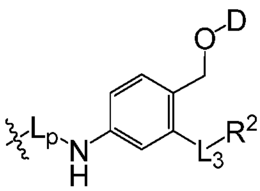

,

where Lp is an enzymatically cleavable bivalent peptide spacer and D, $L_3$ and $R^2$ are as defined herein.

The term "hydrophilic moiety", as used herein, refers to moiety that is has hydrophilic properties which increases the aqueous solubility of the Drug moiety (D) when the Drug moiety (D) is attached to the linker group of the invention. Examples of such hydrophilic groups include, but are not limited to, polyethylene glycols, polyalkylene glycols, sugars, oligosaccharides, polypeptides a $C_2$-$C_6$alkyl substituted with 1 to 3

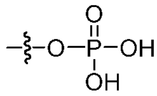

groups.

Drug Moieties

In some embodiments, an intermediate, which is the precursor of the linker moiety, is reacted with the drug moiety (e.g., the Mcl-1 inhibitor) under appropriate conditions. In some embodiments, reactive groups are used on the drug and/or the intermediate or linker. The product of the reaction between the drug and the intermediate, or the derivatized drug (drug plus linker), is subsequently reacted with the antibody or antigen-binding fragment under conditions that facilitate conjugation of the drug and intermediate or derivatized drug and antibody or antigen-binding fragment. Alternatively, the intermediate or linker may first be reacted with the antibody or antigen-binding fragment, or a derivatized antibody or antigen-binding fragment, and then reacted with the drug or derivatized drug.

A number of different reactions are available for covalent attachment of the drug moiety and/or linker moiety to the antibody or antigen-binding fragment. This is often accomplished by reaction of one or more amino acid residues of the antibody or antigen-binding fragment, including the amine groups of lysine, the free carboxylic acid groups of glutamic acid and aspartic acid, the sulfhydryl groups of cysteine, and the various moieties of the aromatic amino acids. For instance, non-specific covalent attachment may be undertaken using a carbodiimide reaction to link a carboxy (or amino) group on a drug moiety to an amino (or carboxy) group on an antibody or antigen-binding fragment. Additionally, bifunctional agents such as dialdehydes or imidoesters may also be used to link the amino group on a drug moiety to an amino group on an antibody or antigen-binding fragment. Also available for attachment of drugs (e.g., an Mcl-1 inhibitor) to binding agents is the Schiff base reaction. This method involves the periodate oxidation of a drug that contains glycol or hydroxy groups, thus forming an aldehyde which is then reacted with the binding agent. Aattachment occurs via formation of a Schiff base with amino groups of the binding agent. Isothiocyanates may also be used as coupling agents for covalently attaching drugs to binding agents. Other techniques are known to the skilled artisan and within the scope of the present disclosure. Examples of drug moieties that can be generated and linked to an antibody or antigen-binding fragment using various chemistries known to in the art include Mcl-1 inhibitors, e.g., the Mcl-1 inhibitors described and exemplified herein.

Suitable drug moieties may comprise a compound of the formulas (I), (II), (III), or an enantiomer, diastereoisomer, atropisomer, deuterated derivative, and/or addition salt thereof with a pharmaceutically acceptable acid or base. Additionally, the drug moiety may comprise any compounds of the Mcl-1 inhibitor (D) described herein.

As used herein, "atropisomers," are stereoisomers arising because of hindered rotation about a single bond, where energy differences due to steric strain or other contributors create a barrier to rotation that is high enough to allow for isolation of individual conformers (Bringmann et al. Angew. Chem. Int. Ed. 2005, 44, 5384-5427). For example, for compounds of formula (II) according to the invention, atropisomers may be as follows:

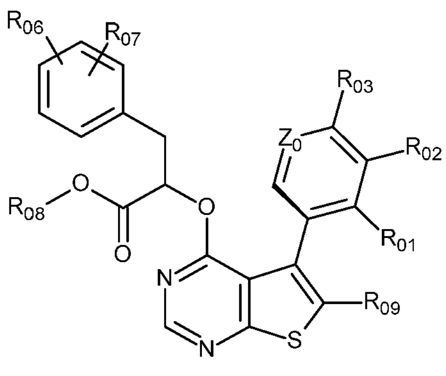

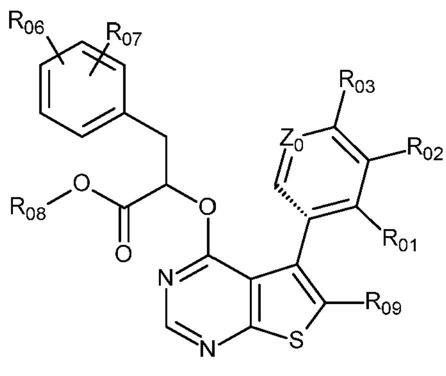

.

For example, a preferred atropisomer may be (5$S_a$), also named (5aS).

A drug moiety of the disclosure may be any one of the compounds disclosed in International Patent Application Publication Nos. WO 2015/097123; WO 2016/207216; WO 2016/207217; WO 2016/207225; WO 2016/207226; WO 2017/125224; WO 2019/035899; WO 2019/035911; WO 2019/035914; WO 2019/035927; WO 2016/033486; WO 2017/147410; WO 2018/183418; and WO 2017/182625, and U.S. Patent Application Publication No. 2019/0055264.

In some embodiments, a drug moiety of the disclosure may comprise a compound of Formula (I):

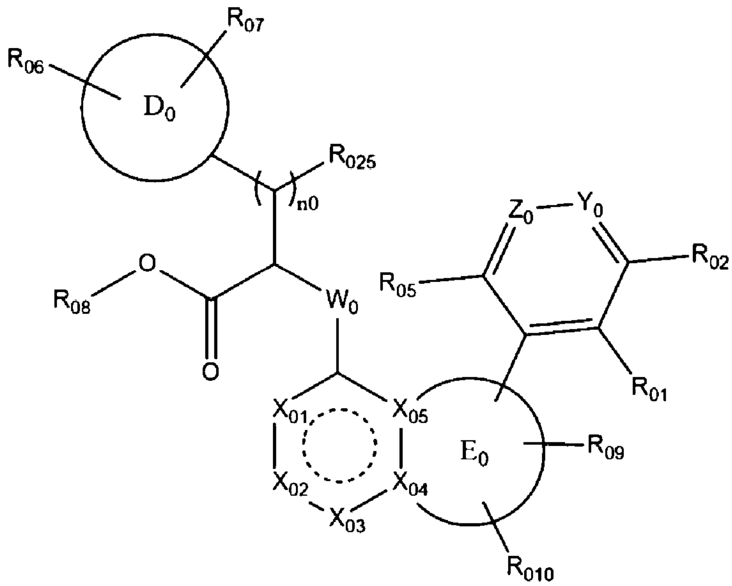

(I)

wherein:

Ring $D_0$ is a cycloalkyl group, a heterocycloalkyl group, an aryl group or a heteroaryl group,
Ring $E_0$ is a furyl, thienyl or pyrrolyl ring,
$X_{01}$, $X_{03}$, $X_{04}$ and $X_{05}$ independently of one another are a carbon atom or a nitrogen atom,
$X_{02}$ is a C-$R_{026}$ group or a nitrogen atom,

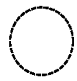

means that the ring is aromatic,
$Y_0$ is a nitrogen atom or a C-$R_{03}$ group,
$Z_0$ is a nitrogen atom or a C-$R_{04}$ group,
$R_{01}$ is a halogen atom, a linear or branched ($C_1$-$C_6$)alkyl group, a linear or branched ($C_2$-$C_6$)alkenyl group, a linear or branched ($C_2$-$C_6$)alkynyl group, a linear or branched ($C_1$-$C_6$)haloalkyl group, a hydroxy group, a hydroxy($C_1$-$C_6$)alkyl group, a linear or branched ($C_1$-$C_6$)alkoxy group, -S-($C_1$-$C_6$)alkyl group, a cyano group, a nitro group,

-$Cy_{08}$, -($C_0$-$C_6$)alkyl-$NR_{011}$ $R_{011}$', -O-($C_1$-$C_6$)alkyl-$NR_{011}R_{011}$', -O-($C_1$-$C_6$)alkyl-$R_{012}$,

-C(O)-$OR_{011}$, -O-C(O)-$R_{011}$, -C(O)-$NR_{011}R_{011}$', -$NR_{011}$-C(O)-$R_{011}$', -$NR_{011}$-C(O)-$OR_{011}$',

-($C_1$-$C_6$)alkyl-$NR_{011}$-C(O)-$R_{011}$', -$SO_2$-$NR_{011}R_{011}$', or -$SO_2$-($C_1$-$C_6$)alkyl,

$R_{02}$, $R_{03}$, $R_{04}$ and $R_{05}$ independently of one another are a hydrogen atom, a halogen atom, a linear or branched ($C_1$-$C_6$)alkyl group, a linear or branched ($C_2$-$C_6$)alkenyl group, a linear or branched ($C_2$-$C_6$)alkynyl group, a linear or branched ($C_1$-$C_6$)haloalkyl, a hydroxy group, a hydroxy($C_1$-$C_6$)alkyl group, a linear or branched ($C_1$-$C_6$)alkoxy group, a -S-($C_1$-$C_6$)alkyl group, a cyano group, a nitro group, -($C_0$-$C_6$)alkyl-$NR_{011}R_{011}$', -O-$Cy_{01}$, -($C_0$-$C_6$)alkyl-$Cy_{01}$, -($C_2$-$C_6$) alkenyl-$Cy_{01}$,

-($C_2$-$C_6$)alkynyl-$Cy_{01}$, -O-($C_1$-$C_6$)alkyl-$NR_{011}R_{011}$', -O-($C_1$-$C_6$)alkyl-$R_{031}$,

-O-($C_1$-$C_6$)alkyl-$R_{012}$, -C(O)-$OR_{011}$, -O-C(O)-$R_{011}$, -C(O)-$NR_{011}$ $R_{011}$', -$NR_{011}$-C(O)-$R_{011}$',

-$NR_{011}$-C(Q)-$OR_{011}$', -($C_1$-$C_6$)alkyl-$NR_{011}$-C(O)-$R_{011}$', -$SO_2$-$NR_{011}R_{011}$',

or

$-SO_2-(C_1-C_6)$alkyl,

or the pair ($R_{01}$, $R_{02}$), ($R_{02}$, $R_{03}$), ($R_{03}$, $R_{04}$), or ($R_{04}$, $R_{05}$) together with the carbon atoms to which they are attached form an aromatic or non-aromatic ring containing 5 to 7 ring members, which optionally contains 1 to 3 heteroatoms selected from O, S and N, wherein the resulting ring is optionally substituted by 1 or 2 groups selected from halogen, linear or branched $(C_1-C_6)$alkyl, $(C_0-C_6)$alkyl-$NR_{011}R_{011}'$, $-NR_{013}R_{013'}$,

$-(C_0-C_6)$alkyl-$Cy_{01}$ or oxo,

$R_{06}$ and $R_{07}$ independently of one another are a hydrogen atom, a halogen atom, a linear or branched $(C_1-C_6)$alkyl group, a linear or branched $(C_2-C_6)$alkenyl group, a linear or branched $(C_2-C_6)$alkynyl group, a linear or branched $(C_1-C_6)$haloalkyl, a hydroxy group, a linear or branched $(C_1-C_6)$alkoxy group, a $-S-(C_1-C_6)$alkyl group, a cyano group, a nitro group, $-(C_0-C_6)$alkyl-$NR_{011}$ $R_{011}'$, $-O-(C_1-C_6)$alkyl-$NR_{011}R_{011'}$, $-O-Cy_{01}$,

$-(C_0-C_6)$alkyl-$Cy_{01}$, $-(C_2-C_6)$alkenyl-$Cy_{01}$, $-(C_2-C_6)$alkynyl-$Cy_{01}$, $-O-(C_1-C_6)$alkyl-$R_{012}$,

$-C(O)-OR_{011}$, $-O-C(O)-R_{011}$, $-C(O)-NR_{011}R_{011}'$, $-NR_{011}-C(O)-R_{011}'$, $-NR_{011}-C(O)-OR_{011}'$,

$-(C_1-C_6)$alkyl-$NR_{011}-C(O)-R_{011}'$, $-SO_2-NR_{011}R_{011}'$, or $-SO_2-(C_1-C_6)$alkyl,

or the pair ($R_{06}$, $R_{07}$), when fused with the two adjacent carbon atoms, together with the carbon atoms to which they are attached form an aromatic or non-aromatic ring containing 5 to 7 ring members, which optionally contains 1 to 3 heteroatoms selected from O, S and N, wherein the resulting ring is optionally substituted by a linear or branched $(C_1-C_6)$alkyl group, $-NR_{013}R_{013'}$, $-(C_0-C_6)$alkyl-$Cy_{01}$ or an oxo,
$W_0$ is a $-CH_2-$ group, a -NH- group or an oxygen atom,
$R_{08}$ is a hydrogen atom, a linear or branched $(C_1-C_8)$alkyl group, a $-CHR_{0a}R_{0b}$ group, an aryl group, a heteroaryl group, an aryl$(C_1-C_6)$alkyl group, or a heteroaryl$(C_1-C_6)$alkyl group,
$R_{09}$ is a hydrogen atom, a linear or branched $(C_1-C_6)$alkyl group, a linear or branched $(C_2-C_6)$alkenyl group, a linear or branched $(C_2-C_6)$alkynyl group, $-Cy_{02}$, $-(C_1-C_6)$alkyl-$Cy_{02}$, $-(C_2-C_6)$alkenyl-$Cy_{02}$, $-(C_2-C_6)$alkynyl-$Cy_{02}$, $-Cy_{02}-Cy_{03}$, $-(C_2-C_6)$alkynyl-$O-Cy_{02}$, $-Cy_{02}-(C_0-C_6)$alkyl-$O-(C_0-C_6)$alkyl-$Cy_{03}$, a halogen atom, a cyano group, $-C(O)-R_{014}$, or $-C(O)-NR_{014}R_{014}'$,
$R_{010}$ is a hydrogen atom, a linear or branched $(C_1-C_6)$alkyl group, a linear or branched $(C_2-C_6)$alkenyl group, a linear or branched $(C_2-C_6)$alkynyl group, an aryl$(C_1-C_6)$alkyl group, a $(C_1-C_6)$cycloalkylalkyl group, a linear or branched $(C_1-C_6)$haloalkyl, or $-(C_1-C_6)$alkyl-$O-Cy_{04}$,
or the pair ($R_{09}$, $R_{010}$), when fused with the two adjacent carbon atoms, together with the carbon atoms to which they are attached form an aromatic or non-aromatic ring containing 5 to 7 ring members, which optionally contains 1 to 3 heteroatoms selected from O, S and N,
$R_{011}$ and $R_{011}'$ independently of one another are a hydrogen atom, an optionally substituted linear or branched $(C_1-C_6)$ alkyl group, or $-(C_0-C_6)$alkyl-$Cy_{01}$, or the pair ($R_{011}$, $R_{011}'$) together with the nitrogen atom to which they are attached form an aromatic or non-aromatic ring containing 5 to 7 ring members, which optionally contains, in addition to the nitrogen atom, 1 to 3 heteroatoms selected from O, S, and N, wherein the N atom may be substituted by 1 or 2 groups selected from a linear or branched $(C_1-C_6)$alkyl group, and wherein one or more of the carbon atoms of the linear or branched $(C_1-C_6)$alkyl group is optionally deuterated,
$R_{012}$ is $-Cy_{05}$, $-Cy_{05}-(C_0-C_6)$alkyl-$O-(C_0-C_6)$alkyl-$Cy_{06}$, $-Cy_{05}-(C_0-C_6)$alkyl-$Cy_{06}$, $-Cy_{05}-(C_0-C_6)$alkyl-$NR_{011}-(C_0-C_6)$ alkyl-$Cy_{06}$, $-Cy_{05}-Cy_{06}-O-(C_0-C_6)$alkyl-$Cy_{07}$, $-Cy_{05}-(C_0-C_6)$alkyl-$O-(C_0-C_6)$alkyl-$Cy_{09}$, $-Cy_{05}-(C_0-C_6)$alkyl-$Cy_{09}$, $-NH-C(O)-NH-R_{011}$, $-Cy_{05}-(C_0-C_6)$alkyl-$NR_{011}-(C_0-C_6)$alkyl-$Cy_{09}$, $-C(O)-NR_{011}R_{011}'$, $-NR_{011}R_{011}'$, $-OR_{011}$, $-NR_{011}-C(O)-R_{011}'$, $-O-(C_1-C_6)$alkyl-$OR_{011}$, $-SO_2-R_{011}$, $-C(O)-OR_{011}$,
$R_{013}$, $R_{013}'$, $R_{014}$ and $R_{014}'$ independently of one another are a hydrogen atom, or an optionally substituted linear or branched $(C_1-C_6)$alkyl group,
$R_{0a}$ is a hydrogen atom or a linear or branched $(C_1-C_6)$alkyl group,
$R_{0b}$ is a $-O-C(O)-O-R_{0c}$ group, a $-O-C(O)-NR_{0c}R_{0c}'$ group, or a $-OP(O)(OR_{0c})_2$ group,
$R_{0c}$ and $R_{0c}'$ independently of one another are a hydrogen atom, a linear or branched $(C_1-C_8)$alkyl group, a cycloalkyl group, a $(C_1-C_6)$alkoxy$(C_1-C_6)$alkyl group, or a $(C_1-C_6)$alkoxycarbonyl$(C_1-C_6)$alkyl group,
or the pair ($R_{0c}$, $R_{0c}'$) together with the nitrogen atom to which they are attached form a non-aromatic ring composed of from 5 to 7 ring members, which may contain in addition to the nitrogen atom from 1 to 3 heteroatoms selected from oxygen and nitrogen, wherein the nitrogen is optionally substituted by a linear or branched $(C_1-C_6)$alkyl group,
$Cy_{01}$, $Cy_{02}$, $Cy_{03}$, $Cy_{04}$, $Cy_{05}$, $Cy_{06}$, $Cy_{07}$, $Cy_{08}$ and $Cy_{010}$ independently of one another, are an optionally substituted cycloalkyl group, an optionally substituted heterocycloalkyl group, an optionally substituted aryl group or an optionally

substituted heteroaryl group,

$Cy_{09}$ is

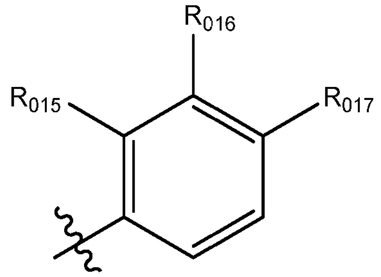

,

or $Cy_{09}$ is a heteroaryl group which is substituted by a group selected from $-O-P(O)(OR_{020})_2$; $-O-P(O)(O^-M^+)_2$; $-(CH_2)_{p0}-O-(CHR_{018}-CHR_{019}-O)_{q0}-R_{020}$; hydroxy; hydroxy$(C_1-C_6)$alkyl; $-(CH_2)_{r0}-U_0-(CH_2)_{s0}$-heterocycloalkyl; and $-U_0-(CH_2)_{q0}-NR_{021}R_{021}'$,

$R_{015}$ is a hydrogen atom; a $-(CH_2)_{p0}-O-(CHR_{018}-CHR_{019}-O)_{q0}-R_{020}$ group; a linear or branched $(C_1-C_6)$alkoxy$(C_1-C_6)$alkyl group; a $-U_0-(CH_2)_{q0}-NR_{021}R_{021}'$ group; or a $-(CH_2)_{r0}-U_0-(CH_2)_{s0}$-heterocycloalkyl group,

$R_{016}$ is a hydrogen atom; a hydroxy group; a hydroxy$(C_1-C_6)$alkyl group; a $-(CH_2)_{r0}-U_0-(CH_2)_{s0}$-heterocycloalkyl group; a $(CH_2)_{r0}-U_0-V_0-O-P(O)(OR_{020})_2$ group; a $-O-P(O)(O^-M^+)_2$ group; a $-O-S(O)_2OR_{020}$ group; a $-S(O)_2OR_{020}$ group; a $-(CH_2)_{p0}-O-(CHR_{018}-CHR_{019}-O)_{q0}-R_{020}$ group; a $-(CH_2)_{p0}-O-C(O)-NR_{022}R_{023}$ group; or a $-U_0-(CH_2)_{q0}-NR_{021}R_{021}'$ group,

$R_{017}$ is a hydrogen atom; a $-(CH_2)_{p0}-O-(CHR_{018}-CHR_{019}-O)_{q0}-R_{020}$ group; a $-CH_2-P(O)(OR_{020})_2$ group, a $-O-P(O)(OR_{020})_2$ group; a $-O-P(O)(O^-M^+)_2$ group; a hydroxy group; a hydroxy$(C_1-C_6)$alkyl group; a $-(CH_2)_{r0}-U_0-(CH_2)_{s0}$-heterocycloalkyl group; a $-U_0-(CH_2)_{q0}-NR_{021}R_{021}'$ group; or an aldonic acid,

$M^+$ is a pharmaceutically acceptable monovalent cation,

$U_0$ is a bond or an oxygen atom,

$V_0$ is a $-(CH_2)_{s0}-$ group or a $-C(O)-$ group,

$R_{018}$ is a hydrogen atom or a $(C_1-C_6)$alkoxy$(C_1-C_6)$alkyl group,

$R_{019}$ is a hydrogen atom or a hydroxy$(C_1-C_6)$alkyl group,

$R_{020}$ is a hydrogen atom or a linear or branched $(C_1-C_6)$alkyl group,

$R_{021}$ and $R_{021}'$ independently of one are a hydrogen atom, a linear or branched $(C_1-C_6)$alkyl group, or a hydroxy$(C_1-C_6)$alkyl group,

or the pair $(R_{021}, R_{021}')$ together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the resulting ring is optionally substituted by a hydrogen atom or a linear or branched $(C_1-C_6)$alkyl group,

$R_{022}$ is a $(C_1-C_6)$alkoxy$(C_1-C_6)$alkyl group, a $-(CH_2)_{p0}-NR_{024}R_{024}'$ group, or a $-(CH_2)_{p0}-O-(CHR_{018}-CHR_{019}-O)_{q0}-R_{020}$ group,

$R_{023}$ is a hydrogen atom or a $(C_1-C_6)$alkoxy$(C_1-C_6)$alkyl group,

or the pair $(R_{022}, R_{023})$ together with the nitrogen atom to which they are attached form an aromatic or non-aromatic ring containing 5 to 18 ring members, which optionally contains, in addition to the nitrogen atom, 1 to 5 heteroatoms selected from O, S and N, wherein the resulting ring is optionally substituted by a hydrogen atom, a linear or branched $(C_1-C_6)$alkyl group or a heterocycloalkyl group,

$R_{024}$ and $R_{024}'$ independently of one another are a hydrogen atom or a linear or branched $(C_1-C_6)$alkyl group,

or the pair $(R_{024}, R_{024}')$ together with the nitrogen atom to which they are attached form an aromatic or non-aromatic ring composed of from 5 to 7 ring members, which may contain in addition to the nitrogen atom from 1 to 3 heteroatoms selected from O, S and N, and wherein the resulting ring is optionally substituted by a hydrogen atom or a linear or branched $(C_1-C_6)$alkyl group,

$R_{025}$ is a hydrogen atom, a hydroxy group, or a hydroxy$(C_1-C_6)$alkyl group,

$R_{026}$ is a hydrogen atom, a halogen atom, a linear or branched $(C_1-C_6)$alkyl group, or a cyano group,

$R_{027}$ is a hydrogen atom or a linear or branched $(C_1-C_6)$alkyl group,

$R_{028}$ is a $-O-P(O)(O^-)(O^-)$ group, a $-O-P(O)(O^-)(OR_{030})$ group, a $-O-P(O)(OR_{030})(OR_{030}')$ group, a $-(CH_2)_{p0}-O-SO_2-$ group, a $-(CH_2)_{p0}-SO_2-O^-$ group, a $-(CH_2)_{p0}-O-SO_2-OR_{030}$ group, $-Cy_{010}$, a $-(CH_2)_{p0}-SO_2-OR_{030}$ group, a $-O-C(O)-R_{029}$ group, a $-O-C(O)-OR_{029}$ group or a $-O-C(O)-NR_{029}R_{029}'$ group;

$R_{029}$ and $R_{029}'$ independently of one another are a hydrogen atom, a linear or branched $(C_1-C_6)$alkyl group or a linear

or branched amino($C_1$-$C_6$)alkyl group,
$R_{030}$ and $R_{030}$' independently of one another are a hydrogen atom, a linear or branched ($C_1$-$C_6$)alkyl group or an aryl($C_1$-$C_6$)alkyl group,
$R_{031}$ is

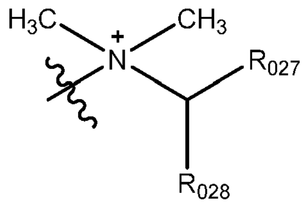
,
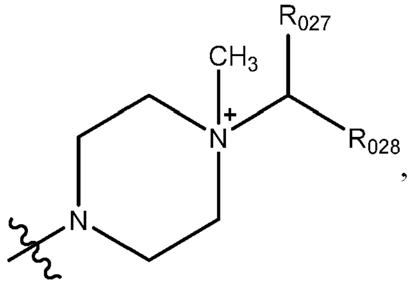
,

or

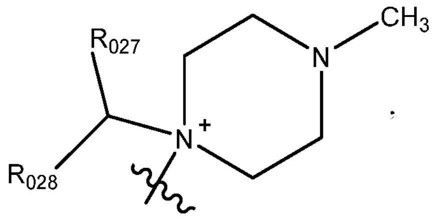
,

wherein the ammonium ion optionally exists as a zwitterionic form or has a monovalent anionic counterion,
$n_0$ is an integer equal to 0 or 1,
$p_0$ is an integer equal to 0, 1, 2, or 3,
$q_0$ is an integer equal to 1, 2, 3 or 4,
$r_0$ and $s_0$ are independently an integer equal to 0 or 1;

wherein, at most, one of the $R_{03}$, $R_{09}$, or $R_{012}$ groups, if present, is covalently attached to the linker, and wherein the valency of an atom is not exceeded by virtue of one or more substituents bonded thereto,
or an enantiomer, diastereoisomer, atropisomer, deuterated derivative, and/or pharmaceutically acceptable salt of any of the foregoing.

In some embodiments, a drug moiety of the disclosure may comprise a compound of Formula (II):

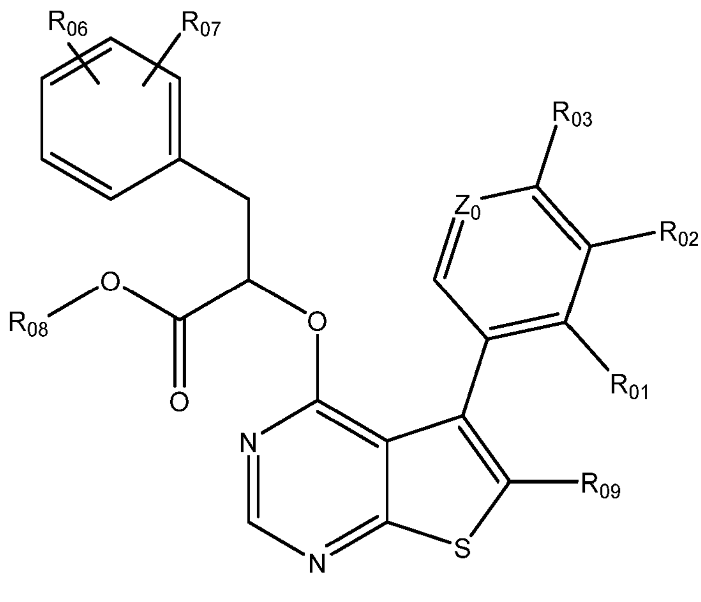
**(II)**

wherein:

$Z_0$ is a nitrogen atom or a C-$R_{04}$ group,
$R_{01}$ is a halogen atom, a linear or branched ($C_1$-$C_6$)alkyl group, a linear or branched ($C_2$-$C_6$)alkenyl group, a linear or branched ($C_2$-$C_6$)alkynyl group, a linear or branched ($C_1$-$C_6$)haloalkyl group, a hydroxy group, a linear or branched ($C_1$-$C_6$)alkoxy group, a -S-($C_1$-$C_6$)alkyl group, a cyano group, -$Cy_{08}$, -$NR_{011}R_{011}$',
$R_{02}$, $R_{03}$ and $R_{04}$ independently of one another are a hydrogen atom, a halogen atom, a linear or branched ($C_1$-$C_6$) alkyl group, a linear or branched ($C_2$-$C_6$)alkenyl group, a linear or branched ($C_2$-$C_6$)alkynyl group, a linear or branched ($C_1$-$C_6$)haloalkyl, a hydroxy group, a linear or branched ($C_1$-$C_6$)alkoxy group, a -S-($C_1$-$C_6$)alkyl group, a cyano group, a nitro group, -($C_0$-$C_6$)alkyl-$NR_{011}$ $R_{011}$', -O-$Cy_{01}$, -($C_0$-$C_6$)alkyl-$Cy_{01}$,

-($C_2$-$C_6$)alkenyl-$Cy_{01}$, -($C_2$-$C_6$)alkynyl-$Cy_{01}$, -O-($C_1$-$C_6$)alkyl-$NR_{011}R_{011}$',

-O-($C_1$-$C_6$)alkyl-$R_{031}$, -C(O)-$OR_{011}$, -O-C(O)-$R_{011}$, -C(O)-$NR_{011}R_{011}$', -$NR_{011}$-C(O)-$R_{011}$',

-$NR_{011}$-C(O)-$OR_{011}$', -($C_1$-$C_6$)alkyl-$NR_{011}$-C(O)-$R_{011}$', -$SO_2$-$NR_{011}R_{011}$',

or

-$SO_2$-($C_1$-$C_6$)alkyl,

or the pair ($R_{02}$, $R_{03}$) or ($R_{03}$, $R_{04}$) together with the carbon atoms to which they are attached form an aromatic or non-aromatic ring containing 5 to 7 ring members, which optionally contains 1 to 3 heteroatoms selected from O, S and N, wherein the ring is optionally substituted by a group selected from a linear or branched ($C_1$-$C_6$)alkyl,

-$NR_{013}R_{013'}$, -($C_0$-$C_6$)alkyl-$Cy_{01}$

and oxo,
$R_{06}$ and $R_{07}$ independently of one another are a hydrogen atom, a halogen atom, a linear or branched ($C_1$-$C_6$)alkyl group, a linear or branched ($C_2$-$C_6$)alkenyl group, a linear or branched ($C_2$-$C_6$)alkynyl group, a linear or branched ($C_1$-$C_6$)haloalkyl, a hydroxy group, a linear or branched ($C_1$-$C_6$)alkoxy group, a -S-($C_1$-$C_6$)alkyl group, a cyano group, a nitro group, -($C_0$-$C_6$)alkyl-$NR_{011}$ $R_{011}$', -O-$Cy_{01}$, -($C_0$-$C_6$)alkyl-$Cy_{01}$, -($C_2$-$C_6$)alkenyl-$Cy_{01}$,

-($C_2$-$C_6$)alkynyl-$Cy_{01}$, -O-($C_1$-$C_6$)alkyl-$R_{012}$, -C(O)-$OR_{011}$, -O-C(O)-$R_{011}$, -C(O)-$NR_{011}R_{011'}$,

-$NR_{011}$-C(O)-$R_{011}$', -$NR_{011}$-C(O)-$OR_{011}$', -($C_1$-$C_6$)alkyl-$NR_{011}$-C(O)-$R_{011}$', -$SO_2$-$NR_{011}R_{011}$', or -$SO_2$-($C_1$-$C_6$)alkyl,

or the pair ($R_{06}$, $R_{07}$), when fused with two adjacent carbon atoms, together with the carbon atoms to which they are attached form an aromatic or non-aromatic ring containing 5 to 7 ring members, which optionally contains 1 to 3 heteroatoms selected from O, S and N, and wherein the resulting ring is optionally substituted by a group selected from a linear or branched ($C_1$-$C_6$)alkyl group, -$NR_{013}R_{013'}$, -($C_0$-$C_6$)alkyl-$C_{Y01}$ and an oxo,
$R_{08}$ is a hydrogen atom, a linear or branched ($C_1$-$C_8$)alkyl group, an aryl group, a heteroaryl group, an aryl-($C_1$-$C_6$) alkylgroup, or a heteroaryl($C_1$-$C_6$)alkyl group,
$R_{09}$ is a linear or branched ($C_1$-$C_6$)alkyl group, a linear or branched ($C_2$-$C_6$)alkenyl group, a linear or branched ($C_2$-$C_6$) alkynyl group, -$Cy_{02}$, -($C_1$-$C_6$)alkyl-$Cy_{02}$, -($C_2$-$C_6$)alkenyl-$Cy_{02}$, -($C_2$-$C_6$)alkynyl-$Cy_{02}$, -$Cy_{02}$-$Cy_{03}$, -($C_2$-$C_6$)alkynyl-O-$Cy_{02}$, -$Cy_{02}$-($C_0$-$C_6$)alkyl-O-($C_0$-$C_6$)alkyl-$Cy_{03}$, a halogen atom, a cyano group, -C(O)-$R_{014}$, -C(O)-$NR_{014}R_{014}$',
$R_{011}$ and $R_{011}$' independently of one another are a hydrogen atom, an optionally substituted linear or branched ($C_1$-$C_6$) alkyl group, or -($C_0$-$C_6$)alkyl-$Cy_{01}$, or the pair ($R_{011}$, $R_{011}$') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom is optionally substituted by a linear or branched ($C_1$-$C_6$)alkyl group, and wherein one or more of the carbon atoms of the linear or branched ($C_1$-$C_6$)alkyl group is optionally deuterated,
$R_{012}$ is -$Cy_{05}$, -$Cy_{05}$-($C_0$-$C_6$)alkyl-$Cy_{06}$, -$Cy_{05}$-($C_0$-$C_6$)alkyl-O-($C_0$-$C_6$)alkyl-$Cy_{06}$, -$Cy_{05}$-($C_0$-$C_6$)alkyl-$NR_{011}$-($C_0$-$C_6$) alkyl-$Cy_{06}$, -$Cy_{05}$-$Cy_{06}$-O-($C_0$-$C_6$)alkyl-$Cy_{07}$, -$Cy_{05}$-($C_0$-$C_6$)alkyl-$Cy_{09}$, -NH-C(O)-NH-$R_{011}$, -C(O)-$NR_{011}$ $R_{011}$', -$NR_{011}R_{011}$', -$OR_{011}$, -$NR_{011}$-C(O)-$R_{011}$', -O-($C_1$-$C_6$)alkyl-$OR_{011}$, -$SO_2$-$R_{011}$, or -C(O)-$OR_{011}$,
$R_{013}$, $R_{013}$', $R_{014}$ and $R_{014}$' independently of one another are a hydrogen atom, or an optionally substituted linear or branched ($C_1$-$C_6$)alkyl group,

$Cy_{01}$, $Cy_{02}$, $Cy_{03}$, $Cy_{05}$, $Cy_{06}$, $Cy_{07}$ and $Cy_{08}$ independently of one another, are an optionally substituted cycloalkyl group, an optionally substituted heterocycloalkyl group, an optionally substituted aryl group or an optionally substituted heteroaryl group,

$Cy_{09}$ is

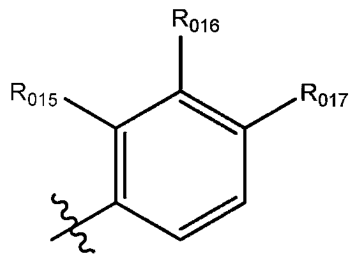

,

wherein $R_{013}$, $R_{016}$, and $R_{017}$ are as defined for formula (I),

$R_{031}$ is

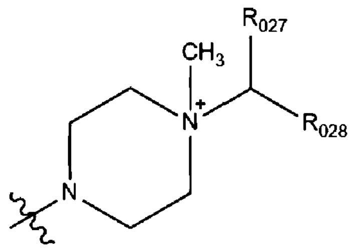

,

wherein $R_{027}$ and $R_{028}$ are as defined for formula (I)

where

in, at most, one of the $R_{03}$, $R_{09}$, or $R_{012}$ groups, if present, is covalently attached to the linker,

or an enantiomer, diastereoisomer, atropisomer, deuterated derivative, and/or pharmaceutically acceptable salt of any of the foregoing.

In some embodiments, a drug moiety of the disclosure may comprise a compound of Formula (III):

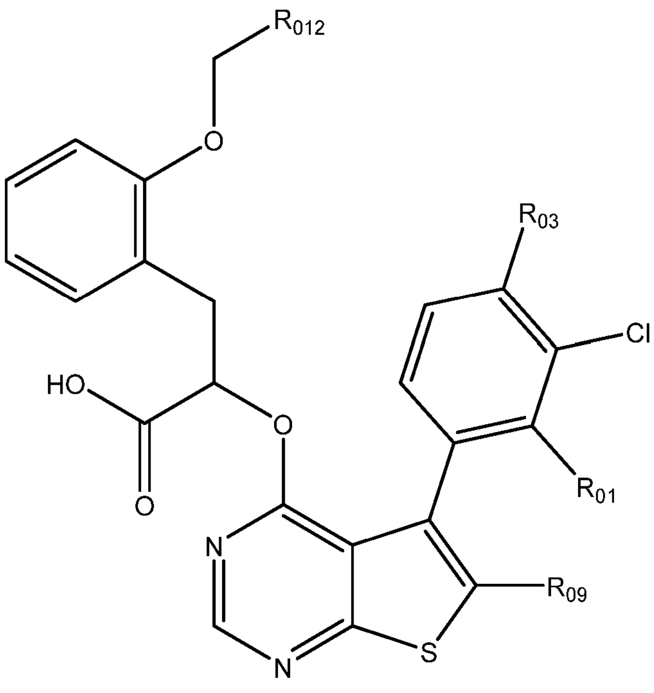

**(III)**

wherein:

$R_{01}$ is a linear or branched ($C_1$-$C_6$)alkyl group,
$R_{03}$ is -O-($C_1$-$C_6$)alkyl-$NR_{011}R_{011}$',
or

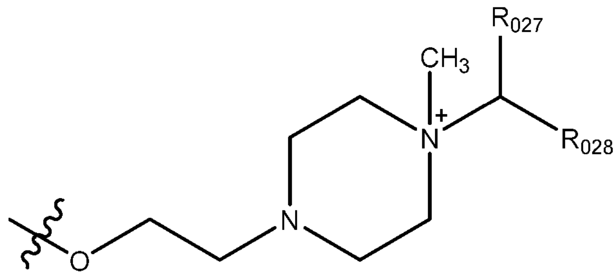

,

wherein $R_{011}$ and $R_{011}$' independently of one another are a hydrogen atom, an optionally substituted linear or branched ($C_1$-$C_6$)alkyl group, or -($C_0$-$C_6$)alkyl-$Cy_{01}$;
or the pair ($R_{011}$, $R_{011}$') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom may be substituted by 1 or 2 groups selected from a hydrogen atom or a linear or branched ($C_1$-$C_6$)alkyl group,
and wherein $R_{027}$ is a hydrogen atom and $R_{028}$ is a -$(CH_2)_{p0}$-O-$SO_2$-$O^-$ group or a -$(CH_2)_{p0}$-$SO_2$-$OR_{030}$ group;
$R_{09}$ is a linear or branched ($C_2$-$C_6$)alkynyl group or -$Cy_{02}$,
$R_{012}$ is -$Cy_{05}$, -$Cy_{05}$-($C_0$-$C_6$)alkyl-$Cy_{06}$, or -$Cy_{05}$-($C_0$-$C_6$)alkyl-$Cy_{09}$,
$Cy_{01}$, $Cy_{02}$, $Cy_{05}$ and $Cy_{06}$ independently of one another, are a cycloalkyl group, a heterocycloalkyl group, an aryl group or a heteroaryl group, each of which is optionally substituted,
$Cy_{09}$ is

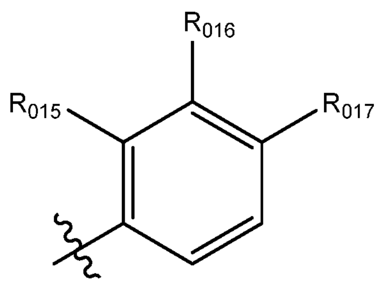

;

$p_0$, $R_{015}$, $R_{016}$, and $R_{017}$ are as defined for formula (I),
wherein, at most, one of the $R_{03}$, $R_{09}$, or $R_{012}$ groups, if present, is covalently attached to the linker,
or the enantiomer, diastereoisomer, atropisomer, deuterated derivative, and/or pharmaceutically acceptable salt of any of the foregoing.

In some embodiments, $Cy_{01}$, $Cy_{02}$, $Cy_{03}$, $Cy_{04}$, $Cy_{05}$, $Cy_{06}$, $Cy_{07}$, $Cy_{08}$ and $Cy_{010}$ independently of one another, are an optionally substituted cycloalkyl group, an optionally substituted heterocycloalkyl group, an optionally substituted aryl group or an optionally substituted heteroaryl group, wherein the optional substituents are selected from optionally substituted linear or branched ($C_1$-$C_6$)alkyl, optionally substituted linear or branched ($C_2$-$C_6$)alkenyl group, optionally substituted linear or branched ($C_2$-$C_6$)alkynyl group, optionally substituted linear or branched ($C_1$-$C_6$)alkoxy, optionally substituted ($C_1$-$C_6$)alkyl-S-, hydroxy, oxo (or N-oxide where appropriate), nitro, cyano, -C(O)-$OR_0$', -O-C(O)-$R_0$', -C(O)-$NR_0$'$R_0$'', -$NR_0$'$R_0$'', -(C=$NR_0$')-$OR_0$'', linear or branched ($C_1$-$C_6$)haloalkyl, trifluoromethoxy, or halogen, wherein $R_0$' and $R_0$'' are each independently a hydrogen atom or an optionally substituted linear or branched ($C_1$-$C_6$)alkyl group, and wherein one or more of the carbon atoms of linear or branched ($C_1$-$C_6$)alkyl group is optionally deuterated.
In some embodiments, the drug moiety (D) comprises:

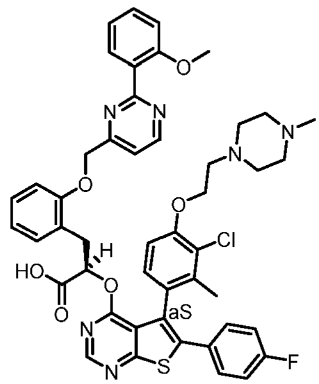,
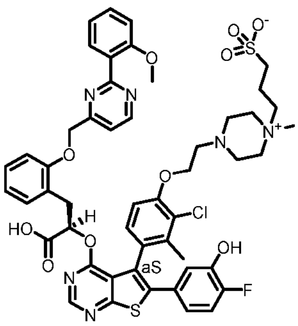,
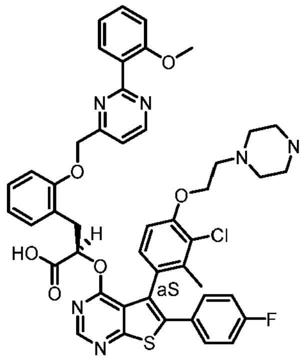,

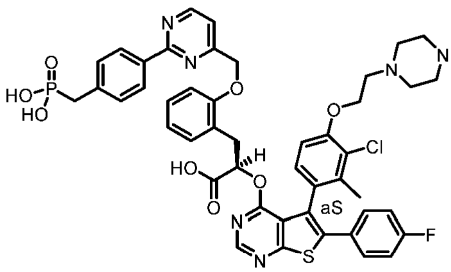,
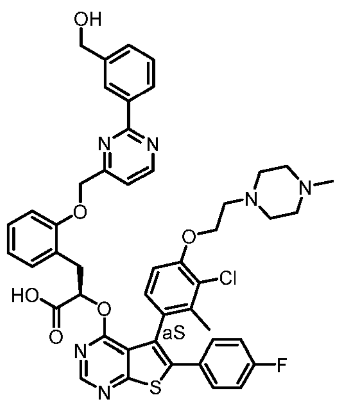,
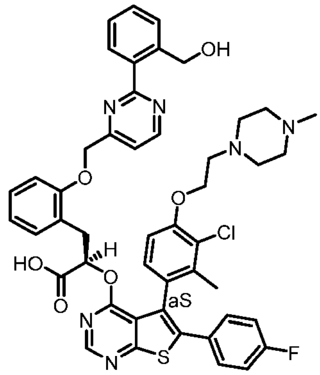,

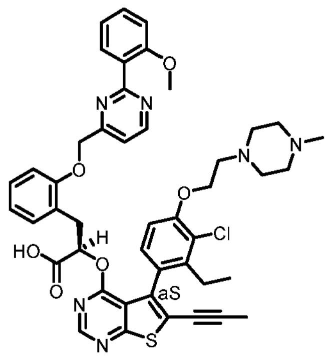,
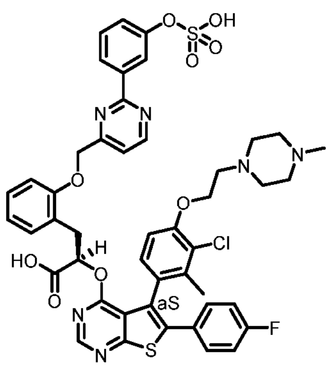,
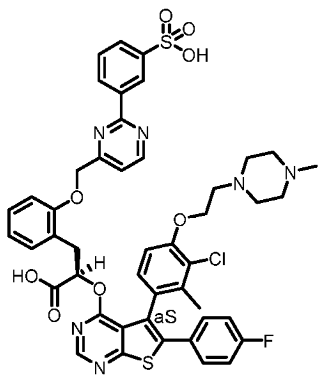,

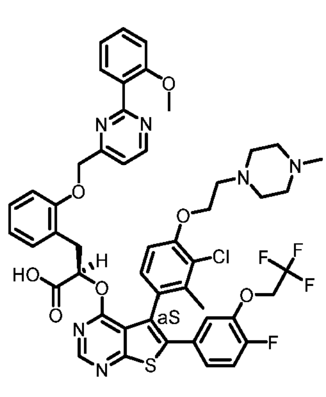,
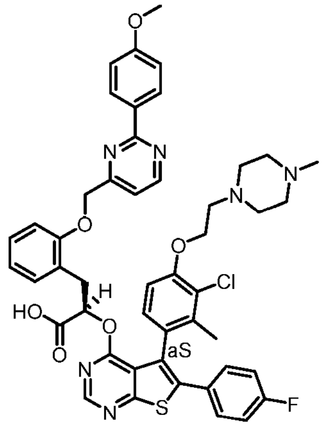,
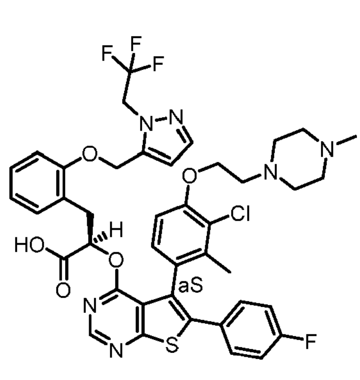,

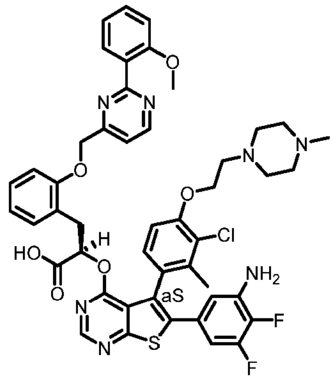,
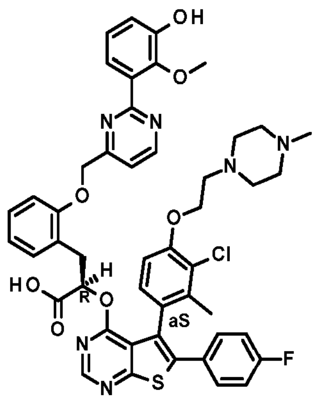,

or an enantiomer, diastereoisomer, atropisomer, deuterated derivative, and/or a pharmaceutically acceptable salt of any of the foregoing.

Additionally, a drug moiety of the disclosure may comprise any one of the following:

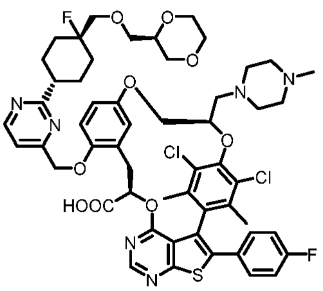,
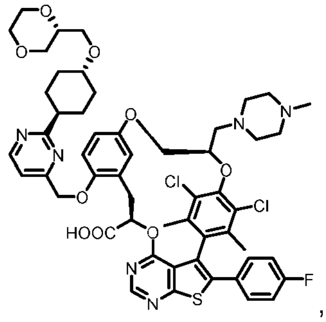,
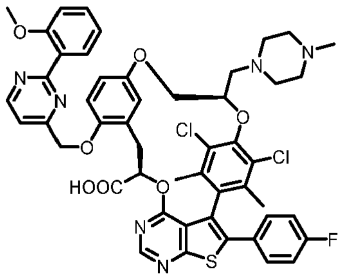,
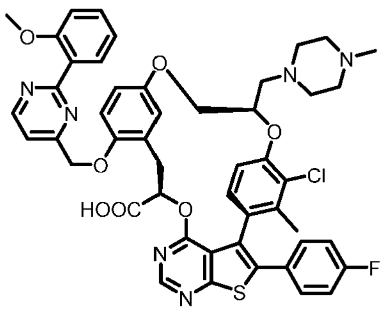,
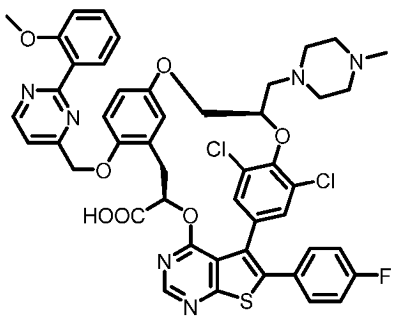,
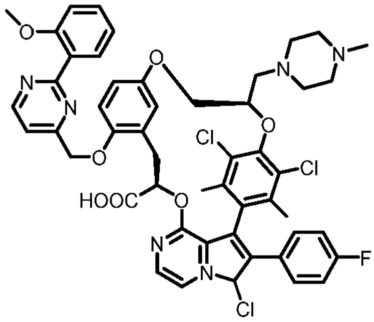,
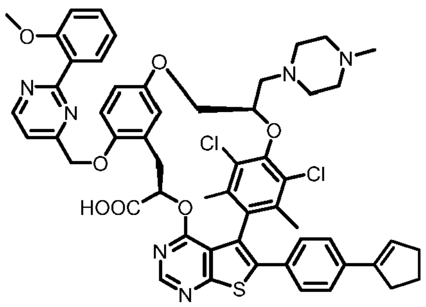,
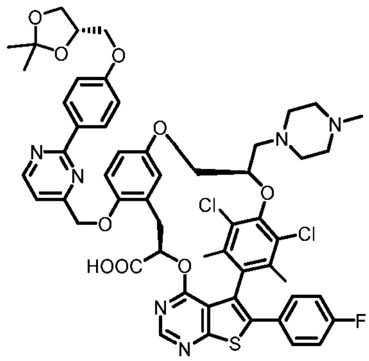,
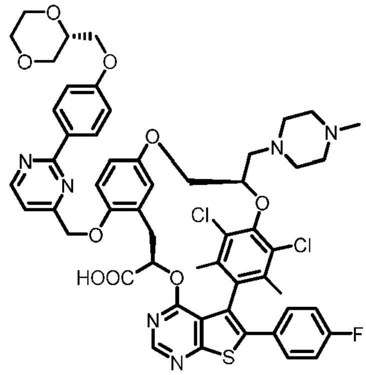,

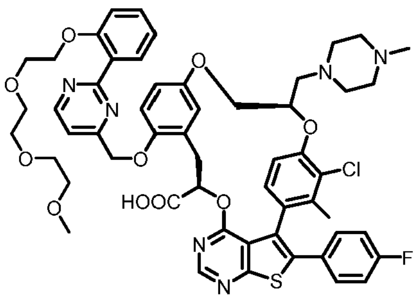,
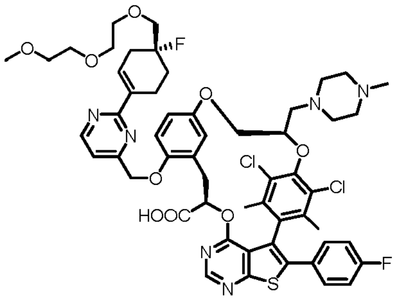,

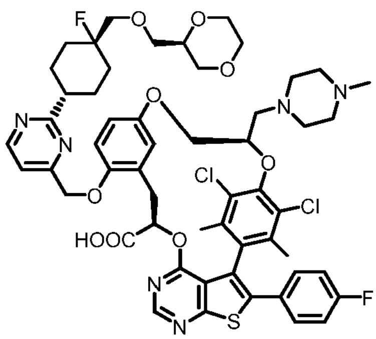,
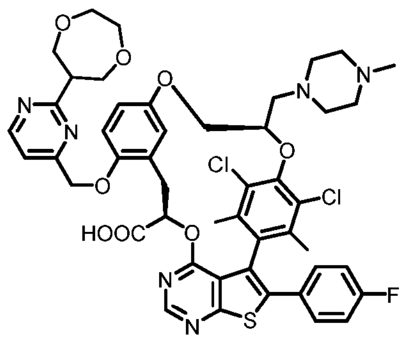,
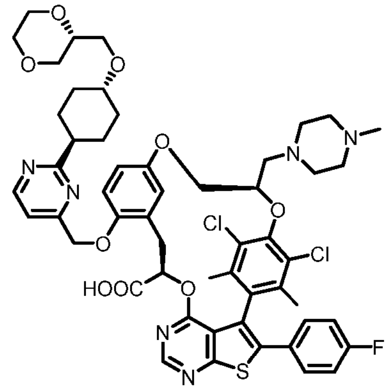,

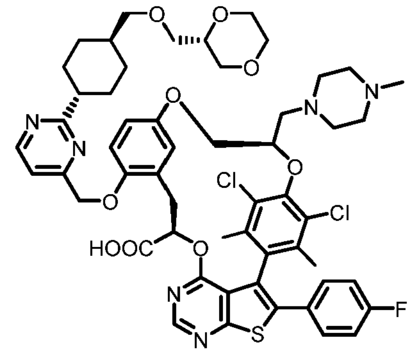,
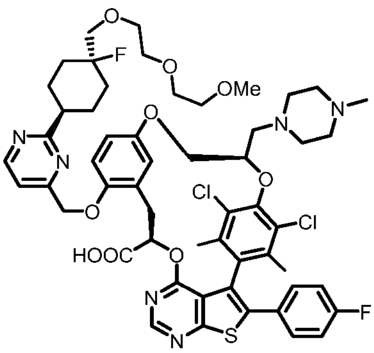,

or

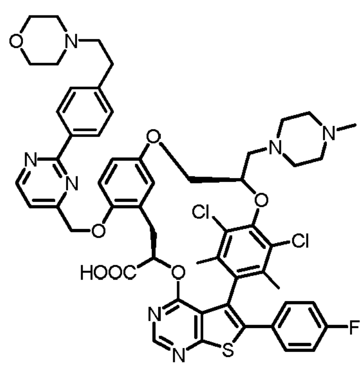.

In some embodiments, the linker-drug (or "linker-payload") moiety -(L-D) may comprise a compound selected from Table A..

<u>Drug Loading</u>

Drug loading is represented by *p,* and is also referred to herein as the drug-to-antibody ratio (DAR). Drug loading

may range from 1 to 16 drug moieties per antibody or antigen-binding fragment. In some embodiments, *p* is an integer from 1 to 16. In some embodiments, *p* is an integer from 1 to 16, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3, or 1 to 2. In some embodiments, *p* is an integer from 2 to 10, 2 to 9, 2 to 8, 2 to 7, 2 to 6, 2 to 5, 2 to 4, or 2 to 3. In some embodiments, *p* is an integer from 1 to 16. In some embodiments, *p* is an integer from 1 to 8. In some embodiments, *p* is an integer from 1 to 5. In some embodiments, *p* is an integer from 2 to 4. In some embodiments, *p* is 1, 2, 3, 4, 5, 6, 7, or 8. In some embodiments, *p* is 2. In some embodiments, *p* is 4.

Drug loading may be limited by the number of attachment sites on the antibody or antigen-binding fragment. In some embodiments, the linker moiety (L) of the ADC attaches to the antibody or antigen-binding fragment through a chemically active group on one or more amino acid residues on the antibody or antigen-binding fragment. For example, the linker may be attached to the antibody or antigen-binding fragment via a free amino, imino, hydroxyl, thiol, or carboxyl group (e.g., to the N- or C-terminus, to the epsilon amino group of one or more lysine residues, to the free carboxylic acid group of one or more glutamic acid or aspartic acid residues, or to the sulfhydryl group of one or more cysteine residues). The site to which the linker is attached can be a natural residue in the amino acid sequence of the antibody or antigen-binding fragment, or it can be introduced into the antibody or antigen-binding fragment, e.g., by DNA recombinant technology (e.g., by introducing a cysteine residue into the amino acid sequence) or by protein biochemistry (e.g., by reduction, pH adjustment, or hydrolysis).

In some embodiments, the number of drug moieties that can be conjugated to an antibody or antigen-binding fragment is limited by the number of free cysteine residues. For example, where the attachment is a cysteine thiol group, an antibody may have only one or a few cysteine thiol groups, or may have only one or a few sufficiently reactive thiol groups through which a linker may be attached. Generally, antibodies do not contain many free and reactive cysteine thiol groups that may be linked to a drug moiety. Indeed, most cysteine thiol residues in antibodies are involved in either interchain or intrachain disulfide bonds. Conjugation to cysteines can therefore, in some embodiments, require at least partial reduction of the antibody. Over-attachment of linker-toxin to an antibody may destabilize the antibody by reducing the cysteine residues available to form disulfide bonds. Therefore, an optimal drug:antibody ratio should increase potency of the ADC (by increasing the number of attached drug moieties per antibody) without destabilizing the antibody or antigen-binding fragment. In some embodiments, an optimal ratio may be 2, 4, 6, or 8. In some embodiments, an optimal ratio may be 2 or 4.

In some embodiments, an antibody or antigen-binding fragment is exposed to reducing conditions prior to conjugation in order to generate one or more free cysteine residues. An antibody, in some embodiments, may be reduced with a reducing agent such as dithiothreitol (DTT) or tris(2-carboxyethyl)phosphine (TCEP), under partial or total reducing conditions, to generate reactive cysteine thiol groups. Unpaired cysteines may be generated through partial reduction with limited molar equivalents of TCEP, which can reduce the interchain disulfide bonds which link the light chain and heavy chain (one pair per H-L pairing) and the two heavy chains in the hinge region (two pairs per H-H pairing in the case of human IgG1) while leaving the intrachain disulfide bonds intact (Stefano et al. (2013) Methods Mol Biol. 1045:145-71). In embodiments, disulfide bonds within the antibodies are reduced electrochemically, e.g., by employing a working electrode that applies an alternating reducing and oxidizing voltage. This approach can allow for on-line coupling of disulfide bond reduction to an analytical device (e.g., an electrochemical detection device, an NMR spectrometer, or a mass spectrometer) or a chemical separation device (e.g., a liquid chromatograph (e.g., an HPLC) or an electrophoresis device (see, e.g., US 2014/0069822)). In some embodiments, an antibody is subjected to denaturing conditions to reveal reactive nucleophilic groups on amino acid residues, such as cysteine.

The drug loading of an ADC may be controlled in different ways, e.g., by: (i) limiting the molar excess of drug-linker intermediate or linker reagent relative to antibody; (ii) limiting the conjugation reaction time or temperature; (iii) partial or limiting reductive conditions for cysteine thiol modification; and/or (iv) engineering by recombinant techniques the amino acid sequence of the antibody such that the number and position of cysteine residues is modified for control of the number and/or position of linker-drug attachments.

In some embodiments, free cysteine residues are introduced into the amino acid sequence of the antibody or antigen-binding fragment. For example, cysteine engineered antibodies can be prepared wherein one or more amino acids of a parent antibody are replaced with a cysteine amino acid. Any form of antibody may be so engineered, i.e. mutated. For example, a parent Fab antibody fragment may be engineered to form a cysteine engineered Fab referred to as a "ThioFab." Similarly, a parent monoclonal antibody may be engineered to form a "ThioMab." A single site mutation yields a single engineered cysteine residue in a ThioFab, whereas a single site mutation yields two engineered cysteine residues in a ThioMab, due to the dimeric nature of the IgG antibody. DNA encoding an amino acid sequence variant of the parent polypeptide can be prepared by a variety of methods known in the art (see, e.g., the methods described in WO 2006/034488). These methods include, but are not limited to, preparation by site-directed (or oligonucleotide-mediated) mutagenesis, PCR mutagenesis, and cassette mutagenesis of an earlier prepared DNA encoding the polypeptide. Variants of recombinant antibodies may also be constructed by restriction fragment manipulation or by overlap extension PCR with synthetic oligonucleotides. ADCs of Formula (1) include, but are not limited to, antibodies that have 1, 2, 3, or 4 engineered cysteine amino acids (Lyon et al. (2012) Methods Enzymol. 502:123-38). In some embodiments, one or more free cysteine residues are already present in an antibody or antigen-binding fragment, without the use of engineering, in

which case the existing free cysteine residues may be used to conjugate the antibody or antigen-binding fragment to a drug moiety.

Where more than one nucleophilic group reacts with a drug-linker intermediate or a linker moiety reagent followed by drug moiety reagent, in a reaction mixture comprising multiple copies of the antibody or antigen-binding fragment and linker moiety, then the resulting product can be a mixture of ADC compounds with a distribution of one or more drug moieties attached to each copy of the antibody or antigen-binding fragment in the mixture. In some embodiments, the drug loading in a mixture of ADCs resulting from a conjugation reaction ranges from 1 to 16 drug moieties attached per antibody or antigen-binding fragment. The average number of drug moieties per antibody or antigen-binding fragment (i.e., the average drug loading, or average *p*) may be calculated by any conventional method known in the art, e.g., by mass spectrometry (e.g., liquid chromatography-mass spectrometry (LC-MS)) and/or high-performance liquid chromatography (e.g., HIC-HPLC). In some embodiments, the average number of drug moieties per antibody or antigen-binding fragment is determined by liquid chromatography-mass spectrometry (LC-MS). In some embodiments, the average number of drug moieties per antibody or antigen-binding fragment is from about 1.5 to about 3.5, about 2.5 to about 4.5, about 3.5 to about 5.5, about 4.5 to about 6.5, about 5.5 to about 7.5, about 6.5 to about 8.5, or about 7.5 to about 9.5. In some embodiments, the average number of drug moieties per antibody or antigen-binding fragment is from about 2 to about 4, about 3 to about 5, about 4 to about 6, about 5 to about 7, about 6 to about 8, about 7 to about 9, about 2 to about 8, or about 4 to about 8.

In some embodiments, the average number of drug moieties per antibody or antigen-binding fragment is about 2. In some embodiments, the average number of drug moieties per antibody or antigen-binding fragment is about 1.5, about 1.6, about 1.7, about 1.8, about 1.9, about 2, about 2.1, about 2.2, about 2.3, about 2.4, or about 2.5. In some embodiments, the average number of drug moieties per antibody or antigen-binding fragment is 2.

In some embodiments, the average number of drug moieties per antibody or antigen-binding fragment is about 4. In some embodiments, the average number of drug moieties per antibody or antigen-binding fragment is about 3.5, about 3.6, about 3.7, about 3.8, about 3.9, about 4, about 4.1, about 4.2, about 4.3, about 4.4, or about 4.5. In some embodiments, the average number of drug moieties per antibody or antigen-binding fragment is 4.

In some embodiments, the term "about," as used with respect to the average number of drug moieties per antibody or antigen-binding fragment, means plus or minus 20%, 15%, 10%, 5%, or 1%. In one embodiment, the term "about" refers to a range of values which are 10% more or less than the specified value. In another embodiment, the term "about" refers to a range of values which are 5% more or less than the specified value. In another embodiment, the term "about" refers to a range of values which are 1% more or less than the specified value.

Individual ADC compounds, or "species," may be identified in the mixture by mass spectroscopy and separated by, e.g., UPLC or HPLC, e.g. hydrophobic interaction chromatography (HIC-HPLC). In some embodiments, a homogeneous or nearly homogenous ADC product with a single loading value may be isolated from the conjugation mixture, e.g., by electrophoresis or chromatography.

In some embodiments, higher drug loading (e.g., $p > 16$) may cause aggregation, insolubility, toxicity, or loss of cellular permeability of certain antibody-drug conjugates. Higher drug loading may also negatively affect the pharmacokinetics (e.g., clearance) of certain ADCs. In some embodiments, lower drug loading (e.g., $p < 2$) may reduce the potency of certain ADCs against target-expressing cells. In some embodiments, the drug loading for an ADC of the present disclosure ranges from about 2 to about 16, about 2 to about 10, about 2 to about 8; from about 2 to about 6; from about 2 to about 5; from about 3 to about 5; from about 2 to about 4; or from about 4 to about 8.

In some embodiments, a drug loading and/or an average drug loading of about 2 is achieved, e.g., using partial reduction of intrachain disulfides on the antibody or antigen-binding fragment, and provides beneficial properties. In some embodiments, a drug loading and/or an average drug loading of about 4 or about 6 or about 8 is achieved, e.g., using partial reduction of intrachain disulfides on the antibody or antigen-binding fragment, and provides beneficial properties. In some embodiments, a drug loading and/or an average drug loading of less than about 2 may result in an unacceptably high level of unconjugated antibody species, which can compete with the ADC for binding to the target antigen CD74 and/or provide for reduced treatment efficacy. In some embodiments, a drug loading and/or average drug loading of more than about 16 may result in an unacceptably high level of product heterogeneity and/or ADC aggregation. A drug loading and/or an average drug loading of more than about 16 may also affect stability of the ADC, due to loss of one or more chemical bonds required to stabilize the antibody or antigen-binding fragment.

The present disclosure includes methods of producing the described ADCs. Briefly, the ADCs comprise an antibody or antigen-binding fragment as the antibody or antigen-binding fragment, a drug moiety (e.g., an Mcl-1 inhibitor), and a linker that joins the drug moiety and the antibody or antigen-binding fragment. In some embodiments, the ADCs can be prepared using a linker having reactive functionalities for covalently attaching to the drug moiety and to the antibody or antigen-binding fragment. In some embodiments, the antibody or antigen-binding fragment is functionalized to prepare a functional group that is reactive with a linker or a drug-linker intermediate. For example, in some embodiments, a cysteine thiol of an antibody or antigen-binding fragment can form a bond with a reactive functional group of a linker or a drug-linker intermediate to make an ADC. In some embodiments, an antibody or antigen-binding fragment is prepared with bacterial

transglutaminase (BTG) - reactive glutamines specifically functionalized with an amine containing cyclooctyne BCN (N-[(1*R*,8*S*,9*s*)-Bicyclo[6.1.0]non-4-yn-9-ylmethyloxycarbonyl]-1,8-diamino-3,6-dioxaoctane) moiety. In some embodiments, site-specific conjugation of a linker or a drug-linker intermediate to a BCN moiety of an antibody or antigen-binding fragment is performed, e.g., as described and exemplified herein. The generation of the ADCs can be accomplished by techniques known to the skilled artisan.

In some embodiments, an ADC is produced by contacting an antibody or antigen-binding fragment with a linker and a drug moiety (e.g., an Mcl-1 inhibitor) in a sequential manner, such that the antibody or antigen-binding fragment is covalently linked to the linker first, and then the pre-formed antibody-linker intermediate reacts with the drug moiety. The antibody-linker intermediate may or may not be subjected to a purification step prior to contacting the drug moiety. In other embodiments, an ADC is produced by contacting an antibody or antigen-binding fragment with a linker-drug compound pre-formed by reacting a linker with a drug moiety. The pre-formed linker-drug compound may or may not be subjected to a purification step prior to contacting the antibody or antigen-binding fragment. In other embodiments, the antibody or antigen-binding fragment contacts the linker and the drug moiety in one reaction mixture, allowing simultaneous formation of the covalent bonds between the antibody or antigen-binding fragment and the linker, and between the linker and the drug moiety. This method of producing ADCs may include a reaction, wherein the antibody or antigen-binding fragment contacts the antibody or antigen-binding fragment prior to the addition of the linker to the reaction mixture, and vice versa. In some embodiments, an ADC is produced by reacting an antibody or antigen-binding fragment with a linker joined to a drug moiety, such as an Mcl-1 inhibitor, under conditions that allow conjugation.

The ADCs prepared according to the methods described above may be subjected to a purification step. The purification step may involve any biochemical methods known in the art for purifying proteins, or any combination of methods thereof. These include, but are not limited to, tangential flow filtration (TFF), affinity chromatography, ion exchange chromatography, any charge or isoelectric point-based chromatography, mixed mode chromatography, e.g., CHT (ceramic hydroxyapatite), hydrophobic interaction chromatography, size exclusion chromatography, dialysis, filtration, selective precipitation, or any combination thereof.

## Therapeutic Uses and Compositions

Disclosed herein are methods of using the compositions described herein, e.g., the disclosed ADC compounds and compositions, in treating a subject for a disorder, e.g., a cancer. Compositions, e.g., ADCs, may be administered alone or in combination with at least one additional inactive and/or active agent, e.g., at least one additional therapeutic agent, and may be administered in any pharmaceutically acceptable formulation, dosage, and dosing regimen. Treatment efficacy may be evaluated for toxicity as well as indicators of efficacy and adjusted accordingly. Efficacy measures include, but are not limited to, a cytostatic and/or cytotoxic effect observed in vitro or in vivo, reduced tumor volume, tumor growth inhibition, and/or prolonged survival.

Methods of determining whether an ADC exerts a cytostatic and/or cytotoxic effect on a cell are known. For example, the cytotoxic or cytostatic activity of an ADC can be measured by, e.g., exposing mammalian cells expressing the target antigen CD74 of the ADC in a cell culture medium; culturing the cells for a period from about 6 hours to about 6 days; and measuring cell viability (e.g., using a CellTiter-Glo® (CTG) or MTT cell viability assay). Cell-based in vitro assays may also be used to measure viability (proliferation), cytotoxicity, and induction of apoptosis (caspase activation) of the ADC.

For determining cytotoxicity, necrosis or apoptosis (programmed cell death) may be measured. Necrosis is typically accompanied by increased permeability of the plasma membrane, swelling of the cell, and rupture of the plasma membrane. Apoptosis can be quantitated, for example, by measuring DNA fragmentation. Commercial photometric methods for the quantitative in vitro determination of DNA fragmentation are available. Examples of such assays, including TUNEL (which detects incorporation of labeled nucleotides in fragmented DNA) and ELISA-based assays, are described in Biochemica (1999) 2:34-7 (Roche Molecular Biochemicals).

Apoptosis may also be determined by measuring morphological changes in a cell. For example, as with necrosis, loss of plasma membrane integrity can be determined by measuring uptake of certain dyes (e.g., a fluorescent dye such as, for example, acridine orange or ethidium bromide). A method for measuring apoptotic cell number has been described by Duke and Cohen, Current Protocols in Immunology (Coligan et al., eds. (1992) pp. 3.17.1-3.17.16). Cells also can be labeled with a DNA dye (e.g., acridine orange, ethidium bromide, or propidium iodide) and the cells observed for chromatin condensation and margination along the inner nuclear membrane. Apoptosis may also be determined, in some embodiments, by screening for caspase activity. In some embodiments, a Caspase-Glo® Assay can be used to measure activity of caspase-3 and caspase-7. In some embodiments, the assay provides a luminogenic caspase-3/7 substrate in a reagent optimized for caspase activity, luciferase activity, and cell lysis. In some embodiments, adding Caspase-Glo® 3/7 Reagent in an "add-mix-measure" format may result in cell lysis, followed by caspase cleavage of the substrate and generation of a "glow-type" luminescent signal, produced by luciferase. In some embodiments, luminescence may be proportional to the amount of caspase activity present, and can serve as an indicator of apoptosis. Other morphological changes that can be measured to determine apoptosis include, e.g., cytoplasmic condensation, increased membrane blebbing, and cellular

shrinkage. Determination of any of these effects on cancer cells indicates that an ADC is useful in the treatment of cancers.

Cell viability may be measured, e.g., by determining in a cell the uptake of a dye such as neutral red, trypan blue, Crystal Violet, or ALAMAR™ blue (see, e.g., Page et al. (1993) Intl J Oncology 3:473-6). In such an assay, the cells are incubated in media containing the dye, the cells are washed, and the remaining dye, reflecting cellular uptake of the dye, is measured spectrophotometrically.

Cell viability may also be measured, e.g., by quantifying ATP, an indicator of metabolically active cells. In some embodiments, in vitro potency and/or cell viability of prepared ADCs or Mcl-1 inhibitor compounds may be assessed using a CellTiter-Glo® (CTG) cell viability assay, as described in the examples provided herein. In this assay, in some embodiments, the single reagent (CellTiter-Glo® Reagent) is added directly to cells cultured in serum-supplemented medium. The addition of reagent results in cell lysis and generation of a luminescent signal proportional to the amount of ATP present. The amount of ATP is directly proportional to the number of cells present in culture

Cell viability may also be measured, e.g., by measuring the reduction of tetrazolium salts. In some embodiments, in vitro potency and/or cell viability of prepared ADCs or Mcl-1 inhibitor compounds may be assessed using an MTT cell viability assay, as described in the examples provided herein. In this assay, in some embodiments, the yellow tetrazolium MTT (3-(4, 5-dimethylthiazolyl-2)-2,5-diphenyltetrazolium bromide) is reduced by metabolically active cells, in part by the action of dehydrogenase enzymes, to generate reducing equivalents such as NADH and NADPH. The resulting intracellular purple formazan can then be solubilized and quantified by spectrophotometric means.

In certain aspects, the present disclosure features a method of killing, inhibiting or modulating the growth of a cancer cell or tissue by disrupting the expression and/or activity of Mcl-1 and/or one or more upstream modulators or downstream targets thereof. The method may be used with any subject where disruption of Mcl-1 expression and/or activity provides a therapeutic benefit. Subjects that may benefit from disrupting Mcl-1 expression and/or activity include, but are not limited to, those having or at risk of having a cancer such as a tumor or a hematological cancer. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer.

Exemplary methods include the steps of contacting a cell with an ADC, as described herein, in an effective amount, i.e., an amount sufficient to kill the cell. The method can be used on cells in culture, e.g., in vitro, in vivo, ex vivo, or in situ. For example, cells that express CD74 (e.g., cells collected by biopsy of a tumor or metastatic lesion; cells from an established cancer cell line; or recombinant cells), can be cultured in vitro in culture medium and the contacting step can be affected by adding the ADC to the culture medium. The method will result in killing of cells expressing CD74, including in particular cancer cells expressing CD74. Alternatively, the ADC can be administered to a subject by any suitable administration route (e.g., intravenous, subcutaneous, or direct contact with a tumor tissue) to have an effect in vivo.

The in vivo effect of a disclosed ADC therapeutic composition can be evaluated in a suitable animal model. For example, xenogeneic cancer models can be used, wherein cancer explants or passaged xenograft tissues are introduced into immune compromised animals, such as nude or SCID mice (Klein et al. (1997) Nature Med. 3:402-8). Efficacy may be predicted using assays that measure inhibition of tumor formation, tumor regression or metastasis, and the like.

In vivo assays that evaluate the promotion of tumor death by mechanisms such as apoptosis may also be used. In some embodiments, xenografts from tumor bearing mice treated with the therapeutic composition can be examined for the presence of apoptotic foci and compared to untreated control xenograft-bearing mice. The extent to which apoptotic foci are found in the tumors of the treated mice provides an indication of the therapeutic efficacy of the composition.

Further provided herein are methods of treating a disorder, e.g., a cancer. The compositions described herein, e.g., the ADCs disclosed herein, can be administered to a non-human mammal or human subject for therapeutic purposes. The therapeutic methods include administering to a subject having or suspected of having a cancer a therapeutically effective amount of a composition comprising an Mcl-1 inhibitor, e.g., an ADC where the inhibitor is linked to a targeting antibody that binds to an antigen (1) expressed on a cancer cell, (2) is accessible to binding, and/or (3) is localized or predominantly expressed on a cancer cell surface as compared to a non-cancer cell.

An exemplary embodiment is a method of treating a subject having or suspected of having a cancer, comprising administering to the subject a therapeutically effective amount of a composition disclosed herein, e.g., an ADC, composition, or pharmaceutical composition (e.g., any of the exemplary ADCs, compositions, or pharmaceutical compositions disclosed herein). In some embodiments, the cancer expresses the target antigen CD74.. In some embodiments, the cancer is a tumor or a hematological cancer. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer.

Another exemplary embodiment is a method of delivering an Mcl-1 inhibitor to a cell expressing CD74, comprising conjugating the Mcl-1 inhibitor to an antibody that immunospecifically binds to a CD74 epitope and exposing the cell to the ADC. Exemplary cancer cells that express CD74 for which the ADCs of the present disclosure are indicated include multiple myeloma cells.

In certain aspects, the present disclosure further provides methods of reducing or inhibiting growth of a tumor (e.g., a CD74-expressing tumor), comprising administering a therapeutically effective amount of an ADC or composition comprising an ADC. In some embodiments, the treatment is sufficient to reduce or inhibit the growth of the patient's tumor, reduce the number or size of metastatic lesions, reduce tumor load, reduce primary tumor load, reduce invasiveness, prolong survival time, and/or maintain or improve the quality of life. In some embodiments, the tumor is resistant or refractory to treatment with the antibody or antigen-binding fragment of the ADC (e.g., an anti-CD74 antibody) when administered alone, and/or the tumor is resistant or refractory to treatment with the Mcl-1 inhibitor drug moiety when administered alone.

An exemplary embodiment is a method of reducing or inhibiting the growth of a tumor in a subject, comprising administering to the subject a therapeutically effective amount of an ADC, composition, or pharmaceutical composition (e.g., any of the exemplary ADCs, compositions, or pharmaceutical compositions disclosed herein). In some embodiments, the tumor expresses the target antigen CD74. In some embodiments, the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer. In some embodiments, the tumor is a gastric cancer. In some embodiments, administration of the ADC, composition, or pharmaceutical composition reduces or inhibits the growth of the tumor by at least about 10%, at least about 20%, at least about 30%, at least about 40%, at least about 50%, at least about 60%, at least about 70%, at least about 80%, at least about 90%, at least about 95%, or at least about 99%, as compared to growth in the absence of treatment.

Another exemplary embodiment is a method of delaying or slowing the growth of a tumor in a subject, comprising administering to the subject a therapeutically effective amount of an ADC, composition, or pharmaceutical composition (e.g., any of the exemplary ADCs, compositions, or pharmaceutical compositions disclosed herein). In some embodiments, the tumor expresses the target antigen CD74. In some embodiments, the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer. In some embodiments, the tumor is a gastric cancer. In some embodiments, administration of the ADC, composition, or pharmaceutical composition delays or slows the growth of the tumor by at least about 10%, at least about 20%, at least about 30%, at least about 40%, at least about 50%, at least about 60%, at least about 70%, at least about 80%, at least about 90%, at least about 95%, or at least about 99%, as compared to growth in the absence of treatment.

In certain aspects, the present disclosure further provides methods of reducing or slowing the expansion of a cancer cell population (e.g., a CD74-expressing cancer cell population), comprising administering a therapeutically effective amount of an ADC or composition comprising an ADC.

An exemplary embodiment is a method of reducing or slowing the expansion of a cancer cell population in a subject, comprising administering to the subject a therapeutically effective amount of an ADC, composition, or pharmaceutical composition (e.g., any of the exemplary ADCs, compositions, or pharmaceutical compositions disclosed herein). In some embodiments, the cancer cell population expresses the target antigen CD74. In some embodiments, the cancer cell population is from a tumor or a hematological cancer. In some embodiments, the cancer cell population is from a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer cell population is from a lymphoma or gastric cancer. In some embodiments, administration of the ADC, composition, or pharmaceutical composition reduces the cancer cell population by at least about 10%, at least about 20%, at least about 30%, at least about 40%, at least about 50%, at least about 60%, at least about 70%, at least about 80%, at least about 90%, at least about 95%, or at least about 99%, as compared to the population in the absence of treatment. In some embodiments, administration of the ADC, composition, or pharmaceutical composition slows the expansion of the cancer cell population by at least about 10%, at least about 20%, at least about 30%, at least about 40%, at least about 50%, at least about 60%, at least about 70%, at least about 80%, at least about 90%, at least about 95%, or at least about 99%, as compared to expansion in the absence of treatment.

Also provided herein are methods of determining whether a subject having or suspected of having a cancer will be responsive to treatment with the disclosed ADCs and compositions. An exemplary embodiment is a method of determining whether a subject having or suspected of having a cancer will be responsive to treatment with an ADC, composition, or

pharmaceutical composition (e.g., any of the exemplary ADCs, compositions, or pharmaceutical compositions disclosed herein) by providing a biological sample from the subject; contacting the sample with the ADC; and detecting binding of the ADC to cancer cells in the sample. In some embodiments, the sample is a tissue biopsy sample, a blood sample, or a bone marrow sample. In some embodiments, the method comprises providing a biological sample from the subject; contacting the sample with the ADC; and detecting one or more markers of cancer cell death in the sample (e.g., increased expression of one or more apoptotic markers, reduced expansion of a cancer cell population in culture, etc.).

Further provided herein are therapeutic uses of the disclosed ADCs and compositions. An exemplary embodiment is an ADC, composition, or pharmaceutical composition (e.g., any of the exemplary ADCs, compositions, or pharmaceutical compositions disclosed herein) for use in treating a subject having or suspected of having a cancer (e.g., a CD74-expressing cancer). Another exemplary embodiment is a use of an ADC, composition, or pharmaceutical composition (e.g., any of the exemplary ADCs, compositions, or pharmaceutical compositions disclosed herein) in treating a subject having or suspected of having a cancer (e.g., a CD74-expressing cancer). Another exemplary embodiment is a use of an ADC, composition, or pharmaceutical composition (e.g., any of the exemplary ADCs, compositions, or pharmaceutical compositions disclosed herein) in a method of manufacturing a medicament for treating a subject having or suspected of having a cancer (e.g., a CD74-expressing cancer). Methods for identifying subjects having cancers that express the target antigen CD74 are known in the art and may be used to identify suitable patients for treatment with a disclosed ADC compound or composition.

Moreover, ADCs of the present disclosure may be administered to a non-human mammal expressing an antigen with which the ADC is capable of binding for veterinary purposes or as an animal model of human disease. Regarding the latter, such animal models may be useful for evaluating the therapeutic efficacy of the disclosed ADCs (e.g., testing of dosages and time courses of administration).

The therapeutic compositions used in the practice of the foregoing methods may be formulated into pharmaceutical compositions comprising a pharmaceutically acceptable carrier suitable for the desired delivery method. An exemplary embodiment is a pharmaceutical composition comprising an ADC of the present disclosure and a pharmaceutically acceptable carrier, e.g., one suitable for a chosen means of administration, e.g., intravenous administration. The pharmaceutical composition may also comprise one or more additional inactive and/or therapeutic agents that are suitable for treating or preventing, for example, a cancer (e.g., a standard-of-care agent, etc.). The pharmaceutical composition may also comprise one or more carrier, excipient, and/or stabilizer components, and the like. Methods of formulating such pharmaceutical compositions and suitable formulations are known in the art (see, e.g., "Remington's Pharmaceutical Sciences," Mack Publishing Co., Easton, PA).

Suitable carriers include any material that, when combined with the therapeutic composition, retains the antitumor function of the therapeutic composition and is generally non-reactive with the patient's immune system. Pharmaceutically acceptable carriers include any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol, mesylate salt, and the like, as well as combinations thereof. In many cases, isotonic agents are included, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the ADC.

A pharmaceutical composition of the present disclosure can be administered by a variety of methods known in the art. The route and/or mode of administration may vary depending upon the desired results. In some embodiments, the therapeutic formulation is solubilized and administered via any route capable of delivering the therapeutic composition to the cancer site. Potentially effective routes of administration include, but are not limited to, parenteral (e.g., intravenous, subcutaneous), intraperitoneal, intramuscular, intratumor, intradermal, intraorgan, orthotopic, and the like. In some embodiments, the administration is intravenous, subcutaneous, intraperitoneal, or intramuscular. The pharmaceutically acceptable carrier should be suitable for the route of administration, e.g., intravenous or subcutaneous administration (e.g., by injection or infusion). Depending on the route of administration, the active compound(s), i.e., the ADC and/or any additional therapeutic agent, may be coated in a material to protect the compound(s) from the action of acids and other natural conditions that may inactivate the compound(s). Administration can be either systemic or local.

The therapeutic compositions disclosed herein may be sterile and stable under the conditions of manufacture and storage, and may be in a variety of forms. These include, for example, liquid, semi-solid, and solid dosage forms, such as liquid solutions (e.g., injectable and infusible solutions), dispersions or suspensions, tablets, pills, powders, liposomes, and suppositories. The form depends on the intended mode of administration and therapeutic application. In some embodiments, the disclosed ADCs can be incorporated into a pharmaceutical composition suitable for parenteral administration. The injectable solution may be composed of either a liquid or lyophilized dosage form in a flint or amber vial, ampule, or pre-filled syringe, or other known delivery or storage device. In some embodiments, one or more of the ADCs or pharmaceutical compositions is supplied as a dry sterilized lyophilized powder or water free concentrate in a hermetically sealed container and can be reconstituted (e.g., with water or saline) to the appropriate concentration for

administration to a subject.

Typically, a therapeutically effective amount or efficacious amount of a disclosed composition, e.g., a disclosed ADC, is employed in the pharmaceutical compositions of the present disclosure. The composition, e.g., one comprising an ADC, may be formulated into a pharmaceutically acceptable dosage form by conventional methods known in the art. Dosages and administration protocols for the treatment of cancers using the foregoing methods will vary with the method and the target cancer, and will generally depend on a number of other factors appreciated in the art.

Dosage regimens for compositions disclosed herein, e.g., those comprising ADCs alone or in combination with at least one additional inactive and/or active therapeutic agent, may be adjusted to provide the optimum desired response (e.g., a therapeutic response). For example, a single bolus of one or both agents may be administered at one time, several divided doses may be administered over a predetermined period of time, or the dose of one or both agents may be proportionally increased or decreased as indicated by the exigencies of the therapeutic situation. In some embodiments, treatment involves single bolus or repeated administration of the ADC preparation via an acceptable route of administration. In some embodiments, the ADC is administered to the patient daily, weekly, monthly, or any time period in between. For any particular subject, specific dosage regimens may be adjusted over time according to the individual's need, and the professional judgment of the treating clinician. Parenteral compositions may be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Dosage values for compositions comprising an ADC and/or any additional therapeutic agent(s), may be selected based on the unique characteristics of the active compound(s), and the particular therapeutic effect to be achieved. A physician or veterinarian can start doses of the ADC employed in the pharmaceutical composition at levels lower than that required to achieve the desired therapeutic effect and gradually increase the dosage until the desired effect is achieved. In general, effective doses of the compositions of the present disclosure, for the treatment of a cancer may vary depending upon many different factors, including means of administration, target site, physiological state of the patient, whether the patient is human or an animal, other medications administered, and whether treatment is prophylactic or therapeutic. The selected dosage level may also depend upon a variety of pharmacokinetic factors including the activity of the particular compositions of the present disclosure employed, or the ester, salt, or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors. Treatment dosages may be titrated to optimize safety and efficacy.

Toxicity and therapeutic efficacy of compounds provided herein can be determined by standard pharmaceutical procedures in cell culture or in animal models. For example, LD50, ED50, EC50, and IC50 may be determined, and the dose ratio between toxic and therapeutic effects (LD50/ED50) may be calculated as the therapeutic index. The data obtained from in vitro and in vivo assays can be used in estimating or formulating a range of dosage for use in humans. For example, the compositions and methods disclosed herein may initially be evaluated in xenogeneic cancer models (e.g., an NCI-H929 multiple myeloma mouse model).

In some embodiments, an ADC or composition comprising an ADC is administered on a single occasion. In other embodiments, an ADC or composition comprising an ADC is administered on multiple occasions. Intervals between single dosages can be, e.g., daily, weekly, monthly, or yearly. Intervals can also be irregular, based on measuring blood levels of the administered agent (e.g., the ADC) in the patient in order to maintain a relatively consistent plasma concentration of the agent. The dosage and frequency of administration of an ADC or composition comprising an ADC may also vary depending on whether the treatment is prophylactic or therapeutic. In prophylactic applications, a relatively low dosage may be administered at relatively infrequent intervals over a long period of time. Some patients continue to receive treatment for the rest of their lives. In therapeutic applications, a relatively higher dosage at relatively shorter intervals is sometimes required until progression of the disease is reduced or terminated, and preferably until the patient shows partial or complete amelioration of one or more symptoms of disease. Thereafter, the patient may be administered a lower, e.g., prophylactic regime.

The above therapeutic approaches can be combined with any one of a wide variety of additional surgical, chemotherapy, or radiation therapy regimens. In some embodiments, the ADCs or compositions disclosed herein are co-formulated and/or co-administered with one or more additional therapeutic agents, e.g., one or more chemotherapeutic agents, one or more standard-of-care agents for the particular condition being treated.

Kits for use in the therapeutic and/or diagnostic applications described herein are also provided. Such kits may comprise a carrier, package, or container that is compartmentalized to receive one or more containers such as vials, tubes, and the like, each of the container(s) comprising one of the separate elements to be used in a method disclosed herein. A label may be present on or with the container(s) to indicate that an ADC or composition within the kit is used for a specific therapy or non-therapeutic application, such as a prognostic, prophylactic, diagnostic, or laboratory application. A label may also indicate directions for either in vivo or in vitro use, such as those described herein. Directions and or other

information may also be included on an insert(s) or label(s), which is included with or on the kit. The label may be on or associated with the container. A label may be on a container when letters, numbers, or other characters forming the label are molded or etched into the container itself. A label may be associated with a container when it is present within a receptacle or carrier that also holds the container, e.g., as a package insert. The label may indicate that an ADC or composition within the kit is used for diagnosing or treating a condition, such as a cancer a described herein.

In some embodiments, a kit comprises an ADC or composition comprising an ADC. In some embodiments, the kit further comprises one or more additional components, including but not limited to: instructions for use; other reagents, e.g., a therapeutic agent (e.g., a standard-of-care agent); devices, containers, or other materials for preparing the ADC for administration; pharmaceutically acceptable carriers; and devices, containers, or other materials for administering the ADC to a subject. Instructions for use can include guidance for therapeutic applications including suggested dosages and/or modes of administration, e.g., in a patient having or suspected of having a cancer. In some embodiments, the kit comprises an ADC and instructions for use of the ADC in treating, preventing, and/or diagnosing a cancer.

## COMBINATION THERAPIES

In some embodiments, the present disclosure provides methods of treatment wherein the antibody-drug conjugates disclosed herein are administered in combination with one or more additional therapeutic agents. Exemplary combination partners are disclosed herein.

In certain embodiments, a combination described herein comprises a PD-1 inhibitor. In some embodiments, the PD-1 inhibitor is chosen from PDR001 (Novartis), Nivolumab (Bristol-Myers Squibb), Pembrolizumab (Merck & Co), Pidilizumab (CureTech), MEDI0680 (Medimmune), REGN2810 (Regeneron), TSR-042 (Tesaro), PF-06801591 (Pfizer), BGB-A317 (Beigene), BGB-108 (Beigene), INCSHR1210 (Incyte), or AMP-224 (Amplimmune). In some embodiments, the PD-1 inhibitor is PDR001. PDR001 is also known as Spartalizumab.

In certain embodiments, a combination described herein comprises a LAG-3 inhibitor. In some embodiments, the LAG-3 inhibitor is chosen from LAG525 (Novartis), BMS-986016 (Bristol-Myers Squibb), or TSR-033 (Tesaro).

In certain embodiments, a combination described herein comprises a TIM-3 inhibitor. In some embodiments, the TIM-3 inhibitor is MBG453 (Novartis), TSR-022 (Tesaro), LY-3321367 (Eli Lily), Sym23 (Symphogen), BGB-A425 (Beigene), INCAGN-2390 (Agenus), BMS-986258 (BMS), RO-7121661 (Roche), or LY-3415244 (Eli Lilly).

In certain embodiments, a combination descdribed herein comprises a PDL1 inhibitor. In one embodiment, the PDL1 inhibitor is chosen from FAZ053 (Novartis), atezolizumab (Genentech), durvalumab (Astra Zeneca), or avelumab (Pfizer).

In certain embodiments, a combination described herein comprises a GITR agonist. In some embodiments, the GITR agonist is chosen from GWN323 (NVS), BMS-986156, MK-4166 or MK-1248 (Merck), TRX518 (Leap Therapeutics), INCAGN1876 (Incyte/Agenus), AMG 228 (Amgen) or INBRX-110 (Inhibrx).

In some embodiments, a combination described herein comprises an IAP inhibitor. In some embodiments, the IAP inhibitor comprises LCL161 or a compound disclosed in International Application Publication No. WO 2008/016893.

In an embodiment, the combination comprises an mTOR inhibitor, e.g., RAD001 (also known as everolimus).

In an embodiment, the combination comprises a HDAC inhibitor, e.g., LBH589. LBH589 is also known as panobinostat.

In an embodiment, the combination comprises an IL-17 inhibitor, e.g., CJM112.

In certain embodiments, a combination described herein comprises an estrogen receptor (ER) antagonist. In some embodiments, the estrogen receptor antagonist is used in combination with a PD-1 inhibitor, a CDK4/6 inhibitor, or both. In some embodiments, the combination is used to treat an ER positive (ER+) cancer or a breast cancer *(e.g.,* an ER+ breast cancer).

In some embodiments, the estrogen receptor antagonist is a selective estrogen receptor degrader (SERD). SERDs are estrogen receptor antagonists which bind to the receptor and result in *e.g.,* degradation or down-regulation of the receptor (Boer K. et al., (2017) Therapeutic Advances in Medical Oncology 9(7): 465-479). ER is a hormone-activated transcription factor important for *e.g.,* the growth, development and physiology of the human reproductive system. ER is activated by, *e.g.,* the hormone estrogen (17beta estradiol). ER expression and signaling is implicated in cancers *(e.g.,* breast cancer), *e.g.,* ER positive (ER+) breast cancer. In some embodiments, the SERD is chosen from LSZ102, fulvestrant, brilanestrant, or elacestrant.

In some embodiments, the SERD comprises a compound disclosed in International Application Publication No. WO 2014/130310.

In some embodiments, the SERD comprises LSZ102. LSZ102 has the chemical name: (E)-3-(4-((2-(2-(1,1-difluoroethyl)-4-fluorophenyl)-6-hydroxybenzo[b]thiophen-3-yl)oxy)phenyl)acrylic acid. In some embodiments, the SERD comprises fulvestrant (CAS Registry Number: 129453-61-8), or a compound disclosed in International Application Publication No. WO 2001/051056. In some embodiments, the SERD comprises elacestrant (CAS Registry Number: 722533-56-4), or a compound disclosed in U.S. Patent No. 7,612,114. Elacestrant is also known as RAD1901, ER-306323

or (6R)-6-{2-[Ethyl({4-[2-(ethylamino)ethyl]phenyl}methyl)amino]-4-methoxyphenyl}-5,6,7,8-tetrahydronaphthalen-2-ol. Elacestrant is an orally bioavailable, non-steroidal combined selective estrogens receptor modulator (SERM) and a SERD. Elacestrant is also disclosed, *e.g.,* in Garner F et al., (2015) Anticancer Drugs 26(9):948-56. In some embodiments, the SERD is brilanestrant (CAS Registry Number: 1365888-06-7), or a compound disclosed in International Application Publication No. WO 2015/136017.

In some embodiments, the SERD is chosen from RU 58668, GW7604, AZD9496, bazedoxifene, pipendoxifene, arzoxifene, OP-1074, or acolbifene, *e.g.,* as disclosed in McDonell et al. (2015) Journal of Medicinal Chemistry 58(12) 4883-4887.

Other exemplary estrogen receptor antagonists are disclosed, *e.g.,* in WO 2011/156518, WO 2011/159769, WO 2012/037410, WO 2012/037411, and US 2012/0071535.

In certain embodiments, a combination described herein comprises an inhibitor of Cyclin-Dependent Kinases 4 or 6 (CDK4/6). In some embodiments, the CDK4/6 inhibitor is used in combination with a PD-1 inhibitor, an estrogen receptor (ER) antagonist, or both. In some embodiments, the combination is used to treat an ER positive (ER+) cancer or a breast cancer *(e.g.,* an ER+ breast cancer). In some embodiments, the CDK4/6 inhibitor is chosen from ribociclib, abemaciclib (Eli Lilly), or palbociclib.

In some embodiments, the CDK4/6 inhibitor comprises ribociclib (CAS Registry Number: 1211441-98-3), or a compound disclosed in U.S. Patent Nos. 8,415,355 and 8,685,980.

In some embodiments, the CDK4/6 inhibitor comprises a compound disclosed in International Application Publication No. WO 2010/020675 and U.S. Patent Nos. 8,415,355 and 8,685,980.

In some embodiments, the CDK4/6 inhibitor comprises ribociclib (CAS Registry Number: 1211441-98-3). Ribociclib is also known as LEE011, KISQALI®, or 7-cyclopentyl-N,N-dimethyl-2-((5-(piperazin-1-yl)pyridin-2-yl)amino)-7H-pyrrolo[2,3-d]pyrimidine-6-carboxamide.

In some embodiments, the CDK4/6 inhibitor comprises abemaciclib (CAS Registry Number: 1231929-97-7). Abemaciclib is also known as LY835219 or N-[5-[(4-Ethyl-1-piperazinyl)methyl]-2-pyridinyl]-5-fluoro-4-[4-fluoro-2-methyl-1-(1-methylethyl)-1H-benzimidazol-6-yl]-2-pyrimidinamine. Abemaciclib is a CDK inhibitor selective for CDK4 and CDK6 and is disclosed, *e.g.,* in Torres-Guzman R et al. (2017) Oncotarget 10.18632/oncotarget. 17778.

In some embodiments, the CDK4/6 inhibitor comprises palbociclib (CAS Registry Number: 571190-30-2). Palbociclib is also known as PD-0332991, IBRANCE® or 6-Acetyl-8-cyclopentyl-5-methyl-2-{[5-(1-piperazinyl)-2-pyridinyl]amino}pyrido[2,3-d]pyrimidin-7(8H)-one. Palbociclib inhibits CDK4 with an IC50 of 11nM, and inhibits CDK6 with an IC50 of 16nM, and is disclosed, *e.g.,* in Finn et al. (2009) Breast Cancer Research 11(5):R77.

In certain embodiments, a combination described herein comprises an inhibitor of chemokine (C-X-C motif) receptor 2 (CXCR2). In some embodiments, the CXCR2 inhibitor is chosen from 6-chloro-3-((3,4-dioxo-2-(pentan-3-ylamino)cyclobut-1-en-1-yl)amino)-2-hydroxy-N-methoxy-N-methylbenzenesulfonamide, danirixin, reparixin, or navarixin.

In some embodiments, the CSF-1/1R binding agent is chosen from an inhibitor of macrophage colony-stimulating factor (M-CSF), *e.g.,* a monoclonal antibody or Fab to M-CSF *(e.g.,* MCS110), a CSF-1R tyrosine kinase inhibitor *(e.g.,* 4-((2-(((1R,2R)-2-hydroxycyclohexyl)amino)benzo[d]thiazol-6-yl)oxy)-N-methylpicolinamide or BLZ945), a receptor tyrosine kinase inhibitor (RTK) (*e.g.,* pexidartinib), or an antibody targeting CSF-1R (e.g., emactuzumab or FPA008). In some embodiments, the CSF-1/1R inhibitor is BLZ945. In some embodiments, the CSF-1/1R binding agent is MCS110. In other embodiments, the CSF-1/1R binding agent is pexidartinib.

In certain embodiments, a combination described herein comprises a c-MET inhibitor. C-MET, a receptor tyrosine kinase overexpressed or mutated in many tumor cell types, plays key roles in tumor cell proliferation, survival, invasion, metastasis, and tumor angiogenesis. Inhibition of c-MET may induce cell death in tumor cells overexpressing c-MET protein or expressing constitutively activated c-MET protein. In some embodiments, the c-MET inhibitor is chosen from capmatinib (INC280), JNJ-3887605, AMG 337, LY2801653, MSC2156119J, crizotinib, tivantinib, or golvatinib.

In certain embodiments, a combination described herein comprises a transforming growth factor beta (also known as TGF-β TGFβ, TGFb, or TGF-beta, used interchangeably herein) inhibitor. In some embodiments, the TGF-β inhibitor is chosen from fresolimumab or XOMA 089.

In certain embodiments, a combination described herein comprises an adenosine A2a receptor (A2aR) antagonist *(e.g.,* an inhibitor of A2aR pathway, *e.g.,* an adenosine inhibitor, *e.g.,* an inhibitor of A2aR or CD-73). In some embodiments, the A2aR antagonist is used in combination with a PD-1 inhibitor, and one or more (*e.g.,* two, three, four, five, or all) of a CXCR2 inhibitor, a CSF-1/1R binding agent, LAG-3 inhibitor, a GITR agonist, a c-MET inhibitor, or an IDO inhibitor. In some embodiments, the combination is used to treat a pancreatic cancer, a colorectal cancer, a gastric cancer, or a melanoma (*e.g.,* a refractory melanoma). In some embodiments, the A2aR antagonist is chosen from PBF509 (NIR178) (Palobiofarma/Novartis), CPI444/V81444 (Corvus/Genentech), AZD4635/HTL-1071 (AstraZeneca/Heptares), Vipadenant (Redox/Juno), GBV-2034 (Globavir), AB928 (Arcus Biosciences), Theophylline, Istradefylline (Kyowa Hakko Kogyo), Tozadenant/SYN-115 (Acorda), KW-6356 (Kyowa Hakko Kogyo), ST-4206 (Leadiant Biosciences), or Preladenant/SCH 420814 (Merck/Schering). Without wishing to be bound by theory, it is believed that in some embodiments,

inhibition of A2aR leads to upregulation of IL-1b.

In certain embodiments, a combination described herein comprises an inhibitor of indoleamine 2,3-dioxygenase (IDO) and/or tryptophan 2,3-dioxygenase (TDO). In some embodiments, the IDO inhibitor is used in combination with a PD-1 inhibitor, and one or more (*e.g.,* two, three, four, or all) of a TGF-β inhibitor, an A2aR antagonist, a CSF-1/1R binding agent, a c-MET inhibitor, or a GITR agonist. In some embodiments, the combination is used to treat a pancreatic cancer, a colorectal cancer, a gastric cancer, or a melanoma (*e.g.,* a refractory melanoma). In some embodiments, the IDO inhibitor is chosen from (4E)-4-[(3-chloro-4-fluoroanilino)-nitrosomethylidene]-1,2,5-oxadiazol-3-amine (also known as epacadostat or INCB24360), indoximod (NLG8189), (1-methyl-D-tryptophan), α-cyclohexyl-5H-Imidazo[5,1-a]isoindole-5-ethanol (also known as NLG919), indoximod, BMS-986205 (formerly F001287).

In certain embodiments, a combination described herein comprises a Galectin, *e.g.,* Galectin-1 or Galectin-3, inhibitor. In some embodiments, the combination comprises a Galectin-1 inhibitor and a Galectin-3 inhibitor. In some embodiments, the combination comprises a bispecific inhibitor (*e.g.,* a bispecific antibody molecule) targeting both Galectin-1 and Galectin-3. In some embodiments, the Galectin inhibitor is used in combination with one or more therapeutic agents described herein. In some embodiments, the Galectin inhibitor is chosen from an anti-Galectin antibody molecule, GR-MD-02 (Galectin Therapeutics), Galectin-3C (Mandal Med), Anginex, or OTX-008 (OncoEthix, Merck).

In some embodiments, a combination described herein comprises a MEK inhibitor. In some embodiments, the MEK inhibitor is chosen from Trametinib, selumetinib, AS703026, BIX 02189, BIX 02188, CI-1040, PD0325901, PD98059, U0126, XL-518, G-38963, or G02443714. In some embodiments, the MEK inhibitor is Trametinib.

In one embodiment, a combination described herein includes an interleukin-1 beta (IL-1β) inhibitor. In some embodiments, the IL-1β inhibitor is chosen from canakinumab, gevokizumab, Anakinra, or Rilonacept.

In certain embodiments, a combination described herein comprises an IL-15/IL-15Ra complex. In some embodiments, the IL-15/IL-15Ra complex is chosen from NIZ985 (Novartis), ATL-803 (Altor) or CYP0150 (Cytune).

In certain embodiments, a combination described herein comprises a mouse double minute 2 homolog (MDM2) inhibitor. The human homolog of MDM2 is also known as HDM2. In some embodiments, an MDM2 inhibitor described herein is also known as a HDM2 inhibitor. In some embodiments, the MDM2 inhibitor is chosen from HDM201 or CGM097.

In an embodiment the MDM2 inhibitor comprises (S)-1-(4-chlorophenyl)-7-isopropoxy-6-methoxy-2-(4-(methyl(((1r,4S)-4-(4-methyl-3-oxopiperazin-1-yl)cyclohexyl)methyl)amino)phenyl)-1,2-dihydroisoquinolin-3(4H)-one (also known as CGM097) or a compound disclosed in PCT Publication No. WO 2011/076786 to treat a disorder, *e.g.,* a disorder described herein). In one embodiment, a therapeutic agent disclosed herein is used in combination with CGM097.

In some embodiments, a combination described herein comprises a hypomethylating agent (HMA). In one some embodiments, the HMA is chosen from decitabine or azacitidine.

In certain embodiments, a combination described herein comprises an inhibitor acting on pro-survival proteins of the Bcl2 family. In certain embodiments, a combination described herein comprises a Bcl-2 inhibitor. In some embodiments, the Bcl-2 inhibitor is venetoclax:

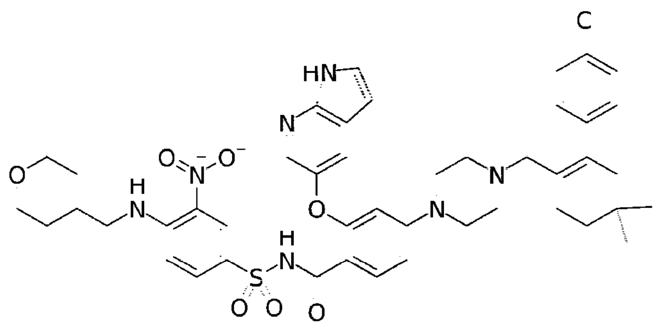

( venetoclax).

In one embodiment, the Bcl-2 inhibitor is selected from the compounds described in WO 2013/110890 and WO 2015/011400. In some embodiments, the Bcl-2 inhibitor comprises navitoclax (ABT-263), ABT-737, BP1002, SPC2996, APG-1252, obatoclax mesylate (GX15-070MS), PNT2258, Zn-d5, BGB-11417, or oblimersen (G3139). In some embodiments, the Bcl-2 inhibitor is (S)-5-(5-chloro-2-(3-(morpholinomethyl)-1,2,3,4-tetrahydroisoquinoline-2-carbonyl)phenyl)-N-(5-cyano-1,2-dimethyl-1H-pyrrol-3-yl)-N-(4-hydroxyphenyl)-1,2-dimethyl-1H-pyrrole-3-carboxamide), compound A1:

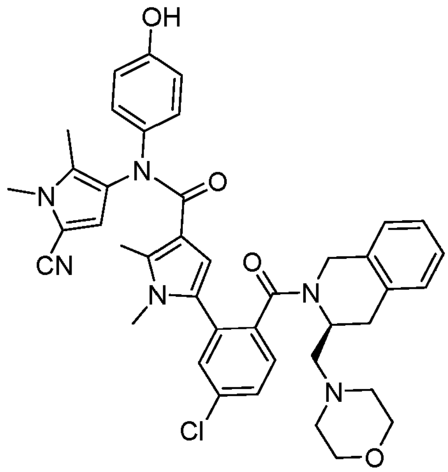
(compound A1).

In some embodiments, the Bcl-2 inhibitor is N-(4-hydroxyphenyl)-3-[6-[(3S)-3-(morpholinomethyl)-3,4-dihydro-1H-isoquinoline-2-carbonyl]-1,3-benzodioxol-5-yl]-N-phenyl-5,6,7,8-tetrahydroindolizine-1-carboxamide, compound A2:

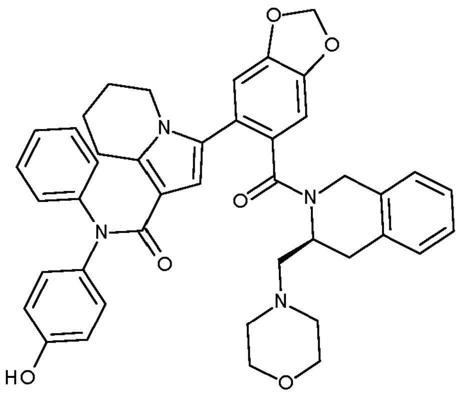
(compound A2).

In one embodiment, the antibody-drug conjugates or combinations disclosed herein are suitable for the treatment of cancer *in vivo.* For example, the combination can be used to inhibit the growth of cancerous tumors. The combination can also be used in combination with one or more of: a standard of care treatment (*e.g.,* for cancers or infectious disorders), a vaccine (*e.g.,* a therapeutic cancer vaccine), a cell therapy, a radiation therapy, surgery, or any other therapeutic agent or modality, to treat a disorder herein. For example, to achieve antigen-specific enhancement of immunity, the combination can be administered together with an antigen of interest. A combination disclosed herein can be administered in either order or simultaneously.

**ADDITIONAL EMBODIMENTS**

The disclosure provides the following additional embodiments for linker-drug groups, antibody-drug conjugates, linker groups, and methods of conjugation.

<u>Linker-Drug Group</u>

In some embodiments, the Linker-Drug group of the invention may be a compound having the structure of Formula (A'), or a pharmaceutically acceptable salt thereof:

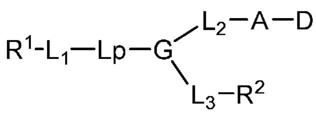
Formula (A')

wherein:

$R^1$ is a reactive group;
$L_1$ is a bridging spacer;
Lp is a bivalent peptide spacer;
G-$L_2$-A is a self-immolative spacer;
$R^2$ is a hydrophilic moiety;
$L_2$ is a bond, a methylene, a neopentylene or a $C_2$-$C_3$alkenylene;
A is a bond, -OC(=O)-*,

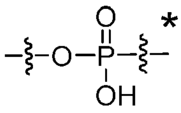 , 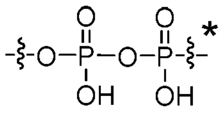 , 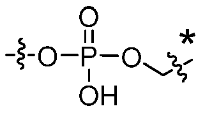 ,

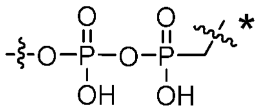 ,

-OC(=O)N($CH_3$)$CH_2CH_2$N($CH_3$)C(=O)-* or - OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety; and
D is a Drug moiety that is capable of inhibiting the activity of the MCI-1 protein when, e.g., released from the Antibody Drug Conjugates or immunoconjugates disclosed herein.

Certain aspects and examples of the Linker-Drug group of the invention are provided in the following listing of enumerated embodiments. It will be recognized that features specified in each embodiment may be combined with other specified features to provide further embodiments of the present invention.

**Embodiment 1.** The compound of Formula (A'), or pharmaceutically acceptable salt thereof, wherein:

$R^1$ is a reactive group;
$L_1$ is a bridging spacer;
Lp is a bivalent peptide spacer comprising two to four amino acid residues;
G-$L_2$-A is a self-immolative spacer;
$R^2$ is a hydrophilic moiety;
$L_2$ is a bond, a methylene, a neopentylene or a $C_2$-$C_3$alkenylene;
A is a bond, -OC(=O)-*,

, 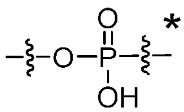 , 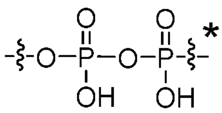 , 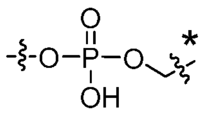 ,

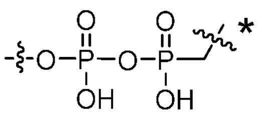 ,

-OC(=O)N($CH_3$)$CH_2CH_2$N($CH_3$)C(=O)-* or - OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety; and
D is a Drug moiety as defined herein, e.g., a MCI-1 inhibitor.

**Embodiment 2.** The compound of Formula (A'), or pharmaceutically acceptable salt thereof, wherein:

$R^1$ is a reactive group;
$L_1$ is a bridging spacer;
Lp is a bivalent peptide spacer comprising two to four amino acid residues;
the

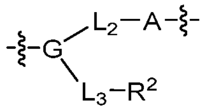

group is selected from:

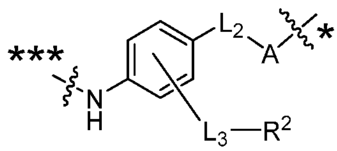

,

wherein the * of

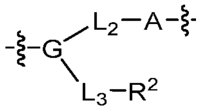

indicates the point of attachment to D (e.g., to an N or a O of the Drug moiety), the *** of

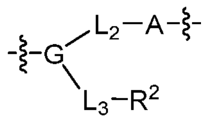

indicates the point of attachment to Lp;
$R^2$ is a hydrophilic moiety;
$L_2$ is a bond, a methylene, a neopentylene or a $C_2$-$C_3$alkenylene;
A is a bond, -OC(=O)-*

, 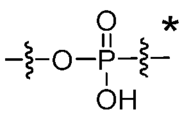 , 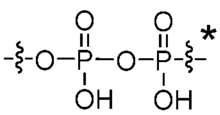 , 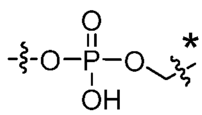 ,

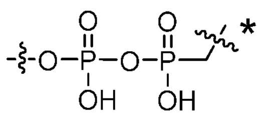 ,

-OC(=O)N($CH_3$)$CH_2CH_2$N($CH_3$)C(=O)-* or - OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety; and
D is a Drug moiety as defined herein, e.g., a MCI-1 inhibitor.

**Embodiment 3.** The compound of Formula (A'), or pharmaceutically acceptable salt thereof, having the structure of Formula (B'):
wherein:

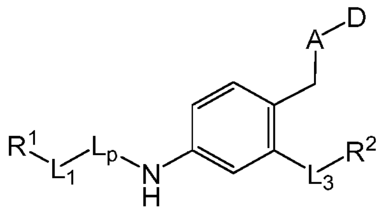

Formula (B')

$R^1$ is a reactive group;
$L_1$ is a bridging spacer;
Lp is a bivalent peptide spacer comprising two to four amino acid residues;
$R^2$ is a hydrophilic moiety;
A is a bond, -OC(=O)-*,

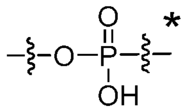 , 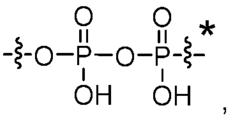 , 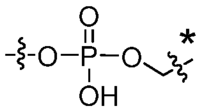 ,

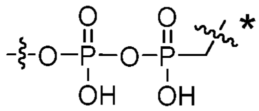 ,

-OC(=O)N($CH_3$)$CH_2CH_2$N($CH_3$)C(=O)-* or - OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety; and
D is a Drug moiety as defined herein and comprising an N or an O, wherein D is connected to A via a direct bond from A to the N or the O of the Drug moiety.

**Embodiment 4.** The compound of Formula (A') or of any one of Embodiments 1 to 3, or pharmaceutically acceptable salt thereof, wherein:

$R^1$ is

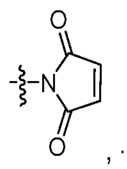 , .

-$ONH_2$, -$NH_2$,

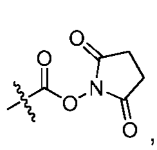 , 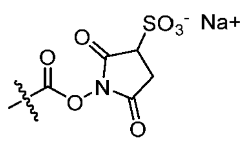 , 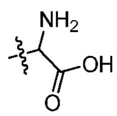 ,

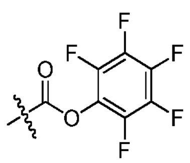 , 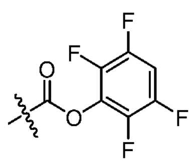 , 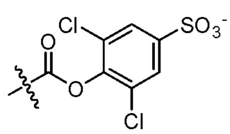 ,

$-N_3$,

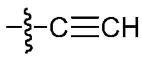

, -SH, $-SR^3$, $-SSR^4$, $-S(=O)_2(CH=CH_2)$, $-(CH_2)_2S(=O)_2(CH=CH_2)$, $-NHS(=O)_2(CH=CH_2)$, - $NHC(=O)CH_2Br$, $-NHC(=O)CH_2I$,

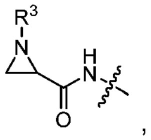

,

-C(O)$NHNH_2$,

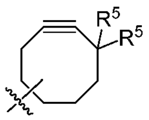

,

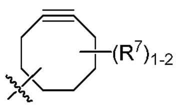

, 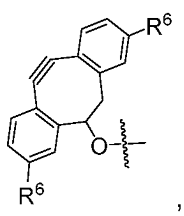

, 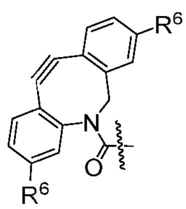

, 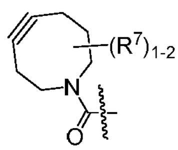

, 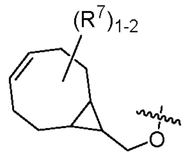

,

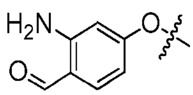

, 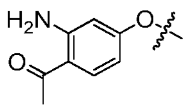

, 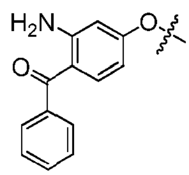

, 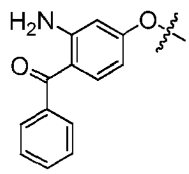

,

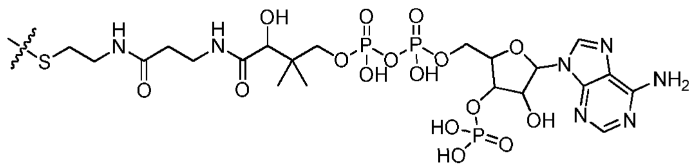

,

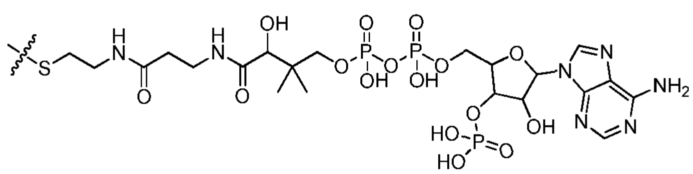

,

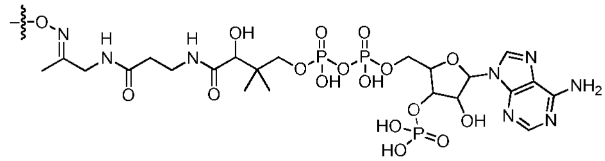

,

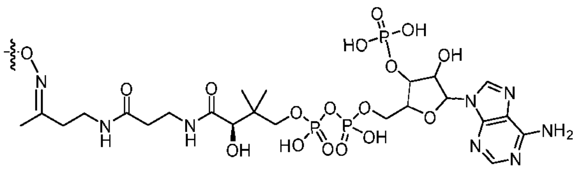

,

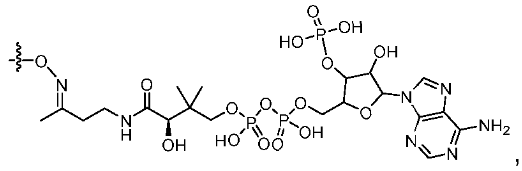

,

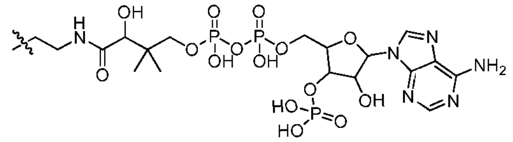

,

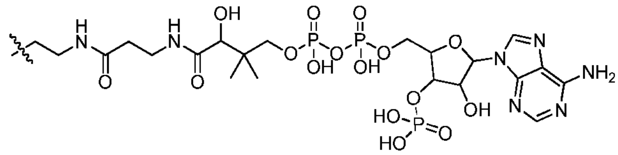

,

or

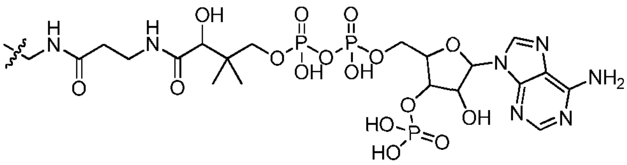

;

$L_1$ is $*-C(=O)(CH_2)_mO(CH_2)_m-**$;

$*-C(=O)((CH_2)_mO)_t(CH_2)_n-**$;

$*-C(=O)(CH_2)_m-**$;

$*-C(=O)NH((CH_2)_mO)_t(CH_2)_n-**$;

$*-C(=O)O(CH_2)_mSSC(R^3)_2(CH_2)_mC(=O)NR^3(CH_2)_mNR^3C(=O)(CH_2)_m-**$;

$*-C(=O)O(CH_2)_mC(=O)NH(CH_2)_m-**$;

$*-C(=O)(CH_2)_mNH(CH_2)_m-**$; $*-C(=O)(CH_2)_mNH(CH_2)_nC(=O)-**$;

$*-C(=O)(CH_2)_mX_1(CH_2)_m-**$; $*-C(=O)((CH_2)_mO)_t(CH_2)_nX_1(CH_2)_n-**$;

$*-C(=O)(CH_2)_mNHC(=O)(CH_2)_n-**$;

$*-C(=O)((CH_2)_mO)_t(CH_2)_nNHC(=O)(CH_2)_n-**$;

$*-C(=O)(CH_2)_mNHC(=O)(CH_2)_nX_1(CH_2)_n-**$;

$*-C(=O)((CH_2)_mO)_t(CH_2)_nNHC(=O)(CH_2)_nX_1(CH_2)_n-**$;

$-C(=O)((CH_2)_mO)_t(CH_2)_nC(=O)NH(CH_2)_m$-**;

*-$C(=O)(CH_2)_mC(R^3)_2$-**;

or

*-$C(=O)(CH_2)_mC(=O)NH(CH_2)_m$-**,

where the * of $L_1$ indicates the point of attachment to Lp, and the ** of $L_1$ indicates the point of attachment to $R^1$;
$R^2$ is a hydrophilic moiety selected from polyethylene glycol, polyalkylene glycol, a sugar, an oligosaccharide, a polypeptide or $C_2$-$C_6$alkyl substituted with 1 to
3

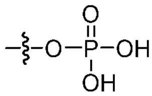

groups;
each $R^3$ is independently selected from H and $C_1$-$C_6$alkyl;
$R^4$ is 2-pyridyl or 4-pyridyl;
each $R^5$ is independently selected from H, $C_1$-$C_6$alkyl, F, Cl, and -OH;
each $R^6$ is independently selected from H, $C_1$-$C_6$alkyl, F, Cl, $-NH_2$, $-OCH_3$, - $OCH_2CH_3$, $-N(CH_3)_2$, -CN, $-NO_2$ and -OH;
each $R^7$ is independently selected from H, $C_{1-6}$alkyl, fluoro, benzyloxy substituted with -C(=O)OH, benzyl substituted with -C(=O)OH, $C_{1-4}$alkoxy substituted with
-C(=O)OH and $C_{1-4}$alkyl substituted with -C(=O)OH;
$X_1$ is

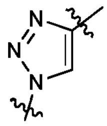 , 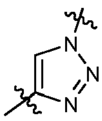 , 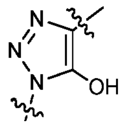 or 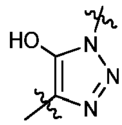 ;

each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer comprising an amino acid residue selected from glycine, valine, citrulline, lysine, isoleucine, phenylalanine, methionine, asparagine, proline, alanine, leucine, tryptophan, and tyrosine;
A is a bond, -OC(=O)-*,

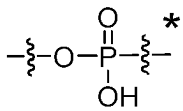 , 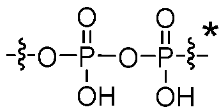 , 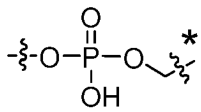 ,

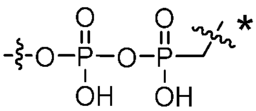 ,

$-OC(=O)N(CH_3)CH_2CH_2N(CH_3)C(=O)$-* or - $OC(=O)N(CH_3)C(R^a)_2C(R^a)_2N(CH_3)C(=O)$-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety having the structure

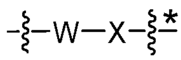,

where

W is $-CH_2O-**$, $-CH_2N(R^b)C(=O)O-**$, $-NHC(=O)C(R^b)_2NHC(=O)O-**$, $-NHC(=O)C(R^b)_2NH-**$, $-NHC(=O)C(R^b)_2NHC(=O)-**$, $-CH_2N(X-R\ ^2)C(=O)O-**$, $-C(=O)N(X-R^2)-**$, $-CH_2N(X-R^2)C(=O)-**$, $-C(=O)NR^b-**$, $-C(=O)NH-**$, $-CH_2NR^bC(=O)-**$, $-CH_2NR^bC(=O)NH-**$, $-CH_2NR^bC(=O)NR^b-**$, -NHC(=O)-**, -NHC(=O)O-**, - NHC(=O)NH-**, -OC(=O)NH-**, $-S(O)_2NH-**$, $-NHS(O)_2-**$, -C(=O)-, -C(=O)O-** or -NH-, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$; and
the * of $L_3$ indicates the point of attachment to $R^2$;

and
D is a Drug moiety as defined herein and comprising an N or an O, wherein D is connected to A via a direct bond from A to the N or the O of the Drug moiety.

**Embodiment 5.** The compound of Formula (A') or of any one of Embodiments 1 to 4, or pharmaceutically acceptable salt thereof, wherein:

$R^1$ is

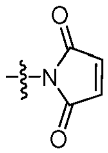, $-ONH_2$, 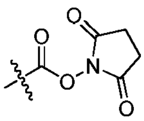, 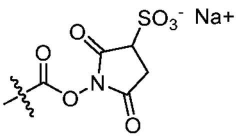, 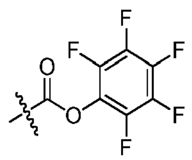

or

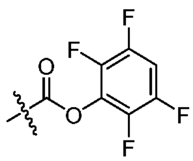;

$L_1$ is $*-C(=O)(CH_2)_mO(CH_2)_m-**$; $*-C(=O)((CH_2)_mO)t(CH_2)_n-**$; $*-C(=O)(CH_2)_m-**$; or $*-C(=0)NH((CH_2)_mO),(CH_2)_n-$, where the * of $L_1$ indicates the point of attachment to Lp, and the ** of $L_1$ indicates the point of attachment to $R^1$;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

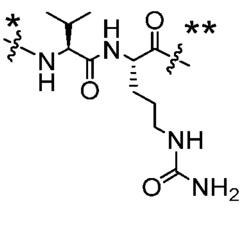 (ValCit),

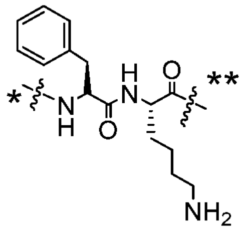 (PheLys), 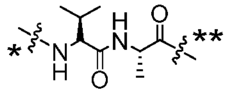 (ValAla), 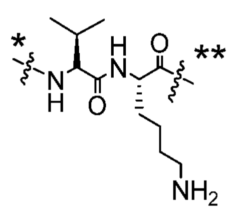

(ValLys) and

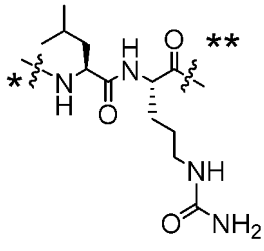 (LeuCit),

where the * of Lp indicates the attachment point to $L_1$ and the ** of Lp indicates the attachment point to the - NH- group of G;

$L_3$ is a spacer moiety having the structure

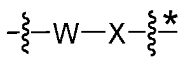 ,

where

W is $-CH_2O$-**, $-CH_2N(R^b)C(=O)O$-**, $-NHC(=O)CH_2NHC(=O)O$-**, $-NHC(=O)CH_2NH$-**, $-NHC(=O)CH_2NHC(=O)$-**, $-CH_2N(X-R^2)C(=O)O$-**, $-C(=O)N(X-R^2)$-**, $-CH_2N(X-R^2)C(=O)$-**, $-C(=O)NR^b$-**, -C(=O)NH-**, $-CH_2NR^bC(=O)$-**, - $CH_2NR^bC(=O)NH$-**, $-CH_2NR^bC(=O)NR^b$-**, -NHC(=O)-**, - NHC(=O)O-**, -NHC(=O)NH-**, -OC(=O)NH-**, $-S(O)_2NH$-**, - $NHS(O)_2$-**, -C(=O)-, -C(=O)O-** or -NH-, wherein each $R^b$ is independently selected from H, $C_1$-$C_6$alkyl or $C_3$-$C_8$cycloalkyl and wherein the ** of W indicates the point of attachment to X;

X is a bond, triazolyl or ***-CH2-triazolyl-*, wherein the *** of X indicates the point of attachment to W and the * of X indicates the point of attachment to $R^2$;

and

the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

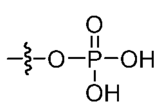

groups;

A is a bond, -OC(=O)-*,

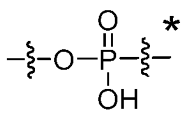 , 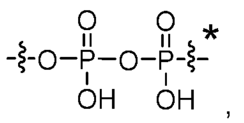 , 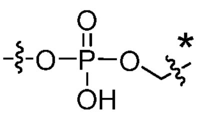 ,

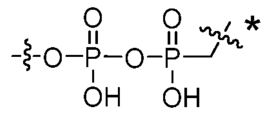,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D; and

D is a Drug moiety as defined herein and comprising an N or an O, wherein D is connected to A via a direct bond from A to the N or the O of the Drug moiety.

Embodiment 6. The compound of Formula (A') or of any one of Embodiments 1 to 5, or pharmaceutically acceptable salt thereof, wherein:

$R^1$ is

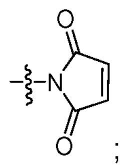;

$L_1$ is $*-C(=O)(CH_2)_mO(CH_2)_m-**$; $*-C(=O)((CH_2)_mO)t(CH_2)_n-**$; $*-C(=O)(CH_2)_m-**$; or $*-C(=O)NH((CH_2)_mO)_t(CH_2)_n-$, where the * of $L_1$ indicates the point of attachment to Lp, and the ** of $L_1$ indicates the point of attachment to $R^1$;

each m is independently selected from 1, 2, 3, 4, 5, 6, 7, 8, 9 and 10;

each n is independently selected from 1, 2, 3, 4, 5, 6, 7, 8, 9 and 10;

each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;

Lp is a bivalent peptide spacer selected from

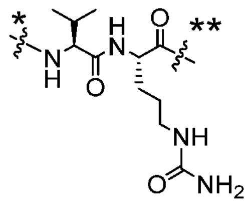 (ValCit),

where the * of Lp indicates the attachment point to $L_1$ and the ** of Lp indicates the attachment point to the -NH- group of G;

$L_3$ is a spacer moiety having the structure

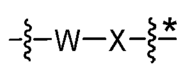,

where

W is $-CH_2O-**$, $-CH_2N(R^b)C(=O)O-**$, $-NHC(=O)CH_2NHC(=O)O-**$, $-CH_2N(X-R^2)C(=O)O-**$, $-C(=O)N(X-R^2)-**$, $-CH_2N(X-R^2)C(=O)-**$, $-C(=O)NR^b-**$, $-C(=O)NH-**$, $-CH_2NR^bC(=O)-**$, $-CH_2NR^bC(=O)NH-**$, $-CH_2NR^bC(=O)NR^b-**$, $-NHC(=O)-**$, $-NHC(=O)O-**$, $-NHC(=O)NH-**$, $-OC(=O)NH-**$, $-S(O)_2NH-**$, $-NHS(O)_2-**$, $-C(=O)-$, $-C(=O)O-**$ or

-NH-, wherein each $R^b$ is independently selected from H, $C_1$-$C_6$alkyl or $C_3$-$C_8$cycloalkyl and wherein the ** of W indicates the point of attachment to X;

X is a bond, triazolyl or $***-CH_2$-triazolyl-*, wherein the *** of X indicates the point of attachment to W and the * of X indicates the point of attachment to $R^2$;

and

the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

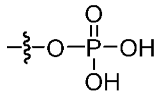

groups;

A is a bond, -OC(=O)-*,

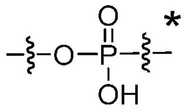 , 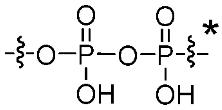 , 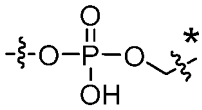 ,

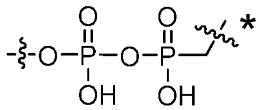 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D;

and

D is a Drug moiety as defined herein and comprising an N or an O, wherein D is connected to A via a direct bond from A to the N or the O of the Drug moiety.

Embodiment 7. The compound of Formula (A') or of any one of Embodiments 1 to 6, or pharmaceutically acceptable salt thereof, wherein:

$R^1$ is

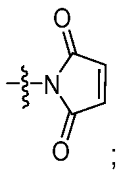 ;

$L_1$ is $*-C(=O)(CH_2)_mO(CH_2)_m-**$; $*-C(=O)((CH_2)_mO)_t(CH_2)_n-**$; $*-C(=O)(CH_2)_m-**$; or $*-C(=O)NH((CH_2)_mO),(CH_2)_n-$, where the * of $L_1$ indicates the point of attachment to Lp and the ** of $L_1$ indicates the point of attachment to $R^1$;

each m is independently selected from 1, 2, 3, 4, 5, 6, 7, 8, 9 and 10;

each n is independently selected from 1, 2, 3, 4, 5, 6, 7, 8, 9 and 10;

each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;

Lp is a bivalent peptide spacer selected from

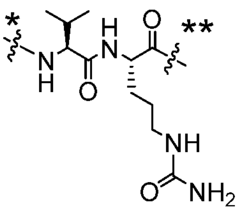 (ValCit),

where the * of Lp indicates the attachment point to $L_1$ and the ** of Lp indicates the attachment point to the -NH- group of G;

$L_3$ is a spacer moiety having the structure

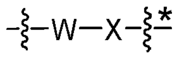

,

where

W is $-CH_2O-**$, $-CH_2N(R^b)C(=O)O-**$, $-NHC(=O)CH_2NHC(=O)O-**$, $-CH_2N(X-R^2)C(=O)O-**$, $-C(=O)N(X-R^2)-**$, $-C(=O)NR^b-**$, - $C(=O)NH-**$, $-CH_2NR^bC(=O)-**$, $-CH_2NR^bC(=O)NH-**$, - $CH_2NR^bC(=O)NR^b-**$, $-NHC(=O)-**$, $-NHC(=O)O-**$, or $-NHC(=O)NH-**$, wherein each $R^b$ is independently selected from H, $C_1$-$C_6$alkyl or $C_3$-$C_8$cycloalkyl and wherein the ** of W indicates the point of attachment to X;

X is a bond, triazolyl or ***-CH2-triazolyl-*, wherein the *** of X indicates the point of attachment to W and the * of X indicates the point of attachment to $R^2$;

and

the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

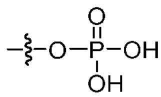

groups;

A is a bond or -OC(=O)*, in which * indicates the attachment point to D;

and

D is a Drug moiety as defined herein and comprising an N or an O, wherein D is connected to A via a direct bond from A to the N or the O of the Drug moiety.

**Embodiment 8.** The compound of Formula (A') or of any one of Embodiments 1 to 7, or pharmaceutically acceptable salt thereof, wherein:

$R^1$ is

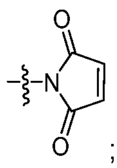

;

$L_1$ is $*-C(=O)(CH_2)_mO(CH_2)_m-**$; $*-C(=O)((CH_2)_mO)_t(CH_2)_n-**$; $*-C(=O)(CH_2)_m-**$; or $*-C(=O)NH((CH_2)_mO),(CH_2)_n-$, where the * of $L_1$ indicates the point of attachment to Lp and the ** of $L_1$ indicates the point of attachment to $R^1$;

each m is independently selected from 1, 2, 3, 4, 5, 6, 7, 8, 9 and 10;

each n is independently selected from 1, 2, 3, 4, 5, 6, 7, 8, 9 and 10;

each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;

Lp is a bivalent peptide spacer selected from

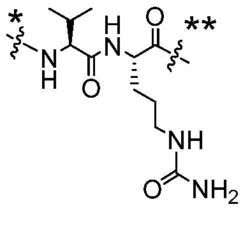

(ValCit),

where the * of Lp indicates the attachment point to $L_1$ and the ** of Lp indicates the attachment point to the -NH- group of G;

$L_3$ is a spacer moiety having the structure

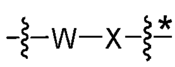

,

where

W is $-CH_2O-$**, $-CH_2N(R^b)C(=O)O-$**, $-NHC(=O)CH_2NHC(=O)O-$**, $-CH_2N(X-R^2)C(=O)O-$**, or $-C(=O)N(X-R^2)-$**, wherein each $R^b$ is independently selected from H, $C_1$-$C_6$alkyl or $C_3$-$C_8$cycloalkyl and wherein the ** of W indicates the point of attachment to X;

X is ***-$CH_2$-triazolyl-*, wherein the *** of X indicates the point of attachment to W and the * of X indicates the point of attachment to $R^2$;

and

the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

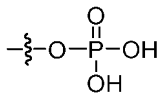

groups;

A is a bond or -OC(=O)* in which * indicates the attachment point to D;

and

D is a Drug moiety as defined herein and comprising an N or an O, wherein D is connected to A via a direct bond from A to the N or the O of the Drug moiety.

**Embodiment 9.** The compound of Formula (A') or of any one of Embodiments 1 to 8, or pharmaceutically acceptable salt thereof, wherein $R^1$ is a reactive group selected from Table 2.

**Embodiment 10.** The compound of Formula (A') or of any one of Embodiments 1 to 9, or pharmaceutically acceptable salt thereof, wherein:

$R^1$ is

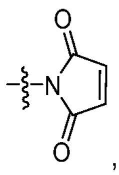

,

$-ONH_2$, $-NH_2$,

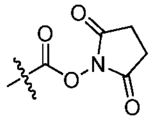 , 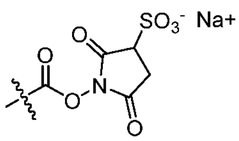 , 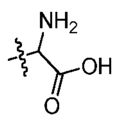 , 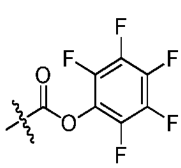 ,

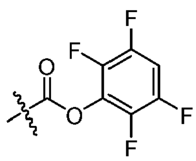 , 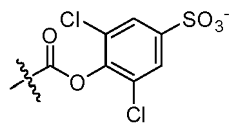 , -

$-N_3$,

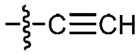
,

-SH, $-SR^3$, $-SSR^4$, $-S(=O)_2(CH=CH_2)$, $-(CH_2)_2S(=O)_2(CH=CH_2)$, $-NHS(=O)_2(CH=CH_2)$, $-NHC(=O)CH_2Br$, $-NHC(=O)CH_2I$,

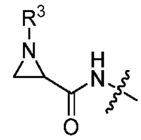

, $-C(O)NHNH_2$,

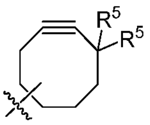
, 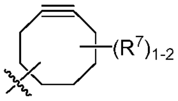
, 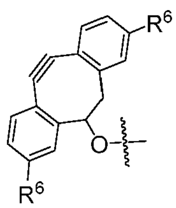
, 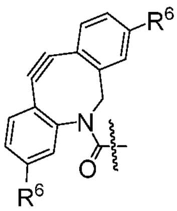
,

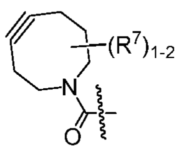
, 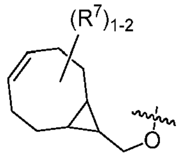
, 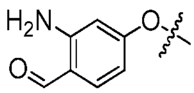
, 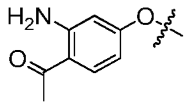
, 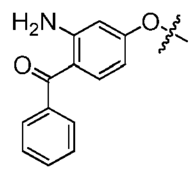
,

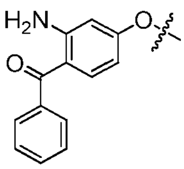
, 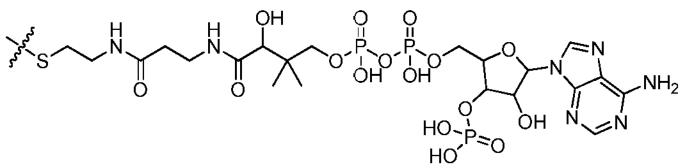
,

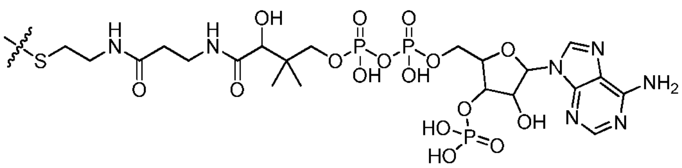
,

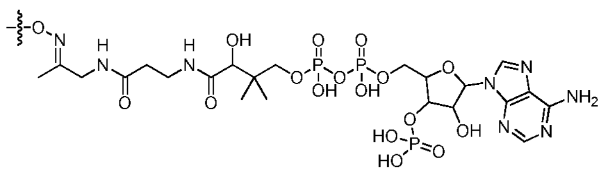
,

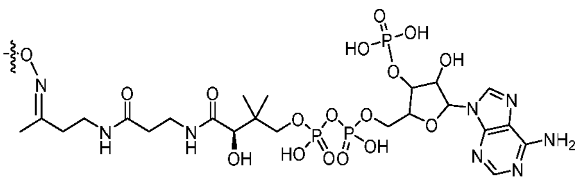
, 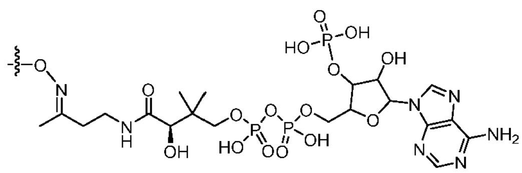
,

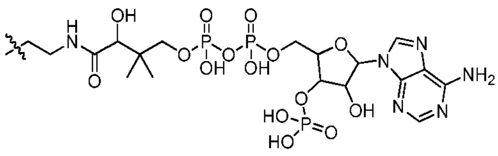,
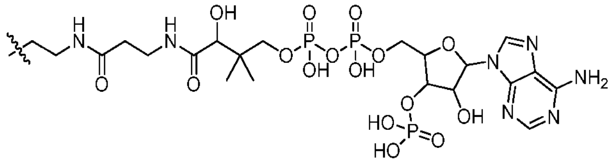,

or

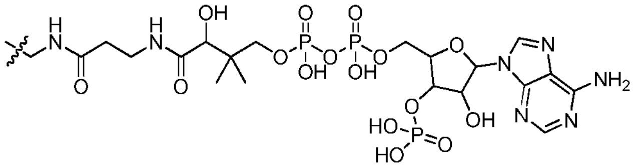.

**Embodiment 11.** The compound of Formula (A') or of any one of Embodiments 1 to 9, or pharmaceutically acceptable salt thereof, wherein:
$R^1$ is

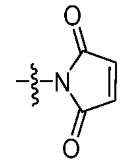,

$-ONH_2$, $-NH_2$,

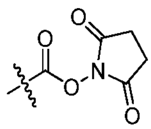, 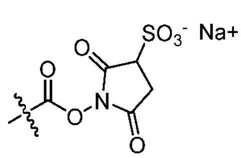, 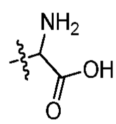, 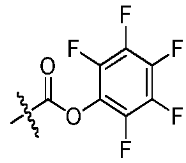,

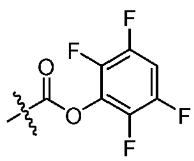, 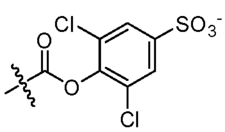,

$-N_3$,

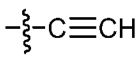,

-SH, $-SR^3$, $-SSR^4$, $-S(=O)_2(CH=CH_2)$, $-(CH_2)_2S(=O)_2(CH=CH_2)$, $-NHS(=O)_2(CH=CH_2)$, $-NHC(=O)CH_2Br$, $-NHC(=O)CH_2I$,

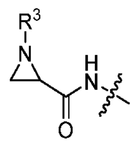,

$-C(O)NHNH_2$,

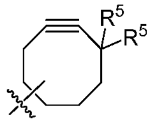, 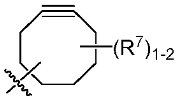, 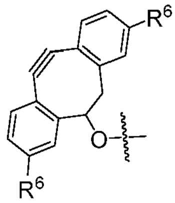, 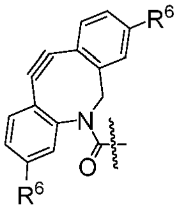,

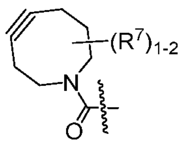

or

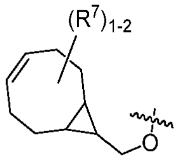.

**Embodiment 12.** The compound of Formula (A') or of any one of Embodiments 1 to 9, or pharmaceutically acceptable salt thereof, wherein:

$R^1$ is

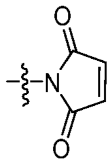,

$-ONH_2$,

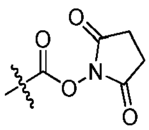, 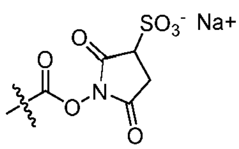, 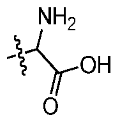, 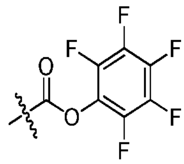,

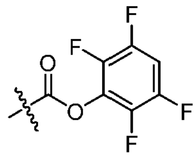, 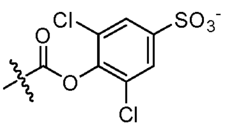, 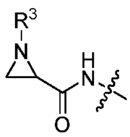, 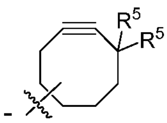, 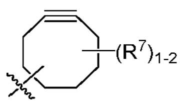,

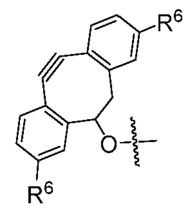, 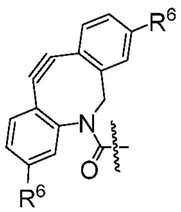, 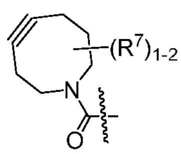 or 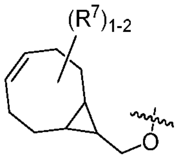.

**Embodiment 13.** The compound of Formula (A') or of any one of Embodiments 1 to 9, or pharmaceutically acceptable salt thereof, wherein:

$R^1$ is

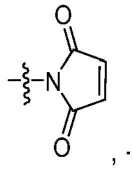

,

-$ONH_2$,

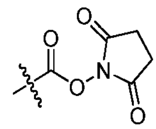

,

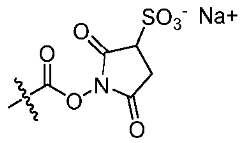

,

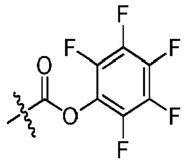

or

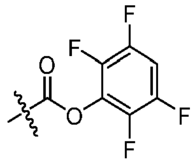

.

**Embodiment 14.** The compound of Formula (A') or of any one of Embodiments 1 to 9, or pharmaceutically acceptable salt thereof, wherein $R^1$ is

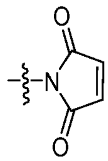

.

**Embodiment 15.** The compound of Formula (A') or of any one of Embodiments 1 to 9, or pharmaceutically acceptable salt thereof, wherein $R^1$ is -$ONH_2$.

**Embodiment 16.** The compound of Formula (A') or of any one of Embodiments 1 to 9, or pharmaceutically acceptable salt thereof, wherein: $R^1$ is

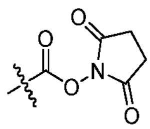

or

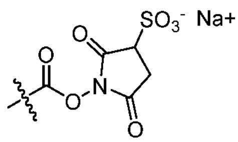

.

**Embodiment 17.** The compound of Formula (A') or of any one of Embodiments 1 to 9, or pharmaceutically acceptable salt thereof, wherein:
$R^1$ is

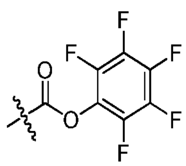

or

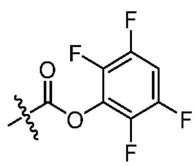

.

**Embodiment 18.** The compound of Formula (A') or of any one of Embodiments 1 to 9, or pharmaceutically acceptable salt thereof, having the structure:

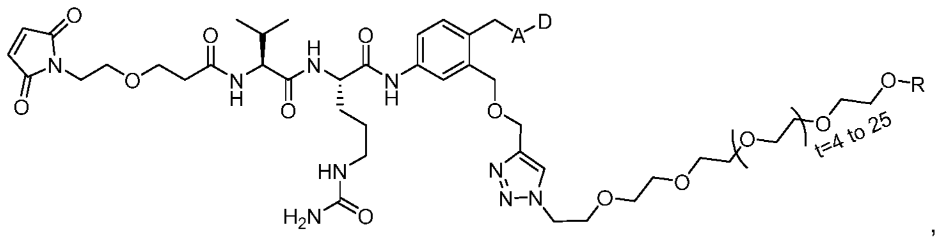

where
R is H, $-CH_3$ or $-CH_2CH_2C(=O)OH$.

**Embodiment 19.** The compound of Formula (A') or of any one of Embodiments 1 to 9, or pharmaceutically acceptable salt thereof, having the structure:

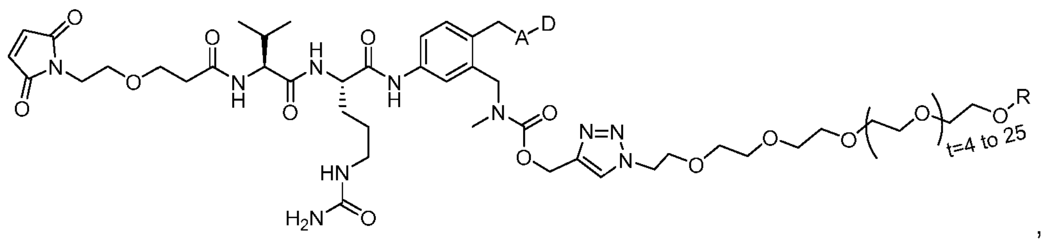

where
R is H, $-CH_3$ or $-CH_2CH_2C(=O)OH$.

**Embodiment 20.** The compound of Formula (A') or of any one of Embodiments 1 to 9, or pharmaceutically acceptable salt thereof, having the structure:

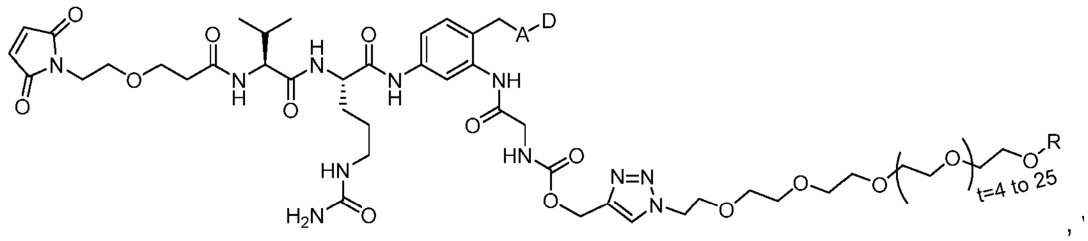

where
R is H, $-CH_3$ or $-CH_2CH_2C(=O)OH$.

**Embodiment 21.** The compound of Formula (A') or of any one of Embodiments 1 to 9, or pharmaceutically acceptable salt thereof, having the structure:

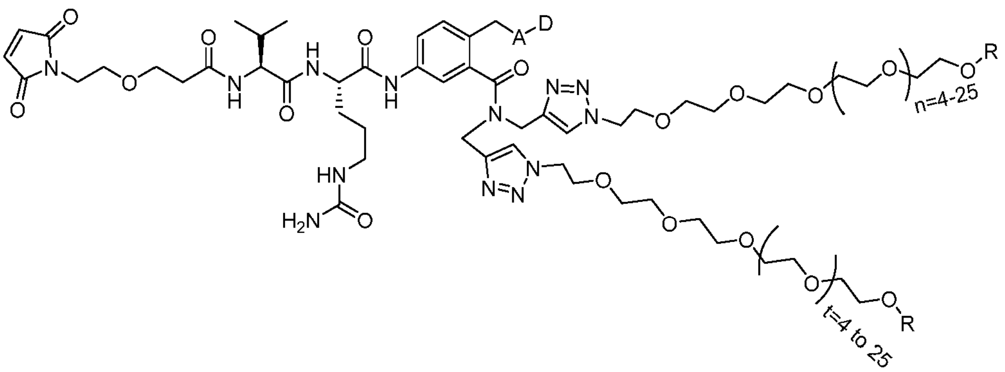

where
each R is independently selected from H, $-CH_3$ or $-CH_2CH_2C(=O)OH$.

**Embodiment 22.** The compound of Formula (A') or of any one of Embodiments 1 to 9, or pharmaceutically acceptable salt thereof, having the structure:

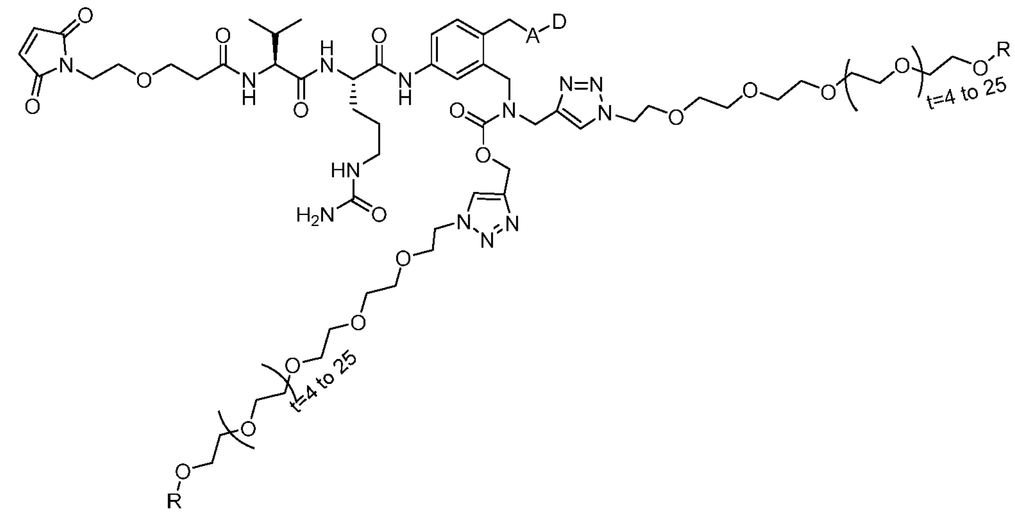

,

where

each R is independently selected from H, $-CH_3$ or $-CH_2CH_2C(=O)OH$.

**Embodiment 23.** The compound of Formula (A') or of any one of Embodiments 1 to 9 or pharmaceutically acceptable salt thereof, having the structure:

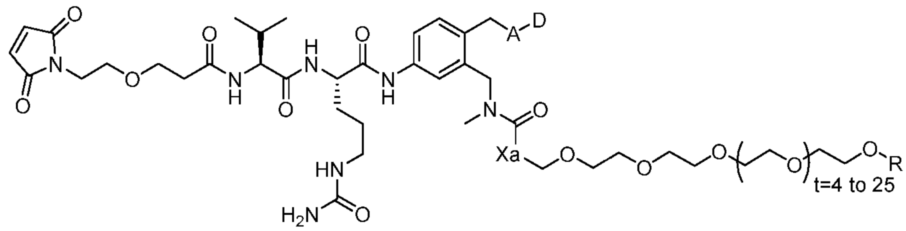

,

where

Xa is $-CH_2-$, $-OCH_2-$, $-NHCH_2-$ or $-NRCH_2-$ and each R independently is H, $-CH_3$ or - $CH_2CH_2C(=O)OH$.

**Embodiment 24.** The compound of Formula (A') or of any one of Embodiments 1 to 9, or pharmaceutically acceptable salt thereof, having the structure:

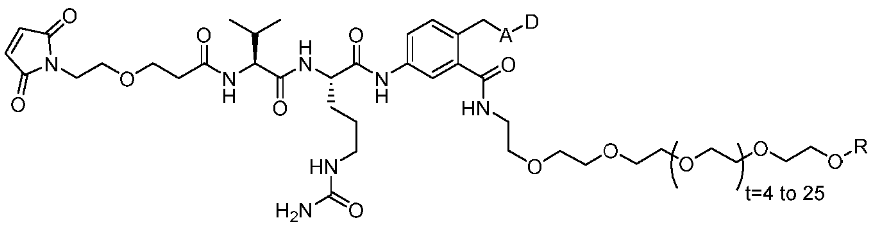

,

where

R is H, $-CH_3$ or $-CH_2CH_2C(=O)OH$.

**Embodiment 25.** The compound of Formula (A') or of any one of Embodiments 1 to 9, or pharmaceutically acceptable salt thereof, having the structure:

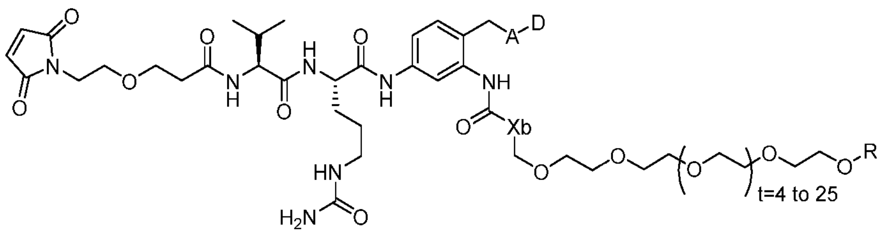

,

where Xb is $-CH_2-$, $-OCH_2-$, $-NHCH_2-$ or $-NRCH_2-$ and each R independently is H, $-CH_3$ or $-CH_2CH_2C(=O)OH$.

**Embodiment 26.** The compound of Formula (A') or of any one of Embodiments 1 to 9, or pharmaceutically acceptable salt thereof, having the structure:

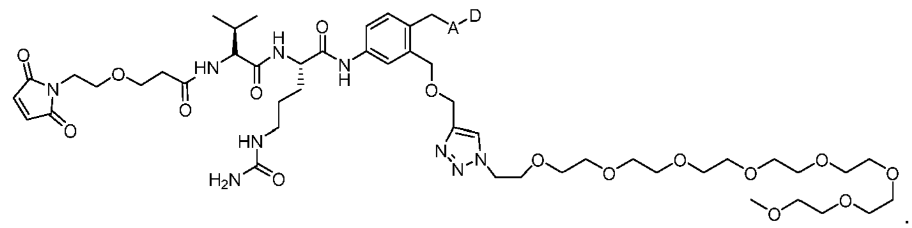

**Embodiment 27.** The compound of Formula (A') or of any one of Embodiments 1 to 9, or pharmaceutically acceptable salt thereof, having the structure:

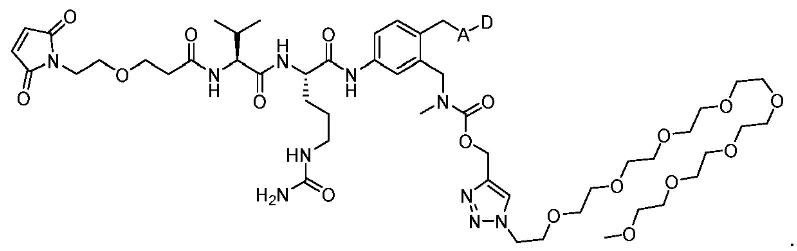

**Embodiment 28.** The compound of Formula (A') or of any one of Embodiments 1 to 9, or pharmaceutically acceptable salt thereof, having the structure:

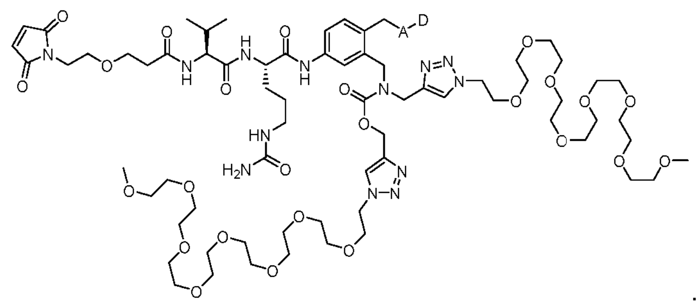

**Embodiment 29.** The compound of Formula (A') or of any one of Embodiments 1 to 9, or pharmaceutically acceptable salt thereof, having the structure:

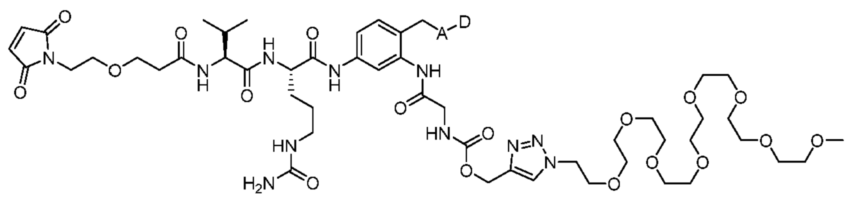

**Embodiment 30.** The compound of Formula (A') or of any one of Embodiments 1 to 9, or pharmaceutically acceptable salt thereof, having the structure:

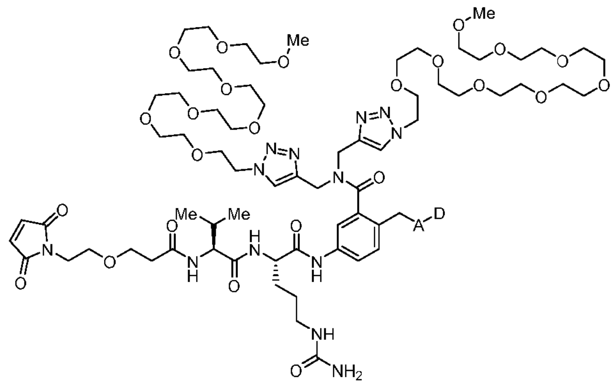

**Embodiment 31.** The compound of Formula (A') or of any one of Embodiments 1 to 9, or pharmaceutically acceptable salt thereof, having the structure of a compound in Table A.

**Embodiment 32.** A linker of the Linker-Drug group of Formula (A') having the structure of Formula (C'),

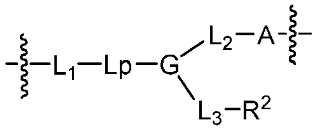

Formula (C')

wherein

$L_1$ is a bridging spacer;
Lp is a bivalent peptide spacer;
G-$L_2$-A is a self-immolative spacer;
$R^2$ is a hydrophilic moiety;
$L_2$ is a bond, a methylene, a neopentylene or a $C_2$-$C_3$alkenylene;
A is a bond, -OC(=O)-*,

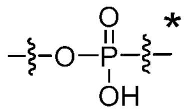,
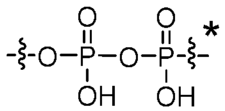,
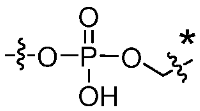,

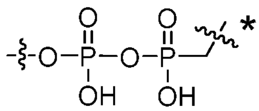,

-OC(=O)N($CH_3$)$CH_2CH_2$N($CH_3$)C(=O)-* or - OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D,
and
$L_3$ is a spacer moiety.

**Embodiment 33.** The linker of Embodiment 32, wherein:

$L_1$ is a bridging spacer;
Lp is a bivalent peptide spacer comprising two to four amino acid residues;
G-$L_2$-A is a self-immolative spacer;
$R^2$ is a hydrophilic moiety;
$L_2$ is a bond, a methylene, a neopentylene or a $C_2$-$C_3$alkenylene;
A is a bond, -OC(=O)-*,

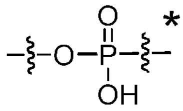, 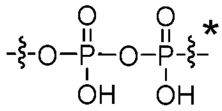, 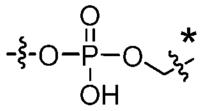,

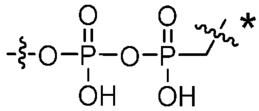,

-OC(=O)N($CH_3$)$CH_2CH_2$N($CH_3$)C(=O)-* or - OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D,
and
$L_3$ is a spacer moiety.

**Embodiment 34.** The linker of Embodiment 32 or 33, wherein:

$L_1$ is a bridging spacer;
Lp is a bivalent peptide spacer comprising two to four amino acid residues;
the

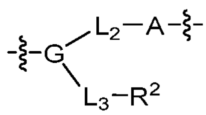

group is selected from:

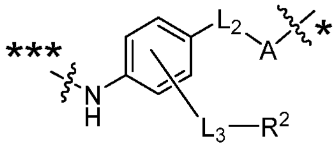,

wherein the * of

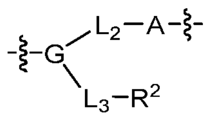

indicates the point of attachment to D (e.g., to an N or a O of the Drug moiety), the *** of

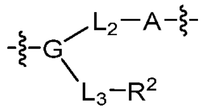

indicates the point of attachment to Lp;
$R^2$ is a hydrophilic moiety;
$L_2$ is a bond, a methylene, a neopentylene or a $C_2$-$C_3$alkenylene;
A is a bond, -OC(=O)-*,

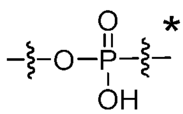, 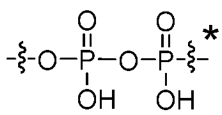, 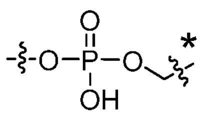,

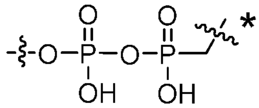,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D,

and

$L_3$ is a spacer moiety.

**Embodiment 35.** The linker of any one of Embodiments 32 to 34, wherein:

$L_1$ is $*-C(=O)(CH_2)_mO(CH_2)_m-**$; $*-C(=O)((CH_2)_mO)_t(CH_2)_n-**$; $*-C(=O)(CH_2)_m-**$; $*-C(=O)NH((CH_2)_mO)_t(CH_2)_n-**$; $*-C(=O)O(CH_2)_mSSC(R^3)_2(CH_2)_mC(=O)NR^3(CH_2)_mNR^3C(=O)(CH_2)_m-**$; $*-C(=O)O(CH_2)_mC(=O)NH(CH_2)_m-**$; $*-C(=O)(CH_2)_mNH(CH_2)_m-**$; $*-C(=O)(CH_2)_mNH(CH_2)_nC(=O)-**$; $*-C(=O)(CH_2)_mX_1(CH_2)_m-**$; $*-C(=O)((CH_2)_mO)_t(CH_2)_nX_1(CH_2)_n-**$; $*-C(=O)(CH_2)_mNHC(=O)(CH_2)_n-**$; $*-C(=O)((CH_2)_mO)_t(CH_2)_nNHC(=O)(CH_2)_n-**$; $*-C(=O)(CH_2)_mNHC(=O)(CH_2)_nX_1(CH_2)_n-**$; $*-C(=O)((CH_2)_mO)_t(CH_2)_nNHC(=O)(CH_2)_nX_1(CH_2)_n-**$; $*-C(=O)((CH_2)_mO)_t(CH_2)_nC(=O)NH(CH_2)_m-**$; $*-C(=O)(CH_2)_mC(R^3)_2-**$ or $*-C(=O)(CH_2)_mC(=O)NH(CH_2)_m-**$, where the * of $L_1$ indicates the point of attachment to Lp;

$R^2$ is a hydrophilic moiety selected from polyethylene glycol, polyalkylene glycol, a sugar, an oligosaccharide, a polypeptide or $C_2$-$C_6$alkyl substituted with 1 to 3

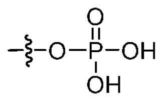

groups;

each $R^3$ is independently selected from H and $C_1$-$C_6$alkyl;

$X_1$ is

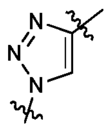, 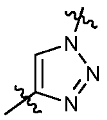, 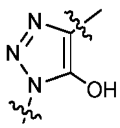 or 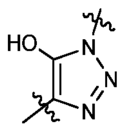;

each m is independently selected from 1, 2, 3, 4, 5, 6, 7, 8, 9 and 10;

each n is independently selected from 1, 2, 3, 4, 5, 6, 7, 8, 9 and 10;

each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;

Lp is a bivalent peptide spacer comprising an amino acid residue selected from glycine, valine, citrulline, lysine, isoleucine, phenylalanine, methionine, asparagine, proline, alanine, leucine, tryptophan, and tyrosine;

A is a bond, $-OC(=O)-*$,

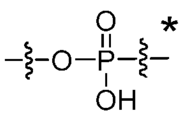, 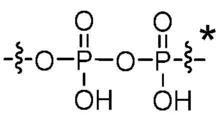, 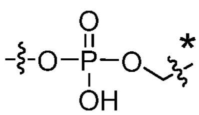,

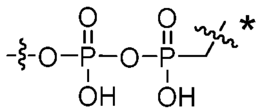,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D;

$L_3$ is a spacer moiety having the structure

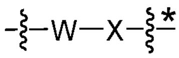,

where

W is $-CH_2O-$**, $-CH_2N(R^b)C(=O)O-$**, $-NHC(=O)C(R^b)_2NHC(=O)O-$**, $-NHC(=O)C(R^b)_2NH-$**, $-NHC(=O)C(R^b)_2NHC(=O)-$**, $-CH_2N(X-R^2)C(=O)O-$**, $-C(=O)N(X-R^2)-$**, $-CH_2N(X-R^2)C(=O)-$**, $-C(=O)NR^b-$**, $-C(=O)NH-$**, $-CH_2NR^bC(=O)-$**, - $CH_2NR^bC(=O)NH-$**, $-CH_2NR^bC(=O)NR^b-$**, $-NHC(=O)-$**, - $NHC(=O)O-$**, $-NHC(=O)NH-$**, $-OC(=O)NH-$**, $-S(O)_2NH-$**, - $NHS(O)_2-$**, $-C(=O)-$, $-C(=O)O-$** or -NH-, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and
the * of $L_3$ indicates the point of attachment to $R^2$.

**Embodiment 36.** The linker of any one of Embodiments 32 to 35, wherein:

$L_1$ is *-$C(=O)(CH_2)_mO(CH_2)_m$-**; *-$C(=O)((CH_2)_mO)_t(CH_2)_n$-**; *-$C(=O)(CH_2)_m$-**; or *-$C(=O)NH((CH_2)_mO)_t(CH_2)_n$-, where the * of $L_1$ indicates the point of attachment to Lp;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

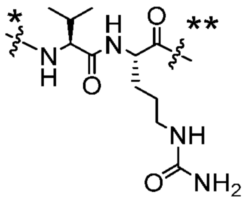 (ValCit),

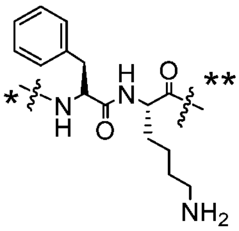 (PheLys), 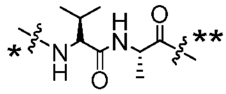 (ValAla), 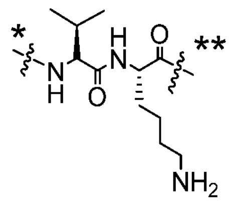

(ValLys) and

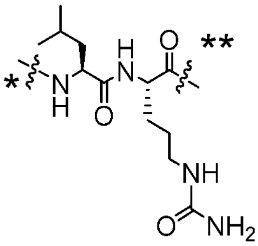 (LeuCit),

where the * of Lp indicates the attachment point to $L_1$;
$L_3$ is a spacer moiety having the structure

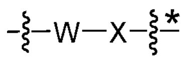,

where

W is $-CH_2O-^{**}$, $-CH_2N(R^b)C(=O)O-^{**}$, $-NHC(=O)CH_2NHC(=O)O-^{**}$, $-NHC(=O)CH_2NH-^{**}$, $-NHC(=O)CH_2NHC(=O)-^{**}$, $-CH_2N(X-R^2)C(=O)O-^{**}$, $-C(=O)N(X-R^2)-^{**}$, $-CH_2N(X-R^2)C(=O)-^{**}$, $-C(=O)NR^b-^{**}$, $-C(=O)NH-^{**}$, $-CH_2NR^bC(=O)-^{**}$, - $CH_2NR^bC(=O)NH-^{**}$, $-CH_2NR^bC(=O)NR^b-^{**}$, $-NHC(=O)-^{**}$, - $NHC(=O)O-^{**}$, $-NHC(=O)NH-^{**}$, $-OC(=O)NH-^{**}$, $-S(O)_2NH-^{**}$, - $NHS(O)_2-^{**}$, -C(=O)-, $-C(=O)O-^{**}$ or -NH-, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

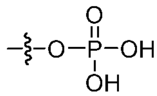

groups;
and
A is a bond, -OC(=O)-*,

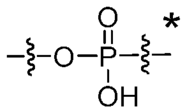, 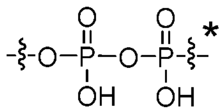, 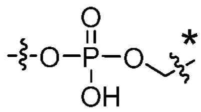,

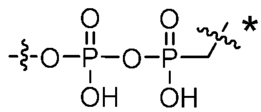,

$-OC(=O)N(CH_3)CH_2CH_2N(CH_3)C(=O)-^*$ or - $OC(=O)N(CH_3)C(R^a)_2C(R^a)_2N(CH_3)C(=O)-^*$, wherein each $R^a$ is independently selected from H, $C_1$-$C_6$ alkyl, and $C_3$-$C_8$ cycloalkyl and the * of A indicates the point of attachment to D.

**Embodiment 37.** The linker of any one of Embodiments 32 to 36, wherein:

$L_1$ is $^*-C(=O)(CH_2)_mO(CH_2)_m-^{**}$; $^*-C(=O)((CH_2)_mO)_t(CH_2)_n-^{**}$; $^*-C(=O)(CH_2)_m-^{**}$; or $^*-C(=O)NH((CH_2)_mO)_t(CH_2)_n-$, where the * of $L_1$ indicates the point of attachment to Lp;

each m is independently selected from 1, 2, 3, 4, 5, 6, 7, 8, 9 and 10;

each n is independently selected from 1, 2, 3, 4, 5, 6, 7, 8, 9 and 10;

each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;

Lp is a bivalent peptide spacer selected from

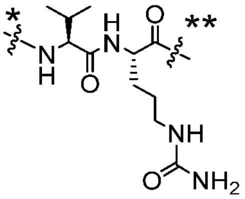 (ValCit),

where the * of Lp indicates the attachment point to $L_1$ and the ** of Lp indicates the attachment point to the -NH-group of G;

$L_3$ is a spacer moiety having the structure

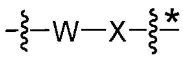 ,

where

W is $-CH_2O$-**, -CH2N(Rb)C(=O)O-**, $-NHC(=O)CH_2NHC(=O)O$-**, $-CH_2N(X-R^2)C(=O)O$-**, $-C(=O)N(X-R^2)$-**, $-CH_2N(X-R^2)C(=O)$-**, $-C(=O)NR^b$-**, -C(=O)NH-**, $-CH_2NR^bC(=O)$-**, - $CH_2NR^bC(=O)NH$-**, $-CH_2NR^bC(=O)NR^b$-**, -NHC(=O)-**, - NHC(=O)O-**, -NHC(=O)NH-**, -OC(=O)NH-**, $-S(O)_2NH$-**, - $NHS(O)_2$-**, -C(=O)-, -C(=O)O-** or
-NH-, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

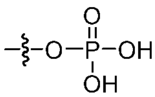

groups;
and

A is a bond, -OC(=O)-*,

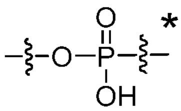 , 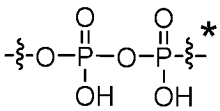 , 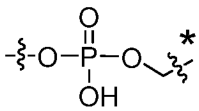 ,

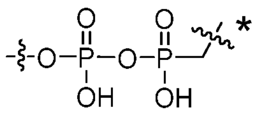 ,

$-OC(=O)N(CH_3)CH_2CH_2N(CH_3)C(=O)$-* or $-OC(=O)N(CH_3)C(R^a)_2C(R^a)_2N(CH_3)C(=O)$-*, wherein each $R^a$ is independently selected from H, $C_1$-$C_6$ alkyl, and $C_3$-$C_8$ cycloalkyl and the * of A indicates the point of attachment to D.

**Embodiment 38.** The linker of any one of Embodiments 32 to 37, wherein:

$L_1$ is *-$C(=O)(CH_2)_mO(CH_2)_m$-**; *-$C(=O)((CH_2)_mO)t(CH_2)_n$-**; *-$C(=O)(CH_2)_m$-**; or *-$C(=O)NH((CH_2)_mO)_t(CH_2)_n$-, where the * of $L_1$ indicates the point of attachment to Lp;

each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

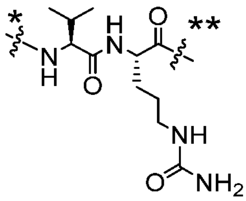 (ValCit),

where the * of Lp indicates the attachment point to $L_1$;
$L_3$ is a spacer moiety having the structure

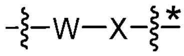 ,

where

W is $-CH_2O-$**, -CH2N(Rb)C(=O)O-**, $-NHC(=O)CH_2NHC(=O)O-$**, $-CH_2N(X-R^2)C(=O)O-$**, $-C(=O)N(X-R^2)-$**, $-C(=O)NR^b-$**, - C(=O)NH-**, $-CH_2NR^bC(=O)-$**, $-CH_2NR^bC(=O)NH-$**, - $CH_2NR^bC(=O)NR^b-$**, -NHC(=O)-**, -NHC(=O)O-**, or -NHC(=O)NH-**, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

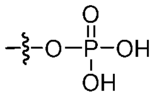

groups;
and
A is a bond or -OC(=O)* in which * indicates the attachment point to D.

**Embodiment 39.** The linker of any one of Embodiments 32 to 38, wherein:

$L_1$ is $*-C(=O)(CH_2)_mO(CH_2)_m-$**; $*-C(=O)((CH_2)_mO)_t(CH_2)_n-$**; $*-C(=O)(CH_2)_m-$**; or $*-C(=O)NH((CH_2)_mO)_t(CH_2)_n-$, where the * of $L_1$ indicates the point of attachment to Lp;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

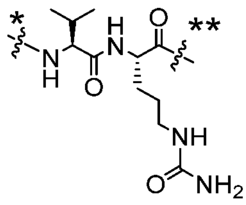

(ValCit),

where the * of Lp indicates the attachment point to $L_1$;
$L_3$ is a spacer moiety having the structure

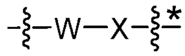

,

where

W is $-CH_2O-$**, -CH2N(Rb)C(=O)O-**, $-NHC(=O)CH_2NHC(=O)O-$**, $-CH_2N(X-R^2)C(=O)O-$**, or $-C(=O)N(X-R^2)-$**, wherein each $R^b$ is independently selected from H, $C_1$-$C_6$alkyl or $C_3$-$C_8$cycloalkyl and wherein the ** of W indicates the point of attachment to X;
X is ***-$CH_2$-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

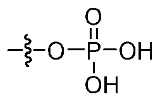

groups;
and
A is a bond or -OC(=O)* in which * indicates the attachment point to D.

**Embodiment 40.** The linker of Formula (C') having the structure having the structure of Formula (D'),

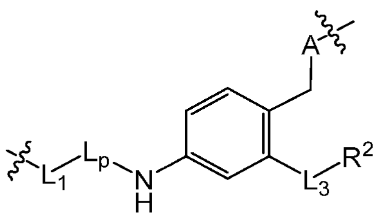

Formula (D')

wherein

$L_1$ is a bridging spacer;
Lp is a bivalent peptide spacer;
$R^2$ is a hydrophilic moiety;
A is a bond, -OC(=O)-*,

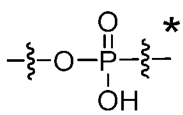
,
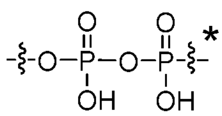
,
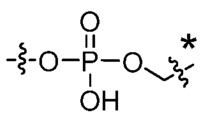
,

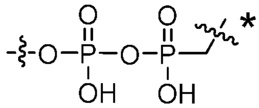,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D,

and

$L_3$ is a spacer moiety.

**Embodiment 41.** The linker of Embodiments 40, wherein:

$L_1$ is a bridging spacer;

Lp is a bivalent peptide spacer comprising two to four amino acid residues;

$R^2$ is a hydrophilic moiety;

A is a bond, -OC(=O)-*

, 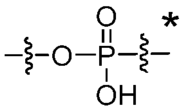 , 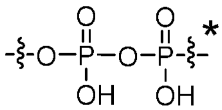 , 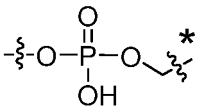 ,

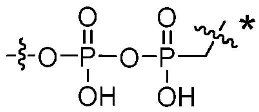,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D,

and

$L_3$ is a spacer moiety.

**Embodiment 42.** The linker of Embodiment 40 or 41, wherein:

$L_1$ is $*-C(=O)(CH_2)_mO(CH_2)_m-**$; $*-C(=O)((CH_2)_mO)_t(CH_2)_n-**$; $*-C(=O)(CH_2)_m-**$; $*-C(=O)NH((CH_2)_mO)_t(CH_2)_n-**$; $*-C(=O)O(CH_2)_mSSC(R^3)_2(CH_2)_mC(=O)NR^3(CH_2)_mNR^3C(=O)(CH_2)_m-**$; $*-C(=O)O(CH_2)_mC(=O)NH(CH_2)_m-**$; $*-C(=O)(CH_2)_mNH(CH_2)_m-**$; $*-C(=O)(CH_2)_mNH(CH_2)_nC(=O)-**$; $*-C(=O)(CH_2)_mX_1(CH_2)_m-**$; $*-C(=O)((CH_2)_mO)_t(CH_2)_nX_1(CH_2)_n-**$; $*-C(=O)(CH_2)_mNHC(=O)(CH_2)_n-**$; $*-C(=O)((CH_2)_mO)_t(CH_2)_nNHC(=O)(CH_2)_n-**$; $*-C(=O)(CH_2)_mNHC(=O)(CH_2)_nX_1(CH_2)_n-**$; $*-C(=O)((CH_2)_mO)_t(CH_2)_nNHC(=O)(CH_2)_nX_1(CH_2)_n-**$; $*-C(=O)((CH_2)_mO)_t(CH_2)_nC(=O)NH(CH_2)_m-**$; $*-C(=O)(CH_2)_mC(R^3)_2-**$ or $*-C(=O)(CH_2)_mC(=O)NH(CH_2)_m-**$, where the * of $L_1$ indicates the point of attachment to Lp;

$R^2$ is a hydrophilic moiety selected from polyethylene glycol, polyalkylene glycol, a sugar, an oligosaccharide, a polypeptide or $C_2$-$C_6$alkyl substituted with 1 to 3

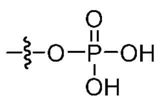

groups;

each $R^3$ is independently selected from H and $C_1$-$C_6$alkyl;

$X_1$ is

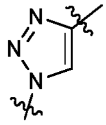, 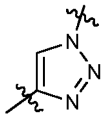, 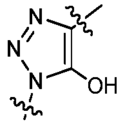 or 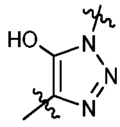;

each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer comprising an amino acid residue selected from glycine, valine, citrulline, lysine, isoleucine, phenylalanine, methionine, asparagine, proline, alanine, leucine, tryptophan, and tyrosine;
A is a bond, -OC(=O)-*

;, 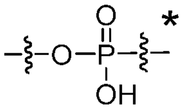, 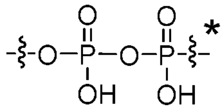, 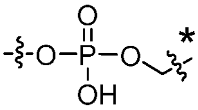,

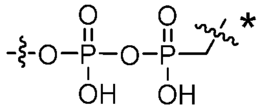,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety having the structure

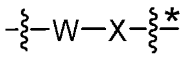,

where

W is -$CH_2$O-**, -CH2N(Rb)C(=O)O-**, -NHC(=O)$CH_2$NHC(=O)O-**, -$CH_2$N(X-$R^2$)C(=O)O-**, -C(=O)N(X-$R^2$)-**, -$CH_2$N(X-$R^2$)C(=O)-**, -C(=O)N$R^b$-**, -C(=O)NH-**, -$CH_2$N$R^b$C(=O)-**, - $CH_2$N$R^b$C(=O)NH-**, -$CH_2$N$R^b$C(=O)N$R^b$-**, -NHC(=O)-**, - NHC(=O)O-**, -NHC(=O)NH-**, -OC(=O)NH-**, -S(O)$_2$NH-**, -NHS(O)$_2$-**, -C(=O)-, -C(=O)O-** or
-NH-, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and
the * of $L_3$ indicates the point of attachment to $R^2$.

**Embodiment 43.** The linker of any one of Embodiments 40 to 42, wherein:

$L_1$ is *-C(=O)($CH_2$)$_m$O($CH_2$)$_m$-**; *-C(=O)(($CH_2$)$_m$O)t($CH_2$)$_n$-**; *-C(=O)($CH_2$)$_m$-**; or *-C(=O)NH(($CH_2$)$_m$O)$_t$ ($CH_2$)$_n$-, where the * of $L_1$ indicates the point of attachment to Lp;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

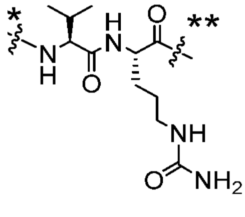 (ValCit),

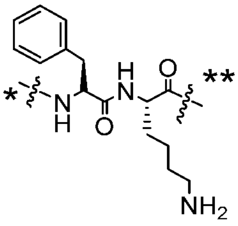 (PheLys), 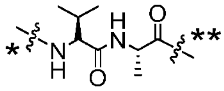 (ValAla), 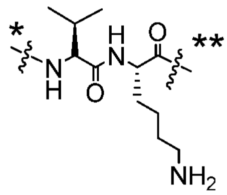

(ValLys) and

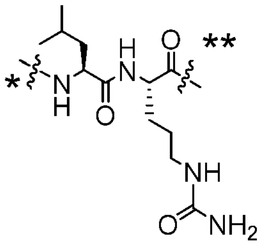 (LeuCit),

where the * of Lp indicates the attachment point to $L_1$ and the ** of Lp indicates the attachment point to the - NH- group of G;

$L_3$ is a spacer moiety having the structure

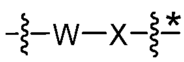,

where

W is $-CH_2O-$**, -CH2N(Rb)C(=O)O-**, $-NHC(=O)CH_2NHC(=O)O-$**, $-CH_2N(X-R^2)C(=O)O-$**, $-C(=O)N(X-R^2)-$**, $-CH_2N(X-R^2)C(=O)-$**, $-C(=O)NR^b-$**, -C(=O)NH-**, $-CH_2NR^bC(=O)-$**, - $CH_2NR^bC(=O)NH-$**, $-CH_2NR^bC(=O)NR^b-$**, -NHC(=O)-**, - NHC(=O)O-**, -NHC(=O)NH-**, -OC(=O)NH-**, $-S(O)_2NH-$**, - $NHS(O)_2-$**, -C(=O)-, -C(=O)O-** or -NH-, wherein each $R^b$ is independently selected from H, $C_1$-$C_6$alkyl or $C_3$-$C_8$cycloalkyl and wherein the ** of W indicates the point of attachment to X;

X is a bond, triazolyl or ***-$CH_2$-triazolyl-*, wherein the *** of X indicates the point of attachment to W and the * of X indicates the point of attachment to $R^2$;

and

the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

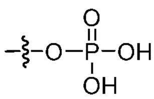

groups;
and
A is a bond, -OC(=O)-*

, 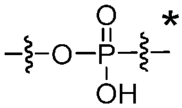 , 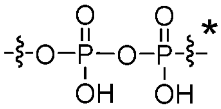 , 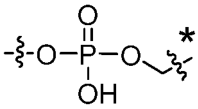 ,

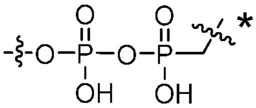 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D.

**Embodiment 44.** The linker of any one of Embodiments 40 to 43, wherein:

$L_1$ is $*-C(=O)(CH_2)_mO(CH_2)_m-**$; $*-C(=O)((CH_2)_mO)_t(CH_2)_n-**$; $*-C(=O)(CH_2)_m-**$; or $*-C(=O)NH((CH_2)_mO)_t(CH_2)_n-$, where the * of $L_1$ indicates the point of attachment to Lp;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

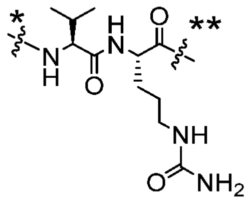 (ValCit),

where the * of Lp indicates the attachment point to $L_1$ and the ** of Lp indicates the attachment point to the -NH- group of G;
$L_3$ is a spacer moiety having the structure

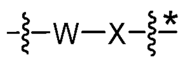 ,

where

W is $-CH_2O-**$, $-CH_2N(R^b)C(=O)O-**$, $-NHC(=O)CH_2NHC(=O)O-**$, $-CH_2N(X-R^2)C(=O)O-**$, $-C(=O)N(X-R^2)-**$, $-CH_2N(X-R^2)C(=O)-**$, $-C(=O)NR^b-**$, $-C(=O)NH-**$, $-CH_2NR^bC(=O)-**$, $-CH_2NR^bC(=O)NH-**$, $-CH_2NR^bC(=O)NR^b-**$, $-NHC(=O)-**$, $-NHC(=O)O-**$, $-NHC(=O)NH-**$, $-OC(=O)NH-**$, $-S(O)_2NH-**$, $-NHS(O)_2-**$, $-C(=O)-$, $-C(=O)O-**$ or
-NH-, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-CH2-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

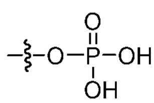

groups;
and
A is a bond, -OC(=O)-*,

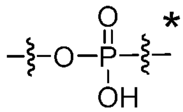,
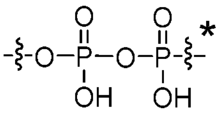,
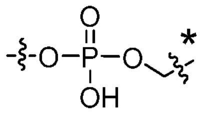,

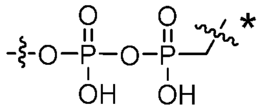,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D.

**Embodiment 45.** The linker of any one of Embodiments 40 to 44, wherein:

$L_1$ is *-C(=O)($CH_2$)$_m$O($CH_2$)$_m$-**; *-C(=O)(($CH_2$)$_m$O)$_t$($CH_2$)$_n$-**; *-C(=O)($CH_2$)$_m$-**; or *-C(=O)NH(($CH_2$)$_m$O)$_t$($CH_2$)$_n$-, where the * of $L_1$ indicates the point of attachment to Lp;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

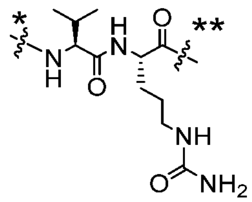 (ValCit),

where the * of Lp indicates the attachment point to $L_1$ and the ** of Lp indicates the attachment point to the -NH- group of G;
$L_3$ is a spacer moiety having the structure

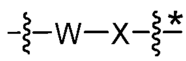,

where

W is -$CH_2$O-**, -$CH_2$N($R^b$)C(=O)O-**, -NHC(=O)$CH_2$NHC(=O)O-**, -$CH_2$N(X-$R^2$)C(=O)O-**, -C(=O)N(X-$R^2$)-**, -C(=O)N$R^b$-**, - C(=O)NH-**, -$CH_2$N$R^b$C(=O)-**, -$CH_2$N$R^b$C(=O)NH-**, - $CH_2$N$R^b$C(=O)N$R^b$-**, -NHC(=O)-**, -NHC(=O)O-**, or -NHC(=O)NH-**, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

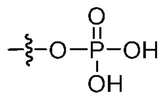

groups;
and
A is a bond or -OC(=O)* in which * indicates the attachment point to D.

**Embodiment 46.** The linker of any one of Embodiments 40 to 45, wherein:

$L_1$ is *-C(=O)$(CH_2)_m$O$(CH_2)_m$-**; *-C(=O)($(CH_2)_m$O)t$(CH_2)_n$-**; *-C(=O)$(CH_2)_m$-**; or *-C(=O)NH($(CH_2)_m$O)$_t$ $(CH_2)_n$-, where the * of $L_1$ indicates the point of attachment to Lp;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

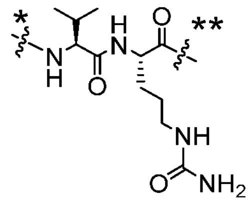 (ValCit),

where the * of Lp indicates the attachment point to $L_1$ and the ** of Lp indicates the attachment point to the -NH- group of G;
$L_3$ is a spacer moiety having the structure

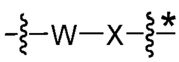,

where

W is -$CH_2$O-**, -$CH_2$N($R^b$)C(=O)O-**, -NHC(=O)$CH_2$NHC(=O)O-**, -$CH_2$N(X-$R^2$)C(=O)O-**, or -C(=O)N(X-$R^2$)-**, wherein each $R^b$ is independently selected from H, $C_1$-$C_6$alkyl or $C_3$-$C_8$cycloalkyl and wherein the ** of W indicates the point of attachment to X;
X is ***-$CH_2$-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

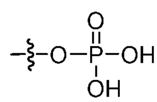

groups;
and
A is a bond or -OC(=O)* in which * indicates the attachment point to D.

**Embodiment 47.** The linker of any one of Embodiments 32 to 46, having the structure:

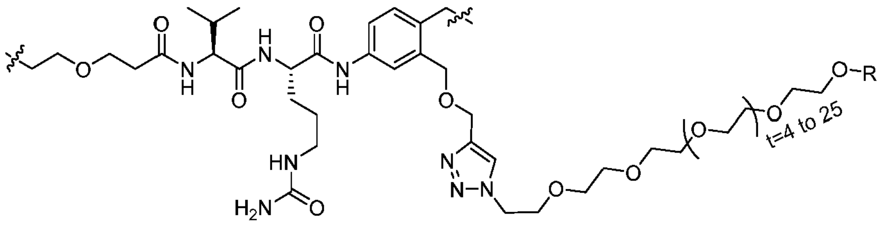

,

where

R is H, $-CH_3$ or $-CH_2CH_2C(=O)OH$.

**Embodiment 48.** The linker of any one of Embodiments 32 to 46, having the structure:

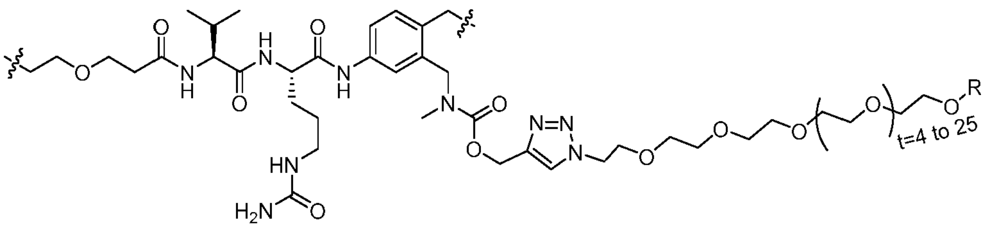

, where

R is H, $-CH_3$ or $-CH_2CH_2C(=O)OH$.

**Embodiment 49.** The linker of any one of Embodiments 32 to 46, having the structure:

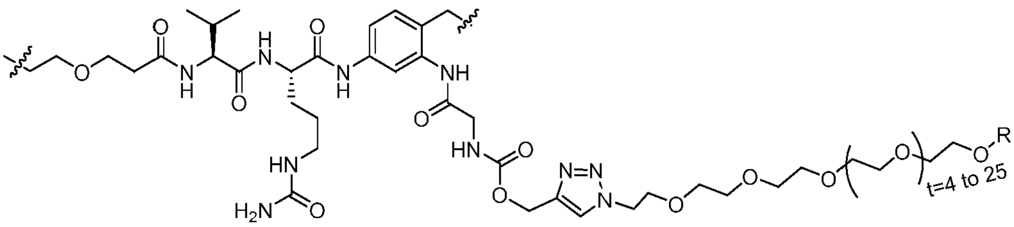

,

where

R is H, $-CH_3$ or $-CH_2CH_2C(=O)OH$.

**Embodiment 50.** The linker of any one of Embodiments 32 to 46, having the structure:

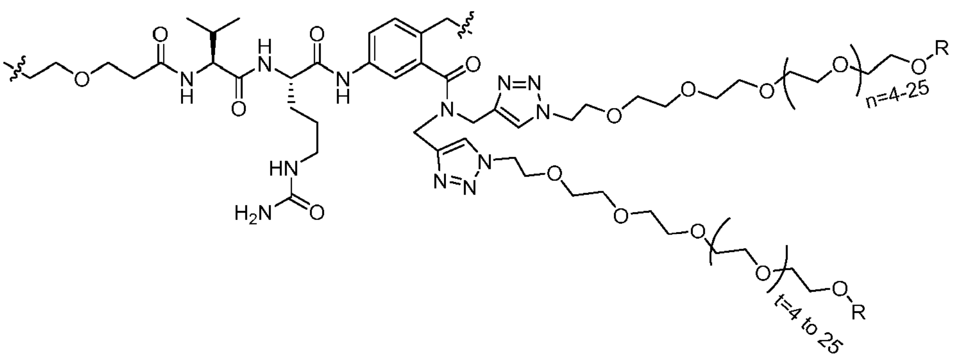

, where

each R is independently selected from H, $-CH_3$ or $-CH_2CH_2C(=O)OH$.

**Embodiment 51.** The linker of any one of Embodiments 32 to 46, having the structure:

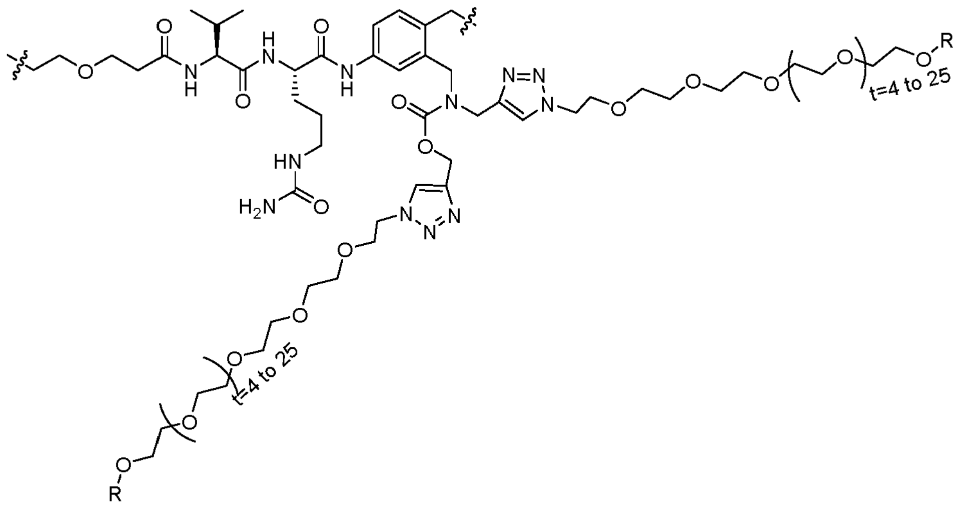

where

each R is independently selected from H, $-CH_3$ or $-CH_2CH_2C(=O)OH$.

**Embodiment 52.** The linker of any one of Embodiments 32 to 46, having the structure:

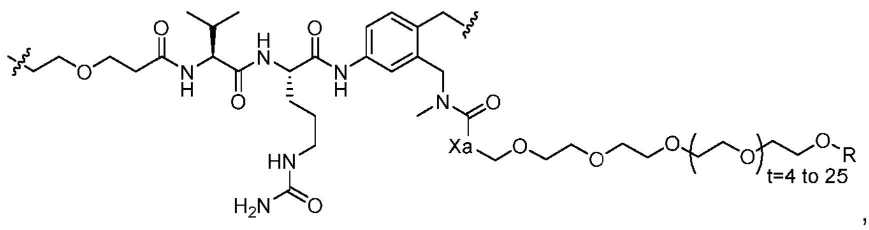

where

Xa is $-CH_2-$, $-OCH_2-$, $-NHCH_2-$ or $-NRCH_2-$ and each R independently is H, $-CH_3$ or - $CH_2CH_2C(=O)OH$.

**Embodiment 53.** The linker of any one of Embodiments 32 to 46, having the structure:

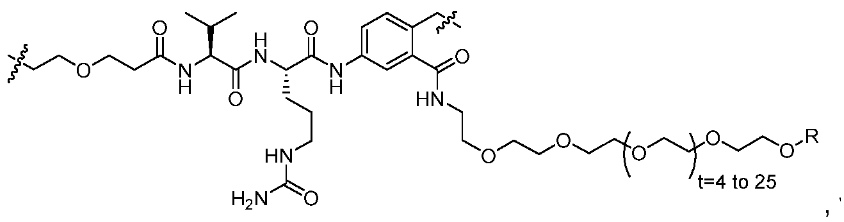

where

R is H, $-CH_3$ or $-CH_2CH_2C(=O)OH$.

**Embodiment 54.** The linker of any one of Embodiments 32 to 46, having the structure:

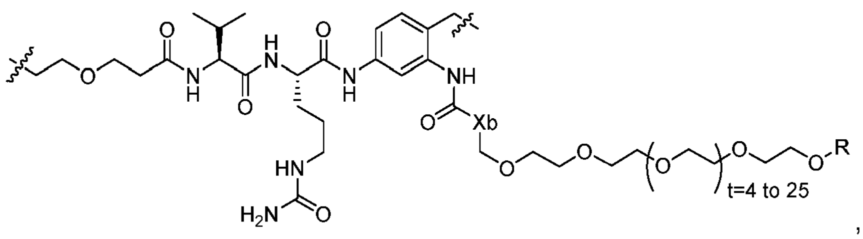

where

Xb is $-CH_2-$, $-OCH_2-$, $-NHCH_2-$ or $-NRCH_2-$ and each R independently is H, $-CH_3$ or - $CH_2CH_2C(=O)OH$.

**Embodiment 55.** The linker of any one of Embodiments 32 to 46, having the structure:

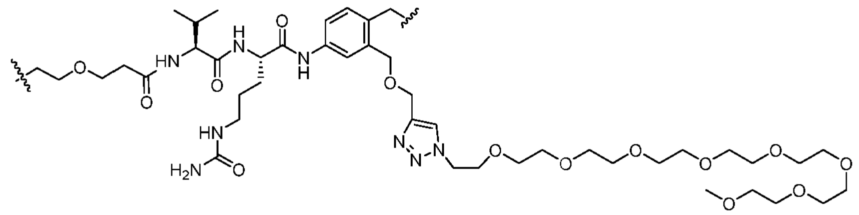

**Embodiment 56.** The linker of any one of Embodiments 32 to 46, having the structure:

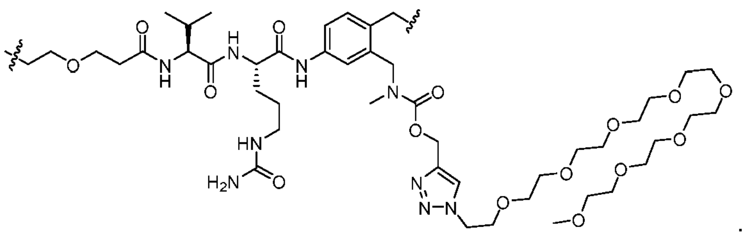

**Embodiment 57.** The linker of any one of Embodiments 32 to 46, having the structure:

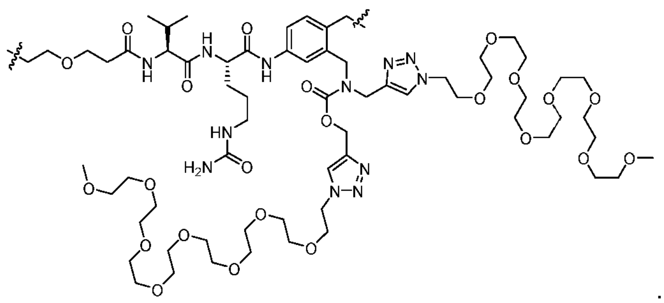

**Embodiment 58.** The linker of any one of Embodiments 32 to 46, having the structure:

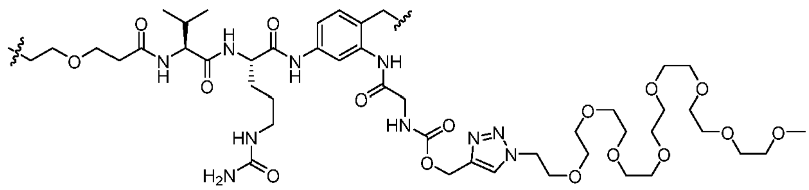

**Embodiment 59.** The linker of any one of Embodiments 32 to 46, having the structure:

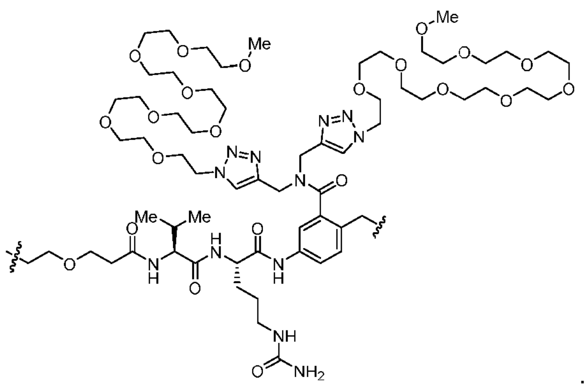

For illustrative purposes, the general reaction schemes depicted herein provide potential routes for synthesizing the compounds of the present invention as well as key intermediates. For a more detailed description of the individual reaction steps, see the Examples section below. Although specific starting materials and reagents are depicted in the

schemes and discussed below, other starting materials and reagents can be easily substituted to provide a variety of derivatives and/or reaction conditions. In addition, many of the compounds prepared by the methods described below can be further modified in light of this disclosure using conventional chemistry well known to those skilled in the art.

By way of example, a general synthesis for compounds of Formula (B') is shown below in Scheme 1.

Scheme 1

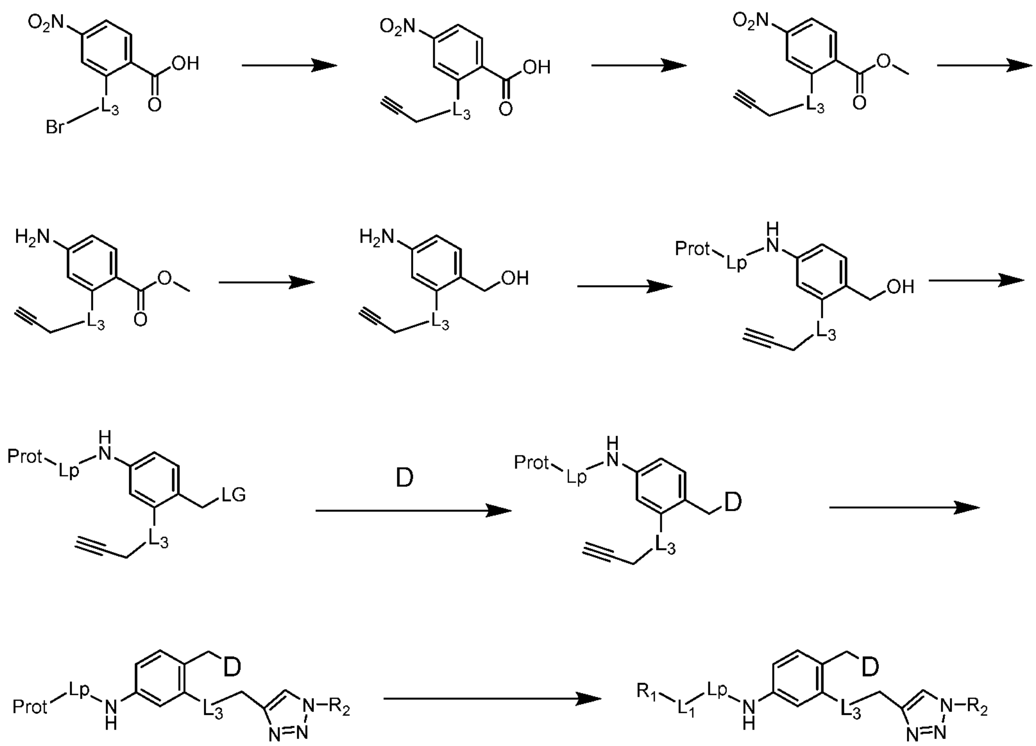

Antibody Drug Conjugates of the Invention

The present invention provides Antibody Drug Conjugates, also referred to herein as immunoconjugates, which comprise linkers which comprise one or more hydrophilic moieties.

The Antibody Drug Conjugates of the invention have the structure of Formula (E'):

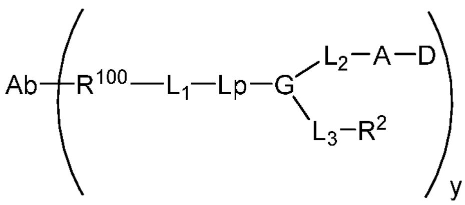

Formula (E')

wherein:

Ab is an antibody or fragment thereof;
$R^{100}$ is a coupling group;
$L_1$ is a bridging spacer;
Lp is a bivalent peptide spacer;
G-$L_2$-A is a self-immolative spacer;
$R^2$ is a hydrophilic moiety;
$L_2$ is a bond, a methylene, a neopentylene or a $C_2$-$C_3$alkenylene;
A is a bond, -OC(=O)-*,

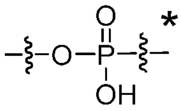, 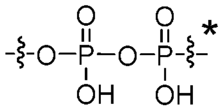, 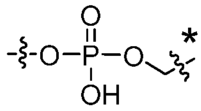,

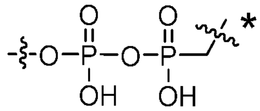,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D;
$L_3$ is a spacer moiety;
D is a Drug moiety as defined herein, e.g., a MCI-1 inhibitor, and may comprise an N or an O, wherein D can be connected to A via a direct bond from A to the N or the O of the Drug moiety,
and
y is 1, 2, 3, 4, 5, 6, 7, 8, 9, 10, 11, 12, 13, 14, 15 or 16.

Certain aspects and examples of the Antibody Drug Conjugates of the invention are provided in the following listing of enumerated embodiments. It will be recognized that features specified in each embodiment may be combined with other specified features to provide further embodiments of the present invention.

**Embodiment 60.** The immunoconjugate of Formula (E') wherein:

Ab is an antibody or fragment thereof;
$R^{100}$ is a coupling group;
$L_1$ is a bridging spacer;
Lp is a bivalent peptide spacer comprising two to four amino acid residues;
G-$L_2$-A is a self-immolative spacer;
$R^2$ is a hydrophilic moiety;
$L_2$ is a bond, a methylene, a neopentylene or a $C_2$-$C_3$alkenylene;
A is a bond, -OC(=O)-*,

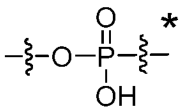, 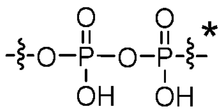, 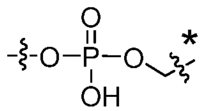,

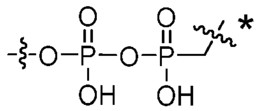,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D;
$L_3$ is a spacer moiety;
D is a Drug moiety as defined herein wherein D is connected to A via a direct bond from A to D (e.g., an N or O of the Drug moiety),
and
y is 1, 2, 3, 4, 5, 6, 7, 8, 9, 10, 11, 12, 13, 14, 15 or 16.

**Embodiment 61.** The immunoconjugate of Formula (E') or Embodiment 60, wherein:

Ab is an antibody or fragment thereof;
$R^{100}$ is a coupling group;
$L_1$ is a bridging spacer;
Lp is a bivalent peptide spacer comprising two to four amino acid residues;

the

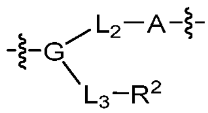

group is selected from:

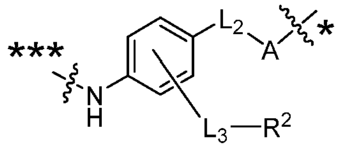

,

wherein the * of

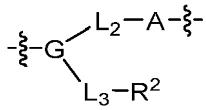

indicates the point of attachment to D (e.g., to an N or a O of the Drug moiety), the *** of

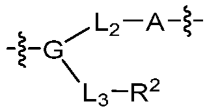

indicates the point of attachment to Lp;
$R^2$ is a hydrophilic moiety;
$L_2$ is a bond, a methylene, a neopentylene or a $C_2$-$C_3$alkenylene;
A is a bond, -OC(=O)-*,

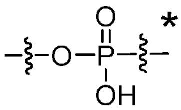

,

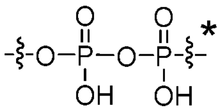

,

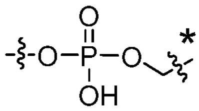

,

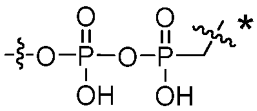

,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety;
D is a Drug moiety as defined herein and comprising an N or an O, wherein D is connected to A via a direct bond from A to the N or the O of the Drug moiety,
and
y is 1, 2, 3, 4, 5, 6, 7, 8, 9, 10, 11, 12, 13, 14, 15 or 16.

**Embodiment 62.** The immunoconjugate of Formula (E') or any one of Embodiments 60 to 61 having the structure of Formula (F'),
wherein:

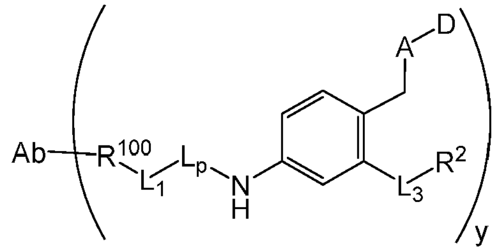

Formula (F')

Ab is an antibody or fragment thereof;
$R^{100}$ is a coupling group;
$L_1$ is a bridging spacer;
Lp is a bivalent peptide spacer comprising two to four amino acid residues;
$R^2$ is a hydrophilic moiety;
A is a bond, -OC(=O)-*

, 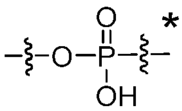 , 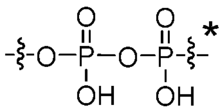 , 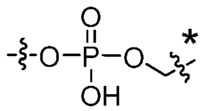 ,

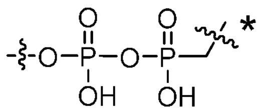 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D;
$L_3$ is a spacer moiety;
D is a Drug moiety as defined herein and comprising an N or an O, wherein D is connected to A via a direct bond from A to the N or the O of the Drug moiety,
and
y is 1, 2, 3, 4, 5, 6, 7, 8, 9, 10, 11, 12, 13, 14, 15 or 16.

**Embodiment 63.** The immunoconjugate of Formula (D') or any one of Embodiments 60 to 62, wherein:

Ab is an antibody or fragment thereof;
$R^{100}$ is

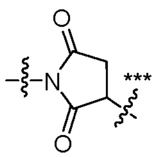 , 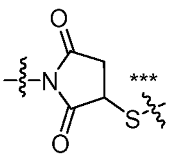 , 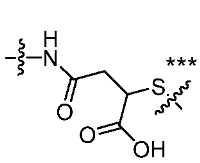 , 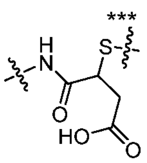 , 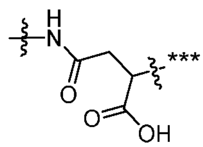 ,

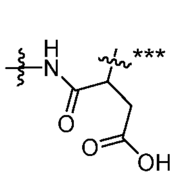 , 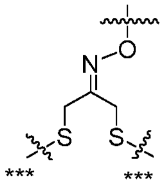 , 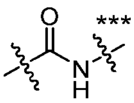 ,

-S-, -C(=O)-, -ON=***, - $NHC(=O)CH_2$-***, $-S(=O)_2CH_2CH_2$-***, $-(CH_2)_2S(=O)_2CH_2CH_2$-***, - $NHS(=O)_2CH_2CH_2$-***,

$-NHC(=O)CH_2CH_2-***$, $-CH_2NHCH_2CH_2-***$, $-NHCH_2CH_2-***$

, 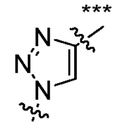 ,

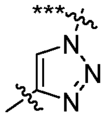 , 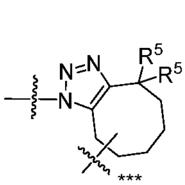 , 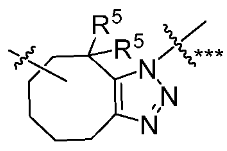 , 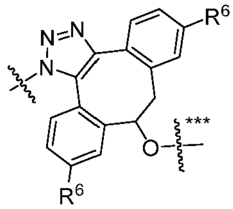 ,

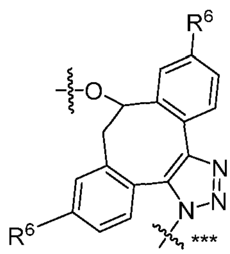 , 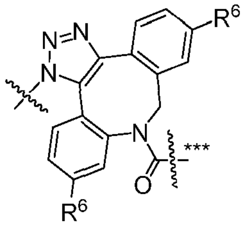 , 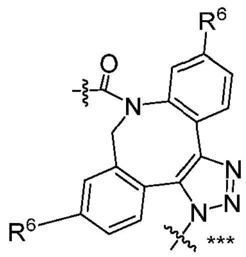 ,

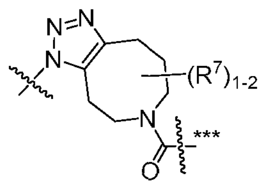 , 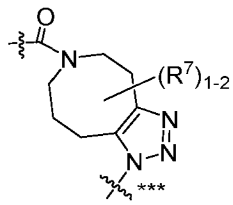 , 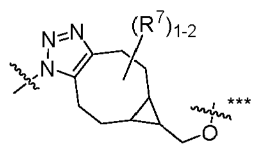 ,

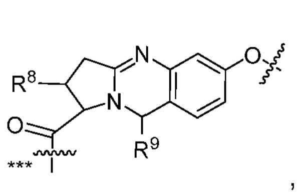 , 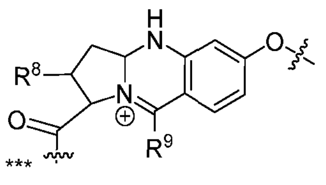 , 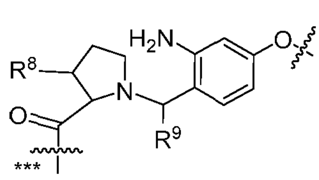 ,

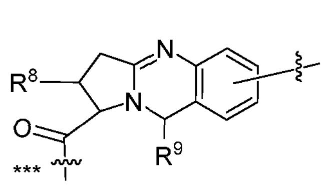 , 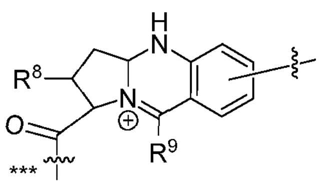 , 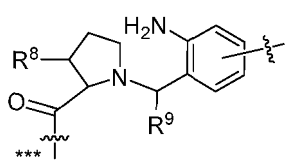 ,

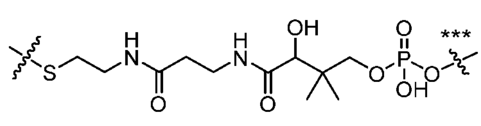 , 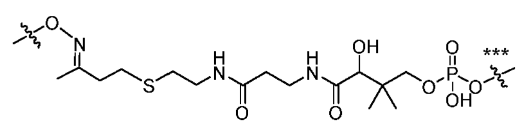 ,

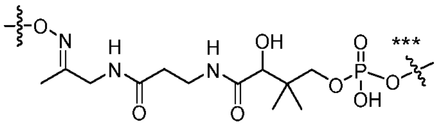, 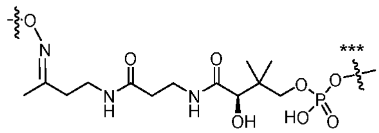,

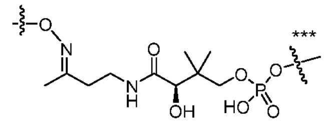, 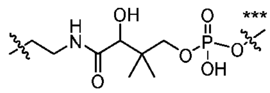, 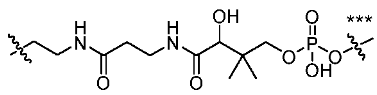,

or

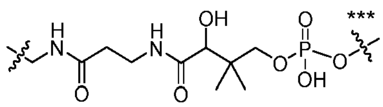,

where the *** of $R^{100}$ indicates the point of attachment to Ab;

$L_1$ is *-C(=O)$(CH_2)_m$O$(CH_2)_m$-**; *-C(=O)($(CH_2)_m$O$)_t$$(CH_2)_n$-**; *-C(=O)$(CH_2)_m$-**; *-C(=O)NH($(CH_2)_m$O$)_t$$(CH_2)_n$-**; *-C(=O)O$(CH_2)_m$SSC$(R^3)_2$$(CH_2)_m$C(=O)N$R^3$$(CH_2)_m$N$R^3$C(=O)$(CH_2)_m$-**; *-C(=O)O$(CH_2)_m$C(=O)NH$(CH_2)_m$-**; *-C(=O)$(CH_2)_m$NH$(CH_2)_m$-**; *-C(=O)$(CH_2)_m$NH$(CH_2)_n$C(=O)-**; *-C(=O)$(CH_2)_m$$X_1$$(CH_2)_m$-**; *-C(=O)($(CH_2)_m$O$)_t$$(CH_2)_n$$X_1$$(CH_2)_n$-**; *-C(=O)$(CH_2)_m$NHC(=O)$(CH_2)_n$-**; *-C(=O)($(CH_2)_m$O$)_t$$(CH_2)_n$NHC(=O)$(CH_2)_n$-**; *-C(=O)$(CH_2)_m$NHC(=O)$(CH_2)_n$$X_1$$(CH_2)_n$-**; *-C(=O)($(CH_2)_m$O$)_t$$(CH_2)_n$NHC(=O)$(CH_2)_n$$X_1$$(CH_2)_n$-**; *-C(=O)($(CH_2)_m$O$)_t$$(CH_2)_n$C(=O)NH$(CH_2)_m$-**; *-C(=O)$(CH_2)_m$C$(R^3)_2$-** or *-C(=O)$(CH_2)_m$C(=O)NH$(CH_2)_m$-**, where the * of $L_1$ indicates the point of attachment to Lp, and the ** of $L_1$ indicates the point of attachment to $R^{100}$;

$R^2$ is a hydrophilic moiety selected from polyethylene glycol, polyalkylene glycol, a sugar, an oligosaccharide, a polypeptide or $C_2$-$C_6$alkyl substituted with 1 to 3

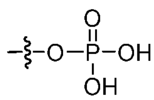

groups;

each $R^3$ is independently selected from H and $C_1$-$C_6$alkyl;

$R^4$ is 2-pyridyl or 4-pyridyl;

each $R^5$ is independently selected from H, $C_1$-$C_6$alkyl, F, Cl, and -OH;

each $R^6$ is independently selected from H, $C_1$-$C_6$alkyl, F, Cl, $-NH_2$, $-OCH_3$, $-OCH_2CH_3$, $-N(CH_3)_2$, -CN, $-NO_2$ and -OH;

each $R^7$ is independently selected from H, $C_{1-6}$alkyl, fluoro, benzyloxy substituted with -C(=O)OH, benzyl substituted with -C(=O)OH, $C_{1-4}$alkoxy substituted with -C(=O)OH and $C_{1-4}$alkyl substituted with -C(=O)OH;

$X_1$ is

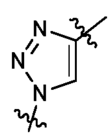, 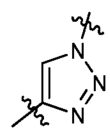, 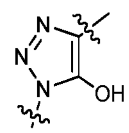 or 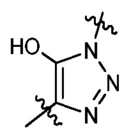;

each m is independently selected from 1, 2, 3, 4, 5, 6, 7, 8, 9 and 10;

each n is independently selected from 1, 2, 3, 4, 5, 6, 7, 8, 9 and 10;

each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;

Lp is a bivalent peptide spacer comprising an amino acid residue selected from valine, citrulline, lysine, isoleucine, phenylalanine, methionine, asparagine, proline, alanine, leucine, tryptophan, and tyrosine;

A is a bond, -OC(=O)-*,

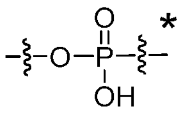, 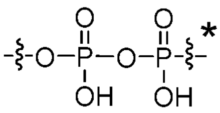, 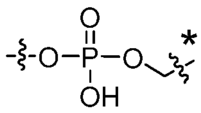,

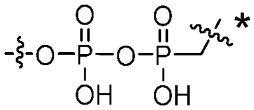

, $-OC(=O)N(CH_3)CH_2CH_2N(CH_3)C(=O)-$* or $-OC(=O)N(CH_3)C(R^a)_2C(R^a)_2N(CH_3)C(=O)-$*, wherein each $R^a$ is independently selected from H, $C_1$-$C_6$ alkyl, and $C_3$-$C_8$ cycloalkyl and the * of A indicates the point of attachment to D;

$L_3$ is a spacer moiety having the structure

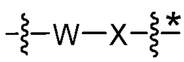,

where

W is $-CH_2O-$**, $-CH_2N(R^b)C(=O)O-$**, $-NHC(=O)C(R^b)_2NHC(=O)O-$** $-NHC(=O)C(R^b)_2NH-$**, $-NHC(=O)C(R^b)_2NHC(=O)-$**, $-CH_2N(X-R^2)C(=O)O-$**, $-C(=O)N(X-R^2)-$**, $-CH_2N(X-R$ $^2)C(=O)-$**, - $C(=O)NR^b-$**, $-C(=O)NH-$**, $-CH_2NR^bC(=O)-$**, $-CH_2NR^bC(=O)NH-$**, $-CH_2NR^bC(=O)NR^b-$**, $-NHC(=O)-$**, $-NHC(=O)O-$**, - $NHC(=O)NH-$**, $-OC(=O)NH-$**, $-S(O)_2NH-$**, $-NHS(O)_2-$**, $-C(=O)-$, $-C(=O)O-$** or $-NH-$, wherein each $R^b$ is independently selected from H, $C_1$-$C_6$alkyl or $C_3$-$C_8$cycloalkyl and wherein the ** of W indicates the point of attachment to X;

X is a bond, triazolyl or ***-$CH_2$-triazolyl-*, wherein the *** of X indicates the point of attachment to W and the * of X indicates the point of attachment to $R^2$;

and

the * of $L_3$ indicates the point of attachment to $R^2$;

D is a Drug moiety as defined herein and comprising an N or an O, wherein D is connected to A via a direct bond from A to the N or the O of the Drug moiety,

and

y is 1, 2, 3, 4, 5, 6, 7, 8, 9, 10, 11, 12, 13, 14, 15 or 16.

**Embodiment 64.** The immunoconjugate of Formula (D') or any one of Embodiments 60 to 63, wherein:

Ab is an antibody or fragment thereof;

$R^{100}$ is

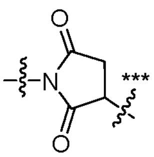, 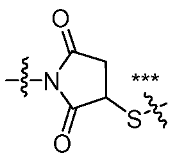, 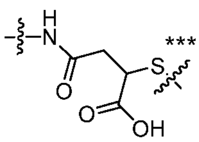, 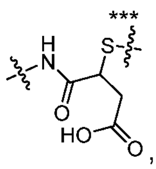, 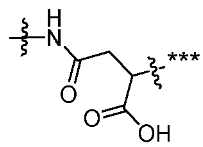,

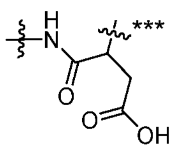, 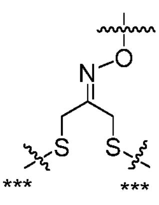 or 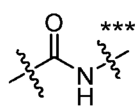,

where the *** of $R^{100}$ indicates the point of attachment to Ab;

$L_1$ is *-$C(=O)(CH_2)_mO(CH_2)_m$-**; *-$C(=O)((CH_2)_mO)t(CH_2)_n$-**; *-$C(=O)(CH_2)_m$-**; or *-$C(=O)NH((CH_2)_mO)_t(CH_2)_n$-, where the * of $L_1$ indicates the point of attachment to Lp, and the ** of $L_1$ indicates the point of attachment

to $R^{100}$;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

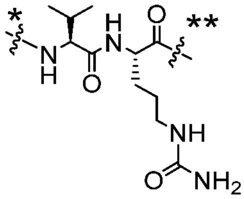 (ValCit),

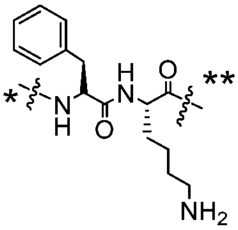 (PheLys), 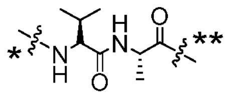 (ValAla), 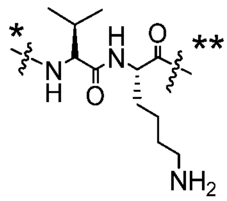

(ValLys) and 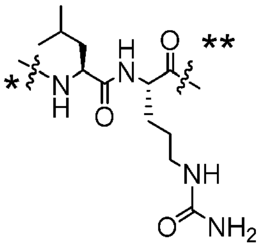 (LeuCit),

where the * of Lp indicates the attachment point to $L_1$ and the ** of Lp indicates the attachment point to the - NH- group of G;
$L_3$ is a spacer moiety having the structure

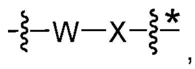,

where

W is $-CH_2O-$**, $-CH_2N(R^b)C(=O)O-$**, $-NHC(=O)CH_2NHC(=O)O-$**, $-NHC(=O)CH_2NH-$**, $-NHC(=O)CH_2NHC(=O)-$**, $-CH_2N(X-R^2)C(=O)O-$**, $-C(=O)N(X-R^2)-$**, $-CH_2N(X-R^2)C(=O)-$**, $-C(=O)NR^b-$**, $-C(=O)NH-$**, $-CH_2NR^bC(=O)-$**, $-CH_2NR^bC(=O)NH-$**, $-CH_2NR^bC(=O)NR^b-$**, $-NHC(=O)-$**, $-NHC(=O)O-$**, $-NHC(=O)NH-$**
$-OC(=O)NH-$**, $-S(O)_2NH-$**, $-NHS(O)_2-$**, $-C(=O)-$, $-C(=O)O-$** or $-NH-$, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

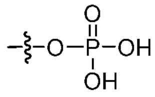

groups;
A is a bond, -OC(=O)-*,

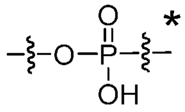 , 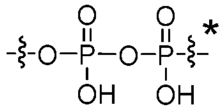 , 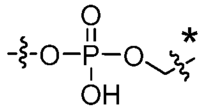 ,

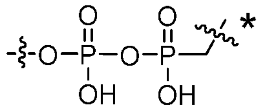 ,

$-OC(=O)N(CH_3)CH_2CH_2N(CH_3)C(=O)-$* or $-OC(=O)N(CH_3)C(R^a)_2C(R^a)_2N(CH_3)C(=O)-$*, wherein each $R^a$ is independently selected from H, $C_1$-$C_6$ alkyl, and $C_3$-$C_8$ cycloalkyl and the * of A indicates the point of attachment to D;
D is a Drug moiety as defined herein and comprising an N or an O, wherein D is connected to A via a direct bond from A to the N or the O of the Drug moiety,
and
y is 1, 2, 3, 4, 5, 6, 7, 8, 9, 10, 11, 12, 13, 14, 15 or 16.

**Embodiment 65.** The immunoconjugate of Formula (E') or any one of Embodiments 60 to 64, wherein:

Ab is an antibody or fragment thereof;
$R^{100}$ is

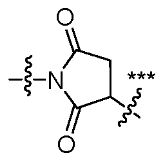 ,

where the *** of $R^{100}$ indicates the point of attachment to Ab;
$L_1$ is *$-C(=O)(CH_2)_mO(CH_2)_m-$**; *$-C(=O)((CH_2)_mO)t(CH_2)_n-$**; *$-C(=O)(CH_2)_m-$**; or *$-C(=0)NH((CH_2)_mO),(CH_2)_n-$, where the * of $L_1$ indicates the point of attachment to Lp, and the ** of $L_1$ indicates the point of attachment to $R^{100}$;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

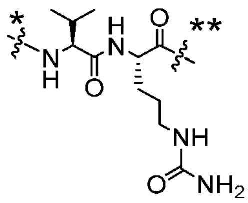 (ValCit),

where the * of Lp indicates the attachment point to $L_1$ and the ** of Lp indicates the attachment point to the -NH- group of G;
$L_3$ is a spacer moiety having the structure

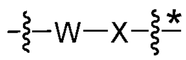
,

where

W is $-CH_2O-$**, $-CH_2N(R^b)C(=O)O-$**, -NHC(=O)$CH_2$NHC(=O)O-**, $-CH_2N(X-R^2)C(=O)O-$**, -C(=O)N(X-$R^2$)-**, $-CH_2N(X-R^2)C(=O)-$**, -C(=O)$NR^b$-**, -C(=O)NH-**, $-CH_2NR^bC(=O)-$**, - $CH_2NR^bC(=O)NH-$**, $-CH_2NR^bC(=O)NR^b-$**, -NHC(=O)-**, - NHC(=O)O-**, -NHC(=O)NH-**, -OC(=O)NH-**, $-S(O)_2NH-$**, - $NHS(O)_2-$**, -C(=O)-, -C(=O)O-** or -NH-, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and
the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3 A is a bond, -OC(=O)-*

, 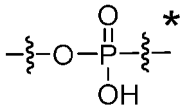 , 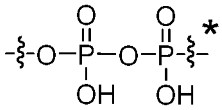 , 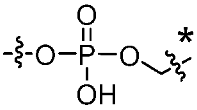 ,

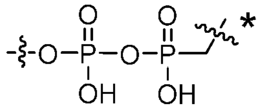 ,

$-OC(=O)N(CH_3)CH_2CH_2N(CH_3)C(=O)-$* or $-OC(=O)N(CH_3)C(R^a)_2C(R^a)_2N(CH_3)C(=O)-$*, wherein each $R^a$ is independently selected from H, $C_1$-$C_6$ alkyl, and $C_3$-$C_8$ cycloalkyl and the * of A indicates the point of attachment to D;
D is a Drug moiety as defined herein and comprising an N or an O, wherein D is connected to A via a direct bond from A to the N or the O of the Drug moiety,
and
y is 1, 2, 3, 4, 5, 6, 7, 8, 9, 10, 11, 12, 13, 14, 15 or 16.

**Embodiment 66.** The immunoconjugate of Formula (E') or any one of Embodiments 60 to 65, wherein:

Ab is an antibody or fragment thereof;
$R^{100}$ is

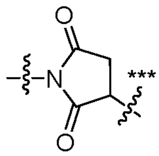
,

where the *** of $R^{100}$ indicates the point of attachment to Ab;
$L_1$ is *-C(=O)$(CH_2)_mO(CH_2)_m$-**; *-C(=O)$((CH_2)_mO)t(CH_2)_n$-**; *-C(=O)$(CH_2)_m$-**; or *-C(=0) NH$((CH_2)_mO),(CH_2)_n$-, where the * of $L_1$ indicates the point of attachment to Lp and the ** of $L_1$ indicates the point of attachment to $R^{100}$;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from

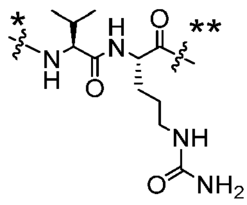 (ValCit),

where the * of Lp indicates the attachment point to $L_1$ and the ** of Lp indicates the attachment point to the -NH- group of G;

$L_3$ is a spacer moiety having the structure

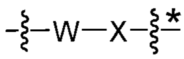 ,

where

W is $-CH_2O$-**, $-CH_2N(R^b)C(=O)O$-**, $-NHC(=O)CH_2NHC(=O)O$-**, $-CH_2N(X-R^2)C(=O)O$-**, $-C(=O)N(X-R^2)$-**, $-C(=O)NR^b$-**, -C(=O)NH-**, $-CH_2NR^bC(=O)$-**, $-CH_2NR^bC(=O)NH$-**, $-CH_2NR^bC(=O)NR^b$-**, -NHC(=O)-**, -NHC(=O)O-**, or - NHC(=O)NH-**, wherein each $R^b$ is independently selected from H, $C_1$-$C_6$alkyl or $C_3$-$C_8$cycloalkyl and wherein the ** of W indicates the point of attachment to X;

X is a bond, triazolyl or ***-$CH_2$-triazolyl-*, wherein the *** of X indicates the point of attachment to W and the * of X indicates the point of attachment to $R^2$;

and

the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

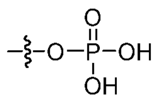

groups;

A is a bond or -OC(=O)* in which * indicates the attachment point to D;

D is a Drug moiety as defined herein and comprising an N or an O, wherein D is connected to A via a direct bond from A to the N or the O of the Drug moiety,

and

y is 1, 2, 3, 4, 5, 6, 7, 8, 9, 10, 11, 12, 13, 14, 15 or 16.

**Embodiment 67.** The immunoconjugate of Formula (E') or any one of Embodiments 60 to 66, wherein:

Ab is an antibody or fragment thereof;

$R^{100}$ is

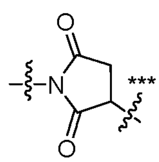 ,

where the *** of $R^{100}$ indicates the point of attachment to Ab;

$L_1$ is *-$C(=O)(CH_2)_mO(CH_2)_m$-**; *-$C(=O)((CH_2)_mO)_t(CH_2)_n$-**; *-$C(=O)(CH_2)_m$-**; or *-$C(=O)NH((CH_2)_mO)_t(CH_2)_n$-, where the * of $L_1$ indicates the point of attachment to Lp and the ** of $L_1$ indicates the point of attachment to $R^{100}$;

each m is independently selected from 1, 2, 3, 4, 5, 6, 7, 8, 9 and 10;

each n is independently selected from 1, 2, 3, 4, 5, 6, 7, 8, 9 and 10;

each t is independently selected from 2, 3, 4, 5, 6, 7, 8, 9, 10, 11, 12, 13, 14, 15, 16, 17, 18, 19, 20, 21, 22, 23, 24, 25, 26, 27, 28, 29 and 30;

Lp is a bivalent peptide spacer selected from

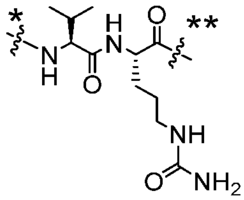 (ValCit),

where the * of Lp indicates the attachment point to $L_1$ and the ** of Lp indicates the attachment point to the -NH- group of G;

$L_3$ is a spacer moiety having the structure

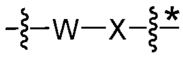 ,

where

W is $-CH_2O-$**, $-CH_2N(R^b)C(=O)O-$**, $-NHC(=O)CH_2NHC(=O)O-$**, $-CH_2N(X-R^2)C(=O)O-$**, or $-C(=O)N(X-R^2)-$**, wherein each $R^b$ is independently selected from H, $C_1$-$C_6$alkyl or $C_3$-$C_8$cycloalkyl and wherein the ** of W indicates the point of attachment to X;

X is ***-$CH_2$-triazolyl-*, wherein the *** of X indicates the point of attachment to W and the * of X indicates the point of attachment to $R^2$;

and

the * of $L_3$ indicates the point of attachment to $R^2$;

$R^2$ is a hydrophilic moiety selected from polyethylene glycol, polyalkylene glycol, a sugar, an oligosaccharide, a polypeptide or $C_2$-$C_6$alkyl substituted with 1 to 3

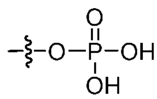

groups;

A is a bond or -OC(=O)* in which * indicates the attachment point to D;

D is a Drug moiety as defined herein and comprising an N or an O, wherein D is connected to A via a direct bond from A to the N or the O of the Drug moiety,

and

y is 1, 2, 3, 4, 5, 6, 7, 8, 9, 10, 11, 12, 13, 14, 15 or 16.

**Embodiment 68.** The immunoconjugate of Formula (E') or any one of Embodiments 60 to 63, wherein $R^{100}$ is

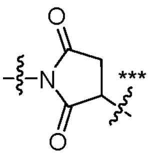 , 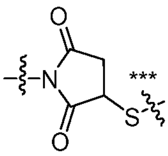 , 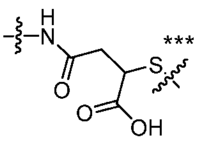 , 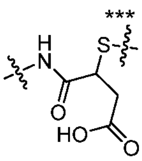 , 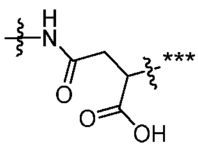 ,

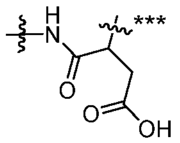, 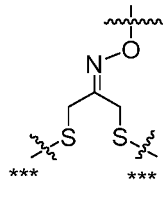, 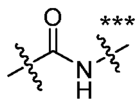,

-S-, -C(=O)-, -ON=***, -NHC(=O)$CH_2$-***, -S(=O)$_2$$CH_2CH_2$-***, -$(CH_2)_2$S(=O)$_2$$CH_2CH_2$-***, -NHS(=O)$_2$$CH_2CH_2$-***, -NHC(=O)$CH_2CH_2$-***, -$CH_2$NH$CH_2CH_2$-***, -NH$CH_2CH_2$-***,

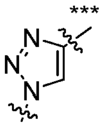, 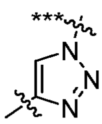,

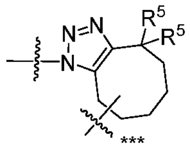, 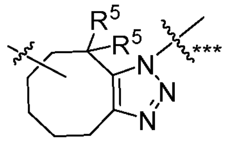, 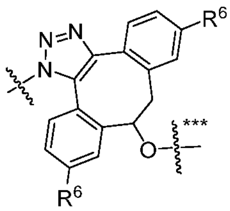,

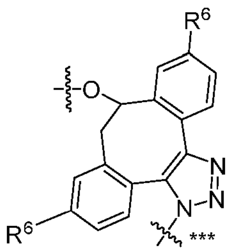, 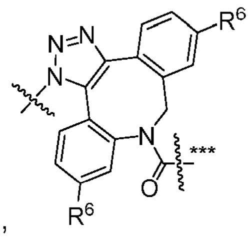, 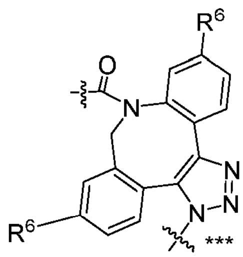, 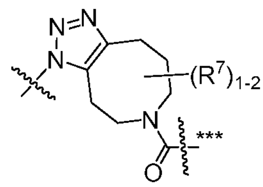,

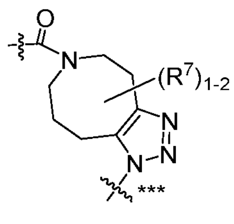 or 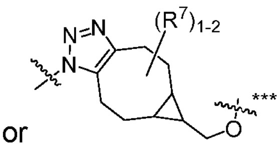,

where the *** of $R^{100}$ indicates the point of attachment to Ab.

**Embodiment 69.** The immunoconjugate of Formula (E') or any one of Embodiments 60 to 63, wherein $R^{100}$ is

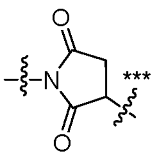, 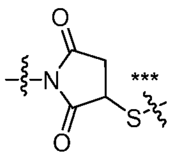, 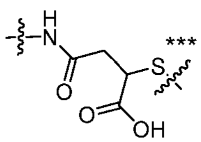, 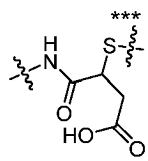, 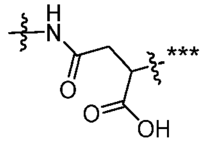,

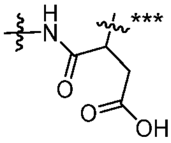, 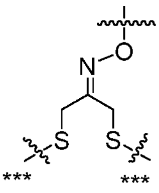 or 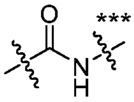,

where the *** of $R^{100}$ indicates the point of attachment to Ab.

**Embodiment 70.** The immunoconjugate of Formula (E') or any one of Embodiments 60 to 63, wherein $R^{100}$ is

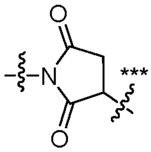, 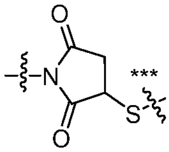, 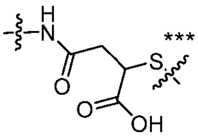, 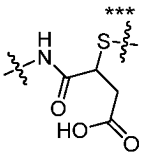, 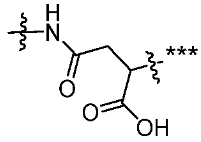,

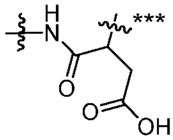, or 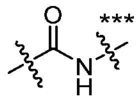

, where the *** of $R^{100}$ indicates the point of attachment to Ab.

**Embodiment 71.** The immunoconjugate of Formula (E') or any one of Embodiments 60 to 70 having the structure:

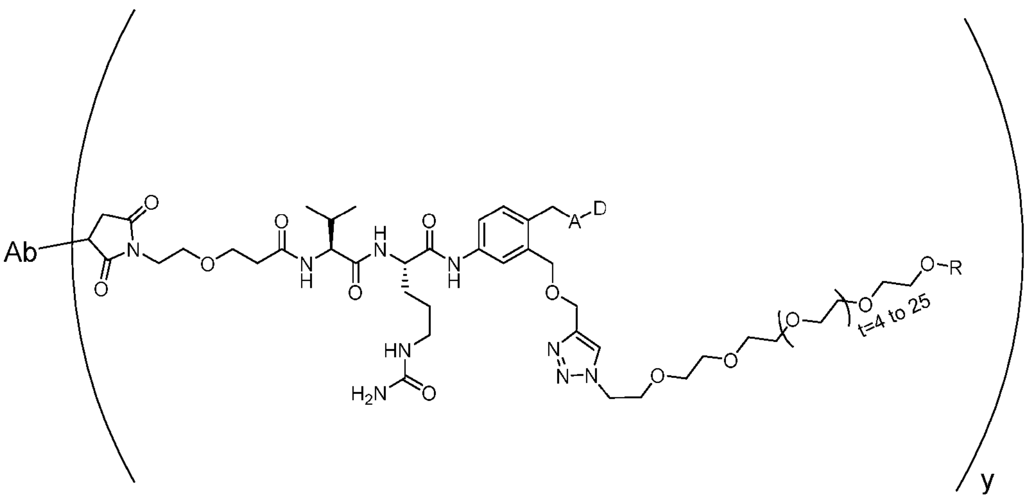,

where

R is H, $-CH_3$ or $-CH_2CH_2C(=O)OH$ and y is 1, 2, 3, 4, 5, 6, 7, 8, 9, 10, 11, 12, 13, 14, 15 or 16.

**Embodiment 72.** The immunoconjugate of Formula (E') or any one of Embodiments 60 to 70 having the structure:

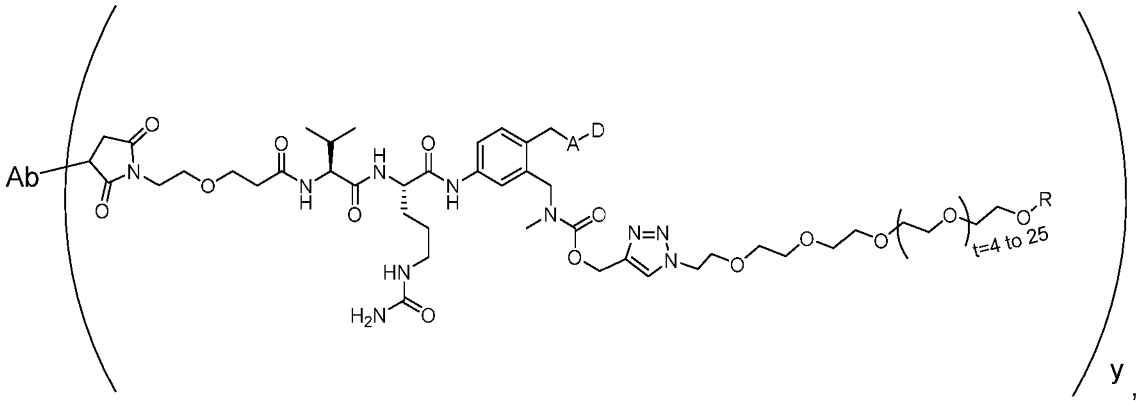

where

R is H, $-CH_3$ or $-CH_2CH_2C(=O)OH$ and y is 1, 2, 3, 4, 5, 6, 7, 8, 9, 10, 11, 12, 13, 14, 15 or 16.

**Embodiment 73.** The immunoconjugate of Formula (E') or any one of Embodiments 60 to 70 having the structure:

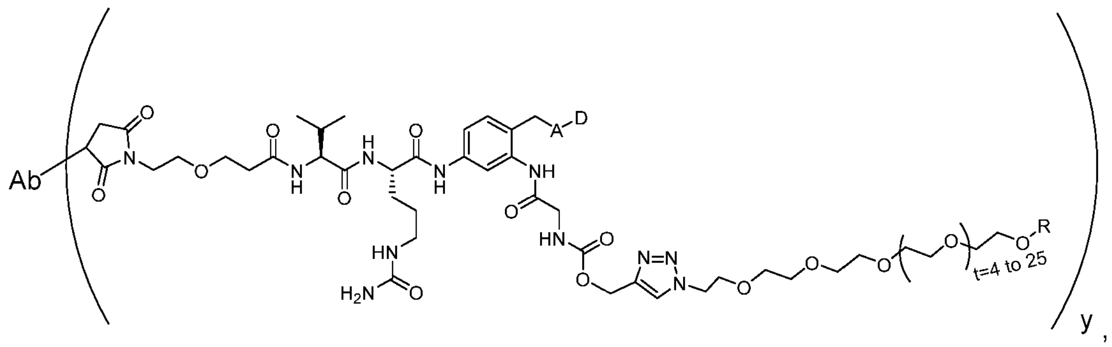

where

R is H, $-CH_3$ or $-CH_2CH_2C(=O)OH$ and y is 1, 2, 3, 4, 5, 6, 7, 8, 9, 10, 11, 12, 13, 14, 15 or 16.

**Embodiment** 74. The immunoconjugate of Formula (E') or any one of Embodiments 60 to 70 having the structure:

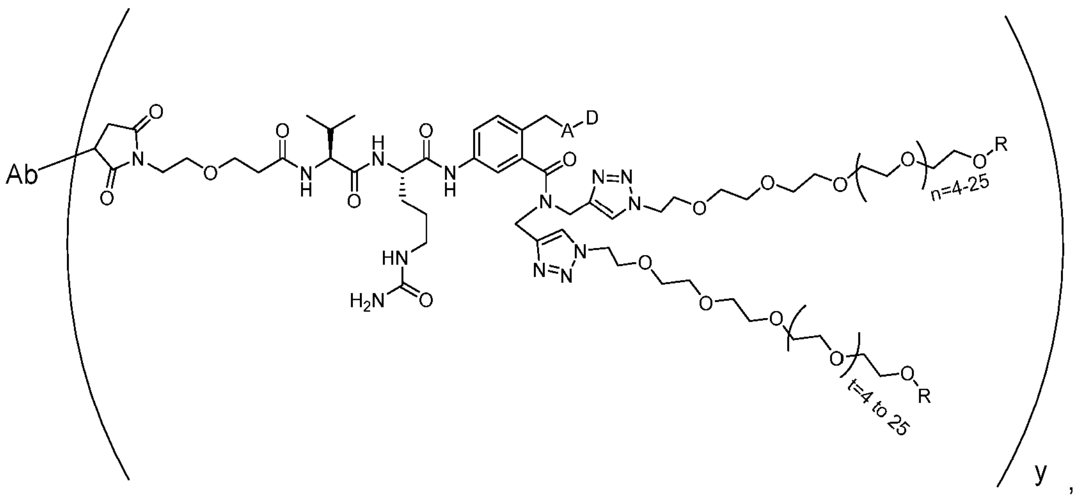

where

each R is independently selected from H, $-CH_3$ or $-CH_2CH_2C(=O)OH$ and y is 1, 2, 3, 4, 5, 6, 7, 8, 9, 10, 11, 12, 13, 14, 15 or 16.

**Embodiment 75.** The immunoconjugate of Formula (E') or any one of Embodiments 60 to 70 having the structure:

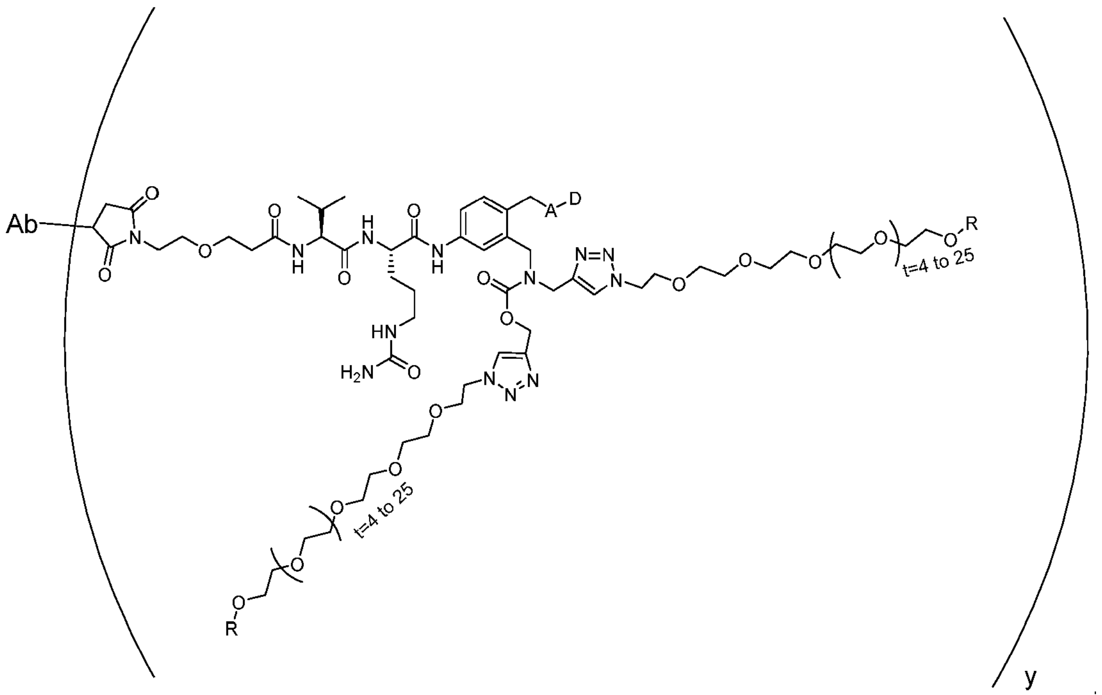

where

each R is independently selected from H, $-CH_3$ or $-CH_2CH_2C(=O)OH$ and y is 1, 2, 3, 4, 5, 6, 7, 8, 9, 10, 11, 12, 13, 14, 15 or 16.

**Embodiment 76.** The immunoconjugate of Formula (E') or any one of Embodiments 60 to 70 having the structure:

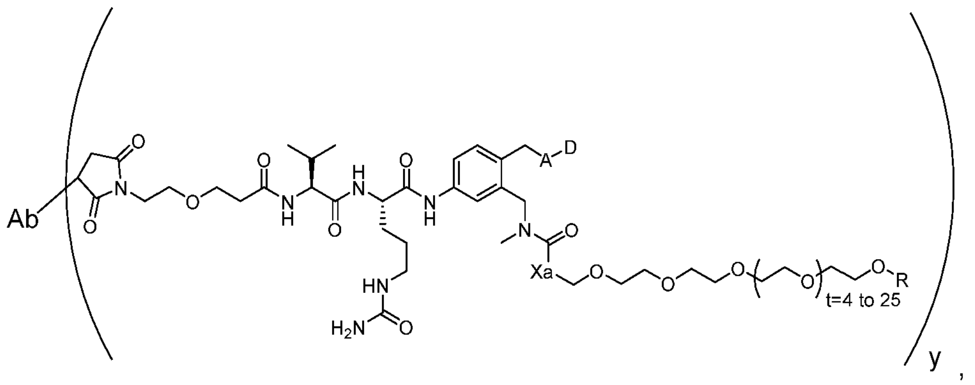

where

Xa is $-CH_2-$, $-OCH_2-$, $-NHCH_2-$ or $-NRCH_2-$ and each R is independently H, $-CH_3$ or $-CH_2CH_2C(=O)OH$ and y is 1, 2, 3, 4, 5, 6, 7, 8, 9, 10, 11, 12, 13, 14, 15 or 16.

**Embodiment 77.** The immunoconjugate of Formula (E') or any one of Embodiments 60 to 70 having the structure:

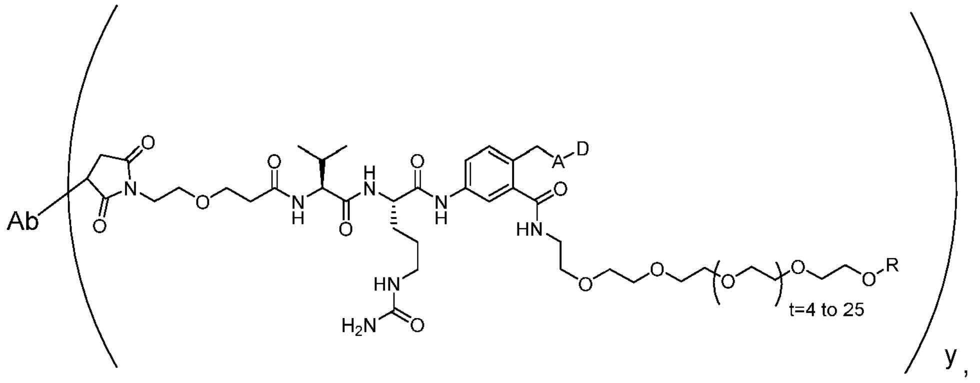

where

R is H, $-CH_3$ or $-CH_2CH_2C(=O)OH$ and y is 1, 2, 3, 4, 5, 6, 7, 8, 9, 10, 11, 12, 13, 14, 15 or 16.

**Embodiment 78.** The immunoconjugate of Formula (E') or any one of Embodiments 60 to 70 having the structure:

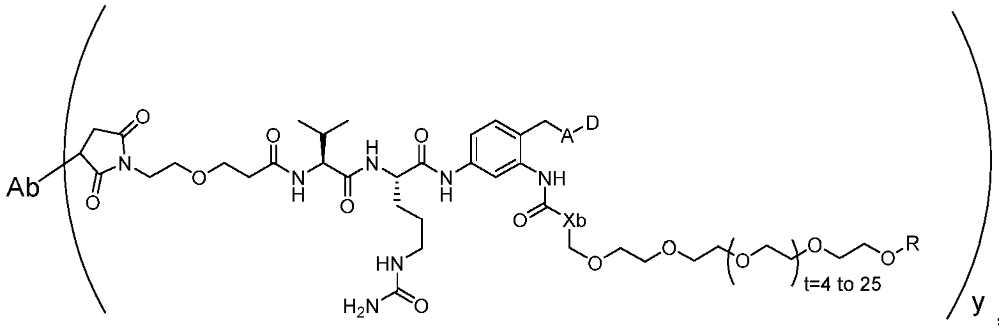

where

Xb is $-CH_2-$, $-OCH_2-$, $-NHCH_2-$ or $-NRCH_2-$ and each R independently is H, $-CH_3$ or $-CH_2CH_2C(=O)OH$ and y is 1, 2, 3, 4, 5, 6, 7, 8, 9, 10, 11, 12, 13, 14, 15 or 16.

**Embodiment 79.** The immunoconjugate of Formula (E') or any one of Embodiments 60 to 70 having the structure:

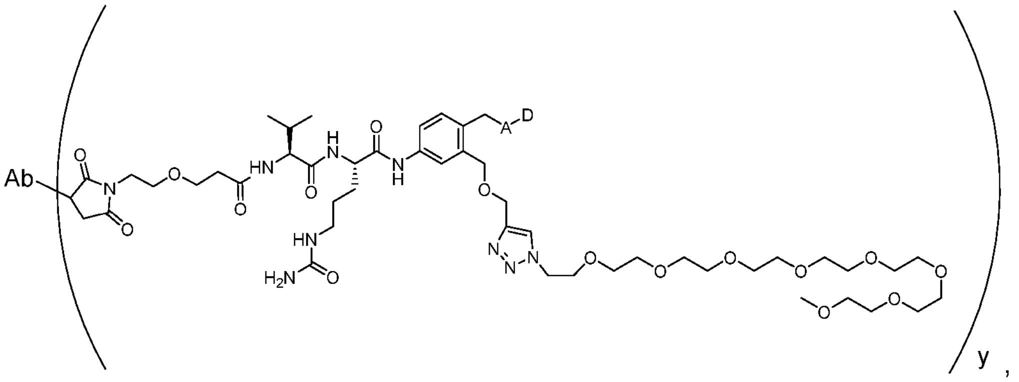

where y is 1, 2, 3, 4, 5, 6, 7, 8, 9, 10, 11, 12, 13, 14, 15 or 16.

**Embodiment 80.** The immunoconjugate of Formula (E') or any one of Embodiments 60 to 70 having the structure:

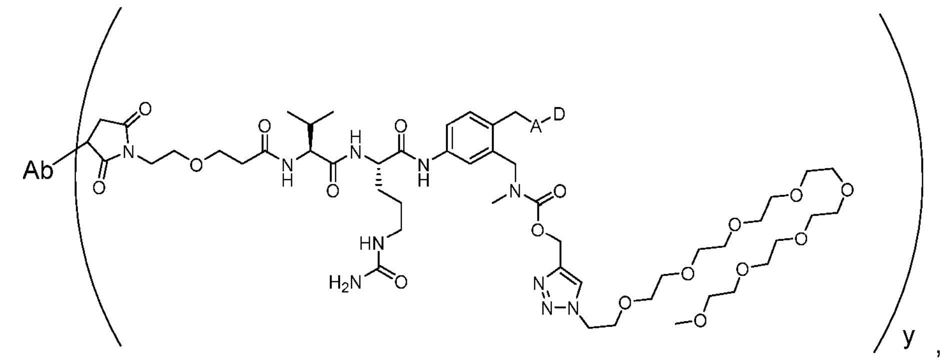

where
y is 1, 2, 3, 4, 5, 6, 7, 8, 9, 10, 11, 12, 13, 14, 15 or 16.

**Embodiment 81.** The immunoconjugate of Formula (E') or any one of Embodiments 60 to 70 having the structure:

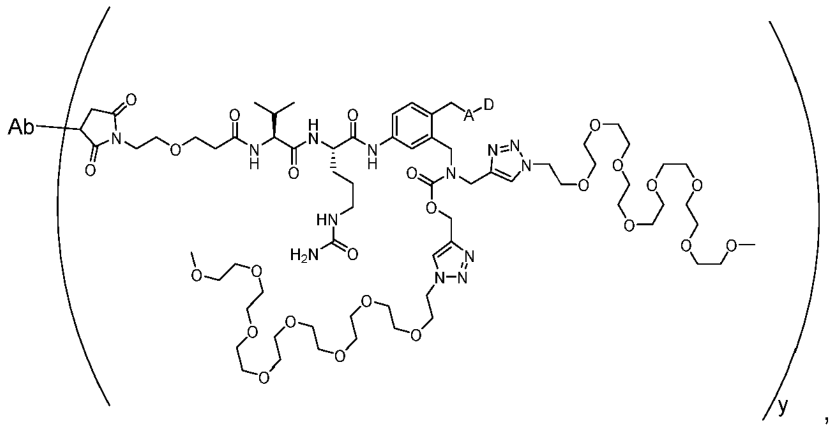

where y is 1, 2, 3, 4, 5, 6, 7, 8, 9, 10, 11, 12, 13, 14, 15 or 16.

**Embodiment 82.** The immunoconjugate of Formula (E') or any one of Embodiments 60 to 70 having the structure:

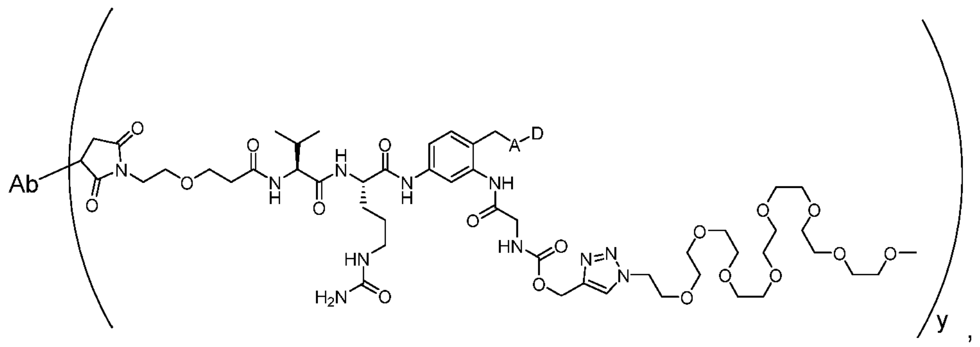

where y is 1, 2, 3, 4, 5, 6, 7, 8, 9, 10, 11, 12, 13, 14, 15 or 16.

**Embodiment 83.** The immunoconjugate of Formula (E') or any one of Embodiments 60 to 70 having the structure:

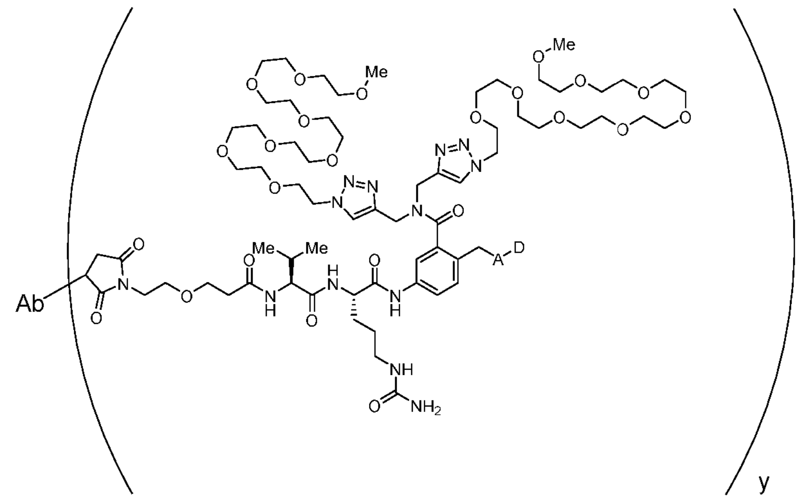
,

where y is 1, 2, 3, 4, 5, 6, 7, 8, 9, 10, 11, 12, 13, 14, 15 or 16.

Certain aspects and examples of the Linker-Drug groups, the Linkers and the Antibody Drug Conjugates of the invention are provided in the following listing of additional enumerated embodiments. It will be recognized that features specified in each embodiment may be combined with other specified features to provide further embodiments of the present invention.

**Embodiment 84.** The compound of Formula (A') or any one of Embodiments 1 to 2, or pharmaceutically acceptable salt thereof, the linker of Formula (C') or any one of Embodiments 32 to 39, and the immunoconjugate of Formula (E') or any one of Embodiments 60 to 61, wherein:

G is

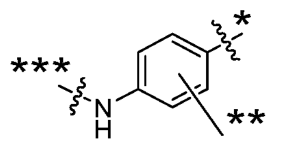
,

where the * of G indicates the point of attachment to $L_2$, and the ** of G indicates the point of attachment to $L_3$ and the *** of G indicates the point of attachment to Lp.

**Embodiment 85.** The compound of Formula (A') or any one of Embodiments 1 to 2, or pharmaceutically acceptable salt thereof, the linker of Formula (C') or any one of Embodiments 32 to 39, and the immunoconjugate of Formula (E') or any one of Embodiments 60 to 61, wherein:

G is

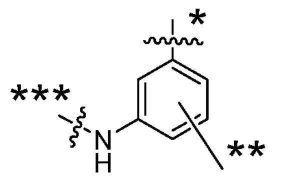
,

where the * of G indicates the point of attachment to $L_2$, and the ** of G indicates the point of attachment to $L_3$ and the *** of G indicates the point of attachment to Lp.

**Embodiment 86.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein:

$L_1$ is *-$C(=O)(CH_2)_mO(CH_2)_m$-**; *-$C(=O)((CH_2)_mO),(CH_2)_n$-**; *-$C(=O)(CH_2)_m$-**; *-$C(=O)NH((CH_2)_mO)_t(CH_2)_n$-**; *-$C(=O)O(CH_2)_mSSC(R^3)_2(CH_2)_mC(=O)NR^3(CH_2)_mNR^3C(=O)(CH_2)_m$-**; *-$C(=O)O(CH_2)_mC(=O)NH(CH_2)_m$-**; *-$C(=O)(CH_2)_mNH(CH_2)_m$-**; *-$C(=O)(CH_2)_mNH(CH_2)_nC(=O)$-**; *-$C(=O)(CH_2)_mX_1(CH_2)_m$-**; *-$C(=O)((CH_2)_mO)_t(CH_2)_nX_1(CH_2)_n$-**; *-$C(=O)(CH_2)_mNHC(=O)(CH_2)_n$-**; *-$C(=O)((CH_2)_mO)_t(CH_2)_nNHC(=O)(CH_2)_n$-**; *-$C(=O)(CH_2)_mNHC(=O)(CH_2)_nX_1(CH_2)_n$-**; *-$C(=O)((CH_2)_mO)t(CH_2)_nNHC(=O)(CH_2)_nX_1(CH_2)_n$-**; *-$C(=O)((CH_2)_mO)_t$

$(CH_2)_nC(=O)NH(CH_2)_m$-**; *-C(=O)$(CH_2)_mC(R^3)_2$-** or *-C(=O)$(CH_2)_m$C(=O)NH$(CH_2)_m$-**, where the * of $L_1$ indicates the point of attachment to Lp, and the ** of $L_1$ indicates the point of attachment to $R^1$ if present or the ** of $L_1$ indicates the point of attachment to $R^{100}$ if present.

**Embodiment 87.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein:

$L_1$ is *-C(=O)$(CH_2)_m$O$(CH_2)_m$-**; *-C(=O)$((CH_2)_mO)_t(CH_2)_n$-**; *-C(=O)$(CH_2)_m$-**; *-C(=O)NH$((CH_2)_mO)_t(CH_2)_n$-**; *-C(=O)$(CH_2)_m$NH$(CH_2)_m$-**; *-C(=O)$(CH_2)_m$NH$(CH_2)_n$C(=O)-**; *-C(=O)$(CH_2)_m$NHC(=O)$(CH_2)_n$-**; *-C(=O)$((CH_2)_m$O)t$(CH_2)_n$NHC(=O)$(CH_2)_n$-**; *-C(=O)$((CH_2)_m$O)t$(CH_2)_n$C(=O)NH$(CH_2)_m$-**; *-C(=O)$(CH_2)_mC(R^3)_2$-** or *-C(=O)$(CH_2)_m$C(=O)NH$(CH_2)_m$-**, where the * of $L_1$ indicates the point of attachment to Lp, and the ** of $L_1$ indicates the point of attachment to $R^1$ if present or the ** of $L_1$ indicates the point of attachment to $R^{100}$ if present.

**Embodiment 88.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein:

$L_1$ is *-C(=O)$(CH_2)_m$O$(CH_2)_m$-**; *-C(=O)$((CH_2)_mO)_t(CH_2)_n$-**; *-C(=O)$(CH_2)_m$-**; *-C(=O)NH$((CH_2)_mO)_t(CH_2)_n$-**; *-C(=O)$(CH_2)_m$NH$(CH_2)_m$-**; *-C(=O)$(CH_2)_m$NH$(CH_2)_n$C(=O)-**; or *-C(=O)$(CH_2)_m$NHC(=O)$(CH_2)_n$-**, where the * of $L_1$ indicates the point of attachment to Lp, and the ** of $L_1$ indicates the point of attachment to $R^1$ if present or the ** of $L_1$ indicates the point of attachment to $R^{100}$ if present.

**Embodiment 89.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein:

$L_1$ is *-C(=O)$(CH_2)_m$O$(CH_2)_m$-**; *-C(=O)$((CH_2)_mO)_t(CH_2)_n$-**; *-C(=O)$(CH_2)_m$-** or *-C(=O)NH$((CH_2)_mO)_t(CH_2)_n$-**, where the * of $L_1$ indicates the point of attachment to Lp, and the ** of $L_1$ indicates the point of attachment to $R^1$ if present or the ** of $L_1$ indicates the point of attachment to $R^{100}$ if present.

**Embodiment 90.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein $L_1$ is *-C(=O)$(CH_2)_m$O$(CH_2)_m$-**, where the * of $L_1$ indicates the point of attachment to Lp, and the ** of $L_1$ indicates the point of attachment to $R^1$ if present or the ** of $L_1$ indicates the point of attachment to $R^{100}$ if present.

**Embodiment 91.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein $L_1$ is *-C(=O)$((CH_2)_mO)_t(CH_2)_n$-**, where the * of $L_1$ indicates the point of attachment to Lp, and the ** of $L_1$ indicates the point of attachment to $R^1$ if present or the ** of $L_1$ indicates the point of attachment to $R^{100}$ if present.

**Embodiment 92.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein $L_1$ is *-C(=O)$(CH_2)_m$-**, where the * of $L_1$ indicates the point of attachment to Lp, and the ** of $L_1$ indicates the point of attachment to $R^1$ if present or the ** of $L_1$ indicates the point of attachment to $R^{100}$ if present.

**Embodiment 93.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein $L_1$ is *-C(=O)NH$((CH_2)_mO)_t(CH_2)_n$-**, where the * of $L_1$ indicates the point of attachment to Lp, and the ** of $L_1$ indicates the point of attachment to $R^1$ if present or the ** of $L_1$ indicates the point of attachment to $R^{100}$ if present.

**Embodiment 94.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 93, wherein Lp is an enzymatically cleavable bivalent peptide spacer.

**Embodiment 95.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 94, wherein Lp is a bivalent peptide spacer comprising an amino acid residue selected from glycine, valine, citrulline, lysine, isoleucine, phenylalanine, methionine, asparagine, proline, alanine, leucine, tryptophan, and tyrosine.

**Embodiment 96.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 95, wherein Lp is a bivalent peptide spacer comprising two to four amino acid residues.

**Embodiment 97.** The compound of Formula (A') or any one of Embodiments 1 to 17, or pharmaceutically acceptable

salt thereof, the linker of Formula (C') or any one of Embodiments 32 to 46, and the immunoconjugate of Formula (E') or any one of Embodiments 60 to 70, or any one of Embodiments 84 to 96, wherein Lp is a bivalent peptide spacer comprising two to four amino acid residues each independently selected from glycine, valine, citrulline, lysine, isoleucine, phenylalanine, methionine, asparagine, proline, alanine, leucine, tryptophan, and tyrosine.

**Embodiment 98.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 97, wherein:

Lp is a bivalent peptide spacer selected from

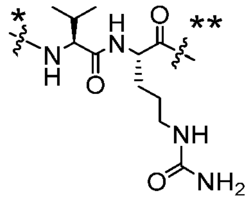 (ValCit),

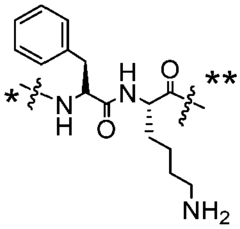 (PheLys), 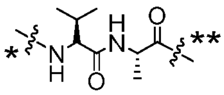 (ValAla), 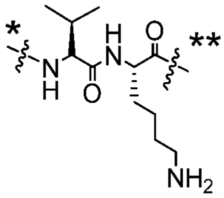 (ValLys)

and

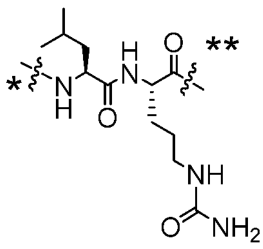 (LeuCit),

where the * of Lp indicates the attachment point to $L_1$ and the ** of Lp indicates the attachment point to the -NH- group of Formula (B') or the ** of Lp indicates the attachment point to the G of Formula (A').

**Embodiment 99.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:

Lp is

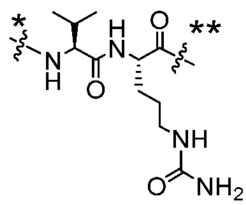 (ValCit),

where the * of Lp indicates the attachment point to $L_1$ and the ** of Lp indicates the attachment point to the -NH- group of Formula (B') or the ** of Lp indicates the attachment point to the G of Formula (A').

**Embodiment 100.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:

Lp is

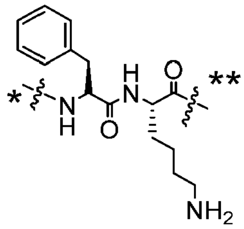

(PheLys),

where the * of Lp indicates the attachment point to $L_1$ and the ** of Lp indicates the attachment point to the -NH- group of Formula (B') or the ** of Lp indicates the attachment point to the G of Formula (A').

**Embodiment 101.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:
Lp is

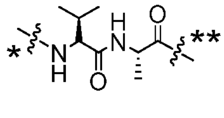

(ValAla),

where the * of Lp indicates the attachment point to $L_1$ and the ** of Lp indicates the attachment point to the -NH- group of Formula (B') or the ** of Lp indicates the attachment point to the G of Formula (A').

**Embodiment 102.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:
Lp is

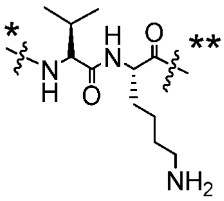

(ValLys),

where the * of Lp indicates the attachment point to $L_1$ and the ** of Lp indicates the attachment point to the -NH- group of Formula (B') or the ** of Lp indicates the attachment point to the G of Formula (A').

**Embodiment 103.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:
Lp is

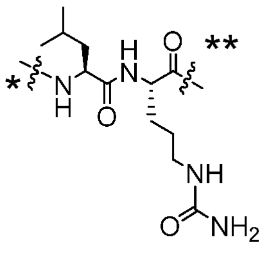

(LeuCit),

where the * of Lp indicates the attachment point to $L_1$ and the ** of Lp indicates the attachment point to -NH- group of Formula (B') or the ** of Lp indicates the attachment point to the G of Formula (A').

**Embodiment 104.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 103, wherein $L_2$ is a bond, a methylene, or a $C_2$-$C_3$alkenylene.

**Embodiment 105.** The compound of Formula (A') or any one of Embodiments 1 to 17, or pharmaceutically acceptable

salt thereof, the linker of Formula (C') or any one of Embodiments 32 to 46, and the immunoconjugate of Formula (E') or any one of Embodiments 60 to 70, or any one of Embodiments 84 to 104, wherein $L_2$ is a bond or a methylene.

**Embodiment 106.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 105, wherein $L_2$ is a bond.

**Embodiment 107.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 105, wherein $L_2$ is a methylene.

**Embodiment 108.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 107, wherein:

A is a bond, -OC(=O)-, $-OC(=O)N(CH_3)CH_2CH_2N(CH_3)C(=O)-$ or $-OC(=O)N(CH_3)C(R^a)_2C(R^a)_2N(CH_3)C(=O)-$, wherein each $R^a$ is independently selected from H, $C_1$-$C_6$alkyl or a $C_3$-$C_8$cycloalkyl.

**Embodiment 109.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 107, wherein A is a bond or - OC(=O).

**Embodiment 110.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 109, wherein A is a bond.

**Embodiment 111.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 109, wherein A is -OC(=O).

**Embodiment 112.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 107, wherein:

A is

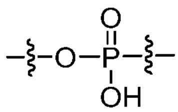 , 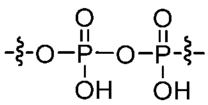 , 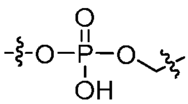 or 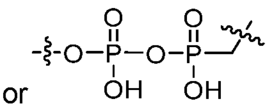 .

**Embodiment 113.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 107, wherein:

A is $-OC(=O)N(CH_3)CH_2CH_2N(CH_3)C(=O)-$ or$-OC(=O)N(CH_3)C(R^a)_2C(R^a)_2N(CH_3)C(=O)-$, wherein each $R^a$ is independently selected from H, $C_1$-$C_6$alkyl or a $C_3$-$C_8$cycloalkyl.

**Embodiment 114.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 113, wherein:

$L_3$ is a spacer moiety having the structure

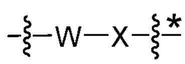 ,

where

W is $-CH_2O-$**, $-CH_2N(R^b)C(=O)O-$**, $-NHC(=O)C(R^b)_2NHC(=O)O-$**, $-NHC(=O)C(R^b)_2NH-$**, $-NHC(=O)C(R^b)_2NHC(=O)-$**, $-CH_2N(X-R^2)C(=O)O-$**, $-C(=O)N(X-R^2)-$**, $-CH_2N(X-R^2)C(=O)-$**, $-C(=O)NR^b-$**, -C(=O)NH-**, $-CH_2NR^bC(=O)-$**, $-CH_2NR^bC(=O)NH-$**, $-CH_2NR^bC(=O)NR^b-$**, -NHC(=O)-**, -NHC(=O)O-**, -NHC(=O)NH-**, -OC(=O)NH-**, $-S(O)_2NH-$**, $-NHS(O)_2-$**, -C(=O)-, -C(=O)O-** or -NH-, wherein each $R^b$ is independently selected from H, $C_1$-$C_6$alkyl or $C_3$-$C_8$cycloalkyl and wherein the ** of W indicates the point of attachment to X;

X is a bond, triazolyl or ***-$CH_2$-triazolyl-*, wherein the *** of X indicates the point of attachment to W and the * of X indicates the point of attachment to $R^2$;

and

the * of $L_3$ indicates the point of attachment to $R^2$.

**Embodiment 115.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 114, wherein:

$L_3$ is a spacer moiety having the structure

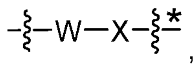

where

W is $-CH_2O-$**, $-CH_2N(R^b)C(=O)O-$**, $-NHC(=O)CH_2NHC(=O)O-$**, $-NHC(=O)CH_2NH-$**, $-NHC(=O)CH_2NHC(=O)-$**, $-CH_2N(X-R^2)C(=O)O-$**, $-C(=O)N(X-R^2)-$**, $-CH_2N(X-R^2)C(=O)-$**, $-C(=O)NR^b-$**, $-C(=O)NH-$**, $-CH_2NR^bC(=O)-$**, $-CH_2NR^bC(=O)NH-$**, $-CH_2NR^bC(=O)NR^b-$**, $-NHC(=O)-$**, $-NHC(=O)O-$**, $-NHC(=O)NH-$**, $-OC(=O)NH-$**, $-S(O)_2NH-$**, $-NHS(O)_2-$**, $-C(=O)-$, $-C(=O)O-$** or $-NH-$, wherein each $R^b$ is independently selected from H, $C_1$-$C_6$alkyl or $C_3$-$C_8$cycloalkyl and wherein the ** of W indicates the point of attachment to X;
X is a bond;
and

the * of $L_3$ indicates the point of attachment to $R^2$.

**Embodiment 116.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:

$L_3$ is a spacer moiety having the structure

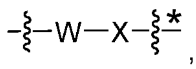

where

W is $-CH_2O-$**, $-CH_2N(R^b)C(=O)O-$**, $-NHC(=O)CH_2NHC(=O)O-$**, $-NHC(=O)CH_2NH-$**, $-NHC(=O)CH_2NHC(=O)-$**, $-CH_2N(X-R^2)C(=O)O-$**, $-C(=O)N(X-R^2)-$**, $-CH_2N(X-R^2)C(=O)-$**, $-C(=O)NR^b-$**, $-C(=O)NH-$**, - $CH_2NR^bC(=O)-$**, $-CH_2NR^bC(=O)NH-$**, $-CH_2NR^bC(=O)NR^b-$**, $-NHC(=O)-$**, $-NHC(=O)O-$**, $-NHC(=O)NH-$**, $-OC(=O)NH-$**, $-S(O)_2NH-$**, $-NHS(O)_2-$**, $-C(=O)-$, $-C(=O)O-$** or $-NH-$, wherein each $R^b$ is independently selected from H, $C_1$-$C_6$alkyl or $C_3$-$C_8$cycloalkyl and wherein the ** of W indicates the point of attachment to X;
X is a triazolyl, wherein the *** of X indicates the point of attachment to W and the * of X indicates the point of attachment to $R^2$;
and

the * of $L_3$ indicates the point of attachment to $R^2$.

**Embodiment 117.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:

$L_3$ is a spacer moiety having the structure

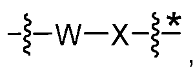

where

W is -$CH_2$O-**, -$CH_2$N($R^b$)C(=O)O-**, -NHC(=O)$CH_2$NHC(=O)O-**, -NHC(=O)$CH_2$NH-**, -NHC(=O)$CH_2$NHC(=O)-**, -$CH_2$N(X-$R^2$)C(=O)O-**, -C(=O)N(X-$R^2$)-**, -$CH_2$N(X-$R^2$)C(=O)-**, -C(=O)N$R^b$-**, -C(=O)NH-**, -$CH_2$N$R^b$C(=O)-**, -$CH_2$N$R^b$C(=O)NH-**, -$CH_2$N$R^b$C(=O)N$R^b$-**, -NHC(=O)-**, -NHC(=O)O-**, -NHC(=O)NH-**, -OC(=O)NH-**, -S(O)$_2$NH-**, -NHS(O)$_2$-**, -C(=O)-, -C(=O)O-** or -NH-, wherein each $R^b$ is independently selected from H, $C_1$-$C_6$alkyl or $C_3$-$C_8$cycloalkyl and wherein the ** of W indicates the point of attachment to X;
X is ***-$CH_2$-triazolyl-*, wherein the *** of X indicates the point of attachment to W and the * of X indicates the point of attachment to $R^2$;
and

the * of $L_3$ indicates the point of attachment to $R^2$.

**Embodiment 118.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:

$L_3$ is a spacer moiety having the structure

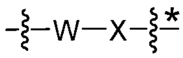
,

where

W is -$CH_2$O-**, -$CH_2$N($R^b$)C(=O)O-**, -NHC(=O)$CH_2$NHC(=O)O-**, -$CH_2$N(X-$R^2$)C(=O)O_**, -C(=O)N(X-$R^2$)-**, wherein each $R^b$ is independently selected from H, $C_1$-$C_6$alkyl or $C_3$-$C_8$cycloalkyl and wherein the ** of W indicates the point of attachment to X;
X is a bond, triazolyl or ***-$CH_2$-triazolyl-*, wherein the *** of X indicates the point of attachment to W and the * of X indicates the point of attachment to $R^2$;
and

the * of $L_3$ indicates the point of attachment to $R^2$.

**Embodiment 119.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:

$L_3$ is a spacer moiety having the structure

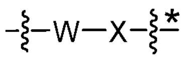
,

where

W is -$CH_2$O-**, -$CH_2$N($R^b$)C(=O)O-**, -NHC(=O)$CH_2$NHC(=O)O-**, -$CH_2$N(X-$R^2$)C(=O)O-**, -C(=O)N(X-$R^2$)-**, wherein each $R^b$ is independently selected from H, $C_1$-$C_6$alkyl or $C_3$-$C_8$cycloalkyl and wherein the ** of W indicates the point of attachment to X;
X is a bond;

and
the * of $L_3$ indicates the point of attachment to $R^2$.

**Embodiment 120.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:

$L_3$ is a spacer moiety having the structure

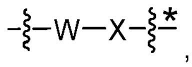

where

W is $-CH_2O-$**, $-CH_2N(R^b)C(=O)O-$**, $-NHC(=O)CH_2NHC(=O)O-$**, $-CH_2N(X-R^2)C(=O)O_$**, $-C(=O)N(X-R^2)-$**, wherein each $R^b$ is independently selected from H, $C_1$-$C_6$alkyl or $C_3$-$C_8$cycloalkyl and wherein the ** of W indicates the point of attachment to X;
X is a triazolyl, wherein the *** of X indicates the point of attachment to W and the * of X indicates the point of attachment to $R^2$;
and

the * of $L_3$ indicates the point of attachment to $R^2$.

**Embodiment 121.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:

$L_3$ is a spacer moiety having the structure

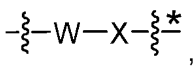

where

W is $-CH_2O-$**, $-CH_2N(R^b)C(=O)O-$**, $-NHC(=O)CH_2NHC(=O)O-$**, $-CH_2N(X-R^2)C(=O)O_$**, $-C(=O)N(X-R^2)-$**, wherein each $R^b$ is independently selected from H, $C_1$-$C_6$alkyl or $C_3$-$C_8$cycloalkyl and wherein the ** of W indicates the point of attachment to X;
X is ***-$CH_2$-triazolyl-*, wherein the *** of X indicates the point of attachment to W and the * of X indicates the point of attachment to $R^2$;
and

the * of $L_3$ indicates the point of attachment to $R^2$.

**Embodiment 122.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 121, wherein $R^2$ is a hydrophilic moiety selected from polyethylene glycol, polyalkylene glycol, a sugar, an oligosaccharide, a polypeptide or $C_2$-$C_6$alkyl substituted with 1 to 3

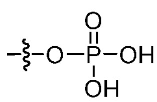

groups..

**Embodiment 123.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is a sugar.

**Embodiment 124.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is an oligosaccharide.

**Embodiment 125.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is a polypeptide.

**Embodiment 126.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is a polyalkylene glycol.

**Embodiment 127.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is a polyalkylene glycol having the structure $-(O(CH_2)_m)_tR'$, where R' is OH, $OCH_3$ or $OCH_2CH_2C(=O)OH$, m is 1-10 and t is 4-40.

**Embodiment 128.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is a polyalkylene glycol having the structure $-((CH_2)_mO)_tR''-$, where R" is H, $CH_3$ or $CH_2CH_2C(=O)OH$, m is 1-10 and t is 4-40.

**Embodiment 129.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is a polyethylene glycol.

**Embodiment 130.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is a polyethylene glycol having the structure $-(OCH_2CH_2)_tR'$, where R' is OH, $OCH_3$ or $OCH_2CH_2C(=O)OH$ and t is 4-40,

**Embodiment 131.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein $R^2$ is a polyethylene glycol having the structure $-(CH_2CH_2O)_tR''-$, where R" is H, $CH_3$ or $CH_2CH_2C(=O)OH$ and t is 4-40.

**Embodiment 132.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein:

$R^2$ is

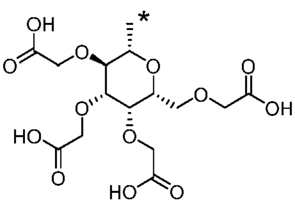 , 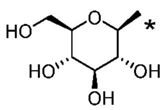 , 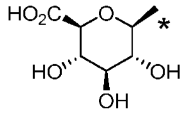 , 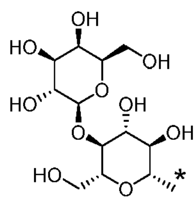 ,

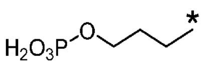 , 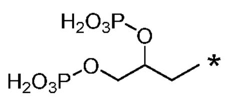 , 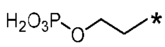 , 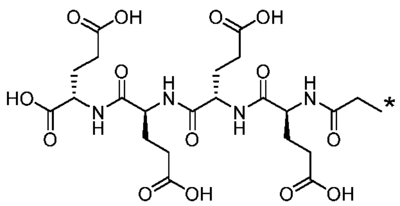

or

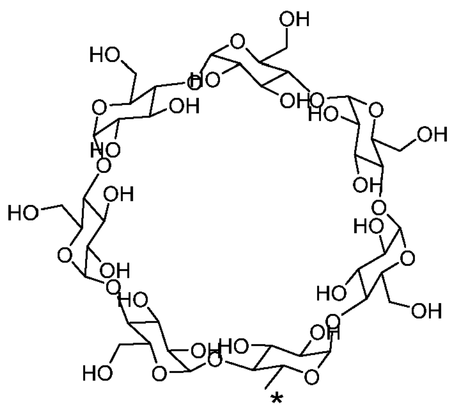 ,

where the * of $R^2$ indicates the point of attachment to X or $L_3$.

**Embodiment 133.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein:
$R^2$ is

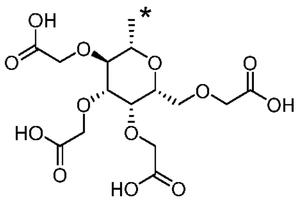 , 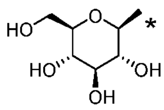 , 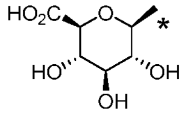 , 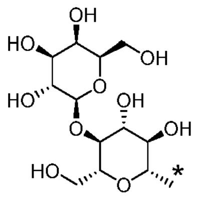

or

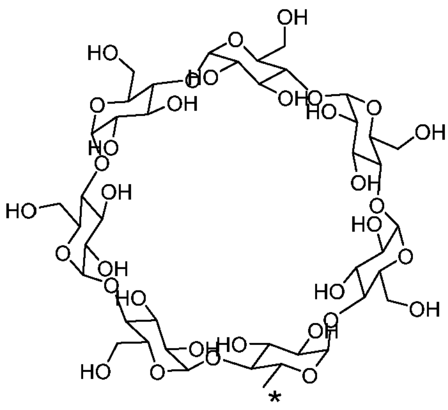

,

where the * of $R^2$ indicates the point of attachment to X or $L_3$.
**Embodiment 134.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein:

$R^2$ is 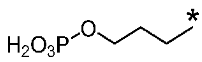 , 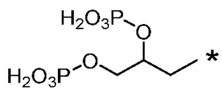 or 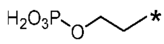 ,

where the * of $R^2$ indicates the point of attachment to X or $L_3$.
**Embodiment 135.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein:
$R^2$ is

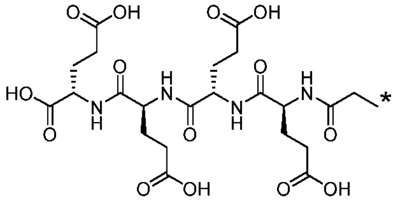 , \

where the * of $R^2$ indicates the point of attachment to X or $L_3$.
**Embodiment 136.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 135, wherein:
$X_1$ is

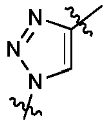, 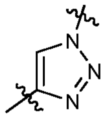, 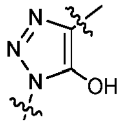 or 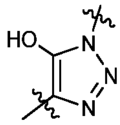.

**Embodiment** 137. The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 135, wherein:
$X_1$ is

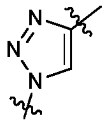 or 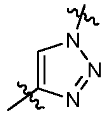.

**Embodiment 138.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 137, wherein:
each m is independently selected from 1, 2, 3, 4, and 5.

**Embodiment 139.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 137, wherein:
each m is independently selected from 1, 2 and 3.

**Embodiment 140.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 139, wherein:
each n is independently selected from 1, 2, 3, 4 and 5.

**Embodiment 141.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 139, wherein:
each n is independently selected from 1, 2 and 3.

**Embodiment 142.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 141, wherein:
each t is independently selected from 2, 3, 4, 5, 6, 7, 8, 9, 10, 11, 12, 13, 14, 15, 16, 17, 18, 19, 20, 21, 22, 23, 24, 25, 26, 27, 28, 29 and 30.

**Embodiment 143.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 141, wherein:
each t is independently selected from 4, 5, 6, 7, 8, 9, 10, 11, 12, 13, 14, 15, 16, 17, 18, 19, 20, 21, 22, 23, 24 or 25.

**Embodiment 144.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 141, wherein:
each t is independently selected from 1, 2, 3, 4, 5, 6, 7, 8, 9, 10, 11, 12, 13, 14, 15, 16, 17 and 18.

**Embodiment 145.** The immunoconjugate of Formula (E') or any one of Embodiments 60 to 70, or any one of Embodiments 84 to 144, wherein y is 1, 2, 3, 4, 5, 6, 7, 8, 9, 10, 11, 12, 13, or 14.

**Embodiment 146.** The immunoconjugate of Formula (E') or any one of Embodiments 60 to 70, or any one of Embodiments 84 to 144, wherein y is 1, 2, 3, **4,** 5, 6, 7, 8, 9, 10, 11 or 12.

**Embodiment 147.** The immunoconjugate of Formula (E') or any one of Embodiments 60 to 70, or any one of Embodiments 84 to 144, wherein y is 1, 2, 3, 4, 5, 6, 7, 8, 9 or 10.

**Embodiment 148.** The immunoconjugate of Formula (E') or any one of Embodiments 60 to 70, or any one of Embodiments 84 to 144, wherein y is 1, 2, 3, 4, 5, 6, 7 or 8.

**Embodiment 149.** The immunoconjugate of Formula (E') or any one of Embodiments 60 to 70, or any one of Embodiments 84 to 144, wherein y is 1, 2, 3, 4, 5 or 6.

**Embodiment 150.** The immunoconjugate of Formula (E') or any one of Embodiments 60 to 70, or any one of Embodiments 84 to 144, wherein y is 1, 2, 3 or 4.

**Embodiment 151.** The immunoconjugate of Formula (E') or any one of Embodiments 60 to 70, or any one of

Embodiments 84 to 144, wherein y is 1 or 2.

**Embodiment 152.** The immunoconjugate of Formula (E') or any one of Embodiments 60 to 70, or any one of Embodiments 84 to 144, wherein y is 2.

**Embodiment 153.** The immunoconjugate of Formula (E') or any one of Embodiments 60 to 70, or any one of Embodiments 84 to 144, wherein y is 4.

**Embodiment 154.** The immunoconjugate of Formula (E') or any one of Embodiments 60 to 70, or any one of Embodiments 84 to 144, wherein y is 6.

**Embodiment 155.** The immunoconjugate of Formula (E') or any one of Embodiments 60 to 70, or any one of Embodiments 84 to 144, wherein y is 8.

**Embodiment 156.** The compound of Formula (A') or any one of Embodiments 1 to 30, or pharmaceutically acceptable salt thereof, the immunoconjugate of Formula (E') or any one of Embodiments 60 to 70, or any one of Embodiments 84 to 155, wherein D is a MCl-1 inhibitor when released from the immunoconjugates.

Other Linker Groups

Other examples of linker groups that are suitable for making ADCs or immunoconjugates of a MCl-1 inhibitor disclosed herein includes those disclosed in international application publications such as WO2018200812, WO2017214456, WO2017214458, WO2017214462, WO2017214233, WO2017214282, WO2017214301, WO2017214322, WO2017214335, WO2017214339, WO2016094509, WO2016094517, and WO2016094505.

For example, the immunoconjugates of MCl-1 inhibitors disclosed herein can have a linker-payload ("-L-D") structure selected from:

$$\text{-}\xi\text{-}(L_c)_x\text{-}C_E\text{-}D,\quad \text{-}\xi\text{-}(L_c)_x\text{-}C_E\text{-}(L_c)_y\text{-}C_E\text{-}D,\ \text{and}\ \text{-}\xi\text{-}((L_c)_x\text{-}C_E)_p\text{-}(L_c)_y\text{-}C_E\text{-}D,$$

wherein:

Lc is a linker component and each Lc is independently selected from a linker component as disclosed herein;
x is an integer selected from 1, 2, 3, 4, 5, 6, 7, 8, 9, 10, 11, 12, 13, 14, 15, 16, 17, 18, 19 and 20;
y is an integer selected from 1, 2, 3, 4, 5, 6, 7, 8, 9, 10, 11, 12, 13, 14, 15, 16, 17, 18, 19 and 20;
p is an integer selected from 1, 2, 3, 4, 5, 6, 7, 8, 9 and 10;
D is a MCl-1 inhibitor disclosed herein;
and each cleavage element ($C_E$) is independently selected from a self-immolative spacer and a group that is susceptible to cleavage selected from acid-induced cleavage, peptidase-induced cleavage, esterase-induced cleavage, glycosidase induced cleavage, phosphodiesterase induced cleavage, phosphatase induced cleavage, protease induced cleavage, lipase induced cleavage or disulfide bond cleavage.

In some embodiments, L has a structure selected from the following, or L comprises a structural component selected from the following:

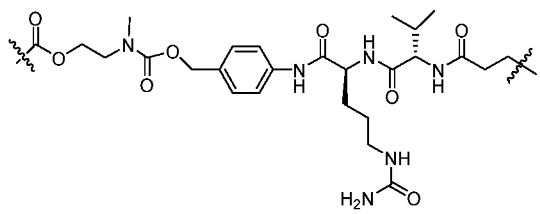

,

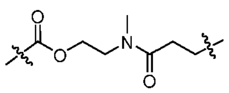

,

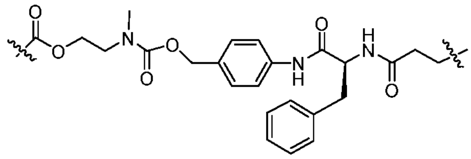

,

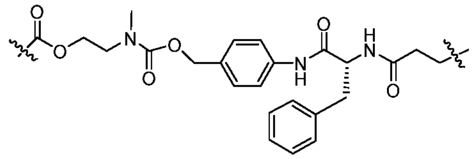

,

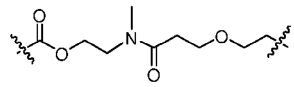, 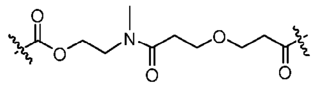, 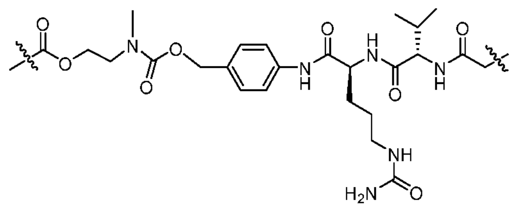,

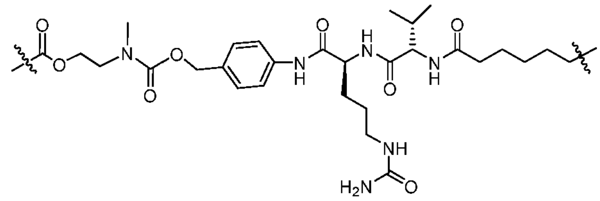,

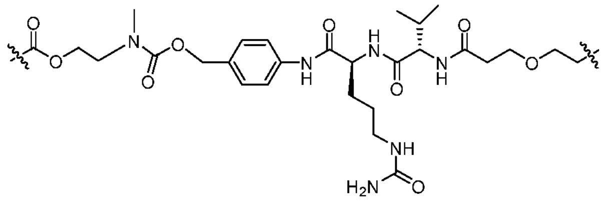,

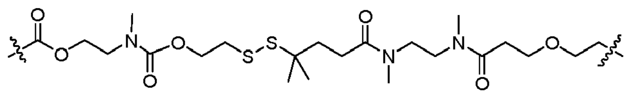, 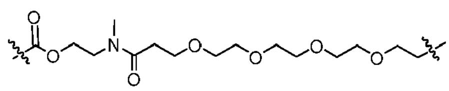,

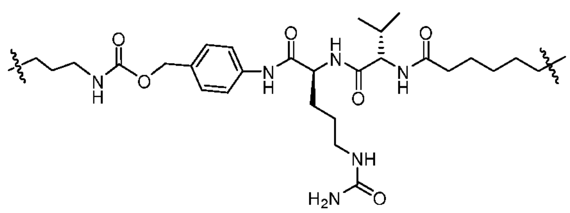, 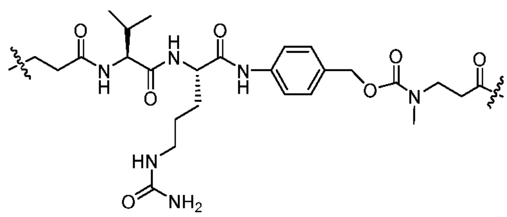,

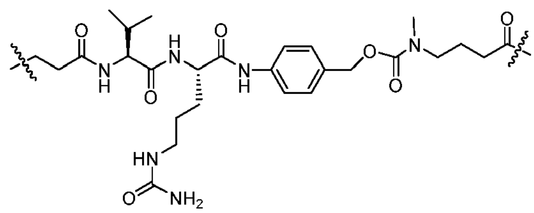, 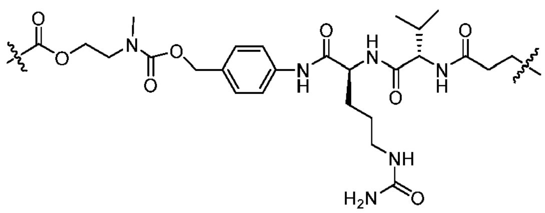,

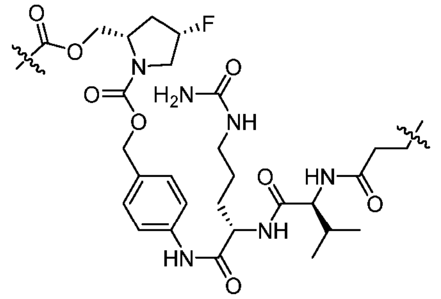, 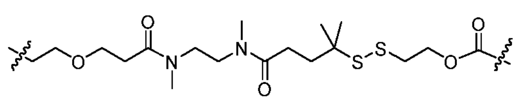,

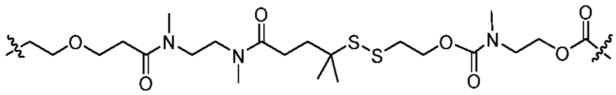,

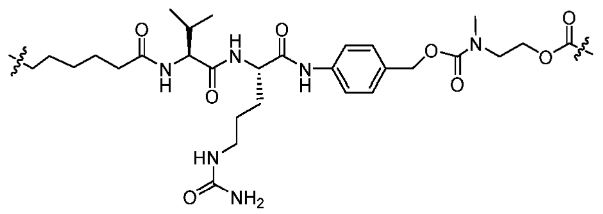,
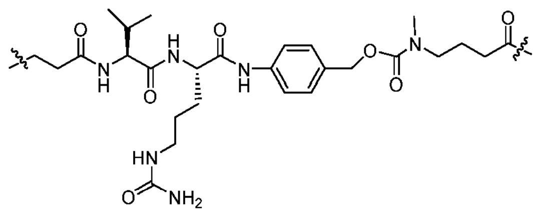,

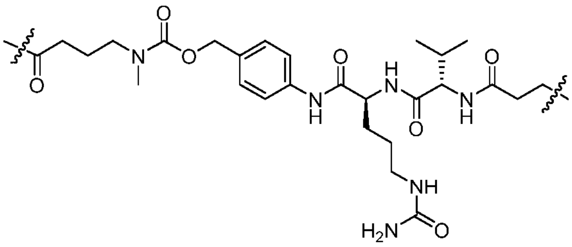,
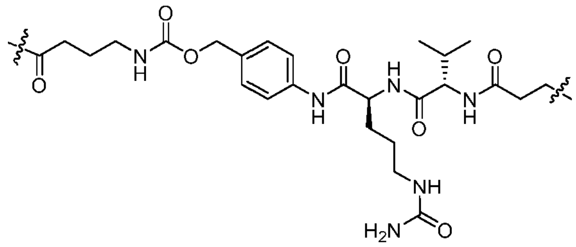,

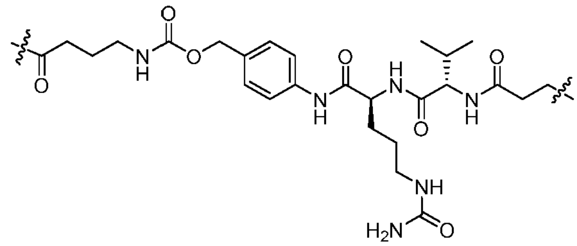,
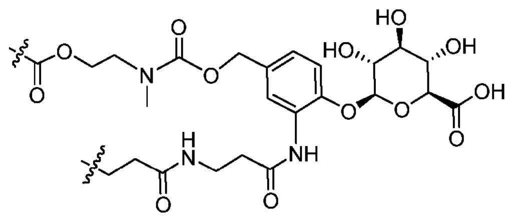,

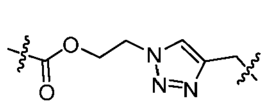,
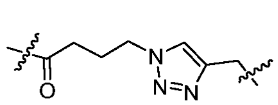,
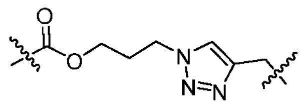,

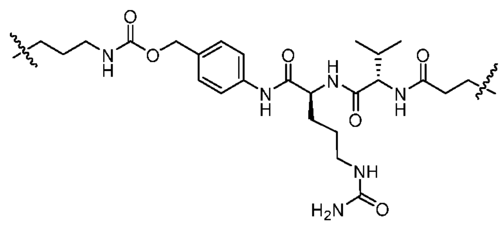,
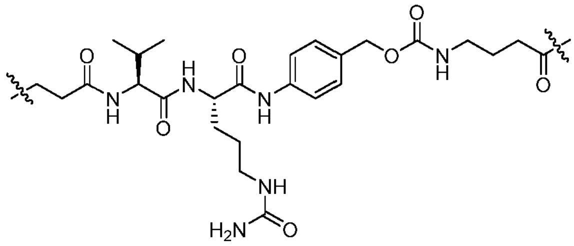,

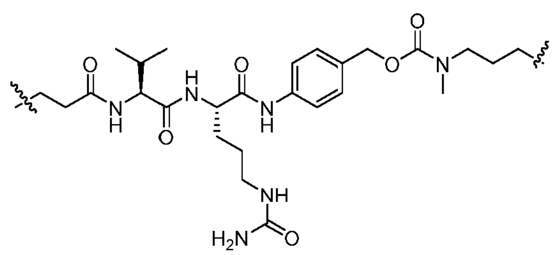,
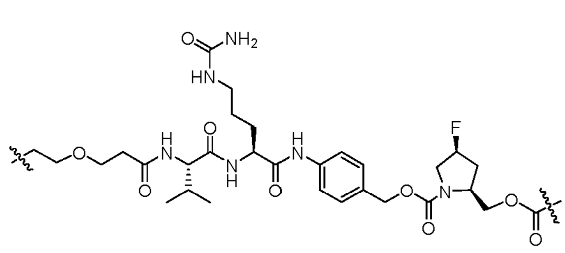,

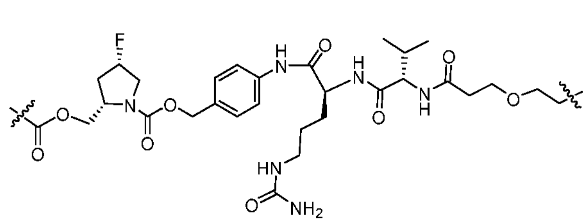,
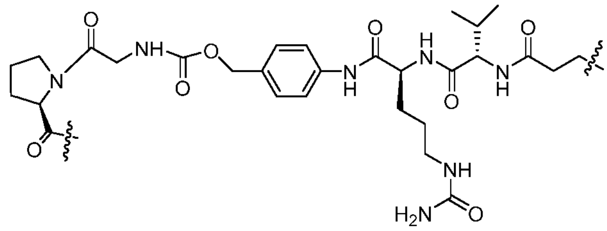,

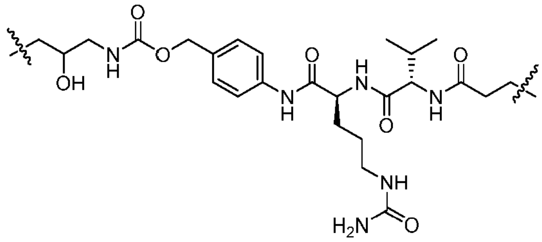,
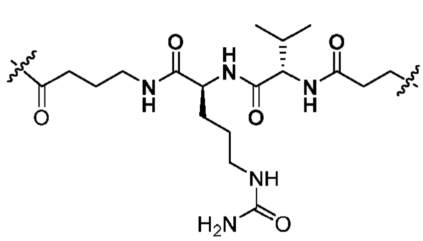,

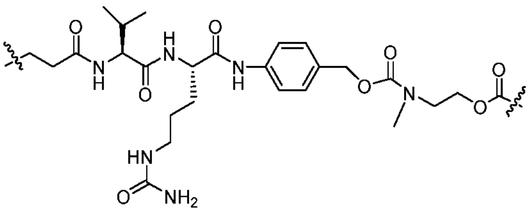,
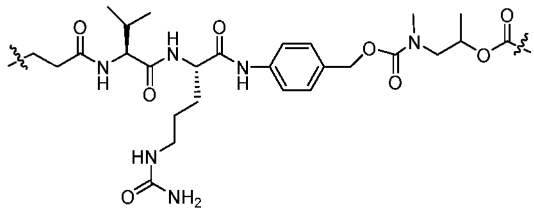,

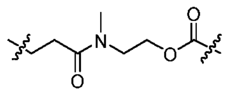,
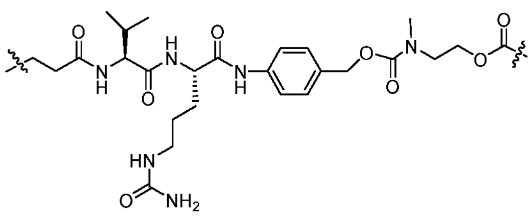,

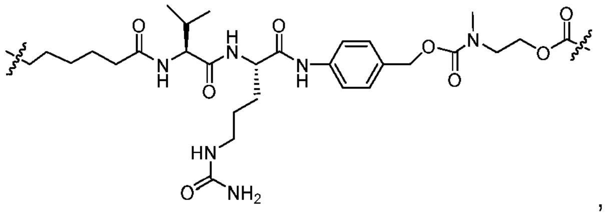, and
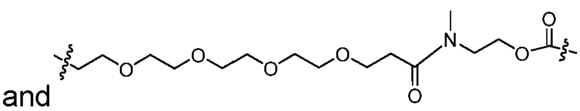.

In some embodiments, Lc is a linker component and each Lc is independently selected from

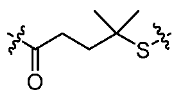,
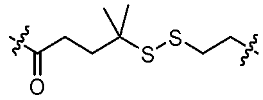,
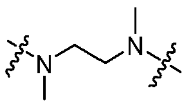,
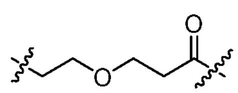,

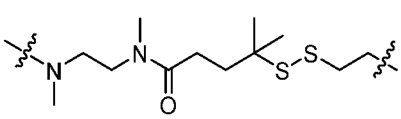,
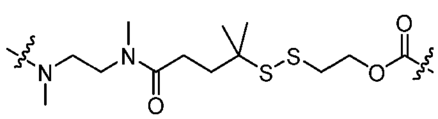,
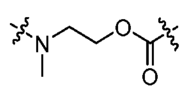,

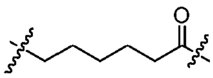, 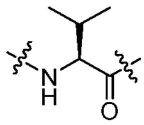, 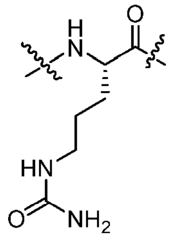, 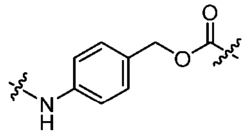, 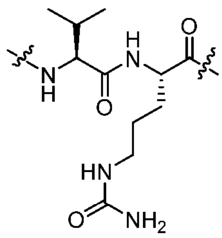,

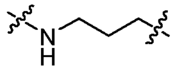, 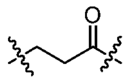, 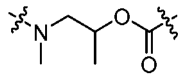, 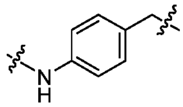, 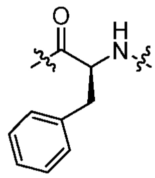, 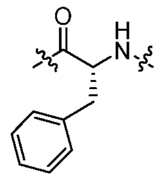

and

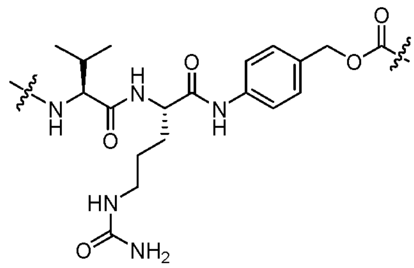.

In some embodiments, the linker L comprises a linker component that is selected from:

$-^{**}C(=O)O(CH_2)_mNR^{11}C(=O)(CH_2)_m-$; $-^{**}C(=O)O(CH_2)_mNR^{11}C(=O)(CH_2)_mO(CH_2)_m-$;

$-^{**}C(=O)O(CH_2)_mNR^{11}C(=O)X_{1a}X_{2a}C(=O)(CH_2)_m-$;

$-^{**}C(=O)OC(R^{12})_2(CH_2)_mNR^{11}C(=O)X_{1a}X_{2a}C(=O)(CH_2)_m-$;

$-^{**}C(=O)O(CH_2)_mNR^{11}C(=O)X_{1a}X_{2a}C(=O)(CH_2)_mO(CH_2)_m-$;

$-^{**}C(=O)O(CH_2)_mNR^{11}C(=O)X_{1a}X_{2a}C(=O)(CH_2)_mO(CH_2)_mC(=O)-$;

$-^{**}C(=O)O(CH_2)_mNR^{11}C(=O)X_4C(=O)NR^{11}(CH_2)_mNR^{11}C(=O)(CH_2)_mO(CH_2)_m-$;

$-^{**}C(=O)O(CH_2)_mN^{11}C(=O)X_5C(=O)(CH_2)_mN^{11}C(=O)(CH_2)_m$;

$-^{**}C(=O)X_4C(=O)NR^{11}(CH_2)_mNR^{11}C(=O)(CH_2)_mO(CH_2)_m-$;

$-^{**}C(=O)(CH_2)_mNR^{11}C(=O)X_{1a}X_{2a}C(=O)(CH_2)_m-$;

$-^{**}C(=O)O(CH_2)_mX_6C(=O)X_{1a}X_{2a}C(=O)(CH_2)_m-$;

$-^{**}C(=O)(CH_2)_mNR^{11}C(=O)((CH_2)_mO)_n(CH_2)_m-$

$-^{**}C(=O)O(CH_2)_mX_6C(=O)(CH_2)_m-$; $-^{**}C(=O)O(CH_2)_mX_6C(=O)(CH_2)_mO(CH_2)_m-$;

$-^{**}C(=O)O(CH_2)_mX_6C(=O)X_{1a}X_{2a}C(=O)(CH_2)_m-$;

$-^{**}C(=O)O(CH_2)_mX_6C(=O)X_{1a}X_{2a}C(=O)(CH_2)_mO(CH_2)_m-$;

$-^{**}C(=O)O(CH_2)_mX_6C(=O)X_{1a}X_{2a}C(=O)(CH_2)_mO(CH_2)_mC(=O)-;$

$-^{**}C(=O)O(CH_2)_mX_6C(=O)X_4C(=O)NR^{11}(CH_2)_mNR^{11}C(=O)(CH_2)_mO(CH_2)_m-;$

$-^{**}C(=O)X_4C(=O)X_6(CH_2)_mNR^{11}C(=O)(CH_2)_mO(CH_2)_m-;$

$-^{**}C(=O)(CH_2)_mX_6C(=O)X_{1a}X_{2a}C(=O)(CH_2)_m-;$

$- ^{**}C(=O)O((CH_2)_mO)_n(CH_2)_mNR^{11}C(=O)X_5C(=O)(CH_2)_m-;$

$-^{**}C(=O)O((CH_2)_mO)_n(CH_2)_mNR^{11}C(=O)X_5C(=O)(CH_2)_mNR^{11}C(=O)(CH_2)_m-;$

$-^{**}C(=O)O((CH_2)_mO)_n(CH_2)_mNR^{11}C(=O)X_5C(=O)(CH_2)_mX_3(CH_2)_m-;$

$-^{**}C(=O)O((CH_2)_mO)_n(CH_2)_mNR^{11}C(=O)X_5C(=O)((CH_2)_mO)_n(CH_2)_m-;$

$-^{**}C(=O)O((CH_2)_mO)_n(CH_2)_mNR^{11}C(=O)X_5C(=O)((CH_2)_mO)_n(CH_2)_mNR^{11}C(=O)(CH_2)_m-;$

$^{**}C(=O)O((CH_2)_mO)_n(CH_2)_mNR^{11}C(=O)X_5C(=O)((CH_2)_mO)_n(CH_2)_mNR^{11}C(=O)(CH_2)_mX_3(CH_2)\ m-;$

$-^{**}C(=O)O((CH_2)_mO)_n(CH_2)_mNR^{11}C(=O))X_5C(=O)((CH_2)_mO)_n(CH_2)_mX_3(CH_2)_m-;$

$-^{**}C(=O)O((CH_2)_mO)_n(CH_2)_mNR^{11}C(=O)X_5C(=O)(CH_2)_mNR^{11}C(=O)((CH_2)_mO)_n(CH_2)_m-;$

$^{**}C(=O)O((CH_2)_mO)_n(CH_2)_mNR^{11}C(=O)X_5C(=O)(CH_2)_mNR^{11}C(=O)((CH_2)_mO)_n(CH_2)_mX_3(CH_2)\ m-;$

$-^{**}C(=O)O((CH_2)_mO)_n(CH_2)_mNR^{11}C(=O)X_5(CH_2)_mX_3(CH_2)_m-;$

$-^{**}C(=O)O((CH_2)_mO)_n(CH_2)_mNR^{11}C(=O)X_5((CH_2)_mO)_n(CH_2)_m-;$

$-^{**}C(=O)O((CH_2)_mO)_n(CH_2)_mNR^{11}C(=O)X_5((CH_2)_mO)_n(CH_2)_mNR^{11}C(=O)(CH_2)_m-;$

$-^{**}C(=O)O((CH_2)_mO)_n(CH_2)_mNR^{11}C(=O)Xs((CH_2)_mO)_n(CH_2)_mNR^{11}C(=O)(CH_2)_{mX3}(CH_2)_m-;$

$- ^{**}C(=O)O((CH_2)_mO)_n(CH_2)_mNR^{11}C(=O)X_5((CH_2)_mO)_n(CH_2)_mX_3(CH_2)_m-;$

$-^{**}C(=O)O((CH_2)_mO)_n(CH_2)_mNR^{11}C(=O)X_5(CH_2)_mNR^{11}((CH_2)_mO)_n(CH_2)_m-;$

$-^{**}C(=O)O((CH_2)_mO)_n(CH_2)_mNR^{11}C(=O)X_5C(=O)(CH_2)_mNR^{11}((CH_2)_mO)_n(CH_2)_mX_3(CH_2)_m-;$

$-^{**}C(=O)O((CH_2)_mO)_n(CH_2)_mNR^{11}C(=O)X_5(CH_2)_m-;$

$- ^{**}C(=O)O((CH_2)_mO)_n(CH_2)_mNR^{11}C(=O)X_5C(=O)((CH_2)_mO)_n(CH2)_m-;$

$-^{**}C(=O)O((CH_2)_mO)_n(CH_2)_mNR^{11}C(=O)X_5(CH_2)_mX_3(CH_2)_m-;$ $-^{**}C(=O)O(CH_2)_m-;$

$-^{**}C(=O)O((CH_2)_mO)_n(CH_2)_m-;$ $-^{**}C(=O)O(CH_2)_mNR^{11}(CH_2)_m-;$

$-^{**}C(=O)O(CH_2)_mNR^{11}(CH_2)_mC(=O)X_{2a}X_{1a}C(=O)-;$

$-^{**}C(=O)O(CH_2)_mX_3(CH_2)_m-;$ $-^{**}C(=O)O((CH_2)_mO)_n(CH_2)_mX_3(CH_2)_m-;$

$-^{**}C(=O)O((CH_2)_mO)_n(CH_2)_mNR^{11}C(=O)(CH_2)_m-;$ -

$^{**}C(=O)O(CH_2)_mNR^{11}C(=O(CH_2)_mX_3(CH_2)_m-;$

$-^{**}C(=O)O((CH_2)_mO)_n(CH_2)_mNR^{11}C(=O)(CH_2)_mX_3(CH_2)_m-;$

$-^{**}C(=O)O((CH_2)_mO)_nX_3(CH_2)_m-$; $-^{**}C(=O)O((CH_2)_mO)_n(CH_2)_mX_3(CH_2)_m-$;

$-^{**}C(=O)O((CH_2)_mO)_n(CH_2)_mC(=O)NR^{11}(CH_2)_m-$; $-^{**}C(=O)O(CH_2)_mC(R^{12})_2-$;

$-^{**}C(=O)OCH_2)_mC(R^{12})_2SS(CH_2)_mNR^{11}C(=O)(CH_2)_m-$,

and

$-^{**}C(=O)O(CH_2)_mC(=O)NR^{11}(CH_2)_m-$,

where: ** indicates point of attachment to the drug moiety (D) and the other end can be connected to $R^{100}$, i.e., the coupling group as described herein;
wherein:

$X_{1a}$ **is**

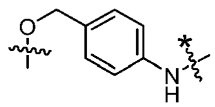 or 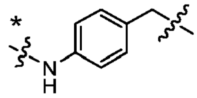 ,

where the * indicates the point of attachment to X2a;
$X_{2a}$ is selected from

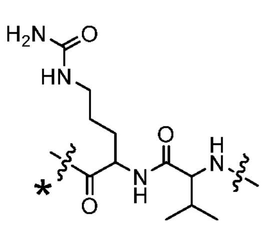 , 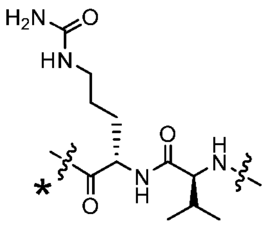 ,

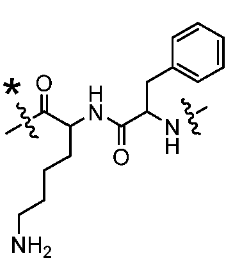 , 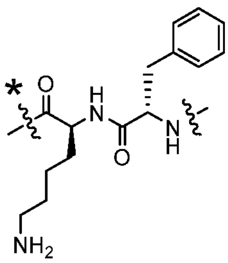 , 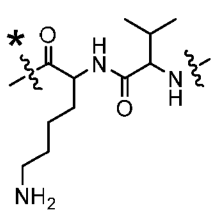 , 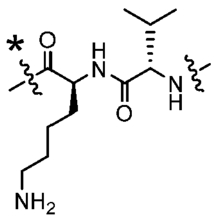 ,

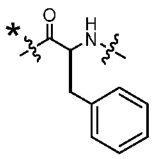 , 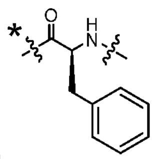 , 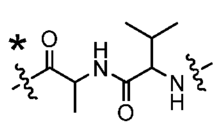 , 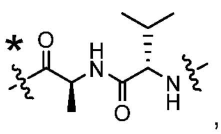 ,

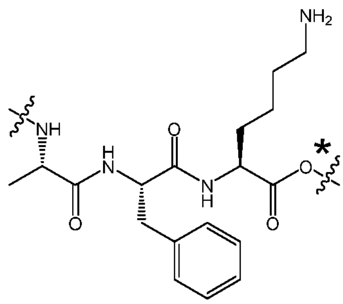, 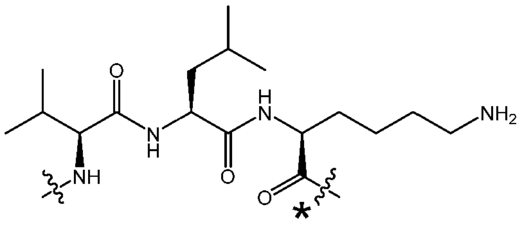,

and

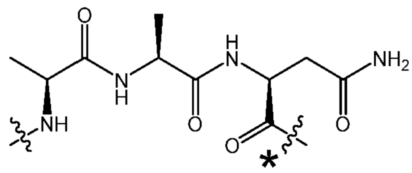;

where the * indicates the point of attachment to $X_{1a}$;
$X_3$ is

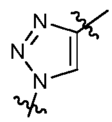, 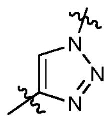, 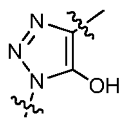 or 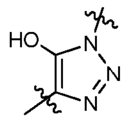;

$X_4$ is $-O(CH_2)_nSSC(R^{12})_2(CH_2)_n-$ or $-(CH_2)_nC(R^{12})_2SS(CH_2)_nO-$;
$X_5$ is

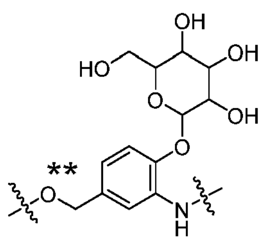, 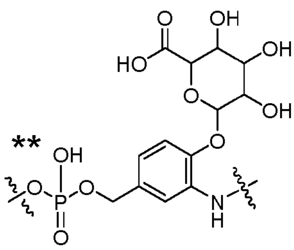 or 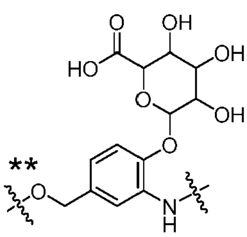,

where the ** indicates orientation toward the Drug moiety;
$X_6$ is

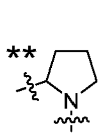, 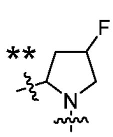, 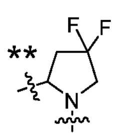, 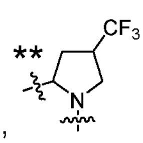 or 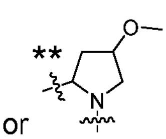,

where the ** indicates orientation toward the Drug moiety;
each $R^{11}$ is independently selected from H and $C_1$-$C_6$alkyl;
each $R^{12}$ is independently selected from H and $C_1$-$C_6$alkyl;
each m is independently selected from 1, 2, 3, 4, 5, 6, 7, 8, 9 and 10, and
each n is independently selected from 1, 2, 3, 4, 5, 6, 7, 8, 9, 10, 11, 12, 13, 14, 15, 16,17 and 18.

Methods of Conjugation

The present invention provides various methods of conjugating Linker-Drug groups of the invention to antibodies or antibody fragments to produce Antibody Drug Conjugates which comprise a linker having one or more hydrophilic moieties.

A general reaction scheme for the formation of Antibody Drug Conjugates of Formula (E') is shown in Scheme 2 below:

Scheme 2

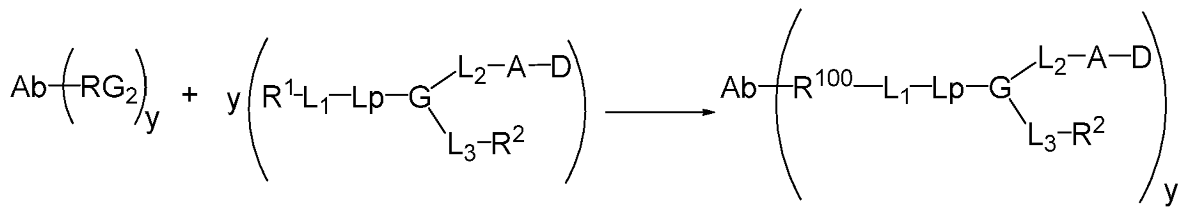

where: $RG_2$ is a reactive group which reacts with a compatible $R^1$ group to form a corresponding $R^{100}$ group (such groups are illustrated in Table 2 and Table 3). D, $R^1$, $L_1$, Lp, Ab, y and $R^{100}$ are as defined herein.

Scheme 3 further illustrates this general approach for the formation of Antibody Drug Conjugates of Formula (E'), wherein the antibody comprises reactive groups ($RG_2$) which react with an $R^1$ group (as defined herein) to covalently attach the Linker-Drug group to the antibody via an $R^{100}$ group (as defined herein). For illustrative purposes only Scheme 3 shows the antibody having four $RG_2$ groups.

Scheme 3

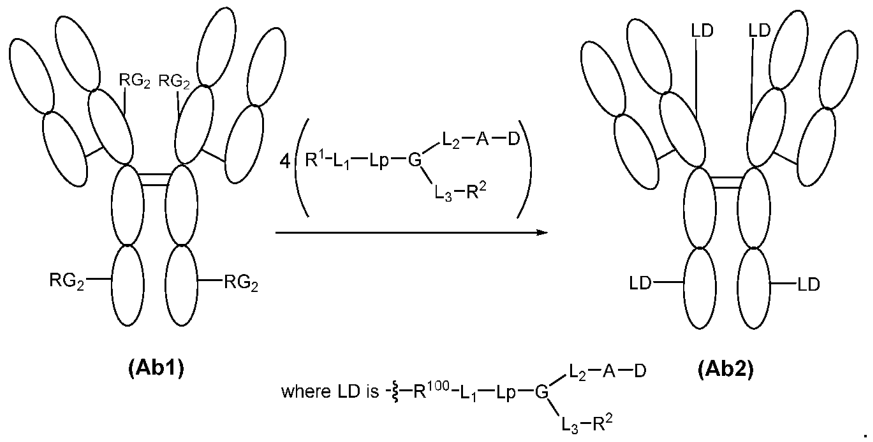

In one aspect, Linker-Drug groups are conjugated to antibodies via modified cysteine residues in the antibodies (see for example WO2014/124316). Scheme 4 illustrates this approach for the formation of Antibody Drug Conjugates of Formula (E') wherein a free thiol group generated from the engineered cysteine residues in the antibody react with an $R^1$ group (where R' is a maleimide) to covalently attach the Linker-Drug group to the antibody via an $R^{100}$ group (where $R^{100}$ is a succinimide ring). For illustrative purposes only Scheme 4 shows the antibody having four free thiol groups.

Scheme 4

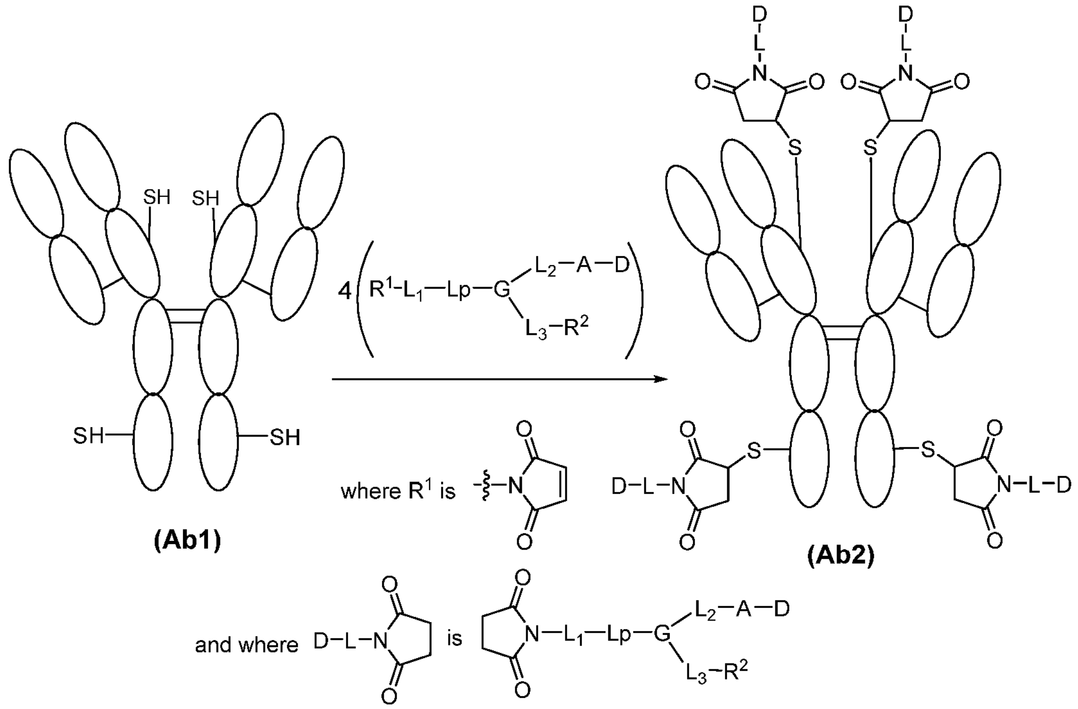


In another aspect, Linker-Drug groups are conjugated to antibodies via lysine residues in the antibodies. Scheme 5 illustrates this approach for the formation of Antibody Drug Conjugates of Formula (E') wherein a free amine group from the lysine residues in the antibody react with an $R^1$ group (where R' is an NHS ester, a pentafluorophenyl or a tetrafluorophenyl) to covalently attach the Linker-Drug group to the antibody via an $R^{100}$ group (where $R^{100}$ is an amide). For illustrative purposes only Scheme 5 shows the antibody having four amine groups.

Scheme 5

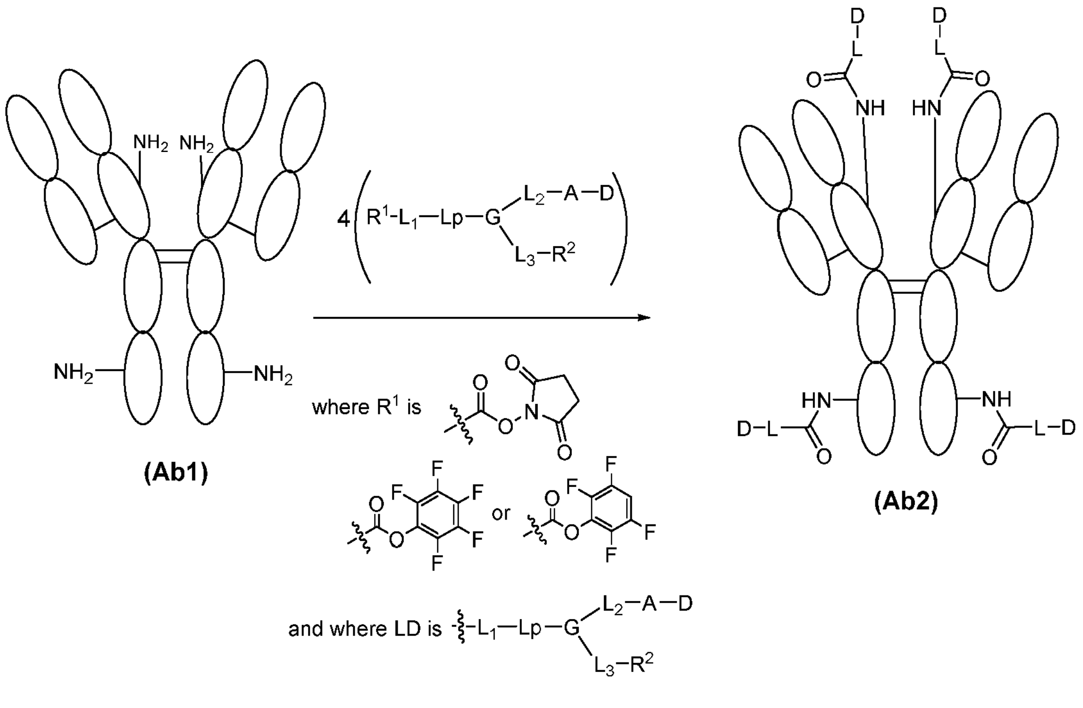


In another aspect, Linker-Drug groups are conjugated to antibodies via formation of an oxime bridge at the naturally occurring disulfide bridges of an antibody. The oxime bridge is formed by initially creating a ketone bridge by

reduction of an interchain disulfide bridge of the antibody and re-bridging using a 1,3-dihaloacetone (e.g. 1,3-dichloroacetone). Subsequent reaction with a Linker-Drug group comprising a hydroxyl amine thereby form an oxime linkage (oxime bridge) which attaches the Linker-Drug group to the antibody (see for example WO2014/083505). Scheme 6 illustrates this approach for the formation of Antibody Drug Conjugates of Formula (E').

Scheme 6

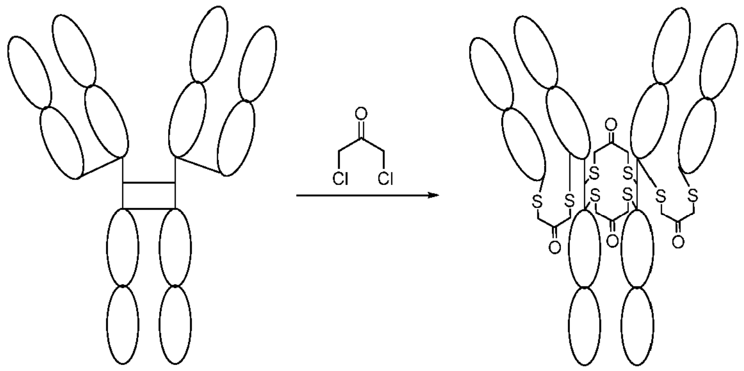

**(Ab1)** **(Ab2)**
**(4 interchain disulfide modified (Ab1))**

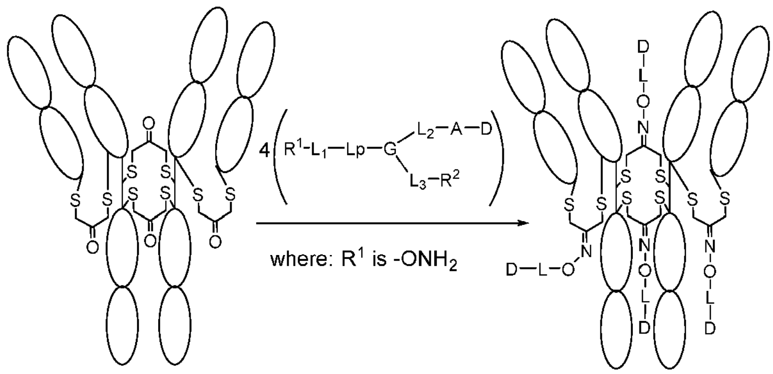


**Ab2** **Ab3**

and where LD is 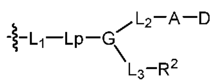.

A general reaction scheme for the formation of Antibody Drug Conjugates of Formula (F') is shown in Scheme 7 below:

Scheme 7

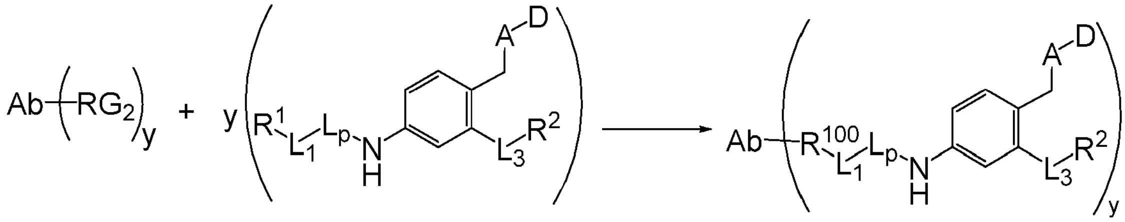

where: $RG_2$ is a reactive group which reacts with a compatible $R^1$ group to form a corresponding $R^{100}$ group (such groups are illustrated in Table 2 and Table 3). D, $R^1$, $L_1$, Lp, Ab, y and $R^{100}$ are as defined herein.

Scheme 8 further illustrates this general approach for the formation of Antibody Drug Conjugates of Formula (F'), wherein the antibody comprises reactive groups ($RG_2$) which react with an $R^1$ group (as defined herein) to covalently attach the Linker-Drug group to the antibody via an $R^{100}$ group (as defined herein). For illustrative purposes only Scheme 8 shows the antibody having four $RG_2$ groups.

Scheme 8

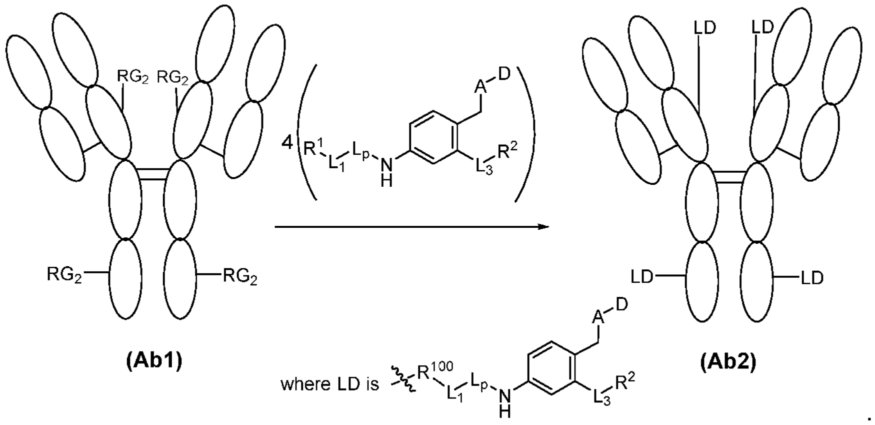


In one aspect, Linker-Drug groups are conjugated to antibodies via modified cysteine residues in the antibodies (see for example WO2014/124316). Scheme 9 illustrates this approach for the formation of Antibody Drug Conjugates of Formula (F’) wherein a free thiol group generated from the engineered cysteine residues in the antibody react with an $R^1$ group (where R’ is a maleimide) to covalently attach the Linker-Drug group to the antibody via an $R^{100}$ group (where $R^{100}$ is a succinimide ring). For illustrative purposes only Scheme 9 shows the antibody having four free thiol groups.

Scheme 9

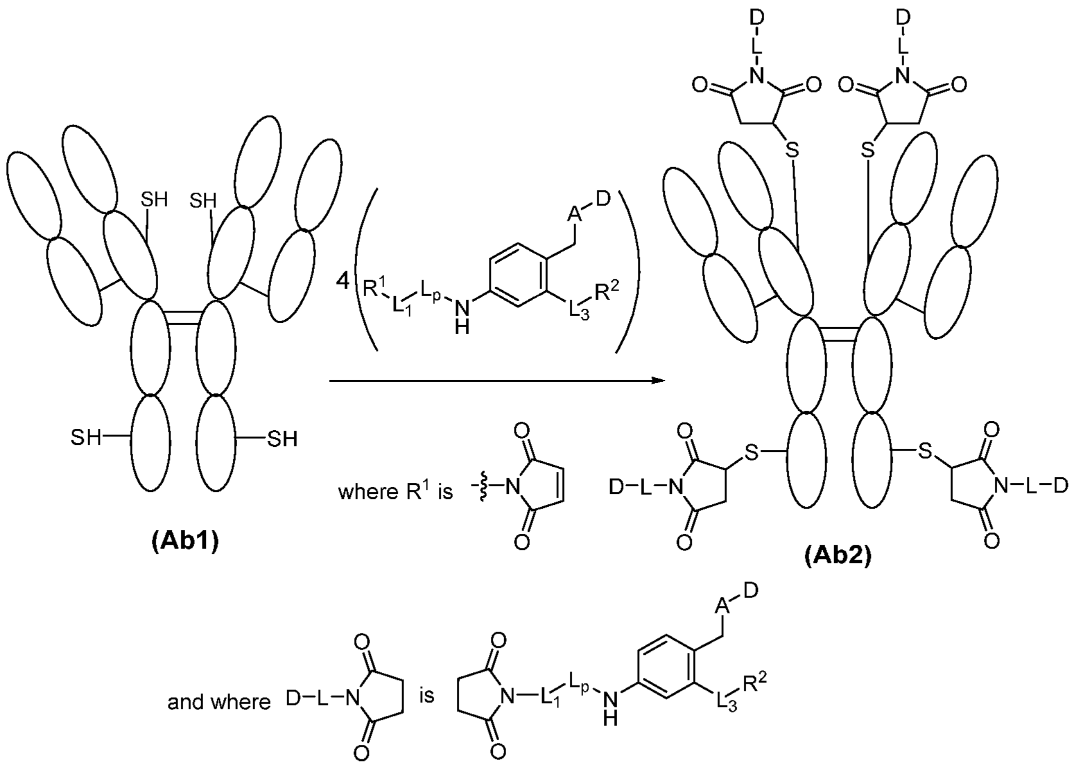


In another aspect, Linker-Drug groups are conjugated to antibodies via lysine residues in the antibodies. Scheme 10 illustrates this approach for the formation of Antibody Drug Conjugates of Formula (F’) wherein a free amine group from the lysine residues in the antibody react with an $R^1$ group (where R’ is an NHS ester, a pentafluorophenyl or a tetrafluorophenyl) to covalently attach the Linker-Drug group to the antibody via an $R^{100}$ group (where $R^{100}$ is an amide). For illustrative purposes only Scheme 10 shows the antibody having four amine groups.

Scheme 10

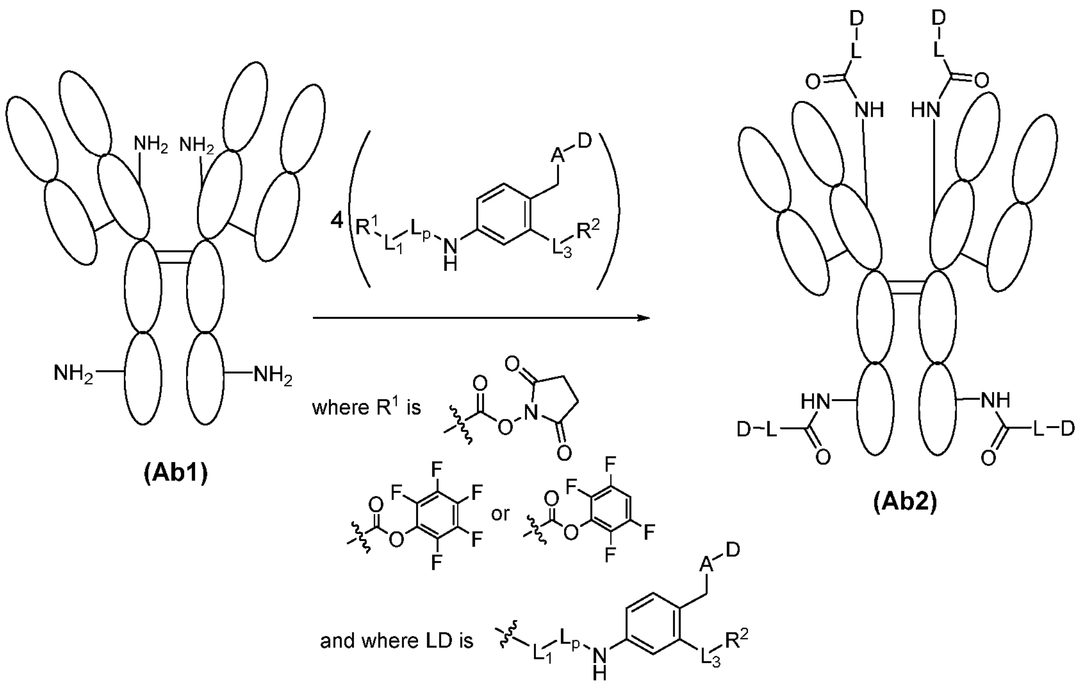


In another aspect, Linker-Drug groups are conjugated to antibodies via formation of an oxime bridge at the naturally occurring disulfide bridges of an antibody. The oxime bridge is formed by initially creating a ketone bridge by reduction of an interchain disulfide bridge of the antibody and re-bridging using a 1,3-dihaloacetone (e.g. 1,3-dichloroacetone). Subsequent reaction with a Linker-Drug group comprising a hydroxyl amine thereby form an oxime linkage (oxime bridge) which attaches the Linker-Drug group to the antibody (see for example WO2014/083505). Scheme 11 illustrates this approach for the formation of Antibody Drug Conjugates of Formula (F').

Scheme 11

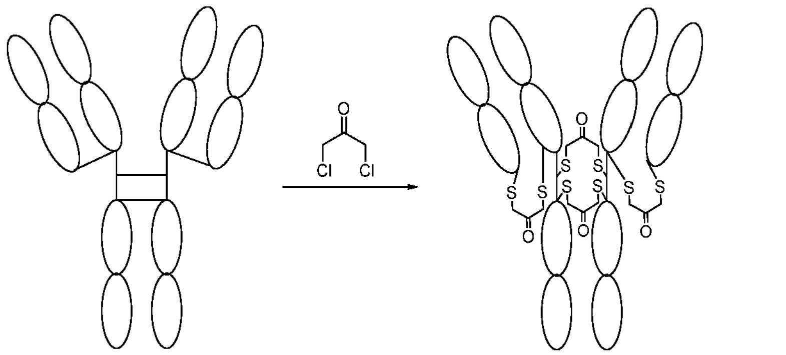

(Ab1) (Ab2)
(4 interchain disulfide modified (Ab1))

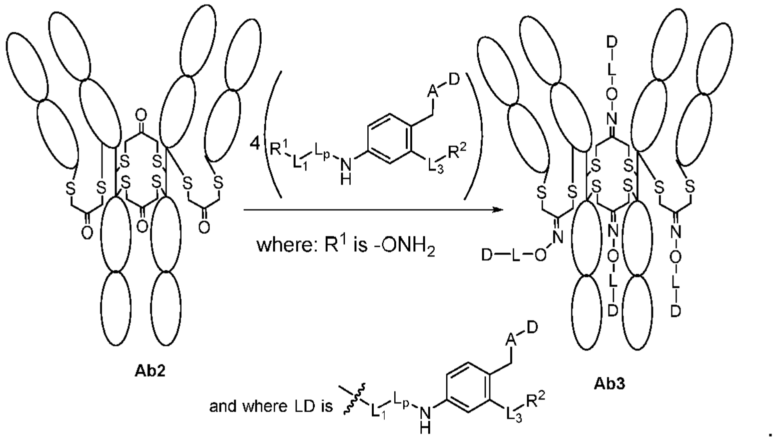


.

Provided are also protocols for some aspects of analytical methodology for evaluating antibody conjugates of the invention. Such analytical methodology and results can demonstrate that the conjugates have favorable properties, for example properties that would make them easier to manufacture, easier to administer to patients, more efficacious, and/or potentially safer for patients. One example is the determination of molecular size by size exclusion chromatography (SEC) wherein the amount of desired antibody species in a sample is determined relative to the amount of high molecular weight contaminants (e.g., dimer, multimer, or aggregated antibody) or low molecular weight contaminants (e.g., antibody fragments, degradation products, or individual antibody chains) present in the sample. In general, it is desirable to have higher amounts of monomer and lower amounts of, for example, aggregated antibody due to the impact of, for example, aggregates on other properties of the antibody sample such as but not limited to clearance rate, immunogenicity, and toxicity. A further example is the determination of the hydrophobicity by hydrophobic interaction chromatography (HIC) wherein the hydrophobicity of a sample is assessed relative to a set of standard antibodies of known properties. In general, it is desirable to have low hydrophobicity due to the impact of hydrophobicity on other properties of the antibody sample such as but not limited to aggregation, aggregation over time, adherence to surfaces, hepatotoxicity, clearance rates, and pharmacokinetic exposure. See Damle, N.K., Nat Biotechnol. 2008; 26(8):884-885; Singh, S.K., Pharm Res. 2015; 32(11):3541-71. When measured by hydrophobic interaction chromatography, higher hydrophobicity index scores (i.e. elution from HIC column faster) reflect lower hydrophobicity of the conjugates. As shown in Examples below, a majority of the tested antibody conjugates showed a hydrophobicity index of greater than 0.8. In some embodiments, provided are antibody conjugates having a hydrophobicity index of 0.8 or greater, as determined by hydrophobic interaction chromatography.

EXAMPLES

The following examples provide illustrative embodiments of the disclosure. One of ordinary skill in the art will recognize the numerous modifications and variations that may be performed. Such modifications and variations are encompassed within the scope of the disclosure. The examples provided do not in any way limit the disclosure.

Example 1. Synthesis **and** Characterization of Linkers, Linker-Payloads, **and** Precursors thereof.

Exemplary linkers, linker-payloads, and precursors thereof were synthesized using exemplary methods described in this example.

**Abbreviations:**

| | |
|---|---|
| CuI | cupper (I) iodide |
| DCC | dicyclohexyl carbodiimide |
| DCM | dichloromethane |
| DEA | N-ethylethanamine |
| DIPEA: | N,N-Diisopropylethylamine |
| DMF: | dimethylformamide |
| DMSO: | dimethylsulfoxyde |
| EEDQ | ethyl 2-ethoxy-2H-quinoline-1-carboxylate |
| Fmoc-Cit-OH | (2S)-2-(9H-fluoren-9-ylmethoxycarbonylamino)-5-ureido-pentanoic acid |
| HBTU: | (2-(1*H*-benzotriazol-1-yl)-1,1,3,3-tetramethyluronium hexafluorophosphate |
| HOAt: | 1-Hydroxy-7-azabenzotriazole |
| THF | tetrahydrofuran |
| $MgSO_4$ | magnesium sulfate |
| $NH_4Cl$ | ammonium chloride |
| NMP | N-methylpyrrolidone |
| $Pd(PPh_3)_2Cl_2$ | dichloro-tri(triphenylphosphine)palladium |
| $PBr_3$ | tribromophosphane |
| Pt/C 10% | platinum over carbon 10% |
| $SOCl_2$ | thionyl chloride |
| TBAI | tetrabutylammonium, iodide |
| TFA | trifluoroacetic acid |

**Materials, Methods & General Procedures:**

All reagents obtained from commercial sources were used without further purification. Anhydrous solvents were obtained from commercial sources and used without further drying. Flash chromatography was performed on CombiFlash Rf (Teledyne ISCO) with pre-packed silica-gel cartridges (Macherey-Nagel Chromabond Flash). Thin layer chromatography was conducted with 5 x 10 cm plates coated with Merck Type 60 F254 silica-gel. Microwave heating was performed in CEM Discover® instrument.

$^1$H-NMR measurements were performed on 400 MHz Bruker Avance or 500 MHz Avance Neo spectrometer, using DMSO-$d_6$ or $CDCl_3$ as solvent. $^1$H NMR data is in the form of chemical shift values, given in part per million (ppm), using the residual peak of the solvent (2.50 ppm for DMSO-$d_6$ and 7.26 ppm for $CDCl_3$) as internal standard. Splitting patterns are designated as: s (singlet), d (doublet), t (triplet), q (quartet), quint (quintet), m (multiplet), br s (broad singlet), br t (broad triplet) dd (doublet of doublets), td (triplet of doublets), dt (doublet of triplets), ddd (doublet of doublet of doublets). IR measurements were performed on a Bruker Tensor 27 equipped with ATR Golden Gate device (SPECAC). HRMS measurements were performed on a LTQ OrbiTrap Velos Pro mass spectrometer (ThermoFisher Scientific). Samples were dissolved in $CH_3CN$/H2O (2/1:v/v) at a concentration range from 0.01 to 0.05 mg/mL approximately and introduced in the source by an injection of 2 μL in a flow of 0.1 mL/min. ESI ionization parameters were as follow: 3.5 kV and 350°C transfer ion capillary. All the spectra were acquired in positive ion mode with a resolving power of 30,000 or 60,000 using a lock mass.

**HRMS** measurements were performed on an LTQ OrbiTrap Velos Pro mass spectrometer (ThermoFisher Scientific GmbH, Bremen, Germany). Samples were dissolved in $CH_3CN/H_2O$ (2/1:v/v) at a concentration range from 0.01 to 0.05 mg/mL approximately and introduced in the source by an injection of 2 μL in a flow of 0.1 mL/min. ESI ionization parameters were as follows: 3.5 kV and 350°C transfer ion capillary. All the spectra were acquired in positive ion mode with a resolving power of 30 000 or 60 000 using a lock mass.

UPLC®-MS:

UPLC®-MS data were acquired using an instrument with the following parameters (Table 4):

**Table 4. UPLC®-MS Parameters**

| | |
|---|---|
| Instrument(s) | Waters Aquity A-class with diode array UV detector "PDA" and "ZQ detector 2" mass device and MassLinks software. |
| ZQ detector 2 | MS scan from 0.15 to 6 min and from 100 to 2372 Da |
| PDA detector | from 190 to 400 nm Aquity UPLC ®BEH column C18, 1.7 μm, 130 Å, |
| Columns | 2.1x50 mm Column used at 40°C with a flowrate of 0.6mL/min |
| Solvent A | water + 0.02% TFA |
| Solvent B | acetonitrile + 0.02% TFA |
| gradient | from 2% B to 100% B in 5 min, then 0.3 min washing with 100% B and 0.5 min equilibration at 2% B for the next injection (total gradient of 6 min). |

Preparative-HPLC:

Preparative-HPLC ("Prep-HPLC") data were acquired using an instrument with the following parameters (Table 5):

**Table 5. Prep-HPLC Parameters**

| | |
|---|---|
| Instrument(s) | Columns Waters X-Bridge 5 or 10 μm with sizes (flowrate) of: 19x50 mm (12 ml/min), 19x100 mm (12 ml/min), 30x100 mm (30-50 ml/min), 30x250 mm (30-50 ml/min), 50x250 mm (80-150 ml/min); Interchim Puriflash 4100 with a maximum of 100 bars and a maximum flowrate of 250 ml/min, or Interchim Puriflash 4250 with a maximum of 250 bars and a maximum flowrate of 250 ml/min; Quaternary solvent pump with the possibility to use 4 solvents at the same time in a gradient |
| UV | 2 wavelengths for the collection between 200 and 400 nm |
| Columns | Waters Xbridge 10μm |
| Collection | 8 ml or 32 ml tubes |

Three Prep-HPLC methods were used:

a. TFA method: solvent: A water + 0.05 % TFA, B acetonitrile + 0.05 % TFA, gradient from 5 to 100% B in 15 to 30 CV

b. $NH_4HCO_3$ method: solvent: A water + 0.02 M $NH_4HCO_3$, B acetonitrile/water 80/20 + 0.02 M $NH_4HCO_3$, gradient from 5 to 100 % B in 15 to 30 CV

c. Neutral method: solvent: A water, B acetonitrile, gradient from 5 to 100% B in 15 to 30 CV

All the fractions containing the pure compound were combined and directly freeze-dried to afford the compound as an amorphous powder.

Preparative SFC purification:

Preparative chiral SFC was performed on a PIC solution Prep200 system. The sample was dissolved in ethanol at a concentration of 150 mg/mL. The mobile phase was held isocratically at 40% ethanol/$CO_2$. The instrument was fitted with a Chiralpak IA column and a loop of 3 mL. The ABPR (automatic back-pressure regulator) was set at 100 bars.

**Preparation of L23-P3:**

**(2R)-2-[(5Sa)-5-[4-[2-[4-[[4-[[(2S)-2-[[(2S)-2-[[2-(2-azidoethoxy)acetyl]amino]-3-methyl-butanoyl]amino]-5-ureido-pentanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid**

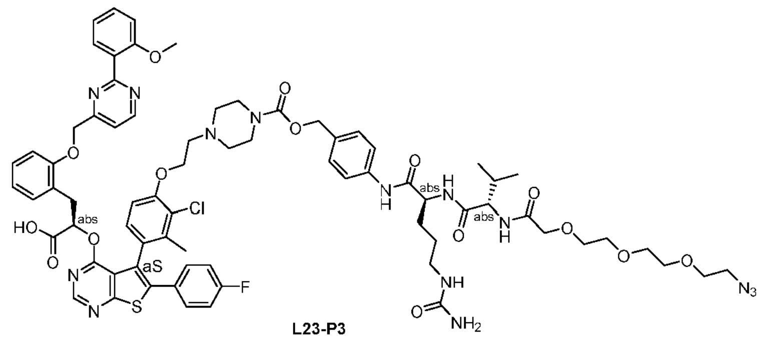

L23-P3

*Step 1: (2S)-2-[[(2S)-2-[[2-[2-[2-(2-azidoethoxy)ethoxy]ethoxy]acetyl]amino]-3-methyl-butanoyl]amino]-N-[4-(hydroxymethyl)phenyl]-5-ureido-pentanamide*

To a solution of 2-[2-[2-(2-azidoethoxy)ethoxy]ethoxy]acetic acid (purchased from Broadpharm, 1.4 g, 6 mmol) in THF (20 mL) was added 1-hydroxypyrrolidine-2,5-dione (690 mg, 6 mmol) and N,N'-Dicyclohexylcarbodiimide (1.2 g, 6 mmol). The reaction mixture was stirred at room temperature overnight. The precipitate was filtered off and the filtrate was concentrated to afford (2,5-dioxopyrrolidin-1-yl) 2-[2-[2-(2-azidoethoxy)ethoxy]ethoxy]acetate (1.9g, 6 mmol), used immediately without further purification.

To a solution of (2,5-dioxopyrrolidin-1-yl) 2-[2-[2-(2-azidoethoxy)ethoxy]ethoxy]acetate (1.6 g; 4.85 mmol) in DMF (15 mL) was added (2S)-2-[[(2S)-2-amino-3-methyl-butanoyl]amino]-N-[4-(hydroxymethyl)phenyl]-5-ureido-pentanamide (1.96 g; 5.17 mmol). The mixture was stirred at room temperature for 2 h and concentrated. The residue was diluted in water (20 mL) and acetonitrile (5 mL) and stirred at room temperature overnight. The mixture was purified by reverse phase C18 chromatography using the neutral method to afford (2S)-2-[[(2S)-2-[[2-[2-[2-(2-azidoethoxy)ethoxy]ethoxy]acetyl]amino]-3-methyl-butanoyl]amino]-N-[4-(hydroxymethyl)phenyl]-5-ureido-pentanamide (1.07g, 1.8 mmol). $^1$H NMR (400 MHz, dmso-d6): δ 9.95 (s, 1H), 8.3 (d, 1H), 7.55 (d, 2H), 7.46 (d, 1H), 7.22 (d, 2H), 5.98 (t, 1H), 5.4 (s, 1H), 5.08 (t, 1H), 4.43 (d, 2H), 4.4 (q, 1H), 4.33 (dd, 1H), 3.95 (s, 2H), 3.6 (m, 10H), 3.38 (t, 2H), 3 (m, 2H), 2 (m, 1H), 1.7/1.6 (2m, 2H), 1.5-1.3 (m, 2H), 0.89/0.82 (2d, 6H).

*Step 2: [4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl (4-nitrophenyl) carbonate*

To a solution of (2S)-2-[[(2S)-2-[[2-[2-[2-(2-azidoethoxy)ethoxy]ethoxy]acetyl]amino]-3-methyl-butanoyl]amino]-N-[4-(hydroxymethyl)phenyl]-5-ureido-pentanamide (100 mg, 0.168 mmol) in DMF (30 mL) was added DIPEA (32 μL, 0.179 mmol) and bis(4-nitrophenyl) carbonate (100 mg, 0.329 mmol). The mixture was stirred at room temperature for 4 h and concentrated to dryness. The residue was purified by silica gel chromatography (gradient of methanol in dichloromethane) to afford 4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl (4-nitrophenyl)carbonate (65 mg, 0.088 mmol).$^1$H NMR (400 MHz, dmso-d6): δ 9.95 (s, 1H), 8.3 (d, 1H), 7.55 (d, 2H), 7.46 (d, 1H), 7.22 (d, 2H), 5.98 (t, 1H), 5.4 (s, 1H), 5.08 (t, 1H), 4.43 (d, 2H), 4.4 (q, 1H), 4.33 (dd, 1H), 3.95 (s, 2H), 3.6 (m, 10H), 3.38 (t, 2H), 3 (m, 2H), 3.02-2.95 (m, 2H), 2 (m, 1H), 1.7 (m, 1H), 1.6 (m, 1H), 0.89 (d, 3H), 0.82 (d, 3H).

*Step 3: (2R)-2-[(5$S_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid **L23-P3***

To a solution of ((2R)-2-[(5$S_a$)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid **P3** (147 mg, 0.17 mmol) in DMF (16 mL) were successively added DIPEA (85 μL, 0.51 mmol), 4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl (4-nitrophenyl)carbonate (136 mg, 0.179 mmol), 2,6-lutidine (99 μL, 0.85 mmol) and HOAt (7 mg, 0.05 mmol). The mixture was stirred at room temperature overnight and purified by C18 reverse phase prep-HPLC by direct deposit of the reaction mixture on the Xbridge column and using the $NH_4HCO_3$ method to afford **L23-P3** (110 mg, 0.074 mmol). $^1$H NMR (400 MHz, dmso-d6): δ 10.05 (s, 1H), 8.87 (d, 1H), 8.59 (s, 1H), 8.32 (d, 1H), 7.67 (br s, 1H), 7.59 (d, 2H), 7.52 (dd, 1H), 7.45 (td, 1H), 7.44 (d, 1H), 7.36 (dl, 1H), 7.29 (m, 2H), 7.27 (d, 2H), 7.2 (t, 2H), 7.19 (d, 1H), 7.14 (d, 1H), 7.13 (t, 1H), 7.03 (t, 1H), 6.99 (d, 1H), 6.71 (t, 1H), 6.24 (dl, 1H), 5.99 (t, 1H), 5.48 (dd, 1H), 5.41 (br s, 1H), 5.23 (m, 2H), 4.97 (s, 2H), 4.39 (m, 1H), 4.32 (dd, 1H), 4.21

(m, 2H), 3.95 (m, 2H), 3.75 (s, 3H), 3.65-3.50 (m, 10H), 3.34 (m, 2H), 3.02/2.95 (m, 2H), 2.73 (t, 2H), 2.49/2.3 (m,2H), 2.45 (m, 4H), 2.3 (m, 4H), 2 (m, 1H), 1.82 (s, 3H), 1.7/1.59 (m, 2H), 1.44/1.37 (m, 2H), 0.87 (d, 3H), 0.82 (d, 3H). $^{13}$C NMR (100 MHz, dmso-d6): δ 158.3, 152.9, 131.6, 131.6, 131.3, 131.3, 131, 129, 128.8, 121, 120.8, 119.5, 116.4, 116.1, 112.8, 112.4, 111.2, 74.5, 70.1, 69.3, 67.7, 66.4, 57, 56.7, 56.2, 53.7, 53.2, 50.4, 43.6, 39, 32.8, 31.6, 29.6, 27.3, 19.3, 17.7. IR Wavelength (cm$^{-1}$): 3500-2500, 2106, 1656. HR-ESI+: m/z [M+H]+ = 1479.5422 / 1479.5405 (measured/theoretical).

**Preparation of L24-P1:**

**(2R)-2-[(5S$_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl] methoxy]phenyl]propanoic acid; 2,2,2-trifluoroacetate; 2,2,2-trifluoroacetic acid**

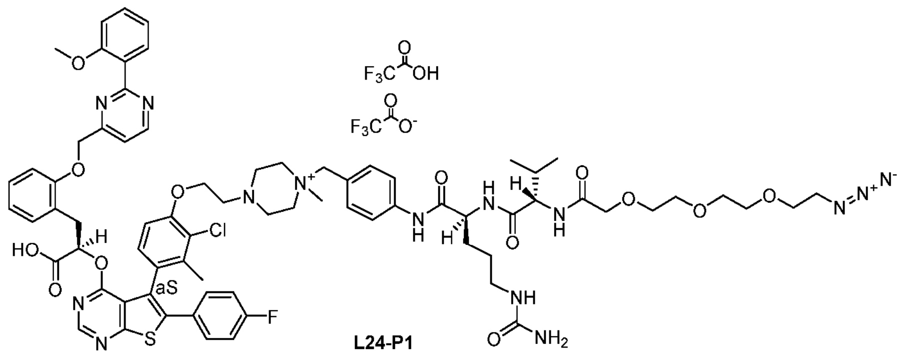

L24-P1

*Step 1: (2S)-2-[[(2S)-2-[[2-[2-[2-(2-azidoethoxy)ethoxy]ethoxy]acetyl]amino]-3-methyl-butanoyl]amino]-N-[4-(bromomethyl)phenyl]-5-ureido-pentanamide*

To a solution of (2S)-2-[[(2S)-2-[[2-[2-[2-(2-azidoethoxy)ethoxy]ethoxy]acetyl]amino]-3-methyl-butanoyl]amino]-N-[4-(hydroxymethyl)phenyl]-5-ureido-pentanamide (330 mg, 0.55 mmol; obtained according to Step 1 of the synthesis of L23-P3) in THF (10 mL) was added dropwise at 0°C a solution of phosphorus tribromide 1 M in dichloromethane (1 mL, 1 mmol). The mixture was stirred at 0°C for 1 h and finely grounded $NaHCO_3$ (100 mg) was added. After 10 min of stirring, the reaction was diluted with ethyl acetate and filtered. The organic layer was dried over Magnesium sulfate and concentrated. The residue (2S)-2-[[(2S)-2-[[2-[2-[2-(2-azidoethoxy)ethoxy]ethoxy]acetyl]amino]-3-methyl-butanoyl]amino]-N-[4-(bromomethyl)phenyl]-5-ureido-pentanamide (283 mg, 0.43 mmol) was used without further purification. HR-ESI+: m/z [M+H]+ = = 595.3200 / 595.3198 (measured/theoretical).

*Step 2: ((2R)-2-[(5S$_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl] propanoic acid; 2,2,2-trifluoroacetate; 2,2,2-trifluoroacetic acid L24-P1*

To a solution of ethyl (2R)-2-[(5S$_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate dichlorhydrate (P1) (345 mg, 0.355 mmol) in DMF (1 mL) were successively added (2S)-2-[[(2S)-2-[[2-[2-[2-(2-azidoethoxy) ethoxy]ethoxy]acetyl]amino]-3-methyl-butanoyl]amino]-N-[4-(bromomethyl)phenyl]-5-ureido-pentanamide (233 mg, 0.355 mmol) and DIPEA (50 μL, 0.304 mmol). The mixture was stirred at room temperature overnight. A solution of lithium hydroxide monohydrate (15 mg, 3.55 mmol) in water (0.5 mL) was added and the reaction was stirred at room temperature for 24 h. The reaction mixture was purified by C18 reverse phase prep-HPLC by direct deposit of the reaction mixture on the Xbridge column and using the TFA method to afford **L24-P1** (80 mg, 0.054 mmol). $^{1}$H NMR (400 MHz, dmso-d6): δ 13.2 (m, 1H), 10.25 (m, 1H), 8.88 (d, 1H), 8.6 (s, 1H), 8.36 (d, 1H), 7.72 (d, 2H), 7.63 (d, 1H), 7.52 (dd, 1H), 7.46 (t, 1H), 7.44 (m, 1H), 7.43 (m, 2H), 7.37 (d, 1H), 7.3 (dd, 2H), 7.21 (t, 2H), 7.2 (d, 1H), 7.15 (d, 1H), 7.15 (t, 1H), 7.03 (t, 1H), 7 (t, 1H), 6.72 (t, 1H), 6.22 (d, 1H), 6 (t, 1H), 5.52 (m, 2H), 5.49 (dd, 1 H), 5.25 (dd, 2H), 4.5 (br s, 2H), 4.39 (m, 1H), 4.32 (m, 1H), 4.25 (m, 2H), 3.95 (br s, 2H), 3.76 (s, 3H), 3.4/3.24 (m, 4H), 3.35 (m, 2H), 3.28/2.51 (m, 2H), 3.04/2.83 (m, 4H), 3.02/2.96 (m, 2H), 2.92 (m, 2H), 2.87 (s, 3H), 1.99 (m,1 H), 1.83 (s, 3H), 1.69/1.61 (m, 2H), 1.46/1.38 (m, 2H), 0.88/0.82 (m, 6H). $^{13}$C NMR

(125 MHz, dmso-d6): δ 134.2, 131.4, 131.3, 131.3, 131.2, 130.7, 128.7, 120.9, 120.5, 119.2, 116.3, 115.8, 112.7, 112.3, 111, 74, 70.2, 69.6, 67.8, 58.9, 56.9, 56.1, 55.4, 54, 50.5, 46.6, 44.9, 39, 32.7, 31.6, 29.8, 27.5, 19.7/18.4, 18. IR Wavelength ($cm^{-1}$): 3700-2200, 3000-2000, 2109, 1662, 1250-1050. HR-ESI+: m/z [M+Na]+ = 1473.5656 / 1473.5628 (measured/theoretical).

**Preparation of L13-C4:**

**(2R)-2-[(5S$_a$)-5-[4-[2-[4-[[4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethox y]ethoxy]propanoylamino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-[4-(phosphonomethyl)phenyl]pyrimidin-4-yl]methoxy]phenyl]propanoic acid**

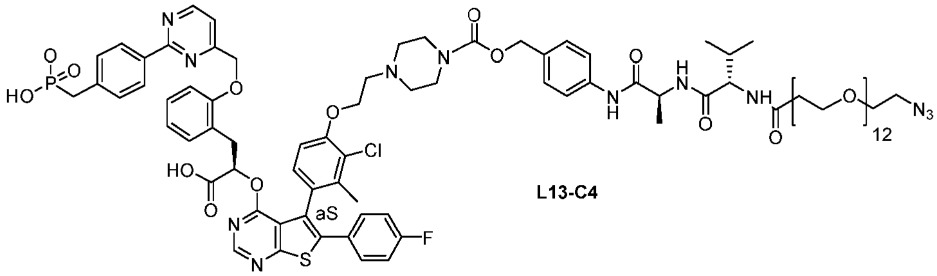

L13-C4

*Step 1: Synthesis of (2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]-N-[(1S)-2-[4-(hydroxymethyl)anilino]-1-methyl-2-oxoethyl]-3-methyl-butanamide*

To a solution of (2S)-2-amino-N-[(1S)-2-[4-(hydroxymethyl)anilino]-1-methyl-2-oxoethyl]-3-methyl-butanamide (0.9 g, 3.07 mmol; obtained according to Step 3 of the synthesis of L18-C3) and 3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoic acid (purchased from Broadpharm, 2 g, 3.07 mmol) in DMF (20 mL) were successively added DIPEA (1 mL, 6.13 mmol), 3-(ethyliminomethyleneamino)propyl-dimethyl-ammonium; chloride (EDC) (0.65 g, 3.37 mmol) and [dimethylamino(triazolo[4,5-b]pyridin-3-yloxy)methylene]-dimethyl-ammonium; hexafluorophosphate (HATU) (1.28 g, 3.37 mmol). The mixture was stirred at room temperature overnight and purified by C18 reverse phase prep-HPLC by direct deposit of the reaction mixture on the Xbridge column and using the $NH_4HCO_3$ method to afford (2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]-N-[(1S)-2-[4-(hydroxymethyl)anilino]-1-methyl-2-oxo-ethyl]-3-methylbutanamide (1.64 g, 1.81 mmol). $^1$H NMR (400 MHz, dmso-d6): δ 9.82 (m, 1H), 8.14 (d, 1H), 7.87 (d, 1H), 7.54 (d, 2H), 7.23 (d, 2H), 5.08 (t, 1H), 4.43 (d, 2H), 4.39 (m, 1H), 4.2 (m, 1H), 3.65-3.44 (m, 48H), 3.39 (t, 2H), 2.50-2.30 (m, 2H), 1.97 (m, 1H), 1.31 (d, 3H), 0.87/0.84 (m, 6 H). IR Wavelength ($cm^{-1}$): 3600-3200, 3287, 2106, 1668, 1630, 1100. HR-ESI+: m/z [M+H]+ = 919.5265 / 919.5234 (measured/theoretical).

*Step 2:[4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]-3-methyl-butanoyl]amino]propanoyl]amino]phenyl]methyl (4-nitrophenyl) carbonate*

To a solution of (2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]-N-[(1S)-2-[4-(hydroxymethyl)anilino]-1-methyl-2-oxoethyl]-3-methylbutanamide (210 mg, 0.228 mmol) in a mixture of THF and dichloromethane (respectively 5 and 2.5 mL) were successively added pyridine (30 μL, 0.479 mmol) and 4-Nitrophenyl chloroformate (97 mg, 0.479 mmol). The reaction was stirred at room temperature for 3h and other portions of 4-Nitrophenyl chloroformate (40 mg, 0.197 mmol) and pyridine (30 μL, 0.479 mmol) were added. The reaction mixture was stirred at 0°C for 55h and evaporated to dryness. The residue was purified by silica-gel chromatography (gradient of MeOH in dichloromethane) to afford [4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]-3-methyl-butanoyl]amino]propanoyl]amino]phenyl]methyl (4-nitrophenyl) carbonate (118 mg, 0.110 mmol). $^1$H NMR (400 MHz, dmso-d6): δ 10.00 (s, 1H), 8.31 (d, 2H), 8.19 (d, 1H), 7.88

(d, 1H), 7.64 (d, 2H), 7.58 (d, 2H), 7.41 (d, 2H), 5.25 (s, 2H), 4.39 (m, 1H), 4.21 (m, 1H), 3.63-3.47 (m, 48H), 3.39 (t, 2H), 2.50-2.35 (m, 2H), 1.98 (m, 1H), 1.31 (d, 3H), 0.89/0.85 (m, 6H). IR Wavelength ($cm^{-1}$): 3278, 2108, 1763, 1633, 1526, 1525, 1350, 1215, 1110.

*Step 3: (2R)-2-[(5$S_a$)-5-[4-[2-[4-[[4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-[4-(phosphonomethyl)phenyl]pyrimidin-4-yl]methoxy]phenyl]propanoic acid (L13-C4)*

To a solution of [4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]-3-methyl-butanoyl]amino]propanoyl]amino]phenyl]methyl (4-nitrophenyl) carbonate (52 mg, 47.6 μmol) in DMF (5 mL) were successively added (2R)-2-[(5$S_a$)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-[4-(phosphonomethyl)phenyl]pyrimidin-4-yl]methoxy]phenyl]propanoic acid **C4** (36.7 mg, 39.7μmol) and DIPEA (26μL, 108 μmol). The reaction was stirred at room temperature for 1 h and purified by C18 reverse phase prep-HPLC by direct deposit of the reaction mixture on the Xbridge column and using the $NH_4HCO_3$ method to afford **L13-C4** (36 mg, 19 μmol). $^1$H NMR (400 MHz, dmso-d6): δ 10.1 (br s, 1H), 8.81 (br s, 1 H), 8.55 (m, 1 H), 8.32 (br s, 1H), 8.19 (d, 2H), 8.02 (br s, 1H), 7.66 (m, 1H), 7.58 (d, 2H), 7.37 (d, 1H), 7.29 (dd, 2H), 7.28 (d, 2H), 7.25 (d, 2H), 7.19 (t, 2H), 7.17 (d, 1H), 7.08 (t, 1H), 6.96 (d, 1H), 6.68 (t, 1H), 6.21 (d, 1H), 5.5 (m, 1H), 5.22 (m, 2H), 4.96 (s, 2H), 4.4 (m, 1H), 4.2 (dd, 1H), 4.18 (m, 2H), 3.62/3.41 (m, 24H), 3.5 (m, 4H), 3.38 (m, 2H), 3.28 (m, 4H), 2.87 (m, 2H), 2.7 (m, 2H), 2.48/2.36 (m, 2H), 2.41 (m, 4H), 1.99 (m, 1H), 1.79 (s, 3H), 1.3 (d, 3H), 0.87/0.83 (m, 6H). $^{13}$C NMR (100 MHz, dmso-d6): δ 130.7, 130.7, 130.6, 130.3, 129, 128.4, 127.4, 121, 119.6, 116.3, 116.1, 112.1, 70.2/67.3, 69.5, 67.5, 66.4, 58.2, 56.4, 53.2, 50.3, 49.6, 43.8, 36.3, 31, 19, 18.5, 17.8. $^{19}$F NMR (376 MHz, dmso-d6): δ -112.4. $^{31}$P NMR (200 MHz, dmso-d6): δ 17.8. IR Wavelength ($cm^{-1}$): 3290, 2102, 1698, 1651, 1237, 1094, 833, 756. HR-ESI+: m/z [M+H]+ = 1867.7129 / 1867.7154 (measured/theoretical).

Preparation of L19-C3:

**(2R)-2-[(5$S_a$)-5-[4-[2-[4-[[4-[[(2S)-2-[[(2S)-2-[[2-[2-[2-(2-azidoethoxy)ethoxy]ethoxy]acetyl]amino]-3-methyl-**butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

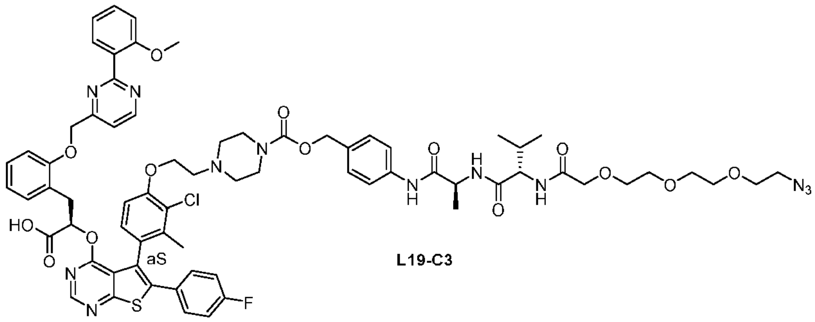

L19-C3

*Step 1: [4-[[(2S)-2-[[(2S)-2-(9H-fluoren-9-ylmethoxycarbonylamino)-3-methyl-butanoyl]amino]propanoyl]amino]phenyl]methyl (4-nitrophenyl) carbonate*

To a suspension of 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate (1 g, 1.66 mmol) in a THF/Dichloromethane mixture (respectively 100 and 30 mL), were successively added pyridine (269 μL, 3.32 mmol) and 4-Nitrophenyl chloroformate (670 mg, 3.30 mmol). The reaction was stirred at room temperature overnight and another portion of 4-Nitrophenyl chloroformate was added (335 mg, 1.66 mmol). The reaction was stirred at room temperature for 3h, concentrated and the residue was purified by silica gel chromatography (gradient of ethyl acetate in heptane) to afford [4-[[(2S)-2-[[(2S)-2-(9H-fluoren-9-ylmethoxycarbonylamino)-3-methyl-butanoyl]amino]propanoyl]amino]phenyl]methyl (4-nitrophenyl) carbonate (658 mg, 0.97 mmol). $^1$H NMR (400 MHz, dmso-d6): δ 10.07 (m, 1H), 8.31 (d, 2H), 8.19 (d, 1H), 7.89 (d, 2H), 7.74 (t, 2H), 7.64 (d, 2H), 7.57 (d, 2H), 7.41 (m, 2H), 7.41 (d, 2H), 7.4 (m, 1H), 7.32 (t, 2H), 5.24 (s, 2H), 4.43 (m, 1H), 4.36-4.19 (m, 3H), 3.92 (dd, 1H), 2 (m, 1H), 1.32 (d, 3H), 0.9/0.87 (m, 6 H). IR Wavelength ($cm^{-1}$): 3350-3200, 1760, 1690, 1670, 1630, 1523, 1290.

*Step 2: (2R)-2-[(56S$_a$)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid*

To a solution of (2R)-2-[(5S$_a$)-5-[3-chloro-2-methyl-4-(2-piperazin-1-ylethoxy)phenyl]-6-(4-fluorophenyl)thieno [2,3-d]pyrimidin-4-yl]oxy-3-[2-[[2-(2-methoxyphenyl)pyrimidin-4-yl]methoxy]phenyl]propanoic acid C3 (100 mg, 0.116 mmol) in DMF (1 mL) were successively added [4-[[(2S)-2-[[(2S)-2-(9H-fluoren-9-ylmethoxycarbonylamino)-3-methyl-butanoyl]amino]propanoyl]amino]phenyl]methyl (4-nitrophenyl) carbonate (87 mg, 0.128 mmol) and DIPEA (38 μL, 0.232 mmol). The reaction mixture was stirred at room temperature overnight and concentrated. The residue was taken up in water, filtered affording (2R)-2-[5-[3-chloro-4-[2-[4-[[4-[[(2S)-2-[[(2S)-2-(9H-fluoren-9-ylmethoxycarbonylamino)-3-methyl-butanoyl]amino]propanoyl]amino]phenyl]methoxycarbonyl]piperazin-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (110 mg, 0.078 mmol) used without further purification in the next step. $^1$H NMR (400 MHz, dmso-d6): δ 10.05 (br s, 1 H), 8.88 (d, 1H), 8.57 (s, 1H), 8.23 (d, 1H), 7.88 (d, 2H), 7.75 (m, 1H), 7.74 (2d, 2H), 7.58 (d, 2H), 7.53 (dd, 1H), 7.45 (m, 1H), 7.45 (d, 1H), 7.41 (m, 1H), 7.4 (m, 2H), 7.31 (m, 2H), 7.29 (m, 2H), 7.26 (d, 2H), 7.2 (t, 2H), 7.18 (m, 1H), 7.14 (d, 1H), 7.11 (t, 1H), 7.03 (t, 1H), 6.98 (d, 1H), 6.69 (t, 1H), 6.2 (d, 1H), 5.46 (d, 1H), 5.22 (m, 2H), 4.97 (s, 2H), 4.42 (t, 1H), 4.26 (m, 2H), 4.21 (m, 1H), 4.2 (m, 2H), 3.91 (m, 1H), 3.75 (s, 3H), 3.35/2.45 (m, 2H), 3.29 (m, 4H), 2.73 (t, 2H), 2.44 (m, 4H), 1.99 (m, 1H), 1.8 (s, 3H), 1.29 (d, 3H), 0.88/0.85 (m, 6H). $^{13}$C NMR (100 MHz, dmso-d6): δ 158.3, 152.7, 131.6, 131.4, 131.3, 131.1, 131.1, 128.9, 128.5, 128, 127.6, 125.8, 120.9, 120.5, 120.5, 119.4, 116.4, 116, 112.7, 112.2, 111.1, 69.4, 67.8, 66.5, 66.1, 60.7, 56.8, 56.1, 53.2, 49.6, 47.1, 43.8, 33.3, 30.9, 19.7, 18.9, 18.1.

*Step 3: (2R)-2-[(5S$_a$)-5-[4-[2-[4-[[4-[[(2S)-2-[[(2S)-2-amino-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-djpyrimidin-4-yljoxy-3-[2-[[2-(2-methoxyphenyl)pyrimidin-4-yl]methoxy]phenyl]propanoic acid*

To a solution of (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]phenyl]methoxycarbonyl]piperazin-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (176 mg, 0.125 mmol) in DMF (3 mL) was added dropwise at 0°C piperidine (300 μL, 1.25 mmol). The reaction mixture was stirred at room temperature for 1 h and concentrated. The residue was purified by C18 reverse phase prep-HPLC by direct deposit of the reaction mixture on the Xbridge column and using the $NH_4HCO_3$ method to afford (2R)-2-[(5S$_a$)-5-[4-[2-[4-[[4-[[(2S)-2-[[(2S)-2-amino-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (130 mg, 0.11 mmol). $^1$H NMR (400 MHz, dmso-d6): δ 10.2 (s, 1H), 8.9 (d, 1H), 8.6 (dl, 1H), 8.55 (s, 1H), 7.85 (d, 1H), 7.6 (d, 2H), 7.55 (dd, 1H), 7.45 (m, 2H), 7.25 (d, 2H), 7.25 (m, 4H), 7.2 (m, 3H), 7.15 (d, 1H), 7.1 (t, 1H), 7.05 (t, 1H), 6.95 (d, 1H), 6.65 (t, 1H), 6.15 (d, 1H), 5.4 (dd, 1H), 5.2 (m, 2H), 4.95 (s, 2H), 4.45 (m, 1H), 4.2 (m, 2H), 3.75 (s, 3H), 3.4/2.35 (m, 2H), 3.3 (m, 5H), 2.6 (t, 2H), 2.4 (m, 4H), 2 (m, 3H), 1.8 (s, 3H), 1.3 (d, 3H), 0.9/0.85 (m, 6H). IR Wavelength ($cm^{-1}$): 3600-2500, 1678.

*Step 4: (2R)-2-[(5S$_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-djpyrimidin-4-yljoxy-3-[2-[[2-(2-methoxyphenyl)pyrimidin-4-yl]methoxy]phenyl]propanoic acid L19-C3*

To a solution of 2R)-2-[(5S$_a$)-5-[4-[2-[4-[[4-[[(2S)-2-[[(2S)-2-amino-3-methyl-butanoyl]amino]propanoyl]amino] phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (50 mg, 0.042 mmol) in DMF (0.3 mL) were successively added DIPEA (14μL, 0.085 mmol) , [dimethylamino-(2,5-dioxopyrrolidin-1-yl)oxy-methylene]-dimethyl-ammonium; tetrafluoroborate (14 mg, 0.046 mmol) and a solution of 2-[2-[2-(2-azidoethoxy)ethoxy]ethoxy]acetic acid (28 mg, 0.12 mmol) in DMF (0.5 mL). The reaction mixture was stirred at room temperature for 2h and purified by C18 reverse phase prep-HPLC by direct deposit of the reaction mixture on the Xbridge column and using the $NH_4HCO_3$ method to afford **L19-C3** (22 mg, 0.016 mmol). $^1$H NMR (400 MHz, dmso-d6): δ 10.02 (s, 1H), 8.88 (d, 1H), 8.4 (d, 1H), 7.72 (br s, 1H), 7.58 (s, 1H), 7.58 (d, 2H), 7.53 (d, 1H), 7.45 (d, 1H), 7.45 (t, 1H), 7.38 (d, 1H), 7.29 (dd, 2H), 7.27 (d, 2H), 7.2 (t, 2H), 7.18 (d, 1H), 7.14 (d, 1H), 7.11 (t, 1H), 7.03 (t, 1H), 6.98 (d, 1H), 6.7 (t, 1H), 6.21 (d, 1H), 5.46 (dd, 1H), 5.23 (m, 2H), 4.97 (s, 2H), 4.4 (m, 1H), 4.29 (dd, 1H), 4.22 (m, 2H), 3.94 (s, 2H), 3.75 (s, 3H), 3.65-3.53 (m, 10H), 3.35 (m, 2H), 3.3 (m, 4H), 3.3/2.5 (m, 2H), 2.73 (t, 2H), 2.44 (m, 4H), 2 (m, 1H), 1.81 (s, 3H), 1.3 (d, 3H), 0.88/0.82 (m, 6H). $^{13}$C NMR (100 MHz, dmso-d6): δ 158, 152.7, 131.4, 131.4, 131.3, 131.1, 131.1, 128.9, 128.6, 120.9, 120.7, 119.5, 116.2, 112.5, 112.1, 111.1, 70.4, 70.4, 69.7, 67.5, 66.2, 56.8, 56.7, 56.1, 53.3, 50.4, 49.5, 43.8, 31.7, 19.5, 0.82, 18.3, 18.2. $^{19}$F NMR (376 MHz, dmso-d6): δ

-112.3. IR Wavelength ($cm^{-1}$): 3294, 2104, 1697, 1663, 1288, 1238, 1120, 1076, 1051, 1020, 833,755. HR-ESI+: m/z [M+H]+ = 1395.5083 / 1395.5070 (measured/theoretical).

**Preparation of L15-C5:**

**(2R)-3-[2-[[2-[3-[[[2-[3-[2-[2-[2-[2-[2-[2-[2-[2-[2-[2-[2-(2-azidoethoxy)ethoxy]ethoxy]ethoxy]ethoxy]ethoxy] ethoxy]ethoxy]ethoxy]ethoxy]ethox y]ethoxy]propanoylamino]ethoxy-hydroxy-phosphoryl]oxy-hydroxy-phosphoryl]oxymethyl]phenyl]pyrimidin-4-yl]methoxy]phenyl]-2-[(5S$_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoic acid**

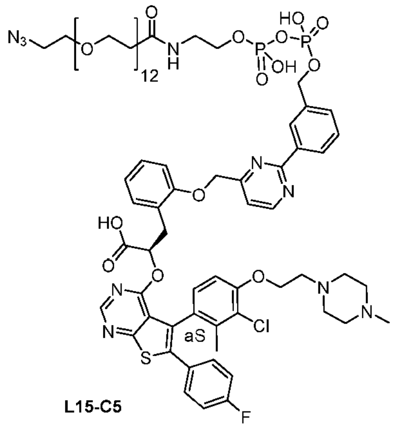

L15-C5

*Step 1: (2R)-2-[(56S$_a$)-5-[3-chloro-2-methyl-4-[2-(4-methylipiperazin-1-yl)ethoxy]phenyl]-6-(4-fluorophenyl)thieno[2,3-d]pyrimidin-4-ylloxy-3-[2-[[2-[3-(phosphonooxymethyl)phenyl]pyrimidin-4-yl]methoxy]phenyl]propanoic acid; bis 2,2,2-trifluoroacetic acid*

To a solution of ethyl (2R)-2-[(5S$_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[3-(hydroxymethyl)phenyl]pyrimidin-4-yl]methoxy]phenyl]propanoate (110 mg, 0.123 mmol; prepared according to WO 2016/207216) in THF (0.5 mL) was added dropwise at -40°C under argon diphosphoryl chloride (51 μL, 0.368 mmol). The reaction mixture was stirred at - 40°C for 30 min. Another portion of diphosphoryl chloride (10 μL, 0.074 mmol) was added at -40°C and the reaction was stirred at -40°C for 20 min, quenched by addition of an aqueous saturated solution of potassium carbonate (0.1 mL) and allowed to warm to room temperature. The pH was adjusted to 10 by addition of potassium carbonate (powder) and the reaction was stirred for 20 min at room temperature. The reaction mixture was acidified to pH 2 by slow addition of aqueous 2 M HCl solution at 0°C, extracted with dichloromethane (4 times). The combined organic layers were concentrated, diluted with dioxane (3 mL) and a solution of lithium hydroxide monohydrate (17 mg, 0.403 mmol) in water (0.3 mL) was added. The reaction mixture was stirred at room temperature for 4 days, neutralized by an aqueous 4 M HCl solution (0.4 mL, 0.4 mmol), and evaporated. The residue was purified by C18 reverse phase prep-HPLC by direct deposit of the reaction mixture on the Xbridge column and using the TFA method to afford (2R)-2-[(5S$_a$)-5-[3-chloro-2-methyl-4-[2-(4-methylpiperazin-1-yl)ethoxy]phenyl]-6-(4-fluorophenyl) thieno[2,3-d]pyrimidin-4-yl]oxy-3-[2-[[2-[3-(phosphonooxymethyl)phenyl]pyrimidin-4-yl]methoxy]phenyl]propanoic acid;2,2,2-trifluoroacetic acid as a 2TFA salt (41 mg, 43 μmol). MS (ESI) m/z [M + 2H]/2+ = 487.5.

*Step 2: 2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy] ethoxy]eth oxy]ethoxy]propanoylamino]ethyl dihydrogen phosphate*

To a solution of 3-[2-[2-[2-[2-[2-[2-[2-[2-[2-[2-[2-(2-azidoethoxy)ethoxy]ethoxy]ethoxy]ethoxy]ethoxy]ethoxy] ethoxy]ethoxy]ethoxy]ethoxy]ethoxy ]propanoic acid (200mg, 0.311 mmol) in dichloromethane (2 mL) were added 1-hydroxypyrrolidine-2,5-dione (79 mg, 0.684 mmol), 3-(ethyliminomethyleneamino)propyl-dimethyl-ammonium; chloride (107 mg, 0.56 mmol). The reaction mixture was stirred at room temperature overnight, diluted with dichloromethane, partitioned with a saturated aqueous solution of $NaHCO_3$ and extracted with dichloromethane. The combined organic layers were washed with brine, dried over Magnesium sulfate and concentrated to approximately 1 mL. The residue was diluted with DMF (1 mL), 2-aminoethyl dihydrogen phosphate (30 mg, 0.214 mmol) was added and the reaction mixture was stirred at 80°C overnight, diluted with dichloromethane, washed with water. The aqueous layer was separated and

freeze-dried to afford 2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]ethyl dihydrogen phosphate (165 mg, 0.2 mmol). $^1H$ NMR (400 MHz, dmso-d6): δ 3.45-3.65 (m, 53H), 3.26-3.39 (m, 2H), 3.12 (m, 2H), 2.27 (t, 2H). HR-ESI+: m/z [M+H]+ = 767.3697 / 767.3686 (measured/theoretical).

*Step 3: (2R)-3-[2-[[2-[3-[[[2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]ethoxy-hydroxy-phosphoryl]oxy-hydroxy-phosphoryl]oxymethyl]phenyl]pyrimidin-4-yl]methoxy]phenyl]-2-[(5S$_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoic acid (L15-C5)*

To a solution of 2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]ethyl dihydrogen phosphate (49 mg, 0.064 mmol) in DMF (0.2 mL) were successively added di(imidazol-1-yl)methanone (11 mg, 0.066 mmol), triethylamine (17μL, 0.066 mmol) and 4Å molecular sieves (50 mg). The reaction was stirred at room temperature for 2 h. The solid was removed by filtration and the filtrate was treated with Zinc chloride (23 mg, 0.172 mmol) and (2R)-2-[(5S$_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[3-(phosphonooxymethyl)phenyl]pyrimidin-4-yl]methoxy]phenyl]propanoic acid; bis 2,2,2-trifluoroacetic acid (41 mg, 0.043 mmol). The mixture was heated to 50°C overnight. The reaction mixture was purified by C18 reverse phase prep-HPLC by direct deposit of the reaction mixture on the Xbridge column and using the $NH_4HCO_3$ method to afford L15-C5 (11 mg, 6μmol). HR-ESI+: m/z [M+H]+ = 1703.5962 / 1703.5959 (measured/theoretical).

**Preparation of L17-C3:**

**(2R)-2-[(5S$_a$)-5-[4-[2-[4-[[4-[[(2S)-2-[[(2S)-2-[[(2S,3R,4S,5R)-6-azido-2,3,4,5-tetrahydroxy-hexyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetic acid**

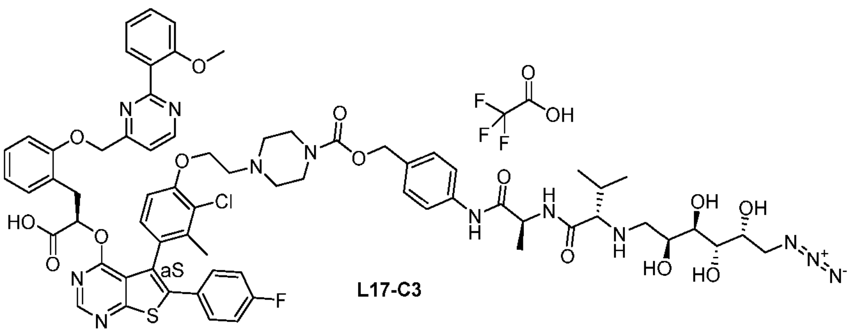

L17-C3

To a solution of (2R)-2-[(5S$_a$)-5-[4-[2-[4-[[4-[[(2S)-2-[[(2S)-2-amino-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (230 mg, 0.194 mmol; obtained according to Step 5 of the preparation of L19-C3) and 6-deoxy-6-azido-D-galactose (120 mg, 0.584 mmol; obtained according to Ekholm et al., ChemMedChem 2016, 11, 2501-2505) in a mixture of DMSO/water 80/20 containing 1 % of DIPEA (20 mL) was added at room temperature sodium cyanoborohydride (24 mg, 0.389 mmol). The reaction mixture was heated at 65°C for 48h. Another portion of sodium cyanoborohydride (24 mg, 0.389 mmol) and 6-deoxy-6-azido-D-galactose (120 mg, 0.584 mmol) were then added at room temperature. The reaction mixture was heated at 65°C for an additional 48h and was purified by C18 reverse phase prep-HPLC by direct deposit of the reaction mixture on the Xbridge column and using the TFA method to afford **L17-C3** (38 mg, 28 μmol). $^1H$ NMR (400 MHz, dmso-d6): δ 13.2 (br s, 1H), 10.2 (s, 1H), 8.88 (d, 1H), 8.85 (d, 1H), 8.62 (s, 1H), 8.53 (br s, 1H), 7.63 (d, 1H), 7.59 (d, 2H), 7.52 (d, 1H), 7.45 (t, 1H), 7.42 (d, 1H), 7.33 (dd, 2H), 7.33 (d, 2H), 7.27 (d, 1H), 7.27 (d, 1H), 7.21 (t, 2H), 7.15 (t, 1H), 7.04 (t, 1H), 7.01 (d, 1H), 6.73 (t, 1H), 6.21 (d, 1H), 5.51 (d, 1H), 5.28/5.22 (m, 2H), 5.04 (br s, 2H), 4.52 (m, 1H), 4.49 (m, 2H), 4.12 (m, 1H), 3.89 (m, 1H), 3.78 (m, 1H), 3.76 (s, 3H), 3.63 (m, 6H), 3.42/3.21 (m, 2H), 3.38 (m, 1H), 3.37 (m, 1H), 3.28/2.52 (m, 2H), 3.22 (m, 4H), 2.96 (m, 2H), 2.21 (m, 1H), 1.86 (s, 3H), 1.36 (d, 3H), 1.03/0.94 (m, 6H). $^{13}C$ NMR (125 MHz, dmso-d6): δ 157.8, 152.5, 131.4, 131.3, 131.3, 130.6, 129.1, 129, 128.8, 120.8, 120.6, 119.4, 116.2, 116.1, 112.3, 111.3, 111.3, 74.2, 71.3, 70.4, 69.5, 69.2, 67.1, 65.6, 64.5, 64.5, 56.2, 54.8,

54.2, 51.9, 50.3, 49.9, 32.7, 29.4, 19.3, 18.9, 18. $^{19}F$ NMR (470 MHz, dmso-d6): δ -74.4, -112.1. IR Wavelength ($cm^{-1}$): 2200-3500, 2104, 1669, 1181, 1132, 798, 758, 720. HR-ESI+: m/z [M+H]+ = 1369.4918 / 1369.4913 (measured/theoretical).

**Preparation of L24-P7:**

**(2R)-2-[(5S$_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl]-4-methyl-piperazin-4-ium-1-yl]ethoxy]-3-chloro-2-ethyl-phenyl]-6-prop-1-ynyl-thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; 2,2,2-trifluoroacetic acid**

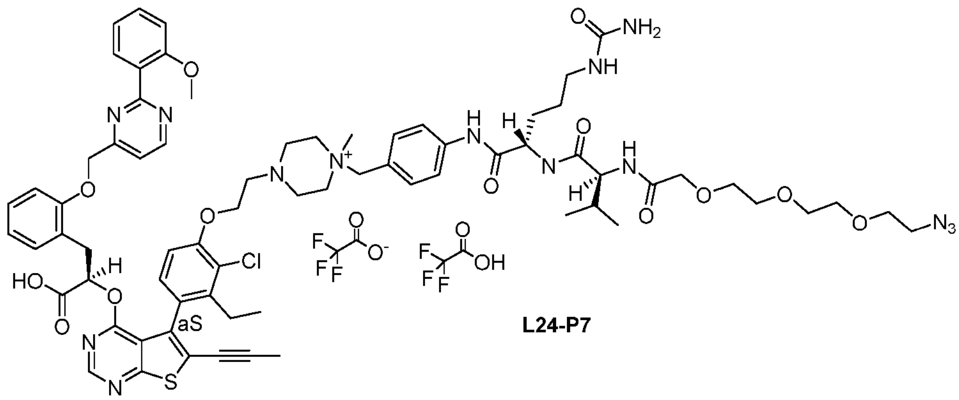

L24-P7

To a solution of (2S)-2-[[(2S)-2-[[2-(2-azidoethoxy)acetyl]amino]-3-methyl-butanoyl]amino]-N-[4-(bromomethyl)phenyl]-5-ureido-pentanamide (72 mg, 0.109 mmol) in THF (5 mL) were successively added (2R)-2-[(5S$_a$)-5-[3-chloro-2-ethyl-4-[2-(4-methylpiperazin-1-yl)ethoxy]phenyl]-6-prop-1-ynyl-thieno[2,3-d]pyrimidin-4-yl]oxy-3-[2-[[2-(2-methoxyphenyl)pyrimidin-4-yl]methoxy]phenyl]propanoic acid **P7** (30 mg, 0.036 mmol) and DIPEA (19 μL, 0.108 mmol). The reaction mixture was stirred overnight at room temperature and was purified by C18 reverse phase prep-HPLC by direct deposit of the reaction mixture on the Xbridge column and using the TFA method to afford **L24-P7** (25 mg, 18μmol). $^1H$ NMR (400 MHz, dmso-d6): δ 10.25 (s, 1H), 8.85 (d, 1H), 8.62 (s, 1H), 8.35 (d, 1H), 7.72 (d, 2H), 7.6 (d, 1H), 7.5 (d, 1H), 7.45 (t, 1H), 7.43 (d, 2H), 7.4 (d, 1H), 7.22 (d, 1H), 7.17 (m, 1H), 7.15 (m, 1H), 7.13 (d, 1H), 7.02 (t, 1H), 7 (d, 1H), 6.78 (t, 1H), 6.3 (d, 1H), 5.98 (br s, 1H), 5.5 (dd, 1H), 5.4 (br s, 1H), 5.28/5.2 (m, 2H), 4.5 (br s, 2H), 4.38 (m, 1H), 4.3 (dd, 1H), 4.25 (m, 2H), 3.94 (br s, 2H), 3.74 (s, 3H), 3.70/3.50 (m, 10H), 3.50 (m, 8 H), 3.35 (t, 2H), 3.22/2.5 (m, 2 H), 3.0 (m, 2H), 2.95 (t, 2H), 2.9 (br s, 3H), 2.55/2.4 (m, 2H), 2.0 (s, 3H), 1.98 (m, 1H), 1.70/1.30 (m, 4H), 0.88/0.82 (m, 6H), 0.72 (t, 3H). IR Wavelength ($cm^{-1}$): 3321, 2111, 1660, 1188, 1124, 798,756,719. HR-ESI+: m/z [M+H-CF3COOH]+ = 1409.59077 / 1409.5903 (measured/theoretical).

**Preparation of L24-P6:**

**(2R)-2-[(5S$_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-[2-(hydroxymethyl)phenyl]pyrimidin-4-yl]methoxy]phenyl]propanoic acid; 2,2,2-trifluoroacetate; 2,2,2-trifluoroacetic acid**

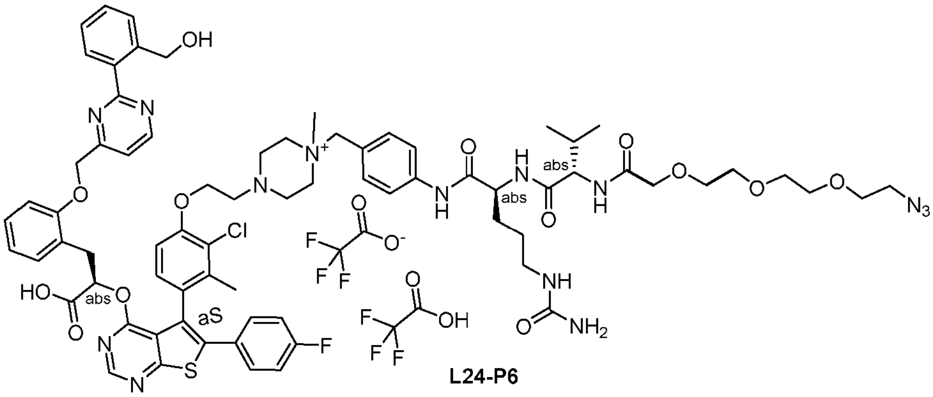

L24-P6

To a solution of (2S)-2-[[(2S)-2-[[2-[2-[2-(2-azidoethoxy)ethoxy]ethoxy]acetyl]amino]-3-methyl-butanoyl]amino]-N-[4-(bromomethyl)phenyl]-5-ureido-pentanamide (55.3 mg, 84 μmol) in DMF (1 mL) were successively added ethyl (2R)-2-[(5$S_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[2-(hydroxymethyl)phenyl]pyrimidin-4-yl]methoxy]phenyl]propanoate (53.2 mg, 59 μmol; synthesized according to EP 2 886 545) and DIPEA (44 μL, 0.252 mmol). The reaction mixture was stirred at room temperature for 1 h and concentrated under reduced pressure. The residue was diluted with dioxane (1 mL) and a solution of lithium hydroxide monohydrate (14 mg, 0.0334 mmol) in water (0.3 mL) was added. The reaction mixture was stirred at room temperature overnight, neutralized by addition of an aqueous 1 M HCl solution (0.33mL, 0.33 mmol), concentrated under reduced pressure. The crude product was purified by C18 reverse phase prep-HPLC by direct deposit of the reaction mixture on the Xbridge column and using the TFA method to afford **L24-P6** (47 mg, 32 μmol). $^{1}H$ NMR (400 MHz, dmso-d6): δ 10.27 (s, 1H), 8.94 (d, 1H), 8.61 (s, 1H), 8.38 (d, 1H), 7.93 (d, 1H), 7.73 (d, 2H), 7.68 (t, 1H), 7.66 (d, 1H), 7.5 (t, 1H), 7.45 (d, 1H), 7.43 (d, 2H), 7.38 (d, 1H), 7.37 (m, 1H), 7.3 (dd, 2H), 7.21 (d, 1H), 7.2 (t, 2H), 7.16 (t, 1H), 7.02 (d, 1H), 6.72 (t, 1H), 6.21 (d, 1H), 6.01 (m, 1H), 5.5 (d, 1H), 5.4 (m, 1H), 5.3 (m, 2H), 4.8 (s, 2H), 4.39 (m, 1H), 4.32 (dd, 1H), 4.25 (m, 2H), 3.95 (s, 2H), 3.57 (m, 16H), 3.42/3.26 (m, 2 H), 3.36 (m, 2H), 3.29/2.51 (m, 2H), 3.11/2.92 (m, 8H), 2.98 (m, 2H), 2.97 (m, 2H), 1.99 (m, 1H), 1.83 (s, 3H), 1.68/1.62 (m, 2H), 1.45/1.39 (m, 2H), 0.88/0.82 (m, 6H). $^{13}C$ NMR (100 MHz, dmso-d6): δ 158.2, 152.1, 134.2, 131.4, 131.3, 130.9, 130.8, 130.2, 128.7, 128.1, 127, 120.8, 119.3, 116.3, 115.7, 112.2, 111, 74, 70.5, 70.1, 69.5, 67.7, 62.3, 58.8, 57.2, 55.5, 54.1, 50.5, 46.6, 38.9, 32.5, 31.5, 29.6, 27.6, 19.6, 18.6, 18.3. $^{19}F$ NMR (376 MHz, dmso-d6): δ -74.6, -112.5. IR Wavelength ($cm^{-1}$): 3303, 2104, 1730, 1662, 1182, 1124, 833,796,761. HR-ESI+: m/z [M+2H]/2+ = 726.2957 / 726.2941 (measured/theoretical).

**Preparation of L20-C6:**

**(2R)-3-[2-[[2-[2-[[2-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methoxycarbonyl-methyl-amino]ethyl-methylcarbamoyl]oxymethyl]phenyl]pyrimidin-4-yl]methoxy]phenyl]-2-[(5$S_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoic acid**

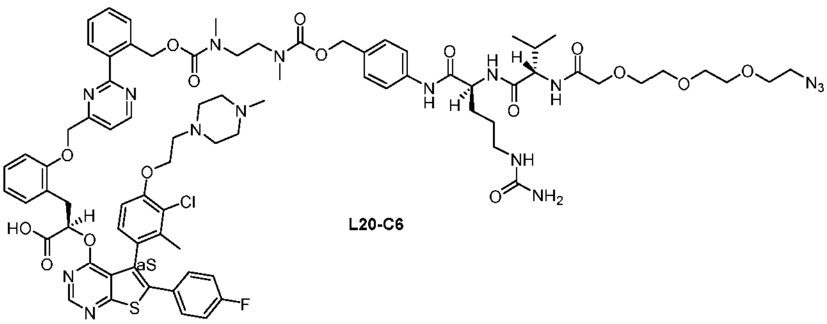

L20-C6

*Step 1: ethyl (2R)-3-[2-[[2-[2-[[2-[tert-butoxycarbonyl(methyl)amino]ethyl-methylcarbamoyl]oxymethyl]phenyl]pyrimidin-4-yl]methoxy]phenyl]-2-[(5$S_a$)-5-[3-chloro-2-methyl-4-[2-(4-methylpiperazin-1-yl)ethoxy]phenyl]-6-(4-fluorophenyl) thieno[2,3-d]pyrimidin-4-ylloxy-propanoate*

To a solution of (ethyl (2R)-2-[(5$S_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[2-(hydroxymethyl)phenyl]pyrimidin-4-yl]methoxy]phenyl]propanoate (50 mg, 55 μmol; synthesized according to EP 2 886 545) in dichloromethane (0.5 mL) were successively added 4-Nitrophenyl chloroformate (19 mg, 94 μmol) and DIPEA (69 μL, 0.5 mmol). The reaction mixture was stirred at room temperature for 1 h and tert-butyl N-methyl-N-[2-(methylamino)ethyl]carbamate (54 mg, 0.287 mmol) was added. The mixture was stirred at room temperature overnight, concentrated under reduced pressure. The residue was purified by silica gel chromatography (gradient of methanol in dichloromethane) to afford ethyl (2R)-3-[2-[[2-[2-[[2-[tert-butoxycarbonyl(methyl)amino]ethyl-methylcarbamoyl]oxymethyl]phenyl]pyrimidin-4-yl]methoxy]phenyl]-2-[(5$S_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoate (30 mg, 27 μmol). $^1$H NMR (500 MHz, dmso-d6): δ 9.00 (d, 1H), 8.58 (s, 1H), 7.98 (m, 1H), 7.61 (d, 1H), 7.51 (t, 1H), 7.48 (d, 1H), 7.45 (t, 1H), 7.31 (dd, 2H), 7.31 (d, 1H), 7.22 (t, 2H), 7.18 (t, 1H), 7.17 (d, 1H), 7.02 (d, 1H), 6.76 (t, 1H), 6.32 (d, 1H), 5.52 (dd, 1H), 5.47 (br s, 2H), 5.26 (m, 2H), 4.2 (m, 2H), 4.07 (m, 2H), 3.24/3.17 (2m, 4H), 3.17/2.6 (2m, 2H), 2.77/2.64 (m, 6H), 2.7 (m, 2H), 2.49/2.28 (m, 8H), 2.12 (br s, 3H), 1.87 (s, 3H), 1.3 (3s, 9H), 1.07 (t, 3H). $^{13}$C NMR (125 MHz, dmso-d6): δ 158.2, 152.4, 131, 130.1, 130.1, 129, 128.3, 128.2, 121.5, 121.4, 120.9, 116.3, 115.8, 112, 111.1, 74.1, 69.2, 68.1, 65.6, 61.2, 56.8, 55.2, 53.1, 46.5, 45.9, 34.5, 32.4, 28.3, 17.4, 14.9. $^{19}$F NMR (470 MHz, dmso-d6): δ-112.2. IR Wavelength ($cm^{-1}$): 1750, 1693, 1221/1160/1120, 834/756.

*Step 2: 2R)-3-[2-[[2-[2-[[2-[[4-[[(2S)-2-[[(2S)-2-[[2-[2-[2-(2-azidoethoxy)ethoxy]ethoxy]acetyl]amino]-3-methyl-butanoyl] amino]-5-ureido-pentanoyl]amino]phenyl]methoxycarbonyl-methyl-amino]ethyl-methyl-carbamoyl]oxymethyl]phenyl] pyrimidin-4-yl]methoxy]phenyl]-2-[(5Sa)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoic acid **L20-C6***

To a solution of ethyl (2R)-3-[2-[[2-[2-[[2-[tert-butoxycarbonyl(methyl)amino]ethyl-methyl-carbamoyl]oxymethyl] phenyl]pyrimidin-4-yl]methoxy]phenyl]-2-[(5$S_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoate (25 mg, 22 μmol) in dichloromethane (0.5 mL) was added at 0°C trifluoroacetic acid (35 μL, 447 mmol). The reaction mixture was stirred at room temperature for 6h and concentrated under reduced pressure. The residue was diluted with DMF (0.5 mL) and [4-[[(2S)-2-[[(2S)-2-[[2-[2-[2-(2-azidoethoxy)ethoxy] ethoxy]acetyl]amino]-3-methyl-butanoyl]amino]-5-ureido-pentanoyl]amino]phenyl]methyl (4-nitrophenyl)carbonate (20 mg, 22 μmol; obtained according to Step 3 of the preparation of L23-P3) and DIPEA (78 μL, 0.447 mmol) were successively added. The reaction mixture was stirred at room temperature overnight, concentrated under reduced pressure, diluted with dioxane (0.5 mL) and a solution of lithium hydroxide monohydrate (3.7 mg, 89 μmol) in water (0.3 mL) was added. The reaction was stirred at room temperature overnight, neutralized at 0°C by a dropwise addition of an aqueous 1M HCl solution until pH7 and concentrated under reduced pressure.

The crude product was purified by C18 reverse phase prep-HPLC by direct deposit of the reaction mixture on the Xbridge column and using the $NH_4HCO_3$ method to afford **L20-C6** (13 mg, 8 μmol). $^1$H NMR (500 MHz, dmso-d6): δ 8.88 (m, 1H), 8.54 (s, 1 H), 7.97 (d, 1H), 7.77 (m, 1H), 7.6 (d, 2H), 7.5 (m, 1H), 7.47 (m, 1H), 7.46 (m, 1H), 7.41 (d, 1H), 7.29 (dd, 2H), 7.21 (t, 2H), 7.19 (d, 1H), 7.18 (m, 2H), 7.12 (t, 1H), 6.97 (d, 1H), 6.7 (t, 1H), 6.19 (d, 1H), 5.49 (d, 1H), 5.45 (m, 4H), 5.23 (m, 2H), 4.89 (m, 2H), 4.4 (m, 1H), 4.32 (dd, 1H), 4.22 (m, 2H), 3.94 (s, 2H), 3.56 (m, 10H), 3.39/2.44 (m, 2H), 3.34 (t, 2H), 3.28 (m, 4H), 2.99 (m, 2H), 2.75/2.7 (m, 6H), 2.73 (m, 2H), 2.5/2.37 (m, 8H), 2.18 (s, 3H), 2.04 (m, 1H), 1.81 (s, 3H), 1.74/1.62 (m, 2H), 1.46/1.38 (m, 2H), 0.86/0.8 (m, 6H). $^{13}$C NMR (125 MHz, dmso-d6): δ 158.3, 152.9, 131.5, 131.4, 131.3, 131, 130, 128.3, 128.3, 128, 127.7, 120.8, 119.3, 116.2, 115.6, 112.1, 111.1, 75.3, 70.5, 70.2, 69.2, 67.6, 66.6, 65.4, 57.2, 56.7, 55.1/52.9, 54, 50.5, 46.5, 45.1, 39.1, 34.4, 31.5, 29.6, 27.4, 19.9, 18.2, 18. $^{19}$F NMR (470 MHz, dmso-d6): δ-112.5. IR Wavelength ($cm^{-1}$): 3323, 2106, 1691, 1660, 1220, 1120, 1051, 759. HR-ESI+: m/z [M+H]+ = 1609.6517 / 1609.6500 (measured/theoretical).

**Preparation of L22-C1:**

**(2R)-2-[(5Sa)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]propanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate;2,2,2-trifluoroacetic acid**

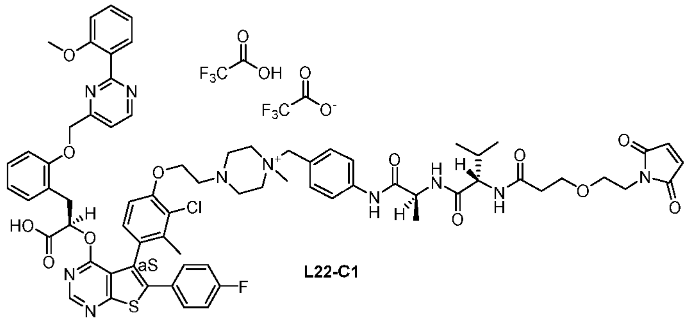

L22-C1

*Step 1: (2R)-2-[(5S$_a$)-5-[4-[2-[4-[[4-[[(2S)-2-[[(2S)-2-amino-3-methyl-butanoyl]amino]propanoyl]amino]phenyl] methyl]-4-methyl-piperazin-4-ium-1-yljethoxyj-3-chloro-2-methyl-phenylj-6-(4-fluorophenyl)thieno[2,3-d]pyrimidin-4-yljoxy-3-[2-[[2-(2-methoxyphenyl)pyrimidin-4-yljmethoxyjphenyljpropanoic acid; 2,2,2-trifluoroacetate; bis-2,2,2-trifluoroacetic acid*

To a solution of 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate (200 mg, 0.388 mmol) in DMF (20 mL) were successively added triphenylphosphine (152 mg, 0.581 mmol) and N-Bromosuccinimide (103 mg, 0.581 mmol). The reaction mixture was stirred at room temperature overnight and (2R)-2-[(**5S$_a$**)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid **C1** (302 mg, 345 mmol) and DIPEA (120 μL, 0.691 mmol) were added. The reaction was stirred at room temperature for 2h and diethylamine (49μL, 486 mmol) was added. The reaction was stirred at room temperature for 24h , concentrated under reduced pressure and purified by C18 reverse phase prep-HPLC by direct deposit of the reaction mixture on the Xbridge column and using the TFA method to afford (2R)-2-[(5S$_a$)-5-[4-[2-[4-[[4-[[(2S)-2-[[(2S)-2-amino-3-methyl-butanoyl]amino]propanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis-2,2,2-trifluoroacetic acid (253 mg, 0.220 mmol). $^1$H NMR (400 MHz, dmso-d6): δ 10.4 (s, 1H), 8.89 (d, 1H), 8.75 (d, 1H), 8.61 (s, 1H), 8.08 (large, 3H), 7.72 (d, 2H), 7.63 (d, 1H), 7.52 (d, 1H), 7.46 (t, 1H), 7.45 (d, 2H), 7.39 (d, 1H), 7.31 (dd, 2H), 7.21 (d, 1H), 7.21 (t, 2H), 7.15 (d, 1H), 7.15 (t, 1H), 7.04 (t, 1H), 7.01 (d, 1H), 6.72 (t, 1H), 6.22 (d, 1H), 5.5 (dd, 1H), 5.25 (m, 2H), 4.53 (m, 2H), 4.52 (m, 1H), 4.28 (m, 2H), 3.76 (s, 3H), 3.62 (m, 1H), 3.43/3.29 (m, 4H), 3.28/2.5 (m, 2H), 3.13/2.94 (m, 4H), 3.01 (m, 2H), 2.9 (br s, 3H), 2.07 (m, 1H), 1.84 (d, 3H), 1.36 (d, 3H), 0.95 (d, 6H). $^{13}$C NMR (125 MHz, dmso-d6): δ 253, 158.2, 134.3, 131.5, 131.4, 131.4, 131.3, 131, 128.9, 121.1, 120.6, 119.5, 116.3, 115.9, 113, 112.3, 111.1, 74.1, 69.8, 67.5, 58.7, 57.9, 56.5, 55.4, 49.8, 46.5, 45.2, 32.9, 30.4, 18.6, 18.4, 18.3. $^{19}$F NMR (470 MHz, dmso-d6): δ -74, -112.6.

*Step 2: (2R)-2-[(5S$_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]propanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis-2,2,2-trifluoroacetic acid **L22-C1***

To a solution of (2R)-2-[(5S$_a$)-5-[4-[2-[4-[[4-[[(2S)-2-[[(2S)-2-amino-3-methyl-butanoyl]amino]propanoyl]amino] phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis-2,2,2-trifluoroacetic acid (150 mg, 0.130 mmol) in DMF (0.4 mL) was added (2,5-dioxopyrrolidin-1-yl) 3-[2-(2,5-dioxopyrrol-1-yl) ethoxy]propanoate (60 mg, 194 mmol). The reaction mixture was stirred at room temperature for 3h, concentrated under reduced pressure and purified by C18 reverse phase prep-HPLC by direct deposit of the reaction mixture on the Xbridge column and using the TFA method to afford L22-C1 (67 mg, 37 μmol). $^1$H NMR (400 MHz, dmso-d6): δ 10.14 (s, 1H), 8.88 (d, 1H), 8.61 (s, 1H), 8.22 (d, 1H), 7.84 (d, 1H), 7.73 (d, 2H), 7.63 (d, 1H), 7.52 (dd, 1H), 7.45 (td, 1H), 7.44 (d, 2H), 7.38 (d, 1H), 7.31 (dd, 2H), 7.21 (d, 1H), 7.21 (t, 2H), 7.15 (t, 1H), 7.14 (d, 1H), 7.02 (t, 1H), 7.01 (d, 1H), 7 (s, 2H), 6.71 (t, 1H), 6.21 (d, 1H), 5.5 (dd, 1H), 5.25 (m, 2H), 4.53 (br s, 2H), 4.38 (m, 1H), 4.25 (m, 2H), 4.19 (dd, 1H), 3.76 (s, 3H), 3.58 (m, 2H), 3.54 (t, 2H), 3.48 (m, 2H), 3.43/3.3 (m, 4H), 3.28/2.51 (m, 2H), 3.16/2.98 (m, 4H), 3.04 (m, 2H), 2.91 (br s, 3H), 2.43/2.33 (m, 2H), 1.93 (m, 1H), 1.84 (s, 3H), 1.31 (d, 3H), 0.87/0.82 (m, 6H). $^{13}$C NMR (100 MHz, dmso-d6): δ 158, 152.8, 135.2, 134, 131.4, 131.3, 131.3, 131.2, 131, 128.9, 120.8, 120.6, 119.3, 116.3, 115.8, 112.4, 112.3, 111.1, 74.2, 69.6, 67.4, 67.4, 67.1, 67, 58.4, 57.9, 56.2, 55.2, 49.7, 46.5, 45.1, 37.1, 36.3, 32.7, 30.9, 19.6, 18.5, 18.2, 18.2. $^{19}$F NMR (376 MHz, dmso-d6): δ -74.6, -112.2. IR Wavelength (cm$^{-1}$): 2000-3500, 1760/1705, 1733, 1668, 1180/1128, 829/798/758/720/696. HR-ESI+: m/z [M+H]+ = 1345.4944 / 1345.4954 (measured/theoretical)

**Preparation of L9-C9:**

**3-[4-[[2-[(2R)-2-carboxy-2-[(5S$_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxyethyl]phenoxy]methyl]pyrimidin-2-yl]benzenesulfonate; 2,2,2-trifluoroacetate; 2,2,2-trifluoroacetic acid**

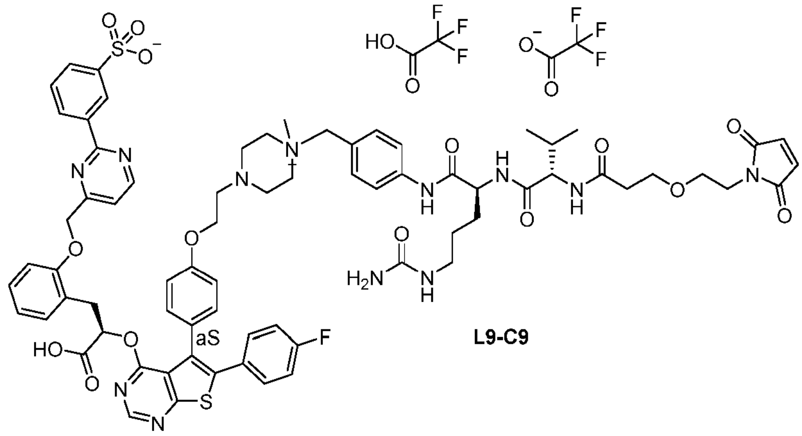

**L9-C9**

*Step 1: (2S)-2-[[(2S)-2-[3-[2-(2,5-dioxopyrrol-1-yl)ethoxy]propanoylamino]-3-methyl-butanoyl]amino]-N-[4-(hydroxymethyl)phenyl]-5-ureido-pentanamide*

To a solution of 3-[2-(2,5-dioxopyrrol-1-yl)ethoxy]propanoic acid (855 mg, 4.01 mmol) in THF (42 mL) were added N,N'-dicyclohexylmethanediimine (1.05 g, 5.08 mmol) and 1-hydroxypyrrolidine-2,5-dione (510 mg, 4.43 mmol). The reaction mixture was stirred at room temperature for 20 h. The precipitate was removed by filtration and the filtrate added to a solution of (2S)-2-[[(2S)-2-amino-3-methyl-butanoyl]amino]-N-[4-(hydroxymethyl)phenyl]-5-ureido-pentanamide (1.27 g, 3.35 mmol) in DMF (42 mL). The reaction mixture was stirred at room temperature for 20 h, diluted with diethyl ether (250 mL). The solid was recovered by filtration to afford (2S)-2-[[(2S)-2-[3-[2-(2,5-dioxopyrrol-1-yl)ethoxy]propanoylamino]-3-methyl-butanoyl]amino]-N-[4-(hydroxymethyl)phenyl]-5-ureido-pentanamide (1.81 g ; 3.15 mmol) as a white solid. $^1$H NMR (400 MHz, dmso-d6): δ 9.87 (s, 1H), 8.05 (d, 1H), 7.82 (d, 1H), 7.53 (d, 2H), 7.21 (d, 2H), 7.00 (s, 2H), 5.95 (t, 1H), 5.39 (s, 2H), 5.07 (t, 1H), 4.41 (d, 2H), 4.34-4.40 (m, 1H), 4.18-4.22 (m, 1H), 3.42-3.65 (m, 4H), 2.88-3.02 (m, 2H), 2.73 (s, 2H), 2.28-2.45 (m, 2H), 1.91-1.99 (m, 1H), 1.53-1.75 (m, 2H), 1.30-1.147 (m, 2H), 0.85 (d, 3H), 0.81 (d, 3H). $^{13}$C NMR (125 MHz, dmso-d6): δ 171.05, 170.83, 170.32, 170.09, 158.82, 137.49, 137.37, 134.50, 126.88, 118.81, 66.66, 66.53, 62.57, 57.49, 53.06, 36.74, 35.76, 30.51, 29.31, 26.79, 25.20, 19.16, 18.07. MS (ESI) m/z [M + H]+ = 575.2.

*Step 2: (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide*

To a solution of (2S)-2-[[(2S)-2-[3-[2-(2,5-dioxopyrrol-1-yl)ethoxy]propanoylamino]-3-methyl-butanoyl]amino]-N-[4-(hydroxymethyl)phenyl]-5-ureido-pentanamide (37.2 mg, 65 μmol) in THF (1 mL) was added dropwise at 0°C under argon phosphorus tribromide (45 μL, 97 mmol). The reaction was stirred at 0°C for 1 h and at room temperature for 2h. The progress of the reaction was followed by UPLC-MS: an aliquot was treated by a large excess of morpholine in acetonitrile, following the formation of the corresponding morpholine adduct. The reaction was diluted with THF (3 mL), quenched by addition of 2 drops of a saturated solution of $NaHCO_3$, stirred for 5 min at room temperature, dried over Magnesium sulfate and filtered. The residue, containing the crude (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (45 mg, 65μmol theoretical) was used immediately in the next step. MS (ESI) m/z [M + H]+ =662.62 (morpholine adduct)

*Step 3: 3-[4-[[2-[(2R)-2-carboxy-2-[(5$S_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl] ethoxy]-2-methyl-phenyl]-6-(4-fluorophenyl)thieno[2,3-djpyrimidin-4-yl]oxy-ethyl]phenoxy]methyl]pyrimidin-2-yl]benzenesulfonate* ***L9-C9***

To a solution of (2R)-2-{[(5$S_a$)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(3-sulfophenyl)pyrimidin-4-yl]methoxy}phenyl)propanoic acid **C9** (15 mg, 16 mmol) in DMF (0.8 mL) was added a solution of (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (45 mg crude, 65 μmol theoretical from step 2) in THF (1 mL) and DIPEA (14μL, 81 μmol). The reaction was stirred at room temperature heated at 50°C for 2h. The crude product was purified by C18 reverse phase prep-HPLC by direct deposit of the reaction mixture on the Xbridge column and using the TFA method to afford **L9-C9** (5.2 mg, 3.5 μmol). HR-ESI+: m/z [M+H]+ = 1481.4917 / 1481.4896 (measured/theoretical).

**Preparation of L9-C13:**

**(2R)-2-[6-(3-amino-4,5-difluoro-phenyl)-(5$S_a$)-5-[3-chloro-4-[2-[4-[[4-[[(2S)-2-[[(2S)-2-[3-[2-(2,5-dioxopyrrol-1-yl) ethoxy]propanoylamino]-3-methyl-butanoyl]amino]-5-ureido-pentanoyl]amino]phenyl]methyl]-4-methyl-piperazin-4-ium-1-yl]ethoxy]-2-methyl-phenyl]thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate;2,2,2-trifluoroacetic acid**

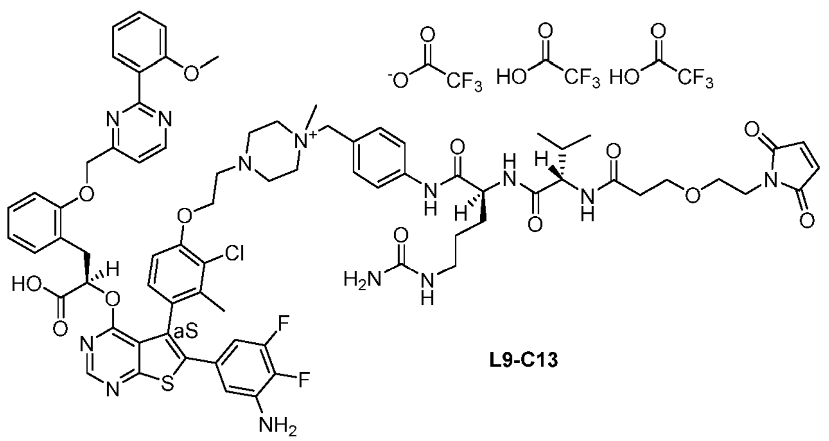

**L9-C13**

The procedure is as in the process of synthesis of **L9-C9,** replacing **C9** used in Step 3 by (2R)-2-{[(5$S_a$)-6-(3-amino-4,5-difluorophenyl)-5-{3-chloro-2-methyl-4-[2-(4-methylpiperazin-1-yl)ethoxy]phenyl}thieno[2,3-d]pyrimidin-4-yl] oxy}-3-(2-{[2-(2-methoxyphenyl)pyrimidin-4-yl]methoxy}phenyl)propanoic acid **C13** and using TFA method for purification. $^1$H NMR (400 MHz, dmso-d6): δ 10.2 (s), 8.9 (d, 1H), 8.6 (s, 1H), 8.12 (d), 7.8 (d), 7.7 (d, 2H), 7.6 (d, 1H), 7.5 (d, 1H), 7.45 (d, 2H), 7.42 (m, 1H), 7.32 (d, 1H), 7.2 (d, 1H), 7.18 (m, 1H), 7.18 (m, 1H), 7.02 (d, 1H), 7 (s, 2H), 7 (m, 1H), 6.75 (t, 1H), 6.65 (d, 1H), 6.25 (d, 1H), 6.15 (dd, 1H), 5.98 (m, 1H), 5.48 (dd, 1H), 5.4 (br s, 1H), 5.24 (dd, 2H), 4.51 (br s, 2H), 4.38 (m, 1H), 4.28 (m, 2H), 4.22 (m, 1H), 3.80-3.40 (m, 8H), 3.75 (s, 3H), 3.26 (m, 4H), 3.1 (m, 2H), 2.98 (m, 4H), 2.9 (br s, 3H), 2.9/2.5 (2m, 2H), 2.43/2.3 (2m, 2H), 1.92 (m, 1H), 1.88 (s, 3H), 1.70-1.30 (m, 4H), 0.82 (2d, 6H). $^{19}$F NMR (470 MHz, dmso-d6): δ -74.3,-139.3, -160.4. HR-ESI+: m/z [M+H]+ = 1464.5482 / 1464.5449 (measured/theoretical).

**Preparation of L14-C3:**

**(2S,3S,4R,5R,6S)-6-[2-[(5$S_a$)-5-[[(2S)-2-[[(2S)-2-[[2-(2-azidoethoxy)acetyl]amino]-3-methyl-butanoyl]amino]propanoyl]amino]-2-[[4-[2-[4-[4-[(1R)-1-carboxy-2-[2-[[2-(2-methoxyphenyl)pyrimidin-4-yl]methoxy]phenyl] ethoxy]-6-(4-fluorophenyl)thieno[2,3-d]pyrimidin-5-yl]-2-chloro-3-methyl-phenoxy]ethyl]piperazine-1-carbonyl]oxymethyl]phenyl]ethyl]-3,4,5-trihydroxy-tetrahydropyran-2-carboxylic** acid

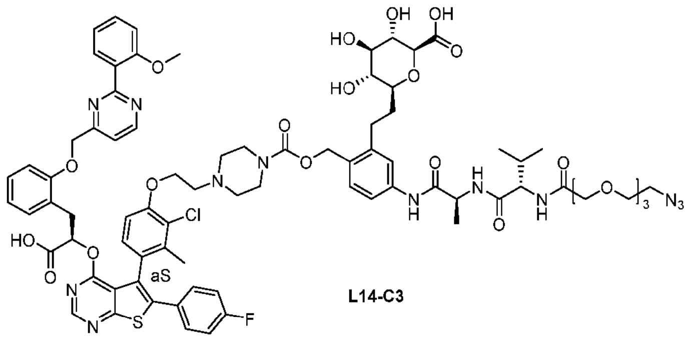

L14-C3

*Step 1: 2-iodo-4-nitro-benzoic acid*

To a solution of 2-amino-4-nitro-benzoic acid (10.0 g, 54.90 mmol) in acetonitrile (280 mL) was added p-toluenesulfonic acid monohydrate (32.0 g, 168.2 mmol). The mixture was stirred at room temperature for 15 min, then a solution of sodium nitrite (8.00 g, 115.9 mmol) and potassium iodide (24.0 g, 144.6 mmol) in solution in water (140 mL) were added dropwise in 15 min. The reaction mixture was stirred for 19 h. After completion of the reaction, the mixture was quenched with sodium thiosulfate (13.02 g, 82.36 mmol) and acidified with an aqueous solution of hydrogen chloride 3 M (25 mL). The aqueous layer was extracted with ethyl acetate (2 x 250 mL) and the combined organic layers were washed with an aqueous solution of hydrogen chloride 1 M (100 mL), dried over sodium sulfate, filtered and concentrated to dryness. The resulting residue was taken up in dichloromethane (1 L) and was washed with an aqueous solution of HCl 1 M (100 mL). The organic layer was dried over sodium sulfate, filtered and concentrated to dryness to afford 2-iodo-4-nitro-benzoic acid (15.0 g, 51.2 mmol) as an orange powder. $^1$H NMR (400 MHz, dmso-d6): δ 13.8 (br s, 1H), 8.64 (s, 1H), 8.27 (d, 1H), 7.86 (d, 1H).

*Step 2: (2-iodo-4-nitro-phenyl)methanol*

To a solution of 2-iodo-4-nitro-benzoic acid (5.0 g, 17.06 mmol) in THF (70 mL) was added a solution of borane 1 M in THF (85 mL, 85.0 mmol). The reaction mixture was stirred at 65°C for 4 h. After the completion of the reaction, the reaction mixture was cooled to room temperature and was quenched with the addition of methanol (200 mL). The mixture was stirred at room temperature for 30 min, then was concentrated to dryness. The crude product was purified by silica gel chromatography (gradient of ethyl acetate in cyclohexane) to afford (2-iodo-4-nitro-phenyl)methanol (3.38 g, 12.11 mmol) as a yellow solid. $^1$H NMR (400 MHz, dmso-d6): δ 8.54 (d, 1H), 8.29 (dd, 1H), 7.70 (d, 1H), 5.82 (t, 1H), 4.47 (d, 2H).

*Step 3: (4-amino-2-iodo-phenyl)methanol*

To a solution of (2-iodo-4-nitro-phenyl)methanol (3.70 g, 13.26 mmol) in ethanol (100 mL) and water (25 mL) were successively added iron (3.70 g, 66.25 mmol) and ammonium chloride (800 mg, 14.96 mmol). The reaction mixture was stirred for 3 hours at 80°C. After completion of the reaction, the reaction mixture was filtered over Celite®, washed with ethanol and concentrated to dryness. The resulting residue was taken up in ethyl acetate (100 mL) and washed with a saturated solution of sodium hydrogen carbonate (100 mL). The organic layer was dried over sodium sulfate, filtered and concentrated to dryness to afford (4-amino-2-iodo-phenyl)methanol (2.48 g, 9.95 mmol) as a yellow oil. $^1$H NMR (400 MHz, dmso-d6): δ 7.02-7.10 (m, 2H), 6.57 (d, 1H), 5.16 (s, 2H), 4.97 (t, 1H), 4.28 (d, 2H).

*Step 4: 4-[[tert-butyl(dimethyl)silyl]oxymethyl]-3-iodo-aniline*

To a solution of (4-amino-2-iodo-phenyl)methanol (3.51 g, 13.37 mmol) in dichloromethane (150 mL) was added imidazole (0.95 g, 13.95 mmol). The mixture was cooled to 0°C, then a solution of tert-butyl-chloro-dimethyl-silane (2.40 mL, 13.85 mmol) in dichloromethane (150 mL) was added dropwise over 15 minutes. The ice bath was removed, and the reaction mixture was stirred at room temperature for 16 h. After completion of the reaction, the reaction mixture was quenched with methanol (20 mL) and concentrated to dryness. The crude product was purified by silica gel chromatography (gradient of ethyl acetate in cyclohexane) to afford 4-[[tert-butyl(dimethyl)silyl]oxymethyl]-3-iodo-aniline (3.64 g ; 10.03 mmol ; 75%) as a yellow oil. $^1$H NMR (400 MHz, dmso-d6): δ 7.05 (s, 1H), 7.03 (d, 1H), 6.55 (d, 1H), 5.24 (s, 2H), 4.46 (s, 2H), 0.88 (s, 9H), 0.06 (s, 6H).

*Step 5: (2S)-2-[[(2S)-2-(9H-fluoren-9-ylmethoxycarbonylamino)-3-methyl-butanoyl]amino]propanoic acid*

To a solution of (2S)-2-aminopropanoic acid (3.22 g, 36.09 mmol) in water (90 mL) were successively added sodium carbonate (7.29 g, 68.74 mmol) and a solution of (2,5-dioxopyrrolidin-1-yl) (2S)-2-(9H-fluoren-9-ylmethoxycarbonylamino)-3-methyl-butanoate (15.0 g, 34.37 mmol) in dimethoxyethane (90 mL). The reaction mixture was stirred for 16 h at room temperature. After completion of the reaction, the mixture was acidified until pH=1 with an aqueous solution of hydrogen chloride 1 M, then the aqueous layer was extracted with ethyl acetate (3 x 500 mL). The combined organic layers were dried over sodium sulfate, filtered and concentrated to dryness to afford the crude mixture which was triturated with diethyl ether (50 mL) to afford (2S)-2-[[(2S)-2-(9H-fluoren-9-ylmethoxycarbonylamino)-3-methyl-butanoyl]amino]propanoic acid (11.25 g, 27.41 mmol) as a white solid. $^1$H NMR (400 MHz, dmso-d6) δ 12.48 (s, 1H), 8.21 (d, 1H), 7.89 (d, 2H), 7.72-7.79 (m, 2H), 7.28-7.46 (m, 5H), 4.15-4.32 (m, 4H), 3.90 (t, 1H), 1.90-2.02 (m, 1H), 1.28 (d, 3H), 0.86-0.90 (m, 6H).

*Step 6: 9H-fluoren-9-ylmethyl N-[(1S)-1-[[(1S)-2-[4-[[tert-butyl(dimethyl)silyl]oxymethyl]3-iodo-anilino]-1-methyl-2-oxo-ethyl]carbamoyl]-2-methyl-propyl]carbamate*

To a solution of (2S)-2-[[(2S)-2-(9H-fluoren-9-ylmethoxycarbonylamino)-3-methyl-butanoyl]amino]propanoic acid (1.50 g, 3.65 mmol) in dichloromethane (18 mL) and methanol (18 mL) were successively added 4-[[tert-butyl(dimethyl)silyl]oxymethyl]-3-iodoaniline (1.33 g, 3.65 mmol) and ethyl 2-ethoxy-2H-quinoline-1-carboxylate (1.36 g, 5.48 mmol). The colorless suspension was stirred for 16 h at room temperature. After concentration to dryness, the crude product was purified by silica gel chromatography (gradient of ethyl acetate in cyclohexane) and then by C18 chromatography (gradient of methanol in water) to afford 9H-fluoren-9-ylmethyl N-[(1S)-1-[[(1S)-2-[4-[[tert-butyl(dimethyl)silyl] oxymethyl]-3-iodo-anilino]-1-methyl-2-oxo-ethyl]carbamoyl]-2-methylpropyl]carbamate (1.18 g, 1.56 mmol) as a white solid. $^1$H NMR (400 MHz, dmso-d6): δ 10.05 (s, 1H). 8.16-8.24 (m, 2H), 7.88 (d, 2H), 7.71-7.77 (m, 2H), 7.55 (d, 1H), 7.37-7.48 (m, 3H), 7.27-7.37 (m, 3H), 4.56 (s, 2H), 4.38 (t, 1H), 4.18-4.33 (m, 3H), 3.91 (t, 1H), 2.08-2.20 (m, 1H), 1.30 (d, 3H), 0.83-0.95 (m, 15H), 0.06 (s, 6H).

*Step 7: (3R,4S,5R,6R)-3,4,5-tribenzyloxy-6-(benzyloxymethyl)tetrahydropyran-2-one*

A suspension of (3R,4S,5R,6R)-3,4,5-tribenzyloxy-6-(benzyloxymethyl)tetrahydropyran-2-ol (30.0 g, 55.49 mmol) in DMSO (120 mL) was stirred for 30 min at room temperature (until full solubilisation) then acetic anhydride (90 mL) was added dropwise at room temperature over 15 min. The beige solution was stirred for 16 h then was cooled to 0°C and an aqueous solution of hydrogen chloride 1 M (100 mL) was slowly added. The reaction mixture was stirred for 20 min at room temperature then acetic acid was evaporated. The resulting residue was diluted with water (200 mL) and ethyl acetate (200 mL). The aqueous layer was extracted with ethyl acetate (2 x 200 mL) and the combined organic layers were washed with water (2 x 500 mL), with a saturated solution of sodium hydrogen carbonate (2 x 500 mL), then dried over sodium sulfate, filtered and concentrated to dryness to afford the crude mixture. The crude product was purified by silica gel chromatography (gradient of ethyl acetate in cyclohexane) to afford (3R,4S,5R,6R)-3,4,5-tribenzyloxy-6-(benzyloxymethyl)tetrahydropyran-2-one (25.05 g, 46.51 mmol) as a colorless oil. $^1$H NMR (400 MHz, dmso-d6): δ 7.19-7.39 (m, 20H), 4.85 (d, 1H), 4.57-4.72 (m, 5H), 4.46-4.56 (m, 3H), 4.36 (d, 1H), 3.98-4.05 (m, 1H), 3.84-3.92 (m, 1H), 3.65-3.76 (m, 2H).

*Step 8: (3R,4S,5R,6R)-3,4,5-tribenzyloxy-6-(benzyloxymethyl)-2-(2-trimethylsilylethynyl)tetrahydropyran-2-ol*

To a solution of trimethylsilylacetylene (24 mL, 168.6 mmol) in THF (325 mL) was added in 20 min at -78°C a solution of butyllithium 2.5 M in hexane (59.41 mL, 148.5 mmol). The colorless solution was stirred for 45 min at -78°C and then 45 min at 0°C. The reaction mixture was cooled to -78°C and a solution of (3R,4S,5R,6R)-3,4,5-tribenzyloxy-6-(benzyloxymethyl)tetrahydropyran-2-one (25.0 g, 46.41 mmol) in THF (325 mL) was added dropwise over 45 min. The reaction mixture was stirred for 4 h at this temperature then was quenched with water (200 mL). The aqueous layer was extracted with ethyl acetate (2 x 200 mL). The combined organic layers were dried over sodium sulfate, filtered and concentrated to dryness to afford (3R,4S,5R,6R)-3,4,5-tribenzyloxy-6-(benzyloxymethyl)-2-(2-trimethylsilylethynyl)tetrahydropyran-2-ol (29.56 g, 46.41 mmol) as a beige oil containing the two diastereoisomers in a ratio 4/6. $^1$H NMR (400 MHz, dmso-d6): δ 7.13-7.43 (m, 20H), 4.87-4.99 (m, 1H), 4.65-4.83 (m, 4H), 3.43-3.57 (m, 3H), 3.70-3.85 (m, 2H), 3.55-3.68 (m, 3H), 3.43-3.53 (m, 2H), 0.11-0.22 (m, 9H).

*Step 9: trimethyl-[2-[(2S,3S,4R,5R,6R)-3,4,5-tribenzyloxy-6-(benzyloxymethyl)tetrahydropyran-2-yl]ethynyl]silane*

To a solution of (3R,4S,5R,6R)-3,4,5-tribenzyloxy-6-(benzyloxymethyl)-2-(2-trimethylsilylethynyl)tetrahydropyran-2-ol (29.56 g, 46.42 mmol) in acetonitrile (83 mL) and dichloromethane (193 mL) were added in 20 min at

-15°C a solution of triethylsilane (44.98 mL, 278.5 mmol) in a mixture of acetonitrile/dichloromethane (37 mL/18 mL) followed by a solution of boron trifluoride diethyl etherate (23.53 mL, 185.7 mmol) in acetonitrile (37 mL) over 30 min at -15°C. The colorless solution was stirred for 5 h at the same temperature, then was diluted with water (500 mL). The aqueous layer was extracted with ethyl acetate (2 x 500 mL). The combined organic layers were dried over sodium sulfate, filtered and concentrated to dryness to afford trimethyl-[2-[(2S,3S,4R,5R,6R)-3,4,5-tribenzyloxy-6-(benzyloxymethyl) tetrahydropyran-2-yl]ethynyl]silane (28.82 g, 46.41 mmol) as a brown oil. $^{1}$H NMR (400 MHz, dmso-d6): δ 7.10-7.44 (m, 20H), 4.93 (d, 1H), 4.67-4.86 (m, 4H), 4.43-4.57 (m, 3H), 4.16-4.28 (m, 1H), 3.42-3.68 (m, 6H), 0.15 (s, 9H).

*Step 10: (2R,3R,4R,5S,6S)-3,4,5-tribenzyloxy-2-(benzyloxymethyl)-6-ethynyl-tetrahydropyran*

To a solution of trimethyl-[2-[(2S,3S,4R,5R,6R)-3,4,5-tribenzyloxy-6-(benzyloxymethyl)tetrahydropyran-2-yl] ethynyl]silane (28.80 g, 46.39 mmol) in methanol (1.12 L) and dichloromethane (240 mL) was added an aqueous solution of sodium hydroxide 1 M (80 mL). The beige solution was stirred for 1 h at room temperature then was acidified until pH = 1 with an aqueous solution of hydrogen chloride 1 M and diluted with water (500 mL). The methanol was evaporated and then the aqueous layer was extracted with ethyl acetate (2 x 1 L). The combined organic layers were dried over sodium sulfate, filtered and concentrated to dryness. The crude product was purified by silica gel chromatography (gradient of ethyl acetate in cyclohexane) to afford (2R,3R,4R,5S,6S)-3,4,5-tribenzyloxy-2-(benzyloxymethyl)-6-ethynyl-tetrahydropyran (20.00 g, 36.45 mmol) as a colorless oil. $^{1}$H NMR (400 MHz, dmso-d6): δ 3.42-3.67 (m, 7H), 4.17 (d, 1H), 4.44-4.56 (m, 3H), 4.67-4.86 (m, 4H), 4.90 (d, 1H), 7.15-7.40 (m, 20H).

*Step 11: (2S,3R,4R,5S,6R)-2-ethynyl-6-(hydroxymethyl)tetrahydropyran-3,4,5-triol*

To a solution of (2R,3R,4R,5S,6S)-3,4,5-tribenzyloxy-2-(benzyloxymethyl)-6-ethynyl-tetrahydropyran (20.00 g, 36.45 mmol) in ethanthiol (400 mL) was added dropwise at room temperature over 5 min, boron trifluoride diethyl etherate (147.8 mL, 1166 mmol). The beige solution was stirred for 16 h at room temperature, then was cooled to 0°C and equipped with a gas trap containing an aqueous saturated solution of sodium hypochlorite. A saturated aqueous solution of sodium hydrogen carbonate (500 mL) was added dropwise at 0°C in 1 h (formation of carbon dioxide). After concentration to dryness, the crude product was purified by silica gel chromatography (gradient of methanol in dichloromethane) to afford (2S,3R,4R,5S,6R)-2-ethynyl-6-(hydroxymethyl)tetrahydropyran-3,4,5-triol (4.05 g, 21.52 mmol) as a white solid. $^{1}$H NMR (400 MHz, dmso-d6): δ 5.28 (d, 1H), 4.99 (d, 1H), 4.91 (d, 1H), 4.52 (t, 1H), 3.77 (d, 1H), 3.60-3.69 (m, 1H), 3.35-3.43 (m, 1H), 3.32 (s, 1H), 2.97-3.13 (m, 4H).

*Step 12: methyl (2S,3S,4R,5R,6S)-6-ethynyl-3,4,5-trihydroxy-tetrahydropyran-2-carboxylate*

To a solution of (2S,3R,4R,5S,6R)-2-ethynyl-6-(hydroxymethyl)tetrahydropyran-3,4,5-triol (4.05 g, 21.52 mmol) in a saturated aqueous solution of sodium hydrogen carbonate (81 mL) and THF (81 mL) was added (2,2,6,6-tétraméthylpipéridin-1-yl)oxyl (168 mg, 1.08 mmol). The yellow suspension was cooled to 0°C and 1,3-dibromo-5,5-dimethyl-imidazolidine-2,4-dione (12.31 g, 43.04 mmol) was added portionwise in 30 min. The reaction mixture was stirred for 4 h at 0°C then was quenched with the addition of methanol (40 mL). After 30 min stirring at this temperature, a saturated aqueous solution of potassium carbonate (10 mL) and dichloromethane (100 mL) were added. The organic layer was extracted with water (2 x 200 mL) then the combined aqueous layers were acidified until pH = 1 with an aqueous solution of hydrogen chloride 3M and concentrated to dryness. The resulting residue was taken up in methanol (100 mL) and in an aqueous solution of hydrogen chloride 3M (20 mL). The mixture was concentrated to dryness and co-evaporated several times with methanol (4 x 100 mL). The crude product was purified by silica gel chromatography (gradient of methanol in dichloromethane Cerium developer) to afford methyl (2S,3S,4R,5R,6S)-6-ethynyl-3,4,5-trihydroxy-tetrahydropyran-2-carboxylate (3.00 g, 13.88 mmol) as a beige solid. $^{1}$H NMR (400 MHz, dmso-d6): δ 5.46 (d, 1H), 5.32 (d, 1H), 5.18 (d, 1H), 3.93-4.00 (m, 1H), 3.75 (dd, 1H), 3.65 (s, 3H), 3.40-3.44 (m, 1H), 3.31 (s, 1H), 3.09-3.19 (m, 2H).

*Step 13: methyl (2S,3S,4R,5S,6S)-3,4,5-triacetoxy-6-ethynyl-tetrahydropyran-2-carboxylate*

To a solution of methyl (2S,3S,4R,5R,6S)-6-ethynyl-3,4,5-trihydroxy-tetrahydropyran-2-carboxylate (3.00 g, 13.88 mmol) in DMF (37.5 mL) and pyridine (12.5 mL) was added N,N-dimethylpyridin-4-amine (84.8 mg, 0.693 mmol). The reaction mixture was cooled to 0°C then acetic anhydride (20.0 mL, 213 mmol) was added dropwise in 5 min. The colorless solution was stirred for 3 h at room temperature then was diluted with an aqueous solution of hydrogen chloride 1 M (200 mL). The aqueous layer was extracted with ethyl acetate (2 x 200 mL). The combined organic layers were washed with an aqueous solution of hydrogen chloride 1 M (2 x 200 mL), followed with a saturated aqueous solution of potassium carbonate (200 mL), then dried over sodium sulfate, filtered and concentrated to dryness to afford the crude mixture. The crude product was purified by silica gel chromatography (gradient of ethyl acetate in cyclohexane cerium developer) to

afford methyl (2S,3S,4R,5S,6S)-3,4,5-triacetoxy-6-ethynyl-tetrahydropyran-2-carboxylate (4.60 g, 13.44 mmol) as a white solid. $^1$H NMR (400 MHz, dmso-d6): δ 5.33 (t, 1H), 4.93-5.01 (m, 2H), 4.70 (d, 1H), 4.44 (d, 1H), 3.67 (s, 1H), 3.64 (s, 3H), 2.02 (s, 3H), 1.94-2.01 (m, 6H).

*Step 14: methyl (2S,3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethynyl]tetrahydropyran-2-carboxylate*

To a solution of methyl (2S,3S,4R,5S,6S)-3,4,5-triacetoxy-6-ethynyl-tetrahydropyran-2-carboxylate (496 mg, 1.45 mmol) in DMF (7.3 mL) were successively added 9H-fluoren-9-ylmethyl N-[(1S)-1-[[(1S)-2-[4-[[tert-butyl(dimethyl)silyl]oxymethyl]-3-iodo-anilino]-1-methyl-2-oxo-ethyl]carbamoyl]-2-methyl-propyl]carbamate (730 mg, 0.966 mmol), DIPEA (738 μL, 4.47 mmol), copper iodide (18.4 mg, 96.6 mmol) and dichloro-bis-(triphenylphosphine)palladium(II) (67.8 mg, 96.6 mmol). The yellow solution was flushed with Argon then was stirred for 16 h at room temperature. After dilution with water (100 mL), the aqueous layer was extracted with ethyl acetate (2 x 100 mL). The combined organic layers were washed with water (2 x 200 mL), and with a saturated aqueous solution of ammonium chloride (2 x 200 mL), then dried over sodium sulfate, filtered and concentrated to dryness. The crude product was purified by silica gel chromatography (gradient of ethyl acetate in cyclohexane) to afford methyl (2S,3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethynyl]tetrahydropyran-2-carboxylate (782 mg, 0.806 mmol) as a yellow solid. $^1$H NMR (400 MHz, dmso-d6): δ 10.09 (s, 1H). 8.20 (d, 1H), 7.89 (d, 2H), 7.70-7.78 (m, 3H), 7.55 (d, 1H), 7.32-7.46 (m, 4H), 7.27-7.32 (m, 2H), 5.41 (t, 1H), 4.96-5.14 (m, 3H), 4.67 (s, 2H), 4.51 (d, 1H), 4.36-4.44 (m, 1H), 4.16-4.32 (m, 3H), 3.88-3.95 (m, 1H), 3.64 (s, 3H), 1.94-2.07 (m, 10H), 1.30 (d, 3H), 0.84-0.93 (m, 15H), 0.08 (s, 6H).

*Step 15: methyl (3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethyl]tetrahydropyran-2-carboxylate*

A solution of methyl (2S,3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethynyl]tetrahydropyran-2-carboxylate (750 mg, 0.773 mmol) in THF (15 mL) was flushed with Argon. Dry Platinum 5% on carbon (75 mg, 50% $^w/_w$) was added. The reaction mixture was successively flushed with argon, with $H_2$ and was stirred for 16 h at room temperature under $H_2$ atmosphere (P atm). The reaction mixture was filtered through a Celite® pad, washed with THF then concentrated to dryness. The complete sequence, (addition of dry platinum 5% on carbon (75 mg, 50% $^w/_w$), stirring for 16 h at room temperature under $H_2$ atmosphere (1 bar) and filtration through a Celite® pad), was performed 4 more times. The crude product was purified by silica gel chromatography (gradient of ethyl acetate in cyclohexane) to afford methyl (3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethyl]tetrahydropyran-2-carboxylate (470 mg, 0.483 mmol) as a white solid. $^1$H NMR (400 MHz, dmso-d6): δ 9.90 (s, 1H), 8.16 (d, 1H), 7.89 (d, 2H), 7.70-7.78 (m, 2H), 7.37-7.49 (m, 4H), 7.27-7.32 (m, 3H), 7.23 (d, 1H), 5.29 (t, 1H), 4.95 (t, 1H), 4.78 (t, 1H), 4.60 (s, 2H), 4.34-4.44 (m, 2H), 4.16-4.32 (m, 3H), 3.88-3.95 (m, 1H), 3.72-3.79 (m, 1H), 3.64 (s, 3H), 2.69-2.78 (m, 1H), 2.50-2.60 (m, 1H), 1.92-2.03 (m, 10H), 1.55-1.75 (m, 2H), 1.30 (d, 3H), 0.84-0.93 (m, 15H), 0.05 (s, 6H).

*Step 16: methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-(hydroxymethyl)phenyl]ethyl]tetrahydropyran-2-carboxylate*

To a solution of methyl (3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethyl]tetrahydropyran-2-carboxylate (470 mg, 0.483 mmol) in THF (540 μL) and water (540 μL) was added acetic acid (1.6 mL, 28.28 mmol). The colorless solution was stirred for 16 h at room temperature then diluted with water (100 mL). The aqueous layer was extracted with ethyl acetate (2 x 100 mL). The combined organic layers were washed with water (2 x 200 mL), and with a saturated aqueous solution of sodium hydrogen carbonate (200 mL), then were dried over sodium sulfate, filtered and concentrated to dryness. The crude product was purified by silica gel chromatography (gradient of ethyl acetate in cyclohexane) to afford methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-(hydroxymethyl)phenyl]ethyl]tetrahydropyran-2-carboxylate (354 mg, 0.412 mmol) as a white solid. $^1$H NMR (400 MHz, dmso-d6): δ 9.87 (s, 1H), 8.16 (d, 1H), 7.89 (d, 2H), 7.70-7.78 (m, 2H), 7.37-7.50 (m, 4H), 7.27-7.37 (m, 3H), 7.25 (d, 1H), 5.29 (t, 1H), 4.91-4.98 (m, 2H), 4.78 (t, 1H), 4.34-4.44 (m, 4H), 4.16-4.32 (m, 3H), 3.88-3.95 (m, 1H), 3.72-3.79 (m, 1H), 3.64 (s, 3H), 2.64-2.73 (m, 1H), 2.50-2.60 (m, 1H), 1.92-2.03 (m, 10H), 1.69-1.79 (m, 1H), 1.52-1.65 (m, 1H), 1.30 (d, 3H), 0.84-0.93 (m, 6H).

*Step 17: methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]phenyl]ethyl]tetrahydropyran-2-carboxylate*

To a solution of methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-(hydroxymethyl)phenyl]ethyl]tetrahydropyran-2-carboxylate (310 mg, 0.361 mmol) in THF (7.75 mL) were successively added pyridine (146 μL, 1.80 mmol) and 4-Nitrophenyl chloroformate (182 mg, 0.901 mmol). The white suspension was stirred for 16 h at room temperature then was concentrated to dryness to afford the crude mixture. The crude product was purified by silica gel chromatography (gradient of ethyl acetate in dichloromethane) to afford methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]phenyl]ethyl]tetrahydropyran-2-carboxylate (257 mg, 0.251 mmol) as a white solid. $^1H$ NMR (400 MHz, dmso-d6): δ 10.04 (s, 1H), 8.31 (d, 2H), 8.20 (d, 1H), 7.89 (d, 2H), 7.66-7.78 (m, 2H), 7.56 (d, 2H), 7.28-7.52 (m, 8H), 5.31 (t, 1H), 5.25 (s, 2H), 4.96 (t, 1H), 4.79 (t, 1H), 4.40 (d, 2H), 4.16-4.32 (m, 3H), 3.88-3.95 (m, 1H), 3.74-3.83 (m, 1H), 3.61 (s, 3H), 2.74-2.84 (m, 1H), 2.60-2.71 (m, 1H), 1.90-2.03 (m, 10H), 1.72-1.83 (m, 1H), 1.58-1.71 (m, 1H), 1.30 (d, 3H), 0.82-0.94 (m, 6H). LC-MS: MS (ESI) m/z [M+Na]+ = 1047.6.

*Step 18: (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yljmethoxyjphenyljpropanoic acid*

To a solution of (2R)-2-[(5Sa)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (C3) (118 mg, 0.121 mmol) in dimethylformamide (3.0 mL) were successively added a solution of methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]phenyl]ethyl]tetrahydropyran-2-carboxylate (130 mg, 0.127 mmol) in dimethylformamide (3.0 mL) and DIPEA (60 μL, 0.363 mmol). The reaction mixture was stirred at room temperature for 2 h. (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid was obtained as a solution in dimethylformamide and was used like this in the next step. UPLC-MS: MS (ESI) m/z [M+H]+ = 1745.6+1747.6.

*Step 19: (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid*

To the solution of 2SR,3SR,4RS,5RS,6SR)-6-[2-[(5S$_a$)-5-[[(2S)-2-[[(2S)-2-amino-3-methyl-butanoyl]amino]propanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]piperazine-1-carbonyl]oxymethyl]phenyl]ethyl]-3,4,5-trihydroxy-tetrahydropyran-2-carboxylic acid (0.121 mmol) in DMF (3.0 mL) from step 18 were successively added methanol (2 mL) and lithium hydroxide monohydrate (64.0 mg, 1.52 mmol) in solution in water (2 ml). The reaction mixture was stirred at room temperature for 1 h. The crude product was purified by C18 reverse phase prep-HPLC by direct deposit of the reaction mixture on the Xbridge column and using the $NH_4HCO_3$ method to afford (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (124 mg, 0.0895 mmol) as a white powder. UPLC-MS: MS (ESI) m/z [M+H]+ = 1384.3+1386.3.

*Step 20: (2,3,4,5,6-pentafluorophenyl) 2-[2-[2-(2-azidoethoxy)ethoxy]ethoxy]acetate 2-[2-[2-(2-azidoethoxy)ethoxy]ethoxy]acetic acid*

To a solution of 2-[2-[2-(2-azidoethoxy)ethoxy]ethoxy]acetic acid (75 mg, 0.342 mmol) in solution in THF (500 μL) were added a solution of 2,3,4,5,6-pentafluorophenol (75.5 mg, 0.410 mmol) in THF (500 μL) and a solution of N,N'-dicyclohexylmethanediimine (84.7 mg, 0.410 mmol) in THF (500 μL). The reaction mixture was stirred for 15 h at room

temperature and the progress of the reaction was followed by UPLC-MS. The (2,3,4,5,6-pentafluorophenyl) 2-[2-[2-(2-azidoethoxy)ethoxy]ethoxy]acetate was obtained as a THF solution by simple filtration of the suspension on a small disposable frit. This solution was used without further purification in the next step. UPLC-MS: MS (ESI) m/z [M-N2+H]+ = 372.3.

*Step 21: (2SR,3SR,4RS,5RS,6SR)-6-[2-[(5S$_a$)-5-[[(2S)-2-[[(2S)-2-[[2-[2-[2-(2-azidoethoxy)ethoxy]ethoxy]acetyl]amino]-3-methyl-butanoyl]amino]propanoyl]amino]-2-[[4-[2-[4-[4-[(1R)-1-carboxy-2-[2-[[2-(2-methoxyphenyl) pyrimidin-4-yl]methoxy]phenyl]ethoxy]-6-(4-fluorophenyl)thieno[2,3-d]pyrimidin-5-yl]-2-chloro-3-methyl-phenoxy]ethyl]piperazine-1-carbonyl]oxymethyl]phenyl]ethyl]-3,4,5-trihydroxy-tetrahydropyran-2-carboxylic acid* ***L14-C3***

To the solution of (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno [2,3-d]pyrimidin-4-yl]oxy-3-[2-[[2-(2-methoxyphenyl)pyrimidin-4-yl]methoxy]phenyl]propanoic acid (118 mg, 0.085 mmol) in DMF (500 μL) were successively added the solution of (2,3,4,5,6-pentafluorophenyl) 2-[2-[2-(2-azidoethoxy) ethoxy]ethoxy]acetate (0.342 mmol) in THF from step 20 and DIPEA (42.2μL, 0.256mmol). The reaction mixture was stirred for 1 h at room temperature and the progress of the reaction was followed by UPLC-MS. The crude product was purified by C18 reverse phase prep-HPLC by direct deposit of the reaction mixture on the Xbridge column and using the $NH_4HCO_3$ method to afford **L14-C3** as a white powder. UPLC-MS: MS (ESI) m/z [M+H]+ = 1599.0+1601.2. IR Wavelength ($cm^{-1}$): 3263, 2105, 1652, 1600, 1284/1240/1089, 756. $^1$H NMR(400 MHz, dmso-d6): δ 9.98 (s), 8.85 (d, 1H), 8.52 (s, 1H), 8.38 (d, 1H), 7.93 (d, 1H), 7.56 (d, 1H), 7.5 (t, 1H), 7.49 (d, 1H), 7.47 (d, 1H), 7.44 (d, 1H), 7.42 (s, 1H), 7.3 (dd, 2H), 7.22 (d, 1H), 7.2 (t, 2H), 7.19 (t, 1H), 7.13 (d, 1H), 7.08 (t, 1H), 7.02 (t, 1H), 6.95 (d, 1H), 6.65 (t, 1H), 6.11 (d, 1H), 5.43 (d, 1H), 5.27/5.2 (m, 2H), 4.93 (br s, 2H), 4.38 (m, 1H), 4.35/4.2 (2m, 2H), 4.3 (m, 1H), 3.94 (s, 2H), 3.75 (s, 3H), 3.58 (m, 10H), 3.57 (m, 1H), 3.51/2.29 (2dd, 2H), 3.35 (m, 2H), 3.25 (m, 4H), 3.2 (m, 1H), 3.2 (m, 1H), 3.06 (m, 1H), 2.96 (m, 1H), 2.75 (m, 2H), 2.72/2.5 (m, 2H), 2.41 (m, 4 H), 2 (m, 1H), 1.99/1.6 (m, 2H), 1.8 (s, 3H), 1.3 (d, 3H), 0.88/0.82 (2d, 6H). $^{13}$C NMR (100 MHz, dmso-d6): δ 158.2, 152.7, 131.9, 131.4, 131.4, 131.3, 131.1, 131, 127.8, 120.6, 120.5, 120.1, 116.8, 116.3, 116, 112.6, 112, 111.6, 79.6, 79.6, 78.5, 76.8, 74.2, 73.1, 70.3, 70.3, 69.3, 66.3, 65.1, 56.8, 56.3, 56.1, 52.6, 50.3, 49.4, 43.8, 34.2, 33.5, 31.7, 28, 19.6/18.4, 18.2, 18. $^{19}$F NMR (376 MHz, dmso-d6): δ-112.5. HR-ESI+: m/z [M+H]+ = 1599.5724 (1599.5704) (measured/theoretical)

Preparation of L18-C3:

**(2R)-2-[(5S$_a$)-5-[4-[2-[4-[[4-[[(2S)-2-[[(2S)-2-[[(2R)-2-[[2-(2-azidoethoxy)acetyl]amino]-3-sulfo-propanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl] methoxy]phenyl]propanoic acid**

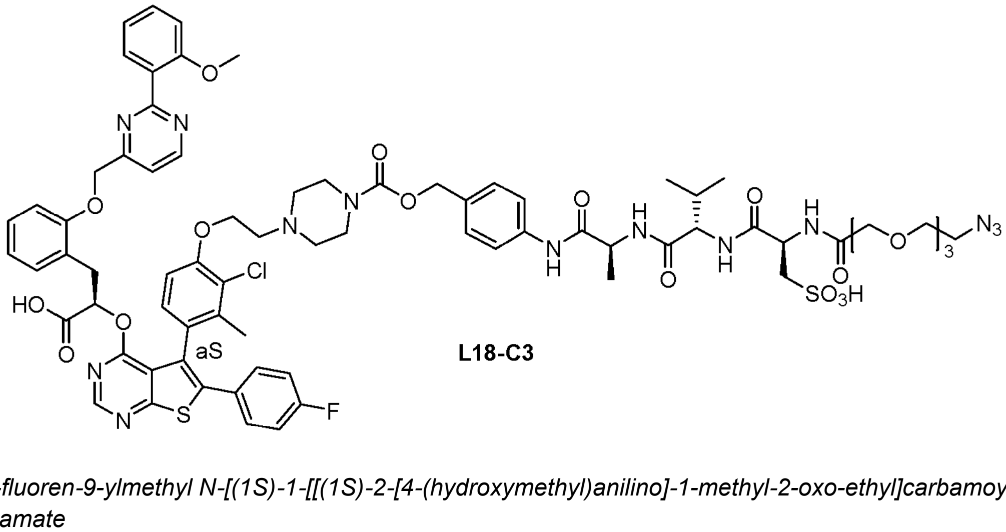

L18-C3

*Step 1: 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate*

To a solution of (2S)-2-[[(2S)-2-(9H-fluoren-9-ylmethoxycarbonylamino)-3-methyl-butanoyl]amino]propanoic

acid (6.0 g, 14.6 mmol; obtained according to Step 5 of the preparation of **L14-C3)** in dichloromethane (70 mL) and methanol (30 mL) were successively added (4-aminophenyl)methanol (2.16 g, 17.5 mmol) and ethyl 2-ethoxy-2H-quinoline-1-carboxylate (5.42 g, 21.93 mmol). The red solution was stirred at room temperature for 16 h (precipitation after few minutes). After completion of the reaction, the reaction mixture was diluted with diethyl ether (70 mL). The resulting precipitate was filtered off and dried to afford 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate (5.16 g, 10.01 mmol) as a beige solid. $^1$H NMR (400 MHz, dmso-d6): δ 9.91 (s, 1H), 8.15 (d, 1H), 7.89 (d, 2H), 7.70-7.78 (m, 2H), 7.53 (d, 2H), 7.38-7.46 (m, 3H), 7.29-7.35 (m, 2H), 7.23 (d, 2H), 5.08 (t, 1H), 4.37-4.50 (m, 3H), 4.16-4.34 (m, 3H), 3.91 (t, 1H), 1.92-2.02 (m, 1H), 1.30 (d, 3H), 0.83-0.91 (m, 6H).

*Step 2: (2S)-2-amino-N-[(1S)-2-[4-(hydroxymethyl)anilino]- 1-methyl-2-oxo-ethyl]-3-methyl-butanamide*

To a solution of 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate (5.16 g, 10.01 mmol) in DMF (120 mL) was added piperidine (52 mL, 525mmol). The reaction mixture was stirred for 2 h at room temperature then the piperidine was evaporated and the resulting solution was diluted with water (500 mL). The resulting solid was filtered off and the filtrate was washed twice with diethyl ether (2 x 500 mL). The aqueous layer was concentrated to dryness to afford the crude reaction mixture. The crude product was purified by silica gel chromatography (gradient of methanol (containing 7M ammonia) in dichloromethane) to afford (2S)-2-amino-N-[(1S)-2-[4-(hydroxymethyl)anilino]-1-methyl-2-oxo-ethyl]-3-methyl-butanamide (2.02 g, 6.89 mmol) as a beige solid. $^1$H NMR (400 MHz, dmso-d6): δ 10.0 (s, 1H), 8.17 (s, 1H), 7.53 (d, 2H), 7.23 (d, 2H), 5.12 (t, 1H), 4.39-4.52 (m, 3H), 2.96-3.02 (m, 1H), 1.86-1.97 (m, 1H), 1.70 (br s, 2H), 1.29 (d, 3H), 0.88 (d, 3H), 0.78 (d, 3H).

*Step 3: [(2R)-2-(9H-fluoren-9-ylmethoxycarbonylamino)-3-oxo-3-sodiooxy-propyl]sulfonyloxysodium*

To a solution of [(2R)-2-amino-3-oxo-3-sodiooxy-propyl]sulfonyloxysodium monohydrate (3.00 g, 12.98 mmol) in water (127 mL) was added sodium carbonate (4.13 g, 38.94 mmol). A solution of 9H-fluoren-9-ylmethyl carbonochloridate (3.69 g, 14.28 mmol) in dioxane (127 mL) was added dropwise in 15 min at room temperature. The mixture was stirred at this temperature for 4 h. After completion of the reaction, the mixture was neutralized to pH = 7 with an aqueous solution of HCl 1M, diluted with a saturated aqueous solution of sodium hydrogenocarbonate (50 mL) and concentration to dryness. The crude product was purified by C18 reverse phase chromatography using the neutral method to afford [(2R)-2-(9H-fluoren-9-ylmethoxycarbonylamino)-3-oxo-3-sodiooxy-propyl]sulfonyloxysodium (4.4 g, 10.11 mmol) as a white solid. $^1$H NMR (400 MHz, dmso-d6): δ 7.88 (d, 2H). 7.70 (d, 2H), 7.39-7.44 (m, 2H), 7.29-7.36 (m, 2H), 6.71 (s, 1H), 3.84-4.25 (m, 4H), 2.73-2.91 (m, 2H).

*Step 4:[(2R)-2-(9H-fluoren-9-ylmethoxycarbonylamino)-3-[[(1S)-1-[[(1S)-2-[4-(hydroxymethyl)anilino]-1-methyl-2-oxo-ethyl]carbamoyl]-2-methyl-propyl]amino]-3-oxo-propyl]sulfonyloxysodium*

To a solution of (2S)-2-amino-N-[(1S)-2-[4-(hydroxymethyl)anilino]-1-methyl-2-oxoethyl]-3-methyl-butanamide (1.19 g, 4.04 mmol) in DMF (395 mL) were successively added [(2R)-2-(9H-fluoren-9-ylmethoxycarbonylamino)-3-oxo-3-sodiooxy-propyl]sulfonyloxysodium (4.40 g, 10.11 mmol), DIPEA (6.01 mL, 36.38 mmol) and HBTU (3.83 g, 10.11 mmol). The white suspension was stirred for 22 h at room temperature and then cooled to 0°C. Dilution with water (1.5 L), with a saturated solution of sodium carbonate (20 mL) and with solid sodium chloride, gave a white emulsion that was filtrated and the filtrate concentrated to dryness to afford the crude mixture. The crude product was purified by reverse phase C18 chromatography (gradient of methanol in water) to afford [(2R)-2-(9H-fluoren-9-ylmethoxycarbonylamino)-3-[[(1S)-1-[[(1S)-2-[4-(hydroxymethyl)anilino]-1-methyl-2-oxoethyl]carbamoyl]-2-methyl-propyl]amino]-3-oxo-propyl]sulfonyloxysodium (936 mg, 1.36 mmol) as a beige solid. $^1$H NMR (400 MHz, dmso-d6): δ 9.39 (s, 1H). 8.25-8.31 (m, 1H), 8.11-8.17 (m, 1H), 7.89 (d, 2H), 7.70 (d, 2H), 7.64 (d, 2H), 7.50-7.55 (m, 1H), 7.38-7.46 (m, 2H), 7.29-7.35 (m, 2H), 7.20 (d, 2H), 5.07 (s, 1H), 4.51 (s, 1H), 4.42 (s, 2H), 4.19-4.33 (m, 4H), 4.01 (s, 1H), 2.90-3.10 (m, 2H), 2.08-2.20 (m, 1H), 1.31 (d, 3H), 0.8-0.93 (m, 6H).

*Step 5: (2R)-2-(9H-fluoren-9-ylmethoxycarbonylamino)-3-[[(1S)-2-methyl-1-[[(1S)-1-methyl-2-[4-[(4-nitrophenoxy)carbonyloxymethyl]anilino]-2-oxo-ethyl]carbamoyl]propyl]amino]-3-oxo-propane-1-sulfonate*

To a suspension of [(2R)-2-(9H-fluoren-9-ylmethoxycarbonylamino)-3-[[(1S)-1-[[(1S)-2-[4-(hydroxymethyl)anilino]-1-methyl-2-oxo-ethyl]carbamoyl]-2-methyl-propyl]amino]-3-oxo-propyl]sulfonyloxysodium (600 mg, 0.87 mmol) in THF (24 mL) were added DIPEA (432 μL, 2.61 mmol), followed by 4-Nitrophenyl chloroformate (439 mg, 2.17 mmol). The mixture was stirred at room temperature for 4 h. Additional 4-Nitrophenyl chloroformate (439 mg, 2.17 mmol) was added and the reaction mixture was stirred at room temperature for 16 h more. Additional 4-Nitrophenyl chloroformate (439 mg,

2.17 mmol) was added. After 5 h stirring at room temperature the mixture was concentrated to dryness and purified by silica gel chromatography (gradient of ethyl acetate in cyclohexane) and then by reverse phase C18 chromatography using the neutral method to afford (2R)-2-(9H-fluoren-9-ylmethoxycarbonylamino)-3-[[(1S)-2-methyl-1-[[(1S)-1-methyl-2-[4-[(4-nitrophenoxy)carbonyloxymethyl]anilino]-2-oxo-ethyl]carbamoyl]propyl]amino]-3-oxo-propane-1-sulfonate (303 mg, 0.32 mmol) as a white solid. $^1$H NMR (400 MHz, dmso-d6): δ 9.52 (s, 1H), 8.25-8.37 (m, 3H), 8.06-8.24 (m, 4H), 7.89 (d, 2H), 7.76 (d, 2H), 7.70 (d, 2H), 7.49-7.61 (m, 3H), 7.35-7.45 (m, 4H), 7.26-7.35 (m, 2H), 5.23 (s, 2H), 4.48 (s, 1H), 4.20-4.33 (m, 4H), 4.01 (s, 1H), 3.57-3.66 (m, 2H), 3.10-3.18 (m, 2H), 2.90-3.10 (m, 2H), 2.08-2.20 (m, 1H), 1.33 (d, 3H), 1.21-1.26 (m, 15H), 0.86-0.92 (m, 6H). UPLC-MS: MS (ESI) m/z [M-H]-: 830.5.

***Step** 6: (2R)-2-[(5S$_a$)-5-[3-chloro-4-[2-[4-[[4-[[(2S)-2-[[(2S)-2-[[(2S)-2-(9H-fluoren-9-ylmethoxycarbonylamino)butanoyl]amino]-3-methyl-butanoyl]amino]propanoyl]amino]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid*

To a solution of (2R)-2-[(5S$_a$)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetic acid (C3) (128 mg, 0.149 mmol) in DMF (1.5mL) were successively added a solution of (2R)-2-(9H-fluoren-9-ylmethoxycarbonylamino)-3-[[(1S)-2-methyl-1-[[(1S)-1-methyl-2-[4-[(4-nitrophenoxy)carbonyloxymethyl]anilino]-2-oxo-ethyl]carbamoyl]propyl]amino]-3-oxo-propane-1-sulfonate (150 mg, 0.156 mmol) in DMF (1.5mL) and DIPEA (77 μl, 0.468 mmol). The reaction mixture was stirred for 2 h at room temperature and the progress of the reaction was followed by UPLC-MS. (2R)-2-[(5S$_a$)-5-[3-chloro-4-[2-[4-[[4-[[(2S)-2-[[(2S)-2-[[(2S)-2-(9H-fluoren-9-ylmethoxycarbonylamino)butanoyl]amino]-3-methyl-butanoyl]amino]propanoyl]amino]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid was obtained as a solution in dimethylformamide that was used like this in the next step. UPLC-MS: MS (ESI) m/z [M+H]+ = 1553.2+1555.3.

***Step** 7: (2R)-2-[(5S$_a$)-5-[4-[2-[4-[[4-[[(2S)-2-[[(2S)-2-[[(2S)-2-aminobutanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid*

To the solution of (2R)-2-[(5S$_a$)-5-[3-chloro-4-[2-[4-[[4-[[(2S)-2-[[(2S)-2-[[(2S)-2-(9H-fluoren-9-ylmethoxycarbonylamino)butanoyl]amino]-3-methyl-butanoyl]amino]propanoyl]amino]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (0.156 mmol) in dimethylformamide (3 mL) obtained in the previous step was added piperidine (30.6 μL, 0.312 mmol). The reaction mixture was stirred at room temperature for 15 h and the progress of the reaction was followed by UPLC-MS. The crude product was purified by C18 reverse phase prep-HPLC by direct deposit of the reaction mixture on the Xbridge column and using the $NH_4HCO_3$ method to afford (2R)-2-[(5S$_a$)-5-[4-[2-[4-[[4-[[(2S)-2-[[(2S)-2-[[(2S)-2-aminobutanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (148 mg = 0.111 mmol) as a white powder. UPLC-MS: MS (ESI) m/z [M+H]+ = 1331.4+1333.5.

***Step** 8: (2R)-2-[(5S$_a$)-5-[4-[2-[4-[[4-[[(2S)-2-[[(2S)-2-[[(2S)-2-[[2-[2-[2-(2-azidoethoxy)ethoxy]ethoxy]acetyl]amino]butanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-djpyrimidin-4-yljoxy-3-[2-[[2-(2-methoxyphenyl)pyrimidin-4-yl]methoxy]phenyl]propanoic acid;2,2,2-trifluoroacetic acid **L18-C3***

To the solution of (2R)-2-[(5S$_a$)-5-[4-[2-[4-[[4-[[(2S)-2-[[(2S)-2-[[(2S)-2-aminobutanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]proanoic acid (148 mg, 0.111 mmol) in DMF (1.5 mL) were successively added the solution of (2,3,4,5,6-pentafluorophenyl) 2-[2-[2-(2-azidoethoxy)ethoxy]ethoxy]acetate (0.596 mmol; obtained according to Step 20 of the preparation of **L14-C3)** in THF (1 mL) and DIPEA (74 μL, 0.447 mmol). The reaction mixture was stirred for 1 h at room temperature and the progress of the reaction was followed by UPLC-MS. The crude product was purified by C18 reverse phase prep-HPLC by direct deposit of the reaction mixture on the Xbridge column and using the $NH_4HCO_3$ method to afford **L18-C3** (60 mg, 0.0389mmol) as a white powder. IR Wavelength ($cm^{-1}$): 3288, 2101, 1659, 1237,1039, 833,755. $^1$H NMR(400 MHz, dmso-d6): δ (m, 10H), 9.42 (s, 1H), 8.88 (d, 1H), 8.58 (s, 1H), 8.32 (d, 1H), 8.18 (d, 1H), 8.12 (d, 1H), 7.71 (m, 1H), 7.7 (d, 2H), 7.54 (dd, 1H), 7.46 (td, 1H), 7.39 (d, 1H), 7.29 (dd, 2H), 7.25 (d, 2H), 7.21 (t, 2H), 7.18 (d, 1H), 7.15 (d, 1H), 7.13 (t, 1H), 7.04 (t, 1H), 6.99 (d,

1H), 6.71 (t, 1H), 6.22 (d, 1H), 5.47 (m, 1H), 5.23 (AB, 2H), 4.98 (s, 2H), 4.71 (q, 1H), 4.3 (m, 1H), 4.24/4.19 (2m, 2H), 3.97 (dd, 1H), 3.92 (m, 2H), 3.76 (s, 3 H), 3.37 (t, 2H), 3.31 (m, 4H), 3.12/2.97 (2dd, 2H), 2.74 (t, 2H), 2.45 (m, 4H), 2.15 (m, 1H), 1.81 (s, 3H), 1.33 (d, 3H), 0.91 (2d, 6H). $^{13}$C NMR (100 MHz, dmso-d6): δ 157.9, 152.3, 131.3, 131.2, 131.1, 131, 130.7, 128.9, 128.6, 120.7, 120.4, 119.6, 116.4, 112.7, 112, 111.3, 70.6, 70.3, 69.4, 67.5, 66.3, 59.7, 56.7, 56.1, 53.4, 52.5, 50.8, 50.4, 49.9, 44, 29.9, 19.6, 17.8, 17.6. $^{19}$F NMR (376 MHz, dmso-d6): δ ppm 112.3. HR-ESI+: m/z [M+H]+ = 1546.503 (1546.5009) (measured/theoretical).

**Preparation of L16-C3:**

**(2R)-2-[(5S$_a$)-5-[4-[2-[4-[[4-[[(2S)-6-amino-2-[[(2S)-2-[[2-(2-azidoethoxy)acetyl]amino]-3-methyl-butanoyl]amino] hexanoyl]amino]phenyl]methoxycarbonyl]piperazin-1-yl]ethoxy]-3-chloro-2-methyl-phenyl]-6-(4-fluorophenyl) thieno[2,3-d]pyrimidin-4-yl]oxy-3-[2-[[2-(2-methoxyphenyl)pyrimidin-4-yl]methoxy]phenyl]propanoic acid**

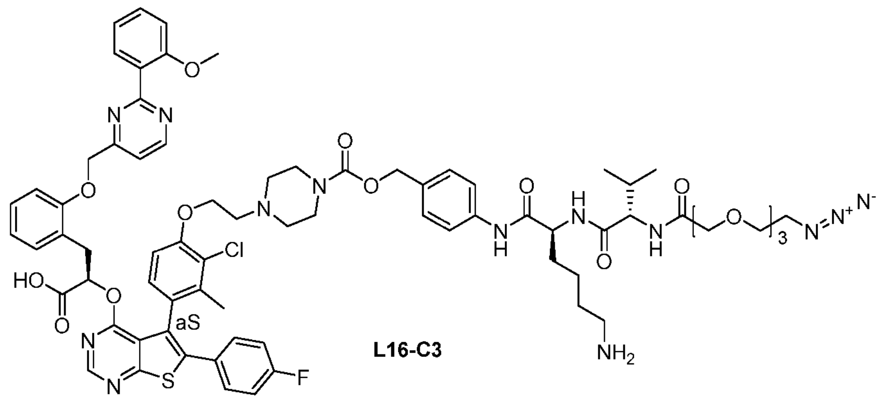

L16-C3

*Step 1: (2S)-6-(tert-butoxycarbonylamino)-2-[[2-(9H-fluoren-9-ylmethoxycarbonylamino)-3-methyl-butanoyl]amino] hexanoic acid*

To a solution of (2S)-2-amino-6-(tert-butoxycarbonylamino)hexanoic acid (2.96 g, 12 mmol) and sodium hydrogene carbonate (1.01 g, 12 mmol) in water (30 mL) was added a solution of (2,5-dioxopyrrolidin-1-yl) 2-(9H-fluoren-9-ylmethoxycarbonylamino)-3-methylbutanoate (5.0 g, 11.5 mmol) in dimethoxyethane (30 mL), THF (15 mL) was added to improve the solubility. The reaction mixture was stirred at room temperature for 16 h. An aqueous solution of hydrochloric acid 1 M (15 mL) was added and the aqueous layer and was extracted with ethyl acetate (3 x 75 mL). The combined organic layers were dried over sodium sulfate, filtered and concentrated to dryness to afford the crude compound. Trituration in dichloromethane/pentane with sonication led to (2S)-6-(tert-butoxycarbonylamino)-2-[[2-(9H-fluoren-9-ylmethoxycarbonylamino)-3-methyl-butanoyl]amino]hexanoic acid (4.9 g, 8.63 mmol) as a white solid. $^1$H NMR (400 MHz, dmso-d6): δ 12.48 (s, 1H), 7.89 (d, 2H), 7.74 (t, 2H), 7.28-7.44 (m, 6H), 6.73 (s, 1H), 4.10-4.33 (m, 5H), 3.9 (t, 1H), 2.82-2.90 (m, 2H), 1.52-1.73 (m, 2H), 1.34 (s, 9H), 1.22-1.31 (m, 4H), 0.83-0.91 (m, 6H).

*Step 2: 9H-fluoren-9-ylmethyl N-[1-[[(1S)-5-(tert-butoxycarbonylamino)-1-[[4-(hydroxymethyl)phenyl]carbamoyl]pentyl] carbamoyl]-2-methyl-propyl]carbamate*

To a solution of (2S)-6-(tert-butoxycarbonylamino)-2-[[2-(9H-fluoren-9-ylmethoxycarbonylamino)-3-methyl-butanoyl]amino]hexanoic acid (1.5 g, 2.64 mmol) in dichloromethane (19 mL) and methanol (9.5 mL) was added (4-aminophenyl)methanol (651.0 mg, 5.28 mmol) in methanol (1.5 mL). Ethyl 2-ethoxy-2H-quinoline-1-carboxylate (1.31 g, 5.28 mmol) was then added. The reaction mixture was stirred at room temperature for 16 h then concentrated to dryness. The crude product was purified by silica gel chromatography (gradient of methanol in dichloromethane) to afford 9H-fluoren-9-ylmethyl N-[1-[[(1S)-5-(tert-butoxycarbonylamino)-1-[[4-(hydroxymethyl)phenyl]carbamoyl]pentyl]carbamoyl]-2-methyl-propyl]carbamate (544 mg, 0.80 mmol) as a pale red solid. $^1$H NMR (400 MHz, dmso-d6): δ 9.93 (s, 1H). 8.01 (d, 1H), 7.89 (d, 2H), 7.74 (t, 2H), 7.52 (d, 2H), 7.37-7.45 (m, 3H), 7.32 (t, 2H), 7.22 (d, 2H), 6.71 (s, 1H), 5.08 (br s, 1H), 4.43 (d, 2H), 4.21-4.40 (m, 4H), 3.92 (t, 1H), 2.83-2.91 (m, 2H), 1.94-2.01 (m, 1H), 1.55-1.74 (m, 2H), 1.21-1.42 (m, 4H), 1.33 (s, 9H), 0.87 (t, 6H).

*Step 3: [4-[[(2S)-6-(tert-butoxycarbonylamino)-2-[[2-(9H-fluoren-9-ylmethoxycarbonylamino)-3-methyl-butanoyl]amino] hexanoyl]amino]phenyl]methyl (4-nitrophenyl) carbonate*

To a solution of 9H-fluoren-9-ylmethyl N-[1-[[(1S)-5-(tert-butoxycarbonylamino)-1-[[4-(hydroxymethyl)phenyl] carbamoyl]pentyl]carbamoyl]-2-methyl-propyl]carbamate (600.0 mg, 0.892 mmol) in THF (19 mL), were added pyridine (361 μL, 4.46 mmol) then 4-Nitrophenyl chloroformate (448 mg, 2.22 mmol). The mixture was stirred at room temperature for 16 h then concentrated to dryness. The crude product was purified by silica gel chromatography (gradient of ethyl acetate in cyclohexane) to afford [4-[[(2S)-6-(tert-butoxycarbonylamino)-2-[[2-(9H-fluoren-9-ylmethoxycarbonylamino)-3-methyl-butanoyl]amino]hexanoyl]amino]phenyl]methyl (4-nitrophenyl) carbonate (524 mg; 0.62 mmol; 70%) as a pale pink solid. $^1$H NMR (400 MHz, dmso-d6): δ 10.13 (s, 1H), 8.31 (d, 2H), 8.1 (d, 1H), 7.89 (d, 2H), 7.74 (t, 2H), 7.63 (d, 2H), 7.57 (d, 2H), 7.28-7.45 (m, 7H), 6.72 (s, 1H), 5.24 (s, 2H), 4.35-4.42 (m, 1H), 4.27-4.33 (m, 1H), 4.22 (s, 2H), 3.92 (t, 1H), 2.83-2.91 (m, 2H), 1.96-2.00 (m, 1H), 1.58-1.73 (m, 2H), 1.20-1.30 (m, 4H), 1.33 (s, 9H), 0.86 (t, 6H). $^{13}$C NMR (100 MHz, dmso-d6): δ 171.22, 170.67, 156.1, 155.5, 155.27, 151.92, 145.15, 143.87, 143.75, 140.68, 139.34, 129.43, 129.31, 127.6, 127.03, 125.38, 125.32, 122.58, 120.07, 119.11, 77.28, 70.23, 65.67, 60.11, 54.89, 53.43, 46.67, 31.69, 30.39, 29.22, 28.23, 22.74, 19.19, 18.26. LC-MS: MS (ESI) m/z [M+Na]+ = 837.4.

*Step 4: (2R)-2-[(5S$_a$)-5-[4-[2-[4-[[4-[[(2S)-6-(tert-butoxycarbonylamino)-2-[[(2S)-2-(9H-fluoren-9-ylmethoxycarbonylamino)-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d1pyrimidin-4-yljoxy-3-[2-[[2-(2-methoxyphenyl)pyrimidin-4-yl]methoxy]phenyl] propanoic acid*

To a solution of (2R)-2-[(5S$_a$)-5-[3-chloro-2-methyl-4-(2-piperazin-1-ylethoxy)phenyl]-6-(4-fluorophenyl)thieno [2,3-d]pyrimidin-4-yl]oxy-3-[2-[[2-(2-methoxyphenyl)pyrimidin-4-yl]methoxy]phenyl]propanoic acid **C3** (166.3 mg, 0.170 mmol) in DMF (1.5 mL) were successively added a solution of [4-[[(2S)-6-(tert-butoxycarbonylamino)-2-[[2-(9H-fluoren-9-ylmethoxycarbonylamino)-3-methyl-butanoyl]amino]hexanoyl]amino]phenyl]methyl (4-nitrophenyl) carbonate (150 mg, 0.179 mmol) in DMF (1.5 mL) and DIPEA (85 μl, 0.510 mmol). The reaction mixture was stirred for 1 h at room temperature and the progress of the reaction was followed by UPLC-MS. The crude product was purified by C18 reverse phase prep-HPLC by direct deposit of the reaction mixture on the Xbridge column and using the $NH_4HCO_3$ method to afford (2R)-2-[(5S$_a$)-5-[4-[2-[4-[[4-[[(2S)-6-(tert-butoxycarbonylamino)-2-[[(2S)-2-(9H-fluoren-9-ylmethoxycarbonylamino)-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (134mg, 0.0859mmol) as a white powder. UPLC-MS: MS (ESI) m/z [M+H]+ = 1559.1+1561.3, [M+Na]+: 1581.0+1583.2. IR Wavelength (cm$^{-1}$): 3309, 1698, 1238, 1162, 757, 744. $^1$H NMR (400 MHz, dmso-d6): δ 10.05 (s, 1H), 8.87 (d, 1H), 8.6 (m, 1H), 8.06 (d, 1H), 7.88 (d, 2H), 7.74 (2d, 2H), 7.64 (m, 1H), 7.57 (d, 2H), 7.52 (dd, 1H), 7.44 (t, 1H), 7.43 (d, 1H), 7.4 (t, 2H), 7.35 (d, 1H), 7.3 (t, 2H), 7.3 (dd, 2H), 7.26 (d, 2H), 7.2 (t, 2H), 7.18 (d, 1H), 7.14 (d, 1H), 7.12 (t, 1H), 7.03 (t, 1H), 6.99 (d, 1H), 6.72 (t, 1H), 6.71 (m, 1H), 6.24 (d, 1H), 5.49 (dd, 1H), 5.23 (m, 2H), 4.97 (s, 2H), 4.38 (m, 1H), 4.29/4.23 (m, 2H), 4.22 (m, 1H), 4.2 (m, 2H), 3.92 (dd, 1H), 3.74 (s, 3H), 3.29 (m, 4H), 3.29/2.5 (2dd, 2H), 2.87 (m, 2H), 2.74 (t, 2H), 2.45 (m, 4H), 1.99 (m, 1H), 1.82 (s, 3 H), 1.68/1.6 (2m, 2H), 1.36/1.28 (2m, 4H), 1.32 (s, 9H), 0.86 (2d, 6H). $^{13}$C NMR (100 MHz, dmso-d6): δ 158, 131.4, 131.2, 131.2, 131, 130.8, 128.9, 128.5, 127.9, 127.5, 125.6, 120.8, 120.5, 120.4, 119.3, 116, 115.9, 112.4, 112.2, 111.2, 74.1, 69.2, 67.9, 66.7, 66.2, 60.6, 56.6, 56.2, 53.8, 53, 47.1, 43.7, 40, 32.6, 32.2, 30.6, 29.8/23.2, 28.5, 18.8, 18.1. $^{19}$F NMR (376 MHz, dmso-d6): δ -112.

*Step 5: (2R)-2-[(5S$_a$)-5-[4-[2-[4-[[4-[[(2S)-2-[[(2S)-2-amino-3-methyl-butanoyl]amino]-6-(tert-butoxycarbonylamino) hexanoyl]amino]phenyl]methoxycarbonyl]piperazin-1-yl]ethoxy]-3-chloro-2-methyl-phenyl]-6-(4-fluorophenyl)thieno [2,3-d]pyrimidin-4-yl]oxy-3-[2-[[2-(2-methoxyphenyl)pyrimidin-4-yl]methoxy]phenyl]propanoic acid*

To the solution of (2R)-2-[(5S$_a$)-5-[4-[2-[4-[[4-[[(2S)-6-(tert-butoxycarbonylamino)-2-[[(2S)-2-(9H-fluoren-9-ylmethoxycarbonylamino)-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl] methoxy]phenyl]propanoic acid (134 mg, 0.0859 mmol) in dimethylformamide (3 mL) was added piperidine (17 μL, 0.172 mmol). The reaction mixture was stirred at room temperature for 18 h and the progress of the reaction was followed by UPLC-MS. The crude product was purified by C18 reverse phase prep-HPLC by direct deposit of the reaction mixture on the Xbridge column and using the $NH_4HCO_3$ method to afford (2R)-2-[(5S$_a$)-5-[4-[2-[4-[[4-[[(2S)-2-[[(2S)-2-amino-3-methyl-butanoyl]amino]-6-(tert-butoxycarbonylamino)hexanoyl]amino]phenyl]methoxycarbonyl]piperazin-1-yl] ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (88 mg, 0.0658 mmol) as a white powder. UPLC-MS: MS (ESI) m/z [M+H]+ = 1337.4+1339.4, [M+Na]+ = 1359.4+1361.4. IR Wavelength (cm$^{-1}$): 3307, 1683, 1290, 1238, 1162, 835, 754. $^1$H NMR (400 MHz, dmso-d6) δ ppm 10.23 (s, 1H), 8.88 (d, 1H), 8.53 (m, 1H), 8.47 (br, 1H), 7.86 (d, 1H), 7.58 (d, 2H), 7.54 (d, 1H), 7.48 (d,

1H), 7.45 (t, 1H), 7.27 (dd, 2H), 7.25 (d, 2H), 7.19 (t, 2H), 7.18 (d, 1H), 7.14 (d, 1H), 7.08 (t, 1H), 7.03 (t, 1H), 6.96 (t, 1H), 6.72 (t, 1H), 6.67 (t, 1H), 6.14 (d, 1H), 5.42 (d, 1H), 5.21 (m, 2H), 4.97 (s, 2H), 4.4 (m, 1H), 4.21 (m, 2H), 3.75 (s, 3H), 3.42/2.35 (m, 2H), 3.29 (m, 4H), 3.24 (m, 1H), 2.87 (q, 2H), 2.72 (t, 2H), 2.43 (m, 4H), 1.99 (m, 1H), 1.78 (s, 3H), 1.7/1.61 (2m, 2H), 1.36 (m, 2H), 1.34 (s, 9H), 1.26 (m, 2H), 0.89/0.82 (2d, 6H). $^{13}C$ NMR (100 MHz, dmso-d6): $\delta$ ppm 158.4, 131.3, 131.2, 131.1, 131, 128.4, 128, 120.8, 120.6, 120.4, 119.7, 116.1, 115.9, 112.7, 111.7, 111.2, 76.2, 69.2, 67.4, 66.3, 59.2, 56.6, 56.3, 53.6, 53.1, 43.8, 40.1, 33.2, 32.4, 31.3, 29.3, 28.8, 22.9, 19.7/17.5, 17.9. $^{19}F$ NMR (376 MHz, dmso-d6): $\delta$ ppm -112.4.

*Step 6: (2R)-2-[(5S$_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-6-(tert-butoxycarbonylamino)hexanoyl]amino]phenyl]methoxycarbonyl]piperazin-1-yljethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid*

To a solution of (2R)-2-[(5S$_a$)-5-[4-[2-[4-[[4-[[(2S)-2-[[(2S)-2-amino-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (82 mg, 0.0613 mmol) in DMF (500 $\mu$L) were successively added the solution of (2,3,4,5,6-pentafluorophenyl) 2-[2-[2-(2-azidoethoxy)ethoxy]ethoxy]acetate (0.245 mmol; obtained according to Step 20 of the preparation of **L14-C3)** in THF and DIPEA (30.4 $\mu$L, 0.184 mmol). The reaction mixture was stirred for 1h at room temperature and the progress of the reaction was followed by UPLC-MS. The crude product was purified by C18 reverse phase prep-HPLC by direct deposit of the reaction mixture on the Xbridge column and using the $NH_4HCO_3$ method to afford (2R)-2-[(5S$_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (60 mg, 0.0386 mmol) as a white powder. UPLC-MS: MS (ESI) m/z [M+H]+ = 1552.2+1554.2, [M+Na]+ = 1574.1+1576.3.

*Step 7: (2R)-2-[(5S$_a$)-5-[4-[2-[4-[[4-[[(2S)-6-amino-2-[[(2S)-2-[[2-[2-[2-(2-azidoethoxy)ethoxy]ethoxy]acetyl]amino]-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d1pyrimidin-4-yljoxy-3-[2-[[2-(2-methoxyphenyl)pyrimidin-4-yl]methoxy]phenyl]propanoic acid* ***L16-C3***

To a solution of (2R)-2-[(5S$_a$)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (23 mg, 0.0148 mmol) in dichloromethane (3 mL) was added 2,2,2-trifluoroacetic acid (400 $\mu$l, 4.57 mmol). The reaction mixture was stirred for 2h at room temperature and the progress of the reaction was followed by UPLC-MS. The crude product was purified by C18 reverse phase prep-HPLC by direct deposit of the reaction mixture on the Xbridge column and using the $NH_4HCO_3$ method to afford **L16-C3** (5 mg, 0.00344 mmol) as a white powder. UPLC-MS: MS (ESI) m/z [M+H]+ = 1552.4+1554.5, [M+Na]+ = 1574.4+1576.4. IR Wavelength (cm$^{-1}$): 3250, 2250-3500, 2102, 1660, 1288, 1238, 1121, 833, 755. $^1H$ NMR(400 MHz, dmso-d6): $\delta$ ppm 10.24 (s, 1H), 8.85 (d, 1H), 8.49 (s, 1H), 8.49 (d, 1H), 7.98 (d, 1H), 7.6 (d, 2H), 7.55 (d, 1H), 7.51 (d, 1H), 7.5 (d, 1H), 7.46 (t, 1H), 7.26 (d, 2H), 7.25 (dd, 2H), 7.18 (t, 2H), 7.17 (d, 1H), 7.14 (d, 1H), 7.05 (t, 1H), 7.02 (t, 1H), 6.89 (d, 1H), 6.6 (t, 1H), 6.05 (d, 1H), 5.32 (d, 1H), 5.21/5.15 (m, 2H), 5.02/4.96 (m, 2H), 4.36 (q, 1H), 4.31 (dd, 1H), 4.2 (m, 2H), 3.94 (s, 2H), 3.77 (s, 3 H), 3.58 (m, 10 H), 3.46/2.28 (d+t, 2H), 3.34 (t, 2H), 3.29 (m, 4 H), 2.8 (m, 2H), 2.67 (t, 2H), 2.43 (m, 4 H), 2.01 (m, 1H), 1.75 (s, 3 H), 1.69/1.6 (2m, 2H), 1.51 (m, 2H), 1.31 (m, 2H), 0.86/0.8 (2d, 6 H). $^{13}C$ NMR (100 MHz, dmso-d6): $\delta$ ppm 157.9, 153.7, 131.4, 131.4, 131.3, 131.1, 130.9, 129.3, 127.6, 120.9, 120.4, 119.8, 116.2, 116.1, 112.6, 111.8, 111.8, 78.1, 70.5, 70.4, 69.3, 66.6, 66.6, 56.9, 56.5, 56.3, 54, 52.3, 50.4, 44, 39, 33.8, 32.2, 31.8, 28.2, 23.1, 19.7/18.1, 18.3. $^{19}F$ NMR (376 MHz, dmso-d6): $\delta$ ppm -112.6. HR-ESI+: m/z [M+H]+ = 1452.5661 (1452.5648) (measured/theoretical).

**Preparation of L21-C1:**

**(2S,3S,4R,5R,6S)-6-[2-[2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]-5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]ethyl]-3,4,5-trihydroxy-tetrahydropyran-2-carboxylic acid; 2,2,2-trifluoroacetate**

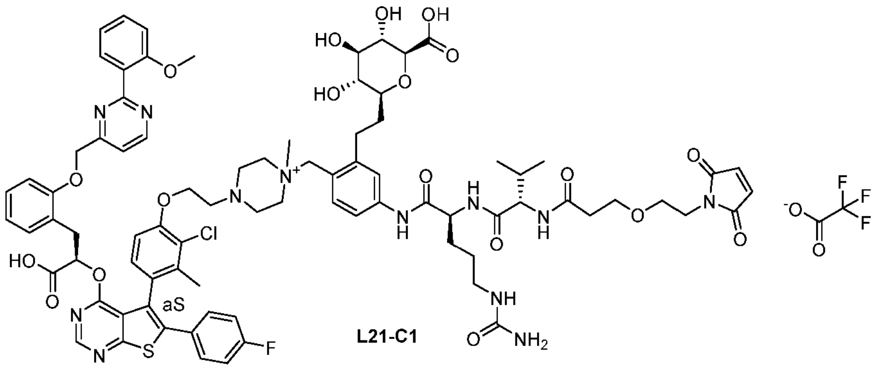

L21-C1

*Step 1: tert-butyl-[(2-iodo-4-nitro-phenyl)methoxy]-dimethyl-silane*

To a solution of (2-iodo-4-nitro-phenyl)methanol (172 g, 61.64 mmol; obtained according to Step 2 of the preparation of **L14-C3)** in dichloromethane (300 mL) was added imidazole (5.04 g, 73.97 mmol). The mixture was cooled to 0°C, then a solution of tert-butyl-chloro-dimethyl-silane (11.15 g, 73.97 mmol) in dichloromethane (300 mL) was added dropwise in 15 min. The ice bath was removed and the reaction mixture was stirred at room temperature for 16 h. After completion of the reaction, the reaction mixture was quenched with methanol (20 mL) and concentrated to dryness. The crude product was purified by silica gel chromatography (gradient of ethyl acetate in cyclohexane) to afford tert-butyl-[(2-iodo-4-nitro-phenyl)methoxy]-dimethyl-silane (19.65 g, 49.96 mmol) as a white solid. $^1$H NMR (400 MHz, dmso-d6): δ 8.57 (s, 1H), 8.31 (d, 1H), 7.66 (d, 1H), 4.67 (s, 2H), 0.92 (s, 9H), 0.14 (s, 6H).

*Step 2: methyl (2S,3S,4R,5S, 6S)-3,4,5-triacetoxy-6-[2-[2-[[tert-butyl(dimethyl)silyl]oxymethyl]-5-nitro-phenyl]ethynyl] tetrahydropyran-2-carboxylate*

To a solution of tert-butyl-[(2-iodo-4-nitro-phenyl)methoxy]-dimethyl-silane compound (3.0 g, 7.63 mmol) in DMF (55 mL) were successively added methyl (2S,3S,4R,5S,6S)-3,4,5-triacetoxy-6-ethynyl-tetrahydropyran-2-carboxylate (3.39 g, 9.92 mmol; obtained according to Step 13 of the preparation of L14-C3), DIPEA (5.80 mL, 35.09 mmol), copper iodide (145 mg, 0.763 mmol) and dichloro-bis-(triphenylphosphine)palladium(II) (535 mg, 0.763 mmol). The yellow solution was flushed with Argon and stirred for 16 h at room temperature. After dilution with water (300 mL), the aqueous layer was extracted with ethyl acetate (2 x 300 mL). The combined organic layers were washed with water (2 x 300 mL) then were dried over sodium sulfate, filtered and concentrated to dryness. The crude product was purified by silica gel chromatography (gradient of ethyl acetate in cyclohexane) to afford methyl (2S,3S,4R,5S,6S)-3,4,5-triacetoxy-6-[2-[2-[[tert-butyl(dimethyl)silyl]oxymethyl]-5-nitro-phenyl]ethynyl]tetrahydropyran-2-carboxylate (4.01 g, 6.60 mmol) as a beige solid. $^1$H NMR (400 MHz, dmso-d6): δ 8.32 (dd, 1H), 8.19 (d, 1H), 7.75 (d, 1H), 5.45 (t, 1H), 5.16 (t, 1H), 5.02-5.07 (m, 2H), 4.82 (s, 2H), 4.55 (d, 1H), 3.65 (s, 3H), 1.98-2.07 (m, 9H), 0.92 (m,9H), 0.14 (s, 6H).

*Step 3: methyl (2S,3S,4R,5S, 6S)-3,4,5-triacetoxy-6-[2-[2-(hydroxymethyl)-5-nitro-phenyl]ethynyl]tetrahydropyran-2-carboxylate*

To a solution of methyl (2S,3S,4R,5S,6S)-3,4,5-triacetoxy-6-[2-[2-[[tert-butyl(dimethyl)silyl]oxymethyl]-5-nitro-phenyl]ethynyl]tetrahydropyran-2-carboxylate (4.01 g, 6.60 mmol) in THF (48 mL) and water (48 mL) was added acetic acid (193 mL, 3.36 mol). The colorless solution was stirred for 2 days at room temperature then diluted with water (300 mL). The aqueous layer was extracted with dichloromethane (2 x 300 mL). The combined organic layers were washed with water (2 x 300 mL), and with a saturated aqueous solution of sodium hydrogen carbonate (400 mL), then dried over sodium sulfate, filtered and concentrated to dryness. The crude product was purified by silica gel chromatography (gradient of ethyl acetate in cyclohexane) to afford methyl (2S,3S,4R,5S,6S)-3,4,5-triacetoxy-6-[2-[2-(hydroxymethyl)-5-nitro-phenyl] ethynyl]tetrahydropyran-2-carboxylate (2.67 g, 5.41 mmol) as a white solid. $^1$H NMR (400 MHz, dmso-d6): δ 8.29 (dd, 1H),

8.15 (d, 1H), 7.79 (d, 1H), 5.68 (t, 1H), 5.45 (t, 1H), 5.16 (t, 1H), 5.02-5.07 (m, 2H), 4.62 (d, 2H), 4.55 (d, 1H), 3.65 (s, 3H), 1.98-2.07 (m, 9H).

*Step 4: methyl (2S,3S,4R,5S,6S)-3,4,5-triacetoxy-6-[2-[5-amino-2-(hydroxymethyl)phenyl]ethyl]tetrahydropyran-2-carboxylate*

A solution of methyl (2S,3S,4R,5S,6S)-3,4,5-triacetoxy-6-[2-[2-(hydroxymethyl)-5-nitro-phenyl]ethynyl]tetrahydropyran-2-carboxylate (2.67 g, 5.41 mmol) in THF (59 mL) was flushed with Argon. Platinum on carbon 5% dry (1.34 g, 50% w/w) was added. The reaction mixture was successively flushed with argon, with $H_2$ and was stirred for 2 days at room temperature under $H_2$ atmosphere (P atm). The reaction mixture was filtered through a Celite® pad, washed with a solution of ethyl acetate/methanol 9/1 (500 mL), then concentrated to dryness. All the sequence, (addition of platinum on carbon 5% dry (1.34 g, 50% w/w), stirring for 16 h at room temperature under $H_2$ (P atm) and filtration through a Celite® pad), was repeated to allow a complete conversion. The crude product was purified by silica gel chromatography (gradient of ethyl acetate in cyclohexane) to afford methyl (2S,3S,4R,5S,6S)-3,4,5-triacetoxy-6-[2-[5-amino-2-(hydroxymethyl)phenyl]ethyl]tetrahydropyran-2-carboxylate (1.12 g, 2.40 mmol) as a white solid. $^1H$ NMR (400 MHz, dmso-d6): δ 6.93 (d, 1H). 6.67-6.33 (m, 2H), 5.30 (t, 1H), 4.96 (t, 1H), 4.88 (s, 2H), 4.81 (t, 1H), 4.61 (t, 1H), 4.39 (d, 1H), 4.29-4.24 (m, 2H), 3.78-3.72 (m, 1H), 3.65 (s, 3H), 2.65-2.54 (m, 2H), 2.07-1.98 (m, 9H), 1.79-1.68 (m, 1H), 1.63-1.52 (m, 1H).

*Step 5: methyl (2S,3S,4R,5S,6S)-3,4,5-triacetoxy-6-[2-[5-[[(2S)-2-(tert-butoxycarbonylamino)-5-ureido-pentanoyl]amino]-2-(hydroxymethyl)phenyl]ethyl]tetrahydropyran-2-carboxylate*

To a solution of methyl (2S,3S,4R,5S,6S)-3,4,5-triacetoxy-6-[2-[5-amino-2-(hydroxymethyl)phenyl]ethyl]tetrahydropyran-2-carboxylate (1.00 g, 2.14 mmol) in DMF (21 mL) were successively added (2S)-2-(tert-butoxycarbonylamino)-5-ureido-pentanoic acid (589 mg, 2.14 mmol), DIPEA (707 μl, 4.28 mmol) and HBTU (1.22 g, 3.21 mmol). The reaction mixture was stirred for 72 hours at room temperature. After completion of the reaction, the mixture was diluted with water (100 mL) and was concentrated to dryness. The crude product was purified by silica gel chromatography (gradient of methanol in dichloromethane) to afford methyl (2S,3S,4R,5S,6S)-3,4,5-triacetoxy-6-[2-[5-[[(2S)-2-(tert-butoxycarbonylamino)-5-ureido-pentanoyl]amino]-2-(hydroxymethyl)phenyl]ethyl]tetrahydropyran-2-carboxylate (1.05 g, 1.45 mmol) as a beige solid. $^1H$ NMR (400 MHz, dmso-d6): δ 9.82 (s, 1H), 7.35-7.42 (m, 2H), 7.24 (d, 1H), 6.95 (d, 1H), 5.94 (t, 1H), 5.37 (s, 2H), 5.30 (t, 1H), 4.91-4.99 (m, 2H), 4.79 (t, 1H), 4.36-4.42 (m, 3H), 4.01-4.08 (m, 1H), 3.76 (t, 1H), 3.65 (s, 3H), 2.95-3.04 (m, 2H), 2.54-2.65 (m, 2H), 1.98-2.07 (m, 9H), 1.68-1.79 (m, 1H), 1.49-1.63 (m, 3H), 1.30-1.42 (m, 11H).

*Step 6: methyl (2S,3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]ethyl]tetrahydropyran-2-carboxylate*

To a solution of compound methyl (2S,3S,4R,5S,6S)-3,4,5-triacetoxy-6-[2-[5-[[(2S)-2-(tert-butoxycarbonylamino)-5-ureido-pentanoyl]amino]-2-(hydroxymethyl)phenyl]ethyl]tetrahydropyran-2-carboxylate (950 mg, 1.31 mmol) in dichloromethane (7.5 mL) was added, at 0°C, trifluoroacetic acid (1.9 mL, 25.6 mmol). The reaction mixture was stirred at room temperature for 3 h. After completion of the reaction, the reaction mixture was concentrated to dryness and was coevaporated with toluene (2 x 50 mL) to afford the crude compound.

To this crude in solution in DMF (13 mL) were successively added (2S)-2-(9H-fluoren-9-ylmethoxycarbonylamino)-3-methyl-butanoic acid (467 mg, 1.38 mmol), DIPEA (867 μl, 5.24 mmol) and HBTU (845 mg, 2.23 mmol). The reaction mixture was stirred for 16 h at room temperature. After completion of the reaction, a saturated aqueous solution of hydrogenocarbonate (20 mL) was added, the mixture was stirred at room temperature for 1 h, was diluted with water (100 mL) and was concentrated to dryness. The crude product was purified by silica gel chromatography (gradient of methanol in dichloromethane) and then by reverse phase C18 chromatography using the neutral method to afford methyl (2S,3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]ethyl]tetrahydropyran-2-carboxylate (680 mg, 0.720 mmol) as a white solid. LC-MS: MS (ESI) m/z [M+H]+ = 946.3. $^1H$ NMR (400 MHz, dmso-d6): δ 9.90 (s, 1H). 8.07 (d, 2H), 7.89 (d, 2H), 7.74 (t, 2H), 7.44-7.38 (m, 3H), 7.36-7.28 (m, 3H), 7.24 (d, 1H), 5.94 (t, 1H), 5.37 (s, 2H), 5.30 (t, 1H), 4.99-4.92 (m, 2H), 4.79 (t, 1H), 4.42-4.36 (m, 4H), 4.32-4.19 (m, 3H), 3.94-3.90 (m, 1H), 3.76 (t, 1H), 3.65 (s, 3H), 2.99-2.94 (m, 2H), 2.65-2.54 (m, 2H), 2.07-1.98 (m, 10H), 1.70-1.55 (m, 4H), 1.46-1.36 (m, 2H), 0.89-0.84 (m, 6H). $^{13}C$ NMR (100 MHz, dmso-d6): δ 171.19, 170.33, 169.58, 169.45, 169.27, 167.77, 158.81, 156.12, 143.89, 143.76, 140.69, 139.48, 137.54, 134.88, 128.44, 127.62, 127.06, 125.35, 120.08, 119.42, 116.65, 75.78, 74.61, 72.65, 71.20, 69.49, 65.68, 60.49, 60.10, 53.14, 52.40, 46.68, 32.32, 30.43, 29.54, 27.19, 26.77, 20.39, 20.34, 20.24, 19.22, 18.25.

*Step 7: methyl (2S,3S,4R,5S, 6S)-3,4,5-triacetoxy-6-[2-[2-(bromomethyl)-5-[[(2S)-2-[[(2S)-2-(9H-fluoren-9-ylmethoxycarbonylamino)-3-methyl-butanoyl]amino]-5-ureido-pentanoyl]amino]phenyl]ethyl]tetrahydropyran-2-carboxylate*

To a solution of compound methyl (2S,3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]ethyl]tetrahydropyran-2-carboxylate (154 mg, 0.163 mmol) in THF (8.2 mL) was successively added triphenylphosphine (85.4 mg, 0.326 mmol) and 1-bromopyrrolidine-2,5-dione (58.0 mg, 0.326 mmol). The reaction mixture was stirred for 2h at room temperature. The progress of the reaction was followed by UPLC-MS: an aliquot was treated by a large exces of MeOH, following the formation of the corresponding methyl ether. The expected bomide derivative was stable in UPLC-MS conditions. After 5h were added triphenylphosphine (85.4 mg, 0.326 mmol) and 1-bromopyrrolidine-2,5-dione (58.0 mg, 0.326 mmol) and the reaction mixture was stirred for 15h at room temperature. The obtained crude methyl (2S,3S,4R,5S,6S)-3,4,5-triacetoxy-6-[2-[2-(bromomethyl)-5-[[(2S)-2-[[(2S)-2-(9H-fluoren-9-ylmethoxycarbonylamino)-3-methyl-butanoyl]amino]-5-ureido-pentanoyl]amino]phenyl]ethyl]tetrahydropyran-2-carboxylate was used like this in the next step. UPLC-MS: MS (ESI) m/z [M+Ome-Br+H]+ = 960.7.

*Step 8: (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]-2-[2-[(2S,3S,4R,5S,6S)-3,4,5-triacetoxy-6-methoxycarbonyl-tetrahydropyran-2-yl]ethyl]phenyl]methyl]-4-methyl-piperazin-4-ium-1-yl]ethoxy]-2-methyl-phenyl]-6-(4-fluorophenyl)thieno[2,3-d] pyrimidin-4-yl]oxy-3-[2-[[2-(2-methoxyphenyl)pyrimidin-4-yl]methoxy]phenyl]propanoic acid*

To the solution of methyl (2S,3S,4R,5S,6S)-3,4,5-triacetoxy-6-[2-[2-(bromomethyl)-5-[[(2S)-2-[[(2S)-2-(9H-fluoren-9-ylmethoxycarbonylamino)-3-methyl-butanoyl]amino]-5-ureido-pentanoyl]amino]phenyl]ethyl]tetrahydropyran-2-carboxylate (0.167mmol) in DMF from the previous step (step 7) was successively added (2R)-2-[(5Sa)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl) pyrimidin-4-yl]methoxy]phenyl]propanoic acid (C1) (143 mg, 0.163 mmol) and DIPEA (114 $\mu$L, 0.652 mmol) The reaction mixture was stirred for 15 h at room temperature and the progress of the reaction was followed by UPLC-MS (aliquot was treated by a large exces of MeOH). The crude product was purified by C18 reverse phase prep-HPLC by direct deposit of the reaction mixture on the Xbridge column and using the TFA method to afford (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl] amino]-2-[2-[(2S,3S,4R,5S,6S)-3,4,5-triacetoxy-6-methoxycarbonyl-tetrahydropyran-2-yl]ethyl]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (21.3 mg, 0.0111 mmol) as a white powder. UPLC-MS: MS (ESI) m/z [M+H]+ = 1802.9+1804.9. IR Wavelength (cm$^{-1}$): 1755, 1672, 1226,1201,1130. $^{1}$H NMR (400 MHz, dmso-d6) $\delta$ ppm 13.3 (br s, 1H), 10.2 (s, 1H), 8.88 (d, 1H), 8.61 (s, 1H), 8.14 (d, 1H), 7.88 (d, 2H), 7.73 (dd, 2H), 7.65 (d, 1H), 7.63 (d, 1H), 7.62 (m, 1H), 7.54 (br s, 1H), 7.51 (dd, 1H), 7.45 (t, 1H), 7.4 (t, 2H), 7.38 (m, 1H), 7.32 (t, 2H), 7.3 (dd, 2H), 7.2 (d, 1H), 7.2 (t, 2H), 7.15 (t, 1H), 7.15 (d, 1H), 7.03 (t, 1H), 7.01 (dd, 1H), 6.72 (t, 1H), 6.22 (d, 1H), 6 (br s, 1H), 5.51 (dd, 1H), 5.34 (t, 1H), 5.3 (br s, 2H), 5.27/5.21 (m, 2H), 4.98 (t, 1H), 4.85 (t, 1H), 4.57/4.49 (m, 2H), 4.39 (m, 1H), 4.35 (d, 1H), 4.27 (m, 2H), 4.26 (m, 2H), 4.23 (m, 1H), 3.93 (t, 1H), 3.76 (s, 3H), 3.71 (m, 1H), 3.64 (s, 3H), 3.4 (m, 4H), 3.29/2.51 (2dd, 2H), 3.13/2.94 (2m, 4H), 3 (m, 2H), 2.98 (m, 2H), 2.93 (br s, 3 H), 2.81 (m, 2H), 1.99/1.95 (3s, 9H), 1.98 (m, 1H), 1.84 (s, 3H), 1.77/1.59 (2m, 2H), 1.64 (2m, 2H), 1.41 (2m, 2H), 0.88/0.85 (2d, 6H). $^{13}$C NMR (100 MHz, dmso-d6): $\delta$ ppm 158.1, 152.9, 135.6, 131.5, 131.4, 131.3, 131.2, 131, 128.9, 128.1, 127.5, 125.7, 120.9, 120.6, 120.4, 120.3, 117, 116, 116, 112.8, 112.2, 111.2, 75.6, 74.9, 73.8, 72.9, 71.4, 69.6, 69.4, 67.5, 66.1, 60.4, 58.3, 56, 55.4, 53.9, 52.8, 47.2, 46.2, 44.3, 39, 32.8, 32.8, 31, 29.6, 27.4, 27.2, 21.1, 19.5/18.7, 18.1. $^{19}$F NMR (376 MHz, dmso-d6) $\delta$ ppm -74, -112.

*Step 9: (2S,3S,4R,5R,6S)-6-[2-[(5S$_a$)-5-[[(2S)-2-[[(2S)-2-amino-3-methyl-butanoyl]amino]-5-ureido-pentanoyl]amino]-2-[[4-[2-[4-[4-[(1R)-1-carboxy-2-[2-[[2-(2-methoxyphenyl) pyrimidin-4-yl]methoxy]phenyl]ethoxy]-6-(4-fluorophenyl) thieno[2,3-d]pyrimidin-5-yl]-2-chloro-3-methyl-phenoxyjethyl]-1-methyl-piperazin-1-ium-1-yl]methyl]phenyl]ethyll-3,4,5-trihydroxy-tetrahydropyran-2-carboxylic acid*

To a solution of (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]-2-[2-[(2S,3S,4R,5S,6S)-3,4,5-triacetoxy-6-methoxycarbonyl-tetrahydropyran-2-yl]ethyl]phenyl]methyl]-4-methyl-piperazin-4-ium-1-yl]ethoxy]-2-methyl-phenyl]-6-(4-fluorophenyl) thieno[2,3-d]pyrimidin-4-yl]oxy-3-[2-[[2-(2-methoxyphenyl)pyrimidin-4-yl]methoxy]phenyl]propanoic acid (21.3 mg, 0.0111 mmol) in methanol (6.0 mL) was added a solution of Lithium hydroxide monohydrate (4.66 mg $\mu$L, 0.111 mmol) in water (4 ml). The reaction mixture was stirred at room temperature for 60 h and the progress of the reaction was followed by UPLC-MS. The crude product was purified by C18 reverse phase prep-HPLC by direct deposit of the reaction mixture on the Xbridge column and using the TFA method to afford (2S,3S,4R,5R,6S)-6-[2-[(5S$_a$)-5-[[(2S)-2-[[(2S)-2-amino-3-methyl-butanoyl]amino]-5-ureido-pentanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]phenyl]ethyl]-3,4,5-trihydroxy-tetrahydropyran-2-carboxylic acid (47.8 mg, 0.029 mmol) as a white powder. UPLC-MS: MS (ESI) m/z [M+H]+ = 1440.6+1442.6.

*Step 10: (2S,3S,4R,5R,6S)-6-[2-[2-[[4-[2-[4-[4-[(1R)-1-carboxy-2-[2-[[2-(2-methoxyphenyl) pyrimidin-4-yl]methoxy] phenyl]ethoxy]-6-(4-fluorophenyl)thieno[2,3-d]pyrimidin-5-yl]-2-chloro-3-methyl-phenoxyjethyl]-1-methyl-piperazin-1-ium-1-yl]methyl]-5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]ethyl]-3,4,5-trihydroxy-tetrahydropyran-2-carboxylic acid;2,2,2-trifluoroacetate* ***L21-C1***

To a solution of (2S,3S,4R,5R,6S)-6-[2-[(5S$_a$)-5-[[(2S)-2-[[(2S)-2-amino-3-methyl-butanoyl]amino]-5-ureido-pentanoyl]amino]-2-[[4-[2-[4-[4-[(1R)-1-carboxy-2-[2-[[2-(2-methoxyphenyl)pyrimidin-4-yl]methoxy]phenyl] ethoxy]-6-(4-fluorophenyl)thieno[2,3-d]pyrimidin-5-yl]-2-chloro-3-methyl-phenoxy]ethyl]-1-methyl-piperazin-1-ium-1-yl] methyl]phenyl]ethyl]-3,4,5-trihydroxy-tetrahydropyran-2-carboxylic acid (47.1 mg, 0.0282 mmol) in DMF (1.5 mL) were successively added the solution of (2,5-dioxopyrrolidin-1-yl) 3-[2-(2,5-dioxopyrrol-1-yl)ethoxy]propanoate (Purshased from Broadpharm, 13.1 mg, 0.0423 mmol) in DMF (500 μL) and DIPEA (17.2 μL, 0.0988 mmol).

The reaction mixture was stirred for 1h at room temperature and the progress of the reaction was followed by UPLC-MS. The crude product was purified by C18 reverse phase prep-HPLC by direct deposit of the reaction mixture on the Xbridge column and using the TFA method to afford **L21-C1** (65 mg, 0.0310 mmol) as a white powder. HR-ESI+: m/z [M+H]+ = 1635.6093 (1635.6068) (measured/theoretical).

**Preparation of L9-C1:**

**(2R)-2-[(5S$_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl] methoxy]phenyl]propanoic acid;2,2,2-trifluoroacetate;2,2,2-trifluoroacetic acid**

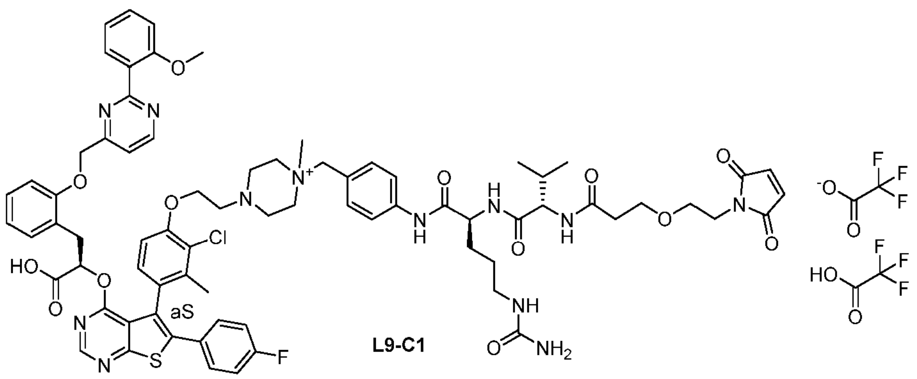

L9-C1

*Step 1: 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate*

A solution of 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate (150 mg, 0.249 mmol) in THF (3.8 ml) was cooled to 0°C. At 0°C was added dropwise tribromophosphane (1 M in dichloromethane) (374 μL, 0.249 mmol). The reaction was stirred 5 min at 0°C and 1h at room temperature. The progress of the reaction was followed by UPLC-MS (aliquot was treated by a large excess of MeOH). The reaction mixture was diluted with ethyl acetate (3 ml) and washed with an aqueous saturated solution of sodium hydrogen carbonate (1x 6ml). The organic layer was dried over magnesium sulfate, filtered. Add DMF (10 ml) and evaporate the ethyl acetate and the THF. The obtained solution of 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate is used like that in the next step. UPLC-MS: MS (ESI) m/z [M+Na]+ = 686.5+688.6.

*Step 2: (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d1pyrimidin-4-yljoxy-3-[2-[[2-(2-methoxyphenyl)pyrimidin-4-yl]methoxy]phenyl]propanoic acid*

To the solution of 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate (0.249 mmol) in DMF from the previous step (step 1) was successively added DMF (10 ml), (2R)-2-[(5Sa)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid **(C1)** (218 mg, 0.249 mmol) and DIPEA (130 μL, 0.748 mmol). The reaction mixture was stirred for 15 h at room temperature and the progress of the reaction was followed by UPLC-MS (aliquot was treated by a large excess of MeOH). The obtained solution of (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid in DMF was used like that in the next step. UPLC-MS: MS (ESI) m/z [M+Na]+ = 1458.7+1460.7.

*Step 3: (2R)-2-[(5S$_a$)-5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis 2,2,2-trifluoroacetic acid*

To the solution of (2R)-2-[(5S$_a$)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (0.249 mmol) in dimethylformamide (3 mL) obtained in the previous step (step 2) was added piperidine (49.3 μL, 0.499 mmol). The reaction mixture was stirred at room temperature for 5 h and the progress of the reaction was followed by UPLC-MS. The crude product was purified by C18 reverse phase prep-HPLC by direct deposit of the reaction mixture on the Xbridge column and using the TFA method to afford (2R)-2-[(5S$_a$)-5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis 2,2,2-trifluoroacetic acid (31.2 mg = 0.0213 mmol) as a white powder. UPLC-MS: MS (ESI) m/z [M+Na]+ = 1236.7+1238.7.

*Step 4: (2R)-2-[(5S$_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; 2,2,2-trifluoroacetic acid **L9-C1***

To a solution of (2R)-2-[(5Sa)-5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (31.2 mg, 0.0213 mmol) in DMF (1.5 mL) were successively added the solution of (2,5-dioxopyrrolidin-1-yl) 3-[2-(2,5-dioxopyrrol-1-yl)ethoxy]propanoate (23.8 mg, 0.0768 mmol) in DMF (500 μL) and DIPEA (31.2 μL, 0.179 mmol). The reaction mixture was stirred for 15h at room temperature and the progress of the reaction was followed by UPLC-MS. The crude product was purified by C18 reverse phase prep-HPLC by direct deposit of the reaction mixture on the Xbridge column and using the TFA method to afford **L9-C1** (6 mg, 0.00303 mmol) as a white powder. HR-ESI+: m/z [M]+ = 1431.5437 (1431.5433) (measured/theoretical).

**Preparation of L9-C8:**

**(2R)-2-[(5S$_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid;2,2,2-trifluoroacetate;2,2,2-trifluoroacetic acid**

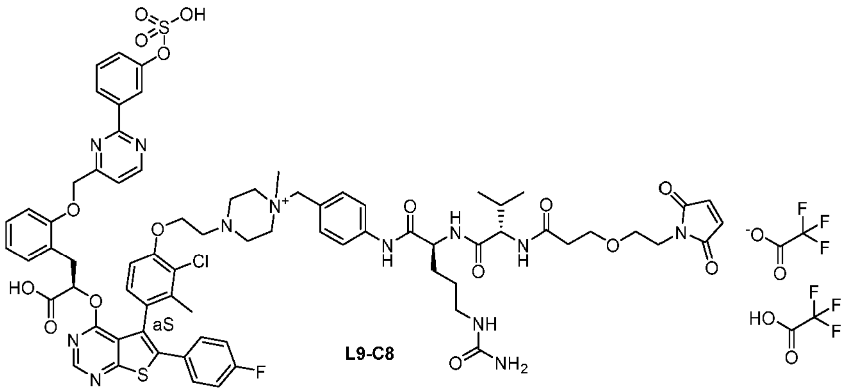

L9-C8

*Step 1: (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid*

To the solution of 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate (0.0230 mmol; obtained according to Step 1 of the preparation of **L9-C1)** in DMF (3 mL) was successively added DMF (5 ml), ammonium; [3-[4-[[2-[(2R)-2-carboxy-2-[(5Sa)-5-[3-chloro-2-methyl-4-[2-(4-methylpiperazin-1-yl)ethoxy]phenyl]-6-(4-fluorophenyl)thieno[2,3-d]pyrimidin-4-yl]oxy-ethyl]phenoxy]methyl]pyrimidin-2-yl]phenyl] sulfate **(C8)** (22 mg, 0.0230 mmol) and DIPEA (12 μL, 0.069 mmol). The reaction mixture was stirred for 15 h at room temperature and the progress of the reaction was followed by UPLC-MS (aliquot was treated by a large exces of MeOH). The obtained solution of (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid in DMF was used in the next step. UPLC-MS: MS (ESI) m/z [M-$SO_3$H]+ = 1444.8+1446.7.

*Step 2: (2R)-2-[(5$S_a$)-5-[4-[2-[4-[[4-[[(2S)-2-[[(2S)-2-amino-3-methyl-butanoyl]amino]-5-ureido-pentanoyllamino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(3-sulfooxyphenyl) pyrimidin-4-ylimethoxy]phenyllpropanoic acid; bis 2,2,2-trifluoroacetic acid*

To the solution of (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid (0.0230 mmol) in dimethylformamide (3 mL) obtained in the previous step (step 1) was added piperidine (9 μL, 0.0920 mmol). The reaction mixture was stirred at room temperature for 5 h and the progress of the reaction was followed by UPLC-MS. The crude product was purified by C18 reverse phase prep-HPLC by direct deposit of the reaction mixture on the Xbridge column and using the TFA method to afford [3-[4-[[2-[(2R)-2-[5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-2-carboxy-ethyl]phenoxy]methyl]pyrimidin-2-yl]phenyl] sulfate; bis 2,2,2-trifluoroacetic acid (10.0 mg = 0.00633 mmol) as a white powder. UPLC-MS: MS (ESI) m/z [M-$SO_3$]+ = 1224.12.

*Step 3: (2R)-2-[(5$S_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid;2,2,2-trifluoroacetate;2,2,2-trifluoroacetic acid*

To a solution of (2R)-2-[(5$S_a$)-5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid (10 mg, 0.00653 mmol) in DMF (1 mL) were successively added the solution of (2,5-dioxopyrrolidin-1-yl) 3-[2-(2,5-dioxopyrrol-1-yl)ethoxy]propanoate (3.1 mg, 0.00980 mmol) in DMF (500 μL) and DIPEA (4 μL, 0.0229 mmol). The reaction mixture was stirred for 15h at room temperature and the progress of the reaction was followed by UPLC-MS. The crude product was purified by C18 reverse phase prep-HPLC by direct deposit of the reaction mixture on the Xbridge column and using the TFA method to afford **L9-C8** (6 mg, 0.00401 mmol) as a white powder. UPLC-MS: MS (ESI) m/z [M+Na]+ =

1519.5+1521.2, [M+H-SO3]+ 1417.7+1419.6. HR-ESI+: m/z [M+H]+ = 1497.486 (1497.4845) (measured/theoretical).

**Preparation of L9-C10:**

**(2R)-2-[(5S$_a$)5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-[4-fluoro-3-(2,2,2-trifluoroethoxy)phenyl]thieno[2,3-d]pyrimidin-4-yl]oxy-3-[2-[[2-(2-methoxy-phenyl)pyrimidin-4-yl]methoxy]phenyl]propanoic acid; 2,2,2-trifluoroacetate; 2,2,2-trifluoroacetic acid**

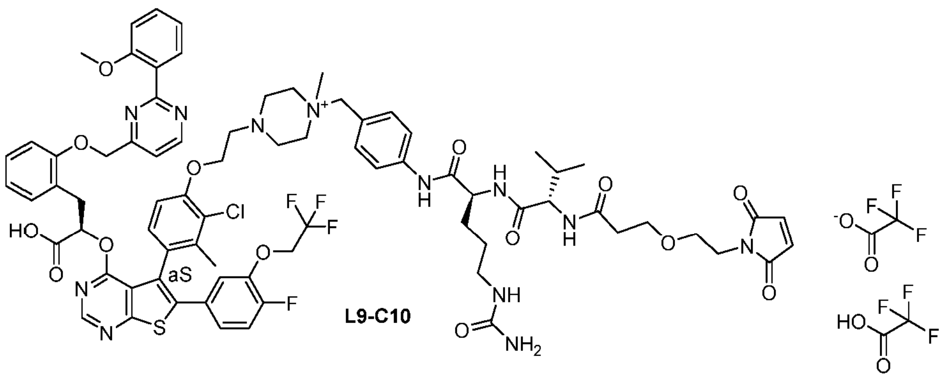

L9-C10

The procedure is as in the process of synthesis of **L9-C9,** replacing **C9** used in Step 3 by (2R)-2-[((5Sa)-5-{3-chloro-2-methyl-4-[2-(4-methylpiperazin-1-yl)ethoxy]phenyl}-6-[4-fluoro-3-(2,2,2-trifluoroethoxy)phenyl]thieno[2,3-d] pyrimidin-4-yl)oxy]-3-(2-{[2-(2-methoxyphenyl)pyrimidin-4-yl]methoxy}phenyl)propanoic acid **C10** and using TFA method for purification. HR-ESI+: m/z [M+H]+ = 1529.543 / 1529.5413 (measured/theoretical).

**Preparation of L9-C11:**

**(2R)-2-[(5S$_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(4-methoxyphenyl)pyrimidin-4-yl] methoxy]phenyl]propanoic acid; 2,2,2-trifluoroacetate; 2,2,2-trifluoroacetic acid.**

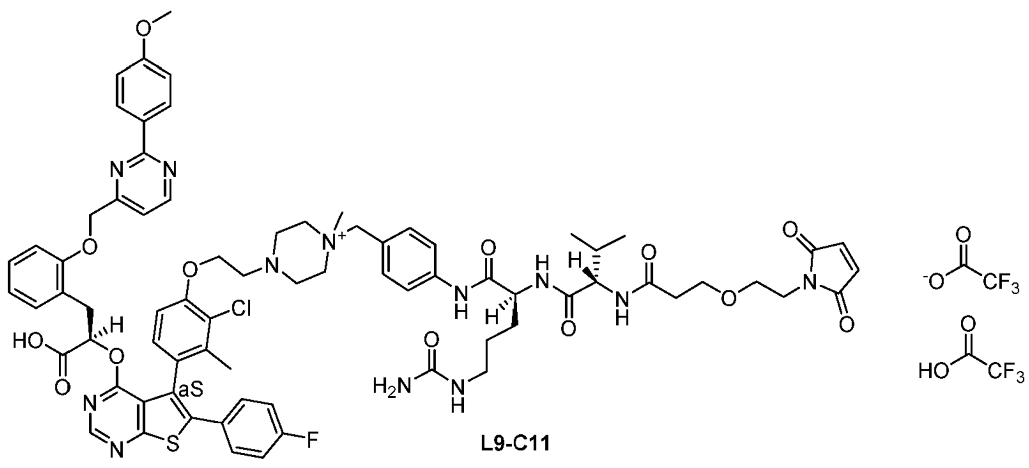

L9-C11

The procedure is as in the process of synthesis of **L9-C9,** replacing **C9** used in Step 3 by (2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl] oxy}-3-(2-{[2-(4-methoxyphenyl)pyrimidin-4-yl]methoxy}phenyl)propanoic acid **C11** and using TFA method for purification. HR-ESI+: m/z [M+H]+ = 1431.5442 / 14.31.5433 (measured/theoretical).

**Preparation of L9-C12:**

**(2R)-2-[(5$S_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(2,2,2-trifluoroethyl)pyrazol-3-yl]methoxy]phenyl]propanoic acid;2,2,2-trifluoroacetate;2,2,2-trifluoroacetic acid**

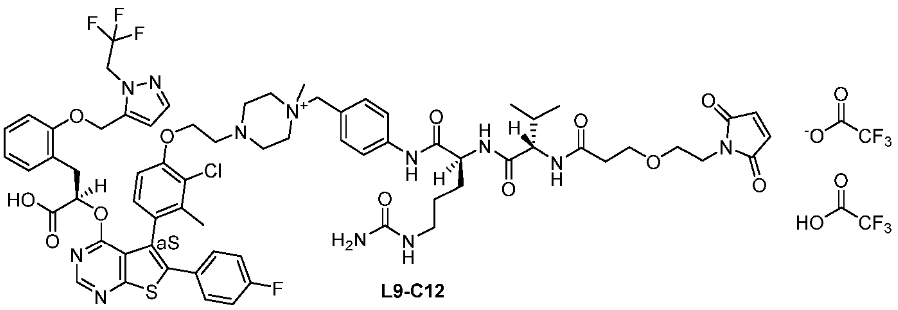

L9-C12

The procedure is as in the process of synthesis of **L9-C9,** replacing **C9** used in Step 3 by (2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[1-(2,2,2-trifluoroethyl)-1H-pyrazol-5-yl]methoxy}phenyl)propanoic acid **C12** using TFA method for purification. HR-ESI+: m/z [M+H]+ = 1395.5048 / 1395;5045 (measured/theoretical).

**Preparation of L9-C14:**

**(2R)-2-[5-[3-chloro-4-[2-[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d] pyrimidin-4-yl]oxy-3-[2-[[2-(3-hydroxy-2-methoxy-phenyl)pyrimidin-4-yl]methoxy]phenyl]propanoic acid;2,2,2-trifluoroacetate**

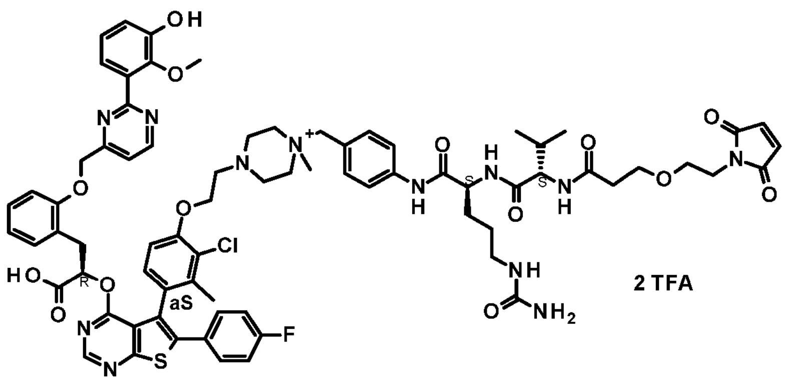

2 TFA

*Step 1: (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxy-3-sulfooxy-phenyl)pyrimidin-4-yl]methoxy]phenyl]propanoic acid*

500 mg ethyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[(2-chloropyrimidin-4-yl)methoxy]phenyl]propanoate (0.60 mmol, WO2016/207216 **Preparation** 1) and 202 mg (3-hydroxy-2-methoxy-phenyl)boronic acid (1.20 mmol) were dissolved in 9 mL 1,4-dioxane, then 42 mg $Pd(PPh_3)_2Cl_2$ (0.06 mmol), 588 mg $Cs_2CO_3$ (1.80 mmol) and 9 mL water were added and the mixture was stirred under $N_2$ atmosphere at 70°C until complete conversion. Then it was diluted with water, neutralized with 2 M aqueous HCl solution, and extracted with DCM. The combined organic phase was dried over $Na_2SO_4$, filtered and the filtrate was concentrated under reduced pressure. The crude ester was purified via flash chromatography using heptane, EtOAc and 0.7 M $NH_3$ solution in MeOH as eluents to obtain a mixture of diastereoisomers. Then it was dissolved in 23.6

mL pyridine, 0.97 mL $SO_3$×pyrimidine (5.98 mmol) was added and the mixture was stirred at 70°C until complete conversion. Then it was concentrated under reduced pressure, and dissolved in 2 mL dioxane, then 200 mg KOH (3.57 mmol) and 1 mL water were added. The mixture was stirred at rt until complete hydrolysis. Then it was neutralized with 2 M aqueous HCl solution, and directly injected on prep-RP-HPLC, using 25 mM aqueous $NH_4HCO_3$ solution and MeCN as eluents. The diastereoisomer eluting later was collected as product of the title. $^1$H NMR (500 MHz, DMSO-$d_6$) δ: 8.92 (d, 1H), 8.63 (s, 1H), 7.68 (dd, 1H), 7.63 (d, 1H), 7.34 (d, 1H), 7.30 (dd, 2H), 7.29 (d, 1H), 7.20 (t, 2H), 7.16 (t, 1H), 7.15 (d, 1H), 7.10 (t, 1H), 7.02 (d, 1H), 6.73 (t, 1H), 6.38 (d, 1H), 5.50 (dd, 1H), 5.29/5.23 (d+d, 2H), 4.21/4.16 (m+m, 2H), 3.84 (s, 3H), 3.25/2.55 (dd+dd, 2H), 3.18-2.75 (m, 10H), 2.65 (brs, 3H), 1.82 (s, 3H).HRMS calculated for $C_{47}H_{44}N_eO_{10}S_2ClF$: 970.2233; found 971.2297 (M+H).

*Step 2: (2R)-2-[5-[3-chloro-4-[2-[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]phenyl]methyl]-4-methyl-piperazin-4-ium- 1-yl]ethoxy]-2-methyl-phenyl]-6-(4-fluorophenyl)thieno[2,3-d] pyrimidin-4-yl]oxy-3-[2-[[2-(3-hydroxy-2-methoxy-phenyl)pyrimidin-4-yl]methoxy] phenyl]propanoic acid;2,2,2-trifluoroacetate* **L9-C14**

To a solution of (2R)-2-[5-[3-chloro-2-methyl-4-[2-(4-methylpiperazin-1-yl)ethoxy]phenyl]-6-(4-fluorophenyl) thieno[2,3-d]pyrimidin-4-yl]oxy-3-[2-[[2-(2-methoxy-3-sulfooxy-phenyl)pyrimidin-4-yl]methoxy]phenyl]propanoic acid (20.0 mg; 0.0206 mmol) in DMF (309 μL), were successively added (2S)-N-[4-(chloromethyl)phenyl]-2-[[(2S)-2-[3-[2-(2,5-dioxopyrrol-1-yl)ethoxy]propanoylamino]-3-methyl-butanoyl]amino]-5-ureido-pentanamide (17.5 mgL; 0.0206 mmol), DIPEA (10.8 μL; 0.0618 mmol) and TBAI (1 mg; 0.0027 mmol). The reaction mixture was stirred at 70°C for 18 hours. The crude product was purified by $C_{18}$ reverse phase prep-HPLC by direct deposit of the reaction mixture on the X-Bridge column and using the TFA method to afford **L9-C14** (10.5 mg; 0.00725 mmol) as a white powder. HR-ESI+: m/z [M+H]+ = 1448.5437 / 1448.5466 [measured/theoretical].

**Preparation of L9-P15:**

**(11R,20R)-23,26-dichloro-20-[[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]phenyl]methyl]-4-methyl-piperazin-4-ium-1-yl]methyl]-3-(4-fluorophenyl)-14-[[2-(2-methoxyphenyl) pyrimidin-4-yl]methoxy]-24,25-dimethyl-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid;2,2,2-trifluoroacetate**

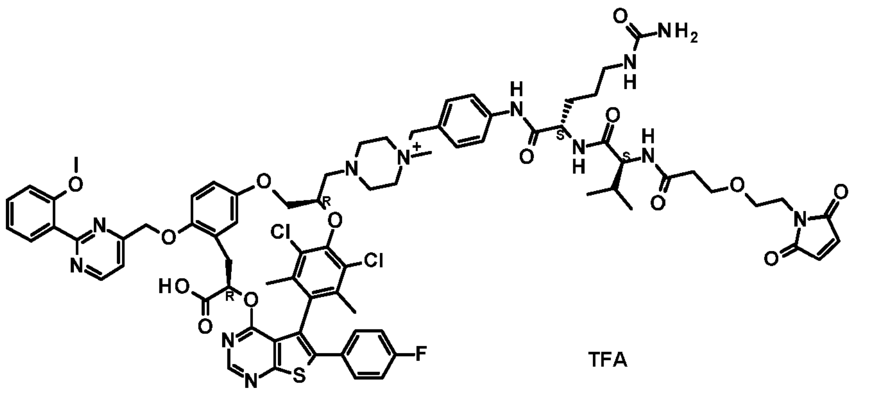

TFA

To a solution of (11R,20R)-23,26-dichloro-3-(4-fluorophenyl)-14-[[2-(2-methoxyphenyl)pyrimidin-4-yl]methoxy]-24,25-dimethyl-20-[(4-methylpiperazin-1-yl)methyl]-10,18,21-trioxa-4-thia-6,8-diazapentacyclo [20.2.2.12,5.113,17.09,28]octacosa-1(25),2,5(28),6,8,13,15,17(27),22(26),23-decaene-11-carboxylic acid **P15** (obtained according to WO 2019/035914; 10.0 mg; 0.0105 mmol) in DMF (630 μL), were successively added (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (10.0 mg; 0.0158 mmol), DIPEA (5.5 μL; 0.0315 mmol) and TBAI (0.5mg, 0.0010mmol). The reaction mixture was stirred at room temperature for 0.5 hour. The crude product was purified by $C_{18}$ reverse phase prep-HPLC by direct deposit of the reaction mixture on the X-Bridge column and using the TFA method to afford **L9-P15** (11.9 mg, 0.00733 mmol) as a white powder. HR-ESI+: m/z [M-$CF_3CO_2$]+ = 1507.5183 / 1507.5155

Preparation of L9-P16:

**(11R,20R)-23,26-dichloro-14-[[2-[4-[[(2S)-1,4-dioxan-2-yl]methoxymethyl]-4-fluoro-cyclohexyl]pyrimidin-4-yl] methoxy]-20-[[4-[[4-[[(2S)-2-[[(2S)-2-[3-[2-(2,5-dioxopyrrol-1-yl)ethoxy]propanoylamino]-3-methyl-butanoyl] amino]-5-ureido-pentanoyl]amino] phenyl]methyl]-4-methyl-piperazin-4-ium-1-yl]methyl]-3-(4-fluorophenyl)-24,25-dimethyl-10,18,21-trioxa-4-thia-6,8-diazapentacyclo[20.2.2.12,5.113,17.09,28]octacosa-1(25),2,5(28),6,8,13,15,17(27),22(26),23-decaene-11-carboxylic acid;2,2,2-trifluoroacetate**

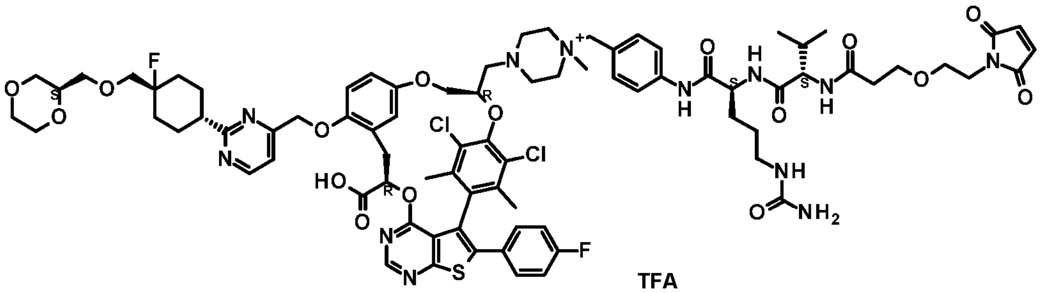

TFA

To a solution of (11R,20R)-23,26-dichloro-14-[[2-[4-[[(2S)-1,4-dioxan-2-yl]methoxymethyl]-4-fluoro-cyclohexyl] pyrimidin-4-yl]methoxy]-3-(4-fluorophenyl)-24,25-dimethyl-20-[(4-methylpiperazin-1-yl)methyl]-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid **P16** (obtained according to WO 2019/035911; 14.7 mg; 0.0137 mmol) in DMF (1 mL), were successively added (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (13.1 mg; 0.0205 mmol) and DIPEA (7.1 $\mu$L; 0.0410 mmol). The reaction mixture was stirred at room temperature for 2 hours. The crude product was purified by direct deposit of the reaction mixture on the X-Bridge column in using the TFA method to afford **L9-P16** (7.9 mg; 0.00420 mmol) as a white powder. IR ($cm^{-1}$): 3327, 1768/1706, 1666, 1199/1118, 831/798. HR-ESI+: m/z [M-$CF_3CO_2$]+ = 1631.6071/1631.6054 [measured/theoretical]

**Preparation of L9-P17:**

**(11R,20R)-23,26-dichloro-14-[[2-[4-[[(2S)-1,4-dioxan-2-yl]methoxy]cyclohexyl]pyrimidin-4-yl]methoxy]-20-[[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]phenyl]methyl]-4-methyl-piperazin-4-ium-1-yl]methyl]-3-(4-fluorophenyl)-24,25-dimethyl-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid;2,2,2-trifluoroacetate_**

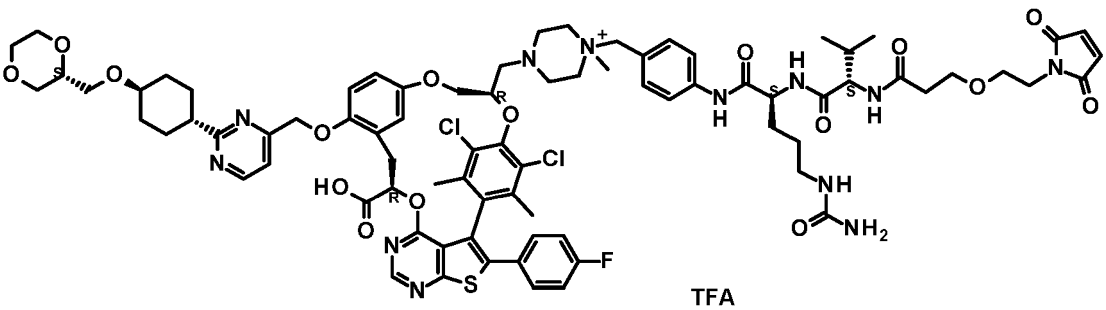

TFA

To a solution of (11R,20R)-23,26-dichloro-14-[[2-[4-[[(2S)-1,4-dioxan-2-yl]methoxy]cyclohexyl]pyrimidin-4-yl] methoxy]-3-(4-fluorophenyl)-24,25-dimethyl-20-[(4-methylpiperazin-1-yl)methyl]-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid **P17** (obtained according to WO 2019/035911; 14.5 mg; 0.0139 mmol) in DMF (1 mL), were successively added (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (13.3 mg; 0.0208 mmol) and DIPEA (7.3 $\mu$L; 0.0417 mmol). The reaction mixture was stirred at room temperature for 8 hours. The crude product was purified by direct deposit of the reaction mixture on the X-Bridge column

and using the TFA method to afford **L9-P17** (7.0 mg, 0.00437 mmol) as a white powder. IR ($cm^{-1}$): 3700-2400, 1771/1738/1705, 1665, 1194/1128. HR-ESI+: m/z [M-$CF_3CO_2$]+ = 1599.6013 / 1599.5992. HR-ESI+: m/z [M-$CF_3CO_2$ +H]2+ = 800.3049 / 800.3035 [measured/theoretical]

**Preparation of L25-P1:**

**2-[[4-[2-[4-[4-[(1R)-1-carboxy-2-[2-[[2-(2-methoxyphenyl)pyrimidin-4-yl]methoxy]phenyl] ethoxy]-6-(4-fluorophenyl)thieno[2,3-d]pyrimidin-5-yl]-2-chloro-3-methyl-phenoxy]ethyl]-1-methyl-piperazin-1-ium-1-yl] methyl]-5-[[(2S)-2-[[1-[2-[2-(2,5-dioxopyrrol-1-yl)ethoxy]ethylcarbamoyl]cyclobutanecarbonyl]amino]-5-ureido-pentanoyl]amino]benzenesulfonate;2,2,2-trifluoroacetic acid**

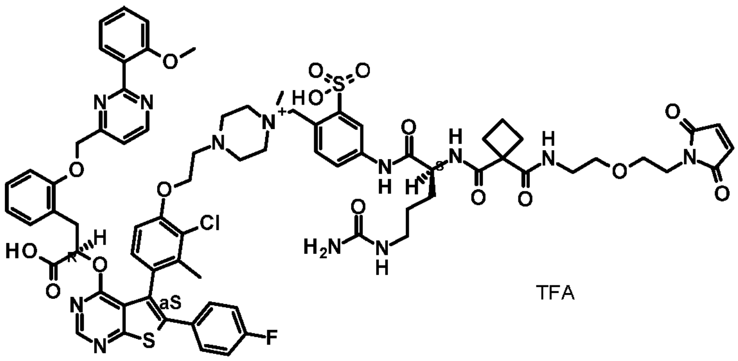

*Step 1: tert-butyl 1-[2-[2-(2,5-dioxopyrrol-1-yl)ethoxy]ethylcarbamoyl] cyclobutanecarboxylate*

To a solution of 1-tert-butoxycarbonylcyclobutanecarboxylic acid (58.6 mg; 0.293 mmol) in DCM (5.85 ml), were successively added 1-[2-(2-aminoethoxy)ethyl]pyrrole-2,5-dione (53.9 mg; 0.293 mmol), EDC (84.2 mg; 0.439 mmol), HOBt (59.3 mg; 0.439 mmol), and DIPEA (204 μL; 1.17 mmol). The reaction mixture was stirred at room temperature for 18 hours. The progress of the reaction was monitored by UPLC-MS. The reaction mixture was concentrated to dryness and solubilized in DMF (1 ml) and the solution was purified by X-Bridge column $C_{18}$ by direct deposit of the reaction mixture on the column and in using the TFA method to afford the title compound (57.3 mg; 0.156 mmol). IR ($cm^{-1}$): 3390, 1697/1666. $^1H$ NMR (400 MHz, dmso-d6) δ ppm 7.5 (t, 1H), 7.02 (s, 2H), 3.55/3.5 (2t, 4H), 3.38 (t, 2H), 3.17 (q, 2H), 2.33 (m, 4H), 1.77 (m, 2H), 1.38 (s, 9H). UPLC-MS: MS(ESI): m/z [M+Na]+ = 389.26 [M+H-tBu]+ = 311.22

*Step 2: 1-[2-[2-(2,5-dioxopyrrol-1-yl)ethoxy]ethylcarbamoyl]cyclobutanecarboxylic acid*

To a solution of tert-butyl 1-[2-[2-(2,5-dioxopyrrol-1-yl)ethoxy]ethylcarbamoyl] cyclobutanecarboxylate (7 mg; 0.0191 mmol) in DCM (0.175 mL), was added TFA (51.2 μL; 0.668 mmol). The reaction mixture was stirred at room temperature for 3.5 hours, then was concentrated to dryness to obtain the title compound (5.8 mg; 0.0187 mmol) as a colorless oil. The crude product was used in the next step. UPLC-MS: MS(ESI): m/z [M+H]+ = 311.35, [M+Na]+ = 333.37

*Step 3: (2,3,4,5,6-pentafluorophenyl) 1-[2-[2-(2,5-dioxopyrrol-1-yl)ethoxy]ethylcarbamoyl]cyclobutanecarboxylate*

To a solution of 1-[2-[2-(2,5-dioxopyrrol-1-yl)ethoxy]ethylcarbamoyl]cyclobutanecarboxylic acid (18.2 mg; 0.0587 mmol) in THF (3 mL), were successively added 2,3,4,5,6-pentafluorophenol (13.0 mg; 0.0704 mmol) and DCC (14.5 mg; 0.0704 mmol). The reaction mixture was stirred at room temperature for 15 hours and the progress of the reaction was monitored by UPLC-MS. The reaction mixture was a suspension, the precipitate is filtered off and washed with THF (1 ml) to afford a solution of (2,5-dioxopyrrolidin-1-yl) 1-[2-[2-(2,5-dioxopyrrol-1-yl)ethoxy]ethylcarbamoyl] cyclobutanecarboxylate in THF. The crude product solution was used in step 9. UPLC-MS: MS(ESI): m/z [M+H]+ = 477.28, [M+Na]+ = 499.23

*Step 4: methyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate*

To a solution of (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid **P1** (5.0 g; 5.712 mmol) in DCM (25 mL) and methanol (25 mL), was added dropwise a solution of diazomethyl(trimethyl)silane (2 M in $Et_2O$) (5.712 mL; 11.42 mmol). The reaction mixture was stirred at room temperature for 2 hours and the progress of the reaction was monitored by UPLC-MS. After completion the reaction was quenched by a slow addition of acetic acid until the yellow color turn to red and was concentrated to dryness to afford the crude mixture. The crude product was purified by silica gel chromatography (gradient of methanol in DCM) to afford the title compound (4.52 g; 5.082 mmol). UPLC-MS: MS(ESI): m/z [M+H]+ = 889.27+891.6, [M+Na]+ = 911.31, [M+2H]2+= 445.59. IR ($cm^{-1}$): 1753, 1238/1053. $^1H$ NMR (400 MHz, dmso-d6) δ ppm 8.6 (s, 1H), 8.45 (d, 1H), 7.6 (d, 1H), 7.52 (dd, 1H), 7.45 (td, 1H), 7.3 (m, 3H), 7.25-7.1 (m, 5H), 7.02 (t+d, 2H), 6.78 (t, 1H), 6.31 (dd, 1H), 5.52 (dd, 1H), 5.25 (AB, 2H), 4.2 (m, 2H), 3.78/3.65 (2s, 6H), 3.2/2.58 (2dd, 2H), 2.71 (t, 2H), 2.5/2.3 (2ml, 8H), 2.12 (s, 3H), 1.88 (s, 3H).

*Step 5: 5-[[(2S)-2-(9H-fluoren-9-ylmethoxycarbonylamino)-5-ureido-pentanoyl]amino]-2-(hydroxymethyl)benzenesulfonic acid*

To a solution of Fmoc-Cit-OH (2.224 g; 5.596 mmol) in DCM (22.2 mL) and methanol (22.2 mL), were successively added sodium 5-amino-2-(hydroxymethyl) benzenesulfonate (1.89 mg; 8.395 mmol) and EEDQ (2.768 g; 11.19 ml). The reaction mixture was stirred at room temperature for 25 hours, then was concentrated to dryness. The crude product was purified by silica gel chromatography (gradient of methanol in DCM) to afford the title compound (2.81 g; 4.823 mmol) as white powder. IR ($cm^{-1}$): 3700-3000, 1660(large), 1180. $^1H$ NMR (400 MHz, dmso-d6) δ ppm 10.02 (s, 1H), 7.88 (m, 3H), 7.76 (2t, 2H), 7.7 (dd, 1H), 7.61 (d, 1H), 5.99 (t, 1H), 5.38 (m, 2H), 5.03 (t, 1H), 4.72 (d, 2H), 4.3-4.2 (m, 3H), 4.15 (m, 1H), 3.06-2.90 (m, 2H), 1.75-1.30 (m, 4H). UPLC-MS: MS(ESI): m/z [M+H]+ = 583.42, [M+Na]+ = 565.31.

*Step 6: 2-(chloromethyl)-5-[[(2S)-2-(9H-fluoren-9-ylmethoxycarbonylamino)-5-ureido-pentanoyl]amino]benzenesulfonic acid*

To a solution of 5-[[(2S)-2-(9H-fluoren-9-ylmethoxycarbonylamino)-5-ureido-pentanoyl]amino]-2-(hydroxymethyl)benzenesulfonic acid (543.6 mg; 0.933 mmol) in NMP (5 mL) were added at room temperature a solution of $SOCl_2$ (68.1 μL; 0.933 mmol) in NMP (200 μL). The reaction mixture was stirred at room temperature for 15 min and the progress of the reaction was monitored by UPLC-MS. To achieve a complete conversion, the $SOCl_2$ addition (68 μL) has to be done 7 more times. The excess $SOCl_2$ was evaporated under vaccum, and the residue was purified by direct deposit of the reaction mixture on an Oasis column in using the TFA method to afford the title compound (362 mg; 0.512 mmol) as a white solid. UPLC-MS: MS(ESI): m/z [M+H]+ = 601.19+603.23 [M+Na]+ = 622.93

*Step 7: 2-[[4-[2-[2-chloro-4-[6-(4-fluorophenyl)-4-[(1R)-2-methoxy-1-[[2-[[2-(2-methoxyphenyl) pyrimidin-4-yl]methoxy] phenyl]methyl]-2-oxo-ethoxy]thieno[2,3-d]pyrimidin-5-yl]-3-methyl-phenoxy]ethyl]-1-methyl-piperazin-1-ium-1-yl] methyl]-5-[[(2S)-2-(9H-fluoren-9-ylmethoxycarbonylamino)-5-ureido-pentanoyl]amino]benzenesulfonate*

To a solution of 2-(chloromethyl)-5-[[(2S)-2-(9H-fluoren-9-ylmethoxycarbonylamino)-5-ureido-pentanoyl]amino]benzenesulfonic acid (195.6 mg; 0.277 mmol) from step 6 in solution in NMP (10 mL), were successively added methyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl] oxy-3-[2-[[2-(2-methoxyphenyl)pyrimidin-4-yl]methoxy]phenyl]propanoate (123 mg; 0.138 mmol) from step 4, DIPEA (385 μL, 2.213 mmol) and TBAI (10 mg, 0.027 mmol). The reaction mixture was stirred at 70°C for 12 hours and the progress of the reaction was monitored by UPLC-MS. The crude product in solution in NMP was directly used in the next step. UPLC-MS: MS(ESI): m/z [M+H]+ = 1231.12+1233.45, [M+2H]2+ = 616.34+617.37

*Step 8: 5-[[(2S)-2-amino-5-ureido-pentanoyllamino]-2-[[4-[2-[4-[4-[(1R)-1-carboxy-2-[2-[[2-(2-methoxyphenyl) pyrimidin-4-yl]methoxy]phenyl]ethoxy]-6-(4-fluorophenyl) thieno[2,3-d]pyrimidin-5-yl]-2-chloro-3-methyl-phenoxy]ethyl]-1-methyl-piperazin-1-ium-1-yl]methyl]benzenesulfonate ;2,2,2-trifluoroacetic acid*

To the previous solution of 2-[[4-[2-[2-chloro-4-[6-(4-fluorophenyl)-4-[(1 R)-2-methoxy-1-[[2-[[2-(2-methoxyphenyl)pyrimidin-4-yl]methoxy]phenyl]methyl]-2-oxo-ethoxy]thieno[2,3-d]pyrimidin-5-yl]-3-methyl-phenoxy]ethyl]-1-methyl-piperazin-1-ium-1-yl]methyl]-5-[[(2S)-2-(9H-fluoren-9-ylmethoxycarbonylamino)-5-ureido-pentanoyl]amino]benzenesulfonate in NMP, was added a solution of lithium hydroxyde mono hydrate (82.2 mg; 1.106 mmol) in water (4 mL). The reaction mixture was stirred at room temperature for 1.5 hours and the progress of the reaction was monitored by UPLC-MS. The crude product solution was purified by direct deposit of the reaction mixture on a X-Bridge column in using the TFA method to afford the title compound (45.6 mg; 0.0374 mmol) as a white powder. UPLC-MS: MS(ESI): m/z [M+H]+ = 1217.46, [M+Na]+ = 1241.16, [M+2H]2+ = 609.61

*Step 9: 2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl] methyl]-5-[[(2S)-2-[[1-[2-[2-(2,5-dioxopyrrol-1-yl)ethoxy]ethylcarbamoyl]cyclobutanecarbonyljamino]-5-ureido-pentanoyl]amino]benzenesulfonate* **L25-P1**

To a solution of 5-[[(2S)-2-amino-5-ureido-pentanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]benzenesulfonate (22.6 mg; 0.0186 mmol) in DMF (1.4 mL), were successively added a THF solution of (2,3,4,5,6-pentafluorophenyl) 1-[2-[2-(2,5-dioxopyrrol-1-yl)ethoxy]ethylcarbamoyl]cyclobutanecarboxylate (from step 3) (26.8 mg; 0.0562 mmol) and DIPEA (12.9 μL; 0.0742 mmol). The reaction mixture was stirred at room temperature for 2 hours. The crude product solution was purified by direct deposit of the reaction mixture on a X-Bridge column and in using the TFA method to afford **L25-P1** (7.5 mg; 0.0050 mmol) as a white powder. IR ($cm^{-1}$): 3321, 1705/1624, 1666, 1581, 1180/1124, 833/798/756/719/696. $^{1}H$ NMR (400/500 MHz, dmso-d6) δ ppm 10.4 (s), 8.88 (d, 1H), 8.61 (s, 1H), 8.13 (df, 1H), 7.92 (dd, 1H), 7.78 (d), 7.74 (t), 7.63 (d, 1H), 7.52 (dd, 1H), 7.47 (d, 1H), 7.46 (t, 1H), 7.38 (d, 1H), 7.3 (dd, 2H), 7.23 (d, 1H), 7.21 (t, 2H), 7.16 (t, 1H), 7.14 (d, 1H), 7.03 (t, 1H), 7.01 (d, 1H), 7 (s, 2H), 6.73 (t, 1H), 6.22 (d, 1H), 5.99 (m), 5.55 (sl), 5.5 (dd, 1H), 5.25 (AB, 2H), 5.1 (sl, 2H), 4.37 (m, 1H), 4.33 (m, 2H), 3.76 (s, 3H), 3.7 (m, 10H), 3.55 (m, 2H), 3.5 (m, 2H), 3.42 (m, 2H), 3.28/2.52 (2dd, 2H), 3.21 (m, 2H), 3.04 (sl, 3H), 2.97 (m, 2H), 2.4 (m, 4H), 1.85 (w, 3H), 1.74/1.62 (2m, 2H), 1.73 (m, 2H), 1.43/1.35 (2m, 2H). $^{13}C$ NMR (400/500 MHz, dmso-d6) δ ppm 157.5, 152.8, 135.4, 134.9, 131.5, 131.4, 131.4, 131.2, 131.1, 128.7, 121, 120.6, 119.2, 119.2, 116.3, 116, 112.8, 112.2, 111, 74, 69.5, 68.9, 67.4, 66.6, 56.2, 55.3/46.5, 54.1, 45.7, 39.4, 39.2, 37.2, 32.9, 29.7, 29.7, 27.3, 18, 16. $^{19}F$ NMR (400/500 MHz, dmso-d6) δ ppm -74.6, -112.2. HR-ESI+: m/z [M+H]+ = 1509.4867 / 1509.4851 [measured/theoretical]

**Preparation of L26-P1:**

**(2R)-2-[5-[3-chloro-4-[2-[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]-2-[3-[2-[2-[2-[2-[2-[2-[2-(2-methoxyethoxy)ethoxy]ethoxy]ethoxy] ethoxy]ethoxy]ethoxy]ethoxy]propyl] phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate;2,2,2-trifluoroacetate_**

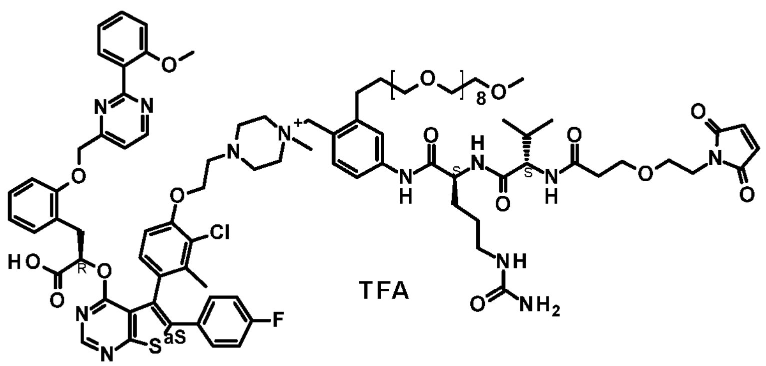

*Step 1: 3-[2-[2-[2-[2-[2-[2-[2-(2-methoxyethoxy)ethoxy]ethoxy]ethoxy]ethoxy]ethoxy]ethoxy] ethoxy]prop-1-yne*

To a solution of 2-[2-[2-[2-[2-[2-[2-(2-methoxyethoxy)ethoxy]ethoxy]ethoxy]ethoxy]ethoxy] ethoxy]ethanol (1.95 g; 6.50 mmol) in THF (25.0 mL), was added at 0°C sodium hydride (260.0 mg; 6.57 mmol). After 5 minutes, a solution of 3-bromoprop-1-yne in toluene (1.42 mL; 13.14 mmol) was added. The reaction mixture was stirred at 0°C for 1 hour and 2 days at room temperature and the progress of the reaction was monitored by UPLC-MS. Then, the reaction mixture was filtered and the filtrate was concentrated to dryness, and purified by silica gel chromatography (gradient DCM in methanol) to afford the title compound (1.74 g; 4.12 mmol) as a colorless oil. $^{1}H$ NMR ($CDCl_3$): 2.43 (t, 1H, *J* = 2.4 Hz), 3.37 (*s*, 3H), 3.53-3.55 *(m,* 2H), 3.64-3.70 *(m,* 30H), 4.20 (*d,* 2H, *J* = 2.4 Hz).

*Step 2: 9H-fluoren-9-ylmethyl N-[(1S)-1-[[4-[[tert-butyl(dimethyl)silyl]oxymethyl]-3-iodo-phenyl]carbamoyl]-4-ureido-butyl]carbamate*

To a solution of [[tert-butyl(dimethyl)silyl]oxymethyl]-3-iodo-aniline (10.0 g; 27.52 mmol) in methanol (70 mL) and DCM (140 mL), were successively added Fmoc-Cit-OH (12.0 g; 30.28 mmol) and EEDQ (8.17 g; 33.03 mmol). The reaction mixture was stirred for 14 hours at room temperature. After the completion of the reaction, the resulting residue was purified by column chromatography on silica gel using DCM / methanol (100/0 to 88/12) as eluent to afford the title compound (17.09 g; 21.97 mmol) as a white solid. $^1$H NMR (DMSO): δ 0.09 (*s,* 6H), 0.91 (*s,* 9H), 1.38-1.48 *(m,* 2H), 1.59-1.68 *(m,* 2H), 2.93-3.05 *(m,* 2H), 4.06-*4.15 (m,* 2H), 4.20-4.29 *(m,* 3H), 4.56 (*s,* 2H), 5.41 (*s,* 2H), 5.98 (*t,* 1H, *J* = 5.5 Hz), 7.30-7.43 *(m,* 5H), 7.55 (*dd,* 1H, *J* = 8.8, 2.1 Hz), 7.69 (*d,* 1H, *J* = 7.8 Hz), 7.74 (*dd,* 1H, *J* = 7.2, 3.4 Hz), 7.89 (*d*, 1H, *J* = 7.5 Hz), 8.25 (*d*, 1H, *J* = 1.5 Hz), 10.12 (*s,* 1H).

*Step 3: (2S)-2-amino-N-[4-[[tert-butyl(dimethyl)silyl]oxymethyl]-3-iodo-phenyl]-5-ureido-pentanamide*

To a solution of 9H-fluoren-9-ylmethyl N-[(1S)-1-[[4-[[tert-butyl(dimethyl)silyl]oxymethyl]-3-iodo-phenyl]carbamoyl]-4-ureido-butyl]carbamate (17.08 g; 23.00 mmol) in THF (120 mL), was added dimethylamine 2M in THF (44.5 mL; 89.00 mmol). The reaction mixture was stirred for 15 hours at room temperature. After concentration to dryness, the resulting residue was purified by column chromatography on $C_{18}$ using water / acetonitrile (98/02 to 0/100) as eluent to afford compound the title compound (5.47 g; 10.50 mmol) as a white solid. $^1$H NMR (DMSO): δ 0.0 (*s,* 6H), 0.81 (*s,* 9H), 1.27-1.38 *(m,* 3H), 1.47-1.53 *(m,* 1H), 2.83-2.89 *(m,* 2H), 3.16-3.19 *(m,* 1H), 4.46 (*s,* 2H), 5.26 (*s,* 2H), 5.82 (*t,* 1H, *J* = 5.6 Hz), 7.24 (*d,* 1H, *J* = 8.5 Hz), 7.50 (*dd,* 1H, *J* = 8.3, 2.0 Hz), 8.17 (*d,* 1H, *J* = 2.0 Hz).

*Step 4: 9H-fluoren-9-ylmethyl N-[(1S)-1-[[(1S)-1-[[4-[[tert-butyl(dimethyl)silyl]oxymethyl]-3-iodo-phenyl]carbamoyl]-4-ureido-butyl]carbamoyl]-2-methyl-propyl]carbamate*

To a solution of (2S)-2-amino-N-[4-[[tert-butyl(dimethyl)silyl]oxymethyl]-3-iodophenyl]-5-ureido-pentanamide (3.00 g; 5.76 mmol) in 2-methyltetrahydrofuran (240 mL), were successively added Fmoc-Val-Osu (8.65 g; 8.65 mmol) and DIPEA (1.90 mL; 11.53 mmol). The reaction mixture was stirred for 15 hours at room temperature. The reaction mixture was filtered through a sintered funnel and the recovered solid was washed with 2-methyltetrahydrofuran (2 x 250 mL), then dried under high vacuum to afford the title compound (3.57 g; 4.24 mmol) as a white solid. $^1$H NMR (DMSO): δ 0.10 (s, 6H), 0.83-0.95 *(m,* 15H), 1.27-1.52 *(m,* 2H), 1.52-1.75 *(m,* 2H), 1.93-2.07 *(m,* 1H), 2.88-3.09 *(m,* 2H), 3.93 (*t,* 1H, *J*= 8.0 Hz), 4.17-4.49 *(m,* 4H), 4.56 (s, 2H), 5.40 (*s,* 2H), 5.96 (*t,* 1H, *J* = 5.6 Hz), 7.27-7.37 *(m,* 3H), 7.37-7.48 *(m,* 3H), 7.54 (*d*, 1H, *J* = 8.0 Hz), 7.74 (*t,* 2H, *J* = 7.2 Hz), 7.88 (*d*, 2H, *J* = 7.6 Hz), 8.13 (d, 1H, *J* = 7.6 Hz), 8.22 (*s,* 1H), 10.11 (*s,* 1H).

*Step 5: 9H-fluoren-9-ylmethylN-[(1S)-1-[[(1S)-1-[[4-[[tert-butyl(dimethyl)silyl]oxymethyl]-3-[3-[2-[2-[2-[2-[2-[2-[2-(2-methoxyethoxy)ethoxy]ethoxy]ethoxy]ethoxy]ethoxy]ethoxy]ethoxy] prop-1-ynyl]phenyl]carbamoyl]-4-ureido-butyl]carbamoyl]-2-methyl-propyl]carbamate*

To a solution of 9H-fluoren-9-ylmethyl N-[(1S)-1-[[(1S)-1-[[4-[[tert-butyl(dimethyl)silyl] oxymethyl]-3-iodo-phenyl]carbamoyl]-4-ureido-butyl]carbamoyl]-2-methyl-propyl]carbamate (1.23 g; 1.46 mmol) in dimethylformamide (15.40 mL), were added successively 3-[2-[2-[2-[2-[2-[2-[2-(2-methoxyethoxy)ethoxy]ethoxy]ethoxy]ethoxy]ethoxy]ethoxy] ethoxy]prop-1-yne (930.0 mg; 2.20 mmol) and DIPEA (2.47 mL; 14.92 mmol). After 3 purges with argon, $Pd(PPh_3)_2Cl_2$ (220 mg; 0.307 mmol) and CuI (68.0 mg; 0.36 mmol) were added and the reaction mixture was purged with argon 3 times. The reaction mixture was stirred for 3 hours at room temperature and the progress of the reaction was monitored by UPLC-MS. The mixture was diluted with isopropyl acetate (200 mL) and washed with brine (3 x 150 mL). The combined organic layers were dried over sodium sulfate, filtered and concentrated to dryness. The crude product was purified by C18 reverse phase prep-HPLC by direct deposit of the reaction mixture on the X-Bridge column ad using neutral method to afford the title compound (790.0 mg; 0.70 mmol) as a pale yellow gum. $^1$H NMR (DMSO): δ 0.08 (s, 6H), 0.85-0.90 (*m*, 15H), 1.36-1.45 (*m*, 2H), 1.58-1.71 (*m*, 2H), 1.97-2.00 (*m*, 1H), 2.93-3.03 (*m*, 2H), 3.23 (*s*, 3H), 3.40-3.43 (*m*, 2H), 3.49-3.52 (*m*, 25H), 3.56-3.58 (*m*, 2H), 3.63-3.66 (*m*, 2H), 3.93 (*dd*, 1H, *J* = 8.9, 6.9 Hz), 4.23-4.32 (*m*, 3H), 4.37-4.43 (*m*, 3H), 4.75 (*s*, 2H), 5.39 (*s*, 2H), 5.97 (*t*, 1H, *J* = 6.1 Hz), 7.30-7.43 (*m*, 6H), 7.51-7.54 (*m*, 1H), 7.72-7.78 (*m*, 3H), 7.88 (*d*, 2H *J* = 7.5 Hz), 8.12 (*d*, 2H, *J* = 7.4 Hz), 10.10 (*s*, 1H).

*Step 6: 9H-fluoren-9-ylmethyl N-[(1S)-1-[[(1S)-1-[[4-(hydroxymethyl)-3-[3-[2-[2-[2-[2-[2-[2-[2-(2-methoxyethoxy) ethoxy]ethoxy]ethoxy]ethoxy]ethoxy]ethoxy]ethoxy]prop-1-ynyl]phenyl]carbamoyl]-4-ureido-butyl]carbamoyl]-2-methyl-propyl]carbamate*

To a solution of 9H-fluoren-9-ylmethyl N-[(1S)-1-[[(1S)-1-[[4-[[tert-butyl(dimethyl)silyl] oxymethyl]-3-[3-[2-[2-[2-[2-[2-[2-[2-(2-methoxyethoxy)ethoxy]ethoxy]ethoxy]ethoxy]ethoxy] ethoxy]ethoxy]prop-1-ynyl] phenyl]carbamoyl]-4-ureido-butyl]carbamoyl]-2-methylpropyl]carbamate (452 mg; 0.40 mmol) in tetrahydrofuran (0.60 mL) and water (0.90 mL), was added acetic acid (4.17 mL; 72.78 mmol). The reaction mixture was stirred for 22 hours at room temperature and the progress of the reaction was monitored by UPLC-MS. After concentration to dryness, the crude product was purified by C18 reverse phase prep-HPLC by direct deposit of the reaction mixture on the X-Bridge column ad using neutral method to afford the title compound (327 mg, 0.32 mmol) as a white gum. $^1$H NMR (DMSO): δ 0.87 (dd, 6H, *J* = 11.7, 6.8 Hz), 1.36-1.45 (*m*, 2H), 1.58-1.71 (*m*, 2H), 1.97-2.00 (*m*, 1H), 2.93-3.02 (*m*, 2H), 3.23 (*s*, 3H), 3.31 (*s*, 5H), 3.40-3.43 (*m*, 2H), 3.48-3.53 (*m*, 21H), 3.54-3.64 (*m*, 6H), 3.91-3.95 (*m*, 1H), 4.23-4.42 (*m*, 4H), 4.56 (*d*, 2H, *J* = 5.5 Hz), 5.19 (*t*, 1H, *J* = 5.6 Hz), 5.39 (*s*, 2H), 5.96 (*t*, 1H, *J* = 5.8 Hz), 7.30-7.34 (*m*, 2H), 7.39-7.43 (*m*, 4H), 7.50-7.52 (*m*, 1H), 7.72-7.76 (*s*, 3H), 7.88 (*d*, 1H *J* = 7.5 Hz), 8.12 (*d*, 2H, *J* = 7.4 Hz), 10.06 (*s*, 1H).

*Step 7: 9H-fluoren-9-ylmethyl N-[(1S)-1-[[(1S)-1-[[4-(hydroxymethyl)-3-[3-[2-[2-[2-[2-[2-[2-[2-(2-methoxyethoxy) ethoxy]ethoxy]ethoxy]ethoxy]ethoxy]ethoxy]ethoxy]propyl]phenyl] carbamoyl]-4-ureido-butyl]carbamoyl]-2-methyl-propyl]carbamate*

To a solution of 9H-fluoren-9-ylmethyl N-[(1S)-1-[[(1S)-1-[[4-(hydroxymethyl)-3-[3-[2-[2-[2-[2-[2-[2-[2-(2-methoxyethoxy)ethoxy]ethoxy]ethoxy]ethoxy]ethoxy]ethoxy]ethoxy]prop-1-ynyl]phenyl]carbamoyl]-4-ureido-butyl]carbamoyl]-2-methyl-propyl]carbamate (327.0 mg; 0.32 mmol) in THF (3.7 mL), was added acetic acid (0.37 mL). After 3 purges with argon, Pt/C 5% (195 mg) was added and after 3 more purges with argon, the reaction mixture was placed under hydrogen atmosphere and stirred for 18 hours at room temperature and the progress of the reaction was monitored by UPLC-MS. The mixture was filtered through PTFE and the filtrate was concentrated to dryness, then triturated in dichloromethane/pentane (1/4 mixture, 50 mL). The precipitate was recovered by filtration to afford, after drying, the title compound (130 mg; 0.13 mmol) as a white solid. $^1$H NMR (DMSO): δ 0.85-0.89 (*m*, 6H), 1.23-1.46 (*m*, 2H), 1.56-1.76 (*m*, 4H), 1.97-2.02 (*m*, 1H), 2.56-2.60 (*m*, 2H), 2.91-3.04 (*m*, 2H), 3.23 (*s*, 3H), 3.38-3.43 (*m*, 4H), 3.48-3.54 (*m*, 30H), 3.93 (*dd*, 1H, *J* = 8.9, 6.9 Hz), 4.21-4.31 (*m*, 3H), 4.38-4.41 (*m*, 1H), 4.45 (*d*, 2H, *J* = 5.3 Hz), 4.94 (*t*, 1H, *J* = 5.3 Hz), 5.37 (*s*, 2H), 5.95 (*t*, 1H, *J* = 5.8 Hz), 7.25 (*d*, 1H, *J* = 8.3 Hz), 7.30-7.34 (*m*, 2H), 7.39-7.43 (*s*, 5H), 7.72-7.76 (*m*, 2H), 7.88 (*d*, 1H *J* = 7.5 Hz), 8.06 (d, 2H, *J* = 7.6 Hz), 9.88 (s, 1H). UPLC-MS: MS (ESI) m/z [M+H]+ = 1026.52

*Step 8: 9H-fluoren-9-ylmethyl N-[(1S)-1-[[(1S)-1-[[4-(bromomethyl)-3-[3-[2-[2-[2-[2-[2-[2-[2-(2-methoxyethoxy) ethoxy] ethoxy]ethoxy]ethoxy]ethoxy]ethoxy]ethoxy]propyl]phenyl] carbamoyl]-4-ureido-butyl]carbamoyl]-2-methyl-propyl]carbamate*

To a solution of 9H-fluoren-9-ylmethyl N-[(1S)-1-[[(1S)-1-[[4-(hydroxymethyl)-3-[3-[2-[2-[2-[2-[2-[2-[2-(2-methoxyethoxy)ethoxy]ethoxy]ethoxy]ethoxy]ethoxy]ethoxy]ethoxy]propyl] phenyl]carbamoyl]-4-ureido-butyl]carbamoyl]-2-methyl-propyl]carbamate (60 mg; 0.0584 mmol) in THF (6.6 mL), was added dropwise at 0°C $PBr_3$ (1M solution in THF) (0.0877 mL; 0.0877 mmol). The solution was then stirred 3 hours at room temperature. The progress of the reaction was monitored by UPLC-MS after addition in an aliquot morpholine to react the bromo expected compound. The reaction was worked up with an aqueous saturated $NH_4Cl$ solution (50 μL). After 5 minutes the mixture was dryed over $MgSO_4$, filtered and washed with THF (2 ml) to afford the bromo title compound as a THF solution used crude in the next step. UPLC-MS analysis is done after methanol and morpholine addition.

*Step 9: (2R)-2-[5-[3-chloro-4-[2-[4-[[4-[[(2S)-2-[[(2S)-2-(9H-fluoren-9-ylmethoxycarbonylamino)-3-methyl-butanoyl] amino]-5-ureido-pentanoyl]amino]-2-[3-[2-[2-[2-[2-[2-[2-[2-(2-methoxyethoxy)ethoxy]ethoxy]ethoxy]ethoxy]ethoxy] ethoxy]ethoxy]propyl] phenyl]methyl]-4-methyl-piperazin-4-ium- 1-yl]ethoxy]-2-methyl-phenyl]-6-(4-fluorophenyl)thieno [2,3-d]pyrimidin-4-yl]oxy-3-[2-[[2-(2-methoxyphenyl)pyrimidin-4-yl]methoxy]phenyl]propanoic acid;2,2,2-trifluoroacetate*

To the THF solution of 9H-fluoren-9-ylmethyl N-[(1S)-1-[[(1S)-1-[[4-(bromomethyl)-3-[3-[2-[2-[2-[2-[2-[2-[2-(2-methoxyethoxy)ethoxy]ethoxy]ethoxy]ethoxy]ethoxy]ethoxy]ethoxy]propyl] phenyl]carbamoyl]-4-ureido-butyl]carbamoyl]-2-methyl-propyl]carbamate from the previous step (0.0584 mmol), were successively added DMF (1.5 mL), (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl] oxy-3-[2-[[2-(2-methoxyphenyl)pyrimidin-4-yl]methoxy]phenyl]propanoic acid **P1** (46.1 mg; 0.0527 mmol) and DIPEA (0.173 mL; 0.995 mmol). The reaction mixture was stirred 20 hours at room temperature and the progress of the reaction was monitored by UPLC-MS. The crude mixture containing the expected title compound and the Fmoc-deprotected one (expected in step 10) is used in the deprotective next step. UPLC-MS: MS (ESI) m/z [M-Fmoc+H+H]+ = 1660.99

*Step 10: (2R)-2-[5-[4-[2-[4-[[4-[[(2S)-2-[[(2S)-2-amino-3-methyl-butanoyl]amino]-5-ureido-pentanoyl]amino]-2-[3-[2-[2-[2-[2-[2-[2-[2-(2-methoxyethoxy)ethoxy]ethoxy]ethoxy]ethoxy] ethoxy]ethoxy]ethoxy]propyl]phenyl] methyl]-4-methyl-piperazin-4-ium-1-yl]ethoxy]-3-chloro-2-methyl-phenyl]-6-(4-fluorophenyl)thieno[2,3-d]pyrimidin-4-yl] oxy-3-[2-[[2-(2-methoxyphenyl) pyrimidin-4-yl]methoxy]phenyl]propanoic acid; 2, 2, 2-trifluoroacetate; 2, 2, 2-trifluoroacetic acid*

To the crude mixture obtained in the previous step in DMF was added piperidine (11.6 μL; 0.117 mmol). The reaction mixture was stirred at room temperature for 15 hours and the progress of the reaction was monitored by UPLC-MS. After completion of the reaction, the crude product was purified by $C_{18}$ reverse phase prep-HPLC by direct deposit of the reaction mixture on the X-Bridge column in using the TFA method to give the title compound (29.2 mg; 0.0155 mmol) as a white powder. IR: 3600-2300, 1672, 1602, 1541+1516. HR-ESI+: m/z [M-$CF_3COO$]+ = 1660.7574 (1660.7575 theoretical)

*Step 11: (2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[3-[2-[2-[2-[2-[2-[2-[2-(2-methoxyethoxy)ethoxy]ethoxy]ethoxy]ethoxy] ethoxy]ethoxy]ethoxy]propyl] phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate;2,2,2-trifluoroacetate* **L26-P1**

To a solution of (2R)-2-[5-[4-[2-[4-[[4-[[(2S)-2-[[(2S)-2-amino-3-methyl-butanoyl]amino]-5-ureido-pentanoyl] amino]-2-[3-[2-[2-[2-[2-[2-[2-[2-(2-methoxyethoxy)ethoxy]ethoxy]ethoxy] ethoxy]ethoxy]ethoxy]ethoxy]propyl]phenyl] methyl]-4-methyl-piperazin-4-ium-1-yl]ethoxy]-3-chloro-2-methyl-phenyl]-6-(4-fluorophenyl)thieno[2,3-d]pyrimidin-4-yl] oxy-3-[2-[[2-(2-methoxyphenyl)pyrimidin-4-yl]methoxy]phenyl]propanoic acid; 2,2,2-trifluoroacetate; 2,2,2-trifluoroacetic acid (42.5 mg; 0.0225 mmol) in DMF (1.28 mL), were successively added a solution of (2,5-dioxopyrrolidin-1-yl) 3-[2-(2,5-dioxopyrrol-1-yl)ethoxy]propanoate (Brodpharm 21854) (7.71 mg; 0.0247 mmol) and DIPEA (19.6 μL; 0.112 mmol). The reaction mixture was stirred at room temperature for 15 hours and the progress of the reaction was monitored by UPLC-MS. The crude product was purified by $C_{18}$ reverse phase prep-HPLC by direct deposit of the reaction mixture on the X-Bridge column and using the TFA method to afford **L26-P1** (28 mg; 0.0151 mmol) as a white powder. HR-ESI+: m/z [M-$CF_3COO$]+ = 1855.8105 (1855.8106 theoretical)

**Preparation of L27-P1:**

**(2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-3-methyl-5-ureido-pentanoyl]amino]-2-sulfo-phenyl]methyl]-4-methyl-piperazin-4-ium-1-yl] ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid**

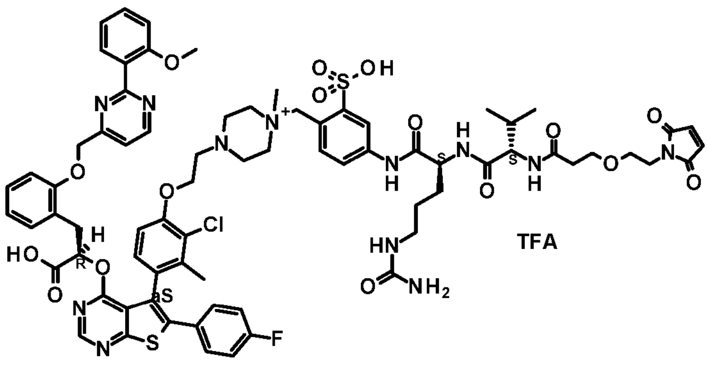

*Step 1: 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonate*

5-[[(2S)-2-[[(2S)-2-(9H-fluoren-9-ylmethoxycarbonylamino)-3-methyl-butanoyl]amino]-5-ureido-pentanoyl] amino]-2-(hydroxymethyl)benzenesulfonic acid (300 mg; 0.4263 mmol) was dissolved in anhydrous NMP (6 mL) at room temperature. In parallel, a solution of $SOCl_2$ (206 μL) in NMP (6 mL) was prepared. To the reaction, were added 6 times

over a 75minutes period, a solution 900 μL of the $SOCl_2$ solution. After the last addition, the reaction mixture was stirred at room temperature for 15minutes. The crude product was purified by direct deposit of the reaction mixture on an Oasis column in using the TFA method to afford the title compound (138 mg; 0.1971 mmol) as a white powder. $^1$H NMR (400 MHz, dmso-d6) δ ppm 10.15+8.1+7.42+6.0 (s+2d+m, 4H), 7.9 (m,HH), 7.75 (m, 3H), 7.42+7.31 (2m, 5H), 5.23 (s, 2H), 4.4 (m, 1H), 4.3-4.2 (m, 3H), 3.95 (dd, 1H), 3.0 (m, 2H), 2.0 (m, 1H), 1.7 + 1.6 (2m, 2H), 1.48 + 1.37 (2m, 2H), 0.88 (2d, 6H). HR-ESI+ : m/z [M+H]+ = 700.2199 / 700.2202 [measured/theoretical]

*Step 2: 5-[[(2S)-2-[[(2S)-2-amino-3-methyl-butanoyl]amino]-5-ureido-pentanoyl]amino]-2-[[4-[2-[2-chloro-4-[6-(4-fluorophenyl)-4-[(1R)-2-methoxy-1-[[2-[[2-(2-methoxyphenyl)pyrimidin-4-yl]methoxy]phenyl]methyl]-2-oxo-ethoxy]thieno [2,3-d]pyrimidin-5-yl]-3-methyl-phenoxy]ethyl]-1-methyl-piperazin-1-ium-1-yl]methyl]benzenesulfonic acid*

To a solution of 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonate (82.4 mg; 0.1177 mmol) in anhydrous NMP (2.5 mL), was added at room temperature DIEA (94 μL; 0.540 mmol) followed by methyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate (60 mg; 0.067 mmol) and TBAI (12.4 mg; 0.034 mmol). The reaction was stirred at 80°C for 4 hours and overnight at room temperature. Then, 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonate was again added (14 mg; 0,017 mmol) followed by TBAI (17 μL; 0.0337 mmol) and the reaction was stirred at 80°C for 4 hours and then overnight at room temperature. The Fmoc deprotection step was realized in adding DEA (53 μL; 0.515 mmol) to the reaction and stirring at room temperature overnight. Purification was realized by direct injection of the mixture on Oasis eluted with a gradient of a solution A :$H_2O/CH_3CN/NH_4HCO_3$ (1960 ml/40/3.16 g) to a solution B: $CH_3CN/H_2O/NH_4HCO_3$ (1600 ml/400 ml/3.16 g) to afford the title compound (17 mg; 0.009 mmol). UPLC-MS: MS (ESI) m/z [M]+ = 1329

*Step 3 : (2R)-2-[5-[4-[2-[4-[[4-[[(2S)-2-[[(2S)-2-amino-3-methyl-butanoyl]amino]-5-ureido-pentanoyl]amino]-2-sulfo-phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid*

To a mixture of 5-[[(2S)-2-[[(2S)-2-amino-3-methyl-butanoyl]amino]-5-ureido-pentanoyl] amino]-2-[[4-[2-[2-chloro-4-[6-(4-fluorophenyl)-4-[(1R)-2-methoxy-1-[[2-[[2-(2-methoxyphenyl)pyrimidin-4-yl]methoxy]phenyl]methyl]-2-oxo-ethoxy]thieno[2,3-d]pyrimidin-5-yl]-3-methyl-phenoxy]ethyl]-1-methyl-piperazin-1-ium-1-yl]methyl]benzenesulfonic acid (18 mg; 0.014 mmol) in dioxane/water (1 mL/1 mL) was added $LiOH \cdot H_2O$ (2.3 mg; 0.054 mmol) and the reaction was stirred at room temperature for 4 hours. The solution was adjusted to pH 6-7 by addition of HCl 1N and dioxane was evaporated under reduced pressure. Purification was realized by direct injection of the mixture on Oasis eluted with a gradient of a solution A: $H_2O/CH_3CN/NH_4HCO_3$ (1960 ml/40/3.16 g) to a solution B: $CH_3CN/H_2O/NH_4HCO_3$ (1600 ml/400 ml/3.16 g) to afford the title compound compound (11mg; 0.008 mmol).

*Step 4: (2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-3-methyl-5-ureido-pentanoyl]amino]-2-sulfo-phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy] phenyl]propanoic acid* **L27-P1**

To a solution of (2R)-2-[5-[4-[2-[4-[[4-[[(2S)-2-[[(2S)-2-amino-3-methyl-butanoyl]amino]-5-ureido-pentanoyl] amino]-2-sulfo-phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (10.5 mg; 0.007 mmol) in DMF (0.4 mL), was added (2,5-dioxopyrrolidin-1-yl) 3-[2-(2,5-dioxopyrrol-1-yl)ethoxy]propanoate (5.7 mg; 0.018 mmol) and the solution was stirred at room temperature for 4 hours.The solution was purified by X-Bridge column $C_{18}$ by direct deposit of the reaction mixture on the column and in using the TFA method to afford **L27-P1** (10 mg; 0.006 mmol). HR-ESI+: [M+H]+ 1511.5018 / 1511.5002 [measured/theoretical]

**Preparation of L28-P1:**

**(2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]propanoyl]amino]-2-sulfophenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate**

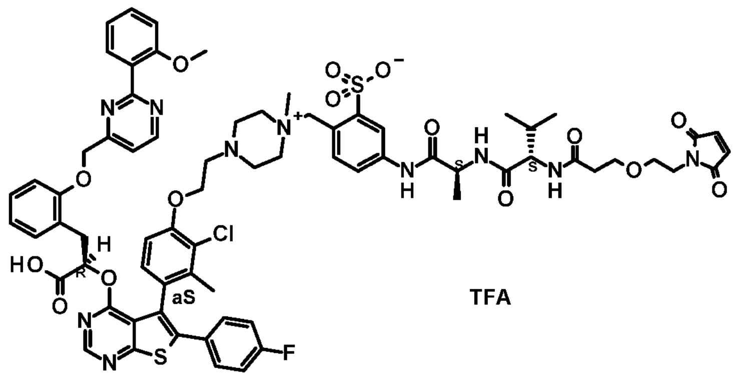

TFA

*Step 1: 9H-fluoren-9-ylmethyl N-[(1S)-1-[[(1S)-2-[4-(chloromethyl)-3-methyl-anilino]-1-methyl-2-oxo-ethyl]carbamoyl]-2-methyl-propyl]carbamate*

To a solution of 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate (504.1 mg; 0.816 mmol) in NMP (5 mL), were added 6 times over a 75 minutes period, a solution of $SOCl_2$ (60 μL; 0.816 mmol) in NMP (500μL). The reaction mixture was stirred at room temperature for 15 minutes. The crude product was purified by direct deposit of the reaction mixture on an Oasis column in using the TFA method to afford (337 mg) as a a mixture of 70% the title compound (384 mmol) and 30% of the starting material (170 mmol) as a white powder. IR ($cm^{-1}$): 3600 to 2400, 1688+1648, 1599, 1518, 1022. UPLC-MS: MS (ESI) m/z [M+H]+ = 614.17+616.18 (Cl)

*Step 2: (2R)-2-[5-[4-[2-[4-[[4-[[(2S)-2-[[(2S)-2-amino-3-methyl-butanoyl]amino]propanoyl]amino]-2-methyl-phenyl] methyl]-4-methyl-piperazin-4-ium-1-yl]ethoxy]-3-chloro-2-methyl-phenyl]-6-(4-fluorophenyl)thieno[2,3-d]pyrimidin-4-yl] oxy-3-[2-[[2-(2-methoxyphenyl)pyrimidin-4-yl]methoxy]phenyl]propanoic acid;2,2,2-trifluoroacetate*

To a solution of methyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate (152 mg; 0.171 mmol) in NMP (4.5 ml), were successively added 9H-fluoren-9-ylmethyl N-[(1S)-1-[[(1S)-2-[4-(chloromethyl)-3-methyl-anilino]-1-methyl-2-oxo-ethyl]carbamoyl]-2-methyl-propyl]carbamate (150 mg; 0.171 mmol), DIPEA (238 μL; 1.37 mmol) and TBAI (76 mg; 0.205mmol). The reaction mixture was stirred at 80°C for 28 hours. The reaction mixture is cooled down to room temperature. A solution of $LiOH.H_2O$ (13.7 mg, 0.342 mmol) in water (500 μL) is then added. The reaction mixture was stirred at room temperature for 48 hours. The crude product was purified by $C_{18}$ reverse phase prep-HPLC by direct deposit of the reaction mixture on the X-Bridge column and using the TFA method to afford the title compound (40 mg; 0.0325 mmol) as a white powder. UPLC-MS: MS (ESI) m/z [M]+ = 1230.61+1232.61 (Cl)

*Step 3: (2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]propanoyl]amino]-2-sulfo-phenyl]methyl]-4-methyl-piperazin-4-ium-1-yl]ethoxy]-2-methy]-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate* **L28-P1**

To a solution of (2R)-2-[5-[4-[2-[4-[[4-[[(2S)-2-[[(2S)-2-amino-3-methyl-butanoyl]amino]propanoyl]amino]-2-methyl-phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate (6.0 mg; 0.0049 mmol) in solution in DMF (180 μL), were successively added (2,5-dioxopyrrolidin-1-yl) 3-[2-[2-(2,5-dioxopyrrol-1-yl)ethoxy]ethylcarbamoyl]oxetane-3-carboxylate (2.3 mg; 0.0073 mmol) and DIPEA (3.0 μL; 0.017 mmol). The reaction mixture was stirred at room temperature for 1.5 hours and was monitored by UPLC-MS. The crude product was purified by direct deposit of the reaction mixture on the X-Bridge column in using the TFA method to afford **L28-P1** (2.9 mg; 0.0020 mmol) as a white powder. HR-ESI+: m/z [M+H]+ = 1425.4534/ 1425.4527 [measured/theoretical]

**Preparation of L29-C3:**

**(2R)-2-[5-[4-[2-[4-[[4-[[(2S)-2-[[(2S)-2-[[2-(2-azidoethoxy)acetyl]amino]-3-methyl-butanoyl]amino]propanoyl]amino]-2-sulfo-phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid**

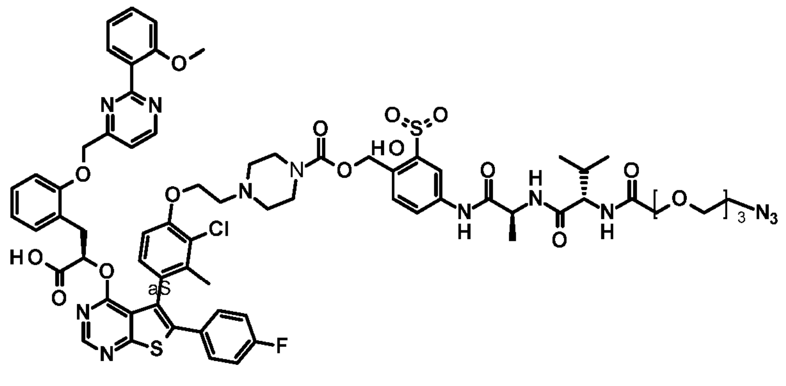

*Step 1: sodium; 5-[[(2S)-2-(tert-butoxycarbonylamino)propanoyl]amino]-2-(hydroxymethyl)benzenesulfonate*

To a solution of Boc-L-Ala-OH (588 mg; 3.11 mmol) in DMF (38.6 mL), were successively added HATU (1.77 g; 4.67 mmol), sodium 5-amino-2-(hydroxymethyl)benzenesulfonate (771 mg; 3.42 mmol) and DIPEA (1.29 mL; 7.78 mmol). The reaction mixture was stirred for 16 hours at room temperature then concentrated to dryness and co-evaporated with water to afford the crude reaction mixture. The resulting residue was purified by column chromatography on silica gel using ethyl acetate/methanol 95:5 to 80:20 as eluent to afford the title compound (1.17 g; 2.95 mmol) as a white solid. $^1H$ NMR (DMSO): δ 1.24 (*s*, 9H), 1.38 (*m*, 3H), 4.05-1.44 (*m*, 1H), 4.73 (*d*, 2H, *J* = 4.8 Hz), 5.04 (*t,* 1H, *J* = 5.6 Hz); 6.97-7.02 (*m*, 1H), 7.33 (*d*, 1H, *J* = 8 Hz), 7.65-7.70 (*m*, 1H), 7.83 (*s*, 1H), 9.91 (s, 1H).

*Step 2: 5-[[(2S)-2-aminopropanoyl]amino]-2-(hydroxymethyl)benzenesulfonate, hydrochloride*

Sodium 5-[[(2S)-2-(tert-butoxycarbonylamino)propanoyl]amino]-2-(hydroxymethyl) benzenesulfonate (1.17 g; 2.95 mmol; 1 eq.) was suspended in a solution of HCl 4N in dioxane (10 mL). The mixture was stirred at room temperature for 2 hours then concentrated to dryness to afford the crude mixture (982 mg; 2.95 mmol) as a white solid. $^1H$ NMR (DMSO): δ 1.45 (*d*, 3H, *J* = 5.6 Hz), 3.91-4.0 (*m*, 1H), 4.76 (*s*, 2H), 7.41 (*d*, 1H, *J* = 7.6 Hz), 7.66 (*d*, 1H, *J* = 7.6 Hz), 7.85 (*s*, 1H), 8.17 (*s*, 2H), 10.44 (*s*, 1H).

*Step 3: 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate*

To a solution of 5-[[(2S)-2-aminopropanoyl]amino]-2-(hydroxymethyl)benzenesulfonate, hydrochloride (981 mg; 2.95 mmol) in DMF (34.5 mL) were added Fmoc-L-Val-Osu (1.29 g; 2.95 mmol; 1 eq.) and DIPEA (975 μL; 5.9 mmol). The mixture was stirred overnight at room temperature then concentrated to dryness and co evaporated with water to afford the crude mixture. The resulting residue was purified by column chromatography on silica gel using ethyl acetate /methanol 95:5 to 80:20 as eluent to afford the title compound (1.28 g; 2.072 mmol) as a colorless oil. $^1H$ NMR (DMSO): δ 0.80-0.92 (*m*, 6H), 1.30 (*d*, 3H, *J* = 6.4 Hz), 2.02-2.10 (*m*, 1H), 4.17-4.31 (*m*, 3H), 4.37-4.44 (*m*, 1H), 4.73 (*d*, 2H, *J* = 5.6 Hz), 5.04 (*t*, 1H, *J* = 6.4 Hz), 7.28-7.36 (*m*, 3H), 7.37-7.47 (*m*, 3H), 7.66 (*d*, 1H, *J* = 8.4 Hz), 7.71-7.77 (*m*, 2H), 7.83-7.85 (*m*, 1H), 7.88 (*d*, 2H, *J* = 7.6 Hz), 8.14 (*d*, 1H, *J* = 6.4 Hz), 9.99 (*s*, 1H).

*Step 4: 5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]benzenesulfonic acid*

To a suspension of 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate (1.28 g; 2.07 mmol) in THF (65 mL), were added pyridine (875 μL; 10.8 mmol), followed by 4-nitrophenyl chloroformate (1.09 g; 5.41 mmol). The mixture was stirred overnight at room temperature. Then additional 4-nitrophenyl chloroformate (1.09 g; 5.41 mmol; 2.5 eq.) was added. After 5 hours stirring at room temperature, the mixture was concentrated to dryness then purified by column chromatography on $C_{18}$ using water/acetonitrile 90/10 to 0/100 as eluent in 30 minutes. Acetonitrile of the combined tubes was removed, and the rest was

lyophilized to afford the title compound (650 mg; 0.83 mmol) as a white solid. $^1$H NMR (DMSO): δ 0.88 (*m*, 6H), 1.31 (*d*, 3H, *J* = 4.8 Hz), 1.97-2.03 (*m*, 1H), 3.92 (*t*, 1H, *J* = 6.8 Hz), 4.23 (*s*, 2H), 4.24-4.34 (*m*, 1H), 4.42 (*t*, $^1$H *J* = 5.6 Hz), 5.69 (*s*, 2H), 7.30-7.48 (*m*, 6H), 7.62 (*d*, 2H, *J* = 8 Hz), 7.72-7.76 (*m*, 3H), 7.89 (*d*, 2H, *J* = 6.4 Hz), 7.94 (*s*, 1H), 8.18 (*d*, 1H, *J* = 5.6 Hz), 8.33 (*d*, 2H, *J* = 7.6 Hz), 10.11 (*s*, 1H). $^{13}$C NMR (DMSO): δ 18.01, 18.26, 19.21, 30.4, 46.66, 49.05, 59.91, 65.67, 67.82, 117.7, 119.1, 120.06, 122.66, 125.37, 126.33, 127.05, 127.62, 128.0, 138.06, 140.67, 143.77, 143.86, 145.1, 146.23, 151.96, 155.47, 156.12, 171.0, 171.15. LCMS (2-100 ACN/$H_2O$+0.05% TFA): 90.41 % Tr = 12.7 min. Positiv mode 578.41 detected. Negativ mode 759.17 detected

*Step 5: (2S)-2-[[5-[3-chloro-4-[2-[4-[[4-[[(2S)-2-[[(2S)-2-(9H-fluoren-9-ylmethoxycarbonylamino)-3-methyl-butanoyl] amino]propanoyl]amino]-2-sulfo-phenyl]methoxycarbonyl]piperazin-1-yl]ethoxy]-2-methy]-phenyl]-6-(4-fluorophenyl) thieno[2,3-d]pyrimidin-4-yl]methyl]-3-[2-[[2-(2-methoxyphenyl)pyrimidin-4-yl]methoxy]phenyl]propanoic acid*

To a suspension of (2S)-2-[[5-[3-chloro-2-methyl-4-(2-piperazin-1-ylethoxy)phenyl]-6-(4-fluorophenyl)thieno [2,3-d]pyrimidin-4-yl]methyl]-3-[2-[[2-(2-methoxyphenyl)pyrimidin-4-yl]methoxy]phenyl]propanoic acid;2,2,2-trifluoroacetic acid (178.4 mg; 0.183 mmol) in DMF (1.5 mL), were successively added 5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]benzenesulfonic acid (150 mg; 0.192 mmol) and DIPEA (91 μL; 0.549 mmol), The mixture was stirred overnight at room temperature for 15 minutes. The crude product was purified by direct deposit of the reaction mixture on the X-Bridge column in using the $NH_4HCO_3$ method to afford the title compound (176 mg, 0.118 mmol) as a white solid. UPLC-MS: MS (ESI) m/z [M+H]+ = 1482.15+1484.56(Cl)

*Step 6: (2S)-2-[[5-[4-[2-[4-[[4-[[(2S)-2-[[(2S)-2-amino-3-methyl-butanoyl]amino]propanoyl]amino]-2-sulfo-phenyl]methoxycarbonyl]piperazin-1-yl]ethoxy]-3-chloro-2-methyl-phenyl]-6-(4-fluorophenyl)thieno[2,3-d]pyrimidin-4-yl] methyl]-3-[2-[[2-(2-methoxyphenyl)pyrimidin-4-yl]methoxy]phenyl]propanoic acid*

To a solution of (2S)-2-[[5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-sulfophenyl]methoxycarbonyl]piperazin-1-yl]ethoxy]-2-methyl-phenyl]-6-(4-fluorophenyl)thieno[2,3-d]pyrimidin-4-yl]methyl]-3-[2-[[2-(2-methoxyphenyl)pyrimidin-4-yl]methoxy]phenyl] propanoic acid (176 mg; 0.118 mmol) in DMF (1.0 mL), was added piperidine (24.17 μL; 0.237 mmol). The reaction mixture was stirred at room temperature for 18 hours. The crude product was purified by direct deposit of the reaction mixture on a X-Bridge column in using the $NH_4HCO_3$ method to afford the title compound (102 mg; 0.0809 mmol) as a white powder. IR ($cm^{-1}$): 3620-2680, 1683, 1235. UPLC-MS: MS (ESI) m/z [M+H]+ = 1260.37+1262.37(Cl). $^1$H NMR (400 MHz, dmso-d6) δ ppm 10.20 (m, NH), 8.90 (m, 2H), 8.90 (m, 1H), 8.60 (m, NH), 8.60 (s, 1H), 7.90 (m, 1H), 7.78 (m, 1H), 7.70 (d, 1H), 7.55 (d, 1H), 7.48 (t, 1H), 7.45 (d, 1H), 7.3/7.2 (m, 4H), 7.20 (m, 1H), 7.20 (d, 1H), 7.15 (t, 1H), 7.15 (d, 1H), 7.05 (t, 1H), 7.00 (d, 1H), 6.7 (t, 1H), 6.2 (d, 1H), 5.48 (s, 2H), 5.50 (m, 1H), 5.23 (s, 2H), 4.50 (m, 1H), 4.25 (m, 2H), 3.75 (s, 3H), 3.4 (m, 4H), 3.40 (m, 1H), 3.35 (m, 1H) 2.80 (m, 2H), 2.5 (m, 4H), 2.50 (m, 1H), 2.05 (m, 1H), 1.80 (s, 3H), 1.30 (d, 3H), 0.90 (dd, 6H)

*Step 7: (2,3,4,5,6-pentafluorophenyl) 2-[2-[2-(2-azidoethoxy)ethoxy]ethoxy]acetate*

To a solution of 2-[2-[2-(2-azidoethoxy)ethoxy]ethoxy]acetic acid (74 mg; 0.317 mmol) in THF (500 μL) were succesively added 2,3,4,5,6-pentafluorophenol (70 mg; 0.380 mmol) in solution in THF (500 μL) and DCC (78.5 mg; 0.380 mmol) in solution in THF (500 μL). The reaction mixture was stirred at room temperature for 18 hours. The crude suspension is filtred through a coton pad in a pasteur pipette and the solution is used without further work-up in step 8.

*Step 8: (2R)-2-[5-[4-[2-[4-[[4-[[(2S)-2-[[(2S)-2-[[2-(2-azidoethoxy)acetyl]amino]-3-methyl-butanoyl]amino]propanoyl] amino]-2-sulfo-phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid* **L29-C3**

To a solution of (2S)-2-[[5-[4-[2-[4-[[4-[[(2S)-2-[[(2S)-2-amino-3-methyl-butanoyl]amino]propanoyl]amino]-2-sulfo-phenyl]methoxycarbonyl]piperazin-1-yl]ethoxy]-3-chloro-2-methyl-phenyl]-6-(4-fluorophenyl)thieno[2,3-d]pyrimidin-4-yl]methyl]-3-[2-[[2-(2-methoxyphenyl)pyrimidin-4-yl]methoxy]phenyl]propanoic acid (100 mg; 0.0793 mmol) in DMF (500 μL), were succesively added the solution of (2,3,4,5,6-pentafluorophenyl) 2-[2-[2-(2-azidoethoxy)ethoxy] ethoxy]acetate in THF from step 7 (0.317 mmol) and DIPEA (39.3 μL; 0.238 mmol). The reaction mixture was stirred at room temperature for 15 minutes. The crude product was purified by direct deposit of the reaction mixture on a X-Bridge column in using the $NH_4HCO_3$ method to afford **L29-C3** (43 mg, 0.0291 mmol) as a white powder. IR ($cm^{-1}$): 3257, 2102, 1663, 1236/1082, 834/756. $^1$H NMR (400 MHz, dmso-d6) δ ppm 10.03 (s, NH), 8.87 (d, 1H), 8.59 (sNC, 1H), 8.35 (d, NH), 7.89 (df, 1H), 7.69 (dd, 1H), 7.66 (m, 1H), 7.53 (d, 1H), 7.45 (t, 1H), 7.36 (d, 1H), 7.29 (dd, 2H), 7.21 (d, 1H), 7.20 (t, 2H), 7.19 (d, 1H), 7.14 (d, 1H), 7.13 (m, 1H), 7.09 (m, NH), 7.03 (t, 1H), 7.00 (d, 1H), 6.72 (t, 1H), 6.24 (d, 1H), 5.48 (dNC, 1H), 5.44 (s,

2H), 5.23(AB, 2H), 4.40 ( t, 1H), 4.30 (dd, 1H), 4.24 (m, 2H), 3.94 (s, 2H), 3.75 (s, 3H), 3.58 (m, 10H), 3.38 (m, 4H), 3.36 (t, 2H), 3.30 (NC, 1H), 2.75 (t, 2H), 2.50 (m, 4H), 2.50 (m, 1H), 2.00 (m, 1H), 1.51 (s, 3H), 1.30 (d, 3H), 0.88/0.82 (2d, 6H). $^{13}C$ NMR (100/126 MHz, dmso-d6) δ ppm 158.2, 131.6, 131.1, 130.9, 130.9, 130.9, 130.9, 128.3, 126.7, 120.7, 120.4, 119.3, 117.9, 116.2, 112.5, 112.1, 110.8, 70.2, 70.2; 69.6, 67.7, 64.0, 56.7, 56.5, 55.8, 53.2, 50.4, 49.2, 43.8, 31.7, 19.7, 18.7, 18.4, 17.8. $^{19}F$ NMR (376/470 MHz, dmso-d6) δ ppm -112.0. HR-ESI+: m/z [M+H]+ = 1475.4643/1475.4638; [2M+H+Ca]3+ = 996.6289/996.6273; [M+2H]2+ = 738.2378/738.2355; [M+H+Na]2+ = 749.2255/749.2265.

**Example 2. Synthesis and Characterization of Additional Linkers, Linker-Payloads, and Precursors thereof.**

Exemplary linkers, linker-payloads, and precursors thereof were synthesized using exemplary methods described in this example.

**Synthesis of 2-(bromomethyl)-4-nitrobenzoic acid**

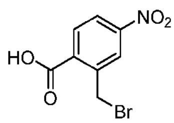

To a stirred solution of 2-methyl-4-nitrobenzoic acid (300 g, 1.5371 mol) in $CCl_4$ (3000 mL) was added NBS (300.93 g, 1.6908 mol) and AIBN (37.86 g, 0.2305 mol) at room temperature. The reaction mixture was stirred at 80°C for 16 h. Reaction mixture was monitored by TLC analysis. The reaction mixture was diluted with sat. $NaHCO_3$ solution (2 L) and extracted with ethyl acetate (2 x 2 L). The combined organic layer was dried over anhydrous sodium sulphate and concentrated under reduced pressure. The crude compound was purified by column chromatography on silica gel using 2-3% of ethyl acetate in petroleum-ether as an eluent and 2-(bromomethyl)-4-nitrobenzoic acid was obtained. $^1H$ NMR (400 MHz, $CDCl_3$): δ 8.35 (d, J=2.0 Hz, 1H), 8.20 (q, J=8.8, 2.4 Hz, 1H), 8.12 (d, J=8.8 Hz, 1H), 4.97 (s, 2H), 4.00 (s, 3H).

**Synthesis of 4-nitro-2-((prop-2-yn-1-yloxy)methyl)benzoic acid**

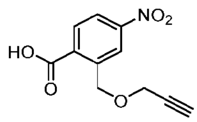

To the mixture of 2-(bromomethyl)-4-nitrobenzoic acid (250 g, 0.9122 mol) in ACN (5000 mL) was added prop-2-yn-1-ol (255.68 g, 265.50 mL, 4.5609 mol, d=0.963 g/mL) and $Cs_2CO_3$ (743.03 g, 2.2805 mol) at room temperature. The resulting mixture was heated to 80°C for 16 h. The reaction mixture was filtered through celite pad washed with ethyl acetate (2 L). The filtrate was concentrated under reduced pressure. The obtained crude compound was added sat. $NaHCO_3$ solution (1 L) and the aq layer was acidified to PH 2 by using 2N HCl (2 L). After filteration vacuum drying 4-nitro-2-((prop-2-yn-1-yloxy)methyl)benzoic acid was obtained. $^1H$ NMR (400 MHz, DMSO): δ 13.61 (brs, 1H), 8.37 (d, J=2.4 Hz, 1H), 8.23 (dd, J=2.4, 8.4 Hz, 1H), 8.10 (d, J=8.8 Hz, 1H), 4.95 (s, 2H), 4.37 (d, J=2.4 Hz, 2H), 3.52 (t, J=2.4 Hz, 1H)

**Synthesis of methyl 4-nitro-2-((prop-2-yn-1-yloxy)methyl)benzoate**

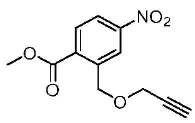

To a stirred solution of 4-nitro-2-((prop-2-yn-1-yloxy)methyl)benzoic acid (130 g, 0.5527 mol) in MeOH (1300 mL) was added $SOCl_2$ (526.08 g, 320.78 mL, 4.4219 mol, d=1.64 g/mL) slowly at 0°C. The reaction stirred at 70°C for 4 h. The

reaction solvent was evaporated under reduced pressure. The obtained residue was dissolved in ethyl acetate (1000 mL) and washed with sat. $NaHCO_3$ (600 mL), water (500 mL) and brine solution (500 mL). The separated organic layer was dried over sodium sulphate, filtered and evaporated under reduced pressure to yield methyl 4-nitro-2-((prop-2-yn-1-yloxy)methyl)benzoate. $^1H$ NMR (400 MHz, $CDCl_3$): δ 8.56 (t, J=0.8 Hz, 1H), 8.18 - 8.09 (m, 2H), 5.03 (s, 2H), 4.35 (d, J=2.4 Hz, 2H), 3.96 (s, 3H), 2.49 (t, J=2.4 Hz, 1H).

**Synthesis of methyl 4-amino-2-((prop-2-yn-1-yloxy)methyl)benzoate**

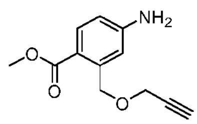

To a solution of methyl 4-nitro-2-((prop-2-yn-1-yloxy)methyl)benzoate (110 g, 0.4414 mol) in a mixture of EtOH (1100 mL) and $H_2O$ (550 mL) was added Fe Powder (197.21 g, 3.5310 mol) and $NH_4Cl$ (188.88 g, 3.5310 mol) at room temperature. The resulting mixture was heated at 80°C for 16 h. The reaction mixture was cooled to room temperature and filtered through celite® and washed with ethyl acetate (2 L). The filtrate was concentrated under reduced pressure up to half of the volume. To the residue, ethyl acetate (1.5 L) was added and separated the two layers and the aqueous layer was extracted with ethyl acetate (2 L). The combined organic layer was dried over anhydrous sodium sulphate and concentrated under reduced pressure to obtain crude product. Purification by $SiO_2$ column chromatography (15-20% of ethyl acetate in petroleum-ether) yielded methyl 4-amino-2-((prop-2-yn-1-yloxy)methyl)benzoate. $^1H$ NMR (400 MHz, $CDCl_3$): δ 7.67 (d, J=8.8 Hz, 1H), 6.78 (t, J=1.6 Hz, 1H), 6.48 (q, J=8.4, 2.4 Hz, 1H), 4.79 (s, 2H), 4.25 (d, J=2.4 Hz, 2H), 3.70 (d, J=4.0 Hz, 3H), 3.42 (t, J=2.4 Hz, 1H).

**Synthesis of (4-amino-2-((prop-2-yn-1-yloxy)methyl)phenyl)methanol**

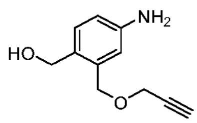

To a stirred solution of THF (1000 mL) was added LiAlH4 (1 M in THF) (21.23 g, 798.2 mmol, 798.2 mL) slowly at 0°C. A solution of methyl 4-amino-2-((prop-2-yn-1-yloxy)methyl)benzoate (70 g, 319.3 mmol) in THF (800 mL) was added slowly at 0°C. The reaction was stirred at room temperature for 4 h. The reaction mixture was cooled to 0°C, then was added water (22 mL) very slowly and followed by the addition of 20% NaOH (22 mL) and water (66 mL). The reaction mixture was stirred at 0°C for 30 min. Anhydrous sodium sulphate was added to absorb excess of water. The mixture was filtered through celite®. The filter cake was washed with ethylacetate (1000 mL) and 10% MeOH/DCM (500 mL). The filtrate was concentrated under reduced pressure. The resulting crude compound was purified by $SiO_2$ column chromatography (35-40% of ethylacetate in petroleum-ether as an eluent) to give yield (4-amino-2-((prop-2-yn-1-yloxy)methyl)phenyl)methanol. $^1H$ NMR (400 MHz, $CDCl_3$): δ 6.98 (d, J=8.0 Hz, 1H), 6.56 (d, J=2.4 Hz, 1H), 6.43 (dd, J=2.4, 8.0 Hz, 1H), 4.98 (s, 2H), 4.64 (t, J=5.2 Hz, 1H), 4.47 (s, 2H), 4.34 (d, J=5.6 Hz, 2H), 4.15 (d, J=2.4 Hz, 2H), 3.46 (t, J=2.4 Hz, 1H).

**Synthesis of (9H-fluoren-9-yl)methyl (S)-(1-((4-(hydroxymethyl)-3-((prop-2-yn-1-yloxy)methyl)phenyl)amino)-1-oxo-5-ureidopentan-2-yl)carbamate**

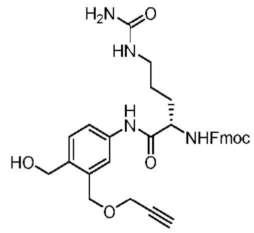

To a solution of (4-amino-2-((prop-2-yn-1-yloxy)methyl)phenyl)methanol (1.92 g, 10.04 mmoles, 1.0 equiv.), (9H-fluoren-9-yl)methyl (S)-(1-amino-1-oxo-5-ureidopentan-2-yl)carbamate (3.99 g, 10.04 mmoles, 1.0 equiv.), and (1-[bis(dimethylamino)methylene]-1H-1,2,3-triazolo[4,5-b]pyridinium 3-oxid hexafluorophosphate (4.20 g, 11.04 mmoles, 1.1 equiv.) in DMF (10 mL) was added N,N-diisopropylethylamine (2.62 mL, 15.06 mmoles, 1.5 equiv.). After stirring at ambient temperature for 1 h, the mixture was poured into water (200 mL). The resulting solids were filtered, rinsed with water, and dried under vacuum, and (9H-fluoren-9-yl)methyl (S)-(1-((4-(hydroxymethyl)-3-((prop-2-yn-1-yloxy)methyl)phenyl)amino)-1-oxo-5-ureidopentan-2-yl)carbamate was obtained . LCMS: MH+=571.5; Rt=0.93 min (2 min acidic method).

**Synthesis of (S)-2-amino-N-(4-(hydroxymethyl)-3-((prop-2-yn-1-yloxy)methyl)phenyl)-5-ureidopentanamide**

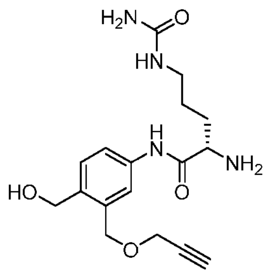

To (9H-fluoren-9-yl)methyl (S)-(1-((4-(hydroxymethyl)-3-((prop-2-yn-1-yloxy)methyl)phenyl)amino)-1-oxo-5-ureidopentan-2-yl)carbamate (6.08 g, 10.65 mmoles, 1.0 equiv.) was added dimethylamine (2 M in THF, 21.31 mL, 42.62 mmoles, 4 equiv.). After stirring at ambient temperature for 1.5 hours, the supernatant solution was decanted from the gum-like residue that had formed. The residue was triturated with ether (3 x 50 mL) and the resulting solids were filtered, washed with ether, and dried under vacuum. (S)-2-amino-N-(4-(hydroxymethyl)-3-((prop-2-yn-1-yloxy)methyl)phenyl)-5-ureidopentanamide was obtained. LCMS: MH+ 349.3; Rt=0.42 min (2 min acidic method).

**Synthesis of tert-butyl ((S)-1-(((S)-1-((4-(hydroxymethyl)-3-((prop-2-yn-1-yloxy)methyl)phenyl)amino)-1-oxo-5-ureidopentan-2-yl)amino)-3-methyl-1-oxobutan-2-yl)carbamate**

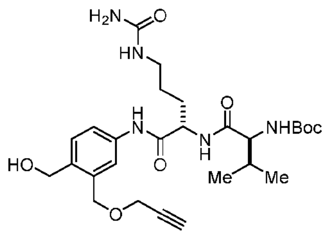

To a solution of (S)-2-amino-N-(4-(hydroxymethyl)-3-((prop-2-yn-1-yloxy)methyl)phenyl)-5-ureidopentanamide (3.50 g, 10.04 mmoles, 1.0 equiv.), (tert-butoxycarbonyl)-L-valine (2.62 g, 12.05 mmol, 1.2 equiv.), and (1-[bis(dimethylamino)methylene]-1H-1,2,3-triazolo[4,5-b]pyridinium 3-oxid hexafluorophosphate (4.58 g, 12.05 mmoles, 1.2 equiv.) in DMF (10 mL) was added N,N-diisopropylethylamine (3.50 mL, 20.08 mmoles, 2.0 equiv). After stirring at ambient temperature for 2 h, the mixture was poured into water (200 mL) and the resulting suspension was extracted with EtOAc (3x100 mL). The combined organic layers were dried over sodium sulfate and concentrated under vacuum. After

purification by ISCO $SiO_2$ chromatography (0-20% methanol / dichloromethane), tert-butyl ((S)-1-(((S)-1-((4-(hydroxymethyl)-3-((prop-2-yn-1-yloxy)methyl)phenyl)amino)-1-oxo-5-ureidopentan-2-yl)amino)-3-methyl-1-oxobutan-2-yl) carbamate was obtained. $^1H$ NMR (400 MHz, DMSO-d6) δ 10.00 (s, 1H), 7.96 (d, J = 7.7 Hz, 1H), 7.55 (dq, J = 4.9, 2.2 Hz, 2H, aryl), 7.32 (d, J = 8.9 Hz, 1H, aryl), 6.76 (d, J = 8.9 Hz, 1H), 5.95 (t, J = 5.8 Hz, 1H), 5.38 (s, 2H), 5.01 (t, J = 5.5 Hz, 1H), 4.54 (s, 2H), 4.45 (dd, J = 25.2, 5.3 Hz, 3H), 4.20 (d, J = 2.4 Hz, 2H), 3.83 (dd, J = 8.9, 6.7 Hz, 1H), 3.49 (t, J = 2.4 Hz, 1H), 2.97 (dh, J = 26.0, 6.5 Hz, 2H), 1.96 (h, J = 6.6 Hz, 1H), 1.74 - 1.50 (m, 2H), 1.39 (m, 11H), 0.84 (dd, J = 16.2, 6.7 Hz, 6H). LCMS: Mna+ 570.5; Rt=0.79 min (2 min acidic method).

**Synthesis of tert-butyl ((S)-1-(((S)-1-((4-(chloromethyl)-3-((prop-2-yn-1-yloxy)methyl)phenyl)amino)-1-oxo-5-ureidopentan-2-yl)amino)-3-methyl-1-oxobutan-2-yl)carbamate**

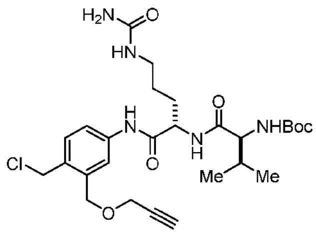

To a solution of tert-butyl ((S)-1-(((S)-1-((4-(hydroxymethyl)-3-((prop-2-yn-1-yloxy)methyl)phenyl)amino)-1-oxo-5-ureidopentan-2-yl)amino)-3-methyl-1-oxobutan-2-yl)carbamate (2.00 grams, 3.65 mmol, 1.0 equiv.) in acetonitrile (13.3 mL) at 0 °C was added thionyl chloride (0.53 mL, 7.30 mmol, 2.0 equiv). After stirring in the ice bath for one hour the solution was diluted with water (40 mL) and the resulting white precipitate was collected by filtration, air drying and drying under high vacuum to yield tert-butyl ((S)-1-(((S)-1-((4-(chloromethyl)-3-((prop-2-yn-1-yloxy)methyl)phenyl)amino)-1-oxo-5-ureidopentan-2-yl)amino)-3-methyl-1-oxobutan-2-yl)carbamate. LCMS: Mna+ 588.5; Rt=2.17 min (5 min acidic method).

**Synthesis of 2-(((tert-butyldiphenylsilyl)oxy)methyl)-5-nitrobenzoic acid**

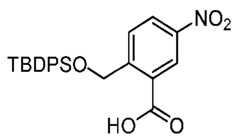

To a solution of 6-nitroisobenzofuran-1 (3H)-one (90 g, 502.43 mmol, 1.00 eq) in MeOH (1000 mL) and KOH (28.19 g, 502.43 mmol, 1.00 eq) in $H_2O$ (150 mL) was added. The brown mixture was stirred at 25°C for 1.5 h. The brown mixture was concentrated under reduced pressure to give a residue and dissolved in DCM (2000 mL). The mixture was added TBDPSCl (296.91 g, 1.08 mol, 277.49 mL, 2.15 eq) and IMIDAZOLE (171.03 g, 2.51 mol, 5.00 eq) stirred at 25°C for 12 h. The mixture was concentrated under reduced pressure to give a residue. The residue was purified by silica gel chromatography (Petroleum ether/Ethyl acetate=1/0, 1/1) and 2-(((tert-butyldiphenylsilyl)oxy)methyl)-5-nitrobenzoic acid was obtained as a white solid. $^1H$ NMR (400 MHz, METHANOL-d4) δ ppm 1.13 (s, 9 H) 5.26 (s, 2 H) 7.34 - 7.48 (m, 6 H) 7.68 (br d, J=8 Hz, 4 H) 8.24 (br d, J=8 Hz, 1 H) 8.46 (br d, J=8 Hz, 1 H) 8.74 (s, 1 H).

**Synthesis of (2-(((tert-butyldiphenylsilyl)oxy)methyl)-5-nitrophenyl)methanol**

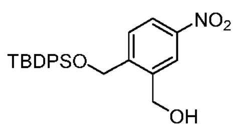

To a mixture of 2-(((tert-butyldiphenylsilyl)oxy)methyl)-5-nitrobenzoic acid (41 g, 94.14 mmol, 1 eq) in THF (205

mL) was added $BH_3$. THF (1 M, 470.68 mL, 5 eq). The yellow mixture was stirred at 60°C for 2h. The mixture was added MeOH (400mL), and concentrated under reduced pressure to give a residue. Then addition of $H_2O$ (200mL) and DCM(300mL), extracted with DCM (3 ×200 mL), washed with brine (300mL), dried over anhydrous $MgSO_4$, filtered, and concentrated under reduced pressure to give a residue. The residue was purified by silica gel chromatography (Petroleum ether/Ethyl acetate=1/0, 1/1). (2-(((tert-butyldiphenylsilyl)oxy)methyl)-5-nitrophenyl)methanol was obtained as a white solid. $^1H$ NMR (400 MHz, METHANOL-d4) δ ppm 1.10 (s, 9 H) 4.58 (s, 2 H) 4.89 (s, 2 H) 7.32 - 7.51 (m, 6 H) 7.68 (dd, J=8, 1.38 Hz, 4 H) 7.76 (d, J=8 Hz, 1 H) 8.15 (dd, J=8 2.26 Hz, 1 H) 8.30 (d, J=2 Hz, 1 H).

**Synthesis of 2-(((tert-butyldiphenylsilyl)oxy)methyl)-5-nitrobenzaldehyde**

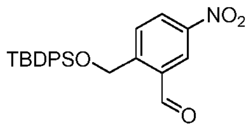

To a solution of (2-(((tert-butyldiphenylsilyl)oxy)methyl)-5-nitrophenyl)methanol (34 g, 80.65 mmol, 1 eq) in DCM (450 mL) was added $MnO_2$ (56.09 g, 645.22 mmol, 8 eq). The black mixture was stirred at 25°C for 36 h. The mixture was added MeOH (400mL), and concentrated under reduced pressure to give a residue. Then addition of $H_2O$ (200mL) and DCM (300mL), extracted with DCM (3 ×200 mL), washed with brine (300mL), dried over anhydrous $MgSO_4$, filtered, and concentrated under reduced pressure to give a residue. The residue was purified by silica gel chromatography ($CH_2Cl_2$=100%). 2-(((tert-butyldiphenylsilyl)oxy)methyl)-5-nitrobenzaldehyde was obtained as a white solid. $^1H$ NMR (400 MHz, CHLOROFORM-d) δ ppm 1.14 (s, 9 H) 5.26 (s, 2 H) 7.34 - 7.53 (m, 6 H) 7.60 - 7.73 (m, 4 H) 8.13 (d, J=8Hz, 1 H) 8.48 (dd, J=8, 2.51 Hz, 1 H) 8.67 (d, J=2 Hz, 1 H) 10.16 (s, 1 H).

**Synthesis of N-(2-(((tert-butyldiphenylsilyl)oxy)methyl)-5-nitrobenzyl)prop-2-yn-1-amine**

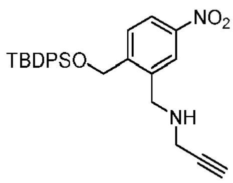

To a solution of 2-(((tert-butyldiphenylsilyl)oxy)methyl)-5-nitrobenzaldehyde (12.6 g, 30.03 mmol, 1 eq) in DCM (130 mL) was added prop-2-yn-1-amine (4.14 g, 75.08 mmol, 4.81 mL, 2.5 eq) and $MgSO_4$ (36.15 g, 300.33 mmol, 10 eq) then the suspension mixture was stirred at 25°C for 24hr. Taking a little reaction solution and treating with $NaBH_4$, the TLC showed one new point was formed. The reaction mixture was filtered and concentrated under reduced pressure to give a residue. (E)-N-[[2-[[tert-butyl(diphenyl)silyl]oxymethyl]-5-nitro-phenyl]methyl]prop-2-yn-1-imine was obtained as a yellow solid. $^1H$ NMR (400 MHz, CHLOROFORM-d) δ ppm 1.11 (s, 9 H) 2.48 (t, J=2.38 Hz, 1 H) 4.52 (t, J=2.13 Hz, 2 H) 5.09 (s, 2 H) 7.35 - 7.49 (m, 6 H) 7.63 - 7.72 (m, 4 H) 7.79 (d, J=8.53 Hz, 1 H) 8.25 (dd, J=8.53, 2.51 Hz, 1 H) 8.68 (d, J=2.26 Hz, 1 H) 8.84 (t, J=1.88 Hz, 1 H).

(E)-N-[[2-[[tert-butyl(diphenyl)silyl]oxymethyl]-5-nitro-phenyl]methyl]prop-2-yn-1-imine (12 g, 26.28 mmol, 1 eq) was dissolved in MeOH (100 mL) and THF (50 mL), then $NaBH_4$ (1.49 g, 39.42 mmol, 1.5 eq) was added and the yellow mixture was stirred at -20°C for 2hr. LCMS showed desired compound was detected. The reaction mixture was quenched by addition MeOH (200 mL) at -20 °C, and then concentrated under reduced pressure to give a residue. The residue was dissolved with EtOAc (500 mL) washed with brine (150 mL), dried over anhydrous $Na_2SO_4$, filtered and concentrated under reduced pressure to give a residue. The residue was purified by flash silica gel chromatography ( Eluent of 0-10% Ethyl acetate/Petroleum ether gradient). N-(2-(((tert-butyldiphenylsilyl)oxy)methyl)-5-nitrobenzyl)prop-2-yn-1-amine was obtained as a pale yellow oil. $^1H$ NMR (400 MHz, CHLOROFORM-d) δ ppm 1.12 (s, 9 H) 2.13 (t, J=2.38 Hz, 1 H) 3.33 (d, J=2.51 Hz, 2 H) 3.80 (s, 2 H) 4.93 (s, 2 H) 7.36 - 7.49 (m, 6 H) 7.69 (dd, J=7.91, 1.38 Hz, 4 H) 7.77 (d, J=8.53 Hz, 1 H) 8.16 (dd, J=8.41, 2.38 Hz, 1 H) 8.24 (d, J=2.26 Hz, 1 H).

**Synthesis of (9H-fluoren-9-yl)methyl (2-(((tert-butyldiphenylsilyl)oxy)methyl)-5-nitrobenzyl)(prop-2-yn-1-yl) carbamate**

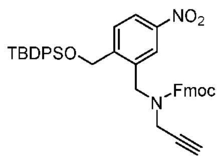

To a solution of N-(2-(((tert-butyldiphenylsilyl)oxy)methyl)-5-nitrobenzyl)prop-2-yn-1-amine (9 g, 19.62 mmol, 1 eq) and FMOC-OSU (7.28 g, 21.59 mmol, 1.1 eq) in dioxane (90 mL) was added sat. $NaHCO_3$ (90 mL) and the white suspension was stirred at 20°C for 12 h. The reaction mixture was diluted with $H_2O$ (150 mL) and extracted with EtOAc (150 mL x 2). The combined organic layers were washed with brine (200 mL), dried over anhydrous $Na_2SO_4$, filtered and concentrated under reduced pressure to give a residue. The residue was purified by flash silica gel chromatography (Eluent of 0-30% Ethyl acetate/Petroleum ether). (9H-fluoren-9-yl)methyl (2-(((tert-butyldiphenylsilyl)oxy)methyl)-5-nitrobenzyl)(prop-2-yn-1-yl)carbamate (7.7 g, 11.08 mmol, 56.48% yield, 98% purity) was obtained as a white solid. $^1H$ NMR (400 MHz, CHLOROFORM-d) δ ppm 1.12 (s, 9 H) 2.17 (br d, J=14.31 Hz, 1 H) 3.87 - 4.97 (m, 9 H) 6.98 - 8.28 (m, 21 H).

**Synthesis of (9H-fluoren-9-yl)methyl (5-amino-2-(((tert-butyldiphenylsilyl)oxy)methyl)benzyl)(prop-2-yn-1-yl) carbamate**

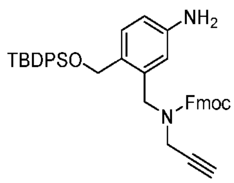

To an ice bath cooled solution of (9H-fluoren-9-yl)methyl (2-(((tert-butyldiphenylsilyl)oxy)methyl)-5-nitrobenzyl) (prop-2-yn-1-yl)carbamate (5.0 g, 7.34 mmoles, 1.0 equiv.) in 10% $AcOH/CH_2Cl_2$ (100 mL) was added Zn (7.20 g, 110 mmoles, 15 equiv.). The ice bath was removed, and the resulting mixture stirred for 2 hours at which time it was filtered through a pad of celite®. The volatiles were removed in vacuo and the residue was dissolved in EtOAc, was washed with $NaHCO_3$(sat.), NaCl(sat.), dried over $MgSO_4$, filtered, concentrated and after ISCO $SiO_2$ chromatography (0-75% EtOAc/Heptanes) (9H-fluoren-9-yl)methyl (5-amino-2-(((tert-butyldiphenylsilyl)oxy)methyl)benzyl)(prop-2-yn-1-yl)carbamate was obtained. LCMS: MH+=651.6; Rt=3.77 min (5 min acidic method).

**Synthesis of (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate**

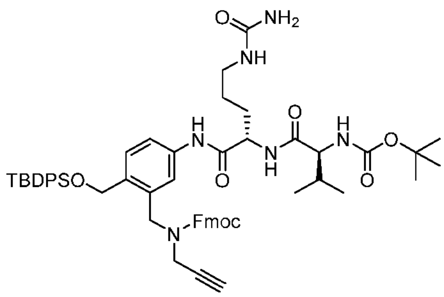

To (9H-fluoren-9-yl)methyl (5-amino-2-(((tert-butyldiphenylsilyl)oxy)methyl)benzyl)(prop-2-yn-1-yl)carbamate

(2.99 g, 4.59 mmoles, 1.0 equiv) and (S)-2-((S)-2-((tert-butoxycarbonyl)amino)-3-methylbutanamido)-5-ureidopentanoic acid (1.72 g, 4.59 mmoles, 1.0 equiv.) in $CH_2Cl_2$ (40 mL) was added ethyl 2-ethoxyquinoline-1(2H)-carboxylate (2.27 g, 9.18 mmoles, 2.0 equiv.). After stirring for 10 min, MeOH (1 mL) was added and the solution became homogeneous. The reaction was stirred for 16 h, the volatiles were removed in vacuo and after purification by ISCO $SiO_2$ chromatography (0-15% MeOH/CH2Cl2) (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate was obtained. LCMS: MH+=1008.8; Rt=3.77 min (5 min acidic method).

**Synthesis of prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate**

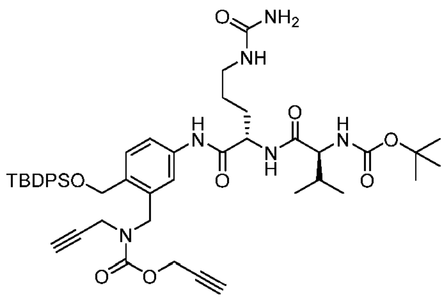

To (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate (1.60 g, 1.588 mmoles, 1.0 equiv.) was added 2M dimethylamine in MeOH (30 mL, 60 mmol, 37 equiv.) and THF (10 mL). After standing for 3 h, the volatiles were removed in vacuo and the residue was triturated with $Et_2O$ to remove FMOC deprotection byproducts. To the resulting solid was added $CH_2Cl_2$ (16 mL) and pyridine (4 mL) and to the heterogeneous solution was added propargyl chloroformate (155 uL, 1.588 mmole, 1.0 equiv.). After stirring for 30 minutes additional propargyl chloroformate (155 uL, 1.588 mmole, 1.0 equiv.) was added. After stirring for an additional 20 min, MeOH (1 mL) was added to quench remaining chloroformate and the volatiles were removed in vacuo. Upon purification by ISCO $SiO_2$ chromatography (0-15% MeOH/$CH_2Cl_2$) prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate was obtained. LCMS: MH+=867.8; Rt=3.40 min (5 min acidic method).

**Synthesis of prop-2-yn-1-yl (5-((S)-2-((S)-2-((tert-butoxycarbonyl)amino)-3-methylbutanamido)-5-ureidopentanamido)-2-(hydroxymethyl)benzyl)(prop-2-yn-1-yl)carbamate**

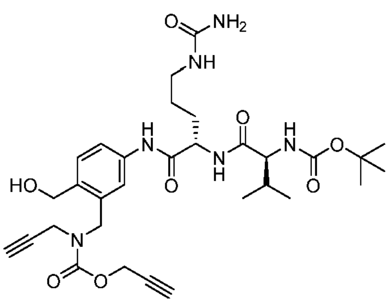

To a solution of prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate (984 mg, 1.135 mmoles, 1.0 equiv.) in THF (7.5 mL) was added 1.0 M tetrabutylammoniumn fluoride in THF (2.27 mL, 2.27 mmoles, 2.0 equiv.). After standing for 6 h, the volatiles were removed in vacuo, the residue was purified by ISCO $SiO_2$ chromatography (0-40% MeOH/CH2Cl2) and prop-2-yn-1-yl (5-((S)-2-((S)-2-((tert-butoxycarbonyl)amino)-3-methylbutanamido)-5-ureidopentanamido)-2-(hydroxymethyl)benzyl) (prop-2-yn-1-yl)carbamate was obtained. LCMS: MH+=629.6; Rt=1.74min (5 min acidic method).

**Synthesis of prop-2-yn-1-yl (5-((S)-2-((S)-2-((tert-butoxycarbonyl)amino)-3-methylbutanamido)-5-ureidopentanamido)-2-(chloromethyl)benzyl)(prop-2-yn-1-yl)carbamate**

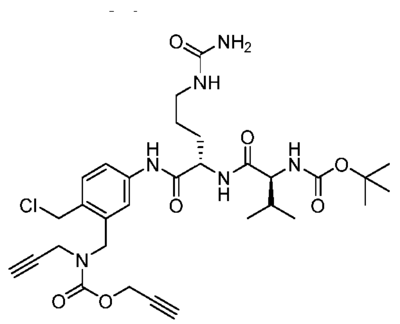

To prop-2-yn-1-yl (5-((S)-2-((S)-2-((tert-butoxycarbonyl)amino)-3-methylbutanamido)-5-ureidopentanamido)-2-(hydroxymethyl)benzyl)(prop-2-yn-1-yl)carbamate (205 mg, 0.326 mmoles, 1.0 equiv.) in $CH_2Cl_2$ (10 mL) was added pyridine (158 uL, 1.96 mmoles, 5 equiv.). The heterogeneous mixture was cooled in a 0 °C ice bath and thionyl chloride (71 uL, 0.98 mmoles, 3 equiv.). After stirring in the ice bath for 3 hours the reaction was directly purified by ISCO $SiO_2$ chromatography (0-30% MeOH/$CH_2Cl_2$) and prop-2-yn-1-yl (5-((S)-2-((S)-2-((tert-butoxycarbonyl)amino)-3-methylbutanamido)-5-ureidopentanamido)-2-(chloromethyl)benzyl)(prop-2-yn-1-yl)carbamate was obtained LCMS: MH+=647.6; Rt=2.54 min (5 min acidic method).

**Synthesis of 2-(hydroxymethyl)-N-methyl-5-nitrobenzamide**

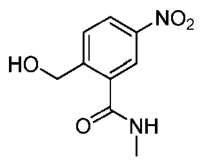

To a stirred suspension of 6-nitroisobenzofuran-1(3H)-one (500 g, 2.79 mol) in MeOH (1500 mL) was added $MeNH_2$ (3.00 kg, 29.94 mol, 600 mL, 31.0% purity) at 25 °C and stirred for 1 h. The solid was filtered and washed with water twice (600 mL) and dried under high vacuum to get a residue. The product 2-(hydroxymethyl)-N-methyl-5-nitrobenzamide was obtained as white solid. LCMS: Rt = 0.537 min, MS m/z = 193.2. $^1$H NMR: 400 MHz DMSO $\delta$ 8.57 (br d, J = 4.4 Hz, 1H), 8.31 (dd, J = 2.4, 8.6 Hz, 1H), 8.21 (d, J = 2.4 Hz, 1H), 7.86 (d, J = 8.8 Hz, 1H), 5.54 (t, J = 5.6 Hz, 1H), 4.72 (d, J = 5.5 Hz, 2H), 2.78 (d, J = 4.4 Hz, 3H).

**Synthesis of (2-((methylamino)methyl)-4-nitrophenyl)methanol**

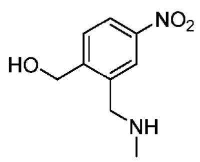

To a solution of 2-(hydroxymethyl)-N-methyl-5-nitrobenzamide (560 g, 2.66 mol) in THF (5000 mL) was cooled to 0 °C, then added BH3-Me2S (506 g, 6.66 mol) (2.0 M in THF) drop wise for 60 min and heated to 70 °C for 5 h. LCMS showed the starting material was consumed. After completion, 4M HCl (1200 mL) in Methanol was added to reaction mixture at 0 °C and heated at 65 °C for 8 h. The reaction mixture was cooled to 0 °C, the solid was filtered and concentrated in reduce pressure. The product (2-((methylamino)methyl)-4-nitrophenyl)methanol (520 g) was obtained as a white solid. LCMS: Rt = 0.742 min, MS m/z = 197.1 [M+H]+. $^1$H NMR: 400 MHz DMSO $\delta$ 9.25 (br s, 2H), 8.37 (d, J = 2.4 Hz, 1H), 8.14 (dd, J = 2.4, 8.5 Hz, 1H), 7.63 (d, J = 8.4 Hz, 1H), 5.72 (br s, 1H), 4.65 (s, 2H), 4.15 (br s, 2H), 2.55 - 2.45 (m, 3H)

**Synthesis of 1-(2-(((tert-butyldiphenylsilyl)oxy)methyl)-5-nitrophenyl)-N-methylmethanamine**

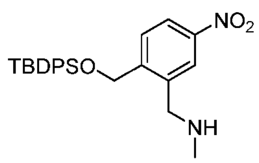

To a solution of (2-((methylamino)methyl)-4-nitrophenyl)methanol (520 g, 2.65 mol) and imidazole (721 g, 10.6 mol) in DCM (2600 mL) was cooled to 0°C was added TBDPS-CL (1.09 kg, 3.98 mol, 1.02 L) drop wise and stirred for 2 h. The mixture was poured in ice cold water (1000 mL) and extracted with ethyl acetate. The combined organic layers were washed with brine, dried over $Na_2SO_4$, filtered and evaporated under vacuum to give crude product. The crude product was purified by chromatography on a silica gel eluted with Ethyl acetate: Petroleum ether (from 10/1 to 1) to give a residue. The product 1-(2-(((tert-butyldiphenylsilyl)oxy)methyl)-5-nitrophenyl)-N-methylmethanamine was obtained as yellow liquid. LCMS: product: Rt = 0.910 min, MS m/z = 435.2 [M+H]+. $^1$H NMR: 400 MHz CDCl3 δ8.23 (d, J=2.4 Hz, 1H), 8.15 (dd, J=2.4, 8.4 Hz, 1H), 7.76 (d, J=8.4 Hz, 1H), 7.71 - 7.66 (m, 4H), 7.50 - 7.37 (m, 6H), 4.88 (s, 2H), 3.65 (s, 2H), 2.39 (s, 3H), 1.12 (s, 9H)

**Synthesis of (9H-fluoren-9-yl)methyl (2-(((tert-butyldiphenylsilyl)oxy)methyl)-5-nitrobenzyl)(methyl)carbamate**

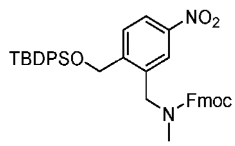

To a solution of 1-(2-(((tert-butyldiphenylsilyl)oxy)methyl)-5-nitrophenyl)-N-methylmethanamine (400 g, 920.3 mmol) in THF (4000 mL) was added FMOC-OSU (341.5 g, 1.01 mol) and $Et_3N$ (186.2 g, 1.84 mol, 256.2 mL), the mixture was stirred at 25 °C for 1 h. The mixture was poured into water (1600 mL) and extracted with ethyl acetate (1000 mL x 2). The combined organic layers were washed with brine, dried over $Na_2SO_4$, filtered and evaporated under vacuum to give crude product. The crude product was purified by chromatography on a silica gel eluted with petroleum ether: ethyl acetate (from 1/0 to 1/1) to give (9H-fluoren-9-yl)methyl (2-(((tert-butyldiphenylsilyl)oxy)methyl)-5-nitrobenzyl)(methyl)carbamate as white solid. LCMS: Rt = 0.931 min, MS m/z = 657.2 [M+H]+. $^1$H NMR: EW16000-26-P1A, 400 MHz CDCl3 δ 8.21 - 7.96 (m, 1H), 7.87 - 7.68 (m, 3H), 7.68 - 7.62 (m, 4H), 7.62 - 7.47 (m, 2H), 7.47 - 7.28 (m, 9H), 7.26 - 7.05 (m, 2H), 4.81 (br s, 1H), 4.62 - 4.37 (m, 4H), 4.31 - 4.19 (m, 1H), 4.08 - 3.95 (m, 1H), 2.87 (br d, J = 5.2 Hz, 3H), 1.12 (s, 9H).

**Synthesis of (9H-fluoren-9-yl)methyl (5-amino-2-(((tert-butyldiphenylsilyl)oxy)methyl)benzyl)(methyl)carbamate**

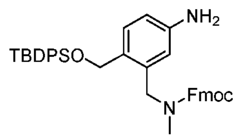

A solution of (9H-fluoren-9-yl)methyl (2-(((tert-butyldiphenylsilyl)oxy)methyl)-5-nitrobenzyl)(methyl)carbamate (3.0 g, 4.57 mmole, 1.0 equiv.) in MeOH (90 mL) and EtOAc (30 mL) was degassed and purged to a balloon of $N_2$ via three way stopcock. After repeating degas/$N_2$ purge 2x, 10% Pd/C deGussa type (0.486 g, 0.457 mmoles, 0.1 equiv.) was added. The resulting mixture was degassed and purged to a balloon of 2 $H_2$ via three-way stopcock. After repeating degas/$H_2$ purge 2x, the reaction stirred under the balloon pressure of $H_2$ for 4 hours. The reaction was degassed and purged to $N_2$, filtered through a pad of celite eluting further with MeOH. After removal of the volatiles in vacuo and pumping on high vac (9H-fluoren-9-yl)methyl (5-amino-2-(((tert-butyldiphenylsilyl)oxy)methyl)benzyl)(methyl)carbamate was obtained. LCMS: MH+=627.7; Rt=1.59 min (2 min acidic method).

**Synthesis of (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate**

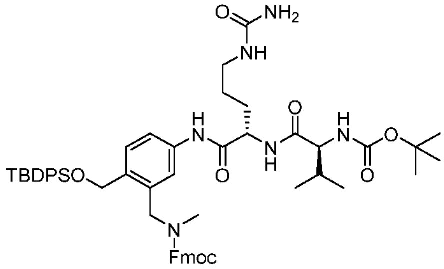

To (9H-fluoren-9-yl)methyl (5-amino-2-(((tert-butyldiphenylsilyl)oxy)methyl)benzyl)(methyl)carbamate (2.86 g, 4.56 mmoles, 1.0 equiv) and (S)-2-((S)-2-((tert-butoxycarbonyl)amino)-3-methylbutanamido)-5-ureidopentanoic acid (1.71 g, 4.56 mmoles, 1.0 equiv.) in 2:1 $CH_2Cl_2$/MeOH (60 mL) was added ethyl 2-ethoxyquinoline-1(2H)-carboxylate (2.256 g, 9.12 mmoles, 2.0 equiv.). The homogeneous solution was stirred for 16 hours at which time additional (S)-2-((S)-2-((tert-butoxycarbonyl)amino)-3-methylbutanamido)-5-ureidopentanoic acid (0.340 g, 0.2 equiv.) and ethyl 2-ethoxyquinoline-1 (2H)-carboxylate (0.452 g, 0.4 equiv.) were addd to drive the reaction to completion. After stirring for an adiditonal 5 hours the volatiles were removed in vacuo and after purification by ISCO $SiO_2$ chromatography (0-5% MeOH/$CH_2Cl_2$) (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate was obtained. LCMS: MH+=984.1; Rt=1.54 min (2 min acidic method).

**Synthesis of prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate**

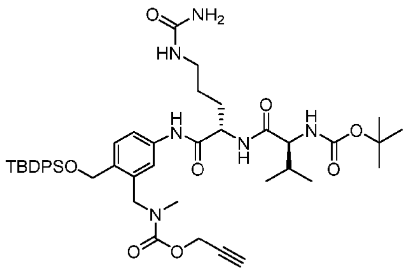

To (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate (2.05 g, 2.085 mmol, 1.0 equiv) in THF (10 mL) was added 2.0 M dimethyl amine in MeOH (10.42 mL, 20.85 mmol, 10 equiv.). After stirring for 16 hours the volatiles were removed in vacuo. The residue was dissolved in $CH_2Cl_2$ (20 mL) and DIEA (0.533 mL, 4.17 mmol, 2 equiv.) and propargyl chloroformate (0.264 mL, 2.71 mmol, 1.3 equiv.) were added. After stirring at rt for 16 hours the reaction was diluted with $CH_2Cl_2$ (20 mL), was washed with $NaHCO_3$ (sat.), NaCl(sat.), dried over $MgSO_4$, filtered, concentrated and purified by ISCO $SiO_2$ chromatography (0-15% MeOH/$CH_2Cl_2$) to yield prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate . LCMS: MH+=843.8; Rt=1.35 min (2 min acidic method).

**Synthesis of prop-2-yn-1-yl (5-((S)-2-((S)-2-((tert-butoxycarbonyl)amino)-3-methylbutanamido)-5-ureidopentanamido)-2-(hydroxymethyl)benzyl)(methyl)carbamate**

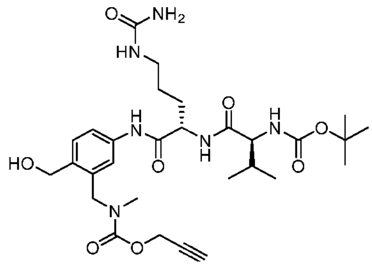

To a 0 °C solution of prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate (1.6 g, 1.90 mmoles, 1.0 equiv.) in THF (10.0 mL) was added 1.0 M tetrabutylammoniumn fluoride in THF (3.80 mL, 3.80 mmoles, 2.0 equiv.). After warming to room temperature and stirring for 16 h the volatiles were removed in vacuo, the residue was dissolved in EtOAc, was washed with $NaHC0_3$(sat.), with NaCl(sat.), dried over $MgSO_4$, filtered, concentrated and the residue was purified by ISCO $SiO_2$ chromatography (0-30% MeOH/CH2Cl2) to yield prop-2-yn-1-yl (5-((S)-2-((S)-2-((tert-butoxycarbonyl)amino)-3-methylbutanamido)-5-ureidopentanamido)-2-(hydroxymethyl)benzyl)(methyl)carbamate. LCMS: MH+=605.7; Rt=0.81 min (2 min acidic method).

**Synthesis of prop-2-yn-1-yl (5-((S)-2-((S)-2-((tert-butoxycarbonyl)amino)-3-methylbutanamido)-5-ureidopentanamido)-2-(chloromethyl)benzyl)(methyl)carbamate**

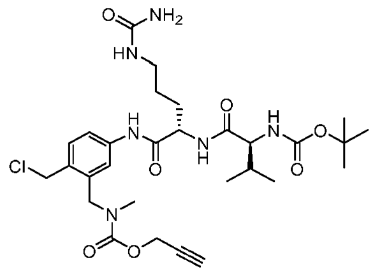

To prop-2-yn-1-yl (5-((S)-2-((S)-2-((tert-butoxycarbonyl)amino)-3-methylbutanamido)-5-ureidopentanamido)-2-(hydroxymethyl)benzyl)(methyl)carbamate (350 mg, 0.579 mmoles, 1.0 equiv.) in $CH_2Cl_2$ (10 mL) was added pyridine (0.278 mL, 3.47 mmoles, 6 equiv.). The heterogeneous mixture was cooled in a 0 °C ice bath and thionyl chloride (0.126 mL, 1.73 mmoles, 3 equiv.). After stirring in the ice bath for 3 h, the reaction was purified by ISCO $SiO_2$ chromatography (0-30% MeOH/$CH_2Cl_2$) and prop-2-yn-1-yl (5-((S)-2-((S)-2-((tert-butoxycarbonyl)amino)-3-methylbutanamido)-5-ureidopentanamido)-2-(chloromethyl)benzyl)(prop-2-yn-1-yl)carbamate was obtained. LCMS: MH+=623.7; Rt=2.19 min (5 min acidic method).

**Synthesis of (9H-fluoren-9-yl)methyl (5-((S)-2-((S)-2-((tert-butoxycarbonyl)amino)-3-methylbutanamido)-5-ureidopentanamido)-2-(hydroxymethyl)benzyl)(methyl)carbamate**

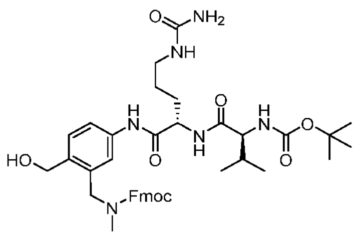

To (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate (2.6 g, 2.64 mmol, 1.0 equiv.) dissolved in THF (20 mL) was added acetic acid (0.757 mL, 13.22 mmol, 5.0 equiv.) and 1.0 M TBAF in THF (2.91 mL, 2.91 mmol, 1.1 equiv.).

The solution was stirred for 72 hours at which time the volatiles were removed in vacuo. After purification by ISCO $SiO_2$ chromatography (0-30% $MeOH/CH_2Cl_2$) (9H-fluoren-9-yl)methyl (5-((S)-2-((S)-2-((tert-butoxycarbonyl)amino)-3-methylbutanamido)-5-ureidopentanamido)-2-(hydroxymethyl)benzyl)(methyl)carbamate was obtained. LCMS: MH+=745.5; Rt=1.07 min (2 min acidic method).

**Synthesis of (9H-fluoren-9-yl)methyl (5-((S)-2-((S)-2-((tert-butoxycarbonyl)amino)-3-methylbutanamido)-5-ureidopentanamido)-2-(chloromethyl)benzyl)(methyl)carbamate**

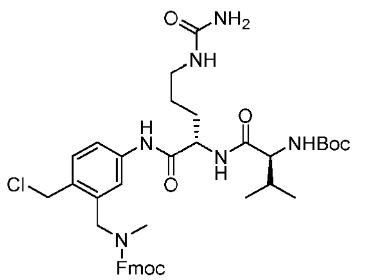

To (9H-fluoren-9-yl)methyl (5-((S)-2-((S)-2-((tert-butoxycarbonyl)amino)-3-methylbutanamido)-5-ureidopentanamido)-2-(hydroxymethyl)benzyl)(methyl)carbamate (200 mg, 0.269 mmoles, 1.0 equiv.) in $CH_2Cl_2$ (10 mL) was added pyridine (0.130 mL, 1.61 mmoles, 6 equiv.). The heterogeneous mixture was cooled in a 0 °C ice bath and thionyl chloride (0.059 mL, 0.806 mmoles, 3 equiv.). After stirring in the ice bath briefly the reaction was stirred as it warmed up to room temperature for 2 hours. The reaction was purified by ISCO $SiO_2$ chromatography (0-30% $MeOH/CH_2Cl_2$) and (9H-fluoren-9-yl)methyl (5-((S)-2-((S)-2-((tert-butoxycarbonyl)amino)-3-methylbutanamido)-5-ureidopentanamido)-2-(chloromethyl)benzyl)(methyl)carbamate was obtained. LCMS: MH+=763.2; Rt=1.18 min (2 min acidic method).

**GENERAL PROCEDURE 1**

**Synthesis of 1-(4-((S)-2-((S)-2-((tert-butoxycarbonyl)amino)-3-methylbutanamido)-5-ureidopentanamido)-2-((prop-2-yn-1-yl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

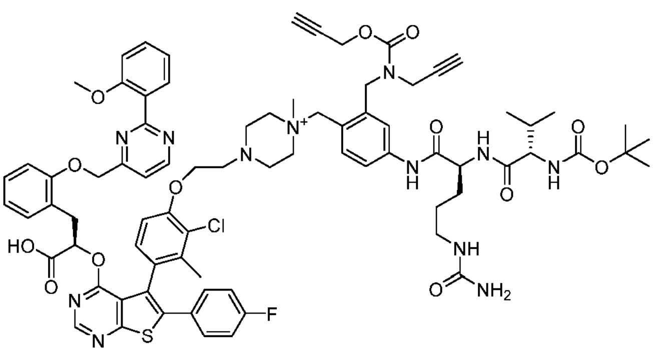

To (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoic acid hydrochloride (73.8 mg, 0.81 mmoles, 1.0 equiv.) and prop-2-yn-1-yl (5-((S)-2-((S)-2-((tert-butoxycarbonyl)amino)-3-methylbutanamido)-5-ureidopentanamido)-2-(chloromethyl)benzyl)(prop-2-yn-1-yl)carbamate (78 mg, 0.122 mmoles, 1.5 equiv.) dissolved in DMF (0.5 mL) was added DIEA (70 uL, 0.405 mmoles, 5.0 equiv.) followed by tetrabutylammonium iodide (25.4 mg, 0.069 mmoles, 0.85 equiv.). After stirring for 5 h, the reaction was diluted with DMSO (3 mL) and was purified by RP-HPLC ISCO gold chromatography (10-100% MeCN/H2O, 0.1% NH4OH modifier). Upon lyophilization, 1-(4-((S)-2-((S)-2-((tert-butoxycarbonyl)amino)-3-methylbutanamido)-5-ureidopentanamido)-2-((prop-2-yn-1-yl((prop-2-yn-1-yloxy)carbonyl) amino)methyl)benzyl)-4-(2-(4-(4-((R)-1-carboxy-2-(2-((2-(2-methoxyphenyl)pyrimidin-4-yl)methoxy)phenyl)

ethoxy)-6-(4-fluorophenyl)thieno[2,3-d]pyrimidin-5-yl)-2-chloro-3-methylphenoxy)ethyl)-1-methylpiperazin-1-ium was obtained. LCMS: M+=1486.3; Rt=2.70 min (5 min basic method).

**GENERAL PROCEDURE 2**

**Synthesis of 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

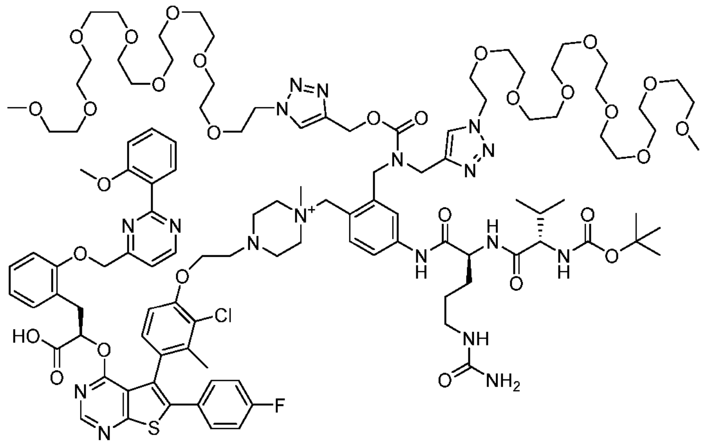

To 1-(4-((S)-2-((S)-2-((tert-butoxycarbonyl)amino)-3-methylbutanamido)-5-ureidopentanamido)-2-((prop-2-yn-1-yl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (24 mg, 0.016 mmoles, 1.0 equiv) and 25-azido-2,5,8,11,14,17,20,23-octaoxapentacosane (26.5 mg, 0.065 mmoles, 4 equiv.) was added t-BuOH (1 mL). The mixture was degassed via house vacuum and purged to a balloon of $N_2$ via a 3-way stopcock. Degas/purge was repeated 3 times. A 16 mg/mL aqueous solution of sodium ascorbate (297 uL, 0.024 mmoles, 1.5 equiv.) was added and the solution was degassed and purged to $N_2$ three times. A 4 mg/mL aqueous solution of copper sulfate (298 uL, 0.0048 mmoles, 0.3 equiv.) was added and the solution was degassed and purged to $N_2$ three times. After stirring under $N_2$ for 3 h the reaction was diluted with DMSO (3 mL) and was purified by RP-HPLC ISCO gold chromatography (10-100% MeCN/H2O, 0.1% TFA modifier). Upon lyophilization 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS M+=2307.0730, Rt=2.69 min (5 min acidic method).

**GENERAL PROCEDURE 3**

**Synthesis of 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L1-P1)**

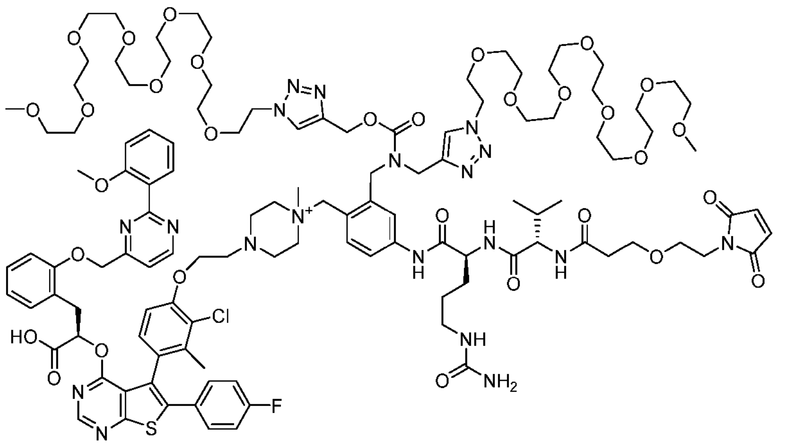

To 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (19 mg, 0.0082 mmoles, 1.0 equiv.) was added 25% TFA/$CH_2Cl_2$ (2 mL). After standing for 45 min, the volatiles were removed in vacuo, $CH_2Cl_2$ was added and the volatiles were removed in vacuo and pumped on. The residue was dissolved in DMF (1 mL) and DIEA (22 uL, 0.124 mmoles, 15 equiv.) and 2,5-dioxopyrrolidin-1-yl 3-(2-(2,5-dioxo-2,5-dihydro-1H-pyrrol-1-yl)ethoxy)propanoate (5.1 mg, 0.016 mmoles, 2 equiv. ) was added. After standing for 18 h, the solution was diluted with DMSO (3 mL) and was purified by RP-ISCO gold chromatography. Upon lyophilization, 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino) methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-methylpiperazin-1-ium **(L1-P1)** was obtained. HRMS: M+=2399.0797, Rt=2.43 min (5 min acidic run). $^1$H NMR (400 MHz, DMSO-$d_6$) δ ppm 0.83 (dd, *J*=13.82, 6.72 Hz, 6 H) 1.30 - 1.52 (m, 2 H) 1.55 - 1.76 (m, 2 H) 1.83 (s, 3 H) 1.88 - 2.08 (m, 1 H) 2.28 - 2.46 (m, 7 H) 2.73 - 2.84 (m, 4 H) 2.84 - 3.08 (m, 8 H) 3.15 - 3.27 (m, 3 H) 3.43 - 3.66 (m, 68 H) 3.73 -3.83 (m, 7 H) 4.16 - 4.30 (m, 3 H) 4.32 - 4.43 (m, 2 H) 4.47 (br s, 6 H) 4.60 (br s, 3 H) 5.16 - 5.30 (m, 3 H) 5.40 (br s, 2 H) 5.44 - 5.52 (m, 1 H) 5.99 (br t, *J*=5.07 Hz, 1 H) 6.21 (d, *J*=6.48 Hz, 1 H) 6.71 (t, *J*=7.40 Hz, 1 H) 6.97 - 7.04 (m, 2 H), 7.00 (s, 2H) 7.12 - 7.23 (m, 5 H) 7.27 - 7.54 (m, 8 H) 7.63 (d, *J*=5.14 Hz, 1 H) 7.78 - 7.94 (m, 3 H) 7.99 (br s, 1 H) 8.05 - 8.23 (m, 2 H) 8.60 (s, 1 H) 8.88 (d, *J*=5.13 Hz, 1 H) 10.24 (br s, 1 H).

**Synthesis of 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

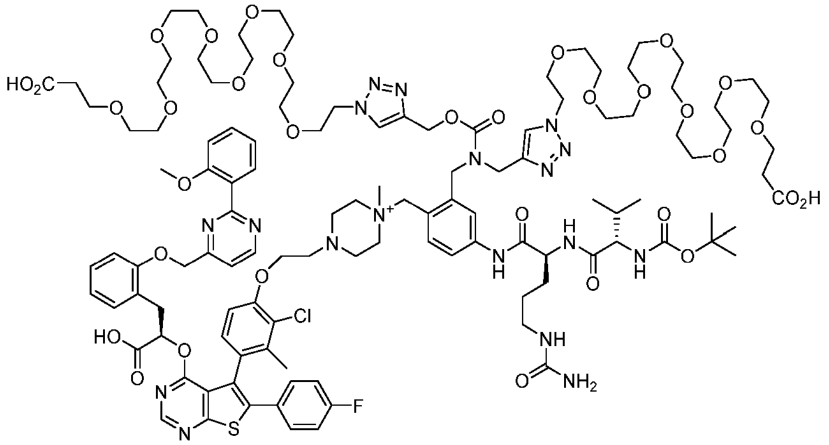

Following **GENERAL PROCEDURE 2** with 1-(4-((S)-2-((S)-2-((tert-butoxycarbonyl)amino)-3-methylbutanamido)-5-ureidopentanamido)-2-((prop-2-yn-1-yl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (30 mg, 0.020 mmoles, 1.0 equiv) and 1-azido-3,6,9,12,15,18,21,24-octaoxaheptacosan-27-oic acid (28.3 mg, 0.061 mmoles, 3 equiv.), 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=2420.0867, Rt=2.57 min (5 min acidic method).

**EXAMPLE 2: Synthesis of 4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-(2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino) methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L10-P1)**

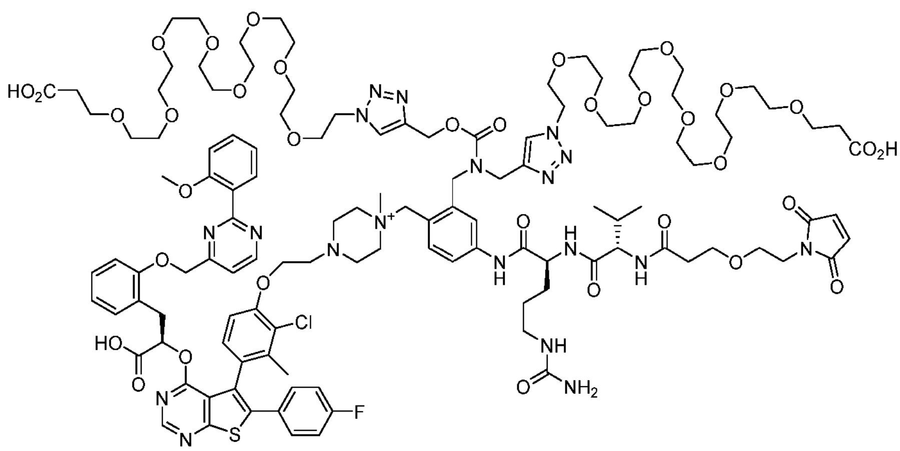

Following **GENERAL PROCEDURE 3** with 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl) benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (30 mg, 0.012 mmoles, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-(2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium **(L10-P1) )** was obtained. HRMS: M+=2515.0879, Rt=2.43 min (5 min acidic method). $^1$H NMR (400 MHz, DMSO-$d_6$) δ ppm 0.83 (dd, *J*=13.82, 6.72 Hz, 6 H) 1.30 - 1.52 (m, 2 H) 1.55 - 1.76 (m, 2 H) 1.83 (s, 3 H) 1.88 - 2.08 (m, 1 H) 2.28 - 2.46 (m, 7 H) 2.73 - 2.84 (m, 4 H) 2.84 - 3.08 (m, 8 H) 3.15 - 3.27 (m, 3 H) 3.43 - 3.66 (m, 68 H) 3.73 - 3.83 (m, 7 H) 4.16 - 4.30 (m, 3 H) 4.32 - 4.43 (m, 2 H) 4.47 (br s, 6 H) 4.60 (br s, 3 H) 5.16 - 5.30 (m, 3 H) 5.40 (br s, 2 H) 5.44 - 5.52 (m, 1 H) 5.99 (br t, *J*=5.07 Hz, 1 H) 6.21 (d, *J*=6.48 Hz, 1 H) 6.71 (t, *J*=7.40 Hz, 1 H) 6.97 - 7.04 (m, 2 H), 7.00 (s, 2H) 7.12 - 7.23 (m, 5 H) 7.27 - 7.54 (m, 8 H) 7.63 (d, *J*=5.14 Hz, 1 H) 7.78 - 7.94 (m, 3 H) 7.99 (br s, 1 H) 8.05 - 8.23 (m, 2 H) 8.60 (s, 1 H) 8.88 (d, *J*=5.13 Hz, 1 H) 10.24 (br s, 1 H).

**Synthesis of 1-(4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

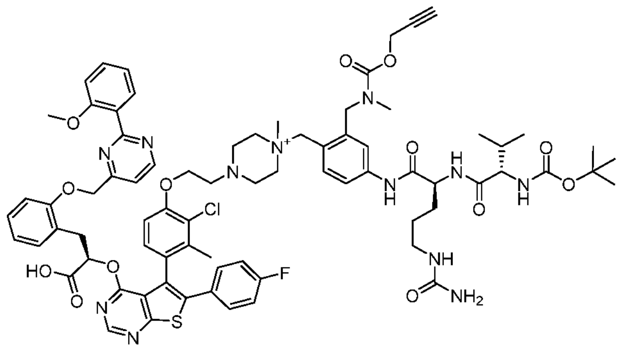

Following GENERAL PROCEDURE 1 with (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoic acid hydrochloride (300 mg, 0.329 mmol, 1.0 equiv.) and prop-2-yn-1-yl (5-((S)-2-((S)-2-((tert-butoxycarbonyl)amino)-3-methylbutanamido)-5-ureidopentanamido)-2-(chloromethyl)benzyl)(methyl)carbamate (246 mg, 0.395 mmol, 1.2 equiv.), 1-(4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained . HRMS: M+=1461.5800, Rt=2.53 min (5 min acidic method).

**Synthesis 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

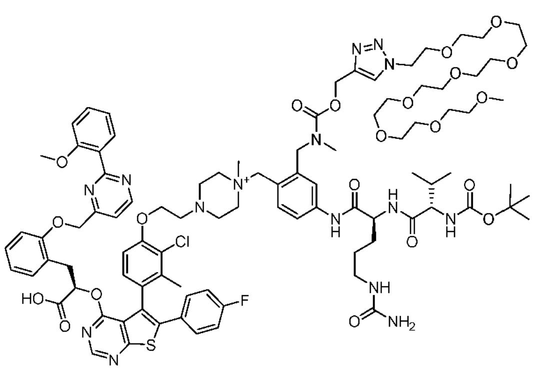

Following **GENERAL PROCEDURE 2** with 1-(4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (20 mg, 0.14 mmol, 1.0 equiv.) and 25-azido-2,5,8,11,14,17,20,23-octaoxapentacosane (16.8 mg, 0.041 mmol, 3.0 equiv.), 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=1872.8359, Rt=2.56 min (5 min acidic method).

**Synthesis of 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L4-P1)**

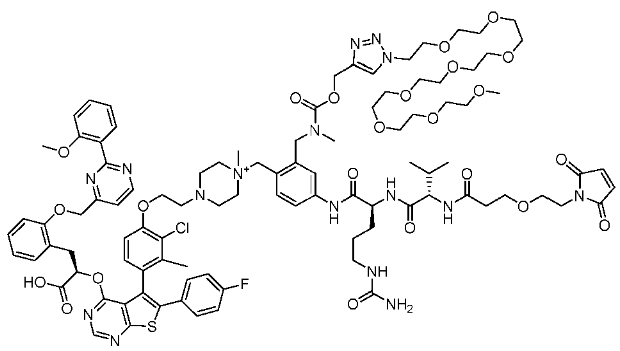

Following **GENERAL PROCEDURE** 3 with 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (16.9 mg, 0.009 mmol, 1.0 equiv.), 1-(2-(((((1-(2,5,8,11,14,17,20,23-octaoxapentacosan-25-yl)-1H-1,2, ,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium **(L4-P1)** was obtained. HRMS: M+= 1967.8375, Rt=2.46 min (5 min acidic method).

**Synthesis of 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)(methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

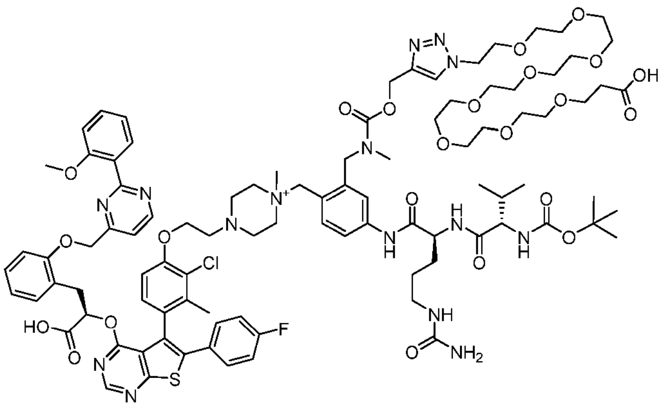

Following **GENERAL PROCEDURE 2** with 1-(4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (12 mg, 0.0082 mmol, 1.0 equiv) and 1-azido-3,6,9,12,15,18,21,24-octaoxaheptacosan-27-oic acid (11.5 mg, 0.025 mmol, 3.0 equiv.), 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)(methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=1928.8459, Rt=2.52 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((1-(26-carboxy-3,6,9,12,15,18,21,24-octaoxahexacosyl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L3-P1)**

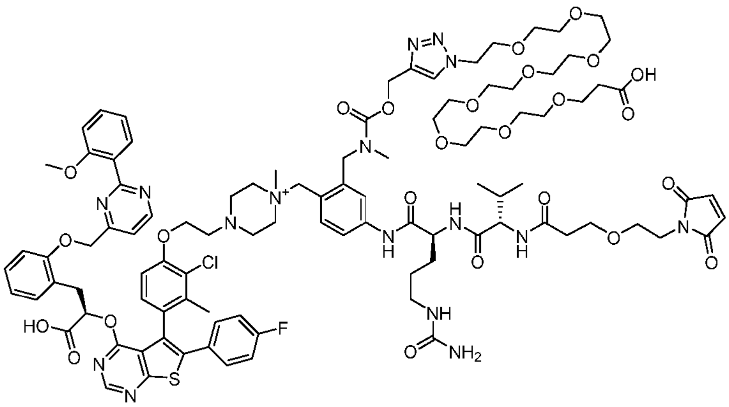

Following **general procedure 3** with 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)(methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (12 mg, 0.006 mmol,1.00 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((1-(26-carboxy-3,6,9,12,15,18,21,24-octaoxahexacosyl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium **(L3-P1)** was obtained . HRMS: M+=2024.8516, Rt=2.42 min (5 min acidic method).

**Synthesis of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

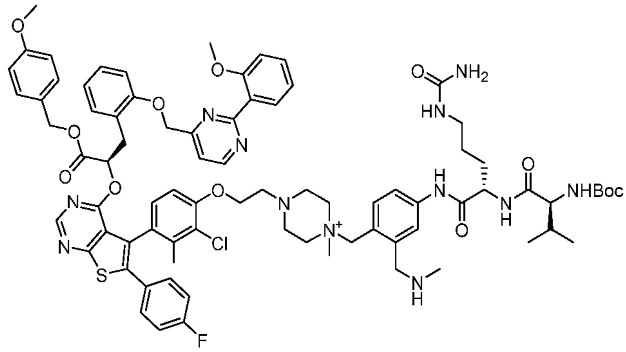

To 4-methoxybenzyl (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoate (160 mg, 0.161 mmol, 1.0 equiv.) and (9H-fluoren-9-yl)methyl (5-((S)-2-((S)-2-((tert-butoxycarbonyl)amino)-3-methylbutanamido)-5-ureidopentanamido)-2-(chloromethyl)benzyl)(methyl)carbamate (153 mg, 0.201 mmoles, 1.25 equiv.) dissolved in DMF (2 mL) was added DIEA (0.056 mL, 0.321 mmoles, 2.0 equiv.) followed by tetrabutylammonium iodide (65.3 mg, 0.177 mmoles, 1.1 equiv.). After standing for 16 h, 2.0 dimethylamine in THF (0.804 mL, 1.67 mmol, 10 equiv.) was added. After standing for 2 h, the volatiles were removed in vacuo, DMSO (6 mL) was added and the solution was purified by RP-HPLC ISCO gold chromatography (10-100% MeCN/H2O, 0.1% TFA modifier). Upon lyophilization, 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1499.3700; Rt=2.59 min (5 min acidic method).

**Synthesis of 1-(4-((R)-2-((R)-2-((tert-butoxycarbonyl)amino)-3-methylbutanamido)-5-ureidopentanamido)-2-(2-methyl-3-oxo-4,7,10,13,16,19,22,25,28-nonaoxa-2-azanonacosyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

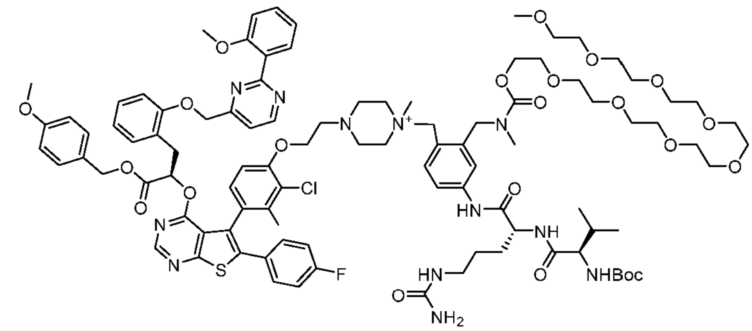

To 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (40 mg, 0.027 mmol, 1.0 equiv.) and 2,5-dioxopyrrolidin-1-yl (2,5,8,11,14,17,20,23-octaoxapentacosan-25-yl) carbonate (30.8 mg, 0.059 mmoles, 2.2 equiv.) dissolved in DMF (1.5 mL) was added DIEA (0.009 mL, 0.053 mmoles, 2.0 equiv.). After standing for 1 hour, DMSO (3 mL) was added and the solution was purified by RP-HPLC ISCO gold chromatography (10-100% MeCN/H2O, 0.1% TFA modifier). Upon lyophilization 1-(4-((R)-2-((R)-2-((tert-butoxycarbonyl)amino)-3-methylbutanamido)-5-ureidopentanamido)-2-(2-methyl-3-oxo-4,7,10,13,16,19,22,25,28-nonaoxa-2-azanonacosyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1909.3800; Rt=2.92 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((R)-2-((R)-2-(3-(2-(2,5-dioxo-2,5-dihydro-1H-pyrrol-1-yl)ethoxy)propanamido)-3-methylbutanamido)-5-ureidopentanamido)-2-(2-methyl-3-oxo-4,7,10,13,16,19,22,25,28-nonaoxa-2-azanonacosyl)benzyl)-1-methylpiperazin-1-ium (L2-P1)**

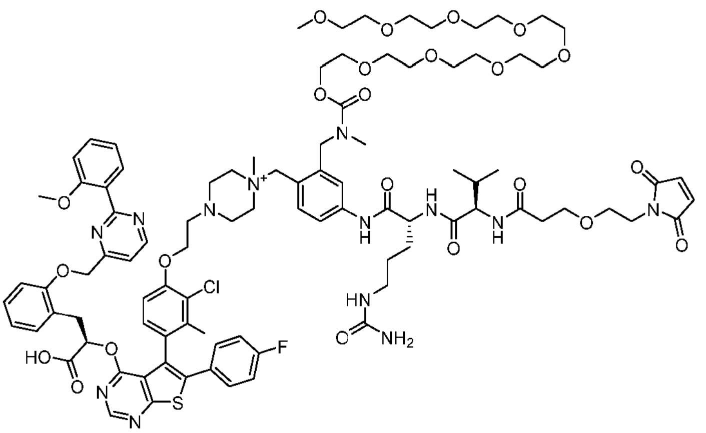

Following **GENERAL PROCEDURE 3** with 1-(4-((R)-2-((R)-2-((tert-butoxycarbonyl)amino)-3-methylbutanamido)-5-ureidopentanamido)-2-(2-methyl-3-oxo-4,7,10,13,16,19,22,25,28-nonaoxa-2-azanonacosyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (25.3 mg, 0.013 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((R)-2-((R)-2-(3-(2-(2,5-dioxo-2,5-dihydro-1H-pyrrol-1-yl)ethoxy)propanamido)-3-methylbutanamido)-5-ureidopentanamido)-2-(2-methyl-3-oxo-4,7,10,13,16,19,22,25,28-nonaoxa-2-azanonacosyl)benzyl)-1-methylpiperazin-1-ium **(L2-P1)** was obtained . HRMS: M+= 1884.7900, Rt=2.50 min (5 min acidic method).

**Synthesis of 1-(4-((S)-2-((S)-2-((tert-butoxycarbonyl)amino)-3-methylbutanamido)-5-ureidopentanamido)-2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

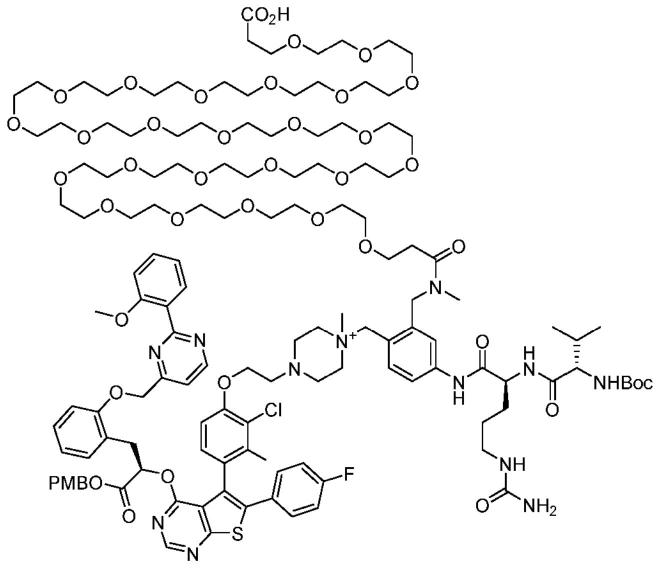

To 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (150 mg, 0.093 mmol, 1.0 equiv.) and 79-((2,5-dioxopyrrolidin-1-yl)oxy)-79-oxo-4,7,10,13,16,19,22,25,28,31,34,37,40,43,46,49,52,55,58,61,64,67,70,73,76-pentacosaoxanonaheptacontanoic acid (134 mg, 0.102 mmoles, 1.1 equiv.) dissolved in DMF (2 mL) was added DIEA (0.081 mL, 0.464 mmoles, 5.0 equiv.). After standing for 18 h, DMSO (6 mL) was added and the solution was purified by RP-HPLC ISCO gold chromatography (10-100% MeCN/H2O, 0.1% TFA modifier). Upon lyophilization 1-(4-((S)-2-((S)-2-((tert-butoxycarbonyl)amino)-3-methylbutanamido)-5-ureidopentanamido)-2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=2700.8701; Rt=2.83 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L11-P1)**

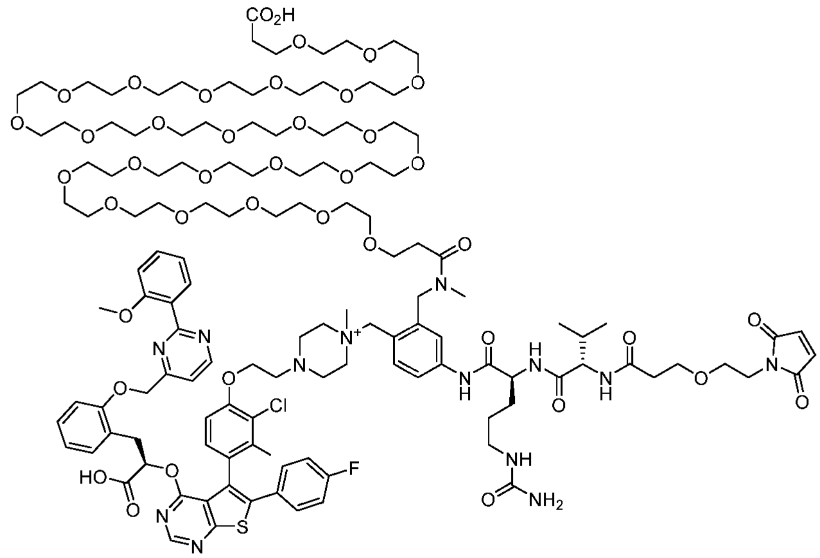

Following **GENERAL PROCEDURE 3** with 1-(4-((S)-2-((S)-2-((tert-butoxycarbonyl)amino)-3-methylbutanamido)-5-ureidopentanamido)-2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium, 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (**L11-P1)** was obtained . HRMS: M+= 2674.8201, Rt=2.44 min (5 min acidic method).

**Synthesis of 1-(4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

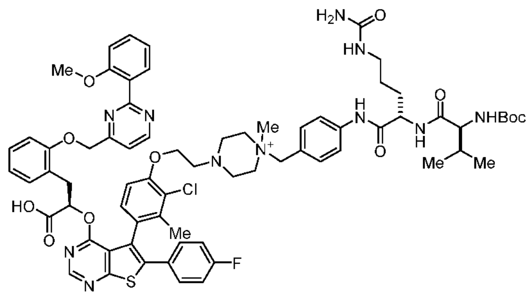

Following GENERAL PROCEDURE 1 with (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoic acid (50 mg, 0.057 mmol, 1.0 equiv.) and tert-butyl ((S)-1-(((S)-1-((4-(chloromethyl)phenyl)amino)-1-oxo-5-ureidopentan-2-yl)amino)-3-methyl-1-oxobutan-2-yl)carbamate (34.1 mg, 0.069 mmol, 1.2 equiv.), 1-(4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. LCMS: M+=1337.2, Rt=1.11 min (2 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium or (2R)-2-[(5S$_a$)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (L9-P1)**

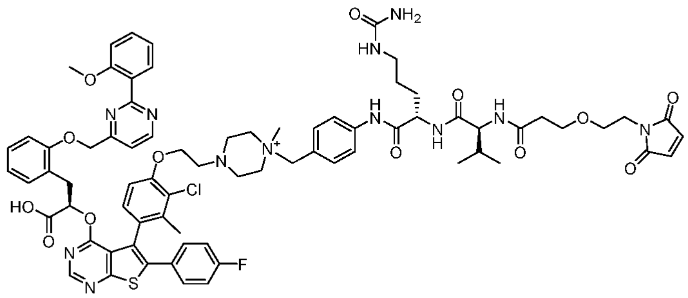

Following **GENERAL PROCEDURE 3** with 1-(4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (55 mg, 0.041 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (**L9-P1**) was obtained. HRMS: M+=1431.5400, Rt=2.50 min (5 min acidic method).

**Synthesis of 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

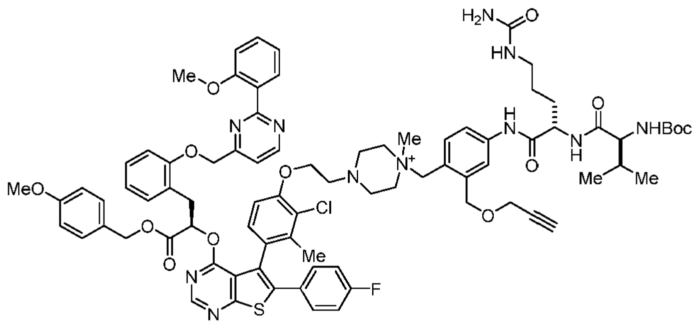

Following GENERAL PROCEDURE 1 with 4-methoxybenzyl (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoate (85 mg, 0.085 mmol, 1.0 equiv.) and tert-butyl ((S)-1-(((S)-1-((4-(chloromethyl)-3-((prop-2-yn-1-yloxy)methyl)phenyl)amino)-1-oxo-5-ureidopentan-2-yl)amino)-3-methyl-1-oxobutan-2-yl)carbamate (58 mg, 0.102 mmol, 1.2 equiv.), 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1524.6200, Rt=2.95 min (5 min acidic method).

**Synthesis of 1-(2-(((1-(2,5,8,11,14,17,20,23-octaoxapentacosan-25-yl)-1H-1,2,3-triazol-4-yl)methoxy) methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

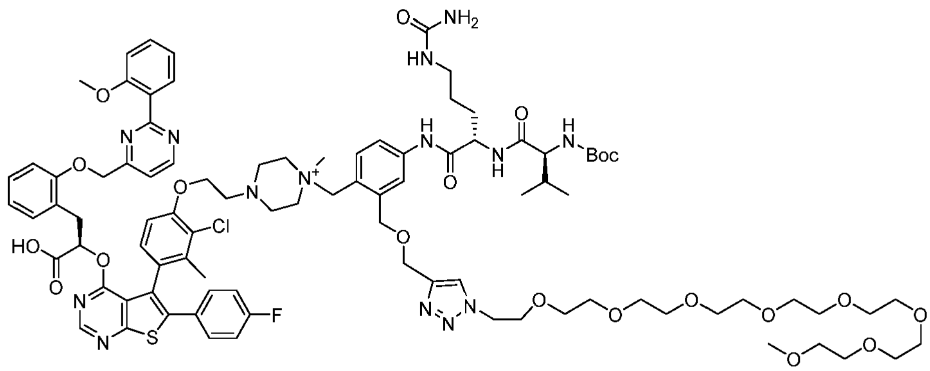

Following GENERAL PROCEDURE 2 with 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl) pyrimidin-4-yl)methoxy)phenyl)ethoxy)-6-(4-fluorophenyl)thieno[2,3-d]pyrimidin-5-yl)-2-chloro-3-methylphenoxy) ethyl)-1-methylpiperazin-1-ium (20 mg, 0.014 mmoles, 1.0 equiv) and 25-azido-2,5,8,11,14,17,20,23-octaoxapentacosane (5.8 mg, 0.014 mmoles, 1 equiv.), 1-(2-(((1-(2,5,8,11,14,17,20,23-octaoxapentacosan-25-yl)-1H-1,2,3-triazol-4-yl) methoxy)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-methylpiperazin-1-ium was obtained. LCMS: [(M+)+H+] +2/2=908.5, Rt=1.15 min (2 min acidic method).

**Synthesis of 1-(2-(((1-(2,5,8,11,14,17,20,23-octaoxapentacosan-25-yl)-1H-1,2,3-triazol-4-yl)methoxy) methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy) phenyl)ethoxy)-6-(4-fluorophenyl)thieno[2,3-d]pyrimidin-5-yl)-2-chloro-3-methylphenoxy)ethyl)-1-methylpiperazin-1-ium (L8-P1)**

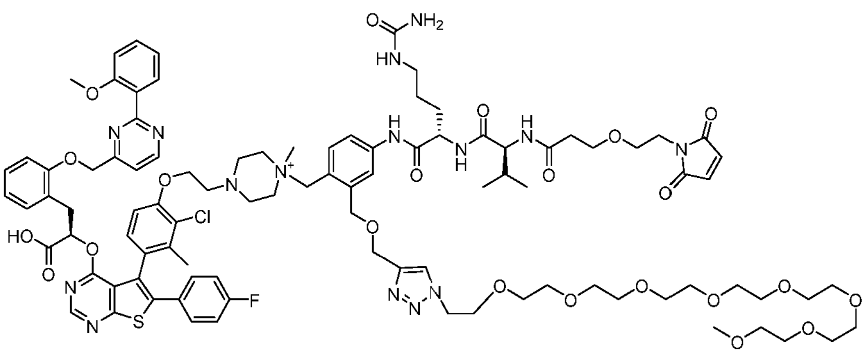

Following **GENERAL PROCEDURE 3** with 1-(2-(((1-(2,5,8,11,14,17,20,23-octaoxapentacosan-25-yl)-1H-1,2,3-triazol-4-yl)methoxy)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-methylpiperazin-1-ium (20 mg, 0.010 mmol, 1.0 equiv.), 1-(2-(((1-(2,5,8,11,14,17,20,23-octaoxapentacosan-25-yl)-1H-1,2,3-triazol-4-yl)methoxy) methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-methylpiperazin-1-ium **(L8-**

**P1)** was obtained . HRMS: M+= 1908.8097, Rt=2.37 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium**

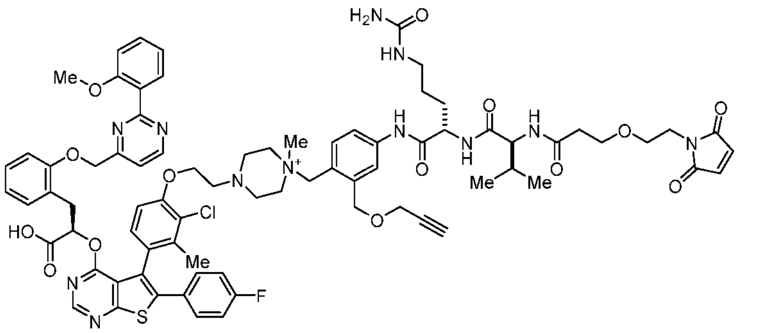

Following **GENERAL PROCEDURE 3** with 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (123.1 mg, 0.075 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium was obtained. HRMS: M+= 1499.5601, Rt=2.50 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(26-carboxy-3,6,9,12,15,18,21,24-octaoxahexacosyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L7-P1)**

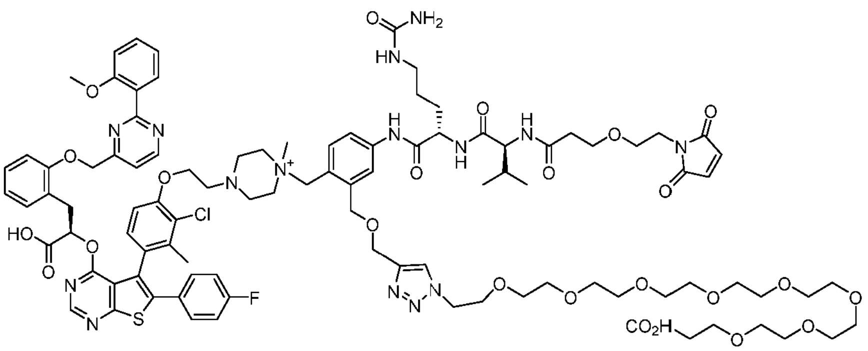

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium (40 mg, 0.027 mmoles, 1.0 equiv) and 1-azido-3,6,9,12,15,18,21,24-octaoxaheptacosan-27-oic acid (37.4 mg, 0.080 mmoles, 3.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(26-carboxy-3,6,9,12,15,18,21,24-octaoxahexacosyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium **(L7-P1)** was obtained. HRMS: M+=1965.5601, Rt=2.35 min (5 min acidic method).

**Synthesis of 1-(2-(((1-(2,5,8,11,14,17,20,23,26,29,32,35,38,41,44,47,50,53,56,59,62,65,68,71-tetracosaoxatriheptacontan-73-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L5-P1)**

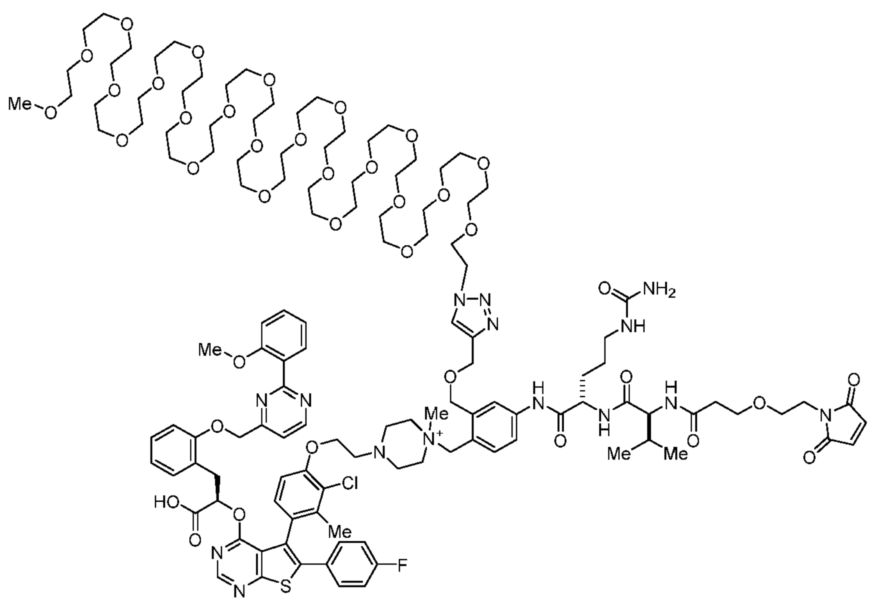

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium (20.4 mg, 0.014 mmoles, 1.0 equiv) and 73-azido-2,5,8,11,14,17,20,23,26,29,32,35,38,41,44,47,50,53,56,59,62,65,68,71-tetracosaoxatriheptacontane (28.1 mg, 0.025 mmoles, 1.5 equiv.), 1-(2-(((1-(2,5,8,11,14,17,20,23,26,29,32,35,38,41,44,47,50,53,56,59,62,65,68,71-tetracosaoxatriheptacontan-73-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L5-P1) was obtained. HRMS: M+=2613.2100, Rt=2.44 min (5 min acidic method).

**Synthesis of prop-2-yn-1-yl (5-((S)-2-((S)-2-((tert-butoxycarbonyl)amino)-3-methylbutanamido)-5-ureidopentanamido)-2-((((4-nitrophenoxy)carbonyl)oxy)methyl)benzyl)(methyl)carbamate**

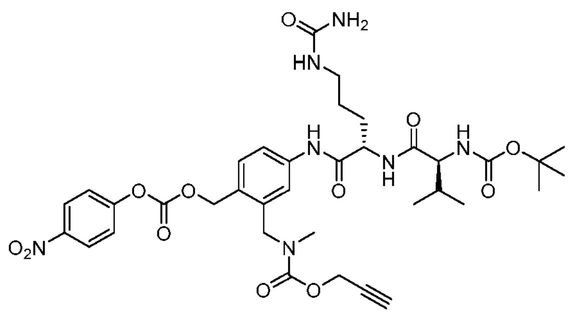

A solution of prop-2-yn-1-yl (5-((S)-2-((S)-2-((tert-butoxycarbonyl)amino)-3-methylbutanamido)-5-ureidopentanamido)-2-(hydroxymethyl)benzyl)(methyl)carbamate (249 mg, 0.412 mmoles) and 4-nitrophenyl (4-nitrosophenyl) carbonate (356 mg, 1.24 mmoles, 3.0 equiv.) in DMF (2 mL) was swirled until homogeneous and sat for 16 hours. The solution was diluted with DMSO (6 mL) and was purified by RP-HPLC ISCO gold chromatography (10-100% MeCN/H2O, no modifier). Upon lyophilization, prop-2-yn-1-yl (5-((S)-2-((S)-2-((tert-butoxycarbonyl)amino)-3-methylbutanamido)-5-ureidopentanamido)-2-((((4-nitrophenoxy)carbonyl)oxy)methyl)benzyl)(methyl)carbamate was obtained. LC/MS MH+=770.7, Rt=2.45 min (5 min acidic method).

**Synthesis of prop-2-yn-1-yl (5-((R)-2-((R)-2-((tert-butoxycarbonyl)amino)-3-methylbutanamido)-5-ureidopentanamido)-2-(((methyl(2-(methylamino)ethyl)carbamoyl)oxy)methyl)benzyl)(methyl)carbamate**

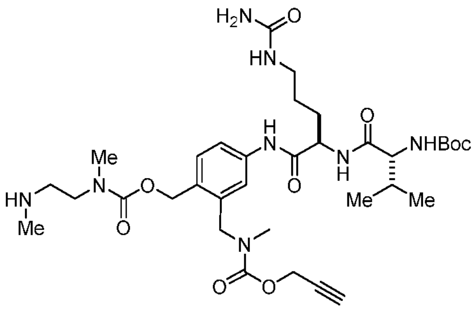

To a solution of prop-2-yn-1-yl (5-((S)-2-((S)-2-((tert-butoxycarbonyl)amino)-3-methylbutanamido)-5-ureidopentanamido)-2-((((4-nitrophenoxy)carbonyl)oxy)methyl)benzyl)(methyl)carbamate (100mg, 0.130 mmol) in DMF (1 ml) was added N,N'-Dimethyl-ethylenediamine (22.90 mg, 0.260 mmol), followed by the addition of DIPEA (0.113 ml, 0.650 mmol) at room temperature. The resulting solution was stirred at room temperature overnight. The reaction was diluted with DMSO was purified by RP-HPLC ISCO gold chromatography (10-100% MeCN/$H_2O$, 0.1% NH4OH modifier). Upon lyophilization prop-2-yn-1-yl (5-((R)-2-((R)-2-((tert-butoxycarbonyl)amino)-3-methylbutanamido)-5-ureidopentanamido)-2-(((methyl(2-(methylamino)ethyl)carbamoyl)oxy)methyl)benzyl)(methyl)carbamate was obtained. LCMS: MH+=719.9, Rt=0.73 min (2 min acidic method).

**Synthesis of (R)-4-(2-(2-chloro-4-(6-(4-fluoro-3-hydroxyphenyl)-4-((1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methyl-1-(3-sulfopropyl)piperazin-1-ium**

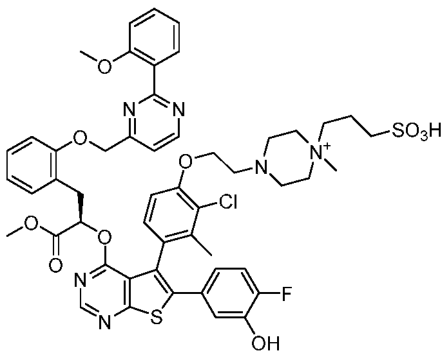

To a solution of (R)-4-(2-(4-(4-(1-carboxy-2-(2-((2-(2-methoxyphenyl)pyrimidin-4-yl)methoxy)phenyl) ethoxy)-6-(4-fluoro-3-hydroxyphenyl)thieno[2,3-d]pyrimidin-5-yl)-2-chloro-3-methylphenoxy)ethyl)-1-methyl-1-(3-sulfopropyl)piperazin-1-ium or (2*R*)-2-[(5*$S_a$*)-5-[3-chloro-2-methyl-4-[2-[4-methyl-4-(3-sulfopropyl)piperazin-4-ium-1-yl] ethoxy]phenyl]-6-(4-fluoro-3-hydroxy-phenyl)thieno[2,3-d]pyrimidin-4-yl]oxy-3-[2-[[2-(2-methoxyphenyl)pyrimidin-4-yl] methoxy]phenyl]propanoic acid (100 mg, 0.099 mmoles) in MeOH (1.5 mL) was added a few drops of $H_2SO_4$(conc.). After stirring overnight, the MeOH was removed in vacuo, the residue was dissolved in DMSO was purified by RP-HPLC ISCO gold chromatography (10-100% MeCN/H2O, 0.1% TFA modifier). Upon lyophilization, (R)-4-(2-(2-chloro-4-(6-(4-fluoro-3-hydroxyphenyl)-4-((1-methoxy-3-(2-((2-(2-methoxyphenyl)pyrimidin-4-yl)methoxy)phenyl)-1-oxopropan-2-yl) oxy)thieno[2,3-d]pyrimidin-5-yl)-3-methylphenoxy)ethyl)-1-methyl-1-(3-sulfopropyl)piperazin-1-ium was obtained. HRMS M+=1027.2900, Rt=2.31 min (5 min acidic method).

**Synthesis of 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino) ethyl)(methyl)carba moyl)oxy)-4-fluorophenyl)-4-(((R)-1-methoxy-3-(2-((2-(2-methoxyphenyl)pyrimidin-4-yl) methoxy)phenyl)-1-oxopropan-2-yl)oxy)thieno[2,3-d]pyrimidin-5-yl)-2-chloro-3-methylphenoxy)ethyl)-1-methyl-1-(3-sulfopropyl)piperazin-1-ium**

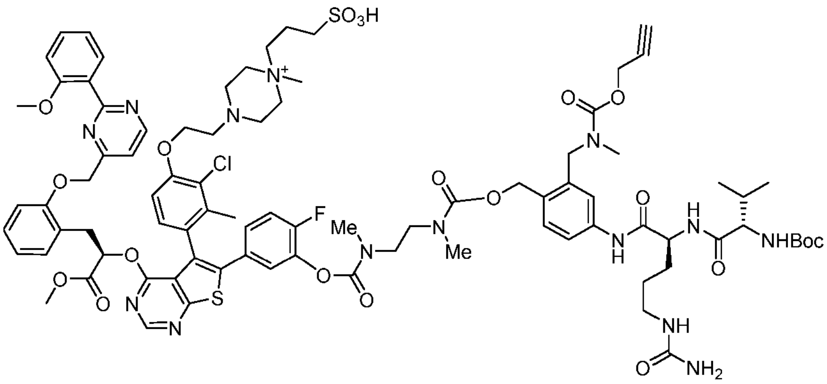

To a solution of (R)-4-(2-(2-chloro-4-(6-(4-fluoro-3-hydroxyphenyl)-4-((1-methoxy-3-(2-((2-(2-methoxyphenyl) pyrimidin-4-yl)methoxy)phenyl)-1-oxopropan-2-yl)oxy)thieno[2,3-d]pyrimidin-5-yl)-3-methylphenoxy)ethyl)-1-methyl-1-(3-sulfopropyl)piperazin-1-ium (50 mg, 0.049 mmoles, 1.0 equiv) in $CH_2Cl_2$ (1 mL) at 0 °C was added TEA (34 uL, 0.243 mmoles, 5.0 equiv.) followed by 4-Nitrophenyl chloroformate (10.8 mg, 0.054 mmoles, 1.1 equiv.). After stirring for 15 min, a solution of prop-2-yn-1-yl (5-((R)-2-((R)-2-((tert-butoxycarbonyl)amino)-3-methylbutanamido)-5-ureidopentanamido)-2-(((methyl(2-(methylamino)ethyl)carbamoyl)oxy)methyl)benzyl)(methyl)carbamate (66.3 mg, 0.092 mmoles, 2.0 equiv) in DMF (1 mL) was added followed by DIEA (40 uL, 0.231 mmoles, 5.0 equiv.). After stirring for 2 h, the volatiles were removed in vacuo, the solution was diluted with DMSO (3 ml) and was purified by RP-HPLC ISCO gold chromatography (10-100% MeCN/H2O, 0.1% TFA modifier). Upon lyophilization 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)-4-(((R)-1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-2-chloro-3-methylphenoxy)ethyl)-1-methyl-1-(3-sulfopropyl)piperazin-1-ium was obtained. HRMS M+=1771.6700, Rt=2.57 min (5 min acidic method).

**Synthesis of 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino) ethyl)(methyl)carba moyl)oxy)-4-fluorophenyl)-4-((R)-1-carboxy-2-(2-((2-(2-methoxyphenyl)pyrimidin-4-yl) methoxy)phenyl)ethoxy)thieno[2,3-d]pyrimidin-5-yl)-2-chloro-3-methylphenoxy)ethyl)-1-methyl-1-(3-sulfopropyl)piperazin-1-ium**

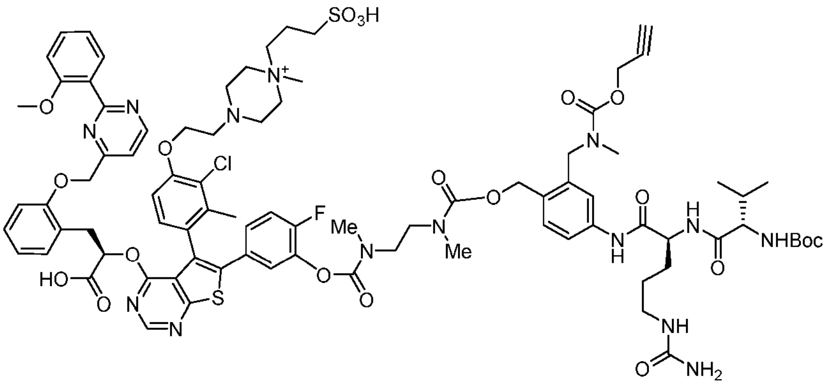

To a solution of 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)-4-(((R)-1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-2-chloro-3-methylphenoxy)ethyl)-1-methyl-1-(3-sulfopropyl)piperazin-1-ium (23 mg, 0.013 mmoles, 1.0 equiv) in THF (1 mL) was added 2N LiOH (0.032 mL, 0.065 mmoles, 5 equiv). After stirring for 2 h, the solution was neutralized with AcOH and purified by RP-HPLC ISCO gold chromatography (10-100% MeCN/H2O, 0.1% TFA modifier). Upon lyophilization, 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium was obtained. HRMS M+=1757.6200, Rt=2.46 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(3-(((2-((((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carba moyl)oxy)-4-fluorophenyl)thieno[2,3-d]pyrimidin-5-yl)-2-chloro-3-methylphenoxy)ethyl)-1-methyl-1-(3-sulfopropyl)piperazin-1-ium**

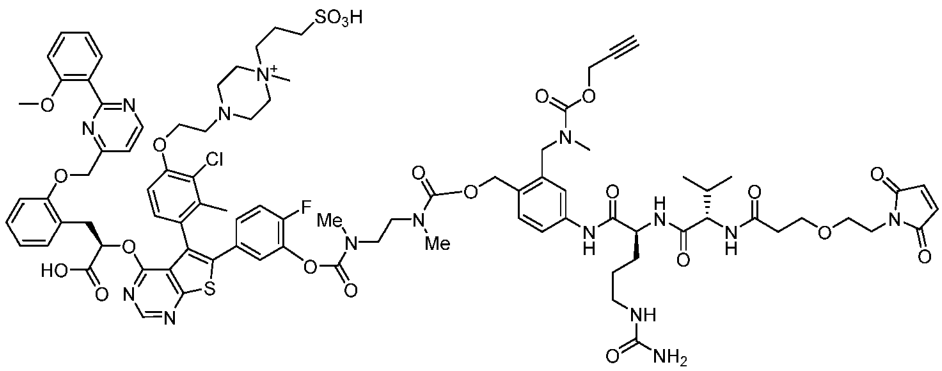

Following **GENERAL PROCEDURE 3** with 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium (17 mg, 0.0097 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(3-(((2-((((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)thieno[2,3-d]pyrimidin-5-yl)-2-chloro-3-methylphenoxy)ethyl)-1-methyl-1-(3-sulfopropyl)piperazin-1-ium was obtained. HRMS: M+= 1852.5200, Rt=2.29 min (5 min acidic method).

**Synthesis of 4-(2-(4-(6-(3-(((2-((((2-(((((1-(2,5,8,11,14,17,20,23-octaoxapentacosan-25-yl)-1H-1,2,3-triazol-4-yl) methoxy)carbonyl)(methyl)amino)methyl)-4-((S)-2-((S)-2-(3-(2-(2,5-dioxo-2,5-dihydro-1H-pyrrol-1-yl)ethoxy) propanamido)-3-methylbutanamido)-5-ureidopentanamido)benzyl)oxy)carbonyl)(methyl)amino)ethyl)(methyl) carbamoyl)oxy) -4-fluorophenyl)-4-((R)-1-carboxy-2-(2-((2-(2-methoxyphenyl)pyrimidin-4-yl)methoxy)phenyl) ethoxy)thieno[2,3-d]pyrimidin-5-yl)-2-chloro-3-methylphenoxy)ethyl)-1-methyl-1-(3-sulfopropyl)piperazin-1-ium (L12-P2)**

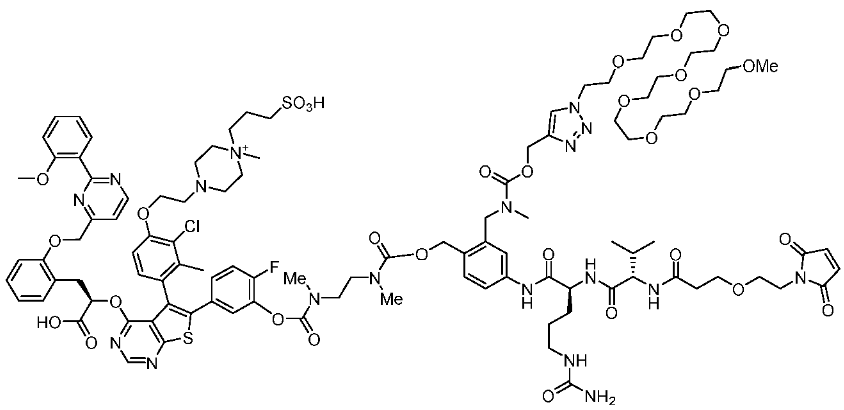

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(3-(((2-((((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy) carbonyl)(methyl)amino)ethyl)(methyl)carbamoyl)ox y)-4-fluorophenyl)thieno[2,3-d]pyrimidin-5-yl)-2-chloro-3-methylphenoxy)ethyl)-1-methyl-1-(3-sulfopropyl)piperazin-1-ium (10 mg, 0.0054 mmoles, 1.0 equiv.) and 25-azido-2,5,8,11,14,17,20,23-octaoxapentacosane (4.4 mg, 0.011 mmoles, 2 equiv.), 4-(2-(4-(6-(3-(((2-((((2-(((((1-(2,5,8,11,14,17,20,23-octaoxapentacosan-25-yl)-1 H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)oxy)carbonyl)(methyl)amino)ethyl)(methyl)carbamoyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium (**L12-P2**) was obtained . HRMS: M+=2261.8601, Rt=2.24 min (5 min acidic method).

**Synthesis of 4-(2-(4-(6-(3-((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium**

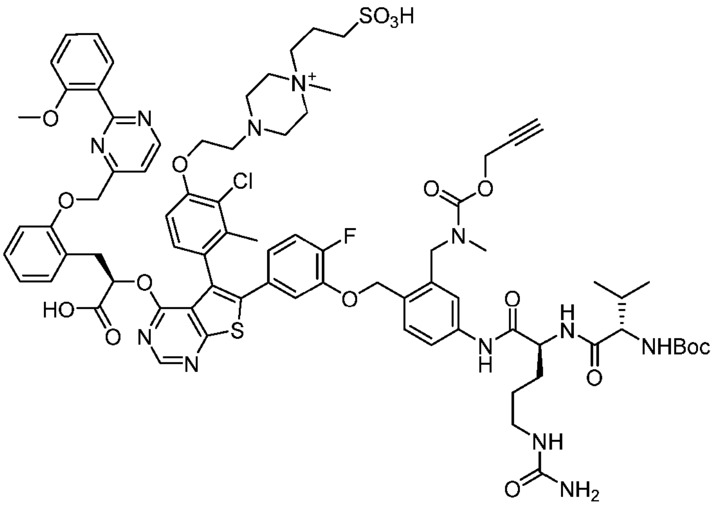

GENERAL PROCEDURE 1 was followed using (R)-4-(2-(2-chloro-4-(6-(4-fluoro-3-hydroxyphenyl)-4-((1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methyl-1-(3-sulfopropyl)piperazin-1-ium (40 mg, 0.039 mmol, 1.0 equiv.) and prop-2-yn-1-yl (5-((R)-2-((R)-2-((tert-butoxycarbonyl)amino)-3-methylbutanamido)-5-ureidopentanamido)-2-(chloromethyl)benzyl)(methyl)carbamate (36.4 mg, 0.058 mmol, 1.5 equiv.), with the modification of after the alkylation was complete adding 2N LiOH (0.097 mL, 0.195 mmoles, 5.0 equiv.) and stirring for 2 h prior to neutralizing and purifying by RP-HPLC. Upon lyophilization, 4-(2-(4-(6-(3-((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium was obtained. HRMS: M+=1599.5856, Rt=1.34 min (2 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(3-((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)-4-fluorophenyl)thieno[2,3-d]pyrimidin-5-yl)-2-chloro-3-methylphenoxy)ethyl)-1-methyl-1-(3-sulfopropyl)piperazin-1-ium**

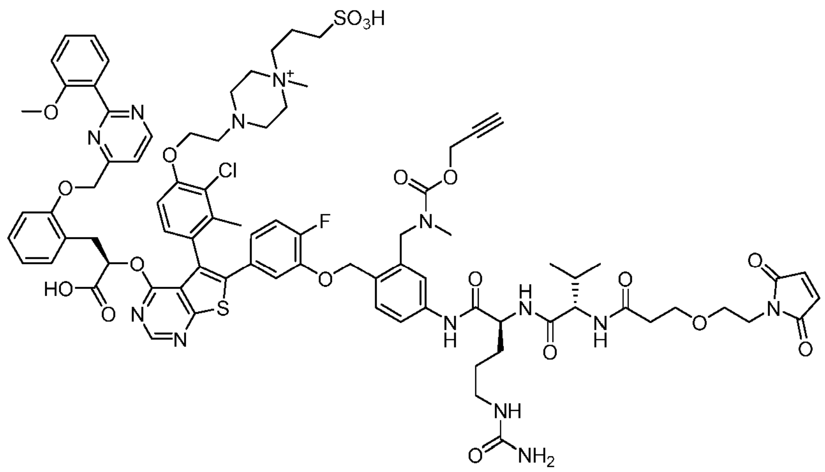

Following **GENERAL PROCEDURE 3** with 4-(2-(4-(6-(3-((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium (46 mg, 0.029 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(3-((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)-4-fluorophenyl)thieno[2,3-d]pyrimidin-5-yl)-2-chloro-3-methylphenoxy)ethyl)-1-methyl-1-(3-sulfopropyl)piperazin-1-ium was obtained. HRMS: M+= 1694.5699, Rt=2.55 min (5 min acidic method).

**Synthesis of 4-(2-(4-(6-(3-((2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium (L4-P2)**

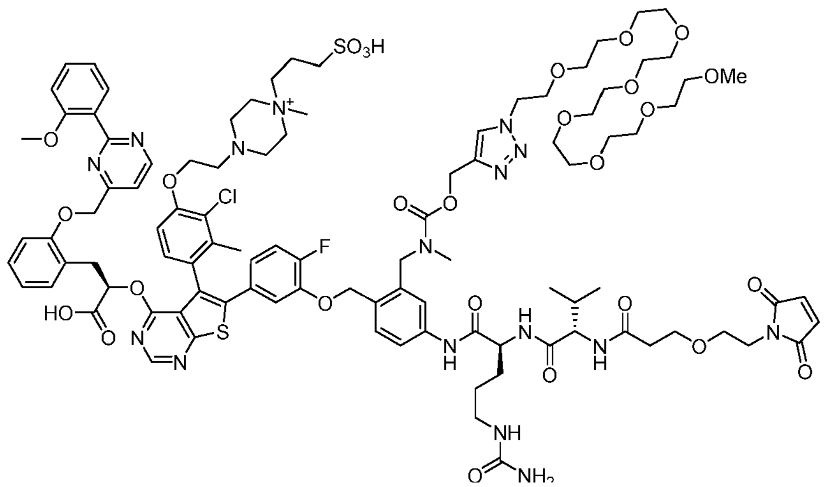

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(3-((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)-4-fluorophenyl)thieno[2,3-d]pyrimidin-5-yl)-2-chloro-3-methylphenoxy)ethyl)-1-methyl-1-(3-sulfopropyl)piperazin-1-ium (44 mg, 0.026 mmoles, 1.0 equiv.) and 25-azido-2,5,8,11,14,17,20,23-octaoxapentacosane (21.2 mg, 0.052 mmoles, 2 equiv.), 4-(2-(4-(6-(3-((2-(((((1-(2,5,8,11,14,17,20,23-octaoxapentacosan-25-yl)-1 H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium **(L4-P2)** was obtained. HRMS: M+=2103.8000, Rt=2.47 min (5 min acidic method).

**GENERAL PROCEDURE 4:**

**Synthesis of 1-(4-((S)-2-((S)-2-((tert-butoxycarbonyl)amino)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

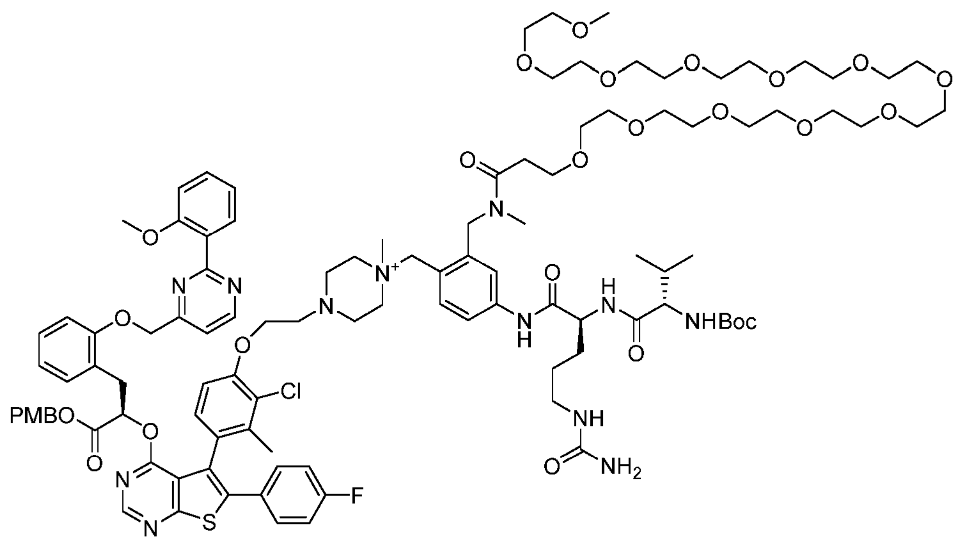

To 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (60 mg, 0.040 mmol, 1.0 equiv.) and 2,5-dioxopyrrolidin-1-yl 2,5,8,11,14,17,20,23,26,29,32,35-dodecaoxaoctatriacontan-38-oate (35.7 mg, 0.052 mmoles, 1.3 equiv.) dissolved in DMF (1 mL) was added DIPEA (0.035 mL, 0.200 mmoles, 5.0 equiv.). After standing for 18 h, DMSO (2 mL) was added and the solution was purified by RP-HPLC ISCO gold chromatography (10-70% MeCN/H2O, 0.1% TFA modifier). Upon lyophilization, 1-(4-((S)-2-((S)-2-((tert-butoxycarbonyl)amino)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: [M+Na]+=2092.9399; Rt=2.88 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-1-methylpiperazin-1-ium (L32-P1)**

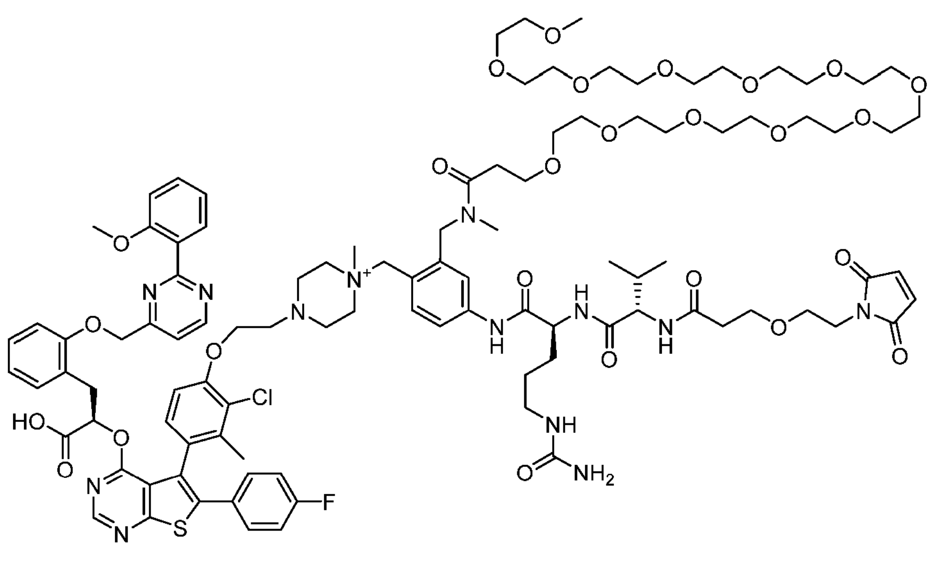

Following **GENERAL PROCEDURE** 3 with 1-(4-((S)-2-((S)-2-((tert-butoxycarbonyl)amino)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (43.4 mg, 0.021 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-1-methylpiperazin-1-ium was obtained. HRMS: [M+Na]+= 2066.8799; Rt=2.44 min (5 min acidic method).

**Synthesis of 1-(4-((S)-2-((S)-2-((tert-butoxycarbonyl)amino)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

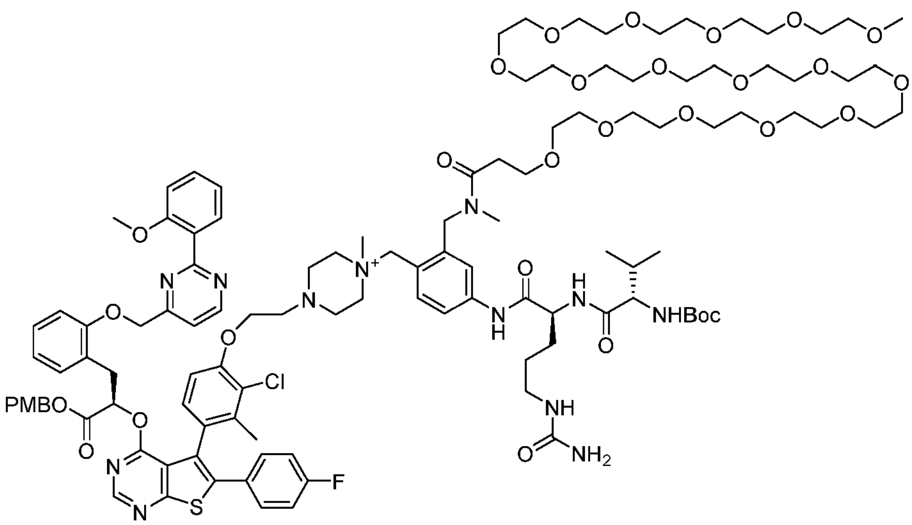

Following **GENERAL PROCEDURE 4** with 2,5-dioxopyrrolidin-1-yl 2,5,8,11,14,17,20,23,26,29,32,35,38,41,44,47-hexadecaoxapentacontan-50-oate (44.8 mg, 0.021 mmol, 1 eq), 1-(4-((S)-2-((S)-2-((tert-butoxycarbonyl)amino)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 2246.0400; Rt=2.88 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-1-methylpiperazin-1-ium (L31-P1)**

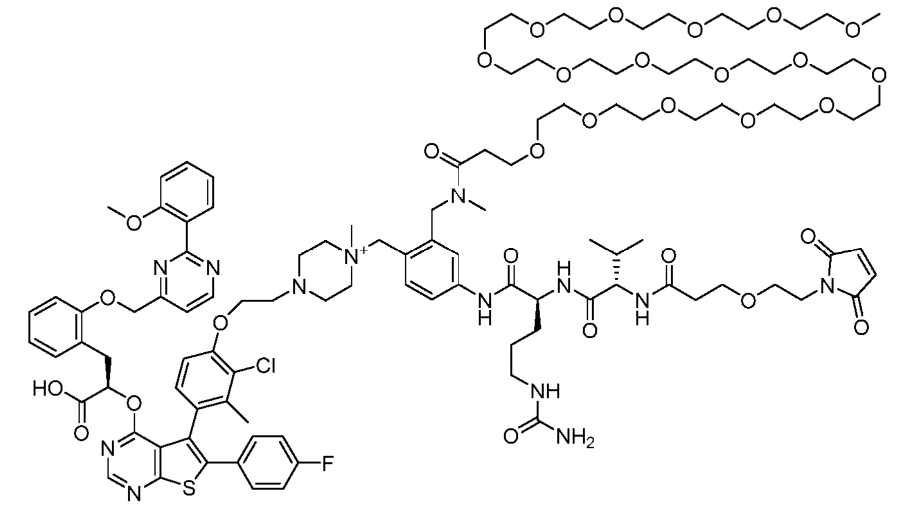

Following **GENERAL PROCEDURE 3** with 1-(4-((S)-2-((S)-2-((tert-butoxycarbonyl)amino)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy) ethyl)-1-methylpiperazin-1-ium (47.5 mg, 0.021 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-1-methylpiperazin-1-ium was obtained. HRMS: M+= 2221.0000; Rt=2.45 min (5 min acidic method).

**Syntheis of 1-(4-((S)-2-((S)-2-((tert-butoxycarbonyl)amino)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

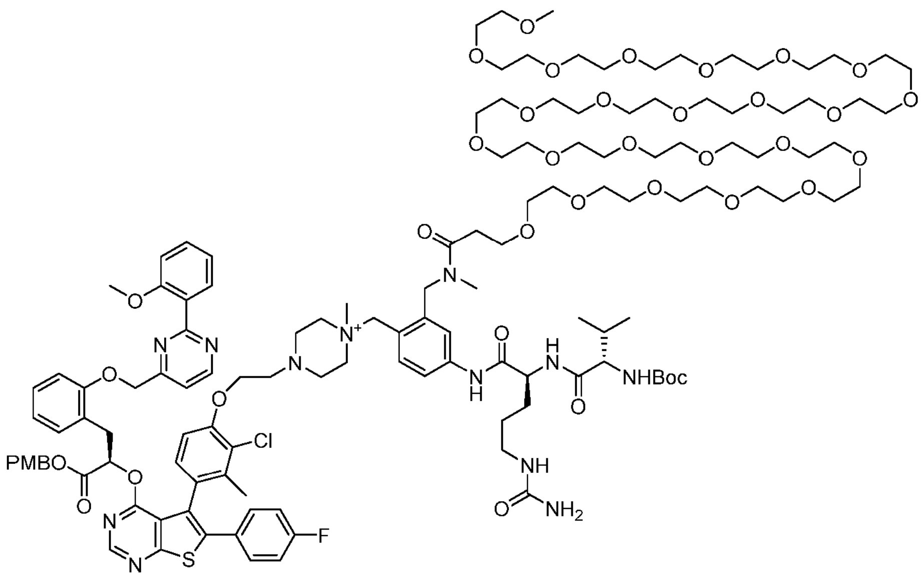

Following **GENERAL PROCEDURE 4** with 2,5-dioxopyrrolidin-1-yl 2,5,8,11,14,17,20,23,26,29,32,35,38,41,44,47,50,53,56,59,62,65,68,71-tetracosaoxatetraheptacontan-74-oate (52.6 mg, 0.043 mmol, 1.3 eq), 1-(4-((S)-2-((S)-2-((tert-butoxycarbonyl)amino)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy) ethyl)-1-methylpiperazin-1-ium was obtained. HRMS: M+= 2598.2500; Rt=2.88 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-1-methylpiperazin-1-ium (L30-P1)**

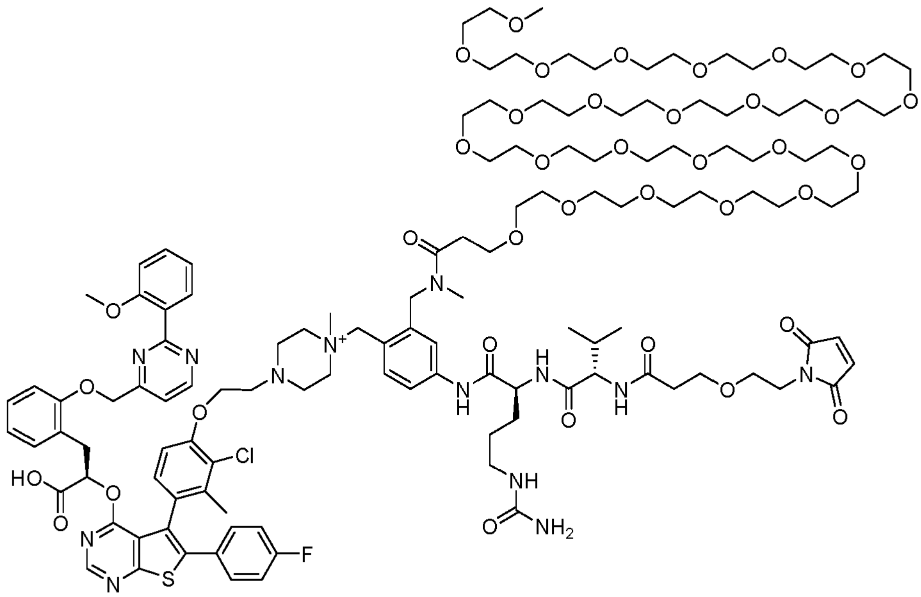

Following **GENERAL PROCEDURE** 3 with 1-(4-((S)-2-((S)-2-((tert-butoxycarbonyl)amino)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-4-(2-(2-chloro-4-(6-(4-fluorophenyl)-4-(((R)-1-((4-methoxybenzyl)oxy)-3-(2-((2-(2-methoxyphenyl) pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (38.8 mg, 0.014 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-1-methylpiperazin-1-ium was obtained. HRMS: M+= 2573.2000; Rt=2.47 min (5 min acidic method).

**Synthesis of 1-(2-(((S)-2-((((9H-fluoren-9-yl)methoxy)carbonyl)amino)-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

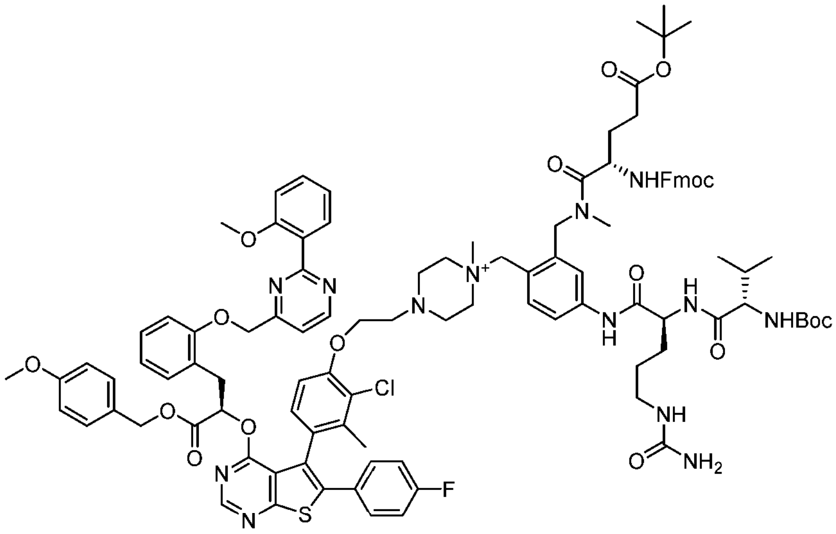

Following **GENERAL PROCEDURE** 4 with 5-(tert-butyl) 1-(2,5-dioxopyrrolidin-1-yl) (((9H-fluoren-9-yl)methoxy)carbonyl)-L-glutamate (39.0 mg, 0.075 mmol, 1.1 equiv.), 1-(2-(((S)-2-((((9H-fluoren-9-yl)methoxy)carbonyl)amino)-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy) ethyl)-1-methylpiperazin-1-ium was obtained. HRMS: M+= 1906.8101; Rt=3.03 min (5 min acidic method).

**Synthesis of 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

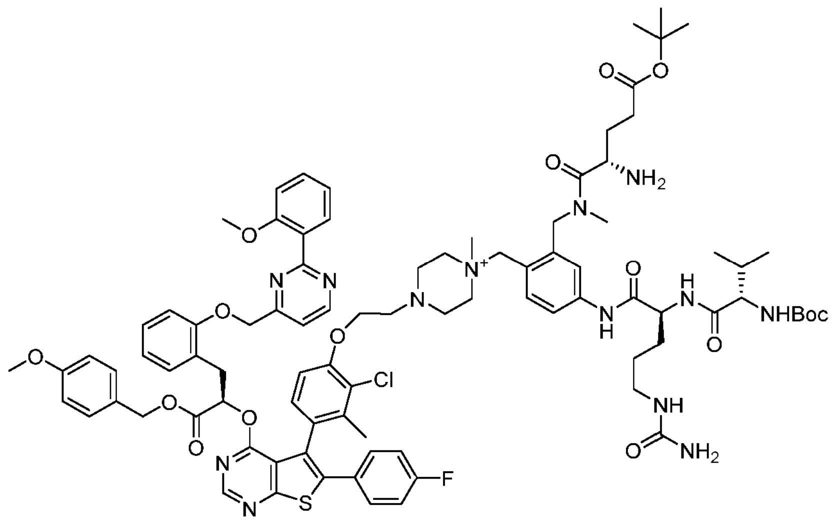

To 11-(2-(((S)-2-((((9H-fluoren-9-yl)methoxy)carbonyl)amino)-5-(tert-butoxy)-N-methyl-5-oxopentanamido) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (38.8 mg, 0.026 mmoles, 1.0 equiv.) dissolved in DMSO (2 mL) was added dimethylamine (0.192 mL, 0.384 mmoles, 20 equiv.). After standing for 4 hr, DMSO (2 mL) was added and the solution was purified by RP-HPLC ISCO gold chromatography (10-70% MeCN/H2O, 0.1% TFA modifier). Upon lyophilization, 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1684.4000; Rt=2.64 min (5 min acidic method).

## GENERAL PROCEDURE 5

**Synthesis of 1-(2-(((R)-2-((((9H-fluoren-9-yl)methoxy)carbonyl)amino)-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

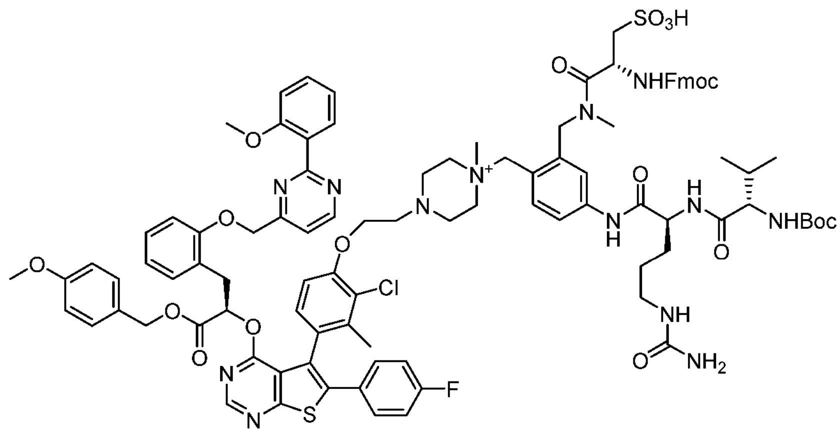

To (((9H-fluoren-9-yl)methoxy)carbonyl)(sulfo)-D-alanine (55.2 mg, 0.141 mmol, 1.3 eq) and 2-(3H-[1,2,3]triazolo[4,5-b]pyridin-3-yl)-1,1,3,3-tetramethylisouronium hexafluorophosphate(V) (41.3 mg, 0.109 mmoles, 1.0 equiv.) dissolved in DMF (2 mL) was added DIPEA (0.024 mL, 0.138 mmoles, 8.0 equiv.). After standing for 10 min, 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (200 mg, 0.109 mmol, 1.0 eq) was added. After standing for 2.5 hr, DMSO (4 mL) was added and the solution was purified by RP-HPLC ISCO gold chromatography (10-70% MeCN/H2O, 0.1% TFA modifier). Upon lyophilization, 1-(2-(((R)-2-((((9H-fluoren-9-yl)methoxy)carbonyl)amino)-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1872.7000; Rt=3.09 min (5 min acidic method).

**Synthesis of 1-(2-(((R)-2-amino-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

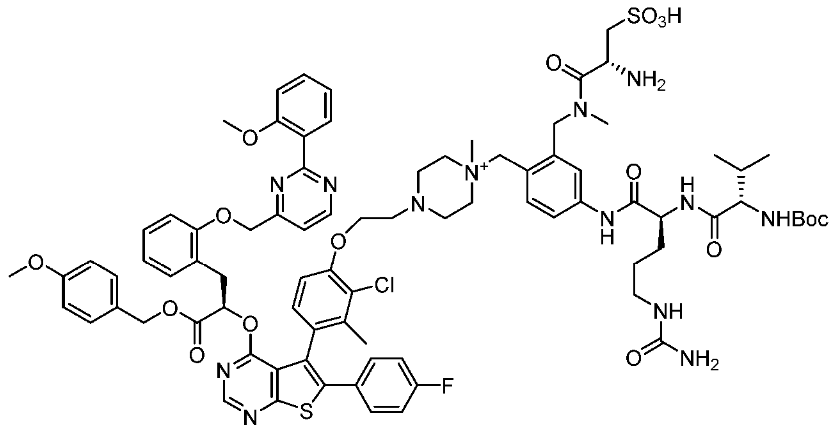

To 1-(2-(((R)-2-((((9H-fluoren-9-yl)methoxy)carbonyl)amino)-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (173 mg, 0.087 mmoles, 1.0 equiv.) dissolved in THF (2 mL) was added dimethylamine (0.870 mL, 1.740 mmoles, 20 equiv.). After standing for 5 hr, all volatiles were removed *in-vacuo.* The solid was triturated with diethyl ether. DMSO (2 mL) was added and the solution was purified by RP-HPLC ISCO gold chromatography (10-100% MeCN/H2O, 0.1% TFA modifier). Upon lyophilization, 1-(2-(((R)-2-amino-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1650.5800; Rt=2.71 min (5 min acidic method).

**Synthesis of 1-(2-(((R)-2-amino-N-methyl-3-sulfopropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L70-P1)**

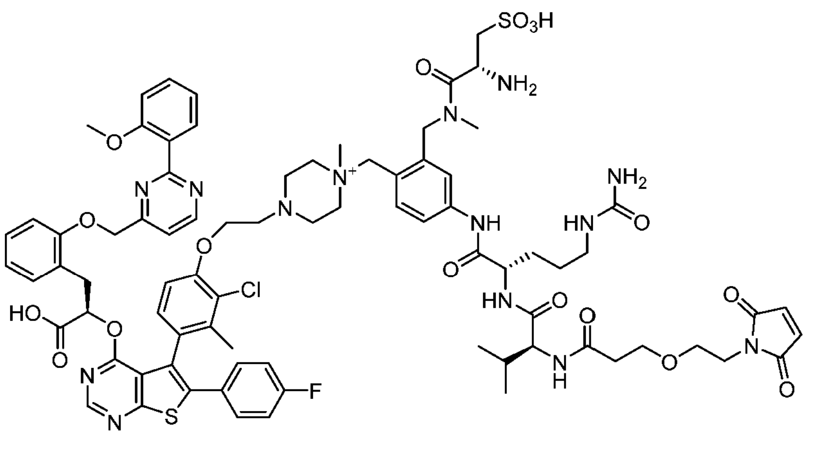

Following **GENERAL PROCEDURE** 3 with 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (145 mg, 0.088 mmol, 1 eq), 1-(2-(((R)-2-amino-N-methyl-3-sulfopropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 1625.5601; Rt=2.32 min (5 min acidic method).

**GENERAL PROCEDURE 6**

**Synthesis of 1-(2-(((S)-5-(tert-butoxy)-N-methyl-5-oxo-2-(2-sulfoacetamido)pentanamido) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

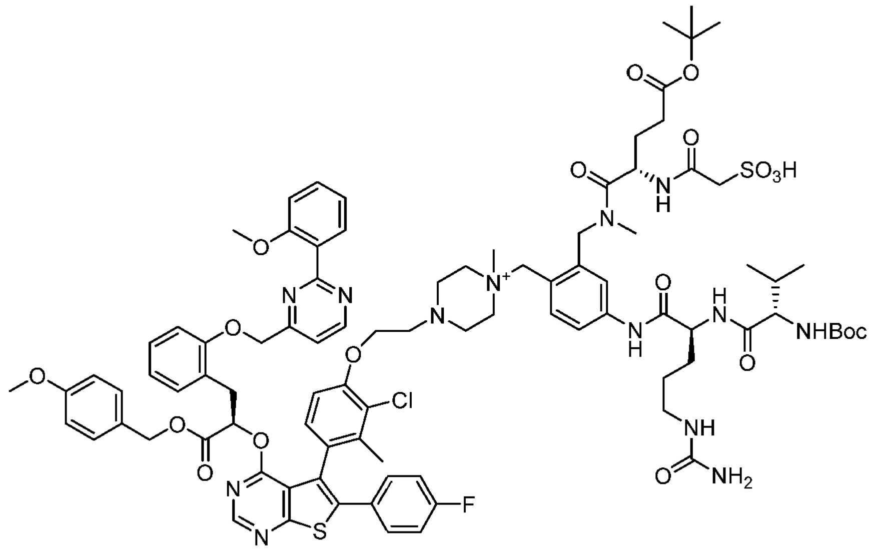

To 2-sulfoacetic acid (4.83 mg, 0.035 mmol, 2.0 equiv.) and 2-(3H-[1,2,3]triazolo[4,5-b]pyridin-3-yl)-1,1,3,3-tetramethylisouronium hexafluorophosphate(V) (9.85 mg, 0.026 mmoles, 1.5 equiv.) dissolved in DMF (1 mL) was added DIPEA (0.024 mL, 0.138 mmoles, 8.0 equiv.). After standing for 10 min, 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido) benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (29.1 mg, 0.017 mmoles, 1.0) was added. After standing for 45 min, DMSO (2 mL) was added and the solution was purified by RP-HPLC ISCO gold chromatography (10-70% MeCN/H2O, 0.1% TFA modifier). Upon lyophilization, 1-(2-(((S)-5-(tert-butoxy)-N-methyl-5-oxo-2-(2-sulfoacetamido)pentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d] pyrimidin-5-yl)-3-methylphenoxy)ethyl)-1-methylpiperazin-1-ium was obtained. HRMS: M+=1806.7000; Rt=3.10 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-4-carboxy-N-methyl-2-(2-sulfoacetamido)butanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L71-P1)**

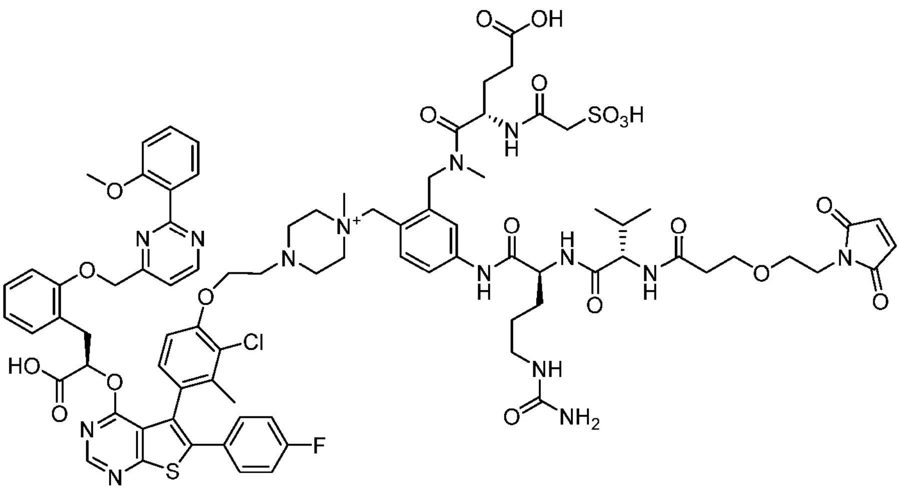

Following **GENERAL PROCEDURE** 3 with 1-(2-(((S)-5-(tert-butoxy)-N-methyl-5-oxo-2-(2-sulfoacetamido)pentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (23.7 mg, 0.011 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-4-carboxy-N-methyl-2-(2-sulfoacetamido)butanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1725.5900; Rt=2.39 min (5 min acidic method).

**Synthesis of 1-(2-((S)-40-(3-(tert-butoxy)-3-oxopropyl)-42-methyl-38,41-dioxo-2,5,8,11,14,17,20,23,26,29,32,35-dodecaoxa-39,42-diazatritetracontan-43-yl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

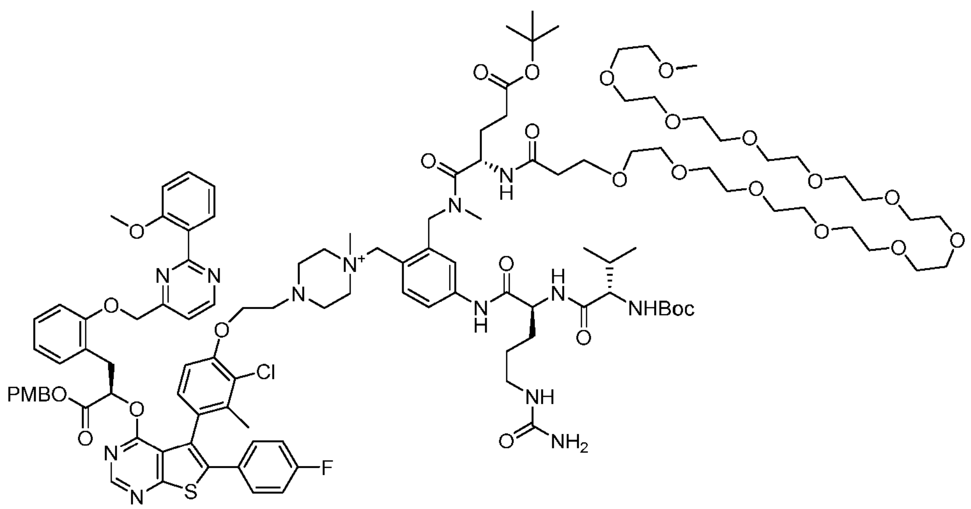

Following **GENERAL PROCEDURE** 4 with 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 0.018 mmol, 1.0 eq) and Mal-PEG12-NHS Ester (18.31 mg, 0.027 mmol, 1.5 eq), 1-(2-((S)-40-(3-(tert-butoxy)-3-oxopropyl)-42-methyl-38,41-dioxo-2,5,8,11,14,17,20,23,26,29,32,35-dodecaoxa-39,42-diazatritetracontan-43-yl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 2255.0400; Rt=2.97 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-((S)-40-(2-carboxyethyl)-42-methyl-38,41-dioxo-2,5,8,11,14,17,20,23,26,29,32,35-dodecaoxa-39,42-diazatritetracontan-43-yl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L72-P1)**

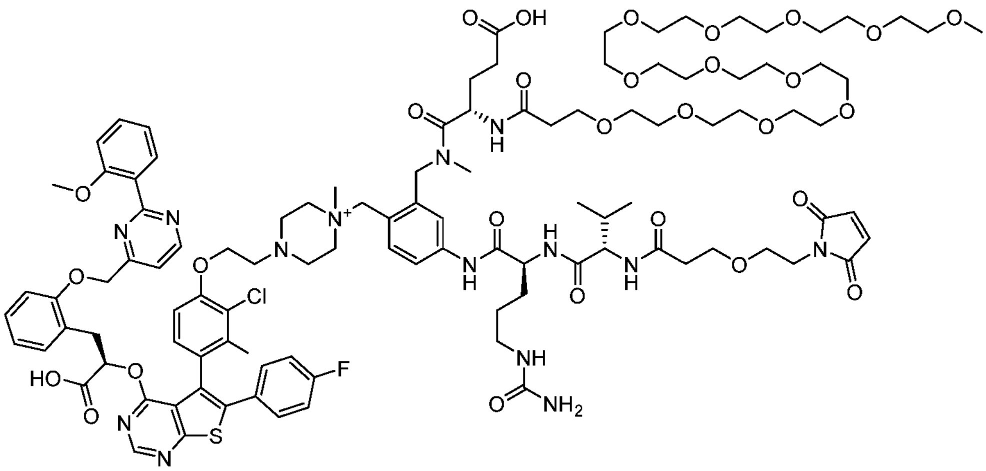

Following **GENERAL PROCEDURE 3** with 1-(2-((S)-40-(3-(tert-butoxy)-3-oxopropyl)-42-methyl-38,41-dioxo-2,5,8,11,14,17,20,23,26,29,32,35-dodecaoxa-39,42-diazatritetracontan-43-yl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20.1 mg, 8.91 μmmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-((S)-40-(2-carboxyethyl)-42-methyl-38,41-dioxo-2,5,8,11,14,17,20,23,26,29,32,35-dodecaoxa-39,42-diazatritetracontan-43-yl)-4-((S)-2-((S)-2-(3-(2-(2,5-dioxo-2,5-dihydro-1H-pyrrol-1-yl)ethoxy)propanamido)-3-**methylbutanamido)-5-ureidopentanamido)benzyl)-1-methylpiperazin-1-ium** was obtained. HRMS: [M]+= 2173.9199; Rt=2.40 min (5 min acidic method).

**Synthesis of 1-(2-((2-(bis(2-(tert-butoxy)-2-oxoethyl)amino)-N-methylacetamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

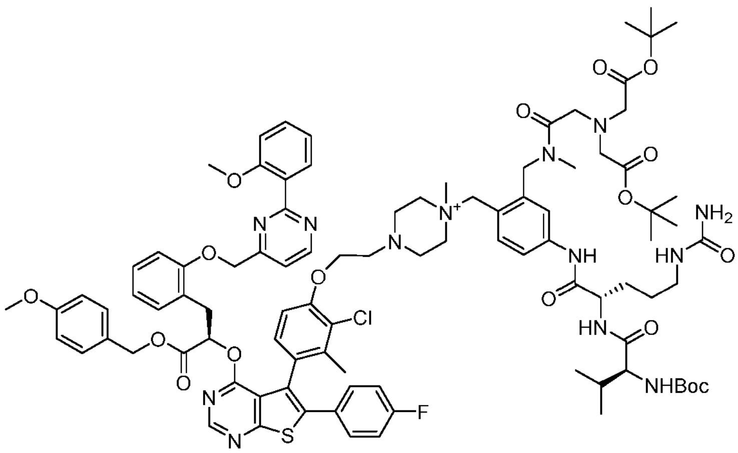

Following **GENERAL PROCEDURE 5** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (17.0 mg, 0.011 mmol, 1.0 eq) and bis(2-(tert-butoxy)-2-oxoethyl) glycine (10.97 mg, 0.017 mmol, 1.5 eq), 1-(2-((2-(bis(2-(tert-butoxy)-2-oxoethyl)amino)-N-methylacetamido) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1784.8000; Rt=3.31 min (5 min acidic method).

**Synthesis of 1-(2-((2-(bis(carboxymethyl)amino)-N-methylacetamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L67-P1)**

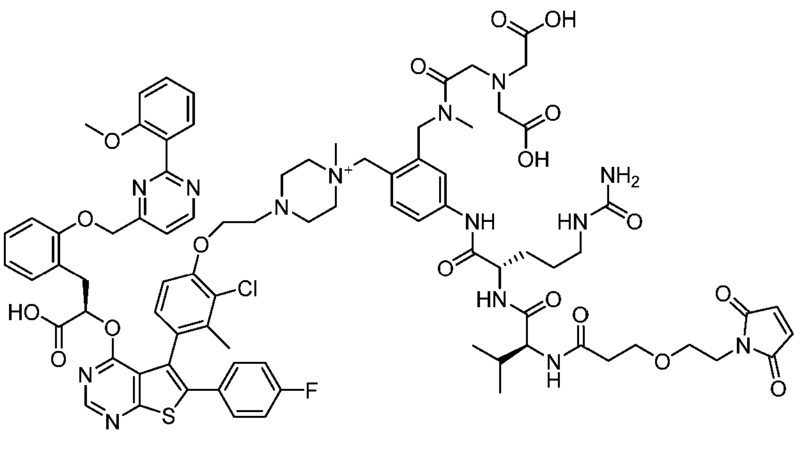

Following **GENERAL PROCEDURE 3** with 1-(2-((2-(bis(2-(tert-butoxy)-2-oxoethyl)amino)-N-methylacetamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (9.2 mg, 5.12 μmmol, 1 eq), 1-(2-((2-(bis(carboxymethyl)amino)-N-methylacetamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 1647.6100; Rt=2.28 min (5 min acidic method).

**General Procedure 7**

**Synthesis of 1-(2-(((S)-3-amino-4-(tert-butoxy)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

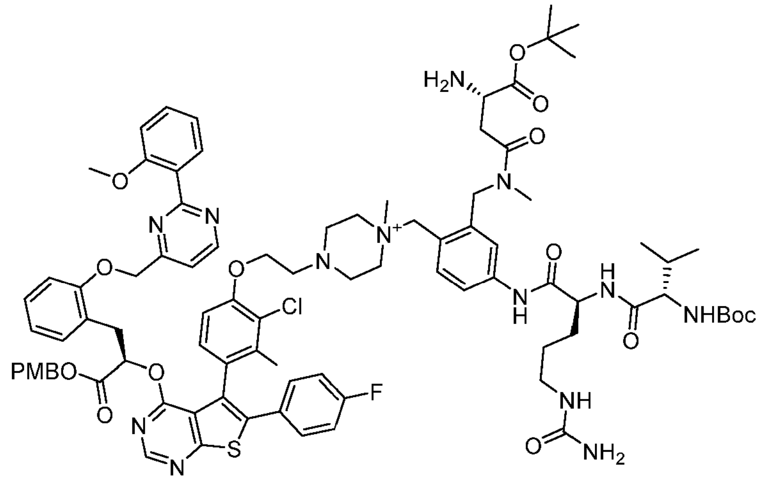

Reagents were used as DMF stock solution. To (S)-3-((((9H-fluoren-9-yl)methoxy)carbonyl)amino)-4-(tert-butoxy)-4-oxobutanoic acid (8.04 mg, 161 μL, 0.020 mmol, 1.2 equiv.) and 2-(3H-[1,2,3]triazolo[4,5-b]pyridin-3-yl)-1,1,3,3-tetramethylisouronium hexafluorophosphate(V) (6.81 mg, 681 μL, 0.018 mmoles, 1.1 equiv.) was added DIPEA (22.68 μL, 0.130 mmoles, 8.0 equiv.). After standing for 10 min, 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 353 μL, 0.016 mmoles, 1.0 equiv.) was added. After standing for 45 min, dimethyl amine (163 μl, 0.326 mmol) was added. After standing for 16 hours, DMSO (2 mL) was added and the solution was purified by RP-HPLC ISCO gold chromatography (10-100% MeCN/H2O, 0.1% TFA modifier). Upon lyophilization, 1-(2-(((S)-3-amino-4-(tert-butoxy)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1670.2300; Rt=2.69 min (5 min acidic method).

**Synthesis of 1-(2-(((S)-4-(tert-butoxy)-N-methyl-4-oxo-3-(2-sulfoacetamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

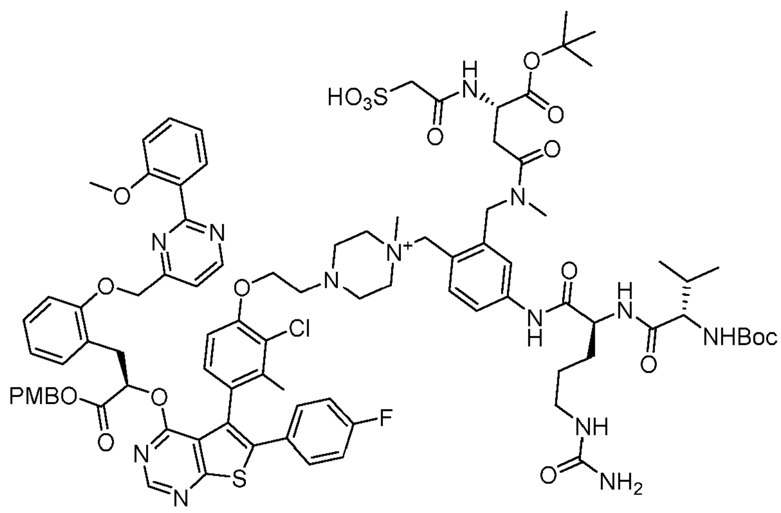

Following **GENERAL PROCEDURE 6** with 1-(2-(((S)-3-amino-4-(tert-butoxy)-N-methyl-4-oxobutanamido) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20.6 mg, 10.23 μmol, 1 eq), 1-(2-(((S)-4-(tert-butoxy)-N-methyl-4-oxo-3-(2-sulfoacetamido)butanamido) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1792.6899; Rt=3.17 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-N-methyl-3-(2-sulfoacetamido)propanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L79-P1)**

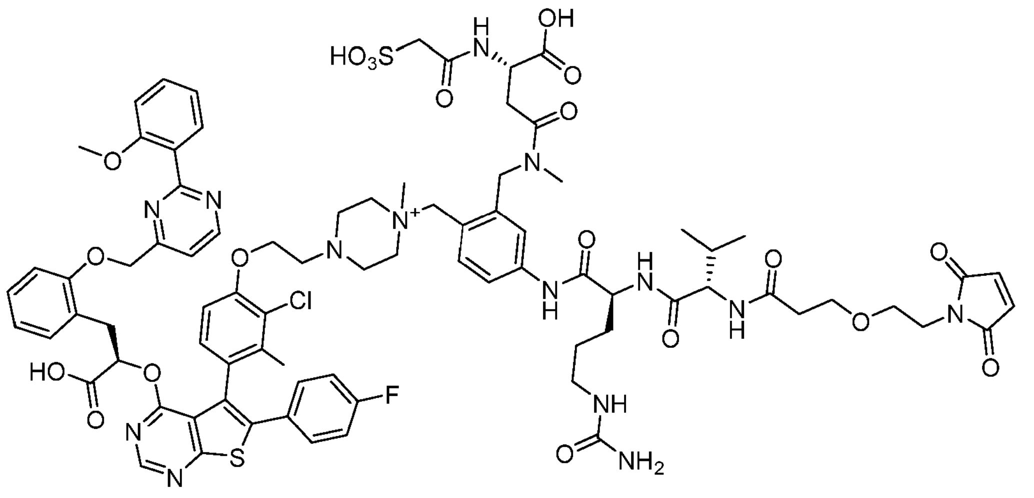

Following **GENERAL PROCEDURE** 3 with 1-(2-(((S)-4-(tert-butoxy)-N-methyl-4-oxo-3-(2-sulfoacetamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl) methoxy)phenyl)-1-oxopropan-2-yl)oxy)thieno[2,3-d]pyrimidin-5-yl)-3-methylphenoxy)ethyl)-1-methylpiperazin-1-ium (25.3 mg, 0.012 mmol, 1 eq), 4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-(2-(((S)-3-carboxy-N-methyl-3-(2-sulfoacetamido)propanamido)methyl)-4-((S)-2-((S)-2-(3-(2-(2,5-dioxo-2,5-dihydro-1 H-pyrrol-1-yl)ethoxy)propanamido)-3-methylbutanamido)-5-ureidopentanamido)benzyl)-1-methylpiperazin-1-ium was obtained. HRMS: M+= 1711.5699; Rt=2.48 min (5 min acidic method).

**Synthesis of 4-benzyl 1-(tert-butyl) ((3-(tert-butoxy)-3-oxopropoxy)carbonyl)-L-aspartate**

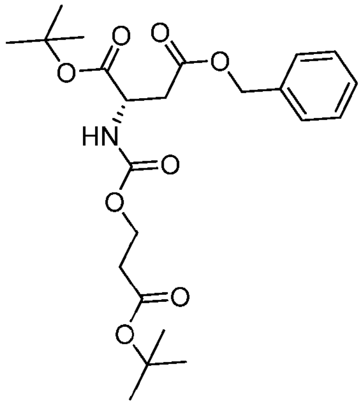

To tert-butyl 3-hydroxypropanoate (111 mg, 0.760 mmol, 1 equiv.) and bis(4-nitrophenyl) carbonate (289 mg, 0.950 mmol, 1.2 equiv.) dissolved in DMF (2 mL) was added DIPEA (0.221 mL, 1.267 mmoles, 2.0 equiv.). After standing for 1 hr, 4-benzyl 1-(tert-butyl) L-aspartate (200 mg, 0.633 mmol) was added. After standing for 16 hr, DMSO (4 mL) was added and the solution was purified by RP-HPLC ISCO gold chromatography (10-100% MeCN/H2O, 0.1% TFA modifier). Upon lyophilization, 4-benzyl 1-(tert-butyl) ((3-(tert-butoxy)-3-oxopropoxy)carbonyl)-L-aspartate was obtained. HRMS: [M+H]+=452.4; Rt=2.68 min (5 min acidic method).

**Synthesis of ((S)-4-(tert-butoxy)-3-(((3-(tert-butoxy)-3-oxopropoxy)carbonyl)amino)-4-oxobutanoic acid**

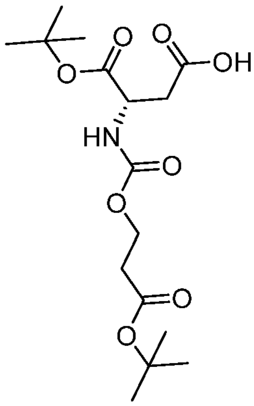

To 4-benzyl 1-(tert-butyl) ((3-(tert-butoxy)-3-oxopropoxy)carbonyl)-L-aspartate (52.7 mg, 0.117 mmol) dissolved in MeOH (2 mL) was added Palladium hydroxide (8.20 mg, 0.012 mmol, 0.1 equiv.). The reaction atmosphere was swtiched to hygrogen. After stirring for 16 hr, the reaction mixture was filtered through a celite pad. The filtrate was removed *in-vacuo* to obtain, 4-benzyl 1-(tert-butyl) ((3-(tert-butoxy)-3-oxopropoxy)carbonyl)-L-aspartate.

**Synthesis of 1-(2-((S)-5-(tert-butoxycarbonyl)-2,13,13-trimethyl-3,7,11-trioxo-8,12-dioxa-2,6-diazatetradecyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

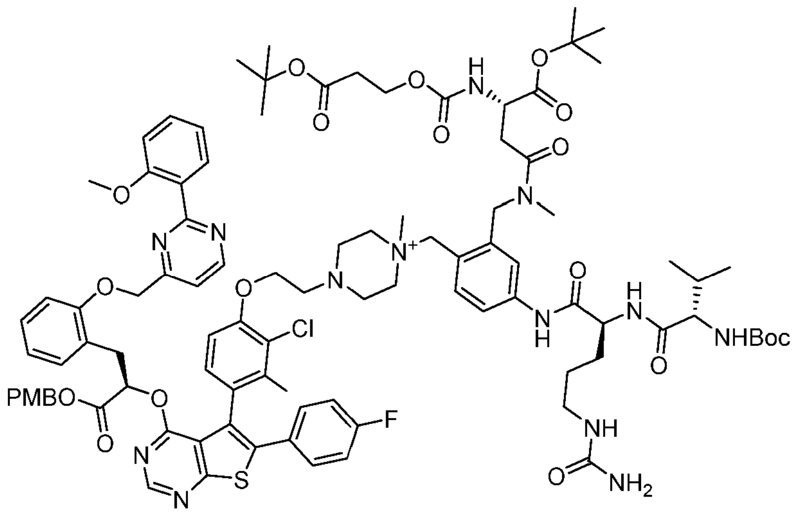

Following **GENERAL PROCEDURE** 7 with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20 mg, 235 μL, 0.011 mmol, 1 eq) and 4-benzyl 1-(tert-butyl) ((3-(tert-butoxy)-3-oxopropoxy)carbonyl)-L-aspartate (7.84 mg, 204 μL, 0.022 mmol, 2 equiv.), 1-(2-((S)-5-(tert-butoxycarbonyl)-2,13,13-trimethyl-3,7,11-trioxo-8,12-dioxa-2,6-diazatetradecyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1842.8000; Rt=3.23 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-3-(((2-carboxyethoxy)carbonyl)amino)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L102-P1)**

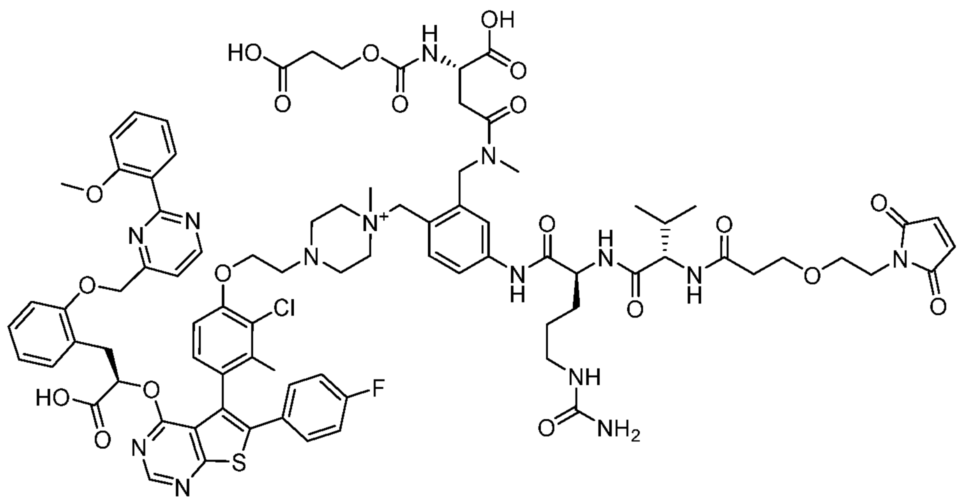

Following **GENERAL PROCEDURE** 3 with 1-(2-((S)-5-(tert-butoxycarbonyl)-2,13,13-trimethyl-3,7,11-trioxo-8,12-dioxa-2,6-diazatetradecyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (17.6 mg, 0.008 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-3-(((2-carboxyethoxy)carbonyl)amino)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1705.6200; Rt=2.41 min (5 min acidic method).

**Synthesis of tert-butyl N-(((9H-fluoren-9-yl)methoxy)carbonyl)-O-((4-nitrophenoxy)carbonyl)-L-serinate**

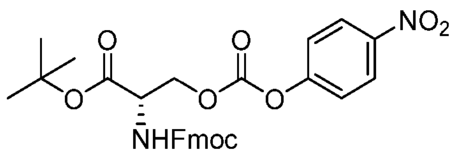

To tert-butyl (((9H-fluoren-9-yl)methoxy)carbonyl)-L-serinate (300 mg, 0.782 mmol, 1 equiv.) and bis(4-nitrophenyl) carbonate (357 mg, 1.174 mmol, 1.5 equiv.) dissolved in DMF (2 mL) was added DIPEA (0.136 mL, 0.782 mmoles, 1.0 equiv.). After standing for 16 hr, DMSO (4 mL) was added and the solution was purified by RP-HPLC ISCO gold chromatography (10-100% MeCN/H2O, 0.1% TFA modifier). Upon lyophilization, tert-butyl N-(((9H-fluoren-9-yl)methoxy) carbonyl)-O-((4-nitrophenoxy)carbonyl)-L-serinate was obtained. HRMS: M+= 566.4; Rt=3.01 min (5 min acidic method).

**Synthesis of 1-(2-(((((S)-2-amino-3-(tert-butoxy)-3-oxopropoxy)carbonyl)(methyl)amino) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

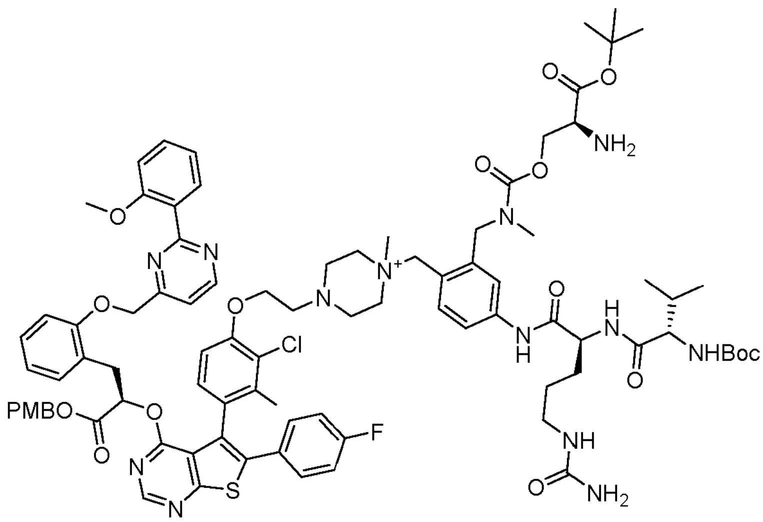

To tert-butyl N-(((9H-fluoren-9-yl)methoxy)carbonyl)-O-((4-nitrophenoxy)carbonyl)-L-serinate (7.14 mg, 143 μL, 0.013mmol, 1.2 equiv.) and 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl) oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20 mg, 235 μL, 0.011 mmol, 1.0 equiv.) was added DIPEA (15.1 μL, 0.087 mmoles, 8.0 equiv.). After standing for 16 hr, Dimethyl amine (109 μl, 0.217 mmol, 20 equiv.) was added. The reaction was stirred at RT for 1 hr. DMSO (4 mL) was added and the solution was purified by RP-HPLC ISCO gold chromatography (10-100% MeCN/H2O, 0.1% TFA modifier). Upon lyophilization, 1-(2-(((((S)-2-amino-3-(tert-butoxy)-3-oxopropoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1686.7200; Rt=2.71 min (5 min acidic method).

**Synthesis 1-(2-(((((S)-3-(tert-butoxy)-3-oxo-2-(2-sulfoacetamido)propoxy)carbonyl)(methyl)amino) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

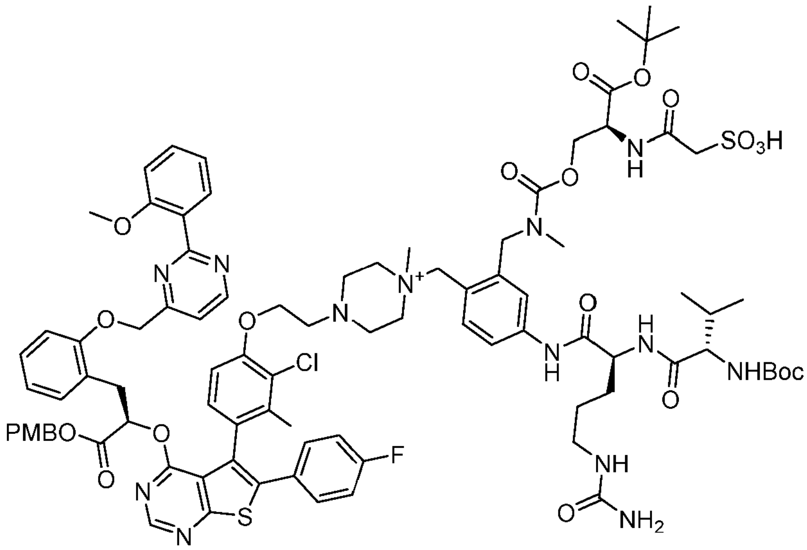

Following **GENERAL PROCEDURE** 6 with 1-(2-(((((S)-2-amino-3-(tert-butoxy)-3-oxopropoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (22.9 mg, 0.011 mmol, 1 eq), 1-(2-(((((S)-3-(tert-butoxy)-3-oxo-2-(2-sulfoacetamido)propoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1808.6899; Rt=3.18 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((S)-2-carboxy-2-(2-sulfoacetamido)ethoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L77-P1)**

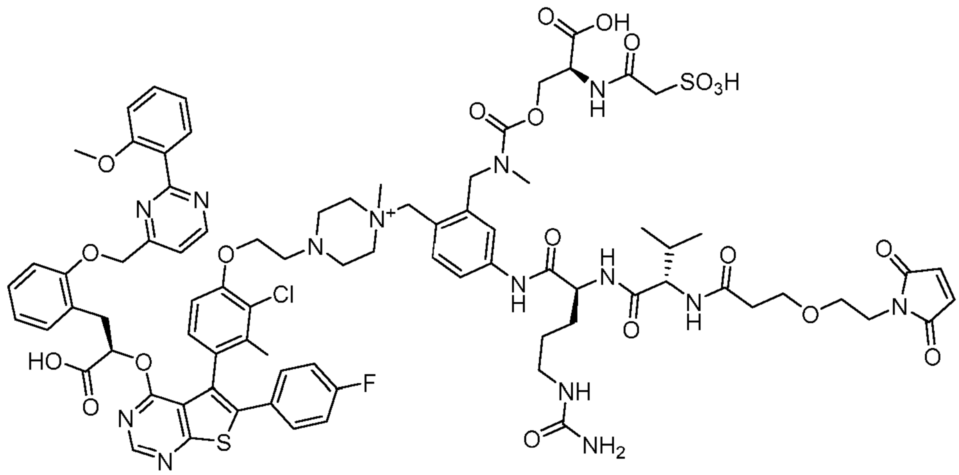

Following **GENERAL PROCEDURE 3** with 1-(2-(((((S)-2-amino-3-(tert-butoxy)-3-oxopropoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (15.3 mg, 7.11 μmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((S)-2-carboxy-2-(2-sulfoacetamido)ethoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1727.5800; Rt=2.47 min (5 min acidic method).

**Synthesis of 4-benzyl 1-(tert-butyl) (4-(diethoxyphosphoryl)butanoyl)-L-aspartate**

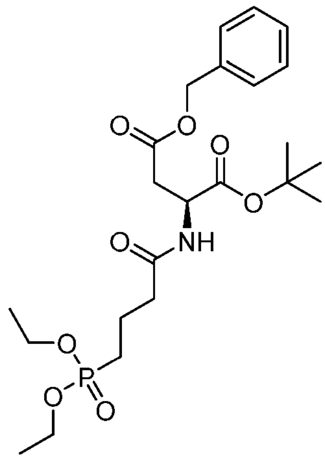

To 4-benzyl 1-(tert-butyl) L-aspartate (200 mg, 0.633 mmol, 1.0 equiv.) and 4-(diethoxyphosphoryl)butanoic acid (213 mg, 0.950 mmol, 1.5 equiv.) dissolved in DMF (2 mL) was added dicyclohexylmethanediimine (157 mg, 0.760 mmol, 1.2 equiv.), 1H-[1,2,3]triazolo[4,5-b]pyridin-1-ol hydrate (146 mg, 0.950 mmol, 1.5 equiv.) and DIPEA (0.110 mL, 0.633 mmol, 1.0 equiv.). After standing for 16 hr, DMSO (4 mL) was added and the solution was purified by RP-HPLC ISCO gold chromatography (10-100% MeCN/H2O, 0.1% TFA modifier). Upon lyophilization, 4-benzyl 1-(tert-butyl) (4-(diethoxyphosphoryl)butanoyl)-L-aspartate was obtained. HRMS: [M+H]+=486.4; Rt=2.15 min (5 min acidic method).

**Synthesis of (S)-4-(tert-butoxy)-3-(4-(diethoxyphosphoryl)butanamido)-4-oxobutanoic acid**

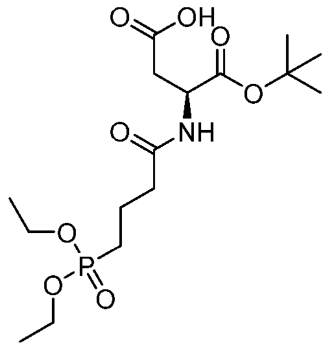

To 4-benzyl 1-(tert-butyl) (4-(diethoxyphosphoryl)butanoyl)-L-aspartate (YUBI5-040-EXP082-001 (100 mg, 0.206 mmol, 1.0 equiv.) dissolved in MeOH (2 mL) was added Palladium hydroxide (14.46 mg, 0.021 mmol, 0.1 equiv.). The reaction atmosphere was switched to hydrogen. After stirring for 16 hr, the reaction mixture was filtered through a celite pad. The filtrate was removed *in-vacuo* to obtain (S)-4-(tert-butoxy)-3-(4-(diethoxyphosphoryl)butanamido)-4-oxobutanoic acid. HRMS: [M+H]+= 396.3; Rt=1.35 min (5 min acidic method).

**Synthesis of 1-(2-(((S)-4-(tert-butoxy)-3-(4-(diethoxyphosphoryl)butanamido)-N-methyl-4-oxobutanamido) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

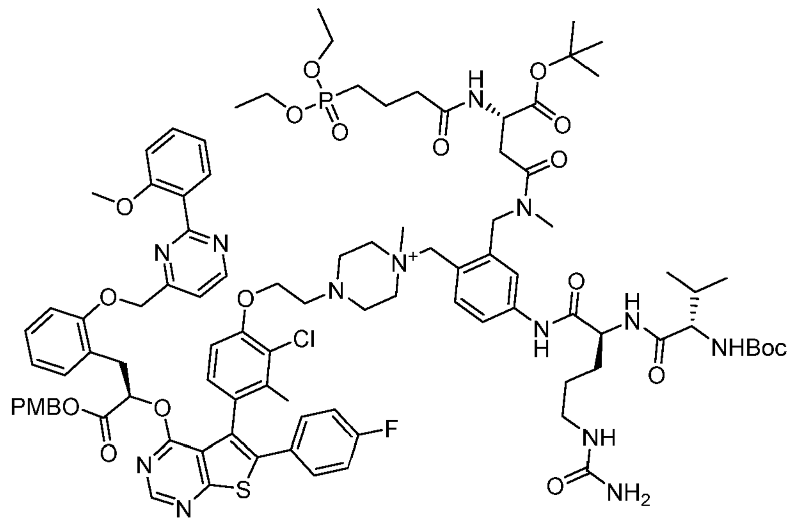

Following **GENERAL PROCEDURE 7** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 353 µL, 0.016 mmol, 1 eq) and (S)-4-(tert-butoxy)-3-(4-(diethoxyphosphoryl)butanamido)-4-oxobutanoic acid (12.87 mg, 161 µl, 0.033 mmol, 2 equiv.), 1-(2-(((S)-4-(tert-butoxy)-3-(4-(diethoxyphosphoryl)butanamido)-N-methyl-4-oxobutanamido) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. MS: M/2+= 940.3; Rt=2.60 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-3-(4-(diethoxyphosphoryl)butanamido)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L103-P1)**

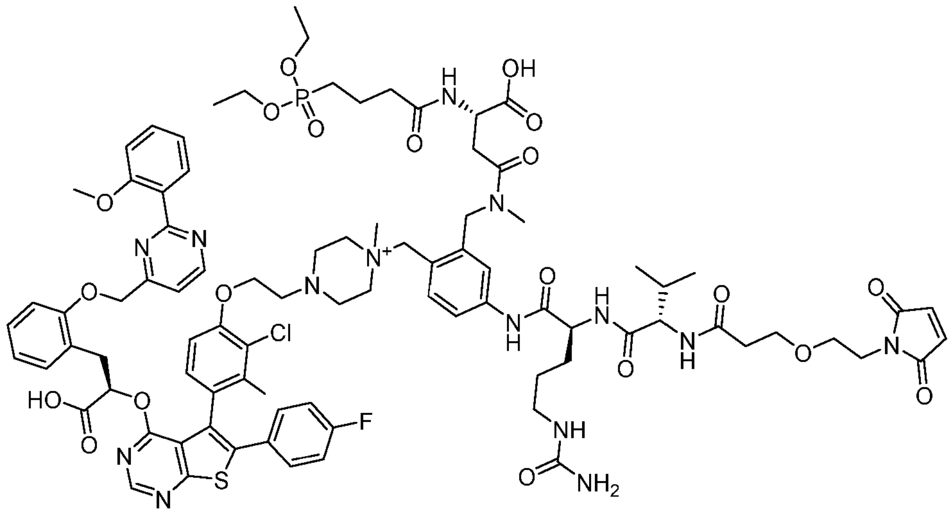

Following **GENERAL PROCEDURE 3** with 1-(2-(((S)-4-(tert-butoxy)-3-(4-(diethoxyphosphoryl)butanamido)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20 mg, 0.009 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-3-(4-(diethoxyphosphoryl)butanamido)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1795.6700; Rt=2.44 min (5 min acidic method).

**Synthesis of 1-(4-((S)-2-((S)-2-((tert-butoxycarbonyl)amino)-3-methylbutanamido)-5-ureidopentanamido)-2-(((S)-2,6-diamino-N-methylhexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

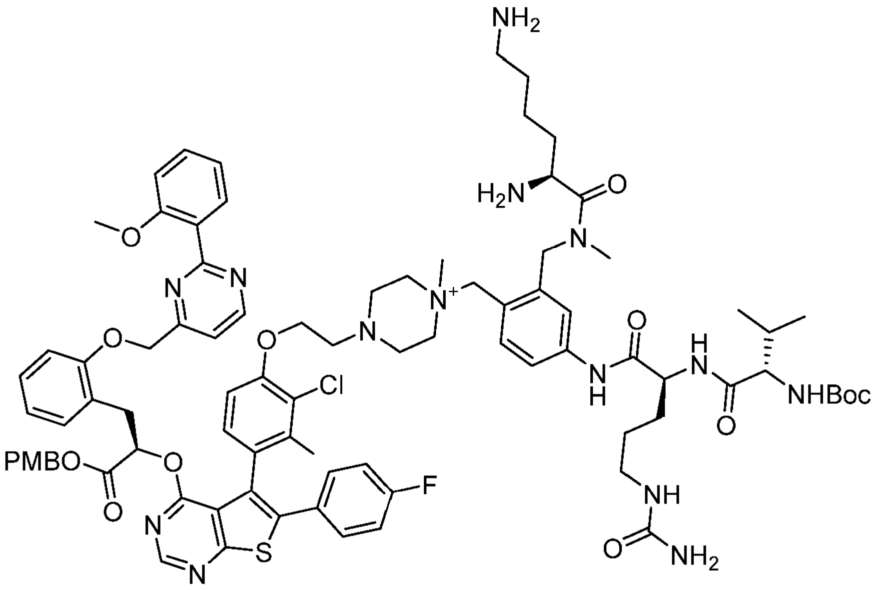

Following **GENERAL PROCEDURE** 7 with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20 mg, 0.011 mmol, 1 eq) and (S)-2,5-bis((((9H-fluoren-9-yl)methoxy)carbonyl)amino)pentanoic acid (7.51 mg, 0.013 mmol, 1.2 eq), 1-(4-((S)-2-((S)-2-((tert-butoxycarbonyl)amino)-3-methylbutanamido)-5-ureidopentanamido)-2-(((S)-2,6-diamino-N-methylhexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1627.4000; Rt=2.48 min (5 min acidic method).

**Synthesis of 1-(4-((S)-2-((S)-2-((tert-butoxycarbonyl)amino)-3-methylbutanamido)-5-ureidopentanamido)-2-(((S)-N-methyl-2,6-bis(2-sulfoacetamido)hexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

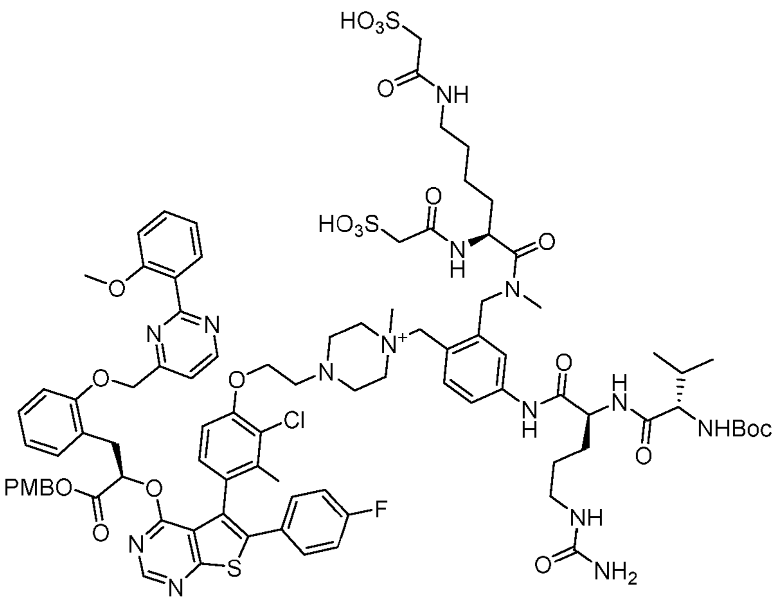

Following **GENERAL PROCEDURE 6** with 1-(4-((S)-2-((S)-2-((tert-butoxycarbonyl)amino)-3-methylbutanamido)-5-ureidopentanamido)-2-(((S)-2,6-diamino-N-methylhexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (9.7 mg, 0.0049 mmol, 1 eq), 1-(4-((S)-2-((S)-2-((tert-butoxycarbonyl)amino)-3-methylbutanamido)-5-ureidopentanamido)-2-(((S)-N-methyl-2,6-bis(2-sulfoacetamido)hexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1871.6700; Rt=3.**02 min (5 min acidic method).**

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((S)-N-methyl-2,6-bis(2-sulfoacetamido)hexanamido)methyl)benzyl)-1-methylpiperazin-1-ium (L78-P1)**

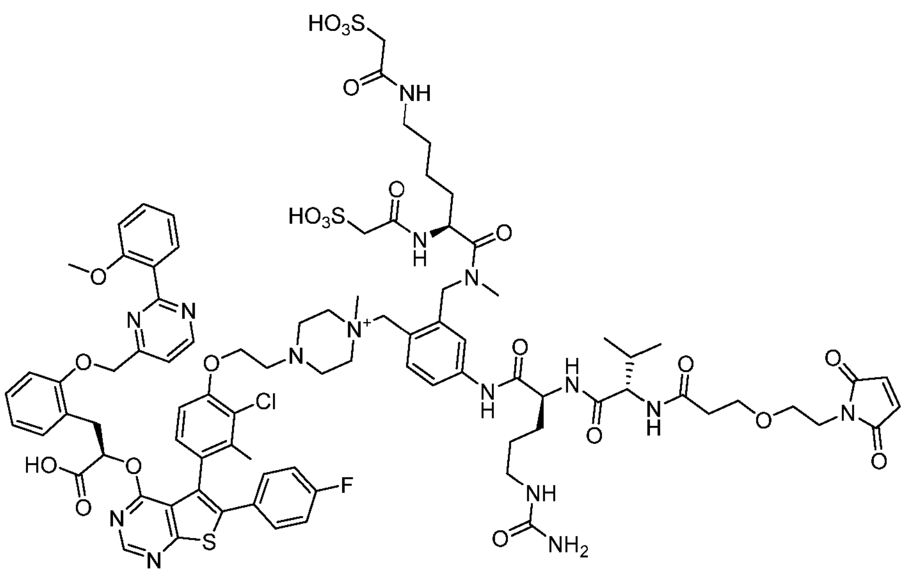

Following **GENERAL PROCEDURE 3** with -(4-((8)-2-((8)-2-((tert-butoxycarbonyl)amino)-3-methylbutanamido)-5-ureidopentanamido)-2-(((S)-N-methyl-2,6-bis(2-sulfoacetamido)hexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (15 mg, 7.55 μmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((S)-N-methyl-2,6-bis(2-sulfoacetamido)hexanamido)methyl)benzyl)-1-methylpiperazin-1-ium was obtained. HRMS: M+= 1846.6000; Rt=2.49 min (5 min acidic method).

**Synthesis of 4-benzyl 1-(tert-butyl) ((2S,3S,4S,5R,6S)-3,4,5,6-tetraacetoxytetrahydro-2H-pyran-2-carbonyl)-L-aspartate**

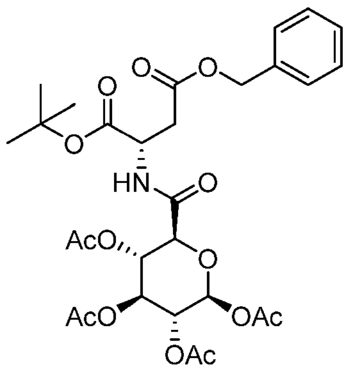

To (2S,3S,4S,5R,6S)-3,4,5,6-tetraacetoxytetrahydro-2H-pyran-2-carboxylic acid (344 mg, 0.950 mmol) and 4-benzyl 1-(tert-butyl) L-aspartate (300 mg, 0.950 mmol) dissolved in DMF (3.2 mL) was added DIPEA (0.165 mL, 0.950 mmol, 1.0 equiv.), 1H-[1,2,3]triazolo[4,5-b]pyridin-1-ol hydrate (154 mg, 0.997 mmol, 1.05 equiv.) and 3-(((ethylimino) methylene)amino)-N,N-dimethylpropan-1-amine hydrochloride (191 mg, 0.997 mmol, 1.05 equiv.) were added. After standing for 16 hr, DMSO (4 mL) was added and the solution was purified by RP-HPLC ISCO gold chromatography (10-70% MeCN/H2O, 0.1% TFA modifier). Upon lyophilization, 4-benzyl 1-(tert-butyl) ((2S,3S,4S,5R,6S)-3,4,5,6-tetraacetoxytetrahydro-2H-pyran-2-carbonyl)-L-aspartate was obtained. HRMS: M+=641.5; Rt=2.55 min (5 min acidic method).

**Synthesis of (S)-4-(tert-butoxy)-4-oxo-3-((2S,3S,4S,5R,6S)-3,4,5,6-tetraacetoxytetrahydro-2H-pyran-2-carboxamido)butanoic acid**

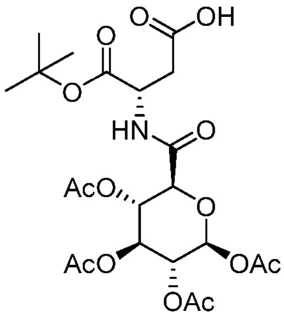

To 4-benzyl 1-(tert-butyl) ((2S,3S,4S,5R,6S)-3,4,5,6-tetraacetoxytetrahydro-2H-pyran-2-carbonyl)-L-aspartate (100 mg, 0.160 mmol, 1.0 eq) dissolved in MeOH (2 mL) was added Palladium hydroxide (11.26 mg, 0.016 mmol, 0.1 equiv.). The reaction atmosphere was swtiched to hygrogen. After stirring for 16 hr, the reaction mixture was filtered through a celite pad. The filtrate was removed in-vacuo to obtain (S)-4-(tert-butoxy)-4-oxo-3-((2S,3S,4S,5R,6S)-3,4,5,6-tetraacetoxytetrahydro-2H-pyran-2-carboxamido)butanoic acid. HRMS: [M-H]-= 532.3, Rt=1.71 min (5 min acidic method).

**Synthesis of 1-(2-(((S)-4-(tert-butoxy)-N-methyl-4-oxo-3-((2R,3R,4R,5S,6R)-3,4,5,6-tetraacetoxytetrahydro-2H-pyran-2-carboxamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-**ium

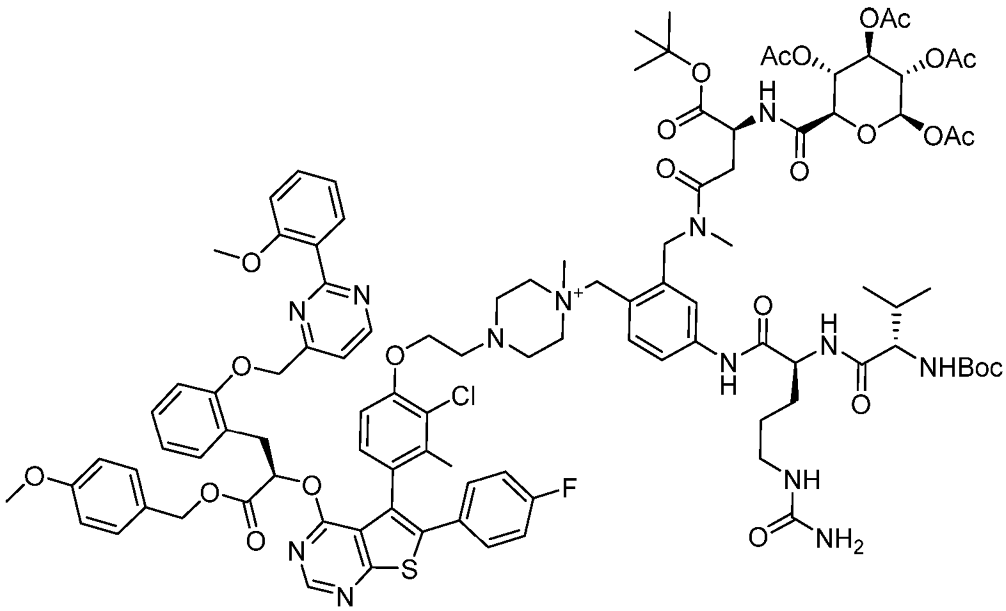

Following **GENERAL PROCEDURE 7** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 0.016 mmol, 1.0 eq) and (S)-4-(tert-butoxy)-4-oxo-3-((2S,3S,4S,5R,6S)-3,4,5,6-tetraacetoxytetrahydro-2H-pyran-2-carboxamido)butanoic acid (12.2 mg, 968 µL, 0.023 mmol, 1.4 eq), 1-(2-(((S)-4-(tert-butoxy)-N-methyl-4-oxo-3-((2R,3R,4R,5S,6R)-3,4,5,6-tetraacetoxytetrahydro-2H-pyran-2-carboxamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=2014.7800; Rt=3.21 min (5 min acidic method).

**Synthesis of 1-(4-((S)-2-((S)-2-amino-3-methylbutanamido)-5-ureidopentanamido)-2-(((S)-3-carboxy-N-methyl-3-((2R,3R,4R,5S,6R)-3,4,5,6-tetraacetoxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

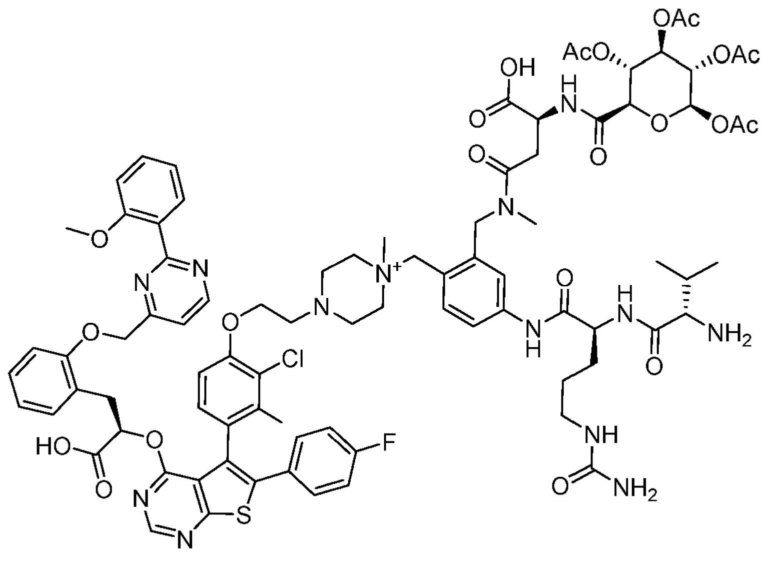

To 1-(2-(((S)-4-(tert-butoxy)-N-methyl-4-oxo-3-((2R,3R,4R,5S,6R)-3,4,5,6-tetraacetoxytetrahydro-2H-pyran-2-carboxamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (22.7 mg, 0.009 mmoles, 1.0 equiv.) dissolved in DCM (32mL) was added TFA (0.67 mL). After stirring for 45 min, the solvent was removed *in-vacuo* to obtain 1-(4-((S)-2-((S)-2-amino-3-methylbutanamido)-5-ureidopentanamido)-2-(((S)-3-carboxy-N-methyl-3-((2R,3R,4R,5S,6R)-3,4,5,6-tetraacetoxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium. HRMS: M+=1738.6200; Rt=2.30 min (5 min acidic method).

**Synthesis of 1-(4-((S)-2-((S)-2-amino-3-methylbutanamido)-5-ureidopentanamido)-2-(((S)-3-carboxy-N-methyl-3-((2R,3R,4R,5S,6S)-3,4,5,6-tetrahydroxytetrahydro-2H-pyran-2-carboxamido)propanamido)methyl) benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

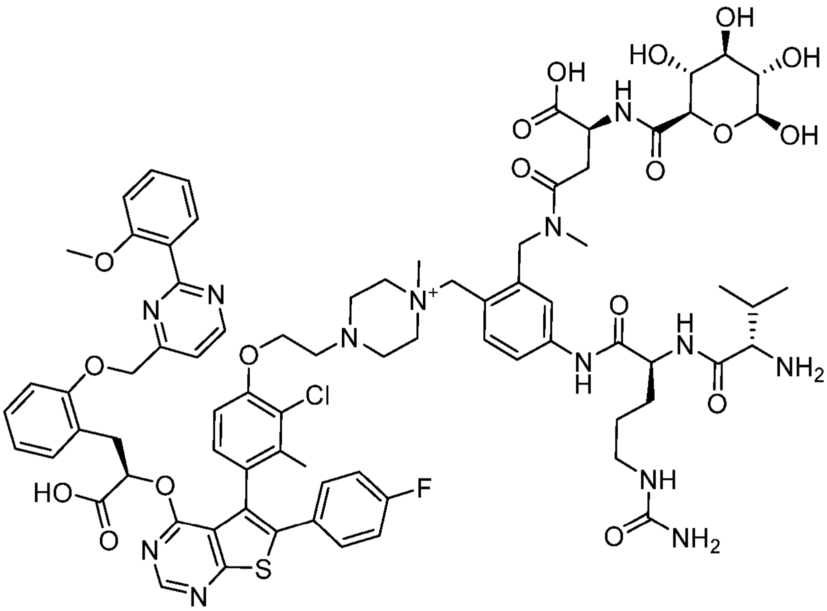

To 1-(4-((S)-2-((S)-2-amino-3-methylbutanamido)-5-ureidopentanamido)-2-(((S)-3-carboxy-N-methyl-3-((2R,3R,4R,5S,6R)-3,4,5,6-tetraacetoxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-methylpiperazin-1-ium (22.7 mg, 0.009 mmoles, 1.0 equiv.) dissolved in THF (1 mL) and MeOH (1 mL) was added lithium hydroxide (5.04 mg, 0.120 mmol, 10 equiv.). After stirring for 2 hour, the solvent was removed *in-vacuo.* Water (1 mL), TFA (0.2 mL), MeCN (1 mL) and DMSO (4 mL) were added and the solution was purified by RP-HPLC ISCO gold chromatography (10-70% MeCN/H2O, 0.1% TFA modifier). Upon lyophilization, 1-(4-((S)-2-((S)-2-amino-3-methylbutanamido)-5-ureidopentanamido)-2-(((S)-3-carboxy-N-methyl-3-((2R,3R,4R,5S,6S)-3,4,5,6-tetrahydroxytetrahydro-2H-pyran-2-carboxamido)propanamido)methylbenzyl)-4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-methylpiperazin-1-ium was obtained. HRMS: M+=1570.5900; Rt=2.02 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-N-methyl-3-((2R,3R,4R,5S,6S)-3,4,5,6-tetrahydroxytetrahydro-2H-pyran-2-carboxamido)propanamido) methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L68-P1)**

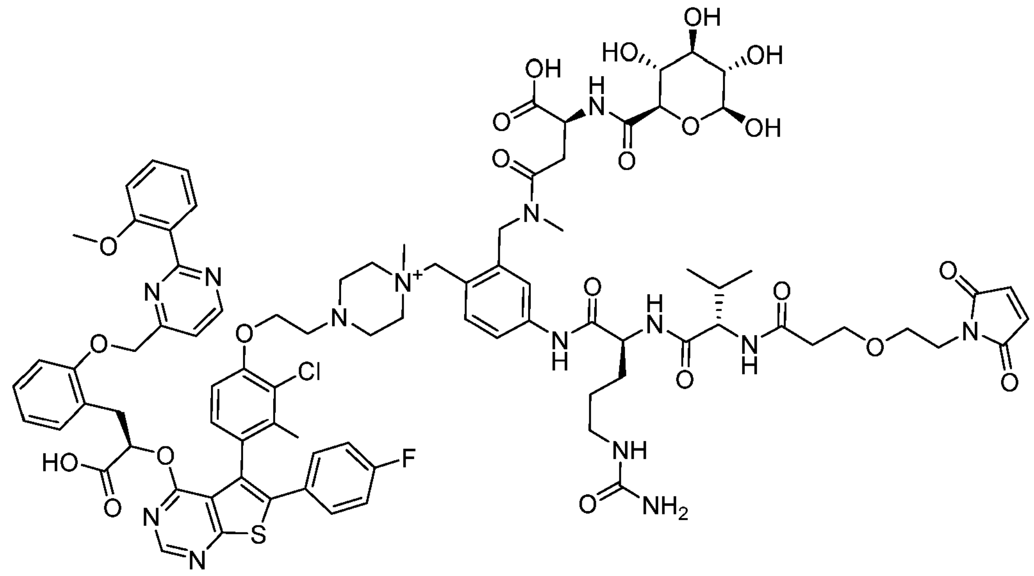

To 1-(4-((S)-2-((S)-2-amino-3-methylbutanamido)-5-ureidopentanamido)-2-(((S)-3-carboxy-N-methyl-3-((2R,3R,4R,5S,6S)-3,4,5,6-tetrahydroxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-methylpiperazin-1-ium (10.8 mg, 0.0056 mmoles, 1.0 equiv.) and 2,5-dioxopyrrolidin-1-yl 3-(2-(2,5-dioxo-2,5-dihydro-1 H-pyrrol-1-yl)ethoxy)propanoate (5.25 mg, 0.017 mmol, 3.5 equiv.) dissolved in DMF (1 mL) was added DIPEA (7.86 μL, 0.045 mmol, 8 equiv.). After standing for 1.5 hour, DMSO (2mL) were added and the solution was purified by RP-HPLC ISCO gold chromatography (10-70% MeCN/H2O, 0.1% TFA modifier). Upon lyophilization, 4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-(2-(((S)-3-carboxy-N-methyl-3-((2R,3R,4R,5S,6S)-3,4,5,6-tetrahydroxytetrahydro-2H-pyran-2-carboxamido)propanamido) methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+=1765.6500; Rt=2.31 min (5 min acidic method).

**Synthesis of 4-benzyl 1-(tert-butyl) ((((3R,4S,5S,6S)-3,4,5-triacetoxy-6-(methoxycarbonyl)tetrahydro-2H-pyran-2-yl)oxy)carbonyl)-L-aspartate**

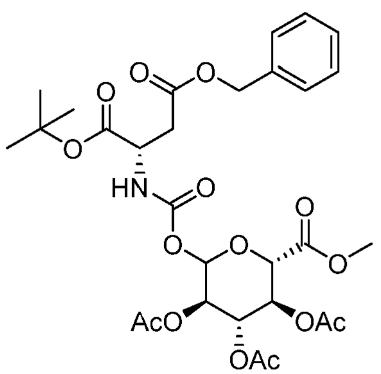

To ((3R,4S,5S,6S)-2-hydroxy-6-(methoxycarbonyl)tetrahydro-2H-pyran-3,4,5-triyl triacetate (254 mg, 0.760 mmol, 1.2 equiv.) and bis(4-nitrophenyl) carbonate (289 mg, 0.950 mmol, 1.5 equiv.) dissolved in DMF (2 mL) was added DIPEA (0.221 mL, 1.267 mmol, 2.0 equiv.). After standing for 1 hr, 4-benzyl 1-(tert-butyl) L-aspartate (200 mg, 0.633 mmol, 1.0 equiv.) was added. After standing for 16 hr, DMSO (6 mL) was added and the solution was purified by RP-HPLC ISCO gold chromatography (10-70% MeCN/H2O, 0.1% TFA modifier). Upon lyophilization, 4-benzyl 1-(tert-butyl) ((((3R,4S,5S,6S)-3,4,5-triacetoxy-6-(methoxycarbonyl)tetrahydro-2H-pyran-2-yl)oxy)carbonyl)-L-aspartate was obtained. HRMS: [M-H]-= 638.4; Rt=2.61 min (5 min acidic method).

**Synthesis of (3S)-4-(tert-butoxy)-4-oxo-3-(((((3R,4S,5S,6S)-3,4,5-triacetoxy-6-(methoxycarbonyl)tetrahydro-2H-pyran-2-yl)oxy)carbonyl)amino)butanoic acid**

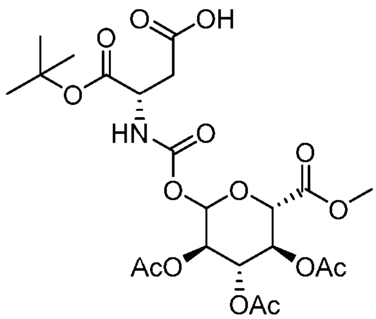

To 4-benzyl 1-(tert-butyl) ((((3R,4S,5S,6S)-3,4,5-triacetoxy-6-(methoxycarbonyl)tetrahydro-2H-pyran-2-yl)oxy) carbonyl)-L-aspartate (50 mg, 0.078 mmol, 1.0 eq) dissolved in MeOH (2 mL) was added Palladium hydroxide (5.49 mg, 7.82 μmol, 0.1 equiv.). The reaction atmosphere was swtiched to hygrogen. After stirring for 16 hr, the reaction mixture was filtered through a celite pad. The filtrate was removed in-vacuo to obtain (3S)-4-(tert-butoxy)-4-oxo-3-(((((3R,4S,5S,6S)-3,4,5-triacetoxy-6-(methoxycarbonyl)tetrahydro-2H-pyran-2-yl)oxy)carbonyl)amino)butanoic acid. HRMS: [M-H]-: 548.4, Rt=1.79 min (5 min acidic method).

**Synthesis of 1-(2-(((3S)-4-(tert-butoxy)-N-methyl-4-oxo-3-(((((3R,4S,5S,6S)-3,4,5-triacetoxy-6-(methoxycarbonyl)tetrahydro-2H-pyran-2-yl)oxy)carbonyl)amino)butanamido)methyl)-4-((S)-2-((S)-2-((tert-butoxycarbonyl) amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno [2,3-d]pyrimidin-5-yl)-3-methylphenoxy)ethyl)-1-methylpiperazin-1-ium**

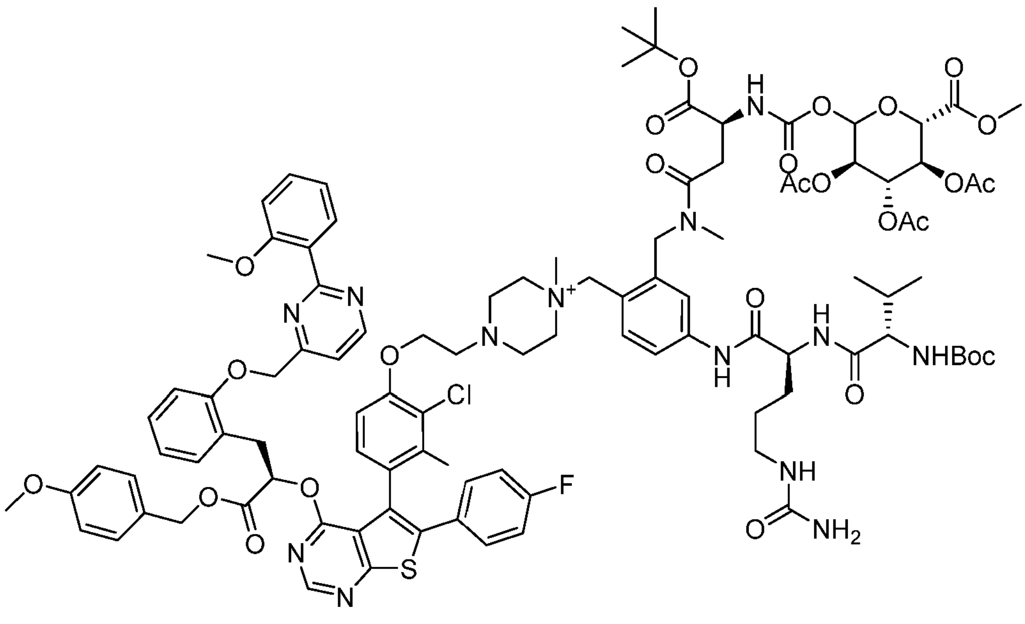

Following **GENERAL PROCEDURE 7** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 0.016 mmol, 1.0 eq) and (3S)-4-(tert-butoxy)-4-oxo-3-(((((3R,4S,5S,6S)-3,4,5-triacetoxy-6-(methoxycarbonyl)tetrahydro-2H-pyran-2-yl)oxy)carbonyl)amino)butanoic acid (12.5 mg, 0.250 mL, 0.023 mmol ,1.4 eq), 1-(2-(((3S)-4-(tert-butoxy)-N-methyl-4-oxo-3-(((((3R,4S,5S,6S)-3,4,5-triacetoxy-6-(methoxycarbonyl)tetrahydro-2H-pyran-2-yl)oxy)carbonyl)amino)butanamido) methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=2030.7900; Rt=3.19 min (5 min acidic method).

**Synthesis of 1-(4-((S)-2-((S)-2-amino-3-methylbutanamido)-5-ureidopentanamido)-2-(((3S)-3-carboxy-N-methyl-3-(((((3R,4S,5S,6S)-3,4,5-triacetoxy-6-(methoxycarbonyl)tetrahydro-2H-pyran-2-yl)oxy)carbonyl)amino) propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

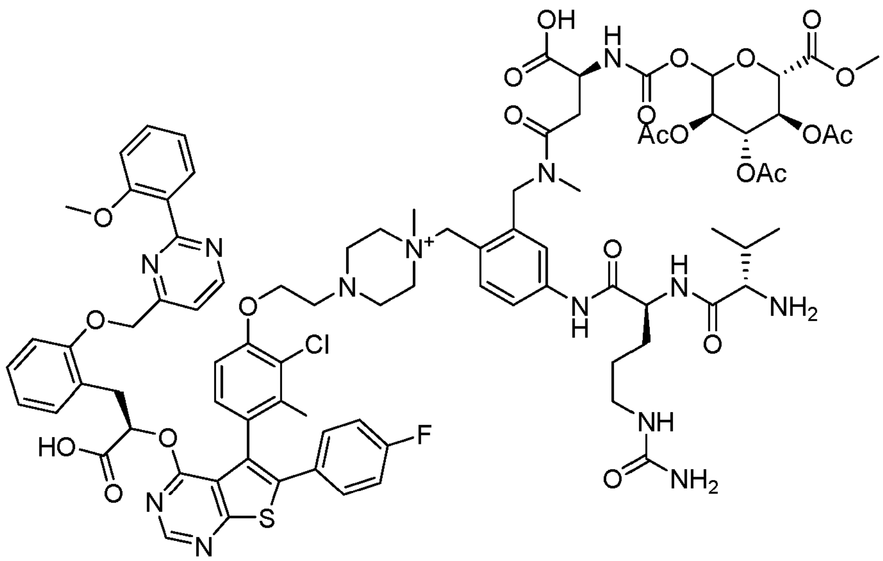

To 1-(2-(((3S)-4-(tert-butoxy)-N-methyl-4-oxo-3-(((((3R,4S,5S,6S)-3,4,5-triacetoxy-6-(methoxycarbonyl)tetrahydro-2H-pyran-2-yl)oxy)carbonyl)amino)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl) oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (25.8 mg, 10.86 μmol, 1.0 equiv.) dissolved in DCM (2 mL) was added TFA (0.67 mL). After stirring for 2 hrs, the solvent was removed *in-vacuo* to obtain 1-(4-((S)-2-((S)-2-amino-3-methylbutanamido)-5-ureidopentanamido)-2-(((3S)-3-carboxy-N-methyl-3-(((((3R,4S,5S,6S)-3,4,5-triacetoxy-6-(methoxycarbonyl)tetrahydro-2H-pyran-2-yl)oxy)carbonyl)amino)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium. HRMS: M+=1754.6200; Rt=2.31 min (5 min acidic method).

**Synthesis of 1-(4-((S)-2-((S)-2-amino-3-methylbutanamido)-5-ureidopentanamido)-2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)benzyl)-4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-methylpiperazin-1-ium**

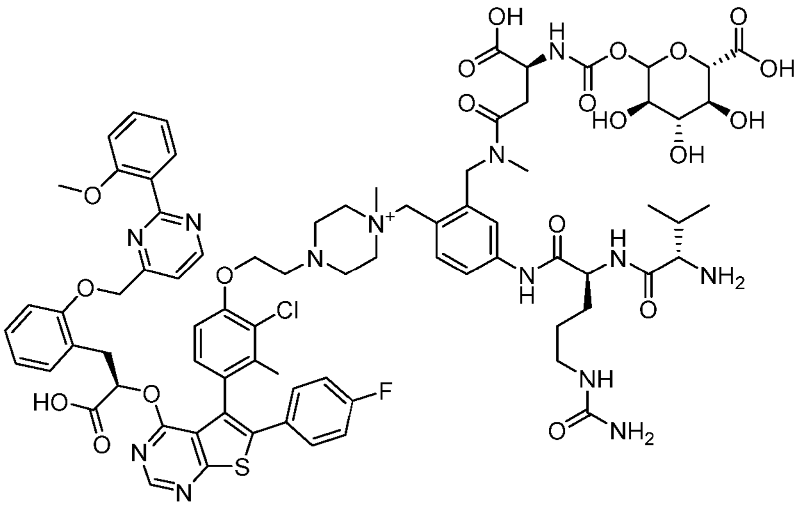

To 1-(4-((S)-2-((S)-2-amino-3-methylbutanamido)-5-ureidopentanamido)-2-(((3S)-3-carboxy-N-methyl-3-(((((3R,4S,5S,6S)-3,4,5-triacetoxy-6-(methoxycarbonyl)tetrahydro-2H-pyran-2-yl)oxy)carbonyl)amino)propanamido)methyl)benzyl)-4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-methylpiperazin-1-ium (26 mg, 0.012 mmol, 1.0 equiv.) dissolved in THF (1 mL) and MeOH (1 mL) was added lithium hydroxide (5.20 mg, 0.124 mmol, 10 equiv.). After stirring for 2 hour, the solvent was removed *in-vacuo.* Water (1 mL), TFA (0.2 mL), MeCN (1 mL) and DMSO (4 mL) were added and the solution was purified by RP-HPLC ISCO gold chromatography (10-70% MeCN/H2O, 0.1% TFA modifier). Upon lyophilization, 1-(4-((S)-2-((S)-2-amino-3-methylbutanamido)-5-ureidopentanamido)-2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=1614.5800; Rt=2.04 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L69-P1)**

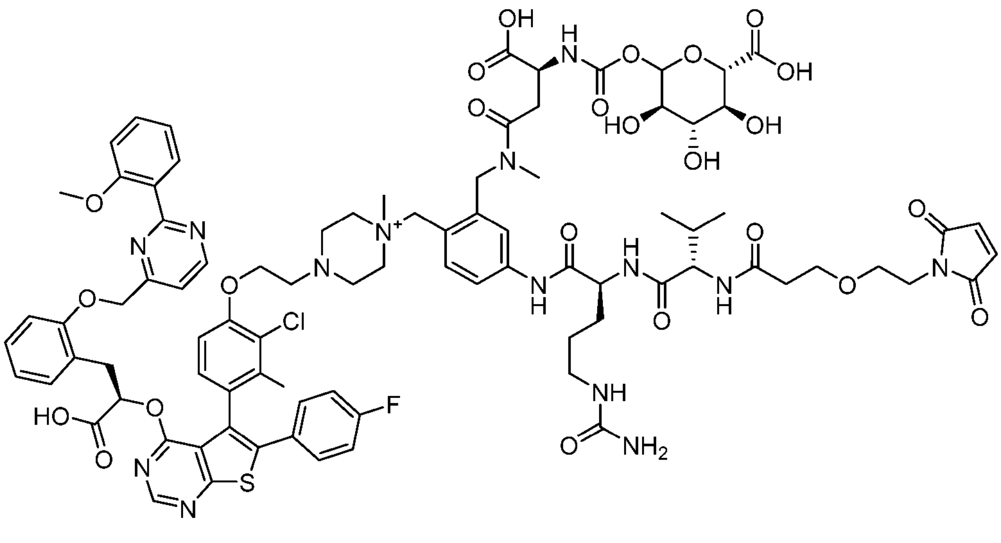

To 1-(4-((S)-2-((S)-2-amino-3-methylbutanamido)-5-ureidopentanamido)-2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (10 mg, 5.11 μmol, 1.0 equiv.) and 2,5-dioxopyrrolidin-1-yl 3-(2-(2,5-dioxo-2,5-dihydro-1 H-pyrrol-1-yl)ethoxy)propanoate (4.75 mg, 0.015 mmol, 3.5 equiv.) dissolved in DMF (1 mL) was added DIPEA (7.12 μl, 0.041 mmol, 8 equiv.). After standing for 1.5 hour, DMSO (2mL) were added and the solution was purified by RP-HPLC ISCO gold chromatography (10-70% MeCN/H2O, 0.1% TFA modifier). Upon lyophilization, 4-(2-(4-(4-((R)-1-carboxy-2-(2-((2-(2-methoxyphenyl)pyrimidin-4-yl)methoxy) phenyl)ethoxy)-6-(4-fluorophenyl)thieno[2,3-d]pyrimidin-5-yl)-2-chloro-3-methylphenoxy)ethyl)-1-(2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+=1809.6300; Rt=2.32 min (5 min acidic method).

**Synthesis of 1-(2-((3-(2-(2-aminoethoxy)ethoxy)-N-methylpropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-**methylpiperazin-1-ium

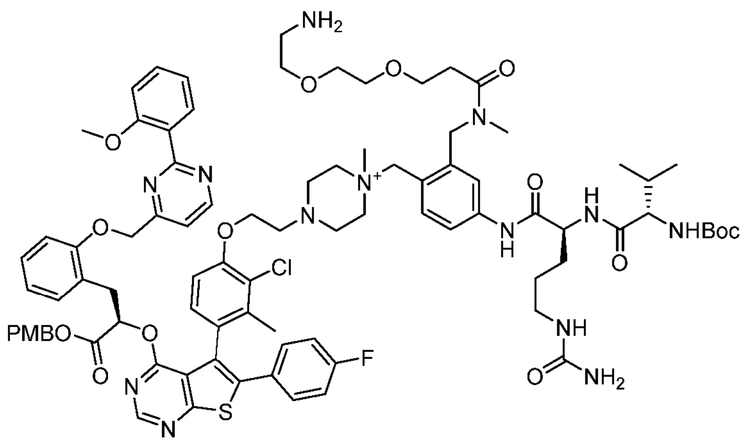

A mixture of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy) ethyl)-1-methylpiperazin-1-ium (45 mg, 0.026 mmol), 1-(9H-fluoren-9-yl)-3-oxo-2,7,10-trioxa-4-azatridecan-13-oic acid (12 mg, 0.030 mmol), HBTU (12 mg, 0.032 mmol), and DIPEA (0.023 mL, 0.13 mmol) in DMF (1 mL) was stirred at RT for 30 min. $Me_2NH$ (2M in THF, 0.065 mL, 0.13 mmol) was added, and the mixture was stirred at RT for 1 h. Additional amount of $Me_2NH$ (2M in THF, 0.1 mL, 0.2 mmol) was added. The mixture was continued to be stirred at RT for 1 h, diluted with DMSO (3 mL), and the solution was purified by RP-HPLC ISCO gold chromatography (MeCN/$H_2O$, 0.1% TFA modifier). Upon lyophilization, 1-(2-((3-(2-(2-aminoethoxy)ethoxy)-N-methylpropanamido)methyl)-4-((S)-2-((S)-2-((tert-butoxycarbonyl) amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1658.7200, Rt=2.57 min (5 min acidic method).

**Synthesis of 1-(4-((S)-2-((S)-2-((tert-butoxycarbonyl)amino)-3-methylbutanamido)-5-ureidopentanamido)-2-(2-methyl-3,13-dioxo-15-phosphono-6,9-dioxa-2,12-diazapentadecyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

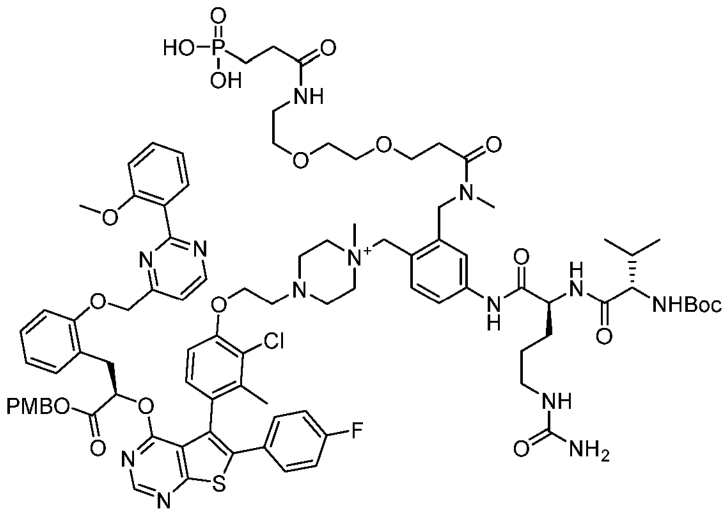

A mixture of 3-phosphopropionic acid (11 mg, 0.071 mmol), HBTU (27 mg, 0.071 mmol), and DIPEA (0.060 mL, 0.34 mmol) in DMF (0.5 mL) was stirred at RT for 10 min. This mixture was added to a solution of 1-(2-((3-(2-(2-aminoethoxy)ethoxy)-N-methylpropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy) ethyl)-1-methylpiperazin-1-ium (40 mg, 0.021 mmol) and DIPEA (0.010 mL, 0.057 mmol) in DMF (0.5 mL). The mixture was stirred at RT for 2 days. The mixture was diluted with DMSO (3 mL), and the solution was purified by RP-HPLC ISCO gold chromatography (MeCN/$H_2O$, 0.1% TFA modifier). Upon lyophilization, 1-(4-((S)-2-((S)-2-((tert-butoxycarbonyl) amino)-3-methylbutanamido)-5-ureidopentanamido)-2-(2-methyl-3,13-dioxo-15-phosphono-6,9-dioxa-2,12-diazapentadecyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1794.7100, Rt=2.78 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(2-methyl-3,13-dioxo-15-phosphono-6,9-dioxa-2,12-diazapentadecyl)benzyl)-1-methylpiperazin-1-ium (L41-P1)**

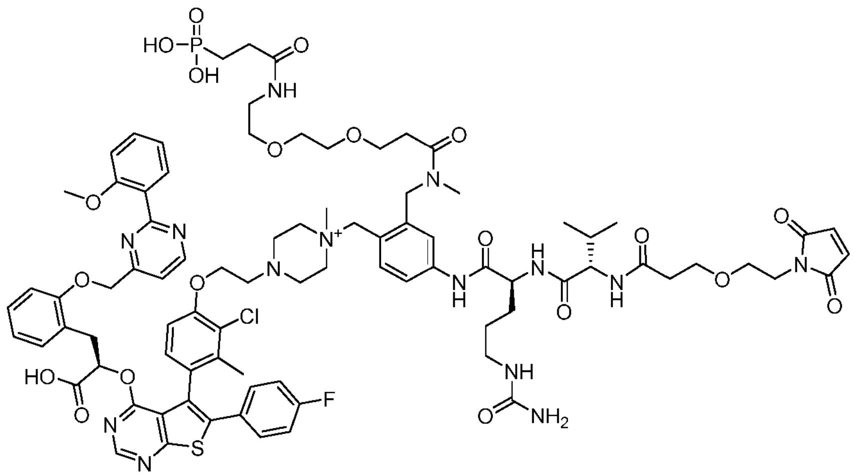

Following **GENERAL PROCEDURE 3** (except that the product after the first step with TFA/$CH_2Cl_2$ was purified by RP-HPLC ISCO gold chromatography [MeCN/$H_2O$, 0.1% $NH_4OH$ modifier]) with 1-(4-((S)-2-((S)-2-((tert-butoxycarbonyl)amino)-3-methylbutanamido)-5-ureidopentanamido)-2-(2-methyl-3,13-dioxo-15-phosphono-6,9-dioxa-2,12-diazapentadecyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium, 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(2-methyl-3,13-dioxo-15-phosphono-6,9-dioxa-2,12-diazapentadecyl)benzyl)-1-methylpiperazin-1-ium was obtained. HRMS: M+= 1769.4500, Rt=2.33 min (5 min acidic method).

**Synthesis of 1-(4-((S)-2-((S)-2-((tert-butoxycarbonyl)amino)-3-methylbutanamido)-5-ureidopentanamido)-2-(2,5,8,11,14,17,20,23,26,29,32,35,38,44,44-pentadecamethyl-3,6,9,12,15,18,21,24,27,30,33,36,39,42-tetradecaoxo-43-oxa-2,5,8,11,14,17,20,23,26,29,32,35,38-tridecaazapentatetracontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

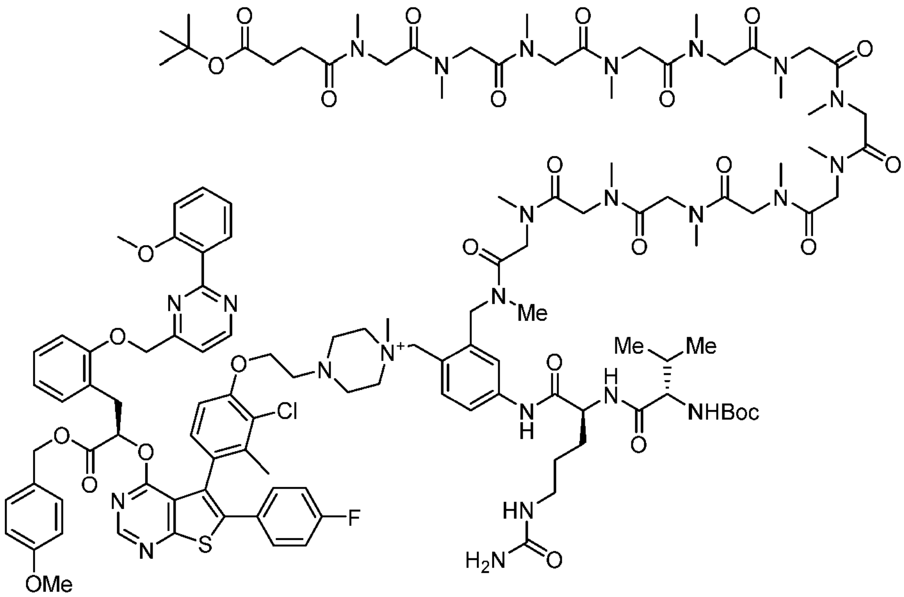

To a stirred solution of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (43.6 mg, 0.025 mmol, 1.0 equiv.), 3,6,9,12,15,18,21,24,27,30,33,36,42,42-tetradecamethyl-4,7,10,13,16,19,22,25,28,31,34,37,40-tridecaoxo-41-oxa-3,6,9,12,15,18,21,24,27,30,33,36-dodecaazatritetracontanoic acid (25.9 mg, 0.025 mmol, 1.0 equiv.), and HATU (10.5 mg, 0.028 mmol, 1.1 equiv.) in DMF (0.25 mL) was added DIPEA (22 μL, 0.126 mmol, 5.0 equiv.). The resulting solution was stirred at ambient temperature for 1 hour. The reaction was diluted with 1 mL DMSO and purified by RP-HPLC ISCO gold chromatography (10-100% MeCN/H2O, 0.1% TFA modifier). Upon lyophilization 1-(4-((S)-2-((S)-2-((tert-butoxycarbonyl)amino)-3-methylbutanamido)-5-ureidopentanamido)-2-(2,5,8,11,14,17,20,23,26,29,32,35,38,44,44-pentadecamethyl-3,6,9,12,15,18,21,24,27,30,33,36,39,42-tetradecaoxo-43-oxa-2,5,8,11, 14, 17,20,23,26,29,32,35,38-tridecaazapentatetracontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS M+ 2508.1499 Rt=2.78 min (5 min acidic method).

**Synthesis of 1-(2-(41-carboxy-2,5,8,11,14,17,20,23,26,29,32,35,38-tridecamethyl-3,6,9,12,15,18,21,24,27,30,33,36,39-tridecaoxo-2,5,8,11,14,17,20,23,26,29,32,35,38-tridecaazahentetracontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L35-P1)**

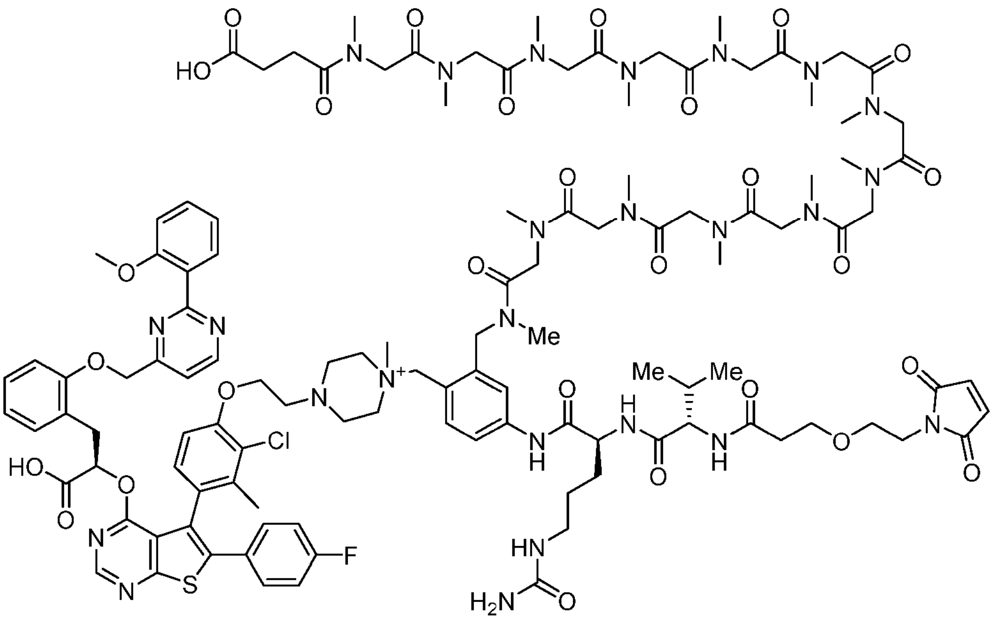

Following **GENERAL PROCEDURE 3** with 1-(4-((S)-2-((S)-2-((tert-butoxycarbonyl)amino)-3-methylbutanamido)-5-ureidopentanamido)-2-(2,5,8,11,14,17,20,23,26,29,32,35,38,44,44-pentadecamethyl-3,6,9,12,15,18,21,24,27,30,33,36,39,42-tetradecaoxo-43-oxa-2,5,8,11, 14, 17,20,23,26,29,32,35,38-tridecaazapentatetracontyl)benzyl)-4-(2-(2-chloro-4-(6-(4-fluorophenyl)-4-(((R)-1-((4-methoxybenzyl)oxy)-3-(2-((2-(2-methoxy-phenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (50.4 mg, 0.019 mmol, 1.0 equiv.), 1-(2-(41-carboxy-2,5,8,11,14,17,20,23,26,29,32,35,38-tridecamethyl-3,6,9,12,15,18,21,24,27,30,33,36,39-tridecaoxo-2,5,8,11,14,17,20,23,26,29,32,35,38-tridecaazahentetracontyl)-4-((S)-2-((S)-2-(3-(2-(2,5-dioxo-2,5-dihydro-1 H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+ 2427.0400, rt = 2.30 min. (5 min acidic method).

**Synthesis of 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,62,62-henicosamethyl-3,6,9,12,15,18,21,24,27,30,33,36,39,42,45,48,51,54,57,60-icosaoxo-61-oxa-2,5,8,11,14,17,20,23,26,29,32,35,38,41,44,47,50,53,56-nonadecaazatrihexacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

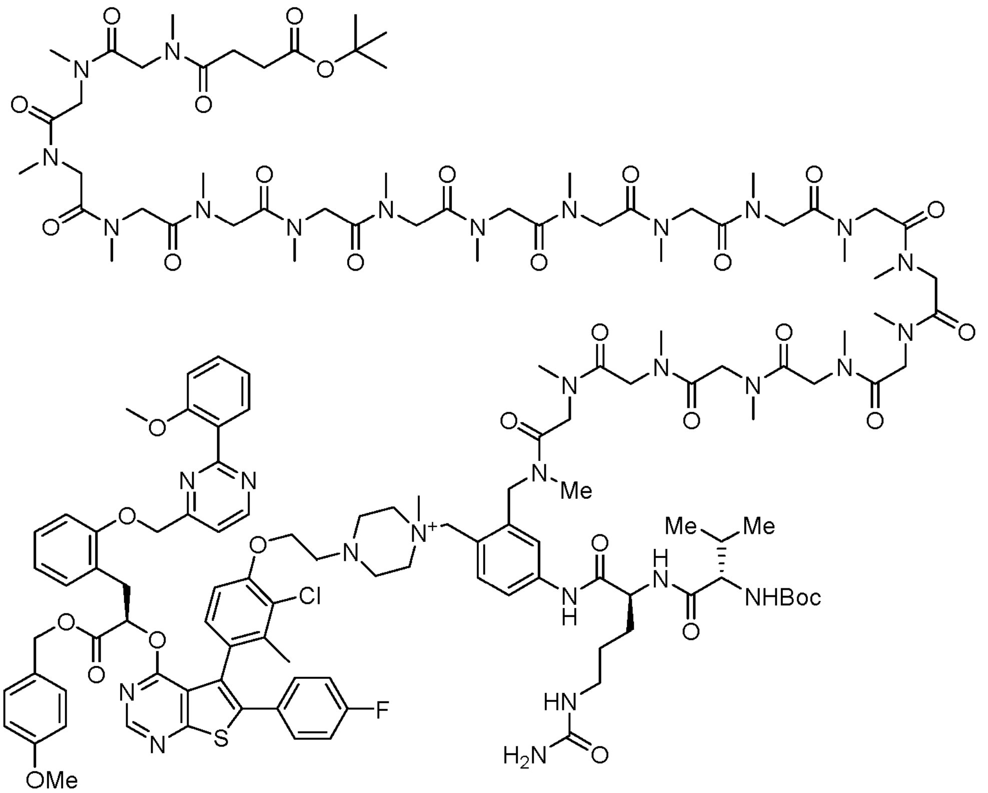

To a stirred solution of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (31.9 mg, 0.018 mmol, 1.0 equiv.), 3,6,9,12,15,18,21,24,27,30,33,36,39,42,45,48,51,54,60,60-icosamethyl-4,7,10,13,16,19,22,25,28,31,34,37,40,43,46,49,52,55,58-nonadecaoxo-59-oxa-3,6,9,12,15,18,21,24,27,30,33,36,39,42,45,48,51,54-octadecaazahenhexacontanoic acid (26.8 mg, 0.018 mmol, 1.0 equiv.), and HATU (7.7 mg, 0.020 mmol, 1.1 equiv.) in DMF (0.25 mL) was added DIPEA (16 $\mu$L, 0.092 mmol, 5.0 equiv.). The resulting solution was stirred at ambient temperature for 1 hour. The reaction was diluted with 1 mL DMSO and purified by RP-HPLC ISCO gold chromatography (10-100% MeCN/H2O, 0.1% TFA modifier). Upon lyophilization 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,62,62-henicosamethyl-3,6,9,12,15,18,21,24,27,30,33,36,39,42,45,48,51,54,57,60-icosaoxo-61-oxa-2,5,8,11,14,17,20,23,26,29,32,35,38,41,44,47,50,53,56-nonadecaazatrihexacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS M+ 2934.3601 Rt=2.73 min (5 min acidic method).

**Synthesis of 1-(2-(59-carboxy-2,5,8,11,14,17,20,23,26,29,32,35,38,41,44,47,50,53,56-nonadecamethyl-3,6,9,12,15,18,21,24,27,30,33,36,39,42,45,48,51,54,57-nonadecaoxo-2,5,8,11,14,17,20,23,26,29,32,35,38,41,44,47,50,53,56-nonadecaazanonapentacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L36-P1)**

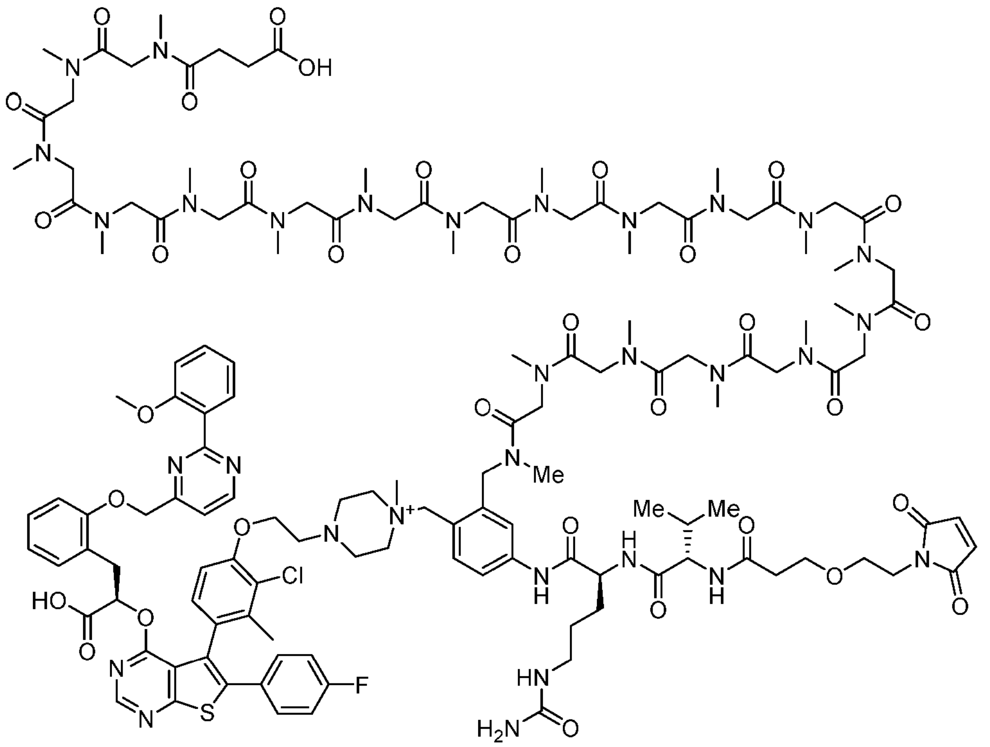

Following **GENERAL PROCEDURE 3** with 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,62,62-henicosamethyl-3,6,9,12,15,18,21,24,27,30,33,36,39,42,45,48,51,54,57,60-icosaoxo-61-oxa-2,5,8,11,14,17,20,23,26,29,32,35,38,41,44,47,50,53,56-nonadecaazatrihexacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (45.5 mg, 0.015 mmol, 1.0 equiv.), 1-(2-(59-carboxy-2,5,8,11,14,17,20,23,26,29,32,35,38,41,44,47,50,53,56-nonadecamethyl-3,6,9,12,15,18,21,24,27,30,33,36,39,42,45,48,51,54,57-nonadecaoxo-2,5,8,11,14,17,20,23,26,29,32,35,38,41,44,47,50,53,56-nonadecaazanonapentacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+ 2853.2766, rt = 2.20 min. (5 min acidic method).

**Synthesis of 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,59,62,65,68,71,74,80,80-heptacosamethyl-3,6,9,12,15,18,21,24,27,30,33,36,39,42,45,48,51,54,57,60,63,66,69,72,75,78-hexacosaoxo-79-oxa-2,5,8,11,14,17,20,23,26,29,32,35,38,41,44,47,50,53,56,59,62,65,68,71,74-pentacosaazahenoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

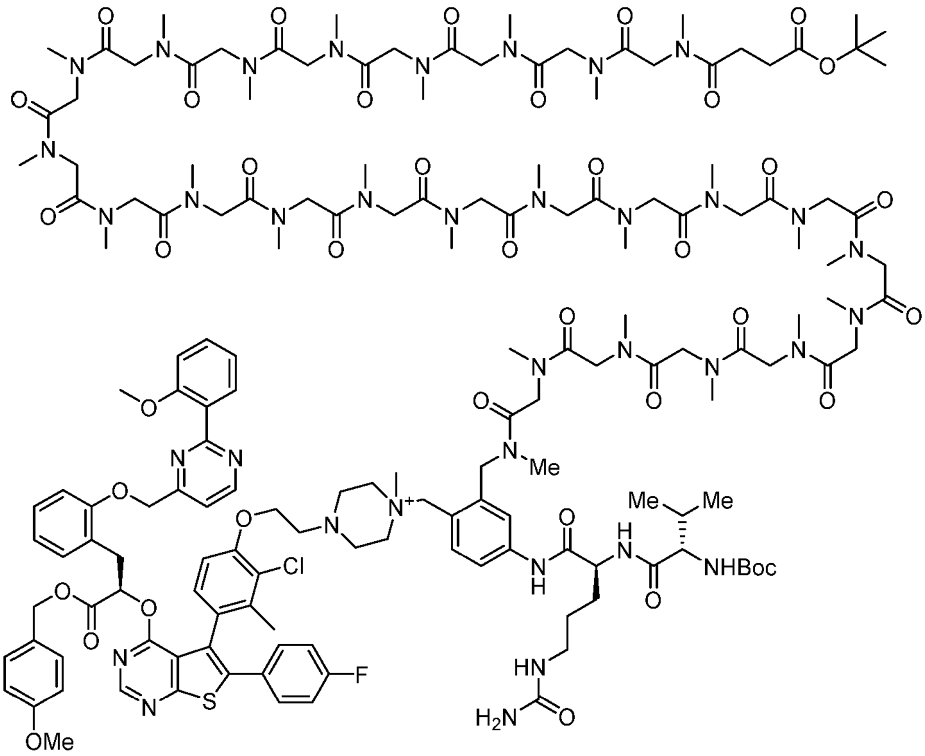

To a stirred solution of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (27.8 mg, 0.016 mmol, 1.0 equiv.), 3,6,9,12,15,18,21,24,27,30,33,36,39,42,45,48,51,54,57,60,63,66,69,72,78,78-hexacosamethyl-4,7,10,13,16,19,22,25,28,31,34,37,40,43,46,49,52,55,58,61,64,67,70,73,76-pentacosaoxo-77-oxa-3,6,9,12,15,18,21,24,27,30,33,36,39,42,45,48,51,54,57,60,63,66,69,72-tetracosaazanonaheptacontanoic acid (30.2 mg, 0.016 mmol, 1.0 equiv.), and HATU (6.7 mg, 0.018 mmol, 1.1 equiv.) in DMF (0.25 mL) was added DIPEA (14 μL, 0.080 mmol, 5.0 equiv.). The resulting solution was stirred at ambient temperature for 1 hour. The reaction was diluted with 1 mL DMSO and purified by RP-HPLC ISCO gold chromatography (10-100% MeCN/H2O, 0.1% TFA modifier). Upon lyophilization 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,59,62,65,68,71,74,80,80-heptacosamethyl-3,6,9,12,15,18,21,24,27,30,33,36,39,42,45,48,51,54,57,60,63,66,69,72,75,78-hexacosaoxo-79-oxa-2,5,8,11,14,17,20,23,26,29,32,35,38,41,44,47,50,53,56,59,62,65,68,71,74-pentacosaazahenoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS M+ 3360.5798 Rt=2.68 min (5 min acidic method).

**Synthesis of 1-(2-(77-carboxy-2,5,8,11,14,17,20,23,26,29,32,35,38,41,44,47,50,53,56,59,62,65,68,71,74-pentacosamethyl-3,6,9,12,15,18,21,24,27,30,33,36,39,42,45,48,51,54,57,60,63,66,69,72,75-pentacosaoxo-2,5,8,11,14,17,20,23,26,29,32,35,38,41,44,47,50,53,56,59,62,65,68,71,74-pentacosaazaheptaheptacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L37-P1)**

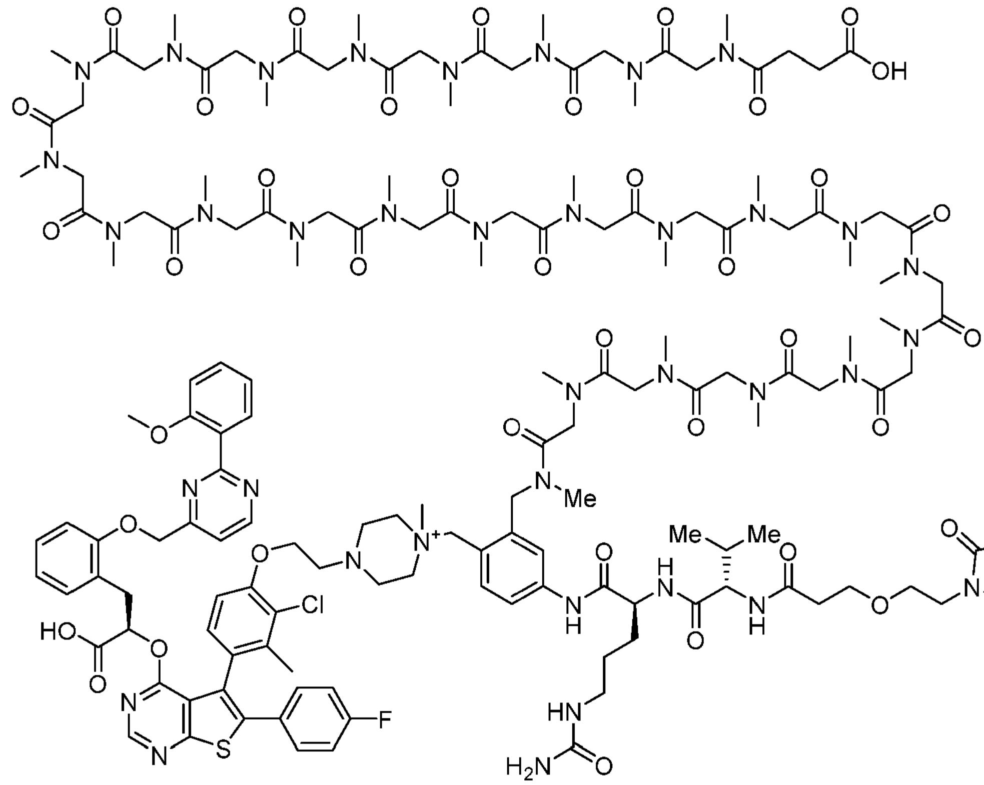

Following GENERAL PROCEDURE 3 with 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,59,62,65,68,71,74,80,80-heptacosamethyl-3,6,9,12,15,18,21,24,27,30,33,36,39,42,45,48,51,54,57,60,63,66,69,72,75,78-hexacosaoxo-79-oxa-2,5,8,11,14,17,20,23,26,29,32,35,38,41,44,47,50,53,56,59,62,65,68,71,74-pentacosaazahenoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (41.3 mg, 0.012 mmol, 1.0 equiv.), 1-(2-(77-carboxy-2,5,8,11,14,17,20,23,26,29,32,35,38,41,44,47,50,53,56,59,62,65,68,71,74-pentacosamethyl-3,6,9,12,15,18,21,24,27,30,33,36,39,42,45,48,51,54,57,60,63,66,69,72,75-pentacosaoxo-2,5,8,11,14,17,20,23,26,29,32,35,38,41,44,47,50,53,56,59,62,65,68,71,74-pentacosaazaheptaheptacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+ 3279.4678, rt = 2.21 min. (5 min acidic method).

## Synthesis of Tert-butyl 1-((N-((2-azidoethoxy)carbonyl)sulfamoyl)amino)-3,6,9,12,15,18-hexaoxahenicosan-21-oate

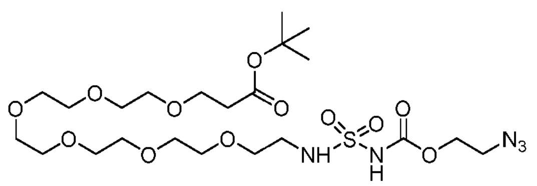

To 2-azidoethan-1-ol (105 mg, 1.21 mmol) in $CH_2Cl_2$ (15 ml) was added sulfurisocyanatidic chloride (0.105 ml, 1.21 mmol) at 0°C. The mixture was stirred at 0°C for 30 min. then TEA (0.336 ml, 2.41 mmol) and tert-butyl 1-amino-3,6,9,12,15,18-hexaoxahenicosan-21-oate (518 mg, 1.27 mmol) in $CH_2Cl_2$ (1 mL) were added. The mixture was stirred at 0°C for 1 h and rt for 2 h, then was quenched with Satd NH4Cl, and 1 N HCl (2.4 mL). The aqueous was extracted with $CH_2Cl_2$ (5X). The organic layers were dried over anh, $Na_2SO_4$, filtered and concentrated via rotary

evaporation to give a clear oil. Purification via flash chromatography (0-15% MeOH in $CH_2Cl_2$, ELSD detection) provided tert-butyl 1-((N-((2-azidoethoxy)carbonyl)sulfamoyl)amino)-3,6,9,12,15,18-hexaoxahenicosan-21-oate as a clear oil (474 mg, 0.788 mmol): LCMS: M+NH4+=619.5, Rt=0.94 min (acidic, 2 min). 1H NMR (400 MHz, DMSO-d6) δ 11.33 (s, 1H), 7.76 (s, 1H), 4.28 - 4.20 (m, 2H), 3.60 (td, J = 5.6, 5.0, 2.9 Hz, 4H), 3.55 - 3.45 (m, 22H), 3.16 - 3.04 (m, 2H), 2.46 - 2.38 (m, 2H), 1.41 (s, 9H).

**Synthesis of 1-((N-((2-azidoethoxy)carbonyl)sulfamoyl)amino)-3,6,9,12,15,18-hexaoxahenicosan-21-oic acid**

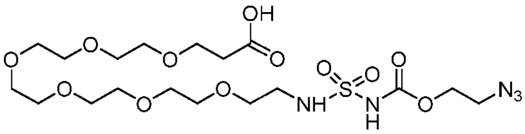

To tert-butyl 1-((N-((2-azidoethoxy)carbonyl)sulfamoyl)amino)-3,6,9,12,15,18-hexaoxahenicosan-21-oate (145 mg, 0.241 mmol) in $CH_2Cl_2$ (1 mL) at 0°C was added TFA (1 mL, 12.98 mmol). The mixture was stirred at rt for 1.45 h, then concentrated via rotary evaporation at 25°C water bath and dried in high vac for 30 min. The residue was azeotropically dried with anh toluene (3X 2 mL), and dried in vacuum overnight to provide 1-((N-((2-azidoethoxy)carbonyl) sulfamoyl)amino)-3,6,9,12,15,18-hexaoxahenicosan-21-oic acid as a clear oil (147 mg, 89% by weight based on theoretical yield): LCMS: M+=546.3, 0.65 min (acidic, 2 min, ELSD); 1H NMR (400 MHz, DMSO-d6) δ 11.30 (s, 1H), 7.77 - 7.71 (m, 1H), 4.25 - 4.19 (m, 2H), 3.63 - 3.55 (m, 4H), 3.54 - 3.45 (m, 22H), 3.07 (q, J = 6.0 Hz, 2H), 2.47 - 2.40 (m, 2H).

**Synthesis of 1-(4-((S)-2-((S)-2-amino-3-methylbutanamido)-5-ureidopentanamido)-2-((prop-2-yn-1-yloxy) methyl)benzyl)-4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-methylpiperazin-1-ium trifluoroacetate**

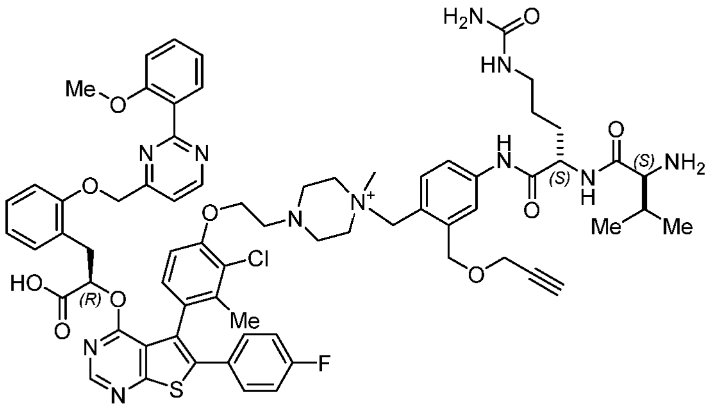

To 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium trifluoroacetate (321 mg, 0.210 mmol) at 0°C was added 25% TFA in $CH_2Cl_2$ (12.3 mL, 40.0 mmol). The reaction mixture was raised to Rt, stirred for 1h, then concentrated under high vacuum at RT water bath. The crude was diluted with DMSO (3 mL) and was purified by RP-HPLC ISCO gold chromatography (150 g, 10-80% MeCN/H2O, 0.1% TFA modifier). Upon lyophilization 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-methylpiperazin-1-ium trifluoroacetate was obtained as a white powder (224 mg, 0.158 mmol): HRMS: M+=1304.5100, Rt=2.15 min (5 min acidic)

Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-**yl)ethoxy)propanamido)-3-methylbutanamido)-5-ureidopentanamido)-2-((prop-2-yn-1-yloxy)methyl)benzyl)-1-methylpiperazin-1-ium trifluoroacetate**

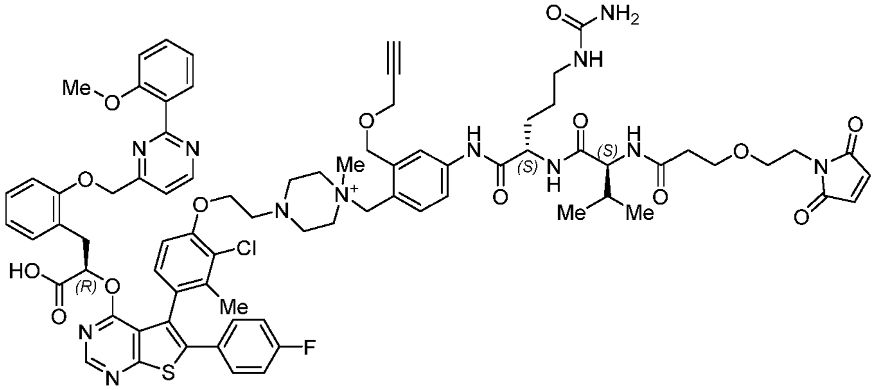

Following **GENERAL PROCEDURE 3** with 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium trifluoroacetate (164 mg, 0.115 mmol) and Mal-Peg1-NHS ester (72 mg, 0.23 mmol), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetatewas obtained as a white powder (170 mg, 0.105 mmol), HRMS: M+ =1499.5699, Rt=2.45 min (5 min acidic); 1H NMR (400 MHz, DMSO-d6) δ 10.16 (s, 1H), 8.81 (d, J = 5.1 Hz, 1H), 8.54 (s, 1H), 8.07 (d, J = 7.2 Hz, 1H), 7.75 (d, J = 8.5 Hz, 1H), 7.69 (d, J = 6.9 Hz, 2H), 7.56 (d, J = 5.1 Hz, 1H), 7.45 (dd, J = 7.5, 1.8 Hz, 1H), 7.42 - 7.29 (m, 3H), 7.24 (dd, J = 8.9, 5.4 Hz, 2H), 7.18 - 7.05 (m, 5H), 6.99 - 6.91 (m, 4H), 6.65 (t, J = 7.4 Hz, 1H), 6.15 (d, J = 7.0 Hz, 1H), 5.91 (s, 1H), 5.43 (dd, J = 9.8, 3.5 Hz, 1H), 5.23 - 5.12 (m, 2H), 4.54 (d, J = 11.2 Hz, 4H), 4.33 - 4.09 (m, 7H), 3.69 (s, 3H), 3.42 - 2.78 (m, 32H, overlapping with DMSO), 2.39 - 2.20 (m, 2H), 1.91 - 1.81 (m, 1H), 1.77 (s, 3H), 1.46 (dd, J = 93.8, 31.3 Hz, 3H), 0.76 (dd, J = 13.8, 6.7 Hz, 6H); 19F NMR (376 MHz, DMSO-d6): -112.18 ppm

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(2-(((N-(20-carboxy-3,6,9,12,15,18-hexaoxaicosyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium trifluoroacetate (L59-P1)**

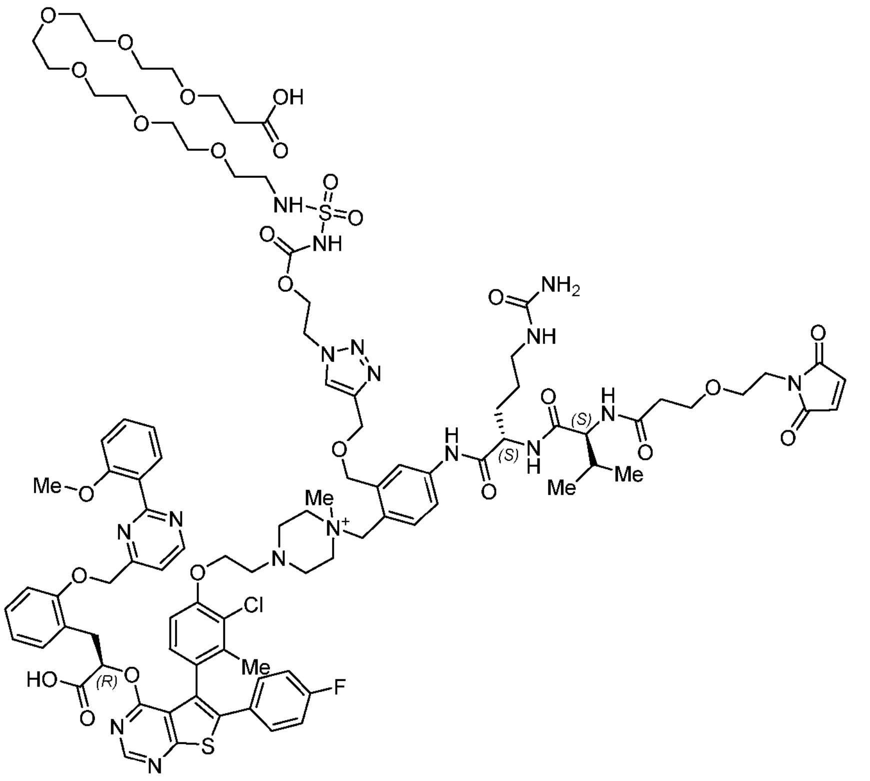

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (32 mg, 0.021 mmol) and 1-((N-((2-azidoethoxy)carbonyl)sulfamoyl)amino)-3,6,9,12,15,18-hexaoxahenicosan-21-oic acid (26.2 mg, 0.043 mmol), 4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-(2-(((1-(2-(((N-(20-carboxy-3,6,9,12,15,18-hexaoxaicosyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium trifluoroacetate was obtained as a white powder: HRMS: M+ =2044.7700, Rt=2.36 min (5 min acidic).

**Synthesis of 3-(2-(2-(2-((N-((2-azidoethoxy)carbonyl)sulfamoyl)amino)ethoxy)ethoxy)ethoxy)propanoic acid**

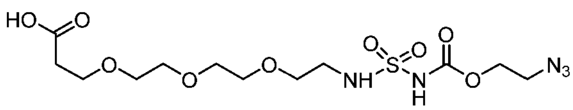

To 2-azidoethan-1-ol (105 mg, 1.21 mmol) in $CH_2Cl_2$ (15 ml) was added sulfurisocyanatidic chloride (0.105 ml, 1.21 mmol) at 0°C. The mixture was stirred at 0°C for 30 min. At 0°C TEA (0.336 ml, 2.41 mmol), and tert-butyl 3-(2-(2-(2-aminoethoxy)ethoxy)ethoxy) propanoate (401 mg, >80% technical purity, 1.447 mmol) in $CH_2Cl_2$ (1 mL) were added. After being stirred at 0°C for 1 h, then rt for 1 h, the mixture was quenched with satd. NH4Cl, and 1 N HCl (2.4 mL). The aqueous was extracted with $CH_2Cl_2$ (5X). The organic layers were dried over anh, $Na_2SO_4$, filtered and concentrated via rotary evaporation to provide a clear oil. Following purification by flash chromatography (0-15% MeOH in $CH_2Cl_2$, ELSD detection) tert-butyl 3-(2-(2-(2-((N-((2-azidoethoxy)carbonyl)sulfamoyl)amino)ethoxy)ethoxy)ethoxy)propanoate was obtained as thick clear oil (356 mg, 0.910 mmol): LCMS: MS+NH4+=487.4, Rt=0.90 min (acidic, 2 min, ELSD); 1H NMR (400 MHz, DMSO-d6) δ 11.31 (s, 1H), 7.78 - 7.70 (m, 1H), 4.26 - 4.19 (m, 2H), 3.58 (td, J = 5.6, 5.0, 3.7 Hz, 4H), 3.53 - 3.39 (m, 10H), 3.10 - 3.03 (m, 2H), 2.44 - 2.39 (m, 2H), 1.40 (s, 9H).

To tert-butyl 3-(2-(2-(2-((N-((2-azidoethoxy)carbonyl)sulfamoyl)amino)ethoxy)ethoxy)ethoxy)propanoate (162 mg, 0.345 mmol) in $CH_2Cl_2$ (1 mL) at 0°C was added TFA (1 mL, 13.0 mmol). After being stirred at rt for 2 h, the mixture was concentrated via rotary evaporation with a water bath at 25 °C. The residue was dried in high vac for 30 min, by azeotropic

distillation with anh. Toluene (3X 2 mL), and in vacuo overnight to provide 3-(2-(2-(2-((N-((2-azidoethoxy)carbonyl) sulfamoyl)amino)ethoxy)ethoxy)ethoxy)propanoic acid as thick oil (139 mg, 0.335 mmol): LCMS: MS+NH4+=431.4, Rt=0.62 min (acidic, 2 min, ELSD): 1H NMR (400 MHz, DMSO-d6) δ 11.32 (d, J = 15.4 Hz, 1H), 7.78 - 7.69 (m, 1H), 4.27-4.15 (m, 2H), 3.67 - 3.54 (m, 8H), 3.49 - 3.42 (m, 4H), 3.07 (q, J = 6.0 Hz, 2H), 2.47 - 2.39 (m, 4H).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(2-(((N-(2-(2-(2-(2-carboxyethoxy)ethoxy)ethoxy)ethyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium trifluoroacetate (L60-P1)**

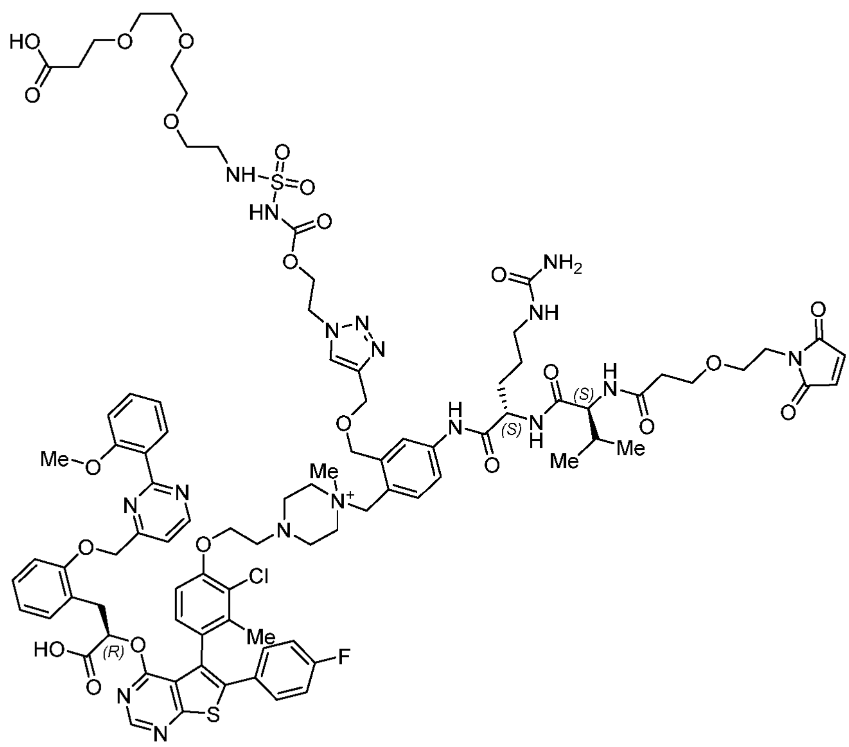

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (15 mg, 10 μmol) and 3-(2-(2-(2-((N-((2-azidoethoxy)carbonyl)sulfamoyl)amino)ethoxy)ethoxy)ethoxy)propanoic acid (12 mg, 0.029 mmol), 4-(2-(4-(4-((R)-1-carboxy-2-(2-((2-(2-methoxyphenyl)pyrimidin-4-yl)methoxy)phenyl)ethoxy)-6-(4-fluorophenyl)thieno [2,3-d]pyrimidin-5-yl)-2-chloro-3-methylphenoxy)ethyl)-1-(2-(((1-(2-(((N-(2-(2-(2-(2-carboxyethoxy)ethoxy)ethoxy) ethyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium trifluoroacetate was obtained as a white powder: HRMS: MS+=1912.7000, Rt=2.38 min (5 min acidic).

**Synthesis of (2R,3R,4S,5R,6R)-2-(acetoxymethyl)-6-(2-(2-((tert-butoxycarbonyl)amino)ethoxy)ethoxy)tetrahydro-2H-pyran-3,4,5-triyl triacetate**

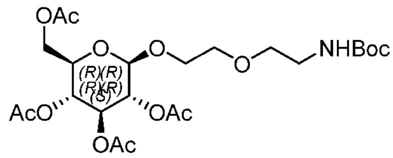

A mixture of tert-butyl (2-(2-hydroxyethoxy)ethyl)carbamate (1535 mg, 7.48 mmol), Ag2CO3 (8248 mg, 14.96 mmol) and a piece of crystalline iodine in $CH_2Cl_2$ (6 mL) was stirred with powdered 4A molecule sieve (1400 mg) for 15 min. To the mixture was added (2R,3R,4S,5R,6R)-2-(acetoxymethyl)-6-bromotetrahydro-2H-pyran-3,4,5-triyl triacetate (2050 mg, 4.99 mmol) in $CH_2Cl_2$ (6.00 ml), also stirred with powdered 4A molecule sieve (1400 mg) for 15 min prior to addition. The resulting mixture was covered with aluminum foil and stirred at rt for 60 h, and then filtered through celite with EtOAc washing. The filtrate was concentrated to give a clear oil that was purified by flash chromatography (0-10% MeOH in $CH_2Cl_2$, ELSD detection) to provide, after concentration of appropriate fractions, a thick oil (640 mg) as a 47/53% mixture of the desired (*2R,3R,4S,5R,6R*)-2-(acetoxymethyl)-6-(2-(2-((tert-butoxycarbonyl)amino)ethoxy)ethoxy)tetrahydro-2H-pyran-3,4,5-triyl triacetate and (*2S,3aR,5R,6R,7S,7aR*)-5-(acetoxymethyl)-2-(2-(2-((tert-butoxycarbonyl)amino)ethoxy)ethoxy)-2-methyltetrahydro-5H-[1,3]dioxolo[4,5-b]pyran-6,7-diyl diacetate: LCMS: MS+=536.4, Rt=0.96 min (2 min, acid, ELSD); 1H NMR (400 MHz, DMSO-d6) δ 6.77 (s, 2H), 5.78 (s, 2H), 5.76 (s, 1H), 5.28 (t, J = 9.5 Hz, 1H), 5.04 (t, J = 3.1 Hz, 1H), 4.91 (t, J = 9.7 Hz, 1H), 4.87 - 4.74 (m, 3H), 4.38 (ddd, J = 5.2, 3.1, 0.9 Hz, 1H), 4.24 - 4.10 (m, 3H), 4.07 - 3.96 (m, 2H), 3.92 (dt, J = 8.8, 4.1 Hz, 1H), 3.80 (dt, J = 11.2, 4.4 Hz, 1H), 3.68 - 3.60 (m, 1H), 3.57 - 3.45 (m, 7H), 3.38 (td, J = 6.2, 1.5 Hz, 7H), 3.07 (dt, J = 6.1, 3.0 Hz, 4H), 2.08 (s, 3H), 2.06 (s, 3H), 2.04 (s, 6H), 2.01 (s, 3H), 2.00 (s, 3H), 1.95 (s, 3H), 1.65 (s, 3H), 1.39 (s, 19H).

**Synthesis of (2R,3R,4S,5R,6S)-2-(acetoxymethyl)-6-(2-(2-aminoethoxy)ethoxy)tetrahydro-2H-pyran-3,4,5-triyl triacetate**

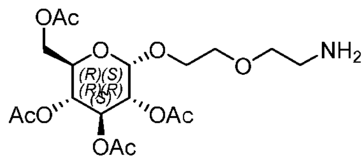

To a 47/53% mixture of (2R,3R,4S,5R,6R)-2-(acetoxymethyl)-6-(2-(2-((tert-butoxycarbonyl)amino)ethoxy)ethoxy)tetrahydro-2H-pyran-3,4,5-triyl triacetate and (2S,3aR,5R,6R,7S,7aR)-5-(acetoxymethyl)-2-(2-(2-((tert-butoxycarbonyl)amino)ethoxy)ethoxy)-2-methyltetrahydro-5H-[1,3]dioxolo[4,5-b]pyran-6,7-diyl diacetate (622 mg, 1.161 mmol) in $CH_2Cl_2$ (16 mL) at 0°C was added TFA (4.0 mL, 52 mmol). The reaction mixture was raised to Rt and stirred for 1 h. The mixture was concentrated and the residue was dried under vacuo for 60 min to give a light yellow oil. The crude product was diluted with DMSO (4 mL) and was purified by RP-HPLC ISCO gold chromatography (5-60% MeCN/H2O, 0.1% TFA modifier, ELSD detection). Upon lyophilization of appropriate fractions, (2R,3R,4S,5R,6S)-2-(acetoxymethyl)-6-(2-(2-aminoethoxy)ethoxy)tetrahydro-2H-pyran-3,4,5-triyl triacetate, as a TFA salt, was obtained as a white powder (195 mg, 0.355 mmol): LCMS: MS+=436.4, Rt=0.58 min (acidic, 2 min); 1H NMR (400 MHz, DMSO-d6) δ 7.72 (s, 3H), 5.27 (t, J = 9.4 Hz, 1H), 4.92 (t, J = 9.6 Hz, 1H), 4.86 - 4.75 (m, 2H), 4.22 - 4.15 (m, 1H), 4.07 - 3.94 (m, 3H), 3.89 - 3.53 (m, 14H, mixed with DMSO), 3.03 - 2.91 (m, 2H), 2.03 (s, 3H), 2.01 (s, 3H), 1.99 (s, 3H), 1.94 (s, 3H).

**Synthesis of 2-azidoethyl (N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamate**

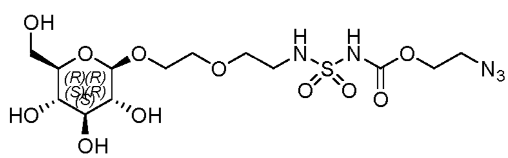

To 2-azidoethan-1-ol (16 mg, 0.18 mmol) in $CH_2Cl_2$ (2.5 ml) was added sulfurisocyanatidic chloride (0.016 ml, 0.184 mmol) at 0°C. The mixture was stirred at 0 C for 1 h, then TEA (0.128 ml, 0.919 mmol) and (*2R,3R,4S,5R,6R*)-2-(acetoxymethyl)-6-(2-(2-aminoethoxy)ethoxy)tetrahydro-2H-pyran-3,4,5-triyl triacetate, as a TFA salt (116 mg, 0.211 mmol) in $CH_2Cl_2$ (1.5 mL) were added. After being stirred at 0°C for 1 h, then rt for 1 h, the mixture was quenched with satd NH4Cl, and 1 N HCl (0.919 mL, 0.919 mmol). The aqueous was extracted with $CH_2Cl_2$ (5X). The organic layer was over anh, $Na_2SO_4$, filtered and concentrated via rotary evaporation to afford *(2R,3R,4S,5R,6R)*-2-(acetoxymethyl)-6-(2-(2-((N-((2-azidoethoxy)carbonyl)sulfamoyl)amino)ethoxy)ethoxy)tetrahydro-2H-pyran-3,4,5-triyl triacetate as a clear oil (121 mg): LCMS: MS+=628, Rt=0.85 min (acidic, 2min, ELSD); 1H NMR (400 MHz, DMSO-d6) δ 11.32 (s, 1H), 7.79 - 7.71 (m, 1H), 5.26 (t, J = 9.5 Hz, 1H), 4.90 (t, J = 9.7 Hz, 1H), 4.85 - 4.73 (m, 2H), 4.25 - 4.14 (m, 3H), 4.06 - 3.94 (m, 2H), 3.79 (ddd, J = 11.3, 5.3, 3.8 Hz, 1H), 3.68 - 3.40 (m, 8H), 3.16 - 3.00 (m, 3H), 2.02 (s, 3H), 2.00 (s, 3H), 1.98 (s, 3H), 1.94 (s, 3H).

The product above was dissolved in dioxane (3 ml) and cooled at 0°C. $LiOH.H_2O$ (0.5 M in water, 2.94 ml, 1.47 mmol) was added. The resulting clear solution was stirred at rt for 1 h and then quenched at 0°C with HCl (5N, 0.147 mL, 0.735 mmol). The mixture was concentrated via rotary evaporation at 20 °C water bath to remove most of dioxane. The residual solution (ca. 3 mL) was purified by prep HPLC (Sunfire 5μm 30x50 mm column, 2-12% of Acetonitrile with 0.1% FA in Water. Flow Rate: 75 mL/min., MS 459.3, 476.3 detection) to provide, after removal of solvent of appropriate fractions via lyophilization, 2-azidoethyl (N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamate as a semi-solid (64 mg, 0.14 mmol): LCMS: M+NH4+=477.3, MS-=458 (acidic, 2min, ELSD); 1H NMR (400 MHz, DMSO-d6) δ 11.32 (s, 1H), 7.71 (s, 1H), 4.96 (d, J = 4.9 Hz, 1H), 4.89 (dd, J = 13.7, 4.8 Hz, 2H), 4.47 (t, J = 5.9 Hz, 1H), 4.22 (t, J = 5.0 Hz, 2H), 4.15 (d, J = 7.8 Hz, 1H), 3.90 - 3.81 (m, 1H), 3.67 (ddd, J = 12.0, 5.7, 2.0 Hz, 1H), 3.62 - 3.39 (m, 8H), 3.19 - 2.90 (m, 6H).

**Syntheis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((1-(2-(((N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamoyl)oxy)ethyl)-1H-1,2,3-triazol-4-yl)methoxy)methyl)benzyl)-1-methylpiperazin-1-ium trifluoroacetate (L63-P1)**

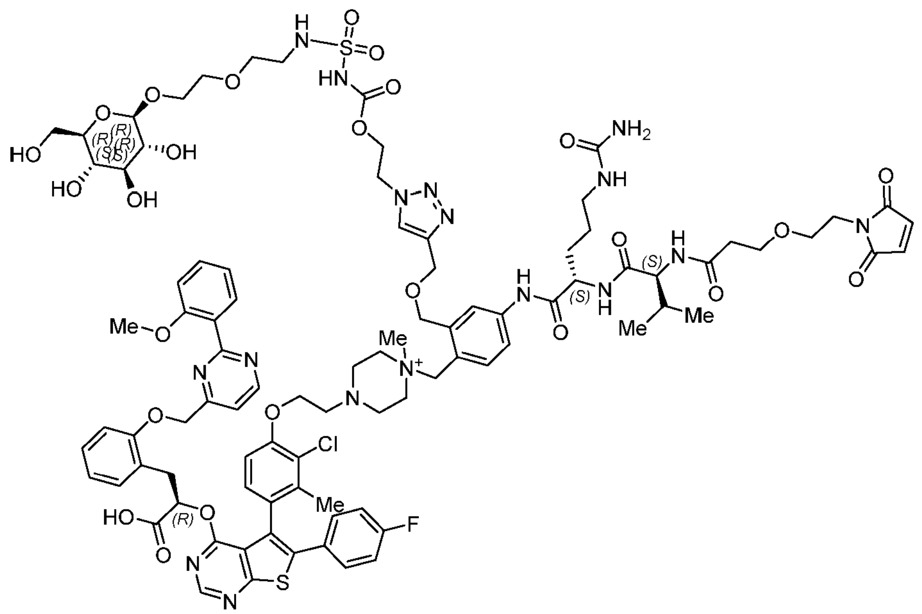

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (36 mg, 0.022 mmol) and 2-azidoethyl (N-(2-(2-(((2R,3R,4S,5S,6R)-3,4,5-trihydroxy-6-(hydroxymethyl)tetrahydro-2H-pyran-2-yl)oxy)ethoxy) ethyl)sulfamoyl)carbamate (22 mg, 0.048 mmol), 4-(2-(4-(4-((R)-1-carboxy-2-(2-((2-(2-methoxyphenyl)pyrimidin-4-yl) methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((1-(2-(((N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamoyl)oxy)ethyl)-1H-1,2,3-triazol-4-yl)methoxy)methyl)benzyl)-1-methylpiperazin-1-ium trifluoroacetate was obtained as a white powder: HRMS: MS+=1958.6899, Rt=2.31 min (5 min acidic); 1H NMR (400 MHz, DMSO-d6) δ 11.17 (s, 1H), 10.16 (s, 1H), 8.81 (d, J = 5.2 Hz, 1H), 8.54 (s, 1H), 8.07 (d, J = 13.5 Hz, 2H), 7.74 (d, J = 8.6 Hz, 1H), 7.68 (q, J = 4.3, 2.7 Hz, 3H), 7.56 (d, J = 5.1 Hz, 1H), 7.45 (dd, J = 7.6, 1.8 Hz, 1H), 7.42 - 7.28 (m, 3H), 7.23 (ddd, J = 8.5, 5.4, 2.6 Hz, 2H), 7.17 - 7.04 (m, 5H), 6.99 - 6.91 (m, 4H), 6.65 (t, J = 7.4 Hz, 1H), 6.14 (dd, J = 7.6, 1.8 Hz, 1H), 5.93 (s, 1H), 5.43 (dd, J = 9.8, 3.6 Hz, 1H), 5.23 - 5.11 (m, 2H), 4.59 (dd, J = 11.3, 7.4 Hz, 8H), 4.42-4.06 (m, 12H), 3.53 - 3.17 (m, 27H, overlapping with DMSO), 3.09 - 2.80 (m, 17H, overlapping with DMSO), 2.39 - 2.20 (m, 3H), 1.86 (h, J = 6.9 Hz, 1H), 1.77 (s, 3H), 1.68-1.21 (m, 5H), 0.76 (dd, J = 13.8, 6.8 Hz, 6H); 19F NMR (376 MHz, DMSO-d6): -112.18 ppm.

**Synthesis of (2R,3R,4S,5R,6R)-2-(acetoxymethyl)-6-((2,2-dimethyl-4-oxo-3,8,11-trioxa-5-azatridecan-13-yl)oxy)tetrahydro-2H-pyran-3,4,5-triyl triacetate**

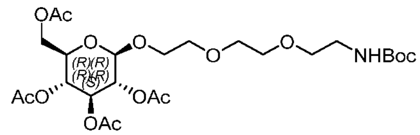

A mixture of tert-butyl (2-(2-(2-hydroxyethoxy)ethoxy)ethyl)carbamate (1046 mg, 4.20 mmol), $Ag_2CO_3$ (4627 mg, 8.39 mmol) and a piece of crystalline iodine in $CH_2Cl_2$ (3 mL) was stirred with powdered 4A molecule sieve (700 mg) for 15 min. To the mixture was added (2R,3R,4S,5R,6R)-2-(acetoxymethyl)-6-bromotetrahydro-2H-pyran-3,4,5-triyl triacetate (1150 mg, 2.80 mmol) in $CH_2Cl_2$ (3.00 mL) (also stirred with powdered 4A molecule sieve (700 mg) for 15 min). The resulting mixture was covered with aluminum foil and stirred at rt for 60 h, then filtered through celite with EtOAc washing. The filtrate was concentrated to give a clear oil. Purification via flash chromatography (20-100% EtOAc in heptane, ELSD detection) provided a 28/72% mixture of (2R,3R,4S,5R,6R)-2-(acetoxymethyl)-6-((2,2-dimethyl-4-oxo-3,8,11-trioxa-5-azatridecan-13-yl)oxy)tetrahydro-2H-pyran-3,4,5-triyl triacetate and (2*S*,3*aR*,5*R*,6*R*,7*S*,7*aR*)-5-(acetoxymethyl)-2-((2,2-dimethyl-4-oxo-3,8,11-trioxa-5-azatridecan-13-yl)oxy)-2-methyltetrahydro-5H-[1,3]dioxolo[4,5-b]pyran-6,7-diyl diacetate as a thick clear oil (307 mg, 0.529 mmol): LCMS: MS+=580.4, Rt=0.96 min (acid, 2 min, ELSD only); 1H NMR (400 MHz, DMSO-d6) δ 6.74 (s, 3H), 5.76 (s, 8H), 5.26 (t, J = 9.5 Hz, 1H), 5.02 (t, J = 3.1 Hz, 3H), 4.90 (t, J = 9.7 Hz, 1H), 4.84 - 4.72 (m, 5H), 4.37 (ddd, J = 5.2, 3.1, 0.9 Hz, 3H), 4.18 (dd, J = 12.2, 5.0 Hz, 1H), 4.11 (d, J = 4.5 Hz, 5H), 4.08 - 3.94 (m, 4H), 3.91 (dt, J = 8.6, 4.1 Hz, 3H), 3.85 - 3.75 (m, 1H), 3.66 - 3.58 (m, 1H), 3.58 - 3.51 (m, 7H), 3.42 - 3.31 (m, 14H), 3.06 (q, J = 5.9 Hz, 8H), 2.07 (s, 7H), 2.05 (s, 7H), 2.02 (s, 10H), 1.99 (d, J = 1.0 Hz, 6H), 1.99 (s, 3H), 1.94 (s, 3H), 1.63 (s, 7H), 1.38 (s, 36H), 1.18 (t, J = 7.1 Hz, 4H).

**Synthesis of (2R,3R,4S,5R,6R)-2-(acetoxymethyl)-6-(2-(2-(2-aminoethoxy)ethoxy)ethoxy)tetrahydro-2H-pyran-3,4,5-triyl triacetate**

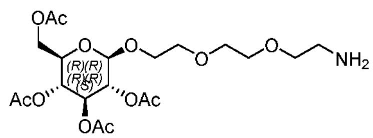

To a mixture of (2R,3R,4S,5R,6R)-2-(acetoxymethyl)-6-((2,2-dimethyl-4-oxo-3,8,11-trioxa-5-azatridecan-13-yl)oxy)tetrahydro-2H-pyran-3,4,5-triyl triacetate and (*2S*,*3aR*,*5R*,*6R*,*7S*,*7aR*)-5-(acetoxymethyl)-2-((2,2-dimethyl-4-oxo-3,8,11-trioxa-5-azatridecan-13-yl)oxy)-2-methyltetrahydro-5H-[1,3]dioxolo[4,5-b]pyran-6,7-diyl diacetate (323 mg, 0.557 mmol) in $CH_2Cl_2$ (8 mL) at 0°C was added TFA (1.9 mL, 25 mmol). After being stirred at rt for for 45 min, the mixture was concentrated and the residue was dried under vacuum for 60 min to give a light yellow oil. Purification via flash chromatography (0-20% MeOH in $CH_2Cl_2$, with 0.2% $NH_4OH$ modifier in MeOH, ELSD detection) afforded (2R,3R,4S,5R,6R)-2-(acetoxymethyl)-6-(2-(2-(2-aminoethoxy)ethoxy)ethoxy)tetrahydro-2H-pyran-3,4,5-triyl triacetate as a clear oil (82 mg, 0.171 mmol). LCMS: MS+=480.4, Rt=0.61 min (acidic, 2 min, ELSD); 1H NMR (400 MHz, DMSO-d6) δ 7.76 (s, 2H), 5.28 (t, J = 9.4 Hz, 1H), 4.93 (t, J = 9.7 Hz, 1H), 4.86 - 4.75 (m, 2H), 4.20 (dd, J = 12.2, 5.0 Hz, 1H), 4.08 - 3.95 (m, 2H), 3.88 - 3.79 (m, 1H), 3.67 - 3.53 (m, 11H, overlapping with DMSO), 3.00 (q, J = 5.5 Hz, 2H), 2.04 (s, 3H), 2.02 (s, 3H), 2.00 (s, 3H), 1.96 (s, 3H).

**Synthesis of 2-azidoethyl (N-(2-(2-(2-(((2R,3R,4S,5S,6R)-3,4,5-trihydroxy-6-(hydroxymethyl)tetrahydro-2H-pyran-2-yl)oxy)ethoxy)ethoxy)ethyl)sulfamoyl)carbamate**

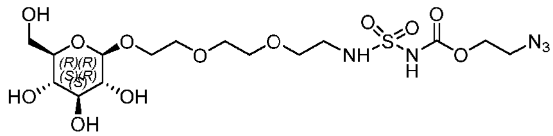

To 2-azidoethan-1-ol (12.5 mg, 0.144 mmol) in $CH_2Cl_2$ (2 mL) was added sulfurisocyanatidic chloride (0.012 ml, 0.14 mmol) at 0°C. The mixture was stirred at 0°C for 45 min, then TEA (0.040 ml, 0.29 mmol) and (2R,3R,4S,5R,6R)-2-(acetoxymethyl)-6-(2-(2-(2-aminoethoxy)ethoxy)ethoxy)tetrahydro-2H-pyran-3,4,5-triyl triacetate (77 mg, 0.16 mmol) in $CH_2Cl_2$ (1 mL) were added. After being stirred at 0°C for 1h, then at rt for 1 h, the mixture was quenched with satd. $NH_4Cl$, and 1 N HCl (0.29 mL). The aqueous was extracted with $CH_2Cl_2$ (5X). The organic layers were dried over anh. $Na_2SO_4$, filtered and concentrated via rotary evaporation to give (2R,3R,4S,5R,6R)-2-(acetoxymethyl)-6-(2-(2-(2-((N-((2-azidoethoxy)carbonyl)sulfamoyl)amino)ethoxy)ethoxy)ethoxy)tetrahydro-2H-pyran-3,4,5-triyl triacetate as a clear oil (75 mg): LCMS: 0.97 min, MS+=672.4, 96, Rt-0.87 min (acidic, 2min, ELSD).

To the product above in dioxane (4 mL) at 0°C was added LiOH.H2O (0.5 M in water, 3.45 ml, 1.72 mmol). The resulting clear solution was stirred at rt for 1 h and then was concentrated via rotary evaporation with 20°C water bath. The residue was purified by prep HPLC (Sunfire 5μm 30x50 mm column, 2-12% of Acetonitrile with 0.1% FA in Water. Flow Rate: 75 mL/min., MS 503.5, 520.3 detection) to provide, after lyophilization, 2-azidoethyl (N-(2-(2-(2-(((2R,3R,4S,5S,6R)-3,4,5-trihydroxy-6-(hydroxymethyl)tetrahydro-2H-pyran-2-yl)oxy)ethoxy)ethoxy)ethyl) sulfamoyl)carbamate as a clear thin film (22 mg, 0.044 mmol): LCMS: MS+=504.3, Rt=0.52 min (acidic, 2min, ELSD); 1H NMR (400 MHz, DMSO-d6) δ 11.31 (s, 1H), 7.74 (s, 1H), 4.96 (d, J = 4.9 Hz, 1H), 4.89 (dd, J = 12.5, 4.8 Hz, 2H), 4.47 (t, J = 5.9 Hz, 1H), 4.22 (t, J = 5.0 Hz, 2H), 4.15 (d, J = 7.8 Hz, 1H), 3.93 - 3.82 (m, 1H), 3.67 (dd, J = 11.2, 5.8 Hz, 1H), 3.62 - 3.40 (m, 12H), 3.17 - 2.90 (m, 6H).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((1-(2-(((N-(2-(2-(2-(((2R,3R,4S,5S,6R)-3,4,5-trihydroxy-6-(hydroxymethyl)tetrahydro-2H-pyran-2-yl)oxy)ethoxy)ethoxy)ethyl)sulfamoyl)carbamoyl)oxy)ethyl)-1H-1,2,3-triazol-4-yl)methoxy)methyl)benzyl)-1-methylpiperazin-1-ium trifluoroacetate (L62-P1)**

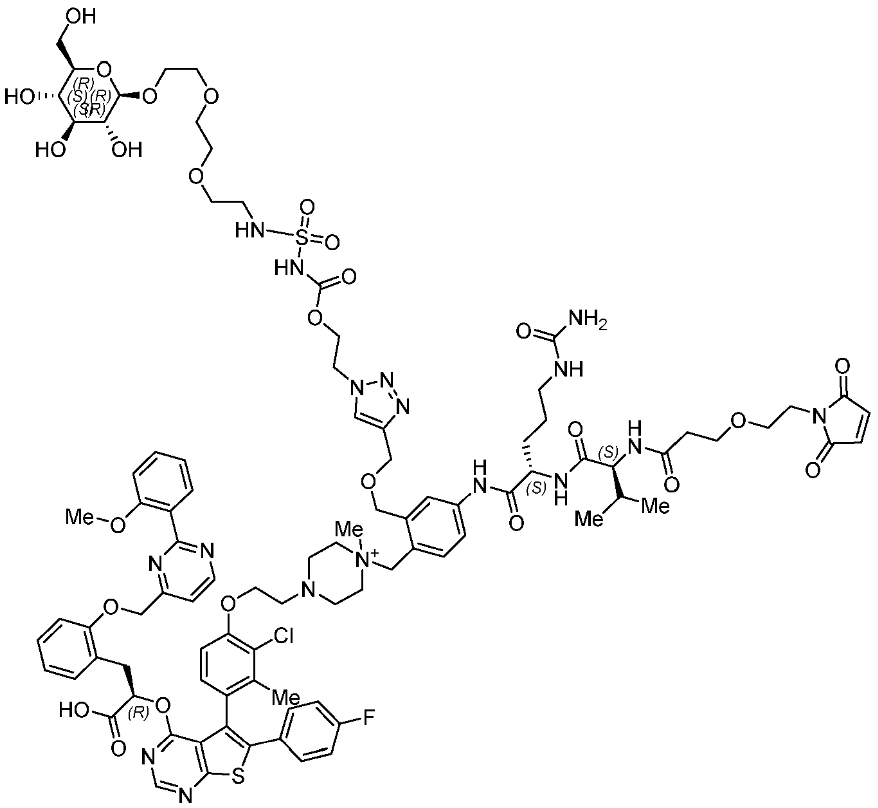

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (32 mg, 0.020 mmol) and 2-azidoethyl (N-(2-(2-(2-(((2S,3R,4S,5S,6R)-3,4,5-trihydroxy-6-(hydroxymethyl)tetrahydro-2H-pyran-2=yl)oxy) ethoxy)ethoxy)ethyl)sulfamoyl)carbamate (21 mg, 0.042 mmol), 4-(2-(4-(4-((R)-1-carboxy-2-(2-((2-(2-methoxyphenyl) pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((1-(2-(((N-(2-(2-(2-(((2R,3R,4S,5S,6R)-3,4,5-trihydroxy-6-(hydroxymethyl)tetrahydro-2H-pyran-2-yl)oxy)ethoxy)ethoxy)ethyl)sulfamoyl)carbamoyl)oxy)ethyl)-1H-1,2,3-triazol-4-yl)methoxy)methyl)benzyl)-1-methylpiperazin-1-ium trifluoroacetate was obtained as a white powder: HRMS: MS+=2002.7100, Rt=2.37 min (5 min acidic).

**Synthesis of Di-tert-butyl 3,3'-((3-((((9H-fluoren-9-yl)methoxy)carbonyl)amino)propanoyl)azanediyl)dipropionate**

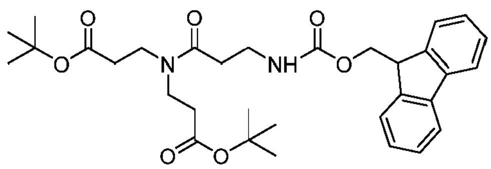

To a mixture of di-tert-butyl 3,3'-azanediyldipropionate (403 mg, 1.47 mmol), 3-((((9H-fluoren-9-yl)methoxy) carbonyl)amino)propanoic acid (505 mg, 1.62 mmol) and DIPEA (0.309 mL, 1.77 mmol) in $CH_2Cl_2$ (3 mL) was added N-(Dimethylaminopropyl)-N'-ethylcarbodiimide hydrochloride.HCl (367 mg, 1.92 mmol). After being stirred at rt for 2h, the mixture was quenched with satd. $NH_4Cl$, and extracted with $CH_2Cl_2$ (3X). The combined organic phase was washed with brine, dried over anh. $Na_2SO_4$, filtered and concentrated. The resulting crude product was purified by flash chromatography (0-50% EtOAc in heptane) to provide di-tert-butyl 3,3'-((3-((((9H-fluoren-9-yl)methoxy)carbonyl)amino)propanoyl) azanediyl)dipropionate as a white foam (290 mg, 0.511 mmol): LCMS: MS+=567.5, Rt=1.32 min (acid, 2 min); PMR: 1H

NMR (400 MHz, DMSO-d6) δ 7.94 - 7.88 (m, 2H), 7.70 (d, J = 7.5 Hz, 2H), 7.47 - 7.40 (m, 2H), 7.40 - 7.31 (m, 2H), 7.22 (t, J = 5.7 Hz, 1H), 4.31 (d, J = 6.8 Hz, 2H), 4.26 - 4.18 (m, 1H), 3.56 - 3.03 (m, 8H), 2.50 - 2.38 (m, 4H), 1.41 (s, 9H), 1.40 (s, 9H).

**Synthesis of Di-tert-butyl 3,3'-((3-aminopropanoyl)azanediyl)dipropionate, 1-(9H-fluoren-9-yl)-N,N-dimethylmethanamine**

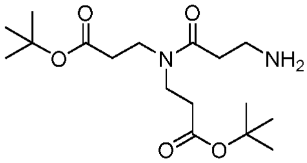

To di-tert-butyl 3,3'-((3-((((9H-fluoren-9-yl)methoxy)carbonyl)amino)propanoyl)azanediyl)dipropionate (288 mg, 0.508 mmol) in $CH_2Cl_2$ (3 mL) was added dimethylamine (2 N in THF, 1 mL, 2 mmol). The mixture was stirred at rt for 1 h. More dimethylamine (2 N in THF, 1 mL, 2 mmol) was added. After being stirred for additional 4 h, the mixture was concentrated and the residue was purified by flash chromatography (0-25% MeOH in $CH_2Cl_2$, 37 min, 0.2% $NH_4OH$ modifier was in MeOH, ELSD detection) to provide di-tert-butyl 3,3'-((3-aminopropanoyl)azanediyl)dipropionate, 1-(9H-fluoren-9-yl)-N,N-dimethylmethanamine as a light brown oil (62 mg, 0.472 mmol): LCMS: MS+=345.4, Rt=0.76 min (acidc, 2 min, ELSD); 1H NMR (400 MHz, DMSO-d6) δ 4.05 (s, 2H), 3.51 (t, *J* = 7.2 Hz, 2H), 3.43 (t, *J* = 7.3 Hz, 2H), 3.31 (s, 2H), 2.79 (t, *J* = 6.4 Hz, 2H), 2.55 - 2.45 (m,17H, overlapping with DMSO), 2.41 (t, *J* = 7.3 Hz, 2H), 1.41 (s, 9H), 1.40 (s, 9H).

**Synthesis of 3,3'-((3-((N-((2-azidoethoxy)carbonyl)sulfamoyl)amino)propanoyl)azanediyl)dipropionic acid**

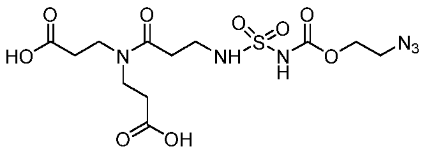

To 2-azidoethan-1-ol (16 mg, 0.18 mmol) in $CH_2Cl_2$ (2.5 ml) was added sulfurisocyanatidic chloride (0.016 ml, 0.18 mmol) at 0°C. The mixture was stirring at 0°C for 30 min, then TEA (0.051 ml, 0.37 mmol) and di-tert-butyl 3,3'-((3-aminopropanoyl)azanediyl)dipropionate (73 mg, 0.21 mmol) in $CH_2Cl_2$ (1 mL). After being stirred at 0°C for 1h and then rt for 1 h, the mixture was quenched with Satd $NH_4Cl$, and 1 N HCl (0.37 mL). The aqueous was extracted with $CH_2Cl_2$ (5X). The organic layers were dried over anh. $Na_2SO_4$, filtered and concentrated via rotary evaporation. The resulting residue was purified by flash chromatography (0-10% MeOH in $CH_2Cl_2$ , ELSD and UV214 detection) to provide di-tert-butyl 3,3'-((3-((N-((2-azidoethoxy)carbonyl)sulfamoyl)amino)propanoyl)azanediyl)dipropionate, as a thick clear oil (70 mg, 0.13 mmol): LCMS MS+= 537.4 , Rt=1.08 min (acidic, 2 min, ELSD); 1H NMR (400 MHz, DMSO-d6) δ 11.39 (s, 1H), 7.65 - 7.59 (m, 1H), 4.28 - 4.23 (m, 2H), 3.63 - 3.59 (m, 2H), 3.54 - 3.47 (m, 2H), 3.47 - 3.38 (m, 2H), 3.18 - 3.09 (m, 2H), 2.61 - 2.54 (m, 4H), 2.43 (dd, J = 8.5, 6.0 Hz, 2H), 1.43 (s, 9H), 1.42 (s, 9H).

To the product above in $CH_2Cl_2$ (2 ml) at 0°C was added TFA (2 ml). After being stirred at rt for 1.5 h, the mixture was concentrated via rotary evaporation at 25 °C water bath. The residue was dried in high vac for 30 min, then by azeotropic distillation with anh. Toluene (3X 3 mL), and further dried in high vac overnight to provide 3,3'-((3-((N-((2-azidoethoxy)carbonyl)sulfamoyl)amino)propanoyl)azanediyl)dipropionic acid as a white solid (72 mg, 77% by weight based on theoretical yield. It was used directly in the next step): LCMS MS+=425.3 , Rt=0.52 min (acidic, 2 min, ELSD); 1H NMR (400 MHz, DMSO-d6) δ 12.28 (s, 1H), 11.35 (s, 1H), 7.58 (t, J = 5.8 Hz, 1H), 4.26 - 4.20 (m, 2H), 3.61 - 3.55 (m, 2H), 3.50 (t, J = 7.4 Hz, 2H), 3.42 (t, J = 7.4 Hz, 2H), 3.12 (q, J = 6.8 Hz, 2H), 2.56 (dd, J = 15.1, 7.4 Hz, 4H), 2.43 (t, J = 7.4 Hz, 2H), 2.08 (s, 1H).

**Synthesis of 1-(2-(((1-(2-(((N-(3-(bis(2-carboxyethyl)amino)-3-oxopropyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium trifluoroacetate (L61-P1)**

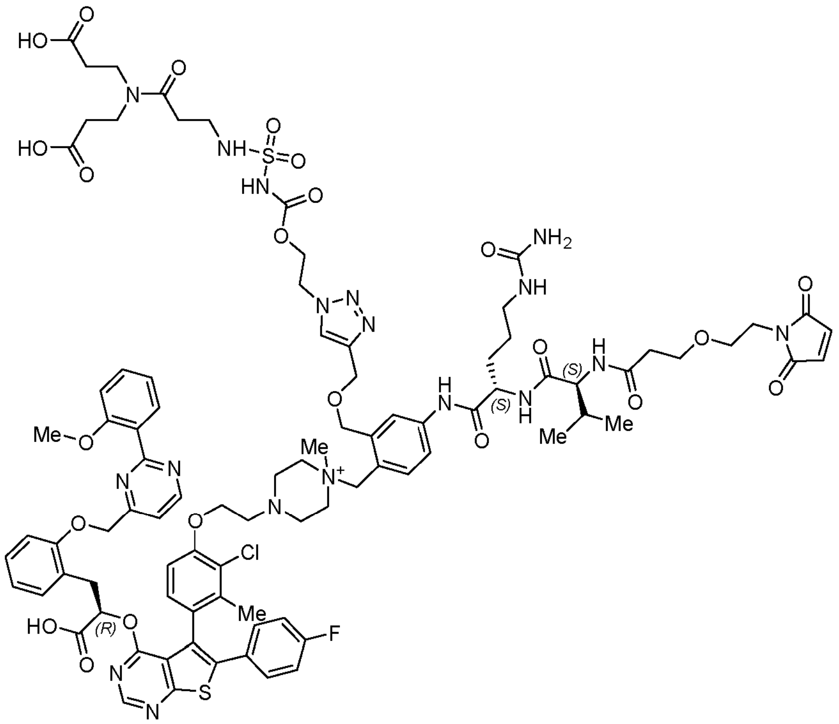

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (20 mg, 0.012 mmol) and 3,3'-((3-((N-((2-azidoethoxy)carbonyl)sulfamoyl)amino)propanoyl)azanediyl)dipropionic acid (15 mg, 0.027 mmol), 1-(2-(((1-(2-(((N-(3-(bis(2-carboxyethyl)amino)-3-oxopropyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium trifluoroacetate was obtained as a white powder: HRMS: MS+=1923.6500 Rt=2.34 min (5 min acidic); 1H NMR (400 MHz, DMSO-d6) δ 11.21 (s, 1H), 10.16 (s, 1H), 8.81 (d, J = 5.1 Hz, 1H), 8.54 (s, 1H), 8.08 (d, J = 13.7 Hz, 2H), 7.77 - 7.50 (m, 5H), 7.47 - 7.20 (m, 6H), 7.18 - 7.04 (m, 5H), 6.99 - 6.90 (m, 4H), 6.65 (t, J = 7.4 Hz, 1H), 6.14 (dd, J = 7.6, 1.7 Hz, 1H), 5.92 (s, 1H), 5.43 (dd, J = 9.8, 3.5 Hz, 1H), 5.24 - 5.11 (m, 3H), 4.65 - 4.51 (m, 9H), 4.45 - 3.81 (m, 52H, overlapping with DMSO), 3.68 (s, 3H), 3.54 - 2.77 (m, 31H), 2.39 - 2.20 (m, 5H), 1.86 (q, J = 6.8 Hz, 1H), 1.77 (s, 3H), 1.69 - 1.00 (m, 6H), 0.76 (dd, J = 13.9, 6.8 Hz, 6H); 19F NMR (376 MHz, DMSO-d6): -112.16 ppm.

**Synthesis of 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-amino-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

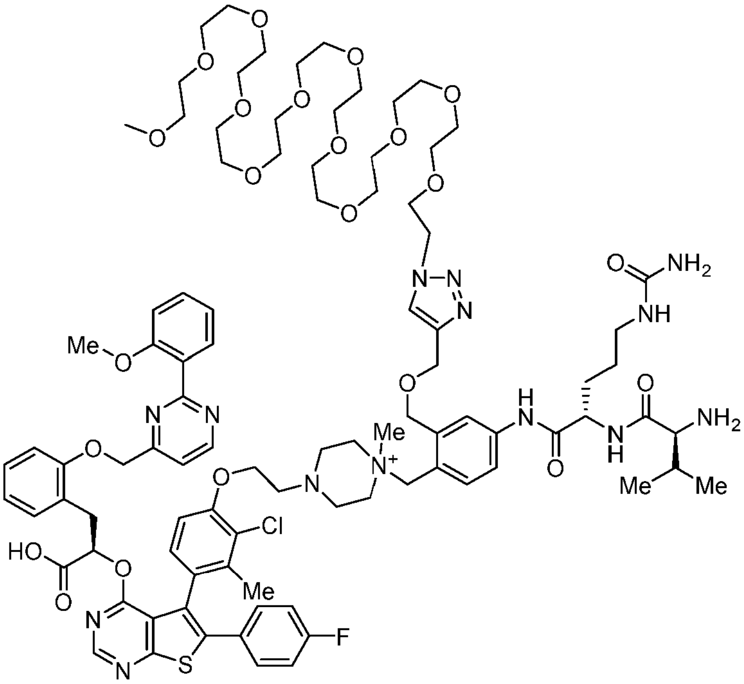

Following **GENERAL PROCEDURE 2** with 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (87.5 mg, 0.063 mmoles, 1.0 equiv) and mPEG12-Azide (73.3 mg, 0.125 mmol, 2 equiv), 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-amino-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 1889.8544, Rt=2.19 min (5 min acidic method).

**Synthesis of 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L104-P1)**

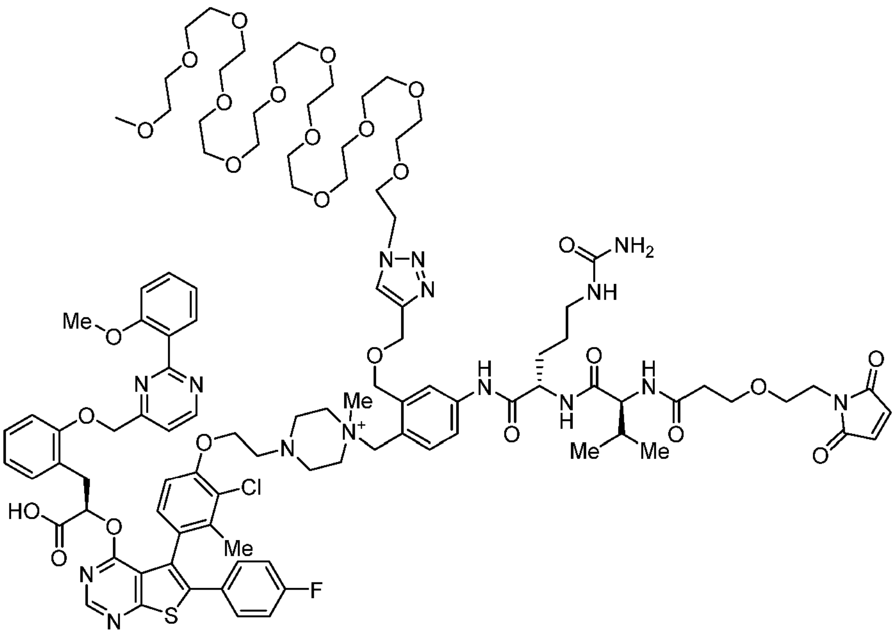

Following GENERAL PROCEDURE 3 with 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-amino-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (22 mg, 0.011 mmol, 1.0 equiv.), 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 2084.9099, Rt=2.45 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium**

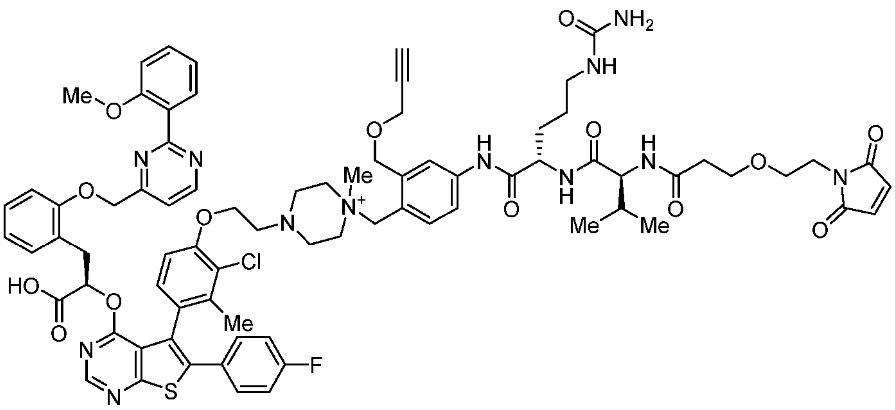

Following **GENERAL PROCEDURE 3** with 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (155 mg, 0.119 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium was obtained. HRMS: M+= 1499.5699, Rt=2.39 min (5 min acidic method).

**Synthesis of 1-(2-(((1-(2,5,8,11,14,17,20,23,26,29,32,35,38,41,44,47-hexadecaoxanonatetracontan-49-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L34-P1)**

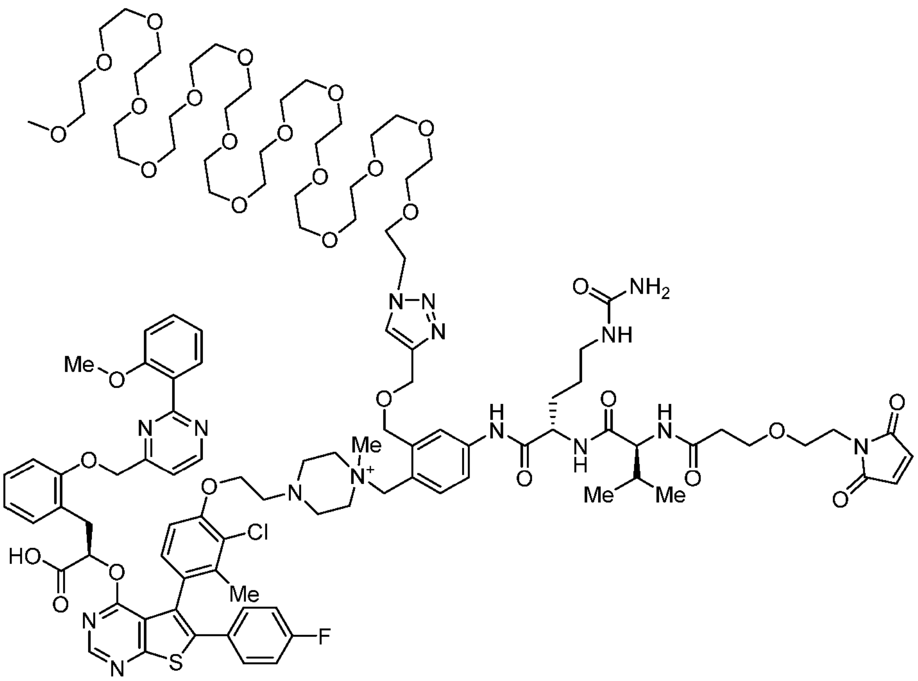

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium (50 mg, 0.033 mmoles, 1.0 equiv) and m-PEG16-azide (from Broadpharm BP-23558) (50.8 mg, 0.067 mmol, 2 equiv), 1-(2-(((1-(2,5,8,11,14,17,20,23,26,29,32,35,38,41,44,47-hexadecaoxanonatetracontan-49-yl)-1H-1,2,3-triazol-4-yl) methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=2261.0196, Rt=2.28 min (5 min acidic method).

**Synthesis of 1-(4-((S)-2-((S)-2-amino-3-methylbutanamido)-5-ureidopentanamido)-2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

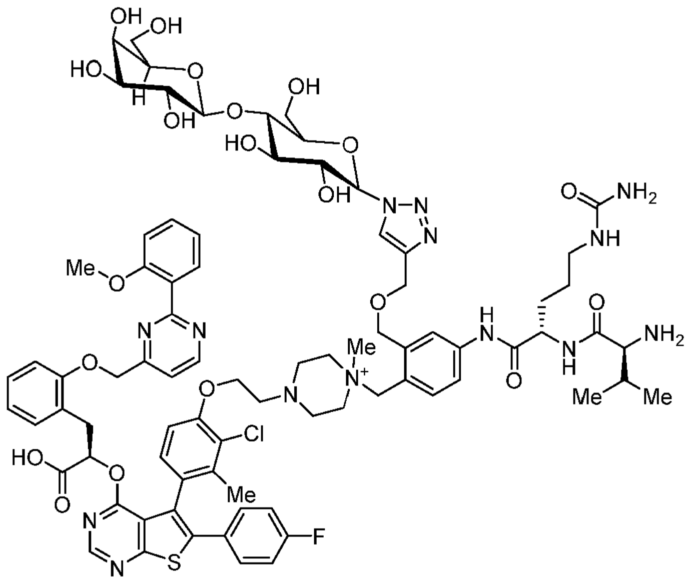

Following **GENERAL PROCEDURE 2** with 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (87.5 mg, 0.063 mmoles, 1.0 equiv) and 1-azido-1-deoxy-beta-D-lactopyranoside (22.99 mg, 0.063 mmol, 1 equiv), 1-(4-((S)-2-((S)-2-amino-3-methylbutanamido)-5-ureidopentanamido)-2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 1671.6400, Rt=1.95 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L46-P1)**

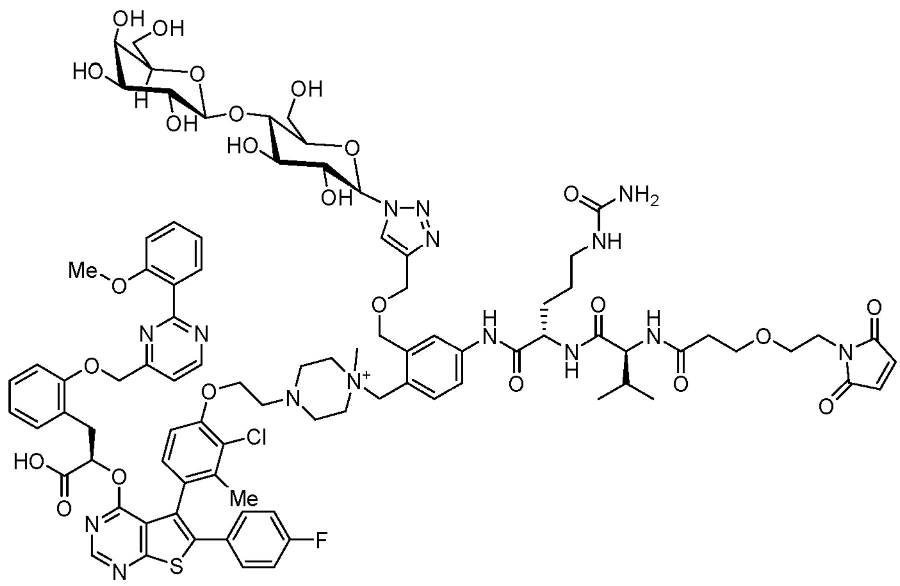

Following **GENERAL PROCEDURE 3** with 1-(4-((S)-2-((S)-2-amino-3-methylbutanamido)-5-ureidopentanamido)-2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-methylpiperazin-1-ium (35 mg, 0.020 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno [2,3-d]pyrimidin-5-yl)-2-chloro-3-methylphenoxy)ethyl)-1-(2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1866.6899, Rt=2.28 min (5 min acidic method).

**Synthesis 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((6S,9S,12S)-9-isopropyl-2,2-dimethyl-4,7,10-trioxo-6-(prop-2-yn-1-yl)-12-(3-ureidopropyl)-3-oxa-5,8,11-triazatridecan-13-amido)-2-((prop-2-yn-1-yloxy)methyl)benzyl)-1-methylpiperazin-1-ium**

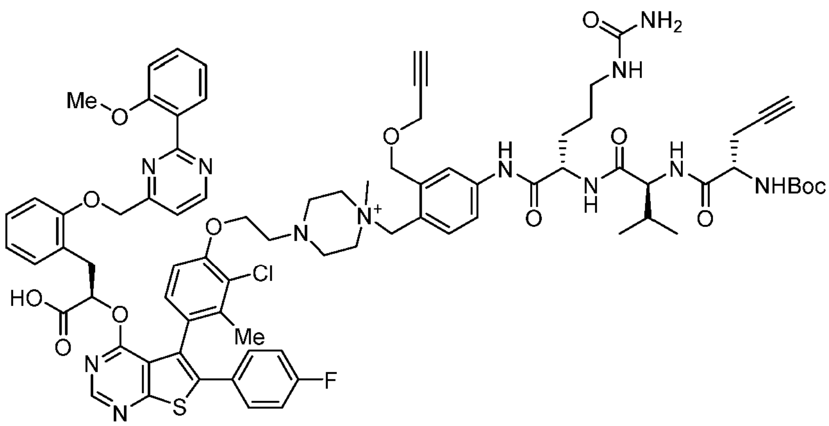

To the mixture of Boc-Propargyl-Gly-OH (40 mg, 0.188 mmol, 1 equiv) and HATU (71.3 mg, 0.188 mmol, 1 equiv) in DMF (0.5 ml) was added DIPEA (65.5 μl, 0.375 mmol, 2 equiv). The mixture was stirred at RT for 30 min. Then a solution of 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-methylpiperazin-1-ium (245 mg, 0.188 mmol, 1 equiv) in DMF (1 ml) was added into the reaction mixture. The reaction mixture was stirred at RT for 30min. The crude mixture was separated with C18 column (100 cartridge, MeCN/Water with 0.1% Formic Acid, 0-100% over 15CV) to obtain 4-(2-(4-(4-((R)-1-carboxy-2-(2-((2-(2-methoxyphenyl)pyrimidin-4-yl)methoxy)phenyl)ethoxy)-6-(4-fluorophenyl)thieno [2,3-d]pyrimidin-5-yl)-2-chloro-3-methylphenoxy)ethyl)-1-(4-((6S,9S,12S)-9-isopropyl-2,2-dimethyl-4,7,10-trioxo-6-(prop-2-yn-1-yl)-12-(3-ureidopropyl)-3-oxa-5,8,11-triazatridecan-13-amido)-2-((prop-2-yn-1-yloxy)methyl)benzyl)-1-methylpiperazin-1-ium. HRMS: M+= 1499.6000 , Rt= 2.91 min (5 min acidic method).

**Synthesis 1-(4-((S)-2-((S)-2-((S)-2-aminopent-4-ynamido)-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl) methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

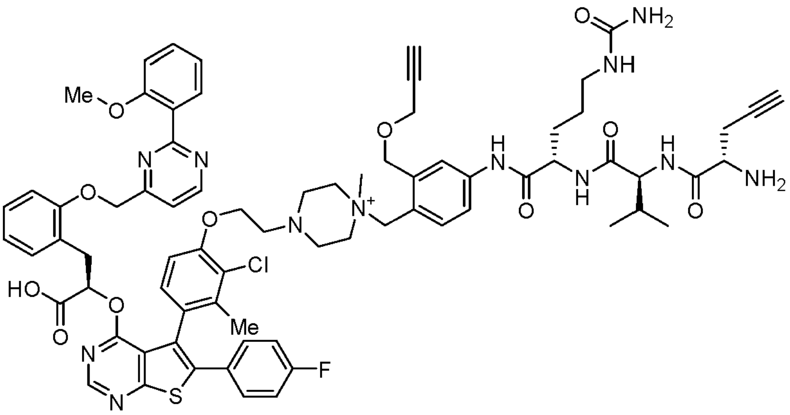

At 0°C ice-water bath, to 4-(2-(4-(4-((R)-1-carboxy-2-(2-((2-(2-methoxyphenyl)pyrimidin-4-yl)methoxy)phenyl) ethoxy)-6-(4-fluorophenyl)thieno[2,3-d]pyrimidin-5-yl)-2-chloro-3-methylphenoxy)ethyl)-1-(4-((6S,9S,12S)-9-isopropyl-2,2-dimethyl-4,7,10-trioxo-6-(prop-2-yn-1-yl)-12-(3-ureidopropyl)-3-oxa-5,8,11-triazatridecan-13-amido)-2-((prop-2-yn-1-yloxy)methyl)benzyl)-1-methylpiperazin-1-ium (56 mg, 0.037 mmol) was added TFA (25% in DCM) 2 mL, Then the reaction mixture was raised to RTand stirred for 1h. The crude mixture was concentrated under high vacuum . Then the mixture was disolved in MeOH, and was purified by C-18 column(50 g cartridge, MeCN/Water with 0.1% Formic Acid 0-100% over 16 CV) to obtain 1-(4-((S)-2-((S)-2-((S)-2-aminopent-4-ynamido)-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl) methoxy)phenyl)ethoxy)-6-(4-fluorophenyl)thieno[2,3-d]pyrimidin-5-yl)-2-chloro-3-methylphenoxy)ethyl)-1-methylpiperazin-1-ium. HRMS: M+= 1399.5400 , Rt= 2.17 min (5 min acidic method).

**Synthesis of 1-(4-((S)-2-((S)-2-((S)-2-amino-3-(1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)propanamido)-3-methylbutanamido)-5-ureidopentanamido)-2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium**

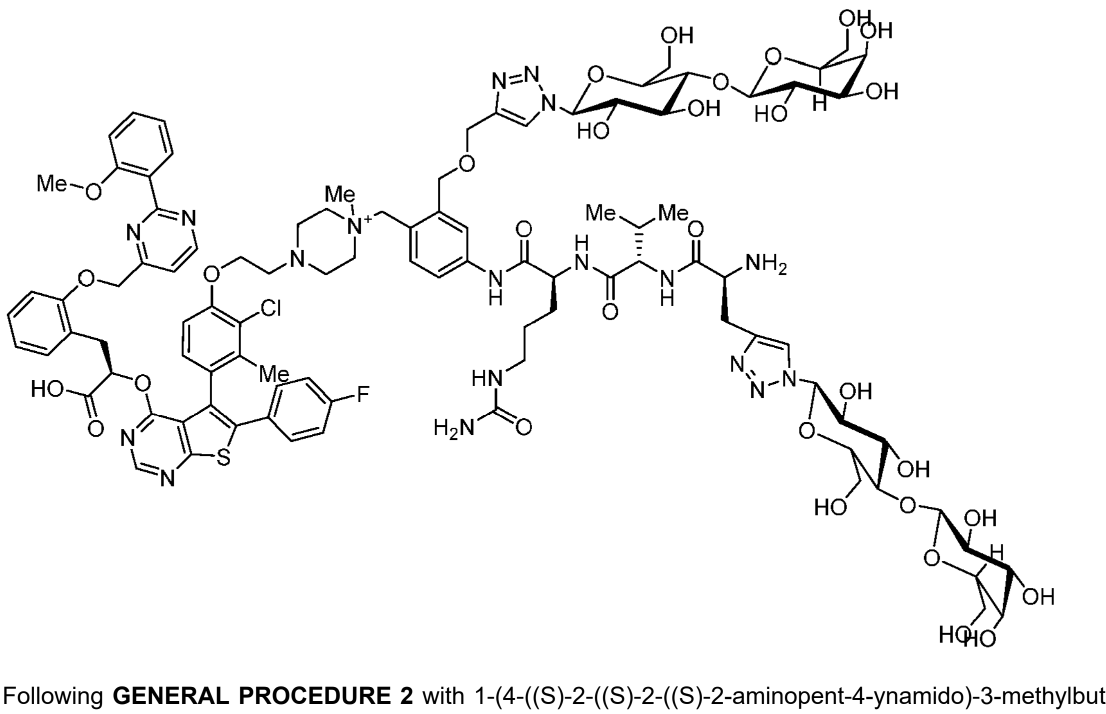

Following **GENERAL PROCEDURE 2** with 1-(4-((S)-2-((S)-2-((S)-2-aminopent-4-ynamido)-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy) ethyl)-1-methylpiperazin-1-ium (44 mg, 0.031 mmol, 1.0 equiv) and 1-azido-1-deoxy-beta-D-lactopyranoside (69.2 mg, 0.188 mmol, 6 eq) , 1-(4-((S)-2-((S)-2-((S)-2-amino-3-(1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)propanamido)-3-methylbutanamido)-5-ureidopentanamido)-2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-methylpiperazin-1-ium was obtained. HRMS: M+= 2133.7800 , Rt= 1.95 min (5 min acidic method).

**Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl) oxy)tetrahydro-2H-pyran-2-yl)-1H-1,2,3-triazol-4-yl)methoxy)methyl)-4-((2S,5S,8S)-8-((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methyl)-15-(2,5-dioxo-2,5-dihydro-1H-pyrrol-1-yl)-5-isopropyl-4,7,10-trioxo-2-(3-ureidopropyl)-13-oxa-3,6,9-triazapentadecanamido)benzyl)-1-methylpiperazin-1-ium (L47-P1)**

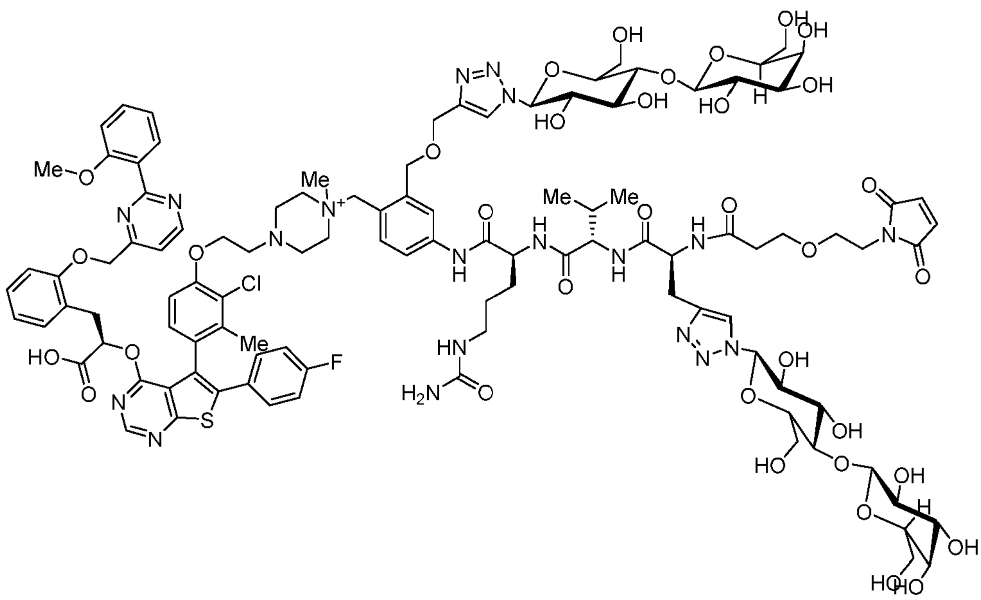

Following **GENERAL PROCEDURE 3** with 1-(4-((S)-2-((S)-2-((S)-2-amino-3-(1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)propanamido)-3-methylbutanamido)-5-ureidopentanamido)-2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl) thieno[2,3-d]pyrimidin-5-yl)-2-chloro-3-methylphenoxy)ethyl)-1-methylpiperazin-1-ium (19 mg, 0.009 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno [2,3-d]pyrimidin-5-yl)-2-chloro-3-methylphenoxy)ethyl)-1-(2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)-4-((2S,5S,8S)-8-((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methyl)-15-(2,5-dioxo-2,5-dihydro-1H-pyrrol-1-yl)-5-isopropyl-4,7,10-trioxo-2-(3-ureidopropyl)-13-oxa-3,6,9-triazapentadecanamido)benzyl)-1-methylpiperazin-1-ium was obtained. HRMS: M+= 2328.8301, Rt=2.15 min (5 min acidic method).

**1-(4-((S)-2-((S)-2-((tert-butoxycarbonyl)amino)-3-methylbutanamido)-5-ureidopentanamido)-2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium**

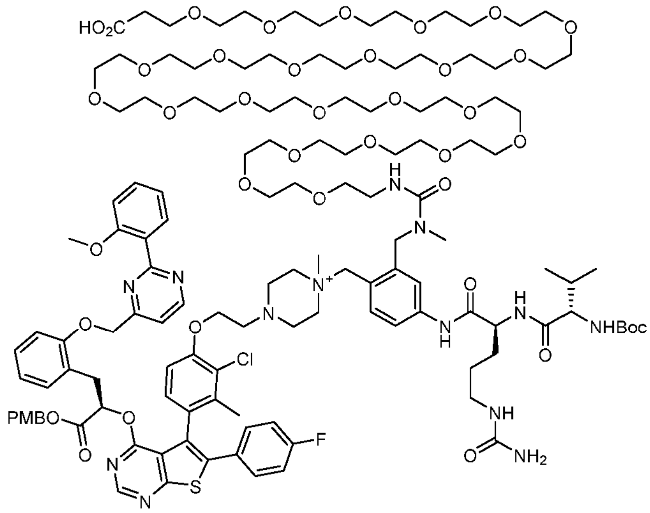

A mixture of 1-amino-3,6,9,12,15,18,21,24,27,30,33,36,39,42,45,48,51,54,57,60,63,66,69,72-tetracosaoxapentaheptacontan-75-oic acid (50 mg, 0.044 mmol), bis(4-nitrophenyl) carbonate (13 mg, 0.043 mmol), and DIPEA (20 μL, 0.12 mmol) in THF (2 mL) was stirred at RT for 2 h. The mixture was concentrated by blowing nitrogen gas to it. The resulting solid residue was taken up in DMF (1 mL). 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (50 mg, 0.029 mmol) and DIPEA (100 μL, 0.573 mmol) were added. The mixture was stirred at RT for 5 min. The mixture was diluted with DMSO (2 mL), and the solution was purified by RP-HPLC ISCO gold chromatography (MeCN/$H_2O$, 0.1% TFA modifier). Upon lyophilization, 1-(4-((S)-2-((S)-2-((tert-butoxycarbonyl)amino)-3-methylbutanamido)-5-ureidopentanamido)-2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 2671.2700, Rt=2.88 min (5 min acidic method).

**4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L42-P1)**

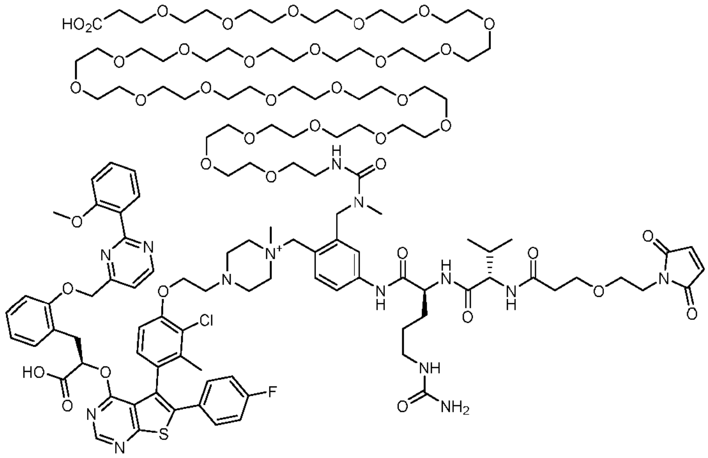

Following **GENERAL PROCEDURE 3** with1-(4-((S)-2-((S)-2-((tert-butoxycarbonyl)amino)-3-methylbutanamido)-5-ureidopentanamido)-2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium, 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L42-P1) was obtained. HRMS: M+= 2646.7700, Rt=2.38 min (5 min acidic method).

The following compounds were prepared using procedures similar to those described above.

| Code | Linker Payload Structure | Synthetic Methods | Characterizations |
|---|---|---|---|
| L33-P1 | 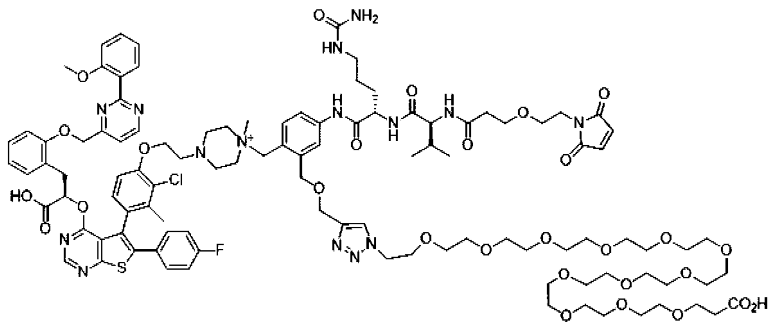 | General Procedure 2 | HRMS: M+ 2142.9099, rt = 2.39 min. (5 min acidic method). |
| L38-P1 | 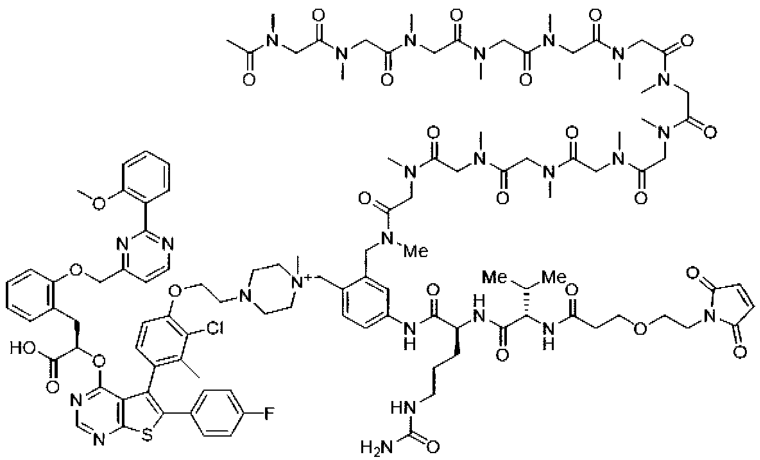 | Similar to synthesis of L35-P1 and using General Procedure 3 | HRMS: M+ 2369.0400, rt = 2.11 min. (5 min acidic method). |

(continued)

| Code | Linker Payload Structure | Synthetic Methods | Characterizations |
|---|---|---|---|
| L39-P1 | 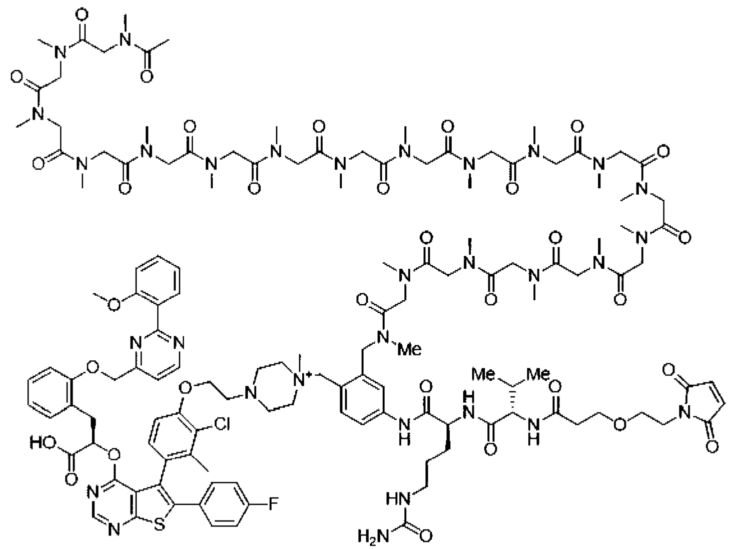 | Similar to synthesis of L35-P1 and using General Procedure 3 | HRMS: M+ 2795.2537, rt = 2.07 min. (5 min acidic method). |
| L40-P 1 | 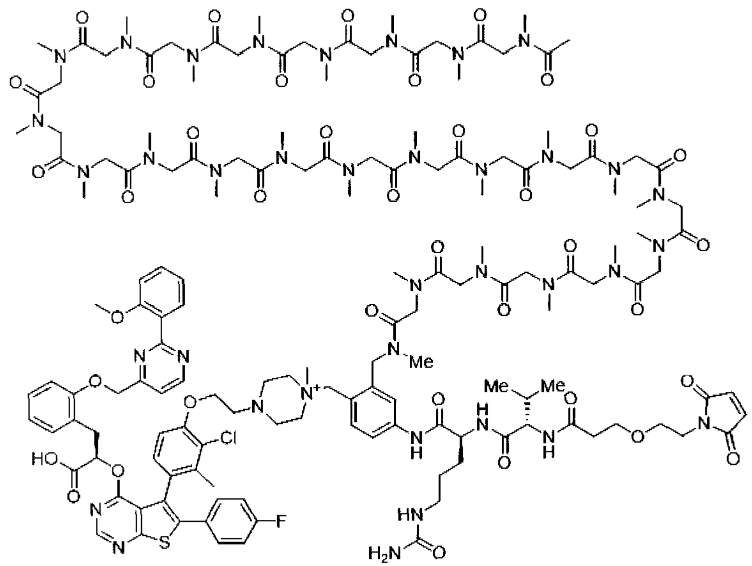 | Similar to synthesis of L35-P1 and using General Procedure 3 | HRMS: M+ 3221.4653, rt = 2.05 min. (5 min acidic method). |

(continued)

| Code | Linker Payload Structure | Synthetic Methods | Characterizations |
|---|---|---|---|
| L43-P1 | 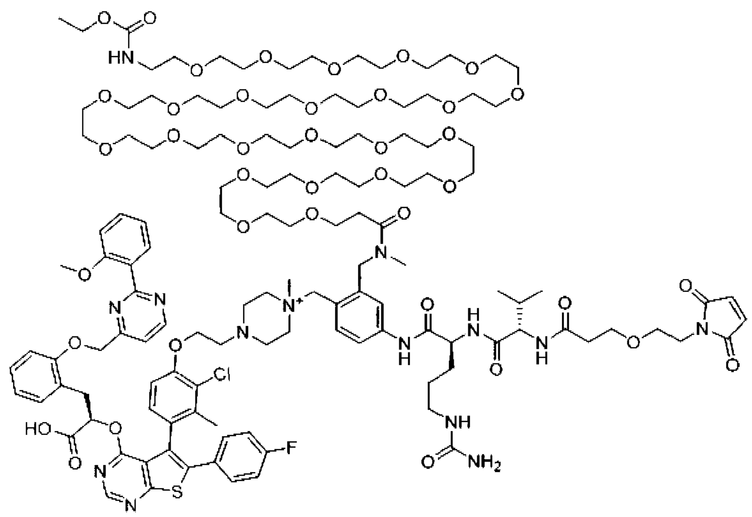 | Similar to synthesis of L35-P1 and using General Procedure 3 | HRMS: M+ 2673.7827, rt = 2.46 min. (5 min acidic method). |
| L44-P1 | 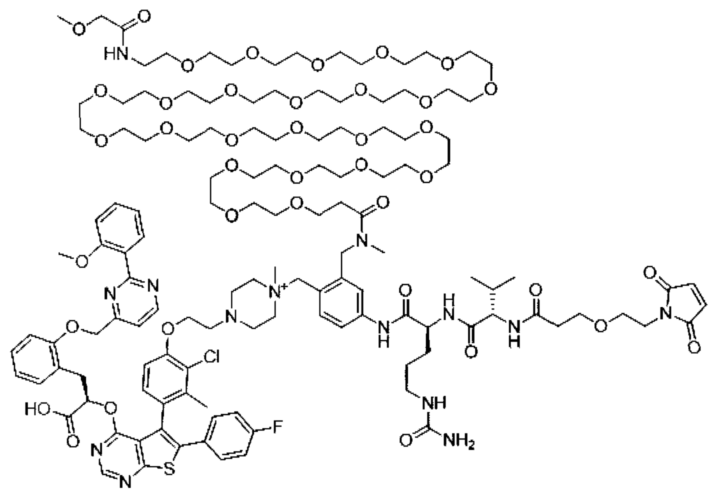 | Similar to synthesis of L35-P1 and using General Procedure 3 | HRMS: M+ 2673.7832, rt = 2.39 min. (5 min acidic method). |

(continued)

| Code | Linker Payload Structure | Synthetic Methods | Characterizations |
|---|---|---|---|
| L45-P1 | 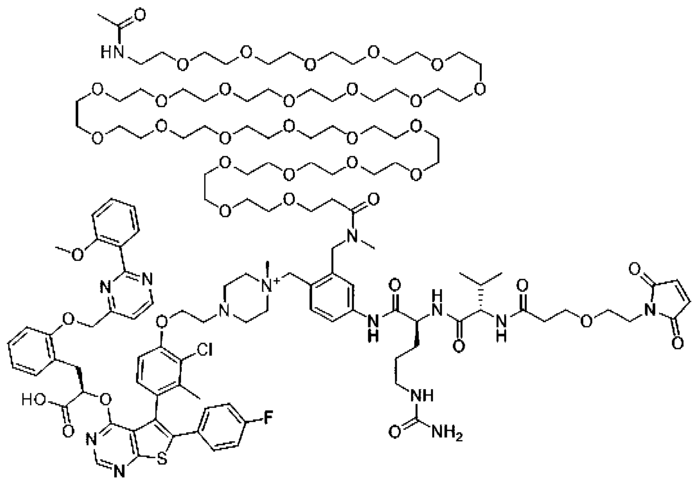 | Similar to synthesis of L35-P1 and using General Procedure 3 | HRMS: M+ 2644.2253, rt = 2.50 min. (5 min acidic method). |
| L95-P1 | 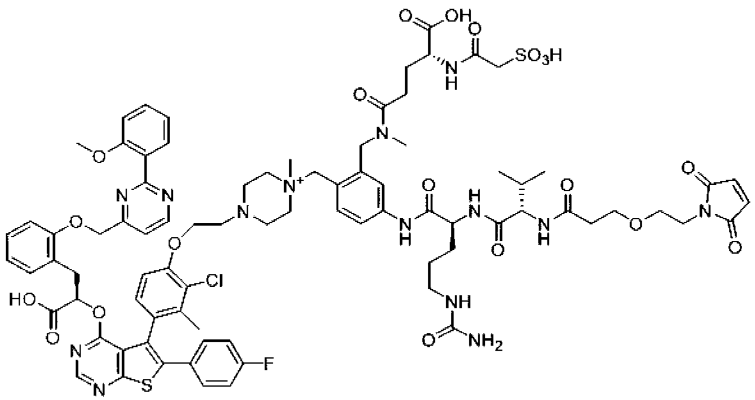 | Similar to synthesis of L71-P1 | HRMS: M+ 1725.6000 , rt = 2.32 min. (5 min acidic method) |

(continued)

| Code | Linker Payload Structure | Synthetic Methods | Characterizations |
|---|---|---|---|
| L101-P1 | 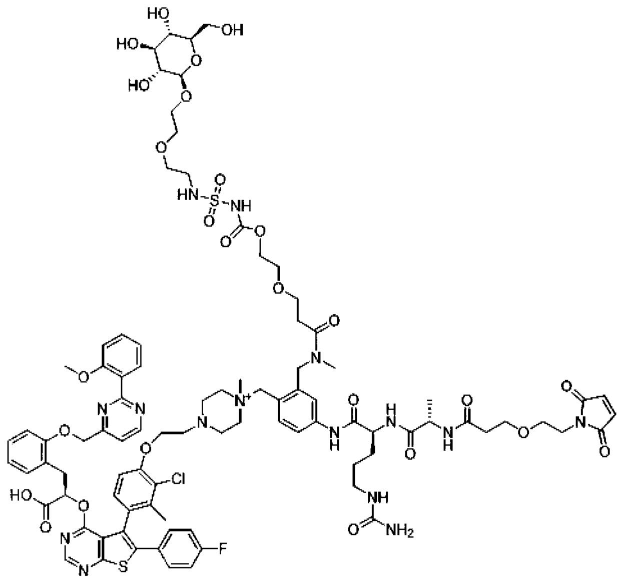 | Similar to synthesis of L62-P1 | HRMS: M+ 1962.7100, rt = 2.50 min. (5 min acidic method). |

(continued)

| Code | Linker Payload Structure | Synthetic Methods | Characterizations |
|---|---|---|---|
| L105-P1 | 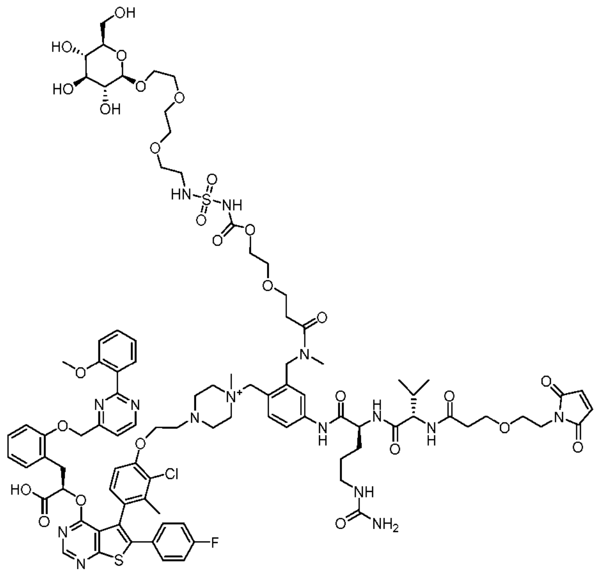 | Similar to synthesis of L62-P1 | HRMS: M+ 1962.7100, rt = 2.50 min. (5 min acidic method). |

The synthetic methods for preparing the polyethylene glycols in L43-P1, L44-P1 and L45-P1 are described below.

**Synthesis of 2-oxo-6,9,12,15,18,21,24,27,30,33,36,39,42,45,48,51,54,57,60,63,66,69,72,75-tetracosaoxa-3-azaoctaheptacontan-78-oic acid**

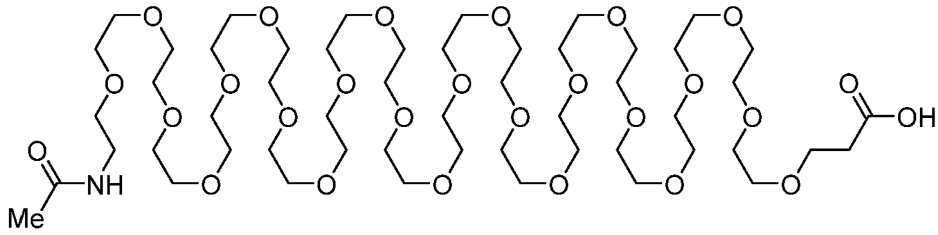

To a stirred solution of 1-amino-3,6,9,12,15,18,21,24,27,30,33,36,39,42,45,48,51,54,57,60,63,66,69,72-tetracosaoxapentaheptacontan-75-oic acid (100 mg, 0.087 mmol, 1.0 equiv.) and DIPEA (24.8 mg, 34 μL, 0.192 mmol, 2.2 equiv.) in dichloromethane (0.5 mL) was added acetic anhydride (8.9 mg, 8.25 μL, 1.0 equiv.). The resulting mixture was stirred at ambient temperature for 1.5 hours. The solvent was removed under reduced pressure. The resulting residue was taken up in DMSO (1 mL) and purified by RP-HPLC ISCO gold chromatography (10-100% MeCN/H2O, 0.1% TFA modifier). Upon lyophilization 2-oxo-6,9,12,15,18,21,24,27,30,33,36,39,42,45,48,51,54,57,60,63,66,69,72,75-tetracosaoxa-3-azaoctaheptacontan-78-oic acid (62.3 mg, 0.052 mmol, 60% yield) was obtained. LC/MS [M-H]- 1187.3 Rt=0.75 min. (2 min acidic method). 1H NMR (400 MHz, DMSO-d6) δ 7.86 (s, 1H), 3.60 (t, J = 6.4 Hz, 3H), 3.50 (d, J = 4.9 Hz, 91H), 3.40 (t, J = 5.9 Hz, 2H), 3.18 (q, J = 5.8 Hz, 2H), 2.44 (t, J = 6.4 Hz, 2H), 1.80 (s, 3H).

**Synthesis of 4-oxo-3,8,11,14,17,20,23,26,29,32,35,38,41,44,47,50,53,56,59,62,65,68,71,74,77-pentacosaoxa-5-azaoctacontan-80-oic acid**

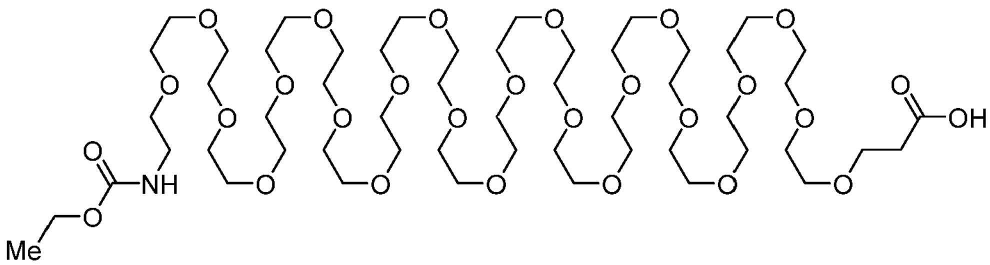

To a stirred solution of 1-amino-3,6,9,12,15,18,21,24,27,30,33,36,39,42,45,48,51,54,57,60,63,66,69,72-tetracosaoxapentaheptacontan-75-oic acid (100 mg, 0.087 mmol, 1.0 equiv.) and DIPEA (24.8 mg, 34 μL, 0.192 mmol, 2.2 equiv.) in dichloromethane (0.5 mL) was added ethyl chloroformate (9.5 mg, 8.34 μL, 1.0 equiv.). The resulting mixture was stirred at ambient temperature for 1.5 hours. The solvent was removed under reduced pressure. The resulting residue was taken up in DMSO (1 mL) and purified by RP-HPLC ISCO gold chromatography (10-100% MeCN/H2O, 0.1% TFA modifier). Upon lyophilization 4-oxo-3,8,11,14,17,20,23,26,29,32,35,38,41,44,47,50,53,56,59,62,65,68,71,74,77-pentacosaoxa-**5-azaoctacontan-80-oic** acid (75 mg, 0.062 mmol, 71% yield) was obtained. LC/MS [M-H]-1217.3 Rt=0.81 min. (2 min acidic method). 1H NMR (400 MHz, DMSO-d6) δ 7.03 (s, 1H), 3.97 (q, J = 7.1 Hz, 2H), 3.60 (t, J = 6.4 Hz, 2H), 3.50 (d, J = 5.0 Hz, 92H), 3.40 (t, J = 6.1 Hz, 2H), 3.11 (q, J = 5.9 Hz, 2H), 2.45 (q, J = 6.5 Hz, 2H), 1.15 (t, J = 7.1 Hz, 3H,).

**Synthesis of 4-oxo-2,8,11,14,17,20,23,26,29,32,35,38,41,44,47,50,53,56,59,62,65,68,71,74,77-pentacosaoxa-5-azaoctacontan-80-oic acid**

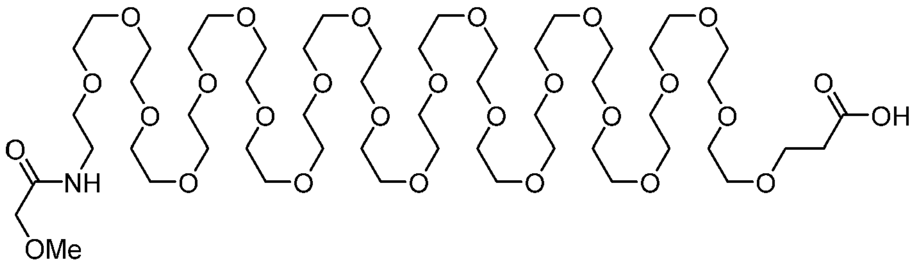

To a stirred solution of 1-amino-3,6,9,12,15,18,21,24,27,30,33,36,39,42,45,48,51,54,57,60,63,66,69,72-tetracosaoxapentaheptacontan-75-oic acid (100 mg, 0.087 mmol, 1.0 equiv.) and DIPEA (24.8 mg, 34 μL, 0.192 mmol, 2.2 equiv.) in dichloromethane (0.5 mL) was added methoxyacetyl chloride (11.36 mg, 9.57 μL, 1.2 equiv.). The resulting mixture was stirred at

ambient temperature for 1.5 hours. The solvent was removed under reduced pressure. The resulting residue was taken up in DMSO (1 mL) and purified by RP-HPLC ISCO gold chromatography (10-100% MeCN/H2O, 0.1% TFA modifier). Upon lyophilization 4-oxo-2,8,11,14,17,20,23,26,29,32,35,38,41,44,47,50,53,56,59,62,65,68,71,74,77-pentacosaoxa-**5-azaoctacontan-80-oic** acid (69 mg, 0.057 mmol, 65% yield) was obtained. LC/MS [M-H]-1217.4 Rt=0.75 min. (2 min acidic method). 1H NMR (400 MHz, DMSO-d6) δ 7.68 (s, 1H), 3.79 (s, 2H), 3.60 (t, J = 6.4 Hz, 2H), 3.51 (s, 92H), 3.43 (t, J = 6.0 Hz, 2H), 3.30 (s, 3H), 3.26 (q, J = 6.0 Hz, 2H), 2.44 (t, J = 6.4 Hz, 2H).

**Example 3. Synthesis and Characterization of Mcl-1 Payloads**

Exemplary payloads were synthesized using exemplary methods described in this example.

**Preparation of C1:**

**(2*R*)-2-{[(*5S$_a$*)-5-{3-chloro-2-methyl-4-[2-(4-methylpiperazin-1-yl)ethoxy]phenyl}-6-(4-fluorophenyl)thieno[2,3-*d*] pyrimidin-4-yl]oxy)-3-(2-([2-(2-methoxyphenyl)pyrimidin-4-yl]methoxy}phenyl)propanoic acid**

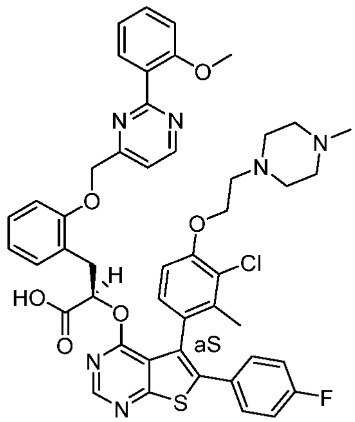

C1 was prepared according to Example 30 in WO 2015/097123.

Preparation of C2:

(2*R*)-2-[(5*S$_a$*)-5-[3-chloro-2-methyl-4-[2-[4-methyl-4-(3-sulfopropyl) piperazin-4-ium-1-yl]ethoxy]phenyl]-6-(4-fluoro-3-hydroxy-phenyl)thieno[2,3-d] pyrimidin-4-yl]oxy-3-[2-[[2-(2-methoxyphenyl)pyrimidin-4-yl]methoxy]phenyl]propanoic acid

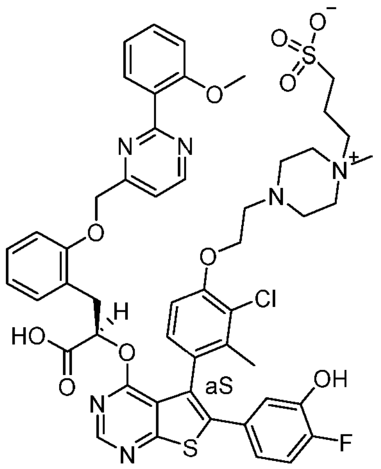

*Step A: 5-bromo-4-chloro-6-(4-fluoro-3-tetrahydropyran-2-yloxy-phenyl)thieno[2,3-d]pyrimidine*

4.49 g 5-bromo-4-chloro-6-iodo-thieno[2,3-d]pyrimidine (11.96 mmol; obtained according to WO 2015/097123, Preparation 1a) and 4.31 g (4-fluoro-3-tetrahydropyran-2-yloxy-phenyl)boronic acid (17.94 mmol) were dissolved in 60 mL THF, then 134 mg Pd(Oac)2 (0.60 mmol), 508 mg tBuXPhos (1.20 mmol), 11.69 g Cs2CO3 (35.88 mmol) and 60 mL water were added and the mixture was stirred at 70°C under N2 atmosphere until no further conversion was observed. Then it was diluted with water, neutralized with 2 M aqueous HCl solution, and extracted with DCM. The combined organic layer was dried over $Na_2SO_4$, filtered and the filtrate was concentrated under reduced pressure. The crude product product was purified via flash chromatography using heptane and EtOAc as eluents to give 5-bromo-4-chloro-6-(4-fluoro-3-tetrahydropyran-2-yloxy-phenyl)thieno[2,3-d]pyrimidine. $^1$H NMR (500 MHz, DMSO-$d_6$) δ: 9.02 (s, 1H), 7.64 (dd, *J* = 7.9, 2.1 Hz, 1H), 7.47 (dd, *J* = 11.0, 8.5 Hz, 1H), 7.36 (m, 1H), 5.63 (m, 1H), 3.81 (m, 1H), 3.61 (m, 1H), 1.94-1.78 (m, 3H), 1.69-1.50 (m, 3H). $^{13}$C NMR (125 MHz, DMSO-$d_6$) δ: 166.6, 153.9, 153.1, 152.7, 144.3, 139.2, 127.7, 126.6, 124.2, 119.9, 117.1, 100.7, 97.2, 61.6, 29.5, 24.5, 18.2. HRMS calculated for $C_{17}H_{13}N_2O_2SBrClF$: 441.9554; found 442.9624 (M+H).

*Step B: ethyl (2R)-2-[5-bromo-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate*

3.09 g 5-bromo-4-chloro-6-(4-fluoro-3-tetrahydropyran-2-yloxy-phenyl)thieno[2,3-d]pyrimidine (6.97 mmol), 3.28 g ethyl (2R)-2-hydroxy-3-[2-[[2-(2-methoxyphenyl)pyrimidin-4-yl]methoxy]phenyl]propanoate (8.02 mmol; obtained according to WO 2015/097123, Preparation 3bs) were dissolved in 70 mL tert-butanol, then 6.82 g $Cs_2CO_3$ (20.9 mmol) was added and the mixture was stirred under $N_2$ atmosphere at 70°C until no further conversion was observed. Then it was diluted with water, neutralized with 2 M aqueous HCl solution, and extracted with DCM. The combined organic layer was dried over $Na_2SO_4$, filtered and the filtrate was concentrated under reduced pressure. The crude product was purified via flash chromatography using heptane and EtOAc as eluents to give ethyl (2R)-2-[5-bromo-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate as a mixture of diastereoisomers. HRMS calculated for $C_{40}H_{36}N_4O_7SBrF$: 814.1472; found 815.1539 (M+H).

*Step C: ethyl (2R)-2-[5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate*

3.65 g ethyl (2R)-2-[5-bromo-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate (4.47 mmol) and 2.12 g 1-[2-[2-chloro-3-methyl-4-(4,4,5,5-tetramethyl-1,3,2-dioxaborolan-2-yl)phenoxy]ethyl]-4-methyl-piperazine (5.36 mmol; obtained according to WO 2015/097123, Preparation 5b) were dissolved in 22 mL dioxane, then 315 mg $PdCl_2 \times$AtaPhos (0.45 mmol), 4.37 g $Cs_2CO_3$ (13.41 mmol) and 22 mL water were added and the mixture was stirred at 70°C under $N_2$ atmosphere until complete conversion. Then it was diluted with water, neutralized with 2 M aqueous HCl solution, and extracted with EtOAc. The combined organic layer was dried over $Na_2SO_4$, filtered and the filtrate was concentrated under reduced pressure. The crude product product was purified via flash chromatography using EtOAc and MeOH, then DCM and MeOH as eluents to give ethyl (2R)-2-[5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate as a mixture of two diastereoisomer pairs. HRMS calculated for $C_{54}H_{56}N_6O_8SClF$:

1002.3553; found 1003.3614 and 1003.3622 (M+H).

*Step D: (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl) pyrimidin-4-yl]methoxy]phenyl]propanoic acid*

3.47 g ethyl (2R)-2-[5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate (3.46 mmol) was dissolved in 35 mL dioxane, then 1.45 g $LiOH\times H_2O$ (34.6 mmol) and 35 mL water were added. The mixture was stirred at room temperature until complete hydrolysis. Then it was diluted with water, acidified to pH 4 with 2 M aqueous HCl solution, and extracted with DCM. The combined organic phase was dried over $Na_2SO_4$, filtered and the filtrate was concentrated under reduced pressure. The atropisomers were purified and separated via preparative reverse phase chromatography using 25 mM aqueous $NH_4HCO_3$ solution and MeCN as eluents. The atropisomer pair eluting later was isolated as (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoic acid HRMS calculated for $C_{52}H_{52}N_6O_8SClF$: 974.3240; found 975.3303 (M+H).

*Step E: (4-methoxyphenyl)methyl (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate*

2.39 g (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoic acid (2.45 mmol), 1.13 g DTBAD (4.91 mmol) and 1.29 g $PPh_3$ (4.91 mmol) were dissolved in 49 mL toluene, then 0.61 mL PMB-OH (4.91 mmol) was added and the reaction mixture was stirred at 50°C until complete conversion. Then the mixture was diluted with DCM and then concentrated under reduced pressure and then purified via flash chromatography, using heptane and EtOAc as eluents to give (4-methoxyphenyl)methyl (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate as a mixture of diastereoisomers. HRMS calculated for $C_{60}H_{60}N_6O_9SClF$: 1094.3815; found 1095.3880 (M+H).

*Step F: (2R)-2-[(5$S_a$)-5-[3-chloro-2-methyl-4-[2-[4-methyl-4-(3-sulfopropyl) piperazin-4-ium-1-yl]ethoxy]phenyl]-6-(4-fluoro-3-hydroxy-phenyl)thieno[2,3-d] pyrimidin-4-yl]oxy-3-[2-[[2-(2-methoxyphenyl)pyrimidin-4-yl]methoxy]phenyl]propanoic acid (C2)*

600 mg (4-methoxyphenyl)methyl (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate (0.548 mmol) was dissolved in 11 mL MeCN, then 0.48 mL oxathiolane 2,2-dioxide (5.48 mmol) was added, and the mixture was stirred under $N_2$ atmosphere at 60°C until complete conversion. Then it was concentrated under reduced pressure, dissolved in 8 mL DCM, then 2.2 mL TFA was added and mixture was stirred at room temperature until complete cleavage of THP and PMB. Then it was concentrated (heating bath removed). It was dissolved in 10 mL THF, and concentrated again under reduced pressure in 30°C bath. The crude product was purified via preparative reverse phase chromatography using 5 mM aqueous $NH_4HCO_3$ solution and MeCN as eluents to obtain give C2. $^1H$ NMR (400 MHz, DMSO-$d_6$) δ: 13.19 (br s, 1H), 10.16 (br s, 1H), 8.89 (d, $J$ = 5.2 Hz, 1H), 8.58 (s, 1H), 7.68 (br s, 1H), 7.52 (dd, $J$ = 7.5, 1.8 Hz, 1H), 7.46 (m, 1H), 7.33 (d, $J$ = 8.3 Hz, 1H), 7.22-7.09 (m, 4H), 7.06-7.00 (m, 2H), 6.86 (dd, $J$ = 8.3, 2.0 Hz, 1H), 6.74 (t, $J$ = 7.4 Hz, 1H), 6.66 (m, 1H), 6.23 (d, $J$ = 6.7 Hz, 1H), 5.46 (dd, $J$ = 9.8, 3.3 Hz, 1H), 5.27 (d, $J$ = 15.2 Hz, 1H), 5.22 (d, $J$ = 15.2 Hz, 1H), 4.23 (m, 2H), 3.76 (s, 3H), 3.46 (m, 2H), 3.41-3.23 (m, 5H), 2.97 (s, 3H), 2.94-2.77 (m, 6H), 2.48 (m, 1H), 2.45 (t, $J$ = 7.0 Hz, 2H), 2.00-1.90 (m, 2H), 1.86 (s, 3H). $^{13}C$ NMR (100 MHz, DMSO-$d_6$) δ: 170.8, 166.2, 165.9, 164.7, 157.7, 157.2, 155.4, 153.6, 152.7, 152.3, 149.9, 145.1, 137.0, 135.9, 131.0, 130.8, 130.3, 129.2, 128.34, 128.32, 128.2, 128.0, 122.0, 120.5, 120.1, 118.8, 118.2, 116.6, 115.6, 112.2, 111.9, 110.6, 73.3, 69.0, 67.3, 59.2, 59.1, 55.71, 55.68, 47.6, 46.1, 31.8, 18.1, 17.6. HRMS calculated for $C_{50}H_{50}N_6O_{10}S_2ClF$: 1012.2703; found 1013.2775 (M+H).

**Preparation of C3:**

**(2R)-2-{[(5Sa)-5-{3-chloro-2-methyl-4-[2-(piperazin-1-yl)ethoxy]phenyl}-6-(4-fluorophenyl)thieno[2,3-d]pyrimidin-4-yl]oxy}-3-(2-{[2-(2-methoxyphenyl)pyrimidin-4-yl]methoxy}phenyl)propanoic acid**

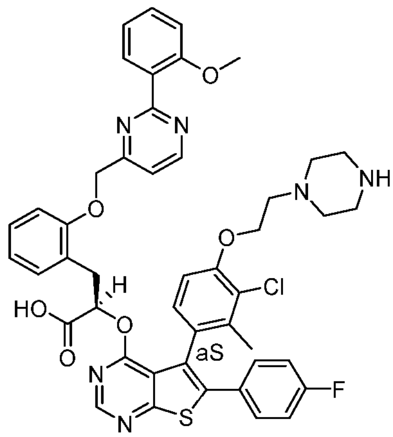

C3 was prepared according to Example 744 in WO 2015/097123.

**Preparation of C4:**

**(2R)-2-{[(5Sa)-5-{3-chloro-2-methyl-4-[2-(piperazin-1-yl)ethoxy]phenyl}-6-(4-fluorophenyl)thieno[2,3-d]pyrimidin-4-yl]oxy}-3-(2-{[2-(2-methoxyphenyl)pyrimidin-4-yl]methoxy}phenyl)propanoic acid**

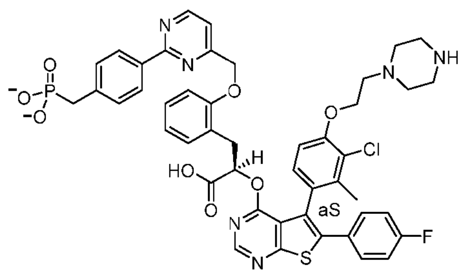

*Step 1: ethyl (2R)-2-[5-bromo-6-(4-fluorophenyl)thieno[2,3-d]pyrimidin-4-yl]oxy-3-[2-[(2-chloropyrimidin-4-yl)methoxy]phenyl]propanoate*

To a solution of ethyl (2R)-3-[2-[(2-chloropyrimidin-4-yl)methoxy]phenyl]-2-hydroxy-propanoate (25 g, 74.2 mmol) in THF (38 mL) were successively added 5-bromo-6-(4-fluorophenyl)-4-iodo-thieno[2,3-d]pyrimidine (23 g, 67.5mmol) and cesium carbonate (67 g, 203 mmol). The reaction was heated at reflux overnight, and the volatiles were evaporated. The residue was diluted with ethyl acetate and water (respectively 500 and 400 mL) and the solution is filtered. The organic layer was separated, washed with brine, dried over magnesium sulfate and concentrated under vacuum. The residue was purified by silica gel chromatography (gradient of ethyl acetate in petroleum ether to afford ethyl (2R)-2-[5-bromo-6-(4-fluorophenyl)thieno[2,3-d]pyrimidin-4-yl]oxy-3-[2-[(2-chloropyrimidin-4-yl)methoxy]phenyl]propanoate as a slightly orange solid. $^1$H NMR (400 MHz, dmso-d6): δ 8.83 (d, 1H), 8.65 (s, 1H), 7.75 (m, 2H), 7.71 (d, 1H), 7.48 (d, 1H), 7.45 (m, 2H), 7.25 (t, 1H), 7.06 (d, 1H), 6.95 (t, 1H), 5.75 (dd, 1H), 5.28 (2*d, 2H), 4.18 (q, 2H), 3.6/3.3 (2*dd, 2H), 1.12 (t, 3H). IR Wavelenght (cm$^{-1}$): 1749.

*Step 2: (4-bromo-2-chloro-3-methyl-phenoxy)-triisopropyl-silane*

To a solution of 4-bromo-2-chloro-3-methyl-phenol (100 g, 482 mmol) in dichloromethane (1.5 L) were added imidazole (82 g, 1.2 mol) and dropwise over 1 h chloro(triisopropyl)silane (102 mL, 482 mmol). The reaction was stirred at room temperature for 1 h and water was added (500 mL). The organic layers were washed with brine (200 mL), dried over Magnesium sulfate and concentrated. The residue was used without further purification. $^1$H NMR (400 MHz, $CDCl_3$): δ 7.48 (s, 1H), 7.2 (dd, 1H), 6.7 (d, 1H), 1.3 (m, 3H), 1.1 (2s, 18H).

*Step 3: tert-butyl-[2-chloro-3-methyl-4-(4,4,5,5-tetramethyl-1,3,2-dioxaborolan-2-yl)phenoxy]-dimethyl-silane*

To a solution of (4-bromo-2-chloro-3-methyl-phenoxy)-triisopropyl-silane (27.2 g, 71.9 mmol) in THF (350 mL) at -78°C under Argon was added dropwise over 30 min a solution of n-butyl lithium 1.6 M in THF (49.5 mL, 79.9 mmol). The reaction was stirred at -78 °C for 2 h and a solution of 2-Isopropoxy-4,4,5,5-tetramethyl-1,3,2-dioxaborolane (16.1 g, 86.4 mmol) in THF (50 mL) was added dropwise over 30 min. After 2h stirring at -78°C, the reaction mixture was quenched by a slow addition of water (20 mL) and warmed to room temperature, diluted with water (200 mL) and extracted with ethyl acetate. The combined organic layers were washed with brine, dried over Magnesium sulfate and concentrated under vacuum. The residue was used without further purification. $^1$H NMR (400 MHz, dmso-d6): δ 7.5 (d, 1H), 6.82 (d, 1H), 2.52 (s, 3H), 1.32 (m, 3H), 1.3 (s, 12H), 1.08 (s, 18H).

*Step 4: 2-chloro-3-methyl-4-(4,4,5,5-tetramethyl-1,3,2-dioxaborolan-2-yl)phenol*

To a solution of tert-butyl-[2-chloro-3-methyl-4-(4,4,5,5-tetramethyl-1,3,2-dioxaborolan-2-yl)phenoxy]-dimethyl-silane (25.4 g, 59.8 mmol) in THF (750 mL) was added dropwise at room temperature a solution of Tetrabutylammonium Fluoride 1 M in THF (90 mL, 90 mmol). The reaction mixture was stirred for 2 h, concentrated, diluted with ethyl acetate, partitioned with water and extracted with ethyl acetate. The combined organic layers were washed with brine, dried over Magnesium sulfate and concentrated under vacuum. The residue was purified by silica gel chromatography (gradient of ethanol in dichloromethane) to afford 2-chloro-3-methyl-4-(4,4,5,5-tetramethyl-1,3,2-dioxaborolan-2-yl)phenol. $^1$H NMR (400 MHz, dmso-d6): δ 10.4 (m, 1H), 7.4 (d, 1H), 6.8 (d, 1H), 2.5 (s, 3H), 1.3 (s, 12H).IR Wavelenght (cm$^{-1}$): 3580-3185, 1591, 857, 827.

*Step 5: ethyl (2R)-2-{[(5S$_a$)-5-(3-chloro-4-hydroxy-2-methylphenyl)-6-(4-fluorophenyl)thieno[2,3-d]pyrimidin-4-ylloxy}-3-{2-[(2-chloropyrimidin-4-yl)methoxy]phenyl)propanoate*

To a solution of ethyl (2R)-2-[5-bromo-6-(4-fluorophenyl)thieno[2,3-d]pyrimidin-4-yl]oxy-3-[2-[(2-chloropyrimidin-4-yl)methoxy]phenyl]propanoate (43.8 g, 61.2 mmol) and 2-chloro-3-methyl-4-(4,4,5,5-tetramethyl-1,3,2-dioxaborolan-2-yl)phenol (19.7 g, 73.5 mmol) in a mixture of THF/$H_2O$ 1/1 (800 mL) was added cesium carbonate (40 g, 122 mmol). The reaction was degased by bubbling argon through the solution for 20 min and Bis(di-tert-butyl(4-dimethylaminophenyl) phosphine)dichloropalladium(II) (4.35g, 6.1 mmol) was added . The reaction mixture was heated at 80°C under argon overnight. The reaction was diluted with water, partitioned with ethyl acetate and extracted with ethyl acetate. The combined organic layers were washed with brine, dried over Magnesium sulfate and concentrated under vacuum. The residue was purified by silica gel chromatography (gradient of methanol in dichloromethane) to afford ethyl (2R)-2-[5-(3-chloro-4-hydroxy-2-methyl-phenyl)-6-(4-fluorophenyl)thieno[2,3-d]pyrimidin-4-yl]oxy-3-[2-[(2-chloropyrimidin-4-yl) methoxy]phenyl]propanoate as a mixture of diastereoisomers 85/15 (aS/aR or $S_a$/$R_a$).Optically pure *aS* (or $S_a$) was obtained by Preparative SFC purification.

*Step 6: tert-butyl 4-(2-{2-chloro-4-[4-{[(2R)-3-{2-[(2-chloropyrimidin-4-yl)methoxy]phenyl}-1-ethoxy-1-oxopropan-2-yl] oxy}-6-(4-fluorophenyl)thieno[2,3-d]pyrimidin-5-yl]-3-methylphenoxy}ethyl)piperazine-1-carboxylate*

To a solution of triphenylphosphine (2.66 g, 10 mmol) in THF was added at room temperature under argon Diisopropyl azodicarboxylate (2.33 g, 10 mmol). After 15 min of stirring, was added a solution of tert-butyl 4-(2-hydroxyethyl)piperazine-1-carboxylate (2.33g, 10 mmol) in THF (8 mL). The reaction was stirred at room temperature for 1 h, then was added dropwise a solution of and (2R)-2-[(5*S*$_a$)-5-(3-chloro-4-hydroxy-2-methyl-phenyl)-6-(4-fluorophenyl)thieno[2,3-d]pyrimidin-4-yl]oxy-3-[2-[(2-chloropyrimidin-4-yl)methoxy]phenyl]propanoic acid (3.57 g, 5 mmol) in THF (8 mL). The reaction was stirred at room temperature for 96 h and concentrated. The residue was purified by silica gel chromatography (gradient of methanol (containing 7M ammonia) in dichloromethane) to afford tert-butyl 4-(2-{2-chloro-4-[4-{[(2R)-3-{2-[(2-chloropyrimidin-4-yl)methoxy]phenyl}-1-ethoxy-1-oxopropan-2-yl]oxy}-6-(4-fluorophenyl)thieno [2,3-d]pyrimidin-5-yl]-3-methylphenoxy}ethyl)piperazine-1-carboxylate.$^1$H NMR (400 MHz, $CDCl_3$): δ 8.95 (d, 1H), 8.58 (s, 1H), 8.32 (d, 2H), 7.58 (d, 1H), 7.41 (dd, 2H), 7.32 (d, 1H), 7.29 (dd, 2H), 7.22 (t, 2H), 7.21 (d, 1H), 7.19 (t, 1H), 7.05 (d, 1H), 6.75 (t, 1H), 6.31 (dd, 1H), 5.53 (dd, 1H), 5.29 (dd, 2H), 4.2 (m, 2H), 4.05 (q, 2H), 3.97 (m, 4H), 3.3 (m, 2H), 3.2 (t, 4H), 3.19/2.59 (m, 2H), 2.72 (t, 2H), 2.4 (t, 4H), 1.87 (s, 3H), 1.37 (s, 9H), 1.18 (t, 6H), 1.05 (t, 3H). $^{13}$C NMR (125 MHz, $CDCl_3$): δ 158, 152, 131, 131, 130, 130, 128, 127, 120.5, 116, 116, 112, 110, 73, 68.5, 67, 62, 61, 56, 52, 43, 32, 32, 28, 17, 16, 14.

*Step 7: tert-butyl 4-(2-{2-chloro-4-[4-{[(2R)-3-{2-[(2-{4-[(diethoxyphosphoryl)methyl]phenyl}pyrimidin-4-yl)methoxy] phenyl}-1-ethoxy-1-oxopropan-2-yl]oxy}-6-(4-fluorophenyl)thieno[2,3-d]pyrimidin-5-yl]-3-methylphenoxy)ethyl)piperazine-1-carboxylate*

To a solution of tert-butyl 4-(2-{2-chloro-4-[4-{[(2R)-3-{2-[(2-chloropyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy}ethyl)piperazine-1-carboxylate (337 mg, 0.367 mmol) and [4-(diethoxyphosphorylmethyl)phenyl]boronic acid (200mg, 0.735 mmol) in dioxane (2.5 mL), were added water (2.5 mL) and cesium carbonate (241 mg, 0.735 mmol). The reaction mixture was degased by bubbling argon through the solution for 30 min, Bis(triphenylphosphine)palladium(II) dichloride (2.5 mg, 3.6 μmol) was added and the reaction mixture was heated by microwave irradiation in a sealed vessel at 90 °C for 3 h. The reaction was diluted with ethyl acetate and water. The aqueous layer was extracted with ethyl acetate and the combined organic layers were washed with brine, dried over Magnesium sulfate and concentrated under vacuum. The residue was purified by silica gel chromatography (gradient of methanol in dichloromethane) to afford tert-butyl 4-(2-{2-chloro-4-[4-{[(2R)-3-{2-[(2-{4-[(diethoxyphosphoryl)methyl]phenyl}pyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy}ethyl)piperazine-1-carboxylate. $^{1}H$ NMR (500 MHz, dmso-d6): δ 8.95 (d, 1H), 8.58 (s, 1H), 8.32 (d, 2H), 7.58 (d, 1H), 7.41 (dd, 2H), 7.32 (d, 1H), 7.29 (dd, 2H), 7.22 (t, 2H), 7.21 (d, 1H), 7.19 (t, 1H), 7.05 (d, 1H), 6.75 (t, 1H), 6.31 (dd, 1H), 5.53 (dd, 1H), 5.29 (2*d, 2H), 4.2 (m, 2H), 4.05 (q, 2H), 3.97 (m, 4H), 3.3 (m, 2H), 3.2 (t, 4H), 3.19/2.59 (2*dd, 2H), 2.72 (t, 2H), 2.4 (t, 4H), 1.87 (s, 3H), 1.37 (s, 9H), 1.18 (t, 6H), 1.05 (t, 3H). $^{13}C$ NMR (125 MHz, dmso-d6) δ 158, 152, 131, 131, 130, 130, 128, 127, 120.5, 116, 116, 112, 110, 73, 68.5, 67, 62, 61, 56, 52, 43, 32, 32, 28, 17, 16, 14

*Step 8: Synthesis of (2R)-2-[(5$S_a$)-S-[3-chloro-2-methyl-4-(2-piperazin-1-ylethoxy)phenyl]-6-(4-fluorophenyl)thieno [2,3-d]pyrimidin-4-yl]oxy-3-[2-[[2-[4-(phosphonomethyl)phenyl]pyrimidin-4-yl]methoxy]phenyl]propanoic acid*

To a solution of tert-butyl 4-(2-{2-chloro-4-[4-{[(2R)-3-{2-[(2-{4-[(diethoxyphosphoryl)methyl]phenyl}pyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy} ethyl)piperazine-1-carboxylate (540 mg, 0.486 mmol) in dichloromethane (5 mL) was added bromotrimethylsilane (186 μL, 1.46 mmol). The reaction mixture was heated to reflux overnight. Another portion of bromotrimethylsilane was added at room temperature (186 μL, 1.46mmol) and the reaction was heated at reflux for 20h and concentrated to dryness. The residue was taken up in methanol, stirred at room temperature for 3h and concentrated to afford a brown viscous oil which was diluted with dioxane (4 mL) and water (4 mL). Lithium hydroxide monohydrate (100 mg, 24 mmol) was added by portions and the reaction mixture was stirred at room temperature for 1 h, heated at 45°C for 3 h and concentrated. The residue was diluted with water (5 mL), acidified to pH2 by dropwise addition of an aqueous 2 M HCl solution. The precipitate was filtered, washed with THF and purified by C18 reverse phase prep-HPLC by direct deposit of the reaction mixture on the Xbridge column and using the $NH_4HCO_3$ method to afford C4. $^{1}H$ NMR (500 MHz, dmso-d6): δ 8.88 (br d, 1 H), 8.25 (d, 2 H), 7.75 (t, 1 H), 7.59 (s, 1 H), 7.52 (d, 1 H), 7.35 (d, 2 H), 7.23 (dd, 2 H), 7.18 (d, 1 H), 7.15 (t, 2 H), 7.11 (t, 1 H), 7.02 (d, 1 H), 6.82 (d, 1 H), 6.64 (m, 1 H), 5.51 (d, 1 H), 5.28/5.07 (m, 2 H), 3.82/3.55 (2m, 2 H), 3.35/2.55 (br s, 2 H), 2.81 (d, 2 H), 2.55 (m, 4 H), 2.4/2.27 (2m, 2 H), 2.21 (m, 4 H), 1.65 (br s, 3 H). $^{13}C$ NMR (125 MHz, dmso-d6): δ 131.5, 130.2, 129.7, 127.4, 127.2, 120.3, 115.9, 115.3, 111.9, 110.3, 75.1, 69.2, 67.3, 56.4, 49.9, 42.4, 40, 38.9, 18.1. $^{31}P$ NMR (200 MHz, dmso-d6): δ 15 HR-ESI+ : m/z [M+H]+ = 925.2356 / 925.2346 (measured/theoretical)

**Preparation of C5:**

(2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(4-hydroxyphenyl)pyrimidin-4-yl]methoxy}phenyl)propanoic acid

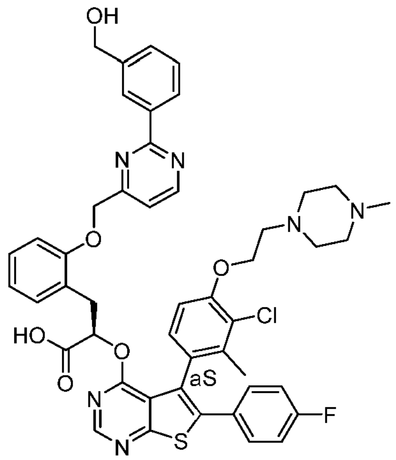

C5 was prepared according to Example 3 in WO 2016/207216.

**Preparation of C6:**

**(2*R*)-2-{[(5$S_a$)-5-{3-chloro-2-methyl-4-[2-(4-methylpiperazin-1-yl)ethoxy]phenyl}-6-(4-fluorophenyl)thieno[2,3-d] pyrimidin-4-yl]oxy}-3-[2-({2-[2-(hydroxymethyl)phenyl]pyrimidin-4-yl}methoxy)phenyl]propanoic acid**

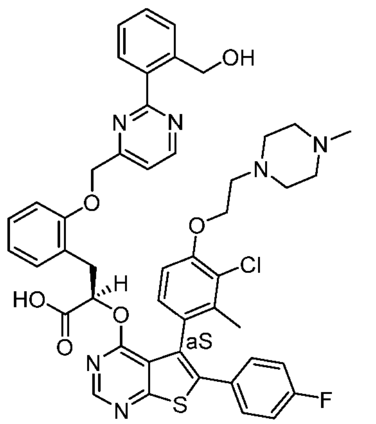

C6 was prepared according to Example 728 in WO 2015/097123.

**Preparation of C7:**

**(2R)-2-[(5$S_a$)-5-[3-chloro-2-ethyl-4-[2-(4-methylpiperazin-1-yl)ethoxy]phenyl]-6-prop-1-ynyl-thieno[2,3-d]pyrimidin-4-yl]oxy-3-[2-[[2-(2-methoxyphenyl)pyrimidin-4-yl]methoxy]phenyl]propanoic acid**

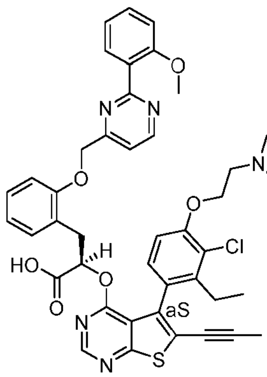

*Step A: ethyl (2R)-2-(5-iodo-6-prop-1-ynyl-thieno[2, 3-d]pyrimidin-4-yl) oxy-3-[2-[[2-(2-methoxyphenyl)pyrimidin-4-yl] methoxy]phenyl]propanoate*

5.0 g 4-chloro-5-iodo-6-prop-1-ynyl-thieno[2,3-d]pyrimidine (15.0 mmol; obtained according to WO 2015/097123, Preparation 2f) and 6.10 g ethyl (2R)-2-hydroxy-3-[2-[[2-(2-methoxyphenyl)pyrimidin-4-yl]methoxy]phenyl]propanoate (15.0 mmol; obtained according to WO 2015/097123, Preparation 3bs) were dissolved in 150 mL tert-butanol, then 14.7 g $Cs_2CO_3$ (45.0 mmol) was added and the mixture was stirred under $N_2$ atmosphere at 50°C until no further conversion was observed. Then water and brine was added and the mixture was extracted with EtOAc. The combined organic layer was dried over $Na_2SO_4$, filtered and the filtrate was concentrated under reduced pressure. The crude product was purified via flash chromatography using heptane and EtOAc as eluents to give ethyl (2R)-2-(5-iodo-6-prop-1-ynyl-thieno[2,3-d]pyrimidin-4-yl)oxy-3-[2-[[2-(2-methoxyphenyl)pyrimidin-4-yl]methoxy]phenyl]propanoate. $^1H$ NMR (400 MHz, DMSO-$d_6$) δ: 8.89 (d, *J* = 5.1 Hz, 1H), 8.59 (s, 1H), 7.62 (d, *J* = 5.2 Hz, 1H), 7.55 (dd, *J* = 7.5, 1.6 Hz, 1H), 7.51 (dd, *J* = 7.5, 1.6 Hz, 1H), 7.43 (m, 1H), 7.26 (m, 1H), 7.11 (m, 2H), 7.02 (td, *J* = 7.5, 0.9 Hz, 1H), 6.94 (td, *J* = 7.4, 0.8 Hz, 1H), 5.79 (dd, *J* = 9.1, 4.6 Hz, 1H), 5.31 (d, *J* = 14.9 Hz, 1H), 5.26 (d, *J* = 14.9 Hz, 1H), 4.13 (m, 2H), 3.76 (s, 3H), 3.60 (dd, *J* = 13.8, 4.5 Hz, 1H), 3.33 (m, 1H), 2.21 (s, 3H), 1.10 (t, *J* = 7.1 Hz, 3H). $^{13}C$ NMR (100 MHz, DMSO-$d_6$) δ: 169.4, 166.5, 165.7, 164.8, 161.3, 157.7, 155.8, 153.6, 132.2, 131.0, 130.8, 128.3, 124.0, 120.9, 120.1, 115.5, 112.2, 112.0, 110.5, 98.9, 79.5, 74.4, 74.3, 69.1, 61.1, 55.7, 13.9, 4.6.

*Step B: 2-chloro-3-ethyl-4-(4,4,5,5-tetramethyl-1,3,2-dioxaborolan-2-yl)phenol*

33.7 g [2-chloro-3-ethyl-4-(4,4,5,5-tetramethyl-1,3,2-dioxaborolan-2-yl)phenoxy]-triisopropyl-silane (76.9 mmol; obtained according to WO 2015/097123, Preparation 5e) was dissolved in 600 mL THF and was cooled to 0°C, then 92.3 mL TBAF (92.3 mmol, 1M solution in THF) was added dropwise and the mixture was stirred until complete conversion. Then it was diluted with brine, acidified with citric acid then extracted with DCM. The combined organic layer was dried over $Na_2SO_4$, filtered and the filtrate was concentrated under reduced pressure. The crude product was purified via flash chromatography using heptane and EtOAc as eluents to give 2-chloro-3-ethyl-4-(4,4,5,5-tetramethyl-1,3,2-dioxaborolan-2-yl)phenol. $^1H$ NMR (400 MHz, $CDCl_3$) δ: 7.63 (d, J= 8.2 Hz, 1H), 6.86 (d, *J*= 8.2 Hz, 1H), 5.87 (s, 1H), 3.09 (q, *J* = 7.44 Hz, 2H), 1.33(s, 12H), 1.15 (t, *J* = 7.44 Hz, 3H).

*Step C: ethyl (2R)-2-[5-(3-chloro-2-ethyl-4-hydroxy-phenyl)-6-prop-1-ynyl-thieno[2,3-d]pyrimidin-4-yl]oxy-3-[2-[[2-(2-methoxyphenyl)pyrimidin-4-yl]methoxy]phenyl]propanoate*

353 mg ethyl (2R)-2-(5-iodo-6-prop-1-ynyl-thieno[2,3-d]pyrimidin-4-yl)oxy-3-[2-[[2-(2-methoxyphenyl)pyrimidin-4-yl]methoxy]phenyl]propanoate (0.50 mmol) and 282 mg 2-chloro-3-ethyl-4-(4,4,5,5-tetramethyl-1,3,2-dioxaborolan-2-yl)phenol (0.55 mmol) were dissolved in 2 mL dioxane, then 35 mg $PdCl_2 \times$AtaPhos (0.05 mmol), 326 mg $Cs_2CO_3$ (1.00 mmol) and 1 mL water were added and the mixture was stirred in a microwave reactor at 100°C under $N_2$ atmosphere for 20 minutes. Then it was diluted with brine, acidified to pH 5 with 1 M aqueous HCl solution and extracted with DCM. The combined organic layer was dried over $Na_2SO_4$, filtered and the filtrate was concentrated under reduced pressure. The crude product was purified via flash chromatography using heptane and EtOAc as eluents. Then it was further purified via preparative reverse phase chromatography using 5 mM aqueous $NH_4HCO_3$ solution and MeCN as eluents to give ethyl (2R)-2-[5-(3-chloro-2-ethyl-4-hydroxyphenyl)-6-prop-1-ynyl-thieno[2,3-d]pyrimidin-4-yl]oxy-3-[2-[[2-(2-methoxyphenyl) pyrimidin-4-yl]methoxy]phenyl]propanoate as a 2:1 mixture of atropisomers. $^1H$ NMR (500 MHz, DMSO-$d_6$) δ: 10.35/10.29 (s, 1H), 8.93 (d, *J* = 5.1 Hz, 1H), 8.59/8.57 (s, 1H), 7.63/7.60 (d, *J* = 5.1 Hz, 1H), 7.54-6.93 (m, 8 H), 6.84/6.74 (t, *J* = 7.5 Hz, 1H), 6.43/6.18 (dd, *J* = 7.5, 1.4 Hz, 1H), 5.51/5.40 (m, 1H), 5.30-5.16 (m, 2H), 4.17-3.99 (m, 2H), 3.76/3.75 (s, 3H), 3.34-3.14 (m, 1H), 2.93-2.35 (m, 3H), 2.02/1.98 (s, 3H), 1.08/1.04 (t, *J* = 7.0 Hz, 3H), 0.94/0.76 (t, *J* = 7.5

Hz, 3H).

*Step D: (2R)-2-[(5Sa)-5-[3-chloro-2-ethyl-4-[2-(4-methylpiperazin-1-yl)ethoxy]phenyl]-6-prop-1-ynyl-thieno[2,3-d]pyrimidin-4-yl]oxy-3-[2-[[2-(2-methoxyphenyl)pyrimidin-4-yl]methoxy]phenyl]propanoic acid, C7*

322 mg ethyl (2R)-2-[5-(3-chloro-2-ethyl-4-hydroxy-phenyl)-6-prop-1-ynyl-thieno[2,3-d]pyrimidin-4-yl]oxy-3-[2-[[2-(2-methoxyphenyl)pyrimidin-4-yl]methoxy]phenyl]propanoate (0.44 mmol), 190 mg 2-(4-methylpiperazin-1-yl)ethanol (1.32 mmol) and 346 mg $PPh_3$ (1.32 mmol) were dissolved in 10 mL dry toluene, then 304 mg DTBAD (1.32 mmol) was added and the mixture was stirred at 50°C under $N_2$ atmosphere until no further conversion was observed. Then the mixture was concentrated under reduced pressure and the residue was purified via flash chromatography using heptane, EtOAc and MeOH as eluents, then further purified via preparative reverse phase chromatography using 5 mM aqueous $NH_4HCO_3$ solution and MeCN as eluents to obtain the ester intermediate. It was dissolved in 2 mL dioxane, then 84 mg $LiOH \times H_2O$ (2.00 mmol) and 1 mL water were added. The mixture was stirred at 50°C until complete hydrolysis. Then it was diluted with brine, neutralized with 2 M aqueous HCl solution, and extracted with DCM. The combined organic phase was dried over $Na_2SO_4$, filtered and the filtrate was concentrated under reduced pressure. The atropoisomers were purified and separated via preparative reverse phase chromatography using 5 mM aqueous $NH_4HCO_3$ solution and MeCN as eluents. The atropoisomer eluting later was isolated as C7. $^1$H NMR (400 MHz, DMSO-$d_6$) $\delta$: 8.88 (d, *J* = 5.2 Hz, 1H), 8.60 (s, 1H), 7.76 (d, *J* = 5.0 Hz, 1H), 7.54 (dd, *J* = 7.6, 1.8 Hz, 1H), 7.46 (m, 1H), 7.26 (d, *J* = 8.5 Hz, 1H), 7.20-7.13 (m, 3H), 7.04 (td, *J* = 7.5, 0.9 Hz, 1H), 7.00 (d, *J* = 8.0 Hz, 1H), 6.78 (t, *J* = 7.5 Hz, 1H), 6.32 (dd, *J* = 7.4, 1.5 Hz, 1H), 5.48 (dd, *J* = 9.7, 2.9 Hz, 1H), 5.27 (d, *J= 15.0* Hz, 1H), 5.19 (d, *J* = 15.0 Hz, 1H), 4.23 (m, 2H), 3.76 (s, 3H), 3.31 (m, 1H), 2.75 (m, 2H), 2.47 (m, 1H), 2.64-2.36 (m, 10H), 2.22 (s, 3H), 2.01 (s, 3H), 0.74 (t, *J* = 7.5 Hz, 3H). $^{13}$C NMR (100 MHz, DMSO-*$d_6$*) $\delta$: 165.95, 165.89, 164.7, 157.8, 157.2, 155.3, 154.0, 141.6, 135.6, 131.0, 130.8, 130.1, 128.4, 128.0, 127.2, 121.4, 120.1, 117.8, 112.2, 111.7, 97.2, 74.9, 68.9, 67.1, 56.1, 55.7, 54.0, 32.7, 24.4, 13.1, 4.4. HRMS calculated for $C_{45}H_{45}N_6O_6SCl$: 832.2810; found 833.2878 (M+H).

**Preparation of C8:**

**(2R)-2-[(5S$_a$)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid**

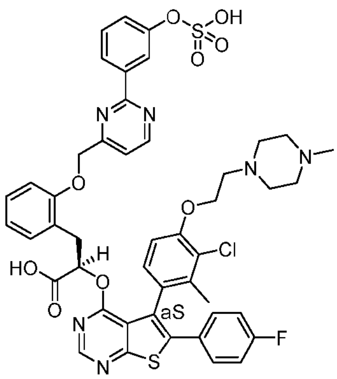

210 mg ethyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(3-hydroxyphenyl)pyrimidin-4-yl]methoxy]phenyl]propanoate (0.24 mmol; obtained according to WO 2016/207216, Preparation 2) was dissolved in 9.3 mL pyridine, than 0.38 mL $SO_3 \times$pyridine (2.36 mmol) was added and the mixture was stirred at 70°C until complete conversion. Then the mixture was concentrated under reduced pressure and the residue was dissolved in 2 mL dioxane, then 200 mg KOH (3.56 mmol) and 1 mL water were added. The mixture was stirred at 70°C until complete hydrolysis of the Et ester. Then it was neutralized with 5 M aqueous HCl solution, and purified via preparative reverse phase chromatography (direct injection) using 25 mM aqueous $NH_4HCO_3$ solution and MeCN as eluents to give **C8.** $^1$H NMR (500 MHz, DMSO-$d_6$) $\delta$: 8.93 (d, *J* = 5.1 Hz, 1H), 8.63 (s, 1H), 8.27 (m, 1H), 8.13 (m, 1H), 7.61 (d, *J* = 5.1 Hz, 1H), 7.43 (t, *J* = 7.8 Hz, 1H), 7.33 (d, *J* = 7.8 Hz, 1H), 7.32-7.27 (m, 3H), 7.23-7.13 (m, 4H), 7.05 (d, *J* = 7.8 Hz, 1H), 6.73 (t, *J* = 7.5 Hz, 1H), 6.31 (dd, *J* = 7.5, 1.3 Hz, 1H), 5.51 (dd, *J* = 9.8, 3.5 Hz, 1H), 5.34 (d, *J* = 15.2 Hz, 1H), 5.27 (d, *J* = 15.2 Hz, 1H), 4.24-4.08 (m, 2H), 3.26 (dd, *J* = 14.3, 3.4 Hz, 1H), 3.10-2.52 (m, 14H), 1.82 (s, 3H). $^{13}$C NMR (125 MHz, DMSO-$d_6$) $\delta$: 170.8, 166.5, 166.4, 162.8, 162.7, 158.3, 155.3, 154.0, 153.6, 152.9, 137.8, 136.8, 135.9, 131.12, 131.05, 130.4, 130.3, 129.1, 128.5, 128.2, 127.8, 124.4, 123.5, 122.7, 121.9, 120.5, 120.2,

118.8, 116.0, 115.9, 112.0, 110.5, 73.5, 69.1, 67.2, 55.4, 43.1, 31.8, 17.5. HRMS calculated for $C_{46}H_{42}N_6O_9S_2ClF$: 940.2127; found 941.2191 (M+H).

**Preparation of C9:**

**2*R*)-2-{[5-{3-chloro-2-methyl-4-[2-(4-methylpiperazin-1-yl)ethoxy]phenyl}-6-(4-fluorophenyl)thieno[2,3-*d*]pyrimidin-4-yl]oxy)-3-(2-([2-(3-sulfophenyl)pyrimidin-4-yl]methoxy}phenyl)propanoic acid**

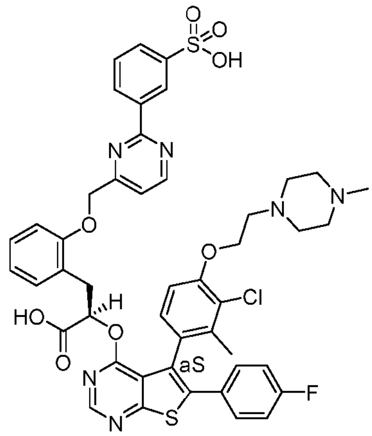

750 mg ethyl (2R)-2-[(5Sa)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[(2-methylsulfanylpyrimidin-4-yl)methoxy]phenyl]propanoate (0.89 mmol; obtained according to WO 2015/097123, Preparation 10a) was dissolved in 9 mL THF, then 726 mg [3-(2,2-dimethylpropoxysulfonyl)phenyl]boronic acid (2.67 mmol), 62 mg $Pd(PPh_3)_4$ (0.05 mmol ) and 509 mg thiophene-2-carbonyloxycopper (2.67 mmol) were added and the mixture was stirred at 75°C until complete conversion. Then it was concentrated under reduced pressure and was purified via flash chromatography using heptane, EtOAc and 0.7 M $NH_3$ solution in MeOH as eluents. Then it was dissolved in 20 mL 1,1,1,3,3,3-hexafluoropropan-2-ol, 4.5 mL TFA was added and the mixture was stirred at 80°C for until complete hydrolysis of the sulfonic ester. The mixture was concentrated under reduced pressure and then dissolved in 5 mL dioxane, then 210 mg $LiOH\times H_2O$ (5.00 mmol) and 2 mL water were added. The mixture was stirred at room temperature until complete hydrolysis. Then it was diluted with brine, neutralized with 2 M aqueous HCl solution, and extracted with DCM. The combined organic phase was dried over $Na_2SO_4$, filtered and the filtrate was concentrated under reduced pressure. The formed atropisomers were purified and separated via preparative reverse phase chromatography using 25 mM aqueous $NH_4HCO_3$ solution and MeCN as eluents, then further purified using 0.1% aqueous TFA solution and MeCN as eluents to give C9. $^1H$ NMR (500 MHz, DMSO-$d_6$) δ: 13.19 (br s, 1H), 9.48 (br s, 1H), 8.94 (d, *J* = 5.1 Hz, 1H), 8.74 (t, *J* = 1.6 Hz, 1H), 8.65 (s, 1H), 8.37 (dt, *J* = 7.8, 2.9 Hz, 1H), 7.78 (dt, *J* = 7.6, 1.5 Hz, 1H), 7.60 (d, *J* = 5.1 Hz, 1H), 7.51 (t, *J* = 7.7 Hz, 1H), 7.29 (m, 3H), 7.22-7.14 (m, 3H), 7.13-7.05 (m, 2H), 6.74 (t, *J* = 7.5 Hz, 1H), 6.38 (d, *J* = 7.6 Hz, 1H), 5.53 (dd, *J* = 9.6, 3.6 Hz, 1H), 5.37 (d, *J* = 15.3 Hz, 1H), 5.31 (d, *J* = 15.3 Hz, 1H), 4.2 (m, 2H), 3.50-2.88 (m, 11H), 2.76 (s, 3H), 2.61 (dd, *J* = 14.2, 9.7 Hz, 1H), 1.79 (s, 3H). $^{13}C$ NMR (125 MHz, DMSO-$d_6$) δ: 170.8, 166.5, 163.0, 162.7, 161.1, 158.4, 155.4, 153.3, 152.9, 148.6, 136.6, 136.0, 131.1, 130.1, 129.0, 128.5, 128.3, 128.1, 127.9, 125.3, 124.4, 121.9, 120.5, 118.8, 116.2, 116.0, 115.9, 112.1, 110.5, 73.2, 69.1, 66.5, 55.0, 51.7, 49.7, 42.1, 31.5, 17.5. HRMS calculated for $C_{46}H_{42}N_6O_8S_2ClF$: 924.2178; found 925.2274 (M+H).

**Preparation of C10:**

**(2R)-2-[(5S$_a$)-5-[3-chloro-2-methyl-4-[2-(4-methylpiperazin-1-yl)ethoxy]phenyl]-6-[4-fluoro-3-(2,2,2-trifluoroethoxy)phenyl]thieno[2,3-d]pyrimidin-4-yl]oxy-3-[2-[[2-(2-methoxyphenyl)pyrimidin-4-yl]methoxy]phenyl] propanoic acid**

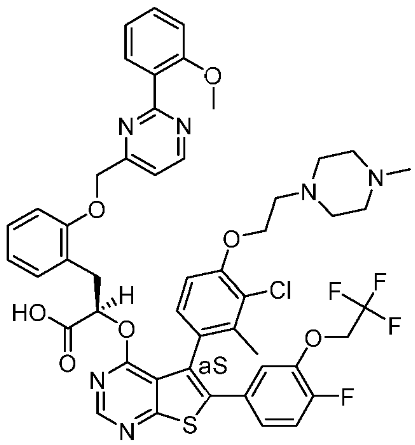

250 mg ethyl (2R)-2-[5-[3-chloro-2-methyl-4-[2-(4-methylpiperazin-1-yl)ethoxy]phenyl]-6-iodo-thieno[2,3-d] pyrimidin-4-yl]oxy-3-[2-[[2-(2-methoxyphenyl)pyrimidin-4-yl]methoxy]phenyl]propanoate (0.27 mmol; obtained according to WO 2015/097123, Preparation 30) and 79 mg [4-fluoro-3-(2,2,2-trifluoroethoxy)phenyl]boronic acid (0.40 mmol) were dissolved in 1 mL THF, then 3.0 mg $PdOAc_2$ (0.013 mmol), 5.7 mg tBuXPhos (0.013 mmol), 174 mg $Cs_2CO_3$ (0.53 mmol) and 0.27 mL water were added and the mixture was stirred at 70°C under $N_2$ atmosphere for 2 hours. Then it was diluted with brine, and extracted with 2-MeTHF. The combined organic layer was dried over $Na_2SO_4$, filtered and the filtrate was concentrated under reduced pressure. The crude ester product was purified via flash chromatography using heptane, EtOAc and 0.7 M $NH_3$ solution in MeOH as eluents. Then it was dissolved in 5.3 mL dioxane, then 64 mg LiOH$\times H_2O$ (1.52 mmol) and 1.3 mL water were added. The mixture was stirred at room temperature until complete hydrolysis. Then it was diluted with brine, neutralized with 2 M aqueous HCl solution, and extracted with DCM. The combined organic phase was dried over $Na_2SO_4$, filtered and the filtrate was concentrated under reduced pressure. The atropisomers were purified and separated via preparative reverse phase chromatography using 25 mM aqueous $NH_4HCO_3$ solution and MeCN as eluents. The atropisomer eluting later was isolated as **C10.** $^1H$ NMR (500 MHz, DMSO-$d_6$) $\delta$: 8.91 (d, *J* = 5.2 Hz, 1H), 8.57 (s, 1H), 7.82 (d, *J* = 5.1 Hz, 1H), 7.53 (dd, *J* = 7.5, 1.7 Hz, 1H), 7.45 (m, 2H), 7.27 (dd, *J* = 11.0, 8.6 Hz, 1H), 7.22 (d, *J* = 8.6 Hz, 1H), 7.16-7.09 (m, 3H), 7.03 (t, *J* = 7.5 Hz, 1H), 6.98 (d, *J* = 8.3 Hz, 1H), 6.92 (m, 1H), 6.69 (t, *J* = 7.4 Hz, 1H), 6.16 (d, *J* = 7.2 Hz, 1H), 5.47 (dd, *J* = 10.3, 2.6 Hz, 1H), 5.25 (d, *J* = 15.1 Hz, 1H), 5.19 (d, *J* = 15.1 Hz, 1H), 4.75-4.53 (m, 2H), 4.21 (t, *J* = 5.5 Hz, 2H), 3.75 (s, 3H), 3.41 (d, *J* = 12.0 Hz, 1H), 2.77-2.30 (m, 12H), 2.24 (s, 3H), 1.81 (s, 3H). $^{13}C$ NMR (125 MHz, DMSO-$d_6$) $\delta$: 171.3, 166.2, 166.0, 164.6, 163.5, 157.9, 157.2, 155.3, 153.7, 153.1, 152.1, 150.5, 144.6, 135.8, 131.0, .130.8, 130.7, 129.6, 129.1, 128.4, 128.1, 127.8, 125.4, 123.7, 122.0, 120.4, 120.1, 118.8, 117.0, 116.7, 115.6, 112.2, 111.7, 110.5, 74.8, 68.9, 67.2, 65.6, 56.0, 55.7, 53.8, 52.0, 44.6, 32.7, 17.5. HRMS calculated for $C_{49}H_{45}N_6O_7SClF_4$: 972.2695; found 973.2761 (M+H).

**Preparation of C11:**

**(2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(4-methoxyphenyl)pyrimidin-4-yl]methoxy}phenyl)propanoic acid**

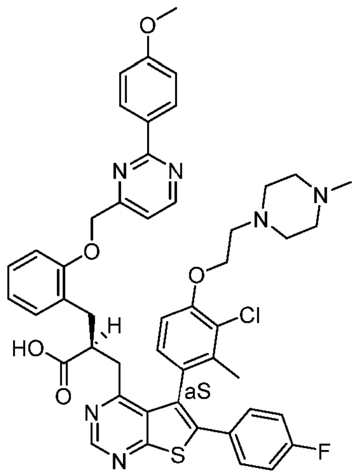

C11 was prepared according to Example 107 in WO 2015/097123.

**Preparation of C12**

**(*2R*)-2-{[(*5S$_a$*)-S-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[1-(2,2,2-trifluoroethyl)-1H-pyrazol-5-yl]methoxy}phenyl)propanoic acid**

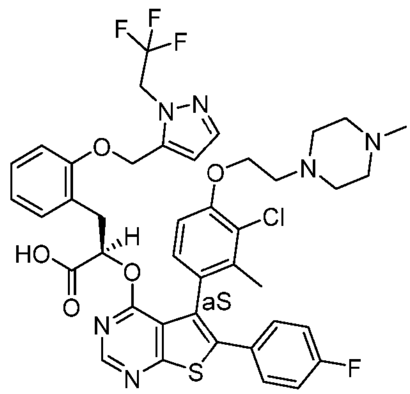

C12 was prepared according to Example 77 in WO 2015/097123.

**Preparation of C13**

**(2R)-2-[(5S$_a$)-6-(3-amino-4,5-difluoro-phenyl)-5-[3-chloro-2-methyl-4-[2-(4-methylpiperazin-1-yl)ethoxy]phenyl] thieno[2,3-d]pyrimidin-4-yl]oxy-3-[2-[[2-(2-methoxyphenyl)pyrimidin-4-yl]methoxy]phenyl]propanoic acid**

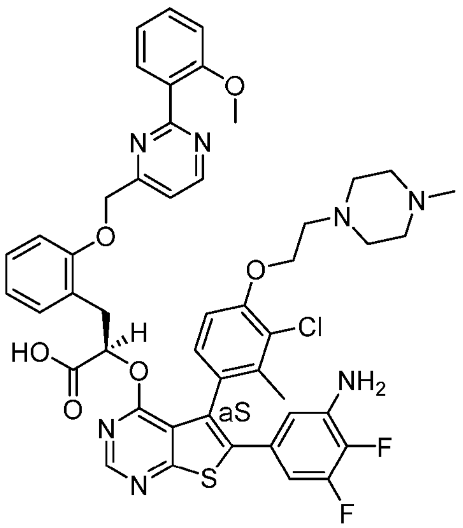

250 mg ethyl (2R)-2-[5-[3-chloro-2-methyl-4-[2-(4-methylpiperazin-1-yl)ethoxy]phenyl]-6-iodo-thieno[2,3-d]pyrimidin-4-yl]oxy-3-[2-[[2-(2-methoxyphenyl)pyrimidin-4-yl]methoxy]phenyl]propanoate (0.27 mmol; obtained according to WO 2015/097123, Preparation 30) and 102 mg 2,3-difluoro-5-(4,4,5,5-tetramethyl-1,3,2-dioxaborolan-2-yl)aniline (0.41 mmol) were dissolved in 1 mL THF, then 3.0 mg $PdOAc_2$ (0.013 mmol), 5.7 mg tBuXPhos (0.013 mmol), 174 mg $Cs_2CO_3$ (0.53 mmol) and 0.27 mL water were added and the mixture was stirred at 70°C under $N_2$ atmosphere for 2 hours. Then it was diluted with brine, and extracted with 2-MeTHF. The combined organic layer was dried over $Na_2SO_4$, filtered and the filtrate was concentrated under reduced pressure. The crude ester product was purified via flash chromatography using heptane, EtOAc and 0.7 M $NH_3$ solution in MeOH as eluents. Then it was dissolved in 0.7 mL dioxane, then 60 mg LiOH×$H_2O$ (1.43 mmol) and 0.18 mL water were added. The mixture was stirred at room temperature until complete hydrolysis. Then it was diluted with brine, neutralized with 2 M aqueous HCl solution, and extracted with DCM. The combined organic phase was dried over $Na_2SO_4$, filtered and the filtrate was concentrated under reduced pressure. The atropoisomers were purified and separated via preparative reverse phase chromatography using 25 mM aqueous $NH_4HCO_3$ solution and MeCN as eluents. The atropisomer eluting later was isolated as **C13.** $^1$H NMR (500 MHz, DMSO-$d_6$) δ: 8.89 (d, *J* = 5.2 Hz, 1H), 8.56 (s, 1H), 7.76 (d, *J* = 5.0 Hz, 1H), 7.53 (dd, *J* = 7.6, 1.8 Hz, 1H), 7.45 (m, 1H), 7.36 (d, *J* = 8.6 Hz, 1H), 7.20 (d, *J* = 8.7 Hz, 1H), 7.13 (m, 2H), 7.03 (td, *J* = 7.5, 1.0 Hz, 1H), 6.99 (d, *J* = 8.1 Hz, 1H), 6.71 (t, *J* = 7.3 Hz, 1H), 6.62 (m, 1H), 6.21 (d, *J* = 7.5, 1.3 Hz, 1H), 6.12 (m, 1H), 5.73 (s, 2H), 5.46 (dd, *J* = 10.1, 3.1 Hz, 1H), 5.25 (d, *J* = 15.1 Hz, 1H), 5.19 (d, *J* = 15.2 Hz, 1H), 4.22 (m, 2H), 3.75 (s, 3H), 3.35 (m, 1H), 2.73 (m, 2H), 2.65-2.35 (m, 9H), 2.22 (s, 3H), 1.85 (s, 3H). $^{13}$C NMR (125 MHz, DMSO-$d_6$) δ: 171.1, 166.0, 165.3, 163.3, 157.8, 157.2, 155.3, 153.7, 152.9, 149.0, 138.9, 137.1, 135.8, 131.0, 130.8, 130.4, 128.7, 128.4, 128.1, 127.8, 125.2, 122.0, 120.4, 120.1, 118.9, 115.6, 112.2, 111.9, 110.6, 103.2, 74.5, 68.9, 67.1, 56.0, 55.7, 53.9, 52.1, 44.7, 32.6, 17.6. HRMS calculated for $C_{47}H_{44}N_7O_6SClF_2$: 907.2730; found 908.2803 (M+H).

**Preparation of C14**

**(2*R*)-2-{[(5Sa)-5-{3-chloro-2-methyl-4-[2-(4-methylpiperazin-1-yl)ethoxy]phenyl}-6-(4-fluorophenyl)thieno[2,3-*d*]pyrimidin-4-yl]oxy}-3-(2-{[2-(3-hydroxy-2-methoxyphenyl)pyrimidin-4-yl]methoxy}phenyl)propanoic acid**

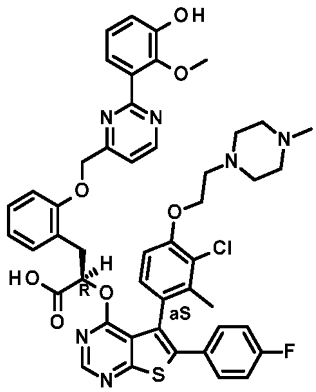

210 mg ethyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[(2-chloropyrimidin-4-yl)methoxy]phenyl]propanoate (0.25 mmol, WO2016/207216 **Preparation 1)** and 84 mg (3-hydroxy-2-methoxy-phenyl)boronic acid (0.50 mmol) were dissolved in 3.8 mL 1,4-dioxane, then 18 mg $Pd(PPh_3)_2Cl_2$ (0.025 mmol), 240 mg $Cs_2CO_3$ (0.75 mmol) and 3.8 mL water were added and the mixture was stirred under $N_2$ atmosphere at 70°C until complete conversion. Then it was diluted with water, neutralized with 2 M aqueous HCl solution, and extracted with DCM. The combined organic phase was dried over $Na_2SO_4$, filtered and the filtrate was concentrated under reduced pressure. The crude ester was purified via flash chromatography using heptane, EtOAc and 0.7 M $NH_3$ solution in MeOH as eluents to obtain a mixture of diastereoisomers. It was in dissolved in 2 mL dioxane, then 245 mg $LiOH\times H_2O$ (5.85 mmol) and 1 mL water were added. The mixture was stirred at rt until complete hydrolysis. Then it was neutralized with 2 M aqueous HCl solution, and directly injected on prep-RP-HPLC, using 0.1% aqueous TFA solution and MeCN as eluents. The diastereoisomer eluting later was collected as **C14.** $^1H$ NMR (500 MHz, DMSO-$d_6$) $\delta$: 9.53 (brs, 1H), 8.91 (d, 1H), 8.56 (s, 1H), 7.79 (d, 1H), 7.42 (d, 1H), 7.26 (m, 2H), 7.19 (d, 1H), 7.18 (m, 2H), 7.12 (t, 1H), 7.06 (dd, 1H), 6.98 (m, 1H), 6.98 (m, 1H), 6.97 (d, 1H), 6.68 (t, 1H), 6.16 (d, 1H), 5.47 (m, 1H), 5.27/5.20 (d+d, 2H), 4.26/4.19 (m+m, 2H), 3.76 (s, 3H), 3.38/2.42 (dd+dd, 2H), 2.74 (m, 2H), 2.55 (br., 4H), 2.47 (br., 4H), 2.25 (s, 3H), 1.80 (s,3 H). HRMS calculated for $C_{47}H_{44}N_6O_7SClF$: 890.2665; found 891.2721 (M+H).

**Preparation of P15**

**(11R,20R)-23,26-dichloro-3-(4-fluorophenyl)-14-[[2-(2-methoxyphenyl)pyrimidin-4-yl]methoxy]-24,25-dimethyl-20-[(4-methylpiperazin-1-yl)methyl]-10,18,21-trioxa-4-thia-6,8-diazapentacyclo[20.2.2.12,5.113,17.09,28] octacosa-1(25),2,5(28),6,8,13,15,17(27),22(26),23-decaene-11-carboxylic acid**

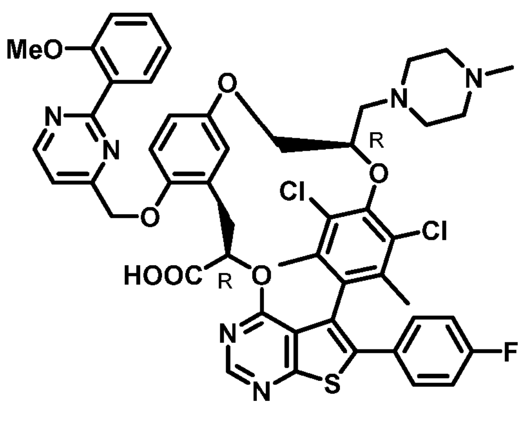

P15 was prepared according to Example 116 in WO 2019/035914.

**Preparation of P16**

**(11R,20R)-23,26-dichloro-14-[[2-[4-[[(2S)-1,4-dioxan-2-yl]methoxymethyl]-4-fluoro-cyclohexyl]pyrimidin-4-yl]methoxy]-3-(4-fluorophenyl)-24,25-dimethyl-20-[(4-methylpiperazin-1-yl)methyl]-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid**

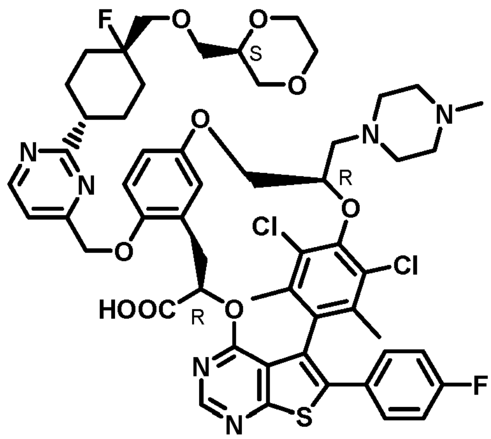

P16 was prepared according to Example 28 in WO 2019/035911.

**Preparation of P17**

**(11R,20R)-23,26-dichloro-14-[[2-[4-[[(2S)-1,4-dioxan-2-yl]methoxy]cyclohexyl]pyrimidin-4-yl]methoxy]-3-(4-fluorophenyl)-24,25-dimethyl-20-[(4-methylpiperazin-1-yl)methyl]-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid**

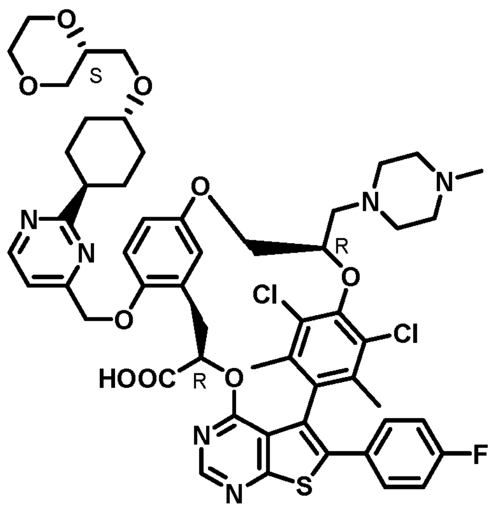

P17 was prepared according to Example 44 in WO 2019/035911.

**Example 4: Conjugation and analytical characterization of anti-CD74 ADCs**

Exemplary antibody-drug conjugates (ADCs) were synthesized using the exemplary methods described below. Antibodies anti-CD74 (Milatuzumab) used for the preparation of the exemplary ADCs is defined by the abbreviation Ab M, CD74, or CD74_CysmAb (Table 6). The term "CysmAb" refers to cysteine mutations in the heavy chain of the antibody that are used to conjugate linker-payloads to the antibody via maleimide group. All full length, conjugated CD74 Abs used herein contain the engineered cysteine mutations E152C and S375C (numbered according to the EU system).

**Table 6. Antibodies used for the synthesis of the exemplified ADCs**

| Antibody abbreviation | Antibody | Fc Mutation | Sequence (Heavy Chain/Light Chain) |
|---|---|---|---|
| Ab M or CD74 | milatuzumab | Wild Type- | SEQ ID Nos 12, 25 |
| Anti-CD74-CysmAb Fc silent or CD74 Fc silent | milatuzumab | DANAPA Fc silencing | SEQ ID Nos 15, 25 |

**Conjugation**

**Method C1:** The antibody was bound on rmp Protein A resin (GE Healthcare) at a ratio of 10 mg Ab to 1 ml resin in PBS for 30 minutes by mixing in Biorad sized disposable column. To deblock the reactive cysteines, cysteine hydrochloride monohydrate was added to a final concentration of 20 mM. The mixture was agitated for 30 minutes at room temperature followed by washing 5 times the resin with 50 column volumes PBS on a vacuum manifold. The resin was then resuspended in an equal volume PBS containing 250 nM $CuCl_2$ and incubated for 1 hour and 30 min. The re-oxidized antibody was washed 5 times with 50 column volumes PBS on a vacuum manifold and resuspended in an equal volume PBS. To the mixture were added 10 fold-molar excess of 20mM linker-payload solution and equal volume of DMSO. The reaction was incubated at room temperature for 2 hours. To monitor the conjugation 20μl of resin slurry were removed, centrifuged, supernatant removed, and then eluted with 40 μl Antibody elution buffer (Thermo Fisher Scientific). The supernatant was analyzed by reverse phase chromatography using an Agilent PLRP-S 4000A 5 um, 4.6 x 50 mm column (Buffer A is water, 0.1% TFA, Buffer B Acetonitrile, 0.1% TFA, column held at 80°C, Flowrate 1.5 ml/min). After elimination the excess of linker-payload by washing the resin 5 times with 50 column volumes PBS on a vacuum manifold, the ADC was eluted from protein A with antibody elution buffer and neutralized with 1/10 volume 1 M Tris pH 9.0. All exemplified ADCs were buffer exchange by dialysis in PBS 1X pH 7.4 (Sigma Life Science, P3813, 10PAK), concentrated using Vivaspin 20, 50KD, PES (Sartorius Stedim, VS2031), filtered sterilely through 0.2μm sterile PES Filter, 25mm (Whatmann, G896-2502) and stored at 4°C. The conjugations were performed in a range of 5mg of antibody. All exemplified ADC were characterized by analytical size exclusion chromatography Superdex 200 Increase 5/150 GL (GE Healthcare, 28990945) to determine monomer percentage and LC-MS for DAR determination (Table16).

**Method C2.** Antibody was incubated with RMP Protein A resin (GE) at a ratio of 10 mg Ab to 1 ml resin in PBS for 15 minutes with mixing in an appropriately sized disposable column. Cysteine HCl was added to a final concentration of 20 mM and incubated with agitation for 30 min at room temperature to allow the reactive cysteines to be deblocked. The resin was quickly washed with 50 column volumes PBS on a vacuum manifold. The resin was then resuspeneded in an equal volume PBS containing 250 nM $CuCl_2$. Reformation of antibody interchain disulfides was monitored by taking time points. At each time point, 25 μL of resin slurry was removed, 1 μL of 20 mM MC-valcit-MMAE was added, and the tube flicked several times. The resin was spun down, supernatant removed, and then eluted with 50 μL Antibody elution buffer (Thermo). The resin was pelleted and the supernatant analyzed by reverse phase chromatography using an Agilent PLRP-S 4000A 5um, 4.6x50mm column (Buffer A is water, 0.1% TFA, Buffer B Acetonitrile, 0.1% TFA, column held at 80 °C, Flowrate 1.5 ml/min). Once it was determined that the antibody has reformed its interchain disulfide bonds, the resin was washed with 10 column volumes PBS and the resin was resuspended in an equal volume PBS and 8-12 equivalents of linker-payload in DMSO was added, with a final concentration of 10% DMSO in reaction and then incubated at room temperature for 3 hours. The resin was then washed with 50 column volumes PBS. The ADC was eluted from the protein A resin with Antibody elution buffer. The ADC was then buffer exchanged into PBS or other suitable buffer and preparative size exclusion chromatography to remove aggregates was performed (S200 Increase; GE), if required to remove aggregates. The following analyses were performed - analytical SEC to determine percent monomer, mass spectroscopy to determine DAR, LAL test to determine endotoxin load and protein concentration was determined by A280 utilizing extinction coefficient and molecular weight of antibody.

**Method C3 (Drug Substance Intermediate (DSi) Preparation).** 200 mg of antibody (1.36 μM) at 10 mg/ml was incubated with 20ml of settled RMP Protein A resin (GE Lifesciences, 17513803) and agitated for 15 minutes. Cysteine HCl monohydrate was added to a final concentration of 20 mM and incubated with agitation for 30 min at room temperature to allow the reactive cysteines to be deblocked. The resin was quickly washed with 50 column volumes PBS on a vacuum manifold. The resin was then resuspeneded in an equal volume PBS containing 250 nM $CuCl_2$. Reformation of antibody interchain disulfides was monitored by taking time points. At each time point, 25 μL of resin slurry was removed, 1 μL of 20 mM MC-valcit-MMAE was added, and the tube flicked several times. The resin was spun down, supernatant removed, and then eluted with 50 μL Antibody elution buffer (Thermo). The resin was pelleted and the supernatant analyzed by reverse phase chromatography using an Agilent PLRP-S 4000A 5um, 4.6x50mm column (Buffer A is water, 0.1% TFA, Buffer B Acetonitrile, 0.1% TFA, column held at 80 C, Flowrate 1.5 ml/min; Gradient 0 minutes - 30%B, 5 minutes - 45%B, 6.5 min - 100%B, 8 minutes - 100%B, 10 minutes - 30%). At 60 minutes after addition of CuCl2, was removed by washing with 50 column volumes of PBS on a vacuum manifold and then20 ml of PBS was added to resuspend and drained by gravity. The

antibody was eluted with 100 ml antibody elution buffer (Thermo Scientific, 21004) and then buffer exchanged into 1X PBS pH 7.2. The material was then concentrated using a centrifugal concentrator using an Amicon Ultra-15, 50KDa, regenerated cellulose (Millipore, UFC0905024), to 6.6 mg/ml aliquoted into 5 mg aliquots and flash frozen in liquid nitrogen and stored at -80 C until used

**(CD74-L5-P1):** 120 mg of antibody (0.816 μmoles, 1.0 equiv.) was incubated with 12 ml of settled RMP Protein A resin (GE Lifesciences, 17513803) and agitated for 15 minutes. Cysteine HCl monohydrate was added to a final concentration of 20 mM and incubated with agitation for 30 min at room temperature to allow the reactive cysteines to be deblocked. The resin was quickly washed with 50 column volumes PBS on a vacuum manifold. The resin was then resuspeneded in an equal volume PBS containing 250 nM $CuCl_2$. Reformation of antibody interchain disulfides was monitored by taking time points. At each time point, 25 μL of resin slurry was removed, 1 μL of 20 mM MC-valcit-MMAE was added, and the tube flicked several times. The resin was spun down, supernatant removed, and then eluted with 50 μL Antibody elution buffer (Thermo, 24001). The resin was pelleted and the supernatant analyzed by reverse phase chromatography using an Agilent PLRP-S 4000A 5um, 4.6x50mm column (Buffer A is water, 0.1% TFA, Buffer B Acetonitrile, 0.1% TFA, column held at 80 C, Flowrate 1.5 ml/min; Gradient 0 minutes - 30%B, 5 minutes - 45%B, 6.5 min - 100%B, 8 minutes - 100%B, 10 minutes - 30%). At 60 minutes after addition of CuCl2, was removed by washing with 50 column volumes of PBS on a vacuum manifold and then 12 ml of PBS was added to resuspend. To this slurry of resin and antibody. **L5-P1** (365 μl of a 20 mM solution in DMSO, 6.528 μmoles, 8 equiv.) was added. The resulting mixture was then incubated at ambient temperature for 3 hours. The resin was then washed with 50 column volumes PBS. The ADC was eluted from the resin with antibody elution buffer (Thermo Scientific, 21004). The ADC was then buffer exchanged into 1X PBS (20X PBS, TeknovaP0191) by dialysis and preparative size exclusion chromatography eluted in Dulbecco's PBS pH 7.2 (Hyclone SH30028.03) was done to remove aggregates was performed with a HiLoad 26/600 Superdex 200 pg (GE Healthcare, 28989336). The material was then concentrated using a centrifugal concentrator using an Amicon Ultra-15, 50KDa, regenerated cellulose (Millipore, UFC0905024), to 8 mg/ml and filtered sterilely through 0.22 μm sterile PVDF Filter, 25mm (Millapore, SLGV013SL) and stored at 4°C. The final yeild was 74.1 mg (0.504 μmol) The following analyses were performed: analytical size-exclusion chromatography (SEC) to determine percent monomer, mass spectroscopy (MS) to determine DAR, LAL test to determine endotoxin load and protein concentration determined by A280 utilizing extinction coefficient and molecular weight of antibody. HRMS data (protein method) indicated a dominant mass of the heavy chain was 54294 da, giving a DAR of 4.4 as calculated by comparing MS intensities of peaks for DAR1 DAR2 and DAR3 species. SEC indicated </= 1% aggregation, as determined by comparison of the area of the high-molecular-weight peak absorbance at 210 and 280 nm with the area of the peak absorbance for monomeric ADC.

**(CD74-L7-P1):** 20 mg of antibody (0.136 μmoles, 1.0 equiv.) was incubated with 2 ml of settled RMP Protein A resin (GE Lifesciences, 17513803) and agitated for 15 minutes. Cysteine HCl monohydrate was added to a final concentration of 20 mM and incubated with agitation for 30 min at room temperature to allow the reactive cysteines to be deblocked. The resin was quickly washed with 50 column volumes PBS on a vacuum manifold. The resin was then resuspeneded in an equal volume PBS containing 250 nM $CuCl_2$. Reformation of antibody interchain disulfides was monitored by taking time points. At each time point, 25 μL of resin slurry was removed, 1 μL of 20 mM MC-valcit-MMAE was added, and the tube flicked several times. The resin was spun down, supernatant removed, and then eluted with 50 μL Antibody elution buffer (Thermo). The resin was pelleted and the supernatant analyzed by reverse phase chromatography using an Agilent PLRP-S 4000A 5um, 4.6x50mm column (Buffer A is water, 0.1% TFA, Buffer B Acetonitrile, 0.1% TFA, column held at 80 C, Flowrate 1.5 ml/min; Gradient 0 minutes - 30%B, 5 minutes - 45%B, 6.5 min - 100%B, 8 minutes - 100%B, 10 minutes - 30%). At 60 minutes after addition of CuCl2, was removed by washing with 50 column volumes of PBS on a vacuum manifold and then 2 ml of PBS was added to resuspend. To this slurry of resin and antibody. **L7-P1** (55 μl of a 20 mM solution in DMSO, 1.088 μmoles, 8 equiv.) was added. The resulting mixture was then incubated at ambient temperature for 3 hours. The resin was then washed with 50 column volumes PBS. The ADC was eluted from the resin with Antibody elution buffer (Thermo Scientific, 21004). The ADC was then buffer exchanged into 1X PBS (20X PBS, TeknovaP0191) by dialysis and preparative size exclusion chromatography eluted in Dulbecco's PBS pH 7.2 (Hyclone SH30028.03) was done to remove aggregates was performed with a HiLoad 16/600 Superdex 200 pg (GE Healthcare, 28989335). The material was then concentrated using a centrifugal concentrator using an Amicon Ultra-15, 50KDa, regenerated cellulose (Millipore, UFC0905024), to 4.5 mg/ml and filtered sterilely through 0.22 μm sterile PVDF Filter, 25mm (Millapore, SLGV013SL) and stored at 4°C. The final yeild was 1.6 mg (0.00681 μmol) The following analyses were performed: analytical size-exclusion chromatography (SEC) to determine percent monomer, mass spectroscopy (MS) to determine DAR, LAL test to determine endotoxin load and protein concentration determined by A280 utilizing extinction coefficient and molecular weight of antibody. HRMS data (protein method) indicated a dominant mass of the heavy chain was 54562 da, giving a DAR of 3.8 was calculated by comparing MS intensities of peaks for DAR1 DAR2 and DAR3 species. SEC indicated 1.8% aggregation, as determined by comparison of the area of the high-molecular-weight peak absorbance at 210 and 280 nm with the area of the peak absorbance for monomeric ADC.

Analytical Methods

**Liquid Chromatography-Mass Spectrometry (LC/MS) I**

**General Methodology:** Drug-to-antibody ratio (DAR) of exemplary ADCs was determined by liquid chromatography-mass spectrometry (LC/MS) according to one of the following methods (i.e., LC-I, LCII and LC-III). For all LC methods, mobile phase A was purified MS grade water (Biosolve, Dieuze, France, 00232141B1BS), mobile phase B was MS grade acetonitrile (Biosolve, Dieuze, France, 0001204101BS), and mobile phase D was purified MS grade water supplemented with 1 % of formic acid (FA) (Honeywell/Fluka, Bucharest, Romania, 56302). Mobile phase D was fixed at 10 % in order to maintain a 0.1% FA mobile phase composition and column temperature was set at 80°C. A general MS method was optimized for all ADCs synthesized, except for one ADC where native MS was used in order to determine average DAR (see MS-I and Table 16 below).

**General Methodology (1):** Drug-to-antibody ratio (DAR) of exemplary ADCs was determined by liquid chromatography-mass spectrometry (LC/MS) according to the following method. For all LC methods, mobile phase A was purified MS grade water (Honeywell, LC015-1), mobile phase B was MS grade 80% Isopropanol (Honeywell LC323-1): 20% acetonitrile (Honeywell, LC015-1), LC323-1), supplemented with 1 % of formic acid (FA) (Thermo Scientific, 85178). The column temperature was set at 80°C. A general MS method was optimized for all ADCs synthesized. The column used for analysis was an Agilent PLRP-S 4000 A; 2.1x150mm, 8um (Agilent, PL1912-3803). Flowrate used was 0.3 ml/min. The gradient used was 0-0.75 minute 95%A, 0.76 -1.9 minute 75%A, 1.91-11.0 minute 50%A, 11.01-11.50 10%A, 11.51-13.50 minute 95%A,13.51-18 minute 95%A ona Acquitty Bio H-Class Quarernary UPLC (Waters). MS system was Xevo G2-XS QToF ESI mass spectrometer (Waters) and data acquired from 1.5-11 minutes and masses were analyzed between 15000-80000 daltons. DAR was determined from the deconvoluted spectra or UV chromatogram by summing the integrated MS (total ion current) or UV (280 nm) peak area of unconjugated and conjugated given species (mAb or associated fragment), weighted by multiplying each area by the number of drug attached. The summed, weighted areas were divided by the sum of total area and the results produced a final average DAR value for the full ADC.

**LC-I:** ADC was loaded onto a MassPREP Micro desalting column (2.1 x 5.0 mm, Waters, Saint- Quentin-en-Yvelines, France, 186004032). For intact mass analysis, a desalting step was performed for 0.5 min at 5% mobile phase B with a flow rate of 0.5 ml/min. Elution step was performed with a gradient from 0.51 min at 5% B to 2.0 min at 90 % B with a flow rate of 0.2 ml/min. Two wash steps were set from 2.1 min to 2.7 min and from 2.8 min to 3.4 min at 5% B to 90% B with a flow rate of 0.5 ml/min. Finally, a conditioning step was used at 3.5 min for 0.5 min at 5 % B (0.5 ml/min).

For ADC analysis in reduced conditions, a desalting step was performed for 0.5 min at 5% B with a flow rate of 0.2 ml/min. Then, the elution step started with a gradient from 0.51 min at 10% B to 7.61 min at 80 % B with a flow rate of 0.2 ml/min. Two washing steps were set from 8.1 min to 8.6 min and from 8.7 min to 9.2 min from 5% B to 90% B (0.5 ml/min). Finally, a conditioning step was performed at 9.3 min for 0.5 min at 5 % B with a flow rate of 0.5 ml/min.

**LC-II:** ADC was loaded onto a MabPac RP column (2.1 x 100 mm, 4 μm, Thermo Fisher Scientific, Rockford, IL, 088647). For analysis in both intact and reduced conditions, a desalting step was performed for 1.4 min at 20% of B with a flow rate of 0.4 mL/min. Then, the elution step was performed with a gradient from 1.5 min at 20% B to 11.5 min at 70 % B with a flow rate of 0.3 ml/min. A wash step was set from 11.75 min to 13.75 min at 90% B with a flow rate of 0.5 ml/min. Finally, a conditioning step was used at 14.0 min for 1.0 min at 20 % B with a flow rate of 0.4 ml/min.

**LC-III:** ADC was loaded onto a Bioresolve RP mAb Polyphenyl, 450A, 2.7 μm, 2.1 x 150 mm (Waters, Saint-Quentin-en-Yvelines, France, 186008946). For analysis in both intact and reduced conditions, a desalting step was performed for 1.4 min at 20% of B with a flow rate of 0.4 ml/min. Elution step was performed with a gradient from 1.5 min at 20% B to 11.5 min at 70 % B with a flow rate of 0.3 ml/min. A wash step was set from 11.75 min to 13.75 min at 90% B with a flow rate of 0.5 ml/min. Finally, a conditioning step was used at 14.0 min for 1.0 min at 20 % B with a flow rate of 0.4 ml/min.

**LC-IV:** ADC was loaded onto a Bioresolve RP mAb Polyphenyl, 450A, 2.7μm, 2.1 x 150mm (Waters, Saint-Quentin-en-Yvelines, France, 186008946). For analysis in both intact and reduced conditions, a desalting step was performed for 1.5 min at 20% of B with a flow rate of 0.6 mL/min. Elution step was performed with a gradient from 1.5 min at 20% B to 16.5 min at 50 % B with a flow rate of 0.6 mL/min. A wash step was set from 16.8 min to 18.8 min at 90% B with a flow rate of 0.6 mL/min. Finally, a conditioning step was used at 19.1 min for 1.9 min at 20 % B with a flow rate of 0.6 mL/min (Total run time = 21minutes).

**MS-I:** ADC was buffer exchanged with a MS compatible buffer and infused directly into a hybrid Q-TOF MS instrument (Synapt G2-S HDMS, Waters, Manchester, UK) equipped with an automated chip-based nanoESI source (Triversa Nanomate, Advion Biosciences, Ithaca, NY, USA) operating in positive ion mode. Instrumental MS parameters were tuned to preserve native 3D structure in the gas phase and ensure efficient ion desolvation and transmission.

LC-MS analysis was performed using a Waters UPLC H-Class Bio chromatography system hyphenated with a Xevo G2 XS Q-TOF ESI mass spectrometer (Waters, Manchester, UK). The ADC was either analyzed in intact condition (no preliminary treatment) or following reduction with 5 mM (final concentration) of DTT (Thermo Scientific, Rockford, IL, 20291). Subsequently, treated ADC was analyzed using the aforementioned **LC-IV** (Table 16). Electrospray-ionization time-of-flight mass spectra of the analytes were acquired using MassLynx™ acquisition software (Waters, Manchester,

UK). Then, the extracted intensity vs. m/z spectrum was deconvoluted using Maximum Entropy (MaxEnt) method of MassLynx™ software in order to determine the mass of each intact antibody species or each reduced antibody fragment depending on the treatment used. Finally, DAR was determined from the deconvoluted spectra or UV chromatogram by summing the integrated MS (total ion current) or UV (280 nm) peak area of unconjugated and conjugated given species (mAb or associated fragment). For the DAR determination by UV chromatogram, relative area percentage of each species was multiplied by the number of drug attached. The summed, weighted areas of each species were divided by the sum of total relative area percentage and the results produced an estimation of the final average DAR value for the full ADC. For the DAR determination by deconvoluted spectra, the percentage of each species identified was calculated by intensity peak value from deconvoluted spectra. The percentage obtained, was multiplied by the number of drug attached. The summed results produced an estimation of the final average DAR value for the full ADC.

**Liquid Chromatography-Mass Spectrometry (LC/MS) II**

LC/MS data were acquired using an instrument with the following parameters (Table 7):

**Table 7. Parameters**

| | |
|---|---|
| Pump | Waters AcQuity UPLC Binary Solvent Manager |
| Sample Manager | Waters AcQuity UPLC Sample Manager |
| Column Compartment | Waters AcQuity UPLC Column Manager |
| Detector | Waters AcQuity UPLC PDA |
| ELSD | Shimadzu ELSD-LTII |
| Mass Spec | Waters SQD |
| Columns | AcQuity UPLC BEH C18 1.7μm 2.1x50mm |
| Eluent A1 | 0.1% Formic Acid in Water |
| Eluent B1 | 0.1% Formic Acid in Acetonitrile |
| Eluent A2 | 5mM Ammonium Hydroxide in Water |
| Eluent B2 | 5mM Ammonium Hydroxide in Acetonitrile |

The methods used to generate LC/MS data were as follows (Tables 8-10):

**Table 8. 2 minute acidic method**

| | | | |
|---|---|---|---|
| 2 min acidic method | | | |
| Eluent A1 | 0.1% Formic Acid in Water | | |
| Eluent B1 | 0.1% Formic Acid in Acetonitrile | | |
| Flow | 1.0 mL/min | | |
| Stop Time | 3.00 min | | |
| pH | 2.6 | | |
| Gradient | Time | %A (Eluent A1) | %B (Eluent B1) |
| | 0.00 | 95 | 5 |
| | 0.20 | 95 | 5 |
| | 2.00 | 5 | 95 |
| | 2.50 | 5 | 95 |
| | 2.60 | 95 | 5 |
| | 3.00 | 95 | 5 |
| Column | AcQuity UPLC BEH C18 1.7μm 2.1x50mm | | |
| Column Temperature | 50°C | | |
| TAC | 210-400 nm | | |
| Mass Range | 120-1500 Da | | |
| Scan Time | 0.3 sec | | |

**Table 9. 2 minute basic method**

| | | | |
|---|---|---|---|
| 2 min basic method | | | |
| Eluent A2 | 5mM Ammonium Hydroxide in Water | | |
| Eluent B2 | 5mM Ammonium Hydroxide in Acetonitrile | | |
| Flow | 1.0 mL/min | | |
| Stop Time | 3.00 min | | |
| pH | 10.2 | | |
| Gradient | Time | %A (Eluent A2) | %B (Eluent B2) |
| | 0.00 | 95 | 5 |
| | 0.20 | 95 | 5 |
| | 2.00 | 5 | 95 |
| | 2.50 | 5 | 95 |
| | 2.60 | 95 | 5 |
| | 3.00 | 95 | 5 |
| Column | AcQuity UPLC BEH C18 1.7μm 2.1x50mm | | |
| Column Temperature | 50°C | | |
| TAC | 210-400 nm | | |
| Mass Range | 120-1500 Da | | |
| Scan Time | 0.3 sec | | |

**Table 10. 5 minute acidic method**

| | | | |
|---|---|---|---|
| 5 min acidic method | | | |
| Flow | 1.0 mL/min | | |
| Stop Time | 5.20 min | | |
| pH | 2.6 | | |
| Gradient | Time | %A (Eluent A1) | %B (Eluent B1) |
| | 0.00 | 98 | 2 |
| | 4.40 | 2 | 98 |
| | 5.15 | 2 | 98 |
| | 5.19 | 98 | 2 |
| Column | AcQuity UPLC BEH C18 1.7μm 2.1x50mm | | |
| Column Temperature | 50°C | | |
| TAC | 210-400 nm | | |
| Mass Range | 120-1500 Da | | |
| Scan Time | 0.3 sec | | |

**High Resolution Mass Spectrometry (HRMS)**

HRMS data were acquired using an instrument with the following parameters (Table 11):

**Table 11. Parameters**

| | |
|---|---|
| Pump | Waters AcQuity UPLC Binary Solvent Manager |
| Sample Manager | Waters AcQuity UPLC Sample Manager |
| Column Compartment | Waters AcQuity UPLC Column Manager |
| Detector | Waters AcQuity UPLC PDA |
| ELSD | n/a |
| Mass Spec | Waters Xevo G2 Qtof |
| Columns | AcQuity UPLC PrST C4 300Å 1.7μm 2.1x100mm<br>AcQuity UPLC CSH C18 1.7 um 2.1x50mm<br>ProSwift RP-3U 4.6x50mm SS |
| Eluent A1 | 0.1% Formic Acid in Water |

(continued)

| | |
|---|---|
| Eluent B1 | 0.1% Formic Acid in Acetonitrile |
| Eluent A2 | 0.05% Trifluoroacetic Acid in Water |
| Eluent B2 | 0.05% Trifluoroacetic Acid in Acetonitrile |

The methods used to generate HRMS data for linker/payloads and synthetic intermediates were as follows (Tables 7 and 8):

**Table 12. 5 minute acidic method**

| | | | | |
|---|---|---|---|---|
| 5 min acidic method: | | | | |
| Flow | 1.0 mL/min | | | |
| Stop Time | 5.2 min | | | |
| pH | 2.6 | | | |
| Gradient | | Time | %A (Eluent A2) | %B (Eluent B2) |
| | | 0.00 | 98 | 2 |
| | | 4.40 | 2 | 98 |
| | | 5.15 | 2 | 98 |
| | | 5.19 | 98 | 2 |
| Column | AcQuity UPLC CSH C18 1.7μm 2.1x50mm | | | |
| Column Temperature | 50°C | | | |
| UV | 210-400 nm | | | |
| Mass Range | 300-4000 Da | | | |
| Scan Time | 0.5 sec | | | |

**Table 13. 2 minute acidic method**

| | | | | |
|---|---|---|---|---|
| 2 min acidic method: | 1.0 mL/min | | | |
| Flow | | | | |
| Stop Time | 2.20 min | | | |
| pH | 2.6 | | | |
| Gradient | | Time | %A (Eluent A2) | %B (Eluent B2) |
| | | 0.00 | 98 | 2 |
| | | 0.06 | 98 | 2 |
| | | 1.76 | 2 | 98 |
| | | 2.00 | 2 | 98 |
| | | 2.16 | 98 | 2 |
| Column | AcQuity UPLC CSH C18 1.7μm 2.1x50mm | | | |
| Column Temperature | 50°C | | | |
| UV | 210-400 nm | | | |
| Mass Range | 100-2050 Da | | | |
| Scan Time | 0.2 sec | | | |

The method used to generate HRMS data for the ADCs was as follows (Table 14):

**Table 14. Protein method**

| | | | | |
|---|---|---|---|---|
| Protein method: | | | | |
| Flow | 1.0 mL/min | | | |
| Stop Time | 3.30 min | | | |
| pH | 2.6 | | | |
| Gradient | | Time | %A (Eluent) | %B (Eluent) |
| | | 0.00 | 98 | 2 |
| | | 0.70 | 98 | 2 |
| | | 2.00 | 2 | 98 |
| | | 2.10 | 2 | 98 |
| | | 2.30 | 98 | 2 |
| | | 3.30 | 98 | 2 |
| Column | ProSwift RP-3U 4.6x50mm SS | | | |
| Column | 50°C | | | |
| TAC | 210-400 nm | | | |
| Mass Range | 600-3900 Da | | | |
| Processing Range | 14000-170000 Da | | | |
| Scan Time | 1.5 sec | | | |

**Size Exclusion Chromatography (SEC) I**

Size exclusion chromatography (SEC) was performed to determine the quality of the ADCs and aggregation percentage (%) after purification. The analysis was performed on analytical column Superdex 200 Increase 5/150 GL (GE Healthcare, 28990945) in isocratic conditions 100% PBS pH 7.4 (Sigma, P3813, 10PAK), flow 0.45 ml/min for 12 minutes. The % aggregate fraction of the ADC sample was quantified based on the peak area absorbance at 280 nm. Calculation was based on the ratio between the high molecular weight eluent at 280 nm divided by the sum of peak area absorbance at the same wavelength of the high molecular weight and monomeric eluents multiplied by 100%.

**Size exclusion chromatography (SEC) (1)** was performed to determine the quality of the ADCs and aggregation percentage (%) after purification. The analysis was performed on analytical column Superdex 200 Increase 5/150 GL (GE Healthcare, 28990945) in isocratic conditions PBS pH 7.2 ((Hyclone SH30028.03)), supplemented with 150 mM NaCl and 1 mM EDTA, flow 0.45 ml/min for 8 minutes. The % aggregate fraction of the ADC sample was quantified based on the peak area absorbance at 280 nm. Calculation was based on the ratio between the high molecular weight eluent at 280 nm divided by the sum of peak area absorbance at the same wavelength of the high molecular weight and monomeric eluents multiplied by 100%. Data was aquired on an Agilent Bio-Inert 1260 HPLC outfitted with a Wyatt miniDAWN light scattering and Treos refractive index detectors (Wyatt Technologies, Santa Barbara, CA).

**Size Exclusion Chromatography (SEC) II**

SEC data were acquired using an instrument with the following parameters (Table 15) and a run length of 12 minutes:

**Table 15. Parameters**

| | |
|---|---|
| Pump | Waters bioAcQuity UPLC Quaternary Solvent Manager |
| Sample Manager | Waters bioAcQuity UPLC Sample Manager FTN |
| Column Compartment | Waters AcQuity UPLC 30cm Column Heater |
| Detector | Waters AcQuity UPLC PDA |
| ELSD | n/a |
| Mass Spec | n/a |
| Columns | Superdex 200 Increase 5/150 GL |
| Eluent A1 | 1X PBS (Phosphate Buffered Saline) + 0.1M NaCl + 5% Isopropanol |
| Eluent B1 | n/a |

Results

Characterization of exemplary ADCs is summarized in Table 16, 16a and 16b (coupling and LC/MS method, aggregation status, and DAR). The average DAR values were determined using the above LC/MS methods (LC/MS I) and percentage aggregation was determined using the above SEC methods (SEC I).

**Table 16. ADC analytical characterization and conjugation methodology**

| ADS | Conjugation Method | LC-MS method | DAR | % Agg. (by SEC I) |
|---|---|---|---|---|
| Ab M - L9-P16 | C1 | LC-IV | 2.8 | 0.2 |
| Ab M - L9-P17 | C1 | LC-IV | 3.2 | 0.2 |
| Ab M - L9-P15 | C1 | LC-IV | 2.7 | 0.2 |
| Ab M - L9-C1 | C1 | LC-IV | 2.7 | 3.8 |

**Table 16a. ADC analytical characterization and conjugation methodology**

| ADC | Conjugation Method | LC-MS Method I | DAR (by LC-MS I) | % Agg. (by SEC I) |
|---|---|---|---|---|
| CD74_Fc Silenced-L5-P1 | C2 | 1 | 4.4 | 0.9 |
| CD74_Fc Silenced-L7-P1 | C2 | 1 | 3.8 | 1.8 |

**Table 16b. ADC analytical characterization and conjugation methodology**

| ADC | Conjugation Method | HRMS Method | DAR (by HRMS) | % Agg. (by SEC II) |
|---|---|---|---|---|
| CD74-L5-P1 | C3 | Protein | 3.9 | 6.0 |
| CD74-L30-P1 | C3 | Protein | 3.8 | 5.7 |
| CD74-L31-P1 | C3 | Protein | 3.7 | 5.3 |
| CD74-L32-P1 | C3 | Protein | 3.6 | 10.3 |
| CD74-L33-P1 | C3 | Protein | 3.5 | 10.7 |
| CD74-L34-P1 | C3 | Protein | 3.9 | 10.1 |
| CD74-L35-P1 | C3 | Protein | 3.8 | 10.0 |
| CD74-L36-P1 | C3 | Protein | 3.8 | 8.0 |
| CD74-L37-P1 | C3 | Protein | 3.8 | 11.0 |
| CD74-L60-P1 | C3 | Protein | 3.7 | 8.0 |
| CD74-L61-P1 | C3 | Protein | 3.8 | 3.4 |
| CD74-L62-P1 | C3 | Protein | 3.8 | 5.5 |
| CD74-L63-P1 | C3 | Protein | 3.7 | 12.0 |
| CD74-L67-P1 | C3 | Protein | 3.7 | 9.1 |
| CD74-L68-P1 | C3 | Protein | 3.8 | 17 |
| CD74-L69-P1 | C3 | Protein | 3.8 | 5.7 |
| CD74-L71-P1 | C3 | Protein | 3.8 | 12.3 |
| CD74-L72-P1 | C3 | Protein | 3.5 | 6.0 |
| CD74-L77-P1 | C3 | Protein | 3.5 | 11.0 |
| CD74-L78-P1 | C3 | Protein | 3.6 | 18.0 |
| CD74-L79-P1 | C3 | Protein | 3.7 | 21.0 |
| CD74-L86-P1 | C3 | Protein | 3.7 | 9.0 |

**Example 5: In Vitro Assessment I**

Inhibition of Mcl-1 by Fluorescence Polarization (FP)

The relative binding potency of each compound was determined via Fluorescence Polarization (FP). The method utilized a fluorescein-labelled ligand (Fluorescein-βAla-Ahx-A-REIGAQLRRMADDLNAQY-OH (SEQ ID NO: 72); MW 2,765) which binds to Mcl-1 protein (UniProt Reference Sequence: Q07820; SEQ ID NO:48), leading to an increased anisotropy measured in milli-polarization (mP) units using a reader. The addition of a compound which binds competitively to the same site as the ligand results in a greater proportion of unbound ligand in the system, as indicated by a decrease in mP units.

An 11-point serial dilution of each compound was prepared in DMSO and 2 μl transferred into flat bottomed, low binding, 384-well plate (final DMSO concentration 5 %). 38 μl of buffer (10 mM 4-(2-hydroxyethyl)-1-piperazineethanesulfonic acid [HEPES], 150 mM NaCl, 0.05 % Tween 20, pH 7.4), containing the fluorescein-labelled ligand (final concentration 1 nM) and Mcl-1 protein (final concentration 5 nM) was then added.

Assay plates were incubated ~2 hours at room temperature before FP was measured on a Biomek Synergy2 reader (Ex. 528 nm, Em. 640 nm, Cut off 510 nm) and mP units calculated. The binding of increasing doses of test compound was expressed as a percentage reduction in mP compared to a window established between '5 % DMSO only' and '100 % inhibition' controls. 11-point dose response curves were plotted with XL-Fit software using a 4-Parameter Logistic Model (Sigmoidal Dose-Response Model) and the inhibitory concentrations that gave a 50 % reduction in mP ($IC_{50}$) were determined. The results are presented in Table 17 below. The results suggest that the tested compounds inhibit interaction between the Mcl-1 protein and the fluorescein-labelled peptide.

In Vitro Cytotoxicity

Cytotoxicity studies were carried out on the NCI-H929 multiple myeloma cell line. The cells were distributed onto microplates and exposed to the test compounds for 48 hours. The cell viability was then quantified by a colorimetric assay, the Microculture Tetrazolium Assay (Carmichael et al. (1987) Cancer Res. 47:936-42).

The results are expressed in $IC_{50}$ (the concentration of compound that inhibits cell viability by 50 %) and are presented in Table 17 below. The results show that the tested compounds are cytotoxic in NCI-H929 cells.

**Table 17. $IC_{50}$ of Mcl-1 inhibition and cytotoxicity in NCI-H929 cells**

| Payload | $IC_{50}$ (M) Mcl-1 FP | $IC_{50}$ (M) MTT H929 | Payload | $IC_{50}$ (M) Mcl-1 FP | $IC_{50}$ (M) MTT H929 |
|---|---|---|---|---|---|
| **C1** | 2.8E-09 | 3.0E-09 | **C8** | 3.0E-09 | 5.74E-07 |
| **C2** | 2.6E-09 | 8.51E-06 | **C9** | 2.5E-09 | 2.27E-06 |
| **C3** | 9.48E-10 | 4.2E-08 | **C10** | 3.4E-09 | 1.35E-08 |
| **C4** | 2.6E-09 | 3.21E-06 | **C11** | 5.3E-09 | 7.0E-09 |
| **C5** | 4.05E-09 | 4.08E-09 | **C12** | 5.9E-09 | 4.2E-08 |
| **C6** | 1.18E-09 | 3.0E-09 | **C13** | 2.2E-09 | 3.76E-09 |
| **C7** | 2.6E-09 | 1.88E-09 | **C14** | 8.4E-10 | 2.61E-09 |

**Example 6: In Vitro Assessment II - In vitro activity of CD74-L7-P1 and P1 payload in DLBCL cell lines (CTG 72h)**

DLBCL cells lines (Karpas422, SUDHL4, NUDHL1, SUDHL6, SUDHL10, DOHHL2 and SUDHL5) were cultivated in RPMI supplemented with 10% heat inactivated fetal bovine serum, penicillin (100 IU/ml), streptomycin (100 μg/ml) and L-glutamine (2 mM). Cells were cultured at 37°C in a humidified atmosphere containing 5% $CO_2$. Cells were seeded in 96 microwell plates and exposed to the ADCs or the corresponding payload for 72h (serially diluted; 9 concentrations each, triplicates). Effects of ADCs or payloads on cell viability were assessed after 3 days of incubation at 37°C/5% $CO_2$ by quantification of cellular ATP levels using CellTiterGlo at 75μL reagent/well. All the conditions were tested in triplicates. Luminescence was quantified on a multipurpose plate reader. $IC_{50}$s were calculated using standard four-parametric curve fitting. $IC_{50}$ is defined as the compound concentration at which the CTG signal is reduced to 50% of that measured for the control. Each experiment was performed at least twice, with results being reproducible. As shown in Table **18** and **FIGs. 1A** and **1B,** the payload P1 and the ADC CD74-L7-P1 induced a dose dependent decrease in the viability of DLBCL cells in CTG assay. **Table 18: $IC_{50}$ of cytotoxicity of ADCs and payload in various cell lines**

| IC50 M (CTG 72h) | Karpas422 | SUDHL4 | NUDHL1 | SUDHL6 | SUDHL10 | DOHHL2 | SUDHL5 |
|---|---|---|---|---|---|---|---|
| IgG-L7-P1 | Not tested | Not tested | Not tested | Not tested | >3.0e-007 | >3.0e-007 | >3.0e-007 |
| CD74-L7-P1 | 1.054e-009 | 4.793e-010 | 1.033e-008 | 1.453e-009 | 9.417e-011 | 1.195e-009 | 3.139e-011 |
| P1 | 1.921e-009 | 9.373e-010 | 1.849e-008 | 3.087e-009 | 2.164e-01-0 | 1.824e-009 | 2.164e-010 |

**Example 7: In Vitro Assessment III - In vitro activity of CD74-Mcl-1 ADCs and payloads in Monomac1 AML cell line (CTG 72h)**Monomac1 cells were cultivated in RPMI supplemented with 10% heat inactivated fetal bovine serum, penicillin (100 IU/ml), streptomycin (100 μg/ml) and L-glutamine (2 mM). Cell lines were cultured at 37°C in a humidified atmosphere containing 5% $CO_2$. Cells were seeded in 96 microwell plates and exposed to the payloads or ADCs for 72h (serially diluted; 9 concentrations each, triplicates). Effects of payloads or ADCs on cell viability were assessed after 3 days of incubation at 37°C/5% $CO_2$ by quantification of cellular ATP levels using CellTiterGlo at 75μL reagent/well. All the conditions were tested in triplicates. Luminescence was quantified on a multipurpose plate reader. $IC_{50}$s were calculated using standard four-parametric curve fitting. $IC_{50}$ is defined as the compound concentration at which the CTG signal is reduced to 50% of that measured for the control. Each experiment was performed at least twice, with results being reproducible. As shown in Table **19** and **FIG. 2,** the payloads and the anti-CD74-Mcl-1 ADCs induced a dose dependent decrease in the viability of Monomac1 cells in CTG assay.

**Table 19. $IC_{50}$ of cytotoxicity of of ADCs and payload in Monomac-1 cell**

| ADCs or payloads | IC50 M (CTG 72h) Monomac1 |
|---|---|
| CD74-L7-P1 | 9.217e-011 |
| P1 | 1.294e-009 |
| CD74-L9-P1 | 4.815e-010 |
| P1 | 1.322e-009 |
| CD74-L9-P17 | 2.367e-011 |
| P17 | 6.201e-010 |
| CD74-L9-P16 | 2.064e-011 |
| P16 | 2.812e-010 |
| CD74-L9-P15 | 3.996e-011 |
| P15 | 3.86e-010 |

**Example 8: In Vitro Assessment IV - In vitro activity of CD74 ADCs in various cancer cell lines**

*Cell Lines*

The CD74 MCL-1 antibody drug conjugate CD74-L7-P1, Isotype IgG-L7-P1 ADC, and MCL-1 free payload P1 were tested against six endogenous cancer cell lines. KMS-21BM (JCRB No. JCRB1185 cultured in RPMI-1640 + 10% FBS), KMS-20 (JCRB No. JCRB1196 cultured in RPMI-1640 + 10% FBS), MONO-MAC-1 (DSMZ No. ACC-252 cultured in RPMI-1640 + 10% FBS + 1x non-essential amino acids + 1 mM sodium pyruvate), NOMO-1 (DSMZ No. ACC-542 cultured in RPMI-1640 + 10% FBS), Kasumi-6 (ATCC No. CRL-2775 cultured in RPMI-1640 + 20% FBS + 1.5 g/L sodium bicarbonate + 4.5 g/L glucose + 10 mM HEPES, + 1 mM sodium pyruvate supplemented with 2 ng/ml human recombinant granulocyte macrophage colony stimulating factor (GM-CSF) and EOL-1 (DSMZ No. ACC-386 cultured in RPMI-1640 + 10% FBS)

*Inhibition of cell proliferation and survival*

The ability of the CD74 MCL-1 antibody drug conjugate (CD74-L7-P1), Isotype IgG-L7-P1 ADC, and MCL-1 free payload P1 to inhibit cell proliferation and survival was assessed using the Promega CellTiter-Glo® proliferation assay. Cell lines were cultured in media that is optimal for their growth at 5% $CO_2$, 37°C in a tissue culture incubator. Prior to seeding for the proliferation assay, the cells were split at least 2 days before the assay to ensure optimal growth density. On the day of seeding, suspension cells were harvested. Cell viability and cell density were determined using a cell counter (Vi-Cell XR

Cell Viability Analyzer, Beckman Coulter). Cells with higher than 85% viability were seeded in white clear bottom 384-well TC treated plates (Corning cat. # 3765). Cells were seeded at a density of 1,000 cells per well in 45 μL of standard growth media. Plates were incubated at 5% $CO_2$, 37°C overnight in a tissue culture incubator. The next day, free MCL-1 payload (P1), targeting MCL-1 ADC, and non-targeting isotype ADCs were prepared at 10X in standard growth media. The prepared drug treatments were then added to the cells resulting in final concentrations of 0.0005 - 500 nM and a final volume of 50 μL per well. Each drug concentration was tested in quadruplets. Plates were incubated at 5% $CO_2$, 37°C for 5 days in a tissue culture incubator, after which cell viability was assessed through the addition of 25 μL of CellTiter Glo® (Promega, cat# G7573), a reagent which lyses cells and measures total adenosine triphosphate (ATP) content. Plates were incubated at room temperature for 10 minutes to stabilize luminescent signals prior to reading using a luminescence reader (EnVision Multilabel Plate Reader, PerkinElmer). To evaluate the effect of the drug treatments, luminescent counts from wells containing untreated cells (100% viability) were used to normalize treated samples. A variable slope model was applied to fit a nonlinear regression curve to the data in GraphPad PRISM version 7.02 software. IC50 and Amax values were extrapolated from the resultant curves.

*Result*

The dose response curves of representative cancer cell lines expressing the CD74 target of interest are shown in **FIG. 3** (square = CD74-L7-P1, circle IgG-L7-P1, triangle = free MCL1 payload P1). The concentrations of treatment required to inhibit 50% of cell growth or survival (IC50) were calculated with representative IC50 values of the cell lines tested summarized in Table 20.

**Table 20: CD74 MCL1 ADCs**

| | CD74-L7-P1 | | IgG-L7-P1 | | P1 | |
|---|---|---|---|---|---|---|
| | IC50 (nM) | Amax | IC50 (nM) | Amax | IC50 (nM) | Amax |
| KMS-21BM | 0.948 | 98.75 | >200 | -- | 0.941 | 85.27 |
| KMS-20 | 0.814 | 101.3 | >200 | -- | 4.946 | 94.34 |
| MONO-MAC-1 | ** | ** | >200 | -- | 1.151 | 82.41 |
| Kasumi-6 | 2.947 | 127.8 | >200 | -- | 9.266 | 88.12 |
| NOMO-1 | 1.433 | 95.88 | >200 | -- | 5.015 | 110.9 |
| EOL-1 | 0.118 | 124.0 | >200 | -- | 1.095 | 67.97 |
| ** tested but curve did not converge | | | | | | |

**Example 9: In Vitro Assessment V - In vitro activity of CD74 ADCs as single agent and in combination with Bcl-2 inhbitors**

*Cell Lines*

The CD74 MCL-1 antibody drug conjugates were tested against three endogenous cancer cell lines. MONO-MAC-1 (DSMZ No. ACC-252 cultured in RPMI-1640 + 10% FBS + 1x non-essential amino acids + 1 mM sodium pyruvate), NOMO-1 (DSMZ No. ACC-542 cultured in RPMI-1640 + 10% FBS) and EOL-1 (DSMZ No. ACC-386 cultured in RPMI-1640 + 10% FBS)

*Inhibition of cell proliferation and survival*

The ability of the CD74 MCL-1 antibody drug conjugates to inhibit cell proliferation in combination with BCL-2 inhibitor and survival was assessed using the Promega CellTiterGlo® proliferation assay. Cell lines were cultured in media that is optimal for their growth at 5% $CO_2$, 37°C in a tissue culture incubator. Prior to seeding for the proliferation assay, the cells were split at least 2 days before the assay to ensure optimal growth density. On the day of seeding, suspension cells were harvested. Cell viability and cell density were determined using a cell counter (Vi-Cell XR Cell Viability Analyzer, Beckman Coulter). Cells with higher than 85% viability were seeded in white clear bottom 384-well TC treated plates (Corning cat. # 3765). Cells were seeded at a density of 1,000 cells per well in 45 μL of standard growth media. Plates were incubated at 5% $CO_2$, 37°C overnight in a tissue culture incubator. The next day, free MCL-1 payload (P1), targeting MCL-1 ADCs and non-targeting isotype ADCs were prepared at 10X in standard growth media. The prepared drug treatments

were then added to the cells resulting in final concentrations of 0.0005 - 300 nM and a final volume of 50 μL per well. Each drug concentration was tested in quadruplets. Three conditions were evaluated for each compound. The compounds were evaluated as single agents and in combination with Venetoclax and in combination with Compound A1. The Venetoclax and Compound A1 were dosed at either 5 nM or 50 nM final concentration. Plates were incubated at 5% $CO_2$, 37°C for 5 days in a tissue culture incubator, after which cell viability was assessed through the addition of 25 μL of CellTiter Glo® (Promega, cat# G7573), a reagent which lyses cells and measures total adenosine triphosphate (ATP) content. Plates were incubated at room temperature for 10 minutes to stabilize luminescent signals prior to reading using a luminescence reader (EnVision Multilabel Plate Reader, PerkinElmer). To evaluate the effect of the drug treatments, luminescent counts from wells containing untreated cells (100% viability) were used to normalize treated samples. A variable slope model was applied to fit a nonlinear regression curve to the data in GraphPad PRISM version 7.02 software. IC50 and Amax values were extrapolated from the resultant curves.

*Result*

The dose response curves of representative cancer cell lines expressing the CD74 targets of interest are shown in **FIG. 4** and **FIG. 5.** The concentrations of treatment required to inhibit 50% of cell growth or survival (IC50) were calculated with representative IC50 values of the cell lines tested summarized in Table 21.

**Table 21: CD74-MCL-1 ADCs in Combination with Venetoclax and Compound A1**

| | NOMO-1 | | MONO-MAC-1 | | EOL-1 | |
|---|---|---|---|---|---|---|
| | IC50 (nM) | Amax (Span) | IC50 (nM) | Amax (Span) | IC50 (nM) | Amax (Span) |
| CD74-L5-P1 | 3.70 | 74.45 | 7.95 | 84.9 | 1.419 | 91.25 |
| CD74-L5-P1 + 5 nM Venetoclax | 1.03 | 89.68 | 1.31 | 115.5 | 0.164 | 113.4 |
| CD74-L5-P1 + 50 nM Venetoclax | 0.278 | 92.24 | 0.409 | 88.73 | n/a | n/a |
| CD74-L5-P1 + 5 nM Compound A1 | 0.457 | 91.5 | 0.636 | 113.5 | 0.1364 | 99.58 |
| CD74-L5-P1 + 50 nM Compound A1 | 0.138 | 100.6 | 0.156 | 115.4 | n/a | n/a |
| | | | | | | |
| P1 | 1.45 | 94.01 | 0.800 | 94.52 | 1.069 | 94.92 |
| P1 + 5 nM Venetoclax | 1.59 | 70.4 | 0.183 | 110.2 | 0.365 | 87.42 |
| P1 + 50 nM Venetoclax | 0.567 | 69.95 | 0.122 | 84.64 | n/a | n/a |
| P1 + 5 nM Compound A1 | 0.640 | 60.65 | 0.100 | 121.5 | 0.2294 | 89.37 |
| P1 + 50 nM Compound A1 | 0.222 | 97.61 | 0.052 | 102.9 | n/a | n/a |
| | | | | | | |
| IgG1-L5-P1 | >50 | -- | 107.9 | 73.83 | >50 | -- |
| IgG1-L5-P1 + 5 nM Venetoclax | >50 | -- | >50 | 107.1 | >50 | -- |
| IgG1-L5-P1 + 50 nM Venetoclax | >50 | -- | 47.57 | 108.7 | n/a | n/a |
| IgG1-L5-P1 + 5 nM Compound A1 | 137.6 | 123.2 | 57.93 | 122.4 | 92.33 | 122.1 |
| IgG1-L5-P1 + 50 nM Compound A1 | >50 | -- | 30.8 | 114.8 | n/a | n/a |

**Example 10: In Vitro Assessment VI - In vitro activity of CD74 MCL-1 ADCs**

*Cell Lines*

The CD74 MCL-1 antibody drug conjugates were tested against three endogenous cancer cell lines. MONO-MAC-1 (DSMZ No. ACC-252 cultured in RPMI-1640 + 10% FBS + 1x non-essential amino acids + 1 mM sodium pyruvate), NOMO-1 (DSMZ No. ACC-542 cultured in RPMI-1640 + 10% FBS) and EOL-1 (DSMZ No. ACC-386 cultured in RPMI-1640 + 10% FBS).

*Inhibition of cell proliferation and survival*

The ability of the CD74 MCL-1 antibody drug conjugates to inhibit cell proliferation and survival was assessed using the Promega CellTiter-Glo® proliferation assay. Cell lines were cultured in media that is optimal for their growth at 5% $CO_2$, 37°C in a tissue culture incubator. Prior to seeding for the proliferation assay, the cells were split at least 2 days before the assay to ensure optimal growth density. On the day of seeding, suspension cells were harvested. Cell viability and cell density were determined using a cell counter (Vi-Cell XR Cell Viability Analyzer, Beckman Coulter). Cells with higher than 85% viability were seeded in white clear bottom 384-well TC treated plates (Corning cat. # 3765). Cells were seeded at a density of 1,000 cells per well in 45 μL of standard growth media. Plates were incubated at 5% $CO_2$, 37°C overnight in a tissue culture incubator. The next day targeting MCL-1 ADCs were prepared at 10X in standard growth media. The prepared drug treatments were then added to the cells resulting in final concentrations of 0.01 - 200 nM and a final volume of 50 μL per well. Each drug concentration was tested in quadruplets. Plates were incubated at 5% $CO_2$, 37°C for 5 days in a tissue culture incubator, after which cell viability was assessed through the addition of 25 μL of CellTiter Glo® (Promega, cat# G7573), a reagent which lyses cells and measures total adenosine triphosphate (ATP) content. Plates were incubated at room temperature for 10 minutes to stabilize luminescent signals prior to reading using a luminescence reader (EnVision Multilabel Plate Reader, PerkinElmer). To evaluate the effect of the drug treatments, luminescent counts from wells containing untreated cells (100% viability) were used to normalize treated samples. A variable slope model was applied to fit a nonlinear regression curve to the data in GraphPad PRISM version 7.02 software. IC50 and Amax values were extrapolated from the resultant curves and shown in Table **22.**

**Table 22: $IC_{50}$ values**

| Linker-Payload in ADC | Liberated Payload | MONO-MAC-1 IC50 (nM) | MONO-MAC-1 Amax | NOMO-1 IC50 (nM) | NOMO-1 Amax | EOL-1 IC50 (nM) | EOL-1 Amax |
|---|---|---|---|---|---|---|---|
| L5-P1 | P1 | 7.95 | 84.9 | 2.221 | 55.58 | 1.018 | 75.06 |
| L30-P1 | P1 | 5.451 | 86.78 | 1.617 | 78.95 | 0.2508 | 197.6 |
| L31-P1 | P1 | 3.959 | 110.3 | 1.195 | 73.4 | 0.466 | 83.98 |
| L32-P1 | P1 | 6.721 | 97.57 | 1.504 | 62.58 | 0.1604 | 125 |
| L33-P1 | P1 | 6.716 | 89.18 | 2.584 | 58.95 | 0.6202 | 66.96 |
| L34-P1 | P1 | 6.275 | 104.4 | 2.961 | 66.95 | 0.736 | 70.92 |
| L35-P1 | P1 | ** | ** | 6.904 | 62.73 | 0.8938 | 75.56 |
| L36-P1 | P1 | 0.519 | 66.3 | 4.578 | 63.34 | 0.6856 | 100.8 |
| L37-P1 | P1 | ** | ** | 5.141 | 83.05 | 0.0425 | 243 |
| L60-P1 | P1 | ** | ** | 2.625 | 76.94 | 0.5348 | 79.04 |
| L61-P1 | P1 | ** | ** | 0.5457 | 45.63 | 0.03283 | 63.76 |
| L62-P1 | P1 | ** | ** | 3.411 | 65.18 | 0.7708 | 83.03 |
| L63-P1 | P1 | ** | ** | 0.7138 | 61.25 | ** | ** |
| L67-P1 | P1 | ** | ** | 2.493 | 79.39 | 0.2614 | 119.3 |
| L68-P1 | P1 | ** | ** | 6.912 | 91.25 | 0.788 | 127.3 |
| L69-P1 | P1 | ** | ** | 14.75 | 95.03 | 0.7043 | 159.6 |
| L71-P1 | P1 | ** | ** | 9.825 | 53.62 | 0.3409 | 93.09 |
| L72-P1 | P1 | ** | ** | ** | 57.68 | 0.3857 | 109.8 |
| L77-P1 | P1 | 0.9865 | 108.2 | 3.185 | 48.53 | 0.3832 | 102.6 |
| L78-P1 | P1 | ** | ** | 3.735 | 53.4 | 0.4304 | 87.22 |
| L79-P1 | P1 | ** | ** | 3.136 | 56.07 | 0.4916 | 91.3 |
| L86-P1 | P1 | ** | ** | 7.475 | 74.52 | 0.7212 | 105.5 |
| **** tested but curve did not converge** | | | | | | | |

**Example 11. In Vivo Assessment I - *In vivo* activity of CD74 ADCs in Nomo1 acute myeloid leukemia model after intraveneous (IV) administration**

The *in vivo* therapeutic effect of a CD74-targeting ADC formulated in Phosphate-Buffered Saline (PBS) is determined in Nomo1 acute myeloid leukemia model after intravenous (IV) administration. The follow compounds were tested: anti-CD74_CysmAb Fc silent_L5-P1, anti-CD74_CysmAb Fc silent and IgG1-Linker-Payload Fc silent.

*Materials and methods*

Nomo1 cells, obtained from DSMZ, were cultured in RPMI supplemented with 10% FBS. 0.1ml containing $5x10^6$ cells were subcutaneously inoculated into the right flank of female SCID mice, provided by Charles River. When tumors reached the appropriate volume, mice were randomized, 6 animals per group, using Easy stat software. IgG1-Linker-Payload Fc silent, anti-CD74_CysmAb Fc silent and anti-CD74_CysmAb Fc silent_L5-P1 (30 mg/kg) were injected once IV in PBS. Venetoclax (synthetized at the Servier Research Institute - IdRS) was formulated in PEG/EtOH/Phosal (*Polyethylene glycol*/ethanol/phosal, 30/10/60) and administered per os (PO) daily on weekdays (50mpk, 4ON/3OFF/4ON). Mice body weight was monitored three times a week and tumor size measured using electronic calipers. Tumor volume was estimated by measuring the minimum and maximum tumor diameters using the formula: (minimum diameter)$^2$(maximum diameter)/2. The last day with at least half of animals of all groups still present in the study (d9), tumor growth inhibition was calculated using the formula:

$$\left(1-\frac{\text{Median (DTV at Dx in treated group)}}{\text{Median (DTV at Dx in Control group)}}\right)\times 100$$

With DTV (Delta Tumor Volume) at Dx, calculated being TV at Dx - TV at Randomization. Mice were sacrificed at the first measurement for which tumor volume exceeded 2000 $mm^3$ or at the first signs of animal health deterioration. All experiments were conducted in accordance with the French regulations in force in 2018 after approval by IdRS Ethical Committee. SCID mice were maintained according to institutional guidelines.

*Results*

The efficacy of anti-CD74_CysmAb Fc silent_L5-P1 on Nomo1 xenografts is illustrated in **FIG. 3.** Treatment was started 10 days post tumor cells inoculation (median size: 191.6 $mm^3$). IgG1-Linker-Payload Fc silent (non-targeting ADC FS), anti-CD74_CysmAb Fc silent (naked antibody FS) and anti-CD74_CysmAb Fc silent_L5-P1 (CD74-targeting ADC FS) were administered once IV at 30mg/kg. The non-targeting ADC FS and the CD74-targeting ADC FS were also combined to venetoclax, which was dosed PO 4ON/3OFF/4ON at 50mg/kg. On day 9, no or very slight Tumor Growth Inhibition (%TGI) was induced by the naked antibody FS, the CD74-targeting ADC FS or the correspondent non-targeting ADC FS at 30mg/kg (TGI= 32.7%, -5.6% and -12.2 respectively), as depicted in FIG. 6 and Table 23. However, in combination with venetoclax, the anti-tumor activity induced by the CD74-targeting ADC FS was much greater than the correspondent non-targeting ADC FS (TGI= 98.6% *vs* 5.5% respectively, $p<0.001$). Of note, venetoclax alone had barely any effect on tumor growth (TGI= 26.2%). No clinically relevant body weight loss or other clinical signs due to the treatment were observed **(FIG. 7).**

**Table 23: Nomo1 tumor growth inhibition**

| Treatment | Dose/Schedule | %TGI (d9) |
|---|---|---|
| IgG1-Linker-Payload Fc silent | 30MK QD, IV | -12.2 |
| anti-CD74_CysmAb Fc silent | 30MK QD, IV | 32.7 |
| anti-CD74_CysmAb Fc silent_L5-P1 | 30MKQD, IV | -5.6 |
| Venetoclax | 50MK 4ON/3OFF/4ON, PO | 26.2 |
| IgG1-Linker-Payload Fc silent+ venetoclax | 30MK QD, IV+ 50MK 4ON/3OFF/4ON, PO | 5.5 |
| anti-CD74_CysmAb Fc silent_L5-P1+ venetoclax | 30MK QD, IV+ 50MK 4ON/3OFF/4ON, PO | 98.6*** |
| *** p <= 0.001 compared to control group. | | |

**Example 12. In Vivo Assessment II - *In vivo* activity of CD74 ADCs in Karpas422 diffuse large B-cell lymphoma model after intraveneous (IV) administration**

The *in vivo* therapeutic effect of a CD74-targeting ADC formulated in Phosphate-Buffered Saline (PBS) was determined in Karpas422 diffuse large B-cell lymphoma model after intravenous (IV) administration. The following compounds were tested: CD74-targeting ADC is anti-CD74_CysmAb Fc silent_L5-P1, IgG1-Linker-Payload Fc silent and anti-CD74_CysmAb Fc silent.

*Materials and methods*

Karpas422 cells, obtained from ATCC, were cultured in RPMI supplemented with 20% FBS. Cells were resuspended in 100% Matrigel (BD Biosciences) and 0.1ml containing $11x10^6$ cells were subcutaneously inoculated into the right flank of female NSG mice, provided by Charles River. When tumors reached the appropriate volume, mice were randomized, 6 animals per group, using Easy stat software. IgG1-Linker-Payload Fc silent, anti-CD74_CysmAb Fc silent and anti-CD74_CysmAb Fc silent_L5-P1 (30 mg/kg) were injected once IV in PBS. Venetoclax (synthetized at the Servier Research Institute - IdRS) was formulated in PEG/EtOH/Phosal (*Polyethylene glycol*/ethanol/phosal, 30/10/60) and administered per os (PO) daily on weekdays (50mpk, 4ON/3OFF/4ON). Mice body weight was monitored three times a week and tumor size measured using electronic calipers. Tumor volume was estimated by measuring the minimum and maximum tumor diameters using the formula: (minimum diameter)$^2$(maximum diameter)/2. The last day with at least half of control animals still present in the study (d23), tumor growth inhibition was calculated using the formula:

$$\left(1-\frac{\text{Median (DTV at Dx in treated group)}}{\text{Median (DTV at Dx in Control group)}}\right)\times 100$$

With DTV (Delta Tumor Volume) at Dx, calculated being TV at Dx - TV at Randomization. Response was evaluated as it follows: CR (Complete Response) if tumor size was ≤25mm$^3$ for at least three measurements, PR (Partial Response) if tumor size was comprised between 25mm$^3$ and 168.3 mm$^3$ (half of the starting size) for at least three measurements. Mice were sacrificed at the first measurement for which tumor volume exceeded 2000 mm$^3$ or at the first signs of animal health deterioration. All experiments were conducted in accordance with the French regulations in force in 2018 after approval by IdRS Ethical Committee. SCID mice were maintained according to institutional guidelines.

*Results*

The efficacy of anti-CD74_CysmAb Fc silent_L5-P1 on Karpas422 xenografts is illustrated in **FIG. 8.** Treatment was started 17 days post tumor cells inoculation (median size: 336.6 mm3). IgG1-Linker-Payload Fc silent (non-targeting ADC FS), anti-CD74_CysmAb Fc silent (naked antibody FS) and anti-CD74_CysmAb Fc silent_L5-P1 (CD74-targeting ADC FS) were administered once IV at 30mg/kg. The non-targeting ADC FS and the CD74-targeting ADC FS were also combined to venetoclax, which was dosed PO 4ON/3OFF/4ON at 50mg/kg.

On day 23, no Tumor Growth Inhibition (%TGI) was induced by the naked antibody FS, the CD74-targeting ADC FS or the correspondent non-targeting ADC FS at 30mg/kg (TGI= -1%, -61.8% and -76.2% respectively), as depicted in **FIG. 8** and Table 24. However, in combination with venetoclax, the anti-tumor activity induced by the CD74-targeting ADC FS was much greater than the correspondent non-targeting ADC FS (TGI= 154.9% vs -9% respectively, p<0.001), and complete regression (CR) was exclusively achieved in this treatment group (Table 24). Of note, venetoclax alone had barely any effect on tumor growth (TGI= 31.1%). No clinical signs due to the treatment were observed, apart from a moderate body weight loss (still not clinically relevant) in the groups dosed with venetoclax **(FIG. 9).**

**Table 24::** Karpas422 tumor growth inhibition

| Treatment | Dose/Schedule | %TGI (d23) | %CR | %PR |
|---|---|---|---|---|
| IgG1-Linker-Payload Fc silent | 30MK QD, IV | -76.2 | 0 | 0 |
| anti-CD74_CysmAb Fc silent | 30MK QD, IV | -1 | 0 | 0 |
| anti-CD74_CysmAb Fc silent_L5-P1 | 30MK QD, IV | -61.8 | 0 | 0 |
| Venetoclax | 50MK 4ON/3OFF/4ON, PO | 31.1 | 0 | 0 |
| IgG1-Linker-Payload Fc silent+ venetoclax | 30MK QD, IV+ 50MK 4ON/3OFF/4ON, PO | -9 | 0 | 0 |

(continued)

| Treatment | Dose/Schedule | %TGI (d23) | %CR | %PR |
|---|---|---|---|---|
| anti-CD74_CysmAb Fc silent_L5-P1+ venetoclax | 30MK QD, IV+ 50MK 4ON/3OFF/4ON, PO | 154.9*** | 33 | 67 |
| *** p <= 0.001 compared to control group | | | | |

**Example 13. In Vivo Assessment III - *In vivo* activity of CD74 ADCs in Monomac1 acute myeloid leukemia model after intraveneous (IV) administration**

The *in vivo* therapeutic effect of a CD74-targeting ADC formulated in Phosphate-Buffered Saline (PBS) was determined in Monomac1 acute myeloid leukemia model after intravenous (IV) administration. The following compounds were tested: CD74-targeting ADC is anti-CD74_CysmAb Fc silent_L5-P1, IgG1-Linker-Payload Fc silent, and anti-CD74_CysmAb Fc silent.

*Materials and methods*

Monomac1 cells, obtained from DSMZ, were cultured in RPMI supplemented with 10% FBS. 0.1ml containing $5x10^6$ cells were subcutaneously inoculated into the right flank of female SCID mice, provided by Charles River. When tumors reached the appropriate volume, mice were randomized, 6 animals per group, using Easy stat software. IgG1-Linker-Payload Fc silent, anti-CD74_CysmAb Fc silent and anti-CD74_CysmAb Fc silent_L5-P1 (30 mg/kg) were injected once IV in PBS. Venetoclax (synthetized at the Servier Research Institute - IdRS) was formulated in PEG/EtOH/Phosal (*Polyethylene glycol*/ethanol/phosal, 30/10/60) and administered per os (PO) daily on weekdays (50mpk, 2ON/2OFF/5ON/2OFF/4ON). Mice body weight was monitored three times a week and tumor size measured using electronic calipers. Tumor volume was estimated by measuring the minimum and maximum tumor diameters using the formula: (minimum diameter)$^2$(maximum diameter)/2. The last day with at least half of control animals still present in the study (d16), tumor growth inhibition was calculated using the formula:

$$\left(1-\frac{\text{Median (DTV at Dx in treated group)}}{\text{Median (DTV at Dx in Control group)}}\right)\times 100$$

With DTV (Delta Tumor Volume) at Dx, calculated being TV at Dx - TV at Randomization. Response was evaluated as it follows: CR (Complete Response) if tumor size was $\leq 25mm^3$ for at least three measurements, PR (Partial Response) if tumor size was comprised between $25mm^3$ and 60.1 $mm^3$ (half of the starting size) for at least three measurements. Mice were sacrificed at the first measurement for which tumor volume exceeded 2000 $mm^3$ or at the first signs of animal health deterioration. All experiments were conducted in accordance with the French regulations in force in 2018 after approval by IdRS Ethical Committee. SCID mice were maintained according to institutional guidelines.

*Results*

The efficacy of anti-CD74_CysmAb Fc silent_L5-P1 on Monomac1 xenografts is illustrated in **FIG. 10.** Treatment was started 7 days post tumor cells inoculation (median size: 120.2 $mm^3$). IgG1-Linker-Payload Fc silent (non-targeting ADC FS), anti-CD74_CysmAb Fc silent (naked antibody FS) and anti-CD74_CysmAb Fc silent_L5-P1 (CD74-targeting ADC FS) were administered once IV at 30mg/kg. The non-targeting ADC FS and the CD74-targeting ADC FS were also combined to venetoclax, which was dosed PO 2ON/2OFF/5ON/2OFF/4ON at 50mg/kg. On day 16, the Tumor Growth Inhibition (%TGI) induced by the CD74-targeting ADC FS at 30mg/kg was greater than the naked antibody FS (TGI= 33.8% *vs* -23% respectively, $p<0.05$) and the correspondent non-targeting ADC FS (TGI= -14.6%), as depicted in **FIG. 10** and Table 25. When combined to venetoclax, the non-targeting ADC FS displayed some anti-tumor activity (TGI= 34.4%, $p<0.05$), but a complete and long-lasting tumor regression was observed only in combination with the CD74-targeting ADC FS (TGI= 108.5%, $p<0.001$). No clinically relevant body weight loss or other clinical signs due to the treatment were observed **(FIG. 11).**

**Table 25: Monomac1 tumor growth inhibition**

| Treatment | Dose/Schedule | %TGI (d16) | %CR | %PR |
|---|---|---|---|---|
| IgG1-Linker-Payload Fc silent | 30MKQD, IV | -14.6 | 0 | 0 |

(continued)

| Treatment | Dose/Schedule | %TGI (d16) | %CR | %PR |
|---|---|---|---|---|
| anti-CD74_CysmAb Fc silent | 30MKQD, IV | -23 | 0 | 0 |
| anti-CD74_CysmAb Fc silent_L5-P1 | 30MKQD, IV | 33.8* | 0 | 0 |
| Venetoclax | 50MK 2ON/2OFF/5ON/2OFF/4ON, PO | 4.5 | 0 | 0 |
| IgG1-Linker-Payload Fc silent+ venetoclax | 30MK QD, IV+50MK 2ON/2OFF/5ON/2OFF/4ON, PO | 34.4* | 0 | 0 |
| anti-CD74_CysmAb Fc silent L5-P1+ venetoclax | 30MK QD, IV+50MK 2ON/2OFF/5ON/2OFF/4ON, PO | 108.5*** | 100 | 0 |
| * p <= 0.05, ** p <= 0.01, *** p <= 0.001 compared to control group | | | | |

**Example 14: *In vivo* efficacy of CD74-L5-P1 Fc silent against the EOL1 human acute myeloid (eosinophilic) leukemia xenograft model in mice**

As the *in vitro* studies had shown target-dependent and potent inhibition of cell growth in the EOL1 cell line by combination of CD74-L5-P1 Fc silent with venetoclax, the antitumor activity of this combination treatment was evaluated *in vivo* in this AML model.

*Methods*

EOL1 luciferase transfected cells were cultured at 37 °C in an atmosphere of 5 % $CO_2$ in air in RPMI1640 (BioConcept Ltd. Amimed) supplemented with 10 % FCS (BioConcept Ltd. Amimed, # 2-01 F30-I), 2 mM L-glutamine (BioConcept Ltd. Amimed, #5-10K00-H) and 1mM sodium pyruvate (BioConcept Ltd. Amimed, #5-60F00-H), 10 mM HEPES (Gibco #11560496). To establish EOL1 xenografts cells were harvested and resuspended in HBSS (Gibco, #14175) before injecting 100 μL containing 5 x $10^6$ cells subcutaneously in the flanks of female SCID beige mice (Charles River, Germany). Tumor growth was monitored regularly post cell inoculation and animals were randomized into treatment groups (n = 6) with a mean tumor volume of about 130 $mm^3$. Control and various treatment groups were dosed as indicated in **FIG. 12.** CD74-L5-P1 Fc silent, isotype IgG1 ADC Fc silent and CD74 Fc silent antibody were administered intravenously (iv) once at the start of treatment at the doses indicated in **FIG. 12** either alone or in combination with venetoclax which was administered orally (po) daily (qd) at 50 mg/kg. Doses were adjusted to individual mouse body weights. The iv dose volume was 10 ml/kg and each ADC was dissolved in 0.9% (w/v) NaCl in water. Tumor volume data on day 10 post treatment were analyzed statistically using Kruskal-Wallis rank sum test (Indigo Software). When applicable, results are presented as mean ± SEM. As a measure of efficacy the %T/C value is calculated on day 10 according to:

$$(\Delta \text{tumor volume}^{\text{treated}}/\Delta \text{tumor volume}^{\text{control}})*100$$

Tumor regression was calculated according to:

$$-(\Delta \text{tumor volume}^{\text{treated}}/\text{tumor volume}^{\text{treated at start}})*100$$

Where Δtumor volumes represent the mean tumor volume on the evaluation day minus the mean tumor volume at the start of the experiment.

*Results: Efficacy and Tolerability*

The antitumor effects of CD74-L5-P1 Fc silent in combination with venetoclax along with various control groups are summarized in **FIG. 12** and Table 26. Limited efficacy was observed with venetoclax (50 mg/kg po qd) alone and the combination of CD74 Fc silent antibody plus venetoclax. A slight tumor growth delay (TGD) was observed with combination of isotype IgG1 ADC (30 mg/kg) plus venetoclax (50 mg/kg) and also with CD74-L5-P1 Fc silent alone (30 mg/kg) but neither were statistically different from the vehicle control group. In contrast complete regressions (CRs) were observed with CD74-L5-P1 Fc silent dosed at 10 or 30 mg/kg iv once in combination with venetoclax 50 mg/kg po

qdx21 (p<0.05 from vehicle group and isotype IgG1 ADC plus venetoclax group). All treatments were well tolerated based on body weight changes **(FIG. 13).**

**Table 26: Antitumor effects of CD74-L5-P1 Fc silent in combination with venetoclax**

| Test agent, dose schedule and route | Day | ΔTumor volume ($mm^3$) | T/C (%) | Regression (%) |
|---|---|---|---|---|
| Vehicle p.o. + vehicle i.v. | 10 | 1082 | 100 | |
| Vehicle po + CD74-L5-P1 Fc silent 30 mq/kq i.v. | 10 | 817 | 76 | |
| Venetoclax 50 mg/kg p.o.qd + vehicle for ADC | 10 | 977 | 90 | |
| Venetoclax 50 mg/kg p.o. qd + CD74-L5-P1 Fc silent 30 mg/kg i.v. | 10 | -128 | | -100*$ |
| Venetoclax 50 mg/kg p.o. qd + CD74-L5-P1 Fc silent 10 mg/kg i.v. | 10 | -127 | | -100*$ |
| Venetoclax 50 mg/kg p.o. qd + isotype-IgG1 ADC Fc silent 30 mg/kg i.v. | 10 | 963 | 88.99 | |
| Venetoclax 50 mg/kg p.o. qd ± CD74 Fc silent 30 mg/kg i.v. | 10 | 1028 | 95.03 | |
| Delta tumor volumes and T/C% values were calculated for day 10 and are presented as means. * Indicates statistical difference (p<0.05) compared to vehicle control and $ indicates statistical difference (<0.05) compared to isotype IgG1 ADC + venetoclax on day 10 (Kruskal-Wallis rank sum test; Indigo Software). | | | | |

**Example 15: *In vivo* efficacy of CD74-ADCs Fc silent with different linker payloads against the EOL1 human acute myeloid (eosinophilic) leukemia xenograft model in mice**

To optimize the linker payload, efficacy of 6 CD74-ADCs with different linker payloads (L5-P1, L30-P1, L31-P1, L32-P1, L33-P1 and L34-P1) was evaluated in the EOL1 model in vivo.

*Methods*

EOL1 luciferase transfected cells were cultured at 37 °C in an atmosphere of 5 % $CO_2$ in air in RPMI1640 (BioConcept Ltd. Amimed) supplemented with 10 % FCS (BioConcept Ltd. Amimed, # 2-01F30-I), 2 mM L-glutamine (BioConcept Ltd. Amimed, #5-10K00-H) and 1mM sodium pyruvate (BioConcept Ltd. Amimed, #5-60F00-H), 10 mM HEPES (Gibco #11560496). To establish EOL1 xenografts cells were harvested and resuspended in HBSS (Gibco, #14175) before injecting 100 µL containing 5 x 106 cells subcutaneously in the flanks of female SCID beige mice (Charles River, Germany). Tumor growth was monitored regularly post cell inoculation and animals were randomized into treatment groups (n = 6) with a mean tumor volume of about 130 $mm^3$. Control and various CD74-ADCs fc silent (L5-P1, L30-P1, L31-P1, L32-P1, L33-P1 and L34-P1) were dosed as indicated in **FIG. 14.** The ADCs were administered intravenously (iv) once at the start of treatment at 2.5 mg/kg or at 3.3 mg/kg in combination with venetoclax which was administered orally (po) daily (qd) at 50 mg/kg. Doses were adjusted to individual mouse body weights. The iv dose volume was 10 ml/kg and each ADC was dissolved in 0.9% (w/v) NaCl in water. Tumor volume data on day 10 post treatment were analyzed statistically using one way ANOVA post hoc Tukey's multiple comparisons test (Indigo Software). Results are presented as mean ± SEM. As a measure of efficacy the %T/C value is calculated on day 10 according to:

$$(\Delta \text{tumor volume}^{\text{treated}}/\Delta \text{tumor volume}^{\text{control}})*100$$

Tumor regression was calculated according to:

$$-(\Delta \text{tumor volume}^{\text{treated}}/\text{tumor volume}^{\text{treated at start}})*100$$

Where Δtumor volumes represent the mean tumor volume on the evaluation day minus the mean tumor volume at the start of the experiment.

*Results: Efficacy and Tolerability*

All ADCs, except CD74-L5-P1 Fc silent, significantly ($p<0.05$) reduced the growth of EOL1 tumors after administering a low dose of 2.5 mg/kg in combination with venetoclax compared with the vehicle control group on day 10 **(FIG. 14** and Table 27). CD74-L34-P1 Fc silent exhibited a dose-related response between 2.5 and 3.3 mg/kg. All treatment schedules were well tolerated based on body weight changes **(FIG. 15).**

**Table 27: Antitumor effects of CD74-ADCs in combination with venetoclax**

| Test agent, dose schedule and route | Day | ΔTumor volume ($mm^3$) | T/C (%) |
|---|---|---|---|
| Vehicle p.o. + vehicle i.v. | 10 | 1082 | 100 |
| Venetoclax 50 mg/kg p.o.qd + CD74-L5-P1 2.5 mg/kg i.v. | 10 | 702 | 70 |
| Venetoclax 50 mg/kg p.o. qd + CD74-L30-P1 2.5 mg/kg i.v. | 10 | 47 | 5*$ |
| Venetoclax 50 mg/kg p.o. qd + CD74-L31-P1 2.5 mg/kg i.v. | 10 | 144 | 14*$ |
| Venetoclax 50 mg/kg. p.o. qd CD74-L32-P1 2.5 mg/kg i.v. | 10 | 38 | 4*$ |
| Venetoclax 50 mg/kg p.o. qd + CD74-L33-P1 2.5 mg/kg i.v. | 10 | 228 | 23*$ |
| Venetoclax 50 mg/kg p.o. qd + CD74-L34-P1 2.5 mg/kg i.v. | 10 | 401 | 40* |
| Venetoclax 50 mg/kg p.o. qd + CD74-L34-P1 3.3 mg/kg i.v. | 10 | 170 | 17*$ |
| Delta tumor volumes and T/C% values were calculated for day 10 and are presented as means. * Indicates statistical difference ($p<0.05$) compared to vehicle control and $ indicates statistical difference (<0.05) compared to CD74-L5-P1 + venetoclax on day 10 (one way ANOVA post hoc Tukey's multiple comparisons test; Indigo Software). | | | |

**Example 16 - expression of humanized LL1 variants**

Expression vectors containing different humanized LL1 heavy chains and humanized LL1 light chains were cotransfected into Expi293 cells (Invitrogen, Carlsbad, CA) using expifectamine and cultured for 7 days. Culture supernatants were harvested by centrifugation, filtered, and antibodies purified by Protein A affinity chromatography. Antibody purity after affinity chromatography was tested by analytical size exclusion chromatography.

The first round of transfections (Table 28) focused on evaluating the different humanized heavy and light chains, along with PTM removal mutations (see **Example 17).**

**Table 28: Expression of humanized LL1 variants, first round**

| Heavy Chain | Light chain | Final yield (mg) | Purity (% monomer) |
|---|---|---|---|
| hLL1 | hLL1 | 2.58 | 99.73 |
| hLL1 | hzLL1-Vk1b | 2.57 | 99.75 |
| hLL1 | hzLL1-Vk1a | 2.28 | 99.79 |
| hLL1 | hLL1 (NQ) | 1.34 | 99.78 |
| hLL1 | hLL1 (QG) | 1.05 | 99.78 |
| hLL1 | hLL1 (TG) | 1.89 | 99.7 |
| hLL1 | hLL1 (SG) | 2.06 | 99.67 |
| hLL1 | hLL1 (NV) | 0.53 | 99.53 |
| hzLL1-VH2 | hzLL1-Vk1b | 2.98 | 99.74 |
| hzLL1-VH1b | hzLL1-Vk1b | 2.60 | 99.5 |
| hzLL1-VH1a | hzLL1-Vk1b | 2.60 | 99.62 |
| hzLL1-VH3 | hLL1 | 3.49 | 99.69 |
| hzLL1-VH2 | hLL1 | 2.10 | 99.81 |
| hzLL 1-VH3 | hzLL1-Vk1a | 2.71 | 99.66 |

(continued)

| Heavy Chain | Light chain | Final yield (mg) | Purity (% monomer) |
|---|---|---|---|
| hzLL1-VH1b | hLL1 | 2.96 | 99.62 |
| hzLL1-VH2 | hzLL1-Vk1a | 2.77 | 99.77 |
| hzLL1-VH1a | hLL1 | 2.80 | 99.67 |
| hzLL1-VH1b | hzLL1-Vk1a | 3.00 | 99.55 |
| hzLL1-VH3 | hzLL1-Vk1b | 3.07 | 99.62 |
| hzLL1-VH1a | hzLL1-Vk1a | 2.64 | 99.61 |

Overall expression and purity was comparable between most of the humanized variants and the original humanized version (hLL1). A reduction in overall yield was seen for most of the PTM removal variants compared to the other humanized variants.

The NQ mutation set was incorporated into the hzLL1-Vk1b and hzLL1-Vk1a light chains, and a second round of variants were expressed using transient transfection into Expi293 cells (Table 29):

**Table 29: Expression of humanized LL1 variants, second round**

| Heavy Chain | Light chain | Final yield (mg) | Purity (% monomer) |
|---|---|---|---|
| hLL1 | hzLL1-Vk1a (NQ) | 2.8 | 96.0 |
| hLL1 | hzLL1-Vk1b (NQ) | 0.5 | 98.0 |
| hzLL1-VH1a Y95F | hzLL1-Vk1a (NQ) | 2.75 | 98.0 |
| hzLL1-VH1a Y95F | hzLL1-Vk1b (NQ) | 0.8 | 99.0 |
| hzLL1-VH1a V5Q Y95F | hzLL1-Vk1a (NQ) | 1.8 | 98.0 |
| hzLL1-VH1a V5Q Y95F | hzLL1-Vk1b (NQ) | 0.8 | 99.0 |

Overall expression of these variants seemed to correlate with the selection of light chain - variants with the hzLL1-Vk1a (NQ) light chain expressed at a higher level than variants with the hzLL1-Vk1b (NQ) light chain.

Humanization variant antibodies were tested using FACS and SPR for binding to human CD74 protein. Variant antibodies with binding characteristics similar to milatuzumab were selected.

**Example 17: humanization and PTM removal from LL1 VH and VL sequences**

Variable domains for the murine anti-CD74 antibody LL1 were described in US7931903 in the context of chimeric antibody sequences (cLL1):

LL1 VH

QVQLQQSGPELKKPGETVKVTCKTSGYTFTNYGVNWIKQTPGEGLQWMGWINPN TGEPTFDDDFKGRFAFSLESSASTAFLQISNLKNEDMGTYFCSRSRGKNEAWFDYWGQGT LVTVSS (SEQ ID NO:49)

LL1 VL

DIQLTQTPLSLPVSLGDQASISCRSSQSLVHRNGNTYLHWYLQKPGQSPKLLIYTVS NRFSGVPDRFSGSGSGTDFTLKISRVEAEDLGLYFCSQSSHVPPTFGAGTKLEIK (SEQ ID NO:50)

These were disclosed at the same time as sequences for humanized VH and VL domains, hLL1 VH and hLL1 VL, respectively:

hLL1 VH

QVQLQQSGSELKKPGASVKVSCKASGYTFTNYGVNWIKQAPGQGLQWMGWINPNTGEPT
FDDDFKGRFAFSLDTSVSTAYLQISSLKADDTAVYFCSRSRGKNEAWFAYWGQGTLVTVS
S (SEQ ID NO:10)

hLL1 VL

DIQLTQSPLSLPVTLGQPASISCRSSQSLVHRNGNTYLHWFQQRPGQSPRLLIYTVSNRFS
GVPDRFSGSGSGTDFTLKISRVEAEDVGVYFCSQSSHVPPTFGAGTRLEIK (SEQ ID
NO:23)

Humanization of these sequences was described in example 5 of US7931903. This humanization process describes humanizing both variable domains using non-germline antibody sequences of humanized antibodies (RT-TS3, HF-21/28) as a template, which introduces a number of murine framework residues into the final antibody sequences. Also, a problematic site (Asn-Gly, or NG) for a common protein post-translational modification (deamidation) was left intact in the CDR L1 region of the humanized light chain. These properties are undesirable for an antibody therapeutic, as they could increase immunogenicity or decrease the binding affinity of the antibody upon storage.

Two separate efforts were undertaken to resolve these complications. First, both the heavy and light chains of LL1 were rehumanized using human germline frameworks as a template. Second, several mutations were deliberately introduced into the Asn-Gly sequence in the CDR L1 region of the LL1 light chain to disrupt the post-translational modification site. Humanized constructs with reasonable expression and binding to CD74 were combined with PTM removal mutations to produce final lead candidates.

*Humanization of LL1*

Initially, murine LL1 VH and VL sequences were compared against mouse germline frameworks. For the VH, the closest V-gene match (79/98 residues) appears to be murine IGHV9-1*02 (IMGT nomenclature), with mutations from germline scattered between CDR and framework regions. For the VL, the closest V-gene match (93/100 residues) appears to be murine IGKV1-100*01, with mutations divided between CDR regions and the N terminus.

Alignment of the murine LL1 VH region to human germlines revealed a reasonable match to three human germlines: IGHV7-4-1*02 (closest match), IGHV1-3*02, and IGHV5-51*02. Several murine residues in framework regions maintained in the prior heavy chain humanization (hLL1 VH) were also flagged as being potentially important to binding. These three human germlines and murine residues were used to prepare 6 variant heavy chains:

1. humanization to IGHV7-4-1*02 (hzLL1-VH1a)

QVQLVQSGSELKKPGASVKVSCKASGYTFTNYGVNWVRQAPGQGLEWMGWINP
NTGEPTFDDDFKGRFVFSLDSSVSTAYLQISSLKAEDTAVYYCSRSRGKNEAWFDYWGQG
TLVTVSS (SEQ ID NO:51)

2. Humanization to IGHV7-4-1*02 with retention of V37I mutation (hzLL1-VH1b)

QVQLVQSGSELKKPGASVKVSCKASGYTFTNYGVNWIRQAPGQGLEWMGWINPNTGEPT
FDDDFKGRFVFSLDSSVSTAYLQISSLKAEDTAVYYCSRSRGKNEAWFDYWGQGTLVTVS
S (SEQ ID NO:52)

3. Humanization to IGHV7-4-1*02 with retention of Y95F mutation (hzLL1-VH1a Y95F)

QVQLVQSGSELKKPGASVKVSCKASGYTFTNYGVNWVRQAPGQGLEWMGWINPNTGEPTFDDDFKGRFVFSLDSSVSTAYLQISSLKAEDTAVYFCSRSRGKNEAWFDYWGQGTLVTVSS (SEQ ID NO:53)

4. Humanization to IGHV7-4-1*02 with retention of V5Q Y95F mutations (hzLL1-VH1a V5Q Y95F)

QVQLQQSGSELKKPGASVKVSCKASGYTFTNYGVNWVRQAPGQGLEWMGWINPNTGEPTFDDDFKGRFVFSLDSSVSTAYLQISSLKAEDTAVYFCSRSRGKNEAWFDYWGQGTLVTVSS (SEQ ID NO:54)

5. Humanization to IGHV1-3*02 (hzLL1-VH2)

QVQLVQSGAEVKKPGASVKVSCKASGYTFTNYGVNWVRQAPGQRLEWMGWINPNTGEPTFDDDFKGRVTITLDSSASTAYMELSSLRSEDMAVYYCSRSRGKNEAWFDYWGQGTLVTVSS (SEQ ID NO:55)

6. Humanization to IGHV5-51*01 (hzLL1-VH3)

EVQLVQSGAEVKKPGESLKISCKGSGYTFTNYGVNWVRQMPGKGLEWMGWINPNTGEPTFDDDFKGQVTISLDSSISTAYLQWSSLKASDTAMYYCSRSRGKNEAWFDYWGQGTLVTVSS (SEQ ID NO:56)

For the light chain, alignment of the murine LL1 light chain to human germlines revealed reasonable matches with two human germlines - IGKV2-40*01 and IGKV2-30*02. These sequences were used to prepare two variant humanized light chains:

1. Humanization to IGKV2-40*01 (hzLL1-Vk1a)

DIVMTQTPLSLPVTPGEPASISCRSSQSLVHRNGNTYLHWYLQKPGQSPQLLIYTVSNRFSGVPDRFSGSGSGTDFTLKISRVEAEDVGVYFCSQSSHVPPTFGQGTKLEIK (SEQ ID NO:27)

2. Humanization to IGKV2-30*02 (hzLL1-Vk1b)

DVVMTQSPLSLPVTLGQPASISCRSSQSLVHRNGNTYLHWYQQRPGQSPRLLIYTVSNRFSGVPDRFSGSGSGTDFTLKISRVEAEDVGVYFCSQSSHVPPTFGQGTKLEIK (SEQ ID NO:31)

Humanized heavy chains and light chains were synthesized and used for recombinant protein expression (see **Example 16).**

*PTM Removal from LL1 light chain*

To remove the deamidation site (Asn-Gly, NG) from the CDR L1 region of the light chain, sequences containing the following 5 alternate residues (QG, SG, TG, NQ, NV) were constructed in the hLL1 VL sequence. This was used to make the following 5 variant light chains:

hLL1 VL (QG)

DIQLTQSPLSLPVTLGQPASISCRSSQSLVHRQGNTYLHWFQQRPGQSPRLLIYTVSNRFS GVPDRFSGSGSGTDFTLKISRVEAEDVGVYFCSQSSHVPPTFGAGTRLEIK (SEQ ID NO:57)

hLL1 VL (SG)

DIQLTQSPLSLPVTLGQPASISCRSSQSLVHRSGNTYLHWFQQRPGQSPRLLIYTVSNRFS GVPDRFSGSGSGTDFTLKISRVEAEDVGVYFCSQSSHVPPTFGAGTRLEIK (SEQ ID NO:58)

hLL1 VL (TG)

DIQLTQSPLSLPVTLGQPASISCRSSQSLVHRTGNTYLHWFQQRPGQSPRLLIYTVSNRFSG VPDRFSGSGSGTDFTLKISRVEAEDVGVYFCSQSSHVPPTFGAGTRLEIK (SEQ ID NO:59)

hLL1 VL (NQ)

DIQLTQSPLSLPVTLGQPASISCRSSQSLVHRNQNTYLHWFQQRPGQSPRLLIYTVSNRFS GVPDRFSGSGSGTDFTLKISRVEAEDVGVYFCSQSSHVPPTFGAGTRLEIK (SEQ ID NO:36)

hLL1 VL (NV)

DIQLTQSPLSLPVTLGQPASISCRSSQSLVHRNVNTYLHWFQQRPGQSPRLLIYTVSNRFSG VPDRFSGSGSGTDFTLKISRVEAEDVGVYFCSQSSHVPPTFGAGTRLEIK (SEQ ID NO:60)

Mutant humanized light chains were synthesized and used for recombinant protein expression.

**Example 18 Biacore $K_D$ Determination**

Binding to human and cyno CD74 for the WT IgG and humanized variant antibodies were assessed for affinity determinations using Biacore. Kinetic rate constants were performed via SPR using the Biacore 8K instrument (Cytiva, formerly GE Healthcare Lifesciences) as described below. The anti-IgG Fc capture method was utilized in order to determine kinetics for the antibodies. A commercially available anti-IgG Fc capture kit was used (Cytiva, formerly GE Healthcare Lifesciences). The provided IgG was immobilized on the chip surface using the provided amine coupling protocol from GE. This immobilized antibody captured the WT and humanized variants, and the soluble human and cyno CD74 flowed over as the analytes. The CD74 concentration started at 100 nM and was serially diluted at one part to one part of running buffer for seven concentrations. Regeneration was performed at the end of each concentration using the specified conditions in the kit.

Double reference subtraction was completed to generate the final data. The raw data was fitted to a 1:1 binding model, with parameter(s) Refractive Index set to local. Due to the nature of the antigen, the following table lists the apparent kinetic results for the WT and humanized IgG variants.

**Table 30 Kinetic Results**

| Sample | Antigen | ka (1/Ms) | kd (1/s) | KD (M) | Antigen | ka (1/Ms) | kd (1/s) | KD (M) |
|---|---|---|---|---|---|---|---|---|
| milatuzumab | Human CD74 | 1.0E+05 | 5.1E-04 | 4.9E-09 | Cyno CD74 | 5.6E+04 | 8.8E-04 | 1.6E-08 |
| Hcmil x LCmil_NQ | | 9.6E+04 | 3.3E-04 | 3.4E-09 | | 3.0E+04 | 7.7E-04 | 2.6E-08 |
| Mil_HC x hzVk1b | | 2.3E+05 | 1.7E-04 | 7.2E-10 | | 1.1E+05 | 5.8E-04 | 5.1E-09 |
| Mil HC x hzVk1a | | 2.1E+05 | 1.8E-04 | 8.5E-10 | | 1.0E+05 | 5.8E-04 | 5.7E-09 |

(continued)

| Sample | Antigen | ka (1/Ms) | kd (1/s) | KD (M) | Antigen | ka (1/Ms) | kd (1/s) | KD (M) |
|---|---|---|---|---|---|---|---|---|
| VHmil x VK1aNQ | | 2.0E+05 | 1.8E-04 | 9.1E-10 | | 1.0E+05 | 6.2E-04 | 5.9E-09 |
| VHmil x VK1bNQ | | 2.2E+05 | 1.6E-04 | 7.6E-10 | | 1.1E+05 | 5.8E-04 | 5.3E-09 |

## Claims

1. An antibody-drug conjugate of Formula (1):

$$Ab\text{-}(L\text{-}D)_p \qquad (1)$$

wherein:

Ab is an anti-CD74 antibody or an antigen-binding fragment thereof;
p is an integer from 1 to 16; and
-(L-D) is of the formula (C):

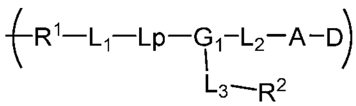

(C),

wherein:

R' is an attachment group;
$L_1$ is a bridging spacer;
$L_p$ is a peptide group comprising 1 to 6 amino acids;
D is an Mcl-1 inhibitor, wherein
(1) D comprises:

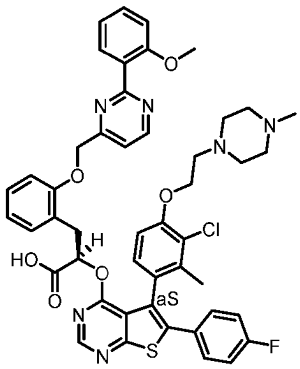

,

or an enantiomer, diastereoisomer, atropisomer, deuterated derivative, and/or pharmaceutically acceptable salt of any of the foregoing;
(2) D comprises:

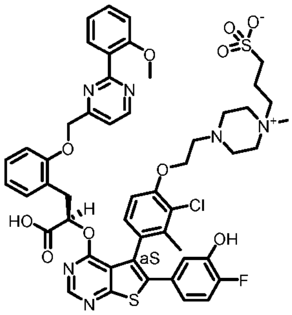

,

or an enantiomer, diastereoisomer, atropisomer, deuterated derivative, and/or pharmaceutically acceptable salt of any of the foregoing;

(3) D comprises:

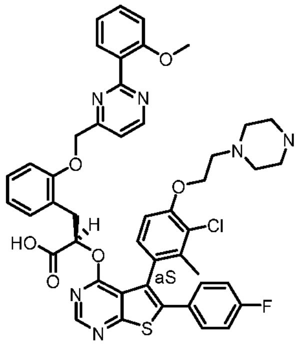

,

or an enantiomer, diastereoisomer, atropisomer, deuterated derivative, and/or pharmaceutically acceptable salt of any of the foregoing;

(4) D comprises:

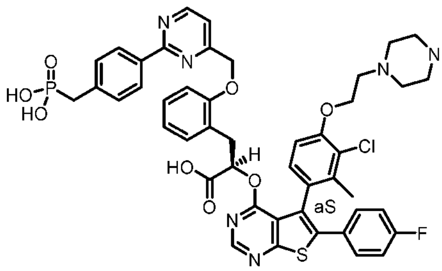

,

or an enantiomer, diastereoisomer, atropisomer, deuterated derivative, and/or pharmaceutically acceptable salt of any of the foregoing;

(5) D comprises:

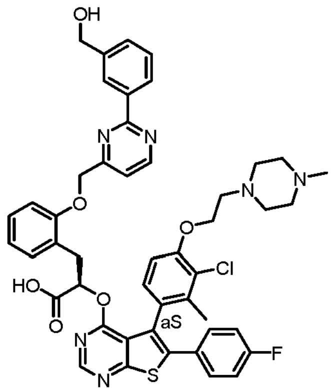

,

or an enantiomer, diastereoisomer, atropisomer, deuterated derivative, and/or pharmaceutically acceptable salt of any of the foregoing;

(6) D comprises:

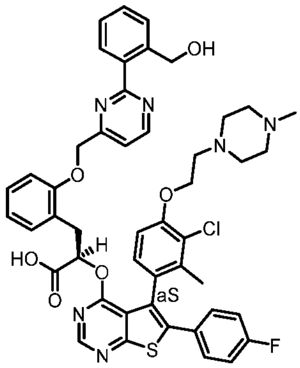

,

or an enantiomer, diastereoisomer, atropisomer, deuterated derivative, and/or pharmaceutically acceptable salt of any of the foregoing;

(7) D comprises:

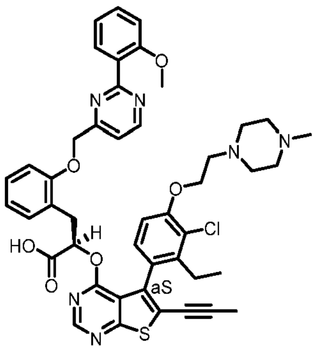

,

or an enantiomer, diastereoisomer, atropisomer, deuterated derivative, and/or pharmaceutically acceptable salt of any of the foregoing;

(8) D comprises:

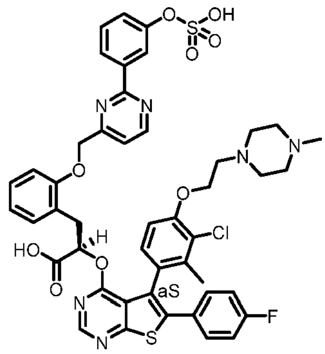

,

or an enantiomer, diastereoisomer, atropisomer, deuterated derivative, and/or pharmaceutically acceptable salt of any of the foregoing;

(9) D comprises:

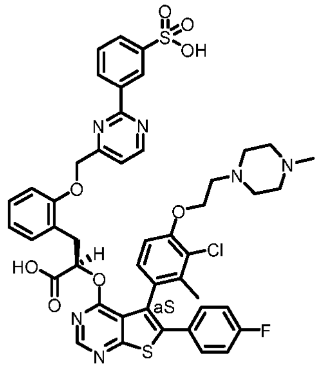

,

or an enantiomer, diastereoisomer, atropisomer, deuterated derivative, and/or pharmaceutically acceptable salt of any of the foregoing;

(10) D comprises:

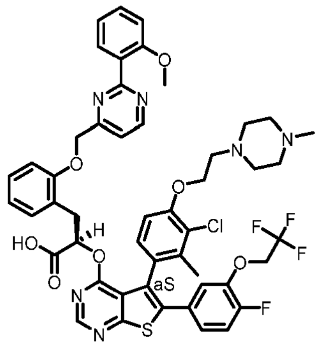

,

or an enantiomer, diastereoisomer, atropisomer, deuterated derivative, and/or pharmaceutically acceptable salt of any of the foregoing;

(11) D comprises:

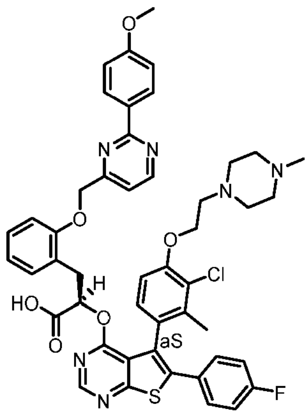

,

or an enantiomer, diastereoisomer, atropisomer, deuterated derivative, and/or pharmaceutically acceptable salt of any of the foregoing;

(12) D comprises:

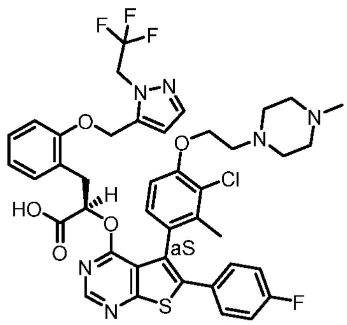

,

or an enantiomer, diastereoisomer, atropisomer, deuterated derivative, and/or pharmaceutically acceptable salt of any of the foregoing; or

(13) D comprises:

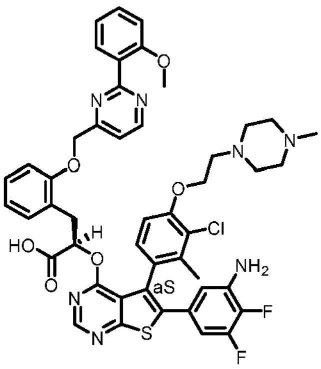

,

or an enantiomer, diastereoisomer, atropisomer, deuterated derivative, and/or pharmaceutically acceptable salt of any of the foregoing;

(14) D comprises:

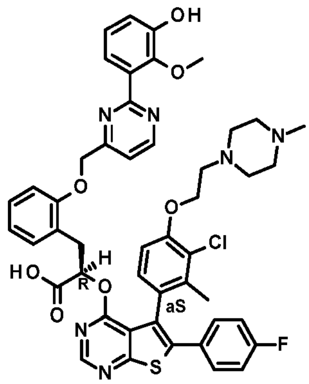

,

or an enantiomer, diastereoisomer, atropisomer, deuterated derivative, and/or pharmaceutically acceptable salt of any of the foregoing;

(15) D comprises:

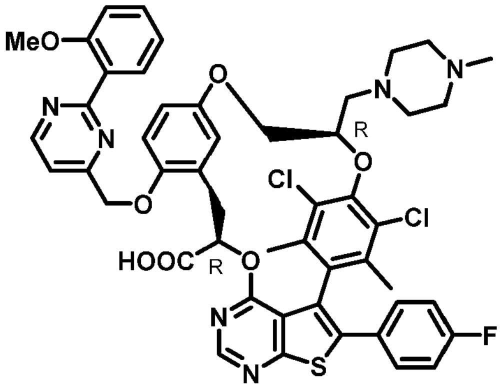

,

or an enantiomer, diastereoisomer, atropisomer, deuterated derivative, and/or pharmaceutically acceptable salt of any of the foregoing;

(16) D comprises:

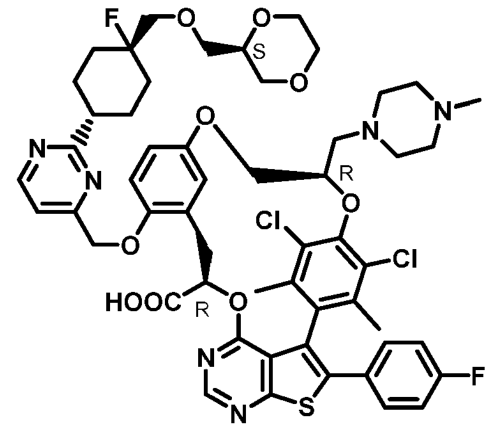

,

or an enantiomer, diastereoisomer, atropisomer, deuterated derivative, and/or pharmaceutically acceptable salt of any of the foregoing; or

(17) D comprises:

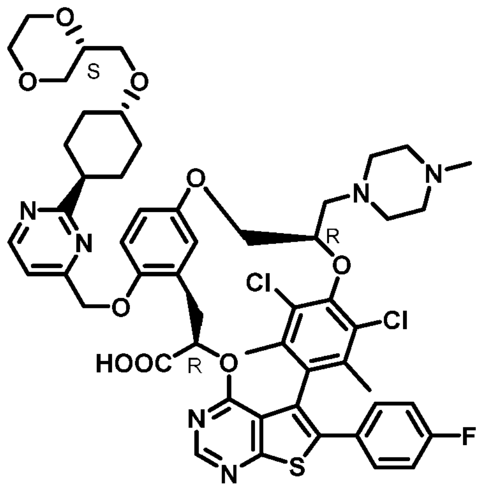

,

or an enantiomer, diastereoisomer, atropisomer, deuterated derivative, and/or pharmaceutically acceptable salt of any of the foregoing;

$G_1$-$L_2$-A is a self-immolative spacer;
$L_2$ is a bond, a methylene, a neopentylene or a $C_2$-$C_3$ alkenylene;
A is a bond, -OC(=O)-*,

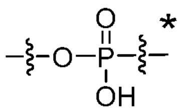

,

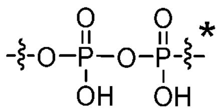

,

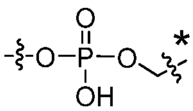

,

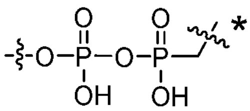

,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D;
$L_3$ is a spacer moiety; and
$R^2$ is a hydrophilic moiety.

**2.** The antibody-drug conjugate of claim 1, or pharmaceutically acceptable salt thereof, wherein -(L-D) is of Formula (D): wherein:

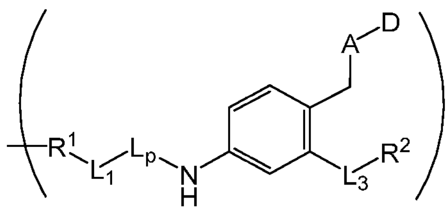

(D)

R' is an attachment group;
$L_1$ is a bridging spacer;
Lp is a peptide group comprising 1 to 6 amino acids;
A is a bond, -OC(=O)-*,

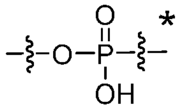, 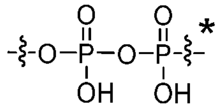, 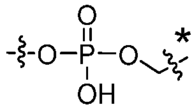,

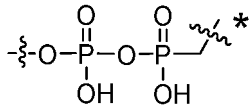,

$-OC(=O)N(CH_3)CH_2CH_2N(CH_3)C(=O)-*$ or $-OC(=O)N(CH_3)C(R^a)_2C(R^a)_2N(CH_3)C(=O)-*$, wherein each $R^a$ is independently selected from H, $C_1$-$C_6$ alkyl, and $C_3$-$C_8$ cycloalkyl and the * of A indicates the point of attachment to D;
$L_3$ is a spacer moiety; and
$R^2$ is a hydrophilic moiety.

**3.** The antibody-drug conjugate of claim 1 or 2, wherein

(i) $L_1$ comprises:

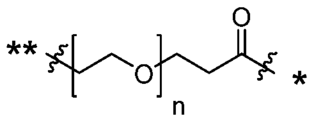, 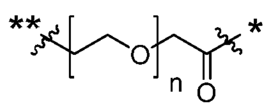,

or

*-CH(OH)CH(OH)CH(OH)CH(OH)-**,

wherein each n is an integer from 1 to 12, wherein the * of $L_1$ indicates the point of direct or indirect attachment to Lp, and the ** of $L_1$ indicates the point of direct or indirect attachment to $R^1$;
(ii) $L_1$ is

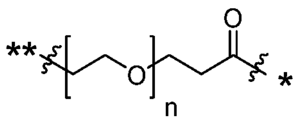,

and n is an integer from 1 to 12 or n is 1 or n is 12, wherein the * of $L_1$ indicates the point of direct or indirect attachment to Lp, and the ** of $L_1$ indicates the point of direct or indirect attachment to $R^1$;
(iii) $L_1$ is

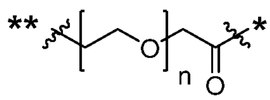,

and n is an integer from 1 to 12, wherein the * of $L_1$ indicates the point of direct or indirect attachment to Lp, and the ** of $L_1$ indicates the point of direct or indirect attachment to $R^1$;
(iv) $L_1$ comprises

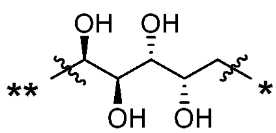,

wherein the * of $L_1$ indicates the point of direct or indirect attachment to Lp, and the ** of $L_1$ indicates the point of direct or indirect attachment to $R^1$; or

(v) $L_1$ is a bridging spacer comprising:

*-$C(=O)(CH_2)_mO(CH_2)_m$-**; *-$C(=O)((CH_2)_mO)_t(CH_2)_n$-**; *-$C(=O)(CH_2)_m$-**;

*-$C(=O)NH((CH_2)_mO)_t(CH_2)_n$-**;

*-$C(=O)O(CH_2)_mSSC(R^3)_2(CH_2)_mC(=O)NR^3(CH_2)_mNR^3C(=O)(CH_2)_m$-**;

*-$C(=O)O(CH_2)_mC(=O)NH(CH_2)_m$-**; *-$C(=O)(CH_2)_mNH(CH_2)_m$-**;

*-$C(=O)(CH_2)_mNH(CH_2)_nC(=O)$-**; *-$C(=O)(CH_2)_mX_1(CH_2)_m$-**;

*-$C(=O)((CH_2)_mO)_t(CH_2)_nX_1(CH_2)_n$-**; *-$C(=O)(CH_2)_mNHC(=O)(CH_2)_n$-**;

*-$C(=O)((CH_2)_mO)_t(CH_2)_nNHC(=O)(CH_2)_n$-**;

*-$C(=O)(CH_2)_mNHC(=O)(CH_2)_nX_1(CH_2)_n$-**;

*-$C(=O)((CH_2)_mO)_t(CH_2)_nNHC(=O)(CH_2)_nX_1(CH_2)_n$-**;

*-$C(=O)((CH_2)_mO)_t(CH_2)_nC(=O)NH(CH_2)_m$-**; *-$C(=O)(CH_2)_mC(R^3)_2$-**

or

*-$C(=O)(CH_2)_mC(=O)NH(CH_2)_m$-**,

wherein the * of $L_1$ indicates the point of direct or indirect attachment to Lp, and the ** of $L_1$ indicates the point of direct or indirect attachment to $R^1$;

$X_1$ is

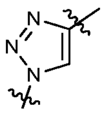 , 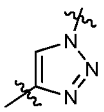 , 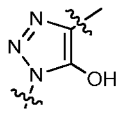 or 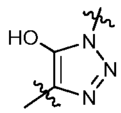 ;

and

each m is independently selected from 1, 2, 3, 4, 5, 6, 7, 8, 9 and 10;

each n is independently selected from 1, 2, 3, 4, 5, 6, 7, 8, 9 and 10; and

each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;

and each $R^3$ is independently selected from H and $C_1$-$C_6$alkyl.

**4.** The antibody-drug conjugate of any one of claims 1 to 3, wherein

(1) $R^2$ is a hydrophilic moiety comprising polyethylene glycol, polyalkylene glycol, a polyol, a polysarcosine, a sugar, an oligosaccharide, a polypeptide, or $C_2$-$C_6$ alkyl substituted with 1 to 3

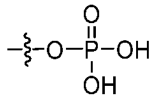 or 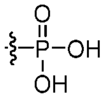

groups;

(2) $R^2$ is

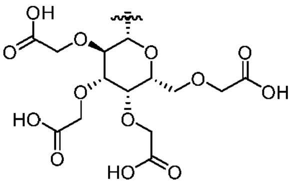, 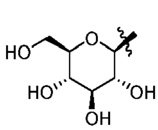, 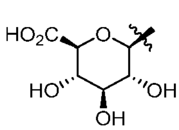, 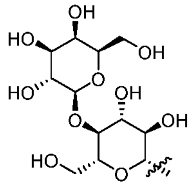, 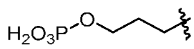,

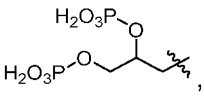, 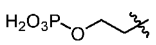, 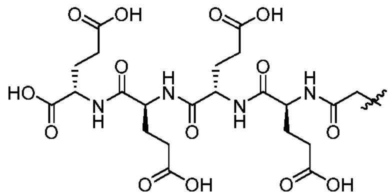,

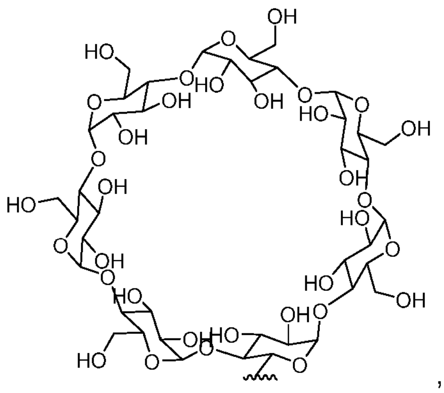, 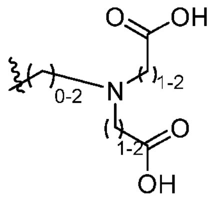, 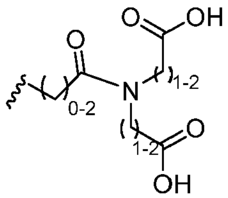,

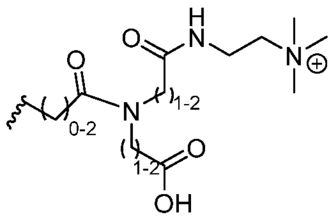, 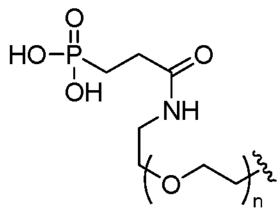

wherein n is an integer between 1 and 6,

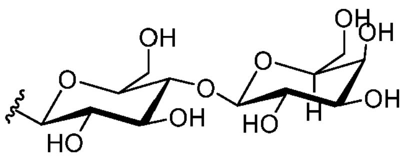, 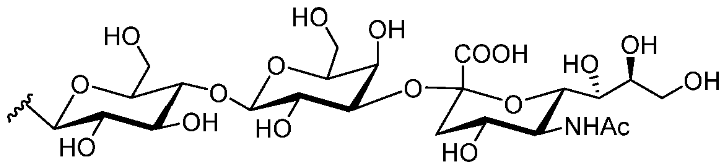,

or

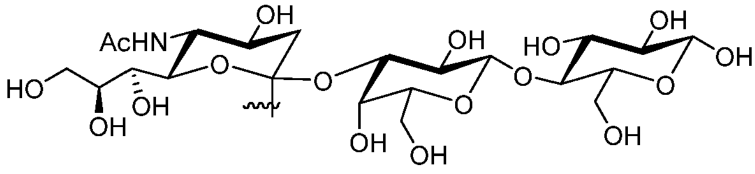;

(3) the hydrophilic moiety represented by $R^2$ comprises:

(i) a polysarcosine, e.g., with the following moiety:

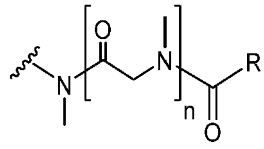,

wherein n is an integer between 3 and 25; and R is H, $-CH_3$ or $-CH_2CH_2C(=O)OH$; or
(ii) a polyethylene glycol of formula:

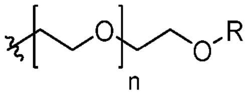 or 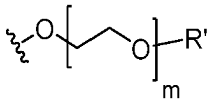,

wherein R is H, $-CH_3$, $CH_2CH_2NHC(=O)OR_a$, $-CH_2CH_2NHC(=O)R_a$, or $-CH_2CH_2C(=O)OR_a$, R' is OH, $-OCH_3$, $-CH_2CH_2NHC(=O)OR_a$, $-CH_2CH_2NHC(=O)R_a$, or $-OCH_2CH_2C(=O)OR_a$, in which $R_a$ is H or $C_{1-4}$ alkyl optionally substituted with either OH or $C_{1-4}$ alkoxyl, and each of m and n is independently an integer between 2 and 25; or

(4) the hydrophilic moiety represented by $R^2$ comprises

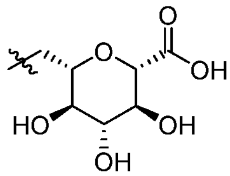.

**5.** The antibody-drug conjugate of any one of claims 1 to 4, wherein:

(i) $L_3$ is a spacer moiety having the structure

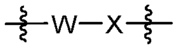,

wherein:

W is $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, $-NHC(=O)C(R^b)_2NHC(=O)-$, $-CH_2N(X-R^2)C(=O)O-$, $-C(=O)N(X-R^2)-$, $-CH_2N(X-R^2)C(=O)-$, $-C(=O)NR^b-$, $-C(=O)NH-$, $-CH_2NR^bC(=O)-$, $-CH_2NR^bC(=O)NH-$, $-CH_2NR^bC(=O)NR^b-$, $-NHC(=O)-$, $-NHC(=O)O-$, $-NHC(=O)NH-$, $-OC(=O)NH-$, $-S(O)_2NH-$, $-NHS(O)_2-$, $-C(=O)-$, $-C(=O)O-$ or $-NH-$, wherein each $R^b$ is independently selected from H, $C_1$-$C_6$alkyl, and $C_3$-$C_8$ cycloalkyl; and
X is a bond, triazolyl, or $-CH_2$-triazolyl-; or

(ii) $L_3$ is a spacer moiety having the structure

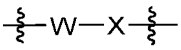,

wherein:

W is $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, $-NHC(=O)C(R^b)_2NHC(=O)-$, $-CH_2N(X-R^2)C(=O)O-$, $-C(=O)N(X-R^2)-$, $-CH_2N(X-R^2)C(=O)-$, $-C(=O)NR^b-$, $-C(=O)NH-$, $-CH_2NR^bC(=O)-$, $-CH_2NR^bC(=O)NH-$, $-CH_2NR^bC(=O)NR^b-$, $-NHC(=O)-$, $-NHC(=O)O-$, $-NHC(=O)NH-$, $-OC(=O)NH-$, $-S(O)_2NH-$, $-NHS(O)_2-$, $-C(=O)-$, $-C(=O)O-$ or $-NH-$, wherein each $R^b$ is independently selected from H, $C_1$-$C_6$alkyl, and $C_3$-$C_8$ cycloalkyl; and
X is $-CH_2$-triazolyl-$C_{1-4}$ alkylene-$OC(O)NHS(O)_2NH-$, $-C_{4-6}$ cycloalkylene-$OC(O)NHS(O)_2NH-$,

$-(CH_2CH_2O)_n-C(O)NHS(O)_2NH-$, $-(CH_2CH_2O)_n-C(O)NHS(O)_2NH-(CH_2CH_2O)_n-$, or $-CH_2$-triazolyl-$C_{1-4}$ alkylene-$OC(O)NHS(O)_2NH-(CH_2CH_2O)_n-$, wherein each n independently is 1 2, or 3.

**6.** The antibody-drug conjugate of any one of claims 1 to 5, wherein

(i) the attachment group is formed by a reaction comprising at least one reactive group;
(ii) the attachment group is formed by reacting:

a first reactive group that is attached to the linker, and
a second reactive group that is attached to the antibody or is an amino acid residue of the antibody;

(iii) the attachment group is formed by a reaction comprising at least one reactive group, wherein at least one of the reactive groups comprises:

a thiol,
a maleimide,
a haloacetamide,
an azide,
an alkyne,
a cyclooctene,
a triaryl phosphine,
an oxanorbornadiene,
a cyclooctyne,
a diaryl tetrazine,
a monoaryl tetrazine,
a norbornene,
an aldehyde,
a hydroxylamine,
a hydrazine,
$NH_2-NH-C(=O)-$,
a ketone,
a vinyl sulfone,
an aziridine,
an amino acid residue,

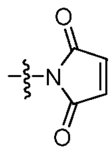 , .

$-ONH_2$, $-NH_2$,

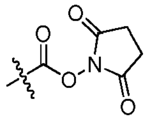 , 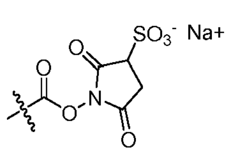 , 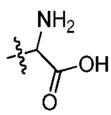 , 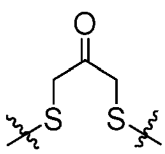 ,

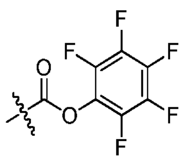 , 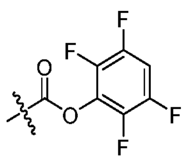 , 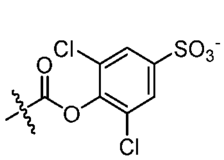 ,

$-N_3$,

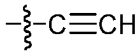,

-SH, $-SR^3$, $-SSR^4$, $-S(=O)_2(CH=CH_2)$, $-(CH_2)_2S(=O)_2(CH=CH_2)$, $-NHS(=O)_2(CH=CH_2)$, $-NHC(=O)CH_2Br$, $-NHC(=O)CH_2I$,

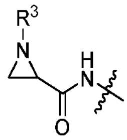,

$-C(O)NHNH_2$,

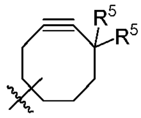,

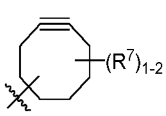, 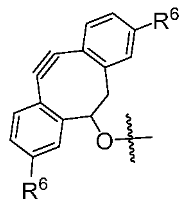, 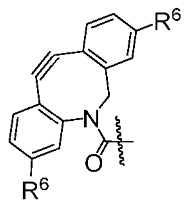, 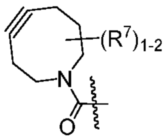, 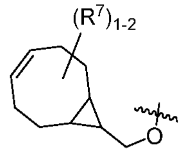,

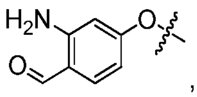, 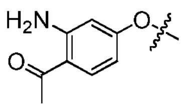, 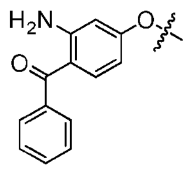, 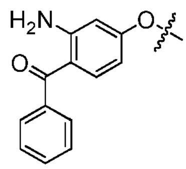,

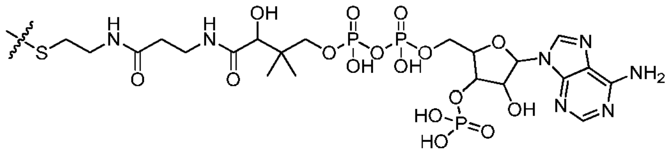,

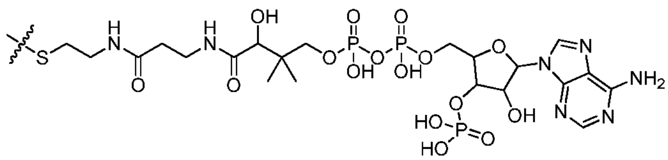,

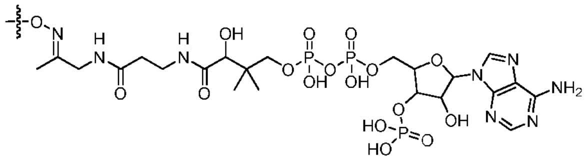,

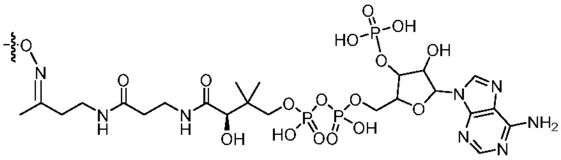,

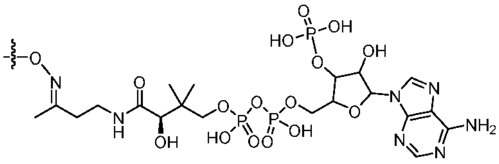, 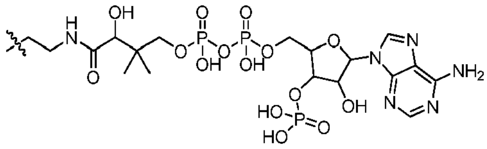,

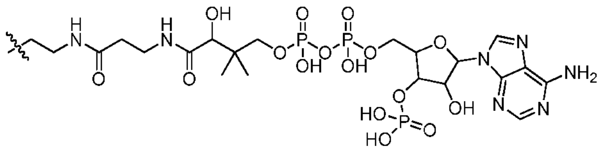,

or

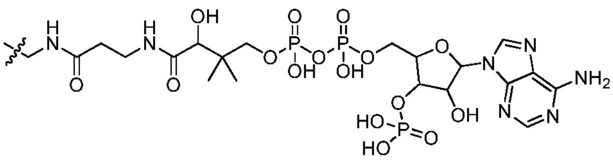;

wherein:

each $R^3$ is independently selected from H and $C_1$-$C_6$alkyl;
each $R^4$ is 2-pyridyl or 4-pyridyl;
each $R^5$ is independently selected from H, $C_1$-$C_6$alkyl, F, Cl, and -OH;
each $R^6$ is independently selected from H, $C_1$-$C_6$alkyl, F, Cl, -$NH_2$, -$OCH_3$, - $OCH_2CH_3$, -$N(CH_3)_2$, -CN, -$NO_2$ and -OH;
each $R^7$ is independently selected from H, $C_{1-6}$alkyl, fluoro, benzyloxy substituted with -C(=O)OH, benzyl substituted with -C(=O)OH, $C_{1-4}$alkoxy substituted with - C(=O)OH and $C_{1-4}$alkyl substituted with -C(=O)OH;

(iv) the attachment group is formed by reacting that is attached to the linker and a second reactive group that is attached to the antibody or is an amino acid residue of the antibody, wherein the first reactive group and second reactive group comprise:

a thiol and a maleimide,
a thiol and a haloacetamide,
a thiol and a vinyl sulfone,
a thiol and an aziridine,
an azide and an alkyne,
an azide and a cyclooctyne,
an azide and a cyclooctene,
an azide and a triaryl phosphine,
an azide and an oxanorbornadiene,
a diaryl tetrazine and a cyclooctene,
a monoaryl tetrazine and a norbornene,
an aldehyde and a hydroxylamine,
an aldehyde and a hydrazine,
an aldehyde and $NH_2$-NH-C(=O)-,
a ketone and a hydroxylamine,

a ketone and a hydrazine,
a ketone and $NH_2$-NH-C(=O)-,
a hydroxylamine and

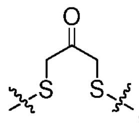,

an amine and

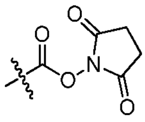, 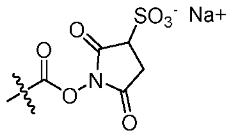, 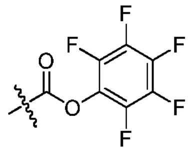, 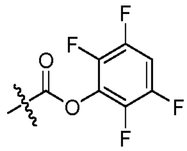,

or

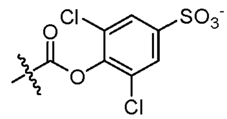,

or
a CoA or CoA analogue and a serine residue; or

(v) the attachment group comprises a group selected from:

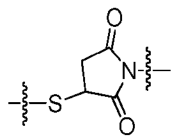;

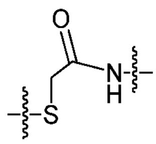;

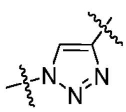 or 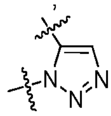;

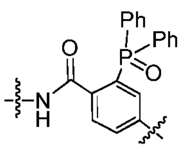;

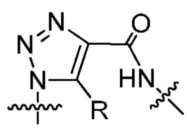;

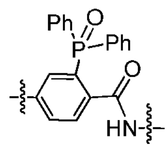;

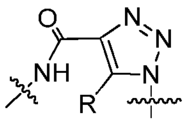;

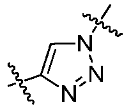 or 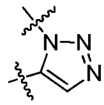;

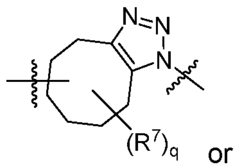 or 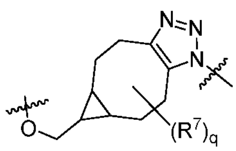 or 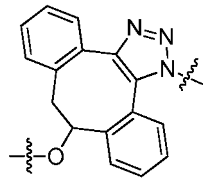;

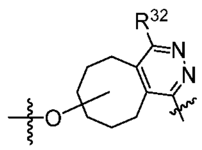 or 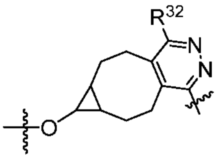;

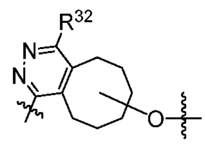 or 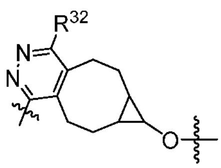;

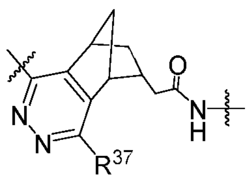;

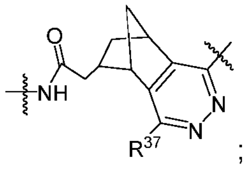;

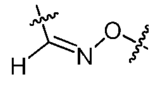;

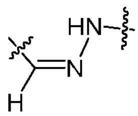
;

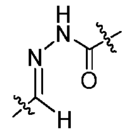
:

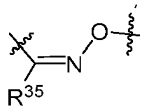
;

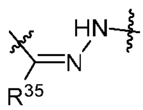
;

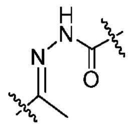
;

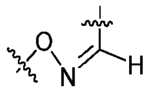
;

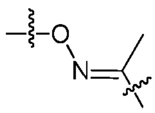
:

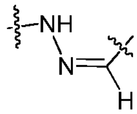
;

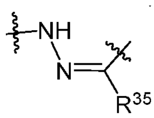
;

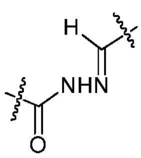
;

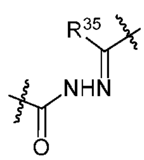
;

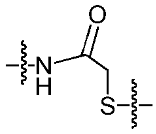;

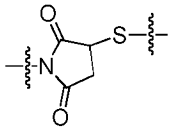;

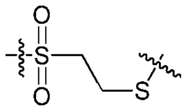:

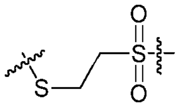;

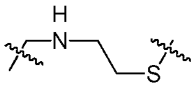 or 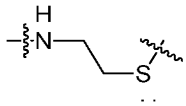;

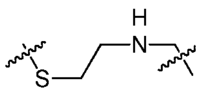 or 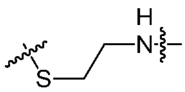;

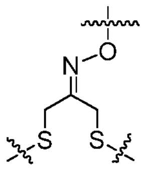;

amide;

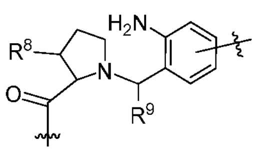;

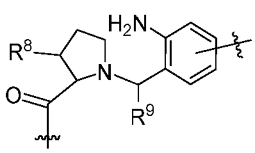;

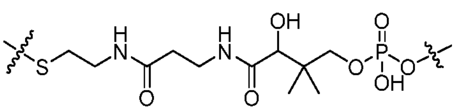:

,

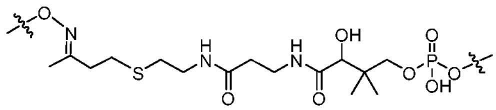

;

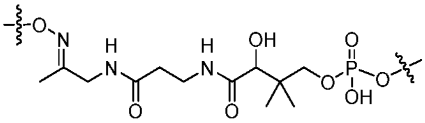

;

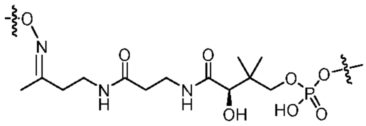

:

,

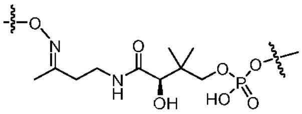

;

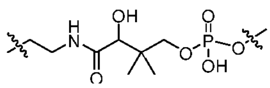

.

- ,

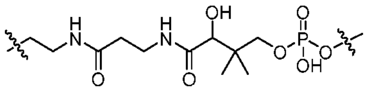

:

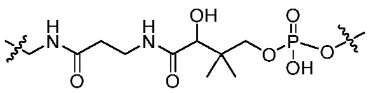

;

and
disulfide,
wherein:

$R^{32}$ is H, $C_{1-4}$ alkyl, phenyl, pyrimidine or pyridine;
$R^{35}$ is H, $C_{1-6}$ alkyl, phenyl or $C_{1-4}$ alkyl substituted with 1 to 3 -OH groups;
each $R^7$ is independently selected from H, $C_{1-6}$ alkyl, fluoro, benzyloxy substituted with - C(=O)OH, benzyl substituted with -C(=O)OH, $C_{1-4}$ alkoxy substituted with -C(=O)OH and $C_{1-4}$ alkyl substituted with -C(=O)OH;
$R^{37}$ is independently selected from H, phenyl and pyridine;
q is 0, 1, 2 or 3;
$R^8$ is H or methyl; and
$R^9$ is H, $-CH_3$ or phenyl.

**7.** The antibody-drug conjugate any one of claims 1 to 6, wherein

(i) the peptide group comprises 1 to 4 amino acid residues, 1 to 3 amino acid residues, or 1 to 2 amino acid residues;
(ii) the peptide group comprises amino acid residues selected from L-glycine (Gly), L-valine (Val), L-citrulline (Cit), L-cysteic acid (sulfo-Ala), L-lysine (Lys), L-isoleucine (Ile), L-phenylalanine (Phe), L-methionine (Met), L-asparagine (Asn), L-proline (Pro), L-alanine (Ala), L-leucine (Leu), L-tryptophan (Trp), and L-tyrosine (Tyr);
(iii) the peptide group comprises Val-Cit, Phe-Lys, Val-Ala, Val-Lys, Leu-Cit, sulfo-Ala-Val, and/or sulfo-Ala-Val-Ala; or
(iv) Lp is selected from:

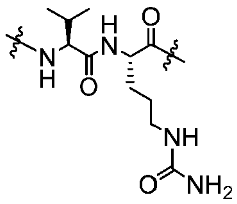, 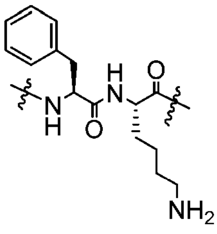, 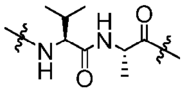, 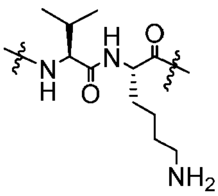, 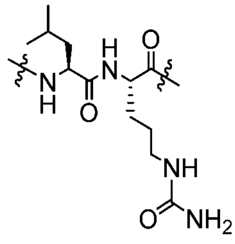,

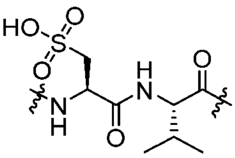, and 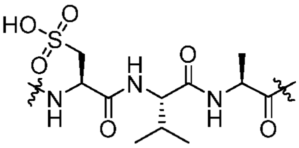.

**8.** An antibody-drug conjugate of formula (1):

Ab-(L-D)$_p$ (1)

wherein:

Ab is an anti-CD74 antibody or an antigen-binding fragment thereof;
p is an integer from 1 to 16; and

wherein -(L-D) comprises or is formed from a compound of formula:

(1)

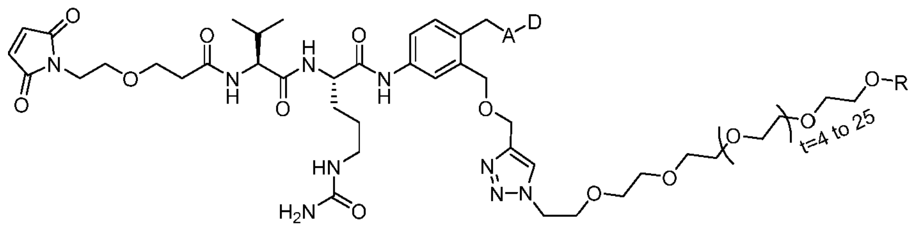

,

wherein:

R is H, $-CH_3$ or $-CH_2CH_2C(=O)OH$;
A is a bond, -OC(=O)-*,

*, 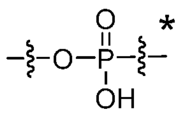, 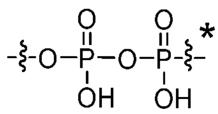, 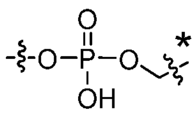,

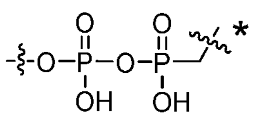,

$-OC(=O)N(CH_3)CH_2CH_2N(CH_3)C(=O)-$* or $-OC(=O)N(CH_3)C(R^a)_2C(R^a)_2N(CH_3)C(=O)-$*,
wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in claim 1;

(2)

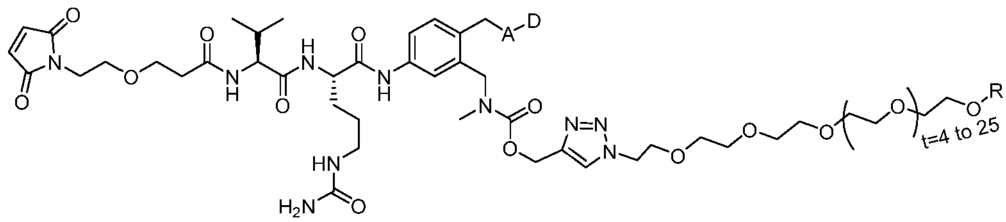

,

wherein:

R is H, $-CH_3$ or $-CH_2CH_2C(=O)OH$;
A is a bond, -OC(=O)-*

, 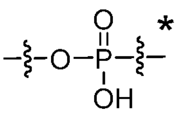 , 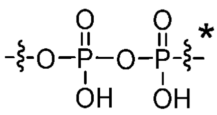 , 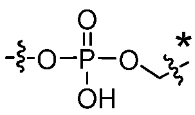 ,

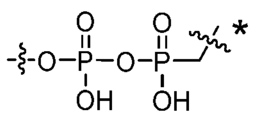 , .

$-OC(=O)N(CH_3)CH_2CH_2N(CH_3)C(=O)-*$ or $-OC(=O)N(CH_3)C(R^a)_2C(R^a)_2N(CH_3)C(=O)-*$,
wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in claim 1;

(3)

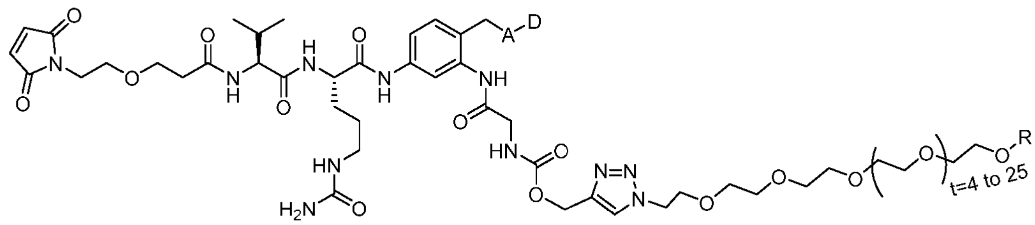

,

wherein:

R is H, $-CH_3$ or $-CH_2CH_2C(=O)OH$;
A is a bond, -OC(=O)-*,

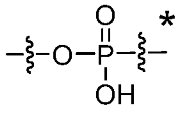 , 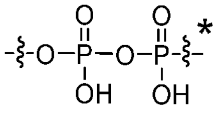 , 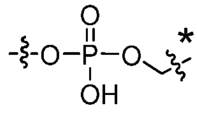 ,

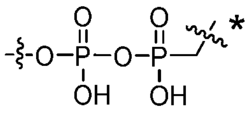 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1;

(4)

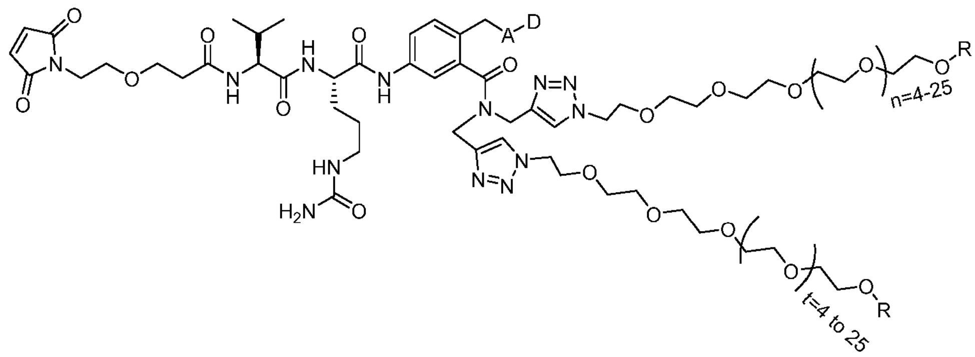

, wherein:

each R is independently selected from H, $-CH_3$, and $-CH_2CH_2C(=O)OH$;

A is a bond, -OC(=O)-*,

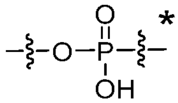 , 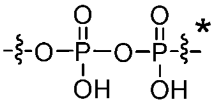 , 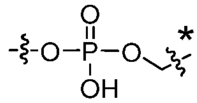 ,

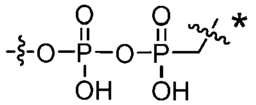 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$,

wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1;

(5)

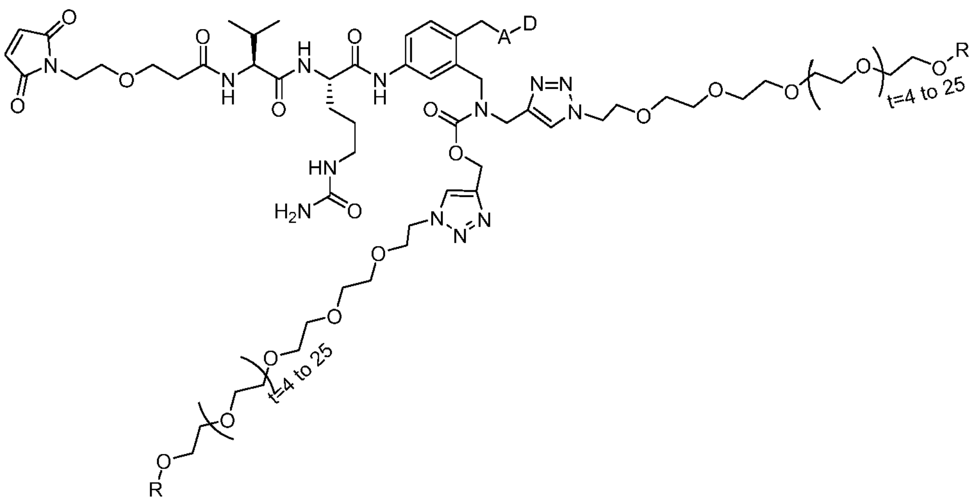

,

wherein:

each R is independently selected from H, $-CH_3$, and $-CH_2CH_2C(=O)OH$;
A is a bond, -OC(=O)-*,

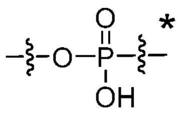 , 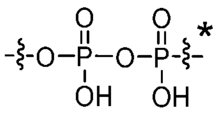 , 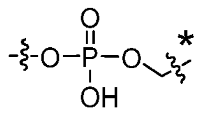 ,

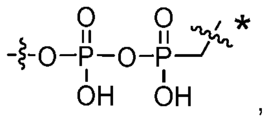 ,

$-OC(=O)N(CH_3)CH_2CH_2N(CH_3)C(=O)-$* or $-OC(=O)N(CH_3)C(R^a)_2C(R^a)_2N(CH_3)C(=O)-$*, wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in claim 1;

(6)

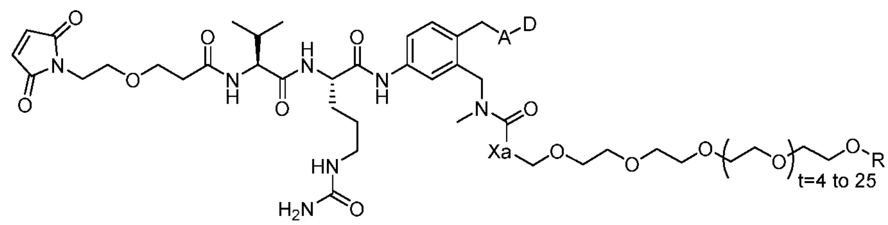 ,

wherein:

Xa is $-CH_2-$, $-OCH_2-$, $-NHCH_2-$ or $-NRCH_2-$ and each R independently is H, $-CH_3$ or - $CH_2CH_2C(=O)OH$;
A is a bond, -OC(=O)-*,

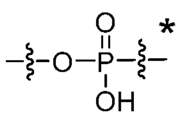 , 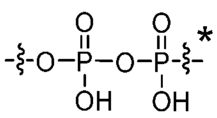 , 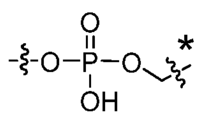 ,

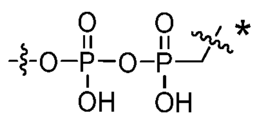

, $-OC(=O)N(CH_3)CH_2CH_2N(CH_3)C(=O)-$* or $-OC(=O)N(CH_3)C(R^a)_2C(R^a)_2N(CH_3)C(=O)-$*, wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in claim 1;

(7)

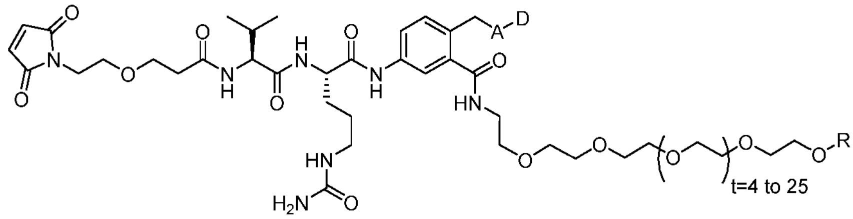

,

wherein:

R is H, $-CH_3$ or $-CH_2CH_2C(=O)OH$;
A is a bond, -OC(=O)-*,

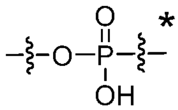 , 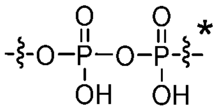 , 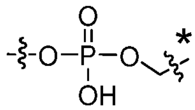 ,

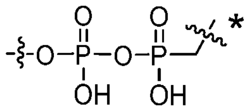 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$,
wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in claim 1;

(8)

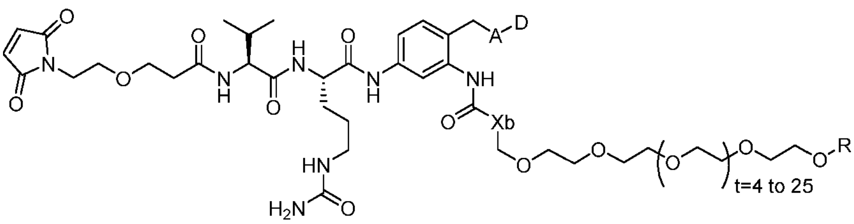

,

wherein:

Xb is $-CH_2-$, $-OCH_2-$, $-NHCH_2-$ or $-NRCH_2-$ and each R independently is H, $-CH_3$ or $-CH_2CH_2C(=O)OH$;
A is a bond, -OC(=O)-*,

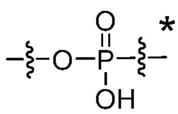 , 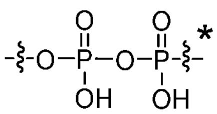 , 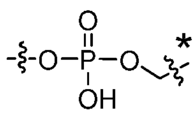 ,

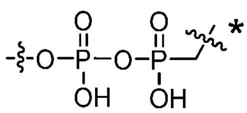 ,

$-OC(=O)N(CH_3)CH_2CH_2N(CH_3)C(=O)-*$ or $-OC(=O)N(CH_3)C(R^a)_2C(R^a)_2N(CH_3)C(=O)-*$,
wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1;

(9)

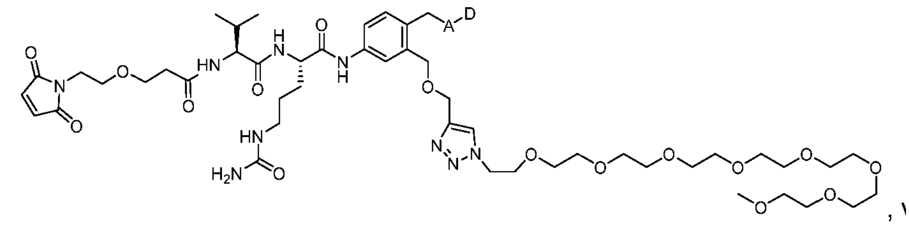

,

wherein:

A is a bond, -OC(=O)-*,

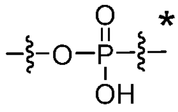 , 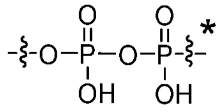 , 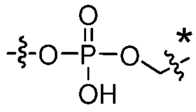 ,

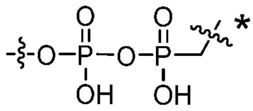 , -

OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and
D is an Mcl-1 inhibitor as defined in claim 1;

(10)

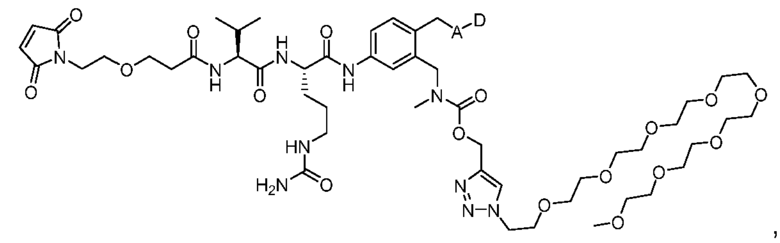

,

wherein:

A is a bond, -OC(=O)-*,

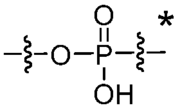 , 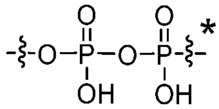 , 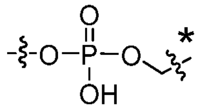 ,

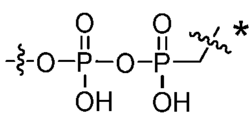 ,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1;

(11)

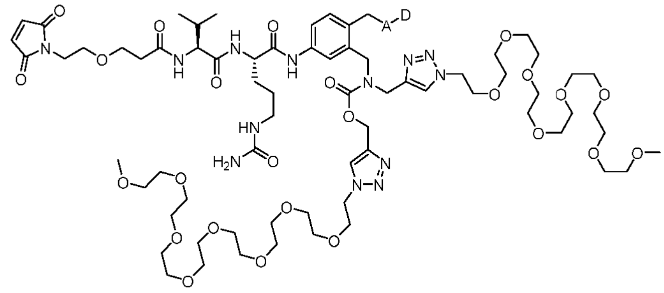

,

wherein:

A is a bond, -OC(=O)-*,

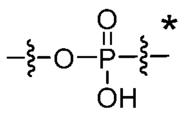

,

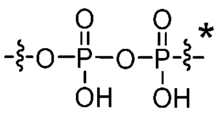

,

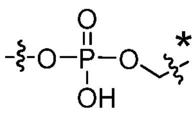

,

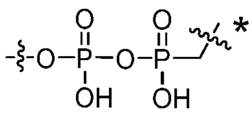

,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1;

(12)

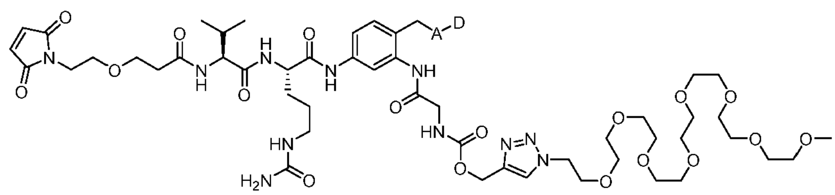

,

wherein:

A is a bond, -OC(=O)-*,

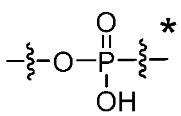

,

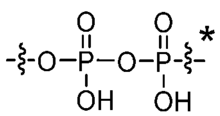

,

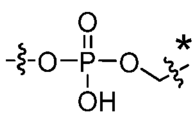

,

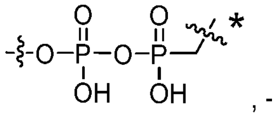

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1;

(13)

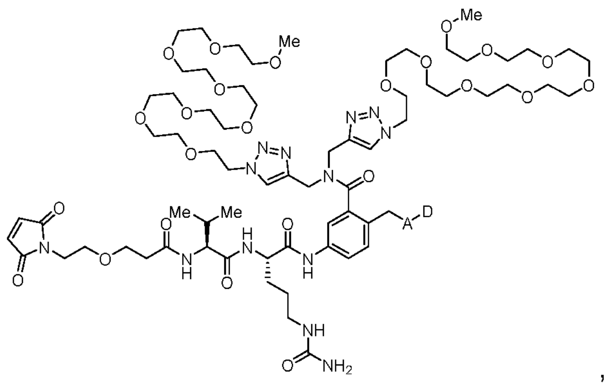

wherein:

A is a bond, -OC(=O)-*,

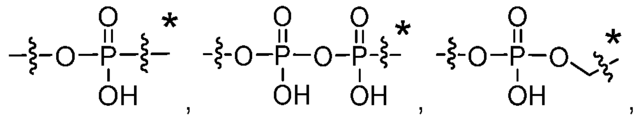

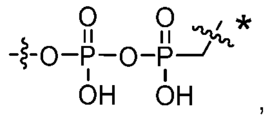

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1;

(14)

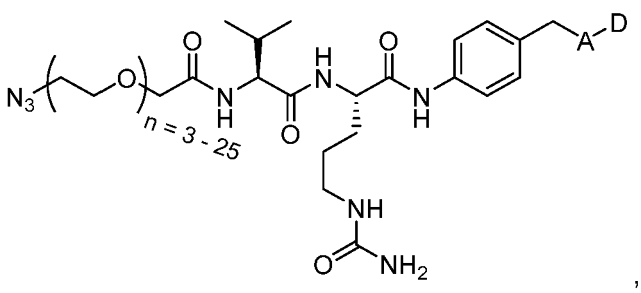

wherein:

A is a bond, -OC(=O)-*,

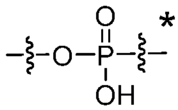 , 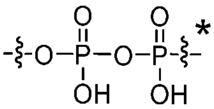 , 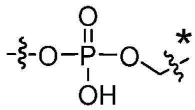 ,

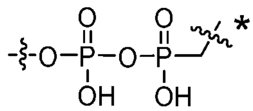 ,

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1;

(15)

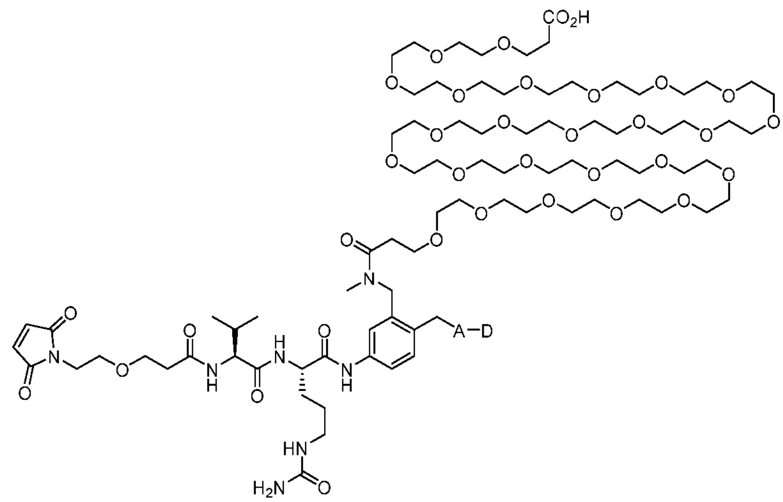 ,

wherein:

A is a bond, -OC(=O)-*,

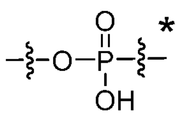 , 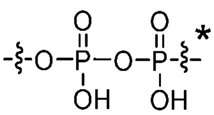 , 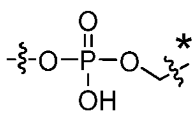 ,

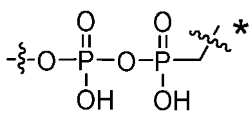 , .

-OC(=O)N($CH_3$)$CH_2CH_2$N($CH_3$)C(=O)-* or -OC(=O)N($CH_3$)C($R^a$)$_2$C($R^a$)$_2$N($CH_3$)C(=O)-*, wherein each $R^a$ is independently selected from H, $C_1$-$C_6$ alkyl, and $C_3$-$C_8$ cycloalkyl and the * of A indicates the point of attachment to D; and

D is an Mcl-1 inhibitor as defined in claim 1; or

(16)

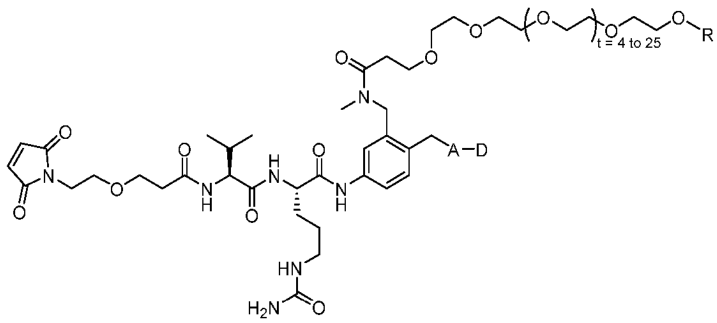

,

wherein:

each R independently is H, $-CH_3$ or $-CH_2CH_2C(=O)OH$;
A is a bond, -OC(=O)-*

, 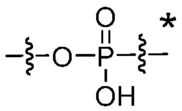 , 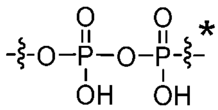 , 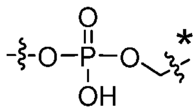 ,

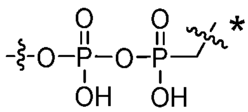 ,

$-OC(=O)N(CH_3)CH_2CH_2N(CH_3)C(=O)$-* or $-OC(=O)N(CH_3)C(R^a)_2C(R^a)_2N(CH_3)C(=O)$-*, wherein each $R^a$ is independently selected from H, $C_1$-$C_6$ alkyl, and $C_3$-$C_8$ cycloalkyl and the of A indicates the point of attachment to D; and
D is an Mcl-1 inhibitor as defined in claim 1.

**9.** The antibody-drug conjugate of any one of claims 1 to 8, wherein A is a bond.

**10.** The antibody-drug conjugate of any one of claims 1-9, wherein R is $-CH_3$.

**11.** The antibody-drug conjugate any one of claims 1 to 10, wherein -(L-D) is formed from a compound selected from Table A or an enantiomer, diastereoisomer, atropisomer, deuterated derivative, and/or pharmaceutically acceptable salt thereof.

**12.** The antibody-drug conjugate of claim 11, wherein the -(L-D) comprises or is formed from the following compound:

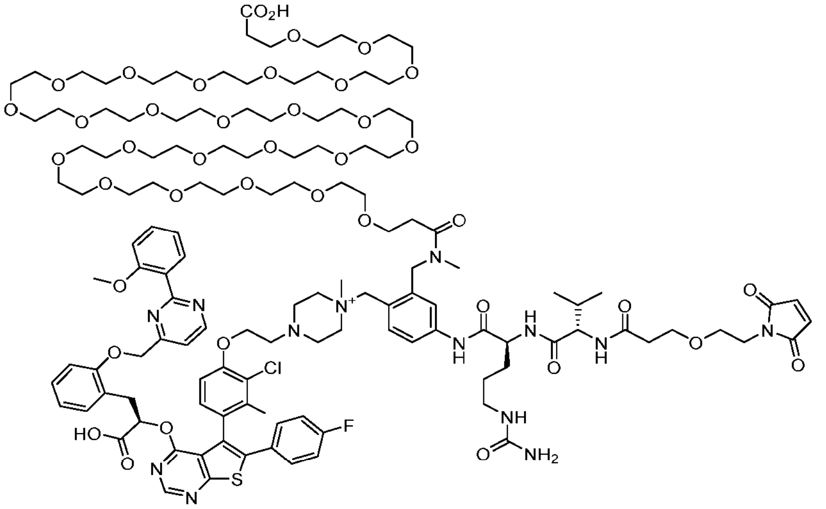

L11-P1,

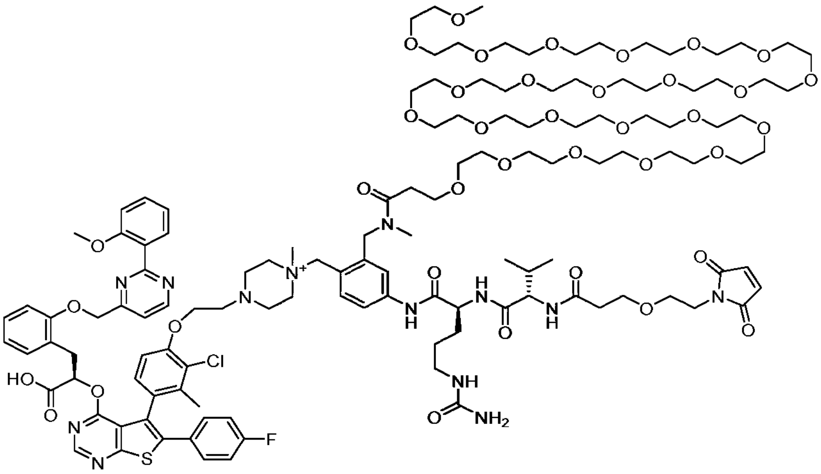

L30-P1,

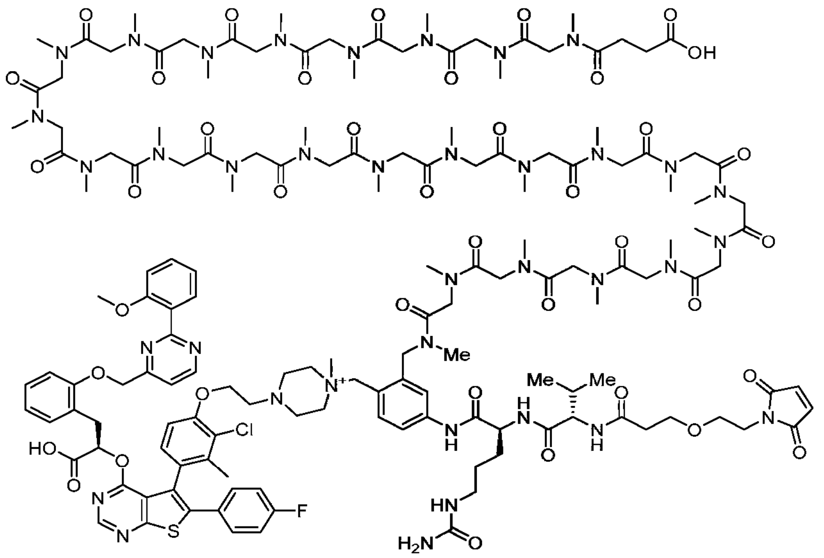

L37-P1,

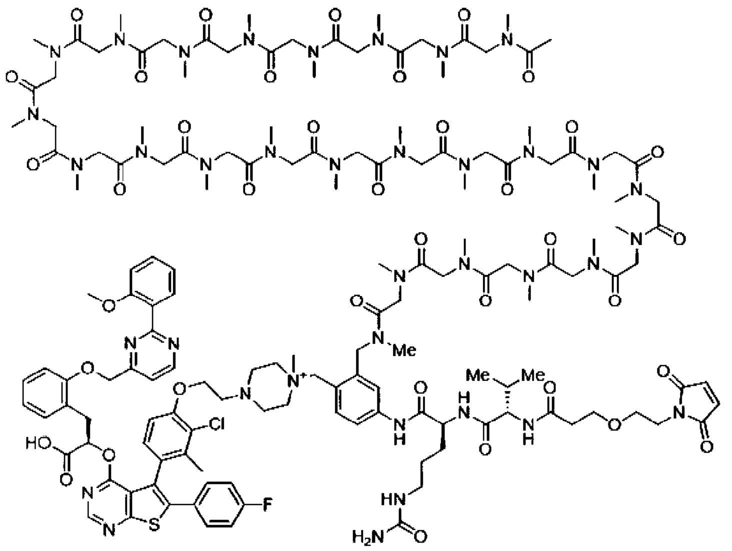

L40-P1,

or

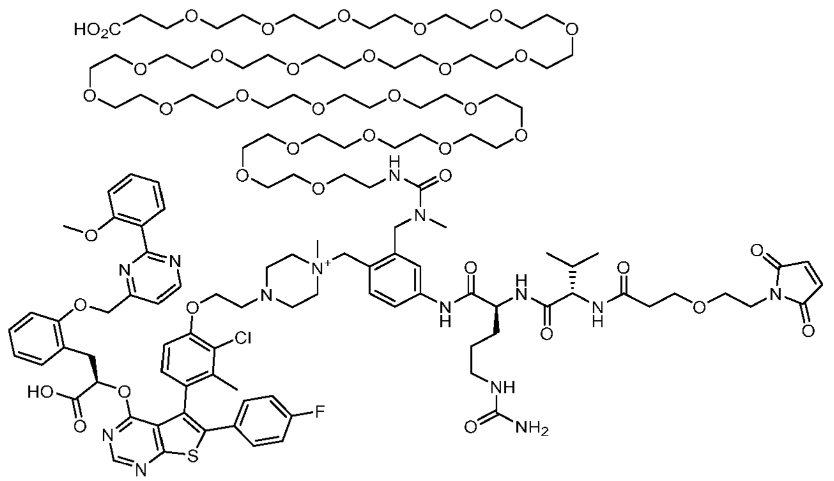

L42-P1.

**13.** The antibody drug conjugate of any one of claims 1-11, wherein

(1) the anti-CD74 antibody comprises an anti-CD74 antibody or antigen binding fragment comprising three heavy chain CDRs and three light chain CDRs as follows:

(i) heavy chain CDR1 (HCDR1) consisting of SEQ ID NO:1, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:16, light chain CDR2 (LCDR2) consisting of SEQ ID NO:70, and light chain CDR3 (LCDR3) consisting of SEQ ID NO:18;
(ii) heavy chain CDR1 (HCDR1) consisting of SEQ ID NO:4, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:16, light chain CDR2 (LCDR2) consisting of SEQ ID NO:70, and light chain CDR3 (LCDR3) consisting of SEQ ID NO:18;
(iii) heavy chain CDR1 (HCDR1) consisting of SEQ ID NO:5, heavy chain CDR2 (HCDR2) consisting of SEQ ID NO:6, heavy chain CDR3 (HCDR3) consisting of SEQ ID NO:3; light chain CDR1 (LCDR1) consisting of SEQ ID NO:19, light chain CDR2 (LCDR2) consisting of SEQ ID NO:20, and light chain CDR3 (LCDR3) consisting of SEQ ID NO:21;
(iv) heavy chain CDR1 (HCDR1) consisting of SEQ ID NO:7, heavy chain CDR2 (HCDR2) consisting of SEQ

ID NO:8, heavy chain CDR3 (HCDR3) consisting of SEQ ID NO:9; light chain CDR1 (LCDR1) consisting of SEQ ID NO:22, light chain CDR2 (LCDR2) consisting of SEQ ID NO:20, and light chain CDR3 (LCDR3) consisting of SEQ ID NO:18;
(v) heavy chain CDR1 (HCDR1) consisting of SEQ ID NO:1, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:35, light chain CDR2 (LCDR2) consisting of SEQ ID NO:70, and light chain CDR3 (LCDR3) consisting of SEQ ID NO:18;
(vi) heavy chain CDR1 (HCDR1) consisting of SEQ ID NO:4, heavy chain CDR2 (HCDR2) consisting of SEQ ID NO:2, heavy chain CDR3 (HCDR3) consisting of SEQ ID NO:3; light chain CDR1 (LCDR1) consisting of SEQ ID NO:35, light chain CDR2 (LCDR2) consisting of SEQ ID NO:70, and light chain CDR3 (LCDR3) consisting of SEQ ID NO:18;
(vii) heavy chain CDR1 (HCDR1) consisting of SEQ ID NO:5, heavy chain CDR2 (HCDR2) consisting of SEQ ID NO:6, heavy chain CDR3 (HCDR3) consisting of SEQ ID NO:3; light chain CDR1 (LCDR1) consisting of SEQ ID NO:71, light chain CDR2 (LCDR2) consisting of SEQ ID NO:20, and light chain CDR3 (LCDR3) consisting of SEQ ID NO:21.or
(viii) heavy chain CDR1 (HCDR1) consisting of SEQ ID NO:7, heavy chain CDR2 (HCDR2) consisting of SEQ ID NO:8, heavy chain CDR3 (HCDR3) consisting of SEQ ID NO:9; light chain CDR1 (LCDR1) consisting of SEQ ID NO:17, light chain CDR2 (LCDR2) consisting of SEQ ID NO:20, and light chain CDR3 (LCDR3) consisting of SEQ ID NO:18;

(2) the anti-CD74 antibody or antigen-binding fragment thereof comprises:

(i) a heavy chain variable region comprising the amino acid sequence of SEQ ID NO:10, and a light chain variable region comprising the amino acid sequence of SEQ ID NO:23;
(ii) a heavy chain variable region comprising the amino acid sequence of SEQ ID NO:10, and a light chain variable region comprising the amino acid sequence of SEQ ID NO:27;(iii) a heavy chain variable region comprising the amino acid sequence of SEQ ID NO:10, and a light chain variable region comprising the amino acid sequence of SEQ ID NO:31;
(iv) a heavy chain variable region comprising the amino acid sequence of SEQ ID NO:10, and a light chain variable region comprising the amino acid sequence of SEQ ID NO:36;
(v) a heavy chain variable region comprising the amino acid sequence of SEQ ID NO:10, and a light chain variable region comprising the amino acid sequence of SEQ ID NO:40; or
(vi) a heavy chain variable region comprising the amino acid sequence of SEQ ID NO:10, and a light chain variable region comprising the amino acid sequence of SEQ ID NO:44; or

(3) the anti-CD74 antibody comprises:

(a) the heavy chain amino acid sequence of SEQ ID NO:12 and the light chain amino acid sequence of SEQ ID NO:25;
(b) the heavy chain amino acid sequence of SEQ ID NO:14 and the light chain amino acid sequence of SEQ ID NO:25;
(c) the heavy chain amino acid sequence of SEQ ID NO:15 and the light chain amino acid sequence of SEQ ID NO:25;
(d) the heavy chain amino acid sequence of SEQ ID NO:12 and the light chain amino acid sequence of SEQ ID NO:29;
(e) the heavy chain amino acid sequence of SEQ ID NO:14 and the light chain amino acid sequence of SEQ ID NO:29;
(f) the heavy chain amino acid sequence of SEQ ID NO:15 and the light chain amino acid sequence of SEQ ID NO:29;
(g) the heavy chain amino acid sequence of SEQ ID NO:12 and the light chain amino acid sequence of SEQ ID NO:33;
(h) the heavy chain amino acid sequence of SEQ ID NO:14 and the light chain amino acid sequence of SEQ ID NO:33;
(i) the heavy chain amino acid sequence of SEQ ID NO:15 and the light chain amino acid sequence of SEQ ID NO:33;
(j) the heavy chain amino acid sequence of SEQ ID NO:12 and the light chain amino acid sequence of SEQ ID NO:38;
(k) the heavy chain amino acid sequence of SEQ ID NO:14 and the light chain amino acid sequence of SEQ ID

NO:38;
(l) the heavy chain amino acid sequence of SEQ ID NO:15 and the light chain amino acid sequence of SEQ ID NO:38;
(m) the heavy chain amino acid sequence of SEQ ID NO:12 and the light chain amino acid sequence of SEQ ID NO:42;
(n) the heavy chain amino acid sequence of SEQ ID NO:14 and the light chain amino acid sequence of SEQ ID NO:42;
(o) the heavy chain amino acid sequence of SEQ ID NO:15 and the light chain amino acid sequence of SEQ ID NO:42;
(p) the heavy chain amino acid sequence of SEQ ID NO:12 and the light chain amino acid sequence of SEQ ID NO:46;
(q) the heavy chain amino acid sequence of SEQ ID NO:14 and the light chain amino acid sequence of SEQ ID NO:46; or
(r) the heavy chain amino acid sequence of SEQ ID NO:15 and the light chain amino acid sequence of SEQ ID NO:46.

**14.** The antibody drug conjugate of any one of claims 1-13, wherein the Ab is a Fc silent antibody.

**15.** A composition comprising multiple copies of the antibody-drug conjugate of any one of claims 1 to 14, wherein the average p of the antibody-drug conjugates in the composition is from about 2 to about 16, e.g., about 2 to about 8, e.g., about 2 to about 4.

**16.** A pharmaceutical composition comprising the antibody-drug conjugate of any one of claims 1 to 14 or the composition of claim 15, and a pharmaceutically acceptable carrier.

**17.** An antibody-drug conjugate of any one of claims 1 to 14, the composition of claim 15, or the pharmaceutical composition of claim 16 for use in treating a subject having a cancer, optionally wherein

(1) the cancer expresses CD74; or
(2) the cancer is a tumor or a hematological cancer, preferably, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer.

**18.** An antibody-drug conjugate of any one of claims 1 to 14, the composition of claim 15, or the pharmaceutical composition of claim 16 for use in reducing or inhibiting the growth of a tumor in a subject, optionally wherein

(1) the tumor expresses CD74; or
(2) the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer.

**19.** An antibody-drug conjugate of any one of claims 1 to 14, the composition of claim 15, or the pharmaceutical composition of claim 16 for use according to claim 18, wherein administration of the antibody-drug conjugate, composition, or pharmaceutical composition reduces or inhibits the growth of the tumor by at least about 10%, at least about 20%, at least about 30%, at least about 40%, at least about 50%, at least about 60%, at least about 70%, at least about 80%, at least about 90%, at least about 95%, or at least about 99%.

**20.** An antibody-drug conjugate of any one of claims 1 to 14, the composition of claim 15, or the pharmaceutical composition of claim 16 for use in reducing or slowing the expansion of a cancer cell population in a subject, optionally wherein

(1) the cancer cell population expresses CD74; or
(2) the cancer cell population is from a tumor or a hematological cancer, preferably the cancer cell population is from a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic

leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer.

**21.** An antibody-drug conjugate of any one of claims 1 to 14, the composition of claim 15, or the pharmaceutical composition of claim 16 for use according to claim 20 , wherein administration of the antibody-drug conjugate, composition, or pharmaceutical composition reduces the cancer cell population or slows the expansion of the cancer cell population by at least about 10%, at least about 20%, at least about 30%, at least about 40%, at least about 50%, at least about 60%, at least about 70%, at least about 80%, at least about 90%, at least about 95%, or at least about 99%.

**22.** A method of determining whether a subject having or suspected of having a cancer will be responsive to treatment with the antibody-drug conjugate of any one of claims 1 to 14, the composition of claim 15, or the pharmaceutical composition of claim 16, comprising providing a biological sample from the subject; contacting the sample with the antibody-drug conjugate; and detecting binding of the antibody-drug conjugate to cancer cells in the sample; optionally wherein

(1) the cancer cells in the sample express CD74;
(2) the cancer expresses CD74; or
(3) the cancer is a tumor or a hematological cancer, preferably the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer.

**23.** The method of claim 22, wherein the sample is a tissue biopsy sample, a blood sample, or a bone marrow sample.

**24.** A method of producing the antibody-drug conjugate of any one of claims 1 to 14, comprising reacting an antibody or antigen-binding fragment with a cleavable linker joined to an MCL1 inhibitor under conditions that allow conjugation.

**25.** An antibody-drug conjugate of any one of claims 1 to 14, the composition of claim 15, or the pharmaceutical composition of claim 16 for use according to any one of claims 17 to 21, further comprising administering to the subject in need thereof at least one additional therapeutic agent, preferably the one additional therapeutic agent is a Bcl-2 inhibitor, more preferably the one additional therapeutic agent is venetoclax, compound A1 or compound A2.

## Patentansprüche

**1.** Antikörper-Wirkstoff-Konjugat der Formel (1):

$$\text{Ab-(L-D)}_p \qquad (1)$$

wobei:

Ab ein Anti-CD74-Antikörper oder ein Antigenbindungsfragment davon ist;
$p$ eine ganze Zahl von 1 bis 16 ist; und
-(L-D) von der Formel (C) ist:

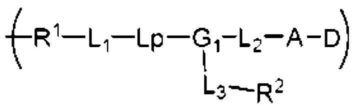

(C),

wobei:

$R^1$ eine Anknüpfungsgruppe ist;
$L_1$ ein überbrückender Spacer ist;

$L_p$ eine Peptidgruppe umfassend 1 bis 6 Aminosäuren ist;
D ein Mcl-1-Inhibitor ist, wobei

(1) D Folgendes umfasst:

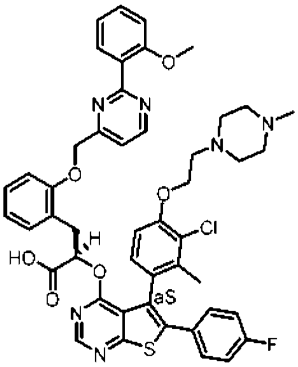

oder ein Enantiomer, Diastereoisomer, Atropisomer, deuteriertes Derivat und/oder pharmazeutisch verträgliches Salz von einem beliebigen des Vorstehenden;
(2) D Folgendes umfasst:

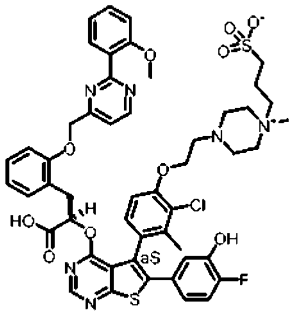

oder ein Enantiomer, Diastereoisomer, Atropisomer, deuteriertes Derivat und/oder pharmazeutisch verträgliches Salz von einem beliebigen des Vorstehenden;
(3) D Folgendes umfasst:

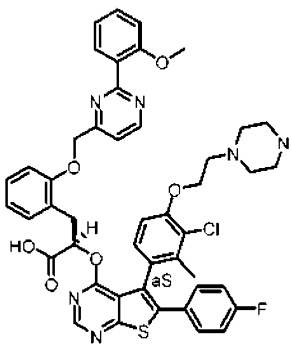

oder ein Enantiomer, Diastereoisomer, Atropisomer, deuteriertes Derivat und/oder pharmazeutisch verträgliches Salz von einem beliebigen des Vorstehenden;
(4) D Folgendes umfasst:

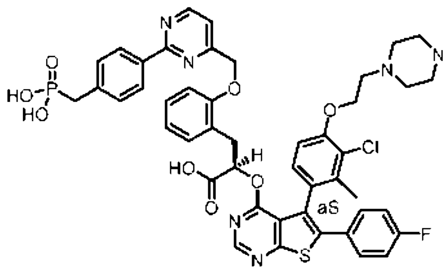

oder ein Enantiomer, Diastereoisomer, Atropisomer, deuteriertes Derivat und/oder pharmazeutisch verträgliches Salz von einem beliebigen des Vorstehenden;
(5) D Folgendes umfasst:

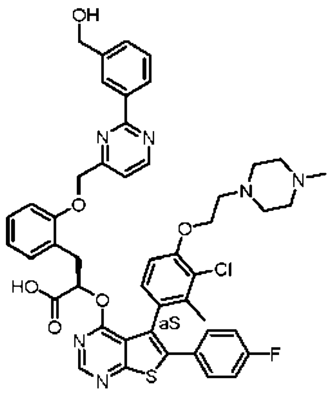

oder ein Enantiomer, Diastereoisomer, Atropisomer, deuteriertes Derivat und/oder pharmazeutisch verträgliches Salz von einem beliebigen des Vorstehenden;
(6) D Folgendes umfasst:

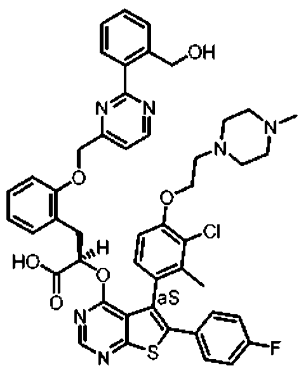

oder ein Enantiomer, Diastereoisomer, Atropisomer, deuteriertes Derivat und/oder pharmazeutisch verträgliches Salz von einem beliebigen des Vorstehenden;
(7) D Folgendes umfasst:

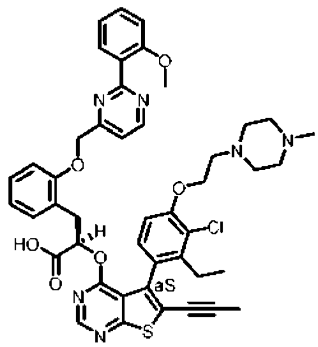

oder ein Enantiomer, Diastereoisomer, Atropisomer, deuteriertes Derivat und/oder pharmazeutisch verträgliches Salz von einem beliebigen des Vorstehenden;
(8) D Folgendes umfasst:

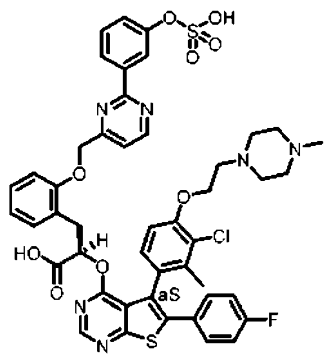

oder ein Enantiomer, Diastereoisomer, Atropisomer, deuteriertes Derivat und/oder pharmazeutisch verträgliches Salz von einem beliebigen des Vorstehenden;
(9) D Folgendes umfasst:

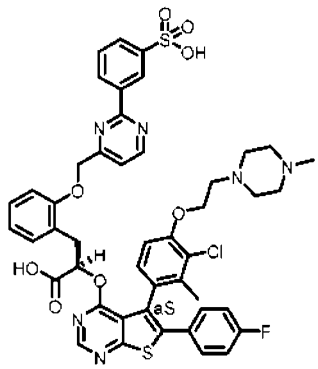

oder ein Enantiomer, Diastereoisomer, Atropisomer, deuteriertes Derivat und/oder pharmazeutisch verträgliches Salz von einem beliebigen des Vorstehenden;
(10) D Folgendes umfasst:

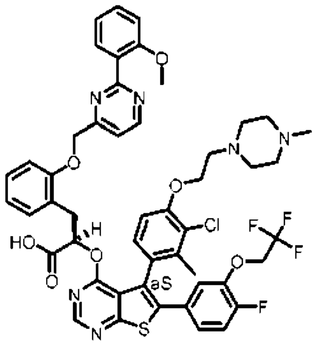

oder ein Enantiomer, Diastereoisomer, Atropisomer, deuteriertes Derivat und/oder pharmazeutisch verträgliches Salz von einem beliebigen des Vorstehenden;

(11) D Folgendes umfasst:

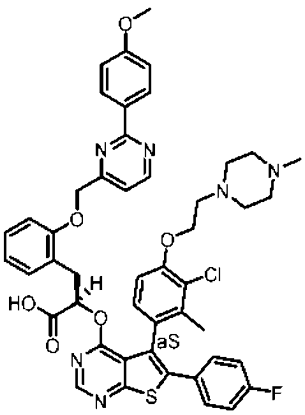

oder ein Enantiomer, Diastereoisomer, Atropisomer, deuteriertes Derivat und/oder pharmazeutisch verträgliches Salz von einem beliebigen des Vorstehenden;

(12) D Folgendes umfasst:

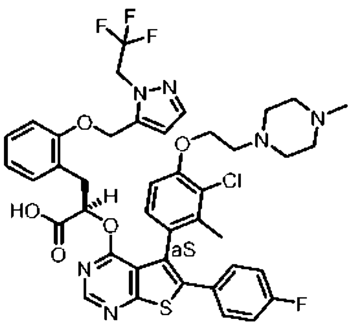

oder ein Enantiomer, Diastereoisomer, Atropisomer, deuteriertes Derivat und/oder pharmazeutisch verträgliches Salz von einem beliebigen des Vorstehenden; oder

(13) D Folgendes umfasst:

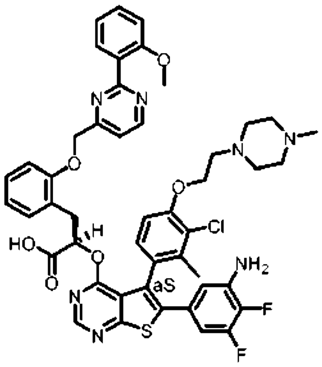

oder ein Enantiomer, Diastereoisomer, Atropisomer, deuteriertes Derivat und/oder pharmazeutisch verträgliches Salz von einem beliebigen des Vorstehenden;

(14) D Folgendes umfasst:

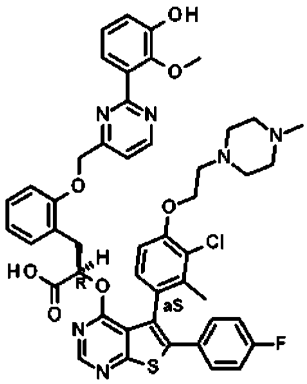

oder ein Enantiomer, Diastereoisomer, Atropisomer, deuteriertes Derivat und/oder pharmazeutisch verträgliches Salz von einem beliebigen des Vorstehenden;

(15) D Folgendes umfasst:

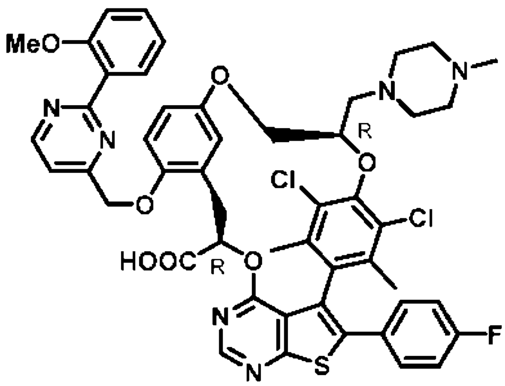

oder ein Enantiomer, Diastereoisomer, Atropisomer, deuteriertes Derivat und/oder pharmazeutisch verträgliches Salz von einem beliebigen des Vorstehenden;

(16) D Folgendes umfasst:

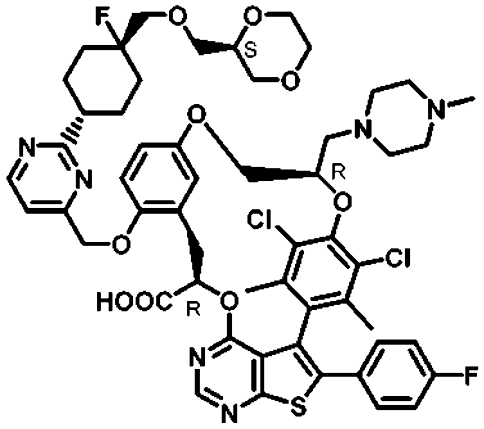

oder ein Enantiomer, Diastereoisomer, Atropisomer, deuteriertes Derivat und/oder pharmazeutisch verträgliches Salz von einem beliebigen des Vorstehenden; oder

(17) D Folgendes umfasst:

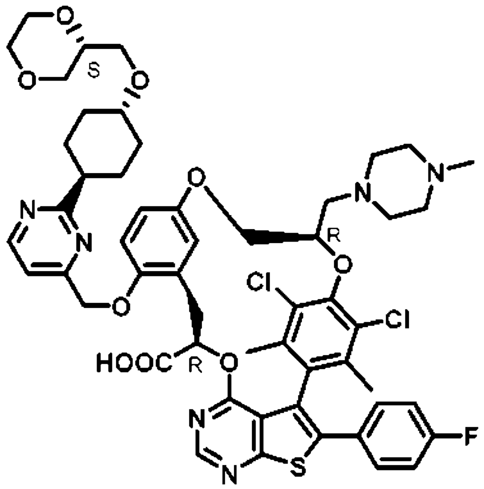

oder ein Enantiomer, Diastereoisomer, Atropisomer, deuteriertes Derivat und/oder pharmazeutisch verträgliches Salz von einem beliebigen des Vorstehenden;

$G_1$-$L_2$-A ein selbstimmolativer Spacer ist;
$L_2$ eine Bindung, ein Methylen, ein Neopentylen oder ein $C_2$-$C_3$-Alkenylen ist;
A eine Bindung, -OC(=O)-*,

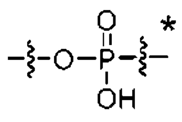 , 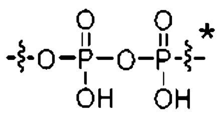 , 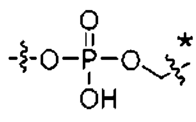 ,

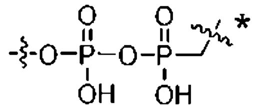 ,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt;
$L_3$ eine Spacer-Einheit ist; und
$R^2$ eine hydrophile Einheit ist.

2. Antikörper-Wirkstoff-Konjugat nach Anspruch 1 oder pharmazeutisch verträgliches Salz davon, wobei -(L-D) von Formel (D) ist: wobei:

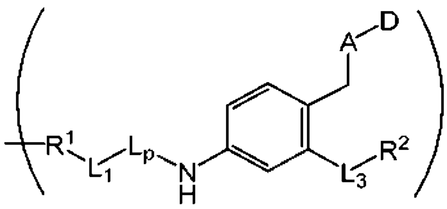

(D),

$R^1$ eine Anknüpfungsgruppe ist;
$L_1$ ein überbrückender Spacer ist;
$L_p$ eine Peptidgruppe umfassend 1 bis 6 Aminosäuren ist;
A eine Bindung, -OC(=O)-*,

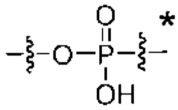 , 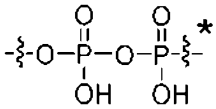 , 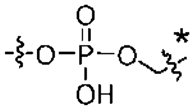 ,

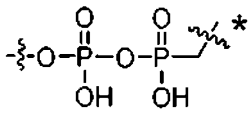 ,

$-OC(=O)N(CH_3)CH_2CH_2N(CH_3)C(=O)-*$ oder - $OC(=O)N(CH_3)C(R^a)_2C(R^a)_2N(CH_3)C(=O)-*$ ist,
wobei jedes $R^a$ unabhängig ausgewählt ist aus H, $C_1$-$C_6$-Alkyl und $C_3$-$C_8$-Cycloalkyl und das * von A den Anknüpfungspunkt an D angibt;
$L_3$ eine Spacer-Einheit ist; und
$R^2$ eine hydrophile Einheit ist.

3. Antikörper-Wirkstoff-Konjugat nach Anspruch 1 oder 2, wobei

(i) $L_1$ Folgendes umfasst:

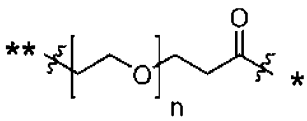 , 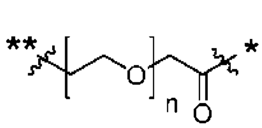

oder * CH(OH)CH(OH)CH(OH)CH(OH)-**,
wobei jedes n eine ganze Zahl von 1 bis 12 ist, wobei das * von $L_1$ den Punkt von direkter oder indirekter Anknüpfung an Lp angibt und das ** von $L_1$ den Punkt von direkter oder indirekter Anknüpfung an $R^1$ angibt;
(ii)

$L_1$ 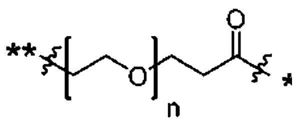

ist und n eine ganze Zahl von 1 bis 12 ist oder n 1 ist oder n 12 ist, wobei das * von $L_1$ den Punkt von direkter oder indirekter Anknüpfung an Lp angibt und das ** von $L_1$ den Punkt von direkter oder indirekter Anknüpfung an $R^1$ angibt;
(iii)

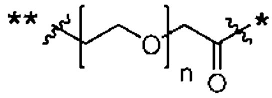

$L_1$

ist und n eine ganze Zahl von 1 bis 12 ist, wobei das * von $L_1$ den Punkt von direkter oder indirekter Anknüpfung an Lp angibt und das ** von Li den Punkt von direkter oder indirekter Anknüpfung an $R^1$ angibt;
(iv)

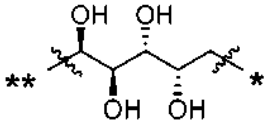

$L_1$

umfasst, wobei das * von $L_1$ den Punkt von direkter oder indirekter Anknüpfung an Lp angibt und das ** von $L_1$ den Punkt von direkter oder indirekter Anknüpfung an $R^1$ angibt; oder
(v) $L_1$ ein überbrückender Spacer ist, umfassend:

$*\text{-}C(=O)(CH_2)_mO(CH_2)_m\text{-}**$; $*\text{-}C(=O)((CH_2)_mO)_t(CH_2)_n\text{-}**$; $*\text{-}C(=O)(CH_2)_m\text{-}**$;

$*\text{-}C(=O)NH((CH_2)_mO)_t(CH_2)_n\text{-}**$;

$*\text{-}C(=O)O(CH_2)_mSSC(R^3)_2(CH_2)_mC(=O)NR^3(CH_2)_mNR^3C(=O)(CH_2)_m\text{-}**$;

$*\text{-}C(=O)O(CH_2)_mC(=O)NH(CH_2)_m\text{-}**$; $*\text{-}C(=O)(CH_2)_mNH(CH_2)_m\text{-}**$;

$*\text{-}C(=O)(CH_2)_mNH(CH_2)_nC(=O)\text{-}**$; $*\text{-}C(=O)(CH_2)_nX_1(CH_2)_{m\text{-}}**$;

$*\text{-}C(=O)((CH_2)_mO)_t(CH_2)_nX_1(CH_2)_n\text{-}**$; $*\text{-}C(=O)(CH_2)_mNHC(=O)(CH_2)_n\text{-}**$;

$*\text{-}C(=O)((CH_2)_mO)_t(CH_2)_nNHC(=O)(CH_2)_n\text{-}**$;

$*\text{-}C(=O)(CH_2)_mNHC(=O)(CH_2)_nX_1(CH_2)_n\text{-}**$;

$*\text{-}C(=O)((CH_2)_mO)_t(CH_2)_nNHC(=O)(CH_2)_nX_1(CH_2)_n\text{-}**$;

$*\text{-}C(=O)((CH_2)_mO)_t(CH_2)_nC(=O)NH(CH_2)_m\text{-}**$; $*\text{-}C(=O)(CH_2)_mC(R^3)_2\text{-}**$

oder

$*\text{-}C(=O)(CH_2)_mC(=O)NH(CH_2)_m\text{-}**$,

wobei das * von $L_1$ den Punkt von direkter oder indirekter Anknüpfung an Lp angibt und das ** von $L_1$ den Punkt von direkter oder indirekter Anknüpfung an $R^1$ angibt;

$X_1$

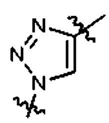
,
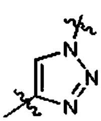
,
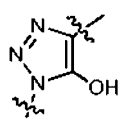
oder
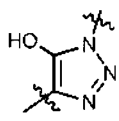

ist; und jedes m unabhängig ausgewählt ist aus 1, 2, 3, 4, 5, 6, 7, 8, 9 und 10;
jedes n unabhängig ausgewählt ist aus 1, 2, 3, 4, 5, 6, 7, 8, 9 und 10; und
jedes t unabhängig ausgewählt ist aus 1, 2, 3, 4, 5, 6, 7, 8, 9, 10, 11, 12, 13, 14, 15, 16, 17, 18, 19, 20, 21, 22, 23, 24, 25, 26, 27, 28, 29 und 30;
und jedes $R^3$ unabhängig ausgewählt ist aus H und $C_1$-$C_6$-Alkyl.

**4.** Antikörper-Wirkstoff-Konjugat nach einem der Ansprüche 1 bis 3, wobei

(1) $R^2$ eine hydrophile Einheit ist, die Polyethylenglykol, Polyalkylenglykol, ein Polyol, ein Polysarcosin, einen Zucker, ein Oligosaccharid, ein Polypeptid oder $C_2$-$C_6$-Alkyl substituiert mit 1 bis 3

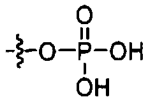 - oder 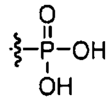

-Gruppen umfasst;
(2)

$R^2$ 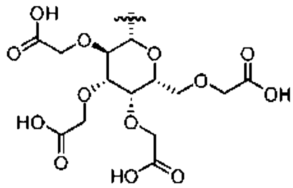, 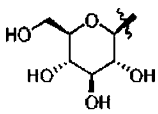, 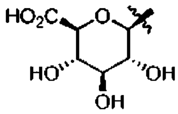, 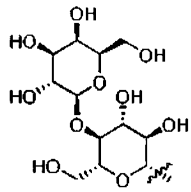, 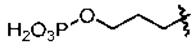,

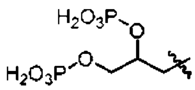, 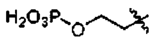, 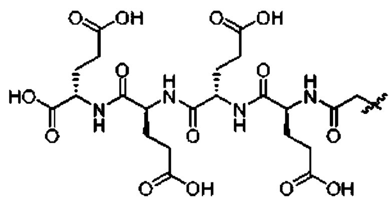,

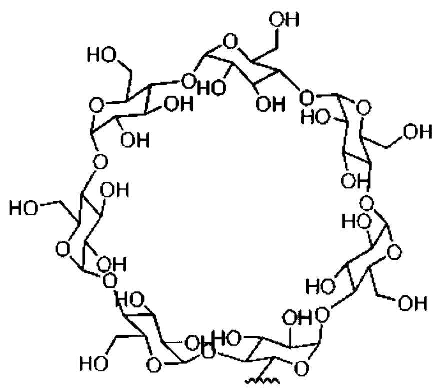, 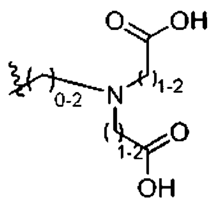, 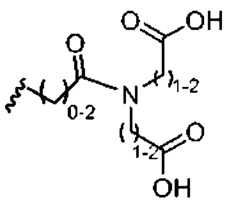,

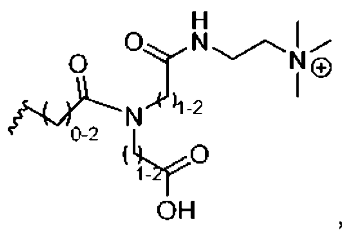, 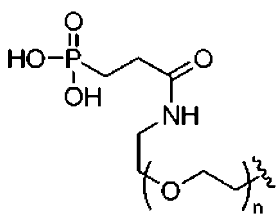,

wobei n eine ganze Zahl zwischen 1 und 6 ist,

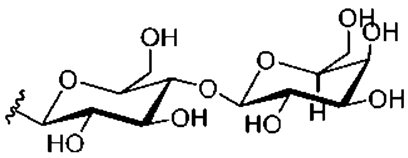 , 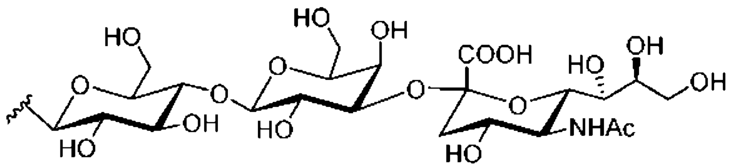

oder

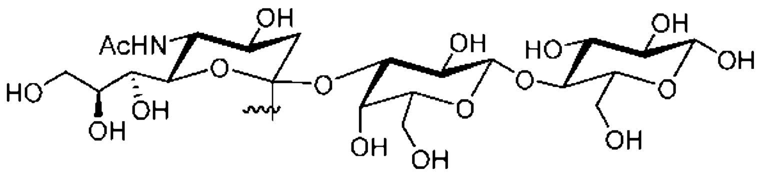

ist;

(3) die hydrophile Einheit dargestellt durch $R^2$ Folgendes umfasst:

(i) ein Polysarkosin, z. B. mit der folgenden Einheit:

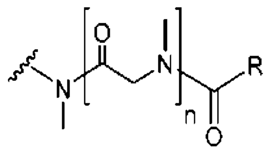 ,

wobei n eine ganze Zahl zwischen 3 und 25 ist; und R H, $-CH_3$ oder - $CH_2CH_2C(=O)OH$ ist; oder

(ii) ein Polyethylenglykol der Formel:

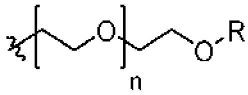 oder 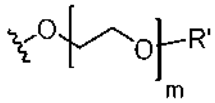

, wobei R H, - $CH_3$, $CH_2CH_2NHC(=O)OR_a$, $-CH_2CH_2NHC(=O)R_a$ oder $-CH_2CH_2C(=O)OR_a$ ist, R' OH, $-OCH_3$, $-CH_2CH_2NHC(=O)OR_a$, $-CH_2CH_2NHC(=O)R_a$ oder $-OCH_2CH_2C(=O)OR_a$ ist, wobei $R_a$ H oder $C_{1-4}$-Alkyl optional substituiert mit entweder OH oder $C_{1-4}$-Alkoxyl ist und jedes von m und n unabhängig eine ganze Zahl zwischen 2 und 25 ist; oder

(4) die hydrophile Einheit dargestellt durch $R^2$

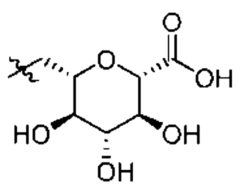

umfasst.

**5.** Antikörper-Wirkstoff-Konjugat nach einem der Ansprüche 1 bis 4, wobei:

(i) $L_3$ eine Spacer-Einheit mit der Struktur

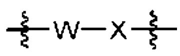

ist,

wobei:

W $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, - NHC(=O) $C(R^b)_2NHC(=O)-$, $-CH_2N(X-R^2)C(=O)O-$, $-C(=O)N(X-R^2)-$, $-CH_2N(X-R^2)C(=O)-$, - C(=O)NR$^b$-, -C(=O)NH-, $-CH_2NR^bC(=O)-$, $-CH_2NR^bC(=O)NH-$, $-CH_2NR^bC(=O)NR^b-$, - NHC(=O)-, -NHC(=O)O-, -NHC(=O)NH-, -OC(=O)NH-, $-S(O)_2NH-$, $-NHS(O)_2-$, -C(=O)-, - C(=O)O- oder -NH- ist, wobei jedes $R^b$ unabhängig ausgewählt ist aus H, $C_1$-$C_6$-Alkyl und $C_3$-$C_8$-Cycloalkyl; und
X eine Bindung, Triazolyl oder $-CH_2$-Triazolyl- ist; oder

(ii) $L_3$ eine Spacer-Einheit mit der Struktur

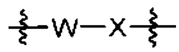

ist,
wobei:

W $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, - NHC(=O) $C(R^b)_2NHC(=O)-$, $-CH_2N(X-R^2)C(=O)O-$, $-C(=O)N(X-R^2)-$, $-CH_2N(X-R^2)C(=O)-$, - C(=O)NR$^b$-, -C(=O)NH-, $-CH_2NR^bC(=O)-$, $-CH_2NR^bC(=O)NH-$, $-CH_2NR^bC(=O)NR^b-$, - NHC(=O)-, -NHC(=O)O-, -NHC(=O)NH-, -OC(=O)NH-, $-S(O)_2NH-$, $-NHS(O)_2-$, -C(=O)-, - C(=O)O- oder -NH- ist, wobei jedes $R^b$ unabhängig ausgewählt ist aus H, $C_1$-$C_6$-Alkyl und $C_3$-$C_8$-Cycloalkyl; und
X $-CH_2$-Triazolyl-$C_{1-4}$-alkylen-$OC(O)NHS(O)_2NH-$, $-C_{4-6}$-Cycloalkylen-$OC(O)NHS(O)_2NH-$, -$(CH_2CH_2O)_n-C(O)NHS(O)_2NH-$, $-(CH_2CH_2O)_n-C(O)NHS(O)_2NH-(CH_2CH_2O)_n-$ oder $-CH_2$-Triazolyl-$C_{1-4}$-alkylen-$OC(O)NHS(O)_2NH-(CH_2CH_2O)_n-$ ist, wobei jedes n unabhängig 1, 2 oder 3 ist.

**6.** Antikörper-Wirkstoff-Konjugat nach einem der Ansprüche 1 bis 5, wobei

(i) die Anknüpfungsgruppe durch eine Reaktion gebildet ist, die zumindest eine reaktive Gruppe umfasst;
(ii) die Anknüpfungsgruppe durch Reagieren von Folgendem gebildet wird:

einer ersten reaktiven Gruppe, die an den Linker angeknüpft ist, und
einer zweiten reaktiven Gruppe, die an den Antikörper angeknüpft ist oder ein Aminosäurerest des Antikörpers ist;

(iii) die Anknüpfungsgruppe durch eine Reaktion gebildet ist, die zumindest eine reaktive Gruppe umfasst, wobei zumindest eine der reaktiven Gruppen Folgendes umfasst:

ein Thiol,
ein Maleimid,
ein Haloacetamid,
ein Azid,
ein Alkin,
ein Cycloocten,
ein Triarylphosphin,
ein Oxanorbornadien,
ein Cyclooctin,
ein Diaryltetrazin,
ein Monoaryltetrazin,
ein Norbornen,
ein Aldehyd,
ein Hydroxylamin,
ein Hydrazin,
$NH_2$-NH-C(=O)-,
ein Keton,
ein Vinylsulfon,
ein Aziridin,
einen Aminosäurerest,

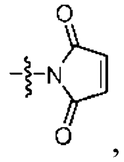,

$-ONH_2$, $-NH_2$,

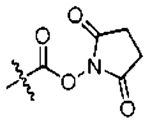, 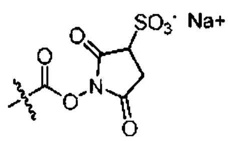, 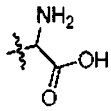, 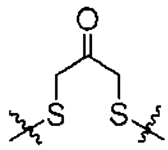,

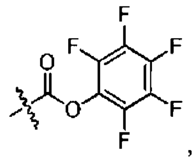, 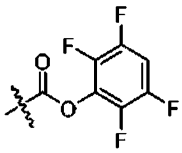, 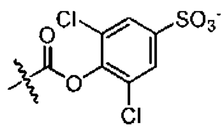,

$-N_3$,

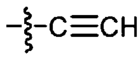,

$-SH$, $-SR^3$, $-SSR^4$, $-S(=O)_2(CH=CH_2)$, $-(CH_2)_2S(=O)_2(CH=CH_2)$, $-NHS(=O)_2(CH=CH_2)$, $-NHC(=O)CH_2Br$, $-NHC(=O)CH_2I$,

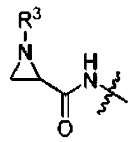,

$-C(O)NHNH_2$,

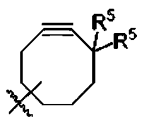,

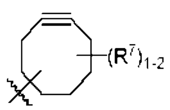, 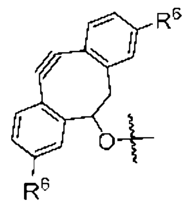, 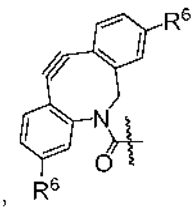, 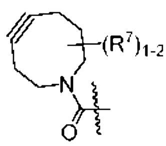, 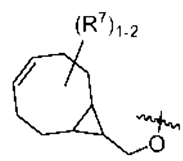,

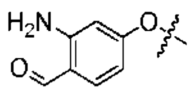, 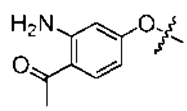, 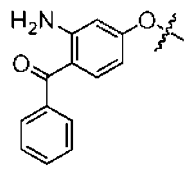, 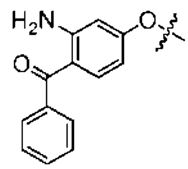,

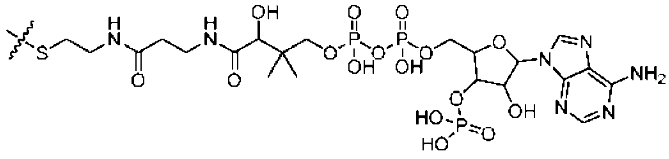

,

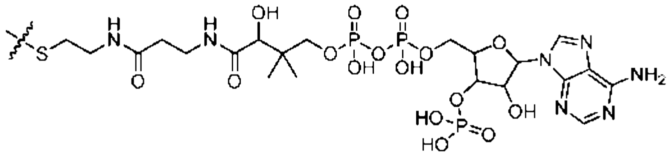

,

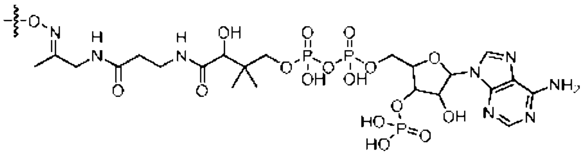

,

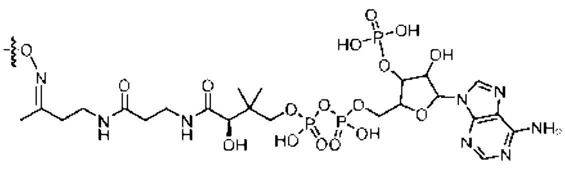

,

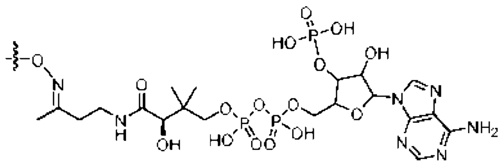

,

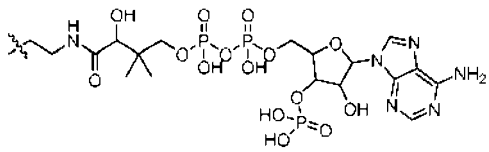

,

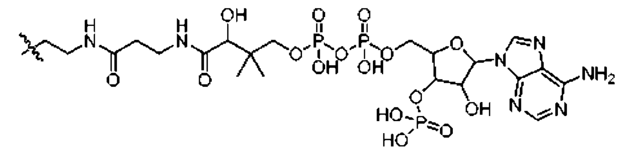

oder

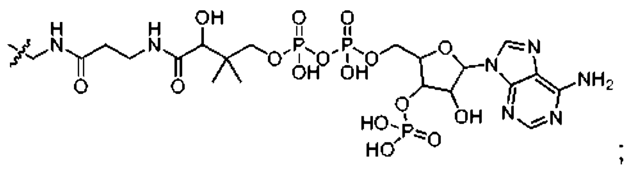

;

wobei:

jedes $R^3$ unabhängig ausgewählt ist aus H und $C_1$-$C_6$-Alkyl;
jedes $R^4$ 2-Pyridyl oder 4-Pyridyl ist;
jedes $R^5$ unabhängig ausgewählt ist aus H, $C_1$-$C_6$-Alkyl, F, Cl und -OH;
jedes $R^6$ unabhängig ausgewählt ist aus H, $C_1$-$C_6$-Alkyl, F, Cl, $-NH_2$, $-OCH_3$, $-OCH_2CH_3$, - $N(CH_3)_2$, -CN, $-NO_2$ und -OH;
jedes $R^7$ unabhängig ausgewählt ist aus H, $C_{1-6}$-Alkyl, Fluor, Benzyloxy substituiert mit - C(=O)OH, Benzyl substituiert mit -C(=O)OH, $C_{1-4}$-Alkoxy substituiert mit -C(=O)OH und $C_{1-4}$-Alkyl substituiert mit -C(=O)OH;

(iv) die Anknüpfungsgruppe durch Reagieren gebildet ist, die an den Linker und eine zweite reaktive Gruppe angeknüpft ist, die an den Antikörper angeknüpft ist oder ein Aminosäurerest des Antikörpers ist, wobei die erste reaktive Gruppe und die zweite reaktive Gruppe Folgendes umfassen:

ein Thiol und ein Maleimid,
ein Thiol und ein Haloacetamid,
ein Thiol und ein Vinylsulfon,
ein Thiol und ein Aziridin,
ein Azid und ein Alkin,
ein Azid und ein Cyclooctin,
ein Azid und ein Cycloocten,
ein Azid und ein Triarylphosphin,
ein Azid und ein Oxanorbornadien,
ein Diaryltetrazin und ein Cycloocten,
ein Monoaryltetrazin und ein Norbornen,
ein Aldehyd und ein Hydroxylamin,
ein Aldehyd und ein Hydrazin,
ein Aldehyd und $NH_2$-NH-C(=O)-,
ein Keton und ein Hydroxylamin,
ein Keton und ein Hydrazin,
ein Keton und $NH_2$-NH-C(=O)-,
ein Hydroxylamin und

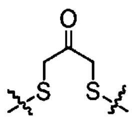,

ein Amin und

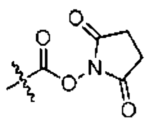, 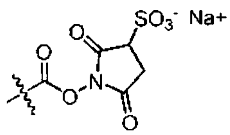, 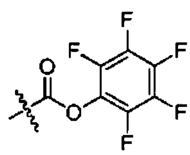, 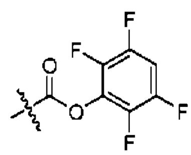

oder

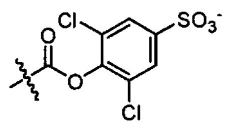

oder ein CoA oder CoA-Analogon und einen Serinrest; oder

(v) die Anknüpfungsgruppe eine Gruppe ausgewählt aus Folgendem umfasst:

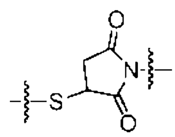;

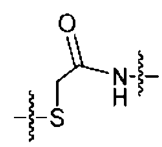;

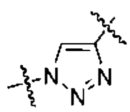 oder 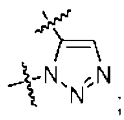 ;

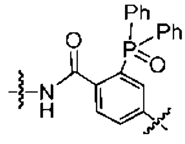 ;

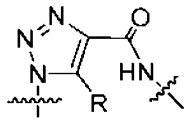 ;

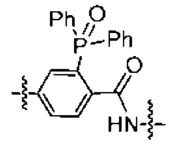 ;

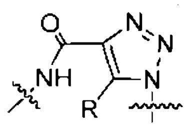 ;

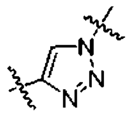 oder 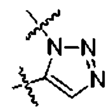 ;

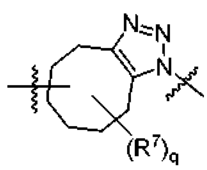 oder 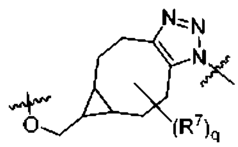 oder 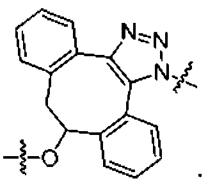 ;

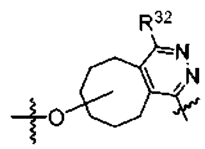 oder 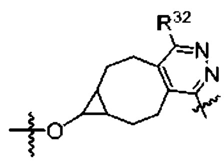 ;

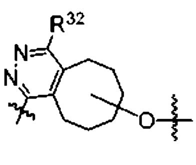 oder 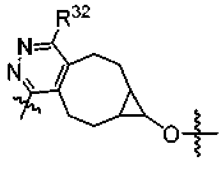 ;

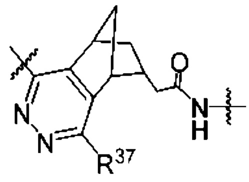
;

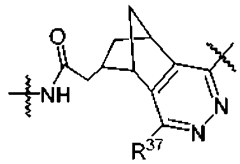
;

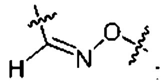
;

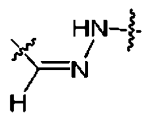
;

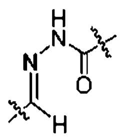
;

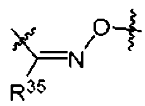
;

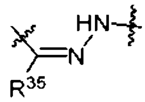
;

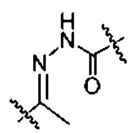
;

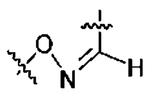
;

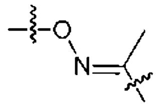
;

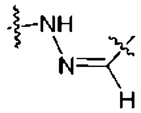
;

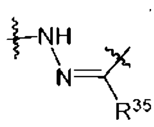;

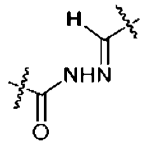;

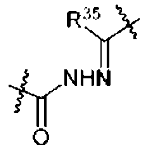;

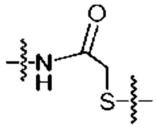;

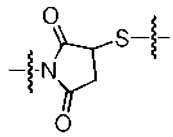;

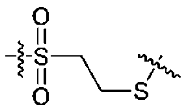;

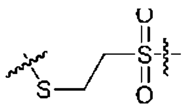;

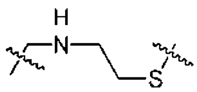 oder 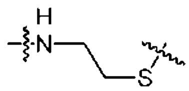;

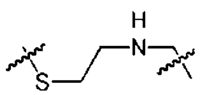 oder 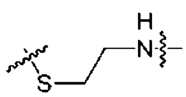;

Amid;

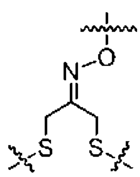;

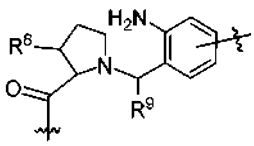

;

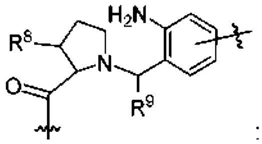

:

,

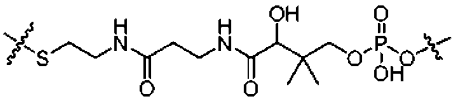

;

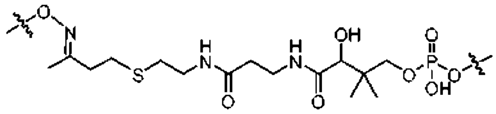

;

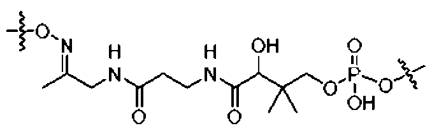

;

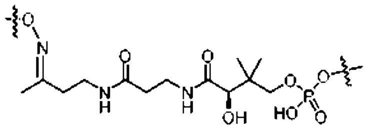

;

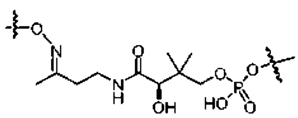

;

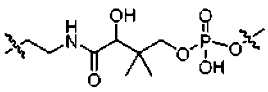

;

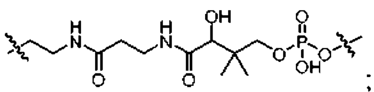

;

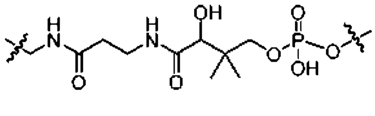

; und
Disulfid,
wobei:

$R^{32}$ H, $C_{1-4}$-Alkyl, Phenyl, Pyrimidin oder Pyridin ist;
$R^{35}$ H, $C_{1-6}$-Alkyl, Phenyl oder $C_{1-4}$-Alkyl substituiert mit 1 bis 3 -OH-Gruppen ist;

jedes $R^7$ unabhängig ausgewählt ist aus H, $C_{1-6}$-Alkyl, Fluor, Benzyloxy substituiert mit - C(=O)OH, Benzyl substituiert mit -C(=O)OH, $C_{1-4}$-Alkoxy substituiert mit -C(=O)OH und $C_{1-4}$-Alkyl substituiert mit -C(=O)OH;
$R^{37}$ unabhängig ausgewählt ist aus H, Phenyl und Pyridin;
q 0, 1, 2 oder 3 ist;
$R^8$ H oder Methyl ist; und
$R^9$ H, $-CH_3$ oder Phenyl ist.

**7.** Antikörper-Wirkstoff-Konjugat nach einem der Ansprüche 1 bis 6, wobei

(i) die Peptidgruppe 1 bis 4 Aminosäurereste, 1 bis 3 Aminosäurereste oder 1 bis 2 Aminosäurereste umfasst;
(ii) die Peptidgruppe Aminosäurereste ausgewählt aus L-Glycin (Gly), L-Valin (Val), L-Citrullin (Cit), L-Cysteinsäure (sulfo-Ala), L-Lysin (Lys), L-Isoleucin (Ile), L-Phenylalanin (Phe), L-Methionin (Met), L-Asparagin (Asn), L-Prolin (Pro), L-Alanin (Ala), L-Leucin (Leu), L-Tryptophan (Trp) und L-Tyrosin (Tyr) umfasst;
(iii) die Peptidgruppe Val-Cit, Phe-Lys, Val-Ala, Val-Lys, Leu-Cit, Sulfo-Ala-Val und/oder Sulfo-Ala-Val-Ala umfasst; oder
(iv) Lp ausgewählt ist aus:

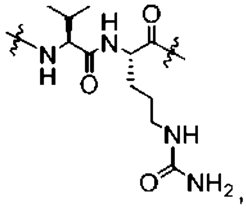,
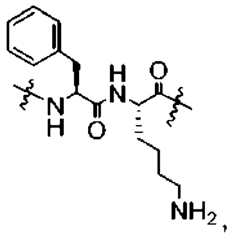,
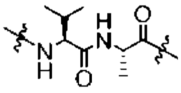,
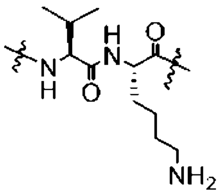,
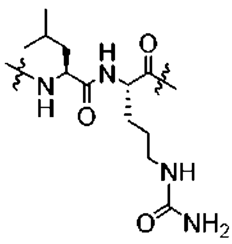,

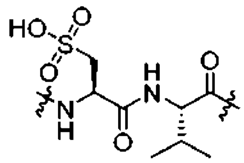
und
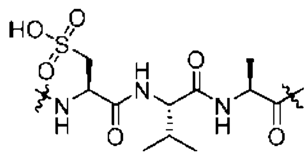.

**8.** Antikörper-Wirkstoff-Konjugat der Formel (1):

Ab-(L-D)$_p$ (1)

wobei:

Ab ein Anti-CD74-Antikörper oder ein Antigenbindungsfragment davon ist;
*p* eine ganze Zahl von 1 bis 16 ist; und
wobei -(L-D) eine Verbindung der folgenden Formel umfasst oder daraus gebildet ist:

(1)

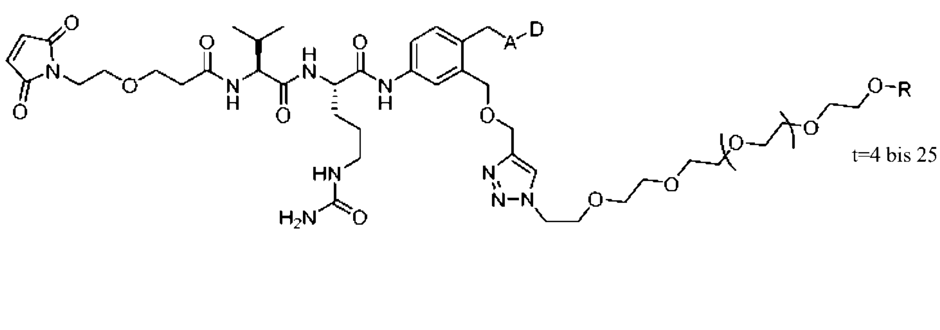


wobei:

R H, $-CH_3$ oder $-CH_2CH_2C(=O)OH$ ist;

A eine Bindung, -OC(=O)-*,

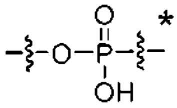 , 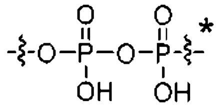 , 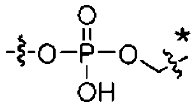 ,

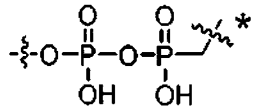 ,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und

D ein Mcl-1-Inhibitor wie definiert in Anspruch 1 ist;

(2)

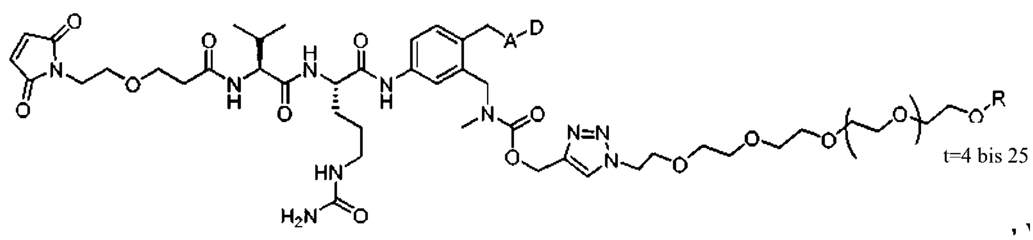

, '

wobei:

R H, -$CH_3$ oder -$CH_2CH_2$C(=O)OH ist;

A eine Bindung, -OC(=O)-*,

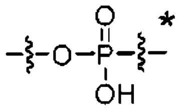 , 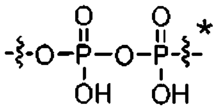 , 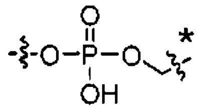 ,

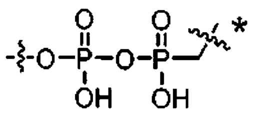 ,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und

D ein Mcl-1-Inhibitor wie definiert in Anspruch 1 ist;

(3)

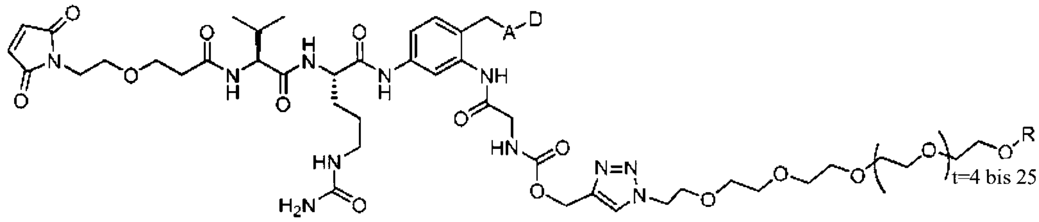,

wobei:

R H, $-CH_3$ oder $-CH_2CH_2C(=O)OH$ ist;
A eine Bindung, -OC(=O)-*,

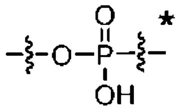, 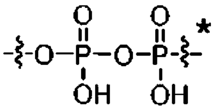, 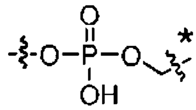,

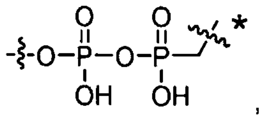,

$-OC(=O)N(CH_3)CH_2CH_2N(CH_3)C(=O)-*$ oder - $OC(=O)N(CH_3)C(R^a)_2C(R^a)_2N(CH_3)C(=O)-*$ ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mcl-1-Inhibitor wie definiert in Anspruch 1 ist;

(4)

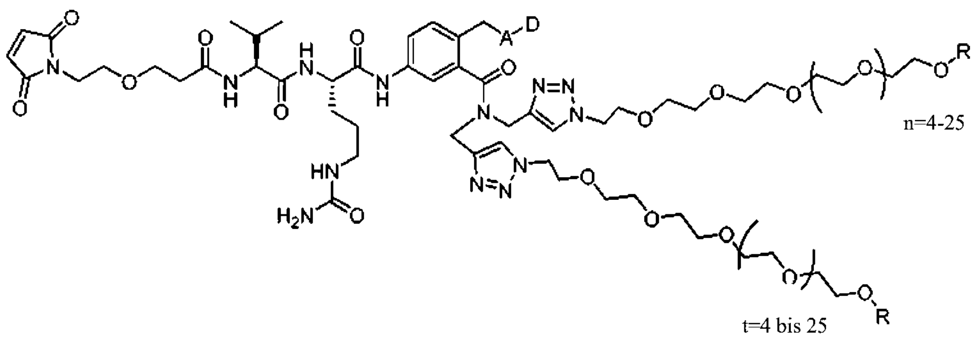,

wobei:

jedes R unabhängig ausgewählt ist aus H, $-CH_3$ und $-CH_2CH_2C(=O)OH$;
A eine Bindung, -OC(=O)-*,

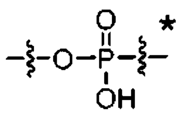, 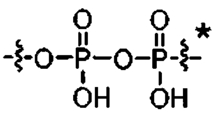, 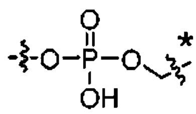,

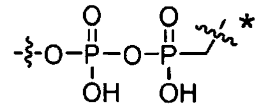

,

$-OC(=O)N(CH_3)CH_2CH_2N(CH_3)C(=O)-*$ oder - $OC(=O)N(CH_3)C(R^a)_2C(R^a)_2N(CH_3)C(=O)-*$ ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und

D ein Mcl-1-Inhibitor wie definiert in Anspruch 1 ist;

(5)

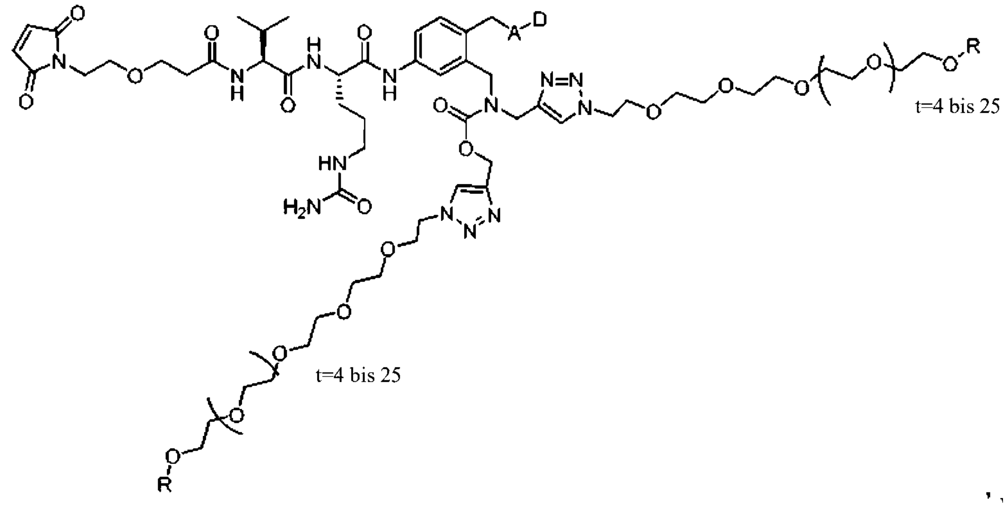

,

wobei:

jedes R unabhängig ausgewählt ist aus H, $-CH_3$ und $-CH_2CH_2C(=O)OH$;

A eine Bindung, -OC(=O)-*,

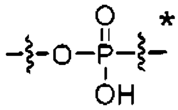 , 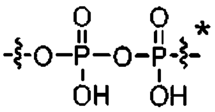 , 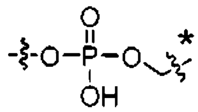 ,

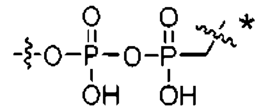

,

$-OC(=O)N(CH_3)CH_2CH_2N(CH_3)C(=O)-*$ oder - $OC(=O)N(CH_3)C(R^a)_2C(R^a)_2N(CH_3)C(=O)-*$ ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und

D ein Mcl-1-Inhibitor wie definiert in Anspruch 1 ist;

(6)

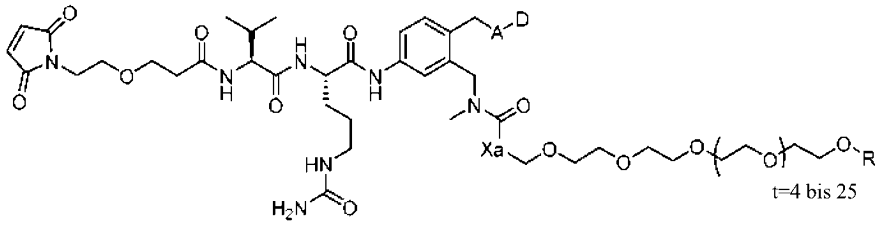

,

wobei:

Xa $-CH_2-$, $-OCH_2-$, $-NHCH_2-$ oder $-NRCH_2-$ ist und jedes R unabhängig H, $-CH_3$ oder $-CH_2CH_2C(=O)OH$ ist;
A eine Bindung, -OC(=O)-*,

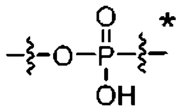 , 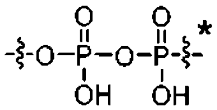 , 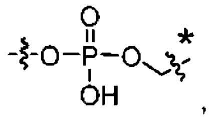 ,

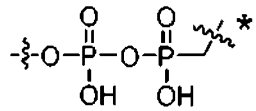 ,

$-OC(=O)N(CH_3)CH_2CH_2N(CH_3)C(=O)-$* oder $-OC(=O)N(CH_3)C(R^a)_2C(R^a)_2N(CH_3)C(=O)-$* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mcl-1-Inhibitor wie definiert in Anspruch 1 ist;

(7)

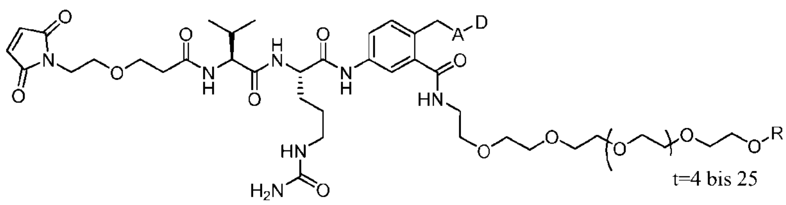

,

wobei:

R H, $-CH_3$ oder $-CH_2CH_2C(=O)OH$ ist;
A eine Bindung, -OC(=O)-*,

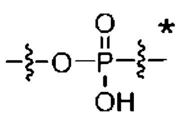 , 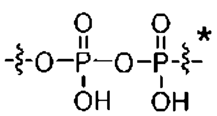 , 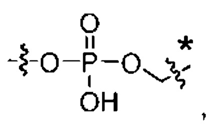 ,

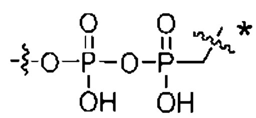 ,

$-OC(=O)N(CH_3)CH_2CH_2N(CH_3)C(=O)-$* oder $-OC(=O)N(CH_3)C(R^a)_2C(R^a)_2N(CH_3)C(=O)-$* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und

D ein Mcl-1-Inhibitor wie definiert in Anspruch 1 ist;

(8)

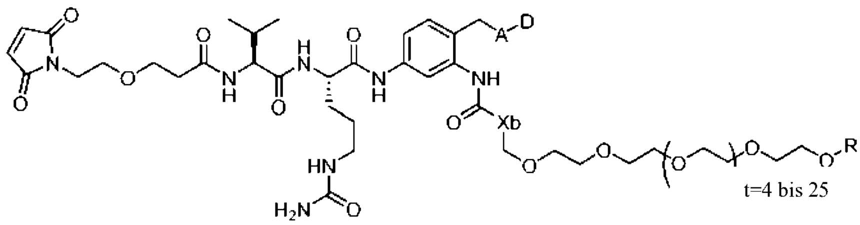

,

wobei:

Xb $-CH_2-$, $-OCH_2-$, $-NHCH_2-$ oder $-NRCH_2-$ ist und jedes R unabhängig H, $-CH_3$ oder - $CH_2CH_2C(=O)OH$ ist;
A eine Bindung, -OC(=O)-*,

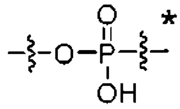 , 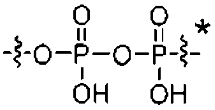 , 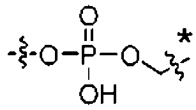 ,

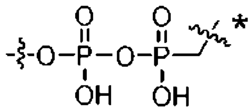 ,

$-OC(=O)N(CH_3)CH_2CH_2N(CH_3)C(=O)-$* oder - $OC(=O)N(CH_3)C(R^a)_2C(R^a)_2N(CH_3)C(=O)-$* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mcl-1-Inhibitor wie definiert in Anspruch 1 ist;

(9)

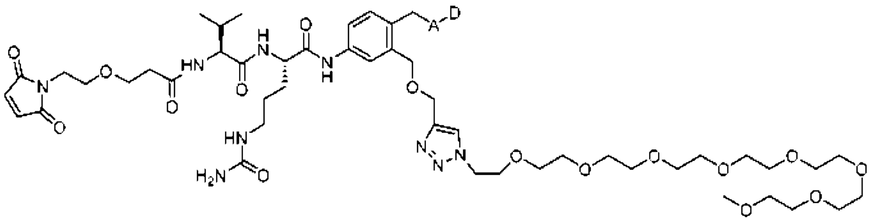

,

, wobei:

A eine Bindung, -OC(=O)-*,

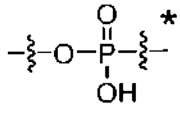 , 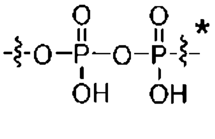 , 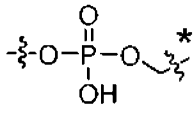 ,

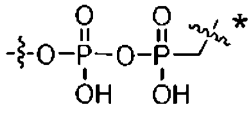 ,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mcl-1-Inhibitor wie definiert in Anspruch 1 ist;

(10)

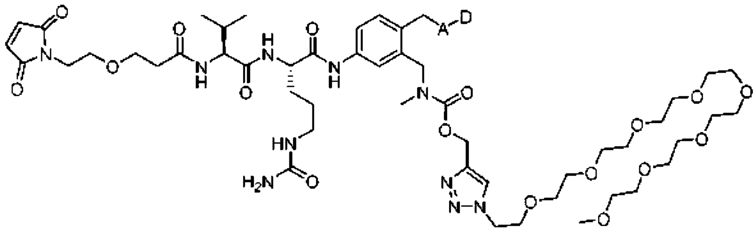

,

wobei:

A eine Bindung, -OC(=O)-*,

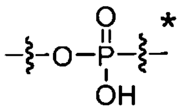 , 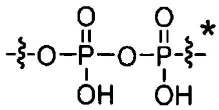 , 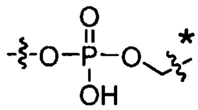 ,

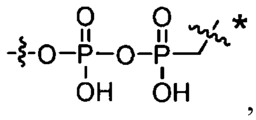 ,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mc1-1-Inhibitor wie definiert in Anspruch 1 ist;

(11)

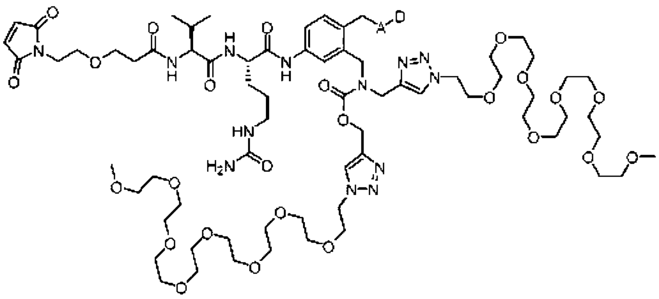

,

wobei:

A eine Bindung, -OC(=O)-*,

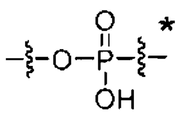 , 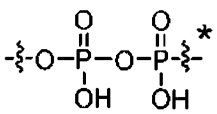 , 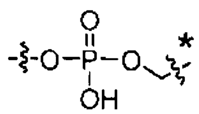 ,

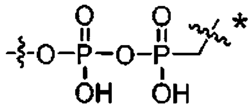

,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mc1-1-Inhibitor wie definiert in Anspruch 1 ist;

(12)

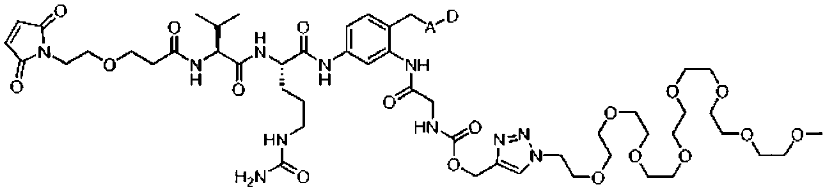

,

wobei:

A eine Bindung, -OC(=O)-*,

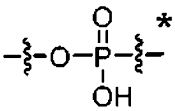

,

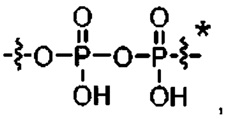

,

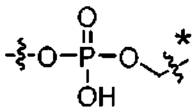

,

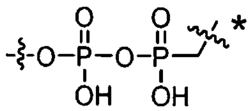

,

-OC(=O)N($CH_3$)$CH_2CH_2$N($CH_3$)C(=O)-* oder-OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mc1-1-Inhibitor wie definiert in Anspruch 1 ist;

(13)

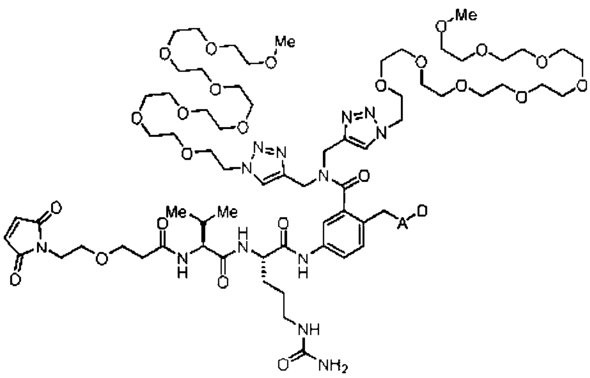

,

wobei:

A eine Bindung, -OC(=O)-*,

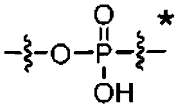 , 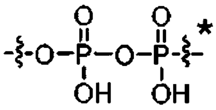 , 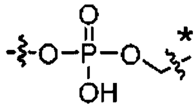 ,

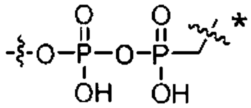 ,

-OC(=O)N($CH_3$)$CH_2CH_2$N($CH_3$)C(=O)-* oder-OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mc1-1-Inhibitor wie definiert in Anspruch 1 ist;

(14)

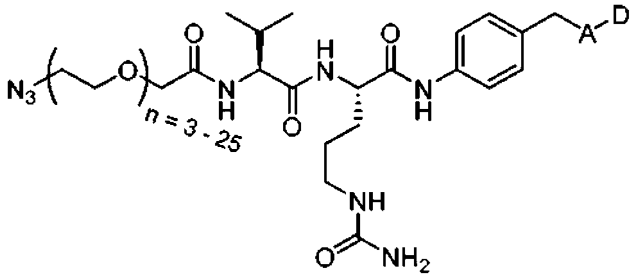 ,

wobei:

A eine Bindung, -OC(=O)-*,

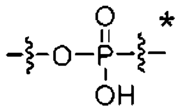 , 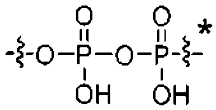 , 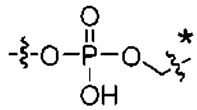 ,

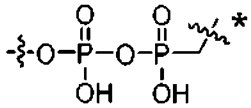 ,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und
D ein Mcl-1-Inhibitor wie definiert in Anspruch 1 ist;

(15)

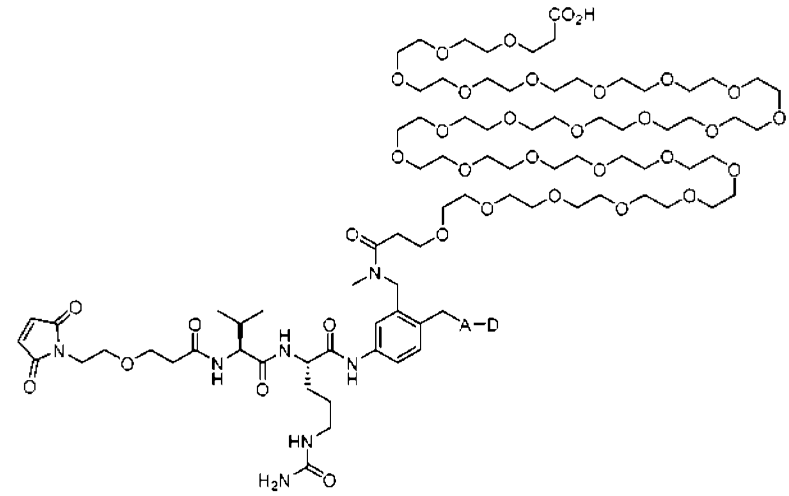

,

wobei:

A eine Bindung, -OC(=O)-*,

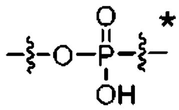 , 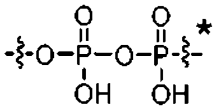 , 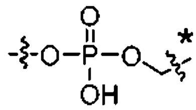 ,

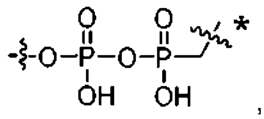 ,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und D ein Mcl-1-Inhibitor wie definiert in Anspruch 1 ist; oder

(16)

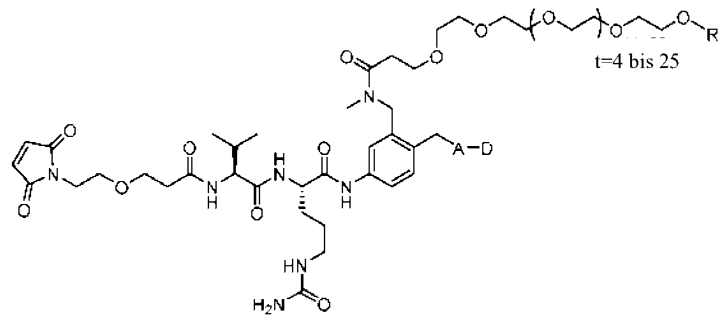

,

wobei:

jedes R unabhängig H, $-CH_3$ oder $-CH_2CH_2C(=O)OH$ ist;
A eine Bindung, -OC(=O)-*,

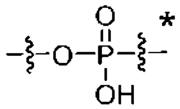, 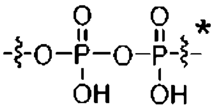, 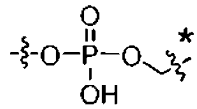,

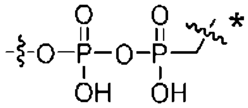,

-OC(=O)N($CH_3$)$CH_2CH_2$N($CH_3$)C(=O)-* oder - OC(=O)N($CH_3$)C($R^a$)$_2$C($R^a$)$_2$N($CH_3$)C(=O)-* ist, wobei jedes $R^a$ unabhängig ausgewählt ist aus H, $C_1$-$C_6$-Alkyl und $C_3$-$C_8$-Cycloalkyl und das * von A den Anknüpfungspunkt an D angibt; und

D ein Mcl-1-Inhibitor wie definiert in Anspruch 1 ist.

**9.** Antikärper-Wirkstoff-Konjugat nach einem der Ansprüche 1 bis 8, wobei A eine Bindung ist.

**10.** Antikärper-Wirkstoff-Konjugat nach einem der Ansprüche 1-9, wobei R -$CH_3$ ist.

**11.** Antikärper-Wirkstoff-Konjugat nach einem der Ansprüche 1 bis 10, wobei -(L-D) aus einer Verbindung ausgewählt aus Tabelle A oder einem Enantiomer, Diastereoisomer, Atropisomer, deuteriertem Derivat und/oder pharmazeutisch verträglichen Salz davon gebildet ist.

**12.** Antikörper-Wirkstoff- Konjugat nach Anspruch 11, wobei das -(L-D) die folgende Verbindung umfasst oder daraus gebildet ist:

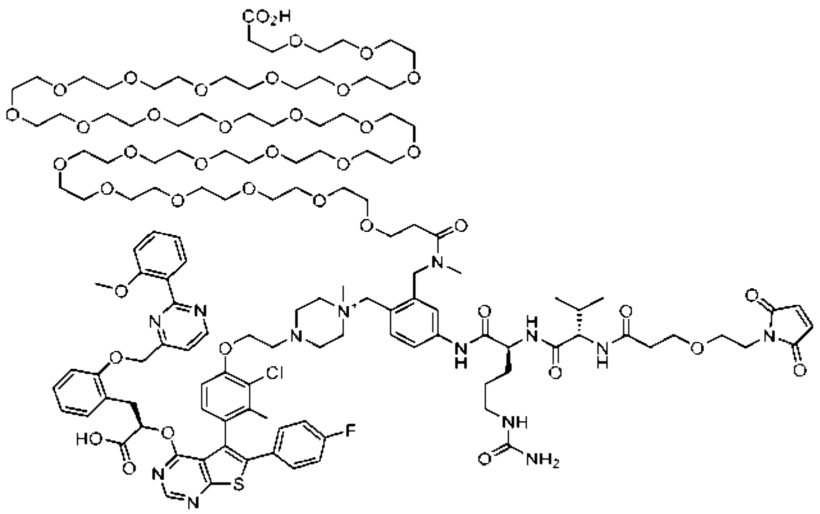

L11-P1,

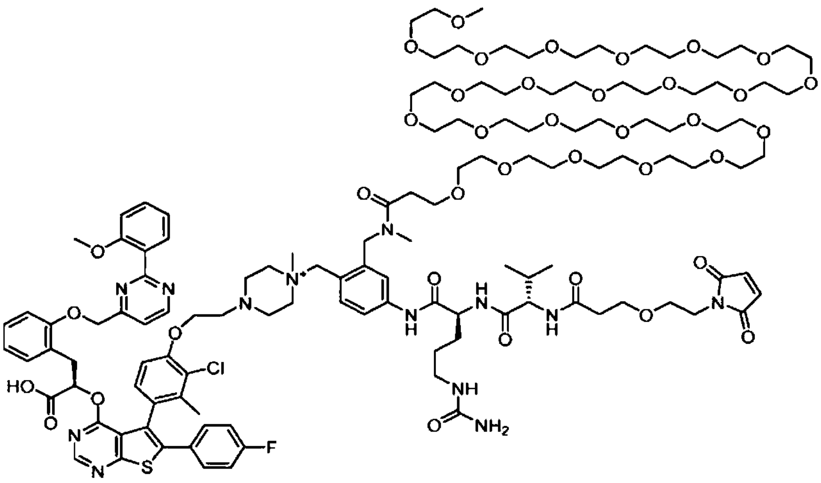

L30-P1,

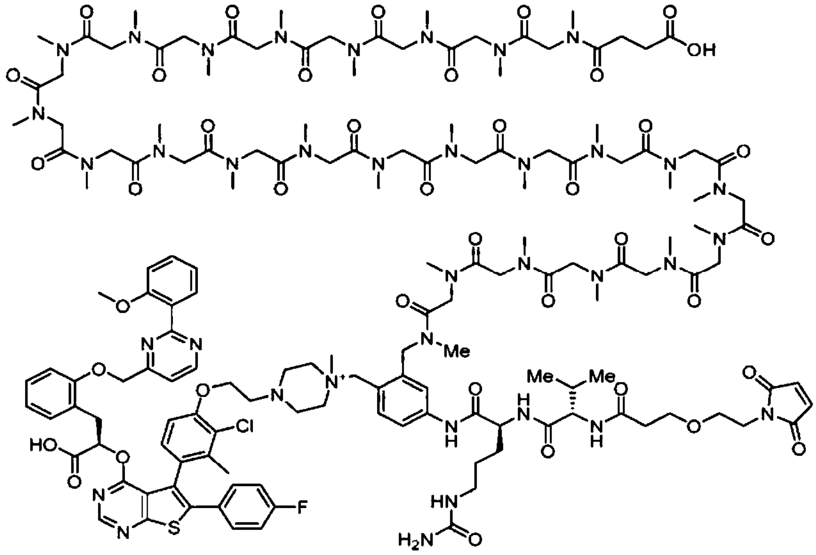

L37-P1,

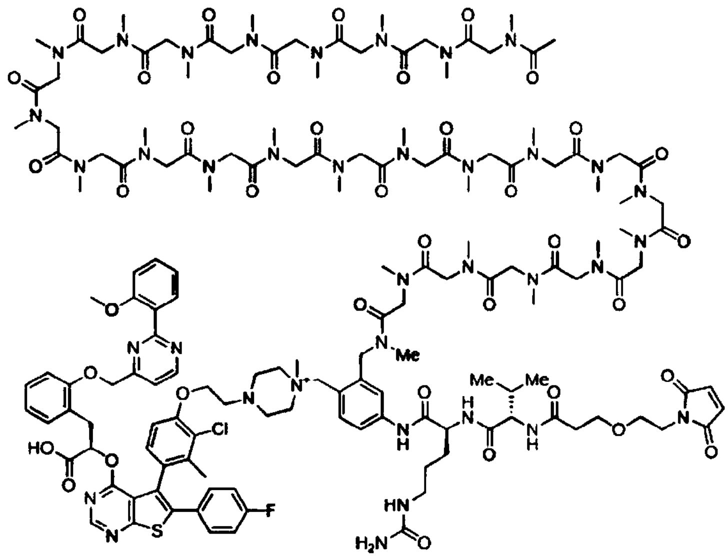

L40-P1

oder

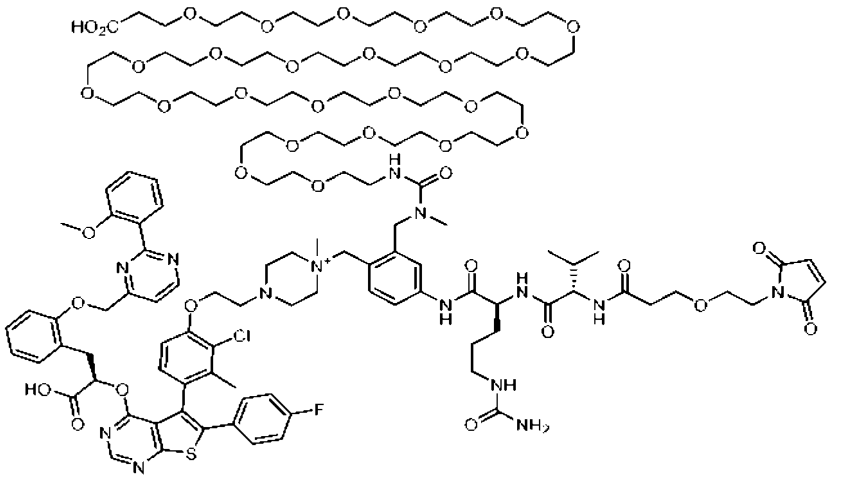

L42-P1.

**13.** Antikärper-Wirkstoff-Konjugat nach einem der Ansprüche 1-11, wobei

(1) der Anti-CD74-Antikörper einen Anti-CD74-Antikörper oder ein Antigenbindungsfragment umfasst, das drei Schwerketten-CDRs und drei Leichtketten-CDRs wie folgt umfasst:

(i) Schwerketten-CDR1 (HCDR1) bestehend aus SEQ ID NO:1, Schwerketten-CDR2 (HCDR2) bestehend aus SEQ ID NO:2, Schwerketten-CDR3 (HCDR3) bestehend aus SEQ ID NO:3; Leichtketten-CDR1 (LCDR1) bestehend aus SEQ ID NO:16, Leichtketten-CDR2 (LCDR2) bestehend aus SEQ ID NO:70 und Leichtketten-CDR3 (LCDR3) bestehend aus SEQ ID NO:18;
(ii) Schwerketten-CDR1 (HCDR1) bestehend aus SEQ ID NO:4, Schwerketten-CDR2 (HCDR2) bestehend aus SEQ ID NO:2, Schwerketten-CDR3 (HCDR3) bestehend aus SEQ ID NO:3; Leichtketten-CDR1 (LCDR1) bestehend aus SEQ ID NO:16, Leichtketten-CDR2 (LCDR2) bestehend aus SEQ ID NO:70 und Leichtketten-CDR3 (LCDR3) bestehend aus SEQ ID NO:18;
(iii) Schwerketten-CDR1 (HCDR1) bestehend aus SEQ ID NO:5, Schwerketten-CDR2 (HCDR2) bestehend aus SEQ ID NO:6, Schwerketten-CDR3 (HCDR3) bestehend aus SEQ ID NO:3; Leichtketten-CDR1 (LCDR1) bestehend aus SEQ ID NO:19, Leichtketten-CDR2 (LCDR2) bestehend aus SEQ ID NO:20 und Leichtketten-CDR3 (LCDR3) bestehend aus SEQ ID NO:21;
(iv) Schwerketten-CDR1 (HCDR1) bestehend aus SEQ ID NO:7, Schwerketten-CDR2 (HCDR2) bestehend aus SEQ ID NO:8, Schwerketten-CDR3 (HCDR3) bestehend aus SEQ ID NO:9; Leichtketten-CDR1 (LCDR1) bestehend aus SEQ ID NO:22, Leichtketten-CDR2 (LCDR2) bestehend aus SEQ ID NO:20 und Leichtketten-CDR3 (LCDR3) bestehend aus SEQ ID NO:18;
(v) Schwerketten-CDR1 (HCDR1) bestehend aus SEQ ID NO:1, Schwerketten-CDR2 (HCDR2) bestehend aus SEQ ID NO:2, Schwerketten-CDR3 (HCDR3) bestehend aus SEQ ID NO:3; Leichtketten-CDR1 (LCDR1) bestehend aus SEQ ID NO:35, Leichtketten-CDR2 (LCDR2) bestehend aus SEQ ID NO:70 und Leichtketten-CDR3 (LCDR3) bestehend aus SEQ ID NO:18;
(vi) Schwerketten-CDR1 (HCDR1) bestehend aus SEQ ID NO:4, Schwerketten-CDR2 (HCDR2) bestehend aus SEQ ID NO:2, Schwerketten-CDR3 (HCDR3) bestehend aus SEQ ID NO:3; Leichtketten-CDR1 (LCDR1) bestehend aus SEQ ID NO:35, Leichtketten-CDR2 (LCDR2) bestehend aus SEQ ID NO:70 und Leichtketten-CDR3 (LCDR3) bestehend aus SEQ ID NO:18;
(vii) Schwerketten-CDR1 (HCDR1) bestehend aus SEQ ID NO:5, Schwerketten-CDR2 (HCDR2) bestehend aus SEQ ID NO:6, Schwerketten-CDR3 (HCDR3) bestehend aus SEQ ID NO:3; Leichtketten-CDR1 (LCDR1) bestehend aus SEQ ID NO:71, Leichtketten-CDR2 (LCDR2) bestehend aus SEQ ID NO:20 und Leichtketten-CDR3 (LCDR3) bestehend aus SEQ ID NO:21 oder
(viii) Schwerketten-CDR1 (HCDR1) bestehend aus SEQ ID NO:7, Schwerketten-CDR2 (HCDR2) bestehend aus SEQ ID NO:8, Schwerketten-CDR3 (HCDR3) bestehend aus SEQ ID NO:9; Leichtketten-CDR1 (LCDR1) bestehend aus SEQ ID NO:17, Leichtketten-CDR2 (LCDR2) bestehend aus SEQ ID NO:20 und Leichtketten-CDR3 (LCDR3) bestehend aus SEQ ID NO:18;

(2) der Anti-CD74-Antikörper oder das Antigenbindungsfragment davon Folgendes umfasst:

(i) eine variable Schwerkettenregion umfassend die Aminosäuresequenz von SEQ ID NO:10 und eine

variable Leichtkettenregion umfassend die Aminosäuresequenz von SEQ ID NO:23;
(ii) eine variable Schwerkettenregion umfassend die Aminosäuresequenz von SEQ ID NO:10 und eine variable Leichtkettenregion umfassend die Aminosäuresequenz von SEQ ID NO:27;
(iii) eine variable Schwerkettenregion umfassend die Aminosäuresequenz von SEQ ID NO:10 und eine variable Leichtkettenregion umfassend die Aminosäuresequenz von SEQ ID NO:31;
(iv) eine variable Schwerkettenregion umfassend die Aminosäuresequenz von SEQ ID NO:10 und eine variable Leichtkettenregion umfassend die Aminosäuresequenz von SEQ ID NO:36;
(v) eine variable Schwerkettenregion umfassend die Aminosäuresequenz von SEQ ID NO:10 und eine variable Leichtkettenregion umfassend die Aminosäuresequenz von SEQ ID NO:40; oder
(vi) eine variable Schwerkettenregion umfassend die Aminosäuresequenz von SEQ ID NO:10 und eine variable Leichtkettenregion umfassend die Aminosäuresequenz von SEQ ID NO:44; oder

(3) der Anti-CD74-Antikörper Folgendes umfasst:

(a) die Schwerketten-Aminosäuresequenz von SEQ ID NO:12 und die Leichtketten-Aminosäuresequenz von SEQ ID NO:25;
(b) die Schwerketten-Aminosäuresequenz von SEQ ID NO:14 und die Leichtketten-Aminosäuresequenz von SEQ ID NO:25;
(c) die Schwerketten-Aminosäuresequenz von SEQ ID NO: 15 und die Leichtketten-Aminosäuresequenz von SEQ ID NO:25;
(d) die Schwerketten-Aminosäuresequenz von SEQ ID NO:12 und die Leichtketten-Aminosäuresequenz von SEQ ID NO:29;
(e) die Schwerketten-Aminosäuresequenz von SEQ ID NO:14 und die Leichtketten-Aminosäuresequenz von SEQ ID NO:29;
(f) die Schwerketten-Aminosäuresequenz von SEQ ID NO:15 und die Leichtketten-Aminosäuresequenz von SEQ ID NO:29;
(g) die Schwerketten-Aminosäuresequenz von SEQ ID NO:12 und die Leichtketten-Aminosäuresequenz von SEQ ID NO:33;
(h) die Schwerketten-Aminosäuresequenz von SEQ ID NO:14 und die Leichtketten-Aminosäuresequenz von SEQ ID NO:33;
(i) die Schwerketten-Aminosäuresequenz von SEQ ID NO:15 und die Leichtketten-Aminosäuresequenz von SEQ ID NO:33;
(j) die Schwerketten-Aminosäuresequenz von SEQ ID NO:12 und die Leichtketten-Aminosäuresequenz von SEQ ID NO:38;
(k) die Schwerketten-Aminosäuresequenz von SEQ ID NO:14 und die Leichtketten-Aminosäuresequenz von SEQ ID NO:38;
(l) die Schwerketten-Aminosäuresequenz von SEQ ID NO:15 und die Leichtketten-Aminosäuresequenz von SEQ ID NO:38;
(m) die Schwerketten-Aminosäuresequenz von SEQ ID NO:12 und die Leichtketten-Aminosäuresequenz von SEQ ID NO:42;
(n) die Schwerketten-Aminosäuresequenz von SEQ ID NO:14 und die Leichtketten-Aminosäuresequenz von SEQ ID NO:42;
(o) die Schwerketten-Aminosäuresequenz von SEQ ID NO:15 und die Leichtketten-Aminosäuresequenz von SEQ ID NO:42;
(p) die Schwerketten-Aminosäuresequenz von SEQ ID NO:12 und die Leichtketten-Aminosäuresequenz von SEQ ID NO:46;
(q) die Schwerketten-Aminosäuresequenz von SEQ ID NO:14 und die Leichtketten-Aminosäuresequenz von SEQ ID NO:46; oder
(r) die Schwerketten-Aminosäuresequenz von SEQ ID NO:15 und die Leichtketten-Aminosäuresequenz von SEQ ID NO:46.

**14.** Antikärper-Wirkstoff-Konjugat nach einem der Ansprüche 1-13, wobei das Ab ein stiller Fc-Antikörper ist.

**15.** Zusammensetzung, umfassend mehrere Kopien des Antikörper-Wirkstoff-Konjugats nach einem der Ansprüche 1 bis 14, wobei der Durchschnitt *p* der Antikörper-Wirkstoff-Konjugate in der Zusammensetzung von etwa 2 bis etwa 16, z. B. etwa 2 bis etwa 8, z. B. etwa 2 bis etwa 4 ist.

**16.** Pharmazeutische Zusammensetzung, umfassend das Antikörper-Wirkstoff-Konjugat nach einem der Ansprüche 1

bis 14 oder die Zusammensetzung nach Anspruch 15 und einen pharmazeutisch verträglichen Träger.

**17.** Antikärper-Wirkstoff-Konjugat nach einem der Ansprüche 1 bis 14, die Zusammensetzung nach Anspruch 15 oder die pharmazeutische Zusammensetzung nach Anspruch 16 zur Verwendung beim Behandeln eines Subjekts, das einen Krebs aufweist, wobei optional

(1) der Krebs CD74 exprimiert; oder
(2) der Krebs ein Tumor oder ein hämatologischer Krebs ist, bevorzugt der Krebs ein Brustkrebs, multiples Myelom, Plasmazellmyelom, Leukämie, Lymphom, Magenkrebs, akute myeloische Leukämie, Blasenkrebs, Hirnkrebs, Knochenmarkkrebs, Gebärmutterhalskrebs, chronische lymphatische Leukämie, Darmkrebs, Speiseröhrenkrebs, Leberzellkrebs, lymphoblastische Leukämie, follikuläres Lymphom, lymphatische Malignitäten mit T-Zell- oder B-Zell-Ursprung, Melanom, myelogene Leukämie, Myelom, Mundkrebs, Eierstockkrebs, nichtkleinzelliger Lungenkrebs, chronische lymphatische Leukämie, Prostatakrebs, kleinzelliger Lungenkrebs oder Milzkrebs ist.

**18.** Antikärper-Wirkstoff-Konjugat nach einem der Ansprüche 1 bis 14, die Zusammensetzung nach Anspruch 15 oder die pharmazeutische Zusammensetzung nach Anspruch 16 zur Verwendung beim Reduzieren oder Inhibieren des Wachstums eines Tumors bei einem Subjekt, wobei optional

(1) der Tumor CD74 exprimiert; oder
(2) der Tumor ein Brustkrebs, Magenkrebs, Blasenkrebs, Hirnkrebs, Gebärmutterhalskrebs, Darmkrebs, Speiseröhrenkrebs, Leberzellkrebs, Melanom, Mundkrebs, Eierstockkrebs, nicht-kleinzelliger Lungenkrebs, Prostatakrebs, kleinzelliger Lungenkrebs oder Milzkrebs ist.

**19.** Antikärper-Wirkstoff-Konjugat nach einem der Ansprüche 1 bis 14, die Zusammensetzung nach Anspruch 15 oder die pharmazeutische Zusammensetzung nach Anspruch 16 zur Verwendung nach Anspruch 18, wobei Verabreichung des Antikörper-Wirkstoff-Konjugats, der Zusammensetzung oder der pharmazeutischen Zusammensetzung das Wachstum des Tumors um zumindest etwa 10 %, zumindest etwa 20 %, zumindest etwa 30 %, zumindest etwa 40 %, zumindest etwa 50 %, zumindest etwa 60 %, zumindest etwa 70 %, zumindest etwa 80 %, zumindest etwa 90 %, zumindest etwa 95 % oder zumindest etwa 99 % reduziert oder inhibiert.

**20.** Antikärper-Wirkstoff-Konjugat nach einem der Ansprüche 1 bis 14, die Zusammensetzung nach Anspruch 15 oder die pharmazeutische Zusammensetzung nach Anspruch 16 zur Verwendung beim Reduzieren oder Verlangsamen der Expansion einer Krebszellpopulation bei einem Subjekt, wobei optional

(1) die Krebszellpopulation CD74 exprimiert; oder
(2) die Krebszellpopulation von einem Tumor oder einem hämatologischen Krebs ist, bevorzugt die Krebszellpopulation von einem Brustkrebs, multiplem Myelom, Plasmazellmyelom, Leukämie, Lymphom, Magenkrebs, akuter myeloischer Leukämie, Blasenkrebs, Hirnkrebs, Knochenmarkkrebs, Gebärmutterhalskrebs, chronischer lymphatischer Leukämie, Darmkrebs, Speiseröhrenkrebs, Leberzellkrebs, lymphoblastischer Leukämie, follikulärem Lymphom, lymphatischen Malignitäten mit T-Zell- oder B-Zell-Ursprung, Melanom, myelogener Leukämie, Myelom, Mundkrebs, Eierstockkrebs, nicht-kleinzelligem Lungenkrebs, chronischer lymphatischer Leukämie, Prostatakrebs, kleinzelligem Lungenkrebs oder Milzkrebs ist.

**21.** Antikärper-Wirkstoff-Konjugat nach einem der Ansprüche 1 bis 14, die Zusammensetzung nach Anspruch 15 oder die pharmazeutische Zusammensetzung nach Anspruch 16 zur Verwendung nach Anspruch 20, wobei Verabreichung des Antikörper-Wirkstoff-Konjugats, der Zusammensetzung oder der pharmazeutischen Zusammensetzung die Krebszellpopulation reduziert oder die Expansion der Krebszellpopulation um zumindest etwa 10 %, zumindest etwa 20 %, zumindest etwa 30 %, zumindest etwa 40 %, zumindest etwa 50 %, zumindest etwa 60 %, zumindest etwa 70 %, zumindest etwa 80 %, zumindest etwa 90 %, zumindest etwa 95 % oder zumindest etwa 99 % verlangsamt.

**22.** Verfahren zum Bestimmen, ob ein Subjekt, das einen Krebs aufweist oder von dem vermutet wird, dass es einen Krebs aufweist, auf Behandlung mit dem Antikörper-Wirkstoff- Konjugat nach einem der Ansprüche 1 bis 14, der Zusammensetzung nach Anspruch 15 oder der pharmazeutischen Zusammensetzung nach Anspruch 16 anspricht, umfassend Bereitstellen einer biologischen Probe von dem Subjekt; Inkontaktbringen der Probe mit dem Antikörper-Wirkstoff-Konjugat; und Detektieren von Bindung des Antikörper-Wirkstoff-Konjugats an Krebszellen in der Probe; wobei optional

(1) die Krebszellen in der Probe CD74 exprimieren;
(2) der Krebs CD74 exprimiert; oder
(3) der Krebs ein Tumor oder ein hämatologischer Krebs ist, bevorzugt der Krebs ein Brustkrebs, multiples Myelom, Plasmazellmyelom, Leukämie, Lymphom, Magenkrebs, akute myeloische Leukämie, Blasenkrebs, Hirnkrebs, Knochenmarkkrebs, Gebärmutterhalskrebs, chronische lymphatische Leukämie, Darmkrebs, Speiseröhrenkrebs, Leberzellkrebs, lymphoblastische Leukämie, follikuläres Lymphom, lymphatische Malignitäten mit T-Zell- oder B-Zell-Ursprung, Melanom, myelogene Leukämie, Myelom, Mundkrebs, Eierstockkrebs, nichtkleinzelliger Lungenkrebs, chronische lymphatische Leukämie, Prostatakrebs, kleinzelliger Lungenkrebs oder Milzkrebs ist.

**23.** Verfahren nach Anspruch 22, wobei die Probe eine Gewebebiopsieprobe, eine Blutprobe oder eine Knochenmarkprobe ist.

**24.** Verfahren zum Produzieren des Antikörper-Wirkstoff-Konjugats nach einem der Ansprüche 1 bis 14, umfassend Reagieren eines Antikörpers oder Antigenbindungsfragments mit einem spaltbaren Linker, der mit einem MCL1-Inhibitor verbunden ist, unter Bedingungen, die Konjugation ermöglichen.

**25.** Antikärper-Wirkstoff-Konjugat nach einem der Ansprüche 1 bis 14, die Zusammensetzung nach Anspruch 15 oder die pharmazeutische Zusammensetzung nach Anspruch 16 zur Verwendung nach einem der Ansprüche 17 bis 21, ferner umfassend Verabreichen von zumindest einem zusätzlichen therapeutischen Mittel an das Subjekt, das dessen bedarf, wobei bevorzugt das eine zusätzliche therapeutische Mittel ein Bcl-2-Inhibitor ist, bevorzugter das eine zusätzliche therapeutische Mittel Venetoclax, Verbindung A1 oder Verbindung A2 ist.

## Revendications

**1.** Conjugué anticorps-médicament de Formule (1) :

$$Ab\text{-}(L\text{-}D)_p \qquad (1)$$

où :

Ab est un anticorps anti-CD74 ou un fragment de liaison à l'antigène de celui-ci ;
*p* est un nombre entier de 1 à 16 ; et
-(L-D) répond à la formule (C) :

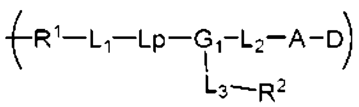
(C),

où :

$R^1$ est un groupe de fixation ;
$L_1$ est un espaceur pontant ;
$L_p$ est un groupe peptidique comprenant 1 à 6 acides aminés ;
D est un inhibiteur de Mcl-1, où

(1) D comprend :

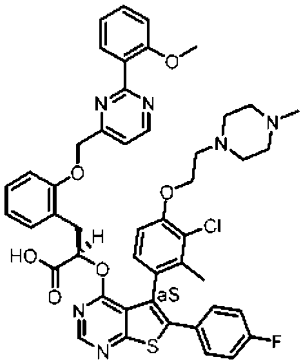

, :

ou un énantiomère, un diastéréoisomère, un atropisomère, un dérivé deutéré et/ou un sel acceptable pharmaceutiquement de l'un quelconque de ce qui précède ;
(2) D comprend :

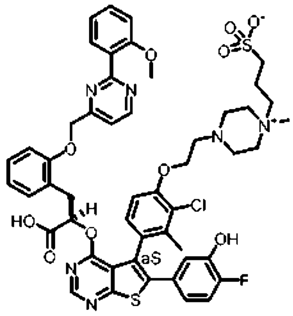

,

ou un énantiomère, un diastéréoisomère, un atropisomère, un dérivé deutéré et/ou un sel acceptable pharmaceutiquement de l'un quelconque de ce qui précède ;
(3) D comprend :

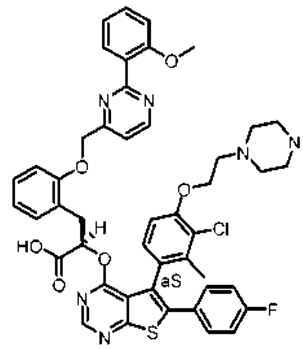

,

ou un énantiomère, un diastéréoisomère, un atropisomère, un dérivé deutéré et/ou un sel acceptable pharmaceutiquement de l'un quelconque de ce qui précède ;
(4) D comprend :

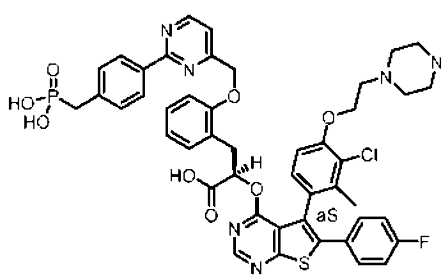

,

ou un énantiomère, un diastéréoisomère, un atropisomère, un dérivé deutéré et/ou un sel acceptable pharmaceutiquement de l'un quelconque de ce qui précède ;
(5) D comprend :

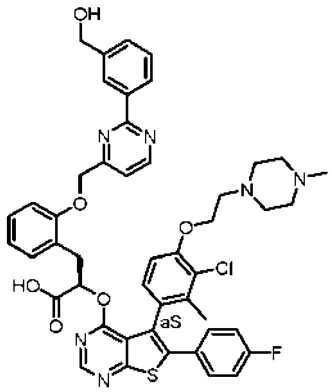

,

ou un énantiomère, un diastéréoisomère, un atropisomère, un dérivé deutéré et/ou un sel acceptable pharmaceutiquement de l'un quelconque de ce qui précède ;
(6) D comprend :

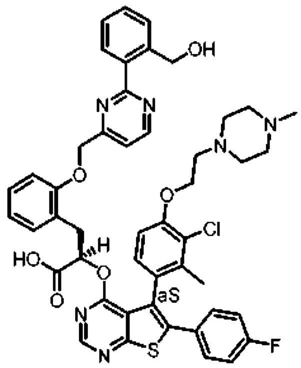

,

ou un énantiomère, un diastéréoisomère, un atropisomère, un dérivé deutéré et/ou un sel acceptable pharmaceutiquement de l'un quelconque de ce qui précède ;
(7) D comprend :

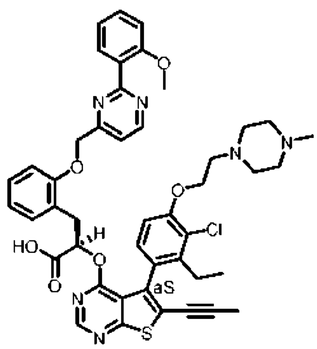

,

ou un énantiomère, un diastéréoisomère, un atropisomère, un dérivé deutéré et/ou un sel acceptable pharmaceutiquement de l'un quelconque de ce qui précède ;
(8) D comprend :

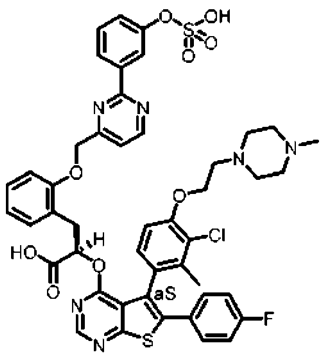

,

ou un énantiomère, un diastéréoisomère, un atropisomère, un dérivé deutéré et/ou un sel acceptable pharmaceutiquement de l'un quelconque de ce qui précède ;
(9) D comprend :

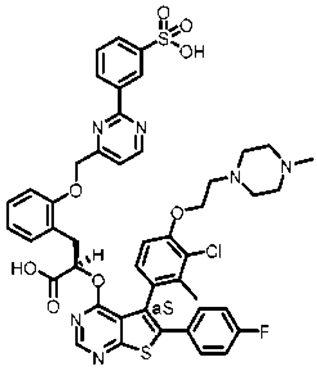

,

ou un énantiomère, un diastéréoisomère, un atropisomère, un dérivé deutéré et/ou un sel acceptable pharmaceutiquement de l'un quelconque de ce qui précède ;
(10) D comprend :

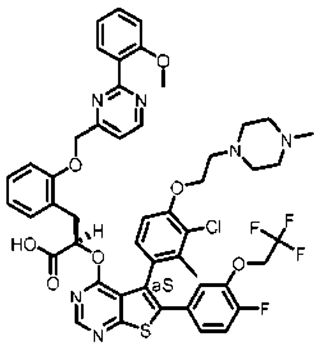

,

ou un énantiomère, un diastéréoisomère, un atropisomère, un dérivé deutéré et/ou un sel acceptable pharmaceutiquement de l'un quelconque de ce qui précède ;
(11) D comprend :

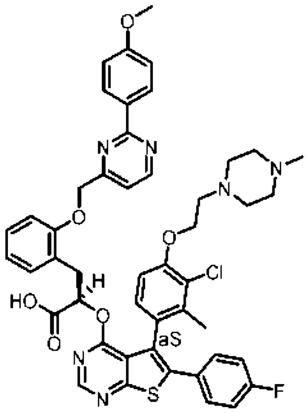

,

ou un énantiomère, un diastéréoisomère, un atropisomère, un dérivé deutéré et/ou un sel acceptable pharmaceutiquement de l'un quelconque de ce qui précède ;

(12) D comprend :

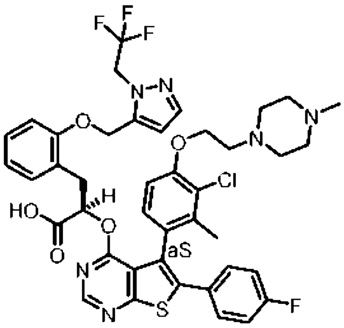

,

ou un énantiomère, un diastéréoisomère, un atropisomère, un dérivé deutéré et/ou un sel acceptable pharmaceutiquement de l'un quelconque de ce qui précède ; ou

(13) D comprend :

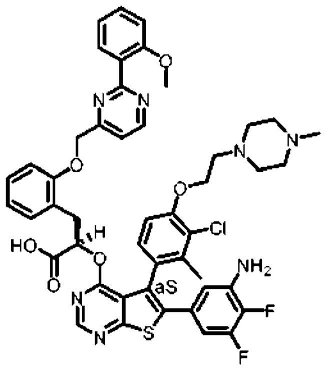

,

ou un énantiomère, un diastéréoisomère, un atropisomère, un dérivé deutéré et/ou un sel acceptable pharmaceutiquement de l'un quelconque de ce qui précède ;

(14) D comprend :

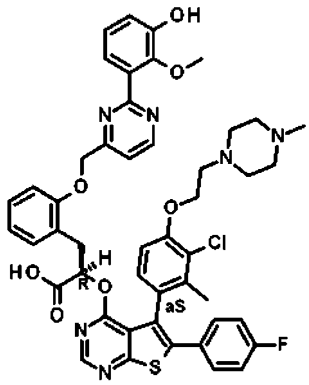

,

ou un énantiomère, un diastéréoisomère, un atropisomère, un dérivé deutéré et/ou un sel acceptable pharmaceutiquement de l'un quelconque de ce qui précède ;
(15) D comprend :

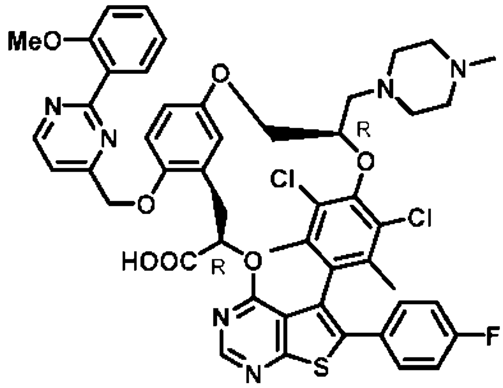

,

ou un énantiomère, un diastéréoisomère, un atropisomère, un dérivé deutéré et/ou un sel acceptable pharmaceutiquement de l'un quelconque de ce qui précède ;
(16) D comprend :

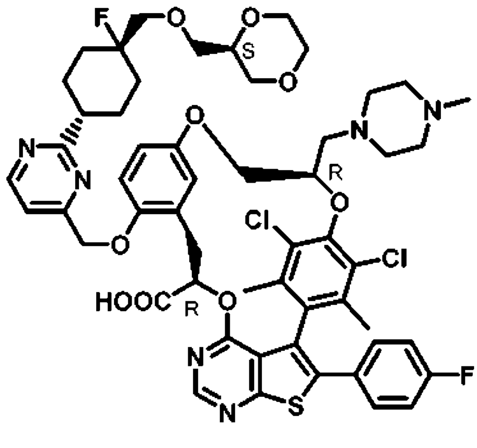

,

ou un énantiomère, un diastéréoisomère, un atropisomère, un dérivé deutéré et/ou un sel acceptable pharmaceutiquement de l'un quelconque de ce qui précède ; ou
(17) D comprend :

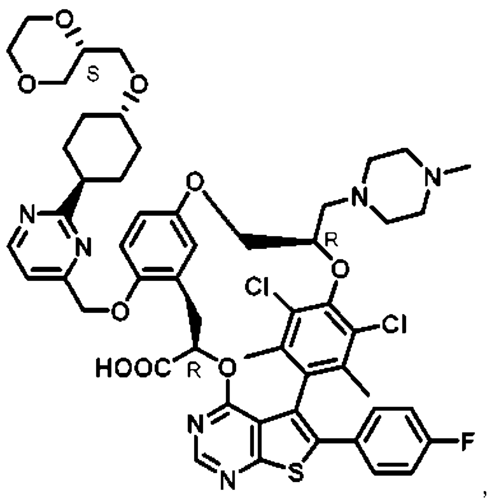

,

ou un énantiomère, un diastéréoisomère, un atropisomère, un dérivé deutéré et/ou un sel acceptable pharmaceutiquement de l'un quelconque de ce qui précède ;

$G_1$-$L_2$-A est un espaceur auto-immolant ;
$L_2$ est une liaison, un méthylène, un néopentylène ou un alcénylène en $C_2$-$C_3$ ;
A est une liaison, -OC(=O)-*

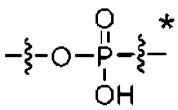

,

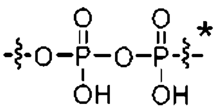

,

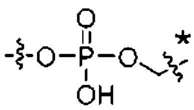

,

,

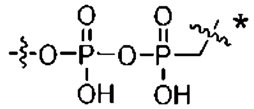

,-

$-OC(=O)N(CH_3)CH_2CH_2N(CH_3)C(=O)-*$ ou $-OC(=O)N(CH_3)C(R^a)_2C(R^a)_2N(CH_3)C(=O)-*$,
où chaque $R^a$ est indépendamment choisi parmi H, un alkyle en $C_1$-$C_6$ et un cycloalkyle en $C_3$-$C_8$ et * de A indique le point de fixation à D ;
$L_3$ est un fragment espaceur ; et
$R^2$ est un fragment hydrophile.

**2.** Conjugué anticorps-médicament de la revendication 1, ou sel acceptable pharmaceutiquement de celui-ci, dans lequel -(L-D) répond à la Formule (D) :

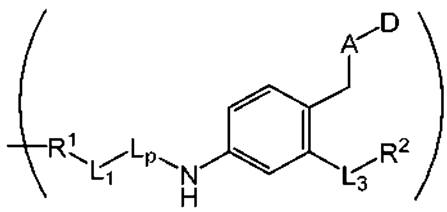

(D)

où :

$R^1$ est un groupe de fixation ;
$L_1$ est un espaceur pontant ;
Lp est un groupe peptidique comprenant 1 à 6 acides aminés ;
A est une liaison, -OC(=O)-*,

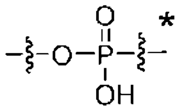, 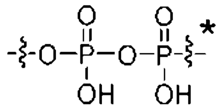, 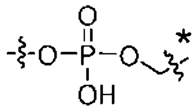, ,

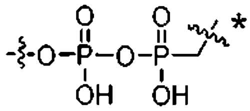,

$-OC(=O)N(CH_3)CH_2CH_2N(CH_3)C(=O)-*$ ou $-OC(=O)N(CH_3)C(R^a)_2C(R^a)_2N(CH_3)C(=O)-*$,
où chaque $R^a$ est indépendamment choisi parmi H, un alkyle en $C_1$-$C_6$ et un cycloalkyle en $C_3$-$C_8$ et * de A indique le point de fixation à D ;
$L_3$ est un fragment espaceur ; et
$R^2$ est un fragment hydrophile.

**3.** Conjugué anticorps-médicament de la revendication 1 ou 2, dans lequel

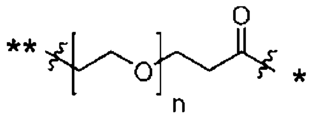, 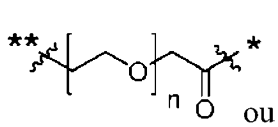 ou

(i) $L_1$ comprend :

*-CH(OH)CH(OH)CH(OH)CH(OH)-**,

où chaque n est un nombre entier de 1 à 12, où * de $L_1$ indique le point de fixation directe ou indirecte à Lp, et ** de $L_1$ indique le point de fixation directe ou indirecte à $R^1$ ;
(ii) $L_1$ est

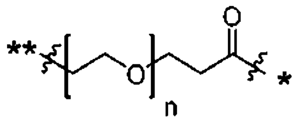,

et n est un nombre entier de 1 à 12 ou n vaut 1 ou n vaut 12, où * de $L_1$ indique le point de fixation directe ou indirecte à Lp, et ** de $L_1$ indique le point de fixation directe ou indirecte à $R^1$ ;
(iii) $L_1$ est

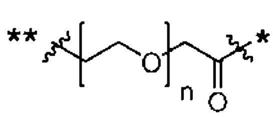,

et n est un nombre entier de 1 à 12, où * de $L_1$ indique le point de fixation directe ou indirecte à Lp, et ** de $L_1$ indique le point de fixation directe ou indirecte à $R^1$ ;
(iv) $L_1$ comprend

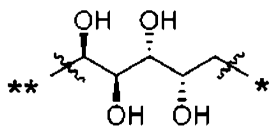,

où * de $L_1$ indique le point de fixation directe ou indirecte à Lp, et ** de $L_1$ indique le point de fixation directe ou indirecte à $R^1$ ; ou

(v) $L_1$ est un espaceur pontant comprenant :

$*-C(=O)(CH_2)_mO(CH_2)_m-**$ ; $*-C(=O)((CH_2)_mO)t(CH_2)_n-**$ ; $*-C(=O)(CH_2)_m-**$ ;

$*-C(=O)NH((CH_2)_mO)t(CH_2)_n-**$;

$*-C(=O)O(CH_2)_mSSC(R^3)_2(CH_2)_mC(=O)NR^3(CH_2)_mNR^3C(=O)(CH_2)_m-**$ ;

$*-C(=O)O(CH_2)_mC(=O)NH(CH_2)_m-**$ ; $*-C(=O)(CH_2)_mNH(CH_2)_m-**$ ;

$*-C(=O)(CH_2)_mNH(CH_2)_nC(=O)-**$ ; $*-C(=O)(CH_2)_mX_1(CH_2)_m-**$ ;

$*-C(=0)((CH_2)_mO)_t(CH_2)_nX_1(CH_2)_n-**$; $*-C(=O)(CH_2)_mNHC(=O)(CH_2)_n-**$;

$*-C(=O)((CH_2)_mO)_t(CH_2)_nNHC(=O)(CH_2)_n-**$ ;

$*-C(=O)(CH_2)_mNHC(=O)(CH_2)_nX_1(CH_2)_n-**$ ;

$*-C(=O)((CH_2)_mO)_t(CH_2)_nNHC(=O)(CH2)_nX_1(CH_2)_n-**$;

$*-C(=O)((CH_2)_mO)_t(CH_2)_nC(=O)NH(CH_2)_m-**$ ; $*-C(=O)(CH_2)_mC(R^3)_2-**$

ou

$*-C(=O)(CH_2)_mC(=O)NH(CH_2)_m-**$,

où * de $L_1$ indique le point de fixation directe ou indirecte à Lp, et ** de $L_1$ indique le point de fixation directe ou indirecte à $R^1$;

$X_1$ est

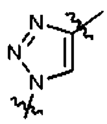 , 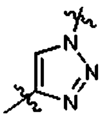 , 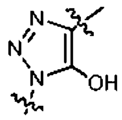 ou 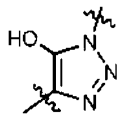 ;

et
chaque m est indépendamment choisi parmi 1, 2, 3, 4, 5, 6, 7, 8, 9 et 10 ;
chaque n est indépendamment choisi parmi 1, 2, 3, 4, 5, 6, 7, 8, 9 et 10 ; et
chaque t est indépendamment choisi parmi 1, 2, 3, 4, 5, 6, 7, 8, 9, 10, 11, 12, 13, 14, 15, 16, 17, 18, 19, 20, 21, 22, 23, 24, 25, 26, 27, 28, 29 et 30 ;
et chaque $R^3$ est indépendamment choisi parmi H et un alkyle en $C_1$-$C_6$.

**4.** Conjugué anticorps-médicament de l'une quelconque des revendications 1 à 3, dans lequel

(1) $R^2$ est un fragment hydrophile comprenant du polyéthylène glycol, du polyalkylène glycol, un polyol, une polysarcosine, un sucre, un oligosaccharide, un polypeptide ou un alkyle en $C_2$-$C_6$ substitué par 1 à 3 groupes

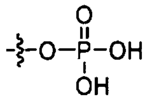 ou 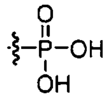 ;

(2) $R^2$ est

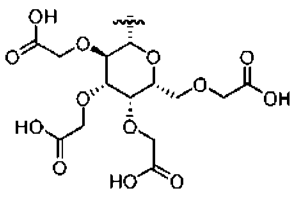, 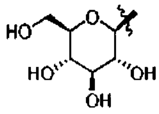, 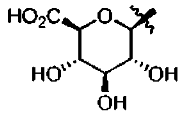, 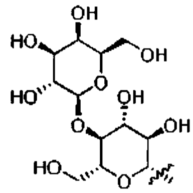, 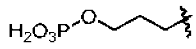,

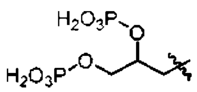, 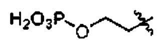, 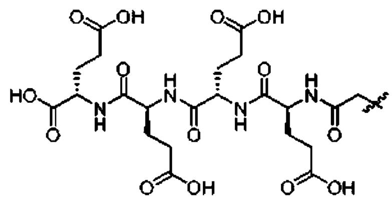,

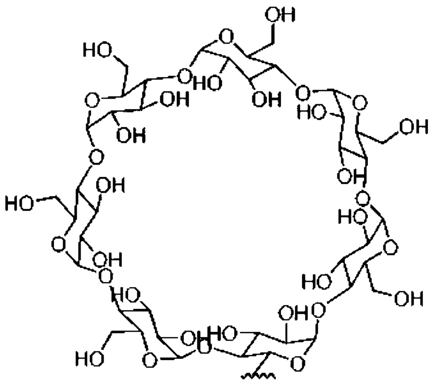, 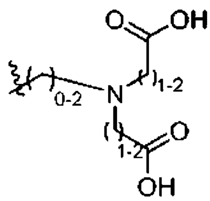, 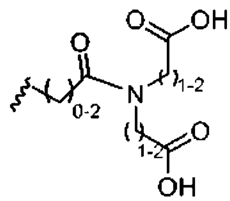,

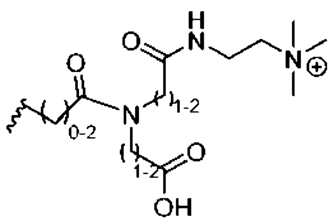, 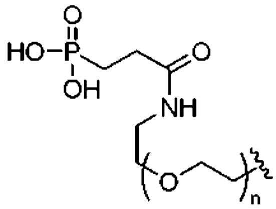

où n est un nombre entier compris entre 1 et 6,

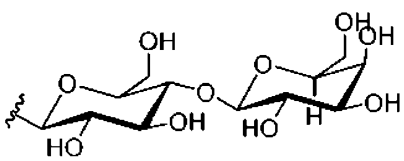, 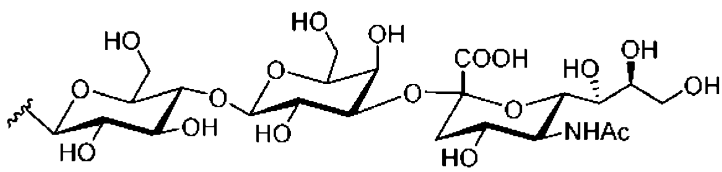,

ou

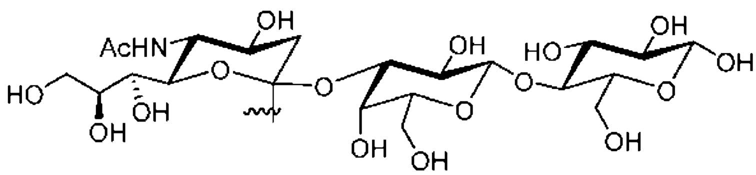;

(3) le fragment hydrophile représenté par $R^2$ comprend :

(i) une polysarcosine, par exemple, avec le fragment suivant :

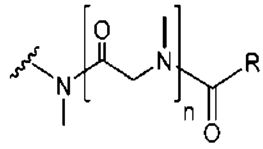,

où n est un nombre entier compris entre 3 et 25 ; et R est H, $-CH_3$ ou-$CH_2CH_2C(=O)OH$ ; ou
(ii) un polyéthylène glycol de formule :

: 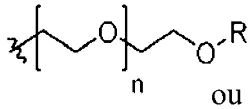 ou 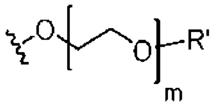,

où R est H, - $CH_3$, $CH_2CH_2NHC(=O)OR_a$, $-CH_2CH_2NHC(=O)R_a$, ou $-CH_2CH_2C(=O)OR_a$, R' est OH, $-OCH_3$, $-CH_2CH_2NHC(=O)OR_a$,
$-CH_2CH_2NHC(=O)R_a$, ou $-OCH_2CH_2C(=O)OR_a$, où $R^a$ est H ou un alkyle en $C_{1-4}$ éventuellement substitué par OH ou un alcoxyle en $C_{1-4}$, et chacun de m et n est indépendamment un nombre entier compris entre 2 et 25 ; ou

(4) le fragment hydrophile représenté par $R^2$ comprend

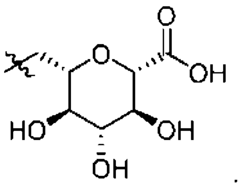.

**5.** Conjugué anticorps-médicament de l'une quelconque des revendications 1 à 4, dans lequel:

(i) $L_3$ est un fragment espaceur répondant à la structure

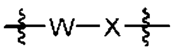,

où:

W est $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, $-NHC(=O)C(R^b)_2NHC(=O)-$, $-CH_2N(X-R^2)C(=O)O-$, $-C(=O)N(X-R^2)-$, $-CH_2N(X-R^2)C(=O)-$, - $C(=O)NR^b-$, $-C(=O)NH-$, $-CH_2N R^b C(=O)-$, $-CH_2NR^b C(=O)NH-$, $-CH_2NR^bC(=O)NR^b-$, - $NHC(=O)-$, $-NHC(=O)O-$, $-NHC(=O)NH-$, $-OC(=O)NH-$, $-S(O)_2NH-$, $-NHS(O)_2-$, $-C(=O)-$, - $C(=O)O-$ ou $-NH-$, où chaque $R^b$ est indépendamment choisi parmi H, un alkyle en $C_1$-$C_4$ et un cycloalkyle en $C_3$-$C_8$ ; et
X est une liaison, un triazolyle ou-$CH_2$-triazolyle- ; ou

(ii) $L_3$ est un fragment espaceur répondant à la structure

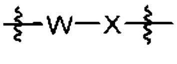,

où :

W est $-CH_2-$, $-CH_2O-$, $-CH_2N(R^b)C(=O)O-$, $-NHC(=O)C(R^b)_2NHC(=O)O-$, $-NHC(=O)C(R^b)_2NH-$, $-NHC(=O)C(R^b)_2NHC(=O)-$, $-CH_2N(X-R^2)C(=O)O-$, $-C(=O)N(X-RZ)-$, $-CH_2N(X-R^2)C(=O)-$, - $C(=O)NR^b-$, $-C(=O)NH-$, $-CH_2NR^bC(=O)-$, $-CH_2NR^bC(=O)NH-$, $-CH_2NR^bC(=O)NRb-$, - $NHC(=O)-$, $-NHC(=O)O-$, $-NHC(=O)NH-$, $-OC(=O)NH-$, $-S(O)_2NH-$, $-NHS(O)_2-$, $-C(=O)-$, - $C(=O)O-$ ou $-NH-$, où chaque $R^b$ est indépendamment choisi parmi H, un alkyle en $C_1$-$C_4$ et un cycloalkyle en $C_3$-$C_8$ ; et

X est $-CH_2$-triazolyl-alkylène en $C_{1-4}$-OC(O)NHS$(O)_2$NH-,

- cycloalkylène en $C_{4-6}$-OC(O)NHS$(O)_2$NH-, $-(CH_2CH_2O)_n$-C(O)NHS$(O)_2$NH-, $-(CH_2CH_2O)_n$-C(O)NHS$(O)_2$NH-$(CH_2CH_2O)_n$-, ou
- $CH_2$-triazolyl-alkylène en $C_{1-4}$-OC(O)NHS$(O)_2$NH-$(CH_2CH_2O)_n$-, où chaque n vaut indépendamment 1, 2 ou 3.

**6.** Conjugué anticorps-médicament de l'une quelconque des revendications 1 à 5, dans lequel

(i) le groupe de fixation est formé par une réaction comprenant au moins un groupe réactif ;
(ii) le groupe de fixation est formé en faisant réagir :

un premier groupe réactif qui est fixé au lieur, et
un second groupe réactif qui est fixé à l'anticorps ou est un résidu d'acide aminé de l'anticorps ;

(iii) le groupe de fixation est formé par une réaction comprenant au moins un groupe réactif, où au moins l'un des groupes réactifs comprend :

un thiol,
un maléimide,
un halogénoacétamide,
un azide,
un alcyne,
un cyclooctène,
une triarylphosphine,
un oxanorbornadiène,
un cyclooctyne,
une diaryltétrazine,
une monoaryltétrazine,
un norbornène,
un aldéhyde,
une hydroxylamine,
une hydrazine,
$NH_2$-NH-C(=O)-,
une cétone,
une vinylsulfone,
une aziridine,
un résidu d'acide aminé,

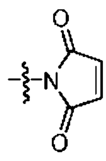,

$-ONH_2$, $-NH_2$,

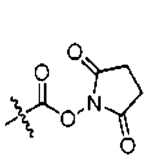, 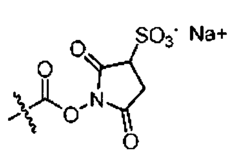, 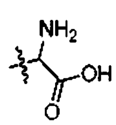, 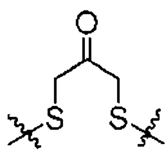,

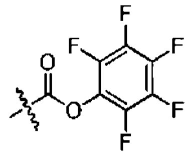, 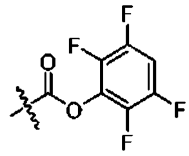, 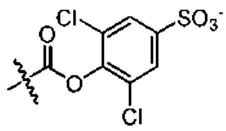;

-$N_3$,

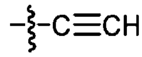;

-SH, -$SR^3$, -$SSR^4$, -$S(=O)_2(CH=CH_2)$, -$(CH_2)_2S(=O)_2(CH=CH_2)$, -$NHS(=O)_2(CH=CH_2)$, - $NHC(=O)CH_2Br$, -$NHC(=O)CH_2I$,

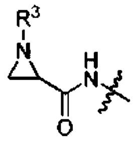,

-$C(O)NHNH_2$,

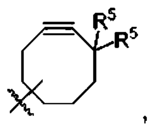,

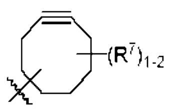, 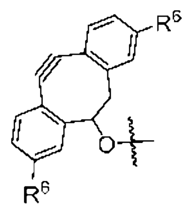, 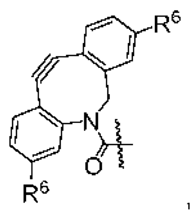, 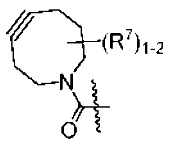, 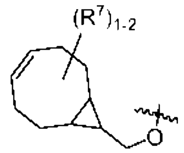,

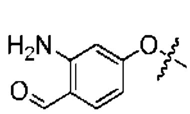, 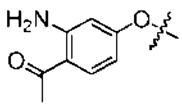, 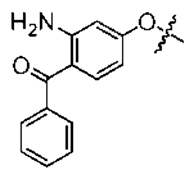, 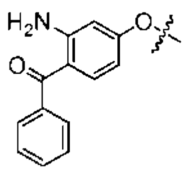,

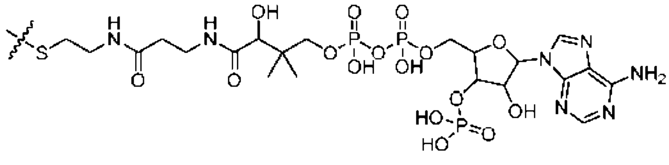,

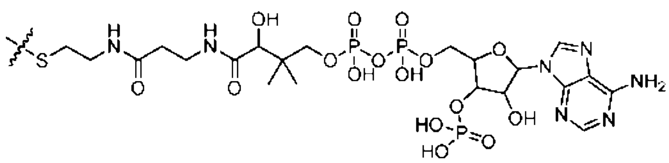,

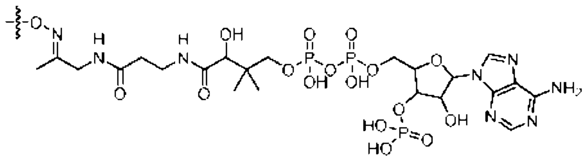,

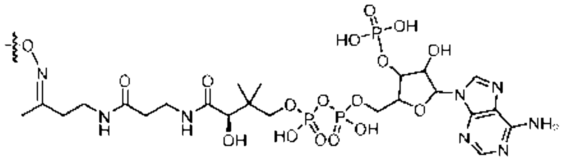,

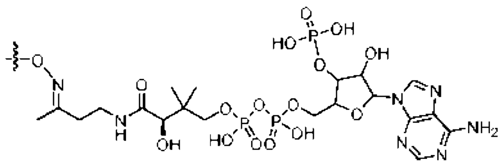, 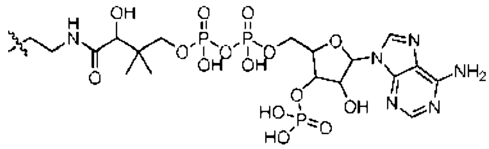,

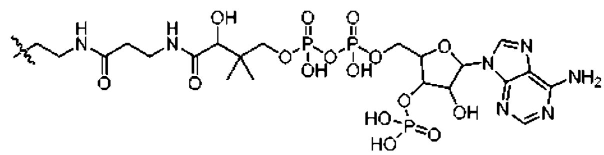,

ou

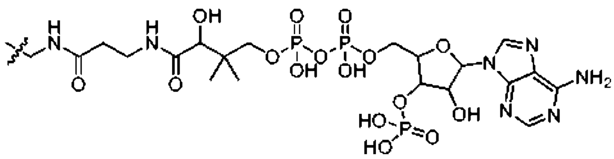;

où :

chaque $R^3$ est indépendamment choisi parmi H et alkyle en $C_1$-$C_6$ ;
chaque $R^4$ est 2-pyridyle ou 4-pyridyle ;
chaque $R^5$ est indépendamment choisi parmi H, un alkyle en $C_1$-$C_6$, F, Cl et -OH ;
chaque $R^6$ est indépendamment choisi parmi H, un alkyle en $C_1$-$C_6$, F, Cl, $-NH_2$, $-OCH_3$, $-OCH_2CH_3$, $-N(CH_3)_2$, -CN, $-NO_2$ et -OH ;
chaque $R^7$ est indépendamment choisi parmi H, un alkyle en $C_{1-6}$, un fluoro, un benzyloxy substitué par -C(=O)OH, un benzyle substitué par -C(=O)OH, un alcoxy en $C_{1-4}$ substitué par -C(=O)OH et un alkyle en $C_{1-4}$ substitué par -C(=O)OH ;

(iv) le groupe de fixation est formé par réaction qui est fixé au lieur et un second groupe réactif qui est fixé à l'anticorps ou est un résidu d'acide aminé de l'anticorps, ledit premier groupe réactif et ledit second groupe réactif comprenant :

un thiol et un maléimide,
un thiol et un halogénoacétamide,
un thiol et une vinylsulfone,
un thiol et une aziridine,
un azide et un alcyne,
un azide et un cyclooctyne,
un azide et un cyclooctène,
un azide et une triarylphosphine,

un azide et un oxanorbornadiène,
une diaryltétrazine et un cyclooctène,
une monoaryltétrazine et un norbornène,
un aldéhyde et une hydroxylamine,
un aldéhyde et une hydrazine,
un aldéhyde et $NH_2$-NH-C(=O)-,
une cétone et une hydroxylamine,
une cétone et une hydrazine,
une cétone et $NH_2$-NH-C(=O)-,
une hydroxylamine et

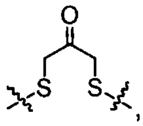,

une amine et

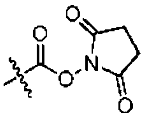, 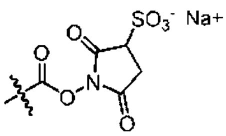, 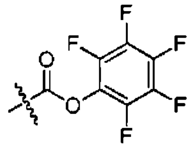, 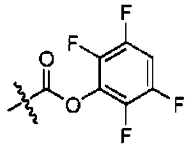,

ou

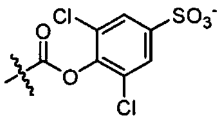

, ou
une CoA ou un analogue de CoA et un résidu de sérine ; ou

(v) le groupe de fixation comprend un groupe choisi parmi :

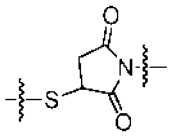 ;

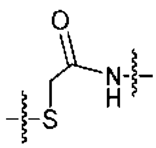 ,

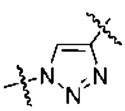 ou 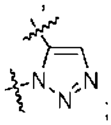 ;

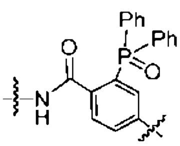 ;

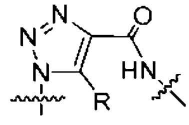
;

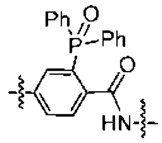
;

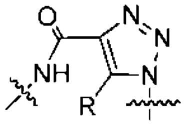
;

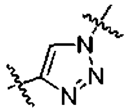
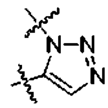
;

ou

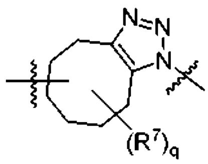
ou
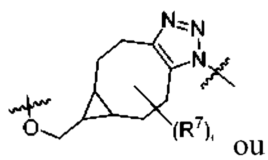
ou
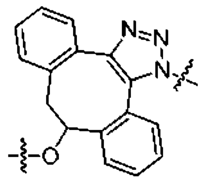

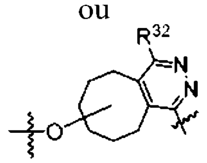
ou
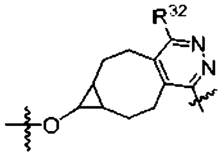
;

ou

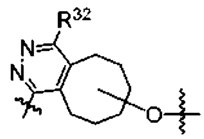
ou
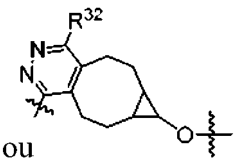
;

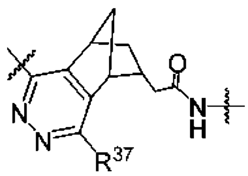
;

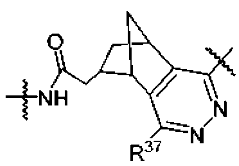
;

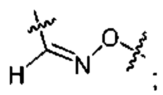
;

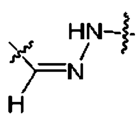
;

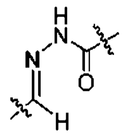
;

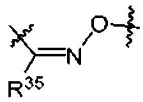
;

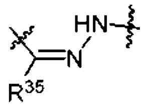
;

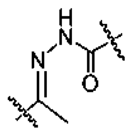
;

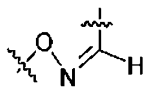
;

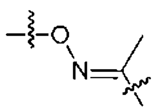
;

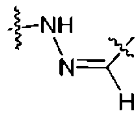
;

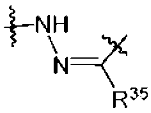
;

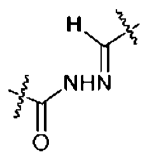
;

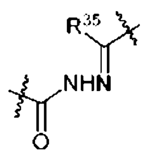
;

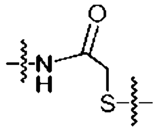;

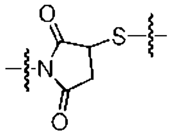.

,

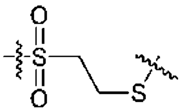;

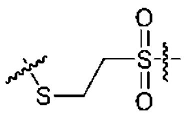;

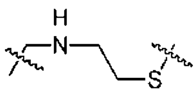

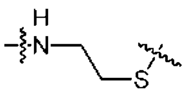;

ou

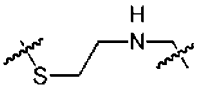

ou

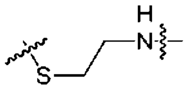;

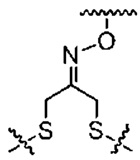;

un amide ;

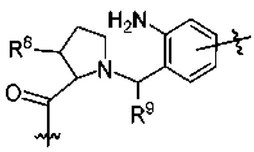;

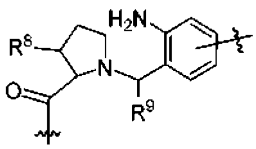;

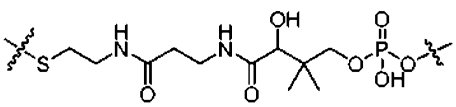;

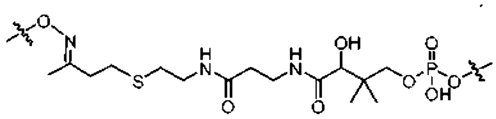

;

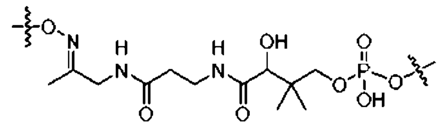

;

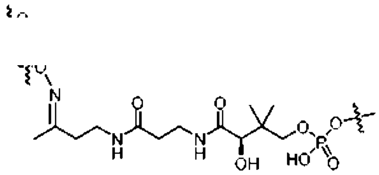

;

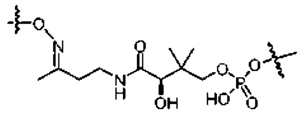

;

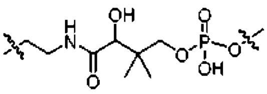

;

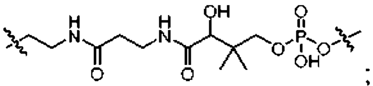

;

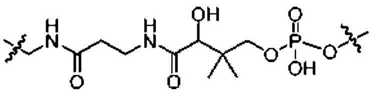

;

et

un disulfure,

où :

$R^{32}$ est H, un alkyle en $C_{1-4}$, un phényle, une pyrimidine ou une pyridine ;

$R^{35}$ est H, un alkyle en $C_{1-6}$, un phényle ou un alkyle en $C_{1-4}$ substitué par 1 à 3 groupes -OH ;

chaque $R^7$ est indépendamment choisi parmi H, un alkyle en $C_{1-6}$, un fluoro, un benzyloxy substitué par -C(=O)OH, un benzyle substitué par -C(=O)OH, un alcoxy en $C_{1-4}$ substitué par -C(=O)OH et un alkyle en $C_{1-4}$ substitué par -C(=O)OH ;

$R^{37}$ est indépendamment choisi parmi H, un phényle et une pyridine ;

q vaut 0, 1, 2 ou 3 ;

$R^8$ est H ou un méthyle ; et

$R^9$ est H, $-CH_3$ ou un phényle.

**7.** Conjugué anticorps-médicament de l'une quelconque des revendications 1 à 6, dans lequel

(i) le groupe peptidique comprend 1 à 4 résidus d'acides aminés, 1 à 3 résidus d'acides aminés ou 1 à 2 résidus d'acides aminés ;

(ii) le groupe peptidique comprend des résidus d'acides aminés choisis parmi la L-glycine (Gly), la L-valine (Val), la L-citrulline (Cit), l'acide L-cystéique (sulfo-Ala), la L-lysine (Lys), la L-isoleucine (Ile), la L-phénylalanine (Phe), la L-méthionine (Met), la L-asparagine (Asn), la L-proline (Pro), la L-alanine (Ala), la L-leucine (Leu), le L-tryptophane (Trp) et la L-tyrosine (Tyr) ;

(iii) le groupe peptidique comprend Val-Cit, Phe-Lys, Val-Ala, Val-Lys, Leu-Cit, sulfo-Ala-Val et/ou sulfo-Ala-Val-Ala ; ou

(iv) Lp est choisi parmi :

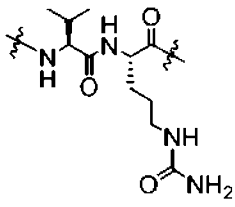, 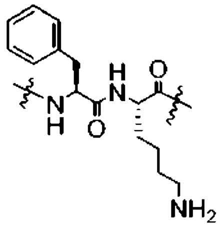, 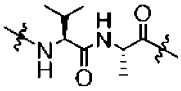, 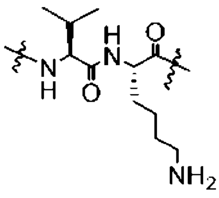, 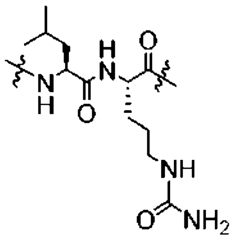,

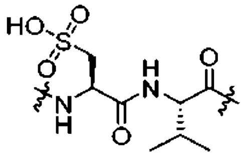, et 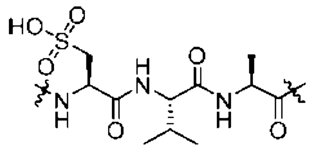.

**8.** Conjugué anticorps-médicament de formule (1) :

Ab-(L-D)$_p$ (1)

où :

Ab est un anticorps anti-CD74 ou un fragment de liaison à l'antigène de celui-ci ;
$p$ est un nombre entier de 1 à 16 ; et
où -(L-D) comprend ou est formé à partir d'un composé de formule :

(1)

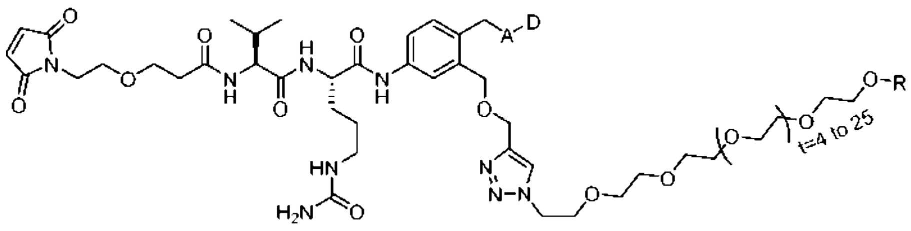

,

où :

R est H, $-CH_3$ ou $-CH_2CH_2C(=O)OH$ ;
A est une liaison, -OC(=O)-*

, 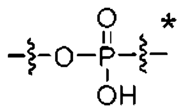, 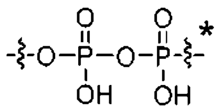, 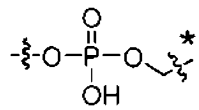,

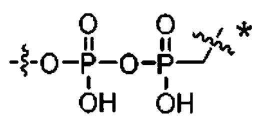,

$-OC(=O)N(CH_3)CH_2CH_2N(CH_3)C(=O)-*$ ou $-OC(=O)N(CH_3)C(R^a)_2C(R^a)_2N(CH_3)C(=O)-*$,

où $R^a$ est indépendamment choisi parmi H, un alkyle en $C_1$-$C_6$, et un cycloalkyle en $C_3$-$C_8$ et * de A indique le point de fixation à D ; et
D est un inhibiteur de Mcl-1 tel que défini dans la revendication 1 ;

(2)

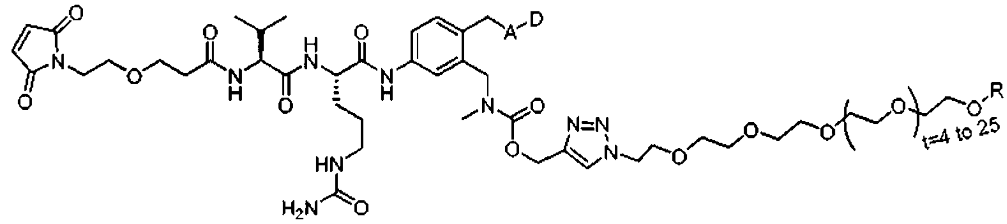

,

où :

R est H, $-CH_3$ ou $-CH_2CH_2C(=O)OH$ ;
A est une liaison, -OC(=O)-*,

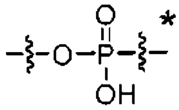 , 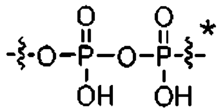 , 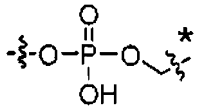 ,

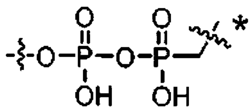

$-OC(=O)N(CH_3)CH_2CH_2N(CH_3)C(=O)$-* ou $-OC(=O)N(CH_3)C(R^a)_2C(R^a)_2N(CH_3)C(=O)$-*,
où chaque $R^a$ est indépendamment choisi parmi H, un alkyle en $C_1$-$C_6$ et un cycloalkyle en $C_3$-$C_8$ et * de A indique le point de fixation à D ; et
D est un inhibiteur de Mcl-1 tel que défini dans la revendication 1 ;

(3)

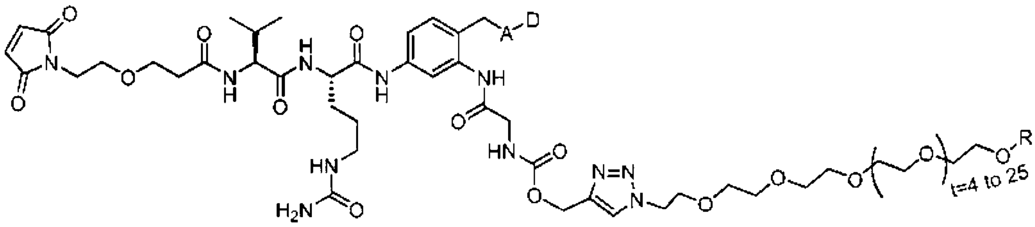

,

où :

R est H, $-CH_3$ ou $-CH_2CH_2C(=O)OH$ ;
A est une liaison, -OC(=O)-*,

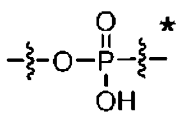 , 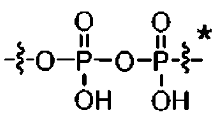 , 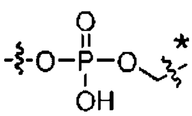 ,

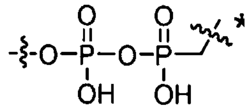

$-OC(=O)N(CH_3)CH_2CH_2N(CH_3)C(=O)-*$ ou $-OC(=O)N(CH_3)C(R^a)_2C(R^a)_2N(CH_3)C(=O)-*$,
où chaque $R^a$ est indépendamment choisi parmi H, un alkyle en $C_1$-$C_6$ et un cycloalkyle en $C_3$-$C_8$ et * de A indique le point de fixation à D ; et
D est un inhibiteur de Mcl-1 tel que défini dans la revendication 1 ;

(4)

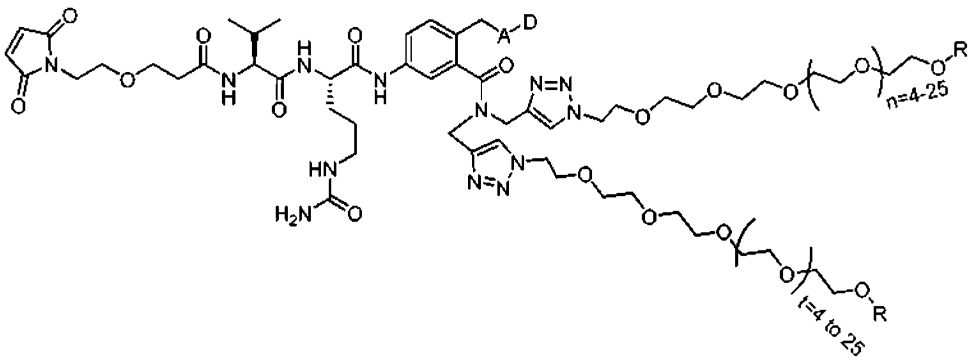

,

où :

chaque R est indépendamment choisi parmi H, $-CH_3$ et $-CH_2CH_2C(=O)OH$ ;
A est une liaison, -OC(=O)-*

,

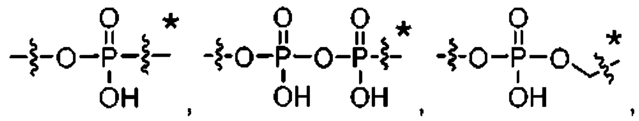

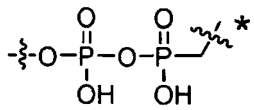

,

$-OC(=O)N(CH_3)CH_2CH_2N(CH_3)C(=O)-*$ ou $-OC(=O)N(CH_3)C(R^a)_2C(R^a)_2N(CH_3)C(=O)-*$,
où chaque $R^a$ est indépendamment choisi parmi H, un alkyle en $C_1$-$C_6$ et un cycloalkyle en $C_3$-$C_8$ et * de A indique le point de fixation à D ; et
D est un inhibiteur de Mcl-1 tel que défini dans la revendication 1 ;

(5)

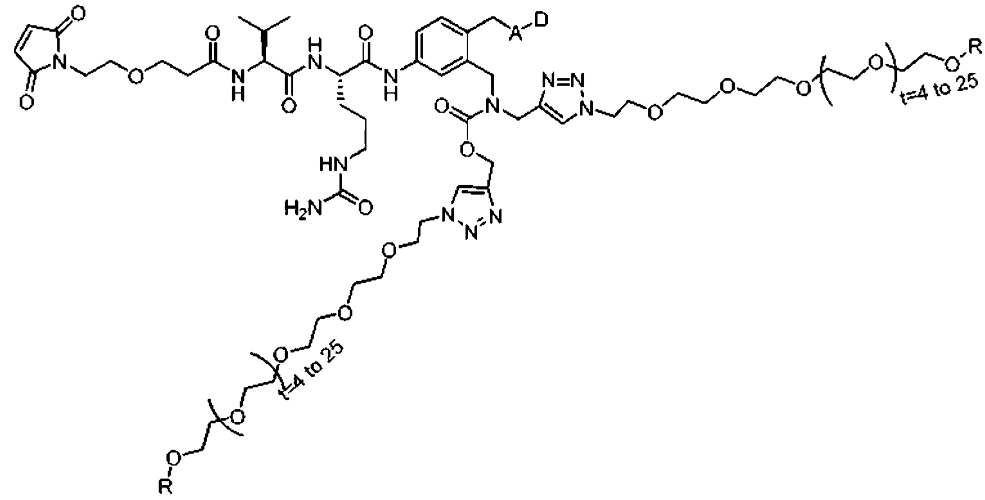

,

où :

chaque R est indépendamment choisi parmi H, $-CH_3$ et $-CH_2CH_2C(=O)OH$ ;
A est une liaison, -OC(=O)-*,

, 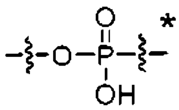 , 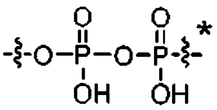 , 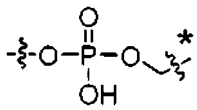 ,

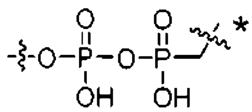

,

$-OC(=O)N(CH_3)CH_2CH_2N(CH_3)C(=O)-*$ ou $-OC(=O)N(CH_3)C(R^a)_2C(R^a)_2N(CH_3)C(=O)-*$,
où chaque $R^a$ est indépendamment choisi parmi H, un alkyle en $C_1$-$C_6$ et un cycloalkyle en $C_3$-$C_8$ et * de A indique le point de fixation à D ; et
D est un inhibiteur de Mcl-1 tel que défini dans la revendication 1 ;

(6)

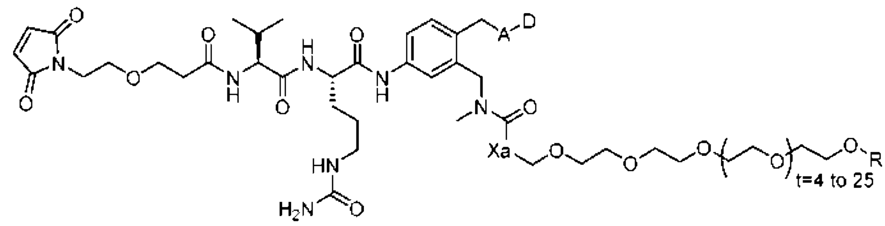

,

où :

Xa est $-CH_2-$, $-OCH_2-$, $-NHCH_2-$ ou $-NRCH_2-$ et chaque R est indépendamment H, $-CH_3$ ou $-CH_2CH_2C(=O)OH$ ;
A est une liaison, -OC(=O)-*,

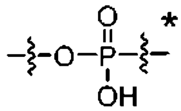 , 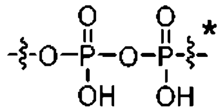 , 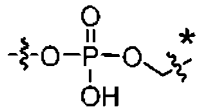 ,

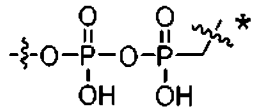 ,

-OC(=O)N($CH_3$)$CH_2CH_2$N($CH_3$)C(=O)-* ou -OC(=O)N($CH_3$)C($R^a$)$_2$C($R^a$)$_2$N($CH_3$)C(=O)-*, où chaque $R^a$ est indépendamment choisi parmi H, un alkyle en $C_1$-$C_6$ et un cycloalkyle en $C_3$-$C_8$ et * de A indique le point de fixation à D ; et

D est un inhibiteur de Mcl-1 tel que défini dans la revendication 1 ;

(7)

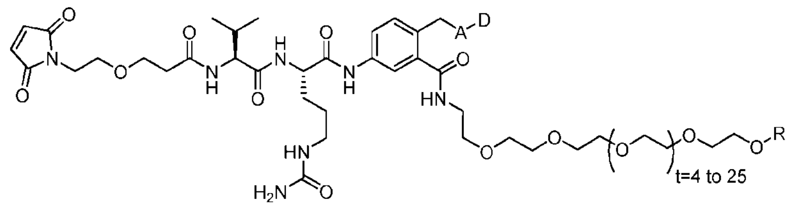

où :

R est H, -$CH_3$ ou -$CH_2CH_2$C(=O)OH ;

A est une liaison, -OC(=O)-*,

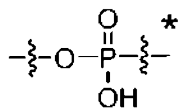 , 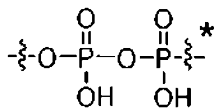 , 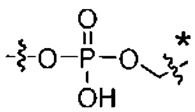 ,

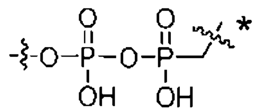 ,

-OC(=O)N($CH_3$)$CH_2CH_2$N($CH_3$)C(=O)-* ou -OC(=O)N($CH_3$)C($R^a$)$_2$C($R^a$)$_2$N($CH_3$)C(=O)-*, où chaque $R^a$ est indépendamment choisi parmi H, un alkyle en $C_1$-$C_6$ et un cycloalkyle en $C_3$-$C_8$ et * de A indique le point de fixation à D ; et

D est un inhibiteur de Mcl-1 tel que défini dans la revendication 1 ;

(8)

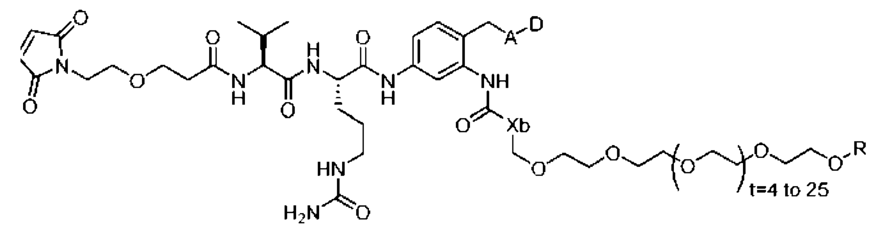
,

où :

$X_b$ est $-CH_2-$, $-OCH_2-$, $-NHCH_2-$ ou $-NRCH_2-$ et chaque R est indépendamment H, $-CH_3$ ou $-CH_2CH_2C(=O)OH$ ;

A est une liaison, -OC(=O)-*,

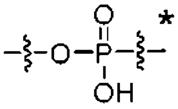,

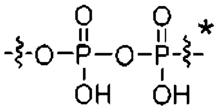,

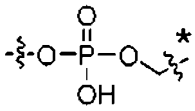,

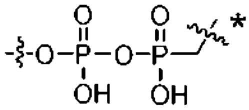;

$-OC(=O)N(CH_3)CH_2CH_2N(CH_3)C(=O)-*$ ou $-OC(=O)N(CH_3)C(R^a)_2C(R^a)_2N(CH_3)C(=O)-*$, où chaque $R^a$ est indépendamment choisi parmi H, un alkyle en $C_1$-$C_6$ et un cycloalkyle en $C_3$-$C_8$ et * de A indique le point de fixation à D ; et

D est un inhibiteur de Mcl-1 tel que défini dans la revendication 1 ;

(9)

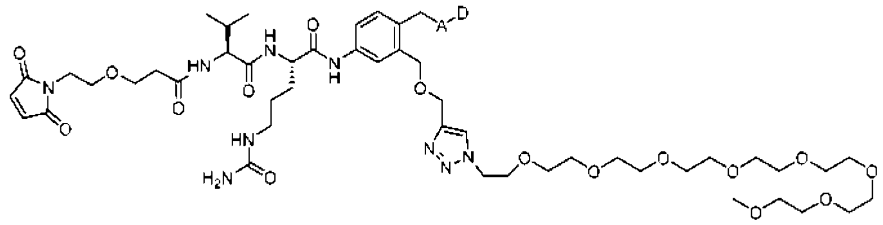, (

où :

A est une liaison, -OC(=O)-*,

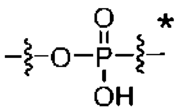,

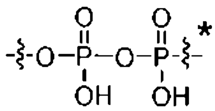,

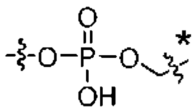,

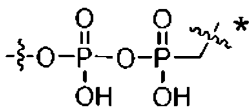,

$-OC(=O)N(CH_3)CH_2CH_2N(CH_3)C(=O)-*$ ou $-OC(=O)N(CH_3)C(R^a)_2C(R^a)_2N(CH_3)C(=O)-*$, où chaque $R^a$ est indépendamment choisi parmi H, un alkyle en $C_1$-$C_6$ et un cycloalkyle en $C_3$-$C_8$ et * de A indique le point de fixation à D ; et

D est un inhibiteur de Mcl-1 tel que défini dans la revendication 1 ;

(10)

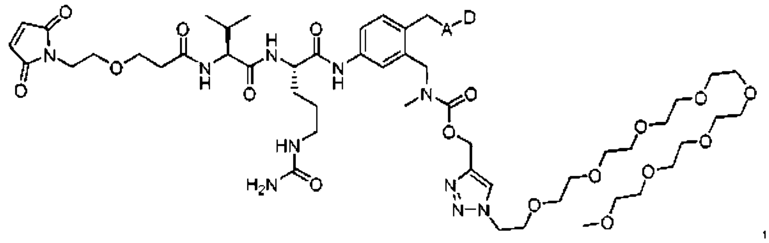

,

où :

A est une liaison, -OC(=O)-*,

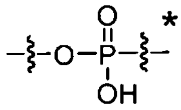 , 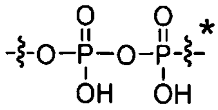 , 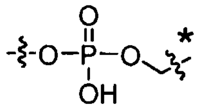 ,

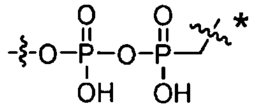

-OC(=O)N($CH_3$)$CH_2CH_2$N($CH_3$)C(=O)-* ou -OC(=O)N($CH_3$)C($R^a$)$_2$C($R^a$)$_2$N($CH_3$)C(=O)-*, où chaque $R^a$ est indépendamment choisi parmi H, un alkyle en $C_1$-$C_6$ et un cycloalkyle en $C_3$-$C_8$ et * de A indique le point de fixation à D ; et D est un inhibiteur de Mcl-1 tel que défini dans la revendication 1 ;

, (11)

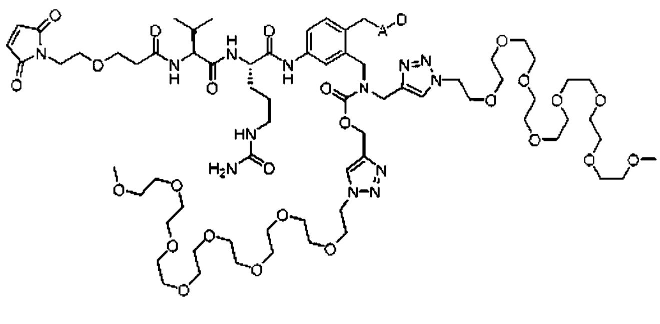

où :

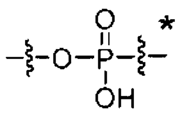 , 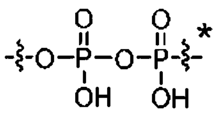 , 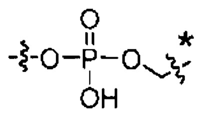 ,

A est une liaison, -OC(=O)-*,

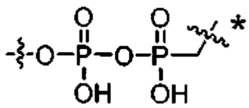

,

-OC(=O)N($CH_3$)$CH_2CH_2$N($CH_3$)C(=O)-* ou -OC(=O)N($CH_3$)C($R^a$)$_2$C($R^a$)$_2$N($CH_3$)C(=O)-*,

où chaque $R^a$ est indépendamment choisi parmi H, un alkyle en $C_1$-$C_6$ et un cycloalkyle en $C_3$-$C_8$ et * de A indique le point de fixation à D ; et

D est un inhibiteur de Mcl-1 tel que défini dans la revendication 1 ;

(12)

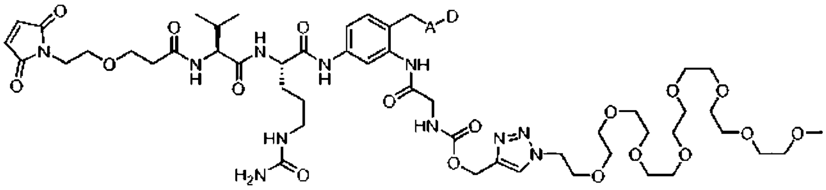

,

où :

A est une liaison, -OC(=O)-*,

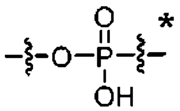
,
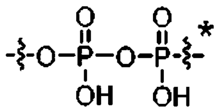
,
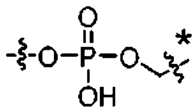
,

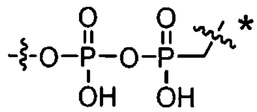

, $-OC(=O)N(CH_3)CH_2CH_2N(CH_3)C(=O)-*$ ou $-OC(=O)N(CH_3)C(R^a)_2C(R^a)_2N(CH_3)C(=O)-*$,

où chaque $R^a$ est indépendamment choisi parmi H, un alkyle en $C_1$-$C_6$ et un cycloalkyle en $C_3$-$C_8$ et * de A indique le point de fixation à D ; et

D est un inhibiteur de Mcl-1 tel que défini dans la revendication 1 ;

(13)

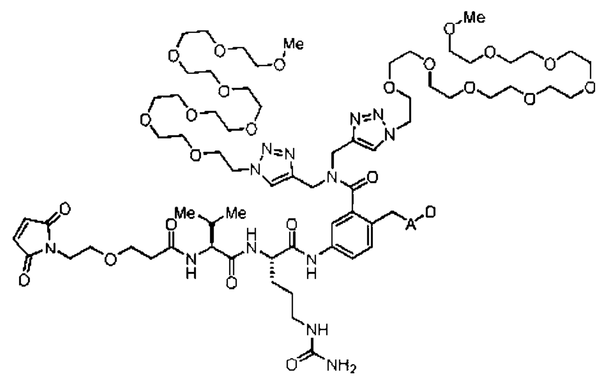

,

où :

A est une liaison, -OC(=O)-*


,

,

,

,

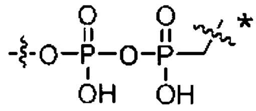

, .

$-OC(=O)N(CH_3)CH_2CH_2N(CH_3)C(=O)-*$ ou $-OC(=O)N(CH_3)C(R^a)_2C(R^a)_2N(CH_3)C(=O)-*$, où chaque $R^a$ est indépendamment choisi parmi H, un alkyle en $C_1$-$C_6$ et un cycloalkyle en $C_3$-$C_8$ et * de A indique le point de fixation à D ; et

D est un inhibiteur de Mcl-1 tel que défini dans la revendication 1 ;

(14)

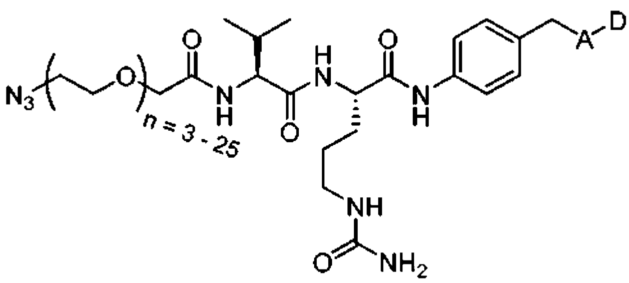

,

où :

A est une liaison, -OC(=O)-*

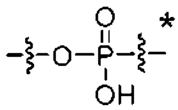

,

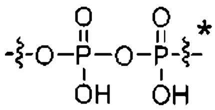

,

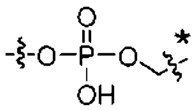

,

,

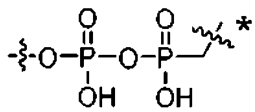

,

$-OC(=O)N(CH_3)CH_2CH_2N(CH_3)C(=O)-*$ ou $-OC(=O)N(CH_3)C(R^a)_2C(R^a)_2N(CH_3)C(=O)-*$, où chaque $R^a$ est indépendamment choisi parmi H, un alkyle en $C_1$-$C_6$ et un cycloalkyle en $C_3$-$C_8$ et * de A indique le point de fixation à D ; et

D est un inhibiteur de Mcl-1 tel que défini dans la revendication 1 ;

(15)

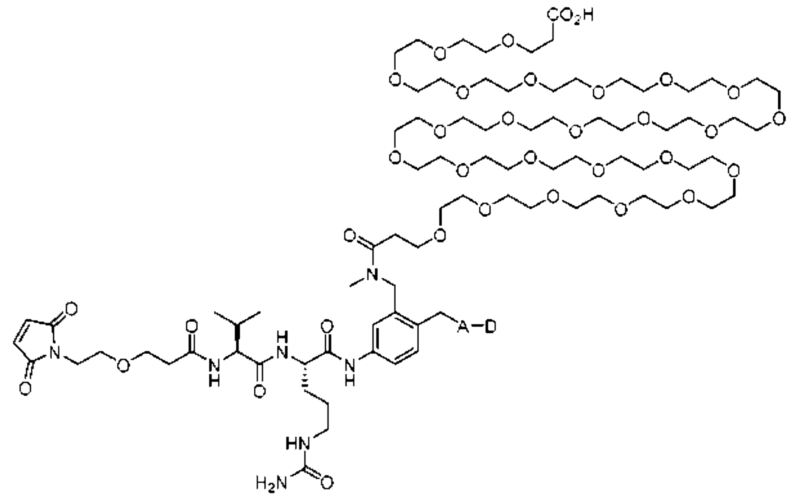

,

où :

A est une liaison, -OC(=O)-*,

, 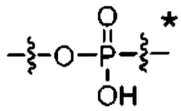 , 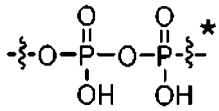 , 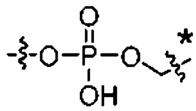 ,

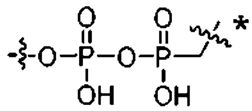 ,

$-OC(=O)N(CH_3)CH_2CH_2N(CH_3)C(=O)-*$ ou $-OC(=O)N(CH_3)C(R^a)_2C(R^a)_2N(CH_3)C(=O)-*$, où chaque $R^a$ est indépendamment choisi parmi H, un alkyle en $C_1$-$C_6$ et un cycloalkyle en $C_3$-$C_8$ et * de A indique le point de fixation à D ; et
D est un inhibiteur de Mcl-1 tel que défini dans la revendication 1 ; ou

(16)

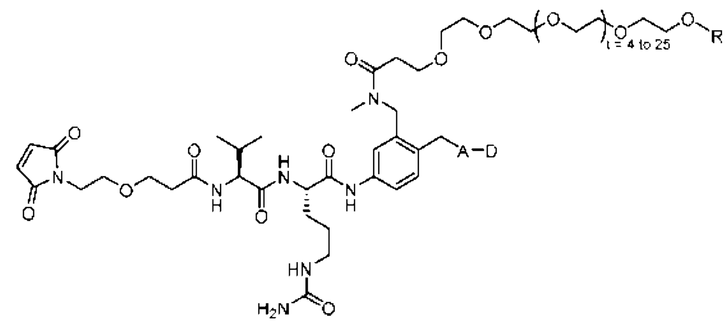

,

où :

chaque R est indépendamment H, $-CH_3$ ou $-CH_2CH_2C(=O)OH$;
A est une liaison, -OC(=O)-*,

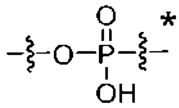,
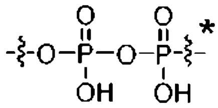,
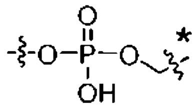,

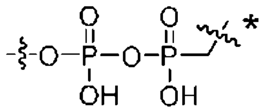,

$-OC(=O)N(CH_3)CH_2CH_2N(CH_3)C(=O)-*$ ou $-OC(=O)N(CH_3)C(R^a)_2C(R^a)_2N(CH_3)C(=O)-*$,
où chaque $R^a$ est indépendamment choisi parmi H, un alkyle en $C_1$-$C_6$ et un cycloalkyle en $C_3$-$C_8$ et * de A indique le point de fixation à D ; et
D est un inhibiteur de Mcl-1 tel que défini dans la revendication 1.

**9.** Conjugué anticorps-médicament de l'une quelconque des revendications 1 à 8, dans lequel A est une liaison.

**10.** Conjugué anticorps-médicament de l'une quelconque des revendications 1 à 9, dans lequel R est $-CH_3$.

**11.** Conjugué anticorps-médicament de l'une quelconque des revendications 1 à 10, dans lequel -(L-D) est formé à partir d'un composé choisi dans le Tableau A ou d'un énantiomère, diastéréoisomère, atropisomère, dérivé deutéré et/ou sel acceptable pharmaceutiquement de celui-ci.

**12.** Conjugué anticorps-médicament de la revendication 11, dans lequel -(L-D) comprend le composé suivant ou est formé à partir de celui-ci :

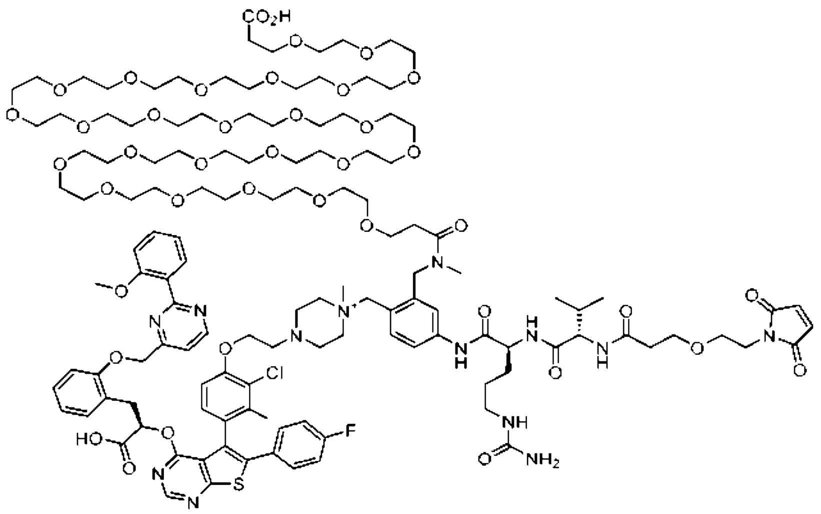

L11-P1,

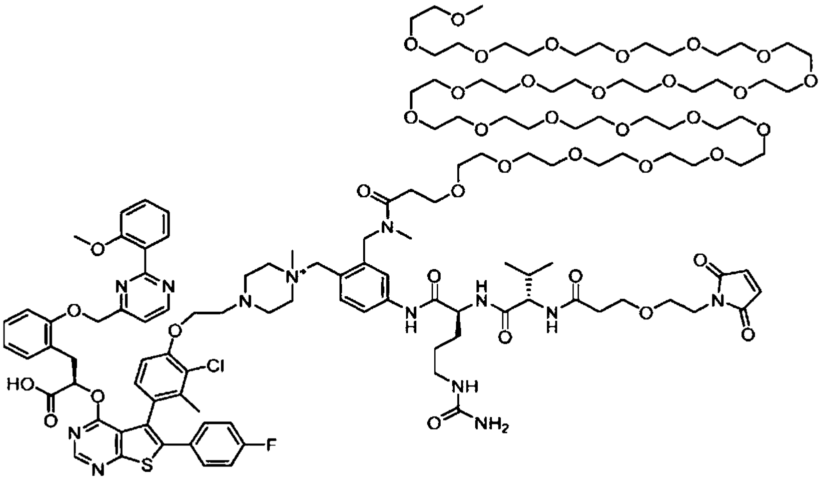

L30-P1,

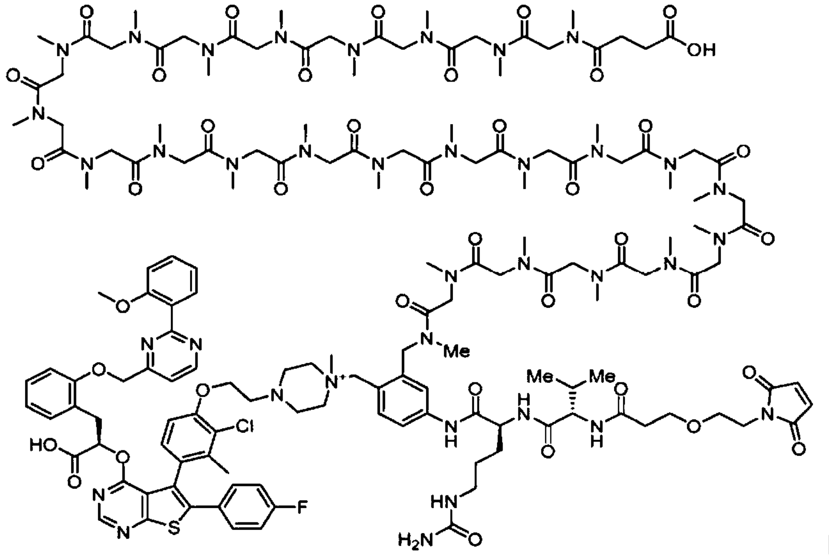

L37-P1,

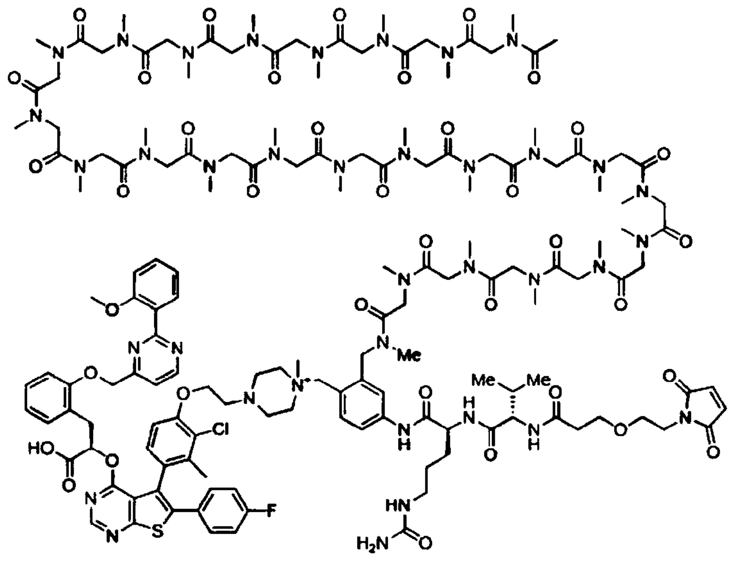

L40-P1,

ou

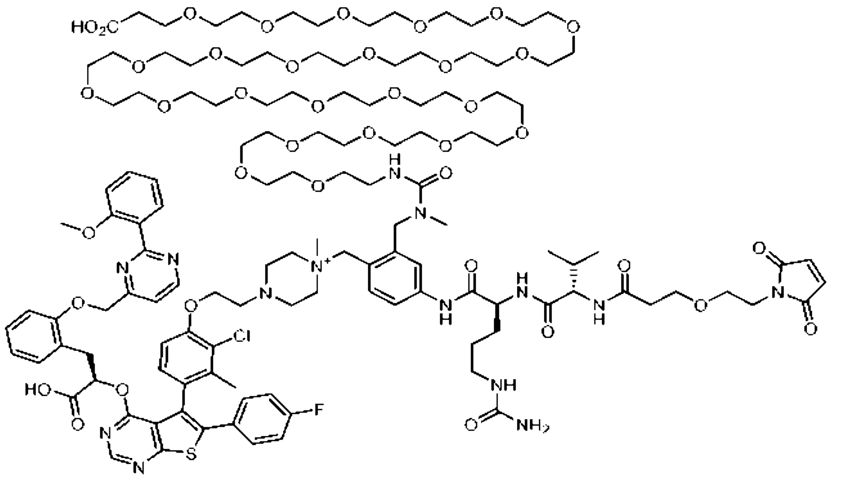

L42-P1.

**13.** Conjugué anticorps-médicament de l'une quelconque des revendications 1 à 11, dans lequel

(1) l'anticorps anti-CD74 comprend un anticorps anti-CD74 ou un fragment de liaison à l'antigène comprenant trois CDR de chaîne lourde et trois CDR de chaîne légère comme suit :

(i) CDR1 de chaîne lourde (HCDR1) constitué de SEQ ID N°: 1, CDR2 de chaîne lourde (HCDR2) constitué de SEQ ID N° : 2, CDR3 de chaîne lourde (HCDR3) constitué de SEQ ID N° : 3 ; CDR1 de chaîne légère (LCDR1) constitué de SEQ ID N° : 16, CDR2 de chaîne légère (LCDR2) constitué de SEQ ID N° : 70 et CDR3 de chaîne légère (LCDR3) constitué de SEQ ID N° : 18 ;
(ii) CDR1 de chaîne lourde (HCDR1) constitué de SEQ ID N° : 4, CDR2 de chaîne lourde (HCDR2) constitué de SEQ ID N° : 2, CDR3 de chaîne lourde (HCDR3) constitué de SEQ ID N° : 3 ; CDR1 de chaîne légère (LCDR1) constitué de SEQ ID N° : 16, CDR2 de chaîne légère (LCDR2) constitué de SEQ ID N° : 70 et CDR3 de chaîne légère (LCDR3) constitué de SEQ ID N° : 18 ;
(iii) CDR1 de chaîne lourde (HCDR1) constitué de SEQ ID N° : 5, CDR2 de chaîne lourde (HCDR2) constitué de SEQ ID N° : 6, CDR3 de chaîne lourde (HCDR3) constitué de SEQ ID N° : 3 ; CDR1 de chaîne légère (LCDR1) constitué de SEQ ID N° : 19, CDR2 de chaîne légère (LCDR2) constitué de SEQ ID N° : 20 et CDR3 de chaîne légère (LCDR3) constitué de SEQ ID N° : 21 ;
(iv) CDR1 de chaîne lourde (HCDR1) constituée de SEQ ID N° : 7, CDR2 de chaîne lourde (HCDR2) constituée de SEQ ID N° : 8, CDR3 de chaîne lourde (HCDR3) constituée de SEQ ID N° : 9 ; CDR1 de chaîne légère (LCDR1) constituée de SEQ ID N° : 22, CDR2 de chaîne légère (LCDR2) constituée de SEQ ID N° : 20 et CDR3 de chaîne légère (LCDR3) constituée de SEQ ID N° : 18 ;
(v) CDR1 de chaîne lourde (HCDR1) constitué de SEQ ID N° : 1, CDR2 de chaîne lourde (HCDR2) constitué de SEQ ID N° : 2, CDR3 de chaîne lourde (HCDR3) constitué de SEQ ID N° : 3 ; CDR1 de chaîne légère (LCDR1) constitué de SEQ ID N° : 35, CDR2 de chaîne légère (LCDR2) constitué de SEQ ID N° : 70, et CDR3 de chaîne légère (LCDR3) constitué de SEQ ID N° : 18 ;
(vi) CDR1 de chaîne lourde (HCDR1) constitué de SEQ ID N° : 4, CDR2 de chaîne lourde (HCDR2) constitué de SEQ ID N° : 2, CDR3 de chaîne lourde (HCDR3) constitué de SEQ ID N° : 3 ; CDR1 de chaîne légère (LCDR1) constitué de SEQ ID N° : 35, CDR2 de chaîne légère (LCDR2) constitué de SEQ ID N° : 70 et CDR3 de chaîne légère (LCDR3) constitué de SEQ ID N° : 18 ;
(vii) CDR1 de chaîne lourde (HCDR1) constitué de SEQ ID N° : 5, CDR2 de chaîne lourde (HCDR2) constitué de SEQ ID N° : 6, CDR3 de chaîne lourde (HCDR3) constitué de SEQ ID N° : 3 ; CDR1 de chaîne légère (LCDR1) constitué de SEQ ID N° : 71, CDR2 de chaîne légère (LCDR2) constitué de SEQ ID N° : 20, et CDR3 de chaîne légère (LCDR3) constitué de SEQ ID N° : 21, ou
(viii) CDR1 de chaîne lourde (HCDR1) constitué de SEQ ID N° : 7, CDR2 de chaîne lourde (HCDR2) constitué de SEQ ID N° : 8, CDR3 de chaîne lourde (HCDR3) constitué de SEQ ID N° : 9; CDR1 de chaîne légère (LCDR1) constitué de SEQ ID N° : 17, CDR2 de chaîne légère (LCDR2) constitué de SEQ ID N° : 20 et CDR3 de chaîne légère (LCDR3) constitué de SEQ ID N° : 18 ;

(2) l'anticorps anti-CD74 ou un fragment de liaison à l'antigène de celui-ci comprend :

(i) une région variable de chaîne lourde comprenant la séquence d'acides aminés de SEQ ID N° : 10, et une

région variable de chaîne légère comprenant la séquence d'acides aminés de SEQ ID N° : 23 ;
(ii) une région variable de chaîne lourde comprenant la séquence d'acides aminés de SEQ ID N° : 10, et une région variable de chaîne légère comprenant la séquence d'acides aminés de SEQ ID N° : 27 ;
(iii) une région variable de chaîne lourde comprenant la séquence d'acides aminés de SEQ ID N° : 10, et une région variable de chaîne légère comprenant la séquence d'acides aminés de SEQ ID N° : 31 ;
(iv) une région variable de chaîne lourde comprenant la séquence d'acides aminés de SEQ ID N° : 10, et une région variable de chaîne légère comprenant la séquence d'acides aminés de SEQ ID N° : 36 ;
(v) une région variable de chaîne lourde comprenant la séquence d'acides aminés de SEQ ID N° : 10, et une région variable de chaîne légère comprenant la séquence d'acides aminés de SEQ ID N° : 40 ; ou
(vi) une région variable de chaîne lourde comprenant la séquence d'acides aminés de SEQ ID N° : 10, et une région variable de chaîne légère comprenant la séquence d'acides aminés de SEQ ID N° : 44 ; ou

(3) l'anticorps anti-CD74 comprend :

(a) la séquence d'acides aminés de chaîne lourde de SEQ ID N° : 12 et la séquence d'acides aminés de chaîne légère de SEQ ID N° : 25 ;
(b) la séquence d'acides aminés de chaîne lourde de SEQ ID N° : 14 et la séquence d'acides aminés de chaîne légère de SEQ ID N° : 25 ;
(c) la séquence d'acides aminés de chaîne lourde de SEQ ID N° : 15 et la séquence d'acides aminés de chaîne légère de SEQ ID N° : 25 ;
(d) la séquence d'acides aminés de chaîne lourde de SEQ ID N° : 12 et la séquence d'acides aminés de chaîne légère de SEQ ID N° : 29 ;
(e) la séquence d'acides aminés de chaîne lourde de SEQ ID N° : 14 et la séquence d'acides aminés de chaîne légère de SEQ ID N° : 29 ;
(f) la séquence d'acides aminés de chaîne lourde de SEQ ID N° : 15 et la séquence d'acides aminés de chaîne légère de SEQ ID N° : 29 ;
(g) la séquence d'acides aminés de chaîne lourde de SEQ ID N° : 12 et la séquence d'acides aminés de chaîne légère de SEQ ID N° : 33 ;
(h) la séquence d'acides aminés de chaîne lourde de SEQ ID N° : 14 et la séquence d'acides aminés de chaîne légère de SEQ ID N° : 33 ;
(i) la séquence d'acides aminés de chaîne lourde de SEQ ID N° : 15 et la séquence d'acides aminés de chaîne légère de SEQ ID N° : 33 ;
(j) la séquence d'acides aminés de chaîne lourde de SEQ ID N° : 12 et la séquence d'acides aminés de chaîne légère de SEQ ID N° : 38 ;
(k) la séquence d'acides aminés de chaîne lourde de SEQ ID N° : 14 et la séquence d'acides aminés de chaîne légère de SEQ ID N° : 38 ;
(l) la séquence d'acides aminés de chaîne lourde de SEQ ID N° : 15 et la séquence d'acides aminés de chaîne légère de SEQ ID N° : 38 ;
(m) la séquence d'acides aminés de chaîne lourde de SEQ ID N° : 12 et la séquence d'acides aminés de chaîne légère de SEQ ID N° : 42 ;
(n) la séquence d'acides aminés de chaîne lourde de SEQ ID N° : 14 et la séquence d'acides aminés de chaîne légère de SEQ ID N° : 42 ;
(o) la séquence d'acides aminés de chaîne lourde de SEQ ID N° : 15 et la séquence d'acides aminés de chaîne légère de SEQ ID N° : 42 ;
(p) la séquence d'acides aminés de chaîne lourde de SEQ ID N° : 12 et la séquence d'acides aminés de chaîne légère de SEQ ID N° : 46 ;
(q) la séquence d'acides aminés de chaîne lourde de SEQ ID N° : 14 et la séquence d'acides aminés de chaîne légère de SEQ ID N° : 46 ; ou
(r) la séquence d'acides aminés de chaîne lourde de SEQ ID N° : 15 et la séquence d'acides aminés de chaîne légère de SEQ ID N° : 46.

**14.** Conjugué anticorps-médicament de l'une quelconque des revendications 1 à 13, dans lequel l'Ab est un anticorps Fc silencieux.

**15.** Composition comprenant de multiples copies du conjugué anticorps-médicament de l'une quelconque des revendications 1 à 14, dans laquelle le p moyen des conjugués anticorps-médicament dans la composition est d'environ 2 à environ 16, par exemple d'environ 2 à environ 8, par exemple d'environ 2 à environ 4.

**16.** Composition pharmaceutique comprenant le conjugué anticorps-médicament de l'une quelconque des revendications 1 à 14 ou la composition de la revendication 15 et un support acceptable pharmaceutiquement.

**17.** Conjugué anticorps-médicament de l'une quelconque des revendications 1 à 14, composition de la revendication 15, ou composition pharmaceutique de la revendication 16 destiné(e) à être utilisé(e) dans le traitement d'un sujet atteint d'un cancer, où éventuellement

(1) le cancer exprime CD74 ; ou
(2) le cancer est une tumeur ou un cancer hématologique, de préférence, le cancer est un cancer du sein, un myélome multiple, un myélome des cellules plasmatiques, une leucémie, un lymphome, un cancer gastrique, une leucémie myéloïde aiguë, un cancer de la vessie, un cancer du cerveau, un cancer de la moelle osseuse, un cancer du col de l'utérus, une leucémie lymphoïde chronique, un cancer colorectal, un cancer de l'œsophage, un cancer hépatocellulaire, une leucémie lymphoblastique, un lymphome folliculaire, des tumeurs malignes lymphoïdes d'origine lymphocytaire T ou B, un mélanome, une leucémie myéloïde, un myélome, un cancer de la cavité buccale, un cancer de l'ovaire, un cancer du poumon non à petites cellules, une leucémie lymphoïde chronique, un cancer de la prostate, un cancer du poumon à petites cellules ou un cancer de la rate.

**18.** Conjugué anticorps-médicament de l'une quelconque des revendications 1 à 14, composition de la revendication 15, ou composition pharmaceutique de la revendication 16 destiné(e) à être utilisé(e) dans la réduction ou l'inhibition de la croissance d'une tumeur chez un sujet, où éventuellement

(1) la tumeur exprime CD74 ; ou
(2) la tumeur est un cancer du sein, un cancer gastrique, un cancer de la vessie, un cancer du cerveau, un cancer du col de l'utérus, un cancer colorectal, un cancer de l'œsophage, un cancer hépatocellulaire, un mélanome, un cancer de la cavité buccale, un cancer de l'ovaire, un cancer du poumon non à petites cellules, un cancer de la prostate, un cancer du poumon à petites cellules ou un cancer de la rate.

**19.** Conjugué anticorps-médicament de l'une quelconque des revendications 1 à 14, composition de la revendication 15, ou composition pharmaceutique de la revendication 16 destiné(e) à être utilisé(e) selon la revendication 18, où l'administration du conjugué anticorps-médicament, de la composition, ou de la composition pharmaceutique réduit ou inhibe la croissance de la tumeur d'au moins environ 10 %, d'au moins environ 20 %, d'au moins environ 30 %, d'au moins environ 40 %, d'au moins environ 50 %, d'au moins environ 60 %, d'au moins environ 70 %, d'au moins environ 80 %, d'au moins environ 90 %, d'au moins environ 95 %, ou d'au moins environ 99 %.

**20.** Conjugué anticorps-médicament de l'une quelconque des revendications 1 à 14, composition de la revendication 15, ou composition pharmaceutique de la revendication 16, destiné(e) à être utilisé(e) dans la réduction ou le ralentissement de l'expansion d'une population de cellules cancéreuses chez un sujet, où éventuellement

(1) la population de cellules cancéreuses exprime CD74 ; ou
(2) la population de cellules cancéreuses provient d'une tumeur ou d'un cancer hématologique, de préférence la population de cellules cancéreuses provient d'un cancer du sein, d'un myélome multiple, d'un myélome des cellules plasmatiques, d'une leucémie, d'un lymphome, d'un cancer gastrique, d'une leucémie myéloïde aiguë, d'un cancer de la vessie, d'un cancer du cerveau, d'un cancer de la moelle osseuse, d'un cancer du col de l'utérus, d'une leucémie lymphoïde chronique, d'un cancer colorectal, d'un cancer de l'œsophage, d'un cancer hépatocellulaire, d'une leucémie lymphoblastique, d'un lymphome folliculaire, de tumeurs malignes lymphoïdes d'origine lymphocytaire T ou B, d'un mélanome, d'une leucémie myéloïde, d'un myélome, d'un cancer de la cavité buccale, d'un cancer de l'ovaire, d'un cancer du poumon non à petites cellules, d'une leucémie lymphoïde chronique, d'un cancer de la prostate, d'un cancer du poumon à petites cellules ou d'un cancer de la rate.

**21.** Conjugué anticorps-médicament de l'une quelconque des revendications 1 à 14, composition de la revendication 15, ou composition pharmaceutique de la revendication 16 destiné(e) à être utilisé(e) selon la revendication 20, dans lequel l'administration du conjugué anticorps-médicament, de la composition, ou de la composition pharmaceutique réduit la population de cellules cancéreuses ou ralentit l'expansion de la population de cellules cancéreuses d'au moins environ 10 %, d'au moins environ 20 %, d'au moins environ 30 %, d'au moins environ 40 %, d'au moins environ 50 %, d'au moins environ 60 %, d'au moins environ 70 %, d'au moins environ 80 %, d'au moins environ 90 %, d'au moins environ 95 %, ou d'au moins environ 99 %.

**22.** Procédé pour déterminer si un sujet atteint ou suspecté d'être atteint d'un cancer répondra à un traitement avec le

conjugué anticorps-médicament de l'une quelconque des revendications 1 à 14, la composition de la revendication 15 ou la composition pharmaceutique de la revendication 16, comprenant la fourniture d'un échantillon biologique du sujet ; la mise en contact de l'échantillon avec le conjugué anticorps-médicament ; et la détection de la liaison du conjugué anticorps-médicament à des cellules cancéreuses dans l'échantillon ; dans lequel éventuellement

(1) les cellules cancéreuses dans l'échantillon expriment CD74 ;
(2) le cancer exprime CD74 ; ou
(3) le cancer est une tumeur ou un cancer hématologique, de préférence, le cancer est un cancer du sein, un myélome multiple, un myélome des cellules plasmatiques, une leucémie, un lymphome, un cancer gastrique, une leucémie myéloïde aiguë, un cancer de la vessie, un cancer du cerveau, un cancer de la moelle osseuse, un cancer du col de l'utérus, une leucémie lymphoïde chronique, un cancer colorectal, un cancer de l'œsophage, un cancer hépatocellulaire, une leucémie lymphoblastique, un lymphome folliculaire, des tumeurs malignes lymphoïdes d'origine lymphocytaire T ou B, un mélanome, une leucémie myéloïde, un myélome, un cancer de la cavité buccale, un cancer de l'ovaire, un cancer du poumon non à petites cellules, une leucémie lymphoïde chronique, un cancer de la prostate, un cancer du poumon à petites cellules ou un cancer de la rate.

**23.** Procédé de la revendication 22, dans lequel l'échantillon est un échantillon de biopsie tissulaire, un échantillon de sang ou un échantillon de moelle osseuse.

**24.** Procédé de production du conjugué anticorps-médicament de l'une quelconque des revendications 1 à 14, comprenant la réaction d'un anticorps ou d'un fragment de liaison à l'antigène avec un lieur clivable lié à un inhibiteur de MCL1 dans des conditions qui permettent la conjugaison.

**25.** Conjugué anticorps-médicament de l'une quelconque des revendications 1 à 14, composition de la revendication 15, ou composition pharmaceutique de la revendication 16, destiné(e) à être utilisé(e) selon l'une quelconque des revendications 17 à 21, comprenant en outre l'administration au sujet en ayant besoin d'au moins un agent thérapeutique supplémentaire, de préférence l'agent thérapeutique supplémentaire est un inhibiteur de Bcl-2, idéalement l'agent thérapeutique supplémentaire est le vénétoclax, le composé A1 ou le composé A2.

FIG. 1A

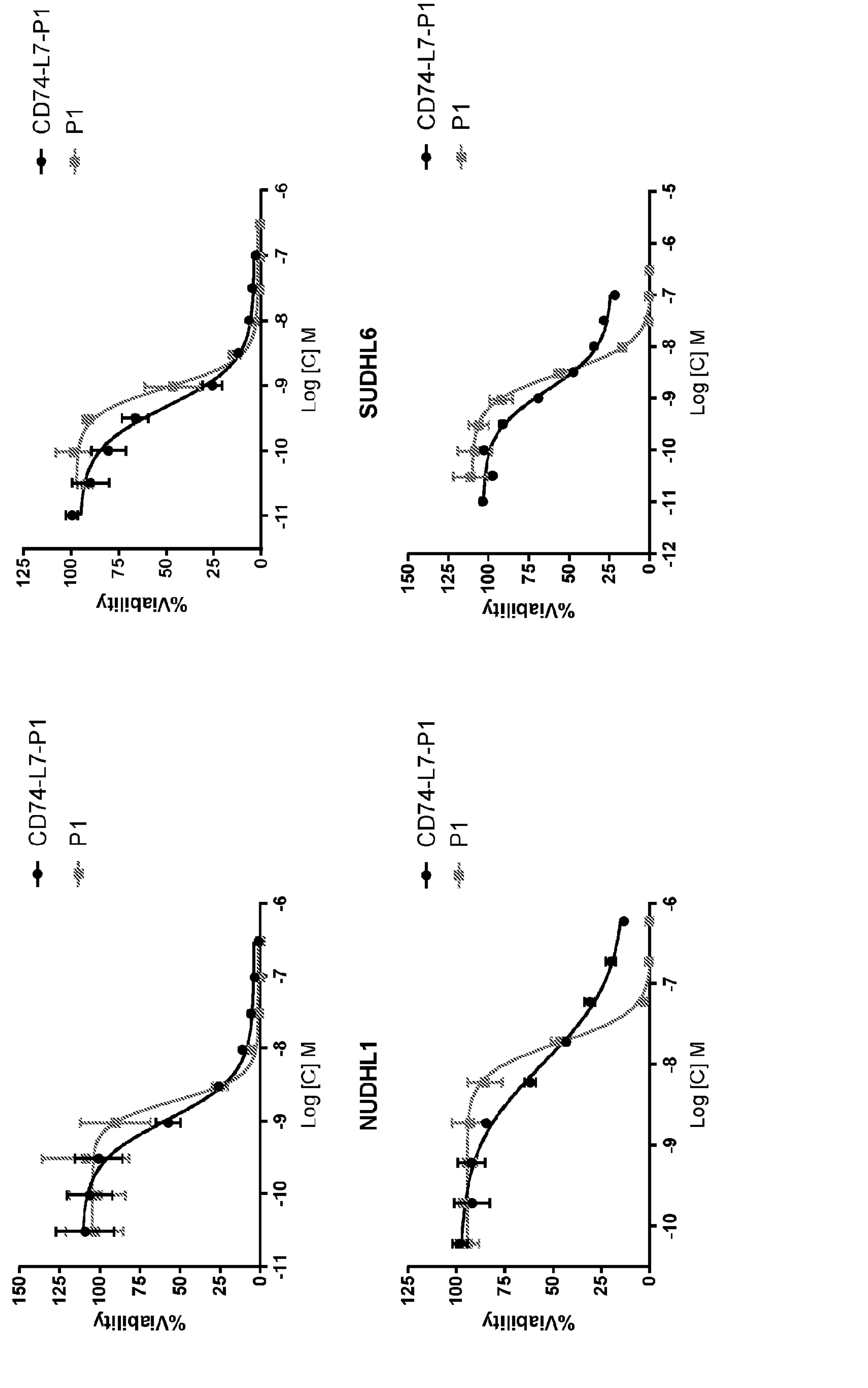
KARPAS422
%Viability
150
125
100
75
50
25
0
-11
-10
-9
-8
-7
-6
Log [C] M
CD74-L7-P1
P1
SUDHL4
%Viability
125
100
75
50
25
0
-11
-10
-9
-8
-7
-6
Log [C] M
CD74-L7-P1
P1
NUDHL1
%Viability
125
100
75
50
25
0
-10
-9
-8
-7
-6
Log [C] M
CD74-L7-P1
P1
SUDHL6
%Viability
150
125
100
75
50
25
0
-12
-11
-10
-9
-8
-7
-6
-5
Log [C] M
CD74-L7-P1
P1

FIG. 1B

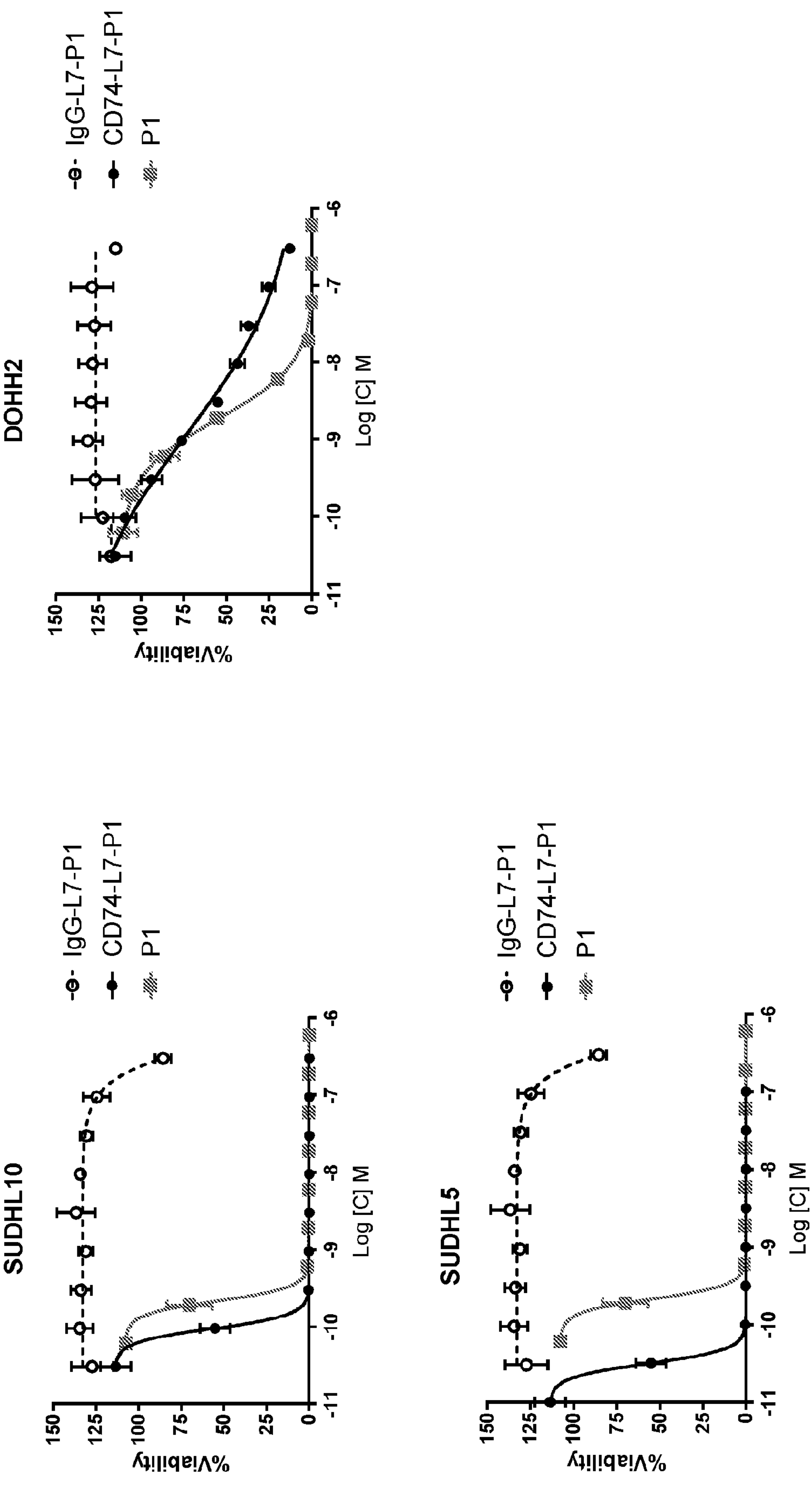
DOHH2
SUDHL10
SUDHL5
%Viability
Log [C] M
150
125
100
75
50
25
0
-11
-10
-9
-8
-7
-6
IgG-L7-P1
CD74-L7-P1
P1

FIG. 2 In vitro activity of CD74 ADCs and payloads in Monomac1 cell line (CTG 72h)

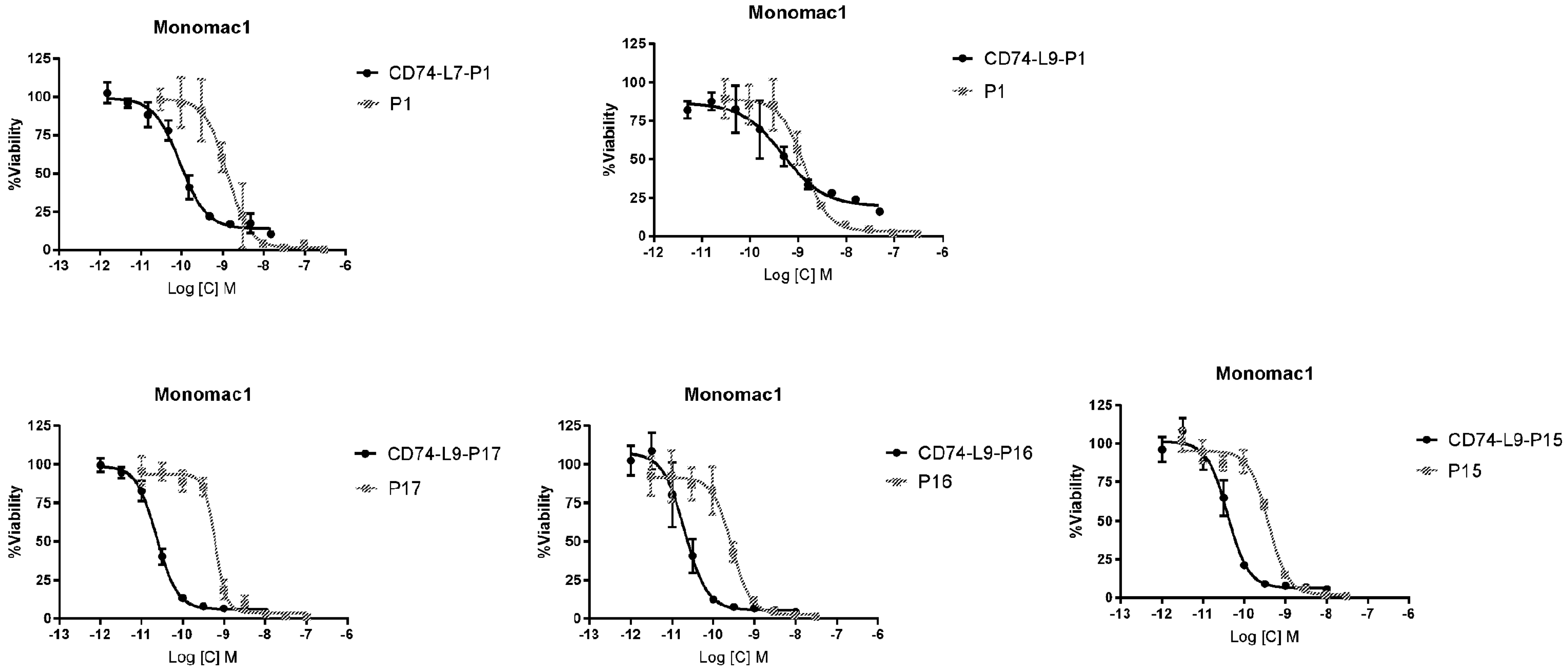

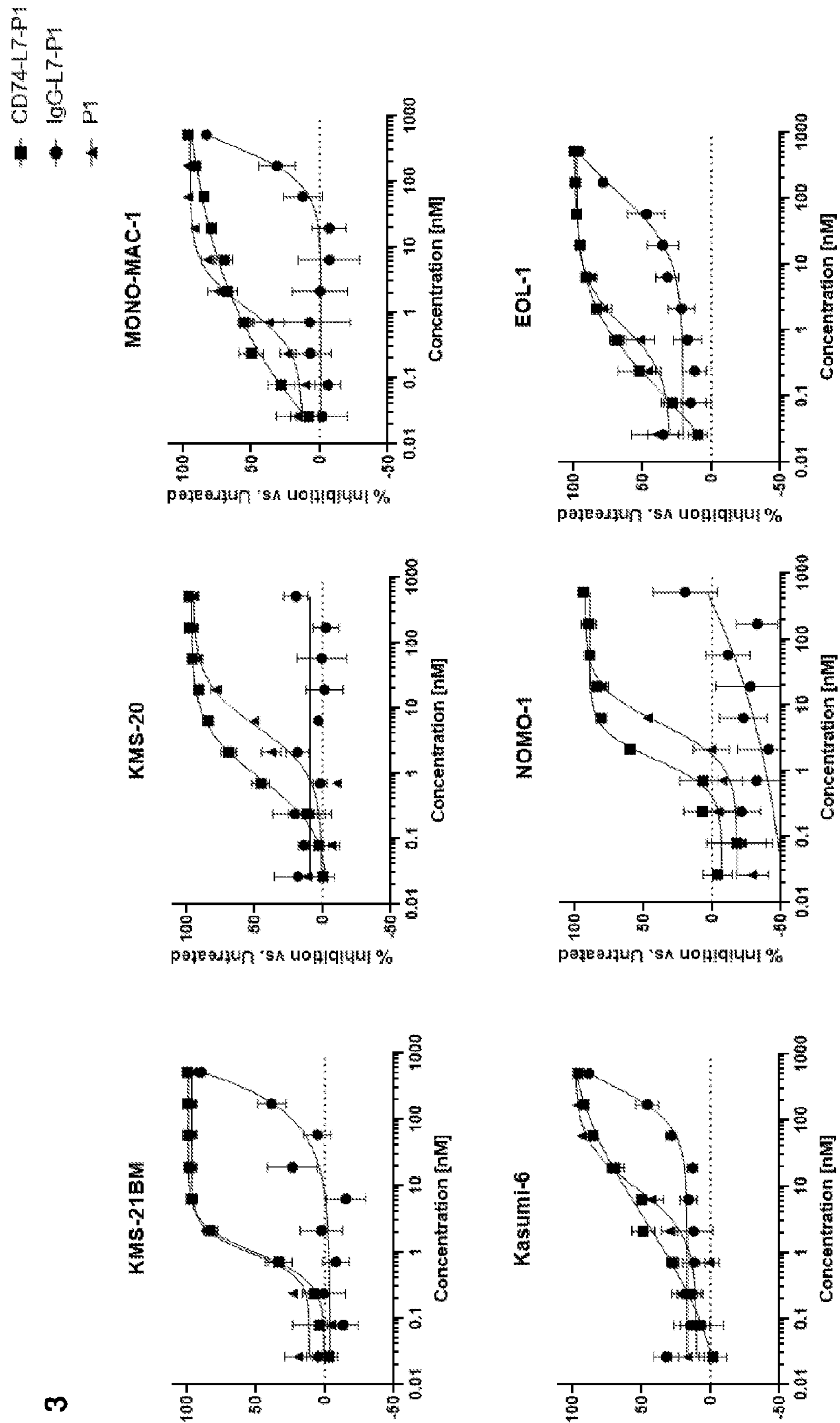
CD74-L7-P1
IgG-L7-P1
P1
KMS-21BM
KMS-20
MONO-MAC-1
Kasumi-6
NOMO-1
EOL-1
% inhibition vs. Untreated
Concentration [nM]
100
50
0
-50
0.01
0.1
1
10
100
1000

FIG. 3

FIG. 4

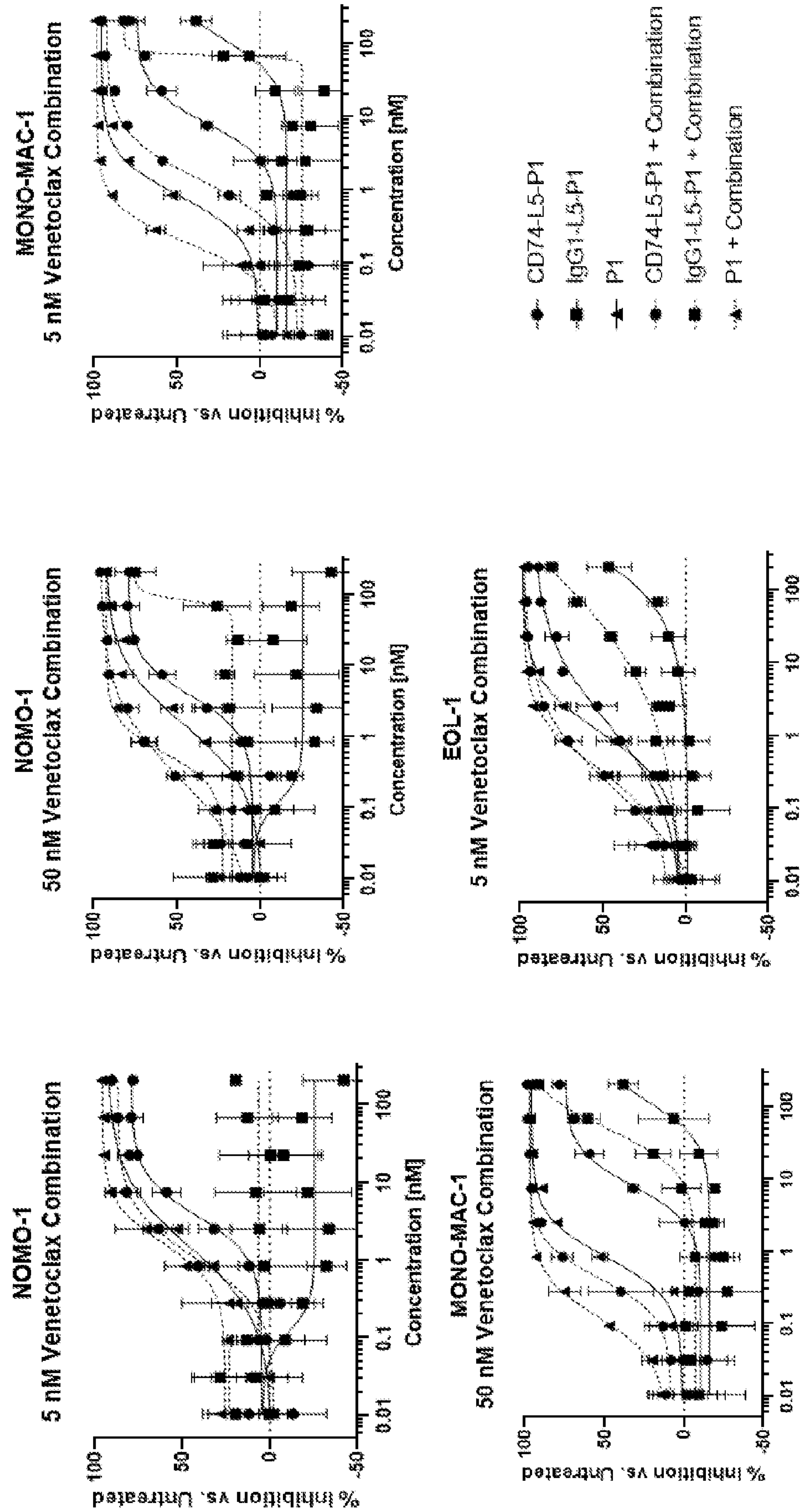
NOMO-1
5 nM Venetoclax Combination
NOMO-1
50 nM Venetoclax Combination
MONO-MAC-1
5 nM Venetoclax Combination
MONO-MAC-1
50 nM Venetoclax Combination
EOL-1
5 nM Venetoclax Combination
% inhibition vs. Untreated
100
50
0
-50
Concentration [nM]
0.01
0.1
1
10
100
CD74-L5-P1
IgG1-L5-P1
P1
CD74-L5-P1 + Combination
IgG1-L5-P1 + Combination
P1 + Combination

FIG. 5

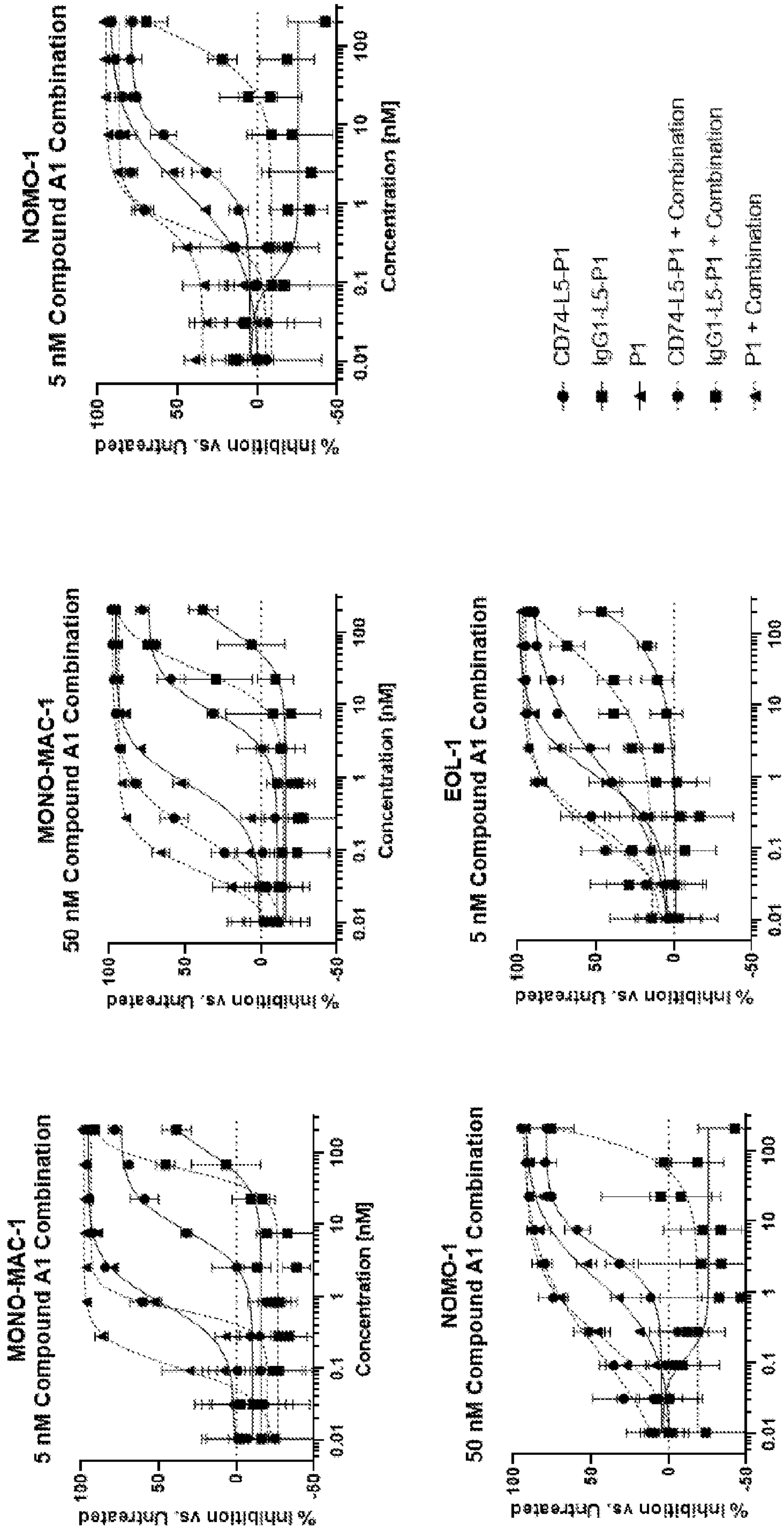
MONO-MAC-1
5 nM Compound A1 Combination
MONO-MAC-1
50 nM Compound A1 Combination
NOMO-1
5 nM Compound A1 Combination
NOMO-1
50 nM Compound A1 Combination
EOL-1
5 nM Compound A1 Combination
% inhibition vs. Untreated
Concentration [nM]
100
50
0
-50
0.01
0.1
1
10
100
CD74-L5-P1
IgG1-L5-P1
P1
CD74-L5-P1 + Combination
IgG1-L5-P1 + Combination
P1 + Combination

FIG. 6

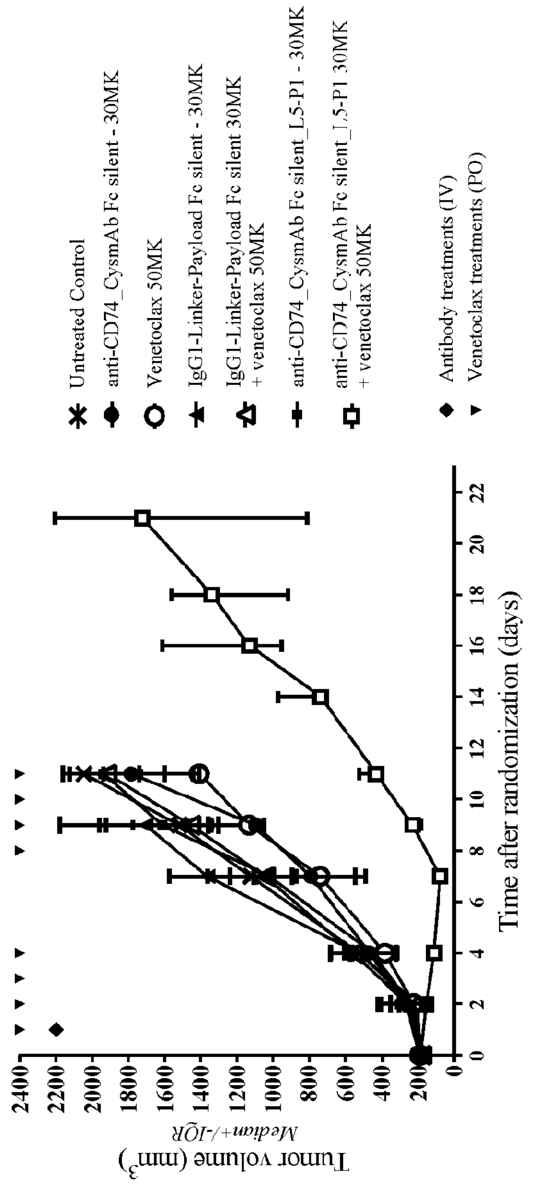
Untreated Control
anti-CD74_CysmAb Fc silent - 30MK
Venetoclax 50MK
IgG1-Linker-Payload Fc silent - 30MK
IgG1-Linker-Payload Fc silent 30MK + venetoclax 50MK
anti-CD74_CysmAb Fc silent_L5-P1 - 30MK
anti-CD74_CysmAb Fc silent_L5-P1 30MK + venetoclax 50MK
Antibody treatments (IV)
Venetoclax treatments (PO)
Tumor volume (mm3)
Median+/-IQR
Time after randomization (days)
0
200
400
600
800
1000
1200
1400
1600
1800
2000
2200
2400
0
2
4
6
8
10
12
14
16
18
20
22

FIG. 7

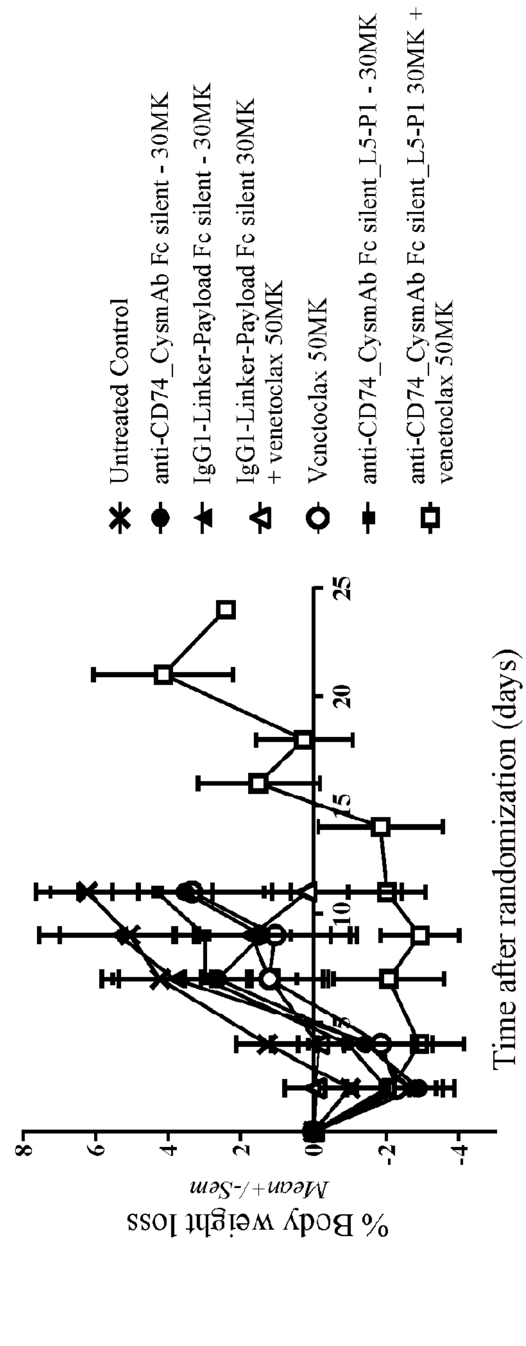
% Body weight loss
Mean+/-Sem
8
6
4
2
0
-2
-4
5
10
15
20
25
Time after randomization (days)
Untreated Control
anti-CD74_CysmAb Fc silent - 30MK
IgG1-Linker-Payload Fc silent - 30MK
IgG1-Linker-Payload Fc silent 30MK + venetoclax 50MK
Venetoclax 50MK
anti-CD74_CysmAb Fc silent_L5-P1 - 30MK
anti-CD74_CysmAb Fc silent_L5-P1 30MK + venetoclax 50MK

FIG. 8

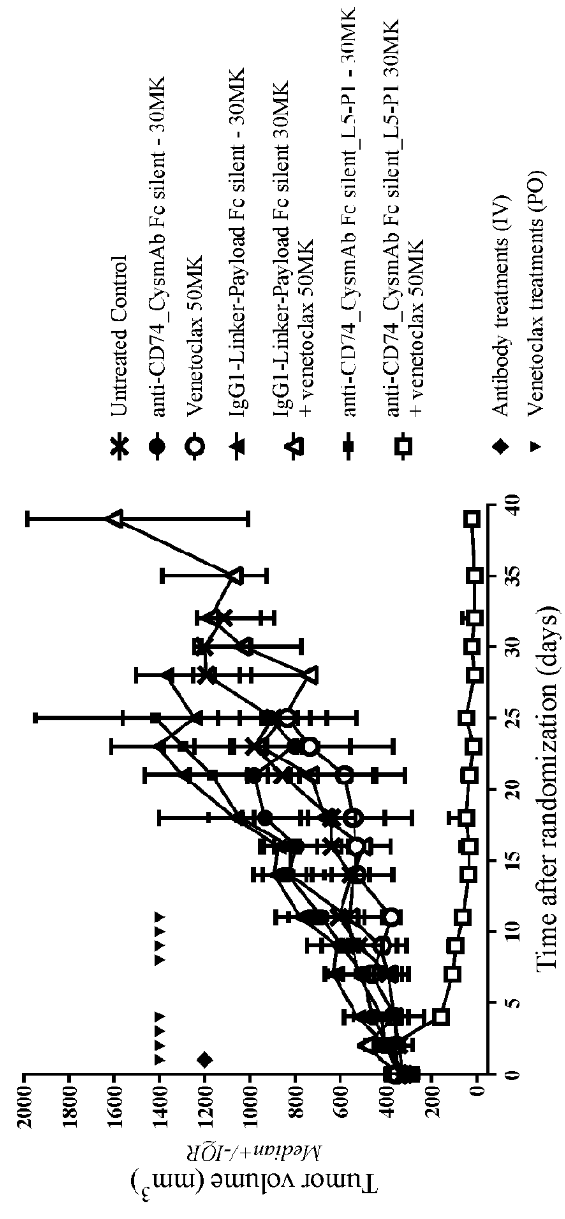
Tumor volume (mm3)
Median+/-IQR
2000
1800
1600
1400
1200
1000
800
600
400
200
0
0
5
10
15
20
25
30
35
40
Time after randomization (days)
Untreated Control
anti-CD74_CysmAb Fc silent - 30MK
Venetoclax 50MK
IgG1-Linker-Payload Fc silent - 30MK
IgG1-Linker-Payload Fc silent 30MK + venetoclax 50MK
anti-CD74_CysmAb Fc silent_L5-P1 - 30MK
anti-CD74_CysmAb Fc silent_L5-P1 30MK + venetoclax 50MK
Antibody treatments (IV)
Venetoclax treatments (PO)

FIG. 9

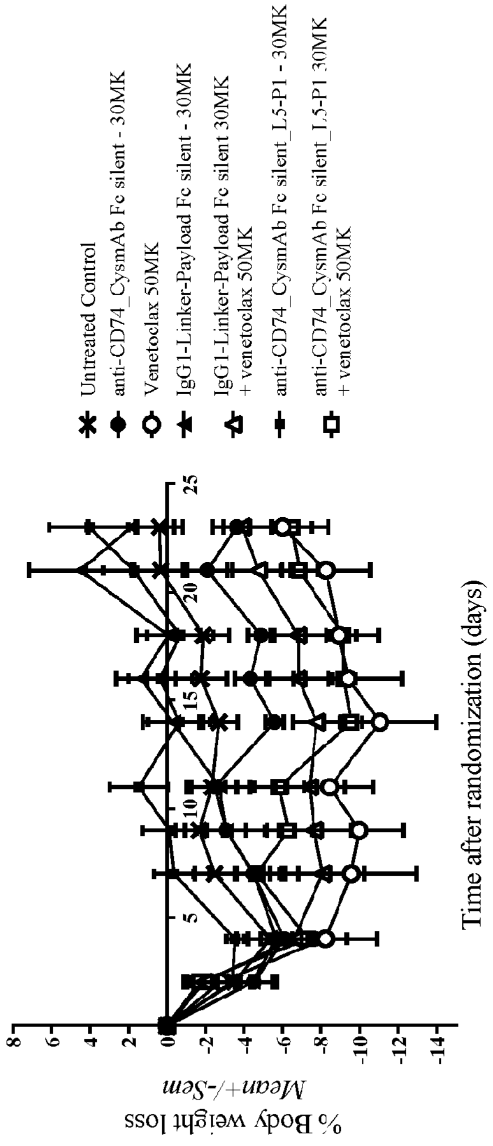
% Body weight loss
Mean+/-Sem
8
6
4
2
0
-2
-4
-6
-8
-10
-12
-14
5
10
15
20
25
Time after randomization (days)
Untreated Control
anti-CD74_CysmAb Fc silent - 30MK
Venetoclax 50MK
IgG1-Linker-Payload Fc silent - 30MK
IgG1-Linker-Payload Fc silent 30MK + venetoclax 50MK
anti-CD74_CysmAb Fc silent_L5-P1 - 30MK
anti-CD74_CysmAb Fc silent_L5-P1 30MK + venetoclax 50MK

FIG. 10

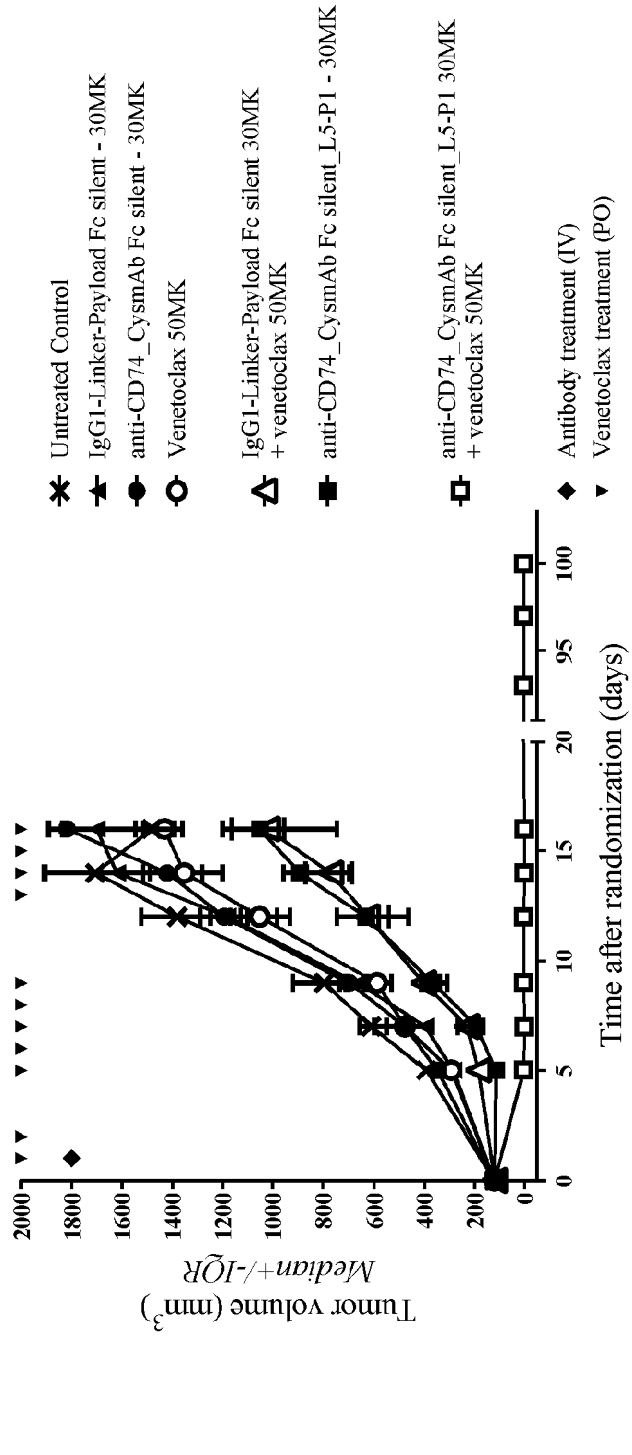

Tumor volume (mm³)
Median+/-IQR
2000
1800
1600
1400
1200
1000
800
600
400
200
0
0
5
10
15
20
95
100
Time after randomization (days)
Untreated Control
IgG1-Linker-Payload Fc silent - 30MK
anti-CD74_CysmAb Fc silent - 30MK
Venetoclax 50MK
IgG1-Linker-Payload Fc silent 30MK + venetoclax 50MK
anti-CD74_CysmAb Fc silent_L5-P1 - 30MK
anti-CD74_CysmAb Fc silent_L5-P1 30MK + venetoclax 50MK
Antibody treatment (IV)
Venetoclax treatment (PO)

FIG. 11

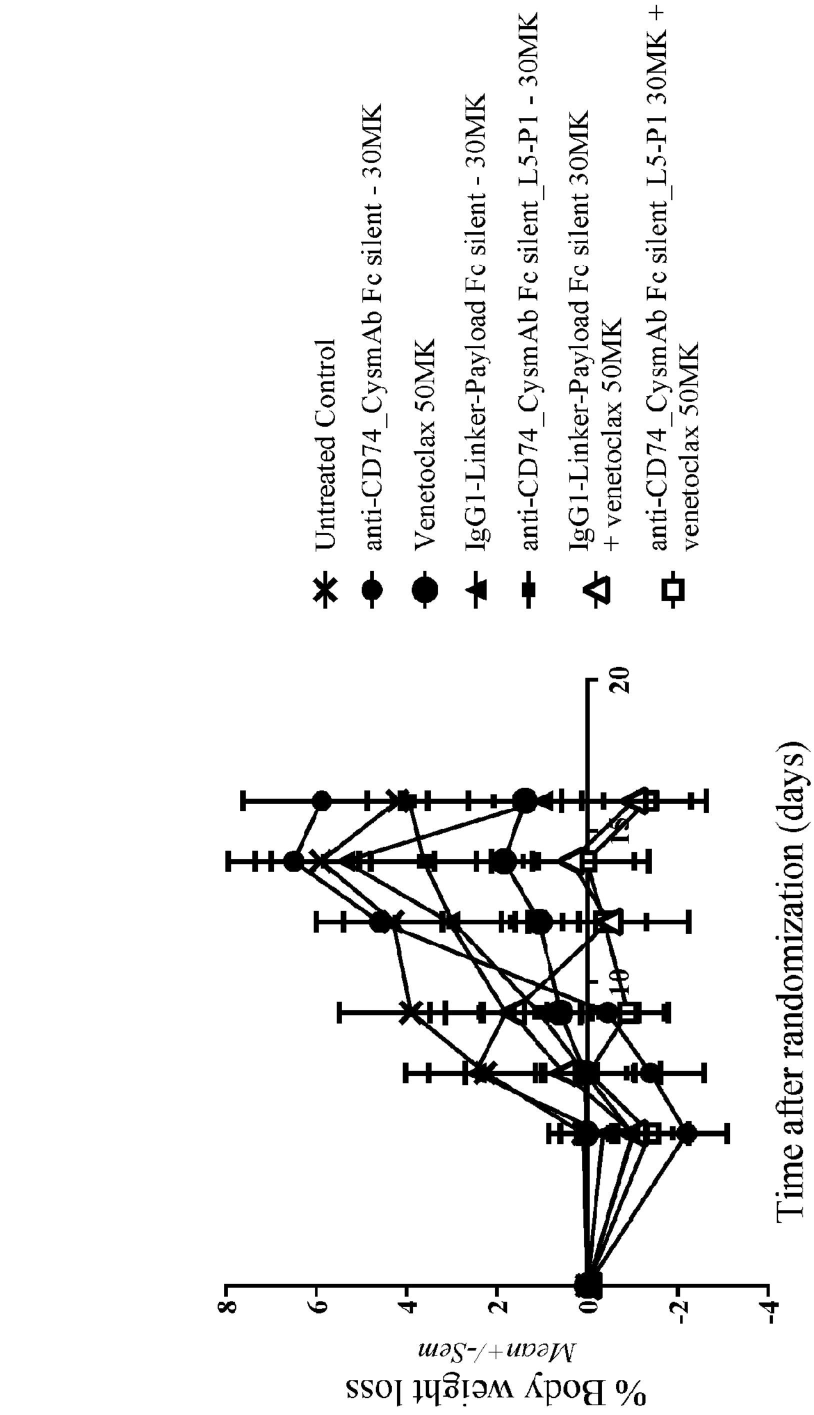
% Body weight loss
Mean+/-Sem
8
6
4
2
0
-2
-4
10
15
20
Time after randomization (days)
Untreated Control
anti-CD74_CysmAb Fc silent - 30MK
Venetoclax 50MK
IgG1-Linker-Payload Fc silent - 30MK
anti-CD74_CysmAb Fc silent_L5-P1 - 30MK
IgG1-Linker-Payload Fc silent 30MK + venetoclax 50MK
anti-CD74_CysmAb Fc silent_L5-P1 30MK + venetoclax 50MK

FIG. 12

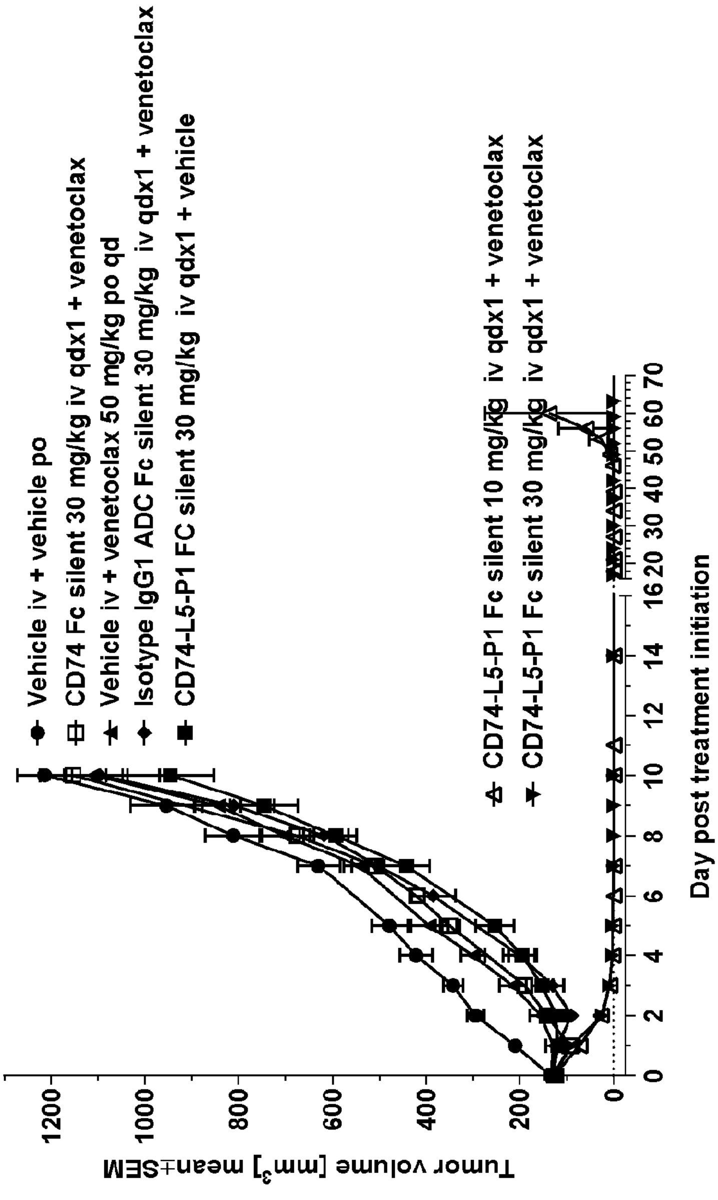
Vehicle iv + vehicle po
CD74 Fc silent 30 mg/kg iv qdx1 + venetoclax
Vehicle iv + venetoclax 50 mg/kg po qd
Isotype IgG1 ADC Fc silent 30 mg/kg iv qdx1 + venetoclax
CD74-L5-P1 FC silent 30 mg/kg iv qdx1 + vehicle
CD74-L5-P1 Fc silent 10 mg/kg iv qdx1 + venetoclax
CD74-L5-P1 Fc silent 30 mg/kg iv qdx1 + venetoclax
Tumor volume [mm3] mean±SEM
Day post treatment initiation
0
200
400
600
800
1000
1200
0
2
4
6
8
10
12
14
16
20
30
40
50
60
70

FIG. 13

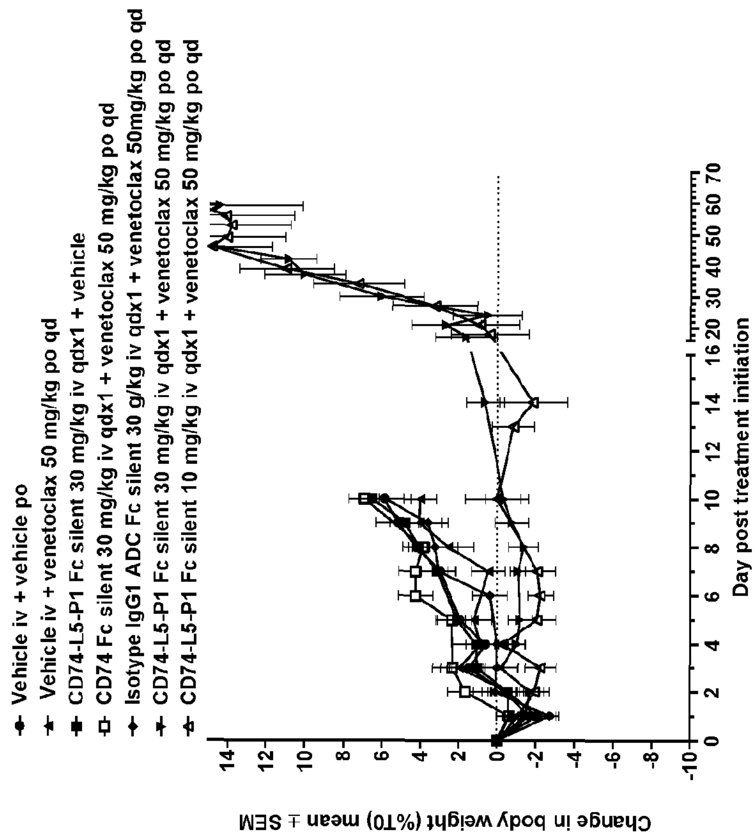
Vehicle iv + vehicle po
Vehicle iv + venetoclax 50 mg/kg po qd
CD74-L5-P1 Fc silent 30 mg/kg iv qdx1 + vehicle
CD74 Fc silent 30 mg/kg iv qdx1 + venetoclax 50 mg/kg po qd
Isotype IgG1 ADC Fc silent 30 g/kg iv qdx1 + venetoclax 50mg/kg po qd
CD74-L5-P1 Fc silent 30 mg/kg iv qdx1 + venetoclax 50 mg/kg po qd
CD74-L5-P1 Fc silent 10 mg/kg iv qdx1 + venetoclax 50 mg/kg po qd
Change in body weight (%T0) mean ± SEM
14
12
10
8
6
4
2
0
-2
-4
-6
-8
-10
0 2 4 6 8 10 12 14 16 20 30 40 50 60 70
Day post treatment initiation

FIG. 14

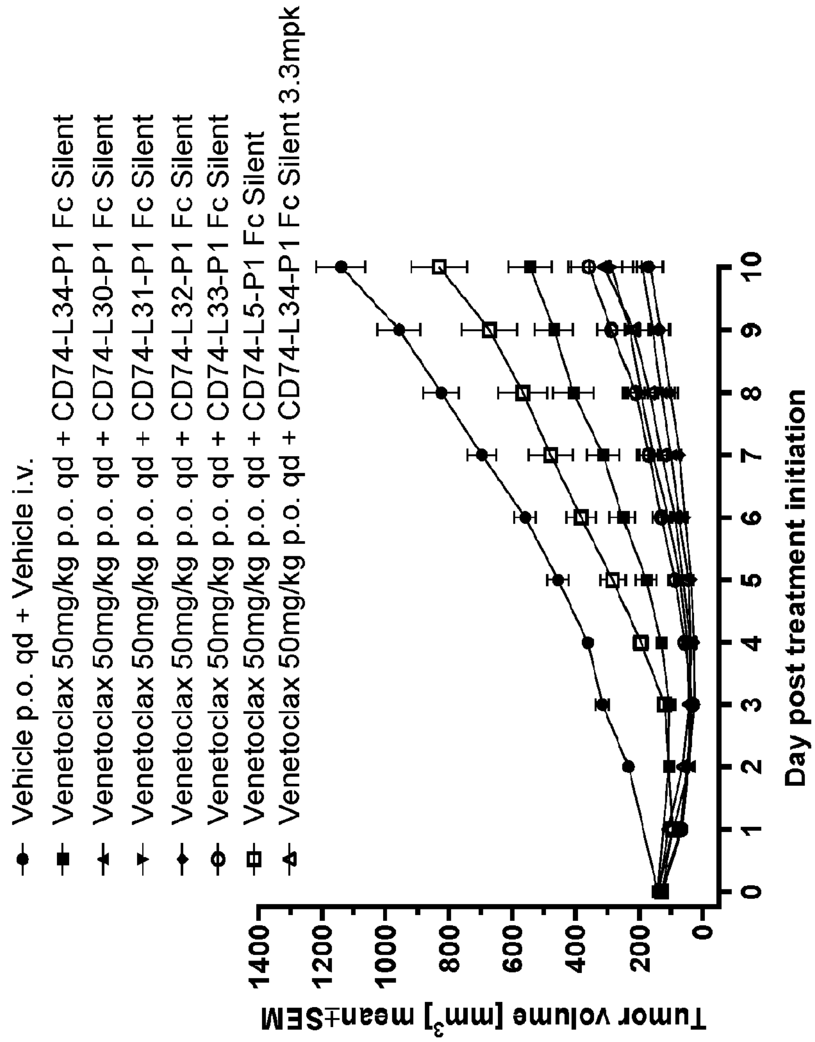
Vehicle p.o. qd + Vehicle i.v.
Venetoclax 50mg/kg p.o. qd + CD74-L34-P1 Fc Silent
Venetoclax 50mg/kg p.o. qd + CD74-L30-P1 Fc Silent
Venetoclax 50mg/kg p.o. qd + CD74-L31-P1 Fc Silent
Venetoclax 50mg/kg p.o. qd + CD74-L32-P1 Fc Silent
Venetoclax 50mg/kg p.o. qd + CD74-L33-P1 Fc Silent
Venetoclax 50mg/kg p.o. qd + CD74-L5-P1 Fc Silent
Venetoclax 50mg/kg p.o. qd + CD74-L34-P1 Fc Silent 3.3mpk
Tumor volume [mm3] mean±SEM
1400
1200
1000
800
600
400
200
0
0
1
2
3
4
5
6
7
8
9
10
Day post treatment initiation

FIG. 15

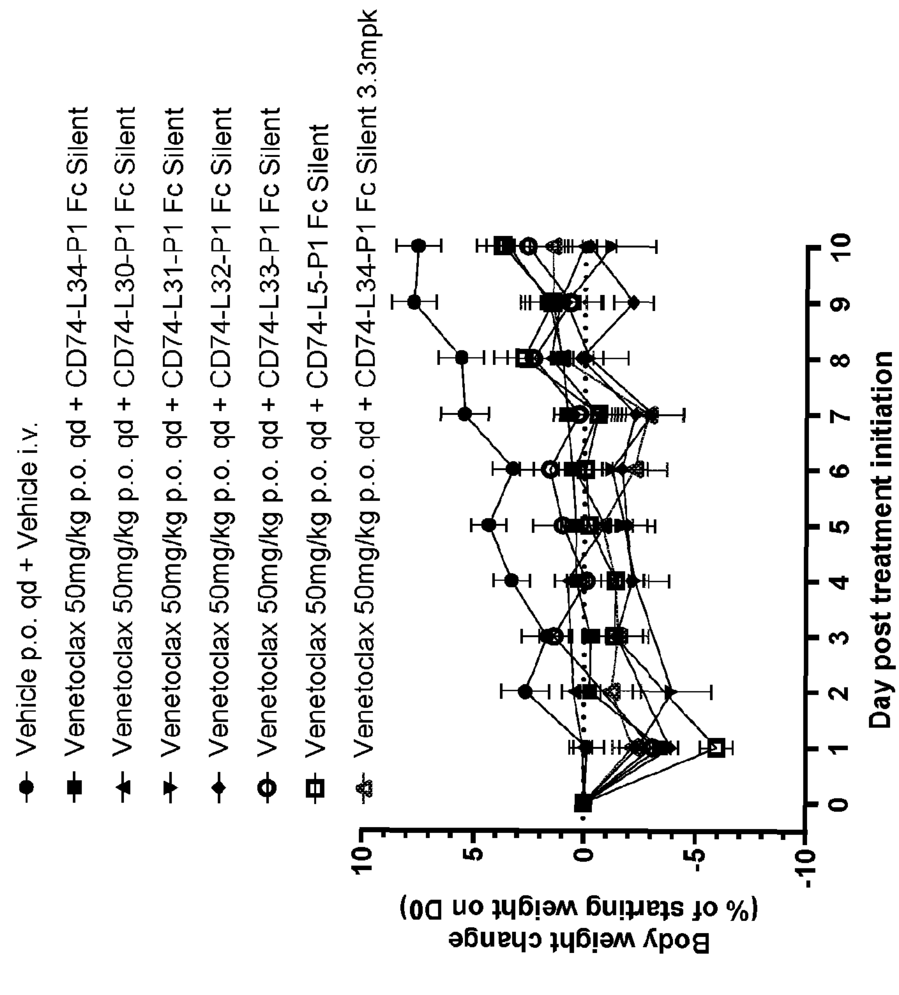
Vehicle p.o. qd + Vehicle i.v.
Venetoclax 50mg/kg p.o. qd + CD74-L34-P1 Fc Silent
Venetoclax 50mg/kg p.o. qd + CD74-L30-P1 Fc Silent
Venetoclax 50mg/kg p.o. qd + CD74-L31-P1 Fc Silent
Venetoclax 50mg/kg p.o. qd + CD74-L32-P1 Fc Silent
Venetoclax 50mg/kg p.o. qd + CD74-L33-P1 Fc Silent
Venetoclax 50mg/kg p.o. qd + CD74-L5-P1 Fc Silent
Venetoclax 50mg/kg p.o. qd + CD74-L34-P1 Fc Silent 3.3mpk
Body weight change
(% of starting weight on D0)
10
5
0
-5
-10
0
1
2
3
4
5
6
7
8
9
10
Day post treatment initiation

FIG. 16

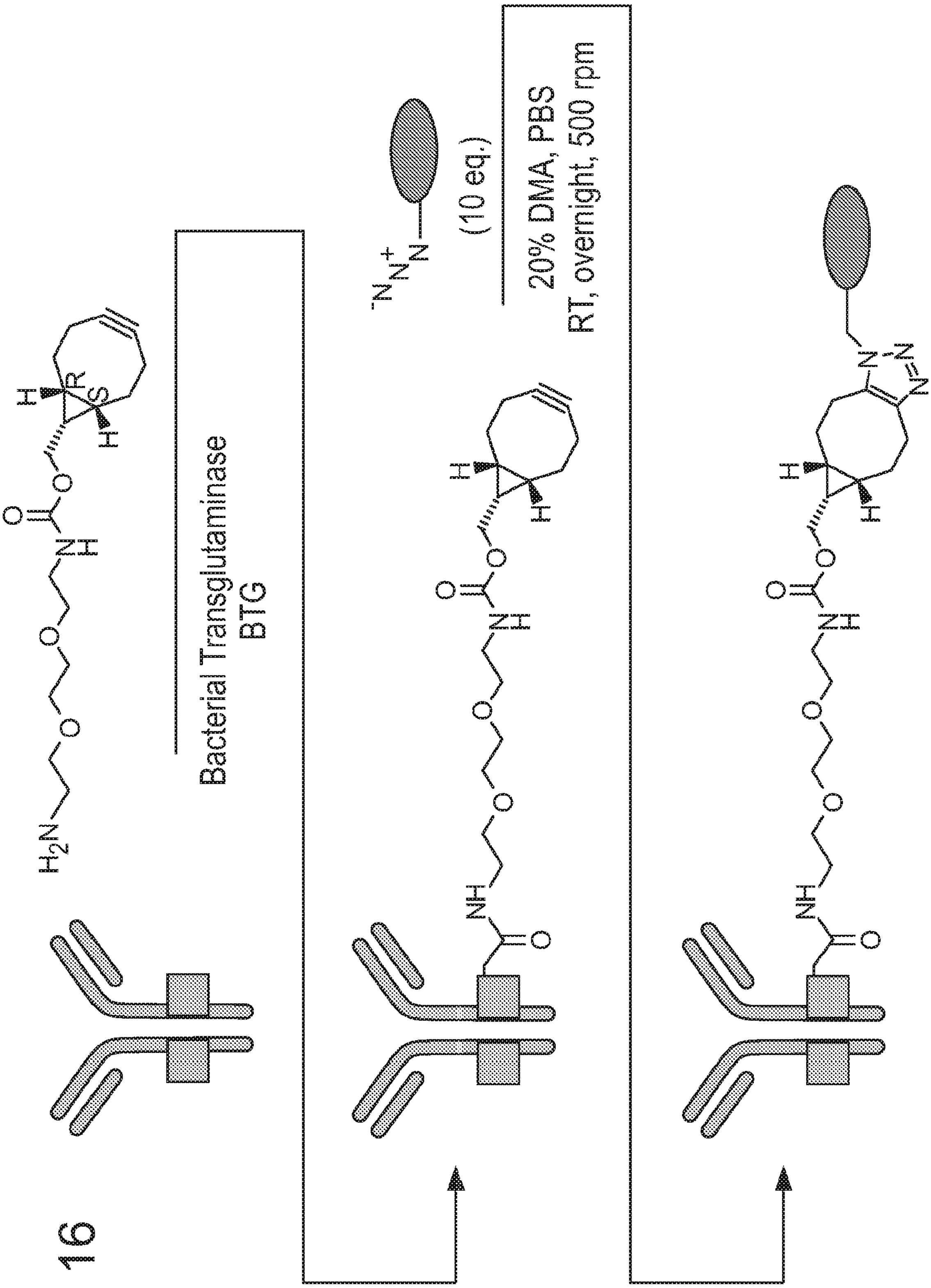
Bacterial Transglutaminase
BTG
(10 eq.)
20% DMA, PBS
RT, overnight, 500 rpm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2886545 A1
- WO 2016207216 A1
- WO 2019035899 A1
- US 6703199 B
- US 4816567 A
- WO 2015097123 A
- WO 2016207216 A
- WO 2016207217 A
- WO 2016207225 A
- WO 2016207226 A
- WO 2017125224 A
- WO 2019035899 A
- WO 2019035911 A
- WO 2019035914 A
- WO 2019035927 A
- US 20190055264 A
- WO 2016033486 A
- WO 2017147410 A
- WO 2018183418 A
- WO 2017182625 A
- US 7931903 B
- US 20030153043 A, Carr
- US 5677425 A, Bodmer
- WO 2011005481 A
- WO 2014124316 A
- WO 2015138615 A
- WO 2014124258 A
- WO 2013184514 A
- US 6165745 A, Ward
- US 5624821 A
- US 5648260 A, Winter
- US 6194551 B, Idusogie
- WO 9429351 A, Bodmer
- US 5714350 A
- US 6350861 B, Co
- US 6277375 B
- US 5869046 A
- US 6121022 A, Presta
- US 20190055264
- US 20140069822 A
- WO 2006034488 A
- WO 2008016893 A
- WO 2014130310 A
- WO 2001051056 A
- US 7612114 B
- WO 2015136017 A
- WO 2011156518 A
- WO 2011159769 A
- WO 2012037410 A
- WO 2012037411 A
- US 20120071535 A
- US 8415355 B
- US 8685980 B
- WO 2010020675 A
- WO 2011076786 A
- WO 2013110890 A
- WO 2015011400 A
- WO 2018200812 A
- WO 2017214456 A
- WO 2017214458 A
- WO 2017214462 A
- WO 2017214233 A
- WO 2017214282 A
- WO 2017214301 A
- WO 2017214322 A
- WO 2017214335 A
- WO 2017214339 A
- WO 2016094509 A
- WO 2016094517 A
- WO 2016094505 A
- WO 2014083505 A
- EP 2886545 A


### Non-patent literature cited in the description


- **CORY et al.** *Nature Review Cancer*, 2002, vol. 2, 647-656
- **HANAHAN et al.** *Cell*, 2000, vol. 100, 57-70
- **BEROUKHIM et al.** *Nature*, 2010, vol. 463 (7283), 899-905
- **CRISTINA L. ABRAHAMS et al.** Targeting CD74 in multiple myeloma with the novel, site-specific antibody-drug conjugate STRO-001. *ONCOTARGET*, 28 December 2018, vol. 9 (102), 37700-37714

- **S. V. GOVINDAN et al.** Milatuzumab-SN-38 Conjugates for the Treatment of CD74+ Cancers. *MOLECULAR CANCER THERAPEUTICS*, 01 June 2013, vol. 12 (6), 968-978
- **HOLLIGER** ; **HUDSON**. *Nat Biotechnol.*, 2005, vol. 23 (9), 1126-36
- **KABAT et al.** Sequences of Proteins of Immunological Interest. 1991
- **AL-LAZIKANI et al.** *J Mol Biol.*, 1997, vol. 273 (4), 927-48
- **LEFRANC**. *Nucleic Acids Res.*, 2001, vol. 29 (1), 207-9
- **LEFRANC et al.** *Dev Comp Immunol.*, 2003, vol. 27 (1), 55-77
- **KOHLER et al.** *Nature*, 1975, vol. 256, 495
- **CLACKSON et al.** *Nature*, 1991, vol. 352, 624-8
- **MARKS et al.** *J Mol Biol.*, 1991, vol. 222, 581-97
- **KNAPPIK et al.** *J Mol Biol.*, 2000, vol. 296 (1), 57-86
- **BAST et al.** *J Clin Invest.*, 1981, vol. 68 (5), 1331-7
- **SCHOLLER** ; **URBAN**. *Biomark Med.*, 2007, vol. 1 (4), 513-23
- **BOUDOUSQ et al.** *PLoS One*, 2013, vol. 8 (7), e69613
- **GERSHONI et al.** *BioDrugs*, 2007, vol. 21, 145-56
- **HAGER-BRAUN** ; **TOMER**. *Expert Rev Proteomics*, 2005, vol. 2, 745-56
- Antibodies, A Laboratory Manual. Cold Spring Harbor Laboratory, 1988
- **TZARTOS**. Methods in Molecular Biology. 1998, vol. 66, 55-66
- **NEEDLEMAN** ; **WUNSCH**. *J Mol Biol.*, 1970, vol. 48, 444-53
- **MEYERS** ; **MILLER**. *CABIOS*, 1989, vol. 4, 11-17
- Remington's Pharmaceutical Sciences. Mack Printing Company, 1990, 1289-1329
- **HAYNES et al.** Commentary: Occurrence of Pharmaceutically Acceptable Anions and Cations in the Cambridge Structural Database. *J. Pharmaceutical Sciences*, 2005, vol. 94 (10)
- **BERGE et al.** Pharmaceutical Salts. *J. Pharmaceutical Sciences*, 1977, vol. 66 (1)
- **AB et al.** *Mol Cancer Ther.*, 2015, vol. 14, 1605-13
- **JUNUTULA JR et al.** *Nat Biotechnol*, 2008, vol. 26, 925-932
- **LYONS et al.** *Protein Eng.*, 1990, vol. 3, 703-708
- **W. OU et al.** *PNAS*, 2011, vol. 108 (26), 10437-10442
- **J.Y. AXUP et al.** *Proc Natl Acad Sci U S A*, 2012, vol. 109, 16101-16106
- **C.C. LIU** ; **P.G. SCHULTZ**. *Annu Rev Biochem*, 2010, vol. 79, 413-444
- **C.H. KIM et al.** *Curr Opin Chem Biol.*, 2013, vol. 17, 412-419
- **STROP P. et al.** *Chem Biol.*, 2013, vol. 20 (2), 161-7
- **RABUKA D.** *Curr Opin Chem Biol.*, December 2010, vol. 14 (6), 790-6
- **RABUKA D et al.** *Nat Protoc.*, 2012, vol. 7 (6), 1052-67
- **GRÜNEWALD et al.** *Bioconjugate Chem.*, 2015, vol. 26 (12), 2554-62
- **JEFFERIS et al.** *MAbs.*, 2009, vol. 1, 332-338
- **DUCRY** ; **STUMP**. *Bioconjugate Chem.*, 2010, vol. 21, 5-13
- *Angew. Chem. Int. Ed.*, 2015, vol. 54, 7492-7509
- **BRINGMANN et al.** *Angew. Chem. Int. Ed.*, 2005, vol. 44, 5384-5427
- **STEFANO et al.** *Methods Mol Biol.*, 2013, vol. 1045, 145-71
- **LYON et al.** *Methods Enzymol.*, 2012, vol. 502, 123-38
- *Biochemica*, 1999, vol. 2, 34-7
- **DUKE** ; **COHEN et al.** *Current Protocols in Immunology*, 1992
- **PAGE et al.** *Intl J Oncology*, 1993, vol. 3, 473-6
- **KLEIN et al.** *Nature Med.*, 1997, vol. 3, 402-8
- Remington's Pharmaceutical Sciences. Mack Publishing Co.
- **BOER K. et al.** *Therapeutic Advances in Medical Oncology*, 2017, vol. 9 (7), 465-479
- *CHEMICAL ABSTRACTS*, 129453-61-8
- *CHEMICAL ABSTRACTS*, 722533-56-4
- **GARNER F et al.** *Anticancer Drugs*, 2015, vol. 26 (9), 948-56
- *CHEMICAL ABSTRACTS*, 1365888-06-7
- **MCDONELL et al.** *Journal of Medicinal Chemistry*, 2015, vol. 58 (12), 4883-4887
- *CHEMICAL ABSTRACTS*, 1211441-98-3
- *CHEMICAL ABSTRACTS*, 1231929-97-7
- **TORRES-GUZMAN R et al.** *Oncotarget*, 2017
- *CHEMICAL ABSTRACTS*, 571190-30-2
- **FINN et al.** *Breast Cancer Research*, 2009, vol. 11 (5), R77
- **DAMLE, N.K.** *Nat Biotechnol.*, 2008, vol. 26 (8), 884-885
- **SINGH, S.K.** *Pharm Res.*, 2015, vol. 32 (11), 3541-71
- **EKHOLM et al.** *ChemMedChem*, 2016, vol. 11, 2501-2505
- **CARMICHAEL et al.** *Cancer Res.*, 1987, vol. 47, 936-42